(19) Europäisches Patentamt
European Patent Office
Office européen des brevets

(11) EP 2 166 019 A2

(12) # EUROPEAN PATENT APPLICATION

(43) Date of publication:
**24.03.2010 Bulletin 2010/12**

(21) Application number: **09075245.2**

(22) Date of filing: **03.07.2001**

(51) Int Cl.:
***C07K 14/295*** *(2006.01)* ***C12N 15/31*** *(2006.01)*
***A61K 39/118*** *(2006.01)*

(84) Designated Contracting States:
**AT BE CH CY DE DK ES FI FR GB GR IE IT LI LU MC NL PT SE TR**

(30) Priority:
**03.07.2000 GB 0016363**
**11.07.2000 GB 0017047**
**21.07.2000 GB 0017983**
**07.08.2000 GB 0019368**
**18.08.2000 GB 0020440**
**14.09.2000 GB 0022583**
**10.11.2000 GB 0027549**
**22.12.2000 GB 0031706**

(62) Document number(s) of the earlier application(s) in accordance with Art. 76 EPC:
**01954278.6 / 1 297 005**

(27) Previously filed application:
**03.07.2001 PCT/IB01/01445**

(71) Applicant: **Novartis Vaccines and Diagnostics S.r.l.**
**53100 Siena (SI) (IT)**

(72) Inventors:
- **Ratti, Giulio**
  **deceased (IT)**
- **Grandi, Guido**
  **53100 Siena (IT)**

(74) Representative: **Marshall, Cameron John et al**
**Carpmaels & Ransford**
**43-45 Bloomsbury Square**
**London WC1A 2RA (GB)**

Remarks:
This application was filed on 29-05-2009 as a divisional application to the application mentioned under INID code 62.

(54) **Immunisation against Chlamydia Pneumoniae**

(57) The published genomic of *Chlamydia pneumoniae* reveals over 1000 putative encoded proteins but does not itself indicate which of these might to useful antigens for immunisation and vaccination or for diagnosis. This difficulty is addressed by the invention, which provides a number of *C. pneumoniae* protein sequences suitable for vaccine production and development and/or for diagnostic purposes.

***FIGURE 190***

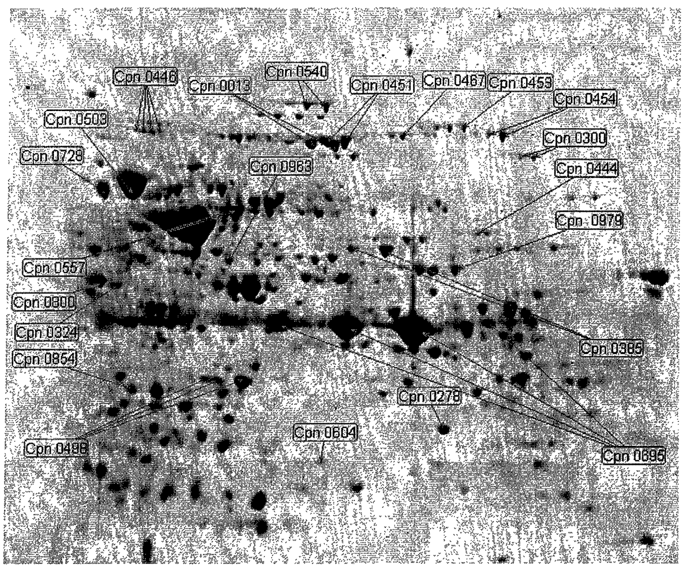


EP 2 166 019 A2

Printed by Jouve, 75001 PARIS (FR)

## Description

All documents cited herein are incorporated by reference in their entirety.

## TECHNICAL FIELD

This invention is in the field of immunisation against chlamydial infection, in particular against infection by *Chlamydia pneumoniae*.

## BACKGROUND ART

*Chlamydiae* are obligate intracellular parasites of eukaryotic cells which are responsible for endemic sexually transmitted infections and various other disease syndromes. They occupy an exclusive eubacterial phylogenic branch, having no close relationship to any other known organisms - they are classified in their own order (*Chlamydiales*) which contains a single family (*Chlamydiaceae*) which in turn contains a single genus (*Chlamydia*). A particular characteristic of the *Chlamydiae* is their unique life cycle, in which the bacterium alternates between two morphologically distinct forms: an extracellular infective form (elementary bodies, EB) and an intracellular non-infective form (reticulate bodies, RB). The life cycle is completed with the re-organization of RB into EB, which subsequently leave the disrupted host cell ready to infect further cells.

Four chlamydial species are currently known - *C.trachomatis, C.pneumoniae, C.pecorum* and *C.psittaci* [*e.g.* Raulston (1995) Mol Microbiol 15:607-616; Everett (2000) Vet Microbiol 75:109-126*]. C.pneumoniae* is closely related to *C.trachomatis,* as the whole genome comparison of at least two isolates from each species has shown [Kalman et al. (1999) Nature Genetics 21:385-389; Read et al. (2000) Nucleic Acids Res 28:1397-406; Stephens et al. (1998) Science 282:754-759]. Based on surface reaction with patient immune sera, the current view is that only one serotype of *C.pneumoniae* exists world-wide.

*C.pneumoniae* is a common cause of human respiratory disease. It was first isolated from the conjunctiva of a child in Taiwan in 1965, and was established as a major respiratory pathogen in 1983. In the USA, *C.pneumoniae* causes approximately 10% of community-acquired pneumonia and 5% of pharyngitis, bronchitis, and sinusitis.

More recently, the spectrum of *C.pneumoniae* infections has been extended to include atherosclerosis, coronary heart disease, carotid artery stenosis, myocardial infarction, cerebrovascular disease, aortic aneurysm, claudication, and stroke. The association of *C.pneumoniae* with atherosclerosis is corroborated by the presence of the organism in atherosclerotic lesions throughout the arterial tree and the near absence of the organism in healthy arterial tissue. *C.pneumoniae* has also been isolated from coronary and carotid atheromatous plaques. The bacterium has also been associated with other acute and chronic respiratory diseases (e.g. otitis media, chronic obstructive pulmonary disease, pulmonary exacerbation of cystic fibrosis) as a result of sero-epidemiologic observations, case reports, isolation or direct detection of the organism in specimens, and successful response to anti-chlamydial antibiotics. To determine whether chronic infection plays a role in initiation or progression of disease, intervention studies in humans have been initiated, and animal models of *C.pneumoniae* infection have been developed.

Considerable knowledge of the epidemiology of *C.pneumoniae* infection has been derived from serologic studies using the *C.pneumoniae*-specific microimmunofluorescence test. Infection is ubiquitous, and it is estimated that virtually everyone is infected at some point in life, with common re-infection. Antibodies against *C.pneumoniae* are rare in children under the age of 5, except in developing and tropical countries. Antibody prevalence increases rapidly at ages 5 to 14, reaching 50% at the age of 20, and continuing to increase slowly to ~80% by age 70.

A current hypothesis is that *C.pneumoniae* can persist in an asymptomatic low-grade infection in very large sections of the human population. When this condition occurs, it believed that the presence of *C.pneumoniae,* and/or the effects of the host reaction to the bacterium, can cause or help progress of cardiovascular illness.

It is not yet clear whether *C.pneumoniae* is actually a causative agent of cardiovascular disease, or whether it is just artefactually associated with it. It has been shown, however, that *C.pneumoniae* infection can induce LDL oxidation by human monocytes [Kalayoglu et al. (1999) J. Infect. Dis. 180:780-90; Kalayoglu et al. (1999) Am. Heart J. 138: S488-490]. As LDL oxidation products are highly atherogenic, this observation provides a possible mechanism whereby *C.pneumoniae* may cause atheromatous degeneration. If a causative effect is confirmed, vaccination (prophylactic and therapeutic) will be universally recommended.

Genomic sequence information has been published for *C.pneumoniae* [Kalman *et al.* (1999) *supra*; Read *et al.* (2000) *supra;* Shirai et al. (2000) J. Infect. Dis. 181(Suppl 3):S524-S527; WO99/27105; WO00/27994] and is available from GenBank. Sequencing efforts have not, however, focused on vaccination, and the availability of genomic sequence does not in itself indicate which of the >1000 genes might encode useful antigens for immunisation and vaccination. WO99/27105, for instance, implies that every one of the 1296 ORFs identified in the *C.pneumoniae* strain CM1 genome is a useful vaccine antigen.

It is thus an object of the present invention to identify antigens useful for vaccine production and development from amongst the many proteins present in *C.pneumoniae.* It is a further object to identify antigens useful for diagnosis (*e.g.* immunodiagnosis) of *C.pneumoniae.*

## DISCLOSURE OF THE INVENTION

The invention provides proteins comprising the *C.pneumoniae* amino acid sequences disclosed in the examples.

It also provides proteins comprising sequences which share at least *x*% sequence identity with the *C.pneumoniae* amino acid sequences disclosed in the examples. Depending on the particular sequence, *x* is preferably 50% or more (*e.g.* 60%, 70%, 80%, 90%, 95%, 99% or more). These include mutants and allelic variants. Typically, 50% identity or more between two proteins is considered to be an indication of functional equivalence. Identity between proteins is preferably determined by the Smith-Waterman homology search algorithm as implemented in the MPSRCH program (Oxford Molecular), using an affine gap search with parameters *gap open penalty=12* and *gap extension penalty=1.*

The invention further provides proteins comprising fragments of the *C.pneumoniae* amino acid sequences disclosed in the examples. The fragments should comprise at least *n* consecutive amino acids from the sequences and, depending on the particular sequence, *n* is 7 or more (*e.g.* 8, 10, 12, 14, 16, 18, 20, 30, 40, 50, 75, 100 or more). Preferably the fragments comprise one or more epitope(s) from the sequence. Other preferred fragments omit a signal peptide.

The proteins of the invention can, of course, be prepared by various means (*e.g.* native expression, recombinant expression, purification from cell culture, chemical synthesis *etc.*) and in various forms (*e.g.* native, fusions *etc.*). They are preferably prepared in substantially pure form (*ie.* substantially free from other *C.pneumoniae* or host cell proteins). Heterologous expression in *E.coli* is a preferred preparative route.

According to a further aspect, the invention provides nucleic acid comprising the *C.pneumoniae* nucleotide sequences disclosed in the examples. In addition, the invention provides nucleic acid comprising sequences which share at least x% sequence identity with the *C.pneumoniae* nucleotide sequences disclosed in the examples. Depending on the particular sequence, *x* is preferably 50% or more (*e.g.* 60%, 70%, 80%, 90%, 95%, 99% or more).

Furthermore, the invention provides nucleic acid which can hybridise to the *C.pneumoniae* nucleic acid disclosed in the examples, preferably under "high stringency" conditions (*e.g.* 65°C in a 0.1xSSC, 0.5% SDS solution).

Nucleic acid comprising fragments of these sequences are also provided. These should comprise at least *n* consecutive nucleotides from the *C.pneumoniae* sequences and, depending on the particular sequence, *n* is 10 or more (*e.g.* 12, 14, 15, 18, 20, 25, 30, 35, 40, 50, 75, 100, 200, 300 or more).

According to a further aspect, the invention provides nucleic acid encoding the proteins and protein fragments of the invention.

It should also be appreciated that the invention provides nucleic acid comprising sequences complementary to those described above (*e.g.* for antisense or probing purposes).

Nucleic acid according to the invention can, of course, be prepared in many ways (*e.g.* by chemical synthesis, from genomic or cDNA libraries, from the organism itself *etc.*) and can take various forms (*e.g.* single stranded, double stranded, vectors, probes *etc.*).

In addition, the term "nucleic acid" includes DNA and RNA, and also their analogues, such as those containing modified backbones, and also peptide nucleic acids (PNA) *etc.*

According to a further aspect, the invention provides vectors comprising nucleotide sequences of the invention (*e.g.* cloning or expression vectors) and host cells transformed therewith.

According to a further aspect, the invention provides immunogenic compositions comprising protein and/or nucleic acid according to the invention. These compositions are suitable for immunisation and vaccination purposes. Vaccines of the invention may be prophylactic or therapeutic, and will typically comprise an antigen which can induce antibodies capable of inhibiting (a) chlamydial adhesion, (b) chlamydial entry, and/or (c) successful replication within the host cell. The vaccines preferably induce any cell-mediated T-cell responses which are necessary for chlamydial clearance from the host.

The invention also provides nucleic acid or protein according to the invention for use as medicaments (*e.g.* as vaccines). It also provides the use of nucleic acid or protein according to the invention in the manufacture of a medicament (*e.g.* a vaccine or an immunogenic composition) for treating or preventing infection due to *C.pneumoniae.*

The invention also provides a method of treating (*e.g.* immunising) a patient, comprising administering to the patient a therapeutically effective amount of nucleic acid or protein according to the invention.

According to further aspects, the invention provides various processes.

A process for producing proteins of the invention is provided, comprising the step of culturing a host cell according to the invention under conditions which induce protein expression.

A process for producing protein or nucleic acid of the invention is provided, wherein the protein or nucleic acid is synthesised in part or in whole using chemical means.

A process for detecting *C.pneuMoniae* in a sample is provided, wherein the sample is contacted with an antibody which binds to a protein of the invention .

A summary of standard techniques and procedures which may be employed in order to perform the invention (*e.g.* to utilise the disclosed sequences for immunisation) follows. This summary is not a limitation on the invention but, rather, gives examples that may be used, but are not required.

## General

The practice of the present invention will employ, unless otherwise indicated, conventional techniques of molecular biology, microbiology, recombinant DNA, and immunology, which are within the skill of the art. Such techniques are explained fully in the literature *e.g.* Sambrook Molecular Cloning; A Laboratory Manual, Second Edition (1989) and Third Edition (2001); DNA Cloning, Volumes I and ii (D.N Glover ed. 1985); Oligonucleotide Synthesis (M.J. Gait ed, 1984); Nucleic Acid Hybridization (B.D. Hames & S.J. Higgins eds. 1984*);* Transcription and Translation (B.D. Hames & S.J. Higgins eds. 1984); Animal Cell Culture (R.I. Freshney ed. 1986); Immobilized Cells and Enzymes (IRL Press, 1986); B. Perbal, A Practical Guide to Molecular Cloning (1984); the Methods in Enzymology series (Academic Press, Inc.), especially volumes 154 & 155; Gene Transfer Vectors for Mammalian Cells (J.H. Miller and M.P. Calos eds. 1987, Cold Spring Harbor Laboratory); Mayer and Walker, eds. (1987), Immunochemical Methods in Cell and Molecular Biology (Academic Press, London); Scopes, (1987) Protein Purification: Principles and Practice, Second Edition (Springer-Verlag, N.Y.), and Handbook of Experimental Immunology, Volumes I-IV (D.M. Weir and C. C. Blackwell eds 1986).

Standard abbreviations for nucleotides and amino acids are used in this specification.

## Definitions

A composition containing X is "substantially free of" Y when at least 85% by weight of the total X+Y in the composition is X. Preferably, X comprises at least about 90% by weight of the total of X+Y in the composition, more preferably at least about 95% or even 99% by weight.

The term "comprising" means "including" as well as "consisting" *e.g.* a composition "comprising" X may consist exclusively of X or may include something additional to X, such as X+Y.

The term "heterologous" refers to two biological components that are not found together in nature. The components may be host cells, genes, or regulatory regions, such as promoters. Although the heterologous components are not found together in nature, they can function together, as when a promoter heterologous to a gene is operably linked to the gene. Another example is where a Chlamydial sequence is heterologous to a mouse host cell. A further examples would be two epitopes from the same or different proteins which have been assembled in a single protein in an arrangement not found in nature.

An "origin of replication" is a polynucleotide sequence that initiates and regulates replication of polynucleotides, such as an expression vector. The origin of replication behaves as an autonomous unit of polynucleotide replication within a cell, capable of replication under its own control. An origin of replication may be needed for a vector to replicate in a particular host cell. With certain origins of replication, an expression vector can be reproduced at a high copy number in the presence of the appropriate proteins within the cell. Examples of origins are the autonomously replicating sequences, which are effective in yeast; and the viral T-antigen, effective in COS-7 cells.

A "mutant" sequence is defined as DNA, RNA or amino acid sequence differing from but having sequence identity with the native or disclosed sequence. Depending on the particular sequence, the degree of sequence identity between the native or disclosed sequence and the mutant sequence is preferably greater than 50% (*e.g.* 60%, 70%, 80%, 90%, 95%, 99% or more, calculated using the Smith-Waterman algorithm as described above). As used herein, an "allelic variant" of a nucleic acid molecule, or region, for which nucleic acid sequence is provided herein is a nucleic acid molecule, or region, that occurs essentially at the same locus in the genome of another or second isolate, and that, due to natural variation caused by, for example, mutation or recombination, has a similar but not identical nucleic acid sequence. A coding region allelic variant typically encodes a protein having similar activity to that of the protein encoded by the gene to which it is being compared. An allelic variant can also comprise an alteration in the 5' or 3' untranslated regions of the gene, such as in regulatory control regions (*e.g.* see US patent 5,753,235).

## Expression systems

The Chlamydial nucleotide sequences can be expressed in a variety of different expression systems; for example those used with mammalian cells, baculoviruses, plants, bacteria, and yeast.

i. Mammalian System

Mammalian expression systems are known in the art. A mammalian promoter is any DNA sequence capable of binding mammalian RNA polymerase and initiating the downstream (3') transcription of a coding sequence (*e.g.* structural gene) into mRNA. A promoter will have a transcription initiating region, which is usually placed proximal to the 5' end of the coding sequence, and a TATA box, usually located 25-30 base pairs (bp) upstream of the transcription initiation site. The TATA box is thought to direct RNA polymerase II to begin RNA synthesis at the correct site. A mammalian promoter will also contain an upstream promoter element, usually located within 100 to 200 bp upstream of the TATA box. An upstream promoter element determines the rate at which transcription is initiated and can act in either orientation [Sambrook et al. (1989) "Expression of Cloned Genes in Mammalian Cells." In Molecular Cloning: A Laboratory Manual, 2nd ed.*].*

Mammalian viral genes are often highly expressed and have a broad host range; therefore sequences encoding mammalian viral genes provide particularly useful promoter sequences. Examples include the SV40 early promoter, mouse mammary tumor virus LTR promoter, adenovirus major late promoter (Ad MLP), and herpes simplex virus promoter. In addition, sequences derived from non-viral genes, such as the murine metallotheionein gene, also provide useful promoter sequences. Expression may be either constitutive or regulated (inducible), depending on the promoter can be induced with glucocorticoid in hormone-responsive cells.

The presence of an enhancer element (enhancer), combined with the promoter elements described above, will usually increase expression levels. An enhancer is a regulatory DNA sequence that can stimulate transcription up to 1000-fold when linked to homologous or heterologous promoters, with synthesis beginning at the normal RNA start site. Enhancers are also active when they are placed upstream or downstream from the transcription initiation site, in either normal or flipped orientation, or at a distance of more than 1000 nucleotides from the promoter [Maniatis et al. (1987) Science 236:1237; Alberts et al. (1989) Molecular Biology of the Cell, 2nd ed.]. Enhancer elements derived from viruses may be particularly useful, because they usually have a broader host range. Examples include the SV40 early gene enhancer [Dijkema et al (1985) EMBO J. 4:761] and the enhancer/promoters derived from the long terminal repeat (LTR) of the Rous Sarcoma Virus [Gorman et al. (1982) PNAS USA 79:6777] and from human cytomegalovirus [Boshart et al. (1985) Cell 41:521]. Additionally, some enhancers are regulatable and become active only in the presence of an inducer, such as a hormone or metal ion [Sassone-Corsi and Borelli (1986) Trends Genet. 2:215; Maniatis et al. (1987) Science 236:1237].

A DNA molecule may be expressed intracellularly in mammalian cells. A promoter sequence may be directly linked with the DNA molecule, in which case the first amino acid at the N-terminus of the recombinant protein will always be a methionine, which is encoded by the ATG start codon. If desired, the N-terminus may be cleaved from the protein by *in vitro* incubation with cyanogen bromide.

Alternatively, foreign proteins can also be secreted from the cell into the growth media by creating chimeric DNA molecules that encode a fusion protein comprised of a leader sequence fragment that provides for secretion of the foreign protein in mammalian cells. Preferably, there are processing sites encoded between the leader fragment and the foreign gene that can be cleaved either *in vivo* or *in vitro.* The leader sequence fragment usually encodes a signal peptide comprised of hydrophobic amino acids which direct the secretion of the protein from the cell. The adenovirus triparite leader is an example of a leader sequence that provides for secretion of a foreign protein in m am m alian cells.

Usually, transcription termination and polyadenylation sequences recognized by mammalian cells are regulatory regions located 3' to the translation stop codon and thus, together with the promoter elements, flank the coding sequence. The 3' terminus of the mature mRNA is formed by site-specific post-transcriptional cleavage and polyadenylation [Birnstiel et al. (1985) Cell 41:349; Proudfoot and Whitelaw (1988) "Termination and 3' end processing of eukaryotic RNA. In Transcription and splicing (ed. B.D. Hames and D.M. Glover); Proudfoot (1989) Trends Biochem. Sci. 14:105]. These sequences direct the transcription of an mRNA which can be translated into the polypeptide encoded by the DNA. Examples of transcription terminater/polyadenylation signals include those derived from SV40 [Sambrook et al (1989) "Expression of cloned genes in cultured mammalian cells." In Molecular Cloning: A Laboratory Manual].

Usually, the above described components, comprising a promoter, polyadenylation signal, and transcription termination sequence are put together into expression constructs. Enhancers, introns with functional splice donor and acceptor sites, and leader sequences may also be included in an expression construct, if desired. Expression constructs are often maintained in a replicon, such as an extrachromosomal element (*e.g.* plasmids) capable of stable maintenance in a host, such as mammalian cells or bacteria. Mammalian replication systems include those derived from animal viruses, which require trans-acting factors to replicate. For example, plasmids containing the replication systems of papovaviruses, such as SV40 [Gluzman (1981) Cell 23:175] or polyomavirus, replicate to extremely high copy number in the presence of the appropriate viral T antigen. Additional examples of mammalian replicons include those derived from bovine papillomavirus and Epstein-Barr virus. Additionally, the replicon may have two replicaton systems, thus allowing it to be maintained, for example, in mammalian cells for expression and in a prokaryotic host for cloning and amplification. Examples of such mammalian-bacteria shuttle vectors include pMT2 [Kaufman et al. (1989) Mol. Cell.

Biol. 9:946] and pHEB0 [Shimizu et al. (1986) Mol. Cell. Biol. 6:1074].

The transformation procedure used depends upon the host to be transformed. Methods for introduction of heterologous polynucleotides into mammalian cells are known in the art and include dextran-mediated transfection, calcium phosphate precipitation, polybrene-mediated transfection, protoplast fusion, electroporation, encapsulation of polynucleotide(s) in liposomes, direct microinjection of the DNA into nuclei.

Mammalian cell lines available as hosts for expression are known in the art and include many immortalized cell lines available from the American Type Culture Collection (ATCC), including but not limited to, Chinese hamster ovary (CHO) cells, HeLa cells, baby hamster kidney (BHK) cells, monkey kidney cells (COS), human hepatocellular carcinoma cells (*e.g.* Hep G2), and a number of other cell lines.

ii. Baculovirus Systems

The polynucleotide encoding the protein can also be inserted into a suitable insect expression vector, and is operably linked to the control elements within that vector. Vector construction employs techniques which are known in the art. Generally, the components of the expression system include a transfer vector, usually a bacterial plasmid, which contains both a fragment of the baculovirus genome, and a convenient restriction site for insertion of the heterologous gene or genes to be expressed; a wild type baculovirus with a sequence homologous to the baculovirus-specific fragment in the transfer vector (this allows for the homologous recombination of the heterologous gene in to the baculovirus genome); and appropriate insect host cells and growth media.

After inserting the DNA sequence encoding the protein into the transfer vector, the vector and the wild type viral genome are transfected into an insect host cell where the vector and viral genome are allowed to recombine. The packaged recombinant virus is expressed and recombinant plaques are identified and purified. Materials and methods for baculovirus/insect cell expression systems are commercially available in kit form from, *inter alia*, Invitrogen, San Diego CA ("MaxBac" kit). These techniques are generally known to those skilled in the art and fully described in Summers and Smith, Texas Agricultural Experiment Station Bulletin No. 1555 (1987) (hereinafter "Summers and Smith").

Prior to inserting the DNA sequence encoding the protein into the baculovirus genome, the above described components, comprising a promoter, leader (if desired), coding sequence of interest, and transcription termination sequence, are usually assembled into an intermediate transplacement construct (transfer vector). This construct may contain a single gene and operably linked regulatory elements; multiple genes, each with its owned set of operably linked regulatory elements; or multiple genes, regulated by the same set of regulatory elements. Intermediate transplacement constructs are often maintained in a replicon, such as an extrachromosomal element (*e.g.* plasmids) capable of stable maintenance in a host, such as a bacterium. The replicon will have a replication system, thus allowing it to be maintained in a suitable host for cloning and amplification.

Currently, the most commonly used transfer vector for introducing foreign genes into AcNPV is pAc373. Many other vectors, known to those of skill in the art, have also been designed. These include, for example, pVL985 (which alters the polyhedrin start codon from ATG to ATT, and which introduces a BamHI cloning site 32 basepairs downstream from the ATT; see Luckow and Summers, Virology (1989) 17:31.

The plasmid usually also contains the polyhedrin polyadenylation signal (Miller et al. (1988) Ann. Rev. Microbiol., 42:177) and a prokaryotic ampicillin-resistance (*amp*) gene and origin of replication for selection and propagation in *E. coli.*

Baculovirus transfer vectors usually contain a baculovirus promoter. A baculovirus promoter is any DNA sequence capable of binding a baculovirus RNA polymerase and initiating the downstream (5' to 3') transcription of a coding sequence (*e.g.* structural gene) into mRNA. A promoter will have a transcription initiation region which is usually placed proximal to the 5' end of the coding sequence. This transcription initiation region usually includes an RNA polymerase binding site and a transcription initiation site. A baculovirus transfer vector may also have a second domain called an enhancer, which, if present, is usually distal to the structural gene. Expression may be either regulated or constitutive.

Structural genes, abundantly transcribed at late times in a viral infection cycle, provide particularly useful promoter sequences. Examples include sequences derived from the gene encoding the viral polyhedron protein, Friesen et al., (1986) "The Regulation of Baculovirus Gene Expression," in: The Molecular Biology of Baculoviruses (ed. Walter Doerfler); EP0 Pub]. Nos. 127 839 and 155 476; and the gene encoding the p10 protein, Vlak et al., (1988), J. Gen. Virol. 69:765.

DNA encoding suitable signal sequences can be derived from genes for secreted insect or baculovirus proteins, such as the baculovirus polyhedrin gene (Carbonell et al. (1988) Gene, 73:409). Alternatively, since the signals for mammalian cell posttranslational modifications (such as signal peptide cleavage, proteolytic cleavage, and phosphorylation) appear to be recognized by insect cells, and the signals required for secretion and nuclear accumulation also appear to be conserved between the invertebrate cells and vertebrate cells, leaders of non-insect origin, such as those derived from genes encoding human $\alpha$-interferon, Maeda et al., (1985), Nature 315:592; human gastrin-releasing peptide, Lebacq-Verheyden et al., (1988), Molec. Cell. Biol. 8:3129; human IL-2, Smith et al., (1985) Proc. Nat'l Acad. Sci. USA, 82:8404; mouse IL-3, (Miyajima et al., (1987) Gene 58:273; and human glucocerebrosidase, Martin et al. (1988) DNA, 7:99, can also be used to provide for secretion in insects.

A recombinant polypeptide or polyprotein may be expressed intracellularly or, if it is expressed with the proper regulatory sequences, it can be secreted. Good intracellular expression of nonfused foreign proteins usually requires heterologous genes that ideally have a short leader sequence containing suitable translation initiation signals preceding an ATG start signal. If desired, methionine at the N-terminus may be cleaved from the mature protein by *in vitro* incubation with cyanogen bromide.

Alternatively, recombinant polyproteins or proteins which are not naturally secreted can be secreted from the insect cell by creating chimeric DNA molecules that encode a fusion protein comprised of a leader sequence fragment that provides for secretion of the foreign protein in insects. The leader sequence fragment usually encodes a signal peptide comprised of hydrophobic amino acids which direct the translocation of the protein into the endoplasmic reticulum.

After insertion of the DNA sequence and/or the gene encoding the expression product precursor of the protein, an insect cell host is co-transformed with the heterologous DNA of the transfer vector and the genomic DNA of wild type baculovirus -- usually by co-transfection. The promoter and transcription termination sequence of the construct will usually comprise a 2-5kb section of the baculovirus genome. Methods for introducing heterologous DNA into the desired site in the baculovirus virus are known in the art. (See Summers and Smith *supra;* Ju et al. (1987); Smith et al., Mol. Cell. Biol. (1983) 3:2156; and Luckow and Summers (1989)). For example, the insertion can be into a gene such as the polyhedrin gene, by homologous double crossover recombination; insertion can also be into a restriction enzyme site engineered into the desired baculovirus gene. Miller et al., (1989), Bioessays 4:91.The DNA sequence, when cloned in place of the polyhedrin gene in the expression vector, is flanked both 5' and 3' by polyhedrin-specific sequences and is positioned downstream of the polyhedrin promoter.

The newly formed baculovirus expression vector is subsequently packaged into an infectious recombinant baculovirus. Homologous recombination occurs at low frequency (between ~1% and ~5%); thus, the majority of the virus produced after cotransfection is still wild-type virus. Therefore, a method is necessary to identify recombinant viruses. An advantage of the expression system is a visual screen allowing recombinant viruses to be distinguished. The polyhedrin protein, which is produced by the native virus, is produced at very high levels in the nuclei of infected cells at late times after viral infection. Accumulated polyhedrin protein forms occlusion bodies that also contain embedded particles. These occlusion bodies, up to 15μm in size, are highly refractile, giving them a bright shiny appearance that is readily visualized under the light microscope. Cells infected with recombinant viruses lack occlusion bodies. To distinguish recombinant virus from wild-type virus, the transfection supernatant is plaqued onto a monolayer of insect cells by techniques known to those skilled in the art. Namely, the plaques are screened under the light microscope for the presence (indicative of wild-type virus) or absence (indicative of recombinant virus) of occlusion bodies. "Current Protocols in Microbiology" Vol. 2 (Ausubel et al. eds) at 16.8 (Supp. 10, 1990); Summers & Smith, *supra;* Miller et al. (1989).

Recombinant baculovirus expression vectors have been developed for infection into several insect cells. For example, recombinant baculoviruses have been developed for, *inter alia: Aedes aegypti, Autographa californica, Bombyx mori, Drosophila melanogaster, Spodoptera frugiperda,* and *Trichoplusia ni* (W0 89/046699; Carbonell et al., (1985) J. Virol. 56:153; Wright (1986) Nature 321:718; Smith et al., (1983) Mol. Cell. Biol. 3:2156; and see generally, Fraser, et al. (1989) In Vitro Cell. Dev. Biol. 25:225).

Cells and cell culture media are commercially available for both direct and fusion expression of heterologous polypeptides in a baculovirus/expression system; cell culture technology is generally known to those skilled in the art. *See, e.g.* Summers and Smith *supra.*

The modified insect cells may then be grown in an appropriate nutrient medium, which allows for stable maintenance of the plasmid(s) present in the modified insect host. Where the expression product gene is under inducible control, the host may be grown to high density, and expression induced. Alternatively, where expression is constitutive, the product will be continuously expressed into the medium and the nutrient medium must be continuously circulated, while removing the product of interest and augmenting depleted nutrients. The product may be purified by such techniques as chromatography, e.g. HPLC, affinity chromatography, ion exchange chromatography, etc.; electrophoresis; density gradient centrifugation; solvent extraction, or the like. As appropriate, the product may be further purified, as required, so as to remove substantially any insect proteins which are also secreted in the medium or result from lysis of insect cells, so as to provide a product which is at least substantially free of host debris, e.g. proteins, lipids and polysaccharides.

In order to obtain protein expression, recombinant host cells derived from the transformants are incubated under conditions which allow expression of the recombinant protein encoding sequence. These conditions will vary, dependent upon the host cell selected. However, the conditions are readily ascertainable to those of ordinary skill in the art, based upon what is known in the art.

iii. Plant Systems

There are many plant cell culture and whole plant genetic expression systems known in the art. Exemplary plant cellular genetic expression systems include those described in patents, such as: US 5,693,506; US 5,659,122; and US 5,608,143. Additional examples of genetic expression in plant cell culture has been described by Zenk, Phytochemistry 30:3861-3863 (1991). Descriptions of plant protein signal peptides may be found in addition to the references described above in Vaulcombe et al., Mol. Gen. Genet. 209:33-40 (1987); Chandler et al., Plant Molecular Biology 3: 407-418 (1984); Rogers, J. Biol. Chem. 260:3731-3738 (1985); Rothstein et al., Gene 55:353-356 (1987); Whittier et al., Nucleic Acids Research 15:2515-2535 (1987); Wirsel et al., Molecular Microbiology 3:3-14 (1989); Yu et al., Gene 122:247-253 (1992). A description of the regulation of plant gene expression by the phytohormone, gibberellic acid and secreted enzymes induced by gibberellic acid can be found in R.L. Jones and J. MacMillin, Gibberellins: in: Advanced Plant Physiology,. Malcolm B. Wilkins, ed., 1984 Pitman Publishing Limited, London, pp. 21-52. References that describe other metabolically-regulated genes: Sheen, Plant Cell, 2:1027-1038(1990); Maas et al., EMBO J. 9:3447-3452 (1990); Benkel and Hickey, Proc. Natl. Acad. Sci. 84:1337-1339 (1987)

Typically, using techniques known in the art, a desired polynucleotide sequence is inserted into an expression cassette comprising genetic regulatory elements designed for operation in plants. The expression cassette is inserted into a desired expression vector with companion sequences upstream and downstream from the expression cassette suitable for expression in a plant host. The companion sequences will be of plasmid or viral origin and provide necessary characteristics to the vector to permit the vectors to move DNA from an original cloning host, such as bacteria, to the desired plant host. The basic bacterial/plant vector construct will preferably provide a broad host range prokaryote replication origin; a prokaryote selectable marker; and, for Agrobacterium transformations, T DNA sequences for Agrobacterium-mediated transfer to plant chromosomes. Where the heterologous gene is not readily amenable to detection, the construct will preferably also have a selectable marker gene suitable for determining if a plant cell has been transformed. A general review of suitable markers, for example for the members of the grass family, is found in Wilmink and Dons, 1993, Plant Mol. Biol. Reptr, 11(2):165-185.

Sequences suitable for permitting integration of the heterologous sequence into the plant genome are also recommended. These might include transposon sequences and the like for homologous recombination as well as Ti sequences which permit random insertion of a heterologous expression cassette into a plant genome. Suitable prokaryote selectable markers include resistance toward antibiotics such as ampicillin or tetracycline. Other DNA sequences encoding additional functions may also be present in the vector, as is known in the art.

The nucleic acid molecules of the subject invention may be included into an expression cassette for expression of the protein(s) of interest. Usually, there will be only one expression cassette, although two or more are feasible. The recombinant expression cassette will contain in addition to the heterologous protein encoding sequence the following elements, a promoter region, plant 5' untranslated sequences, initiation codon depending upon whether or not the structural gene comes equipped with one, and a transcription and translation termination sequence. Unique restriction enzyme sites at the 5' and 3' ends of the cassette allow for easy insertion into a pre-existing vector.

A heterologous coding sequence may be for any protein relating to the present invention. The sequence encoding the protein of interest will encode a signal peptide which allows processing and translocation of the protein, as appropriate, and will usually lack any sequence which might result in the binding of the desired protein of the invention to a membrane. Since, for the most part, the transcriptional initiation region will be for a gene which is expressed and translocated during germination, by employing the signal peptide which provides for translocation, one may also provide for translocation of the protein of interest. In this way, the protein(s) of interest will be translocated from the cells in which they are expressed and may be efficiently harvested. Typically secretion in seeds are across the aleurone or scutellar epithelium layer into the endosperm of the seed. While it is not required that the protein be secreted from the cells in which the protein is produced, this facilitates the isolation and purification of the recombinant protein.

Since the ultimate expression of the desired gene product will be in a eucaryotic cell it is desirable to determine whether any portion of the cloned gene contains sequences which will be processed out as introns by the host's splicosome machinery. If so, site-directed mutagenesis of the "intron" region may be conducted to prevent losing a portion of the genetic message as a false intron code, Reed and Maniatis, Cell 41:95-105, 1985.

The vector can be microinjected directly into plant cells by use of micropipettes to mechanically transfer the recombinant DNA. Crossway, Mol. Gen. Genet, 202:179-185, 1985. The genetic material may also be transferred into the plant cell by using polyethylene glycol, Krens, et al., Nature, 296, 72-74, 1982. Another method of introduction of nucleic acid segments is high velocity ballistic penetration by small particles with the nucleic acid either within the matrix of small beads or particles, or on the surface, Klein, et al., Nature, 327, 70-73, 1987 and Knudsen and Muller, 1991, Planta, 185:330-336 teaching particle bombardment of barley endosperm to create transgenic barley. Yet another method of introduction would be fusion of protoplasts with other entities, either minicells, cells, lysosomes or other fusible lipid-surfaced bodies, Fraley, et al., Proc. Natl. Acad. Sci. USA, 79, 1859-1863, 1982.

The vector may also be introduced into the plant cells by electroporation. (Fromm et al., Proc. Natl Acad. Sci. USA 82:5824, 1985). In this technique, plant protoplasts are electroporated in the presence of plasmids containing the gene construct. Electrical impulses of high field strength reversibly permeabilize biomembranes allowing the introduction of the plasmids. Electroporated plant protoplasts reform the cell wall, divide, and form plant callus.

All plants from which protoplasts can be isolated and cultured to give whole regenerated plants can be transformed by the present invention so that whole plants are recovered which contain the transferred gene. It is known that practically all plants can be regenerated from cultured cells or tissues, including but not limited to all major species of sugarcane, sugar beet, cotton, fruit and other trees, legumes and vegetables. Some suitable plants include, for example, species from the genera *Fragaria, Lotus, Medicago, Onobrychis, Trifolium, Trigonella, Vigna, Citrus, Linum, Geranium, Manihot, Daucus, Arabidopsis, Brassica, Raphanus, Sinapis, Atropa, Capsicum, Datura, Hyoscyamus, Lycopersion, Nicotiana, Solanum, Petunia, Digitalis, Majorana, Cichorium, Helianthus, Lactuca, Bromus, Asparagus, Antirrhinum, Hererocallis, Nemesia, Pelargonium, Panicum, Pennisetum, Ranunculus, Senecio, Salpiglossis, Cucumis, Browaalia, Glycine, Lolium, Zea, Triticum, Sorghum,* and *Datura.*

Means for regeneration vary from species to species of plants, but generally a suspension of transformed protoplasts containing copies of the heterologous gene is first provided. Callus tissue is formed and shoots may be induced from callus and subsequently rooted. Alternatively, embryo formation can be induced from the protoplast suspension. These embryos germinate as natural embryos to form plants. The culture media will generally contain various amino acids and hormones, such as auxin and cytokinins. It is also advantageous to add glutamic acid and proline to the medium, especially for such species as corn and alfalfa. Shoots and roots normally develop simultaneously. Efficient regeneration will depend on the medium, on the genotype, and on the history of the culture. If these three variables are controlled, then regeneration is fully reproducible and repeatable.

In some plant cell culture systems, the desired protein of the invention may be excreted or alternatively, the protein may be extracted from the whole plant. Where the desired protein of the invention is secreted into the medium, it may be collected. Alternatively, the embryos and embryoless-balf seeds or other plant tissue may be mechanically disrupted to release any secreted protein between cells and tissues. The mixture may be suspended in a buffer solution to retrieve soluble proteins. Conventional protein isolation and purification methods will be then used to purify the recombinant protein. Parameters of time, temperature pH, oxygen, and volumes will be adjusted through routine methods to optimize expression and recovery of heterologous protein.

iv. Bacterial Systems

Bacterial expression techniques are known in the art. A bacterial promoter is any DNA sequence capable of binding bacterial RNA polymerase and initiating the downstream (3') transcription of a coding sequence (e.g. structural gene) into mRNA. A promoter will have a transcription initiation region which is usually placed proximal to the 5' end of the coding sequence. This transcription initiation region usually includes an RNA polymerase binding site and a transcription initiation site. A bacterial promoter may also have a second domain called an operator, that may overlap an adjacent RNA polymerase binding site at which RNA synthesis begins. The operator permits negative regulated (inducible) transcription, as a gene repressor protein may bind the operator and thereby inhibit transcription of a specific gene. Constitutive expression may occur in the absence of negative regulatory elements, such as the operator. In addition, positive regulation may be achieved by a gene activator protein binding sequence, which, if present is usually proximal (5') to the RNA polymerase binding sequence. An example of a gene activator protein is the catabolite activator protein (CAP), which helps initiate transcription of the lac operon in Escherichia coli (E. coli) [Raibaud et al. (1984) Annu. Rev. Genet. 18:173]. Regulated expression may therefore be either positive or negative, thereby either enhancing or reducing transcription.

Sequences encoding metabolic pathway enzymes provide particularly useful promoter sequences. Examples include promoter sequences derived from sugar metabolizing enzymes, such as galactose, lactose (*lac*) [Chang et al. (1977) Nature 198:1056], and maltose. Additional examples include promoter sequences derived from biosynthetic enzymes such as tryptophan (*trp*) [Goeddel et al. (1980) Nuc. Acids Res. 8:4057; Yelverton et al. (1981) Nucl. Acids Res. 9:731;US patent 4,738,921; EP-A-0036776 and EP-A-0121775]. The g-laotamase (*bla*) promoter system [Weissmann (1981) "The cloning of interferon and other mistakes." In Interferon 3 (ed. I. Gresser)], bacteriophage lambda PL [Shimatake et al. (1981) Nature 292:128] and T5 [US patent 4,689,406] promoter systems also provide useful promoter sequences.

In addition, synthetic promoters which do not occur in nature also function as bacterial promoters. For example, transcription activation sequences of one bacterial or bacteriophage promoter may be joined with the operon sequences of another bacterial or bacteriophage promoter, creating a synthetic hybrid promoter [US patent 4,551, 433]. For example, the *tac* promoter is a hybrid *trp-lac* promoter comprised of both *trp* promoter and *lac* operon sequences that is regulated by the *lac* repressor [Amann et al. (1983) Gene 25:167; de Boer et al. (1983) Proc. Natl. Acad. Sci. 80:21]. Furthermore, a bacterial promoter can include naturally occurring promoters of non-bacterial origin that have the ability to bind bacterial RNA polymerase and initiate transcription. A naturally occurring promoter of non-bacterial origin can also be coupled with a compatible RNA polymerase to produce high levels of expression of some genes in prokaryotes. The bacteriophage T7 RNA polymerase/promoter system is an example of a coupled promoter system [Studier et al. (1986) J. Mol. Biol. 189:113; Tabor et al. (1985) Proc Natl. Acad. Sci. 82:1074]. In addition, a hybrid promoter can also be comprised of a bacteriophage promoter and an *E. coli* operator region (EPO-A-0 267 851).

In addition to a functioning promoter sequence, an efficient ribosome binding site is also useful for the expression of foreign genes in prokaryotes. In *E. coli*, the ribosome binding site is called the Shine-Daigarno (SD) sequence and includes an initiation codon (ATG) and a sequence 3-9 nucleotides in length located 3-11 nucleotides upstream of the initiation codon [Shine et al. (1975) Nature 254:34]. The SD sequence is thought to promote binding of mRNA to the ribosome by the pairing of bases between the SD sequence and the 3' and of *E. coli* 16S rRNA [Steitz et al. (1979) "Genetic signals and nucleotide sequences in messenger RNA." In Biological Regulation and Development: Gene Expression (ed. R.F. Goldberger)]. To express eukaryotic genes and prokaryotic genes with weak ribosome-binding site [Sambrook et al. (1989) "Expression of cloned genes in Escherichia coli." In Molecular Cloning: A Laboratory Manual].

A DNA molecule may be expressed intracellularly. A promoter sequence may be directly linked with the DNA molecule, in which case the first amino acid at the N-terminus will always be a methionine, which is encoded by the ATG start codon. If desired, methionine at the N-terminus may be cleaved from the protein by *in vitro* incubation with cyanogen bromide or by either *in vivo* on *in vitro* incubation with a bacterial methionine N-terminal peptidase (EPO-A-0 219 237).

Fusion proteins provide an alternative to direct expression. Usually, a DNA sequence encoding the N-terminal portion of an endogenous bacterial protein, or other stable protein, is fused to the 5' end of heterologous coding sequences. Upon expression, this construct will provide a fusion of the two amino acid sequences. For example, the bacteriophage lambda cell gene can be linked at the 5' terminus of a foreign gene and expressed in bacteria. The resulting fusion protein preferably retains a site for a processing enzyme (factor Xa) to cleave the bacteriophage protein from the foreign gene [Nagai et al. (1984) Nature 309:810]. Fusion proteins can also be made with sequences from the *lac*Z [Jia et al. (1987) Gene 60:197], *trpE* [Allen et al. (1987) J. Biotechnol. 5:93; Makoff et al. (1989) J. Gen. Microbiol. 135:11], and Chey [EP-A-0 324 647] genes. The DNA sequence at the junction of the two amino acid sequences may or may not encode a cleavable site. Another example is a ubiquitin fusion protein. Such a fusion protein is made with the ubiquitin region that preferably retains a site for a processing enzyme (*e.g.* ubiquitin specific processing-protease) to cleave the ubiquitin from the foreign protein. Through this method, native foreign protein can be isolated [Miller et al. (1989) Bio/ Technology 7:698].

Alternatively, foreign proteins can also be secreted from the cell by creating chimeric DNA molecules that encode a fusion protein comprised of a signal peptide sequence fragment that provides for secretion of the foreign protein in bacteria [US patent 4,336,336]. The signal sequence fragment usually encodes a signal peptide comprised of hydrophobic amino acids which direct the secretion of the protein from the cell. The protein is either secreted into the growth media (gram-positive bacteria) or into the periplasmic space, located between the inner and outer membrane of the cell (gram-negative bacteria). Preferably there are processing sites, which can be cleaved either *in vivo* or *in vitro* encoded between the signal peptide fragment and the foreign gene.

DNA encoding suitable signal sequences can be derived from genes for secreted bacterial proteins, such as the *E. coli* outer membrane protein gene (*ompA*) [Masui et al. (1983), in: Experimental Manipulation of Gene Expression; Ghrayeb et al. (1984) EMBO J. 3:2437] and the *E. coli* alkaline phosphatase signal sequence (*phoA*) [Oka et al. (1985) Proc. Natl. Acad. Sci. 82:7212]. As an additional example, the signal sequence of the alpha-amylase gene from various Bacillus strains can be used to secrete heterologous proteins from *B. subtilis* [Palva et al. (1982) Proc. Natl. Acad. Sci. USA 79:5582; EP-A-0 244 042].

Usually, transcription termination sequences recognized by bacteria are regulatory regions located 3' to the translation stop codon, and thus together with the promoter flank the coding sequence. These sequences direct the transcription of an mRNA which can be translated into the polypeptide encoded by the DNA. Transcription termination sequences frequently include DNA sequences of about 50 nucleotides capable of forming stem loop structures that aid in terminating transcription. Examples include transcription termination sequences derived from genes with strong promoters, such as the *trp* gene in *E. coli* as well as other biosynthetic genes.

Usually, the above described components, comprising a promoter, signal sequence (if desired), coding sequence of interest, and transcription termination sequence, are put together into expression constructs. Expression constructs are often maintained in a replicon, such as an extrachromosomal element (*e.g.* plasmids) capable of stable maintenance in a host, such as bacteria. The replicon will have a replication system, thus allowing it to be maintained in a prokaryotic host either for expression or for cloning and amplification. In addition, a replicon may be either a high or low copy number plasmid. A high copy number plasmid will generally have a copy number ranging from about 5 to about 200, and usually about 10 to about 150. A host containing a high copy number plasmid will preferably contain at least about 10, and more preferably at least about 20 plasmids. Either a high or low copy number vector may be selected, depending upon the effect of the vector and the foreign protein on the host.

Alternatively, the expression constructs can be integrated into the bacterial genome with an integrating vector. Integrating vectors usually contain at least one sequence homologous to the bacterial chromosome that allows the vector to integrate. Integrations appear to result from recombinations between homologous DNA in the vector and the bacterial chromosome. For example, integrating vectors constructed with DNA from various Bacillus strains integrate into the Bacillus chromosome (EP-A- 0 127 328). Integrating vectors may also be comprised of bacteriophage or transposon sequences.

Usually, extrachromosomal and integrating expression constructs may contain selectable markers to allow for the selection of bacterial strains that have been transformed. Selectable markers can be expressed in the bacterial host and may include genes which render bacteria resistant to drugs such as ampicillin, chloramphenicol, erythromycin, kanamycin (neomycin), and tetracycline [Davies et al. (1978) Annu. Rev. Microbiol. 32:469]. Selectable markers may also include biosynthetic genes, such as those in the histidine, tryptophan, and leucine biosynthetic pathways.

Alternatively, some of the above described components can be put together in transformation vectors. Transformation vectors are usually comprised of a selectable market that is either maintained in a replicon or developed into an integrating vector, as described above.

Expression and transformation vectors, either extra-chromosomal replicons or integrating vectors, have been developed for transformation into many bacteria. For example, expression vectors have been developed for, *inter alia*, the following bacteria: Bacillus subtilis [Palva et al, (1982) Proc. Natl. Acad. Sci. USA 79:5582; EP-A-0 036 259 and EP-A-0 063 953; WO 84/04541], Escherichia coli [Shimatake et al. (1981) Nature 292:128; Amann et al. (1985) Gene 40: 183; Studier et al. (1986) J. Mol. Biol. 189:113; EP-A-0 036 776,EP-A-0 136 829 and EP-A-0 136 907], Streptococcus cremoris [Powell et al. (1988) Appl. Environ. Microbiol. 54:655]; Streptococcus lividans [Powell et al. (1988) Appl. Environ. Microbiol. 54:655], Streptomyces lividans [US patent 4,745,056].

Methods of introducing exogenous DNA into bacterial hosts are well-known in the art, and usually include either the transformation of bacteria treated with $CaCl_2$ or other agents, such as divalent cations and DMSO. DNA can also be introduced into bacterial cells by electroporation. Transformation procedures usually vary with the bacterial species to be transformed. See *e.g.* [Masson et al. (1989) FEMS Microbiol. Lett. 60:273; Palva et al. (1982) Proc. Natl. Acad. Sci. USA 79:5582; EP-A-0 036 259 and EP-A-0 063 953; WO 84104541, Bacillus], [Miller et al. (1988) Proc. Natl. Acad. Sci. 85:856; Wang et al. (1990) J. Bacteriol. 172:949, Campylobacter], [Cohen et al. (1973) Proc. Natl. Acad. Sci. 69: 2110; Dower et al. (1988) Nucleic Acids Res. 16:6127; Kushner (1978) "An improved method for transformation of Escherichia coli with ColE1-derived plasmids. In Genetic Engineering: Proceedings of the International Symposium on Genetic Engineering (eds. H.W. Boyer and S. Nicosia); Mandel et al. (1970) J. Mol. Biol. 53:159; Taketo (1988) Biochim. Biophys. Acta 949:318; Escherichia], [Chassy et al. (1987) FEMS Microbiol. Lett. 44:173 Lactobacillus]; [Fiedler et al. (1988) Anal. Biochem 170:38, Pseudomonas]; [Augustin et al. (1990) FEMS Microbiol. Lett. 66:203, Staphylococcus], [Barany et al. (1980) J. Bacteriol. 144:698; Harlander (1987) "Transformation of Streptococcus lactis by electroporation, in: Streptococcal Genetics (ed. J. Ferretti and R. Curtiss III); Perry et al. (1981) Infect. Immun. 32:1295; Powell et al. (1988) Appl. Environ. Microbiol. 54:655; Somkuti et al. (1987) Proc. 4th Evr. Cong. Biotechnology 1:412, Streptococcus].

v. Yeast Expression

Yeast expression systems are also known to one of ordinary skill in the art. A yeast promoter is any DNA sequence capable of binding yeast RNA polymerase and initiating the downstream (3') transcription of a coding sequence (e.g. structural gene) into mRNA. A promoter will have a transcription initiation region which is usually placed proximal to the 5' end of the coding sequence. This transcription initiation region usually includes an RNA polymerase binding site (the "TATA Box") and a transcription initiation site. A yeast promoter may also have a second domain called an upstream activator sequence (UAS), which, if present, is usually distal to the structural gene. The UAS permits regulated (inducible) expression. Constitutive expression occurs in the absence of a UAS. Regulated expression may be either positive or negative, thereby either enhancing or reducing transcription.

Yeast is a fermenting organism with an active metabolic pathway, therefore sequences encoding enzymes in the metabolic pathway provide particularly useful promoter sequences. Examples include alcohol dehydrogenase (ADH) (EP-A-0 284 044), enolase, glucokinase, glucose-6-phosphate isomerase, glyceraldehyde-3-phosphate-dehydrogenase (GAP or GAPDH), hexokinase, phosphofructokinase, 3-phosphoglycerate mutase, and pyruvate kinase (PyK) (EPO-A-0 329 203). The yeast *PH05* gene, encoding acid phosphatase, also provides useful promoter sequences [Myanohara et al. (1983) Proc. Natl. Acad. Sci. USA 80:1].

In addition, synthetic promoters which do not occur in nature also function as yeast promoters. For example, UAS sequences of one yeast promoter may be joined with the transcription activation region of another yeast promoter, creating a synthetic hybrid promoter. Examples of such hybrid promoters include the ADH regulatory sequence linked to the GAP transcription activation region (US Patent Nos. 4,876,197 and 4,880,734). Other examples of hybrid promoters include promoters which consist of the regulatory sequences of either the *ADH2, GAL4, GAL10,* 0R *PH05* genes, combined with the transcriptional activation region of a glycolytic enzyme gene such as GAP or PyK (EP-A-0 164 556). Furthermore, a yeast promoter can include naturally occurring promoters of non-yeast origin that have the ability to bind yeast RNA polymerase and initiate transcription. Examples of such promoters include, *inter alia*, [Cohen et al. (1980) Proc. Natl. Acad. Sci. USA 77:1078; Henikoff et al. (1981) Nature 283:835; Hollenberg et al. (1981) Curr. Topics Microbiol. Immunol. 96:119; Hollenberg et al. (1979) "The Expression of Bacterial Antibiotic Resistance Genes in the Yeast Saccharomyces cerevisiae," in: Plasmids of Medical, Environmental and Commercial Importance (eds. K.N. Timmis and A.

Puhler); Mercerau-Puigalon et al. (1980) Gene 11:163; Panthier et al. (1980) Curr. Genet. 2:109;].

A DNA molecule may be expressed intracellularly in yeast. A promoter sequence may be directly linked with the DNA molecule, in which case the first amino acid at the N-terminus of the recombinant protein will always be a methionine, which is encoded by the ATG start codon. If desired, methionine at the N-terminus may be cleaved from the protein by *in vitro* incubation with cyanogen bromide.

Fusion proteins provide an alternative for yeast expression systems, as well as in mammalian, baculovirus, and bacterial expression systems. Usually, a DNA sequence encoding the N-terminal portion of an endogenous yeast protein, or other stable protein, is fused to the 5' end of heterologous coding sequences. Upon expression, this construct will provide a fusion of the two amino acid sequences. For example, the yeast or human superoxide dismutase (SOD) gene, can be linked at the 5' terminus of a foreign gene and expressed in yeast. The DNA sequence at the junction of the two amino acid sequences may or may not encode a cleavable site. See e.g. EP-A-0 196 056. Another example is a ubiquitin fusion protein. Such a fusion protein is made with the ubiquitin region that preferably retains a site for a processing enzyme (e.g. ubiquitin-specific processing protease) to cleave the ubiquitin from the foreign protein. Through this method, therefore, native foreign protein can be isolated (*e.g.* WO88/024066).

Alternatively, foreign proteins can also be secreted from the cell into the growth media by creating chimeric DNA molecules that encode a fusion protein comprised of a leader sequence fragment that provide for secretion in yeast of the foreign protein. Preferably, there are processing sites encoded between the leader fragment and the foreign gene that can be cleaved either *in vivo* or *in vitro.* The leader sequence fragment usually encodes a signal peptide comprised of hydrophobic amino acids which direct the secretion of the protein from the cell.

DNA encoding suitable signal sequences can be derived from genes for secreted yeast proteins, such as the genes for invertase (EP-A-0012873; JPO 62,096,086) and A-factor (US patent 4,588,684). Alternatively, leaders of non-yeast origin exit, such as an interferon leader, that also provide for secretion in yeast (EP-A-0060057).

A preferred class of secretion leaders are those that employ a fragment of the yeast alpha-factor gene, which contains both a "pre" signal sequence, and a "pro" region. The types of alpha-factor fragments that can be employed include the full-length pre-pro alpha factor leader (about 83 amino acid residues) as well as truncated alpha-factor leaders (usually about 25 to about 50 amino acid residues) (US Patents 4,546,083 and 4,870,008; EP-A-0 324 274). Additional leaders employing an alpha-factor leader fragment that provides for secretion include hybrid alpha-factor leaders made with a presequence of a first yeast, but a pro-region from a second yeast alphafactor. (*e.g.* see WO 89/02463.)

Usually, transcription termination sequences recognized by yeast are regulatory regions located 3' to the translation stop codon, and thus together with the promoter flank the coding sequence. These sequences direct the transcription of an mRNA which can be translated into the polypeptide encoded by the DNA. Examples of transcription terminator sequence and other yeast-recognized termination sequences, such as those coding for glycolytic enzymes.

Usually, the above described components, comprising a promoter, leader (if desired), coding sequence of interest, and transcription termination sequence, are put together into expression constructs. Expression constructs are often maintained in a replicon, such as an extrachromosomal element (*e.g.* plasmids) capable of stable maintenance in a host, such as yeast or bacteria. The replicon may have two replication systems, thus allowing it to be maintained, for example, in yeast for expression and in a prokaryotic host for cloning and amplification. Examples of such yeast-bacteria shuttle vectors include YEp24 [Botstein et al. (1979) Gene 8:17-24], pCl/1 [Brake et al. (1984) Proc, Natl. Acad. Sci USA 81:4642-4646], and YRp17 [Stinchcomb et al. (1982) J. Mol. Biol. 158:157]. In addition, a replicon may be either a high or low copy number plasmid. A high copy number plasmid will generally have a copy number ranging from about 5 to about 200, and usually about 10 to about 150. A host containing a high copy number plasmid will preferably have at least about 10, and more preferably at least about 20. Enter a high or low copy number vector may be selected, depending upon the effect of the vector and the foreign protein on the host. See *e.g.* Brake *et al., supra*.

Alternatively, the expression constructs can be integrated into the yeast genome with an integrating vector. Integrating vectors usually contain at least one sequence homologous to a yeast chromosome that allows the vector to integrate, and preferably contain two homologous sequences flanking the expression construct. Integrations appear to result from recombinations between homologous DNA in the vector and the yeast chromosome [Orr-Weaver et al. (1983) Methods in Enzymol. 101:228-245]. An integrating vector may be directed to a specific locus in yeast by selecting the appropriate homologous sequence for inclusion in the vector. See Orr-Weaver *et al., supra*. One or more expression construct may integrate, possibly affecting levels of recombinant protein produced [Rine et al, (1983) Proc. Natl. Acad. Sci. USA 80:6750]. The chromosomal sequences included in the vector can occur either as a single segment in the vector, which results in the integration of the entire vector, or two segments homologous to adjacent segments in the chromosome and flanking the expression construct in the vector, which can result in the stable integration of only the expression construct.

Usually, extrachromosomal and integrating expression constructs may contain selectable markers to allow for the selection of yeast strains that have been transformed. Selectable markers may include biosynthetic genes that can be expressed in the yeast host, such as *ADE2, HIS4, LEU2, TRP1*, and *ALG7*, and the G418 resistance gene, which confer resistance in yeast cells to tunicamycin and G418, respectively. In addition, a suitable selectable marker may also provide yeast with the ability to grow in the presence of toxic compounds, such as metal. For example, the presence of *CUP1* allows yeast to grow in the presence of copper ions [Butt et al. (1987) Microbiol, Rev. 51:351].

Alternatively, some of the above described components can be put together into transformation vectors. Transformation vectors are usually comprised of a selectable marker that is either maintained in a replicon or developed into an integrating vector, as described above.

Expression and transformation vectors, either extrachromosomal replicons or integrating vectors, have been developed for transformation into many yeasts. For example, expression vectors have been developed for, *inter alia*, the following yeasts:Candida albicans [Kurtz, et al. (1986) Mol. Cell. Biol. 6:142], Candida maltosa [Kunze, et al. (1985) J. Basic Microbiol. 25:141]. Hansenula polymorpha [Gleeson, et al. (1986) J. Gen. Microbiol. 132:3459; Roggenkamp et al. (1986) Mol. Gen. Genet. 202:302], Kluyveromyces fragilis [Das, et al. (1984) J. Bacteriol. 158:1165], Kluyveromyces lactis [De Louvencourt et al. (1983) J. Bacteriol. 154:737; Van den Berg et al. (1990) Bio/Technology 8:135], Pichia guillerimondii [Kunze et al. (1985) J. Basic Microbiol. 25:141], Pichia pastoris [Cregg, et al. (1985) Mol. Cell. Biol. 5: 3376; US Patent Nos. 4,837,148 and 4,929,555], Saccharomyces cerevisiae [Hinnen et al. (1978) Proc. Natl. Acad. Sci. USA 75:1929; Ito et al. (1983) J. Bacteriol. 153:163], Schizosaccharomyces pombe [Beach and Nurse (1981) Nature 300:706], and Yarrowia lipolytica [Davidow, et al. (1985) Curr. Genet. 10:380471 Gaillardin, et al. (1985) Curr. Genet. 10:49].

Methods of introducing exogenous DNA into yeast hosts are well-known in the art, and usually include either the transformation of spheroplasts or of intact yeast cells treated with alkali cations. Transformation procedures usually vary with the yeast species to be transformed. See *e.g.* [Kurtz et al. (1986) Mol. Cell. Biol. 6:142; Kunze et al. (1985) J. Basic Microbiol. 25:141; Candida]; [Gleeson et al. (1986) J. Gen. Microbiol. 132:3459; Roggenkamp et al. (1986) Mol. Gen. Genet. 202:302; Hansenula]; [Das et al. (1984) J. Bacteriol. 158:1165; De Louvencourt et al. (1983) J. Bacteriol. 154:1165; Van den Berg et al. (1990) Bio/Technology 8:135; Kluyveromyces]; [Cregg et al. (1985) Mol. Cell. Biol. 5: 3376; Kunze et al. (1985) J. Basic Microbiol. 25:141; US Patents 4,837,148 & 4,929,555; Pichia]; [Hinnen et al. (1978) Proc. Natl. Acad. Sci. USA 75;1929; Ito et al. (1983) J. Bacteriol. 153:163 Saccharomyces]; [Beach & Nurse (1981) Nature 300:706; Schizosaccharomyces]; [Davidow et al. (1985) Curr. Genet. 10:39; Gaillardin et al. (1985) Curr. Genet. 10:49; Yarrowia].

*Pharmaceutical Compositions*

Pharmaceutical compositions can comprise polypeptides and/or nucleic acid of the invention. The pharmaceutical compositions will comprise a therapeutically effective amount of either polypeptides, antibodies, or polynucleotides of the claimed invention.

The term "therapeutically effective amount" as used herein refers to an amount of a therapeutic agent to treat, ameliorate, or prevent a desired disease or condition, or to exhibit a detectable therapeutic or preventative effect. The effect can be detected by, for example, chemical markers or antigen levels. Therapeutic effects also include reduction in physical symptoms, such as decreased body temperature. The precise effective amount for a subject will depend upon the subject's size and health, the nature and extent of the condition, and the therapeutics or combination of therapeutics selected for administration. Thus, it is not useful to specify an exact effective amount in advance. However, the effective amount for a given situation can be determined by routine experimentation and is within the judgement of the clinician.

For purposes of the present invention, an effective dose will be from about 0.01 mg/ kg to 50 mg/kg or 0.05 mg/kg to about 10 mg/kg of the DNA constructs in the individual to which it is administered.

A pharmaceutical composition can also contain a pharmaceutically acceptable carrier. The term "pharmaceutically acceptable carrier" refers to a carrier for administration of a therapeutic agent, such as antibodies or a polypeptide, genes, and other therapeutic agents. The term refers to any pharmaceutical carrier that does not itself induce the production of antibodies harmful to the individual receiving the composition, and which may be administered without undue toxicity. Suitable carriers may be large, slowly metabolized macromolecules such as proteins, polysaccharides, polylactic acids, polyglycolic acids, polymeric amino acids, amino acid copolymers, and inactive virus particles. Such carriers are well known to those of ordinary skill in the art.

Pharmaceutically acceptable salts can be used therein, for example, mineral acid salts such as hydrochlorides, hydrobromides, phosphates, sulfates, and the like; and the salts of organic acids such as acetates, propionates, malonates, benzoates, and the like. A thorough discussion of pharmaceutically acceptable excipients is available in Remington's Pharmaceutical Sciences (Mack Pub. Co., N.J. 1991).

Pharmaceutically acceptable carriers in therapeutic compositions may contain liquids such as water, saline, glycerol and ethanol. Additionally, auxiliary substances, such as wetting or emulsifying agents, pH buffering substances, and the like, may be present in such vehicles. Typically, the therapeutic compositions are prepared as injectables, either as liquid solutions or suspensions; solid forms suitable for solution in, or suspension in, liquid vehicles prior to injection may also be prepared. Liposomes are included within the definition of a pharmaceutically acceptable carrier.

*Delivery Methods*

Once formulated, the compositions of the invention can be administered directly to the subject. The subjects to be treated can be animals; in particular, human subjects can be treated.

Direct delivery of the compositions will generally be accomplished by injection, either subcutaneously, intraperitoneally, intravenously or intramuscularly or delivered to the interstitial space of a tissue. The compositions can also be administered into a lesion. Other modes of administration include oral and pulmonary administration, suppositories, and transdermal or transcutaneous applications (*e.g.* see WO98/20734), needles, and gene guns or hyposprays. Dosage treatment may be a single dose schedule or a multiple dose schedule.

*Vaccines*

Vaccines according to the invention may either be prophylactic (*ie*. to prevent infection) or therapeutic (*ie*. to treat disease after infection).

Such vaccines comprise immunising antigen(s), immunogen(s), polypeptide(s), protein(s) or nucleic acid, usually in combination with "pharmaceutically acceptable carriers," which include any carrier that does not itself induce the production of antibodies harmful to the individual receiving the composition. Suitable carriers are typically large, slowly metabolized macromolecules such as proteins, polysaccharides, polylactic acids, polyglycolic acids, polymeric amino acids, amino acid copolymers, lipid aggregates (such as oil droplets or liposomes), and inactive virus particles. Such carriers are well known to those of ordinary skill in the art. Additionally, these carriers may function as immunostimulating agents ("adjuvants"). Furthermore, the antigen or immunogen may be conjugated to a bacterial toxoid, such as a toxoid from diphtheria, tetanus, cholera, H. *pylori, etc*. pathogens.

Preferred adjuvants to enhance effectiveness of the composition include, but are not limited to: (1) aluminum salts (alum), such as aluminum hydroxide, aluminum phosphate, aluminum sulfate, etc; (2) oil-in-water emulsion formulations (with or without other specific immunostimulating agents such as muramyl peptides (see below) or bacterial cell wall components), such as for example (a) MF59™ (W0 90114837; Chapter 10 in Vaccine design: the subunit and adjuvant approach, eds. Powell & Newman, Plenum Press 1995), containing 5% Squalene, 0.5% Tween 80, and 0.5% Span 85 (optionally containing various amounts of MTP-PE (see below), although not required) formulated into submicron particles using a microfluidizer such as Model 110Y microfluidizer (Microfluidics, Newton, MA), (b) SAF, containing 10% Squalane, 0.4% Tween 80, 5% pluronic-blocked polymer L121, and thr-MDP (see below) either microfluidized into a submicron emulsion or vortexed to generate a larger particle size emulsion, and (c) Ribi™ adjuvant system (RAS), (Ribi Immunochem, Hamilton, MT) containing 2% Squalene, 0.2% Tween 80, and one or more bacterial cell wall components from the group consisting of monophosphorylipid A (MPL), trehalose dimycolate (TDM), and cell wall skeleton (CWS), preferably MPL + CWS (Detox™); (3) saponin adjuvants, such as Stimulon™ (Cambridge Bioscience, Worcester, MA) may be used or particles generated therefrom such as ISCOMs (immunostimulating complexes); (4) Complete Freund's Adjuvant (CFA) and Incomplete Freund's Adjuvant (IFA); (5) cytokines, such as interleukins (*e.g.* IL-1, IL-2, IL-4, IL-5, IL-6, IL-7, IL-12, *etc.),* interferons (*e.g.* gamma interferon), macrophage colony stimulating factor (M-CSF), tumor necrosis factor (TNF), etc; and (6) other substances that act as immunostimulating agents to enhance the effectiveness of the composition. Alum and MF59™ are preferred.

As mentioned above, muramyl peptides include, but are not limited to, N-acetyl-muramyl-L-threonyl-D-isoglutamine (thr-MDP), N-acetyl-normuramyl-L-alanyl-D-isoglutamine (nor-MDP), N-acetylmuramyl-L-alanyl-D-isoglutaminyl-L-alanine-2-(1'-2'-dipalmitoyl-*sn*-glycero-3-hydroxyphosphoryloxy)-ethylamine (MTP-PE), *etc.*

The immunogenic compositions (*e.g.* the immunising antigen/immunogen/polypeptide/protein/ nucleic acid, pharmaceutically acceptable carrier, and adjuvant) typically will contain diluents, such as water, saline, glycerol, ethanol, etc. Additionally, auxiliary substances, such as wetting or emulsifying agents, pH buffering substances, and the like, may be present in such vehicles.

Typically, the immunogenic compositions are prepared as injectables, either as liquid solutions or suspensions; solid forms suitable for solution in, or suspension in, liquid vehicles prior to injection may also be prepared. The preparation also may be emulsified or encapsulated in liposomes for enhanced adjuvant effect, as discussed above under pharmaceutically acceptable carriers.

Immunogenic compositions used as vaccines comprise an immunologically effective amount of the antigenic or immunogenic polypeptides, as well as any other of the above-mentioned components, as needed. By "immunologically effective amount", it is meant that the administration of that amount to an individual, either in a single dose or as part of a series, is effective for treatment or prevention. This amount varies depending upon the health and physical condition of the individual to be treated, the taxonomic group of individual to be treated (*e.g.* nonhuman primate, primate, *etc.*), the capacity of the individual's immune system to synthesize antibodies, the degree of protection desired, the formulation of the vaccine, the treating doctor's assessment of the medical situation, and other relevant factors. It is expected that the amount will fall in a relatively broad range that can be determined through routine trials.

The immunogenic compositions are conventionally administered parenterally, *e.g.* by injection, either subcutaneously, intramuscularly, or transdermally/transcutaneously (*e.g.* W098120734). Additional formulations suitable for other modes of administration include oral and pulmonary formulations, suppositories, and transdermal applications. Dosage treatment may be a single dose schedule or a multiple dose schedule. The vaccine may be administered in conjunction with other immunoregulatory agents.

As an alternative to protein-based vaccines, DNA vaccination may be employed [*e.g.* Robinson & Torres (1997) Seminars in Immunology 9:271-283; Donnelly et al. (1997) Annu Rev Immunol 15:617-648; see later herein].

*Gene Delivery Vehicles*

Gene therapy vehicles for delivery of constructs including a coding sequence of a therapeutic of the invention, to be delivered to the mammal for expression in the mammal, can be administered either locally or systemically. These constructs can utilize viral or non-viral vector approaches in *in vivo* or *ex vivo* modality. Expression of such coding sequence can be induced using endogenous mammalian or heterologous promoters. Expression of the coding sequence in vivo can be either constitutive or regulated.

The invention includes gene delivery vehicles capable of expressing the contemplated nucleic acid sequences. The gene delivery vehicle is preferably a viral vector and, more preferably, a retroviral, adenoviral, adeno-associated viral (AAV), herpes viral, or alphavirus vector. The viral vector can also be an astrovirus, coronavirus, orthomyxovirus, papovavirus, paramyxovirus, parvovirus, picornavirus, poxvirus, or togavirus viral vector. See generally, Jolly (1994) Cancer Gene Therapy 1:51-64; Kimura (1994) Human Gene Therapy 5:845-852; Connelly (1995) Human Gene Therapy 6:185-193; and Kaplitt (1994) Nature Genetics 6:148-153. Retroviral vectors are well known in the art and we contemplate that any retroviral gene therapy vector is employable in the invention, including B, C and D type retroviruses, xenotropic retroviruses (for example, NZB-X1, NZB-X2 and NZB9-1 (see O'Neill (1985) J. Virol. 53:160) polytropic retroviruses *e.g.* MCF and MCF-MLV (see Kelly (1983) J. Virol. 45:291), spumaviruses and lentiviruses. See RNA Tumor Viruses, Second Edition, Cold Spring Harbor Laboratory, 1985.

Portions of the retroviral gene therapy vector may be derived from different retroviruses. For example, retrovector LTRs may be derived from a Murine Sarcoma Virus, a tRNA binding site from a Rous Sarcoma Virus, a packaging signal from a Murine Leukemia Virus, and an origin of second strand synthesis from an Avian Leukosis Virus.

These recombinant retroviral vectors may be used to generate transduction competent retroviral vector particles by introducing them into appropriate packaging cell lines (see US patent 5,591,624). Retrovirus vectors can be constructed for site-specific integration into host cell DNA by incorporation of a chimeric integrase enzyme into the retroviral particle (see WO96/37626). It is preferable that the recombinant viral vector is a replication defective recombinant virus.

Packaging cell lines suitable for use with the above-described retrovirus vectors are well known in the art, are readily prepared (see W 095/30763 and W 092105266), and can be used to create producer cell lines (also termed vector cell lines or "VCLs") for the production of recombinant vector particles. Preferably, the packaging cell lines are made from human parent cells (*e.g.* HT1080 cells) or mink parent cell lines, which eliminates inactivation in human serum.

Preferred retroviruses for the construction of retroviral gene therapy vectors include Avian Leukosis Virus, Bovine Leukemia, Virus, Murine Leukemia Virus, Mink-Cell Focus-Inducing Virus, Murine Sarcoma Virus, Reticuloendotheliosis Virus and Rous Sarcoma Virus. Particularly preferred Murine Leukemia Viruses include 4070A and 1504A (Hartley and Rowe (1976) J Virol 19:19-25), Abelson (ATCC No. VR-999), Friend (ATCC No. VR-245), Graffi, Gross (ATCC Nol VR-590), Kirsten, Harvey Sarcoma Virus and Rauscher (ATCC No. VR-998) and Moloney Murine Leukemia Virus (ATCC No. VR-190). Such retroviruses may be obtained from depositories or collections such as the American Type Culture Collection ("ATCC") in Rockville, Maryland or isolated from known sources using commonly available techniques.

Exemplary known retroviral gene therapy vectors employable in this invention include those described in patent applications GB2200651, EP0415731, EP0345242, EP0334301, WO89/02468; WO89/05349, WO89/09271, WO90/02806, WO90/07936, WO94/03622, WO93/25698, WO93/25234, W 093/11230, WO93/10218, WO91/02805, WO91/02825, WO95/07994, US 5,219,740, US 4,405,712, US 4,861,719, US 4,980,289, US 4,777,127, US 5,591,624. See also Vile (1993) Cancer Res 53:3860-3864; Vile (1993) Cancer Res 53:962-967; Ram (1993) Cancer Res 53 (1993) 83-88; Takamiya (1992) J Neurosci Res 33:493-503; Baba (1993) J Neurosurg 79:729-735; Mann (1983) Cell 33:153; Cane (1984) Proc Natl Acad Sci 81:6349; and Miller (1990) Human Gene Therapy 1.

Human adenoviral gene therapy vectors are also known in the art and employable in this invention. See, for example, Berkner (1988) Biotechniques 6:616 and Rosenfeld (1991) Science 252:431, and WO93/07283, WO93/06223, and WO93/07282. Exemplary known adenoviral gene therapy vectors employable in this invention include those described in the above referenced documents and in WO94/12649, WO93/03769, WO93/19191, WO94/28938, WO95/11984, WO95/00655, WO95/27071, WO95/29993, WO95/34671, WO96/05320, WO94/08026, WO94/11506, WO93/06223, WO94/24299, WO95/14102, WO95/24297, WO95/02697, WO94/28152, WO94/24299, WO95/09241, WO95/25807, WO95/05835, WO94/18922 and WO95/09654. Alternatively, administration of DNA linked to killed adenovirus as described in Curiel (1992) Hum. Gene Ther. 3:147-154 may be employed. The gene delivery vehicles of the invention also include adenovirus associated virus (AAV) vectors. Leading and preferred examples of such vectors for use in this invention are the AAV-2 based vectors disclosed in Srivastava, WO93/09239. Most preferred AAV vectors comprise the two AAV inverted terminal repeats in which the native D-sequences are modified by substitution of nucleotides, such that at least 5 native nucleotides and up to 18 native nucleotides, preferably at least 10 native nucleotides up to 18 native nucleotides, most preferably 10 native nucleotides are retained and the remaining nucleotides of the D-sequence are deleted or replaced with non-native nucleotides. The native D-sequences of the AAV inverted terminal repeats are sequences of 20 consecutive nucleotides in each AAV inverted terminal repeat (*ie*. there is one sequence at each end) which are not involved in HP formation. The non-native replacement nucleotide may be any nucleotide other than the nucleotide found in the native D-sequence in the same position. Other employable exemplary AAV vectors are pWP-19, pWN-1, both of which are disclosed in Nahreini (1993) Gene 124:257-262. Another example of such an AAV vector is psub201 (see Samulski (1987) J. Virol. 61:3096). Another exemplary AAV vector is the Double-D ITR vector. Construction of the Double-D ITR vector is disclosed in US Patent 5,478,745. Still other vectors are those disclosed in Carter US Patent 4,797,368 and Muzyczka US Patent 5,139,941, Chartejee US Patent 5,474,935, and Kotin WO94/288157. Yet a further example of an AAV vector employable in this invention is SSV9AFABTKneo, which contains the AFP enhancer and albumin promoter and directs expression predominantly in the liver. Its structure and construction are disclosed in Su (1996) Human Gene Therapy 7:463-470. Additional AAV gene therapy vectors are described in US 5,354,678, US 5,173,414, US 5,139,941, and US 5,252,479.

The gene therapy vectors of the invention also include herpes vectors. Leading and preferred examples are herpes simplex virus vectors containing a sequence encoding a thymidine kinase polypeptide such as those disclosed in US 5,288,641 and EP0176170 (Roizman). Additional exemplary herpes simplex virus vectors include HFEM/ICP6-LacZ disclosed in WO95/04139 (Wistar), pHSVlac described in Geller (1988) Science 241:1667-1669 and in WO90/09441 & WO92/07945, HSV Us3::pgC-lacZ described in Fink (1992) Human Gene Therapy 3:11-19 and HSV 7134, 2 RH 105 and GAL4 described in EP 0453242 (Breakefield), and those deposited with ATCC as accession numbers ATCC VR-977 and ATCC VR-260.

Also contemplated are alpha virus gene therapy vectors that can be employed in this invention. Preferred alpha virus vectors are Sindbis viruses vectors. Togaviruses, Semliki Forest virus (ATCC VR-67; ATCC VR-1247), Middleberg virus (ATCC VR-370), Ross River virus (ATCC VR-373; ATCC VR-1246), Venezuelan equine encephalitis virus (ATCC VR923; ATCC VR-1250; ATCC VR-1249; ATCC VR-532), and those described in US patents 5,091,309, 5,217,879, and WO92/10578. More particularly, those alpha virus vectors described in US Serial No. 08/405,627, filed March 15, 1995,WO94/21792, WO92/10578, WO95/07994, US 5,091,309 and US 5,217,879 are employable. Such alpha viruses may be obtained from depositories or collections such as the ATCC in Rockville, Maryland or isolated from known sources using commonly available techniques. Preferably, alphavirus vectors with reduced cytotoxicity are used (see USSN 08/679640).

DNA vector systems such as eukaryotic layered expression systems are also useful for expressing the nucleic acids of the invention. See WO95/07994 for a detailed description of eukaryotic layered expression systems. Preferably, the eukaryotic layered expression systems of the invention are derived from alphavirus vectors and most preferably from Sindbis viral vectors.

Other viral vectors suitable for use in the present invention include those derived from poliovirus, for example ATCC VR-58 and those described in Evans, Nature 339 (1989) 385 and Sabin (1973) J. Biol. Standardization 1:115; rhinovirus, for example ATCC VR-1110 and those described in Arnold (1990) J Cell Biochem L401; pox viruses such as canary pox virus or vaccinia virus, for example ATCC VR-111 and ATCC VR-2010 and those described in Fisher-Hoch (1989) Proc Natl Acad Sci 86:317; Flexner (1989) Ann NY Acad Sci 569:86, Flexner (1990) Vaccine 8:17; in US 4,603,112 and US 4,769,330 and WO89/01973; SV40 virus, for example ATCC VR-305 and those described in Mulligan (1979) Nature 277:108 and Madzak (1992) J Gen Virol 73:1533; influenza virus, for example ATCC VR-797 and recombinant influenza viruses made employing reverse genetics techniques as described in US 5,166,057 and in Enami (1990) Proc Natl Acad Sci 87:3802-3805; Enami & Palese (1991) J Virol 65:2711-2713 and Luytjes (1989) Cell 59:110, (see also McMichael (1983) NEJ Med 309:13, and Yap (1978) Nature 273:238 and Nature (1979) 277:108); human immunodeficiency virus as described in EP-0386882 and in Buchschacher (1992) J. Virol, 66:2731; measles virus, for example ATCC VR-67 and VR-1247 and those described in EP-0440219; Aura virus, for example ATCC VR-368; Bebaru virus, for example ATCC VR-600 and ATCC VR-1240; Cabassou virus, for example ATCC VR-922; Chikungunya virus, for example ATCC VR-64 and ATCC VR-1241; Fort Morgan Virus, for example ATCC VR-924; Getah virus, for example ATCC VR-369 and ATCC VR-1243; Kyzylagach virus, for example ATCC VR-927; Mayaro virus, for example ATCC VR-66; Mucambo virus, for example ATCC VR-580 and ATCC VR-1244; Ndumu virus, for example ATCC VR-371; Pixuna virus, for example ATCC VR-372 and ATCC VR-1245; Tonate virus, for example ATCC VR-925; Triniti virus, for example ATCC VR-469; Una virus, for example ATCC VR-374; Whataroa virus, for example ATCC VR-926; Y-62-33 virus, for example ATCC VR-375; 0'Nyong virus, Eastern encephalitis virus, for example ATCC VR-65 and ATCC VR-1242; Western encephalitis virus, for example ATCC VR-70, ATCC VR-1251, ATCC VR-622 and ATCC VR-1252; and coronavirus, for example ATCC VR-740 and those described in Hamre (1966) Proc Soc Exp Biol Med 121:190.

Delivery of the compositions of this invention into cells is not limited to the above mentioned viral vectors. Other delivery methods and media may be employed such as, for example, nucleic acid expression vectors, polycationic condensed DNA linked or unlinked to killed adenovirus alone, for example see US Serial No. 08/366,787, filed December 30, 1994 and Curiel (1992) Hum Gene Ther 3:147-154 ligand linked DNA, for example see Wu (1989) J Biol Chem 264: 16985-16987, eucaryotic cell delivery vehicles cells, for example see US Serial No.08/240,030, filed May 9, 1994, and US Serial No. 08/404,796, deposition of photopolymerized hydrogel materials, hand-held gene transfer particle gun, as described in US Patent 5,149,655, ionizing radiation as described in US5,206,152 and in WO92/11033, nucleic charge neutralization or fusion with cell membranes. Additional approaches are described in Philip (1994) Mol Cell Biol 14: 2411-2418 and in W offendin (1994) Proc Natl Acad Sci 91:1581-1585.

Particle mediated gene transfer may be employed, for example see US Serial No. 60/023,867. Briefly, the sequence can be inserted into conventional vectors that contain conventional control sequences for high level expression, and then incubated with synthetic gene transfer molecules such as polymeric DNA-binding cations like polylysine, protamine, and albumin, linked to cell targeting ligands such as asialoorosomucoid, as described in Wu & Wu (1987) J. Biol. Chem. 262:4429-4432, insulin as described in Hucked (1990) Biochem Pharmacol 40:253-263, galactose as described in Plank (1992) Bioconjugate Chem 3:533-539, lactose or transferrin. Naked DNA may also be employed. Exemplary naked DNA introduction methods are described in WO90/11092 and US 5,580,859. Uptake efficiency may be improved using biodegradable latex beads. DNA coated latex beads are efficiently transported into cells after endocytosis initiation by the beads. The method may be improved further by treatment of the beads to increase hydrophobicity and thereby facilitate disruption of the endosome and release of the DNA into the cytoplasm.

Liposomes that can act as gene delivery vehicles are described in US 5,422,120, WO95/13796, WO94/23697, WO91/14445 and EP-524,968. As described in USSN. 60/023,867, on non-viral delivery, the nucleic acid sequences encoding a polypeptide can be inserted into conventional vectors that contain conventional control sequences for high level expression, and then be incubated with synthetic gene transfer molecules such as polymeric DNA-binding cations like polylysine, protamine, and albumin, linked to cell targeting ligands such as asialoorosomucoid, insulin, galactose, lactose, or transferrin. Other delivery systems include the use of liposomes to encapsulate DNA comprising the gene under the control of a variety of tissue-specific or ubiquitously-active promoters. Further non-viral delivery suitable for use includes mechanical delivery systems such as the approach described in Woffendin et al (1994) Proc, Natl. Acad. Sci. USA 91(24):11581-11585. Moreover, the coding sequence and the product of expression of such can be delivered through deposition of photopolymerized hydrogel materials. Other conventional methods for gene delivery that can be used for delivery of the coding sequence include, for example, use of hand-held gene transfer particle gun, as described in US 5,149,655; use of ionizing radiation for activating transferred gene, as described in US 5,206,152 and WO92/11033

Exemplary liposome and polycationic gene delivery vehicles are those described in US 5,422,120 and 4,762,915; in WO 95/13796; WO94/23697; and WO91/14445; in EP-0524968; and in Stryer, Biochemistry, pages 236-240 (1975) W.H. Freeman, San Francisco; Szoka (1980) Biochem Biophys Acta 600:1; Bayer (1979) Biochem Biophys Acta 550:464; Rivnay (1987) Meth Enzymol 149:119; Wang (1987) Proc Natl Acad Sci 84:7851; Plant (1989) Anal Biochem 176:420.

A polynucleotide composition can comprises therapeutically effective amount of a gene therapy vehicle, as the term is defined above. For purposes of the present invention, an effective dose will be from about 0.01 mg/ kg to 50 mg/kg or 0.05 mg/kg to about 10 mg/kg of the DNA constructs in the individual to which it is administered.

*Delivery Methods*

Once formulated, the polynucleotide compositions of the invention can be administered (1) directly to the subject; (2) delivered *ex vivo*, to cells derived from the subject; or (3) *in vitro* for recombinant protein expression. The subjects to be treated can be mammals or birds. Also, human subjects can be treated.

Direct delivery of the compositions will generally be accomplished by injection, either subcutaneously, intraperitoneally, intravenously or intramuscularly or delivered to the interstitial space of a tissue. The compositions can also be administered into a lesion. Other modes of administration include oral and pulmonary administration, suppositories, and transdermal or transcutaneous applications (*e.g.* see WO98/20734), needles, and gene guns or hyposprays. Dosage treatment may be a single dose schedule or a multiple dose schedule.

Methods for the *ex vivo* delivery and reimplantation of transformed cells into a subject are known in the art and described in *e.g.* WO93/14778. Examples of cells useful in ex vivo applications include, for example, stem cells, particularly hematopoetic, lymph cells, macrophages, dendritic cells, or tumor cells.

Generally, delivery of nucleic acids for both *ex vivo* and *in vitro* applications can be accomplished by the following procedures, for example, dextran-mediated transfection, calcium phosphate precipitation, polybrene mediated transfection, protoplast fusion, electroporation, encapsulation of the polynucleotide(s) in liposomes, and direct microinjection of the DNA into nuclei, all well known in the art.

*Polynucleotide and polypeptide pharmaceutical compositions*

In addition to the pharmaceutically acceptable carriers and salts described above, the following additional agents can be used with polynucleotide and/or polypeptide compositions.

A.Polypeptides

One example are polypeptides which include, without limitation: asioloorosomucoid (ASOR); transferrin; asialoglycoproteins; antibodies; antibody fragments; ferritin; interleukins; interferons, granulocyte, macrophage colony stimulating factor (GM-CSF), granulocyte colony stimulating factor (G-CSF), macrophage colony stimulating factor (M-CSF), stem cell factor and erythropoietin. Viral antigens, such as envelope proteins, can also be used. Also, proteins from other invasive organisms, such as the 17 amino acid peptide from the circumsporozoite protein of plasmodium falciparum known as RII.

B.Hormones, Vitamins, *etc.*

Other groups that can be included are, for example: hormones, steroids, androgens, estrogens, thyroid hormone, or vitamins, folic acid.

C.Polyalkylenes, Polysaccharides, *etc.*

Also, polyalkylene glycol can be included with the desired polynucleotides/polypeptides. In a preferred embodiment, the polyalkylene glycol is polyethlylene glycol. In addition, mono-, di-, or polysaccharides can be included. In a preferred embodiment of this aspect, the polysaccharide is dextran or DEAE-dextran. Also, chitosan and poly(lactide-co-glycolide)

D.Linids, and Liposomes

The desired polynucleotide/polypeptide can also be encapsulated in lipids or packaged in liposomes prior to delivery to the subject or to cells derived therefrom.

Lipid encapsulation is generally accomplished using liposomes which are able to stably bind or entrap and retain nucleic acid. The ratio of condensed polynucleotide to lipid preparation can vary but will generally be around 1:1 (mg DNA:micromoles lipid), or more of lipid. For a review of the use of liposomes as carriers for delivery of nucleic acids, see, Hug and Sleight (1991) Biochim. Biophys. Acta. 1097:1-17; Straubinger (1983) Meth. Enzymol. 101:512-527.

Liposomal preparations for use in the present invention include cationic (positively charged), anionic (negatively charged) and neutral preparations. Cationic liposomes have been shown to mediate intracellular delivery of plasmid DNA (Felgner (1987) Proc. Natl. Acad. Sci. USA 84:7413-7416); mRNA (Malone (1989) Proc. Natl. Acad. Sci. USA 86: 6077-6081); and purified transcription factors (Debs (1990) J. Biol. Chem. 265:10189-10192), in functional form.

Cationic liposomes are readily available. For example, N[1-2,3-dioleyloxy)propyl]-N,N,N-triethylammonium (DOTMA) liposomes are available under the trademark Lipofectin, from GIBCO BRL, Grand Island, NY. (See, also, Feigner *supra*). Other commercially available liposomes include transfectace (DDAB/DOPE) and DOTAP/DOPE (Boerhinger). Other cationic liposomes can be prepared from readily available materials using techniques well known in the art. See, *e.g.* Szoka (1978) Proc. Natl. Acad. Sci. USA 75:4194-4198; WO90/11092 for a description of the synthesis of DOTAP (1,2-bis(oleoyloxy)-3-(trimethylammonio)propane) liposomes.

Similarly, anionic and neutral liposomes are readily available, such as from Avanti Polar Lipids (Birmingham, AL), or can be easily prepared using readily available materials. Such materials include phosphatidyl choline, cholesterol, phosphatidyl ethanolamine, dioleoylphosphatidyl choline (DOPC), dioleoylphosphatidyl glycerol (DOPG), dioleoylphoshatidyl ethanolamine (DOPE), among others. These materials can also be mixed with the DOTMA and DOTAP starting materials in appropriate ratios. Methods for making liposomes using these materials are well known in the art.

The liposomes can comprise multilammelar vesicles (MLVs), small unilamellar vesicles (SUVs), or large unilamellar vesicles (LUVs). The various liposome-nucleic acid complexes are prepared using methods known in the art. See *e.g.* Straubinger (1983) Meth. Immunol. 101:512-527; Szoka (1978) Proc. Natl. Acad. Sci. USA 75:4194-4198; Papahadjopoulos (1975) Biochim. Biophys. Acta 394:483; Wilson (1979) Cell 17:77); Deamer & Bangham (1976) Biochim. Biophys. Acta 443:629; Ostro (1977) Biochem. Biophys. Res. Commun. 76:836; Fraley (1979) Proc. Natl. Acad. Sci. USA 76:3348); Enoch & Strittmatter (1979) Proc. Natl. Acad. Sci. USA 76:145; Fraley (1980) J. Biol. Chem. (1980) 255:10431; Szoka & Papahadjopoulos (1978) Proc. Natl. Acad. Sci. USA 75:145; and Schaefer-Ridder (1982) Science 215:166.

E.Liponroteins

In addition, lipoproteins can be included with the polynucleotide/polypeptide to be delivered. Examples of lipoproteins to be utilized include: chylomicrons, HDL, IDL, LDL, and VLDL. Mutants, fragments, or fusions of these proteins can also be used. Also, modifications of naturally occurring lipoproteins can be used, such as acetylated LDL. These lipoproteins can target the delivery of polynucleotides to cells expressing lipoprotein receptors. Preferably, if lipoproteins are including with the polynucleotide to be delivered, no other targeting ligand is included in the composition.

Naturally occurring lipoproteins comprise a lipid and a protein portion. The protein portion are known as apoproteins. At the present, apoproteins A, B, C, D, and E have been isolated and identified. At least two of these contain several proteins, designated by Roman numerals, AI, AII, AIV; CI, CII, CIII.

A lipoprotein can comprise more than one apoprotein. For example, naturally occurring chylomicrons comprises of A, B, C, & E, over time these lipoproteins lose A and acquire C and E apoproteins. VLDL comprises A, B, C, & E apoproteins, LDL comprises apoprotein B; HDL comprises apoproteins A, C, & E.

The amino acid of these apoproteins are known and are described in, for example, Breslow (1985) Annu Rev. Biochem 54:699; Law (1986) Adv. Exp Med. Biol. 151:162; Chen (1986) J Biol Chem 261:12918; Kane (1980) Proc Natl Acad Sci USA 77:2465; and Utermann (1984) Hum Genet 65:232.

Lipoproteins contain a variety of lipids including, triglycerides, cholesterol (free and esters), and phospholipids. The composition of the lipids varies in naturally occurring lipoproteins. For example, chylomicrons comprise mainly triglycerides. A more detailed description of the lipid content of naturally occurring lipoproteins can be found, for example, in Meth. Enzymol. 128 (1986). The composition of the lipids are chosen to aid in conformation of the apoprotein for receptor binding activity. The composition of lipids can also be chosen to facilitate hydrophobic interaction and association with the polynucleotide binding molecule.

Naturally occurring lipoproteins can be isolated from serum by ultracentrifugation, for instance. Such methods are described in *Meth. Enzymol.* (*supra*); Pitas (1980) J. Biochem. 255:5454-5460 and Mahey (1979) J Clin. Invest 64: 743-750. Lipoproteins can also be produced by *in vitro* or recombinant methods by expression of the apoprotein genes in a desired host cell. See, for example, Atkinson (1986) Annu Rev Biophys Chem 15:403 and Radding (1958) Biochim Biophys Acta 30: 443. Lipoproteins can also be purchased from commercial suppliers, such as Biomedical Techniologies, Inc., Stoughton, Massachusetts, USA. Further description of lipoproteins can be found in Zuckermann et al. PCT/US97/14465.

F.Polycationic Agents

Polycationic agents can be included, with or without lipoprotein, in a composition with the desired polynucleotide/polypeptide to be delivered.

Polycationic agents, typically, exhibit a net positive charge at physiological relevant pH and are capable of neutralizing the electrical charge of nucleic acids to facilitate delivery to a desired location. These agents have both in vitro, ex vivo, and in vivo applications. Polycationic agents can be used to deliver nucleic acids to a living subject either intramuscularly, subcutaneously, etc.

The following are examples of useful polypeptides as polycationic agents: polylysine, polyarginine, polyornithine, and protamine. Other examples include histones, protamines, human serum albumin, DNA binding proteins, non-histone chromosomal proteins, coat proteins from DNA viruses, such as (X174, transcriptional factors also contain domains that bind DNA and therefore may be useful as nucleic aid condensing agents. Briefly, transcriptional factors such as C/CEBP, c-jun, c-fos, AP-1, AP-2, AP-3, CPF, Prot-1, Sp-1, Oct-1, Oct-2, CREP, and TFIID contain basic domains that bind DNA sequences.

Organic polycationic agents include: spermine, spermidine, and purtrescine.

The dimensions and of the physical properties of a polycationic agent can be extrapolated from the list above, to construct other polypeptide polycationic agents or to produce synthetic polycationic agents.

Synthetic polycationic agents which are useful include, for example, DEAE-dextran, polybrene. Lipofectin™, and lipofectAMINE™ are monomers that form polycationic complexes when combined with polynucleotides/polypeptides.

*Nucleic Acid Hybridisation*

"Hybridization" refers to the association of two nucleic acid sequences to one another by hydrogen bonding. Typically, one sequence will be fixed to a solid support and the other will be free in solution. Then, the two sequences will be placed in contact with one another under conditions that favor hydrogen bonding. Factors that affect this bonding include: the type and volume of solvent; reaction temperature; time of hybridization; agitation; agents to block the non-specific attachment of the liquid phase sequence to the solid support (Denhardt's reagent or BLOTTO); concentration of the sequences; use of compounds to increase the rate of association of sequences (dextran sulfate or polyethylene glycol); and the stringency of the washing conditions following hybridization. See Sambrook *et al.* [*supra*] vol.2, chapt. 9, pp.9.47 to 9.57.

"Stringency" refers to conditions in a hybridization reaction that favor association of very similar sequences over sequences that differ. For example, the combination of temperature and salt concentration should be chosen that is approximately 120 to 200°C below the calculated Tm of the hybrid under study. The temperature and salt conditions can often be determined empirically in preliminary experiments in which samples of genomic DNA immobilized on filters are hybridized to the sequence of interest and then washed under conditions of different stringencies. See Sambrook *et al.* at page 9.50.

Variables to consider when performing, for example, a Southern blot are (1) the complexity of the DNA being blotted and (2) the homology between the probe and the sequences being detected. The total amount of the fragment (s) to be studied can vary a magnitude of 10, from 0.1 to 1μg for a plasmid or phage digest to $10^{-9}$ to $10^{-8}$ g for a single copy gene in a highly complex eukaryotic genome. For lower complexity polynucleotides, substantially shorter blotting, hybridization, and exposure times, a smaller amount of starting polynucleotides, and lower specific activity of probes can be used. For example, a single-copy yeast gene can be detected with an exposure time of only 1 hour starting with 1 μg of yeast DNA, blotting for two hours, and hybridizing for 4-8 hours with a probe of $10^8$ cpm/μg. For a single-copy mammalian gene a conservative approach would start with 10 μg of DNA, blot overnight, and hybridize overnight in the presence of 10% dextran sulfate using a probe of greater than $10^8$ cpm/μg, resulting in an exposure time of ~24 hours.

Several factors can affect the melting temperature (Tm) of a DNA-DNA hybrid between the probe and the fragment of interest, and consequently, the appropriate conditions for hybridization and washing. In many cases the probe is not 100% homologous to the fragment. Other commonly encountered variables include the length and total G+C content of the hybridizing sequences and the ionic strength and formamide content of the hybridization buffer. The effects of all of these factors can be approximated by a single equation:

$$Tm = 81 + 16.6(\log_{10}Ci) + 0.4[\%(G + C)] - 0.6(\%formamide) - 600/n - 1.5(\%mismatch).$$

where Ci is the salt concentration (monovalent ions) and n is the length of the hybrid in base pairs (slightly modified from Meinkoth & Wahl (1984)Anal. Biochem. 138: 267-284).

In designing a hybridization experiment, some factors affecting nucleic acid hybridization can be conveniently altered. The temperature of the hybridization and washes and the salt concentration during the washes are the simplest to adjust. As the temperature of the hybridization increases (*ie*. stringency), it becomes less likely for hybridization to occur between strands that are nonhomologous, and as a result, background decreases. If the radiolabeled probe is not completely homologous with the immobilized fragment (as is frequently the case in gene family and interspecies hybridization experiments), the hybridization temperature must be reduced, and background will increase. The temperature of the washes affects the intensity of the hybridizing band and the degree of background in a similar manner. The stringency of the washes is also increased with decreasing salt concentrations.

In general, convenient hybridization temperatures in the presence of 50% formamide are 42°C for a probe with is 95% to 100% homologous to the target fragment, 37°C for 90% to 95% homology, and 32°C for 85% to 90% homology. For lower homologies, formamide content should be lowered and temperature adjusted accordingly, using the equation above. If the homology between the probe and the target fragment are not known, the simplest approach is to start with both hybridization and wash conditions which are nonstringent. If non-specific bands or high background are observed after autoradiography, the filter can be washed at high stringency and reexposed. If the time required for exposure makes this approach impractical, several hybridization and/or washing stringencies should be tested in parallel.

*Nucleic Acid Probe Assays*

Methods such as PCR, branched DNA probe assays, or blotting techniques utilizing nucleic acid probes according to the invention can determine the presence of cDNA or mRNA. A probe is said to "hybridize" with a sequence of the invention if it can form a duplex or double stranded complex, which is stable enough to be detected.

The nucleic acid probes will hybridize to the Chlamydial nucleotide sequences of the invention (including both sense and antisense strands). Though many different nucleotide sequences will encode the amino acid sequence, the native Chlamydial sequence is preferred because it is the actual sequence present in cells. mRNA represents a coding sequence and so a probe should be complementary to the coding sequence; single-stranded cDNA is complementary to mRNA, and so a cDNA probe should be complementary to the non-coding sequence.

The probe sequence need not be identical to the Chlamydial sequence (or its complement) - some variation in the sequence and length can lead to increased assay sensitivity if the nucleic acid probe can form a duplex with target nucleotides, which can be detected. Also, the nucleic acid probe can include additional nucleotides to stabilize the formed duplex. Additional Chlamydial sequence may also be helpful as a label to detect the formed duplex. For example, a non-complementary nucleotide sequence may be attached to the 5' end of the probe, with the remainder of the probe sequence being complementary to a Chlamydial sequence. Alternatively, non-complementary bases or longer sequences can be interspersed into the probe, provided that the probe sequence has sufficient complementarity with the a Chlamydial sequence in order to hybridize therewith and thereby form a duplex which can be detected.

The exact length and sequence of the probe will depend on the hybridization conditions, such as temperature, salt condition and the like. For example, for diagnostic applications, depending on the complexity of the analyte sequence, the nucleic acid probe typically contains at least 10-20 nucleotides, preferably 15-25, and more preferably ≥30 nucleotides, although it may be shorter than this. Short primers generally require cooler temperatures to form sufficiently stable hybrid complexes with the template.

Probes may be produced by synthetic procedures, such as the triester method of Matteucci et al. [J. Am. Chem. Soc. (1981) 103:3185], or according to Urdea et al. [Proc. Natl. Acad. Sci. USA (1983) 80: 7461], or using commercially available automated oligonucleotide synthesizers.

The chemical nature of the probe can be selected according to preference. For certain applications, DNA or RNA are appropriate. For other applications, modifications may be incorporated *e.g.* backbone modifications, such as phosphorothioates or methylphosphonates, can be used to increase *in vivo* half-life, alter RNA affinity, increase nuclease resistance *etc.* [*e.g.* see Agrawal & Iyer (1995) Curr Opin Biotechnol 6:12-19; Agrawal (1996) TIBTECH 14:376-387]; analogues such as peptide nucleic acids may also be used [*e.g.* see Corey (1997) TIBTECH 15:224-229; Buchardt et al. (1993) TIBTECH 11:384-386].

Alternatively, the polymerase chain reaction (PCR) is another well-known means for detecting small amounts of target nucleic acids. The assay is described in: Mullis et al. [Meth. Enzymol. (1987) 155: 335-350]; US patents 4,683,195 & 4,683,202. Two 'primers' hybridize with the target nucleic acids and are used to prime the reaction. The primers can comprise sequence that does not hybridize to the sequence of the amplification target (or its complement) to aid with duplex stability or, for example, to incorporate a convenient restriction site. Typically, such sequence will flank the desired Chlamydial sequence.

A thermostable polymerase creates copies of target nucleic acids from the primers using the original target nucleic acids as a template. After a threshold amount of target nucleic acids are generated by the polymerase, they can be detected by more traditional methods, such as Southern blots. When using the Southern blot method, the labelled probe will hybridize to the Chlamydial sequence (or its complement).

Also, mRNA or cDNA can be detected by traditional blotting techniques described in Sambrook *et al* [*supra*]. mRNA, or cDNA generated from mRNA using a polymerase enzyme, can be purified and separated using gel electrophoresis. The nucleic acids on the gel are then blotted onto a solid support, such as nitrocellulose. The solid support is exposed to a labelled probe and then washed to remove any unhybridized probe. Next, the duplexes containing the labeled probe are detected. Typically, the probe is labelled with a radioactive moiety.

## BRIEF DESCRIPTION OF THE DRAWINGS

**Figures 1-189** show data pertaining to examples 1-189.

**Figure 190** shows a representative 2D gel of proteins in elementary bodies.

**Figure 191** shows an alignment of sequences in five (six) proteins of the invention.

## EXAMPLES

The examples indicate *C.pneumoniae* proteins, together with evidence to support the view that the proteins are useful antigens for vaccine production and development or for diagnostic purposes. This evidence takes the form of:

- Computer prediction based on sequence information from CWL029 strain (*e.g.* using the PSORT algorithm available from *www.psort.nibb.ac.jp*).
- Data on recombinant expression and purification of the proteins cloned from IOL207 strain.
- Western blots to demonstrate immunoreactivity in serum (typically a blot of an EB extract of *C.pneumoniae strain* FB/96 stained with mouse antiserum against the recombinant protein).

- FACS analysis of *C.pneumoniae* bacteria or purified EBs to confirm accessibility of the antigen to the immune system (see also table III).

- An indication if the protein was identified by MALDI-TOF from a 2D gel electrophoresis map of proteins from purified elementary bodies from strain FB/96. This confirms that the protein is expressed *in vivo* (see also table V).

Various tests can be used to assess the *in vivo* immunogenicity of the proteins identified in the examples. For example, the proteins can be expressed recombinantly and used to screen patient sera by immunoblot. A positive reaction between the protein and patient serum indicates that the patient has previously mounted an immune response to the protein in question *ie.* the protein is an immunogen. This method can also be used to identify immunodominant proteins.

The recombinant protein can also be conveniently used to prepare antibodies *e.g.* in a mouse. These can be used for direct confirmation that a protein is located on the cell-surface. Labelled antibody (*e.g.* fluorescent labelling for FACS) can be incubated with intact bacteria and the presence of label on the bacterial surface confirms the location of the protein.

In particular, the following methods (A) to (O) were used to express, purify and biochemically characterise the proteins of the invention:

## CLONING OF CPN ORFs FOR EXPRESSION IN *E.COLI*

ORFs of *Chlamydia pneumoniae* (Cpn) were cloned in such a way as to potentially obtain three different kind of proteins:

a) proteins having an hexa-histidine tag at the C-terminus (cpn-His)
b) proteins having a GST fusion partner at the N-terminus (Gst-cpn)
c) proteins having both hexa-histidine tag at the C-terminus and GST at the N-terminus (GST/His fusion; $NH_2$-GST-cpn-$(His)_6$-COOH)

The type a) proteins were obtained upon cloning in the pET21b+ (Novagen). The type b) and c) proteins were obtained upon cloning in modified pGEX-KG vectors [Guan & Dixon (1991) Anal. Biochem. 192:262]. For instance pGEX-KG was modified to obtain pGEX-NN, then by modifying pGEX-NN to obtain pGEX-NNH. The Gst-cpn and Gst-cpn-His proteins were obtained in pGEX-NN and pGEX-NNH respectively.

The modified versions of pGEX-KG vector were made with the aim of allowing the cloning of single amplification products in all three vectors after only one double restriction enzyme digestion and to minimise the presence of extraneous amino acids in the final recombinant proteins.

### (A) Construction of pGEX-NN and pGEX-NNH expression vectors

Two couples of complementary oligodeoxyribonucleotides were synthesised using the DNA synthesiser ABI394 (Perkin Elmer) and the reagents from Cruachem (Glasgow, Scotland). Equimolar amounts of the oligo pairs (50 ng each oligo) were annealed in T4 DNA ligase buffer (New England Biolabs) for 10 min in a final volume of 50μl and then were left to cool slowly at room temperature. With the described procedure he following DNA linkers were obtained:

gexNN linker:

```
NdeI   NheI XmaI  EcoRI   NcoI        SalI      XhoI       SacI              NotI
GATCCCATATGGCTAGCCCGGGGAATTCGTCCATGGAGTGAGTCGACTGACTCGAGTGATCGAGCTCCTGAGCGGCCGCATGAA
    GGTATACCGATCGGGCCCCTTAAGCAGGTACCTCACTCAGCTGACTGAGCTCACTAGCTCGAGGACTCGCCGGCGTACTTTCGA
```

gexNNH linker:

```
     HindIII NotI  XhoI   --Hexa-Histidine--
TCGACAAGCTTGCGGCCGCACTCGAGCATCACCATCACCATCACTGAT
    GTTCGAACGCCGGCGTGAGCACGTAGAGGTAGTGGTAGTGACTATCGA
```

The plasmid pGEX-KG was digested with BamHI and HindIII and 100 ng were ligated overnight at 16 °C to the linker gexNN with a molar ratio of 3:1 linker/plasmid using 200 units of T4 DNA ligase (New england Biolabs). After transformation of the ligation product in *E. coli* DH5, a clone containing the pGEX-NN plasmid, having the correct linker, was selected by means of restriction enzyme analysis and DNA sequencing.

The new plasmid pGEX-NN was digested with SalI and HindIII and ligated to the linker gexNNH. After transformation of the ligation product in *E. coli* DH5, a clone containing the pGEX-NNH plasmid, having the correct linker, was selected by means of restriction enzyme analysis and DNA sequencing.

**(B) Chromosomal DNA preparation**

The chromosomal DNA of elementary bodies (EB) of *C.pneumoniae* strain 10L-207 was prepared by adding 1.5 ml of lysis buffer (10 mM Tris-HCl, 150 mM NaCl, 2 mM EDTA, 0,6 % SDS, 100 μg/ml Proteinase K, pH 8) to 450 μl EB suspension (400.000/μl) and incubating overnight at 37 °C. After sequential extraction with phenol, phenol-chloroform, and chloroform, the DNA was precipitated with 0,3 M sodium acetate, pH 5,2 and 2 volumes of absolute ethanol. The DNA pellet was washed with 70 % ethanol. After solubilization with distilled water and treatment with 20 μg/ml RNAse A for 1 hour at RT, the DNA was extracted again with phenol-chloroform, alcohol precipitated and suspended with 300 μl 1 mM Tris-HCl pH 8,5. The DNA concentration was evaluated by measuring $OD_{260}$ of the sample.

**(C) Oligonucleotide design**

Synthetic oligonucleotide primers were designed on the basis of the coding sequence of each ORF using the sequence of *C.pneumoniae* strain CWL029. Any predicted signal peptide were omitted, by deducing the 5' end amplification primer sequence immediately downstream from the predicted leader sequence. For most ORFs, the 5' tail of the primers (table I) included only one restriction enzyme recognition site (NdeI, or NheI, or SpeI depending on the gene's own restriction pattern); the 3' primer tails (tableI) included a XhoI or a NotI or a HindIII restriction site.

**Table I.** Oligonucleotide tails of the primers used to amplify Cpn genes.

| 5' tails | | 3' tails | |
|---|---|---|---|
| NdeI | 5' GTGCGT**CATATG** 3' | XhoI | 5' GCGT**CTCGAG** 3' |
| NheI | 5' GTGCGT**GCTAGC** 3' | NotI | 5' ACTCGCTA**GCGGCCGC** 3' |
| SpeI | 5' GTGCGT**ACTAGT** 3' | HindIII | 5' GCGT**AAGCTT** 3' |

As well as containing the restriction enzyme recognition sequences, the primers included nucleotides which hybridized to the sequence to be amplified. The number of hybridizing nucleotides depended on the melting temperature of the primers which was determined as described [(Breslauer et al. (1986) PNAS USA 83:3746-50]. The average melting temperature of the selected oligos was 50-55°C for the hybridizing region alone and 65-75°C for the whole oligos. Table II shows the forward and reverse primers used for each amplification.

**(D) Amplification**

The standard PCR protocol was as follow: 50 ng genomic DNA were used as template in the presence of 0,2 μM each primer, 200 μM each dNTP, 1,5 mM $MgCl_2$, 1x PCR buffer minus Mg (Gibco-BRL), and 2 units of Taq DNA polymerase (Platinum Taq, Gibco-BRL) in a final volume of 100 μl. Each sample underwent a double-step amplification: the first 5 cycles were performed using as the hybridizing temperature the one of the oligos excluding the restriction enzyme tail, followed by 25 cycles performed according to the hybridization temperature of the whole lenght primers. The standard cycles were as follow:

denaturation : 94 °C, 2 min

denaturation: 94 °C, 30 seconds
hybridization: 51 °C, 50 seconds } 5 cycles
elongation: 72 °C, 1 min or 2 min and 40 sec

denaturation: 94 °C, 30 seconds
hybridization: 70 °C, 50 seconds } 25 cycles
elongation: 72 °C, 1 min or 2 min and 40 sec

72 °C, 7 min
4 °C

The elongation time was 1 min for ORFs shorter than 2000 bp, and 2 min and 40 seconds for ORFs longer than 2000 bp. The amplifications were performed using a Gene Amp PCR system 9600 (Perkin Elmer).

To check the amplification results, 4 μl of each PCR product was loaded onto 1-1.5 agarose gel and the size of amplified fragments compared with DNA molecular weight standards (DNA markers III or IX, Roche). The PCR products were loaded on agarose gel and after electrophoresis the right size bands were excised from the gel. The DNA was purified from the agarose using the Gel Extraction Kit (Qiagen) following the instruction of the manufacturer. The final elution volume of the DNA was 50 μl TE (10 mM Tris-HCl, 1 mM EDTA, pH 8). One μl of each purified DNA was loaded onto agarose gel to evaluate the yield.

**(E) Digestion of PCR fragments**

One-two μg of purified PCR product were double digested overnight at 37 °C with the appropriate restriction enzymes (60 units of each enzyme) using the appropriate restriction buffer in 100 μl final volume. The restriction enzymes and the digestion buffers were from New England Biolabs. After purification of the digested DNA (PCR purification Kit, Qiagen) and elution with 30 μl TE, 1 μl was subjected to agarose gel electrophoresis to evaluate the yield in comparison to titrated molecular weight standards (DNA markers III or IX, Roche).

**(F) Digestion of the cloning vectors (pET21b+, pGEX-NN, and pGEX-NNH)**

10 μg of plasmid was double digested with 100 units of each restriction enzyme in 400 μl reaction volume in the presence of appropriate buffer by overnight incubation at 37 °C. After electrophoresis on a 1% agarose gel, the band corresponding to the digested vector was purified from the gel using the Qiagen Qiaex II Gel Extraction Kit and the DNA was eluted with 50 μl TE. The DNA concentration was evaluated by measuring $OD_{260}$ of the sample.

**(G) Cloning**

75ng of the appropriately digested and purified vectors and the digested and purified fragments corresponding to each ORF, were ligated in final volumes of 10-20 μl with a molar ratio of 1:1 fragment/vector, using 400 units T4 DNA ligase (New England Biolabs) in the presence of the buffer supplied by the manufacturer. The reactions were incubated overnight at 16 °C.

Transformation in *E coli* DH5 competent cells was performed as follow: the ligation reaction was mixed with 200 μl of competent DH5 cells and incubated on ice for 30 min and then at 42 °C for 90 seconds. After cooling on ice, 0.8 ml LB was added and the cells were incubated for 45 min at 37 °C under shaking. 100 and 900 μl of cell suspensions were plated on separate plates of agar LB 100 μg/ml Ampicillin and the plates were incubated overnight at 37 °C. The screening of the transformants was done by growing randomly chosen clones in 6 ml LB 100 μg/ml Ampicillin, by extracting the DNA using the Qiagen Qiaprep Spin Miniprep Kit following the manufacturer instructions, and by digesting

2 μl of plasmid minipreparation with the restriction enzymes specific for the restriction cloning sites. After agarose gel electrophoresis of the digested plasmid mini-preparations, positive clones were chosen on the basis of the correct size of the restriction fragments, as evaluated by comparison with appropriate molecular weight markers (DNA markers III or IX, Roche).

**(H) Expression**

1 μl of each right plasmid mini-preparation was transformed in 200 μl of competent E. *coli* strain suitable for expression of the recombinant protein. All pET21b+ recombinant plasmids were transformed in BL21 DE3 (Novagen) *E. coli* cells, whilst all pGEX-NN and all pGEX-NNH recombinant plasmids were transformed in BL21 cells (Novagen). After plating transformation mixtures on LB/Amp agar plates and incubation overnight at 37 °C, single colonies were inoculated in 3 ml LB 100 μg/ml Ampicillin and grown at 37 °C overnight. 70 μl of the overnight culture was inoculated in 2 ml LB/Amp and grown at 37 °C until $OD_{600}$ of the pET clones reached the 0,4-0,8 value or until $OD_{600}$ of the pGEX clones reached the 0,8-1 value. Protein expression was then induced by adding IPTG (Isopropil β-D thio-galacto-piranoside) to the mini-cultures. pET clones were induced using 1 mM IPTG, whilst pGEX clones were induced using 0.2 mM IPTG. After 3 hours incubation at 37 °C the final $OD_{600}$ was checked and the cultures were cooled on ice. After centrifugation of 0.5 ml culture, the cell pellet was suspended in 50 μl of protein Loading Sample Buffer (60 mM TRIS-HCl pH 6.8, 5% w/v SDS, 10% v/v glycerin, 0.1 % w/v Bromophenol Blue, 100 mM DTT) and incubated at 100 °C for 5 min. A volume of boiled sample corresponding to 0.1 $OD_{600}$ culture was analysed by SDS-PAGE and Coomassie Blue staining to verify the presence of induced protein band.

**PURIFICATION OF THE RECOMBINANT PROTEINS**

Single colonies were inoculated in 25 ml LB 100 μg/ml Ampicillin and grown at 37 °C overnight. The overnight culture was inoculated in 500 ml LB/Amp and grown under shaking at 25 °C until $OD_{600}$ 0,4-0,8 value for the pET clones, or until $OD_{600}$ 0,8-1 value for the pGEX clones. Protein expression was then induced by adding IPTG to the cultures. pET clones were induced using 1 mM IPTG, whilst pGEX clones were induced using 0.2 mM IPTG. After 4 hours incubation at 25 °C the final $OD_{600}$ was checked and the cultures were cooled on ice. After centrifugation at 6000 rpm (JA10 rotor, Beckman), the cell pellet was processed for purification or frozen at -20°C.

**(I) Procedure for the purification of soluble His-tagged proteins from *E.coli***

1. Transfer the pellets from -20°C to ice bath and reconstitute with 10 ml 50 mM $NaHPO_4$ buffer, 300 mM NaCl, pH 8,0, pass in 40-50 ml centrifugation tubes and break the cells as per the following outline:
2. Break the pellets in the French Press performing <u>three</u> passages with in-line washing.
3. Centrifuge at about 30-40000 x g per 15-20 min. If possible use rotor JA 25.50 (21000 rpm, 15 min.) or JA-20 (18000 rpm, 15 min.)
4. Equilibrate the Poly-Prep columns with 1 ml Fast Flow Chelating Sepharose resin with 50 mM phosphate buffer, 300 mM NaCl, pH 8,0.
5. Store the centrifugation pellet at -20°C, and load the supernatant in the columns.
6. Collect the flow through.
7. Wash the columns with 10 ml (2 ml + 2 ml + 4 ml) 50 mM phosphate buffer, 300 mM NaCl, pH 8,0.
8. Wash again with 10 ml 20 mM imidazole buffer, 50 mM phosphate, 300 mM NaCl, pH 8,0.
9. Elute the proteins bound to the columns with 4,5 ml (1,5 ml + 1,5 ml + 1,5 ml) 250 mM imidazole buffer, 50 mM phosphate, 300 mM NaCl, pH 8,0 and collect the 3 corresponding fractions of ~1,5 ml each. Add to each tube 15 μl DTT 200 mM (final concentration 2 mM)
10. Measure the protein concentration of the first two fractions with the Bradford method, collect a 10 μg aliquot of proteins from each sample and analyse by SDS-PAGE. (<u>N.B.:</u> should the sample be too diluted, load 21 μl + 7 μl loading buffer).
11. Store the collected fractions at +4°C while waiting for the results of the SDS-PAGE analysis.
12. For immunisation prepare 4-5 aliquots of 100 μg each in 0,5 ml in 40% glycerol. The dilution buffer is the above elution buffer, plus 2 mM DTT. Store the aliquots at -20°C until immunisation.

**(J) Purification of His-tagged proteins from Inclusion bodies**

Purifications were carried out essentially according the following protocol:

1. Bacteria are collected from 500 ml cultures by centrifugation. If required store bacterial pellets at -20°C. For extraction, resuspend each bacterial pellet in 10 ml 50 mM TRIS-HCl buffer, pH 8,5 on an ice bath.

2. Disrupt the resuspended bacteria with a French Press, performing two passages.

3. Centrifuge at 35000 x g for 15 min and collect the pellets. Use a Beckman rotor JA 25.50 (21000 rpm, 15 min.) or JA-20 (18000 rpm, 15 min.).

4. Dissolve the centrifugation pellets with 50 mM TRIS-HCl, 1 mM TCEP {Tris(2-carboxyethyl)-phosphine hydrochloride, Pierce} , 6M guanidium chloride, pH 8,5. Stir for ~ 10 min. with a magnetic bar.

5. Centrifuge as described above, and collect the supernatant..

6. Prepare an adequate number of Poly-Prep (Bio-Rad) columns containing 1 ml of Fast Flow Chelating Sepharose (Pharmacia) saturated with Nichel according to manufacturer recommendations.. Wash the columns twice with 5 ml of $H_2O$ and equilibrate with 50 mM TRIS-HCl, 1 mM TCEP, 6M guanidinium chloride, pH 8,5.

7. Load the supernatants from step 5 onto the columns, and wash with 5 ml of 50 mM TRIS-Hcl buffer, 1 mM TCEP, 6M urea, pH 8,5

8. Wash the columns with 10 ml of 20 mM imidazole, 50 mM TRIS-HCl , 6M urea, 1 mM TCEP, pH 8,5. Collect and set aside the first 5 ml for possible further controls.

9. Elute the proteins bound to the columns with 4,5 ml of a buffer containing 250 mM imidazole, 50 mM TRIS-HCl, 6M urea, 1 mM TCEP, pH 8,5. Add the elution buffer in three 1,5 ml aliquots, and collect the corresponding 3 fractions. Add to each fraction 15 μl DTT (final concentration 2 mM).

10. Measure eluted protein concentration with the Bradford method, and analyze aliquots of ca 10 μg of protein by SDS-PAGE.

11. Store proteins at -20°C in 40% (v/v) glycerol, 50 mM TRIS-HCl, 2M urea, 0.5 M arginine, 2 mM DTT, 0.3 mM TCEP, 83.3 mM imidazole, pH 8,5

**(K) Procedure for the purification of GST-fusion proteins from *E.coli***

1. Transfer the bacterial pellets from -20°C to an ice bath and resuspend with 7,5 ml PBS, pH 7,4 to which a mixture of protease inhibitors (C∅MPLET™ - Boehringer Mannheim, 1 tablet every 25 ml of buffer) has been added. Transfer to 40-50 ml centrifugation tubes and sonicate according to the following procedure:

   a) Position the probe at about 0,5 cm from the bottom of the tube
   b) Block the tube with the clamp
   c) Dip the tube in an ice bath
   d) Set the sonicator as follows: Timer → Hold, Duty Cycle → 55, Out. Control → 6.
   e) perform 5 cycles of 10 impulses at a time lapse of 1 minute (i.e. one cycle = 10 impulses + ~45" hold; b. 10 impulses + ~45" hold; c. 10 impulses + ~45" hold; d. 10 impulses + ~45" hold; e. 10 impulses + ~45" hold)

2. Centrifuge at about 30-40000 x g for 15-20 min. E.g.: use rotor Beckman JA 25.50 at 21000 rpm, for 15 min.

3. Store the centrifugation pellets at -20°C, and load the supernatants on the chromatography columns, as follows

4. Equilibrate the Poly-Prep (Bio-Rad) columns with 0,5 ml (≅1 ml suspension) of Glutathione-Sepharose 4B resin, wash with 2 ml (1 + 1) $H_2O$, and then with 10 ml (2 + 4 + 4) PBS, pH 7,4.

5. Load the supernatants on the columns and discard the flow through.

6. Wash the columns with 10 ml (2 + 4 + 4) PBS, pH 7,4.

7. Elute the proteins bound to the columns with 4,5 ml of 50 mM TRIS buffer, 10 mM reduced glutathione, pH 8.0, adding 1,5 ml + 1,5 ml + 1,5 ml and collecting the respective 3 fractions of ~1,5 ml each.

8. Measure the protein concentration of the first two fractions with the Bradford method, analyse a 10 μg aliquot of proteins from each sample by SDS-PAGE. (N.B.: if the sample is too diluted load 21 μl (+ 7 μl loading buffer).

9. Store the collected fractions at +4°C while waiting for the results of the SDS-PAGE analysis.

10. For each protein destined to the immunisation prepare 4-5 aliquots of 100 μg each in 0,5 ml of 40% glycerol. The dilution buffer is 50 mM TRIS.HCl, 2 mM DTT, pH 8,0. Store the aliquots at -20°C until immunisation..

**SEROLOGY**

**(L) Protocol of immunization**

1. Groups of four CD1 female mice aged between 6 and 7 weeks were immunized with 20 μg of recombinant protein resuspended in 100 μl.

2. Four mice for each group received 3 doses with a 14 days interval schedule.

3. Immunization was performed through intra-peritoneal injection of the protein with an equal volume of Complete Freund's Adjuvant (CFA) for the first dose and Incomplete Freund's Adjuvant (IFA) for the following two doses.

4. Sera were collected before each immunization. Mice were sacrified 14 days after the third immunization and the collected sera were pooled and stored at -20°C.

**(M) Western blot analysis of Cpn elementary body proteins with mouse sera**

Aliquots of elementary bodies containing approximately 4 μg of proteins, mixed with SDS loading buffer (1x: 60 mM TRIS-HCl pH 6.8, 5% w/v SDS, 10% v/v glycerin, 0.1% Bromophenol Blue, 100 mM DTT) and boiled 5 minutes at 95° C, were loaded on a 12% SDS-PAGE gel. The gel was run using a SDS-PAGE running buffer containing 250 mM TRIS, 2.5 mM Glycine and 0.1 %SDS. The gel was electroblotted onto nitrocellulose membrane at 200 mA for 30 minutes. The membrane was blocked for 30 minutes with PBS, 3% skimmed milk powder and incubated O/N at 4° C with the appropriate dilution (1/100) of the sera. After washing twice with PBS + 0.1% Tween (Sigma) the membrane was incubated for 2 hours with peroxidase-conjugated secondary anti-mouse antibody (Sigma) diluted 1:3000. The nitrocellulose was washed twice for 10 minutes with PBS +0.1% Tween-20 and once with PBS and thereafter developed by Opti-4CN Substrate Kit (Biorad).

Lanes shown in Western blots are: (P) = pre-immune control serum; (I) = immune serum.

**(N) FACS analysis of *Chlamydia pneumoniae* elementary bodies with mouse sera**

1. $2x10^5$ Elementary Bodies (EB)/well were washed with 200 μl of PBS-0.1%BSA in a 96 wells U bottom plate and centrifuged for 10 min. at 1200rpm, at 4°C.

2. The supernatant was discarded and the E.B. resuspended in 10 μl of PBS-0.1%BSA.

3. 10μl mouse sera diluted in PBS-0.1%BSA were added to the E.B. suspention to a final dilution of 1:400, and incubated on ice for 30 min.

4. EB were washed by adding 180μl PBS-0.1%BSA and centrifuged for 10min. at 1200rpm, 4°C.

5. The supernatant was discarded and the E.B. resuspended in 101 of PBS-0.1%BSA.

6. 10μl of a goat anti-mouse IgG, $F(ab')_2$ fragment specific-R-Phycoerythrin-conjugated (Jackson Immunoresearch Laboratories Inc., cat.N°115-116-072) was added to the EB suspension to a final dilution of 1:100, and incubated on ice for 30 min. in the dark.

7. EB were washed by adding 180μl PBS-0.1%BSA and centrifuged for 10min. at 1200rpm, 4°C.

8. The supernatant was discarded and the E.B. resuspended in 150 μl of PBS-0.1%BSA.

9. E.B. suspension was passed through a cytometric chamber of a FACS Calibur (Becton Dikinson, Mountain View, CA USA) and 10.000 events were acquired.

10. Data were analysed using Cell Quest Software (Becton Dikinson, Mountain View, CA USA) by drawing a morphological dot plot (using forward and side scatter parameters) on E.B. signals. An histogram plot was then created on FL2 intensity of fluorescence log scale recalling the morphological region of EB.

NB: the results of FACS depend not only on the extent of accessibility of the native antigens but also on the quality of the antibodies elicited by the recombinant antigens, which may have structures with a variable degree of correct folding as compared with the native protein structures. Therefore, even if a FACS assay appears negative this does not necessarily mean that the protein is not abundant or accessible on the surface. PorB antigen, for instance, gave negative results in FACS but is a surface-exposed neutralising antigen [Kubo & Stephens (2000) Mol. Microbiol. 38:772-780].

**(O) Mass Spectrometry analysis of two-dimensional electrophoretic protein maps**

Gradient purified EBs from strain FB/96 were solubilized at a final concentration of 5.5mg/ml with immobiline rehydratation buffer (7M urea, 2M thiourea, 2% (w/v) CHAPS, 2% (w/v) ASB 14 [Chevallet et al. (1998) Electrophor. 19: 1901-9], 2% (v/v) C.A 3-10NL (Amersham Pharmacia Biotech), 2 mM tributyl phosphine, 65 mM DTT). Samples (250 μg protein) were adsorbed overnight on Immobiline DryStrips (7 cm, pH 3-10 non linear). Electrophocusing was performed in a IPGphor Isoelectric Focusing Unit (Amersham Pharmacia Biotech). Before PAGE separation, the focused strips were incubated in 4M urea, 2M thiourea, 30% (v/v) glycerol, 2% (w/v) SDS, 5mM tributyl phosphine 2.5%(w/v) acrylamide, 50mM Tris-HCl pH 8.8, as described [Herbert et al. (1998) Electrophor. 19:845-51]. SDS-PAGE was performed on linear 9-16% acrylamide gradients. Gels were stained with colloidal Coomassie (Novex, San Diego) [Doherty et al. (1998) Electrophor. 19:355-63]. Stained gels were scanned with a Personal Densitometer SI (Molecular Dynamics) at 8 bits and 50μm per pixel. Map images were annotated with the software Image Master 2D Elite, version 3.10 (Amersham Pharmacia Biotech). Protein spots were excised from the gel, using an Ettan Spot picker (Amersham Pharmacia Biotech), and dried in a vacuum centrifuge. In-gel digestion of samples for mass spectrometry and extraction of peptides were performed as described by Wilm et al. [Nature (1996) 379:466-9]. Samples were desalted with a ZIP TIP (Millipore), eluted with a saturated solution of alpha-cyano-4-hydroxycinnamic acid in 50% acetonitrile, 0.1% TFA and directly loaded onto a SCOUT 381 multiprobe plate (Bruker). Spectra were acquired on a Bruker Biflex II MALDI-TOF. Spectra were calibrated using a combination of known standard peptides, located in spots adjacent to the samples. Resulting values for monoisotopic peaks were used for database searches using the computer program Mascot (www.matrixscience.com). All searches were performed using an error of 200-500ppm as constraint. A representative gel is shown in Figure 190.

**Example 1**

The following *C.pneumoniae* protein (PID 4376552) was expressed <SEQ ID 1; cp6552>:

```
  1  MKKKLSLLVG LIFVLSSCHK EDAQNKIRIV ASPTPHAELL ESLQEEAKDL

 51  GIKLKILPVD DYRIPNRLLL DKQVDANYFQ HQAFLDDECE RYDCKGELVV
101  IAKVHLEPQA IYSKKHSSLE RLKSQKKLTI AIPVDRTNAQ RALHLLEECG
151  LIVCKGPANL NMTAKDVCGK ENRSINILEV SAPLLVGSLP DVDAAVIPGN
201  FAIAANLSPK KDSLCLEDLS VSKYTNLVVI RSEDVGSPKM IKLQKLFQSP
251  SVQHFFDTKY HGNILTMTQD NG*
```

A predicted signal peptide is highlighted.

The cp6552 nucleotide sequence <SEQ ID 2> is:

```
  1 ATGAAAAAAA AATTATCATT ACTTGTAGGT TTAATTTTTG TTTTGAGTTC
 51 TTGCCATAAG GAAGATGCTC AGAATAAAAT ACGTATTGTA GCCAGTCCGA
101 CACCTCATGC GGAATTATTG GAGAGTTTAC AGGAAGAGGC TAAAGATCTT
151 GGAATCAAGC TGAAAATACT TCCAGTAGAT GATTATCGTA TTCCTAATCG
201 TTTGCTTTTG GATAAACAAG TAGATGCAAA TTACTTTCAA CATCAAGCTT
251 TTCTTGATGA CGAATGCGAG CGTTATGATT GTAAGGGTGA ATTAGTTGTT
301 ATCGCTAAAG TTCATTTGGA ACCTCAAGCA ATTTATTCTA AGAAACATTC
351 TTCTTTAGAG CGCTTAAAAA GCCAGAAGAA ACTGACTATA GCGATTCCTG
401 TGGATCGTAC GAATGCTCAG CGTGCTCTAC ACTTGTTAGA AGAGTGCGGA
451 CTCATTGTTT GCAAAGGGCC TGCTAATTTA AATATGACAG CTAAAGATGT
501 CTGTGGGAAA GAAAATAGAA GTATCAACAT ATTAGAGGTG TCAGCTCCTC
551 TTCTTGTCGG ATCTCTTCCT GACGTTGATG CTGCTGTCAT TCCTGGAAAT
601 TTTGCTATAG CAGCAAACCT TTCTCCAAAG AAAGATAGTC TTTGTTTAGA
651 GGATCTTTCG GTATCTAAGT ATACAAACCT TGTTGTCATT CGTTCTGAAG
701 ACGTAGGTTC TCCTAAAATG ATAAAATTAC AGAAGCTGTT TCAATCTCCT
751 TCTGTACAAC ATTTTTTTGA TACAAAATAT CATGGGAATA TTTTGACAAT
801 GACTCAAGAC AATGGTTAG
```

The PSORT algorithm predicts an inner membrane location (0.127).

The protein was expressed in *E.coli* and purified as a his-tag product, as shown in Figure 1A, and also as a GST-fusion. The recombinant protein was used to immunise mice, whose sera were used in a Western blot (Figure 1B) and for FACS analysis (Figure 1C).

The cp6552 protein was also identified in the 2D-PAGE experiment (Cpn0278).

These experiments show that cp6552 is a surface-exposed and immunoaccessible protein, and that it is a useful immunogen. These properties are not evident from the sequence alone.

## Example 2

The following *C.pneumoniae* protein (PID 4376736) was expressed <SEQ ID 3; cp6736>:

1 **MKTSIRKFLI STTLAPCFAS TAFT**VEVIMP SENFDGSSGK IFPYTTLSDP<br>
51 RGTLCIFSGD LYIANLDNAI SRTSSSCFSN RAGALQILGK GGVFSFLNIR<br>
101 SSADGAAISS VITQNPELCP LSFSGFSQMI FDNCESLTSD TSASNVIPHA<br>
151 SAIYATTPML FTNNDSILFQ YNRSAGFGAA IRGTSITIEN TKKSLLFNGN<br>
201 GSISNGGALT GSAAINLINN SAPVIFSTNA TGIYGGAIYL TGGSMLTSGN<br>
251 LSGVLFVNNS SRSGGAIYAN GNVTFSNNSD LTFQNNTASP QNSLPAPTPP<br>
301 PTPPAVTPLL GYGGAIFCTP PATPPPTGVS LTISGENSVT FLENIASEQG<br>
351 GALYGKKISI DSNKSTIFLG NTAGKGGAIA IPESGELSLS ANQGDILFNK<br>
401 NLSITSGTPT RNSIHFGKDA KFATLGATQG YTLYFYDPIT SDDLSAASAA<br>
451 ATVVVNPKAS ADGAYSGTIV FSGETLTATE AATPANATST LNQKLELEGG<br>
501 TLALRNGATL NVHNFTQDEK SVVIMDAGTT LATTNGANNT DGAITLNKLV<br>
551 INLDSLDGTK AAVVNVQSTN GALTISGTLG LVKNSQDCCD NHGMFNKDLQ<br>
601 QVPILELKAT SNTVTTTDFS LGTNGYQQSP YGYQGTWEFT IDTTTHTVTG<br>
651 NWKKTGYLPH PERLAPLIPN SLWANVIDLR AVSQASAADG EDVPGKQLSI<br>
701 TGITNFFHAN HTGDARSYRH MGGGYLINTY TRITPDAALS LGFGQLFTKS<br>
751 KDYLVGHGHS NVYFATVYSN ITKSLFGSSR FFSGGTSRVT YSRSNEKVKT<br>
801 SYTKLPKGRC SWSNNCWLGE LEGNLPITLS SRILNLKQII PFVKAEVAYA<br>
851 THGGIQENTP EGRIFGHGHL LNVAVPVGVR FGKNSHNRPD FYTIIVAYAP<br>
901 DVYRHNPDCD TTLPINGATW TSIGNNLTRS TLLVQASSHT SVNDVLEIFG<br>
951 HCGCDIRRTS RQYTLDIGSK LRF*

A predicted signal peptide is highlighted.

The cp6736 nucleotide sequence <SEQ ID 4> is:

1 ATGAAAACGT CTATTCGTAA GTTCTTAATT TCTACCACAC TGGCGCCATG
51 TTTTGCTTCA ACAGCGTTTA CTGTAGAAGT TATCATGCCT TCCGAGAACT
101 TTGATGGATC GAGTGGGAAG ATTTTTCCTT ACACAACACT TTCTGATCCT
151 AGAGGGACAC TCTGTATTTT TTCAGGGGAT CTCTACATTG CGAATCTTGA
201 TAATGCCATA TCCAGAACCT CTTCCAGTTG CTTTAGCAAT AGGGCGGGAG
251 CACTACAAAT CTTAGGAAAA GGTGGGGTTT TCTCCTTCTT AAATATCCGT
301 TCTTCAGCTG ACGGAGCCGC GATTAGTAGT GTAATCACCC AAAATCCTGA
351 ACTATGTCCC TTGAGTTTTT CAGGATTTAG TCAGATGATC TTCGATAACT
401 GTGAATCTTT GACTTCAGAT ACCTCAGCGA GTAATGTCAT ACCTCACGCA
451 TCGGCGATTT ACGCTACAAC GCCCATGCTC TTTACAAACA ATGACTCCAT
501 ACTATTCCAA TACAACCGTT CTGCAGGATT TGGAGCTGCC ATTCGAGGCA
551 CAAGCATCAC AATAGAAAAT ACGAAAAAGA GCCTTCTCTT TAATGGTAAT
601 GGATCCATCT CTAATGGAGG GGCCCTCACG GGATCTGCAG CGATCAACCT
651 CATCAACAAT AGCGCTCCTG TGATTTTCTC AACGAATGCT ACAGGGATCT
701 ATGGTGGGGC TATTTACCTT ACCGGAGGAT CTATGCTCAC CTCTGGGAAC
751 CTCTCAGGAG TCTTGTTCGT TAATAATAGC TCGCGCTCAG GAGGCGCTAT
801 CTATGCTAAC GGAAATGTCA CATTTTCTAA TAACAGCGAC CTGACTTTCC
851 AAAACAATAC AGCATCTCCA CAAAACTCCT TACCTGCACC TACACCTCCA
901 CCTACACCAC CAGCAGTCAC TCCTTTGTTA GGATATGGAG GCGCCATCTT
951 CTGTACTCCT CCAGCTACCC CCCCACCAAC AGGTGTTAGC CTGACTATAT
1001 CTGGAGAAAA CAGCGTTACA TTCCTAGAAA ACATTGCCTC CGAACAAGGA
1051 GGAGCCCTCT ATGGCAAAAA GATCTCTATA GATTCTAATA AATCTACAAT
1101 ATTTCTTGGA AATACAGCTG GAAAAGGAGG CGCTATTGCT ATTCCCGAAT
1151 CTGGGGAGCT CTCTCTATCC GCAAATCAAG GTGATATCCT CTTTAACAAG
1201 AACCTCAGCA TCACTAGTGG GACACCTACT CGCAATAGTA TTCACTTCGG
1251 AAAAGATGCC AAGTTTGCCA CTCTAGGAGC TACGCAAGGC TATACCCTAT
1301 ACTTCTATGA TCCGATTACA TCTGATGATT TATCTGCTGC ATCCGCAGCC
1351 GCTACTGTGG TCGTCAATCC CAAAGCCAGT GCAGATGGTG CGTATTCAGG
1401 GACTATTGTC TTTTCAGGAG AAACCCTCAC TGCTACCGAA GCAGCAACCC
1451 CTGCAAATGC TACATCTACA TTAAACCAAA AGCTAGAACT TGAAGGCGGT
1501 ACTCTCGCTT TAAGAAACGG TGCTACCTTA AATGTTCATA ACTTCACGCA
1551 AGATGAAAAG TCCGTCGTCA TCATGGATGC AGGGACCACA TTAGCAACTA
1601 CAAATGGAGC TAATAATACT GACGGTGCTA TCACCTTAAA CAAGCTTGTA
1651 ATCAATCTGG ATTCTTTGGA TGGCACTAAA GCGGCTGTCG TTAATGTGCA
1701 GAGTACCAAT GGAGCTCTCA CTATATCCGG AACTTTAGGA CTTGTGAAAA
1751 ACTCTCAAGA TTGCTGTGAC AACCACGGGA TGTTTAATAA AGATTTACAG
1801 CAAGTTCCGA TTTTAGAACT CAAAGCGACT TCAAATACTG TAACCACTAC
1851 GGACTTCAGT CTCGGCACAA ACGGCTATCA GCAATCTCCC TATGGGTATC
1901 AAGGAACTTG GGAGTTTACC ATAGACACGA CAACCCATAC GGTCACAGGA
1951 AATTGGAAAA AAACCGGTTA TCTTCCTCAT CCGGAGCGTC TTGCTCCCCT
2001 CATTCCTAAT AGCCTATGGG CAAACGTCAT AGATTTACGA GCTGTAAGTC
2051 AAGCGTCAGC AGCTGATGGC GAAGATGTCC CTGGGAAGCA ACTGAGCATC
2101 ACAGGAATTA CAAATTTCTT CCATGCGAAT CATACCGGTG ATGCACGCAG
2151 CTACCGCCAT ATGGGTGGAG GCTACCTCAT CAATACCTAC ACACGCATCA
2201 CTCCAGATGC TGCGTTAAGT CTAGGTTTTG GACAGCTGTT TACAAAATCT
2251 AAGGATTACC TCGTAGGTCA CGGTCATTCT AACGTTTATT TCGCTACAGT
2301 ATACTCTAAC ATCACCAAGT CTCTGTTTGG ATCATCGAGA TTCTTCTCAG
2351 GAGGCACTTC TCGAGTTACC TATAGCCGTA GCAATGAGAA AGTAAAGACT
2401 TCATATACAA AATTGCCTAA AGGGCGCTGC TCTTGGAGTA ACAATTGCTG
2451 GTTAGGAGAA CTCGAAGGGA ACCTTCCCAT CACTCTCTCT TCTCGCATCT
2501 TAAACCTCAA GCAGATCATT CCCTTTGTAA AAGCTGAAGT TGCTTACGCG
2551 ACTCATGGGG GCATCCAAGA AAATACCCCC GAGGGGAGGA TTTTTGGACA
2601 CGGTCATCTA CTCAACGTTG CAGTTCCCGT AGGCGTCCGC TTTGGTAAAA
2651 ATTCTCATAA TCGACCAGAT TTTTACACTA TAATCGTAGC CTATGCTCCT
2701 GATGTCTATC GTCACAATCC TGATTGCGAT ACGACATTAC CTATTAATGG
2751 AGCTACGTGG ACCTCTATAG GGAATAATCT AACCAGAAGT ACTTTGCTAG
2801 TACAAGCATC CAGCCATACT TCAGTAAATG ATGTTCTAGA GATCTTCGGG
2851 CACTGTGGAT GTGATATTCG CAGAACCTCC CGTCAATATA CTCTAGATAT
2901 AGGAAGCAAA TTACGATTTT AA

The PSORT algorithm predicts an outer membrane location (0.917).

The protein was expressed in *E.coli* and purified as a his-tag product, as shown in Figure 2A, and also as a GST-fusion. Both proteins were used to immunise mice, whose sera were used in a Western blot (Figure 2B) and for FACS analysis (Figure 2C).

The cp6736 protein was also identified in the 2D-PAGE experiment (Cpn0453) and showed good cross-reactivity with human sera, including sera from patients with pneumonitis.

These experiments show that cp6736 is a surface-exposed and immunoaccessible protein, and that it is a useful immunogen. These properties are not evident from the sequence alone.

**Example 3**

The following *C.pneumoniae* protein (PID 4376751) was expressed <SEQ ID 5; cp6751>:

```
  1 MRFFCFGMLL PFTFVLANEG LQLPLETYIT LSPEYQAAPQ VGFTHNQNQD
 51 LAIVGNHNDF ILDYKYYRSN GGALTCKNLL ISENIGNVFF EKNVCPNSGG
101 AIYAAQNCTI SKNQNYAFTT NLVSDNPTAT AGSLLGGALF AINCSITNNL
151 GQGTFVDNLA LNKGGALYTE TNLSIKDNKG PIIIKQNRAL NSDSLGGGIY
201 SGNSLNIEGN SGAIQITSNS SGSGGGIFST QTLTISSNKK LIEISENSAF
251 ANNYGSNFNP GGGGLTTTFC TILNNREGVL FNNNQSQSNG GAIHAKSIII
301 KENGPVYFLN NTATRGGALL NLSAGSGNGS FILSADNGDI IFNNNTASKH
351 ALNPPYRNAI HSTPNMNLQI GARPGYRVLF YDPIEHELPS SFPILFNFET
401 GHTGTVLFSG EHVHQNFTDE MNFFSYLRNT SELRQGVLAV EDGAGLACYK
451 FFQRGGTLLL GQGAVITTAG TIPTPSSTPT TVGSTITLNH IAIDLPSILS
501 FQAQAPKIWI YPTKTGSTYT EDSNPTITIS GTLTLRNSNN EDPYDSLDLS
551 HSLEKVPLLY IVDVAAQKIN SSQLDLSTLN SGEHYGYQGI WSTYWVETTT
601 ITNPTSLLGA NTKHKLLYAN WSPLGYRPHP ERRGEFITNA LWQSAYTALA
651 GLHSLSSWDE EKGHAASLQG IGLLVHQKDK NGFKGFRSHM TGYSATTEAT
701 SSQSPNFSLG FAQFFSKAKE HESQNSTSSH HYFSGMCIEN TLFKEWIRLS
751 VSLAYMFTSE HTHTMYQGLL EGNSQGSFHN HTLAGALSCV FLPQPHGESL
801 QIYPFITALA IRGNLAAFQE SGDHAREFSL HRPLTDVSLP VGIRASWKNH
851 HRVPLVWLTE ISYRSTLYRQ DPELHSKLLI SQGTWTTQAT PVTYNALGIK
901 VKNTMQVFPK VTLSLDYSAD ISSSTLSHYL NVASRMRF*
```

A predicted signal peptide is highlighted.

The cp6751 nucleotide sequence <SEQ ID 6> is:

```
   1 ATGCGCTTTT TTTGCTTCGG AATGTTGCTT CCTTTTACTT TTGTATTGGC
  51 TAATGAAGGT CTCCAACTTC CTTTGGAGAC CTATATTACA TTAAGTCCTG
 101 AATATCAAGC AGCCCCTCAA GTAGGGTTTA CTCATAACCA AAATCAAGAT
 151 CTCGCAATTG TCGGGAATCA CAATGATTTC ATCTTGGACT ATAAGTACTA
 201 TCGGTCGAAT GGAGGTGCTC TTACCTGTAA GAATCTTCTG ATCTCTGAAA
 251 ATATAGGGAA TGTCTTCTTT GAGAAGAATG TCTGTCCCAA TTCTGGCGGG
 301 GCAATTTATG CTGCTCAAAA TTGCACGATC TCCAAGAATC AGAACTATGC
 351 ATTTACTACA AACTTGGTCT CTGACAATCC TACAGCCACT GCGGGATCAC
 401 TATTGGGTGG AGCTCTCTTT GCCATAAATT GCTCTATTAC TAATAACCTA
 451 GGACAGGGAA CTTTCGTTGA CAATCTCGCT TTAAATAAGG GGGGTGCCCT
 501 CTATACTGAG ACGAACTTAT CTATTAAAGA CAATAAAGGC CCGATCATAA
 551 TCAAGCAGAA TCGGGCACTA AATTCGGACA GTTTAGGAGG AGGGATTTAT
 601 AGTGGGAACT CTCTAAATAT AGAGGGAAAT TCTGGAGCTA TACAGATCAC
 651 AAGCAACTCT TCAGGATCTG GGGGAGGCAT ATTTTCTACC CAAACACTCA
 701 CGATCTCCTC GAATAAAAAA CTCATAGAAA TCAGTGAAAA TTCCGCGTTC
 751 GCAAATAACT ATGGATCGAA CTTCAATCCA GGAGGAGGAG GTCTTACTAC
 801 CACCTTTTGC ACGATATTGA ACAACCGAGA AGGGGTACTC TTTAACAATA
 851 ACCAAAGCCA GAGCAACGGT GGAGCCATTC ATGCGAAATC TATCATTATC
 901 AAAGAAAATG GTCCTGTATA CTTTTTAAAT AACACTGCAA CTCGGGGAGG
 951 GGCTCTCCTC AACTTATCAG CAGGTTCTGG AAACGGAAGC TTCATCTTAT
1001 CTGCAGATAA TGGAGATATT ATCTTTAACA ATAATACGGC CTCCAAGCAT
1051 GCCCTCAATC CTCCATACAG AAACGCCATT CACTCGACTC CTAATATGAA
1101 TCTGCAAATA GGAGCCCGTC CCGGCTATCG AGTGCTGTTC TATGATCCCA
1151 TAGAACATGA GCTCCCTTCC TCCTTCCCCA TACTCTTTAA TTTCGAAACC
1201 GGTCATACAG GTACAGTTTT ATTTTCAGGG GAACATGTAC ACCAGAACTT
```

```
1251  TACCGATGAA ATGAATTTCT TTTCCTATTT AAGGAACACT TCGGAACTAC
1301  GTCAAGGAGT CCTTGCTGTT GAAGATGGTG CGGGGCTGGC CTGCTATAAG
1351  TTCTTCCAAC GAGGAGGCAC TCTACTTCTA GGTCAAGGTG CGGTGATCAC
1401  GACAGCAGGA ACGATTCCCA CACCATCCTC AACACCAACG ACAGTAGGAA
1451  GTACTATAAC TTTAAATCAC ATTGCCATTG ACCTTCCTTC TATTCTTTCT
1501  TTTCAAGCTC AGGCTCCAAA AATTTGGATT TACCCCACAA AAACAGGATC
1551  TACCTATACT GAAGATTCCA ACCCGACAAT CACAATCTCA GGAACTCTCA
1601  CCTTACGCAA CAGCAACAAC GAAGATCCCT ACGATAGTCT GGATCTCTCG
1651  CACTCTCTTG AGAAAGTTCC CCTTCTTTAT ATTGTCGATG TCGCTGCACA
1701  AAAAATTAAC TCTTCGCAAC TGGATCTATC CACATTAAAT TCTGGCGAAC
1751  ACTATGGGTA TCAAGGCATC TGGTCGACCT ATTGGGTAGA AACTACAACA
1801  ATCACGAACC CTACATCTCT ACTAGGCGCG AATACAAAAC ACAAGCTGCT
1851  CTATGCAAAC TGGTCTCCTC TAGGCTACCG TCCTCATCCC GAACGTCGAG
1901  GAGAATTCAT TACGAATGCC TTGTGGCAAT CGGCATATAC GGCTCTTGCA
1951  GGACTCCACT CCCTCTCCTC CTGGGATGAA GAGAAGGGTC ATGCAGCTTC
2001  CCTACAAGGC ATTGGTCTTC TGGTTCATCA AAAAGACAAA AACGGTTTTA
2051  AGGGATTTCG TAGTCATATG ACAGGTTATA GTGCTACCAC CGAAGCAACC
2101  TCTTCTCAAA GTCCGAATTT CTCTTTAGGA TTTGCTCAGT TCTTCTCCAA
2151  AGCTAAAGAA CATGAATCTC AAAATAGCAC GTCCTCTCAC CACTATTTCT
2201  CTGGAATGTG CATAGAAAAT ACTCTCTTCA AAGAGTGGAT ACGTCTATCT
2251  GTGTCTCTTG CTTATATGTT TACCTCGGAA CATACCCATA CAATGTATCA
2301  GGGTCTCCTG GAAGGGAACT CTCAGGGATC TTTCCACAAC CATACCTTAG
2351  CAGGGGCTCT CTCCTGTGTT TTCTTACCTC AACCTCACGG CGAGTCCCTG
2401  CAGATCTATC CCTTTATTAC TGCCTTAGCC ATCCGAGGAA ATCTTGCTGC
2451  GTTTCAAGAA TCTGGAGACC ATGCTCGGGA ATTTTCCCTA CACCGCCCCC
2501  TAACGGACGT CTCCCTCCCT GTAGGAATCC GCGCTTCTTG GAAGAACCAC
2551  CACCGAGTTC CCCTAGTCTG GCTCACAGAA ATTTCCTATC GCTCTACTCT
2601  CTATAGGCAA GATCCTGAAC TCCACTCGAA ATTACTGATT AGCCAAGGTA
2651  CGTGGACGAC GCAGGCCACT CCTGTGACCT ACAATGCTTT AGGGATCAAA
2701  GTGAAAAATA CCATGCAGGT GTTTCCTAAA GTCACTCTCT CCTTAGATTA
2751  CTCTGCGGAT ATTTCTTCCT CCACGCTGAG TCACTACTTA AACGTGGCGA
2801  GTAGAATGAG ATTTTAA
```

The PSORT algorithm predicts an outer membrane location (0.923).

The protein was expressed in *E.coli* and purified as a GST-fusion product, as shown in Figure 3A, and also in his-tagged form. The GST-fusion recombinant protein was used to immunise mice, whose sera were used in a Western blot (Figure 3B) and for FACS analysis (Figure 3C).

This protein also showed good cross-reactivity with human sera, including sera from patients with pneumonitis.

These experiments show that cp6751 is a surface-exposed and immunoaccessible protein, and that it is a useful immunogen. These properties are not evident from the sequence alone.

## Example 4

The following *C.pneumoniae* protein (PID 4376752) was expressed <SEQ ID 7; cp6752>:

```
  1  MFGMTPAVYS LQTDSLEKFA LERDEEFRTS FPLLDSLSTL TGFSPITTFV
 51  GNRHNSSQDI VLSNYKSIDN ILLLWTSAGG AVSCNNFLLS NVEDHAFFSK
101  NLAIGTGGAI ACQGACTITK NRGPLIFFSN RGLNNASTGG ETRGGAIACN
151  GDFTISQNQG TFYFVNNSVN NWGGALSTNG HCRIQSNRAP LLFFNNTAPS
201  GGGALRSENT TISDNTRPIY FKNNCGNNGG AIQTSVTVAI KNNSGSVIFN
251  NNTALSGSIN SGNGSGGAIY TTNLSIDDNP GTILFNNNYC IRDGGAICTQ
301  FLTIKNSGHV YFTNNQGNWG GALMLLQDST CLLFAEQGNI AFQNNEVFLT
351  TFGRYNAIHC TPNSNLQLGA NKGYTTAFFD PIEHQHPTTN PLIFNPNANH
401  QGTILFSSAY IPEASDYENN FISSSKNTSE LRNGVLSIED RAGWQFYKFT
451  QKGGILKLGH AASIATTANS ETPSTSVGSQ VIINNLAINL PSILAKGKAP
501  TLWIRPLQSS APFTEDNNPT ITLSGPLTLL NEENRDPYDS IDLSEPLQNI
551  HLLSLSDVTA RHINTDNFHP ESLNATEHYG YQGIWSPYWV ETITTTNNAS
601  IETANTLYRA LYANWTPLGY KVNPEYQGDL ATTPLWQSFH TMFSLLRSYN
651  RTGDSDIERP FLEIQGIADG LFVHQNSIPG APGFRIQSTG YSLQASSETS
```

```
701  LHQKISLGFA QFFTRTKEIG SSNNVSAHNT VSSLYVELPW FQEAFATSTV
751  LAYGYGDHHL HSLHPSHQEQ AEGTCYSHTL AAAIGCSFPW QQKSYLHLSP
801  FVQAIAIRSH QTAFEEIGDN PRKFVSQKPF YNLTLPLGIQ GKWQSKFHVP
851  TEWTLELSYQ PVLYQQNPQI GVTLLASGGS WDILGHNYVR NALGYKVHNQ
901  TALFRSLDLF LDYQGSVSSS TSTHHLQAGS TLKF*
```

The cp6752 nucleotide sequence <SEQ ID 8> is:

```
   1 ATGTTCGGGA TGACTCCTGC AGTGTATAGT TTACAAACGG ACTCCCTTGA
  51 AAAGTTTGCT TTAGAGAGGG ATGAAGAGTT TCGTACGAGC TTTCCTCTCT
 101 TAGACTCTCT CTCCACTCTT ACAGGATTTT CTCCAATAAC TACGTTTGTT
 151 GGAAATAGAC ATAATTCCTC TCAAGACATT GTACTTTCTA ACTACAAGTC
 201 TATTGATAAC ATCCTTCTTC TTTGGACATC GGCTGGGGGA GCTGTGTCCT
 251 GTAATAATTT CTTATTATCA AATGTTGAAG ACCATGCCTT CTTCAGTAAA
 301 AATCTCGCGA TTGGGACTGG AGGCGCGATT GCTTGCCAGG GAGCCTGCAC
 351 AATCACGAAG AATAGAGGAC CCCTTATTTT TTTCAGCAAT CGAGGTCTTA
 401 ACAATGCGAG TACAGGAGGA GAAACTCGTG GGGGTGCGAT TGCCTGTAAT
 451 GGAGACTTCA CGATTTCTCA AAATCAAGGG ACTTTCTACT TTGTCAACAA
 501 TTCCGTCAAC AACTGGGGAG GAGCCCTCTC CACCAATGGA CACTGCCGCA
 551 TCCAAAGCAA CAGGGCACCT CTACTCTTTT TTAACAATAC AGCCCCTAGT
 601 GGAGGGGGTG CGCTTCGTAG TGAAAATACA ACGATCTCTG ATAACACGCG
 651 TCCTATTTAT TTTAAGAACA ACTGTGGGAA CAATGGCGGG GCCATTCAAA
 701 CAAGCGTTAC TGTTGCGATA AAAAATAACT CCGGGTCGGT GATTTTCAAT
 751 AACAACACAG CGTTATCTGG TTCGATAAAT TCAGGAAATG GTTCAGGAGG
 801 GGCGATTTAT ACAACAAACC TATCCATAGA CGATAACCCT GGAACTATTC
 851 TTTTCAATAA TAACTACTGC ATTCGCGATG GCGGAGCTAT CTGTACACAA
 901 TTTTTGACAA TCAAAAATAG TGGCCACGTA TATTTCACCA ACAATCAAGG
 951 AAACTGGGGA GGTGCTCTTA TGCTCCTACA GGACAGCACC TGCCTACTCT
1001 TCGCGGAACA AGGAAATATC GCATTTCAAA ATAATGAGGT TTTCCTCACC
1051 ACATTTGGTA GATACAACGC CATACATTGT ACACCAAATA GCAACTTACA
1101 ACTTGGAGCT AATAAGGGGT ATACGACTGC TTTTTTTGAT CCTATAGAAC
1151 ACCAACATCC AACTACAAAT CCTCTAATCT TTAATCCCAA TGCGAACCAT
1201 CAGGGAACGA TCTTATTTTC TTCAGCCTAT ATCCCAGAAG CTTCTGACTA
1251 CGAAAATAAT TTCATTAGCA GCTCGAAAAA TACCTCTGAA CTTCGCAATG
1301 GTGTCCTCTC TATCGAGGAT CGTGCGGGAT GGCAATTCTA TAAGTTCACT
1351 CAAAAAGGAG GTATCCTTAA ATTAGGGCAT GCGGCGAGTA TTGCAACAAC
1401 TGCCAACTCT GAGACTCCAT CAACTAGTGT AGGCTCCCAG GTCATCATTA
1451 ATAACCTTGC GATTAACCTC CCCTCGATCT TAGCAAAAGG AAAAGCTCCT
1501 ACCTTGTGGA TCCGTCCTCT ACAATCTAGT GCTCCTTTCA CAGAGGACAA
1551 TAACCCTACA ATTACTTTAT CAGGTCCTCT GACACTCTTA AATGAGGAAA
1601 ACCGCGATCC CTACGACAGT ATAGATCTCT CTGAGCCTTT ACAAAACATT
1651 CATCTTCTTT CTTTATCGGA TGTAACAGCA CGTCATATCA ATACCGATAA
1701 CTTTCATCCT GAAAGCTTAA ATGCGACTGA GCATTACGGT TATCAAGGCA
1751 TCTGGTCTCC TTATTGGGTA GAGACGATAA CAACAACAAA TAACGCTTCT
1801 ATAGAGACGG CAAACACCCT CTACAGAGCT CTGTATGCCA ATTGGACTCC
1851 CTTAGGATAT AAGGTCAATC CTGAATACCA AGGAGATCTT GCTACGACTC
1901 CCCTATGGCA ATCCTTTCAT ACTATGTTCT CTCTATTAAG AAGTTATAAT
1951 CGAACTGGTG ATTCTGATAT CGAGAGGCCT TTCTTAGAAA TTCAAGGGAT
2001 TGCCGACGGC CTCTTTGTTC ATCAAAATAG CATCCCCGGG GCTCCAGGAT
2051 TCCGTATCCA ATCTACAGGG TATTCCTTAC AAGCATCCTC CGAAACTTCT
2101 TTACATCAGA AATCTCCTT AGGTTTTGCA CAGTTCTTCA CCCGCACTAA
2151 AGAAATCGGA TCAAGCAACA ACGTCTCGGC TCACAATACA GTCTCTTCAC
2201 TTTATGTTGA GCTTCCGTGG TTCCAAGAGG CCTTTGCAAC ATCCACAGTG
2251 TTAGCGTATG GCTATGGGGA CCATCACCTC CACAGCCTAC ATCCCTCACA
2301 TCAAGAACAG GCAGAAGGGA CGTGTTATAG CCATACATTA GCAGCAGCTA
2351 TCGGCTGTTC TTTCCCTTGG CAACAGAAAT CCTATCTTCA CCTCAGCCCG
2401 TTCGTTCAGG CAATTGCAAT ACGTTCTCAC CAAACAGCGT TCGAAGAGAT
2451 TGGTGACAAT CCCCGAAAGT TTGTCTCTCA AAAGCCTTTC TATAATCTGA
2501 CCTTACCTCT AGGAATCCAA GGAAAATGGC AGTCAAAATT CCACGTACCT
2551 ACAGAATGGA CTCTAGAACT TTCTTACCAA CCGGTACTCT ATCAACAAAA
2601 TCCCCAAATC GGTGTCACGC TACTTGCGAG CGGAGGTTCC TGGGATATCC
2651 TAGGCCATAA CTATGTTCGC AATGCTTTAG GGTACAAAGT CCACAATCAA
2701 ACTGCGCTCT TCCGTTCTCT CGATCTATTC TTGGATTACC AAGGATCGGT
2751 CTCCTCCTCG ACATCTACGC ACCATCTCCA AGCAGGAAGT ACCTTAAAAT
2801 TCTAA
```

The PSORT algorithm predicts a cytoplasmic location (0.138).

The protein was expressed in *E.coli* and purified as a his-tag product, as shown in Figure 4A, and also as a GST-fusion. The recombinant proteins were used to immunise mice, whose sera were used in a Western blot (4B) and the his-tagged protein was used for FACS analysis (4C).

The cp6752 protein was also identified in the 2D-PAGE experiment (Cpn0467).

These experiments show that cp6752 is a surface-exposed and immunoaccessible protein, and that it is a useful immunogen. These properties are not evident from the sequence alone.

**Example 5**

The following *C.pneumoniae* protein (PID 4376850) was expressed <SEQ ID 9; cp6850>:

```
  1  MKKAVLIAAM FCGVVSLSSC CRIVDCCFED PCAPSSCNPC EVIRKKERSC
 51  GGNACGSYVP SCSNPCGSTE CNSQSPQVKG CTSPDGRCKQ *
```

A predicted signal peptide is highlighted.

The cp6850 nucleotide sequence <SEQ ID 10> is:

```
  1  ATGAAGAAAG CTGTTTTAAT TGCTGCAATG TTTTGTGGAG TAGTTAGCTT
 51  AAGTAGCTGC TGCCGCATTG TAGATTGTTG TTTTGAGGAT CCTTGCGCAC
101  CCTCTTCTTG CAATCCTTGT GAAGTAATAA GAAAAAAAGA AAGATCTTGC
151  GGCGGTAATG CTTGTGGGTC CTACGTTCCT TCTTGTTCTA ATCCATGTGG
201  TTCAACAGAG TGTAACTCTC AAAGCCCACA AGTTAAAGGT TGTACATCAC
251  CTGATGGCAG ATGCAAACAG TAA
```

The PSORT algorithm predicts an inner membrane location (0.329).

The protein was expressed in *E.coli* and purified as a GST-fusion product, as shown in Figure 5A. The recombinant protein was used to immunise mice, whose sera were used in a Western blot (Figure 5B) and for FACS analysis (Figure 5B). A his-tagged protein was also expressed.

These experiments show that cp6850 is a surface-exposed and immunoaccessible protein, and that it is a useful immunogen. These properties are not evident from the sequence alone.

**Example 6**

The following *C.pneumoniae* protein (PID 4376900) was expressed <SEQ ID 11; cp6900>:

```
  1  MKIKFSWKVN FLICLLAVGL IFFGCSRVKR EVLVGRDATW FPKQFGIYTS
 51  DTNAFLNDLV SEINYKENLN INIVNQDWVH LFENLDDKKT QGAFTSVLPT
101  LEMLEHYQFS DPILLTGPVL VVAQDSPYQS IEDLKGRLIG VYKFDSSVLV
151  AQNIPDAVIS LYQHVPIALE ALTSNCYDAL LAPVIEVTAL IETAYKGRLK
201  IISKPLNADG LRLAILKGTN GDLLEGFNAG LVKTRRSGKY DAIKQRYRLP
```

The cp6900 nucleotide sequence <SEQ ID 12> is:

```
  1  GTGAAGATAA AATTTTCTTG GAAGGTAAAT TTTTTAATAT GTTTACTGGC
 51  TGTGGGACTG ATCTTTTTCG GGTGCTCTCG AGTAAAAAGA GAAGTTCTCG
101  TAGGTCGTGA TGCCACCTGG TTTCCAAAAC AATTCGGCAT TTATACATCC
151  GATACCAACG CATTTTTAAA CGATCTTGTT TCTGAGATTA ACTATAAAGA
201  GAATCTAAAT ATTAATATTG TAAATCAAGA TTGGGTGCAT CTCTTTGAGA
251  ATTTAGATGA TAAAAAGACC CAAGGAGCAT TTACATCTGT ATTGCCTACT
301  CTTGAGATGC TCGAACACTA TCAATTTTCT GATCCCATTT TACTCACAGG
351  TCCTGTCCTT GTCGTCGCTC AAGACTCTCC TTACCAATCT ATAGAGGATC
401  TTAAAGGTCG TCTTATTGGA GTGTATAAGT TTGACTCTTC AGTTCTTGTA
451  GCTCAAAATA TCCCTGACGC TGTGATTAGC CTCTACCAAC ATGTTCCAAT
501  AGCATTGGAA GCCTTAACAT CGAATTGTTA CGACGCTCTT CTAGCTCCTG
551  TAATTGAAGT GACCGCGCTA ATAGAAACAG CATATAAAGG AAGACTGAAA
601  ATTATTTCAA AACCCTTAAA CGCAGATGGT TTGCGGCTTG CAATACTGAA
```

```
651   AGGGACAAAC GGAGATTTGC TTGAAGGGTT TAACGCAGGA CTTGTGAAAA
701   CACGACGCTC AGGAAAATAC GATGCTATAA AACAGCGGTA TCGTCTTCCC
751   TAA
```

The PSORT algorithm predicts an inner membrane location (0.452).

The protein was expressed in *E.coli* and purified as a GST-fusion product, as shown in Figure 6A. The recombinant protein was used to immunise mice, whose sera were used for FACS analysis (Figure 6B). A his-tagged protein was also expressed.

The cp6900 protein was also identified in the 2D-PAGE experiment (Cpn0604).

These experiments show that cp6900 is a surface-exposed and immunoaccessible protein, and that it is a useful immunogen. These properties are not evident from the sequence alone.

**Example 7**

The following *C.pneumoniae* protein (PID 4377033) was expressed <SEQ ID 13; cp7033>:

```
  1   MVNPIGPGPI DETERTPPAD LSAQGLEASA ANKSAEAQRI AGAEAKPKES
 51   KTDSVERWSI LRSAVNALMS LADKLGIASS NSSSSTSRSA DVDSTTATAP
101   TPPPPTFDDY KTQAQTAYDT IFTSTSLADI QAALVSLQDA VTNIKDTAAT
151   DEETAIAAEW ETKNADAVKV GAQITELAKY ASDNQAILDS LGKLTSFDLL
201   QAALLQSVAN NNKAAELLKE MQDNPVVPGK TPAIAQSLVD QTDATATQIE
251   KDGNAIRDAY FAGQNASGAV ENAKSNNSIS NIDSAKAAIA TAKTQIAEAQ
301   KKFPDSPILQ EAEQMVIQAE KDLKNIKPAD GSDVPNPGTT VGGSKQQGSS
351   IGSIRVSMLL DDAENETASI LMSGFRQMIH MFNTENPDSQ AAQQELAAQA
401   RAAKAAGDDS AAAALADAQK ALEAALGKAG QQQGILNALG QIASAAVVSA
451   GVPPAAASSI GSSVKQLYKT SKSTGSDYKT QISAGYDAYK SINDAYGRAR
501   NDATRDVINN VSTPALTRSV PRARTEARGP EKTDQALARV ISGNSRTLGD
551   VYSQVSALQS VMQIIQSNPQ ANNEEIRQKL TSAVTKPPQF GYPYVQLSND
601   STQKFIAKLE SLFAEGSRTA AEIKALSFET NSLFIQQVLV NIGSLYSGYL
651   Q*
```

The cp7033 nucleotide sequence <SEQ ID 14> is:

```
   1 ATGGTTAATC CTATTGGTCC AGGTCCTATA GACGAAACAG AACGCACACC
  51 TCCCGCAGAT CTTTCTGCTC AAGGATTGGA GGCGAGTGCA GCAAATAAGA
 101 GTGCGGAAGC TCAAAGAATA GCAGGTGCGG AAGCTAAGCC TAAAGAATCT
 151 AAGACCGATT CTGTAGAGCG ATGGAGCATC TTGCGTTCTG CAGTGAATGC
 201 TCTCATGAGT CTGGCAGATA AGCTGGGTAT TGCTTCTAGT AACAGCTCGT
 251 CTTCTACTAG CAGATCTGCA GACGTGGACT CAACGACAGC GACCGCACCT
 301 ACGCCTCCTC CACCCACGTT TGATGATTAT AAGACTCAAG CGCAAACAGC
 351 TTACGATACT ATCTTTACCT CAACATCACT AGCTGACATA CAGGCTGCTT
 401 TGGTGAGCCT CCAGGATGCT GTCACTAATA TAAAGGATAC AGCGGCTACT
 451 GATGAGGAAA CCGCAATCGC TGCGGAGTGG GAAACTAAGA ATGCCGATGC
 501 AGTTAAAGTT GGCGCGCAAA TTACAGAATT AGCGAAATAT GCTTCGGATA
 551 ACCAAGCGAT TCTTGACTCT TTAGGTAAAC TGACTTCCTT CGACCTCTTA
 601 CAGGCTGCTC TTCTCCAATC TGTAGCAAAC AATAACAAAG CAGCTGAGCT
 651 TCTTAAAGAG ATGCAAGATA ACCCAGTAGT CCCAGGGAAA ACGCCTGCAA
 701 TTGCTCAATC TTTAGTTGAT CAGACAGATG CTACAGCGAC ACAGATAGAG
 751 AAAGATGGAA ATGCGATTAG GGATGCATAT TTTGCAGGAC AGAACGCTAG
 801 TGGAGCTGTA GAAAATGCTA AATCTAATAA CAGTATAAGC AACATAGATT
 851 CAGCTAAAGC AGCAATCGCT ACTGCTAAGA CACAAATAGC TGAAGCTCAG
 901 AAAAAGTTCC CCGACTCTCC AATTCTTCAA GAAGCGGAAC AAATGGTAAT
 951 ACAGGCTGAG AAAGATCTTA AAAATATCAA ACCTGCAGAT GGTTCTGATG
1001 TTCCAAATCC AGGAACTACA GTTGGAGGCT CCAAGCAACA AGGAAGTAGT
1051 ATTGGTAGTA TTCGTGTTTC CATGCTGTTA GATGATGCTG AAAATGAGAC
1101 CGCTTCCATT TTGATGTCTG GGTTTCGTCA GATGATTCAC ATGTTCAATA
1151 CGGAAAATCC TGATTCTCAA GCTGCCCAAC AGGAGCTCGC AGCACAAGCT
1201 AGAGCAGCGA AAGCCGCTGG AGATGACAGT GCTGCTGCAG CGCTGGCAGA
1251 TGCTCAGAAA GCTTTAGAAG CGGCTCTAGG TAAAGCTGGG CAACAACAGG
1301 GCATACTCAA TGCTTTAGGA CAGATCGCTT CTGCTGCTGT TGTGAGCGCA
1351 GGAGTTCCTC CCGCTGCAGC AAGTTCTATA GGGTCATCTG TAAAACAGCT
1401 TTACAAGACC TCAAAATCTA CAGGTTCTGA TTATAAAACA CAGATATCAG
```

```
1451 CAGGTTATGA TGCTTACAAA TCCATCAATG ATGCCTATGG TAGGGCACGA
1501 AATGATGCGA CTCGTGATGT GATAAACAAT GTAAGTACCC CCGCTCTCAC
1551 ACGATCCGTT CCTAGAGCAC GAACAGAAGC TCGAGGACCA GAAAAAACAG
1601 ATCAAGCCCT CGCTAGGGTG ATTTCTGGCA ATAGCAGAAC TCTTGGAGAT
1651 GTCTATAGTC AAGTTTCGGC ACTACAATCT GTAATGCAGA TCATCCAGTC
1701 GAATCCTCAA GCGAATAATG AGGAGATCAG ACAAAAGCTT ACATCGGCAG
1751 TGACAAAGCC TCCACAGTTT GGCTATCCTT ATGTGCAACT TTCTAATGAC
1801 TCTACACAGA AGTTCATAGC TAAATTAGAA AGTTTGTTTG CTGAAGGATC
1851 TAGGACAGCA GCTGAAATAA AAGCACTTTC CTTTGAAACG AACTCCTTGT
1901 TTATTCAGCA GGTGCTGGTC AATATCGGCT CTCTATATTC TGGTTATCTC
1951 CAATAA
```

The PSORT algorithm predicts a cytoplasmic location (0.272).

The protein was expressed in *E.coli* and purified as a GST-fusion product, as shown in Figure 7A. A his-tagged protein was also expressed. The recombinant proteins were used to immunise mice, whose sera were used for FACS (Figure 7B) and Western blot (7C) analyses.

The cp7033 protein was also identified in the 2D-PAGE experiment (Cpn0728) and showed good cross-reactivity with human sera, including sera from patients with pneumonitis.

These experiments show that cp7033 a surface-exposed and immunoaccessible protein, and that it is a useful immunogen. These properties are not evident from the sequence alone.

## Example 8

The following *C.pneumoniae* protein (PID 6172321) was expressed <SEQ ID 15; cp0017>:

```
  1  MGIKGTGIIV WVDDATAKTK NATLTWTKTG YKPNPERQGP LVPNSLWGSF
 51  VDVRSIQSLM DRSTSSLSSS TNLWVSGIAD FLHEDQKGNQ RSYRHSSAGY
101  ALGGGFFTAS ENFFNFAFCQ LFGYDKDHLV AKNHTHVYAG AMSYRHLGES
151  KTLAKILSGN SDSLPFVFNA RFAYGHTDNN MTTKYTGYSP VKGSWGNDAF
201  GIECGGAIPV VASGRRSWVD THTPFLNLEM IYAHQNDFKE NGTEGRSFQS
251  EDLFNLAVPV GIKFEKFSDK STYDLSIAYV PDVIRNDPGC TTTLMVSGDS
301  WSTCGTSLSR QALLVRAGNH HAFASNFEVF SQFEVELRGS SRSYAIDLGG
351  RFGF*
```

The cp0017 nucleotide sequence <SEQ ID 16> is:

```
   1  ATGGGTATCA AGGGAACTGG AATAATTGTT TGGGTCGACG ATGCAACTGC
  51  AAAAACAAAA AATGCTACCT TAACTTGGAC TAAAACAGGA TACAAGCCGA
 101  ATCCAGAACG TCAGGGACCT TTGGTTCCTA ATAGCCTGTG GGGTTCTTTT
 151  GTCGATGTCC GCTCCATTCA GAGCCTCATG GACCGGAGCA CAAGTTCGTT
 201  ATCTTCGTCA ACAAATTTGT GGGTATCAGG AATCGCGGAC TTTTTGCATG
 251  AAGATCAGAA AGGAAACCAA CGTAGTTATC GTCATTCTAG CGCGGGTTAT
 301  GCATTAGGAG GAGGATTCTT CACGGCTTCT GAAAATTTCT TTAATTTTGC
 351  TTTTTGTCAG CTTTTTGGCT ACGACAAGGA CCATCTTGTG GCTAAGAACC
 401  ATACCCATGT ATATGCAGGG GCAATGAGTT ACCGACACCT CGGAGAGTCT
 451  AAGACCCTCG CTAAGATTTT GTCAGGAAAT TCTGACTCCC TACCTTTTGT
 501  CTTCAATGCT CGGTTTGCTT ATGGCCATAC CGACAATAAC ATGACCACAA
 551  AGTACACTGG CTATTCTCCT GTTAAGGGAA GCTGGGGAAA TGATGCCTTC
 601  GGTATAGAAT GTGGAGGAGC TATCCCGGTA GTTGCTTCAG GACGTCGGTC
 651  TTGGGTGGAT ACCCACACGC CATTTCTAAA CCTAGAGATG ATCTATGCAC
 701  ATCAGAATGA CTTTAAGGAA AACGGCACAG AAGGCCGTTC TTTCCAAAGT
 751  GAAGACCTCT TCAATCTAGC GGTTCCTGTA GGGATAAAAT TTGAGAAATT
 801  CTCCGATAAG TCTACGTATG ATCTCTCCAT AGCTTACGTT CCCGATGTGA
 851  TTCGTAATGA TCCAGGCTGC ACGACAACTC TTATGGTTTC TGGGGATTCT
 901  TGGTCGACAT GTGGTACAAG CTTGTCTAGA CAAGCTCTTC TTGTACGTGC
 951  TGGAAATCAT CATGCCTTTG CTTCAAACTT TGAAGTTTTC AGTCAGTTTG
1001  AAGTCGAGTT GCGAGGTTCT TCTCGTAGCT ATGCTATCGA TCTTGGAGGA
1051  AGATTCGGAT TTTAA
```

This sequence is frame-shifted with respect to cp0016.

The PSORT algorithm predicts a cytoplasmic location (0.075).

The protein was expressed in *E.coli* and purified as a GST-fusion product, as shown in Figure 8A. The recombinant protein was used to immunise mice, whose sera were used in a Western blot (Figure 8B) and for FACS analysis (Figure 8C). A his-tagged protein was also expressed.

This protein also showed good cross-reactivity with human sera, including sera from patients with pneumonitis.

These experiments show that cp0017 is a surface-exposed and immunoaccessible protein, and that it is a useful immunogen. These properties are not evident from the sequence alone.

**Example 9**

The following *C.pneumoniae* protein (PID 6172315) was expressed <SEQ ID 17; cp0014>:

```
  1  MKSSFPKFVF STFAIFPLSM IATETVLDSS ASFDGNKNGN FSVRESQEDA
 51  GTTYLFKGNV TLENIPGTGT AITKSCFNNT KGDLTFTGNG NSLLFQTVDA
101  GTVAGAAVNS SVVDKSTTFI GFSSLSFIAS PGSSITTGKG AVSCSTGSLS
151  LTKMSVCSSA KTFQRIMAVL SPQKLFH*
```

The cp0014 nucleotide sequence <SEQ ID 18> is:

```
  1 ATGAAGTCTT CTTTCCCCAA GTTTGTATTT TCTACATTTG CTATTTTCCC
 51 TTTGTCTATG ATTGCTACCG AGACAGTTTT GGATTCAAGT GCGAGTTTCG
101 ATGGGAATAA AAATGGTAAT TTTTCAGTTC GTGAGAGTCA GGAAGATGCT
151 GGAACTACCT ACCTATTTAA GGGAAATGTC ACTCTAGAAA ATATTCCTGG
201 AACAGGCACA GCAATCACAA AAAGCTGTTT TAACAACACT AAGGGCGATT
251 TGACTTTCAC AGGTAACGGG AACTCTCTAT TGTTCCAAAC GGTGGATGCA
301 GGGACTGTAG CAGGGGCTGC TGTTAACAGC AGCGTGGTAG ATAAATCTAC
351 CACGTTTATA GGGTTTTCTT CGCTATCTTT TATTGCGTCT CCTGGAAGTT
401 CGATAACTAC CGGCAAAGGA GCCGTTAGCT GCTCTACGGG TAGCTTGAGT
451 TTGACAAAAA TGTCAGTTTG CTCTTCAGCA AAAACTTTTC AACGGATAAT
501 GGCGGTGCTA TCACCGCAAA AACTCTTTCA TTAA
```

This protein is frame-shifted with respect to cp0015.

The PSORT algorithm predicts an inner membrane location (0.047).

The protein was expressed in *E.coli* and purified as a his-tag product, as shown in Figure 9A. A GST-fusion was also expressed. The recombinant proteins were used to immunise mice, whose sera were used in an immunoassay (Figure 9B) and for FACS analysis (Figure 9C).

This protein also showed good cross-reactivity with human sera, including sera from patients with pneumonitis.

These experiments suggest that cp0014 is a useful immunogen. These properties are not evident from the sequence alone.

### Example 10

The following *C.pneumoniae* protein (PID 6172317) was expressed <SEQ ID 19; cp0015>:

```
  1 MSALFSENTS SKKGGAIQTS DALTITGNQG EVSFSDNTSS DSGAAIFTEA
 51 SVTISNNAKV SFIDNKVTGA SSSTTGDMSG GAICAYKTST DTKVTLTGNQ
101 MLLFSNNTST TAGGAIYVKK LELASGGLTL FSRNSVNGGT APKGGAIAIE
151 DSGELSLSAD SGDIVFLGNT VTSTTPGTNR SSIDLGTSAK MTALRSAAGR

201 AIYFYDPITT GSSTTVTDVL KVNETPADSA LQYTGNIIFT GEKLSETEAA
251 DSKNLTSKLL QPVTLSGGTL SLKHGVTLQT QAFTQQADSR LEMDVGTTLE
301 PADTSTINNL VINISSIDGA KKAKIETKAT SKNLTLSGTI TLLDPTGTFY
351 ENHSLRNPQS YDILELKASG TVTSTAVTPD PIMGEKFHYG YQGTWGPIVW
401 GTGASTTATF NWTKTGYIPN PERIGSLVPN SLWNAFIDIS SLHYLMETAN
451 EGLQGDRAFW CAGLSNFFHK DSTKTRRGFR HLSGGYVIGG NLHTCSDKIL
501 SAAFCQLFGR DRDYFVAKNQ GTVYGGTLYY QHNETYISLP CKLRPCSLSY
551 VPTEIPVLFS GNLSYTHTDN DLKTKYTTYP TVKGSWGNDS FALEFGGRAP
601 ICLDESALFE QYMPFMKLQF VYAHQEGFKE QGTEAREFGS SRLVNLALPI
651 GIRFDKESDC QDATYNLTLG YTVDLVRSNP DCTTTLRISG DSWKTFGTNL
701 ARQALVLRAG NHFCFNSNFE AFSQFSFELR GSSRNYNVDL GAKYQF*
```

This sequence is frame-shifted with respect to cp0014.

The cp0015 nucleotide sequence <SEQ ID 20> is:

```
   1 ATGTCAGCTC TGTTTTCTGA AAATACCTCC TCAAAGAAAG GCGGAGCCAT
  51 TCAGACTTCC GATGCCCTTA CCATTACTGG AAACCAAGGG GAAGTCTCTT
 101 TTTCTGACAA TACTTCTTCG GATTCTGGAG CTGCAATTTT TACAGAAGCC
 151 TCGGTGACTA TTTCTAATAA TGCTAAAGTT TCCTTTATTG ACAATAAGGT
 201 CACAGGAGCG AGCTCCTCAA CAACGGGGGA TATGTCAGGA GGTGCTATCT
 251 GTGCTTATAA AACTAGTACA GATACTAAGG TCACCCTCAC TGGAAATCAG
 301 ATGTTACTCT TCAGCAACAA TACATCGACA ACAGCGGGAG GAGCTATCTA
 351 TGTGAAAAAG CTCGAACTGG CTTCCGGAGG ACTTACCCTA TTCAGTAGAA
 401 ATAGTGTCAA TGGAGGTACA GCTCCTAAAG GTGGAGCCAT AGCTATCGAA
 451 GATAGTGGGG AATTGAGTTT ATCCGCCGAT AGTGGTGACA TTGTCTTTTT
 501 AGGGAATACA GTCACTTCTA CTACTCCTGG GACGAATAGA AGTAGTATCG
 551 ACTTAGGAAC GAGTGCAAAG ATGACAGCTT TGCGTTCTGC TGCTGGTAGA
 601 GCCATCTACT TCTATGATCC CATAACTACA GGATCATCCA CAACAGTTAC
 651 AGATGTCTTA AAAGTTAATG AGACTCCGGC AGATTCTGCA CTACAATATA
 701 CAGGGAACAT CATCTTCACA GGAGAAAAGT TATCAGAGAC AGAGGCCGCA
 751 GATTCTAAAA ATCTTACTTC GAAGCTACTA CAGCCTGTAA CTCTTTCAGG
 801 AGGTACTCTA TCTTTAAAAC ATGGAGTGAC TCTGCAGACT CAGGCATTCA
 851 CTCAACAGGC AGATTCTCGT CTCGAAATGG ACGTAGGAAC TACTCTAGAA
 901 CCTGCTGATA CTAGCACCAT AAACAATTTG GTCATTAACA TCAGTTCTAT
 951 AGACGGTGCA AAGAAGGCAA AAATAGAAAC CAAAGCTACG TCAAAAAATC
1001 TGACTTTATC TGGAACCATC ACTTTATTGG ACCCGACGGG CACGTTTTAT
1051 GAAAATCATA GTTTAAGAAA TCCTCAGTCC TACGACATCT TAGAGCTCAA
1101 AGCTTCTGGA ACTGTAACAA GCACCGCAGT GACTCCAGAT CCTATAATGG
1151 GTGAGAAATT CCATTACGGC TATCAGGGAA CTTGGGGCCC AATTGTTTGG
1201 GGGACAGGGG CTTCTACGAC TGCAACCTTC AACTGGACTA AAACTGGCTA
1251 TATTCCTAAT CCCGAGCGTA TCGGCTCTTT AGTCCCTAAT AGCTTATGGA
1301 ATGCATTTAT AGATATTAGC TCTCTCCATT ATCTTATGGA GACTGCAAAC
1351 GAAGGGTTGC AGGGAGACCG TGCTTTTTGG TGTGCTGGAT TATCTAACTT
1401 CTTCCATAAG GATAGTACAA AAACACGACG CGGGTTTCGC CATTTGAGTG
1451 GCGGTTATGT CATAGGAGGA AACCTACATA CTTGTTCAGA TAAGATTCTT
1501 AGTGCTGCAT TTTGTCAGCT CTTTGGAAGA GATAGAGACT ACTTTGTAGC
1551 TAAGAATCAA GGTACAGTCT ACGGAGGAAC TCTCTATTAC CAGCACAACG
1601 AAACCTATAT CTCTCTTCCT TGCAAACTAC GGCCTTGTTC GTTGTCTTAT
1651 GTTCCTACAG AGATTCCTGT TCTCTTTTCA GGAAACCTTA GCTACACCCA
1701 TACGGATAAC GATCTGAAAA CCAAGTATAC AACATATCCT ACTGTTAAAG
1751 GAAGCTGGGG GAATGATAGT TTCGCTTTAG AATTCGGTGG AAGAGCTCCG
1801 ATTTGCTTAG ATGAAAGTGC TCTATTTGAG CAGTACATGC CCTTCATGAA
1851 ATTGCAGTTT GTCTATGCAC ATCAGGAAGG TTTTAAAGAA CAGGGAACAG
1901 AAGCTCGTGA ATTTGGAAGT AGCCGTCTTG TGAATCTTGC CTTACCTATC
1951 GGGATCCGAT TTGATAAGGA ATCAGACTGC CAAGATGCAA CGTACAATCT
2001 AACTCTTGGT TATACTGTGG ATCTTGTTCG TAGTAACCCC GACTGTACGA
2051 CAACACTGCG AATTAGCGGT GATTCTTGGA AAACCTTCGG TACGAATTTG
2101 GCAAGACAAG CTTTAGTCCT TCGTGCAGGG AACCATTTTT GCTTTAACTC
2151 AAATTTTGAA GCCTTTAGCC AATTTTCTTT TGAATTGCGT GGGTCATCTC
2201 GCAATTACAA TGTAGACTTA GGAGCAAAAT ACCAATTCTA A
```

The PSORT algorithm predicts a cytoplasmic location (0.274).

The protein was expressed in *E.coli* and purified as a GST-fusion product, as shown in Figure 10A. The recombinant protein was used to immunise mice, whose sera were used in a Western blot (Figure 10B) and for FACS analysis. A his-tagged protein was also expressed.

These experiments show that cp0015 is a useful immunogen. These properties are not evident from the sequence alone.

## Example 11

The following *C.pneumoniae* protein (PID 6172325) was expressed <SEQ ID 21; cp0019>:

```
  1  LQDSQDYSFV KLSPGAGGTI ITQDASQKPL EVAPSRPHYG YQGHWNVQVI
 51  PGTGTQPSQA NLEWVRTGYL PNPERQGSLV PNSLWGSFVD QRAIQEIMVN
101  SSQILCQERG VWGAGIANFL HRDKINEHGY RHSGVGYLVG VGTHAFSDAT
151  INAAFCQLFS RDKDYVVSKN HGTSYSGVVF LEDTLEFRSP QGFYTDSSSE
201  ACCNQVVTID MQLSYSHRNN DMKTKYTTYP EAQGSWANDV FGLEFGATTY
251  YYPNSTFLFD YYSPFLRLQC TYAHQEDFKE TGGEVRHFTS GDLFNLAVPI
301  GVKFERFSDC KRGSYELTLA YVPDVIRKDP KSTATLASGA TWSTHGNNLS
351  RQGLQLRLGN HCLINPGIEV FSHGAIELRG SSRNYNINLG GKYRF*
```

This sequence is frame-shifted with respect to cp0018.

The cp0019 nucleotide sequence <SEQ ID 22> is:

```
   1  TTGCAAGACT CTCAAGACTA TAGCTTTGTA AAGTTATCTC CAGGAGCGGG
  51  AGGGACTATA ATTACTCAAG ATGCTTCTCA GAAGCCTCTT GAAGTAGCTC
 101  CTTCTAGACC ACATTATGGC TATCAAGGAC ATTGGAATGT GCAAGTCATC
 151  CCAGGAACGG GAACTCAACC GAGCCAGGCA AATTTAGAAT GGGTGCGGAC
 201  AGGATACCTT CCGAATCCCG AACGGCAAGG ATCTTTAGTT CCCAATAGCC
 251  TGTGGGGTTC TTTTGTTGAT CAGCGTGCTA TCCAAGAAAT CATGGTAAAT
 301  AGTAGCCAAA TCTTATGTCA GGAACGGGGA GTCTGGGGAG CTGGAATTGC
 351  TAATTTCCTA CATAGAGATA AAATTAATGA GCACGGCTAT CGCCATAGCG
 401  GTGTCGGTTA TCTTGTGGGA GTTGGCACTC ATGCTTTTTC TGATGCTACG
 451  ATAAATGCGG CTTTTTGCCA GCTCTTCAGT AGAGATAAAG ACTACGTAGT
 501  ATCCAAAAAT CATGGAACTA GCTACTCAGG GGTCGTATTT CTTGAGGATA
 551  CCCTAGAGTT TAGAAGTCCA CAGGGATTCT ATACTGATAG CTCCTCAGAA
 601  GCTTGCTGTA ACCAAGTCGT CACTATAGAT ATGCAGTTGT CTTACAGCCA
 651  TAGAAATAAT GATATGAAAA CCAAATACAC GACATATCCA GAAGCTCAGG
 701  GATCTTGGGC AAATGATGTT TTTGGTCTTG AGTTTGGAGC GACTACATAC
 751  TACTACCCTA ACAGTACTTT TTTATTTGAT TACTACTCTC CGTTTCTCAG
 801  GCTGCAGTGC ACCTATGCTC ACCAGGAAGA CTTCAAAGAG ACAGGAGGTG
 851  AGGTTCGTCA CTTTACTAGC GGAGATCTTT TCAATTTAGC AGTTCCTATT
 901  GGCGTGAAGT TTGAGAGATT TTCAGACTGT AAAAGGGGAT CTTATGAACT
 951  TACCCTTGCT TATGTTCCTG ATGTGATTCG CAAAGATCCC AAGAGCACGG
1001  CAACATTGGC TAGTGGAGCT ACGTGGAGCA CCCACGGAAA CAATCTCTCC
1051  AGACAAGGAT TACAACTGCG TTTAGGGAAC CACTGTCTCA TAAATCCTGG
1101  AATTGAGGTG TTCAGTCACG GAGCTATTGA ATTGCGGGGA TCCTCTCGTA
1151  ATTATAACAT CAATCTCGGG GGTAAATACC GATTTTAA
```

The PSORT algorithm predicts a cytoplasmic location (0.189).

The protein was expressed in *E.coli* and purified as a GST-fusion product, as shown in Figure 11A. This protein was used to immunise mice, whose sera were used in a Western blot (Figure 11B) and an immunoblot assay (Figure 11C). A his-tagged protein was also expressed.

These experiments show that cp0019 is a useful immunogen. These properties are not evident from the sequence alone.

## Example 12

The following *C.pneumoniae* protein (PID 4376466) was expressed <SEQ ID 23; cp6466>:

```
  1  MRKISVGICI TILLSLSVVL QGCKESSHSS TSRGELAINI RDEPRSLDPR
 51  QVRLLSEISL VKHIYEGLVQ ENNLSGNIEP ALAEDYSLSS DGLTYTFKLK
101  SAFWSNGDPL TAEDFIESWK QVATQEVSGI YAFALNPIKN VRKIQEGHLS
151  IDHFGVHSPN ESTLVVTLES PTSHFLKLLA LPVFFPVHKS QRTLQSKSLP
201  IASGAFYPKN IKQKQWIKLS KNPHYYNQSQ VETKTITIHF IPDANTAAKL
```

```
251  FNQGKLNWQG PPWGERIPQE TLSNLQSKGH LHSFDVAGTS WLTFNINKFP
301  LNNMKLREAL ASALDKEALV STIFLGRAKT ADHLLPTNIH SYPEHQKQEM
351  AQRQAYAKKL FKEALEELQI TAKDLEHLNL IFPVSSSASS LLVQLIREQW
401  KESLGFAIPI VGKEFALLQA DLSSGNFSLA TGGWFADFAD PMAFLTIFAY
451  PSGVPPYAIN HKDFLEILQN IEQEQDHQKR SELVSQASLY LETFHIIEPI
501  YHDAFQFAMN KKLSNLGVSP TGVVDFRYAK EN*
```

A predicted signal peptide is highlighted.

The cp6466 nucleotide sequence <SEQ ID 24> is:

```
   1  ATGCGCAAGA TATCAGTGGG AATCTGTATC ACCATTCTCC TTAGCCTCTC
  51  CGTAGTCCTC CAAGGCTGCA AGGAGTCCAG TCACTCCTCT ACATCTCGGG
 101  GAGAACTCGC TATTAATATA AGAGATGAAC CCCGTTCTTT AGATCCAAGA
 151  CAAGTGCGAC TTCTTTCAGA AATCAGCCTT GTCAAACATA TCTATGAGGG
 201  ATTAGTTCAA GAAAATAATC TTTCAGGAAA TATAGAGCCT GCTCTTGCAG
 251  AAGACTACTC TCTTTCCTCG GACGGACTCA CTTATACTTT TAAACTGAAA
 301  TCAGCTTTTT GGAGTAATGG CGACCCCTTA ACAGCTGAAG ACTTTATAGA
 351  ATCTTGGAAA CAAGTAGCTA CTCAAGAAGT CTCAGGAATC TATGCTTTTG
 401  CCTTGAATCC AATTAAAAAT GTACGAAAGA TCCAAGAGGG ACACCTCTCC
 451  ATAGACCATT TTGGAGTGCA CTCTCCTAAT GAATCTACAC TTGTTGTTAC
 501  CCTGGAATCC CCAACCTCGC ATTTCTTAAA ACTTTTAGCT CTTCCAGTCT
 551  TTTTCCCCGT TCATAAATCT CAAAGAACCC TGCAATCCAA ATCTCTACCT
 601  ATAGCAAGCG GAGCTTTCTA TCCTAAAAAT ATCAAACAAA AACAATGGAT
 651  AAAACTCTCA AAAAACCCTC ACTACTATAA TCAAAGTCAG GTGGAAACTA
 701  AAACGATTAC GATTCACTTC ATTCCCGATG CAAACACAGC AGCAAAACTA
 751  TTTAATCAGG GAAAACTCAA TTGGCAAGGA CCTCCTTGGG GAGAACGCAT
 801  TCCTCAAGAA ACCCTATCCA ATTTACAGTC TAAGGGGCAC TTACACTCTT
 851  TTGATGTCGC AGGAACCTCA TGGCTCACCT TCAATATCAA TAAATTCCCC
 901  CTCAACAATA TGAAGCTTAG AGAAGCCTTA GCATCAGCCT TAGATAAGGA
 951  AGCTCTTGTC TCAACTATAT TCTTAGGCCG TGCAAAAACT GCCGATCATC
1001  TCCTACCTAC AAATATTCAT AGCTATCCCG AACATCAAAA ACAAGAGATG
1051  GCACAACGCC AAGCTTACGC TAAAAAACTC TTTAAAGAAG CTTTAGAAGA
1101  ACTCCAAATC ACTGCTAAAG ATCTCGAACA TCTTAATCTT ATCTTTCCCG
1151  TTTCCTCGTC AGCAAGTTCT TTACTAGTCC AACTTATACG AGAACAGTGG
1201  AAAGAAAGTT TAGGGTTCGC TATCCCTATT GTCGGAAAGG AATTTGCTCT
1251  TCTCCAAGCA GACCTATCTT CAGGGAACTT CTCTTTAGCT ACAGGAGGAT
1301  GGTTCGCAGA CTTTGCTGAT CCTATGGCAT TTCTAACGAT CTTTGCTTAT
1351  CCATCAGGAG TTCCTCCTTA TGCAATCAAC CATAAGGACT TCCTAGAAAT
1401  TCTACAAAAC ATAGAACAAG AGCAAGATCA CCAAAAACGC TCGGAATTAG
1451  TGTCGCAAGC TTCTCTTTAC CTAGAGACCT TTCATATTAT TGAGCCGATC
1501  TACCACGACG CATTTCAATT TGCTATGAAT AAAAAACTTT CTAATCTAGG
1551  AGTCTCACCA ACAGGAGTTG TGGACTTCCG TTATGCTAAG GAAAATTAG
```

The PSORT algorithm predicts that the protein is an outer membrane lipoprotein (0.790).

The protein was expressed in *E.coli* and purified both as a GST-fusion product and a His-tag fusion product. Purification of the protein as a GST-fusion product is shown in Figure 12A. The recombinant proteins were used to immunise mice, whose sera were used in Western blots (Figures 12B and 12C). FACS analysis was also performed.

These experiments show that cp6466 is a useful immunogen. These properties are not evident from the sequence alone.

## Example 13

The following *C.pneumoniae* protein (PID 4376468) was expressed <SEQ ID 25; cp6468>:

```
  1  MFSRWITLFL LFISLTGCSS YSSKHKQSLI IPIHDDPVAF SPEQAKRAMD
 51  LSIAQLLFDG LTRETHRESN DLELAIASRY TVSEDFCSYT FFIKDSALWS
101  DGTPITSEDI RNAWEYAQEN SPHIQIFQGL NFSTPSSNAI TIHLDSPNPD
151  FPKLLAFPAF AIFKPENPKL FSGPYTLVEY FPGHNIHLKK NPNYYDYHCV
201  SINSIKLLII PDIYTAIHLL NRGKVDWVGQ PWHQGIPWEL HKQSQYHYYT
251  YPVEGAFWLC LNTKSPHLND LQNRHRLATC IDKRSIIEEA LQGTQQPAET

301  LSRGAPQPNQ YKKQKPLTPQ EKLVLTYPSD ILRCQRIAEI LKEQWKAAGI
351  DLILEGLEYH LFVNKRKVQD YAIATQTGVA YYPGANLISE EDKLLQNFEI
401  IPIYYLSYDY LTQDFIEGVI YNASGAVDLK YTYFP*
```

A predicted signal peptide is highlighted.

The cp6468 nucleotide sequence <SEQ ID 26> is:

```
   1  ATGTTTTCAC GATGGATCAC CCTCTTTTTA TTATTCATTA GCCTTACTGG
  51  ATGCTCCTCC TACTCTTCAA AACATAAACA ATCTTTAATT ATTCCCATAC
 101  ATGACGACCC TGTAGCTTTT TCTCCTGAAC AAGCAAAACG GGCCATGGAC
 151  CTTTCTATTG CCCAACTTCT TTTTGATGGT CTGACTAGAG AAACTCATCG
 201  CGAATCCAAT GATTTGGAAT TAGCGATTGC CAGTCGCTAT ACAGTCTCTG
 251  AAGACTTTTG CTCTTATACG TTCTTTATCA AAGACAGCGC TTTATGGAGC
 301  GACGGAACAC CAATCACCTC CGAAGATATC CGTAACGCTT GGGAGTATGC
 351  ACAGGAGAAC TCTCCCCACA TACAGATCTT CCAAGGACTT AACTTCTCAA
 401  CTCCTTCATC AAATGCAATT ACGATTCATC TCGACTCGCC CAACCCCGAT
 451  TTTCCTAAGC TTCTTGCCTT TCCTGCATTT GCTATCTTTA AACCAGAAAA
 501  CCCGAAGCTC TTTAGCGGTC CGTATACTCT TGTAGAGTAT TTCCCAGGGC
 551  ATAACATTCA TTTAAAGAAA AACCCTAACT ATTACGACTA CCACTGCGTC
 601  TCCATCAACT CCATCAAACT GCTCATTATT CCTGATATAT ATACAGCCAT
 651  CCACCTCCTA AACAGAGGCA AGGTGGACTG GGTAGGACAA CCCTGGCATC
 701  AAGGGATTCC TTGGGAGCTC CATAAACAAT CGCAATATCA CTACTACACC
 751  TATCCTGTAG AAGGTGCCTT CTGGCTTTGT CTAAATACAA AATCCCCACA
 801  CTTAAATGAT CTTCAAAACA GACATAGACT CGCTACTTGT ATTGATAAAC
 851  GTTCTATCAT TGAAGAAGCT CTTCAAGGAA CCCAACAACC AGCGGAAACA
 901  CTGTCCCGAG GAGCTCCACA ACCAAATCAA TATAAAAAAC AAAAGCCTCT
 951  AACTCCACAA GAAAAACTCG TGCTTACCTA TCCCTCAGAT ATTCTAAGAT
1001  GCCAACGCAT AGCAGAAATC TTAAAGGAAC AATGGAAAGC TGCTGGAATA
1051  GATTTAATCC TTGAAGGACT CGAATACCAT CTGTTTGTTA ACAAACGAAA
1101  AGTCCAAGAC TACGCCATAG CAACACAGAC TGGAGTTGCT TATTACCCAG
1151  GAGCAAATCT AATTTCTGAA GAAGACAAGC TCCTGCAAAA CTTTGAGATT
1201  ATCCCGATCT ACTATCTGAG CTATGACTAT CTCACTCAAG ATTTTATAGA
1251  GGGAGTAATC TATAATGCTT CTGGAGCTGT AGATCTCAAA TATACCTATT
1301  TCCCCTAG
```

The PSORT algorithm predicts that this protein is an outer membrane lipoprotein (0.790).

The protein was expressed in *E.coli* and purified as a GST-fusion product, as shown in Figure 13A. The recombinant protein was used to immunise mice, whose sera were used in a Western blot (Figure 13B) and for FACS analysis. A his-tagged protein was also expressed.

These experiments show that cp6468 is a useful immunogen. These properties are not evident from the sequence alone.

## Example 14

The following *C.pneumoniae* protein (PID 4376469) was expressed <SEQ ID 27; cp6469>:

```
  1  MKMHRLKPTL KSLIPNLLFL LLTLSSCSKQ KQEPLGKHLV IAMSHDLADL
 51  DPRNAYLSRD ASLAKALYEG LTRETDQGIA LALAESYTLS KDHKVYTFKL
101  RPSVWSDGTP LTAYDFEKSI KQLYFEEFSP SIHTLLGVIK NSSAIHNAQK
151  SLETLGIQAK DDLTLVITLE QPFPYFLTLI ARPVFSPVHH TLRESYKKGT
201  PPSTYISNGP FVLKKHEHQN YLILEKNPHY YDHESVKLDR VTLKIIPDAS
251  TATKLFKSKS IDWIGSPWSA PISNEDQKVL SQEKILTYSV SSTTLLIYNL
301  QKPLIQNKAL RKAIAHAIDR KSILRLVPSG QEAVTLVPPN LSQLNLQKEI
351  STEERQTKAR AYFQEAKETL SEKELAELSI LYPIDSSNSS IIAQEIQRQL
401  KDTLGLKIKI QGMEYHCFLK KRRQGDFFIA TGGWIAEYVS PVAFLSILGN
451  PRDLTQWRNS DYEKTLEKLY LPHAYKENLK RAEMIIEEET PIIPLYHGKY
501  IYAIHPKIQN TFGSLLGHTD LKNIDILS*
```

A predicted signal peptide is highlighted.

The cp6469 nucleotide sequence <SEQ ID 28> is:

```
   1  ATGAAGATGC ATAGGCTTAA ACCTACCTTA AAAAGTCTGA TCCCTAATCT
  51  TCTTTTCTTA TTGCTCACTC TTTCAAGCTG CTCAAAGCAA AAACAAGAAC
 101  CCTTAGGAAA ACATCTCGTT ATTGCGATGA GCCATGATCT CGCCGACCTA
 151  GATCCTCGCA ATGCCTATTT AAGCAGAGAT GCTTCCCTAG CAAAAGCCCT
 201  CTATGAAGGA CTGACAAGAG AAACTGATCA AGGAATCGCA CTGGCTCTTG
 251  CAGAAAGTTA TACCCTGTCA AAAGATCATA AGGTCTATAC CTTTAAACTC
 301  AGACCTTCTG TGTGGAGCGA TGGCACTCCA CTCACTGCTT ATGACTTTGA
 351  AAAATCTATA AAACAACTGT ACTTCGAAGA ATTTTCACCT TCCATACATA
 401  CTTTACTCGG CGTGATTAAA AATTCTTCGG CAATCCACAA TGCTCAAAAA
 451  TCTCTGGAAA CTCTTGGGAT ACAGGCAAAA GATGATCTTA CTTTGGTGAT
 501  TACCCTAGAG CAACCTTTCC CATACTTTCT CACACTTATC GCTCGCCCCG
 551  TATTCTCCCC TGTTCATCAC ACCCTTAGGG AATCCTATAA GAAAGGAACA
 601  CCCCCATCCA CATACATCTC CAATGGGCCC TTTGTCTTAA AAAAACATGA
 651  ACACCAAAAC TACTTAATTT TAGAAAAAAA TCCTCACTAC TATGATCATG
 701  AATCAGTAAA GTTAGACCGA GTCACCTTAA AAATTATCCC AGACGCCTCC
 751  ACAGCCACGA AACTTTTCAA AAGTAAATCT ATAGATTGGA TTGGCTCACC
 801  TTGGAGCGCT CCGATATCTA ACGAAGACCA AAAAGTTCTC TCCCAAGAAA
 851  AGATTCTTAC CTATTCTGTT TCAAGCACCA CCCTTCTTAT CTATAACCTG
 901  CAAAAACCTC TAATACAAAA TAAAGCCCTC AGGAAAGCCA TTGCTCATGC
 951  TATTGATAGA AAATCTATCT TAAGACTCGT GCCTTCAGGA CAAGAAGCTG
1001  TAACTCTAGT TCCCCCAAAT CTTTCACAAC TCAATCTTCA AAAAGAGATC
1051  TCAACAGAAG AACGACAAAC AAAAGCCAGA GCATATTTTC AAGAAGCTAA
1101  AGAAACACTT TCTGAAAAAG AACTCGCAGA ACTCAGCATC CTCTATCCTA
1151  TAGATTCCTC GAATTCCTCC ATCATAGCTC AAGAAATCCA AAGACAACTT
1201  AAAGATACCT TAGGATTGAA AATCAAAATC CAAGGCATGG AGTACCACTG
1251  CTTTTTAAAG AAACGTCGTC AAGGAGATTT CTTCATAGCG ACAGGAGGAT
1301  GGATTGCGGA ATACGTAAGC CCCGTAGCCT TCCTATCTAT TCTAGGCAAC
1351  CCCAGAGACC TCACACAATG GAGAAACAGT GATTACGAAA AGACTTTAGA
1401  GAAACTCTAT CTCCCTCATG CCTACAAAGA GAATTTAAAA CGCGCAGAAA
1451  TGATAATAGA AGAAGAAACC CCGATTATCC CCCTGTATCA CGGCAAATAT
1501  ATTTACGCTA TACATCCTAA AATCCAGAAT ACATTCGGAT CTCTTCTAGG
1551  CCACACAGAT CTCAAAAATA TCGATATCTT AAGTTAG
```

The PSORT algorithm predicts a periplasmic location (0.934).

The protein was expressed in *E.coli* and purified as a GST-fusion product, as shown in Figure 14A. The recombinant protein was used to immunise mice, whose sera were used in a Western blot (Figure 14B) and for FACS analysis. A his-tagged protein was also expressed.

These experiments show that cp6469 is a useful immunogen. These properties are not evident from the sequence alone.

### Example 15

The following *C.pneumoniae* protein (PID 4376602) was expressed <SEQ ID 29; cp6602>:

```
  1 MAASGGTGGL GGTQGVNLAA VEAAAAKADA AEVVASQEGS EMNMIQQSQD
 51 LTNPAAATRT KKKEEKFQTL ESRKKGEAGK AEKKSESTEE KPDTDLADKY
101 ASGNSEISGQ ELRGLRDAIG DDASPEDILA LVQEKIKDPA LQSTALDYLV
151 QTTPPSQGKL KEALIQARNT HTEQFGRTAI GAKNILFASQ EYADQLNVSP
201 SGLRSLYLEV TGDTHTCDQL LSMLQDRYTY QDMAIVSSFL MKGMATELKR
251 QGPYVPSAQL QVLMTETRNL QAVLTSYDYF ESRVPILLDS LKAEGIQTPS
301 DLNFVKVAES YHKIINDKFP TASKVEREVR NLIGDDVDSV TGVLNLFFSA
351 LRQTSSRLFS SADKRQQLGA MIANALDAVN INNEDYPKAS DFPKPYPWS*
```

The cp6602 nucleotide sequence <SEQ ID 30> is:

```
   1 ATGGCAGCAT CAGGAGGCAC AGGTGGTTTA GGAGGCACTC AGGGTGTCAA
  51 CCTTGCAGCT GTAGAAGCTG CAGCTGCAAA AGCAGATGCA GCAGAAGTTG
 101 TAGCCAGCCA AGAAGGTTCT GAGATGAACA TGATTCAACA ATCTCAGGAC
 151 CTGACAAATC CCGCAGCAGC AACACGCACG AAAAAAAAGG AAGAGAAGTT
 201 TCAAACTCTA GAATCTCGGA AAAAAGGAGA AGCTGGAAAG GCTGAGAAAA
 251 AATCTGAATC TACAGAAGAG AAGCCTGACA CAGATCTTGC TGATAAGTAT
 301 GCTTCTGGGA ATTCTGAAAT CTCTGGTCAA GAACTTCGCG GCCTGCGTGA
 351 TGCAATAGGA GACGATGCTT CTCCAGAAGA CATTCTTGCT CTTGTACAAG

 401 AGAAAATTAA AGACCCAGCT CTGCAATCCA CAGCTTTGGA CTACCTGGTT
 451 CAAACGACTC CACCCTCCCA AGGTAAATTA AAAGAAGCGC TTATCCAAGC
 501 AAGGAATACT CATACGGAGC AATTCGGACG AACTGCTATT GGTGCGAAAA
 551 ACATCTTATT TGCCTCTCAA GAATATGCAG ACCAACTGAA TGTTTCTCCT
 601 TCAGGGCTTC GCTCTTTGTA CTTAGAAGTG ACTGGAGACA CACATACCTG
 651 TGATCAGCTA CTTTCTATGC TTCAAGACCG CTATACCTAC CAAGATATGG
 701 CTATTGTCAG CTCCTTTCTA ATGAAAGGAA TGGCAACAGA ATTAAAAAGG
 751 CAGGGTCCCT ACGTACCCAG TGCGCAACTA CAAGTTCTCA TGACAGAAAC
 801 TCGTAACCTG CAAGCAGTTC TTACCTCGTA CGATTACTTT GAAAGTCGCG
 851 TTCCTATTTT ACTCGATAGC TTAAAAGCTG AGGGAATCCA AACTCCTTCT
 901 GATCTAAACT TTGTGAAGGT AGCTGAGTCC TACCATAAAA TCATTAACGA
 951 TAAGTTCCCA ACAGCATCTA AAGTAGAACG AGAAGTCCGC AATCTCATAG
1001 GAGACGATGT TGATTCTGTG ACCGGTGTCT TGAACTTATT CTTTTCTGCT
1051 TTACGTCAAA CGTCGTCACG CCTTTTCTCT TCAGCAGACA AACGTCAGCA
1101 ATTAGGAGCT ATGATTGCTA ATGCTTTAGA TGCTGTAAAT ATAAACAATG
1151 AAGATTATCC CAAAGCATCA GACTTCCCTA AACCCTATCC TTGGTCATGA
```

The PSORT algorithm predicts a cytoplasmic location (0.080).

The protein was expressed in *E.coli* and purified as both a His-tag and a GST-fusion product, as shown in Figure 15A. The recombinant proteins were used to immunise mice, whose sera were used in a Western blot (Figure 15B) and for FACS analysis (Figure 15C).

The cp6602 protein was also identified in the 2D-PAGE experiment (Cpn0324).

These experiments show that cp6602 is a surface-exposed and immunoaccessible protein, and that it is a useful immunogen. These properties are not evident from the sequence alone.

## Example 16

The following *C.pneumoniae* protein (PID 4376727) was expressed <SEQ ID 31; cp6727>:

1 **MKYSLPWLLT SSALVF**SLHP LMAANTDLSS SDNYENGSSG SAAFTAKETS
51 DASGTTYTLT SDVSITNVSA ITPADKSCFT NTGGALSFVG ADHSLVLQTI
101 ALTHDGAAIN NTNTALSFSG FSSLLIDSAP ATGTSGGKGA ICVTNTEGGT
151 ATFTDNASVT LQKNTSEKDG AAVSAYSIDL AKTTTAALLD QNTSTKNGGA
201 LCSTANTTVQ GNSGTVTFSS NTATDKGGGI YSKEKDSTLD ANTGVVTFKS
251 NTAKTGGAWS SDDNLALTGN TQVLFQENKT TGSAAQANNP EGCGGAICCY
301 LATATDKTGL AISQNQEMSF TSNTTTANGG AIYATKCTLD GNTTLTFDQN
351 TATAGCGGAI YTETEDFSLK GSTGTVTFST NTAKTGGALY SKGNSSLTGN
401 TNLLFSGNKA TGPSNSSANQ EGCGGAILAF IDSGSVSDKT GLSIANNQEV
451 SLTSNAATVS GGAIYATKCT LTGNGSLTFD GNTAGTSGGA IYTETEDFTL
501 TGSTGTVTFS TNTAKTGGAL YSKGNNSLSG NTNLLFSGNK ATGPSNSSAN
551 QEGCGGAILS FLESASVSTK KGLWIEDNEN VSLSGNTATV SGGAIYATKC
601 ALHGNTTLTF DGNTAETAGG AIYTETEDFT LTGSTGTVTF STNTAKTAGA
651 LHTKGNTSFT KNKALVFSGN SATATATTTT DQEGCGGAIL CNISESDIAT
701 KSLTLTENES LSFINNTAKR SGGGIYAPKC VISGSESINF DGNTAETSGG
751 AIYSKNLSIT ANGPVSFTNN SGGKGGAIYI ADSGELSLEA IDGDITFSGN
801 RATEGTSTPN SIHLGAGAKI TKLAAAPGHT IYFYDPITME APASGGTIEE
851 LVINPVVKAI VPPPQPKNGP IASVPVVPVA PANPNTGTIV FSSGKLPSQD
901 ASIPANTTTI LNQKINLAGG NVVLKEGATL QVYSFTQQPD STVFMDAGTT
951 LETTTTNNTD GSIDLKNLSV NLDALDGKRM ITIAVNSTSG GLKISGDLKF
1001 HNNEGSFYDN PGLKANLNLP FLDLSSTSGT VNLDDFNPIP SSMAAPDYGY
1051 QGSWTLVPKV GAGGKVTLVA EWQALGYTPK PELRATLVPN SLWNAYVNIH
1101 SIQQEIATAM SDAPSHPGIW IGGIGNAFHQ DKQKENAGFR LISRGYIVGG
1151 SMTTPQEYTF AVAFSQLFGK SKDYVVSDIK SQVYAGSLCA QSSYVIPLHS
1201 SLRRHVLSKV LPELPGETPL VLHGQVSYGR NHHNMTTKLA NNTQGKSDWD
1251 SHSFAVEVGG SLPVDLNYRY LTSYSPYVKL QVVSVNQKGF QEVAADPRIF
1301 DASHLVNVSI PMGLTFKHES AKPPSALLLT LGYAVDAYRD HPHCLTSLTN
1351 GTSWSTFATN LSRQAFFAEA SGHLKLLHGL DCFASGSCEL RSSSRSYNAN
1401 CGTRYSF*

A predicted signal peptide is highlighted.

The cp6727 nucleotide sequence <SEQ ID 32> is:

```
1    ATGAAATATT CTTTACCTTG GCTACTTACC TCTTCGGCTT TAGTTTTCTC
51   CCTACATCCA CTAATGGCTG CTAACACGGA TCTCTCATCA TCCGATAACT
101  ATGAAAATGG TAGTAGTGGT AGCGCAGCAT TCACTGCCAA GGAAACTTCG
151  GATGCTTCAG GAACTACCTA CACTCTCACT AGCGATGTTT CTATTACGAA
201  TGTATCTGCA ATTACTCCTG CAGATAAAAG CTGTTTTACA AACACAGGAG
251  GAGCATTGAG TTTTGTTGGA GCTGATCACT CATTGGTTCT GCAAACCATA
301  GCGCTTACGC ATGATGGTGC TGCAATTAAC AATACCAACA CAGCTCTTTC
351  TTTCTCAGGA TTCTCGTCAC TCTTAATCGA CTCAGCTCCA GCAACAGGAA
401  CTTCGGGCGG CAAGGGTGCT ATTTGTGTGA CAAATACAGA GGGAGGTACT
451  GCGACTTTTA CTGACAATGC CAGTGTCACC CTCCAAAAAA ATACTTCAGA
501  AAAAGATGGA GCTGCAGTTT CTGCCTACAG CATCGATCTT GCTAAGACTA
551  CGACAGCAGC TCTCTTAGAT CAAAATACTA GCACAAAAAA TGGCGGGGCC
601  CTCTGTAGTA CAGCAAACAC TACAGTCCAA GGAAACTCAG GAACGGTGAC
651  CTTCTCCTCA AATACTGCTA CAGATAAAGG TGGGGGGATC TACTCAAAAG
701  AAAAGGATAG CACGCTAGAT GCCAATACAG GAGTCGTTAC CTTCAAATCT
751  AATACTGCAA AGACGGGGGG TGCTTGGAGC TCTGATGACA ATCTTGCTCT
801  TACCGGCAAC ACTCAAGTAC TTTTTCAGGA AAATAAAACA ACCGGCTCAG
851  CAGCACAGGC AAATAACCCG GAAGGTTGTG GTGGGGCAAT CTGTTGTTAT
901  CTTGCTACAG CAACAGACAA AACTGGATTA GCCATTTCTC AGAATCAAGA
951  AATGAGCTTC ACTAGTAATA CAACAACTGC GAATGGTGGA GCGATCTACG
1001 CTACTAAATG TACTCTGGAT GGAAACACAA CTCTTACCTT CGATCAGAAT
1051 ACTGCGACAG CAGGATGTGG CGGAGCTATC TATACAGAAA CTGAAGATTT
1101 TTCTCTTAAG GGAAGTACGG GAACCGTGAC CTTCAGCACA AATACAGCAA
1151 AGACAGGCGG CGCCTTATAT TCTAAAGGAA ACAGCTCGCT GACTGGAAAT
1201 ACCAACCTGC TCTTTTCAGG GAACAAAGCT ACGGGCCCGA GTAATTCTTC
1251 AGCAAATCAA GAGGGTTGCG GTGGGGCAAT CCTAGCCTTT ATTGATTCAG
1301 GATCCGTAAG CGATAAAACA GGACTATCGA TTGCAAACAA CCAAGAAGTC
1351 AGCCTCACTA GTAATGCTGC AACAGTAAGT GGTGGTGCGA TCTATGCTAC
1401 CAAATGTACT CTAACTGGAA ACGGCTCCCT GACCTTTGAC GGCAATACTG
1451 CTGGAACTTC AGGAGGGGCG ATCTATACAG AAACTGAAGA TTTTACTCTT
1501 ACAGGAAGTA CAGGAACCGT GACCTTCAGC ACAAATACAG CAAAGACAGG
1551 CGGCGCCTTA TATTCTAAAG GCAACAACTC TCTGTCTGGT AATACCAACC
1601 TGCTCTTTTC AGGGAACAAA GCTACGGGCC CGAGTAATTC TTCAGCAAAT
1651 CAAGAGGGTT GCGGTGGGGC AATCCTATCG TTTCTTGAGT CAGCATCTGT
1701 AAGTACTAAA AAAGGACTCT GGATTGAAGA TAACGAAAAC GTGAGTCTCT
1751 CTGGTAATAC TGCAACAGTA AGTGGCGGTG CGATCTATGC GACCAAGTGT
1801 GCTCTGCATG GAAACACGAC TCTTACCTTT GATGGCAATA CTGCCGAAAC
1851 TGCAGGAGGA GCGATCTATA CAGAAACCGA AGATTTTACT CTTACGGGAA
1901 GTACGGGAAC CGTGACCTTC AGCACAAATA CAGCAAAGAC AGCAGGGGCT
1951 CTACATACTA AAGGAAATAC TTCCTTTACC AAAAATAAGG CTCTTGTATT
2001 TTCTGGAAAT TCAGCAACAG CAACAGCAAC AACAACTACA GATCAAGAAG
2051 GTTGTGGTGG AGCGATCCTC TGTAATATCT CAGAGTCTGA CATAGCTACA
2101 AAAAGCTTAA CTCTTACTGA AAATGAGAGT TTAAGTTTCA TTAACAATAC
2151 GGCAAAAAGA AGTGGTGGTG GTATTTATGC TCCTAAGTGT GTAATCTCAG
2201 GCAGTGAATC CATAAACTTT GATGGCAATA CTGCTGAAAC TTCGGGAGGA
2251 GCGATTTATT CGAAAAACCT TTCGATTACA GCTAACGGTC CTGTCTCCTT
2301 TACCAATAAT TCTGGAGGCA AGGGAGGCGC CATTTATATA GCCGATAGCG
2351 GAGAACTTTC CTTAGAGGCT ATTGATGGGG ATATTACTTT CTCAGGGAAC
2401 CGAGCGACTG AGGGAACTTC AACTCCCAAC TCGATCCATT TAGGTGCAGG
2451 GGCTAAGATC ACTAAGCTTG CAGCAGCTCC TGGTCATACG ATTTATTTTT
2501 ATGATCCTAT TACGATGGAA GCTCCTGCAT CTGGAGGAAC AATAGAGGAG
2551 TTAGTCATCA ATCCTGTTGT CAAAGCTATT GTTCCTCCTC CCCAACCAAA
2601 AAATGGTCCT ATAGCTTCAG TGCCTGTAGT CCCTGTAGCA CCTGCAAACC
2651 CAAACACGGG AACTATAGTA TTTTCTTCTG GAAAACTCCC CAGTCAAGAT
2701 GCCTCGATTC CTGCAAATAC TACCACCATA CTGAACCAGA AGATCAACTT
2751 AGCAGGAGGA AATGTCGTTT TAAAAGAAGG AGCCACCCTA CAAGTATATT
2801 CCTTCACACA GCAGCCTGAT TCTACAGTAT TCATGGATGC AGGAACGACC
2851 TTAGAGACCA CGACAACTAA CAATACAGAT GGCAGCATCG ATCTAAAGAA
2901 TCTCTCTGTA AATCTGGATG CTTTAGATGG CAAGCGTATG ATAACGATTG
2951 CCGTAAACAG CACAAGTGGG GGATTAAAAA TCTCAGGGGA TCTGAAATTC
3001 CATAACAATG AAGGAAGTTT CTATGACAAT CCTGGGTTGA AAGCAAACTT
3051 AAATCTTCCT TTCTTAGATC TTTCTTCTAC TTCAGGAACT GTAAATTTAG
3101 ACGACTTCAA TCCGATTCCT TCTAGCATGG CTGCTCCGGA TTATGGGTAT
3151 CAAGGGAGTT GGACTCTGGT TCCTAAAGTA GGAGCTGGAG GGAAGGTGAC
3201 TTTGGTCGCG GAATGGCAAG CGTTAGGATA CACTCCTAAA CCAGAGCTTC
3251 GTGCGACTTT AGTTCCTAAT AGCCTTTGGA ATGCTTATGT AAACATCCAT
```

```
3301  TCTATACAGC AGGAGATCGC CACTGCGATG TCGGACGCTC CCTCACATCC
3351  AGGGATTTGG ATTGGAGGTA TTGGCAACGC CTTCCATCAA GACAAGCAAA
3401  AGGAAAATGC AGGATTCCGT TTGATTTCCA GAGGTTATAT TGTTGGTGGC
3451  AGCATGACCA CCCCTCAAGA ATATACCTTT GCTGTTGCAT TCAGCCAACT
3501  CTTTGGCAAA TCTAAGGATT ACGTAGTCTC GGATATTAAA TCTCAAGTCT
3551  ATGCAGGATC TCTCTGTGCT CAGAGCTCTT ATGTCATTCC CCTGCATAGC
3601  TCATTACGTC GCCACGTCCT CTCTAAGGTC CTTCCAGAGC TCCCAGGAGA
3651  AACTCCCCTT GTTCTCCATG GTCAAGTTTC CTATGGAAGA AACCACCATA
3701  ATATGACGAC AAAGCTTGCG AACAACACAC AAGGGAAATC AGACTGGGAC
3751  AGCCATAGCT TCGCTGTTGA AGTCGGTGGT TCTCTTCCTG TAGATCTAAA
3801  CTACAGATAC CTTACCAGCT ACTCTCCCTA TGTGAAACTC CAAGTTGTGA
3851  GTGTAAATCA AAAAGGATTC CAAGAGGTTG CTGCTGATCC ACGTATCTTT
3901  GACGCTAGCC ATCTGGTCAA CGTGTCTATC CCTATGGGAC TCACCTTCAA
3951  ACACGAATCA GCAAAGCCCC CCAGTGCTTT GCTTCTTACT TTAGGTTACG
4001  CTGTAGATGC TTACCGGGAT CACCCTCACT GCCTGACCTC CTTAACAAAT
4051  GGCACCTCGT GGTCTACGTT TGCTACAAAC TTATCACGAC AAGCTTTCTT
4101  TGCTGAGGCT TCTGGACATC TGAAGTTACT TCATGGTCTT GACTGCTTCG
4151  CTTCTGGAAG TTGTGAACTG CGCAGCTCCT CAAGAAGCTA TAATGCAAAC
4201  TGTGGAACTC GTTATTCTTT CTAA
```

The PSORT algorithm predicts an outer membrane location (0.915).

The protein was expressed in *E.coli* and purified as a his-tag product, as shown in Figure 16A. The recombinant protein was used to immunise mice, whose sera were used in a Western blot (Figure 16B) and for FACS analysis (Figure 16C). A GST-fusion protein was also expressed.

The cp6727 protein was also identified in the 2D-PAGE experiment (Cpn0444).

These experiments show that cp6727 is a surface-exposed and immunoaccessible protein, and that it is a useful immunogen. These properties are not evident from the sequence alone.

**Example 17**

The following *C.pneumoniae* protein (PID 4376731) was expressed <SEQ ID 33; cp6731>:

```
  1  MKSSLHWFLI SSSLALPLSL NFSAFAAVVE INLGPTNSFS GPGTYTPPAQ
 51  TTNADGTIYN LTGDVSITNA GSPTALTASC FKETTGNLSF QGHGYQFLLQ
101  NIDAGANCTF TNTAANKLLS FSGFSYLSLI QTTNATTGTG AIKSTGACSI
151  QSNYSCYFGQ NFSNDNGGAL QGSSISLSLN PNLTFAKNKA TQKGGALYST
201  GGITINNTLN SASFSENTAA NNGGAIYTEA SSFISSNKAI SFINNSVTAT
251  SATGGAIYCS STSAPKPVLT LSDNGELNFI GNTAITSGGA IYTDNLVLSS
301  GGPTLFKNNS AIDTAAPLGG AIAIADSGSL SLSALGGDIT FEGNTVVKGA
351  SSSQTTTRNS INIGNTNAKI VQLRASQGNT IYFYDPITTS ITAALSDALN
401  LNGPDLAGNP AYQGTIVFSG EKLSEAEAAE ADNLKSTIQQ PLTLAGGQLS
451  LKSGVTLVAK SFSQSPGSTL LMDAGTTLET ADGITINNLV LNVDSLKETK
501  KATLKATQAS QTVTLSGSLS LVDPSGNVYE DVSWNNPQVF SCLTLTADDP
551  ANIHITDLAA DPLEKNPIHW GYQGNWALSW QEDTATKSKA ATLTWTKTGY
601  NPNPERRGTL VANTLWGSFV DVRSIQQLVA TKVRQSQETR GIWCEGISNF
651  FHKDSTKINK GFRHISAGYV VGATTTLASD NLITAAFCQL FGKDRDHFIN
701  KNRASAYAAS LHLQHLATLS SPSLLRYLPG SESEQPVLFD AQISYIYSKN
751  TMKTYYTQAP KGESSWYNDG CALELASSLP HTALSHEGLF HAYFPFIKVE
801  ASYIHQDSFK ERNTTLVRSF DSGDLINVSV PIGITFERFS RNERASYEAT
851  VIYVADVYRK NPDCTTALLI NNTSWKTTGT NLSRQAGIGR AGIFYAFSPN
901  LEVTSNLSME IRGSSRSYNA DLGGKFQF*
```

A predicted signal peptide is highlighted.

The cp6731 nucleotide sequence <SEQ ID 34> is:

```
   1  ATGAAATCCT CTCTTCATTG GTTTTTAATC TCGTCATCTT TAGCACTTCC
  51  CTTGTCACTA AATTTCTCTG CGTTTGCTGC TGTTGTTGAA ATCAATCTAG
 101  GACCTACCAA TAGCTTCTCT GGACCAGGAA CCTACACTCC TCCAGCCCAA
 151  ACAACAAATG CAGATGGAAC TATCTATAAT CTAACAGGGG ATGTCTCAAT
 201  CACCAATGCA GGATCTCCGA CAGCTCTAAC CGCTTCCTGC TTTAAAGAAA
```

```
 251  CTACTGGGAA TCTTTCTTTC CAAGGCCACG GCTACCAATT TCTCCTACAA
 301  AATATCGATG CGGGAGCGAA CTGTACCTTT ACCAATACAG CTGCAAATAA
 351  GCTTCTCTCC TTTTCAGGAT TCTCCTATTT GTCACTAATA CAAACCACGA
 401  ATGCTACCAC AGGAACAGGA GCCATCAAGT CCACAGGAGC TTGTTCTATT
 451  CAGTCGAACT ATAGTTGCTA CTTTGGCCAA AACTTTTCTA ATGACAATGG
 501  AGGCGCCCTC CAAGGCAGCT CTATCAGTCT ATCGCTAAAC CCCAACCTAA
 551  CGTTTGCCAA AAACAAAGCA ACGCAAAAAG GGGGTGCCCT CTATTCCACG
 601  GGAGGGATTA CAATTAACAA TACGTTAAAC TCAGCATCAT TTTCTGAAAA
 651  TACCGCGGCG AACAATGGCG GAGCCATTTA CACGGAAGCT AGCAGTTTTA
 701  TTAGCAGCAA CAAAGCAATT AGCTTTATAA ACAATAGTGT GACCGCAACC
 751  TCAGCTACAG GGGGAGCCAT TTACTGTAGT AGTACATCAG CCCCCAAACC
 801  AGTCTTAACT CTATCAGACA ACGGGGAACT GAACTTTATA GGAAATACAG
 851  CAATTACTAG TGGTGGGGCG ATTTATACTG ACAATCTAGT TCTTTCTTCT
 901  GGAGGACCTA CGCTTTTTAA AAACAACTCT GCTATAGATA CTGCAGCTCC
 951  CTTAGGAGGA GCAATTGCGA TTGCTGACTC TGGATCTTTG AGTCTTTCGG
1001  CTCTTGGTGG AGACATCACT TTTGAAGGAA ACACAGTAGT CAAAGGAGCT
1051  TCTTCGAGTC AGACCACTAC CAGAAATTCT ATTAACATCG GAAACACCAA
1101  TGCTAAGATT GTACAGCTGC GAGCCTCTCA AGGCAATACT ATCTACTTCT
1151  ATGATCCTAT AACAACTAGC ATCACTGCAG CTCTCTCAGA TGCTCTAAAC
1201  TTAAATGGTC CTGACCTTGC AGGGAATCCT GCATATCAAG GAACCATCGT
1251  ATTTTCTGGA GAGAAGCTCT CGGAAGCAGA AGCTGCAGAA GCTGATAATC
1301  TCAAATCTAC AATTCAGCAA CCTCTAACTC TTGCGGGAGG GCAACTCTCT
1351  CTTAAATCAG GAGTCACTCT AGTTGCTAAG TCCTTTTCGC AATCTCCGGG
1401  CTCTACCCTC CTCATGGATG CAGGGACCAC ATTAGAAACC GCTGATGGGA
1451  TCACTATCAA TAATCTTGTT CTCAATGTAG ATTCCTTAAA AGAGACCAAG
1501  AAGGCTACGC TAAAAGCAAC ACAAGCAAGT CAGACAGTCA CTTTATCTGG
1551  ATCGCTCTCT CTTGTAGATC CTTCTGGAAA TGTCTACGAA GATGTCTCTT
1601  GGAATAACCC TCAAGTCTTT TCTTGTCTCA CTCTTACTGC TGACGACCCC
1651  GCGAATATTC ACATCACAGA CTTAGCTGCT GATCCCCTAG AAAAAAATCC
1701  TATCCATTGG GGATACCAAG GGAATTGGGC ATTATCTTGG CAAGAGGATA
1751  CTGCGACTAA ATCCAAAGCA GCGACTCTTA CCTGGACAAA AACAGGATAC
1801  AATCCGAATC CTGAGCGTCG TGGAACCTTA GTTGCTAACA CGCTATGGGG
1851  ATCCTTTGTT GATGTGCGCT CCATACAACA GCTTGTAGCC ACTAAAGTAC
1901  GCCAATCTCA AGAAACTCGC GGCATCTGGT GTGAAGGGAT CTCGAACTTC
1951  TTCCATAAAG ATAGCACGAA GATAAATAAA GGTTTTCGCC ACATAAGTGC
2001  AGGTTATGTT GTAGGAGCGA CTACAACATT AGCTTCTGAT AATCTTATCA
2051  CTGCAGCCTT CTGCCAATTA TTCGGGAAAG ATAGAGATCA CTTTATAAAT
2101  AAAAATAGAG CTTCTGCCTA TGCAGCTTCT CTCCATCTCC AGCATCTAGC
2151  GACCTTGTCT TCTCCAAGCT TGTTACGCTA CCTTCCTGGA TCTGAAAGTG
2201  AGCAGCCTGT CCTCTTTGAT GCTCAGATCA GCTATATCTA TAGTAAAAAT
2251  ACTATGAAAA CCTATTACAC CCAAGCACCA AAGGGAGAGA GCTCGTGGTA
2301  TAATGACGGT TGCGCTCTGG AACTTGCGAG CTCCCTACCA CACACTGCTT
2351  TAAGCCATGA GGGTCTCTTC CACGCGTATT TTCCTTTCAT CAAAGTAGAA
2401  GCTTCGTACA TACACCAAGA TAGCTTCAAA GAACGTAATA CTACCTTGGT
2451  ACGATCTTTC GATAGCGGTG ATTTAATTAA CGTCTCTGTG CCTATTGGAA
2501  TTACCTTCGA GAGATTCTCG AGAAACGAGC GTGCGTCTTA CGAAGCTACT
2551  GTCATCTACG TTGCCGATGT CTATCGTAAG AATCCTGACT GCACGACAGC
2601  TCTCCTAATC AACAATACCT CGTGGAAAAC TACAGGAACG AATCTCTCAA
2651  GACAAGCTGG TATCGGAAGA GCAGGGATCT TTTATGCCTT CTCTCCAAAT
2701  CTTGAGGTCA CAAGTAACCT ATCTATGGAA ATTCGTGGAT CTTCACGCAG
2751  CTACAATGCA GATCTTGGAG GTAAGTTCCA GTTCTAA
```

The PSORT algorithm predicts an outer membrane location (0.926).

The protein was expressed in *E.coli* and purified as a his-tag product, as shown in Figure 17A. A GST-fusion protein was also expressed. The recombinant proteins were used to immunise mice, whose sera were used in a Western blot (Figure 17B; his-tag) and for FACS analysis (Figure 17C; his-tag and GST-fusion).

The GST-fusion protein also showed good cross-reactivity with human sera, including sera from patients with pneumonitis. Less cross-reactivity was seen with the his-fusion.

These experiments show that cp6731 is a surface-exposed and immunoaccessible protein, and that it is a useful immunogen. These properties are not evident from the sequence alone.

**Example 18**

The following *C.pneumoniae* protein (PID 4376737) was expressed <SEQ ID 35; cp6737>:

```
  1 MPLSFKSSSF CLLACLCSAS CAFAETRLGG NFVPPITNQG EEILLTSDFV
 51 CSNFLGASFS SSFINSSSNL SLLGKGLSLT FTSCQAPTNS NYALLSAAET
101 LTFKNFSSIN FTGNQSTGLG GLIYGKDIVF QSIKDLIFTT NRVAYSPASV
151 TTSATPAITT VTTGASALQP TDSLTVENIS QSIKFFGNLA NFGSAISSSP
201 TAVVKFINNT ATMSFSHNFT SSGGGVIYGG SSLLFENNSG CIIFTANSCV
251 NSLKGVTPSS GTYALGSGGA ICIPTGTFEL KNNQGKCTFS YNGTPNDAGA
301 IYAETCNIVG NQGALLLDSN TAARNGGAIC AKVLNIQGRG PIEFSRNRAE
351 KGGAIFIGPS VGDPAKQTST LTILASEGDI AFQGNMLNTK PGIRNAITVE
401 AGGEIVSLSA QGGSRLVFYD PITHSLPTTS PSNKDITINA NGASGSVVFT
451 SKGLSSTELL LPANTTTILL GTVKIASGEL KITDNAVVNV LGFATQGSGQ
501 LTLGSGGTLG LATPTGAPAA VDFTIGKLAF DPFSFLKRDF VSASVNAGTK
551 NVTLTGALVL DEHDVTDLYD MVSLQTPVAI PIAVFKGATV TKTGFPDGEI
601 ATPSHYGYQG KWSYTWSRPL LIPAPDGGFP GGPSPSANTL YAVWNSDTLV
651 RSTYILDPER YGEIVSNSLW ISFLGNQAFS DILQDVLLID HPGLSITAKA
701 LGAYVEHTPR QGHEGFSGRY GGYQAALSMN YTDHTTLGLS FGQLYGKTNA
751 NPYDSRCSEQ MYLLSFFGQF PIVTQKSEAL ISWKAAYGYS KNHLNTTYLR
801 PDKAPKSQGQ WHNNSYYVLI SAEHPFLNWC LLTRPLAQAW DLSGFISAEF
851 LGGWQSKFTE TGDLQRSFSR GKGYNVSLPI GCSSQWFTPF KKAPSTLTIK
901 LAYKPDIYRV NPHNIVTVVS NQESTSISGA NLRRHGLFVQ IHDVVDLTED
951 TQAFLNYTFD GKNGFTNHRV STGLKSTF*
```

A predicted signal peptide is highlighted.

The cp6737 nucleotide sequence <SEQ ID 36> is:

```
   1  ATGCCTCTTT CTTTCAAATC TTCATCTTTT TGTCTACTTG CCTGTTTATG
  51  TAGTGCAAGT TGCGCGTTTG CTGAGACTAG ACTCGGAGGG AACTTTGTTC
 101  CTCCAATTAC GAATCAGGGT GAAGAGATCT TACTCACTTC AGATTTTGTT
 151  TGTTCAAACT TCTTGGGGGC GAGTTTTTCA AGTTCCTTTA TCAATAGTTC
 201  CAGCAATCTC TCCTTATTAG GGAAGGGCCT TTCCTTAACG TTTACCTCTT
 251  GTCAAGCTCC TACAAATAGT AACTATGCGC TACTTTCTGC CGCAGAGACT
 301  CTGACCTTCA AGAATTTTTC TTCTATAAAC TTTACAGGGA ACCAATCGAC
 351  AGGACTTGGC GGCCTCATCT ACGGAAAAGA TATTGTTTTC CAATCTATCA
 401  AAGATTTGAT CTTCACTACG AACCGTGTTG CCTATTCTCC AGCATCTGTA
 451  ACTACGTCGG CAACTCCCGC AATCACTACA GTAACTACAG GAGCCTCTGC
 501  TCTCCAACCT ACAGACTCAC TCACTGTCGA AAACATATCC CAATCGATCA
 551  AGTTTTTTGG GAACCTTGCC AACTTCGGCT CTGCAATTAG CAGTTCTCCC
 601  ACGGCAGTCG TTAAATTCAT CAATAACACC GCTACCATGA GCTTCTCCCA
 651  TAACTTTACT TCGTCAGGAG GCGGCGTGAT TTATGGAGGA AGCTCTCTCC
 701  TTTTTGAAAA CAATTCTGGA TGCATCATCT TCACCGCCAA CTCCTGTGTG
 751  AACAGCTTAA AAGGCGTCAC CCCTTCATCA GGAACCTATG CTTTAGGAAG
 801  TGGCGGAGCC ATCTGCATCC CTACGGGAAC TTTCGAATTA AAAAACAATC
 851  AGGGGAAGTG CACCTTCTCT TATAATGGTA CACCAAATGA TGCGGGTGCG
 901  ATCTACGCCG AAACCTGCAA CATCGTAGGG AACCAGGGTG CCTTGCTCCT
 951  AGATAGCAAC ACTGCAGCGA GAAATGGCGG AGCCATCTGT GCTAAAGTGC
1001  TCAATATTCA AGGACGCGGT CCTATTGAAT TCTCTAGAAA CCGCGCGGAG
1051  AAGGGTGGAG CTATTTTCAT AGGCCCCTCT GTTGGAGACC CTGCGAAGCA
1101  AACATCGACA CTTACGATTT TGGCTTCCGA AGGTGATATT GCGTTCCAAG
1151  GAAACATGCT CAATACAAAA CCTGGAATCC GCAATGCCAT CACTGTAGAA
1201  GCAGGGGGAG AGATTGTGTC TCTATCTGCA CAAGGAGGCT CACGTCTTGT
1251  ATTTTATGAT CCCATTACAC ATAGCCTCCC AACCACAAGT CCGTCTAATA
1301  AAGACATTAC AATCAACGCT AATGGCGCTT CAGGATCTGT AGTCTTTACA
1351  AGTAAGGGAC TCTCCTCTAC AGAACTCCTG TTGCCTGCCA ACACGACAAC
1401  TATACTTCTA GGAACAGTCA AGATCGCTAG TGGAGAACTG AAGATTACTG
1451  ACAATGCGGT TGTCAATGTT CTTGGCTTCG CTACTCAGGG CTCAGGTCAG
1501  CTTACCCTGG GCTCTGGAGG AACCTTAGGG CTGGCAACAC CCACGGGAGC
1551  ACCTGCCGCT GTAGACTTTA CGATTGGAAA GTTAGCATTC GATCCTTTTT
1601  CCTTCCTAAA AAGAGATTTT GTTTCAGCAT CAGTAAATGC AGGCACAAAA
1651  AACGTCACTT TAACAGGAGC TCTGGTTCTT GATGAACATG ACGTTACAGA
```

```
1701  TCTTTATGAT ATGGTGTCAT TACAAACTCC AGTAGCAATT CCTATCGCTG
1751  TTTTCAAAGG AGCAACCGTT ACTAAGACAG GATTTCCTGA TGGGGAGATT
1801  GCGACTCCAA GCCACTACGG CTACCAAGGA AAGTGGTCCT ACACATGGTC
1851  CCGTCCCCTG TTAATTCCAG CTCCTGATGG AGGATTTCCT GGAGGTCCCT
1901  CTCCTAGCGC AAATACTCTC TATGCTGTAT GGAATTCAGA CACTCTCGTG
1951  CGTTCTACCT ATATCTTAGA TCCCGAGCGT TACGGAGAAA TTGTCAGCAA
2001  CAGCTTATGG ATTTCCTTCT TAGGAAATCA GGCATTCTCT GATATTCTCC
2051  AAGATGTTCT TTTGATAGAT CATCCCGGGT TGTCCATAAC CGCGAAAGCT
2101  TTAGGAGCCT ATGTCGAACA CACACCAAGA CAAGGACATG AGGGCTTTTC
2151  AGGTCGCTAT GGAGGCTACC AAGCTGCGCT ATCTATGAAC TACACGGACC
2201  ACACTACGTT AGGACTTTCT TTCGGGCAGC TTTATGGAAA AACTAACGCC
2251  AACCCCTACG ATTCACGTTG CTCAGAACAA ATGTATTTAC TCTCGTTCTT
2301  TGGTCAATTC CCTATCGTGA CTCAAAAGAG CGAGGCCTTA ATTTCCTGGA
2351  AAGCAGCTTA TGGTTATTCC AAAAATCACC TAAATACCAC CTACCTCAGA
2401  CCTGACAAAG CTCCAAAATC TCAAGGGCAA TGGCATAACA ATAGTTACTA
2451  TGTTCTTATT TCTGCAGAAC ATCCTTTCCT AAACTGGTGT CTTCTTACAA
2501  GACCTCTGGC TCAAGCTTGG GATCTTTCAG GTTTTATTTC CGCAGAATTC
2551  CTAGGTGGTT GGCAAAGTAA GTTCACAGAA ACTGGAGATC TGCAACGTAG
2601  CTTTAGTAGA GGTAAAGGGT ACAATGTTTC CCTACCGATA GGATGTTCTT
2651  CTCAATGGTT CACACCATTT AAGAAGGCTC CTTCTACACT GACCATCAAA
2701  CTTGCCTACA AGCCTGATAT CTATCGTGTC AACCCTCACA ATATTGTGAC
2751  TGTCGTCTCA AACCAAGAGA GCACTTCGAT CTCAGGAGCA AATCTACGCC
2801  GCCACGGTTT GTTTGTACAA ATCCATGATG TAGTAGATCT CACCGAGGAC
2851  ACTCAGGCCT TTCTAAACTA TACCTTTGAC GGGAAAAATG GATTTACAAA
2901  CCACCGAGTG TCTACAGGAC TAAAATCCAC ATTTTAA
```

The PSORT algorithm predicts an outer membrane location (0.940).

The protein was expressed in *E.coli* and purified as a GST-fusion product, as shown in Figure 18A. The recombinant protein was used to immunise mice, whose sera were used in an immunoblot analysis blot (Figure 18B) and for FACS analysis (Figure 18C). A his-tagged protein was also expressed.

The cp6737 protein was also identified in the 2D-PAGE experiment (Cpn0454) and showed good cross-reactivity with human sera, including sera from patients with pneumonitis.

These experiments show that cp6737 is a surface-exposed and immunoaccessible protein, and that it is a useful immunogen. These properties are not evident from the sequence alone.

**Example 19**

The following *C.pneumoniae* protein (PID 4377090) was expressed <SEQ ID 37; cp7090>:

```
  1 MNIHSLWKLC TLLALLALPA CSLSPNYGWE DSCNTCHHTR RKKPSSFGFV
 51 PLYTEEDFNP NFTFGEYDSK EEKQYKSSQV AAFRNITFAT DSYTIKGEEN
101 LAILTNLVHY MKKNPKATLY IEGHTDERGA ASYNLALGAR RANAIKEHLR
151 KQGISADRLS TISYGKEHPL NSGHNELAWQ QNRRTEFKIH AR*
```

A predicted signal peptide is highlighted.

The cp7090 nucleotide sequence <SEQ ID 38> is:

```
  1 ATGAATATAC ATTCCCTATG GAAACTTTGT ACTTTATTGG CTTTACTTGC
 51 ATTGCCAGCA TGTAGCCTTT CCCCTAATTA TGGCTGGGAG GATTCCTGTA
101 ATACATGCCA TCATACAAGA CGAAAAAAGC CTTCTTCTTT TGGCTTTGTT
151 CCTCTCTATA CCGAAGAGGA CTTTAACCCT AATTTTACCT TCGGTGAGTA
201 TGATTCCAAA GAAGAAAAAC AATACAAGTC AAGCCAAGTT GCAGCATTTC
251 GTAATATCAC CTTTGCTACA GACAGCTATA CAATTAAAGG TGAAGAGAAC
301 CTTGCGATTC TCACGAACTT GGTTCACTAC ATGAAGAAAA ACCCGAAAGC
351 TACACTGTAC ATTGAAGGGC ATACTGACGA GCGTGGAGCT GCATCCTATA
401 ACCTTGCTTT AGGAGCACGA CGAGCCAATG CGATTAAAGA GCATCTCCGA
451 AAGCAGGGAA TCTCTGCAGA TCGTCTATCT ACTATTTCCT ACGGAAAAGA

501 ACATCCTTTA AATTCGGGAC ACAACGAACT AGCATGGCAA CAAAATCGCC
551 GTACAGAGTT TAAGATTCAT GCACGCTAA
```

The PSORT algorithm predicts an outer membrane location (0.790).

The protein was expressed in *E.coli* and purified as a GST-fusion product, as shown in Figure 19A. A his-tagged protein was also expressed. The recombinant proteins were used to immunise mice, whose sera were used in a Western blot (Figure 19B) and for FACS analysis.

These experiments show that cp7090 is useful immunogen. These properties are not evident from the sequence alone.

**Example 20**

The following *C.pneumoniae* protein (PID 4377091) was expressed <SEQ ID 39; cp7091>:

```
  1 MLRQLCFQVF FFCFASLVYA EELEVVVRSE HITLPIEVSC QTDTKDPKIQ
 51 KYLSSLTEIF CKDIALGDCL QPTAASKESS SPLAISLRLH VPQLSVVLLQ
101 SSKTPQTLCS FTISQNLSVD RQKIHHAADT VHYALTGIPG ISAGKIVFAL
151 SSLGKDQKLK QGELWTTDYD GKNLAPLTTE CSLSITPKWV GVGSNFPYLY
201 VSYKYGVPKI FLGSLENTEG KKVLPLKGNQ LMPTFSPRKK LLAFVADTYG
251 NPDLFIQPFS LTSGPMGRPR RLLNENFGTQ GNPSFNPEGS QLVFISNKDG
301 RPRLYIMSLD PEPQAPRLLT KKYRNSSCPA WSPDGKKIAF CSVIKGVRQI
351 CIYDLSSGED YQLTTSPTNK ESPSWAIDSR HLVFSAGNAE ESELYLISLV
401 TKKTNKIAIG VGEKRFPSWG AFPQQPIKRT L*
```

A predicted signal peptide is highlighted.

The cp7091 nucleotide sequence <SEQ ID 40> is:

```
   1 ATGTTACGGC AACTATGCTT CCAAGTTTTT TTCTTTTGCT TCGCATCGCT
  51 AGTCTATGCT GAAGAATTAG AAGTTGTTGT CCGTTCCGAA CATATCACGC
 101 TCCCTATTGA GGTCTCTTGC CAGACCGATA CGAAAGATCC AAAAATACAG
 151 AAATACCTCA GCTCGCTAAC GGAGATATTT TGCAAGGACA TTGCCCTAGG
 201 AGATTGTCTA CAACCCACAG CGGCTTCTAA AGAATCGTCA TCTCCTTTAG
 251 CAATATCTTT ACGGTTGCAT GTACCTCAGC TATCTGTAGT GCTTTTACAG
 301 TCTTCAAAAA CTCCTCAAAC CTTATGTTCT TTTACTATTT CTCAAAATCT
 351 TTCTGTAGAT CGTCAAAAAA TCCATCACGC TGCTGATACA GTTCATTACG
 401 CCCTCACAGG GATTCCTGGA ATCAGTGCTG GGAAAATTGT TTTTGCTCTA
 451 AGTTCTTTAG GAAAAGATCA AAAGCTCAAG CAAGGAGAAT TATGGACTAC
 501 AGATTACGAT GGGAAAAACC TCGCCCCTTT AACCACAGAA TGTTCGCTCT
 551 CTATAACTCC AAAATGGGTG GGTGTGGGAT CAAATTTTCC CTATCTCTAT
 601 GTTTCGTATA AGTATGGTGT GCCTAAAATT TTTCTTGGTT CCCTAGAGAA
 651 CACTGAAGGT AAAAAAGTCC TTCCGTTAAA AGGCAACCAA CTCATGCCTA
 701 CGTTTTCTCC AAGAAAAAAG CTTTTAGCTT TCGTTGCTGA TACGTATGGA
 751 AATCCTGATT TATTTATTCA ACCGTTCTCA CTAACTTCAG GACCTATGGG
 801 TCGCCCACGT CGCCTCCTTA ATGAGAATTT CGGGACTCAA GGGAATCCCT
 851 CCTTCAACCC TGAAGGATCC CAGCTTGTCT TTATATCGAA CAAAGACGGC
 901 CGTCCGCGTC TTTATATTAT GTCCCTCGAT CCTGAACCCC AAGCACCTCG
 951 CTTGCTGACA AAAAAATACA GAAATAGCAG TTGCCCTGCA TGGTCTCCAG
1001 ATGGTAAAAA AATAGCCTTC TGCTCTGTAA TTAAAGGGGT GCGACAAATT
1051 TGTATTTACG ATCTCTCCTC TGGAGAGGAT TACCAACTCA CTACGTCTCC
1101 CACAAATAAA GAGAGTCCTT CTTGGGCTAT AGACAGCCGT CATCTTGTCT
1151 TTAGTGCGGG GAATGCTGAA GAATCAGAGT TATATTTAAT CAGTCTAGTC
1201 ACCAAAAAAA CTAACAAAAT TGCTATAGGA GTAGGAGAAA AACGGTTCCC
1251 CTCCTGGGGT GCTTTCCCTC AGCAACCGAT AAAGAGAACA CTATGA
```

The PSORT algorithm predicts an inner membrane location (0.109).

The protein was expressed in *E.coli* and purified as a GST-fusion product, as shown in Figure 20A. A his-tagged protein was also expressed. The recombinant proteins were used to immunise mice, whose sera were used in a Western blot (Figure 20B) and for FACS analysis.

These experiments show that cp7091 is a useful immunogen. These properties are not evident from the sequence alone.

## Example 21

The following *C.pneumoniae* protein (PID 4376260) was expressed <SEQ ID 41; cp6260>:

```
  1 MRFSLCGFPL VFSFTLLSVF DTSLSATTIS LTPEDSFHGD SQNAERSYNV
 51 QAGDVYSLTG DVSISNVDNS ALNKACFNVT SGSVTFAGNH HGLYFNNISS
101 GTTKEGAVLC CQDPQATARF SGFSTLSFIQ SPGDIKEQGC LYSKNALMLL
151 NNYVVRFEQN QSKTKGGAIS GANVTIVGNY DSVSFYQNAA TFGGAIHSSG
201 PLQIAVNQAE IRFAQNTAKN GSGGALYSDG DIDIDQNAYV LFRENEALTT
251 AIGKGGAVCC LPTSGSSTPV PIVTFSDNKQ LVFERNHSIM GGGAIYARKL
301 SISSGGPTLF INNISYANSQ NLGGAIAIDT GGEISLSAEK GTITFQGNRT
351 SLPFLNGIHL LQNAKFLKLQ ARNGYSIEFY DPITSEADGS TQLNINGDPK
401 NKEYTGTILF SGEKSLANDP RDFKSTIPQN VNLSAGYLVI KEGAEVTVSK
451 FTQSPGSHLV LDLGTKLIAS KEDIAITGLA IDIDSLSSSS TAAVIKANTA
501 NKQISVTDSI ELISPTGNAY EDLRMRNSQT FPLLSLEPGA GGSVTVTAGD
551 FLPVSPHYGF QGNWKLAWTG TGNKVGEFFW DKINYKPRPE KEGNLVPNIL
601 WGNAVDVRSL MQVQETHASS LQTDRGLWID GIGNFFHVSA SEDNIRYRHN
651 SGGYVLSVNN EITPKHYTSM AFSQLFSRDK DYAVSNNEYR MYLGSYLYQY
701 TTSLGNIFRY ASRNPNVNVG ILSRRFLQNP LMIFHFLCAY GHATNDMKTD
751 YANFPMVKNS WRNNCWAIEC GGSMPLLVFE NGRLFQGAIP FMKLQLVYAY
801 QGDFKETTAD GRRFSNGSLT SISVPLGIRF EKLALSQDVL YDFSFSYIPD
851 IFRKDPSCEA ALVISGDSWL VPAAHVSRHA FVGSGTGRYH FNDYTELLCR
901 GSIECRPHAR NYNINCGSKF RF*
```

A predicted signal peptide is highlighted.

The cp6260 nucleotide sequence <SEQ ID 42> is:

```
   1  ATGCGATTTT CGCTCTGCGG ATTTCCTCTA GTTTTTTCTT TTACATTGCT
  51  CTCAGTCTTC GACACTTCTT TGAGTGCTAC TACGATTTCT TTAACCCCAG
 101  AAGATAGTTT TCATGGAGAT AGTCAGAATG CAGAACGTTC TTATAATGTT
 151  CAAGCTGGGG ATGTCTATAG CCTTACTGGT GATGTCTCAA TATCTAACGT
 201  CGATAACTCT GCATTAAATA AAGCCTGCTT CAATGTGACC TCAGGAAGTG
 251  TGACGTTCGC AGGAAATCAT CATGGGTTAT ATTTTAATAA TATTTCCTCA
 301  GGAACTACAA AGGAAGGGGC TGTACTTTGT TGCCAAGATC CTCAAGCAAC
 351  GGCACGTTTT TCTGGGTTCT CCACGCTCTC TTTTATTCAG AGCCCCGGAG
 401  ATATTAAAGA ACAGGGATGT CTCTATTCAA AAAATGCACT TATGCTCTTA
 451  AACAATTATG TAGTGCGTTT TGAACAAAAC CAAAGTAAGA CTAAAGGCGG
 501  AGCTATTAGT GGGGCGAATG TTACTATAGT AGGCAACTAC GATTCCGTCT
 551  CTTTCTATCA GAATGCAGCC ACTTTTGGAG GTGCTATCCA TTCTTCAGGT
 601  CCCCTACAGA TTGCAGTAAA TCAGGCAGAG ATAAGATTTG CACAAAATAC
 651  TGCCAAGAAT GGTTCTGGAG GGGCTTTGTA CTCCGATGGT GATATTGATA
 701  TTGATCAGAA TGCTTATGTT CTATTTCGAG AAAATGAGGC ATTGACTACT
 751  GCTATAGGTA AGGGAGGGGC TGTCTGTTGT CTTCCCACTT CAGGAAGTAG
 801  TACTCCAGTT CCTATTGTGA CTTTCTCTGA CAATAAACAG TTAGTCTTTG
 851  AAAGAAACCA TTCCATAATG GGTGGCGGAG CCATTTATGC TAGGAAACTT
 901  AGCATCTCTT CAGGAGGTCC TACTCTATTT ATCAATAATA TATCATATGC
 951  AAATTCGCAA AATTTAGGTG GAGCTATTGC CATTGATACT GGAGGGGAGA
1001  TCAGTTTATC AGCAGAGAAA GGAACAATTA CATTCCAAGG AAACCGGACG
1051  AGCTTACCGT TTTTGAATGG CATCCATCTT TTACAAAATG CTAAATTCCT
1101  GAAATTACAG GCGAGAAATG GATACTCTAT AGAATTTTAT GATCCTATTA
1151  CTTCTGAAGC AGATGGGTCT ACCCAATTGA ATATCAACGG AGATCCTAAA
1201  AATAAAGAGT ACACAGGGAC CATACTCTTT TCTGGAGAAA AGAGTCTAGC
1251  AAACGATCCT AGGGATTTTA AATCTACAAT CCCTCAGAAC GTCAACCTGT
1301  CTGCAGGATA CTTAGTTATT AAAGAGGGGG CCGAAGTCAC AGTTTCAAAA
1351  TTCACGCAGT CTCCAGGATC GCATTTAGTT TTAGATTTAG GAACCAAACT
1401  GATAGCCTCT AAGGAAGACA TTGCCATCAC AGGCCTCGCG ATAGATATAG
1451  ATAGCTTAAG CTCATCCTCA ACAGCAGCTG TTATTAAAGC AAACACCGCA
1501  AATAAACAGA TATCCGTGAC GGACTCTATA GAACTTATCT CGCCTACTGG
1551  CAATGCCTAT GAAGATCTCA GAATGAGAAA TTCACAGACG TTCCCTCTGC
1601  TCTCTTTAGA GCCTGGAGCC GGGGGTAGTG TGACTGTAAC TGCTGGAGAT
1651  TTCCTACCGG TAAGTCCCCA TTATGGTTTT CAAGGCAATT GGAAATTAGC
1701  TTGGACAGGA ACTGGAAACA AAGTTGGAGA ATTCTTCTGG GATAAAATAA

1751  ATTATAAGCC TAGACCTGAA AAAGAAGGAA ATTTAGTTCC TAATATCTTG
1801  TGGGGGAATG CTGTAGATGT CAGATCCTTA ATGCAGGTTC AAGAGACCCA
1851  TGCATCGAGC TTACAGACAG ATCGAGGGCT GTGGATCGAT GGAATTGGGA
1901  ATTTCTTCCA TGTATCTGCC TCCGAAGACA ATATAAGGTA CCGTCATAAC
1951  AGCGGTGGAT ATGTTCTATC TGTAAATAAT GAGATCACAC CTAAGCACTA
2001  TACTTCGATG GCATTTTCCC AACTCTTTAG TAGAGACAAG GACTATGCGG
2051  TTTCCAACAA CGAATACAGA ATGTATTTAG GATCGTATCT CTATCAATAT
2101  ACAACCTCCC TAGGGAATAT TTTCCGTTAT GCTTCGCGTA ACCCTAATGT
2151  AAACGTCGGG ATTCTCTCAA GAAGGTTTCT TCAAAATCCT CTTATGATTT
2201  TTCATTTTTT GTGTGCTTAT GGTCATGCCA CCAATGATAT GAAAACAGAC
2251  TACGCAAATT TCCCTATGGT GAAAAACAGC TGGAGAAACA ATTGTTGGGC
2301  TATAGAGTGC GGAGGGAGCA TGCCTCTATT GGTATTTGAG AACGGAAGAC
2351  TTTTCCAAGG TGCCATCCCA TTTATGAAAC TACAATTAGT TTATGCTTAT
2401  CAGGGAGATT TCAAAGAGAC GACTGCAGAT GGCCGTAGAT TTAGTAATGG
2451  GAGTTTAACA TCGATTTCTG TACCTCTAGG CATACGCTTT GAGAAGCTGG
2501  CACTTTCTCA GGATGTACTC TATGACTTTA GTTTCTCCTA TATTCCTGAT
2551  ATTTTCCGTA AGGATCCCTC ATGTGAAGCT GCTCTGGTGA TTAGCGGAGA
2601  CTCCTGGCTT GTTCCGGCAG CACACGTATC AAGACATGCT TTTGTAGGGA
2651  GTGGAACGGG TCGGTATCAC TTTAACGACT ATACTGAGCT CTTATGTCGA
2701  GGAAGTATAG AATGCCGCCC CCATGCTAGG AATTATAATA TAAACTGTGG
2751  AAGCAAATTT CGTTTTTAG
```

The PSORT algorithm predicts an outer membrane location (0.921).

The protein was expressed in *E.coli* and purified both as a his-tag and GST-fusion product. The GST-fusion is shown in Figure 21A. This recombinant protein was used to immunise mice, whose sera were used in a Western blot (Figure 21B) and for FACS analysis (Figure 21C).

This protein also showed good cross-reactivity with human sera, including sera from patients with pneumonitis.

These experiments show that cp6260 is a surface-exposed and immunoaccessible protein, and that it is a useful immunogen. These properties are not evident from the sequence alone.

**Example 22**

The following *C.pneumoniae* protein (PID 4376456) was expressed <SEQ ID 43; cp6456>:

```
  1  MSSPVNNTPS APNIPIPAPT TPGIPTTKPR SSFIEKVIIV AKYILFAIAA
 51  TSGALGTILG LSGALTPGIG IALLVIFFVS MVLLGLILKD SISGGEERRL
101  REEVSRFTSE NQRLTVITTT LETEVKDLKA AKDQLTLEIE AFRNENGNLK
151  TTAEDLEEQV SKLSEQLEAL ERINQLIQAN AGDAQEISSE LKKLISGWDS
201  KVVEQINTSI QALKVLLGQE WVQEAQTHVK AMQEQIQALQ AEILGMHNQS
251  TALQKSVENL LVQDQALTRV VGELLESENK LSQACSALRQ EIEKLAQHET
301  SLQQRIDAML AQEQNLAEQV TALEKMKQEA QKAESEFIAC VRDRTFGRRE
351  TPPPTTPVVE GDESQEEDEG GTPPVSQPSS PVDRATGDGQ *
```

The cp6456 nucleotide sequence <SEQ ID 44> is:

```
   1  ATGTCATCTC CTGTAAATAA CACACCCTCA GCACCAAACA TTCCAATACC
  51  AGCGCCCACG ACTCCAGGTA TTCCTACAAC AAAACCTCGT TCTAGTTTCA
 101  TTGAAAAGGT TATCATTGTA GCTAAGTACA TACTATTTGC AATTGCAGCC
 151  ACATCAGGAG CACTCGGAAC AATTCTAGGT CTATCTGGAG CGCTAACCCC
 201  AGGAATAGGT ATTGCCCTTC TTGTTATCTT CTTTGTTTCT ATGGTGCTTT
 251  TAGGTTTAAT CCTTAAAGAT TCTATAAGTG GAGGAGAAGA ACGCAGGCTC
 301  AGAGAAGAGG TCTCTCGATT TACAAGTGAG AATCAACGGT TGACAGTCAT
 351  AACCACAACA CTTGAGACTG AAGTAAAGGA TTTAAAAGCA GCTAAAGATC
 401  AACTTACACT TGAAATCGAA GCATTTAGAA ATGAAAACGG TAATTTAAAA
 451  ACAACTGCTG AGGACTTAGA AGAGCAGGTT TCTAAACTTA GCGAACAATT
 501  AGAAGCACTA GAGCGAATTA ATCAACTTAT CCAAGCAAAC GCTGGAGATG
 551  CTCAAGAAAT TTCGTCTGAA CTAAAGAAAT TAATAAGCGG TTGGGATTCC
 601  AAAGTTGTTG AACAGATAAA TACTTCTATT CAAGCATTGA AAGTGTTATT
 651  GGGTCAAGAG TGGGTGCAAG AGGCTCAAAC ACACGTTAAA GCAATGCAAG
 701  AGCAAATTCA AGCATTGCAA GCTGAAATTC TAGGAATGCA CAATCAATCT

 751  ACAGCATTGC AAAAGTCAGT TGAGAATCTA TTAGTACAAG ATCAAGCTCT
 801  AACAAGAGTA GTAGGTGAGT TGTTAGAGTC TGAGAACAAG CTAAGCCAAG
 851  CTTGTTCTGC GCTACGTCAA GAAATAGAAA AGTTGGCCCA ACATGAAACA
 901  TCTTTGCAAC AACGTATTGA TGCGATGCTA GCCCAAGAGC AAAATTTGGC
 951  AGAGCAGGTC ACAGCCCTTG AAAAAATGAA ACAAGAAGCT CAGAAGGCTG
1001  AGTCCGAGTT CATTGCTTGT GTACGTGATC GAACTTTCGG ACGTCGTGAA
1051  ACACCTCCAC CAACAACACC TGTAGTTGAA GGTGATGAAA GTCAAGAAGA
1101  AGACGAAGGA GGTACTCCCC CAGTATCACA ACCATCTTCA CCCGTAGATA
1151  GAGCAACAGG AGATGGTCAG TAA
```

The PSORT algorithm predicts inner membrane (0.127).

The protein was expressed in *E.coli* and purified as a GST-fusion product, as shown in Figure 22A. The recombinant protein was used to immunise mice, whose sera were used in a Western blot (Figure 22B) and for FACS analysis (Figure 22C). A his-tag protein was also expressed.

These experiments show that cp6456 is a surface-exposed and immunoaccessible protein, and that it is a useful immunogen. These properties are not evident from the sequence alone.

**Example 23**

The following *C.pneumoniae* protein (PID 4376729) was expressed <SEQ ID 45; cp6729>:

```
  1 MKIPLHKLLI SSTLVTPILL SIATYGADAS LSPTDSFDGA GGSTFTPKST
 51 ADANGTNYVL SGNVYINDAG KGTALTGCCF TETTGDLTFT GKGYSFSFNT
101 VDAGSNAGAA ASTTADKALT FTGFSNLSFI AAPGTTVASG KSTLSSAGAL
151 NLTDNGTILF SQNVSNEANN NGGAITTKTL SISGNTSSIT FTSNSAKKLG
201 GAIYSSAAAS ISGNTGQLVF MNNKGETGGG ALGFEASSSI TQNSSLFFSG
251 NTATDAAGKG GAIYCEKTGE TPTLTISGNK SLTFAENSSV TQGGAICAHG
301 LDLSAAGPTL FSNNRCGNTA AGKGGAIAIA DSGSLSLSAN QGDITFLGNT
351 LTSTSAPTST RNAIYLGSSA KITNLRAAQG QSIYFYDPIA SNTTGASDVL
401 TINQPDSNSP LDYSGTIVFS GEKLSADEAK AADNFTSILK QPLALASGTL
451 ALKGNVELDV NGFTQTEGST LLMQPGTKLK ADTEAISLTK LVVDLSALEG
501 NKSVSIETAG ANKTITLTSP LVFQDSSGNF YESHTINQAF TQPLVVFTAA
551 TAASDIYIDA LLTSPVQTPE PHYGYQGHWE ATWADTSTAK SGTMTWVTTG
601 YNPNPERRAS VVPDSLWASF TDIRTLQQIM TSQANSIYQQ RGLWASGTAN
651 FFHKDKSGTN QAFRHKSYGY IVGGSAEDFS ENIFSVAFCQ LFGKDKDLFI
701 VENTSHNYLA SLYLQHRAFL GGLPMPSFGS ITDMLKDIPL ILNAQLSYSY
751 TKNDMDTRYT SYPEAQGSWT NNSGALELGG SLALYLPKEA PFFQGYFPFL
801 KFQAVYSRQQ NFKESGAEAR AFDDGDLVNC SIPVGIRLEK ISEDEKNNFE
851 ISLAYIGDVY RKNPRSRTSL MVSGASWTSL CKNLARQAFL ASAGSHLTLS
901 PHVELSGEAA YELRGSAHIY NVDCGLRYSF *
```

A predicted signal peptide is highlighted.

The cp6729 nucleotide sequence <SEQ ID 46> is:

```
  1 ATGAAAATAC CCTTGCACAA ACTCCTGATC TCTTCGACTC TTGTCACTCC
 51 CATTCTATTG AGCATTGCAA CTTACGGAGC AGATGCTTCT TTATCCCCTA
101 CAGATAGCTT TGATGGAGCG GGCGGCTCTA CATTTACTCC AAAATCTACA
151 GCAGATGCCA ATGGAACGAA CTATGTCTTA TCAGGAAATG TCTATATAAA
201 CGATGCTGGG AAAGGCACAG CATTAACAGG CTGCTGCTTT ACAGAAACTA
251 CGGGTGATCT GACATTTACT GGAAAGGGAT ACTCATTTTC ATTCAACACG
301 GTAGATGCGG GTTCGAATGC AGGAGCTGCG GCAAGCACAA CTGCTGATAA
351 AGCCCTAACA TTCACAGGAT TTTCTAACCT TTCCTTCATT GCAGCTCCTG
401 GAACTACAGT TGCTTCAGGA AAAAGTACTT TAAGTTCTGC AGGAGCCTTA
451 AATCTTACCG ATAATGGAAC GATTCTCTTT AGCCAAAACG TCTCCAATGA
501 AGCTAATAAC AATGGCGGAG CGATCACCAC AAAAACTCTT TCTATTTCTG
551 GGAATACCTC TTCTATAACC TTCACTAGTA ATAGCGCAAA AAAATTAGGT
601 GGAGCGATCT ATAGCTCTGC GGCTGCAAGT ATTTCAGGAA ACACCGGCCA
651 GTTAGTCTTT ATGAATAATA AAGGAGAAAC TGGGGGTGGG GCTCTGGGCT
701 TTGAAGCCAG CTCCTCGATT ACTCAAAATA GCTCCCTTTT CTTCTCTGGA
751 AACACTGCAA CAGATGCTGC AGGCAAGGGC GGGGCCATTT ATTGTGAAAA
801 AACAGGAGAG ACTCCTACTC TTACTATCTC TGGAAATAAA AGTCTGACCT
851 TCGCCGAGAA CTCTTCAGTA ACTCAAGGCG GAGCAATCTG TGCCCATGGT
```

```
 901  CTAGATCTTT CCGCTGCTGG CCCTACCCTA TTTTCAAATA ATAGATGCGG
 951  GAACACAGCT GCAGGCAAGG GCGGCGCTAT TGCAATTGCC GACTCTGGAT
1001  CTTTAAGTCT CTCTGCAAAT CAAGGAGACA TCACGTTCCT TGGCAACACT
1051  CTAACCTCAA CCTCCGCGCC AACATCGACA CGGAATGCTA TCTACCTGGG
1101  ATCGTCAGCA AAAATTACGA ACTTAAGGGC AGCCCAAGGC CAATCTATCT
1151  ATTTCTATGA TCCGATTGCA TCTAACACCA CAGGAGCTTC AGACGTTCTG
1201  ACCATCAACC AACCGGATAG CAACTCGCCT TTAGATTATT CAGGAACGAT
1251  TGTATTTTCT GGGGAAAAGC TCTCTGCAGA TGAAGCGAAA GCTGCTGATA
1301  ACTTCACATC TATATTAAAG CAACCATTGG CTCTAGCCTC TGGAACCTTA
1351  GCACTCAAAG GAAATGTCGA GTTAGATGTC AATGGTTTCA CACAGACTGA
1401  AGGCTCTACA CTCCTCATGC AACCAGGAAC AAAGCTCAAA GCAGATACTG
1451  AAGCTATCAG TCTTACCAAA CTTGTCGTTG ATCTTTCTGC CTTAGAGGGA
1501  AATAAGAGTG TGTCCATTGA AACAGCAGGA GCCAACAAAA CTATAACTCT
1551  AACCTCTCCT CTTGTTTTCC AAGATAGTAG CGGCAATTTT TATGAAAGCC
1601  ATACGATAAA CCAAGCCTTC ACGCAGCCTT TGGTGGTATT CACTGCTGCT
1651  ACTGCTGCTA GCGATATTTA TATCGATGCG CTTCTCACTT CTCCAGTACA
1701  AACTCCAGAA CCTCATTACG GGTATCAGGG ACATTGGGAA GCCACTTGGG
1751  CAGACACATC AACTGCAAAA TCAGGAACTA TGACTTGGGT AACTACGGGC
1801  TACAACCCTA ATCCTGAGCG TAGAGCTTCC GTAGTTCCCG ATTCATTATG
1851  GGCATCCTTT ACTGACATTC GCACTCTACA GCAGATCATG ACATCTCAAG
1901  CGAATAGTAT CTATCAGCAA CGAGGACTCT GGGCATCAGG AACTGCGAAT
1951  TTCTTCCATA AGGATAAATC AGGAACTAAC CAAGCATTCC GACATAAAAG
2001  CTACGGCTAT ATTGTTGGAG GAAGTGCTGA AGATTTTTCT GAAAATATCT
2051  TCAGTGTAGC TTTCTGCCAG CTCTTCGGTA AAGATAAAGA CCTGTTTATA
2101  GTTGAAAATA CCTCTCATAA CTATTTAGCG TCGCTATACC TGCAACATCG
2151  AGCATTCCTA GGAGGACTTC CCATGCCCTC ATTTGGAAGT ATCACCGACA
2201  TGCTGAAAGA TATTCCTCTC ATTTTGAATG CCCAGCTAAG CTACAGCTAC
2251  ACTAAAAATG ATATGGATAC TCGCTATACT TCCTATCCTG AAGCTCAAGG
2301  CTCTTGGACC AATAACTCTG GGGCTCTAGA GCTCGGAGGA TCTCTGGCTC
2351  TATATCTCCC TAAAGAAGCA CCGTTCTTCC AGGGATATTT CCCCTTCTTA
2401  AAGTTCCAGG CAGTCTACAG CCGCCAACAA AACTTTAAAG AGAGTGGCGC
2451  TGAAGCCCGT GCTTTTGATG ATGGAGACCT AGTGAACTGC TCTATCCCTG
2501  TCGGCATTCG GTTAGAAAAA ATCTCCGAAG ATGAAAAAAA TAATTTCGAG
2551  ATTTCTCTAG CCTACATTGG TGATGTGTAT CGTAAAAATC CCCGTTCGCG
2601  TACTTCTCTA ATGGTCAGTG GAGCCTCTTG GACTTCGCTA TGTAAAAACC
2651  TCGCACGACA AGCCTTCTTA GCAAGTGCTG GAAGCCATCT GACTCTCTCC
2701  CCTCATGTAG AACTCTCTGG GGAAGCTGCT TATGAGCTTC GTGGCTCAGC
2751  ACACATCTAC AATGTAGATT GTGGGCTAAG ATACTCATTC TAG
```

The PSORT algorithm predicts outer membrane (0.927).

The protein was expressed in *E.coli* and purified as a GST-fusion product, as shown in Figure 23A. The recombinant protein was used to immunise mice, whose sera were used in a Western blot (Figure 23B) and for FACS analysis (Figure 23C). A his-tag protein was also expressed.

The cp6729 protein was also identified in the 2D-PAGE experiment (Cpn0446) and showed good cross-reactivity with human sera, including sera from patients with pneumonitis.

These experiments show that cp6729 is a surface-exposed and immunoaccessible protein, and that it is a useful immunogen. These properties are not evident from the sequence alone.

## Example 24

The following *C.pneumoniae* protein (PID 4376849) was expressed <SEQ ID 47; cp6849>:

```
  1  MSKLIRRVVT VLALTSMASC FASGGIEAAV AESLITKIVA SAETKPAPVP
 51  MTAKKVRLVR RNKQPVEQKS RGAFCDKEFY PCEEGRCQPV EAQQESCYGR
101  LYSVKVNDDC NVEICQSVPE YATVGSPYPI EILAIGKKDC VDVVITQQLP
151  CEAEFVSSDP ETTPTSDGKL VWKIDRLGAG DKCKITVWVK PLKEGCCFTA
201  ATVCACPELR SYTKCGQPAI CIKQEGPDCA CLRCPVCYKI EVVNTGSAIA
251  RNVTVDNPVP DGYSHASGQR VLSFNLGDMR PGDKKVFTVE FCPQRRGQIT
301  NVATVTYCGG HKCSANVTTV VNEPCVQVNI SGADWSYVCK PVEYSISVSN
351  PGDLVLHDVV IQDTLPSGVT VLEAPGGEIC CNKVVWRIKE MCPGETLQFK
```

```
401  LVVKAQVPGR FTNQVAVTSE SNCGTCTSCA ETTTHWKGLA ATHMCVLDTN
451  DPICVGENTV YRICVTNRGS AEDTNVSLIL KFSKELQPIA SSGPTKGTIS
501  GNTVVFDALP KLGSKESVEF SVTLKGIAPG DARGEAILSS DTLTSPVSDT
551  ENTHVY*
```

A predicted signal peptide is highlighted.

The cp6849 nucleotide sequence <SEQ ID 48> is:

```
   1  ATGTCCAAAC TCATCAGACG AGTAGTTACG GTCCTTGCGC TAACGAGTAT
  51  GGCGAGTTGC TTTGCCAGCG GGGGTATAGA GGCCGCTGTA GCAGAGTCTC
 101  TGATTACTAA GATCGTCGCT AGTGCGGAAA CAAAGCCAGC ACCTGTTCCT
 151  ATGACAGCGA AGAAGGTTAG ACTTGTCCGT AGAAATAAAC AACCAGTTGA
 201  ACAAAAAAGC CGTGGTGCTT TTTGTGATAA AGAATTTTAT CCCTGTGAAG
 251  AGGGACGATG TCAACCTGTA GAGGCTCAGC AAGAGTCTTG CTACGGAAGA
 301  TTGTATTCTG TAAAAGTAAA CGATGATTGC AACGTAGAAA TTTGCCAGTC
 351  CGTTCCAGAA TACGCTACTG TAGGATCTCC TTACCCTATT GAAATCCTTG
 401  CTATAGGCAA AAAAGATTGT GTTGATGTTG TGATTACACA ACAGCTACCT
 451  TGCGAAGCTG AATTCGTAAG CAGTGATCCA GAAACAACTC CTACAAGTGA
 501  TGGGAAATTA GTCTGGAAAA TCGATCGCCT GGGTGCAGGA GATAAATGCA
 551  AAATTACTGT ATGGGTAAAA CCTCTTAAAG AAGGTTGCTG CTTCACAGCT
 601  GCTACTGTAT GTGCTTGCCC AGAGCTCCGT TCTTATACTA AATGCGGTCA
 651  ACCAGCCATT TGTATTAAGC AAGAAGGACC TGACTGTGCT TGCCTAAGAT
 701  GCCCTGTATG CTACAAAATC GAAGTAGTGA ACACAGGATC TGCTATTGCC
 751  CGTAACGTAA CTGTAGATAA TCCTGTTCCC GATGGCTATT CTCATGCATC
 801  TGGTCAAAGA GTTCTCTCTT TTAACTTAGG AGACATGAGA CCTGGCGATA
 851  AAAAGGTATT TACAGTTGAG TTCTGCCCTC AAAGAAGAGG TCAAATCACT
 901  AACGTTGCTA CTGTAACTTA CTGCGGTGGA CACAAATGTT CTGCAAATGT
 951  AACTACAGTT GTTAATGAGC CTTGTGTACA AGTAAATATC TCTGGTGCTG
1001  ATTGGTCTTA CGTATGTAAA CCTGTGGAGT ACTCTATCTC AGTATCGAAT
1051  CCTGGAGACT TGGTTCTTCA TGATGTCGTG ATCCAAGATA CACTCCCTTC
1101  TGGTGTTACA GTACTCGAAG CTCCTGGTGG AGAGATCTGC TGTAATAAAG
1151  TTGTTTGGCG TATTAAAGAA ATGTGCCCAG GAGAAACCCT CCAGTTTAAA
1201  CTTGTAGTGA AAGCTCAAGT TCCTGGAAGA TTCACAAATC AAGTTGCAGT
1251  AACTAGTGAG TCTAACTGCG GAACATGTAC ATCTTGCGCA GAAACAACAA
1301  CACATTGGAA AGGTCTTGCA GCTACCCATA TGTGCGTATT AGACACAAAT
1351  GATCCTATCT GTGTAGGAGA AAATACTGTC TATCGTATCT GTGTAACTAA
1401  CCGTGGTTCT GCTGAAGATA CTAACGTATC TTTAATCTTG AAGTTCTCAA
1451  AAGAACTTCA GCCAATAGCT TCTTCAGGTC CAACTAAAGG AACGATTTCA
1501  GGTAATACCG TTGTTTTCGA CGCTTTACCT AAACTCGGTT CTAAGGAATC
1551  TGTAGAGTTT TCTGTTACCT TGAAAGGTAT TGCTCCCGGA GATGCTCGCG
1601  GCGAAGCTAT TCTTTCTTCT GATACACTGA CTTCACCAGT ATCAGACACA
1651  GAAAATACCC ACGTGTATTA A
```

The PSORT algorithm predicts periplasmic space (0.93).

The protein was expressed in *E.coli* and purified as a GST-fusion product, as shown in Figure 24A, and also as a his-tag protein. The recombinant proteins were used to immunise mice, whose sera were used in a Western blot (Figure 24B) and for FACS analysis (Figure 24C).

The cp6849 protein was also identified in the 2D-PAGE experiment (Cpn0557).

These experiments show that cp6849 is a surface-exposed and immunoaccessible protein, and that it is a useful immunogen. These properties are not evident from the sequence alone.

## Example 25

The following *C.pneumoniae* protein (PID 4376273) was expressed <SEQ ID 49; cp6273>:

1 **MGLFHLTLFG LLLCSLPISL VAKFPESVGH KILYISTQST QQALA**TYLEA
51 LDAYGDHDFF VLRKIGEDYL KQSIHSSDPQ TRKSTIIGAG LAGSSEALDV
101 LSQAMETADP LQQLLVLSAV SGHLGKTSDD LLFKALASPY PVIRLEAAYR
151 LANLKNTKVI DHLHSFIHKL PEEIQCLSAA IFLRLETEES DAYIRDLLAA
201 KKSAIRSATA LQIGEYQQKR FLPTLRNLLT SASPQDQEAI LYALGKLKDG

251 QSYYNIKKQL QKPDVDVTLA AAQALIALGK EEDALPVIKK QALEERPRAL
301 YALRHLPSEI GIPIALPIFL KTKNSEAKLN VALALLELGC DTPKLLEYIT
351 ERLVQPHYNE TLALSFSKGR TLQNWKRVNI IVPQDPQERE RLLSTTRGLE
401 EQILTFLFRL PKEAYLPCIY KLLASQKTQL ATTAISFLSH TSHQEALDLL
451 FQAAKLPGEP IIRAYADLAI YNLTKDPEKK RSLHDYAKKL IQETLLFVDT
501 ENQRPHPSMP YLRYQVTPES RTKLMLDILE TLATSKSSED IRLLIQLMTE
551 GDAKNFPVLA GLLIKIVE*

A predicted signal peptide is highlighted.

The cp6273 nucleotide sequence <SEQ ID 50> is:

1 ATGGGACTAT TCCATCTAAC TCTCTTTGGA CTTTTATTGT GTAGTCTTCC
51 CATTTCTCTT GTTGCTAAAT TCCCTGAGTC TGTAGGTCAT AAGATCCTTT
101 ATATAAGTAC GCAATCTACA CAGCAGGCCT TAGCAACATA TCTGGAAGCT
151 CTAGATGCCT ACGGTGATCA TGACTTCTTC GTTTTAAGAA AAATCGGAGA
201 AGACTATCTC AAGCAAAGCA TCCACTCCTC AGATCCGCAA ACTAGAAAAA
251 GCACCATCAT TGGAGCAGGC CTGGCGGGAT CTTCAGAAGC CTTGGACGTG
301 CTCTCCCAAG CTATGGAAAC TGCAGACCCC CTGCAGCAGC TACTGGTTTT
351 ATCGGCAGTC TCAGGACATC TTGGGAAAAC TTCTGACGAC TTACTGTTTA
401 AAGCTTTAGC ATCTCCCTAT CCTGTCATCC GCTTAGAAGC CGCCTATAGA
451 CTTGCTAATT TGAAGAACAC TAAAGTCATT GATCATCTAC ATTCTTTCAT
501 TCATAAGCTT CCCGAAGAAA TCCAATGCCT ATCTGCGGCA ATATTCCTAC
551 GCTTGGAGAC TGAAGAATCT GATGCTTATA TTCGGGATCT CTTAGCTGCC
601 AAGAAAAGCG CGATTCGGAG TGCCACAGCT TTGCAGATCG GAGAATACCA
651 ACAAAAACGC TTTCTTCCGA CACTTAGGAA TTTGCTAACG AGTGCGTCTC
701 CTCAAGATCA AGAAGCTATT CTTTATGCTT TAGGGAAGCT TAAGGATGGT
751 CAGAGCTACT ACAATATAAA AAAGCAATTG CAGAAGCCTG ATGTGGATGT
801 CACTTTAGCA GCAGCTCAAG CTTTAATTGC TTTGGGGAAA GAAGAGGACG
851 CTCTTCCCGT GATAAAAAAG CAAGCACTTG AGGAGCGGCC TCGAGCCCTG
901 TATGCCTTAC GGCATCTACC CTCTGAGATA GGGATTCCGA TTGCCCTGCC
951 GATATTCCTA AAAACTAAGA ACAGCGAAGC CAAGTTGAAT GTAGCTTTAG
1001 CTCTCTTAGA GTTAGGGTGT GACACCCCTA AACTACTGGA ATACATTACC
1051 GAAAGGCTTG TCCAACCACA TTATAATGAG ACTCTAGCCT TGAGTTTCTC
1101 TAAGGGGCGT ACTTTACAAA ATTGGAAGCG GGTGAACATC ATAGTCCCTC
1151 AAGATCCCCA GGAGAGGGAA AGGTTGCTCT CCACAACCCG AGGTCTTGAA
1201 GAGCAGATCC TTACGTTTCT CTTCCGCCTA CCTAAAGAAG CTTACCTCCC
1251 CTGTATTTAT AAGCTTTTGG CGAGTCAGAA AACTCAGCTT GCCACTACTG
1301 CGATTTCTTT TTTAAGTCAC ACCTCACATC AGGAAGCCTT AGATCTACTT
1351 TTCCAAGCTG CGAAGCTTCC TGGAGAACCT ATCATCCGCG CCTATGCAGA
1401 TCTTGCTATT TATAATCTCA CCAAAGATCC TGAAAAAAAA CGTTCTCTCC
1451 ATGATTATGC AAAAAAGCTA ATTCAGGAAA CCTTGTTATT TGTGGACACG
1501 GAAAACCAAA GACCCCATCC CAGCATGCCC TATCTACGTT ATCAGGTCAC
1551 CCCAGAAAGC CGTACGAAGC TCATGTTGGA TATTCTAGAG ACACTAGCCA
1601 CCTCGAAGTC TTCCGAAGAT ATCCGTTTAT TGATACAACT GATGACGGAA
1651 GGAGATGCAA AAAATTTCCC AGTCCTTGCA GGCTTACTCA TAAAAATTGT
1701 GGAGTAA

The PSORT algorithm predicts a periplasmic location (0.922).

The protein was expressed in *E.coli* and purified as a his-tag product and as a GST-fusion product, as shown in Figure 25A. The recombinant GST-fusion was used to immunise mice, whose sera were used in a Western blot (Figure 25B) and for FACS analysis (Figure 25C).

This protein also showed good cross-reactivity with human sera, including sera from patients with pneumonitis.

These experiments show that cp6273 is a surface-exposed and immunoaccessible protein, and that it is a useful immunogen. These properties are not evident from the sequence alone.

**Example 26**

The following *C.pneumoniae* protein (PID 4376735) was expressed <SEQ ID 51; cp6735>:

```
  1  MTILRNFLTC SALFLALPAA AQVVYLHESD GYNGAINNKS LEPKITCYPE
 51  GTSYIFLDDV RISNVKHDQE DAGVFINRSG NLFFMGNRCN FTFHNLMTEG
101  FGAAISNRVG DTTLTLSNFS YLAFTSAPLL PQGQGAIYSL GSVMIENSEE
151  VTFCGNYSSW SGAAIYTPYL LGSKASRPSV NLSGNRYLVF RDNVSQGYGG
201  AISTHNLTLT TRGPSCFENN HAYHDVNSNG GAIAIAPGGS ISISVKSGDL
251  IFKGNTASQD GNTIHNSIHL QSGAQFKNLR AVSESGVYFY DPISHSESHK
301  ITDLVINAPE GKETYEGTIS FSGLCLDDHE VCAENLTSTI LQDVTLAGGT
351  LSLSDGVTLQ LHSFKQEASS TLTMSPGTTL LCSGDARVQN LHILIEDTDN
401  FVPVRIRAED KDALVSLEKL KVAFEAYWSV YDFPQFKEAF TIPLLELLGP
451  SFDSLLLGET TLERTQVTTE NDAVRGFWSL SWEEYPPSLD KDRRITPTKK
501  TVFLTWNPEI TSTP*
```

A predicted signal peptide is highlighted.

The cp6735 nucleotide sequence <SEQ ID 52> is:

```
   1  ATGACCATAC TTCGAAATTT TCTTACCTGC TCGGCTTTAT TCCTCGCTCT
  51  CCCTGCAGCA GCACAAGTTG TATATCTTCA TGAAAGTGAT GGTTATAACG
 101  GTGCTATCAA TAATAAAAGC TTAGAACCTA AAATTACCTG TTATCCAGAA
 151  GGAACTTCTT ACATCTTTCT AGATGACGTG AGGATTTCCA ACGTTAAGCA
 201  TGATCAAGAA GATGCTGGGG TTTTTATAAA TCGATCTGGG AATCTTTTTT
 251  TCATGGGCAA CCGTTGCAAC TTCACTTTTC ACAACCTTAT GACCGAGGGT
 301  TTTGGCGCTG CCATTTCGAA CCGCGTTGGA GACACCACTC TCACTCTCTC
 351  TAATTTTTCT TACTTAGCGT TCACCTCAGC ACCTCTACTA CCTCAAGGAC
 401  AAGGAGCGAT TTATAGTCTT GGTTCCGTGA TGATCGAAAA TAGTGAGGAA
 451  GTGACTTTCT GTGGGAACTA CTCTTCGTGG AGTGGAGCTG CGATTTATAC
 501  TCCCTACCTT TTAGGTTCTA AGGCGAGTCG TCCTTCAGTA AATCTCAGCG
 551  GGAACCGCTA CCTGGTGTTT AGAGACAATG TGAGCCAAGG TTATGGCGGC
 601  GCCATATCTA CCCACAATCT CACACTCACG ACTCGAGGAC CTTCGTGTTT
 651  TGAAAATAAT CATGCTTATC ATGACGTGAA TAGTAATGGA GGAGCCATTG
 701  CCATTGCTCC TGGAGGATCG ATCTCTATAT CCGTGAAAAG CGGAGATCTC
 751  ATCTTCAAAG GAAATACAGC ATCACAAGAC GGAAATACAA TACACAACTC
 801  CATCCATCTG CAATCTGGAG CACAGTTTAA GAACCTACGT GCTGTTTCAG
 851  AATCCGGAGT TTATTTCTAT GATCCTATAA GCCATAGCGA GTCGCATAAA
 901  ATTACAGATC TTGTAATCAA TGCTCCTGAA GGAAAGGAAA CTTATGAAGG
 951  AACAATTAGC TTCTCAGGAC TATGCCTGGA TGATCATGAA GTTTGTGCGG
1001  AAAATCTTAC TTCCACAATC CTACAAGATG TCACATTAGC AGGAGGAACT
1051  CTCTCTCTAT CGGATGGGGT TACCTTGCAA CTGCATTCTT TTAAGCAGGA
1101  AGCAAGCTCT ACGCTTACTA TGTCTCCAGG AACCACTCTG CTCTGCTCAG
1151  GAGATGCTCG GGTTCAGAAT CTGCACATCC TGATTGAAGA TACCGACAAC
1201  TTTGTTCCTG TAAGGATTCG CGCCGAGGAC AAGGATGCTC TTGTCTCATT
1251  AGAAAAACTT AAAGTTGCCT TTGAGGCTTA TTGGTCCGTC TATGACTTTC
1301  CTCAATTTAA GGAAGCCTTT ACGATTCCTC TTCTTGAACT TCTAGGGCCT
1351  TCTTTTGACA GTCTTCTCCT AGGGGAGACC ACTTTGGAGA GAACCCAAGT
1401  CACAACAGAG AATGACGCCG TTCGAGGTTT CTGGTCCCTA AGCTGGGAAG
1451  AGTACCCCCC TTCTCTGGAT AAAGACAGAA GGATCACACC AACTAAGAAA
1501  ACTGTTTTCC TCACTTGGAA TCCTGAGATC ACTTCTACGC CATAA
```

The PSORT algorithm predicts an outer membrane location (0.922).

The protein was expressed in *E.coli* and purified as a as a his-tag product and as a GST-fusion product, as shown in Figure 26A. The recombinant GST-fusion protein was used to immunise mice, whose sera were used in a Western blot (Figure 26B).

These experiments show that cp6735 is a surface-exposed and immunoaccessible protein, and that it is a useful immunogen. These properties are not evident from the sequence alone.

**Example 27**

The following *C.pneumoniae* protein (PID 4376784) was expressed <SEQ ID 53; cp6784>:

```
  1  MNRRKARWVV ALFAMTALIS VGCCPWSQAK SRCSIDKYIP VVNRLLEVCG
 51  LPEAENVEDL IESSSAWVLT PEERFSGELV SICQVKDEHA FYNDLSLLHM
101  TQAVPSYSAT YDCAVVFGGP LPALRQRLDF LVREWQRGVR FKKIVFLCGE
151  RGRYQSIEEQ EHFFDSRYNP FPTEENWESG NRVTPSSEEE IAKFVWMQML

201  LPRAWRDSTS GVRVTFLLAK PEENRVVANR KDTLLLFRSY QEAFPGRVLF
251  VSSQPFIGLD ACRVGQFFKG ESYDLAGPGF AQGVLKYHWA PRICLHTLAE
301  WLKETNGCLN ISEGCFG*
```

A predicted signal peptide is highlighted.

The cp6784 nucleotide sequence <SEQ ID 54> is:

```
  1  ATGAATAGAA GAAAAGCAAG ATGGGTAGTG GCATTGTTCG CAATGACGGC
 51  GCTCATTTCT GTTGGGTGTT GTCCTTGGTC ACAAGCGAAA TCAAGATGTT
101  CTATTGATAA GTATATTCCT GTAGTCAATC GTTTACTAGA AGTTTGTGGA
151  CTTCCTGAAG CTGAGAATGT TGAGGATTTA ATCGAGTCCT CGTCTGCTTG
201  GGTACTGACT CCTGAAGAAC GTTTTTCTGG AGAGTTAGTC TCTATCTGTC
251  AGGTTAAAGA TGAGCATGCT TTCTATAACG ATTTGTCTTT ATTACATATG
301  ACTCAGGCTG TGCCTTCGTA TTCTGCAACG TATGATTGTG CTGTAGTTTT
351  TGGCGGGCCT TTGCCAGCGC TACGTCAGCG CTTAGATTTT TTGGTGCGAG
401  AGTGGCAGCG TGGCGTGCGC TTTAAGAAAA TCGTTTTTCT ATGTGGAGAG
451  CGAGGGCGCT ATCAGTCTAT TGAAGAACAA GAGCATTTCT TTGATTCTCG
501  GTACAATCCT TTCCCTACTG AAGAGAACTG GGAATCTGGT AACCGAGTTA
551  CTCCCTCTTC TGAAGAAGAG ATTGCCAAAT TTGTTTGGAT GCAAATGCTT
601  TTACCTAGAG CATGGCGAGA TAGTACTTCA GGAGTCAGAG TGACATTTCT
651  TCTAGCAAAG CCAGAGGAAA ATCGTGTGGT TGCGAATCGT AAGGACACCT
701  TACTTTTATT CCGTTCTTAT CAAGAAGCGT TTCCGGGACG CGTGTTATTT
751  GTAAGTAGTC AACCCTTTAT CGGTTTAGAT GCTTGCAGGG TCGGGCAGTT
801  TTTCAAAGGG GAAAGCTATG ATCTTGCTGG ACCTGGATTT GCTCAAGGAG
851  TCTTGAAGTA TCATTGGGCT CCAAGGATTT GTCTACATAC TTTAGCGGAA
901  TGGTTAAAGG AAACGAACGG CTGCTTAAAT ATTTCAGAGG GTTGTTTTGG
951  ATGA
```

The PSORT algorithm predicts a periplasmic location (0.894).

The protein was expressed in *E.coli* and purified as a his-tag product and as a GST-fusion product, as shown in Figure 27A. The recombinant proteins were used to immunise mice, whose sera were used in a Western blot (Figure 27B). The GST-fusion product was used for FACS analysis (Figure 27C).

The cp6784 protein was also identified in the 2D-PAGE experiment (Cpn0498).

These experiments show that cp6784 is a surface-exposed and immunoaccessible protein, and that it is a useful immunogen. These properties are not evident from the sequence alone.

**Example 28**

The following *C.pneumoniae* protein (PID 4376960) was expressed <SEQ ID 55; cp6960>:

```
  1  MNRRWNLVLA TVALALSVAS CDVRSKDKDK DQGSLVEYKD NKDTNDIELS
 51  DNQKLSRTFG HLLARQLRKS EDMFFDIAEV AKGLQAELVC KSAPLTETEY
101  EEKMAEVQKL VFEKKSKENL SLAEKFLKEN SKNAGVVEVQ PSKLQYKIIK
151  EGAGKAISGK PSALLHYKGS FINGQVFSSS EGNNEPILLP LGQTIPGFAL
201  GMQGMKEGET RVLYIHPDLA YGTAGQLPPN SLLIFEINLI QASADEVAAV
251  PQEGNQGE*
```

A predicted signal peptide is highlighted.

The cp6960 nucleotide sequence <SEQ ID 56> is:

```
  1 ATGAACAGAC GGTGGAATTT AGTTTTAGCA ACAGTAGCTC TGGCACTCTC
 51 CGTCGCTTCT TGTGACGTAC GGTCTAAGGA TAAAGACAAG GATCAGGGGT
101 CGTTAGTGGA ATATAAAGAT AACAAAGATA CCAATGACAT AGAATTATCC
151 GATAATCAAA AGTTATCCAG AACATTTGGT CATTTATTAG CACGCCAATT
201 ACGCAAGTCA GAAGATATGT TTTTTGATAT TGCAGAAGTG GCTAAGGGGT
251 TGCAGGCGGA ATTGGTTTGT AAAAGTGCTC CTTTAACAGA AACAGAGTAT
301 GAAGAAAAAA TGGCTGAAGT ACAGAAGTTG GTTTTTGAAA AAAAATCAAA
351 AGAAAATCTT TCATTGGCAG AAAAATTCTT AAAAGAAAAT AGCAAGAACG
401 CTGGTGTTGT TGAAGTGCAA CCAAGTAAAT TGCAATACAA AATTATTAAA
451 GAAGGTGCAG GGAAAGCAAT TTCAGGTAAA CCTTCAGCTC TATTGCACTA
501 CAAGGGTTCC TTCATCAATG GCCAAGTATT TAGCAGTTCA GAAGGCAACA
551 ATGAGCCTAT CTTGCTTCCT CTAGGCCAAA CAATTCCTGG TTTTGCTTTA
601 GGTATGCAGG GCATGAAAGA AGGAGAAACT CGAGTTCTCT ACATCCATCC
651 TGATCTTGCT TACGGAACCG CAGGACAACT TCCTCCAAAC TCTTTATTAA
701 TTTTTGAAAT TAACTTGATT CAGGCTTCAG CAGATGAAGT TGCTGCTGTA
751 CCCCAAGAAG GAAATCAAGG TGAATGA
```

The PSORT algorithm predicts periplasmic space location (0.930).

The protein was expressed in *E.coli* and purified as a his-tag product and as a GST-fusion product, as shown in Figure 28A. The recombinant proteins were used to immunise mice, whose sera were used in a Western blot (Figure 28B) and for FACS analysis (Figure 28C).

The cp6960 protein was also identified in the 2D-PAGE experiment.

These experiments show that cp6960 is a surface-exposed and immunoaccessible protein, and that it is a useful immunogen. These properties are not evident from the sequence alone.

**Example 29**

The following *C.pneumoniae* protein (PID 4376968) was expressed <SEQ ID 57; cp6968>:

```
  1 MKFLLYVPLL LVLVSTGCDA KPVSFEPFSG KLSTQRFEPQ HSAEEYFSQG
 51 QEFLKKGNFR KALLCFGIIT HHFPRDILRN QAQYLIGVCY FTQDHPDLAD
101 KAFASYLQLP DAEYSEELFQ MKYAIAQRFA QGKRKRICRL EGFPKLMNAD
151 EDALRIYDEI LTAFPSKDLG AQALYSKAAL LIVKNDLTEA TKTLKKLTLQ
201 FPLHILSSEA FVRLSEIYLQ QAKKEPHNLQ YLHFAKLNEE AMKKQHPNHP
251 LNEVVSANVG AMREHYARGL YATGRFYEKK KKAEAANIYY RTAITNYPDT
301 LLVAKCQKRL DRISKHTS*
```

A predicted signal peptide is highlighted.

The cp6968 nucleotide sequence <SEQ ID 58> is:

```
  1  ATGAAATTTC TATTATACGT TCCACTTCTT CTTGTTCTCG TATCTACGGG
 51  GTGCGATGCA AAACCTGTTT CTTTTGAGCC CTTTTCAGGA AAGCTTTCCA
101  CCCAGCGTTT TGAGCCTCAG CACTCTGCTG AAGAATATTT TTCTCAGGGA
151  CAGGAATTCT TAAAAAAAGG AAATTTCAGA AAAGCTTTAC TATGCTTTGG
201  AATCATTACG CATCACTTCC CTAGGGACAT CTTGCGTAAT CAAGCACAGT
251  ATCTTATAGG AGTCTGTTAC TTCACGCAGG ATCACCCAGA TTTAGCAGAC
301  AAGGCATTTG CATCTTACTT ACAACTTCCT GATGCGGAGT ACTCTGAAGA
351  GTTGTTCCAG ATGAAATATG CGATTGCTCA AAGATTTGCT CAAGGGAAGC
401  GTAAACGGAT TTGTCGATTA GAGGGCTTCC CAAAACTAAT GAATGCTGAT
451  GAAGATGCGC TACGCATTTA TGACGAGATT CTAACAGCGT TTCCTAGTAA
501  AGACTTAGGA GCTCAGGCCC TCTATAGTAA AGCTGCGTTA CTTATTGTAA
551  AAAACGATCT TACAGAAGCC ACCAAAACCT TAAAAAAACT CACGTTACAA
601  TTTCCTCTAC ATATTTTATC TTCAGAGGCC TTTGTACGTT TATCGGAAAT
651  CTATTTACAG CAAGCTAAGA AAGAGCCTCA CAATCTTCAA TATCTTCATT
701  TTGCAAAGCT TAATGAAGAG GCAATGAAAA AGCAGCATCC TAACCATCCT
751  CTGAATGAGG TTGTTTCTGC TAATGTTGGA GCTATGCGGG AACATTATGC
801  TCGAGGTTTG TATGCCACAG GTCGTTTCTA TGAGAAGAAG AAAAAAGCCG
851  AGGCTGCGAA TATCTATTAC CGCACTGCGA TTACAAACTA CCCAGACACT
901  TTATTAGTGG CTAAATGTCA AAAGCGTCTA GATAGAATAT CTAAGCATAC
951  TTCCTAA
```

The PSORT algorithm predicts an inner membrane location (0.790).

The protein was expressed in *E.coli* and purified as a his-tag product and as a GST-fusion product, as shown in Figure 29A. The recombinant GST-fusion was used to immunise mice, whose sera were used in a Western blot (Figure 29B) and for FACS analysis (Figure 29C).

This protein also showed good cross-reactivity with human sera, including sera from patients with pneumonitis.

These experiments show that cp6968 is a surface-exposed and immunoaccessible protein, and that it is a useful immunogen. These properties are not evident from the sequence alone.

### Example 30

The following *C.pneumoniae* protein (PID 4376998) was expressed <SEQ ID 59; cp6998>:

```
  1  MKKLLKSALL SAAFAGSVGS LQALPVGNPS DPSLLIDGTI WEGAAGDPCD
 51  PCATWCDAIS LRAGFYGDYV FDRILKVDAP KTFSMGAKPT GSAAANYTTA
101  VDRPNPAYNK HLHDAEWFTN AGFIALNIWD RFDVFCTLGA SNGYIRGNST
151  AFNLVGLFGV KGTTVNANEL PNVSLSNGVV ELYTDTSFSW SVGARGALWE
201  CGCATLGAEF QYAQSKPKVE ELNVICNVSQ FSVNKPKGYK GVAFPLPTDA
251  GVATATGTKS ATINYHEWQV GASLSYRLNS LVPYIGVQWS RATFDADNIR
301  IAQPKLPTAV LNLTAWNPSL LGNATALSTT DSFSDFMQIV SCQINKFKSR
351  KACGVTVGAT LVDADKWSLT AEARLINERA AHVSGQFRF*
```

A predicted signal peptide is highlighted.

The cp6998 nucleotide sequence <SEQ ID 60> is:

```
   1  ATGAAAAAAC TCTTAAAGTC GGCGTTATTA TCCGCCGCAT TTGCTGGTTC
  51  TGTTGGCTCC TTACAAGCCT TGCCTGTAGG GAACCCTTCT GATCCAAGCT
 101  TATTAATTGA TGGTACAATA TGGGAAGGTG CTGCAGGAGA TCCTTGCGAT
 151  CCTTGCGCTA CTTGGTGCGA CGCTATTAGC TTACGTGCTG GATTTTACGG
 201  AGACTATGTT TTCGACCGTA TCTTAAAAGT AGATGCACCT AAAACATTTT
 251  CTATGGGAGC CAAGCCTACT GGATCCGCTG CTGCAAACTA TACTACTGCC
 301  GTAGATAGAC CTAACCCGGC CTACAATAAG CATTTACACG ATGCAGAGTG
 351  GTTCACTAAT GCAGGCTTCA TTGCCTTAAA CATTTGGGAT CGCTTTGATG
 401  TTTTCTGTAC TTTAGGAGCT TCTAATGGTT ACATTAGAGG AAACTCTACA
 451  GCGTTCAATC TCGTTGGTTT ATTCGGAGTT AAAGGTACTA CTGTAAATGC
 501  AAATGAACTA CCAAACGTTT CTTTAAGTAA CGGAGTTGTT GAACTTTACA
 551  CAGACACCTC TTTCTCTTGG AGCGTAGGCG CTCGTGGAGC CTTATGGGAA
 601  TGCGGTTGTG CAACTTTGGG AGCTGAATTC CAATATGCAC AGTCCAAACC
 651  TAAAGTTGAA GAACTTAATG TGATCTGTAA CGTATCGCAA TTCTCTGTAA
 701  ACAAACCCAA GGGCTATAAA GGCGTTGCTT TCCCCTTGCC AACAGACGCT
 751  GGCGTAGCAA CAGCTACTGG AACAAAGTCT GCGACCATCA ATTATCATGA
 801  ATGGCAAGTA GGAGCCTCTC TATCTTACAG ACTAAACTCT TTAGTGCCAT
 851  ACATTGGAGT ACAATGGTCT CGAGCAACTT TTGATGCTGA TAACATCCGC
 901  ATTGCTCAGC CAAAACTACC TACAGCTGTT TTAAACTTAA CTGCATGGAA
 951  CCCTTCTTTA CTAGGAAATG CCACAGCATT GTCTACTACT GATTCGTTCT
1001  CAGACTTCAT GCAAATTGTT TCCTGTCAGA TCAACAAGTT TAAATCTAGA
1051  AAAGCTTGTG GAGTTACTGT AGGAGCTACT TTAGTTGATG CTGATAAATG
1101  GTCACTTACT GCAGAAGCTC GTTTAATTAA CGAGAGAGCT GCTCACGTAT
1151  CTGGTCAGTT CAGATTCTAA
```

The PSORT algorithm predicts an outer membrane location (0.707).

The protein was expressed in *E.coli* and purified as a GST-fusion (Figure 30A) and as a his-tag product. The recombinant GST-fusion protein was used to immunise mice, whose sera were used in a Western blot (Figure 30B) and for FACS analysis (Figure 30C).

The cp6998 protein was also identified in the 2D-PAGE experiment (Cpn0695) and showed good cross-reactivity with human sera, including sera from patients with pneumonitis.

These experiments show that cp6998 is a surface-exposed and immunoaccessible protein, and that it is a useful immunogen. These properties are not evident from the sequence alone.

**Example 31**

The following *C.pneumoniae* protein (PID 4377102) was expressed <SEQ ID 61; cp7102>:

```
   1  MKHTFTKRVL FFFFLVIPIP LLLNLMVVGF FSFSAAKANL VQVLHTRATN
  51  LSIEFEKKLT IHKLFLDRLA NTLALKSYAS PSAEPYAQAY NEMMALSNTD
 101  FSLCLIDPFD GSVRTKNPGD PFIRYLKQHP EMKKKLSAAV GKAFLLTIPG
 151  KPLLHYLILV EDVASWDSTT TSGLLVSFYP MSFLQKDLFQ SLHITKGNIC
 201  LVNKYGEVLF CAQDSESSFV FSLDLPNLPQ FQARSPSAIE IEKASGILGG
 251  ENLITVSINK KRYLGLVLNK IPIQGTYTLS LVPVSDLIQS ALKVPLNICF
 301  FYVLAFLLMW WIFSKINTKL NKPLQELTFC MEAAWRGNHN VRFEPQPYGY
 351  EFNELGNIFN CTLLLLLNSI EKADIDYHSG EKLQKELGIL SSLQSALLSP
 401  DFPTFPKVTF SSQHLRRRQL SGHFNGWTVQ DGGDTLLGII GLAGDIGLPS
 451  YLYALSARSL FLAYASSDVS LQKISKDTAD SFSKTTEGNE AVVAMTFIKY
 501  VEKDRSLELL SLSEGAPTMF LQRGESFVRL PLETHQALQP GDRLICLTGG
 551  EDILKYFSQL PIEELLKDPL NPLNTENLID SLTMMLNNET EHSADGTLTI
 601  LSFS*
```

A predicted signal peptide is highlighted.

The cp7102 nucleotide sequence <SEQ ID 62> is:

```
   1  ATGAAACATA CCTTTACCAA GCGTGTTCTA TTTTTTTTCT TTTTAGTGAT
  51  TCCCATTCCC CTACTCCTCA ATCTTATGGT CGTAGGTTTT TTCTCATTTT
 101  CTGCCGCTAA AGCAAATTTA GTACAGGTCC TCCATACCCG TGCTACGAAC
 151  TTAAGTATAG AATTCGAAAA AAAACTGACG ATACACAAGC TTTTCCTCGA
 201  TAGACTTGCC AACACATTAG CCTTAAAATC CTATGCATCT CCTTCTGCAG
 251  AGCCCTATGC ACAGGCATAC AATGAGATGA TGGCACTCTC CAATACAGAC
 301  TTTTCCTTAT GCCTTATAGA TCCCTTTGAT GGATCTGTAA GGACGAAAAA
 351  TCCTGGAGAC CCTTTCATTC GCTATCTAAA ACAGCATCCT GAAATGAAGA
 401  AAAAGCTATC CGCAGCTGTA GGGAAAGCCT TTTTATTGAC CATTCCAGGT
 451  AAACCACTTT TACATTATCT TATTCTAGTT GAAGATGTCG CATCTTGGGA
 501  TTCTACAACG ACTTCAGGAC TGCTTGTAAG TTTCTATCCC ATGTCTTTTT
 551  TACAGAAAGA TTTATTCCAA TCCTTACACA TCACCAAAGG AAATATCTGC
 601  CTTGTAAATA AGTATGGCGA GGTCCTCTTC TGTGCTCAGG ACAGTGAATC
 651  TTCTTTTGTA TTTTCTCTAG ATCTCCCTAA TTTACCGCAA TTCCAAGCAA
 701  GAAGCCCCTC TGCCATAGAA ATTGAGAAAG CTTCTGGAAT TCTTGGTGGG
 751  GAGAACCTAA TCACAGTGAG TATCAACAAG AAACGCTACC TAGGATTGGT
 801  ACTGAATAAA ATTCCTATCC AAGGGACCTA CACTCTATCT TTAGTTCCAG
 851  TTTCTGATCT CATCCAATCC GCCTTGAAAG TTCCTCTCAA TATTTGTTTT
 901  TTCTATGTAC TTGCTTTCCT CCTCATGTGG TGGATTTTCT CTAAGATCAA
 951  CACCAAACTT AACAAGCCTC TTCAAGAACT GACCTTCTGT ATGGAAGCTG
1001  CCTGGCGAGG AAACCATAAC GTGAGGTTTG AACCCCAGCC TTACGGTTAT
1051  GAATTCAATG AACTAGGAAA TATTTTCAAT TGCACTCTCC TACTCTTATT
1101  GAATTCCATT GAGAAAGCAG ATATCGATTA CCATTCAGGC GAAAAATTAC
1151  AAAAAGAATT AGGGATTTTA TCTTCACTAC AAAGTGCGTT ACTAAGTCCG
1201  GATTTCCCTA CGTTCCCTAA AGTTACCTTT AGTTCCCAAC ATCTCCGGAG
1251  AAGGCAACTT TCCGGTCATT TTAATGGTTG GACAGTTCAA GATGGTGGCG
1301  ATACCCTTTT AGGGATCATA GGGCTCGCTG GCGATATTGG TCTTCCTTCC
1351  TATCTCTATG CTTTATCCGC ACGGAGTCTT TTTCTTGCCT ATGCTTCCTC
1401  GGACGTTTCG TTACAAAAAA TCAGCAAGGA TACTGCCGAC AGCTTCTCAA
1451  AAACAACAGA AGGCAATGAG GCTGTAGTTG CTATGACTTT CATTAAATAT
1501  GTAGAAAAAG ATCGATCTCT AGAGCTCCTC TCGTTAAGCG AGGGAGCTCC
1551  TACCATGTTT CTACAACGAG GAGAATCTTT CGTACGTCTC CCCTTAGAGA
1601  CTCACCAAGC TCTACAGCCT GGAGATCGGT TGATCTGCCT CACTGGAGGA
1651  GAAGACATCC TCAAGTACTT TTCTCAGCTT CCTATTGAAG AGCTCTTAAA
1701  AGATCCTTTA AACCCTCTAA ATACAGAGAA TCTTATTGAT TCTCTAACCA
1751  TGATGTTAAA CAACGAAACC GAACATTCTG CAGATGGAAC TCTGACCATC
1801  CTTTCATTTT CATAA
```

The PSORT algorithm predicts an inner membrane location (0.338).

The protein was expressed in *E.coli* and purified as a his-tag product and as a GST-fusion product. The purified GST-fusion product is shown in Figure 31A. The recombinant GST-fusion protein was used to immunise mice, whose sera were used in a Western blot and for FACS analysis (Figure 31B). These experiments show that cp7102 is a surface-exposed and immunoaccessible protein, and that it is a useful immunogen. These properties are not evident from the sequence alone.

### Example 32

The following *C.pneumoniae* protein (PID 4377106) was expressed <SEQ ID 63; cp7106>:

```
  1  MKDLGTLGGT SSTAKTVSPD GKVIMGRSQI ADGSWHAFMC HTDFSSNNVL
 51  FDLDNTYKTL RENGRQLNSI FNLQNMMLQR ASDHEFTEFG RSNIALGAGL
101  YVNALQNLPS NLAAQYFGIA YKIRPKYRLG VFLDHNFSSH VPNNFNVSHN
151  RLWMGAFIGW QDSDALGSSV KVSFGYGKQK ATITREQLEN TEAGSGESHF
201  EGVAAQIEGR YGKSLGGHVR VQPFLGLQFV HITRKEYTEN AVQFPVHYDP
251  IDYSTGVVYL GIGSHIALVD SLHVGTRMGM EQNFAAHTDR FSGSIASIGN
301  FVFEKLDVTH TRAFAEMRVN YELPYLQSLN LILRVNQQPL QGVMGFSSDL
351  RYALGF*
```

The cp7106 nucleotide sequence <SEQ ID 64> is:

```
   1  ATGAAAGATT TGGGGACTCT TGGGGGTACC TCTTCTACAG CAAAAACAGT
  51  GTCCCCAGAT GGTAAAGTGA TCATGGGTAG ATCACAAATT GCTGATGGCA
 101  GTTGGCACGC ATTTATGTGT CATACGGATT TCTCCTCTAA TAATGTACTC
 151  TTTGATCTCG ATAATACGTA TAAAACTCTA AGAGAAAATG GCCGTCAGCT
 201  AAATTCCATA TTCAACCTAC AAAATATGAT GTTACAGAGA GCCTCAGATC
 251  ATGAGTTCAC AGAGTTTGGA AGGAGTAACA TCGCTCTTGG TGCCGGGCTT
 301  TATGTGAATG CCTTGCAGAA TCTCCCTAGC AATTTAGCAG CACAATATTT
 351  TGGAATCGCA TACAAAATAC GTCCTAAATA TCGTTTGGGG GTGTTTTTGG
 401  ACCATAATTT CAGCTCCCAC GTTCCTAATA ATTTTAACGT AAGCCACAAT
 451  AGACTCTGGA TGGGAGCCTT TATTGGATGG CAGGATTCTG ATGCTCTAGG
 501  ATCTAGTGTC AAGGTGTCTT TCGGATATGG AAAACAAAAA GCCACGATTA
 551  CAAGAGAGCA ATTAGAGAAT ACAGAAGCCG GGAGTGGGGA GAGCCATTTT
 601  GAAGGGGTCG CTGCTCAGAT AGAAGGGCGG TATGGTAAGA GCCTCGGAGG
 651  ACATGTCAGG GTCCAGCCTT TCCTAGGACT GCAGTTTGTC CACATTACAA
 701  GGAAAGAATA TACCGAAAAT GCAGTGCAAT TTCCTGTACA CTATGATCCT
 751  ATAGACTATT CTACAGGTGT AGTGTATTTA GGAATTGGAT CTCATATTGC
 801  ACTTGTAGAT TCTTTACATG TAGGCACACG CATGGGAATG GAGCAAAACT
 851  TTGCAGCCCA TACGGACAGG TTCTCAGGAT CTATAGCGTC TATTGGAAAC
 901  TTTGTGTTTG AAAAGCTTGA TGTGACTCAC ACAAGGGCAT TTGCGGAAAT
 951  GCGTGTCAAC TATGAGCTTC CCTATCTACA GTCTCTGAAT CTTATTCTAC
1001  GAGTTAATCA ACAGCCTCTA CAAGGGGTTA TGGGATTTTC CAGTGATCTT
1051  AGGTATGCCT TAGGATTCTA A
```

The PSORT algorithm predicts a cytoplasmic location (0.224).

The protein was expressed in *E.coli* and purified as a his-tag product and as a GST-fusion product. The purified GST-fusion product is shown in Figure 32A. The recombinant GST-fusion protein was used to immunise mice, whose sera were used in a Western blot (Figure 32B) and for FACS analysis (Figure 32C).

This protein also showed very good cross-reactivity with human sera, including sera from patients with pneumonitis.

These experiments show that cp7106 is a surface-exposed and immunoaccessible protein, and that it is a useful immunogen. These properties are not evident from the sequence alone.

**Example 33**

The following *C.pneumoniae* protein (PID 4377228) was expressed <SEQ ID 65; cp7228>:

```
  1  MTAVLILTSF PSEESARSLA RHLITERLAS CVHVFPKGTS TYLWEGKLCE
 51  SEEHHIQIKS IDIRFSEICL AIQEFSGYEV PEVLLFPIEN GDPRYLNWLT
101  ILSYPEKPPL SD*
```

The cp7228 nucleotide sequence <SEQ ID 66> is:

```
  1  ATGACTGCTG TTCTTATTCT TACATCTTTC CCTTCGGAGG AAAGTGCTCG
 51  CTCCTTAGCT AGACATCTGA TTACAGAGCG TCTTGCTTCC TGTGTGCATG
101  TATTCCCTAA AGGCACATCG ACATATCTAT GGGAAGGCAA GCTATGTGAG
151  TCTGAAGAAC ATCATATACA AATCAAATCG ATAGACATAC GCTTCTCGGA
201  AATTTGTCTT GCTATTCAGG AGTTCTCTGG CTATGAGGTT CCTGAAGTCT
251  TACTATTTCC TATTGAAAAT GGGGATCCGA GGTACTTGAA TTGGTTAACG
301  ATTCTCAGCT ATCCAGAGAA GCCTCCGCTT TCAGATTAG
```

The PSORT algorithm predicts an inner membrane location (0.040).

The protein was expressed in *E.coli* and purified as a his-tag product and as a GST-fusion product, as shown in Figure 33A (his-tag = left-hand arrow, GST = right-hand arrow). The proteins were used to immunise mice, whose sera were used in a Western blot (Figure 33B) and FACS analysis.

These experiments show that cp7228 is a surface-exposed and immunoaccessible protein, and that it is a useful immunogen. These properties are not evident from the sequence alone.

**Example 34**

The following *C.pneumoniae* protein (PID 4377170) was expressed <SEQ ID 67; cp7170>:

```
  1 MNSKMLKHLR LATLSFSMFF GIVSSPAVYA LGAGNPAAPV LPGVNPEQTG
 51 WCAFQLCNSY DLFAALAGSL KFGFYGDYVF SESAHITNVP VITSVTTSGT
101 GTTPTITSTT KNVDFDLNNS SISSSCVFAT IALQETSPAA IPLLDIAFTA
151 RVGGLKQYYR LPLNAYRDFT SNPLNAESEV TDGLIEVQSD YGIVWGLSLQ
201 KVLWKDGVSF VGVSADYRHG SSPINYIIVY NKANPEIYFD ATDGNLSYKE
251 WSASIGISTY LNDYVLPYAS VSIGNTSRKA PSDSFTELEK QFTNFKFKIR
301 KITNFDRVNF CFGTTCCISN NFYYSVEGRW GYQRAINITS GLQF*
```

A predicted signal peptide is highlighted.

The cp7170 nucleotide sequence <SEQ ID 68> is:

```
   1 ATGAATAGCA AGATGCTAAA ACATTTACGT TTAGCAACCC TTTCCTTCTC
  51 TATGTTCTTC GGGATTGTAT CTTCTCCCGC AGTATATGCC CTAGGGGCTG
 101 GAAACCCTGC AGCTCCAGTA CTCCCAGGTG TGAATCCTGA GCAAACGGGA
 151 TGGTGTGCCT TCCAACTTTG TAATAGTTAC GATCTTTTTG CTGCTCTTGC
 201 AGGAAGCCTC AAATTTGGGT TCTATGGAGA TTATGTCTTC TCAGAAAGTG
 251 CCCATATTAC CAATGTCCCT GTCATTACCT CCGTTACGAC TTCAGGCACA
 301 GGAACAACGC CAACCATTAC CTCTACAACT AAAAACGTAG ACTTTGATCT
 351 TAACAACAGC TCCATCAGCT CGAGCTGTGT TTTTGCAACC ATAGCTCTAC
 401 AGGAAACATC CCCAGCTGCC ATTCCCCTTT TAGATATAGC CTTCACTGCA
 451 CGTGTCGGAG GACTTAAGCA GTACTACCGC CTCCCTCTCA ATGCTTACAG
 501 AGACTTCACT TCAAATCCTT TAAATGCAGA ATCTGAAGTT ACAGATGGTC
 551 TCATTGAAGT CCAGTCAGAC TATGGAATTG TCTGGGGTCT GAGTTTACAA
 601 AAAGTATTGT GGAAAGATGG AGTGTCTTTT GTAGGGGTGA GCGCTGACTA
 651 CCGTCACGGT TCCAGTCCCA TCAACTATAT CATCGTTTAC AACAAGGCCA
 701 ACCCCGAGAT CTATTTCGAT GCTACTGATG GAAACCTAAG CTATAAAGAA
 751 TGGTCTGCAA GCATCGGCAT CTCTACGTAT CTTAATGACT ATGTGCTTCC
 801 CTATGCATCC GTATCTATAG GAAATACTTC AAGAAAAGCT CCTTCTGATA
 851 GCTTCACAGA ACTCGAAAAG CAATTTACGA ATTTTAAATT TAAAATTCGT
 901 AAAATCACAA ACTTCGACAG AGTAAACTTC TGCTTCGGAA CTACCTGCTG
 951 CATCTCAAAT AACTTCTACT ATAGTGTAGA AGGCCGTTGG GGATATCAGC
1001 GTGCTATCAA CATTACGTCA GGTCTGCAGT TTTAG
```

The PSORT algorithm predicts a bacterial outer membrane location (0.936).

The protein was expressed in *E.coli* and purified as a his-tag product and as a GST-fusion product. The purified GST-fusion product is shown in Figure 34A. The GST-fusion protein was used to immunise mice, whose sera were used in a Western blot (34B) and for FACS analysis (34C).

The cp7170 protein was also identified in the 2D-PAGE experiment (Cpn0854).

These experiments show that cp7170 is a surface-exposed and immunoaccessible protein, and that it is a useful immunogen. These properties are not evident from the sequence alone.

**Example 35**

The following *C.pneumoniae* protein (PID 4377072) was expressed <SEQ ID 69; cp7072>:

```
  1 MDIKKLFCLF LCSSLIAMSP IYGKTGDYEK LTLTGINIID RNGLSETICS
 51 KEKLKKYTKV DFLAPQPYQK VMRMYKNKRG DNVSCLTAYH TNGQIKQYLE
101 CLNNRAYGRY REWHVNGNIK IQAEVIGGIA DLHPSAESGW LFDQTTFAYN
151 DEGILEAAIV YEKGLLEGSS VYYHTNGNIW KECPYHKGVP QGKFLTYTSS
201 GKLLKEQNYQ QGKRHGLSIR YSEDSEEDVL AWEEYHEGRL LKAEYLDPQT
251 HEIYATIHEG NGIQAIYGKY AVIETRAFYR GEPYGKVTRF DNSGTQIVQT
301 YNLLQGAKHG EEFFYPETG KPKLLLNWHE GILNGIVKTW YPGGTLESCK
351 ELVNNKKSGL LTIYYPEGQI MATEEYDNDL LIKGEYFRPG DRHPYSKIDR
401 GCGTAVFFSS AGTITKKIPY QDGKPLLN*
```

A predicted signal peptide is highlighted.

The cp7072 nucleotide sequence <SEQ ID 70> is:

```
   1  ATGGATATAA AAAAACTCTT TTGCTTATTT CTATGTTCTT CTCTAATTGC
  51  CATGAGTCCC ATTTATGGGA AAACAGGTGA CTATGAGAAA CTCACCCTTA
 101  CAGGGATCAA TATCATTGAT AGAAACGGCC TGTCAGAAAC TATTTGCTCT
 151  AAAGAGAAGC TAAAGAAATA CACCAAGGTA GACTTTCTTG CTCCCCAGCC
 201  CTATCAAAAG GTCATGAGGA TGTATAAAAA CAAACGCGGA GATAACGTTT
 251  CTTGTTTAAC AGCCTATCAC ACTAACGGGC AAATTAAGCA GTACCTGGAG
 301  TGTCTCAATA ATCGTGCTTA TGGAAGATAT CGTGAATGGC ACGTCAACGG
 351  GAATATCAAA ATCCAAGCTG AGGTTATCGG AGGTATTGCG GATCTTCATC
 401  CCTCAGCAGA GTCTGGCTGG CTATTTGATC AAACTACATT TGCCTATAAT
 451  GATGAAGGTA TCTTAGAAGC CGCTATCGTC TATGAAAAAG GGCTGCTCGA
 501  AGGATCTTCG GTGTATTACC ATACTAATGG GAATATTTGG AAAGAGTGTC
 551  CCTATCATAA GGGAGTTCCT CAAGGTAAAT TCCTGACATA CACATCTTCG
 601  GGGAAACTGC TCAAAGAACA GAATTACCAA CAAGGCAAAA GACACGGTCT
 651  TTCGATTCGC TACAGCGAAG ATTCCGAAGA AGATGTTTTA GCCTGGGAAG
 701  AATATCATGA GGGACGACTC CTAAAAGCAG AGTACTTAGA TCCTCAAACT
 751  CACGAAATCT ATGCGACTAT ACACGAAGGG AACGGCATTC AAGCAATCTA
 801  CGGCAAGTAT GCCGTTATAG AAACTAGGGC ATTTTACCGA GGGGAACCTT
 851  ATGGAAAAGT TACCAGATTC GACAACTCCG GAACACAGAT TGTCCAAACG
 901  TATAACCTTT TGCAAGGCGC GAAGCACGGA GAAGAATTTT TCTTTTATCC
 951  TGAGACAGGG AAACCCAAGC TGCTTCTTAA TTGGCATGAA GGAATTTTAA
1001  ATGGGATAGT AAAAACTTGG TATCCCGGAG GAACCTTAGA AAGTTGTAAA
1051  GAACTCGTAA ATAACAAAAA ATCCGGGTTA CTGACCATTT ACTACCCTGA
1101  AGGACAGATC ATGGCGACCG AAGAGTATGA TAATGATCTT CTAATTAAAG
1151  GAGAGTACTT CCGCCCTGGA GACCGTCATC CCTACTCTAA AATAGATCGT
1201  GGTTGTGGGA CTGCAGTATT TTTCTCGTCG GCGGGAACTA TTACTAAAAA
1251  AATCCCCTAT CAGGACGGCA AACCTTTGCT CAACTAG
```

The PSORT algorithm predicts a periplasmic location (0.688).

The protein was expressed in *E.coli* and purified as a his-tag product (Figure 35A) and as a GST-fusion product (Figure35B). The recombinant his-tag protein was used to immunise mi ce, whose sera were used in a Western blot (Figure 35C) and for FACS analysis.

These experiments show that cp7072 is a useful immunogen. These properties are not evident from the sequence alone.

## Example 36

The following *C.pneumoniae* protein (PID 4376879) was expressed <SEQ ID 71; cp6879>:

```
  1  MATPAQKSPT FQDPSFVREL GSNHPVFSPL TLEERGEMAI ARVQQCGWNH
 51  TIVKVSLIIL ALLTILGGGL LVGLLPAVPM FIGTGLIALG AVIFALALIL
101  CLYDSQGLPE ELPPVPEPQQ IQIEDLRNET REVLEGTLLE VLLKDRDAKD
151  PAVPQVVVDC EKRLGMLDRK LRREEEILYR STAHLKDEER YEFLLELLEM
201  RSLVADRLEF NRRSYERFVQ GIMTVRSEEG EKEISRLQDL ISLQQQTVQD
251  LRSRIDDEQK RCWTALQRIN QSQKDIQRAH DREASQRACE GTEMDCAERQ
301  QLEKDLRRQL KSMQEWIEMR GTIHQQEKAW RKQNAKLERL QEDLRLTGIA
351  FDEQSLFYRE YKEKYLSQKL DMQKILQEVN AEKSEKACLE SLVHDYEKQL
401  EQKDANLKKA AAVWEEELGK QQQEDYEQTQ EIRRLSTFIL EYQDSLREAE
451  KVEKDFQELQ QRYSRLQEEK QVKEKILEES MNHFADLFEK AQKENMAYKK
501  KLADLEGAAA PTEIGEDDDW VLTDSASLSQ KKIRELVEEN QELLKALAFK
551  SNELTQLVAD AVEAEKEISK LREHIEEQKE GLRALDKMHA QAIKDCEAAQ
601  RKCCDLESLL SPVREDAGMR FELEVELQRL QEENAQLRAE VERLEQEQFQ
651  G*
```

The cp6879 nucleotide sequence <SEQ ID 72> is:

```
   1  ATGGCAACAC CCGCTCAAAA ATCCCCTACA TTTCAAGATC CTAGTTTTGT
  51  AAGAGAGCTA GGCAGTAACC ACCCTGTCTT TTCCCCGCTA ACGCTTGAGG
 101  AAAGAGGGGA GATGGCAATA GCTCGAGTCC AGCAGTGTGG ATGGAATCAT
 151  ACAATTGTTA AGGTAAGTCT TATTATTCTT GCTCTTCTTA CTATTTTAGG
 201  GGGAGGATTA CTCGTAGGAT TGCTGCCAGC AGTTCCTATG TTTATTGGAA
 251  CAGGTCTGAT TGCTTTGGGA GCCGTTATAT TTGCTTTGGC TTTGATTTTA
 301  TGTCTTTATG ATTCTCAGGG CCTTCCTGAG GAACTCCCTC CGGTTCCTGA
 351  ACCACAACAA ATTCAGATTG AAGATTTAAG AAACGAGACC AGAGAAGTTC
 401  TTGAAGGGAC TCTTTTAGAG GTTCTCTTAA AGGATAGAGA CGCTAAGGAC
 451  CCTGCGGTGC CCCAGGTGGT TGTAGACTGT GAAAAGCGTC TTGGAATGTT
 501  GGATCGTAAG CTGCGACGTG AAGAGGAGAT TCTGTATCGC TCGACGGCCC
 551  ATCTTAAAGA CGAGGAAAGG TATGAGTTCT TGCTGGAGCT CTTGGAAATG
 601  CGTAGTCTGG TTGCCGATCG GCTAGAATTT AACCGTAGAA GTTATGAGCG
 651  ATTTGTTCAA GGAATTATGA CAGTTAGATC AGAGGAGGGG GAAAAAGAGA
 701  TTTCTCGTCT ACAAGATCTA ATCAGTTTGC AGCAGCAGAC GGTGCAAGAT
 751  TTAAGGAGTC GGATCGATGA CGAGCAGAAG AGATGCTGGA CGGCTTTACA
 801  ACGTATTAAC CAATCTCAGA AGGATATACA ACGGGCTCAT GATCGCGAGG
 851  CTTCGCAGCG TGCCTGTGAG GGCACAGAGA TGGATTGTGC AGAACGCCAG
 901  CAACTGGAGA AGGATTTAAG GAGACAGCTG AAATCTATGC AGGAGTGGAT
 951  TGAGATGAGG GGCACAATCC ATCAACAAGA GAAGGCTTGG CGTAAGCAGA
1001  ATGCCAAATT AGAAAGATTA CAAGAGGATC TGAGACTTAC TGGGATTGCT
1051  TTTGACGAAC AATCTCTGTT CTATCGCGAA TATAAAGAGA AATATCTGAG
1101  TCAGAAACTA GATATGCAAA AGATTTTACA GGAAGTCAAC GCAGAGAAAA
1151  GTGAGAAGGC TTGCTTAGAG AGTCTGGTCC ATGACTATGA GAAGCAGCTC
1201  GAACAAAAAG ATGCTAATCT GAAGAAAGCA GCAGCTGTTT GGGAAGAAGA
1251  ATTAGGGAAG CAGCAACAGG AAGACTACGA ACAAACCCAA GAAATTAGAC
1301  GTCTGAGTAC ATTCATTCTT GAGTACCAGG ACAGTCTGCG TGAGGCAGAA
1351  AAAGTTGAGA AAGATTTCCA AGAGCTACAA CAAAGGTATA GCCGTCTTCA
1401  AGAGGAGAAA CAGGTAAAAG AAAAAATCTT AGAAGAAAGT ATGAATCATT
1451  TTGCCGATCT CTTTGAGAAG GCTCAAAAGG AAAACATGGC CTACAAGAAG
1501  AAGTTAGCGG ATTTAGAGGG TGCCGCTGCT CCTACTGAGA TCGGTGAGGA
1551  CGATGACTGG GTACTCACAG ATTCTGCTTC TCTCAGCCAG AAGAAGATCC
1601  GCGAACTCGT GGAAGAGAAT CAAGAACTCC TGAAAGCACT TGCATTTAAA
1651  TCTAACGAAT TGACTCAACT GGTTGCCGAT GCTGTAGAAG CTGAAAAAGA
1701  AATCAGCAAG CTTCGAGAAC ACATAGAAGA GCAGAAAGAA GGATTACGAG
1751  CTCTTGATAA GATGCATGCA CAAGCGATCA AAGATTGCGA AGCTGCTCAG
1801  AGAAAATGCT GTGACCTTGA GAGCCTTCTC TCTCCTGTTC GAGAAGATGC
1851  TGGAATGAGA TTTGAGCTAG AGGTCGAGCT TCAAAGATTG CAAGAAGAAA
1901  ATGCACAGCT TAGAGCGGAG GTTGAAAGAC TAGAGCAAGA GCAATTTCAA
1951  GGATAA
```

The PSORT algorithm predicts an inner membrane location (0.646).

The protein was expressed in *E.coli* and purified as a his-tag product and as a GST-fusion product. The purified GST-fusion product is shown in Figure 36A. The recombinant GST-fusion protein was used to immunise mice, whose sera were used in a Western blot (Figure 36B) and for FACS analysis.

These experiments show that cp6879 is useful immunogen. These properties are not evident from the sequence alone.

## Example 37

The following *C.pneumoniae* protein (PID 4376767) was expressed <SEQ ID 73; cp6767>:

```
  1  MIKQIGRFFR AFIFIMPLSL TSCESKIDRN RIWIVGTNAT YPPFEYVDAQ
 51  GEVVGFDIDL AKAISEKLGK QLEVREFAFD ALILNLKKHR IDAILAGMSI
101  TPSRQKEIAL LPYYGDEVQE LMVVSKRSLE TPVLPLTQYS SVAVQTGTFQ
151  EHYLLSQPGI CVRSFDSTLE VIMEVRYGKS PVAVLEPSVG RVVLKDFPNL
201  VATRLELPPE CWVLGCGLGV AKDRPEEIQT IQQAITDLKS EGVIQSLTKK
251  WQLSEVAYE*
```

The cp6767 nucleotide sequence <SEQ ID 74> is:

```
  1  ATGATAAAAC AAATAGGCCG TTTTTTTAGA GCATTTATTT TTATAATGCC
 51  TTTATCTTTA ACAAGTTGTG AGTCTAAAAT CGATCGAAAT CGCATCTGGA
101  TTGTAGGTAC GAATGCTACA TATCCTCCTT TTGAGTATGT GGATGCTCAG
151  GGGGAAGTTG TAGGTTTCGA TATAGATTTG GCAAAGGCAA TTAGTGAAAA
201  ACTTGGCAAG CAATTGGAAG TTAGAGAATT CGCTTTCGAT GCTTTAATTT
251  TAAATTTAAA AAAACATCGT ATCGATGCAA TTTTAGCAGG AATGTCCATT
301  ACTCCTTCGC GTCAGAAGGA AATCGCCCTG CTTCCCTATT ATGGCGATGA
351  GGTTCAAGAG CTGATGGTGG TTTCTAAGCG GTCTTTAGAG ACCCCTGTGC
401  TTCCCCTAAC ACAGTATTCT TCTGTTGCTG TTCAGACAGG AACGTTTCAG
451  GAGCATTATC TTTTATCTCA GCCCGGAATT TGTGTCCGTT CTTTTGATAG
501  CACCTTGGAG GTGATTATGG AAGTTCGTTA TGGGAAATCT CCGGTTGCCG
551  TTCTAGAACC CTCGGTAGGA CGTGTCGTTC TTAAAGACTT CCCTAATCTT
601  GTTGCAACAA GATTAGAGCT CCCTCCTGAA TGTTGGGTGT TGGGCTGTGG
651  TCTCGGCGTA GCTAAAGATC GTCCTGAAGA AATACAAACG ATTCAACAAG
701  CGATTACAGA TTTAAAGAGC GAAGGGGTGA TTCAATCTTT AACCAAGAAA
751  TGGCAACTTT CTGAAGTTGC TTACGAATAG
```

The PSORT algorithm predicts an inner membrane location (0.083).

The protein was expressed in *E.coli* and purified as a his-tag product and as a GST-fusion product. The purified his-tag product is shown in Figure 37A. The recombinant his-tag protein was used to immunise mice, whose sera were used in a Western blot (Figure 37B) and for FACS analysis (Figure 37C). The GST-fusion was also used in a Western blot (Figure 37D).

The cp6767 protein was also identified in the 2D-PAGE experiment and showed good cross-reactivity with human sera, including sera from patients with pneumonitis.

These experiments show that cp6767 is a useful immunogen. These properties are not evident from the sequence alone.

**Example 38**

The following *C.pneumoniae* protein (PID 4376717) was expressed <SEQ ID 75; cp6717>:

```
  1  MMSRLRFRLA ALGIFFILLV PNSVSAKTIV ASDKEKVGVL VYDNSVEAFQ
 51  QILDCIDHAN FYVELCPCMT GGRTLKEMVD HLEARMDLVP ELCSYIIIQP
101  TFTDAEDQKL LKALKERHPN RFFYVFTGCP PSTSILAPNV IEMHIKLSII
151  DGKYCILGGT NFEEFMCTPG DEVPEKVDNP RLFVSGVRRP LAFRDQDIML
201  RSTAFGLQLR EEYHKQFAMW DYYAHHMWFI DNPEQFAGAC PPLTLEQAEE
251  TVFPGFDKHE DLVLVDSSKI RIVLGGPHDK QPNPVTQEYL KLIQGARSSV
301  KLAHMYFIPK DELLNALVDV SHNHGVHLSL ITNGCHELSP AITGPYAWGN
351  RINYFALLYG KRYPLWKKWF CEKLKPYERV SIYEFAIWET QLHKKCMIID
401  DEIFVIGSYN FGKKSDAFDY ESIVVIESPE VAAKANKVFN KDIGLSIPVS
451  HGDIFSWYFH SVHHTLGHLQ LTYMPA*
```

A predicted signal peptide is highlighted.

The cp6717 nucleotide sequence <SEQ ID 76> is:

```
   1  ATGATGAGTC GGTTGCGTTT TCGCTTGGCA GCTCTTGGAA TATTTTTTAT
  51  TTTGCTGGTT CCTAATTCTG TTTCAGCAAA GACAATCGTA GCTTCAGACA
 101  AGGAGAAGGT TGGAGTTCTT GTTTATGACA ATAGTGTAGA GGCCTTTCAA
 151  CAGATATTGG ATTGCATAGA TCATGCAAAT TTTTATGTAG AACTGTGTCC
 201  CTGCATGACA GGAGGCCGAA CGCTTAAAGA GATGGTAGAT CACCTCGAGG
 251  CTCGTATGGA TCTGGTTCCA GAGCTCTGTA GCTATATCAT TATCCAACCC
 301  ACGTTTACCG ATGCTGAAGA CCAAAAATTA CTCAAAGCTC TCAAAGAACG
 351  TCATCCCAAC CGGTTTTTCT ACGTTTTTAC AGGGTGCCCA CCCTCAACAA
 401  GCATCCTCGC TCCTAATGTC ATTGAAATGC ATATCAAACT TTCTATCATC
 451  GATGGGAAAT ATTGTATTTT AGGTGGTACC AATTTTGAAG AGTTTATGTG
 501  CACTCCAGGG GATGAGGTTC CTGAGAAAGT GGATAACCCA CGTTTATTTG
 551  TCAGTGGAGT GCGTCGGCCC CTAGCATTTC GTGATCAGGA TATCATGTTG
 601  CGTTCTACAG CATTCGGTTT GCAGCTCAGA GAAGAATATC ATAAGCAATT
 651  TGCTATGTGG GACTACTATG CACATCATAT GTGGTTCATT GATAATCCTG
 701  AACAGTTTGC AGGCGCCTGT CCTCCACTGA CTTTAGAACA AGCCGAGGAG
 751  ACAGTATTTC CTGGATTTGA CAAACATGAA GATCTTGTTC TTGTCGACTC
 801  TTCCAAGATC AGGATAGTTT TAGGTGGTCC CCACGATAAG CAACCCAATC
 851  CTGTGACTCA AGAATATTTG AAACTTATCC AGGGAGCTAG ATCTTCTGTG
 901  AAGCTTGCTC ACATGTATTT CATCCCTAAG GACGAGCTTT TAAATGCTCT
 951  TGTCGACGTT TCTCATAATC ACGGTGTTCA TCTGAGTTTA ATTACGAACG
1001  GCTGTCATGA ATTAAGTCCT GCAATTACAG GACCCTATGC TTGGGGAAAC
1051  CGTATTAACT ATTTCGCCTT GCTCTATGGG AAACGGTATC CTCTTTGGAA
1101  AAAATGGTTT TGCGAAAAGC TAAAACCTTA TGAGCGGGTT TCTATTTATG
1151  AGTTTGCTAT TTGGGAAACG CAGTTGCACA AGAAGTGTAT GATTATCGAT
1201  GATGAAATTT TTGTGATCGG AAGTTATAAT TTTGGAAAGA AAAGTGATGC
1251  CTTTGATTAC GAAAGTATTG TAGTTATCGA ATCTCCAGAA GTCGCTGCAA
1301  AAGCTAACAA AGTCTTCAAT AAAGATATCG GATTGTCGAT TCCTGTAAGT
1351  CATGGCGACA TTTTCTCTTG GTATTTCCAT TCCGTACACC ACACTTTGGG
1401  ACATTTGCAG CTGACCTATA TGCCAGCCTA G
```

The PSORT algorithm predicts a periplasmic location (0.939).

The protein was expressed in *E.coli* and purified as a GST-fusion (Figure 38A), as a his-tagged protein, and as a GST/his fusion product. The proteins were used to immunise mice, whose sera were used in a Western blot (Figure 38B) and for FACS analysis.

These experiments show that cp6717 is a useful immunogen. These properties are not evident from the sequence alone.

## Example 39

The following *C.pneumoniae* protein (PID 4376577) was expressed <SEQ ID 77; cp6577>:

  1  **MKKLLFSTFL LVLGSTSAAH A**NLGYVNLKR CLEESDLGKK ETEELEAMKQ
 51  QFVKNAEKIE EELTSIYNKL QDEDYMESLS DSASEELRKK FEDLSGEYNA
101  YQSQYYQSIN QSNVKRIQKL IQEVKIAAES VRSKEKLEAI LNEEAVLAIA
151  PGTDKTTEII AILNESFKKQ N*

A predicted signal peptide is highlighted.

The cp6577 nucleotide sequence <SEQ ID 78> is:

```
  1  ATGAAAAAAT TATTATTTTC TACATTTCTT CTTGTTTTAG GATCAACAAG
 51  CGCAGCTCAT GCAAATTTAG GCTATGTTAA TTTAAAGCGA TGTCTTGAAG
101  AATCCGATCT AGGTAAAAAG GAAACTGAAG AATTGGAAGC TATGAAACAG
151  CAGTTTGTAA AAAATGCTGA GAAAATAGAA GAAGAACTCA CTTCTATTTA
201  TAATAAGTTG CAAGATGAAG ATTACATGGA AAGCCTATCG GATTCTGCCT
251  CTGAAGAGTT GCGAAAGAAA TTCGAAGATC TTTCAGGAGA GTACAATGCG
301  TACCAGTCTC AGTACTATCA ATCTATCAAT CAAAGTAATG TAAAACGCAT
351  TCAAAAACTC ATTCAAGAAG TAAAAATAGC TGCAGAATCA GTGCGGTCCA
401  AAGAAAAACT AGAAGCTATC CTTAATGAAG AAGCTGTCTT AGCAATAGCA
451  CCTGGGACTG ATAAAACAAC CGAAATTATT GCTATTCTTA ACGAATCTTT
501  CAAAAAACAA AACTAG
```

The PSORT algorithm predicts a periplasmic space location (0.932).

The protein was expressed in *E.coli* and purified as a his-tag product (Figure 39A) and as a GST-fusion product (Figure 39B). The recombinant GST-fusion protein was used to immunise mice, whose sera were used in a Western blot (Figure 39C) and for FACS analysis.

The cp6577 protein was also identified in the 2D-PAGE experiment.

These experiments show that cp6577 is a useful immunogen. These properties are not evident from the sequence alone.

### Example 40

The following *C.pneumoniae* protein (PID 4376446) was expressed <SEQ ID 79; cp6446>:

1 **MKQPMSLIFS SVCLGLGLGS LSS**CNQKPSW NYHNTSTSEE FFVHGNKSVS
51 QLPHYPSAFR TTQIFSEEHN DPYVVAKTDE ESRKIWREIH KNLKIKGSYI
101 PISTYGSLMH PKSAALTLKT YRPHPIWING YERSFNIDTG KYLKNGSRRR
151 TSHDGPKNRA VLNLIKSSGR RCNAIGLEMT EEDFVIARRR EGVYSLYPVE
201 VCSYPQGNPF VIAYAWIADE SACSKEVLPV KGYYSLVWES VSSSDSLNAF
251 GDSFAEDYLR STFLANGTSI LCVHESYKKV PPQP*

A predicted signal peptide is highlighted.

The cp6446 nucleotide sequence <SEQ ID 80> is:

1 ATGAAACAGC CCATGTCTCT TATCTTTTCA AGTGTATGTT TAGGATTAGG
51 TCTTGGATCT CTTTCCTCCT GTAATCAAAA GCCCTCTTGG AATTATCACA
101 ACACTTCAAC GAGCGAAGAA TTCTTTGTTC ATGGAAATAA GAGTGTTTCG
151 CAACTGCCTC ATTATCCTTC TGCATTTCGT ACGACTCAAA TCTTTTCTGA
201 AGAGCACAAT GATCCTTATG TCGTAGCTAA GACTGATGAA GAGTCTCGTA
251 AAATTTGGAG AGAAATCCAT AAAAATCTCA AAATCAAAGG TTCTTACATT
301 CCCATATCGA CTTATGGAAG TCTGATGCAC CCAAAATCAG CAGCTCTTAC
351 ATTAAAAACG TATCGTCCAC ATCCTATTTG GATAAATGGA TACGAGCGTT
401 CTTTTAATAT AGACACAGGA AAGTACTTAA AAAACGGAAG TCGCCGTAGA
451 ACTTCTCACG ATGGTCCGAA AAATCGAGCT GTACTGAATC TCATTAAATC
501 TTCGGGACGA CGCTGTAATG CTATAGGCCT TGAGATGACA GAAGAAGACT
551 TTGTAATAGC TAGAAGGCGA GAAGGTGTTT ATAGCCTGTA TCCCGTTGAA
601 GTGTGCTCGT ATCCTCAGGG GAATCCTTTT GTCATTGCTT ATGCCTGGAT
651 TGCAGATGAG AGTGCTTGCT CAAAAGAGGT CCTACCTGTA AAAGGGTACT
701 ATTCTTTAGT CTGGGAAAGC GTTTCTTCCT CTGATTCTCT GAATGCTTTT
751 GGAGATTCCT TTGCAGAGGA CTACCTCAGA AGCACGTTTT TAGCAAACGG
801 AACTTCTATA CTCTGTGTTC ATGAAAGCTA TAAGAAAGTT CCTCCTCAGC
851 CCTAA

The PSORT algorithm predicts an inner membrane location (0.177).

The protein was expressed in *E.coli* and purified as a his-tag product and a GST-fusion product. The GST-fusion product is shown in Figure 40A. The recombinant his-tag protein was used to immunise mice, whose sera were used in a Western blot (Figure 40B) and for FACS analysis.

These experiments show that cp6446 is a useful immunogen. These properties are not evident from the sequence alone.

### Example 41

The following *C.pneumoniae* protein (PID 4377108) was expressed <SEQ ID 81; cp7108>:

1 **MSKKIKVLGH LTLCTLFRGV LCA**AALSNIG YASTSQESPY QKSIEDWKGY
51 TFTDLELLSK EGWSEAHAVS GNGSRIVGAS GAGQGSVTAV IWESHLIKHL
101 GTLGGEASSA EGISKDGEVV VGWSDTREGY THAFVFDGRD MKDLGTLGAT
151 YSVARGVSGD GSIIVGVSAT ARGEDYGWQV GVKWEKGKIK QLKLLPQGLW

```
201  SEANAISEDG TVIVGRGEIS RNHIVAVKWN KNAVYSLGTL GGSVASAEAI
251  SANGKVIVGW STTNNGETHA FMHKDETMHD LGTLGGGFSV ATGVSADGRA
301  IVGFSAVKTG EIHAFYYAEG EMEDLTTLGG EEARVFDISS EGNDIIGSIK
351  TDAGAERAYL FHIHK*
```

A predicted signal peptide is highlighted.

The cp7108 nucleotide sequence <SEQ ID 82> is:

```
   1  ATGAGTAAGA AGATAAAGGT TCTAGGTCAT TTGACGCTCT GCACTCTGTT
  51  TAGAGGAGTG CTGTGTGCAG CGGCCCTTTC CAACATAGGA TATGCGAGTA
 101  CTTCTCAGGA ATCACCATAT CAGAAGTCTA TAGAAGACTG GAAAGGGTAT
 151  ACCTTTACAG ATCTTGAGTT ACTGAGTAAG GAAGGGTGGT CTGAAGCTCA
 201  TGCAGTTTCT GGAAATGGCA GTAGAATTGT AGGAGCTTCG GGAGCTGGCC
 251  AAGGTAGTGT GACTGCTGTC ATATGGGAAA GTCACCTGAT AAAACATCTC
 301  GGCACTTTAG GTGGCGAGGC TTCATCTGCA GAGGGAATTT CAAAGGATGG
 351  AGAGGTGGTC GTTGGGTGGT CAGATACTAG AGAGGGATAT ACTCATGCCT
 401  TTGTCTTCGA CGGTAGAGAT ATGAAAGATC TCGGTACTCT AGGAGCTACC
 451  TATTCTGTAG CAAGGGGTGT TTCTGGAGAT GGTAGTATCA TCGTAGGAGT
 501  CTCTGCAACT GCTCGTGGAG AGGATTACGG ATGGCAAGTT GGTGTCAAGT
 551  GGGAAAAAGG GAAAATCAAA CAATTGAAGT TGTTGCCTCA AGGTCTCTGG
 601  TCTGAGGCGA ATGCAATCTC TGAGGATGGT ACGGTGATTG TCGGGAGAGG
 651  GGAAATCTCT CGCAATCACA TCGTTGCTGT AAAATGGAAT AAAAATGCTG
 701  TGTATAGTTT GGGGACTCTC GGAGGTAGTG TCGCTTCAGC AGAGGCTATA
 751  TCGGCAAATG GGAAAGTAAT TGTAGGATGG TCCACGACTA ATAATGGTGA
 801  GACTCATGCC TTTATGCACA AAGATGAGAC AATGCACGAT CTCGGCACTC
 851  TAGGAGGAGG TTTTTCTGTC GCAACTGGAG TTTCTGCTGA TGGGAGAGCC
 901  ATCGTAGGAT TTTCAGCAGT GAAGACCGGA GAAATTCATG CTTTTTACTA
 951  TGCAGAAGGA GAAATGGAGG ATTTAACAAC TTTGGGAGGG GAAGAAGCTC
1001  GAGTGTTCGA CATATCTAGC GAAGGAAACG ATATCATTGG CTCTATAAAA
1051  ACTGACGCTG GAGCTGAACG CGCCTATCTG TTCCATATAC ATAAATAA
```

The PSORT algorithm predicts an outer membrane location (0.921).

The protein was expressed in *E.coli* and purified as a GST-fusion product, as shown in Figure 41A. The recombinant protein was used to immunise mice, whose sera were used in a Western blot (Figure 41B) and for FACS analysis (Figure 41C). A his-tagged protein was also expressed.

The cp7108 protein was also identified in the 2D-PAGE experiment.

These experiments show that cp7108 is a surface-exposed and immunoaccessible protein, and that it is a useful immunogen. These properties are not evident from the sequence alone.

### Example 42

The following *C.pneumoniae* protein (PID 4377287) was expressed <SEQ ID 83; cp7287>:

```
   1  MVAKKTVRSY RSSFSHSVIV AILSAGIAFE AHSLHSSELD LGVFNKQFEE
  51  HSAHVEEAQT SVLKGSDPVN PSQKESEKVL YTQVPLTQGS SGESLDLADA
 101  NFLEHFQHLF EETTVFGIDQ KLVWSDLDTR NFSQPTQEPD TSNAVSEKIS
 151  SDTKENRKDL ETEDPSKKSG LKEVSSDLPK SPETAVAAIS EDLEISENIS
 201  ARDPLQGLAF FYKNTSSQSI SEKDSSFQGI IFSGSGANSG LGFENLKAPK
 251  SGAAVYSDRD IVFENLVKGL SFISCESLED GSAAGVNIVV THCGDVTLTD
 301  CATGLDLEAL RLVKDFSRGG AVFTARNHEV QNNLAGGILS VVGNKGAIVV
 351  EKNSAEKSNG GAFACGSFVY SNNENTALWK ENQALSGGAI SSASDIDIQG
 401  NCSAIEFSGN QSLIALGEHI GLTDFVGGGA LAAQGTLTLR NNAVVQCVKN
 451  TSKTHGGAIL AGTVDLNETI SEVAFKQNTA ALTGGALSAN DKVIIANNFG
 501  EILFEQNEVR NHGGAIYCGC RSNPKLEQKD SGENINIIGN SGAITFLKNK
 551  ASVLEVMTQA EDYAGGGALW GHNVLLDSNS GNIQFIGNIG GSTFWIGEYV
 601  GGGAILSTDR VTISNNSGDV VFKGNKGQCL AQKYVAPQET APVESDASST
 651  NKDEKSLNAC SHGDHYPPKT VEEEVPPSLL EEHPVVSSTD IRGGGAILAQ
 701  HIFITDNTGN LRFSGNLGGG EESSTVGDLA IVGGGALLST NEVNVCSNQN
 751  VVFSDNVTSN GCDSGGAILA KKVDISANHS VEFVSNGSGK FGGAVCALNE
 801  SVNITDNGSA VSFSKNRTRL GGAGVAAPQG SVTICGNQGN IAFKENFVFG

 851  SENQRSGGGA IIANSSVNIQ DNAGDILFVS NSTGSYGGAI FVGSLVASEG
 901  SNPRTLTITG NSGDILFAKN STQTAASLSE KDSFGGGAIY TQNLKIVKNA
 951  GNVSFYGNRA PSGAGVQIAD GGTVCLEAFG GDILFEGNIN FDGSFNAIHL
1001  CGNDSKIVEL SAVQDKNIIF QDAITYEENT IRGLPDKDVS PLSAPSLIFN
1051  SKPQDDSAQH HEGTIRFSRG VSKIPQIAAI QEGTLALSQN AELWLAGLKQ
1101  ETGSSIVLSA GSILRIFDSQ VDSSAPLPTE NKEETLVSAG VQINMSSPTP
1151  NKDKAVDTPV LADIISITVD LSSFVPEQDG TLPLPPEIII PKGTKLHSNA
1201  IDLKIIDPTN VGYENHALLS SHKDIPLISL KTAEGMTGTP TADASLSNIK
1251  IDVSLPSITP ATYGHTGVWS ESKMEDGRLV VGWQPTGYKL NPEKQGALVL
1301  NNLWSHYTDL RALKQEIFAH HTIAQRMELD FSTNVWGSGL GVVEDCQNIG
1351  EFDGFKHHLT GYALGLDTQL VEDFLIGGCF SQFFGKTESQ SYKAKNDVKS
1401  YMGAAYAGIL AGPWLIKGAF VYGNINNDLT TDYGTLGIST GSWIGKGFIA
1451  GTSIDYRYIV NPRRFISAIV STVVPFVEAE YVRIDLPEIS EQGKEVRTFQ
1501  KTRFENVAIP FGFALEHAYS RGSRAEVNSV QLAYVFDVYR KGPVSLITLK
1551  DAAYSWKSYG VDIPCKAWKA RLSNNTEWNS YLSTYLAFNY EWREDLIAYD
1601  FNGGIRIIF*
```

A predicted signal peptide is highlighted.

The cp7287 nucleotide sequence <SEQ ID 84> is:

```
   1 ATGGTAGCGA AAAAAACAGT ACGATCTTAT AGGTCTTCAT TTTCTCATTC
  51 CGTAATAGTA GCAATATTGT CAGCAGGCAT TGCTTTTGAA GCACATTCCT
 101 TACACAGCTC AGAACTAGAT TTAGGTGTAT TCAATAAACA GTTTGAGGAA
 151 CATTCTGCTC ATGTTGAAGA GGCTCAAACA TCTGTTTTAA AGGGATCAGA
 201 TCCTGTAAAT CCCTCTCAGA AAGAATCCGA GAAGGTTTTG TACACTCAAG
 251 TGCCTCTTAC CCAAGGAAGC TCTGGAGAGA GTTTGGATCT CGCCGATGCT
 301 AATTTCTTAG AGCATTTTCA GCATCTTTTT GAAGAGACTA CAGTATTTGG
 351 TATCGATCAA AAGCTGGTTT GGTCAGATTT AGATACTAGG AATTTTTCCC
 401 AACCCACTCA AGAACCTGAT ACAAGTAATG CTGTAAGTGA GAAAATCTCC
 451 TCAGATACCA AAGAGAATAG AAAAGACCTA GAGACTGAAG ATCCTTCAAA
 501 AAAAAGTGGC CTTAAAGAAG TTTCATCAGA TCTCCCTAAA AGTCCTGAAA
 551 CTGCAGTAGC AGCTATTTCT GAAGATCTTG AAATCTCAGA AAACATTTCA
 601 GCAAGAGATC CTCTTCAGGG TTTAGCATTT TTTTATAAAA ATACATCTTC
 651 TCAGTCTATC TCTGAAAAGG ATTCTTCATT TCAAGGAATT ATCTTTTCTG
 701 GTTCAGGAGC TAATTCAGGG CTAGGTTTTG AAAATCTTAA GGCGCCGAAA
 751 TCTGGGGCTG CAGTTTATTC TGATCGAGAT ATTGTTTTTG AAAATCTTGT
 801 TAAAGGATTG AGTTTTATAT CTTGTGAATC TTTAGAAGAT GGCTCTGCCG
 851 CAGGTGTAAA CATTGTTGTG ACCCATTGTG GTGATGTAAC TCTCACTGAT
 901 TGTGCCACTG GTTTAGACCT TGAAGCTTTA CGTCTGGTTA AAGATTTTTC
 951 TCGTGGAGGA GCTGTTTTCA CTGCTCGCAA CCATGAAGTG CAAAATAACC
1001 TTGCAGGTGG AATTCTATCC GTTGTAGGCA ATAAAGGAGC TATTGTTGTA
1051 GAGAAAAATA GTGCTGAGAA GTCCAATGGA GGAGCTTTTG CTTGCGGAAG
1101 TTTTGTTTAC AGTAACAACG AAAACACCGC CTTGTGGAAA GAAAATCAAG
1151 CATTATCAGG AGGAGCCATA TCCTCAGCAA GTGATATTGA TATTCAAGGG
1201 AACTGTAGCG CTATTGAATT TTCAGGAAAC CAGTCTCTAA TTGCTCTTGG
1251 AGAGCATATA GGGCTTACAG ATTTTGTAGG TGGAGGAGCT TTAGCTGCTC
1301 AAGGGACGCT TACCTTAAGA AATAATGCAG TAGTGCAATG TGTTAAAAAC
1351 ACTTCTAAAA CACATGGTGG AGCTATTTTA GCAGGTACTG TTGATCTCAA
1401 CGAAACAATT AGCGAAGTTG CCTTTAAGCA GAATACAGCA GCTCTAACTG
1451 GAGGTGCTTT AAGTGCAAAT GATAAGGTTA TAATTGCAAA TAACTTTGGA
1501 GAAATTCTTT TTGAGCAAAA CGAAGTGAGG AATCACGGAG GAGCCATTTA
1551 TTGTGGATGT CGATCTAATC CTAAGTTAGA ACAAAAGGAT TCTGGAGAGA
1601 ACATCAATAT TATTGGAAAC TCCGGAGCTA TCACTTTTTT AAAAAATAAG
1651 GCTTCTGTTT TAGAAGTGAT GACACAAGCT GAAGATTATG CTGGTGGAGG
1701 CGCTTTATGG GGGCATAATG TTCTTCTAGA TTCCAATAGT GGGAATATTC
1751 AATTTATAGG AAATATAGGT GGAAGTACCT TCTGGATAGG AGAATATGTC
1801 GGTGGTGGTG CGATTCTCTC TACTGATAGA GTGACAATTT CTAATAACTC
1851 TGGAGATGTT GTTTTTAAAG GAAACAAAGG CCAATGTCTT GCTCAAAAAT
1901 ATGTAGCTCC TCAAGAAACA GCTCCCGTGG AATCAGATGC TTCATCTACA
1951 AATAAAGACG AGAAGAGCCT TAATGCTTGT AGTCATGGAG ATCATTATCC
2001 TCCTAAAACT GTAGAAGAGG AAGTGCCACC TTCATTGTTA GAAGAACATC
2051 CTGTTGTTTC TTCGACAGAT ATTCGTGGTG GTGGGGCCAT TCTAGCTCAA
2101 CATATCTTTA TTACAGATAA TACAGGAAAT CTGAGATTCT CTGGGAACCT
2151 TGGTGGTGGT GAAGAGTCTT CTACTGTCGG TGATTTAGCT ATCGTAGGAG
2201 GAGGTGCTTT GCTTTCTACT AATGAAGTTA ATGTTTGCAG TAACCAAAAT
2251 GTTGTTTTTT CTGATAACGT GACTTCAAAT GGTTGTGATT CAGGGGGAGC
2301 TATTTTAGCT AAAAAAGTAG ATATCTCCGC GAACCACTCG GTTGAATTTG
```

```
2351  TCTCTAATGG TTCAGGGAAA TTCGGTGGTG CCGTTTGCGC TTTAAACGAA
2401  TCAGTAAACA TTACGGACAA TGGCTCGGCA GTATCATTCT CTAAAAATAG
2451  AACACGTCTT GGCGGTGCTG GAGTTGCAGC TCCTCAAGGC TCTGTAACGA
2501  TTTGTGGAAA TCAGGGAAAC ATAGCATTTA AAGAGAACTT TGTTTTTGGC
2551  TCTGAAAATC AAAGATCAGG TGGAGGAGCT ATCATTGCTA ACTCTTCTGT
2601  AAATATTCAG GATAACGCAG GAGATATCCT ATTTGTAAGT AACTCTACGG
2651  GATCTTATGG AGGTGCTATT TTTGTAGGAT CTTTGGTTGC TTCTGAAGGC
2701  AGCAACCCAC GAACGCTTAC AATTACAGGC AACAGTGGGG ATATCCTATT
2751  TGCTAAAAAT AGCACGCAAA CAGCCGCTTC TTTATCAGAA AAAGATTCCT
2801  TTGGTGGAGG GGCCATCTAT ACACAAAACC TCAAAATTGT AAAGAATGCA
2851  GGGAACGTTT CTTTCTATGG CAACAGAGCT CCTAGTGGTG CTGGTGTCCA
2901  AATTGCAGAC GGAGGAACTG TTTGTTTAGA GGCTTTTGGA GGAGATATCT
2951  TATTTGAAGG GAATATCAAT TTTGATGGGA GTTTCAATGC GATTCACTTA
3001  TGCGGGAATG ACTCAAAAAT CGTAGAGCTT TCTGCTGTTC AAGATAAAAA
3051  TATTATTTTC CAAGATGCAA TTACTTATGA AGAGAACACA ATTCGTGGCT
3101  TGCCAGATAA AGATGTCAGT CCTTTAAGTG CCCCTTCATT AATTTTTAAC
3151  TCCAAGCCAC AAGATGACAG CGCTCAACAT CATGAAGGGA CGATACGGTT
3201  TTCTCGAGGG GTATCTAAAA TTCCTCAGAT TGCTGCTATA CAAGAGGGAA
3251  CCTTAGCTTT ATCACAAAAC GCAGAGCTTT GGTTGGCAGG ACTTAAACAG
3301  GAAACAGGAA GTTCTATCGT ATTGTCTGCG GGATCTATTC TCCGTATTTT
3351  TGATTCCCAG GTTGATAGCA GTGCGCCTCT TCCTACAGAA AATAAAGAGG
3401  AGACTCTTGT TTCTGCCGGA GTTCAAATTA ACATGAGCTC TCCTACACCC
3451  AATAAAGATA AAGCTGTAGA TACTCCAGTA CTTGCAGATA TCATAAGTAT
3501  TACTGTAGAT TTGTCTTCAT TTGTTCCTGA GCAAGACGGA ACTCTTCCTC
3551  TTCCTCCTGA AATTATCATT CCTAAGGGAA CAAAATTACA TTCTAATGCC
3601  ATAGATCTTA AGATTATAGA TCCTACCAAT GTGGGATATG AAAATCATGC
3651  TCTTCTAAGT TCTCATAAAG ATATTCCATT AATTTCTCTT AAGACAGCGG
3701  AAGGAATGAC AGGGACGCCT ACAGCAGATG CTTCTCTATC TAATATAAAA
3751  ATAGATGTAT CTTTACCTTC GATCACACCA GCAACGTATG GTCACACAGG
3801  AGTTTGGTCT GAAAGTAAAA TGGAAGATGG AAGACTTGTA GTCGGTTGGC
3851  AACCTACGGG ATATAAGTTA AATCCTGAGA AGCAAGGGGC TCTAGTTTTG
3901  AATAATCTCT GGAGTCATTA TACAGATCTT AGAGCTCTTA AGCAGGAGAT
3951  CTTTGCTCAT CATACGATAG CTCAAAGAAT GGAGTTAGAT TTCTCGACAA
4001  ATGTCTGGGG ATCAGGATTA GGTGTTGTTG AAGATTGTCA GAACATCGGA
4051  GAGTTTGATG GGTTCAAACA TCATCTCACA GGGTATGCCC TAGGCTTGGA
4101  TACACAACTA GTTGAAGACT TCTTAATTGG AGGATGTTTC TCACAGTTCT
4151  TTGGTAAAAC TGAAAGCCAA TCCTACAAAG CTAAGAACGA TGTGAAGAGT
4201  TATATGGGAG CTGCTTATGC GGGGATTTTA GCAGGTCCTT GGTTAATAAA
4251  AGGAGCTTTT GTTTACGGTA ATATAAACAA CGATTTGACT ACAGATTACG
4301  GTACTTTAGG TATTTCAACA GGTTCATGGA TAGGAAAAGG GTTTATCGCA
4351  GGCACAAGCA TTGATTACCG CTATATTGTA AATCCTCGAC GGTTTATATC
4401  GGCAATCGTA TCCACAGTGG TTCCTTTTGT AGAAGCCGAG TATGTCCGTA
4451  TAGATCTTCC AGAAATTAGC GAACAGGGTA AAGAGGTTAG AACGTTCCAA
4501  AAAACTCGTT TTGAGAATGT CGCCATTCCT TTTGGATTTG CTTTAGAACA
4551  TGCTTATTCG CGTGGCTCAC GTGCTGAAGT GAACAGTGTA CAGCTTGCTT
4601  ACGTCTTTGA TGTATATCGT AAGGGACCTG TCTCTTTGAT TACACTCAAG
4651  GATGCTGCTT ATTCTTGGAA GAGTTATGGG GTAGATATTC CTTGTAAAGC
4701  TTGGAAGGCT CGCTTGAGCA ATAATACGGA ATGGAATTCA TATTTAAGTA
4751  CGTATTTAGC GTTTAATTAT GAATGGAGAG AAGATCTGAT AGCTTATGAC
4801  TTCAATGGTG GTATCCGTAT TATTTTCTAG
```

The PSORT algorithm predicts an inner membrane location (0.106).

The protein was expressed in *E.coli* and purified as a GST-fusion product, as shown in Figure 42A. The recombinant protein was used to immunise mice, whose sera were used in a Western blot (Figure 42B) and for FACS analysis (Figure 42C). A his-tagged protein was also expressed.

The cp7287 protein was also identified in the 2D-PAGE experiment and showed good cross-reactivity with human sera, including sera from patients with pneumonitis.

These experiments show that cp7287 is a surface-exposed and immunoaccessible protein, and that it is a useful immunogen. These properties are not evident from the sequence alone.

## Example 43

The following *C.pneumoniae* protein (PID 4377105) was expressed <SEQ ID 85; cp7105>:

```
  1  MSLYQKWWNS QLKKSLCYST VAALIFMIPS QESFADSLID LNLGLDPSVE
 51  CLSGDGAFSV GYFTKAGSTP VEYQPFKYDV SKKTFTILSV ETANQSGYAY
101  GISYDGTITV GTCSLGAGKY NGAKWSADGT LTPLTGITGG TSHTEARAIS
151  KDTQVIEGFS YDASGQPKAV QWASGATTVT QLADISGGSR SSYAYAISDD
201  GTIIVGSMES TITRKTTAVK WVNNVPTYLG TLGGDASTGL YISGDGTVIV
251  GAANTATVTN GNQESHAYMY KDNQMKD*
```

The cp7105 nucleotide sequence <SEQ ID 86> is:

```
  1  GTGAGTCTAT ATCAAAAATG GTGGAACAGT CAGTTAAAGA AGAGCCTCTG
 51  CTATTCGACT GTTGCTGCTC TAATATTTAT GATTCCTTCT CAAGAATCCT
101  TTGCAGATAG TCTTATAGAT TTAAATTTAG GTTTAGATCC TTCGGTCGAA
151  TGTCTGTCAG GAGATGGTGC ATTTTCTGTT GGGTATTTTA CTAAGGCGGG
201  ATCGACTCCC GTAGAATATC AGCCGTTTAA ATACGACGTA TCTAAGAAGA
251  CATTCACAAT CCTTTCCGTA GAAACGGCAA ATCAGAGCGG CTATGCTTAC
301  GGAATCTCCT ACGATGGCAC GATCACTGTA GGAACGTGTA GCCTAGGTGC
351  AGGAAAATAT AACGGCGCAA AATGGAGTGC GGATGGCACT TTAACACCCT
401  TAACTGGAAT CACGGGGGGG ACGTCACATA CGGAAGCGCG TGCGATTTCT
451  AAGGATACTC AGGTGATCGA GGGTTTCTCA TATGATGCTT CAGGGCAACC
501  CAAGGCTGTG CAGTGGGCAA GCGGAGCGAC TACAGTAACA CAATTAGCAG
551  ATATTTCAGG AGGCTCTAGA AGCTCTTATG CGTATGCTAT ATCTGATGAT
601  GGCACGATTA TTGTTGGGTC TATGGAGAGC ACGATAACAA GGAAAACTAC
651  AGCTGTAAAA TGGGTAAATA ATGTTCCTAC GTATCTGGGA ACCTTAGGAG
701  GAGATGCTTC TACAGGTCTT TATATTTCTG GAGACGGCAC CGTGATTGTA
751  GGTGCGGCAA ATACAGCAAC TGTAACCAAT GGGAATCAGG AATCCCACGC
801  CTATATGTAT AAAGATAACC AAATGAAAGA TTGA
```

The PSORT algorithm predicts an inner membrane location (0.100).

The protein was expressed in *E.coli* and purified as a GST-fusion product, as shown in Figure 43A. The recombinant protein was used to immunise mice, whose sera were used in a Western blot (Figure 43B) and for FACS analysis (Figure 43C). A his-tagged protein was also expressed.

This protein also showed good cross-reactivity with human sera, including sera from patients with pneumonitis.

These experiments show that cp7105 is a surface-exposed and immunoaccessible protein, and that it is a useful immunogen. These properties are not evident from the sequence alone.

**Example 44**

The following *C.pneumoniae* protein (PID 4376802) was expressed <SEQ ID 87; cp6802>:

```
  1  MSNQLQPCIS LGCVSYINSF PLSLQLIKRN DIRCVLAPPA DLLNLLIEGK
 51  LDVALTSSLG AISHNLGYVP GFGIAANQRI LSVNLYAAPT FFNSPQPRIA
101  ATLESRSSIG LLKVLCRHLW RIPTPHILRF ITTKVLRQTP ENYDGLLLIG
151  DAALQHPVLP GFVTYDLASG WYDLTKLPFV FALLLHSTSW KEHPLPNLAM
201  EEALQQFESS PEEVLKEAHQ HTGLPPSLLQ EYYALCQYRL GEEHYESFEK
251  FREYYGTLYQ QARL*
```

A predicted signal peptide is highlighted.

The cp6802 nucleotide sequence <SEQ ID 88> is:

```
  1  ATGTCTAACC AACTCCAGCC ATGTATAAGC TTAGGCTGCG TAAGTTATAT
 51  TAATTCCTTT CCGCTGTCCC TACAACTCAT AAAAAGAAAC GATATTCGCT
101  GTGTTCTTGC TCCCCCTGCA GACCTCCTCA ACTTGCTAAT CGAAGGGAAA
151  CTCGATGTTG CTTTGACCTC ATCCCTAGGA GCTATCTCTC ATAACTTGGG
201  GTATGTCCCC GGCTTTGGAA TTGCAGCAAA CCAACGTATC CTCAGTGTAA
```

```
251  ACCTCTATGC AGCTCCCACT TTCTTTAACT CACCGCAACC TCGGATTGCC
301  GCAACTTTAG AAAGTCGCTC CTCTATAGGA CTCTTAAAAG TGCTTTGTCG
351  TCATCTCTGG CGCATCCCAA CTCCTCATAT CCTAAGATTC ATAACTACAA
401  AAGTACTCAG ACAAACCCCT GAAAATTATG ATGGCCTCCT CCTAATCGGA
451  GATGCAGCGC TACAACATCC TGTACTTCCT GGATTTGTAA CCTATGACCT
501  TGCCTCGGGG TGGTATGATC TTACAAAGCT ACCTTTTGTA TTTGCTCTTC
551  TTCTACACAG CACCTCTTGG AAAGAACATC CCCTACCCAA CCTTGCGATG
601  GAAGAAGCCC TCCAACAGTT CGAATCTTCA CCCGAAGAAG TCCTTAAAGA
651  AGCTCATCAA CATACAGGTC TGCCCCCTTC TCTTCTTCAA GAATACTATG
701  CCCTATGCCA GTACCGTCTA GGAGAAGAAC ACTACGAAAG CTTTGAAAAA
751  TTCCGGGAAT ATTATGGAAC CCTCTACCAA CAAGCCCGAC TGTAA
```

The PSORT algorithm predicts an inner membrane location (0.060).

The protein was expressed in *E.coli* and purified as a GST-fusion product, as shown in Figure 44A. The recombinant protein was used to immunise mice, whose sera were used in a Western blot (Figure 44B) and for FACS analysis (Figure 44C). A his-tagged protein was also expressed.

These experiments show that cp6802 is a surface-exposed and immunoaccessible protein, and that it is a useful immunogen. These properties are not evident from the sequence alone.

## Example 45

The following *C.pneumoniae* protein (PID 4376390) was expressed <SEQ ID 89; cp6390>:

```
  1  MVFSYYCMGL FFFSGAISSC GLLVSLGVGL GLSVLGVLLL LLAGLLLFKI
 51  QSMLREVPKA PDLLDLEDAS ERLRVKASRS LASLPKEISQ LESYIRSAAN
101  DLNTIKTWPH KDQRLVETVS RKLERLAAAQ NYMISELCEI SEILEEEEHH
151  LILAQESLEW IGKSLFSTFL DMESFLNLSH LSEVRPYLAV NDPRLLEITE
201  ESWEVVSHFI NVTSAFKKAQ ILFKNNEHSR MKKKLESVQE LLETFIYKSL
251  KRSYRELGCL SEKMRIIHDN PLFPWVQDQQ KYAHAKNEFG EIARCLEEFE
301  KTFFWLDEEC AISYMDCWDF LNESIQNKKS RVDRDYISTK KIALKDRART
351  YAKVLLEENP TTEGKIDLQD AQRAFERQSQ EFYTLEHTET KVRLEALQQC
401  FSDLREATNV RQVRFTNSEN ANDLKESFEK IDKERVRYQK EQRLYWETID
451  RNEQELREEI GESLRLQNRR KGYRAGYDAG RLKGLLRQWK KNLRDVEAHL
501  EDATMDFEHE VSKSELCSVR ARLEVLEEEL MDMSPKVADI EELLSYEERC
551  ILPIRENLER AYLQYNKCSE ILSKAKFFFP EDEQLLVSEA NLREVGAQLK
601  QVQGKCQERA QKFAIFEKHI QEQKSLIKEQ VRSFDLAGVG FLKSELLSIA
651  CNLYIKAVVK ESIPVDVPCM QLYYSYYEDN EAVVRNRLLN MTERYQNFKR
701  SLNSIQFNGD VLLRDPVYQP EGHETRLKER ELQETTLSCK KLKVAQDRLS
751  ELESRLSRR
```

A predicted signal peptide is highlighted.

The cp6390 nucleotide sequence <SEQ ID 90> is:

```
   1  TTGGTATTCT CATACTATTG CATGGGATTA TTTTTTTTCT CTGGAGCTAT
  51  TTCTAGTTGT GGTCTTTTAG TGTCTCTAGG AGTTGGTTTA GGACTTAGTG
 101  TTTTAGGAGT ACTTTTACTT CTCTTAGCAG GTCTTTTGCT TTTTAAGATC
 151  CAAAGTATGC TTCGAGAGGT GCCTAAGGCT CCTGATCTAT TAGATTTAGA
 201  AGATGCAAGT GAACGGCTTA GAGTAAAGGC TAGCCGTTCT TTAGCAAGCC
 251  TCCCGAAGGA AATCAGTCAG CTAGAGAGCT ACATTCGTTC TGCAGCTAAT
 301  GATCTAAATA CAATTAAGAC TTGGCCGCAT AAAGATCAAA GACTCGTCGA
 351  GACCGTGTCA CGAAAATTAG AGCGTCTGGC AGCTGCTCAA AACTATATGA
 401  TTTCTGAACT CTGCGAGATT AGTGAGATTC TTGAGGAAGA GGAGCATCAT
 451  CTAATTTTGG CTCAGGAATC TCTAGAATGG ATAGGTAAGA GTCTATTTTC
 501  TACCTTTCTG GACATGGAAT CTTTTTTAAA TTTGAGCCAT CTATCTGAAG
 551  TGCGTCCGTA CTTAGCTGTA AATGATCCTA GATTATTAGA AATTACCGAA
 601  GAATCTTGGG AAGTAGTGAG TCATTTCATA AATGTAACGT CTGCTTTTAA
 651  GAAAGCTCAG ATTCTTTTTA AGAACAACGA ACATTCTCGG ATGAAGAAGA
 701  AGTTAGAAAG TGTTCAAGAG TTACTGGAAA CATTTATTTA TAAGAGTTTA
 751  AAGAGAAGTT ATCGAGAATT AGGATGCTTA AGTGAAAAGA TGAGAATCAT
 801  TCACGACAAT CCTCTCTTCC CTTGGGTGCA AGATCAGCAG AAGTATGCTC
 851  ATGCTAAGAA TGAATTTGGA GAGATTGCGC GGTGTTTAGA GGAGTTTGAA
 901  AAGACGTTCT TCTGGTTGGA TGAGGAGTGT GCTATTTCTT ACATGGACTG
```

```
 951  TTGGGATTTT CTAAATGAGT CTATTCAGAA TAAGAAGTCC AGAGTAGATC
1001  GAGATTATAT ATCCACGAAG AAAATTGCAT TAAAGGATAG AGCCCGCACT
1051  TATGCTAAGG TTCTTTTAGA AGAGAATCCG ACTACAGAGG GTAAAATAGA
1101  TTTGCAAGAC GCTCAAAGAG CCTTTGAGCG TCAAAGTCAG GAGTTTTATA
1151  CACTAGAGCA TACGGAAACA AAGGTGAGAC TAGAAGCACT TCAACAGTGC
1201  TTCTCGGATC TTAGGGAGGC GACGAACGTA AGGCAAGTTA GGTTTACAAA
1251  TTCTGAAAAT GCGAATGATT TAAAGGAGAG TTTCGAGAAG ATAGATAAAG
1301  AGCGTGTGCG ATATCAAAAA GAGCAAAGGC TCTATTGGGA AACAATAGAT
1351  CGCAATGAGC AAGAGCTTAG GGAAGAGATT GGGGAGTCGC TTCGTTTACA
1401  AAATCGGAGA AAAGGGTATA GGGCTGGATA TGATGCTGGG CGTTTAAAAG
1451  GTTTGTTGCG TCAGTGGAAG AAAAATCTCC GCGATGTGGA AGCCCACCTT
1501  GAAGATGCAA CTATGGATTT TGAGCATGAA GTAAGCAAGA GCGAATTGTG
1551  CAGTGTTCGG GCGAGGCTCG AGGTTCTAGA AGAAGAGCTG ATGGATATGT
1601  CTCCTAAAGT TGCGGATATA GAAGAGTTGT TGTCCTATGA AGAGCGTTGT
1651  ATTCTTCCTA TTAGGGAAAA TTTAGAAAGG GCATACCTCC AATATAATAA
1701  GTGTTCTGAA ATTTTATCCA AGGCAAAGTT CTTCTTTCCG GAAGACGAGC
1751  AATTGCTAGT TTCGGAAGCG AATCTAAGAG AGGTGGGTGC CCAGTTAAAA
1801  CAAGTACAGG GAAAATGTCA AGAGAGGGCC CAAAAGTTCG CAATATTTGA
1851  AAAGCATATT CAGGAGCAGA AAAGCCTTAT TAAAGAGCAA GTGCGGAGTT
1901  TTGATCTAGC GGGAGTTGGG TTTTTAAAGA GTGAGCTTCT TAGTATTGCT
1951  TGTAACCTTT ATATAAAGGC GGTTGTTAAG GAGTCTATAC CAGTTGATGT
2001  GCCTTGTATG CAGTTATATT ATAGTTATTA CGAAGATAAT GAAGCTGTAG
2051  TGCGAAACCG CCTTTTAAAT ATGACGGAGA GGTATCAAAA TTTTAAAAGG
2101  AGTTTGAATT CCATACAATT TAATGGTGAC GTTCTTTTAC GGGATCCGGT
2151  CTATCAACCT GAAGGTCATG AGACCAGGCT AAAGGAACGG GAGCTACAAG
2201  AAACAACTTT GTCTTGTAAG AAATTAAAAG TGGCTCAAGA TCGTCTTTCT
2251  GAATTAGAGT CAAGGCTGTC TAGGAGATAG
```

The PSORT algorithm predicts a periplasmic location (0.932).

The protein was expressed in *E.coli* and purified as a GST-fusion product, as shown in Figure 45A. The recombinant protein was used to immunise mice, whose sera were used in a Western blot (Figure 45B) and for FACS analysis (Figure 45C). A his-tagged protein was also expressed.

This protein also showed good cross-reactivity with human sera, including sera from patients with pneumonitis.

These experiments show that cp6390 is a surface-exposed and immunoaccessible protein, and that it is a useful immunogen. These properties are not evident from the sequence alone.

### Example 46

The following *C.pneumoniae* protein (PID 4376272) was expressed <SEQ ID 91; cp6272>:

```
  1  MKRCFLFLAS FVLMGSSADA LTHQEAVKKK NSYLSHFKSV SGIVTIEDGV
 51  LNIHNNLRIQ ANKVYVENTV GQSLKLVAHG NVMVNYRAKT LVCDYLEYYE
101  DTDSCLLTNG RFAMYPWFLG GSMITLTPET IVIRKGYIST SEGPKKDLCL
151  SGDYLEYSSD SLLSIGKTTL RVCRIPILFL PPFSIMPMEI PKPPINFRGG
201  TGGFLGSYLG MSYSPISRKH FSSTFFLDSF FKHGVGMGFN LHCSQKQVPE
251  NVFNMKSYYA HRLAIDMAEA HDRYRLHGDF CFTHKHVNFS GEYHLSDSWE
301  TVADIFPNNF MLKNTGPTRV DCTWNDNYFE GYLTSSVKVN SFQNANQELP
351  YLTLRQYPIS IYNTGVYLEN IVECGYLNFA FSDHIVGENF SSLRLAARPK
401  LHKTVPLPIG TLSSTLGSSL IYYSDVPEIS SRHSQLSAKL QLDYRFLLHK
451  SYIQRRHIIE PFVTFITETR PLAKNEDHYI FSIQDAFHSL NLLKAGIDTS
501  VLSKTNPRFP RIHAKLWTTH ILSNTESKPT FPKTACELSL PFGKKNTVSL
551  DAEWIWKKHC WDHMNIRWEW IGNDNVAMTL ESLHRSKYSL IKCDRENFIL
601  DVSRPIDQLL DSPLSDHRNL ILGKLFVRPH PCWNYRLSLR YGWHRQDTPN
651  YLEYQMILGT KIFEHWQLYG VYERREADSR FFFFLKLDKP KKPPF*
```

A predicted signal peptide is highlighted.

The cp6272 nucleotide sequence <SEQ ID 92> is:

```
  1  ATGAAACGTT GCTTCTTATT TCTAGCTTCC TTTGTTCTTA TGGGTTCCTC
```

```
  51 AGCTGATGCT TTGACTCATC AAGAGGCTGT GAAAAAGAAA AACTCCTATC
 101 TTAGTCACTT TAAGAGTGTT TCTGGGATTG TGACCATCGA AGATGGGGTA
 151 TTGAATATCC ATAACAACCT GCGGATACAA GCCAATAAAG TGTATGTAGA
 201 AAATACTGTG GGTCAAAGCC TGAAGCTTGT CGCACATGGC AATGTTATGG
 251 TGAACTATAG GGCAAAAACC CTAGTTTGTG ATTACCTAGA GTATTACGAA
 301 GATACAGACT CTTGTCTTCT TACTAATGGA AGATTCGCGA TGTATCCTTG
 351 GTTTCTAGGG GGGTCTATGA TCACTCTAAC CCCAGAAACC ATAGTCATTC
 401 GGAAGGGATA TATCTCTACC TCCGAGGGTC CCAAAAAAGA CCTGTGCCTC
 451 TCCGGAGATT ACCTGGAATA TTCTTCAGAT AGTCTTCTTT CTATAGGGAA
 501 GACAACATTA AGGGTGTGTC GCATTCCGAT ACTTTTCTTA CCTCCATTTT
 551 CTATCATGCC TATGGAGATC CCTAAGCCTC CGATAAACTT TCGAGGAGGA
 601 ACAGGAGGAT TTCTGGGATC CTATTTGGGG ATGAGCTACT CGCCGATTTC
 651 TAGGAAGCAT TTCTCCTCGA CATTTTTCTT GGATAGCTTT TTCAAGCATG
 701 GCGTCGGCAT GGGATTCAAC CTCCATTGTT CTCAGAAGCA GGTTCCTGAG
 751 AATGTCTTCA ATATGAAAAG CTATTATGCC CACCGCCTTG CTATCGATAT
 801 GGCAGAAGCT CATGATCGCT ATCGCCTACA CGGAGATTTC TGCTTCACGC
 851 ATAAGCATGT AAATTTTTCT GGAGAATACC ATCTCAGCGA TAGTTGGGAA
 901 ACTGTTGCTG ACATTTTCCC CAACAACTTC ATGTTGAAAA ATACAGGCCC
 951 CACACGTGTC GATTGCACTT GGAATGACAA CTATTTTGAA GGGTATCTCA
1001 CCTCTTCTGT TAAGGTAAAC TCTTTCCAAA ATGCCAACCA AGAGCTCCCT
1051 TATTTAACAT TAAGGCAGTA CCCGATTTCT ATTTATAATA CGGGAGTGTA
1101 CCTTGAAAAC ATCGTAGAAT GTGGGTATTT AAACTTTGCT TTTAGCGATC
1151 ATATCGTTGG CGAGAATTTC TCTTCACTAC GTCTTGCTGC GCGCCCTAAG
1201 CTCCATAAAA CTGTGCCTCT ACCTATAGGA ACGCTCTCCT CCACCCTAGG
1251 GAGTTCTCTG ATTTACTATA GCGATGTTCC TGAGATCTCC TCGCGCCATA
1301 GTCAGCTTTC CGCGAAGCTA CAACTTGATT ATCGCTTTCT ATTACATAAG
1351 TCCTACATTC AAAGACGCCA TATTATAGAG CCGTTCGTTA CCTTCATTAC
1401 AGAGACTCGT CCTCTAGCTA AGAATGAAGA TCATTATATC TTTTCTATTC
1451 AAGATGCCTT TCACTCCTTA AACCTTCTGA AAGCGGGTAT AGATACCTCG
1501 GTACTGAGTA AGACTAACCC TCGATTCCCG AGAATCCATG CGAAGCTGTG
1551 GACTACCCAC ATCTTGAGCA ATACAGAAAG CAAACCCACG TTTCCCAAAA
1601 CTGCATGCGA GCTATCTCTA CCTTTTGGAA AGAAAAATAC AGTCTCCTTA
1651 GATGCTGAAT GGATTTGGAA AAAGCACTGT TGGGATCACA TGAACATACG
1701 TTGGGAGTGG ATCGGAAATG ACAATGTGGC TATGACTCTA GAATCCCTGC
1751 ATAGAAGCAA ATACAGCCTG ATTAAGTGTG ACAGGGAGAA CTTCATTTTA
1801 GATGTCAGCC GTCCCATTGA CCAGCTTTTA GACTCCCCTC TCTCTGATCA
1851 TAGGAATCTC ATTTTAGGGA AATTATTTGT ACGACCTCAT CCCTGTTGGA
1901 ATTACCGCTT ATCCTTACGC TATGGCTGGC ATCGCCAGGA CACTCCGAAC
1951 TACCTAGAAT ACCAGATGAT TCTAGGGACG AAGATCTTCG AACATTGGCA
2001 GCTCTATGGG GTGTATGAAC GCCGAGAAGC AGATAGTCGA TTTTTCTTCT
2051 TCTTAAAGCT CGACAAACCT AAAAAACCTC CCTTCTAA
```

The PSORT algorithm predicts an outer membrane location (0.48).

The protein was expressed in *E.coli* and purified as a GST-fusion product, as shown in Figure 46A. The recombinant protein was used to immunise mice, whose sera were used in a Western blot and for FACS analysis (Figure 46B). A his-tagged protein was also expressed.

This protein also showed good cross-reactivity with human sera, including sera from patients with pneumonitis.

These experiments show that cp6272 is a surface-exposed and immunoaccessible protein, and that it is a useful immunogen. These properties are not evident from the sequence alone.

### Example 47

The following *C.pneumoniae* protein (PID 4377111) was expressed <SEQ ID 93; cp7111>:

```
  1 MFEAVIADIQ AREILDSRGY PTLHVKVTTS TGSVGEARVP SGASTGKKEA
 51 LEFRDTDSPR YQGKGVLQAV KNVKEILFPL VKGCSVYEQS LIDSLMMDSD
101 GSPNKETLGA NAILGVSLAT AHAAAATLRR PLYRYLGGCF ACSLPCPMMN
151 LINGGMHADN GLEFQEFMIR PIGASSIKEA VNMGADVFHT LKKLLHERGL
201 STGVGDEGGF APNLASNEEA LELLLLAIEK AGFTPGKDIS LALDCAASSF
```

```
251  YNVKTGTYDG RHYEEQIAIL SNLCDRYPID SIEDGLAEED YDGWALLTEV
301  LGEKVQIVGD DLFVTNPELI LEGISNGLAN SVLIKPNQIG TLTETVYAIK
351  LAQMAGYTTI ISHRSGETTD TTIADLAVAF NAGQIKTGSL SRSERVAKYN
401  RLMEIEEELG SEAIFTDSNV FSYEDSEE*
```

A predicted signal peptide is highlighted.

The cp7111 nucleotide sequence <SEQ ID 94> is:

```
   1  ATGTTTGAAG CTGTCATTGC CGATATCCAG GCTAGGGAAA TCTTGGATTC
  51  TCGCGGGTAT CCCACTTTAC ATGTTAAAGT AACCACTAGC ACAGGTTCTG
 101  TTGGAGAAGC TCGGGTTCCT TCAGGAGCAT CCACAGGGAA AAAAGAAGCC
 151  TTAGAGTTTC GTGATACAGA TTCTCCTCGT TATCAAGGCA AAGGGGTTTT
 201  GCAAGCTGTA AAAAACGTAA AAGAAATTCT TTTTCCCCTC GTCAAGGGAT
 251  GTAGTGTTTA TGAGCAATCC TTAATTGATT CTCTGATGAT GGATTCTGAC
 301  GGCTCTCCGA ACAAAGAAAC TCTAGGGGCC AATGCTATTT TAGGAGTCTC
 351  TCTAGCTACA GCACATGCAG CAGCAGCAAC ACTACGCAGA CCTCTGTATC
 401  GTTATTTAGG AGGGTGTTTT GCCTGCAGTC TTCCCTGTCC TATGATGAAT
 451  CTGATCAATG GAGGCATGCA TGCCGATAAC GGCTTGGAGT TCCAAGAATT
 501  TATGATCCGT CCTATTGGAG CCTCTTCCAT CAAAGAAGCT GTCAACATGG
 551  GTGCTGACGT TTTTCATACT TTGAAAAAAT TACTCCATGA AAGAGGCTTA
 601  TCTACTGGAG TGGGTGACGA AGGAGGCTTC GCCCCGAATC TTGCTTCTAA
 651  TGAAGAAGCT CTAGAGCTCC TATTGCTGGC TATTGAAAAA GCAGGCTTTA
 701  CTCCAGGAAA AGATATATCG CTAGCCTTAG ACTGCGCAGC ATCCTCATTC
 751  TATAACGTAA AAACAGGCAC GTATGATGGG AGGCACTATG AAGAGCAAAT
 801  CGCAATCCTT TCTAATTTAT GTGATCGCTA TCCTATAGAC TCCATAGAAG
 851  ATGGTCTTGC TGAAGAAGAC TATGACGGGT GGGCCTTGTT AACTGAAGTT
 901  CTTGGAGAAA AAGTACAGAT TGTGGGTGAT GACCTATTTG TTACAAATCC
 951  GGAATTAATA TTAGAGGGTA TTAGCAATGG ATTAGCGAAC TCTGTGTTGA
1001  TTAAACCAAA TCAGATAGGG ACGCTTACTG AAACAGTGTA TGCTATCAAG
1051  CTTGCGCAAA TGGCTGGCTA TACTACAATT ATTTCTCATC GCTCAGGAGA
1101  AACTACGGAC ACTACGATTG CAGATCTTGC TGTTGCCTTC AACGCCGGTC
1151  AAATCAAAAC AGGCTCTTTA TCACGTTCTG AGCGTGTTGC AAAATACAAT
1201  AGACTCATGG AAATTGAAGA AGAGCTTGGA TCCGAAGCAA TTTTCACAGA
1251  TTCTAATGTA TTTTCTTAC GAGGATTCT GAGGAATAG
```

The PSORT algorithm predicts an inner membrane location (0.100).

The protein was expressed in *E.coli* and purified as a GST-fusion product, as shown in Figure 47A. The recombinant protein was used to immunise mice, whose sera were used in a Western blot (Figure 47B) and for FACS analysis (Figure 47C). A his-tagged protein was also expressed.

The cp7111 protein was also identified in the 2D-PAGE experiment and showed good cross-reactivity with human sera, including sera from patients with pneumonitis.

These experiments show that cp7111 is a surface-exposed and immunoaccessible protein, and that it is a useful immunogen. These properties are not evident from the sequence alone.

**Example 48**

The following *C.pneumoniae* protein (PID 4455886) was expressed <SEQ ID 95; cp0010>:

```
  1 MKSQFSWLVL SSTLACFTSC STVFAATAEN IGPSDSFDGS TNTGTYTPKN
 51 TTTGIDYTLT GDITLQNLGD SAALTKGCFS DTTESLSFAG KGYSLSFLNI
101 KSSAEGAALS VTTDKNLSLT GFSSLTFLAA PSSVITTPSG KGAVKCGGDL
151 TFDNNGTILF KQDYCEENGG AISTKNLSLK NSTGSISFEG NKSSATGKKG
201 GAICATGTVD ITNNTAPTLF SNNIAEAAGG AINSTGNCTI TGNTSLVFSE
251 NSVTATAGNG GALSGDADVT ISGNQSVTFS GNQAVANGGA IYAKKLTLAS
301 GGGGVSPFLT IIVQGTTAGN GGAISILAAG ECSLSAEAGD ITFNGNAIVA
351 TTPQTTKRNS IDIGSTAKIT NLRAISGHSI FFYDPITANT AADSTDTLNL
401 NKADAGNSTD YSGSIVFSGE KLSEDEAKVA DNLTSTLKQP VTLTAGNLVL
451 KRGVTLDTKG FTQTAGSSVI MDAGTTLKAS TEEVTLTGLS IPVDSLGEGK
501 KVVIAASAAS KNVALSGPIL LLDNQGNAYE NHDLGKTQDF SFVQLSALGT


551 ATTTDVPAVP TVATPTHYGY QGTWGMTWVD DTASTPKTKT ATLAWTNTGY
601 LPNPERQGPL VPNSLWGSFS DIQAIQGVIE RSALTLCSDR GFWAAGVANF
651 LDKDKKGEKR KYRHKSGGYA IGGAAQTCSE NLISFAFCQL FGSDKDFLVA
701 KNHTDTYAGA FYIQHITECS GFIGCLLDKL PGSWSHKPLV LEGQLAYSHV
751 SNDLKTKYTA YPEVKGSWGN NAFNMMLGAS SHSYPEYLHC FDTYAPYIKL
801 NLTYIRQDSF SEKGTEGRSF DDSNLFNLSL PIGVKFEKFS DCNDFSYDLT
851 LSYVPDLIRN DPKCTTALVI SGASWETYAN NLARQALQVR AGSHYAFSPM
901 FEVLGQFVFE VRGSSRIYNV DLGGKFQF*
```

A predicted signal peptide is highlighted.

The cp0010 nucleotide sequence <SEQ ID 96> is:

```
   1 ATGAAATCGC AATTTTCCTG GTTAGTGCTC TCTTCGACAT TGGCATGTTT
  51 TACTAGTTGT TCCACTGTTT TTGCTGCAAC TGCTGAAAAT ATAGGCCCCT
 101 CTGATAGCTT TGACGGAAGT ACTAACACAG GCACCTATAC TCCTAAAAAT
 151 ACGACTACTG GAATAGACTA TACTCTGACA GGAGATATAA CTCTGCAAAA
 201 CCTTGGGGAT TCGGCAGCTT TAACGAAGGG TTGTTTTTCT GACACTACGG
 251 AATCTTTAAG CTTTGCCGGT AAGGGGTACT CACTTTCTTT TTTAAATATT
 301 AAGTCTAGTG CTGAAGGCGC AGCACTTTCT GTTACAACTG ATAAAAATCT
 351 GTCGCTAACA GGATTTTCGA GTCTTACTTT CTTAGCGGCC CCATCATCGG
 401 TAATCACAAC CCCCTCAGGA AAAGGTGCAG TTAAATGTGG AGGGGATCTT
 451 ACATTTGATA ACAATGGAAC TATTTTATTT AAACAAGATT ACTGTGAGGA
 501 AAATGGCGGA GCCATTTCTA CCAAGAATCT TTCTTTGAAA AACAGCACGG
 551 GATCGATTTC TTTTGAAGGG AATAAATCGA GCGCAACAGG GAAAAAAGGT
 601 GGGGCTATTT GTGCTACTGG TACTGTAGAT ATTACAAATA ATACGGCTCC
 651 TACCCTCTTC TCGAACAATA TTGCTGAAGC TGCAGGTGGA GCTATAAATA
 701 GCACAGGAAA CTGTACAATT ACAGGGAATA CGTCTCTTGT ATTTTCTGAA
 751 AATAGTGTGA CAGCGACCGC AGGAAATGGA GGAGCTCTTT CTGGAGATGC
 801 CGATGTTACC ATATCTGGGA ATCAGAGTGT AACTTTCTCA GGAAACCAAG
 851 CTGTAGCTAA TGGCGGAGCC ATTTATGCTA AGAAGCTTAC ACTGGCTTCC
 901 GGGGGGGGGG GGGTATCTCC TTTTCTAACA ATAaTAGTCC AAGGTACCAC
 951 TGCAGGTAAT GGTGGAGCCA TTTCTATACT GGCAGCTGGA GAGTGTAGTC
1001 TTTCAGCAGA AGCAGGGGAC ATTACCTTCA ATGGGAATGC CATTGTTGCA
1051 ACTACACCAC AAACTACAAA AAGAAATTCT ATTGACATAG GATCTACTGC
1101 AAAGATCACG AATTTACGTG CAATATCTGG GCATAGCATC TTTTTCTACG
1151 ATCCGATTAC TGCTAATACG GCTGCGGATT CTACAGATAC TTTAAATCTC
1201 AATAAGGCTG ATGCAGGTAA TAGTACAGAT TATAGTGGGT CGATTGTTTT
1251 TTCTGGTGAA AAGCTCTCTG AAGATGAAGC AAAAGTTGCA GACAACCTCA
1301 CTTCTACGCT GAAGCAGCCT GTAACTCTAA CTGCAGGAAA TTTAGTACTT
1351 AAACGTGGTG TCACTCTCGA TACGAAAGGC TTTACTCAGA CCGCGGGTTC
1401 CTCTGTTATT ATGGATGCGG GCACAACGTT AAAAGCAAGT ACAGAGGAGG
1451 TCACTTTAAC AGGTCTTTCC ATTCCTGTAG ACTCTTTAGG CGAGGGTAAG
1501 AAAGTTGTAA TTGCTGCTTC TGCAGCAAGT AAAAATGTAG CCCTTAGTGG
1551 TCCGATTCTT CTTTTGGATA ACCAAGGGAA TGCTTATGAA AATCACGACT
1601 TAGGAAAAAC TCAAGACTTT TCATTTGTGC AGCTCTCTGC TCTGGGTACT
1651 GCAACAACTA CAGATGTTCC AGCGGTTCCT ACAGTAGCAA CTCCTACGCA
1701 CTATGGGTAT CAAGGTACTT GGGGAATGAC TTGGGTTGAT GATACCGCAA
1751 GCACTCCAAA GACTAAGACA GCGACATTAG CTTGGACCAA TACAGGCTAC
1801 CTTCCGAATC CTGAGCGTCA AGGACCTTTA GTTCCTAATA GCCTTTGGGG
1851 ATCTTTTTCA GACATCCAAG CGATTCAAGG TGTCATAGAG AGAAGTGCTT
1901 TGACTCTTTG TTCAGATCGA GGCTTCTGGG CTGCGGGAGT CGCCAATTTC
1951 TTAGATAAAG ATAAGAAAGG GGAAAAACGC AAATACCGTC ATAAATCTGG
2001 TGGATATGCT ATCGGAGGTG CAGCGCAAAC TTGTTCTGAA AACTTAATTA
2051 GCTTTGCCTT TTGCCAACTC TTTGGTAGCG ATAAAGATTT CTTAGTCGCT
2101 AAAAATCATA CTGATACCTA TGCAGGAGCC TTCTATATCC AACACATTAC
2151 AGAATGTAGT GGGTTCATAG GTTGTCTCTT AGATAAACTT CCTGGCTCTT
2201 GGAGTCATAA ACCCCTCGTT TTAGAAGGGC AGCTCGCTTA TAGCCACGTC
2251 AGTAATGATC TGAAGACAAA GTATACTGCG TATCCTGAGG TGAAAGGTTC
2301 TTGGGGGAAT AATGCTTTTA ACATGATGTT GGGAGCTTCT TCTCATTCTT
2351 ATCCTGAATA CCTGCATTGT TTTGATACCT ATGCTCCATA CATCAAACTG
2401 AATCTGACCT ATATACGTCA GGACAGCTTC TCGGAGAAAG GTACAGAAGG
2451 AAGATCTTTT GATGACAGCA ACCTCTTCAA TTTATCTTTG CCTATAGGGG
2501 TGAAGTTTGA GAAGTTCTCT GATTGTAATG ACTTTTCTTA TGATCTGACT
2551 TTATCCTATG TTCCTGATCT TATCCGCAAT GATCCCAAAT GCACTACAGC
2601 ACTTGTAATC AGCGGAGCCT CTTGGGAAAC TTATGCCAAT AACTTAGCAC
2651 GACAGGCCTT GCAAGTGCGT GCAGGCAGTC ACTACGCCTT CTCTCCTATG
2701 TTTGAAGTGC TCGGCCAGTT TGTCTTTGAA GTTCGTGGAT CCTCACGGAT

2751 TTATAATGTA GATCTTGGGG GTAAGTTCCA ATTCTAG
```

The PSORT algorithm predicts an outer membrane location (0.922).

The protein was expressed in *E.coli* and purified as a GST-fusion product, as shown in Figure 48A. The recombinant protein was used to immunise mice, whose sera were used in a Western blot (Figure 48B) and for FACS analysis (Figure 48C). A his-tagged protein was also expressed.

The cp0010 protein was also identified in the 2D-PAGE experiment and showed good cross-reactivity with human sera, including sera from patients with pneumonitis.

These experiments show that cp0010 is a surface-exposed and immunoaccessible protein, and that it is a useful immunogen. These properties are not evident from the sequence alone.

**Example 49**

The following *C.pneumoniae* protein (PID 4376296) was expressed <SEQ ID 97; cp6296>:

```
  1  MEEVSEYLQQ VENQLESCSK RLTKMETFAL GVRLEAKEEI ESIILSDVVN
 51  RFEVLCRDIE DMLSRVEEIE RMLRMAELPL LPIKEALTKA FVQHNSCKEK
101  LTKVEPYFKE SPAYLTSEER LQSLNQTLQR AYKESQKVSG LESEVRACRE
151  QLKDQVRQFE TQGVSLIKEE ILFVTSTFRT KFSYHSFRLH VPCMRLYEEY
201  YDDIDLERTR ARWMAMSERY RDAFQAFQEM LKEGLVEEAQ ALRETEYWLY
251  REERKSKKKH*
```

The cp6296 nucleotide sequence <SEQ ID 98> is:

```
  1  ATGGAGGAGG TGTCTGAGTA TCTTCAGCAA GTAGAAAATC AGTTGGAATC
 51  CTGTTCCAAG CGATTAACCA AGATGGAAAC TTTTGCCTTA GGTGTGAGGT
101  TGGAAGCTAA AGAAGAGATA GAGTCTATCA TACTTTCTGA TGTAGTGAAC
151  CGTTTTGAGG TTTTATGTAG AGATATTGAA GATATGCTAT CTCGAGTCGA
201  GGAGATAGAG CGGATGTTAC GTATGGCGGA GCTTCCTCTA CTTCCTATAA
251  AAGAAGCGCT TACCAAGGCT TTTGTACAAC ATAACAGCTG TAAAGAGAAG
301  TTAACCAAGG TAGAGCCTTA CTTTAAAGAG AGCCCTGCAT ATCTAACTAG
351  TGAAGAGCGA TTGCAGAGTT TGAATCAGAC TTTACAACGT GCGTACAAAG
401  AGTCCCAAAA GGTTTCAGGT TTAGAATCGG AAGTGAGAGC CTGTCGAGAG
451  CAGCTTAAAG ATCAAGTAAG ACAGTTTGAA ACTCAAGGAG TGAGCTTGAT
501  AAAAGAAGAG ATTCTCTTTG TGACTAGTAC CTTTAGAACT AAATTTAGCT
551  ATCATTCATT TCGATTACAT GTTCCTTGCA TGAGGTTGTA TGAGGAGTAT
601  TATGATGACA TTGATCTAGA GAGAACTCGA GCTCGATGGA TGGCGATGTC
651  TGAGAGGTAT AGAGATGCTT TTCAGGCATT CCAGGAGATG TTGAAGGAAG
701  GCCTAGTTGA AGAAGCTCAG GCTCTTAGAG AAACCGAGTA CTGGTTATAT
751  CGAGAGGAGA GAAAGAGTAA AAAGAAACAT TGA
```

The PSORT algorithm predicts a cytoplasmic location (0.523).

The protein was expressed in *E.coli* and purified as a GST-fusion product, as shown in Figure 49A. The recombinant protein was used to immunise mice, whose sera were used in a Western blot (Figure 49B) and for FACS analysis (Figure 49C). A his-tagged protein was also expressed.

These experiments show that cp6296 is a surface-exposed and immunoaccessible protein, and that it is a useful immunogen. These properties are not evident from the sequence alone.

**Example 50**

The following *C.pneumoniae* protein (PID 4376664) was expressed <SEQ ID 99; cp6664>:

```
  1  MVLFHAQASG RNRVKADAIV LPFWHFKDAK NAASFEAEFE PSYLPALENF
 51  QGKTGEIELL YSSPKAKEKR IVLLGLGKNE ELTSDVVFQT YATLTRVLRK
101  AKCSTVNIIL PTISELRLSA EEFLVGLSSG ILSLNYDYPR YNKVDRNLET
```

```
151  PLSKVTVIGI VPKMADAIFR KEAAIFEGVY LTRDLVNRNA DEITPKKLAE
201  VALNLGKEFP SIDTKVLGKD AIAKEKMGLL LAVSKGSCVD PHFIVVRYQG
251  RPKSKDHTVL IGKGVTFDSG GLDLKPGKSM LTMKEDMAGG ATVLGILSAL
301  AVLELPINVT GIIPATENAI DGASYKMGDV YVGMSGLSVE ICSTDAEGRL
351  ILADAITYAL KYCKPTRIID FATLTGAMVV SLGEEVAGFF SNNDVLAEDL
401  LEASAETSEP LWRLPLVKKY DKTLHSDIAD MKNLGSNRAG AITAALFLQR
451  FLEESSVAWA HLDIAGTAYH EKEEDRYPKY ASGFGVRSIL YYLENSLSK*
```

The cp6664 nucleotide sequence <SEQ ID 100> is:

```
   1  GTGGTTTTAT TTCATGCTCA AGCCTCTGGG CGTAATCGTG TTAAGGCAGA
  51  TGCTATAGTC CTGCCCTTTT GGCATTTTAA GGATGCAAAA AATGCAGCTT
 101  CTTTTGAAGC CGAGTTTGAA CCCTCGTATC TCCCCGCTTT AGAAAACTTT
 151  CAAGGAAAAA CCGGGGAGAT TGAACTCCTT TATAGTAGTC CTAAAGCTAA
 201  GGAAAAACGC ATTGTCCTCT TAGGCTTAGG GAAAAATGAA GAGCTCACCT
 251  CTGATGTTGT TTTCCAAACC TATGCGACAC TAACTCGTGT CTTACGTAAA
 301  GCAAAGTGTT CCACAGTCAA TATCATCTTA CCTACAATTT CTGAATTGCG
 351  GCTTTCTGCC GAAGAATTCT TAGTGGGGTT GTCCTCAGGA ATTTTGTCAT
 401  TAAACTATGA CTACCCACGT TATAATAAGG TAGATCGTAA TCTTGAAACT
 451  CCTCTTTCTA AAGTCACGGT TATCGGTATC GTTCCCAAAA TGGCGGATGC
 501  TATCTTTAGG AAAGAAGCAG CCATTTTCGA AGGCGTATAT CTCACTCGAG
 551  ATCTTGTGAA CAGGAATGCT GATGAAATTA CCCCTAAGAA ATTGGCAGAG
 601  GTTGCTCTGA ATCTGGGAAA AGAGTTCCCT AGTATTGATA CTAAGGTCTT
 651  GGGAAAAGAT GCCATCGCCA AAGAGAAAAT GGGACTCCTA TTGGCTGTTT
 701  CCAAGGGTTC TTGTGTGGAT CCACACTTTA TCGTTGTCCG TTATCAAGGA
 751  CGTCCTAAGT CTAAAGATCA CACCGTCTTG ATAGGGAAAG GGGTCACTTT
 801  TGACTCTGGA GGTTTAGACC TCAAGCCTGG AAAATCCATG CTTACTATGA
 851  AAGAAGACAT GGCAGGTGGG GCTACAGTCC TCGGGATTCT CTCGGCGTTA
 901  GCAGTTTTAG AGCTTCCTAT AAATGTCACG GGGATCATTC CTGCTACAGA
 951  GAATGCTATC GATGGCGCCT CCTATAAAAT GGGAGATGTC TATGTAGGAA
1001  TGTCGGGGCT TTCTGTTGAG ATTTGTAGTA CCGATGCTGA GGGACGTCTT
1051  ATCCTCGCTG ATGCGATTAC ATATGCTTTA AAATATTGTA AACCGACACG
1101  TATTATAGAT TTTGCAACTC TAACAGGAGC TATGGTAGTC TCTCTAGGAG
1151  AAGAGGTTGC AGGTTTCTTT TCCAATAACG ATGTTTTAGC TGAAGATCTT
1201  TTAGAGGCGT CAGCCGAAAC CTCCGAGCCG TTATGGAGAC TTCCTCTAGT
1251  TAAGAAGTAT GATAAAACAT TGCATTCTGA TATTGCTGAT ATGAAAAATC
1301  TAGGCAGTAA CCGTGCAGGG GCTATTACAG CAGCATTATT CTTGCAGAGA
1351  TTTTTGGAAG AATCTTCGGT AGCTTGGGCA CATCTTGATA TTGCAGGTAC
1401  TGCATATCAT GAAAAAGAAG AAGACCGTTA TCCAAAATAT GCTTCAGGTT
1451  TTGGTGTTCG TTCTATTCTT TATTACTTAG AAAATAGTCT TTCTAAGTAG
```

The PSORT algorithm predicts an inner membrane location (0.268).

The protein was expressed in *E.coli* and purified as a GST-fusion (Figure 50A), as a his-tagged protein, and as a GST/His fusion. The proteins were used to immunise mice, whose sera were used in Western blot Western blot (50B) and FACS (50C) analyses.

The cp6664 protein was also identified in the 2D-PAGE experiment (Cpn0385) and showed good cross-reactivity with human sera, including sera from patients with pneumonitis.

These experiments show that cp6664 is a surface-exposed and immunoaccessible protein, and that it is a useful immunogen. These properties are not evident from the sequence alone.

**Example 51**

The following *C.pneumoniae* protein (PID 4376696) was expressed <SEQ ID 101; cp6696>:

```
  1 MTLIFVIIIV WCNAFLIKLC VIMGLQSRLQ HCIEVSQNSN FDSQVKQFIY
 51 ACQDKTLRQS VLKIFRYHPL LKIHDIARAV YLLMALEEGE DLGLSFLNVQ
101 QYPSGAVELF SCGGFPWKGL PYPAEHAEFG LLLLQIAEFY EESQAYVSKM
151 SHFQQALFDH QGSVFPSLWS QENSRLLKEK TTLSQSFLFQ LGMQIHPEYS
201 LEDPALGFWM QRTRSSSAFV AASGCQSSLG AYSSGDVGVI AYGPCSGDIS
251 DCYYFGCCGI AKEFVCQKSH QTTEISFLTS TGKPHPRNTG FSYLRDSYVH
301 LPIRCKITIS DKQYRVHAAL AEATSAMTFS IFCKGKNCQV VDGPRLRSCS

351 LDSYKGPGND IMILGENDAI NIVSASPYME IFALQGKEKF WNADFLINIP
401 YKEEGVMLIF EKKVTSEKGR FFTKMN*
```

A predicted signal peptide is highlighted.

The cp6696 nucleotide sequence <SEQ ID 102> is:

```
   1 TTGACTCTAA TTTTTGTTAT TATTATCGTT TGGTGCAATG CTTTTCTGAT
  51 CAAATTGTGC GTGATAATGG GGCTGCAATC CAGGTTACAA CATTGTATAG
 101 AAGTGTCCCA GAATTCGAAC TTTGATTCAC AAGTAAAACA GTTTATCTAT
 151 GCGTGCCAAG ATAAGACATT AAGGCAGTCT GTACTCAAGA TTTTCCGCTA
 201 CCATCCTTTA CTAAAAATTC ATGATATTGC TCGGGCCGTC TATCTTTTGA
 251 TGGCCTTAGA AGAAGGCGAG GATTTAGGCT TAAGCTTTTT AAATGTACAG
 301 CAGTACCCTT CAGGTGCTGT AGAACTGTTT TCTTGTGGGG GATTTCCTTG
 351 GAAAGGATTA CCTTATCCTG CAGAACATGC GGAATTTGGC CTACTCCTGT
 401 TACAGATCGC AGAGTTTTAT GAAGAGAGTC AGGCATACGT CTCTAAAATG
 451 AGTCATTTTC AACAGGCACT CTTTGATCAC CAAGGGAGCG TCTTTCCCTC
 501 TCTCTGGAGC CAGGAGAACT CTCGACTCCT AAAAGAAAAG ACAACTCTTA
 551 GCCAATCGTT TCTCTTCCAA TTAGGAATGC AAATTCACCC AGAATACAGT
 601 CTTGAGGATC CTGCACTAGG GTTCTGGATG CAAAGAACGC GTTCTTCATC
 651 CGCTTTTGTA GCCGCTTCAG GATGTCAAAG TAGCTTGGGA GCGTATTCCT
 701 CAGGGGATGT CGGTGTTATC GCTTATGGAC CTTGCTCTGG AGACATTAGT
 751 GATTGTTATT ATTTTGGATG TTGTGGAATC GCTAAAGAGT TCGTGTGCCA
 801 AAAATCTCAC CAAACTACAG AGATTTCTTT TCTCACCTCT ACAGGAAAGC
 851 CTCATCCCAG AAATACGGGA TTTTCCTACC TTCGAGATTC CTATGTACAT
 901 CTGCCGATCC GCTGTAAGAT CACTATTTCC GACAAGCAAT ATCGCGTGCA
 951 CGCTGCGTTG GCTGAGGCCA CCTCTGCCAT GACGTTTTCT ATTTTCTGTA
1001 AGGGGAAGAA TTGTCAGGTT GTTGACGGCC CTCGCTTGCG CTCCTGTTCC
1051 CTAGATTCTT ATAAAGGTCC CGGAAACGAC ATTATGATTC TTGGGGAAAA
1101 TGACGCAATC AACATTGTTT CTGCAAGTCC CTATATGGAA ATTTTTGCTT
1151 TGCAAGGCAA AGAAAAATTT TGGAATGCAG ACTTTTTGAT TAATATTCCT
1201 TACAAAGAAG AGGGCGTCAT GTTAATTTTT GAAAAAAAAG TGACCTCTGA
1251 GAAAGGAAGA TTCTTTACGA AGATGAATTA A
```

The PSORT algorithm predicts an inner membrane location (0.463).

The protein was expressed in *E.coli* and purified as a GST-fusion product, as shown in Figure 51A. The recombinant protein was used to immunise mice, whose sera were used in a Western blot (Figure 51B) and for FACS analysis (Figure 51C). A his-tagged protein was also expressed.

This protein also showed good cross-reactivity with human sera, including sera from patients with pneumonitis.

These experiments show that cp6696 is a surface-exposed and immunoaccessible protein, and that it is a useful immunogen. These properties are not evident from the sequence alone.

**Example 52**

The following *C.pneumoniae* protein (PID 4376790) was expressed <SEQ ID 103; cp6790>:

```
  1  MSEHKKSSKI IGIDLGTTNS CVSVMEGGQA KVITSSEGTR TTPSIVAFKG
 51  NEKLVGIPAK RQAVTNPEKT LGSTKRFIGR KYSEVASEIQ TVPYTVTSGS
101  KGDAVFEVDG KQYTPEEIGA QILMKMKETA EAYLGETVTE AVITVPAYFN
151  DSQRASTKDA GRIAGLDVKR IIPEPTAAAL AYGIDKVGDK KIAVFDLGGG
201  TFDISILEIG DGVFEVLSTN GDTLLGGDDF DEVIIKWMIE EFKKQEGIDL
251  SKDNMALQRL KDAAEKAKIE LSGVSSTEIN QPFITMDAQG PKHLALTLTR
301  AQFEKLAASL IERTKSPCIK ALSDAKLSAK DIDDVLLVGG MSRMPAVQET
351  VKELFGKEPN KGVNPDEVVA IGAAIQGGVL GGEVKDVLLL DVIPLSLGIE
401  TLGGVMTTLV ERNTTIPTQK KQIFSTAADN QPAVTIVVLQ GERPMAKDNK
451  EIGRFDLTDI PPAPRGHPQI EVSFDIDANG IFHVSAKDVA SGKEQKIRIE
501  ASSGLQEDEI QRMVRDAEIN KEEDKKRREA SDAKNEADSM IFRAEKAIKD
551  YKEQIPETLV KEIEERIENV RNALKDDAPI EKIKEVTEDL SKHMQKIGES
601  MQSQSASAAA SSAANAKGGP NINTEDLKKH SFSTKPPSNN GSSEDHIEEA

651  DVEIIDNDDK*
```

The cp6790 nucleotide sequence <SEQ ID 104> is:

```
   1  ATGAGTGAAC ACAAAAAATC AAGCAAAATT ATAGGTATAG ACTTAGGCAC
  51  AACAAACTCC TGCGTATCTG TTATGGAAGG AGGACAAGCT AAAGTAATTA
 101  CATCATCCGA AGGAACAAGA ACCACGCCAT CGATCGTTGC CTTCAAAGGT
 151  AATGAGAAAT TAGTGGGGAT TCCAGCAAAA CGTCAAGCAG TGACAAATCC
 201  AGAAAAAACT CTCGGCTCTA CAAAACGCTT TATTGGCCGT AAGTACTCTG
 251  AAGTAGCTTC GGAAATCCAA ACCGTTCCTT ATACAGTCAC CTCCGGATCT
 301  AAAGGTGATG CCGTTTTCGA AGTTGATGGC AAACAATACA CTCCAGAAGA
 351  AATTGGCGCA CAAATCTTAA TGAAAATGAA AGAGACAGCA GAAGCTTATC
 401  TAGGCGAAAC TGTCACAGAA GCAGTGATCA CCGTCCCCGC ATACTTCAAT
 451  GATTCTCAAC GAGCATCCAC AAAAGATGCT GGACGCATTG CAGGTCTAGA
 501  TGTAAAACGT ATCATTCCAG AACCTACCGC AGCAGCTCTT GCCTACGGAA
 551  TCGATAAAGT CGGTGATAAA AAAATCGCTG TCTTCGACCT TGGTGGAGGA
 601  ACTTTTGATA TCTCCATCCT AGAAATCGGT GATGGCGTCT TCGAAGTTCT
 651  ATCTACAAAT GGAGATACTC TCCTCGGTGG AGACGACTTT GATGAAGTCA
 701  TTATCAAATG GATGATCGAA GAATTCAAAA AACAAGAAGG CATTGATCTT
 751  AGCAAAGATA ATATGGCCTT ACAAAGACTT AAAGATGCTG CTGAGAAAGC
 801  AAAAATAGAA CTTTCAGGAG TCTCTTCCAC AGAAATCAAT CAGCCATTCA
 851  TCACAATGGA TGCACAAGGA CCTAAACACC TTGCATTGAC ACTCACACGT
 901  GCGCAATTCG AGAAACTCGC AGCCTCTCTA ATCGAAAGAA CAAAATCTCC
 951  ATGCATCAAA GCACTCAGTG ACGCAAAACT TTCCGCTAAG GATATCGATG
1001  ATGTTCTCTT AGTTGGAGGT ATGTCAAGAA TGCCCGCAGT GCAAGAAACT
1051  GTAAAAGAAC TCTTCGGCAA AGAGCCTAAT AAAGGAGTCA ACCCCGACGA
1101  AGTTGTTGCT ATTGGAGCCG CAATTCAAGG TGGTGTTCTT GGCGGAGAAG
1151  TTAAGGATGT TCTACTTCTA GACGTTATCC CCCTATCTCT GGGTATCGAA
1201  ACTCTAGGAG GCGTCATGAC GACTCTGGTA GAGAGAAATA CTACAATCCC
1251  TACACAGAAA AAACAAATCT TCTCCACAGC TGCTGATAAC CAGCCTGCGG
1301  TTACCATCGT AGTTCTCCAA GGAGAGCGTC CCATGGCCAA AGATAACAAG
1351  GAAATCGGAA GATTCGATCT TACAGATATC CCTCCGGCTC CTCGAGGCCA
1401  TCCTCAAATC GAAGTCTCCT TCGATATCGA TGCAAACGGA ATTTTCCATG
1451  TCTCAGCTAA AGATGTTGCC AGCGGTAAAG AACAGAAAAT TCGTATCGAA
1501  GCAAGCTCAG GACTTCAAGA AGATGAAATC CAAAGAATGG TTCGAGATGC
1551  CGAAATTAAT AAGGAAGAAG ATAAAAAACG TCGTGAAGCT TCAGATGCTA
1601  AAAATGAAGC CGATAGCATG ATCTTCAGAG CCGAAAAAGC TATTAAAGAT
1651  TATAAGGAGC AAATTCCTGA AACTTTAGTT AAAGAAATCG AAGAGCGAAT
1701  CGAAAACGTG CGCAACGCAC TCAAAGATGA CGCTCCTATT GAAAAAATTA
1751  AAGAGGTTAC TGAAGACCTA AGCAAGCATA TGCAAAAAAT TGGAGAGTCT
1801  ATGCAATCGC AGTCTGCATC AGCAGCAGCA TCATCGGCAG CCAATGCTAA
1851  AGGTGGACCT AACATCAATA CAGAAGATTT GAAAAAACAT AGTTTCAGTA
1901  CGAAGCCTCC TTCAAATAAC GGTTCTTCAG AAGACCATAT CGAAGAAGCT
1951  GATGTAGAAA TTATTGATAA CGACGATAAG TAA
```

The PSORT algorithm predicts an inner membrane location (0.151).

The protein was expressed in *E.coli* and purified as a GST-fusion product (Figure 52A) and a his-tagged product.

The proteins were used to immunise mice, whose sera were used in Western blot (Figure 52B) and FACS (Figure 52C) analyses.

The cp6790 protein was also identified in the 2D-PAGE experiment (Cpn0503).

These experiments show that cp6790 is a surface-exposed and immunoaccessible protein, and that it is a useful immunogen. These properties are not evident from the sequence alone.

**Example 53**

The following *C.pneumoniae* protein (PID 4376878) was expressed <SEQ ID 105; cp6878>:

```
1    MNVPDSKNLH PPAYELLEIK ARITQSYKEA SAILTAIPDG ILLLSETGHF
51   LICNSQAREI LGIDENLEIL NRSFTDVLPD TCLGFSIQEA LESLKVPKTL
101  RLSLCKESKE KEVELFIRKN EISGYLFIQI RDRSDYKQLE NAIERYKNIA
151  ELGKMTATLA HEIRNPLSGI VGFASILKKE ISSPRHQRML SSIISGTRSL
201  NNLVSSMLEY TKSQPLNLKI INLQDFFSSL IPLLSVSFPN CKFVREGAQP

251  LFRSIDPDRM NSVVWNLVKN AVETGNSPIT LTLHTSGDIS VTNPGTIPSE
301  IMDKLFTPFF TTKREGNGLG LAEAQKIIRL HGGDIQLKTS DSAVSFFIII
351  PELLAALPKE RAAS*
```

The cp6878 nucleotide sequence <SEQ ID 106> is:

```
1    ATGAACGTCC CTGATTCCAA GAACCTCCAT CCTCCTGCAT ACGAACTCCT
51   AGAGATCAAG GCTCGCATCA CACAATCTTA TAAAGAAGCG AGTGCTATAC
101  TGACAGCGAT TCCTGATGGT ATCCTATTAC TTTCTGAAAC AGGACACTTT
151  CTTATCTGCA ATTCACAAGC ACGTGAAATT CTAGGAATTG ATGAAAATCT
201  AGAAATTCTT AATAGATCCT TTACCGATGT TCTCCCCGAT ACGTGTCTTG
251  GATTTTCTAT TCAAGAGGCT CTTGAATCTC TAAAAGTCCC TAAAACTCTT
301  AGACTCTCTC TCTGTAAAGA ATCTAAAGAA AAAGAAGTGG AACTCTTCAT
351  CCGTAAAAAC GAGATCAGTG GATACCTGTT TATCCAAATC CGCGATCGGT
401  CCGACTATAA ACAACTAGAA AACGCTATAG AAAGATATAA AAATATCGCA
451  GAACTTGGGA AAATGACGGC TACCCTAGCT CACGAAATCC GCAATCCGCT
501  AAGTGGAATC GTTGGATTTG CCTCTATCCT AAAGAAAGAG ATTTCCTCTC
551  CTCGCCACCA ACGAATGCTC TCCTCAATCA TCTCCGGCAC AAGGTCTCTA
601  AATAACCTTG TCTCTTCTAT GTTAGAATAT ACAAAATCAC AACCGTTGAA
651  CCTAAAGATT ATAAATTTAC AAGACTTCTT CTCTTCTCTT ATCCCTCTGC
701  TCTCCGTCTC TTTCCCGAAT TGCAAGTTTG TAAGAGAGGG CGCACAACCT
751  CTATTCAGAT CTATAGATCC TGATCGGATG AACAGTGTCG TTTGGAACCT
801  AGTGAAAAAT GCTGTAGAAA CAGGGAACTC TCCGATCACT CTGACCCTGC
851  ATACATCGGG AGACATCTCG GTAACGAACC CCGGAACGAT TCCTTCCGAG
901  ATCATGGACA AGCTCTTCAC TCCATTCTTC ACAACAAAGA GAGAGGGAAA
951  TGGTTTGGGA CTTGCTGAAG CTCAAAAAAT TATAAGACTC CATGGAGGAG
1001 ATATCCAATT AAAAACAAGC GACTCCGCCG TTAGCTTCTT CATAATCATC
1051 CCCGAACTTC TAGCGGCCCT ACCCAAAGAA AGAGCCGCTA G
```

The PSORT algorithm predicts an inner membrane location (0.204).

The protein was expressed in *E.coli* and purified as a his-tag product (Figure 53A) and as a GST-fusion product. The recombinant GST-fusion protein was used to immunise mice, whose sera were used in a Western blot (Figure 53B) and for FACS analysis.

These experiments show that cp6878 is a surface-exposed and immunoaccessible protein, and that it is a useful immunogen. These properties are not evident from the sequence alone.

**Example 54**

The following *C.pneumoniae* protein (PID 4377224) was expressed <SEQ ID 107; cp7224>:

```
  1  MMKKIRKVAL AVGGSGGHIV PALSVKEAFS REGIDVLLLG KGLKNHPSLQ
 51  QGISYREIPS GLPTVLNPIK IMSRTLSLCS GYLKARKELK IFDPDLVIGF
101  GSYHSLPVLL AGLSHKIPLF LHEQNLVPGK VNQLFSRYAR GIGVNFSPVT
151  KHFRCPAEEV FLPKRSFSLG SPMMKRCTNH TPTICVVGGS QGAQILNTCV
201  PQALVKLVNK YPNMYVHHIV GPKSDVMKVQ HVYNRGEVLC CVKPFEEQLL
251  DVLLAADLVI SRAGATILEE ILWAKVPGIL IPYPGAYGHQ EVNAKFFVDV
301  LEGGTMILEK ELTEKLLVEK VTFALDSHNR EKQRNSLAAY SQQRSTKTFH
351  AFICECL*
```

The cp7224 nucleotide sequence <SEQ ID 108> is:

```
   1  ATGATGAAGA AAATTCGAAA AGTAGCCTTG GCTGTAGGAG GTTCAGGAGG
  51  CCACATTGTC CCAGCTCTCT CGGTAAAGGA AGCTTTTTCT CGTGAAGGAA
 101  TAGACGTATT ACTACTAGGG AAAGGTCTCA AGAACCATCC TTCTTTGCAA
 151  CAGGGAATCA GCTATCGGGA AATCCCCTCA GGACTTCCTA CAGTCCTTAA
 201  TCCCATAAAG ATCATGAGCA GGACCCTTTC TCTATGTTCA GGATACCTGA
 251  AAGCAAGAAA GGAACTTAAA ATTTTTGACC CTGACCTGGT CATAGGATTT
 301  GGGAGCTACC ACTCTCTTCC CGTGTTGCTC GCAGGACTGT CCCATAAAAT
 351  TCCCTTATTT CTACACGAAC AAAATCTAGT TCCTGGAAAA GTAAATCAAT
 401  TGTTTTCCCG CTATGCTCGA GGTATTGGAG TGAATTTCTC CCCCGTTACT
 451  AAACACTTCC GCTGCCCCGC AGAAGAGGTC TTCCTTCCTA AACGAAGCTT
 501  CTCCTTAGGA AGCCCTATGA TGAAGCGATG TACAAATCAT ACCCCTACAA
 551  TCTGTGTTGT TGGAGGTTCT CAGGGAGCAC AGATATTAAA TACTTGTGTT
 601  CCCCAAGCTC TTGTCAAGCT AGTCAATAAG TACCCAAATA TGTACGTCCA

 651  TCATATTGTA GGACCTAAAA GTGATGTTAT GAAGGTGCAA CATGTTTACA
 701  ATCGTGGAGA GGTCCTCTGC TGTGTGAAGC CGTTCGAAGA GCAACTCCTA
 751  GATGTCTTGC TTGCCGCAGA TTTGGTCATC AGTAGGGCAG GAGCCACAAT
 801  TTTAGAAGAA ATTCTTTGGG CAAAAGTTCC CGGAATTTTA ATTCCCTATC
 851  CAGGAGCTTA TGGACATCAG GAAGTTAATG CTAAATTCTT TGTAGACGTC
 901  TTAGAAGGGG GAACTATGAT CCTAGAAAAA GAATTAACAG AGAAGCTATT
 951  AGTAGAAAAA GTAACGTTTG CTTTAGACTC CCATAACAGA GAAAAACAAC
1001  GCAATTCCCT AGCGGCGTAT AGTCAGCAAA GGTCAACAAA AACATTCCAT
1051  GCATTCATTT GTGAATGCTT ATAG
```

The PSORT algorithm predicts an inner membrane location (0.164).

The protein was expressed in *E.coli* and purified as a GST-fusion product, as shown in Figure 54A. The recombinant protein was used to immunise mice, whose sera were used in a Western blot (Figure 54B) and for FACS analysis (Figure 54C). A his-tagged protein was also expressed.

This protein also showed good cross-reactivity with human sera, including sera from patients with pneumonitis.

These experiments show that cp7224 is a surface-exposed and immunoaccessible protein, and that it is a useful immunogen. These properties are not evident from the sequence alone.

### Example 55

The following *C.pneumoniae* protein (PID 4377140) was expressed <SEQ ID 109; cp7140>:

```
  1  MVRRSISFCL FFLMTLLCCT SCNSRSLIVH GLPGREANEI VVLLVSKGVA
 51  AQKLPQAAAA TAGAATEQMW DIAVPSAQIT EALAILNQAG LPRMKGTSLL
101  DLFAKQGLVP SELQEKIRYQ EGLSEQMAST IRKMDGVVDA SVQISFTTEN
151  EDNLPLTASV YIKHRGVLDN PNSIMVSKIK RLIASAVPGL VPENVSVVSD
201  RAAYSDITIN GPWGLTEEID YVSVWGIILA KSSLTKFRLI FYVLILILFV
251  ISCGLLWVIW KTHTLIMTMG GTKGFFNPTP YTKNALEAKK AEGAAADKEK
301  KEDADSQGES KNAETSDKDS SDKDAPEGSN EIEGA*
```

A predicted signal peptide is highlighted.

The cp7140 nucleotide sequence <SEQ ID 110> is:

```
   1  ATGGTTCGTC GATCTATTTC TTTTTGCTTG TTCTTTCTAA TGACATTGCT
  51  GTGCTGTACA AGCTGTAACA GCAGGTCTCT AATTGTGCAC GGTCTTCCTG
 101  GCAGAGAAGC GAATGAGATT GTGGTGCTTT TGGTAAGCAA AGGGGTGGCT
 151  GCACAAAAAT TGCCTCAAGC TGCAGCGGCT ACAGCCGGAG CAGCTACTGA
 201  GCAAATGTGG GATATCGCGG TTCCGTCAGC ACAAATCACA GAGGCCCTTG
 251  CCATTCTAAA TCAAGCGGGT CTTCCACGTA TGAAAGGGAC AAGCCTGTTA
 301  GATCTTTTTG CAAAACAAGG TCTTGTTCCT TCCGAGCTTC AGGAAAAAAT
 351  CCGTTATCAA GAAGGCTTAT CAGAACAGAT GGCCTCTACG ATTAGAAAAA
 401  TGGATGGCGT TGTCGATGCC TCAGTACAGA TTTCCTTCAC TACAGAAAAT
 451  GAAGATAATC TTCCTTTAAC AGCCTCTGTG TATATTAAGC ATCGAGGGGT
 501  TTTGGACAAT CCGAACAGCA TTATGGTTTC CAAAATTAAG CGCCTTATTG
 551  CAAGTGCTGT TCCAGGACTT GTGCCAGAGA ACGTCTCTGT AGTGAGCGAT
 601  CGCGCAGCTT ATAGTGATAT TACAATTAAT GGTCCTTGGG GATTAACAGA
 651  AGAAATCGAT TATGTTTCTG TTTGGGGTAT TATTCTTGCG AAGTCTTCGC
 701  TCACCAAATT CCGTCTCATT TTTTATGTCT TGATTCTCAT TTTATTTGTT
 751  ATTTCTTGTG GTCTCCTTTG GGTCATTTGG AAAACTCATA CTCTCATTAT
 801  GACTATGGGA GGTACAAAAG GGTTCTTCAA CCCTACACCA TATACAAAGA
 851  ATGCCTTGGA AGCCAAGAAA GCCGAGGGAG CAGCTGCTGA CAAAGAGAAA
 901  AAAGAAGATG CAGATTCACA GGGGGAAAGC AAAAATGCGG AAACCAGTGA
 951  TAAAGACTCT AGTGATAAAG ATGCTCCAGA AGGAAGCAAT GAAATTGAGG
1001  GTGCTTAG
```

The PSORT algorithm predicts an inner membrane location (0.650).

The protein was expressed in *E.coli* and purified as a GST-fusion product, as shown in Figure 55A. The recombinant protein was used to immunise mice, whose sera were used in a Western blot (Figure 55B) and for FACS analysis (Figure 55C). A his-tagged protein was also expressed.

These experiments show that cp7140 is a surface-exposed and immunoaccessible protein, and that it is a useful immunogen. These properties are not evident from the sequence alone.

**Example 56**

The following *C.pneumoniae* protein (PID 4377306) was expressed <SEQ ID 111; cp7306>:

```
  1  MITKQLRSWL AVLVGSSLLA LPLSGQAVGK KESRVSELPQ DVLLKEISGG
 51  FSKVATKATP AVVYIESFPK SQAVTHPSPG RRGPYENPFD YFNDEFFNRF
101  FGLPSQREKP QSKEAVRGTG FLVSPDGYIV TNNHVVEDTG KIHVTLHDGQ
151  KYPATVIGLD PKTDLAVIKI KSQNLPYLSF GNSDHLKVGD WAIAIGNPFG
201  LQATVTVGVI SAKGRNQLHI ADFEDFIQTD AAINPGNSGG PLLNIDGQVI
251  GVNTAIVSGS GGYIGIGFAI PSLMANRIID QLIRDGQVTR GFLGVTLQPI
301  DAELAACYKL EKVYGALVTD VVKGSPADKA GLKQEDVIIA YNGKEVDSLS
351  MFRNAVSLMN PDTRIVLKVV REGKVIEIPV TVSQAPKEDG MSALQRVGIR
401  VQNLTPETAK KLGIAPETKG ILIISVEPGS VAASSGIAPG QLILAVNRQK
451  VSSIEDLNRT LKDSNNENIL LMVSQGDVIR FIALKPEE*
```

A predicted signal peptide is highlighted.

The cp7306 nucleotide sequence <SEQ ID 112> is:

```
   1 ATGATAACTA AGCAATTGCG TTCGTGGCTA GCTGTACTTG TTGGTTCAAG
  51 TCTGCTAGCT CTTCCTTTAT CAGGGCAAGC TGTCGGGAAA AAAGAATCTC
 101 GAGTTTCCGA GCTGCCTCAA GACGTTCTTC TTAAAGAGAT CTCGGGAGGG
 151 TTTTCTAAGG TCGCTACCAA GGCGACTCCC GCTGTTGTGT ACATAGAAAG
 201 TTTCCCAAAG AGCCAGGCTG TAACACATCC TTCTCCTGGA CGCCGTGGGC
 251 CTTATGAAAA TCCTTTTGAT TATTTTAATG ATGAGTTTTT CAATCGTTTT
 301 TTTGGTCTAC CTTCACAGAG GGAAAAACCT CAAAGTAAAG AGGCGGTTCG
 351 AGGAACAGGT TTCCTAGTAT CTCCAGATGG CTATATTGTG ACTAATAACC
 401 ATGTTGTCGA AGATACAGGT AAGATTCACG TAACTCTTCA TGATGGGCAA
 451 AAGTACCCAG CAACTGTAAT CGGACTCGAT CCTAAAACAG ACCTTGCAGT
 501 CATTAAAATT AAATCCCAAA ACCTCCCGTA TCTTTCTTTT GGAAACTCCG
 551 ACCACTTAAA AGTCGGAGAT TGGGCAATTG CAATTGGAAA TCCCTTCGGT
 601 CTTCAAGCTA CGGTCACCGT AGGTGTCATC AGTGCTAAAG GAAGAAATCA
 651 ACTCCACATT GCAGATTTTG AAGATTTTAT TCAGACAGAT GCTGCGATTA
 701 ATCCAGGCAA CTCTGGAGGC CCTCTTCTAA ATATTGATGG ACAGGTCATC
 751 GGTGTTAATA CTGCCATTGT CAGTGGTAGT GGTGGCTATA TTGGAATCGG
 801 GTTTGCGATT CCTAGCCTTA TGGCAAATAG AATCATAGAT CAGCTGATTC
 851 GTGATGGTCA AGTTACCCGA GGATTCTTAG GAGTGACTTT ACAACCTATA
 901 GATGCGGAAC TCGCTGCTTG CTACAAACTC GAAAAGGTTT ATGGCGCTTT
 951 AGTCACAGAT GTTGTTAAAG GATCTCCAGC AGATAAAGCA GGGCTAAAAC
1001 AAGAAGATGT GATCATTGCT TATAATGGGA AAGAAGTCGA TTCACTGAGT
1051 ATGTTCCGTA ATGCTGTTTC TTTAATGAAT CCAGATACAC GTATTGTTCT
1101 AAAGGTAGTT CGTGAAGGAA AGGTTATCGA AATACCCGTG ACAGTTTCTC
1151 AAGCTCCAAA AGAAGATGGA ATGTCGGCTT TACAGCGTGT GGGAATCCGT
1201 GTGCAAAACC TAACTCCTGA AACTGCTAAG AAGCTGGGAA TTGCTCCAGA
1251 GACTAAAGGC ATTTTGATTA TAAGTGTTGA ACCAGGGTCT GTAGCAGCTT
1301 CTTCAGGAAT TGCTCCTGGT CAGCTGATCC TTGCTGTGAA TAGACAAAAA
1351 GTATCTTCGA TTGAAGATCT GAATAGAACG TTAAAAGATT CTAACAATGA
1401 GAATATTCTT CTTATGGTTT CTCAAGGAGA TGTTATTCGC TTCATTGCCC
1451 TGAAACCTGA AGAATAA
```

The PSORT algorithm predicts a periplasmic location (0.923).

The protein was expressed in *E.coli* and purified as a his-tag product (Figure 56A) and as a GST-fusion product (Figure 56B). The recombinant proteins were used to immunise mice, whose sera were used in a Western blot (Figure 56C) and for FACS (Figure 56D) analyses.

The cp7306 protein was also identified in the 2D-PAGE experiment (Cpn0979) and showed good cross-reactivity with human sera, including sera from patients with pneumonitis.

These experiments show that cp7306 is a surface-exposed and immunoaccessible protein, and that it is a useful immunogen. These properties are not evident from the sequence alone.

**Example 57**

The following *C.pneumoniae* protein (PID 4377132) was expressed <SEQ ID 113; cp7132>:

```
  1 MCNSIAMKKQ KRGFVLMELL MSFTLIALLL GTLGFWYRKI YTVQKQKERI
 51 YNFYIEESRA YKQLRTLFSM SLSSSYEEPG SLFSLIFDRG VYRDPKLAGA
101 VRASLHHDTK DQRLELRICN IKDQSYFETQ RLLSHVTHVV LSFQRNPDPE
151 KLPETIALTI TREPKAYPPR TLTYQFAVGK*
```

A predicted signal peptide is highlighted.

The cp7132 nucleotide sequence <SEQ ID 114> is:

```
  1 ATGTGTAACT CTATAGCTAT GAAAAAGCAA AAGCGTGGCT TTGTGCTTAT
 51 GGAATTACTC ATGTCGTTCA CTCTAATTGC TTTGTTATTA GGGACTTTAG
101 GATTTTGGTA TCGGAAAATT TATACTGTAC AAAAGCAAAA AGAACGTATT
151 TATAACTTTT ATATCGAAGA AAGCCGAGCC TACAAGCAGC TCAGAACCCT
201 GTTTAGCATG TCCTTGTCTT CATCTTACGA GGAGCCTGGA TCATTATTTT
251 CTTTAATCTT TGATCGGGGT GTTTATCGAG ATCCTAAGCT GGCAGGTGCG
301 GTACGAGCTT CTCTCCATCA TGACACCAAG GATCAGAGAT TGGAACTTCG
351 TATTTGTAAT ATTAAGGATC AGTCTTACTT TGAAACACAG CGACTGCTCT
401 CCCACGTGAC CCATGTTGTA CTTTCCTTCC AGAGAAATCC TGATCCTGAA
451 AAACTTCCTG AAACAATTGC TTTAACTATA ACACGGGAAC CTAAAGCATA
501 TCCTCCAAGG ACGTTAACAT ACCAATTTGC GGTTGGGAAA TAA
```

The PSORT algorithm predicts a periplasmic location (0.915).

The protein was expressed in *E.coli* and purified as a his-tag product (Figure 57A) or as a GST-fusion. The recombinant proteins were used to immunise mice, whose sera were used in a Western blot (Figure 57B) and FACS (Figure 57C) analyses.

These experiments show that cp7132 is a surface-exposed and immunoaccessible protein, and that it is a useful immunogen. These properties are not evident from the sequence alone.

## Example 58

The following *C.pneumoniae* protein (PID 4376733) was expressed <SEQ ID 115; cp6733>:

```
  1 MKTSIPWVLV SSVLAFSCHL QSLANEELLS PDDSFNGNID SGTFTPKTSA
 51 TTYSLTGDVF FYEPGKGTPL SDSCFKQTTD NLTFLGNGHS LTFGFIDAGT
101 HAGAAASTTA NKNLTFSGFS LLSFDSSPST TVTTGQGTLS SAGGVNLENI
151 RKLVVAGNFS TADGGAIKGA SFLLTGTSGD ALFSNNSSST KGGAIATTAG
201 ARIANNTGYV RFLSNIASTS GGAIDDEGTS ILSNNKFLYF EGNAAKTTGG
251 AICNTKASGS PELIISNNKT LIFASNVAET SGGAIHAKKL ALSSGGFTEF
301 LRNNVSSATP KGGAISIDAS GELSLSAETG NITFVRNTLT TTGSTDTPKR
351 NAINIGSNGK FTELRAAKNH TIFFYDPITS EGTSSDVLKI NNGSAGALNP
401 YQGTILFSGE TLTADELKVA DNLKSSFTQP VSLSGGKLLL QKGVTLESTS
451 FSQEAGSLLG MDSGTTLSTT AGSITITNLG INVDSLGLKQ PVSLTAKGAS
501 NKVIVSGKLN LIDIEGNIYE SHMFSHDQLF SLLKITVDAD VDTNVDISSL
551 IPVPAEDPNS EYGFQGQWNV NWTTDTATNT KEATATWTKT GFVPSPERKS
601 ALVCNTLWGV FTDIRSLQQL VEIGATGMEH KQGFWVSSMT NFLHKTGDEN
651 RKGFRHTSGG YVIGGSAHTP KDDLFTFAFC HLFARDKDCF IAHNNSRTYG
701 GTLFFKHSHT LQPQNYLRLG RAKFSESAIE KFPREIPLAL DVQVSFSHSD
751 NRMETHYTSL PESEGSWSNE CIAGGIGLDL PFVLSNPHPL FKTFIPQMKV
801 EMVYVSQNSF FESSSDGRGF SIGRLLNLSI PVGAKFVQGD IGDSYTYDLS

851 GFFVSDVYRN NPQSTATLVM SPDSWKIRGG NLSRQAFLLR GSNNYVYNSN
901 CELFGHYAME LRGSSRNYNV DVGTKLRF*
```

A predicted signal peptide is highlighted.

The cp6733 nucleotide sequence <SEQ ID 116> is:

```
   1  ATGAAGACTT CGATTCCTTG GGTTTAGTT TCCTCCGTGT TAGCTTTCTC
  51  ATGTCACCTA CAGTCACTAG CTAACGAGGA ACTTTTATCA CCTGATGATA
 101  GCTTTAATGG AAATATCGAT TCAGGAACGT TTACTCCAAA AACTTCAGCC
 151  ACAACATATT CTCTAACAGG AGATGTCTTC TTTTACGAGC CTGGAAAAGG
 201  CACTCCCTTA TCTGACAGTT GTTTTAAGCA AACCACGGAC AATCTTACCT
 251  TCTTGGGGAA CGGTCATAGC TTAACGTTTG GCTTTATAGA TGCTGGCACT
 301  CATGCAGGTG CTGCTGCATC TACAACAGCA AATAAGAATC TTACCTTCTC
 351  AGGGTTTTCC TTACTGAGTT TTGATTCCTC TCCTAGCACA ACGGTTACTA
 401  CAGGTCAGGG AACGCTTTCC TCAGCAGGAG GCGTAAATTT AGAAAATATT
 451  CGTAAACTTG TAGTTGCTGG GAATTTTTCT ACTGCAGATG GTGGAGCTAT
 501  CAAAGGAGCG TCTTTCCTTT TAACTGGCAC TTCTGGAGAT GCTCTTTTTA
 551  GTAACAACTC TTCATCAACA AAGGGAGGAG CAATTGCTAC TACAGCAGGC
 601  GCTCGCATAG CAAATAACAC AGGTTATGTT AGATTCCTAT CTAACATAGC
 651  GTCTACGTCA GGAGGCGCTA TCGATGATGA AGGCACGTCG ATACTATCGA
 701  ACAACAAATT TCTATATTTT GAAGGGAATG CAGCGAAAAC TACTGGCGGT
 751  GCGATCTGCA ACACCAAGGC GAGTGGATCT CCTGAACTGA TAATCTCTAA
 801  CAATAAGACT CTGATCTTTG CTTCAAACGT AGCAGAAACA AGCGGTGGCG
 851  CCATCCATGC TAAAAAGCTA GCCCTTTCCT CTGGAGGCTT TACAGAGTTT
 901  CTACGAAATA ATGTCTCATC AGCAACTCCT AAGGGGGGTG CTATCAGCAT
 951  CGATGCCTCA GGAGAGCTCA GTCTTTCTGC AGAGACAGGA AACATTACCT
1001  TTGTAAGAAA TACCCTTACA ACAACCGGAA GTACCGATAC TCCTAAACGT
1051  AATGCGATCA ACATAGGAAG TAACGGGAAA TTCACGGAAT TACGGGCTGC
1101  TAAAAATCAT ACAATTTTCT TCTATGATCC CATCACTTCA GAAGGAACCT
1151  CATCAGACGT ATTGAAGATA AATAACGGCT CTGCGGGAGC TCTCAATCCA
1201  TATCAAGGAA CGATTCTATT TTCTGGAGAA ACCCTAACAG CAGATGAACT
1251  TAAAGTTGCT GACAATTTAA AATCTTCATT CACGCAGCCA GTCTCCCTAT
1301  CCGGAGGAAA GTTATTGCTA CAAAAGGGAG TCACTTTAGA GAGCACGAGC
1351  TTCTCTCAAG AGGCCGGTTC TCTCCTCGGC ATGGATTCAG GAACGACATT
1401  ATCAACTACA GCTGGGAGTA TTACAATCAC GAACCTAGGA ATCAATGTTG
1451  ACTCCTTAGG TCTTAAGCAG CCCGTCAGCC TAACAGCAAA AGGTGCTTCA
1501  AATAAAGTGA TCGTATCTGG GAAGCTCAAC CTGATTGATA TTGAAGGGAA
1551  CATTTATGAA AGTCATATGT TCAGCCATGA CCAGCTCTTC TCTCTATTAA
1601  AAATCACGGT TGATGCTGAT GTTGATACTA ACGTTGACAT CAGCAGCCTT
1651  ATCCCTGTTC CTGCTGAGGA TCCTAATTCA GAATACGGAT TCCAAGGACA
1701  ATGGAATGTT AATTGGACTA CGGATACAGC TACAAATACA AAAGAGGCCA
1751  CGGCAACTTG GACCAAAAACA GGATTTGTTC CCAGCCCCGA AAGAAAATCT
1801  GCGTTAGTAT GCAATACCCT ATGGGGAGTC TTTACTGACA TTCGCTCTCT
1851  GCAACAGCTT GTAGAGATCG GCGCAACTGG TATGGAACAC AAACAAGGTT
1901  TCTGGGTTTC CTCCATGACG AACTTCCTGC ATAAGACTGG AGATGAAAAT
1951  CGCAAAGGCT TCCGTCATAC CTCTGGAGGC TACGTCATCG GTGGAAGTGC
2001  TCACACTCCT AAAGACGACC TATTTACCTT TGCGTTCTGC CATCTCTTTG
2051  CTAGAGACAA AGATTGTTTT ATCGCTCACA ACAACTCTAG AACCTACGGT
2101  GGAACTTTAT TCTTCAAGCA CTCTCATACC CTACAACCCC AAAACTATTT
2151  GAGATTAGGA AGAGCAAAGT TTTCTGAATC AGCTATAGAA AAATTCCCTA
2201  GGGAAATTCC CCTAGCCTTG GATGTCCAAG TTTCGTTCAG CCATTCAGAC
2251  AACCGTATGG AAACGCACTA TACCTCATTG CCAGAATCCG AAGGTTCTTG
2301  GAGCAACGAG TGTATAGCTG GTGGTATCGG CCTAGACCTT CCTTTTGTTC
2351  TTTCCAACCC ACATCCTCTT TTCAAGACCT TCATTCCACA GATGAAAGTC
2401  GAAATGGTTT ATGTATCACA AAATAGCTTC TTCGAAAGCT CTAGTGATGG
2451  CCGTGGTTTT AGTATTGGAA GGCTGCTTAA CCTCTCGATT CCTGTGGGTG
2501  CGAAATTCGT GCAGGGGGAT ATCGGAGATT CCTACACCTA TGATCTCTCA
2551  GGATTCTTTG TTTCCGATGT CTATCGTAAC AATCCCCAAT CTACAGCGAC
2601  TCTTGTGATG AGCCCAGACT CTTGGAAAAT TCGCGGTGGC AATCTTTCAA
2651  GACAGGCATT TTTACTGAGG GGTAGCAACA ACTACGTCTA CAACTCCAAT
2701  TGTGAGCTCT TCGGACATTA CGCTATGGAA CTCCGTGGAT CTTCAAGGAA
2751  CTACAATGTA GATGTTGGTA CCAAACTCCG ATTCTAG
```

The PSORT algorithm predicts an outer membrane location (0.924).

The protein was expressed in *E.coli* and purified as a his-tag product, as shown in Figure 58A. The recombinant protein was used to immunise mice, whose sera were used in a Western blot (Figure 58B) and for FACS (Figure 58C) analyses. A GST-fusion protein was also expressed.

The cp6733 protein was also identified in the 2D-PAGE experiment (Cpn0451).

These experiments show that cp6733 is a surface-exposed and immunoaccessible protein, and that it is a useful immunogen. These properties are not evident from the sequence alone.

**Example 59**

The following *C.pneumoniae* protein (PID 4376814) was expressed <SEQ ID 117; cp6814>:

1 MHDALLSILA IQELDIKMIR LMRVKKEHQK ELAKVQSLKS DIRRKVQEKE

51 LEMENLKTQI RDGENRIQEI SEQINKLENQ QAAVKKMDEF NALTQEMTTA

101 NKERRSLEHQ LSDLMDKQAG GEDLIVSLKE SLASTENSSS VIEKEIFESI

151 KKINEEGKAL LEQRTELKHA TNPELLSIYE RLLNNKKDRV VVPIENRVCS

201 GCHIVLTPQH ENLVRKKDRL IFCEHCSRIL YWQESQVNAQ ENSTAKRRRR

251 RAAV*

The cp6814 nucleotide sequence <SEQ ID 118> is:

1 ATGCATGACG CACTTCTAAG CATTTTGGCT ATTCAAGAGC TTGATATTAA

51 AATGATTCGC CTTATGCGCG TAAAGAAAGA ACATCAGAAA GAATTGGCTA

101 AAGTCCAATC TTTAAAAAGT GATATTCGTA GAAAAGTTCA GGAAAAAGAA

151 CTCGAAATGG AGAATTTGAA AACTCAAATT CGAGATGGAG AGAATCGCAT

201 CCAAGAGATT TCTGAACAAA TCAATAAATT AGAAAATCAG CAAGCTGCTG

251 TAAAAAAAAT GGATGAGTTT AACGCTCTTA CCCAAGAAAT GACTACAGCA

301 AACAAAGAAC GTCGCTCTTT AGAGCACCAG CTTAGCGATC TCATGGATAA

351 GCAAGCTGGA GGCGAAGACC TTATTGTCTC TCTAAAAGAA AGCTTAGCTT

401 CTACAGAAAA TAGTAGCAGT GTCATTGAAA AAGAAATTTT TGAAAGCATC

451 AAAAAGATTA ATGAAGAAGG CAAAGCTTTG CTTGAACAAC GGACAGAGTT

501 AAAGCATGCG ACGAATCCCG AACTACTCAG CATCTATGAG CGTCTATTAA

551 ACAATAAAAA AGATCGCGTT GTTGTTCCTA TTGAAAATCG TGTCTGCAGT

601 GGTTGTCATA TTGTTCTAAC TCCTCAACAC GAAAATCTTG TAAGAAAGAA

651 AGACCGACTC ATTTTTTGCG AACATTGCTC TCGAATTCTC TATTGGCAAG

701 AATCCCAAGT CAATGCTCAG GAAAATTCCA CAGCAAAACG TCGTCGTCGT

751 CGCGCAGCTG TATAA

The PSORT algorithm predicts an inner membrane location (0.070).

The protein was expressed in *E.coli* and purified as a GST-fusion (Figure 59A) or his-tagged product. The recombinant proteins were used to immunise mice, whose sera were used in Western blot (Figure 59B) and FACS (Figure 59C) analyses.

These experiments show that cp6814 is a surface-exposed and immunoaccessible protein, and that it is a useful immunogen. These properties are not evident from the sequence alone.

**Example 60**

The following *C.pneumoniae* protein (PID 4376830) was expressed <SEQ ID 119; cp6830>:

1 **MKWLPATAVF AAVLPALTAF G**DPASVEIST SHTGSGDPTS DAALTGFTQS

51 STETDGTTYT IVGDITFSTF TNIPVPVVTP DANDSSSNSS KGGSSSSGAT

101 SLIRSSNLHS DFDFTKDSVL DLYHLFFPSA SNTLNPALLS SSSSGGSSSS

151 SSSSSSGSAS AVVAADPKGG AAFYSNEANG TLTFTTDSGN PGSLTLQNLK

201 MTGDGAAIYS KGPLVFTGLK NLTFTGNESQ KSGGAAYTEG ALTTQAIVEA

251 VTFTGNTSAG QGGAIYVKEA TLFNALDSLK FEKNTSGQAG GGIYTESTLT

301 ISNITKSIEF ISNKASVPAP APEPTSPAPS SLINSTTIDT STLQTRAASA

351 TPAVAPVAAV TPTPISTQET AGNGGAIYAK QGISISTFKD LTFKSNSASV

```
 401 DATLTVDSST IGESGGAIFA ADSIQIQQCT GTTLFSGNTA NKSGGGIYAV
 451 GQVTLEDIAN LKMTNNTCKG EGGAIYTKKA LTINNGAILT TFSGNTSTDN
 501 GGAIFAVGGI TLSDLVEVRF SKNKTGNYSA PITKAASNTA PVVSSSTTAA
 551 SPAVPAAAAA PVTNAAKGGA LYSTEGLTVS GITSILSFEN NECQNQGGGA
 601 YVTKTFQCSD SHRLQFTSNK AADEGGGLYC GDDVTLTNLT GKTLFQENSS
 651 EKHGGGLSLA SGKSLTMTSL ESFCLNANTA KENGGGANVP ENIVLTFTYT
 701 PTPNEPAPVQ QPVYGEALVT GNTATKSGGG IYTKNAAFSN LSSVTFDQNT
 751 SSENGGALLT QKAADKTDCS FTYITNVNIT NNTATGNGGG IAGGKAHFDR
 801 IDNLTVQSNQ AKKGGGVYLE DALILEKVIT GSVSQNTATE SGGGIYAKDI
 851 QLQALPGSFT ITDNKVETSL TTSTNLYGGG IYSSGAVTLT NISGTFGITG
 901 NSVINTATSQ DADIQGGGIY ATTSLSINQC NTPILFSNNS AATKKTSTTK
 951 QIAGGAIFSA AVTIENNSQP IIFLNNSAKS EATTAATAGN KDSCGGAIAA
1001 NSVTLTNNPE ITFKGNYAET GGAIGCIDLT NGSPPRKVSI ADNGSVLFQD
1051 NSALNRGGAI YGETIDISRT GATFIGNSSK HDGSAICCST ALTLAPNSQL
1101 IFENNKVTET TATTKASINN LGAAIYGNNE TSDVTISLSA ENGSIFFKNN
1151 LCTATNKYCS IAGNVKFTAI EASAGKAISF YDAVNVSTKE TNAQELKLNE
1201 KATSTGTILF SGELHENKSY IPQKVTFAHG NLILGKNAEL SVVSFTQSPG
1251 TTITMGPGSV LSNHSKEAGG IAINNVIIDF SEIVPTKDNA TVAPPTLKLV
1301 SRTNADSKDK IDITGTVTLL DPNGNLYQNS YLGEDRDITL FNIDNSASGA
1351 VTATNVTLQG NLGAKKGYLG TWNLDPNSSG SKIILKWTFD KYLRWPYIPR
1401 DNHFYINSIW GAQNSLVTVK QGILGNMLNN ARFEDPAFNN FWASAIGSFL
1451 RKEVSRNSDS FTYHGRGYTA AVDAKPRQEF ILGAAFSQVF GHAESEYHLD
1501 NYKHKGSGHS TQASLYAGNI FYFPAIRSRP ILFQGVATYG YMQHDTTTYY
1551 PSIEEKNMAN WDSIAWLFDL RFSVDLKEPQ PHSTARLTFY TEAEYTRIRQ
1601 EKFTELDYDP RSFSACSYGN LAIPTGFSVD GALAWREIIL YNKVSAAYLP
1651 VILRNNPKAT YEVLSTKEKG NVVNVLPTRN AARAEVSSQI YLGSYWTLYG
1701 TYTIDASMNT LVQMANGGIR FVF*
```

A predicted signal peptide is highlighted.

The cp6830 nucleotide sequence <SEQ ID 120> is:

```
   1 ATGAAGTGGC TACCAGCTAC AGCTGTTTTT GCTGCCGTAC TCCCCGCACT
  51 AACAGCCTTC GGAGATCCCG CGTCTGTTGA AATAAGTACC AGCCATACAG
 101 GATCCGGGGA TCCTACAAGC GACGCTGCCT TAACAGGATT TACACAAAGT
 151 TCCACAGAAA CTGACGGTAC TACCTATACC ATTGTCGGTG ATATCACCTT
 201 CTCTACTTTT ACGAATATTC CTGTTCCCGT AGTAACTCCA GACGCCAACG
 251 ATAGTTCCAG CAATAGCTCT AAAGGAGGAA GTAGCAGTAG TGGAGCTACA
 301 TCTCTAATCC GATCCTCAAA CCTACACTCC GATTTTGATT TTACAAAAGA
 351 TAGCGTGTTA GACCTCTATC ACCTTTTCTT TCCTTCAGCT TCAAATACTC
 401 TCAATCCTGC ACTCCTTTCT TCCAGTAGCA GCGGTGGATC CTCGAGCAGC
 451 AGTAGCTCCT CATCATCTGG AAGTGCATCT GCTGTTGTTG CTGCGGACCC
 501 AAAAGGAGGC GCTGCCTTTT ATAGTAACGA GGCTAACGGA ACTTTAACCT
 551 TCACTACAGA CTCTGGAAAT CCCGGCTCCC TGACTCTTCA GAATCTTAAA
 601 ATGACCGGAG ATGGAGCCGC CATCTACTCG AAGGGTCCTC TAGTATTTAC
 651 TGGTTTAAAA AATCTAACCT TTACAGGAAA TGAATCTCAG AAATCTGGAG
 701 GTGCTGCCTA TACTGAAGGC GCACTCACAA CACAAGCAAT CGTTGAAGCC
 751 GTAACTTTTA CTGGCAACAC CTCGGCAGGG CAAGGAGGCG CTATCTATGT
 801 TAAAGAAGCT ACCCTATTCA ATGCTCTAGA CAGCCTCAAA TTTGAAAAAA
 851 ACACTTCTGG GCAAGCTGGT GGTGGAATCT ATACAGAGTC TACGCTCACA
 901 ATCTCGAACA TCACAAAATC TATTGAATTT ATCTCTAATA AAGCTTCTGT
 951 CCCTGCCCCC GCTCCTGAGC CCACCTCTCC GGCTCCAAGT AGCTTAATAA
1001 ATTCTACAAC GATCGATACC TCGACTCTCC AAACCCGAGC AGCATCCGCA
1051 ACTCCAGCAG TGGCTCCTGT TGCTGCCGTA ACTCCAACAC CAATCTCTAC
1101 TCAAGAGACC GCAGGAAATG GAGGCGCTAT CTATGCTAAA CAAGGTATTT
1151 CGATATCCAC GTTTAAAGAT CTGACCTTCA AGTCTAACTC TGCATCGGTA
1201 GATGCCACCC TTACTGTCGA TTCTAGCACT ATTGGAGAAT CTGGAGGTGC
1251 TATCTTTGCA GCAGACTCTA TACAAATCCA ACAGTGCACG GGAACCACCT
1301 TATTCAGTGG CAATACTGCC AATAAGTCTG GTGGGGGTAT TTACGCTGTA
1351 GGACAAGTCA CCCTAGAAGA TATAGCGAAT CTGAAGATGA CCAACAACAC
1401 CTGTAAAGGT GAAGGTGGAG CCATCTACAC TAAAAAGGCT TTAACTATCA
1451 ACAACGGTGC CATTCTCACT ACATTTTCTG GAAATACATC GACAGATAAT
1501 GGTGGGGCTA TTTTTGCTGT AGGTGGCATC ACTCTCTCTG ATCTTGTAGA
1551 AGTCCGCTTT AGTAAAAATA AGACCGGAAA TTATTCCGCT CCTATTACCA
1601 AAGCGGCTAG CAACACAGCT CCTGTAGTTT CTAGCTCTAC AACTGCTGCA
1651 TCTCCTGCGG TCCCTGCTGC CGCTGCAGCA CCTGTTACAA ACGCAGCAAA
1701 AGGAGGGGCT TTATATAGTA CAGAAGGACT GACTGTATCT GGAATCACAT
1751 CGATATTGTC GTTTGAAAAC AACGAATGCC AGAATCAAGG AGGTGGGGCT
```

```
1801  TACGTTACTA AAACCTTCCA GTGTTCCGAT TCTCATCGCC TCCAGTTTAC
1851  TAGTAATAAA GCAGCAGATG AAGGCGGGGG CCTGTATTGT GGTGACGATG
1901  TCACGCTAAC GAACCTGACA GGGAAAACAC TATTTCAAGA GAATAGCAGT
1951  GAGAAACATG GAGGTGGGCT CTCTCTCGCC TCAGGAAAAT CTCTGACTAT
2001  GACATCGTTA GAGAGCTTCT GCTTAAATGC AAATACAGCA AAGGAAAACG
2051  GAGGCGGTGC GAATGTCCCT GAAAATATTG TACTCACCTT CACCTATACT
2101  CCCACTCCAA ATGAACCTGC GCCTGTGCAG CAGCCCGTGT ATGGAGAAGC
2151  TCTTGTTACT GGAAATACAG CCACAAAAAG TGGTGGGGGC ATTTACACGA
2201  AAAATGCGGC CTTCTCAAAT TTATCTTCTG TAACTTTTGA TCAAAATACC
2251  TCTTCAGAAA ATGGTGGTGC CTTACTTACC CAAAAAGCTG CAGATAAAAC
2301  GGACTGTTCT TTCACCTATA TTACAAATGT CAATATCACC AACAATACAG
2351  CTACAGGAAA TGGTGGGGGC ATTGCTGGGG GAAAAGCACA TTTCGATCGC
2401  ATTGATAATC TTACAGTCCA AAGCAACCAA GCAAAGAAAG GTGGTGGGGT
2451  TTATCTTGAA GATGCCCTCA TCCTGGAAAA GGTTATTACA GGTTCTGTCT
2501  CACAAAATAC AGCTACAGAA AGTGGTGGGG GTATCTACGC TAAGGATATT
2551  CAACTACAAG CTCTACCTGG AAGCTTCACA ATTACCGATA ATAAAGTCGA
2601  AACTAGTCTT ACTACTAGCA CTAATTTATA TGGTGGGGGC ATCTATTCCA
2651  GTGGAGCTGT CACGCTAACC AATATATCTG GAACCTTTGG CATTACAGGA
2701  AACTCTGTTA TCAATACAGC GACATCCCAG GATGCAGATA TACAAGGTGG
2751  GGGCATTTAT GCAACCACGT CTCTCTCAAT AAATCAATGT AATACACCCA
2801  TTCTATTTAG CAACAACTCT GCTGCCACTA AAAAAACATC AACAACAAAG
2851  CAAATTGCTG GTGGGGCTAT CTTCTCCGCT GCAGTAACTA TCGAGAATAA
2901  CTCTCAGCCC ATTATTTTCT TAAATAATTC CGCAAAGTCG GAAGCAACTA
2951  CAGCAGCAAC TGCAGGAAAT AAAGATAGCT GTGGAGGAGC CATTGCAGCT
3001  AACTCTGTTA CTTTAACAAA TAACCCTGAA ATAACCTTTA AAGGAAATTA
3051  TGCAGAAACT GGAGGAGCGA TTGGCTGTAT TGATCTTACT AATGGCTCAC
3101  CTCCCCGTAA AGTCTCTATT GCAGACAACG GTTCTGTCCT TTTTCAAGAC
3151  AACTCTGCGT TAAATCGCGG AGGCGCTATC TATGGAGAGA CTATCGATAT
3201  CTCCAGGACA GGTGCGACTT TCATCGGTAA CTCTTCAAAA CATGATGGAA
3251  GTGCAATTTG CTGTTCAACA GCCCTAACTC TTGCGCCAAA CTCCCAACTT
3301  ATCTTTGAAA ACAATAAGGT TACGGAAACC ACAGCCACTA CAAAAGCTTC
3351  CATAAATAAT TTAGGAGCTG CAATTTATGG AAATAATGAG ACTAGTGACG
3401  TCACTATCTC TTTATCAGCT GAGAATGGAA GTATTTTCTT TAAAAACAAT
3451  CTATGCACAG CAACAAACAA ATACTGCAGT ATTGCTGGAA ACGTAAAATT
3501  TACAGCAATA GAAGCTTCAG CAGGGAAAGC TATATCTTTC TATGATGCAG
3551  TTAACGTTTC CACCAAAGAA ACAAATGCTC AAGAGCTAAA ATTAAATGAA
3601  AAAGCGACAA GTACAGGAAC GATTCTATTT TCTGGGGAAC TTCACGAAAA
3651  TAAATCCTAT ATTCCACAGA AAGTCACTTT CGCACATGGG AATCTCATTC
3701  TAGGTAAAAA TGCAGAACTT AGCGTAGTTT CCTTTACCCA ATCTCCAGGC
3751  ACCACAATCA CTATGGGCCC AGGATCGGTT CTTTCCAACC ATAGCAAAGA
3801  AGCAGGAGGA ATCGCTATAA ACAATGTCAT CATTGATTTT AGTGAAATCG
3851  TTCCTACTAA AGATAATGCA ACAGTAGCTC CACCCACTCT TAAATTAGTA
3901  TCGAGAACTA ATGCAGATAG TAAAGATAAG ATTGATATTA CAGGAACTGT
3951  GACTCTTCTA GATCCTAATG GCAACTTATA TCAAAATTCT TATCTTGGTG
4001  AAGACCGCGA TATCACTCTT TTCAATATAG ACAATTCTGC AAGTGGGGCA
4051  GTTACAGCCA CGAATGTCAC CCTTCAAGGG AATTTAGGAG CTAAAAAAGG
4101  ATATTTAGGA ACCTGGAATT TGGATCCAAA TTCCTCGGGT TCAAAAATTA
4151  TTCTAAAATG GACCTTTGAC AAATACCTGC GCTGGCCCTA CATCCCTAGA
4201  GACAACCACT TCTACATCAA CTCTATTTGG GGAGCACAAA ACTCTTTAGT
4251  GACTGTGAAA CAAGGGATCT TAGGGAACAT GTTGAACAAT GCAAGGTTTG
4301  AAGATCCTGC TTTCAACAAC TTCTGGGCTT CGGCTATAGG ATCTTTCCTT
4351  AGGAAAGAAG TATCTCGAAA TTCTGACTCA TTCACCTATC ATGGCAGAGG
4401  CTATACCGCT GCTGTGGATG CCAAACCTCG CCAAGAATTT ATTTTAGGAG
4451  CTGCCTTCAG TCAGGTTTTT GGTCACGCCG AGTCTGAATA TCACCTTGAC
4501  AACTATAAGC ATAAAGGCTC AGGTCACTCT ACACAAGCAT CTCTTTATGC
4551  TGGCAATATC TTCTATTTTC CTGCGATACG GTCTCGGCCT ATTCTATTCC
4601  AAGGTGTGGC GACCTATGGT TATATGCAAC ATGACACCAC AACCTACTAT
4651  CCTTCTATTG AAGAAAAAAA TATGGCAAAC TGGGATAGCA TTGCTTGGTT
4701  ATTTGATCTG CGTTTCAGTG TGGATCTTAA AGAACCTCAA CCTCACTCTA
4751  CAGCAAGGCT TACCTTCTAT ACAGAAGCTG AGTATACCAG AATTCGCCAG
4801  GAGAAATTCA CAGAGCTAGA CTATGATCCT AGATCTTTCT CTGCATGCTC
4851  TTATGGAAAC TTAGCAATTC CTACTGGATT CTCTGTAGAC GGAGCATTAG
4901  CTTGGCGTGA GATTATTCTA TATAATAAAG TATCAGCTGC GTACCTCCCT
4951  GTGATTCTCA GGAATAATCC AAAAGCGACC TATGAAGTTC TCTCTACAAA
5001  AGAAAAGGGC AACGTAGTCA ACGTTCTCCC TACAAGAAAC GCAGCTCGTG
5051  CAGAGGTGAG CTCTCAAATT TATCTTGGAA GTTACTGGAC ACTCTACGGC
5101  ACGTATACTA TTGATGCTTC AATGAATACT TTAGTGCAAA TGGCCAACGG
5151  AGGGATCCGG TTTGTATTCT AG
```

The PSORT algorithm predicts an outer membrane location (0.926).

The protein was expressed in *E.coli* and purified as a GST-fusion (Figure 60A) or his-tagged product. The recombinant proteins were used to immunise mice, whose sera were used in Western blot (Figure 60B) and FACS (Figure 60C) analyses.

The cp6830 protein was also identified in the 2D-PAGE experiment (Cpn0540) and showed good cross-reactivity with human sera, including sera from patients with pneumonitis.

These experiments show that cp6830 is a surface-exposed and immunoaccessible protein, and that it is a useful immunogen. These properties are not evident from the sequence alone.

**Example 61**

The following *C.pneumoniae* protein (PID 4376854) was expressed <SEQ ID 121; cp6854>:

```
  1 MSIAIAREQY AAILDMHPKP SIAMFSSEQA RTSWEKRQAH PYLYRLLEII
 51 WGVVKFLLGL IFFIPLGLFW VLQKICQNFI LLGAGGWIFR PICRDSNLLR
101 QAYAARLFSA SFQDHVSSVR RVCLQYDEVF IDGLELRLPN AKPDRWMLIS
151 NGNSDCLEYR TVLQGEKDWI FRIAEESQSN ILIFNYPGVM KSQGNITRNN
201 VVKSYQACVR YLRDEPAGPQ ARQIVAYGYS LGASVQAEAL SKEIADGSDS
251 VRWFVVKDRG ARSTGAVAKQ FIGSLGVWLA NLTHWNINSE KRSKDLHCPE
301 LFIYGKDSQG NLIGDGLFKK ETCFAAPFLD PKNLEECSGK KIPVAQTGLR
351 HDHILSDDVI KEVAGHIQRH FDN*
```

The cp6854 nucleotide sequence <SEQ ID 122> is:

```
   1 ATGTCAATAG CTATTGCAAG GGAACAATAC GCAGCTATAT TGGATATGCA
  51 TCCTAAACCT TCGATCGCCA TGTTTTCTTC GGAGCAGGCG AGAACTTCTT
 101 GGGAGAAACG ACAGGCTCAT CCTTACCTTT ATCGTCTTCT TGAGATCATA
 151 TGGGGTGTTG TGAAATTTCT TCTCGGCTTA ATCTTCTTTA TTCCCTTGGG
 201 TCTTTTCTGG GTCCTTCAGA AGATATGTCA GAATTTTATT CTTCTTGGTG
 251 CAGGAGGGTG GATTTTTAGA CCCATATGCA GGGACTCTAA TTTATTGCGA
 301 CAAGCTTACG CCGCGCGTCT TTTCTCCGCT TCATTCCAAG ATCATGTCTC
 351 CTCTGTGCGA AGGGTTTGCT TACAGTATGA CGAGGTCTTT ATTGACGGAT
 401 TGGAGTTACG TCTTCCCAAT GCTAAGCCAG ATCGATGGAT GTTAATCTCC
 451 AATGGAAACT CCGATTGCTT AGAGTATAGG ACAGTGCTGC AAGGGGAAAA
 501 GGACTGGATA TTCCGTATTG CTGAAGAGTC TCAATCCAAC ATTTTAATCT
 551 TCAATTACCC AGGAGTCATG AAGAGCCAAG GGAATATAAC AAGAAACAAT
 601 GTAGTCAAAT CTTATCAAGC ATGCGTACGC TATCTTAGAG ATGAACCCGC
 651 AGGACCTCAG GCGCGTCAAA TCGTTGCTTA TGGCTATTCT TTAGGAGCTA
 701 GTGTTCAAGC CGAAGCATTA AGTAAAGAGA TCGCAGACGG AAGTGATAGC
 751 GTCCGTTGGT TTGTCGTTAA AGATCGAGGA GCTCGCTCTA CAGGAGCCGT
 801 TGCTAAACAG TTTATTGGAA GTCTAGGAGT TTGGCTGGCG AATCTTACCC
 851 ATTGGAATAT TAATTCTGAA AAGAGAAGCA AGGACTTGCA TTGCCCAGAA
 901 CTCTTTATTT ATGGCAAGGA TTCCCAAGGT AATCTTATCG GGGATGGATT
 951 GTTCAAAAAA GAGACGTGCT TCGCAGCACC ATTTTTAGAT CCTAAAAACT
1001 TGGAAGAGTG TTCAGGGAAG AAAATCCCTG TAGCTCAGAC CGGTCTAAGA
1051 CACGATCATA TCCTTTCCGA TGATGTGATT AAAGAAGTTG CAGGTCATAT
1101 TCAAAGACAT TTCGATAATT A
```

The PSORT algorithm predicts an inner membrane location (0.461).

The protein was expressed in *E.coli* and purified as a GST-fusion product, as shown in Figure 61A. The recombinant protein was used to immunise mice, whose sera were used in Western blot (Figure 61B) and FACS (Figure 61C) analyses. A his-tagged protein was also expressed.

These experiments show that cp6854 is a surface-exposed and immunoaccessible protein, and that it is a useful immunogen. These properties are not evident from the sequence alone.

**Example 62**

The following *C.pneumoniae* protein (PID 4377101) was expressed <SEQ ID 123; cp7101>:

```
  1  MYSCYSKGIS HNYLLHPMSR LDIFVFDSLI ANQDQNLLEE IFCSEDTVLF
 51  KAYRTTALQS PLAAKNLNIA RKVANYILAD NGEIDTVKLV EAIHHLSQCT
101  YPLGPHRHNE AQDREHLLKM LKALKENPKL KESIKTLFVP SYSTIQNLIR
151  HTLALNPQTI LSTIHVRQAA LTALFTYLRQ DVGSCFATAP AILIHQEYPE
201  RFLKDLNDLI SSGKLSRIVN QREIAVPINL SGCIGELFKP LRILDLYPDP
251  LVKLSSSPGL KKAFSAANLI ETLGDSEAQI QQLLSHQYLM QKLQNVHETL
301  TANDIIKSTL LHYYQLQEST VRAIFFKEGL FSKEQVAFST QHPRELSEIQ
351  RVYHYLHAYE EAKSAFIHDT QNPLLKAWEY TLATLADASQ PTISNHIRLA
401  LGWKSEDPHS LVSLVTHFVE EEVENIRILV QQCEQTYHEA RSQLEYIEGR
451  MRNPLNNQDS QILTMDHMRF RQELNKALYE WDSAQEKAKK FLHLPEFLLS
501  FYTKQIPLYF RSSYDAFIQE FAHLYANAPA GFRILFTHGR THPNTWSPIY
551  SINEFIRFLS EFFTSTESEL LGKHAVINLE KETSRLVHNI TAMLHTDVFQ
601  EALLTRILEA YQLPVPPSIL NHLDQLSQTP WVYVSGGTVD TLLLDYFESS
651  EPLTLTEKHP ENPHELAAFY ADALKDLPTG IKSYLEEGSH SLLSSSPTHV
701  FSIIAGSPLF REAWDNDWYS YTWLRDVWVK QHQDFLQDTI LPQLSIYAFI
751  ENFCNKYALQ HVVHDFHDFC SDHSLTLPEL YDKGSRFLSS LFTKDKTVAL
801  IYIRRLLYLM VREVPYVSEQ QLPEVLDNVS SYLGISSRIT YEKFRSLIEE
851  TIPKMTLLSS ADLRHIYKGL LMQSYQKIYT EEDTYLRLTT AMRHHNLAYP
901  APLLFADSNW PSIYFGFILN PGTTEIDLWK FNYAGLQGQP LDNIQELFAT
951  SRPWTLYANP IDYGMPPPPG YRSRLPKEFF *
```

The cp7101 nucleotide sequence <SEQ ID 124> is:

```
   1  ATGTATTCGT GTTACAGCAA AGGAATATCC CATAACTATC TTCTACATCC
  51  TATGTCACGT TTGGATATTT TTGTTTTCGA TTCTCTGATC GCAAACCAGG
 101  ATCAAAATCT TCTTGAGGAA ATTTTCTGTT CTGAAGACAC AGTTTTATTT
 151  AAAGCCTACC GTACTACGGC TCTACAATCC CCTCTAGCTG CTAAGAACCT
 201  AAATATCGCC CGTAAAGTCG CAAATTATAT CTTAGCTGAC AATGGGGAAA
 251  TCGATACAGT AAAGCTTGTC GAAGCCATTC ACCATCTCTC ACAATGTACC
 301  TATCCTTTAG GGCCTCATCG CCATAATGAA GCTCAAGATC GTGAACACCT
 351  CCTTAAAATG CTAAAAGCTC TAAAGGAAAA TCCTAAATTA AAAGAAAGCA
 401  TCAAAACTCT CTTTGTCCCT TCATACTCTA CAATCCAAAA CCTAATTCGC
 451  CATACACTAG CATTGAATCC ACAGACAATT CTCTCTACGA TTCATGTGCG
 501  TCAAGCAGCA CTCACAGCGC TCTTCACCTA CCTTCGGCAA GATGTAGGTT
 551  CCTGTTTTGC TACGGCTCCT GCCATTCTCA TTCACCAAGA ATATCCAGAA
 601  CGATTCCTTA AAGATCTCAA TGATCTCATT AGCAGTGGCA AACTCTCTAG
 651  AATCGTAAAC CAAAGGGAAA TTGCGGTTCC TATAAACCTT TCGGGATGCA
 701  TTGGAGAGCT ATTCAAGCCT TTAAGGATTC TAGATCTTTA TCCTGATCCT
 751  CTGGTTAAGC TCTCCTCATC TCCAGGACTC AAAAAAGCCT TTTCTGCTGC
 801  CAATCTTATT GAAACTCTTG GGGATTCTGA AGCACAAATC CAACAGTTGC
 851  TCTCGCATCA ATATTTGATG CAAAAACTAC AAAATGTCCA TGAGACCTTA
 901  ACTGCTAACG ACATTATCAA ATCGACACTT CTGCACTACT ATCAGCTCCA
 951  AGAAAGTACT GTACGAGCTA TTTTCTTCAA AGAAGGGTTG TTCAGCAAAG
1001  AACAAGTGGC ATTCTCGACG CAACACCCCA GAGAGCTCTC AGAAATACAA
1051  CGGGTATACC ACTACTTACA TGCCTATGAA GAAGCAAAAT CTGCTTTTAT
1101  CCATGACACT CAAAATCCCT TACTGAAAGC CTGGGAGTAT ACTTTAGCGA
1151  CTCTTGCGGA TGCTAGCCAA CCTACCATCT CAAACCATAT CCGCCTTGCC
1201  TTAGGATGGA AAAGTGAAGA CCCTCACAGT CTTGTATCTC TAGTTACACA
1251  CTTTGTTGAA GAGGAAGTAG AAAACATCCG AATTTTAGTC CAACAATGTG
1301  AACAGACCTA TCACGAAGCA CGCTCCCAAC TAGAATATAT TGAAGGGCGG
1351  ATGCGCAACC CACTAAATAA TCAAGACAGT CAGATTTTGA CGATGGATCA
1401  CATGCGCTTC CGTCAAGAAC TCAATAAAGC TCTTTATGAG TGGGATAGTG
1451  CTCAAGAAAA GGCAAAGAAA TTTCTACATC TTCCTGAATT CTTACTTTCT
1501  TTCTATACAA AGCAAATTCC CTTATACTTT CGTAGTTCTT ACGATGCCTT
1551  CATTCAAGAA TTTGCTCATC TCTATGCTAA TGCTCCCGCT GGCTTCCGTA
1601  TTCTTTTCAC GCATGGACGC ACCCATCCGA ACACATGGTC CCCCATCTAT
1651  TCGATTAATG AATTTATACG TTTTCTTTCT GAATTCTTCA CCTCCACAGA
1701  GTCAGAACTT CTGGGGAAAC ATGCCGTGAT CAATTTAGAG AAAGAAACAT
1751  CTCGGCTCGT CCACAACATC ACTGCCATGC TACACACGGA TGTTTTCCAA
1801  GAAGCTCTCC TTACAAGAAT TTTAGAAGCC TATCAGCTTC CTGTGCCTCC
1851  CTCCATCTTA AACCACTTAG ATCAGCTGTC ACAAACTCCC TGGGTTTATG
1901  TTTCTGGAGG AACAGTGGAC ACTCTTCTTT TGGATTATTT TGAAAGCTCA
1951  GAACCTCTGA CACTTACAGA AAAGCATCCT GAAAATCCTC ATGAGCTTGC
2001  AGCTTTCTAC GCAGACGCCC TTAAAGATCT CCCTACAGGA ATTAAAAGTT

2051  ATCTAGAAGA AGGATCCCAC TCTCTACTTA GCTCATCACC CACCCACGTT
2101  TTCTCTATAA TCGCAGGATC TCCTTTATTT CGGGAAGCTT GGGATAATGA
2151  TTGGTACAGC TATACCTGGC TTCGTGATGT CTGGGTGAAA CAACACCAAG
2201  ATTTCCTTCA AGATACTATA TTACCTCAGC TAAGTATCTA TGCTTTCATA
2251  GAGAATTTTT GTAACAAATA TGCTTTGCAA CATGTAGTTC ATGACTTTCA
2301  TGATTTCTGC TCCGACCACT CCTTGACTCT TCCGGAGCTC TATGACAAAG
2351  GATCGCGTTT TCTAAGCTCC TTATTCACCA AAGATAAGAC CGTAGCTCTT
2401  ATCTATATAC GCCGTCTTCT CTACCTTATG GTCCGTGAAG TCCCTTATGT
2451  TTCAGAACAA CAGCTTCCAG AAGTCTTAGA TAACGTCTCT TCATATCTCG
2501  GGATTTCCTC TCGTATTACC TATGAGAAAT TCCGCTCCCT GATAGAGGAA
2551  ACCATCCCTA AAATGACCTT ACTCTCCTCA GCAGACCTGA GGCATATCTA
2601  TAAAGGTCTC CTCATGCAAA GTTATCAAAA GATCTACACC GAAGAAGATA
2651  CGTACCTCCG CCTCACCACG GCAATGAGGC ATCATAATCT TGCCTATCCC
2701  GCTCCTTTGC TCTTTGCAGA CAGTAACTGG CCTTCTATTT ATTTTGGATT
2751  CATCCTAAAT CCAGGAACCA CAGAGATCGA TCTTTGGAAA TTTAACTATG
2801  CAGGGCTGCA AGGACAGCCT CTTGACAATA TCCAGGAGCT GTTCGCAACG
2851  TCAAGACCCT GGACCCTCTA TGCAAATCCT ATAGATTATG GCATGCCACC
2901  GCCTCCAGGC TACCGCAGCC GCCTCCCTAA AGAATTTTTC TAG
```

The PSORT algorithm predicts a cytoplasmic location (0.206).

The protein was expressed in *E.coli* and purified as a GST-fusion (Figure 62A) or his-tagged product. The proteins were used to immunise mice, whose sera were used in Western blot (Figure 62B) and FACS (Figure 62C) analyses.

This protein also showed good cross-reactivity with human sera, including sera from patients with pneumonitis.

These experiments show that cp7101 is a surface-exposed and immunoaccessible protein, and that it is a useful immunogen. These properties are not evident from the sequence alone.

**Example 63**

The following *C.pneumoniae* protein (PID 4377107) was expressed <SEQ ID 125; cp7107>:

```
  1  MSIVRNSALP LPCLSRSETF KKVRSHMKFM KVLTPWIYRK DLWVTAFLLT
 51  AIPGSFAHTL VDIAGEPRHA AQATGVSGDG KIVIGMKVPD DPFAITVGFQ
101  YIDGHLQPLE AVRPQCSVYP NGITPDGTVI VGTNYAIGMG SVAVKWVNGK
151  VSELPMLPDT LDSVASAVSA DGRVIGGNRN INLGASVAVK WEDDVITQLP
201  SLPDAMNACV NGISSDGSII VGTMVDVSWR NTAVQWIGDQ LSVIGTLGGT
251  TSVASAISTD GTVIVGGSEN ADSQTHAYAY KNGVMSDIGT LGGFYSLAHA
301  VSSDGSVIVG VSTNSEHRYH AFQYADGQMV DLGTLGGPES YAQGVSGDGK
351  VIVGRAQVPS GDWHAFLCPF QAPSPAPVHG GSTVVTSQNP RGMVDINATY
401  SSLKNSQQQL QRLLIQHSAK VESVSSGAPS FTSVKGAISK QSPAVQNDVQ
451  KGTFLSYRSQ VHGNVQNQQL LTGAFMDWKL ASAPKCGFKV ALHYGSQDAL
501  VERAALPYTE QGLGSSVLSG FGGQVQGRYD FNLGETVVLQ PFMGIQVLHL
551  SREGYSEKNV RFPVSYDSVA YSAATSFMGA HVFASLSPKM STAATLGVER
601  DLNSHIDEFK GSVSAMGNFV LENSTVSVLR PFASLAMYYD VRQQQLVTLS
651  VVMNQQPLTG TLSLVSQSSY NLSF*
```

The cp7107 nucleotide sequence <SEQ ID 126> is:

```
  1  ATGAGTATAG TCAGAAATTC TGCATTGCCA CTTCCGTGTT TAAGCAGATC
 51  CGAAACCTTT AAAAAAGTTA GGTCGCATAT GAAATTTATG AAAGTCCTTA
101  CTCCATGGAT TTATCGAAAA GATCTTTGGG TAACAGCATT CTTACTGACA
151  GCAATTCCAG GATCTTTTGC ACATACTCTT GTTGATATAG CAGGAGAACC
201  TCGGCATGCT GCTCAAGCAA CAGGAGTTTC TGGAGATGGT AAAATTGTTA
251  TAGGAATGAA AGTTCCGGAT GATCCTTTTG CTATAACTGT AGGATTTCAA
301  TATATTGATG GGCATTTGCA ACCCTTAGAG GCAGTACGTC CTCAATGCTC
351  TGTATACCCT AATGGTATAA CCCCGGACGG AACGGTTATT GTGGGTACAA
401  ACTATGCCAT CGGGATGGGT AGTGTTGCTG TGAAATGGGT AAATGGCAAG
451  GTTTCTGAAC TTCCCATGCT CCCTGACACC CTCGATTCTG TAGCATCGGC
501  AGTTTCTGCA GATGGAAGAG TGATTGGAGG GAATAGAAAT ATAAATCTTG
551  GCGCTTCTGT TGCTGTGAAA TGGGAGGACG ACGTGATTAC ACAACTTCCT
601  TCTCTTCCTG ATGCTATGAA TGCTTGTGTT AACGGAATTT CTTCAGATGG
```

```
 651  TTCTATAATT GTAGGAACCA TGGTAGACGT GTCATGGAGA AATACCGCAG
 701  TACAATGGAT CGGGGATCAG CTCTCTGTTA TTGGGACTTT AGGAGGAACT
 751  ACTTCTGTTG CTAGTGCAAT CTCAACAGAT GGCACTGTGA TTGTAGGAGG
 801  TTCTGAAAAT GCAGATTCTC AGACTCATGC CTATGCTTAT AAAAACGGTG
 851  TTATGAGCGA TATAGGGACC CTCGGAGGTT TTTATTCTTT AGCACATGCA
 901  GTATCTTCAG ATGGTTCTGT GATTGTAGGA GTATCCACGA ACTCTGAGCA
 951  TAGATATCAT GCATTCCAAT ATGCTGATGG ACAGATGGTA GATTTAGGAA
1001  CTTTAGGAGG GCCTGAATCT TATGCTCAAG GTGTGTCTGG AGATGGAAAG
1051  GTAATTGTGG GTAGAGCACA AGTACCATCT GGAGATTGGC ATGCGTTCCT
1101  ATGTCCTTTC CAAGCTCCGA GCCCTGCTCC TGTCCATGGG GGAAGCACTG
1151  TCGTAACTAG CCAGAATCCA CGTGGAATGG TAGATATCAA TGCTACGTAC
1201  TCCTCTTTGA AAAATAGCCA ACAACAACTA CAAAGATTGC TTATCCAGCA
1251  TAGTGCAAAA GTTGAAAGTG TATCCTCAGG AGCACCATCT TTTACAAGTG
1301  TGAAAGGTGC GATCTCAAAA CAGAGCCCTG CAGTGCAAAA TGATGTACAG
1351  AAAGGGACGT TTTTAAGTTA CCGTTCCCAA GTTCATGGAA ACGTGCAGAA
1401  TCAGCAATTG CTCACAGGAG CTTTTATGGA CTGGAAACTC GCTTCAGCTC
1451  CTAAATGCGG CTTTAAAGTA GCTCTCCACT ATGGCTCTCA AGATGCTCTC
1501  GTAGAACGTG CAGCTCTTCC TTACACAGAA CAAGGCTTAG GAAGCAGTGT
1551  CTTGTCAGGT TTTGGAGGAC AAGTTCAAGG ACGCTATGAC TTTAATTTAG
1601  GAGAAACTGT TGTTCTGCAA CCCTTTATGG GCATTCAAGT TCTCCACCTA
1651  AGTAGAGAAG GGTATTCTGA GAAGAATGTT CGATTTCCTG TAAGCTATGA
1701  TTCTGTAGCC TACTCAGCAG CTACTAGCTT TATGGGTGCG CATGTATTTG
1751  CCTCCCTAAG CCCTAAAATG AGTACAGCAG CAACTTTAGG TGTGGAGAGA
1801  GATCTGAATT CACATATAGA TGAATTTAAG GGATCCGTCT CTGCTATGGG
1851  AAACTTTGTC TTGGAAAATT CTACAGTGAG TGTTTTAAGA CCTTTTGCTT
1901  CTCTTGCTAT GTACTATGAC GTAAGACAAC AGCAACTCGT GACGTTGTCA
1951  GTAGTTATGA ATCAACAACC CTTAACAGGC ACACTAAGCT TAGTAAGCCA
2001  AAGTAGCTAT AATCTTAGCT TCTAA
```

The PSORT algorithm predicts an inner membrane location (0.100).

The protein was expressed in *E.coli* and purified as a GST-fusion (Figure 63A) or his-tagged product. The proteins were used to immunise mice, whose sera were used in Western blot (Figure 63B) and FACS (Figure 63C) analyses.

These experiments show that cp7107 is a surface-exposed and immunoaccessible protein, and that it is a useful immunogen. These properties are not evident from the sequence alone.

## Example 64

The following *C.pneumoniae* protein (PID 4376467) was expressed <SEQ ID 127; cp6467>:

```
  1  MLRFFAVFIS TLWLITSGCS PSQSSKGIFV VNMKEMPRSL DPGKTRLIAD
 51  QTLMRHLYEG LVEEHSQNGE IKPALAESYT ISEDGTRYTF KIKNILWSNG
101  DPLTAQDFVS SWKEILKEDA SSVYLYAFLP IKNARAIFDD TESPENLGVR
151  ALDKRHLEIQ LETPCAHFLH FLTLPIFFPV HETLRNYSTS FEEMPITCGA
201  FRPVSLEKGL RLHLEKNPMY HNKSRVKLHK IIVQFISNAN TAAILFKHKK
251  LDWQGPPWGE PIPPEISASL HQDDQLFSLP GASTTWLLFN IQKKPWNNAK
301  LRKALSLAID KDMLTKVVYQ GLAEPTDHIL HPRLYPGTYP ERKRQNERIL
351  EAQQLFEEAL DELQMTREDL EKETLTFSTF SFSYGRICQM LREQWKKVLK
401  FTIPIVGQEF FTIQKNFLEG NYSLTVNQWT AAFIDPMSYL MIFANPGGIS
451  PYHLQDSHFQ TLLIKITQEH KKHLRNQLII EALDYLEHCH ILEPLCHPNL
501  RIALNKNIKN FNLFVRRTSD FRFIEKL*
```

A predicted signal peptide is highlighted.

The cp6467 nucleotide sequence <SEQ ID 128> is:

```
   1  ATGCTCCGTT TCTTCGCTGT ATTTATATCA ACTCTTTGGC TCATTACCTC
  51  AGGATGTTCC CCATCCCAAT CCTCTAAAGG AATTTTTGTG GTAAATATGA
 101  AGGAAATGCC ACGCTCCTTG GATCCTGGAA AAACTCGTCT CATTGCAGAC
 151  CAAACTCTAA TGCGTCATCT ATATGAAGGA CTCGTCGAAG AACATTCCCA
 201  AAATGGAGAG ATTAAACCAG CCCTTGCAGA AAGCTACACC ATCTCCGAAG
 251  ACGGGACTCG GTACACATTT AAAATCAAAA ACATCCTTTG GAGTAACGGA
 301  GACCCTCTGA CAGCTCAAGA CTTTGTCTCC TCTTGGAAGG AAATCCTAAA
```

```
 351  GGAAGATGCG TCCTCCGTAT ATCTCTATGC GTTTTTACCT ATCAAAAATG
 401  CTCGGGCAAT CTTTGATGAT ACTGAGTCTC CAGAAAATCT AGGAGTCCGA
 451  GCTTTAGATA AGCGTCATCT CGAAATTCAG TTAGAAACTC CCTGCGCGCA
 501  TTTCCTACAT TTCTTGACTC TTCCTATTTT TTTCCCTGTT CATGAAACTC
 551  TGCGAAACTA TAGCACCTCT TTTGAAGAGA TGCCCATTAC CTGCGGTGCT
 601  TTCCGCCCTG TGTCTCTAGA AAAAGGCCTG AGACTCCATC TAGAGAAAAA
 651  CCCTATGTAC CATAATAAAA GCCGTGTGAA ACTACATAAA ATTATTGTAC
 701  AGTTTATCTC AAACGCTAAC ACTGCAGCCA TTCTATTCAA ACATAAGAAA
 751  TTAGATTGGC AAGGACCTCC TTGGGGAGAA CCTATCCCTC CAGAAATCTC
 801  AGCTTCTCTA CATCAAGATG ACCAGCTCTT TTCTCTTCCG GGCGCTTCGA
 851  CTACATGGTT ACTCTTTAAT ATACAAAAAA AACCTTGGAA CAATGCTAAA
 901  TTACGCAAGG CATTGAGCCT TGCAATAGAC AAAGATATGT TAACCAAAGT
 951  GGTATACCAA GGTCTTGCAG AACCTACAGA TCATATCCTA CATCCAAGAC
1001  TTTATCCAGG GACCTATCCC GAACGGAAAA GACAAAACGA AAGAATTCTT
1051  GAGGCTCAAC AACTCTTTGA AGAAGCTCTA GACGAACTTC AAATGACACG
1101  CGAAGATCTA GAAAAGGAAA CTTTGACTTT CTCAACCTTT TCTTTTTCTT
1151  ACGGAAGGAT TTGCCAAATG CTAAGAGAAC AATGGAAGAA AGTCTTAAAA
1201  TTTACTATCC CTATAGTAGG CCAAGAGTTT TTCACAATAC AAAAAAACTT
1251  CCTAGAGGGG AACTATTCCC TAACCGTGAA CCAATGGACC GCAGCATTTA
1301  TTGATCCGAT GTCTTATCTC ATGATCTTTG CCAATCCTGG AGGAATTTCC
1351  CCCTATCACC TCCAAGATTC ACACTTTCAA ACTCTTCTCA TAAAGATCAC
1401  TCAAGAACAT AAAAAACACC TACGAAATCA GCTTATTATT GAAGCCCTTG
1451  ACTATTTAGA ACACTGTCAC ATTCTCGAAC CACTATGTCA TCCAAATCTT
1501  CGAATTGCTT TGAACAAAAA CATTAAAAAC TTTAATCTTT TTGTTCGACG
1551  AACTTCAGAC TTTCGTTTTA TAGAAAAACT ATAG
```

The PSORT algorithm predicts an outer membrane lipoprotein (0.790).

The protein was expressed in *E.coli* and purified as a his-tag product and a GST-fusion protein, as shown in Figure 64A. The recombinant his-tag protein was used to immunise mice, whose sera were used in a Western blot (Figure 64B). The recombinant GST-fusion protein was also used to immunise mice, whose sera were used in a Western blot (Figure 64C) and for FACS analysis (Figure 64D).

These experiments show that cp6467 is a surface-exposed and immunoaccessible protein, and that it is a useful immunogen. These properties are not evident from the sequence alone.

**Example 65**

The following *C.pneumoniae* protein (PID 4376679) was expressed <SEQ ID 129; cp6679>:

```
   1  MRKMLVLLAS LGLLSPTLSS CTHLGSSGSY HPKLYTSGSK TKGVIAMLPV
  51  FHRPGKSLEP LPWNLQGEFT EEISKRFYAS EKVFLIKHNA SPQTVSQFYA
 101  PIANRLPETI IEQFLPAEFI VATELLEQKT GKEAGVDSVT ASVRVRVFDI
 151  RHHKIALIYQ EIIECSQPLT TLVNDYHRYG WNSKHFDSTP MGLMHSRLFR
 201  EVVARVEGYV CANYS*
```

A predicted signal peptide is highlighted.

The cp6679 nucleotide sequence <SEQ ID 130> is:

```
  1  ATGCGAAAAA TGTTGGTATT ATTGGCATCT TTAGGACTTC TATCCCCAAC
 51  CCTATCCAGC TGCACTCACT TAGGCTCTTC AGGAAGTTAT CATCCTAAGC
101  TATACACTTC AGGGAGCAAA ACTAAAGGTG TGATTGCGAT GCTTCCTGTA
151  TTTCATCGCC CAGGAAAGAG TCTTGAACCT TTACCTTGGA ACCTCCAAGG
201  AGAATTTACT GAAGAGATCA GCAAAAGGTT TTATGCTTCG GAAAAGGTCT
251  TCCTGATCAA GCACAATGCT TCACCTCAGA CAGTCTCTCA GTTCTATGCT
301  CCGATTGCGA ATCGTCTACC CGAAACAATT ATTGAGCAAT TTCTTCCTGC
351  AGAATTCATT GTTGCTACAG AACTGTTAGA ACAAAAGACA GGGAAAGAAG
401  CAGGTGTCGA TTCTGTAACA GCGTCTGTAC GTGTTCGCGT TTTGATATC
451  CGTCATCATA AAATAGCTCT CATTTATCAA GAGATTATCG AATGCAGCCA
501  GCCTTTAACT ACCCTAGTCA ATGATTATCA TCGCTATGGC TGGAACTCAA
551  AACATTTTGA TTCAACGCCC ATGGCTTAA TGCATAGCCG TCTTTTCCGC

601  GAAGTTGTTG CCAGAGTTGA GGGCTATGTT TGTGCTAACT ACTCGTAG
```

The PSORT algorithm predicts an inner membrane location (0.149).

The protein was expressed in *E.coli* and purified as a his-tag product (Figure 65A) and as a GST-fusion product (Figure 65B). The recombinant protein was used to immunise mice, whose sera were used in a Western blot (Figure 65C) and for FACS analysis.

These experiments show that cp6679 is a surface-exposed and immunoaccessible protein, and that it is a useful immunogen. These properties are not evident from the sequence alone.

**Example 66**

The following *C.pneumoniae* protein (PID 4376890) was expressed <SEQ ID 131; cp6890>:

```
  1  MKQLLFCVCV FAMSCSAYAS PRRQDPSVMK ETFRNNYGII VSGQEWVKRG
 51  SDGTITKVLK NGATLHEVYS GGLLHGEITL TFPHTTALDV VQIYDQGRLV
101  SRKTFFVNGL PSQEELFNED GTFVLTRWPD NNDSDTITKP YFIETTYQGH
151  VIEGSYTSFN GKYSSSIHNG EGVRSVFSSN NILLSEETFN EGVMVKYTTF
201  YPNRDPESIT HYQNGQPHGL RLTYLQGGIP NTIEEWRYGF QDGTTIVFKN
251  GCKTSEIAYV KGVKEGLELR YNEQEIVAEE VSWRNDFLHG ERKIYAGGIQ
301  KHEWYYRGRS VSKAKFERLN AAG*
```

A predicted signal peptide is highlighted.

The cp6890 nucleotide sequence <SEQ ID 132> is:

```
  1  ATGAAACAAT TACTTTTCTG TGTTTGCGTA TTTGCTATGT CATGTTCTGC
 51  TTACGCATCC CCACGACGAC AAGATCCTTC TGTTATGAAG GAAACATTCC
101  GAAATAATTA TGGCATTATT GTTTCCGGTC AAGAATGGGT AAAGCGTGGT
151  TCTGACGGCA CCATCACCAA AGTACTCAAA AATGGAGCTA CCCTGCATGA
201  AGTTTATTCT GGAGGCCTCC TTCATGGGGA AATTACCTTA ACGTTTCCCC
251  ATACCACAGC ATTGGACGTT GTTCAAATCT ATGATCAAGG TAGACTCGTT
301  TCTCGCAAAA CCTTTTTTGT GAACGGTCTT CCATCTCAAG AAGAGCTGTT
351  CAATGAAGAT GGCACGTTTG TCCTCACACG ATGGCCGGAC AACAACGACA
401  GTGATACCAT CACAAAGCCT TACTTCATAG AAACGACATA TCAAGGGCAT
451  GTCATAGAAG GAAGTTATAC TTCCTTTAAT GGGAAATACT CCTCATCCAT
501  CCACAATGGA GAGGGAGTTC GTTCTGTGTT CTCCTCCAAT AACATCCTTC
551  TTTCTGAAGA GACCTTCAAT GAAGGTGTCA TGGTGAAATA TACCACATTC
601  TATCCGAATC GCGATCCCGA ATCGATTACT CATTATCAAA ATGGACAGCC
651  TCACGGCTTA CGGCTAACAT ATCTACAAGG TGGCATCCCC AATACGATAG
701  AGGAGTGGCG TTATGGCTTT CAAGACGGAA CGACCATCGT ATTTAAAAAT
751  GGTTGTAAGA CATCTGAGAT CGCTTATGTT AAGGGAGTGA AAGAAGGTTT
801  AGAACTGCGC TACAATGAAC AGGAAATTGT AGCTGAAGAA GTTTCTTGGC
851  GTAATGATTT TCTGCATGGA GAACGTAAGA TCTATGCTGG AGGAATCCAA
901  AAGCATGAAT GGTATTACCG CGGGAGATCT GTATCTAAAG CCAAATTCGA
951  GCGGCTAAAT GCTGCAGGAT AG
```

The PSORT algorithm predicts an outer membrane location (0.940).

The protein was expressed in *E.coli* and purified as a GST-fusion product, as shown in Figure 66A. The recombinant protein was used to immunise mice, whose sera were used in a Western blot (Figure 66B) and for FACS analysis. A his-tagged protein was also expressed.

These experiments show that cp6890 is a surface-exposed and immunoaccessible protein, and that it is a useful immunogen. These properties are not evident from the sequence alone.

**Example 67**

The following *C.pneumoniae* protein (PID 6172323) was expressed <SEQ ID 133; cp0018>:

```
  1 MKTSVSMLLA LLCSGASSIV LHAATTPLNP EDGFIGEGNT NTFSPKSTTD
 51 AAGTTYSLTG EVLYIDPGKG GSITGTCFVE TAGDLTFLGN GNTLKFLSVD
101 AGANIAVAHV QGSKNLSFTD FLSLVITESP KSAVTTGKGS LVSLGAVQLQ
151 DINTLVLTSN ASVEDGGVIK GNSCLIQGIK NSAIFGQNTS SKKGGAISTT
201 QGLTIENNLG TLKFNENKAV TSGGALDLGA ASTFTANHEL IFSQNKTSGN
251 AANGGAINCS GDLTFTDNTS LLLQENSTMQ DGGALCSTGT ISITGSDSIN
301 VIGNTSGQKG GAISAASLKI LGGQGGALFS NNVVTHATPL GGAIFINTGG
351 SLQLFTQGGD IVFEGNQVTT TAPNATTKRN VIHLESTAKW TGLAASQGNA
401 IYFYDPITTN DTGASDNLRI NEVSANQKLS GSIVFSGERL STAEAIAENL
451 TSRINQPVTL VEGSLVLKQG VTLITQGFSQ EPESTLLLDL GTSL*
```

A predicted signal peptide is highlighted.

The cp0018 nucleotide sequence <SEQ ID 134> is:

```
   1 ATGAAGACTT CAGTTTCTAT GTTGTTGGCC CTGCTTTGCT CGGGGGCTAG
  51 CTCTATTGTA CTCCATGCCG CAACCACTCC ACTAAATCCT GAAGATGGGT
 101 TTATTGGGGA GGGCAATACA AATACTTTTT CTCCGAAATC TACAACGGAT
 151 GCTGCAGGAA CTACCTACTC TCTCACAGGA GAGGTTCTGT ATATAGATCC
 201 GGGGAAAGGT GGTTCAATTA CAGGAACTTG CTTTGTAGAA ACTGCTGGCG
 251 ATCTTACATT TTTAGGTAAT GGAAATACCC TAAAGTTCCT GTCGGTAGAT
 301 GCAGGTGCTA ATATCGCGGT TGCTCATGTA CAAGGAAGTA AGAATTTAAG
 351 CTTCACAGAT TTCCTTTCTC TGGTGATCAC AGAATCTCCA AAATCCGCTG
 401 TTACTACAGG AAAAGGTAGC CTAGTCAGTT TAGGTGCAGT CCAACTGCAA
 451 GATATAAACA CTCTAGTTCT TACAAGCAAT GCCTCTGTCG AAGATGGTGG
 501 CGTGATTAAA GGAAACTCCT GCTTGATTCA GGGAATCAAA AATAGTGCGA
 551 TTTTTGGACA AAATACATCT TCGAAAAAAG GAGGGGCGAT CTCCACGACT
 601 CAAGGACTTA CCATAGAGAA TAACTTAGGG ACGCTAAAGT TCAATGAAAA
 651 CAAAGCAGTG ACCTCAGGAG GCGCCTTAGA TTTAGGAGCC GCGTCTACAT
 701 TCACTGCGAA CCATGAGTTG ATATTTTCAC AAAATAAGAC TTCTGGGAAT
 751 GCTGCAAATG GCGGAGCCAT AAATTGCTCA GGGGACCTTA CATTTACTGA
 801 TAACACTTCT TTGTTACTTC AAGAAAATAG CACAATGCAG GATGGTGGAG
 851 CTTTGTGTAG CACAGGAACC ATAAGCATTA CCGGTAGTGA TTCTATCAAT
 901 GTGATAGGAA ATACTTCAGG ACAAAAAGGA GGAGCGATTT CTGCAGCTTC
 951 TCTCAAGATT TTGGGAGGGC AGGGAGGCGC TCTCTTTTCT AATAACGTAG
1001 TGACTCATGC CACCCCTCTA GGAGGTGCCA TTTTTATCAA CACAGGAGGA
1051 TCCTTGCAGC TCTTCACTCA AGGAGGGGAT ATCGTATTCG AGGGGAATCA
1101 GGTCACTACA ACAGCTCCAA ATGCTACCAC TAAGAGAAAT GTAATTCACC
1151 TCGAGAGCAC CGCGAAGTGG ACGGGACTTG CTGCAAGTCA AGGTAACGCT
1201 ATCTATTTCT ATGATCCCAT TACCACCAAC GATACGGGAG CAAGCGATAA
1251 CTTACGTATC AATGAGGTCA GTGCAAATCA AAAGCTCTCG GGATCTATAG
1301 TATTTTCTGG AGAGAGATTG TCGACAGCAG AAGCTATAGC TGAAAATCTT
1351 ACTTCGAGGA TCAACCAGCC TGTCACTTTA GTAGAGGGGA GCTTAGTACT
1401 TAAACAGGGA GTGACCTTGA TCACACAAGG ATTCTCGCAG GAGCCAGAAT
1451 CCACGCTTCT TTTGGATCTG GGGACCTCAT TATAA
```

The PSORT algorithm predicts outer membrane (0.935).

The protein was expressed in *E.coli* and purified as a GST-fusion product (Figure 67A). The recombinant protein was used to immunise mice, whose sera were used in a Western blot (Figure 67B) and for FACS analysis.

These experiments show that cp0018 is a surface-exposed and immunoaccessible protein, and that it is a useful immunogen. These properties are not evident from the sequence alone.

**Example 68**

The following *C.pneumoniae* protein (PID 4376262) was expressed <SEQ ID 135; cp6262>:

```
  1 MRKLRILAIV LIALSIILIA GGVVLLTVAI PGLSSVISSP AGMGACALGC
 51 VMLALGIDVL LKKREVPIVL ASVTTTPGTG SPRSGISISG ADSTIRSLPT
101 YLLDEGHPQS MRKLRILAIV LIVFSIILIA SGVVLLTVAI PGLSSVISSP
151 AGMGACALGC VMLALGIDVL LKKREVPIVL ASVTTTPGTG SPRSGISISG
201 ADSTIRSLPT YPLDEGHPQS MRKLRILAIV LIVFSIILIA SGVVLLTVAI
251 PGLSSIISSP AEMGACALGC VMLALGIDVL LKKREVPIVV PAPIPEEVVI

301 DDIDEESIRL QQEAEAALAR LPEEMSAFEG YIKVVESHLE NMKSLPYDGH
351 GLEEKTKHQI RVVRSSLKAM VPEFLDIRRI FEEEEFFFLS ARKRLIDLAT
401 TLVERKILTE QLERNNLRKA FSYLYQDSIF KKIIDNFEKL AWKFMILSKS
451 ICRFTIIFEN HEHGVAKSLL HKNAVLLEKV IYRSLQKSYR DIGMSSAKMK
501 ILHGNPFFSL EDNKKTIMKE HAEMLESLSS YRKVFLALSD ENVVDTPSDP
551 KKWDLSGIPC RDALSEISRD EQWQKKAHLK HQESLYTQAR DRLTDQSSKE
601 NQKELEKAEQ EYISSWERVK KFEIERVQER IRAIQKLYPN ILEREEETTG
651 QETVTPTVQG TTASSDLTDI LGRIEVSSRE DNQNQESCVK VLRSHEVEMS
701 WEVKQEYGPK KKEFQDQMGS LERFFTEHIE ELEVLQKDYS KHLSYFKKVN
751 NKKEVQYAKF RLKVLESDLE GILAQTESAE SLLTQEELPI LATRGALEKA
801 VFKGSLCCAL ASKAKPYFEE DPRFQDSDTQ LRALTLRLQE AKASLEEEIK
851 RFSNLENDIA EERRLLKESK QTFERAGLGV LREIAVESTY DLRSLTNTWE
901 GTPESEKVYF SMYLNYYNEE KRRAKTRLVE MTQRYRDFKM ALEAMQFNEE
951 ALLQEELSIQ APSE*
```

A predicted signal peptide is highlighted.

The cp6262 nucleotide sequence <SEQ ID 136> is:

```
   1  ATGAGGAAAC TTCGTATTCT TGCGATCGTT CTCATAGCTT TGAGCATTAT
  51  TTTGATTGCA GGTGGTGTGG TATTGCTTAC TGTAGCGATC CCTGGATTAA
 101  GTTCAGTCAT TTCTTCCCCG GCAGGGATGG GTGCCTGTGC TTTGGGATGT
 151  GTGATGCTTG CTTTAGGGAT CGATGTTCTT CTGAAGAAAC GAGAAGTCCC
 201  TATAGTTCTC GCATCTGTAA CTACGACACC AGGAACTGGC AGCCCTAGAA
 251  GTGGTATTTC TATTTCAGGA GCTGATAGCA CCATACGTTC TCTTCCTACG
 301  TATCTCTTGG ACGAGGGACA TCCACAATCC ATGAGGAAAC TTCGTATTCT
 351  TGCGATCGTT CTCATAGTTT TTAGCATTAT TTTGATTGCA AGTGGTGTGG
 401  TATTGCTTAC TGTAGCGATC CCTGGATTAA GTTCAGTCAT TTCTTCCCCG
 451  GCAGGGATGG GTGCCTGTGC TTTGGGATGT GTGATGCTTG CTTTAGGGAT
 501  CGATGTTCTT CTGAAGAAAC GAGAAGTCCC TATAGTTCTC GCATCTGTAA
 551  CTACGACACC AGGAACTGGC AGCCCTAGAA GTGGTATTTC TATTTCAGGA
 601  GCTGATAGCA CCATACGTTC TCTTCCTACG TATCCCTTGG ACGAGGGACA
 651  TCCACAATCC ATGAGGAAAC TTCGTATTCT TGCGATCGTT CTCATAGTTT
 701  TTAGCATTAT TTTGATTGCA AGTGGTGTGG TATTGCTTAC TGTAGCGATC
 751  CCTGGATTAA GCTCGATCAT TTCTTCCCCA GCGGAGATGG GTGCTTGTGC
 801  TTTGGGATGT GTGATGCTTG CTTTGGGGAT CGACGTTCTT CTGAAGAAAC
 851  GAGAAGTCCC TATAGTAGTT CCCGCACCTA TTCCTGAAGA AGTCGTCATA
 901  GATGATATAG ATGAAGAGAG TATACGGCTG CAGCAGGAAG CTGAAGCCGC
 951  TTTAGCAAGA CTTCCTGAGG AGATGAGTGC ATTTGAAGGT TACATAAAAG
1001  TTGTCGAGAG TCATTTGGAG AACATGAAAA GCCTGCCTTA TGATGGTCAT
1051  GGGCTAGAAG AGAAAACGAA ACATCAGATA AGAGTCGTCA GATCTTCTTT
1101  GAAGGCTATG GTTCCAGAAT TTTTAGATAT CAGAAGAATT TTTGAAGAAG
1151  AAGAGTTCTT TTTTCTCTCA GCTCGCAAAC GACTTATAGA TTTAGCTACT
1201  ACTTTAGTAG AGAGAAAAAT TTTAACAGAG CAACTTGAGC GCAATAATTT
1251  AAGGAAAGCG TTTTCTTATT TATATCAGGA CTCAATTTTT AAAAAAATTA
1301  TTGATAACTT CGAGAAGTTA GCATGGAAAT TTATGATTTT GAGTAAATCA
1351  ATTTGTCGAT TTACAATTAT TTTTGAAAAT CATGAACATG GTGTAGCAAA
1401  GAGCCTGTTA CACAAGAATG CAGTGTTACT GGAGAAGGTA ATCTATAGGA
1451  GTTTGCAAAA AAGCTATAGA GATATAGGCA TGTCATCTGC AAAGATGAAA
1501  ATCTTGCACG GCAACCCTTT TTTCTCTTTG GAAGATAATA AAAAGACGAT
1551  AATGAAAGAA CACGCAGAGA TGCTTGAAAG TCTCAGTAGC TATAGGAAGG
1601  TATTTTTAGC TCTATCTGAT GAGAACGTTG TAGATACACC TAGCGATCCA
1651  AAGAAATGGG ATTTGTCAGG AATCCCCTGT AGGGACGCGT TGTCTGAGAT
1701  TTCTCGTGAT GAACAGTGGC AGAAGAAAGC ACATCTAAAG CATCAAGAGT
1751  CCCTCTATAC GCAAGCTAGG GATCGTTTAA CAGACCAGAG CTCTAAAGAA
1801  AATCAGAAAG AGTTAGAGAA AGCTGAACAA GAGTACATAT CTTCTTGGGA
1851  ACGGGTTAAA AAATTTGAGA TTGAGAGAGT ACAGGAGAGG ATACGGGCAA
1901  TTCAAAAGCT TTATCCTAAT ATCCTCGAGA GAGAAGAAGA AACCACAGGT
1951  CAGGAGACTG TGACTCCAAC TGTTCAAGGG ACGACGGCTT CATCCGATTT
2001  AACAGATATT TTAGGAAGAA TAGAGGTCTC CAGTAGGGAG GATAATCAGA
2051  ATCAAGAGTC TTGTGTAAAA GTCTTAAGAA GTCATGAGGT AGAAATGAGC
2101  TGGGAAGTCA AACAAGAGTA TGGCCCTAAG AAAAAAGAAT TTCAGGATCA
2151  AATGGGTTCT TTAGAGAGGT TTTTTACAGA GCATATTGAA GAGTTAGAAG
2201  TATTACAGAA GGACTACTCT AAACACTTGT CTTATTTTAA AAAAGTAAAC
2251  AATAAGAAAG AGGTTCAATA TGCGAAGTTT AGGTTGAAGG TTTTAGAGTC
2301  AGATTTAGAA GGGATTCTAG CTCAGACTGA GAGTGCTGAG AGTCTGTTAA
2351  CTCAAGAAGA ACTTCCGATT CTTGCAACTC GGGGAGCCTT AGAGAAAGCT
2401  GTTTTCAAAG GGAGTCTATG TTGCGCGCTA GCAAGCAAAG CAAAACCCTA

2451  TTTTGAAGAG GATCCCAGAT TCCAAGATTC TGATACGCAA TTGCGAGCTC
2501  TGACTCTAAG GTTACAGGAG GCTAAGGCAA GCCTGGAAGA AGAGATAAAG
2551  AGATTTTCAA ATCTTGAGAA CGATATTGCA GAGGAAAGAC GCCTTCTTAA
2601  AGAGAGCAAG CAGACGTTCG AAAGAGCAGG TTTAGGGGTT CTCCGAGAAA
2651  TTGCAGTCGA GTCTACTTAT GATTTGCGTT CCTTAACAAA TACATGGGAA
2701  GGGACCCCAG AGAGTGAGAA GGTCTATTTT AGCATGTATC TTAATTATTA
2751  CAACGAAGAG AAACGTAGGG CTAAAACAAG ATTGGTTGAA ATGACACAGA
2801  GGTATAGAGA TTTTAAAATG GCCTTGGAAG CTATGCAGTT TAATGAAGAA
2851  GCCCTTTTGC AAGAGGAACT CTCTATTCAA GCTCCCAGTG AATAA
```

The PSORT algorithm predicts inner membrane (0.660).

The protein was expressed in *E.coli* and purified as a GST-fusion product (Figure 68A). The recombinant protein was used to immunise mice, whose sera were used in a Western blot (Figure 68B) and for FACS analysis.

These experiments show that cp6262 is a surface-exposed and immunoaccessible protein, and that it is a useful immunogen. These properties are not evident from the sequence alone.

**Example 69**

The following *C.pneumoniae* protein (PID 4376269) was expressed <SEQ ID 137; cp6269>:

```
  1  MYQENLRLLE RLLYNSVQKS YADRLFSYEK TKMVHDTPLI PWEEDKEKCA
 51  EAEKAFLEQQ KILLDYGKSI FWLNENDEIN LNDPWSWGLN TVRTRKVFQE
101  VDDSERWNHK VLIQKLEDDY EKLLEESSKE STEANKKLLS DLVDRLEDAK
151  TKFFLKKQEE VETRVKDLRA RYGGTVDPKQ DTEAKKKVEL EASLETFLDS
201  IESELVQCLE DQDIYWKEQD VKDLARTQEL EEQDIEAKRE EAAEDLRSLN
251  ERLKKSKTML DRAKWHIENA EDSITWWTSQ IEMKDMKARL KILKEDITSV
301  LPEIDEIETC LSLEELPLLT TRELLTKSYL KFKICSETLL KMTSVFENNI
351  YVQEYEVQLQ NLGFKLQGIS QRFGKKQDDF ANLEEQVALQ KKRLRELTQN
401  FEIQGFNFMK EDFKAAAKDL YIRSTAEQKM NFDVPCMELF RRYHEEVNKP
451  LLELMYNCAD SYRDAKKKLC SLRLDEKELL QKEIKKEEFY QKKQQRHADR
501  SRHTTYQKLR IAEELALELK KKI*
```

The cp6269 nucleotide sequence <SEQ ID 138> is:

```
   1  ATGTACCAGG AGAATCTAAG ATTGTTGGAA AGGCTTCTTT ATAATAGTGT
  51  TCAAAAGAGC TATGCGGATC GGCTGTTTTC CTATGAAAAG ACAAAGATGG
 101  TGCACGATAC TCCGCTGATT CCTTGGGAAG AGGATAAGGA AAAATGTGCT
 151  GAAGCTGAGA AAGCTTTCTT AGAGCAACAG AAGATTCTCC TAGATTATGG
 201  AAAATCTATC TTTTGGCTGA ATGAGAACGA TGAGATCAAT TTAAACGATC
 251  CTTGGAGTTG GGGTCTTAAT ACGGTGAGGA CTAGGAAAGT ATTCCAAGAG
 301  GTTGACGACA GTGAACGTTG GAATCATAAG GTACTCATTC AAAAACTCGA
 351  GGACGATTAT GAGAAACTTC TAGAGGAAAG TTCAAAAGAG TCTACTGAAG
 401  CAAATAAGAA GCTTTTATCT GACTTAGTAG ATCGTCTTGA AGATGCTAAG
 451  ACAAAATTTT TCCTGAAGAA ACAGGAGGAG GTGGAGACTC GCGTTAAGGA
 501  TCTTAGAGCT CGATATGGAG GCACAGTAGA TCCTAAGCAG GATACGGAAG
 551  CTAAGAAGAA AGTCGAATTG GAGGCTAGCT TAGAAACCTT TTTAGATTCC
 601  ATCGAATCAG AGCTAGTACA GTGTTTAGAA GATCAAGATA TATATTGGAA
 651  AGAACAGGAT GTCAAAGATC TAGCACGTAC GCAAGAGCTC GAGGAACAAG
 701  ATATTGAAGC GAAGAGGGAA GAAGCTGCCG AAGACCTAAG AAGTCTTAAT
 751  GAGCGTTTAA AGAAGTCAAA AACTATGTTA GATAGGGCTA AATGGCATAT
 801  TGAAAATGCT GAGGACAGTA TTACCTGGTG GACTAGTCAG ATAGAAATGA
 851  AGGATATGAA AGCAAGACTG AAGATCTTAA AAGAAGATAT AACAAGTGTT
 901  CTACCTGAAA TAGATGAGAT TGAAACGTGT TTAAGCTTAG AGGAGCTTCC
 951  TTTGCTTACG ACCAGGGAAC TCTTAACTAA GTCCTACCTA AAGTTTAAGA
1001  TTTGTTCGGA AACACTATTA AAAATGACTT CTGTGTTTGA GAACAATATC
1051  TATGTTCAGG AGTACGAGGT TCAGCTGCAA AATCTAGGGT TTAAGTTACA
1101  AGGTATATCT CAGAGATTCG GAAAGAAACA AGACGATTTT GCGAATCTAG
1151  AGGAACAGGT TGCTTTGCAA AAGAAACGAC TCAGAGAGCT CACTCAGAAT
1201  TTTGAAATAC AAGGATTCAA TTTCATGAAA GAAGATTTTA AGGCAGCCGC
1251  TAAAGATCTT TATATAAGAA GTACAGCTGA ACAAAAGATG AACTTTGATG
1301  TGCCTTGCAT GGAGCTCTTC CGTAGGTATC ATGAGGAGGT CAACAAGCCG
1351  CTTCTTGAGT TGATGTACAA TTGTGCAGAC AGTTATAGAG ATGCTAAGAA

1401  AAAGCTTTGC TCTCTACGTC TTGATGAAAA AGAGTTATTA CAAAAAGAAA
1451  TCAAGAAAGA GGAATTTTAT CAAAAGAAAC AACAAAGGCA TGCAGATAGA
1501  TCACGTCATA CTACGTATCA AAAGCTACGA ATTGCTGAAG AGCTTGCTCT
1551  TGAGCTGAAG AAGAAAATCT AA
```

The PSORT algorithm predicts cytoplasmic location (0.412).

The protein was expressed in *E.coli* and purified as a GST-fusion product (Figure 69A). The recombinant protein was used to immunise mice, whose sera were used in a Western blot (Figure 69B) and for FACS analysis.

These experiments show that cp6269 is a surface-exposed and immunoaccessible protein, and that it is a useful immunogen. These properties are not evident from the sequence alone.

**Example 70**

The following *C.pneumoniae* protein (PID 4376270) was expressed <SEQ ID 139; cp6270>:

```
  1  MKIPLRFLLI SLVPTLSMSN LLGAATTEEL SASNSFDGTT STTSFSSKTS
 51  SATDGTNYVF KDSVVIENVP KTGETQSTSC FKNDAAAGDL NFLGGGFSFT
101  FSNIDATTAS GAAIGSEAAN KTVTLSGFSA LSFLKSPAST VTNGLGAINV
151  KGNLSLLDND KVLIQDNFST GDGGAINCAG SLKIANNKSL SFIGNSSSTR
201  GGAIHTKNLT LSSGGETLFQ GNTAPTAAGK GGAIAIADSG TLSISGDSGD
251  IIFEGNTIGA TGTVSHSAID LGTSAKITAL RAAQGHTIYF YDPITVTGST
301  SVADALNINS PDTGDNKEYT GTIVFSGEKL TEAEAKDEKN RTSKLLQNVA
351  FKNGTVVLKG DVVLSANGFS QDANSKLIMD LGTSLVANTE SIELTNLEIN
401  IDSLRNGKKI KLSAATAQKD IRIDRPVVLA ISDESFYQNG FLNEDHSYDG
451  ILELDAGKDI VISADSRSID AVQSPYGYQG KWTINWSTDD KKATVSWAKQ
501  SFNPTAEQEA PLVPNLLWGS FIDVRSFQNF IELGTEGAPY EKRFWVAGIS
551  NVLHRSGREN QRKFRHVSGG AVVGASTRMP GGDTLSLGFA QLFARDKDYF
601  MNTNFAKTYA GSLRLQHDAS LYSVVSILLG EGGLREILLP YVSKTLPCSF
651  YGQLSYGHTD HRMKTESLPP PPPTLSTDHT SWGGYVWAGE LGTRVAVENT
701  SGRGFFQEYT PFVKVQAVYA RQDSFVELGA ISRDFSDSHL YNLAIPLGIK
751  LEKRFAEQYY HVVAMYSPDV CRSNPKCTTT LLSNQGSWKT KGSNLARQAG
801  IVQASGFRSL GAAAELFGNF GFEWRGSSRS YNVDAGSKIK F*
```

A predicted signal peptide is highlighted.

The cp6270 nucleotide sequence <SEQ ID 140> is:

```
   1  ATGAAGATTC CACTCCGCTT TTTATTGATA TCATTAGTAC CTACGCTTTC
  51  TATGTCGAAT TTATTAGGAG CTGCTACTAC CGAAGAGTTA TCGGCTAGCA
 101  ATAGCTTCGA TGGAACTACA TCAACAACAA GCTTTTCTAG TAAAACATCA
 151  TCGGCTACAG ATGGCACCAA TTATGTTTTT AAAGATTCTG TAGTTATAGA
 201  AAATGTACCC AAAACAGGGG AAACTCAGTC TACTAGTTGT TTTAAAAATG
 251  ACGCTGCAGC TGGAGATCTA AATTTCTTAG GAGGGGGATT TTCTTTCACA
 301  TTTAGCAATA TCGATGCAAC CACGGCTTCT GGAGCTGCTA TTGGAAGTGA
 351  AGCAGCTAAT AAGACAGTCA CGTTATCAGG ATTTTCGGCA CTTTCTTTTC
 401  TTAAATCCCC AGCAAGTACA GTGACTAATG GATTGGGAGC TATCAATGTT
 451  AAAGGGAATT TAAGCCTATT GGATAATGAT AAGGTATTGA TTCAGGACAA
 501  TTTCTCAACA GGAGATGGCG GAGCAATTAA TTGTGCAGGC TCCTTGAAGA
 551  TCGCAAACAA TAAGTCCCTT TCTTTTATTG GAAATAGTTC TTCAACACGT
 601  GGCGGAGCGA TTCATACCAA AAACCTCACA CTATCTTCTG GTGGGGAAAC
 651  TCTATTTCAG GGGAATACAG CGCCTACGGC TGCTGGTAAA GGAGGTGCTA
 701  TCGCGATTGC AGACTCTGGC ACCCTATCCA TTTCTGGAGA CAGTGGCGAC
 751  ATTATCTTTG AAGGCAATAC GATAGGAGCT ACAGGAACCG TCTCTCATAG
 801  TGCTATTGAT TTAGGAACTA GCGCTAAGAT AACTGCGTTA CGTGCTGCGC
 851  AAGGACATAC GATATACTTT TATGATCCGA TTACTGTAAC AGGATCGACA
 901  TCTGTTGCTG ATGCTCTCAA TATTAATAGC CCTGATACTG GAGATAACAA
 951  AGAGTATACG GGAACCATAG TCTTTTCTGG AGAGAAGCTC ACGGAGGCAG
1001  AAGCTAAAGA TGAGAAGAAC CGCACTTCTA AATTACTTCA AAATGTTGCT
1051  TTTAAAAATG GGACTGTAGT TTTAAAAGGT GATGTCGTTT TAAGTGCGAA
1101  CGGTTTCTCT CAGGATGCAA ACTCTAAGTT GATTATGGAT TTAGGGACGT
1151  CGTTGGTTGC AAACACCGAA AGTATCGAGT TAACGAATTT GGAAATTAAT
1201  ATAGACTCTC TCAGGAACGG GAAAAAGATA AAACTCAGTG CTGCCACAGC
```

```
1251  TCAGAAAGAT ATTCGTATAG ATCGTCCTGT TGTACTGGCA ATTAGCGATG
1301  AGAGTTTTTA TCAAAATGGC TTTTTGAATG AGGACCATTC CTATGATGGG
1351  ATTCTTGAGT TAGATGCTGG GAAAGACATC GTGATTTCTG CAGATTCTCG
1401  CAGTATAGAT GCTGTACAAT CTCCGTATGG CTATCAGGGA AAGTGGACGA
1451  TCAATTGGTC TACTGATGAT AAGAAAGCTA CGGTTTCTTG GGCGAAGCAG
1501  AGTTTTAATC CCACTGCTGA GCAGGAGGCT CCGTTAGTTC CTAATCTTCT
1551  TTGGGGTTCT TTTATAGATG TTCGTTCCTT CCAGAATTTT ATAGAGCTAG
1601  GTACTGAAGG TGCTCCTTAC GAAAAGAGAT TTTGGGTTGC AGGCATTTCC
1651  AATGTTTTGC ATAGGAGCGG TCGTGAAAAT CAAAGGAAAT TCCGTCATGT
1701  GAGTGGAGGT GCTGTAGTAG GTGCTAGCAC GAGGATGCCG GGTGGTGATA
1751  CCTTGTCTCT GGGTTTTGCT CAGCTCTTTG CGCGTGACAA AGACTACTTT
1801  ATGAATACCA ATTTCGCAAA GACCTACGCA GGATCTTTAC GTTTGCAGCA
1851  CGATGCTTCC CTATACTCTG TGGTGAGTAT CCTTTTAGGA GAGGGAGGAC
1901  TCCGCGAGAT CCTGTTGCCT TATGTTTCCA AGACTCTGCC GTGCTCTTTC
1951  TATGGGCAGC TTAGCTACGG CCATACGGAT CATCGCATGA AGACCGAGTC
2001  TCTACCCCCC CCCCCCCCGA CGCTCTCGAC GGATCATACT TCTTGGGGAG
2051  GATATGTCTG GGCTGGAGAG CTGGGAACTC GAGTTGCTGT TGAAAATACC
2101  AGCGGCAGAG GATTTTTCCA AGAGTACACT CCATTTGTAA AAGTCCAAGC
2151  TGTTTACGCT CGCCAAGATA GCTTTGTAGA ACTAGGAGCT ATCAGTCGTG
2201  ATTTTAGTGA TTCGCATCTT TATAACCTTG CGATTCCTCT TGGAATCAAG
2251  TTAGAGAAAC GGTTTGCAGA GCAATATTAT CATGTTGTAG CGATGTATTC
2301  TCCAGATGTT TGTCGTAGTA ACCCCAAATG TACGACTACC CTACTTTCCA
2351  ACCAAGGGAG TTGGAAGACC AAAGGTTCGA ACTTAGCAAG ACAGGCTGGT
2401  ATTGTTCAGG CCTCAGGTTT TCGATCTTTG GGAGCTGCAG CAGAGCTTTT
2451  CGGGAACTTT GGCTTTGAAT GGCGGGGATC TTCTCGTAGC TATAATGTAG
2501  ATGCGGGTAG CAAAATCAAA TTTTAG
```

The PSORT algorithm predicts outer membrane (0.92).

The protein was expressed in *E.coli* and purified as a GST-fusion product (Figure 70A). The recombinant protein was used to immunise mice, whose sera were used in a Western blot and for FACS analysis (Figure 70B).

The cp6270 protein was also identified in the 2D-PAGE experiment (Cpn0013).

These experiments show that cp6270 is a surface-exposed and immunoaccessible protein, and that it is a useful immunogen. These properties are not evident from the sequence alone.

### Example 71

The following *C.pneumoniae* protein (PID 4376402) was expressed <SEQ ID 141; cp6402>:

```
  1  MNVADLLSHL ETLLSSKIFQ DYGPNGLQVG DPQTPVKKIA VAVTADLETI
 51  KQAVAAEANV LIVHHGIFWK GMPYPITGMI HKRIQLLIEH NIQLIAYHLP
101  LDAHPTLGNN WRVALDLNWH DLKPFGSSLP YLGVQGSFSP IDIDSFIDLL
151  SQYYQAPLKG SALGGPSRVS SAALISGGAY RELSSAATSQ VDCFITGNFD
201  EPAWSTALES NINFLAFGHT ATEKVGPKSL AEHLKSEFPI STTFIDTANP
251  F*
```

The cp6402 nucleotide sequence <SEQ ID 142> is:

```
  1  ATGAATGTTG CGGATCTCCT TTCTCATCTT GAGACTCTTC TCTCATCAAA
 51  AATATTTCAG GATTATGGAC CCAACGGACT TCAAGTTGGA GATCCCCAAA
101  CTCCGGTAAA GAAAATCGCT GTTGCAGTTA CCGCAGATCT AGAAACCATA
151  AAACAAGCTG TTGCGGCCGA AGCAAACGTT CTCATTGTAC ACCACGGAAT
201  TTTTTGGAAA GGTATGCCCT ATCCTATTAC CGGCATGATC CATAAGCGCA
251  TCCAATTACT AATAGAACAC AATATCCAAC TCATTGCCTA CCACCTTCCT
301  TTGGATGCTC ACCCTACCTT AGGAAATAAC TGGAGAGTTG CCCTGGATCT
351  AAATTGGCAT GACTTGAAGC CCTTTGGTTC TTCCCTCCCT TATTTAGGAG
401  TGCAAGGCTC TTTCTCTCCT ATCGATATAG ATTCTTTCAT TGACCTGTTA
451  TCTCAATATT ACCAAGCTCC CCTAAAAGGA TCTGCCTTGG GCGGCCCCTC
501  TAGAGTCTCC TCAGCAGCTC TGATCTCAGG AGGAGCTTAT AGAGAACTCT
551  CTTCGGCAGC CACGTCCCAA GTCGATTGCT TCATCACAGG AAATTTTGAT
601  GAACCTGCAT GGTCGACAGC TCTAGAAAGC AATATCAACT TCCTAGCATT
651  TGGACATACA GCCACAGAAA AAGTAGGTCC AAAATCTCTT GCAGAGCATC

701  TAAAAAGCGA ATTTCCTATT TCCACAACCT TTATAGATAC GGCCAACCCC
751  TTCTAA
```

The PSORT algorithm predicts cytoplasmic (0.158).

The protein was expressed in *E.coli* and purified as a GST-fusion product (Figure 71A). The recombinant protein was used to immunise mice, whose sera were used in a Western blot (Figure 71B) and for FACS analysis.

These experiments show that cp6402 is a surface-exposed and immunoaccessible protein, and that it is a useful immunogen. These properties are not evident from the sequence alone.

## Example 72

The following *C.pneumoniae* protein (PID 4376520) was expressed <SEQ ID 143; cp6520>:

```
  1  MKHYLSFSPS ADFFSKQGAI ETQVLFGERV LVKGSTCYAY SQLFHNELLW
 51  KPYPGHSFRS TLVPCTPEFH IHPNVSVVSV DAFLDPWGIP LPFGTLLHVN
101  SQNTVIFPKD ILNHMNTIWG SGTPQCDPRH LRRLNYNFFA ELLIKDADLL
151  LNFPYVWGGR SVHESLEKPG VDCSGFINIL YQAQGYNVPR NAADQYADCH
201  WISSFENLPS GGLIFLYPKE EKRISHVMLK QDSSTLIHAS GGGKKVEYFI
251  LEQDGKFLDS TYLFFRNNQR GRAFFGIPRK RKAFL*
```

The cp6520 nucleotide sequence <SEQ ID 144> is:

```
  1  ATGAAACACT ACCTATCATT TTCTCCTTCT GCTGATTTTT TCTCTAAACA
 51  GGGTGCTATT GAAACTCAAG TCCTTTTTGG AGAGCGCGTC TTAGTCAAAG
101  GGAGCACCTG CTATGCATAT TCCCAATTAT TCCACAATGA GCTGTTATGG
151  AAGCCCTATC CAGGTCATAG CTTTCGTTCT ACCCTAGTCC CCTGCACTCC
201  TGAATTTCAT ATCCATCCAA ATGTTTCTGT GGTTTCTGTG GATGCATTTT
251  TAGATCCTTG GGGGATCCCT CTTCCTTTTG GAACTTTACT CCATGTGAAT
301  TCTCAAAATA CCGTTATTTT CCCTAAGGAT ATTCTCAATC ATATGAACAC
351  CATCTGGGGC TCCGGCACAC CTCAATGCGA TCCTAGACAT CTACGTCGTC
401  TAAATTATAA CTTCTTTGCT GAACTTTTAA TTAAAGACGC AGACCTTTTA
451  CTGAACTTTC CCTATGTATG GGGAGGACGG TCTGTACACG AAAGTCTGGA
501  AAAGCCGGGT GTTGATTGTT CGGGATTTAT CAATATCCTT TACCAGGCAC
551  AGGGATACAA CGTCCCTAGA AACGCTGCAG ATCAATATGC GGATTGTCAT
601  TGGATCTCTA GCTTTGAGAA CCTTCCTTCT GGTGGGTTAA TATTTCTTTA
651  CCCTAAAGAA GAAAAGCGTA TTTCTCATGT TATGTTGAAA CAGGATAGTT
701  CCACCCTCAT TCATGCTTCT GGTGGAGGGA AAAAAGTGGA GTATTTCATT
751  TTAGAACAAG ATGGGAAGTT TTTAGATTCG ACTTATCTAT TTTTTAGAAA
801  TAATCAGAGG GGACGGGCAT TTTTTGGGAT CCCTAGAAAA AGAAAAGCCT
851  TTCTGTAA
```

The PSORT algorithm predicts cytoplasmic (0.265).

The protein was expressed in *E.coli* and purified as a GST-fusion product (Figure 72A). The recombinant protein was used to immunise mice, whose sera were used in a Western blot (Figure 72B) and for FACS analysis.
These experiments show that cp6520 is a surface-exposed and immunoaccessible protein, and that it is a useful immunogen. These properties are not evident from the sequence alone.

**Example 73**

The following *C.pneumoniae* protein (PID 4376567) was expressed <SEQ ID 145; cp6567>:

```
  1  MTSPIPFQSS GDASFLAEQP QQLPSTSESQ LVTQLLTMMK HTQALSETVL
 51  QQQRDRLPTA SIILQVGGAP TGGAGAPFQP GPADDHHHPI PPPVVPAQIE
101  TEITTIRSEL QLMRSTLQQS TKGARTGVLV VTAILMTISL LAIIIIILAV
151  LGFTGVLPQV ALLMQGETNL IWAMVSGSII CFIALIGTLG LILTNKNTPL

201  PAS*
```

The cp6567 nucleotide sequence <SEQ ID 146> is:

```
  1  ATGACCTCAC CGATCCCCTT TCAGTCTAGT GGCGATGCCT CTTTCCTTGC
 51  CGAGCAGCCA CAGCAACTCC CGTCTACTTC TGAATCTCAG CTAGTAACTC
101  AATTGCTAAC CATGATGAAG CATACTCAAG CATTATCCGA AACGGTTCTT
151  CAACAACAAC GCGATCGATT ACCAACCGCA TCTATTATCC TTCAAGTAGG
201  AGGAGCTCCT ACAGGAGGAG CGGGTGCGCC TTTTCAACCA GGACCGGCAG
251  ATGATCATCA TCATCCCATA CCGCCGCCTG TTGTACCAGC TCAAATAGAA
301  ACAGAAATCA CCACTATAAG ATCCGAGTTA CAGCTCATGC GATCTACTCT
351  ACAACAAAGC ACAAAAGGAG CTCGTACAGG AGTTCTAGTG GTTACTGCAA
401  TCTTAATGAC GATCTCCTTA TTGGCTATTA TTATCATAAT ACTAGCTGTG
451  CTTGGATTTA CGGGCGTCTT GCCTCAAGTA GCTTTATTGA TGCAGGGTGA
501  AACAAATCTG ATTTGGGCTA TGGTGAGCGG TTCTATTATT TGCTTTATTG
551  CGCTAATTGG AACTCTAGGA TTAATTTTAA CAAATAAGAA CACGCCTCTA
601  CCGGCTTCTT AA
```

The PSORT algorithm predicts inner membrane (0.694).
The protein was expressed in *E.coli* and purified as a GST-fusion product (Figure 73A). The recombinant protein was used to immunise mice, whose sera were used in a Western blot (Figure 73B) and for FACS analysis.
These experiments show that cp6567 is a surface-exposed and immunoaccessible protein, and that it is a useful immunogen. These properties are not evident from the sequence alone.

**Example 74**

The following *C.pneumoniae* protein (PID 4376576) was expressed <SEQ ID 147; cp6576>:

```
  1 MLIMRNKVIL QISILALIQT PLTLFSTEKV KEGHVVVDSI TIITEGENAS
 51 NKHPLPKLKT RSGALFSQLD FDEDLRILAK EYDSVEPKVE FSEGKTNIAL
101 HLIAKPSIRN IHISGNQVVP EHKILKTLQI YRNDLFEREK FLKGLDDLRT
151 YYLKRGYFAS SVDYSLEHNQ EKGHIDVLIK INEGPCGKIK QLTFSGISRS
201 EKSDIQEFIQ TKQHSTTTSW FTGAGLYHPD IVEQDSLAIT NYLHNNGYAD
251 AIVNSHYDLD DKGNILLYMD IDRGSRYTLG HVHIQGFEVL PKRLIEKQSQ
301 VGPNDLYCPD KIWDGAHKIK QTYAKYGYIN TNVDVLFIPH ATRPIYDVTY
351 EVSEGSPYKV GLIKITGNTH TKSDVILHET SLFPGDTFNR LKLEDTEQRL
401 RNTGYFQSVS VYTVRSQLDP MGNADQYRDI FVEVKETTTG NLGLFLGFSS
451 LDNLFGGIEL SESNFDLFGA RNIFSKGFRC LRGGGEHLFL KANFGDKVTD
501 YTLKWTKPHF LNTPWILGIE LDKSINRALS KDYAVQTYGG NVSTTYILNE
551 HLKYGLFYRG SQTSLHEKRK FLLGPNIDSN KGFVSAAGVN LNYDSVDSPR
601 TPTTGIRGGV TFEVSGLGGT YHFTKLSLNS SIYRKLTRKG ILKIKGEAQF
651 IKPYSNTTAE GVPVSERFFL GGETTVRGYK SFIIGPKYSA TEPQGGLSSL
701 LISEEFQYPL IRQPNISAFV FLDSGFVGLQ EYKISLKDLR SSAGFGLRFD
751 VMNNVPVMLG FGWPFRPTET LNGEKIDVSQ RFFFALGGMF *
```

A predicted signal peptide is highlighted.

The cp6576 nucleotide sequence <SEQ ID 148> is:

```
  1 ATGCTCATCA TGCGAAATAA AGTTATCTTG CAAATATCTA TTCTAGCGTT
 51 AATCCAAACC CCTTTAACTT TATTTTCTAC TGAAAAAGTT AAAGAAGGCC
101 ATGTGGTGGT AGACTCTATC ACAATCATAA CGGAAGGAGA AAATGCTTCA
151 AATAAACATC CCTTACCCAA ATTAAAGACC AGAAGTGGGG CTCTTTTTTC
201 TCAATTAGAT TTTGATGAAG ACTTGAGAAT TCTAGCTAAA GAATACGACT
251 CTGTTGAGCC TAAAGTAGAA TTTTCTGAAG GGAAAACTAA CATAGCCCTT
301 CACCTAATAG CTAAACCCTC AATTCGAAAT ATTCATATCT CAGGAAATCA
351 AGTCGTTCCT GAACATAAAA TTCTTAAAAC CCTACAAATT TACCGTAATG
401 ATCTCTTTGA ACGAGAAAAA TTTCTTAAGG GTCTTGATGA TCTAAGAACG
451 TATTATCTCA AGCGAGGATA TTTCGCATCC AGTGTAGACT ACAGTCTGGA
501 ACACAATCAA GAAAAAGGTC ACATCGATGT TTTAATTAAA ATCAATGAAG
551 GTCCTTGCGG GAAAATTAAA CAGCTTACGT TCTCAGGAAT CTCTCGATCA
601 GAAAAATCAG ATATCCAAGA ATTTATTCAA ACCAAGCAGC ACTCTACAAC
```

```
 651  TACAAGTTGG TTTACTGGAG CTGGACTCTA TCACCCAGAT ATTGTTGAAC
 701  AAGATAGCTT GGCAATTACG AATTACCTAC ATAATAACGG GTACGCTGAT
 751  GCTATAGTCA ACTCTCACTA TGACCTTGAC GACAAAGGGA ATATTCTTCT
 801  TTACATGGAT ATTGATCGAG GGTCGCGATA TACCTTAGGA CACGTCCATA
 851  TCCAAGGGTT TGAGGTTTTG CCAAAACGCC TTATAGAAAA GCAATCCCAA
 901  GTCGGCCCCA ATGATCTTTA TTGCCCCGAT AAAATATGGG ATGGGGCTCA
 951  TAAGATCAAA CAAACTTATG CAAAGTATGG CTACATCAAT ACCAATGTAG
1001  ACGTTCTCTT CATCCCTCAC GCAACCCGCC CTATTTATGA TGTAACTTAT
1051  GAGGTAAGTG AAGGGTCTCC TTATAAAGTT GGGTTAATTA AAATTACTGG
1101  GAATACCCAT ACAAAATCTG ACGTTATTTT ACACGAAACC AGTCTCTTCC
1151  CAGGAGATAC ATTCAATCGC TTAAAGCTAG AAGATACTGA GCAACGTTTA
1201  AGAAATACAG GCTACTTCCA AAGCGTTAGT GTCTATACAG TTCGTTCTCA
1251  ACTTGATCCT ATGGGCAATG CGGATCAATA CCGAGATATT TTTGTAGAAG
1301  TCAAAGAAAC AACAACAGGA AACTTAGGCT TATTCTTAGG ATTTAGTTCT
1351  CTTGACAATC TTTTTGGAGG AATTGAACTA TCTGAAAGTA ATTTTGATCT
1401  ATTTGGAGCT AGAAATATAT TTTCTAAAGG TTTTCGTTGT CTAAGAGGCG
1451  GTGGAGAACA TCTATTCTTA AAAGCCAACT TCGGGGACAA AGTCACAGAC
1501  TATACTTTGA AGTGGACCAA ACCTCATTTT CTAAACACTC CTTGGATTTT
1551  AGGAATTGAA TTAGATAAAT CAATTAACAG AGCATTATCT AAAGATTATG
1601  CTGTCCAAAC CTATGGCGGG AACGTCAGCA CAACGTATAT CTTGAACGAA
1651  CACCTGAAAT ACGGTCTATT TTATCGAGGA AGTCAAACGA GTTTACATGA
1701  AAAACGTAAG TTCCTCCTAG GGCCAAATAT AGACAGCAAT AAAGGATTTG
1751  TCTCTGCTGC AGGTGTCAAC TTGAATTACG ATTCTGTAGA TAGTCCTAGA
1801  ACTCCAACTA CAGGGATTCG CGGGGGGGTG ACTTTTGAGG TTTCTGGTTT
1851  GGGAGGAACT TATCATTTTA CAAAACTCTC TTTAAACAGC TCTATCTATA
1901  GAAAACTTAC GCGTAAAGGT ATTTTGAAAA TCAAAGGGGA AGCTCAATTT
1951  ATTAAACCCT ATAGCAATAC TACAGCTGAA GGAGTTCCTG TCAGTGAGCG
2001  CTTCTTCCTA GGTGGAGAGA CTACAGTTCG GGGATATAAA TCCTTTATTA
2051  TCGGTCCAAA ATACTCTGCT ACAGAACCTC AGGGAGGACT CTCTTCGCTC
2101  CTTATTTCAG AAGAGTTTCA ATACCCTCTC ATCAGACAAC CTAATATTAG
2151  TGCCTTTGTA TTCTTAGACT CAGGTTTTGT CGGTTTACAA GAGTATAAGA
2201  TTTCGTTAAA AGATCTACGT AGTAGTGCTG GATTTGGTCT GCGCTTCGAT
2251  GTAATGAATA ATGTTCCTGT TATGTTAGGA TTTGGTTGGC CCTTCCGTCC
2301  AACCGAGACT TTGAATGGAG AAAAAATTGA TGTATCTCAG CGATTCTTCT
2351  TTGCTTTAGG GGGCATGTTC TAA
```

The PSORT algorithm predicts outer membrane (0.7658).

The protein was expressed in *E.coli* and purified as GST-fusion (Figure 74A), his-tag and his-tag/GST-fusion products. The recombinant proteins were used to immunise mice, whose sera were used in a Western blot (Figure 74B) and for FACS analysis (Figure 74C).

The cp6576 protein was also identified in the 2D-PAGE experiment (Cpn0300).

These experiments show that cp6576 is a surface-exposed and immunoaccessible protein, and that it is a useful immunogen. These properties are not evident from the sequence alone.

## Example 75

The following *C.pneumoniae* protein (PID 4376607) was expressed <SEQ ID 149; cp6607>:

```
  1  MNKRQKDKLK ICVIISTLIL VGIFARAPRG DTFKTFLKSE EAIIYSNQCN
 51  EDMRKILCDA IEHADEEIFL RIYNLSEPKI QQSLTRQAQA KNKVTIYYQK
101  FKIPQILKQA SNVTLVEQPP AGRKLMHQKA LSIDKKDAWL GSANYTNLSL
151  RLDNNLILGM HSSELCDLII TNTSGDFSIK DQTGKYFVLP QDRKIAIQAV
201  LEKIQTAQKT IQVAMFALTH SEIIQALHQA KQRGIHVDII IDRSHSKLTF
251  KQLRQLNINK DFVSINTAPC TLHHKFAVID NKTLLAGSIN WSKGRFSLND
301  ESLIILENLT KQQNQKLRMI WKDLAKHSEH PTVDDEEKEI IEKSLPVEEQ
351  EAA*
```

A predicted signal peptide is highlighted.

The cp6607 nucleotide sequence <SEQ ID 150> is:

```
   1  ATGAATAAAA GACAAAAAGA TAAATTAAAA ATCTGTGTTA TTATTAGCAC
  51  GTTGATTTTA GTAGGAATTT TTGCAAGAGC TCCTCGTGGT GACACTTTTA
 101  AGACTTTTTT AAAGTCTGAA GAAGCTATCA TCTACTCAAA TCAATGCAAT
 151  GAGGACATGC GTAAAATTCT ATGCGATGCT ATAGAACACG CTGATGAAGA
 201  GATCTTCCTA CGTATTTATA ACCTCTCAGA ACCCAAGATC CAACAGAGTT
 251  TAACTCGACA AGCTCAAGCA AAAAACAAAG TTACGATCTA CTATCAAAAA
 301  TTTAAAATTC CCCAAATCTT AAAGCAAGCC AGCAATGTAA CTTTAGTCGA
 351  GCAACCTCCA GCAGGGCGTA AACTGATGCA TCAAAAAGCT CTTTCCATAG
 401  ATAAGAAAGA TGCTTGGCTA GGATCTGCGA ACTACACCAA TCTTTCTCTA
 451  CGTTTAGATA ATAATCTCAT TCTAGGAATG CATAGCTCGG AGCTCTGTGA
 501  TCTCATTATC ACAAATACCT CTGGAGACTT TTCTATAAAG GATCAAACAG
 551  GAAAGTATTT TGTTCTTCCT CAAGATCGTA AAATTGCAAT ACAAGCTGTA
 601  CTCGAAAAAA TCCAGACAGC TCAGAAAACC ATCCAAGTTG CTATGTTTGC
 651  TCTGACCCAC TCGGAGATTA TTCAAGCCTT ACATCAAGCA AAACAACGAG
 701  GAATCCATGT AGATATTATC ATTGATAGAA GTCATAGCAA ACTTACTTTT
 751  AAGCAATTAC GACAATTAAA TATCAATAAA GACTTTGTTT CTATAAATAC
 801  CGCACCCTGT ACTCTTCACC ATAAGTTTGC AGTTATAGAT AATAAAACTC
 851  TACTTGCAGG ATCTATAAAT TGGTCTAAAG GAAGATTCTC CTTAAATGAT
 901  GAAAGCTTGA TCATACTGGA AAACCTGACC AAACAACAAA ATCAGAAACT
 951  TCGAATGATT TGGAAAGATC TAGCTAAGCA TTCAGAACAT CCTACAGTAG
1001  ACGATGAAGA AAAAGAAATT ATAGAAAAAA GTCTTCCAGT AGAAGAGCAA
1051  GAAGCAGCGT GA
```

The PSORT algorithm predicts periplasmic (0.934).

The protein was expressed in *E.coli* and purified as a his-tagged product (Figure 75A) and also as a GST-fusion. The GST-fusion protein was used to immunise mice, whose sera were used in a Western blot (Figure 75B) and for FACS analysis.

These experiments show that cp6607 is a surface-exposed and immunoaccessible protein, and that it is a useful immunogen. These properties are not evident from the sequence alone.

### Example 76

The following *C.pneumoniae* protein (PID 4376624) was expressed <SEQ ID 151; cp6624>:

```
  1  MDAKMGYIFK VMRWIFCFVA CGITFGCTNS GFQNANSRPC ILSMNRMIHD
 51  CVERVVGNRL ATAVLIKGSL DPHAYEMVKG DKDKIAGSAV IFCNGLGLEH
101  TLSLRKHLEN NPNSVKLGER LIARGAFVPL EEDGICDPHI WMDLSIWKEA
151  VIEITEVLIE KFPEWSAEFK ANSEELVCEM SILDSWAKQC LSTIPENLRY
201  LVSGHNAFSY FTRRYLATPE EVASGAWRSR CISPEGLSPE AQISVRDIMA
251  VVDYINEHDV SVVFPEDTLN QDALKKIVSS LKKSHLVRLA QKPLYSDNVD
301  DNYFSTFKHN VCLITEELGG VALECQR*
```

The cp6624 nucleotide sequence <SEQ ID 152> is:

```
  1  ATGGATGCGA AAATGGGATA TATATTTAAA GTGATGCGTT GGATTTTCTG
 51  TTTCGTGGCA TGTGGTATAA CTTTTGGATG TACCAATTCT GGGTTTCAGA
101  ATGCAAATTC ACGTCCTTGT ATACTATCCA TGAATCGCAT GATTCATGAT
151  TGTGTTGAAA GAGTCGTGGG GAATAGGCTT GCTACCGCTG TTTTGATCAA
201  AGGATCCTTA GACCCTCATG CGTATGAGAT GGTTAAAGGG GATAAGGACA
251  AGATTGCTGG AAGTGCCGTA ATTTTTTGTA ACGGCCTGGG TCTTGAGCAT
301  ACATTAAGTT TGCGGAAGCA TTTAGAAAAT AATCCCAATA GTGTCAAGTT
351  AGGGGAGCGG TTGATAGCGC GTGGGGCCTT TGTTCCTCTA GAAGAAGACG
401  GTATTTGCGA TCCTCATATC TGGATGGATC TTTCTATTTG GAAGGAAGCT
451  GTCATAGAAA TTACAGAAGT TCTCATTGAA AAGTTCCCTG AATGGTCTGC
501  TGAATTTAAA GCAAATAGTG AGGAACTTGT TTGTGAAATG TCTATTTTAG
551  ATTCTTGGGC GAAACAATGC TTGAGCACAA TTCCTGAAAA TTTACGGTAT
601  CTTGTCTCAG GTCATAATGC GTTCAGTTAC TTTACACGTC GCTATTTAGC
651  TACTCCTGAA GAAGTGGCTT CCGGAGCATG GAGGTCTCGT TGTATTTCTC
701  CTGAGGGTCT ATCTCCAGAA GCTCAAATCA GTGTTCGTGA TATTATGGCG
751  GTTGTAGATT ATATTAATGA GCATGATGTC AGTGTGGTTT TCCCTGAGGA
801  TACTCTGAAC CAAGATGCGT TGAAAAAAAT TGTTTCTTCT CTGAAGAAAA
851  GTCATTTAGT TCGTCTAGCT CAAAAACCAT TGTATAGTGA TAATGTGGAC
901  GACAATTATT TTAGCACCTT TAAACATAAT GTCTGCCTTA TCACAGAAGA

951  ATTAGGAGGG GTGGCTCTTG AATGTCAAAG ATGA
```

The PSORT algorithm predicts inner membrane (0.168).

The protein was expressed in *E.coli* and purified as a his-tag product (Figure 76A). The recombinant protein was used to immunise mice, whose sera were used in a Western blot (Figure 76B) and for FACS analysis.

The cp6624 protein was also identified in the 2D-PAGE experiment.

These experiments show that cp6624 is a surface-exposed and immunoaccessible protein, and that it is a useful immunogen. These properties are not evident from the sequence alone.

### Example 77

The following *C.pneumoniae* protein (PID 4376728) was expressed <SEQ ID 153; cp6728>:

```
  1  MKSSVSWLFF SSIPLFSSLS IVAAEVTLDS SNNSYDGSNG TTFTVFSTTD
 51  AAAGTTYSLL SDVSFQNAGA LGIPLASGCF LEAGGDLTFQ GNQHALKFAF
101  INAGSSAGTV ASTSAADKNL LFNDFSRLSI ISCPSLLLSP TGQCALKSVG
151  NLSLTGNSQI IFTQNFSSDN GGVINTKNFL LSGTSQFASF SRNQAFTGKQ
201  GGVVYATGTI TIENSPGIVS FSQNLAKGSG GALYSTDNCS ITDNFQVIFD
251  GNSAWEAAQA QGGAICCTTT DKTVTLTGNK NLSFTNNTAL TYGGAISGLK
301  VSISAGGPTL FQSNISGSSA GQGGGGAINI ASAGELALSA TSGDITFNNN
351  QVTNGSTSTR NAINIIDTAK VTSIRAATGQ SIYFYDPITN PGTAASTDTL
401  NLNLADANSE IEYGGAIVFS GEKLSPTEKA IAANVTSTIR QPAVLARGDL
451  VLRDGVTVTF KDLTQSPGSR ILMDGGTTLS AKEANLSLNG LAVNLSSLDG
501  TNKAALKTEA ADKNISLSGT IALIDTEGSF YENHNLKSAS TYPLLELTTA
551  GANGTITLGA LSTLTLQEPE THYGYQGNWQ LSWANATSSK IGSINWTRTG
601  YIPSPERKSN LPLNSLWGNF IDIRSINQLI ETKSSGEPFE RELWLSGIAN
651  FFYRDSMPTR HGFRHISGGY ALGITATTPA EDQLTFAFCQ LFARDRNHIT
701  GKNHGDTYGA SLYFHHTEGL FDIANFLWGK ATRAPWVLSE ISQIIPLSFD
751  AKFSYLHTDN HMKTYYTDNS IIKGSWRNDA FCADLGASLP FVISVPYLLK
801  EVEPFVKVQY IYAHQQDFYE RHAEGRAFNK SELINVEIPI GVTFERDSKS
851  EKGTYDLTLM YILDAYRRNP KCQTSLIASD ANWMAYGTNL ARQGFSVRAA
901  NHFQVNPHME IFGQFAFEVR SSSRNYNTNL GSKFCF*
```

The cp6728 nucleotide sequence <SEQ ID 154> is:

```
   1  ATGAAGTCCT CTGTCTCTTG GTTGTTCTTT TCTTCAATCC CGCTCTTTTC
  51  ATCGCTCTCT ATAGTCGCGG CAGAGGTGAC CTTAGATAGC AGCAATAATA
 101  GCTATGATGG ATCTAACGGA ACTACCTTCA CGGTCTTTTC CACTACGGAC
 151  GCTGCTGCAG GAACTACCTA TTCCTTACTT TCCGACGTAT CCTTTCAAAA
 201  TGCAGGGGCT TTAGGAATTC CCTTAGCCTC AGGATGCTTC CTAGAAGCGG
 251  GCGGCGATCT TACTTTCCAA GGAAATCAAC ATGCACTGAA GTTTGCATTT
 301  ATCAATGCGG GCTCTAGCGC TGGAACTGTA GCCAGTACCT CAGCAGCAGA
 351  TAAGAATCTT CTCTTTAATG ATTTTTCTAG ACTCTCTATT ATCTCTTGTC
 401  CCTCTCTTCT TCTCTCTCCT ACTGGACAAT GTGCTTTAAA ATCTGTGGGG
 451  AATCTATCTC TAACTGGCAA TTCCCAAATT ATATTTACTC AGAACTTCTC
 501  GTCAGATAAC GGCGGTGTTA TCAATACGAA AAACTTCTTA TTATCAGGGA
 551  CATCTCAGTT TGCGAGCTTT TCGAGAAACC AAGCCTTCAC AGGGAAGCAA
 601  GGCGGTGTAG TTTACGCTAC AGGAACTATA ACTATCGAGA ACAGCCCTGG
 651  GATAGTTTCC TTCTCTCAAA ACCTAGCGAA AGGATCTGGC GGTGCTCTGT
 701  ACAGCACTGA CAACTGTTCG ATTACAGATA ACTTTCAAGT GATCTTTGAC
 751  GGCAATAGTG CTTGGGAAGC CGCTCAAGCT CAGGGCGGGG CTATTTGTTG
 801  CACTACGACA GATAAAACAG TGACTCTTAC TGGGAACAAA AACCTCTCTT
 851  TCACAAATAA TACAGCATTG ACATATGGCG GAGCCATCTC TGGACTCAAG
 901  GTCAGTATTT CCGCTGGAGG TCCTACTCTA TTTCAAAGTA ATATCTCAGG
 951  AAGTAGCGCC GGTCAGGGAG GAGGAGGAGC GATCAATATA GCATCTGCTG
1001  GGGAACTCGC TCTCTCTGCT ACTTCTGGAG ATATTACCTT CAATAACAAC
1051  CAAGTCACCA ACGGAAGCAC AAGTACAAGA AACGCAATAA ATATCATTGA
1101  TACCGCTAAA GTCACATCGA TACGAGCTGC TACGGGGCAA TCTATCTATT
1151  TCTATGATCC CATCACAAAT CCAGGAACCG CAGCTTCTAC CGACACATTG
1201  AACTTAAACT TAGCAGATGC GAACAGTGAG ATCGAGTATG GGGGTGCGAT
1251  TGTCTTTTCT GGAGAAAAGC TTTCCCCTAC AGAAAAAGCA ATCGCTGCAA

1301  ACGTCACCTC TACTATCCGA CAACCTGCAG TATTAGCGCG GGGAGATCTT
1351  GTACTTCGTG ATGGAGTCAC CGTAACTTTC AAGGATCTGA CTCAAAGTCC
1401  AGGATCCCGC ATCTTAATGG ATGGGGGGAC TACACTTAGT GCTAAAGAGG
1451  CAAATCTTTC GCTTAATGGC TTAGCAGTAA ATCTCTCCTC TTTAGATGGA
1501  ACCAACAAGG CAGCTTTAAA AACAGAAGCT GCAGATAAAA ATATCAGCCT
1551  ATCGGGAACG ATTGCGCTTA TTGACACGGA AGGGTCATTC TATGAGAATC
1601  ATAACTTAAA AAGTGCTAGT ACCTATCCTC TTCTTGAACT TACCACCGCA
1651  GGAGCCAACG GAACGATTAC TCTGGGAGCT CTTTCTACCC TGACTCTTCA
1701  AGAACCTGAA ACCCACTACG GGTATCAAGG AAACTGGCAG TTGTCTTGGG
1751  CAAATGCAAC ATCCTCAAAA ATAGGAAGCA TCAACTGGAC CCGTACAGGA
1801  TACATTCCTA GTCCTGAGAG AAAAAGTAAT CTCCCTCTAA ATAGCTTATG
1851  GGGAAACTTT ATAGATATAC GCTCGATCAA TCAGCTTATA GAAACCAAGT
1901  CCAGTGGGGA GCCTTTTGAG CGTGAGCTAT GGCTTTCAGG AATTGCGAAT
1951  TTCTTCTATA GAGATTCTAT GCCCACCCGC CATGGTTTCC GCCATATCAG
2001  CGGGGGTTAT GCACTAGGGA TCACAGCAAC AACTCCTGCC GAGGATCAGC
2051  TTACTTTTGC CTTCTGCCAG CTCTTTGCTA GAGATCGCAA TCATATTACA
2101  GGTAAGAACC ACGGAGATAC TTACGGTGCC TCTTTGTATT TCCACCATAC
2151  AGAAGGGCTC TTCGACATCG CCAATTTCCT CTGGGGAAAA GCAACCCGAG
2201  CTCCCTGGGT GCTCTCTGAG ATCTCCCAGA TCATTCCTTT ATCGTTCGAT
2251  GCTAAATTCA GTTATCTCCA TACAGACAAC CACATGAAGA CATATTATAC
2301  CGATAACTCT ATCATCAAGG GTTCTTGGAG AAACGATGCC TTCTGTGCAG
2351  ATCTTGGAGC TAGCCTGCCT TTTGTTATTT CCGTTCCGTA TCTTCTGAAA
2401  GAAGTCGAAC CTTTTGTCAA AGTACAGTAT ATCTATGCGC ATCAGCAAGA
2451  CTTCTACGAG CGTCATGCTG AAGGACGCGC TTTCAATAAA AGCGAGCTTA
2501  TCAACGTAGA GATTCCTATA GGCGTCACCT TCGAAAGAGA CTCAAAATCA
2551  GAAAAGGGAA CTTACGATCT TACTCTTATG TATATACTCG ATGCTTACCG
2601  ACGCAATCCT AAATGTCAAA CTTCCCTAAT AGCTAGCGAT GCTAACTGGA
2651  TGGCCTATGG TACCAACCTC GCACGACAAG GTTTTTCTGT TCGTGCTGCG
2701  AACCATTTCC AAGTGAACCC CCACATGGAA ATCTTCGGTC AATTCGCTTT
2751  TGAAGTACGA AGTTCTTCAC GAAATTATAA TACAAACCTA GGCTCTAAGT
2801  TTTGTTTCTA G
```

The PSORT algorithm predicts inner membrane (0.187).

The protein was expressed in *E.coli* and purified as a GST-fusion product (Figure 77A). The recombinant protein was used to immunise mice, whose sera were used in a Western blot (Figure 77B) and for FACS analysis.

The cp6728 protein was also identified in the 2D-PAGE experiment.

These experiments show that cp6728 is a surface-exposed and immunoaccessible protein, and that it is a useful immunogen. These properties are not evident from the sequence alone.

**Example 78**

The following *C.pneumoniae* protein (PID 4376847) was expressed <SEQ ID 155; cp6847>:

```
  1  MFVMKKLVRL CVVLLSLLPN VLFSSDLLRE EGIKKMMDKL IEYHVDAQEV
 51  STDILSRSLS SYIQSFDPHK SYLSNQEVAV FLQSPETKKR LLKNYKAGNF
101  AIYRNINQLI HESILRARQW RNEWVKNPKE LVLEASSYQI SKQPMQWSKS
151  LDEVKQRQRA LLLSYLSLHL AGASSSRYEG KEEQLAALCL RQIENHENVY
201  LGINDHGVAM DRDEEAYQFH IRVVKALAHS LDAHTAYFSK DEALAMRIQL
251  EKGMCGIGVV LKEDIDGVVV REIIPGGPAA KSGDLQLGDI IYRVDGKDIE
301  HLSFRGVLDC LRGGHGSTVV LDIHRGESDH TIALRREKIL LEDRRVDVSY
351  EPYGDGVIGK VTLHSFYEGE NQVSSEQDLR RAIQGLKEKN LLGLVLDIRE
401  NTGGFLSQAI KVSGLFMTNG VVVVSRYADG TMKCYRTVSP KKFYDGPLAI
451  LVSKSSASAA EIVAQTLQDY GVALVVGDEQ TYGKGTIQHQ TITGDASQDD
501  CFKVTVGKYY SPSGKSTQLQ GVKSDILIPS LYAEDRLGER FLEHPLPADC
551  CDNVLHDPLT DLDTQTRPWF QKYYLPNLQK QETLWREMLP QLTKNSEQRL
601  SENSNFQAFL SQIKSSEKTD LSYGSNDLQL EESINILKDM ILLQQCRK*
```

A predicted signal peptide is highlighted.

The cp6847 nucleotide sequence <SEQ ID 156> is:

```
   1 ATGTTCGTAA TGAAAAAACT TGTCCGTCTA TGCGTAGTTC TTCTTTCTTT
  51 ACTTCCGAAT GTATTATTTT CTTCGGATCT TTTACGAGAA GAGGGCATCA
 101 AAAAGATGAT GGACAAGCTG ATCGAGTATC ATGTCGATGC TCAAGAGGTT
 151 TCTACGGATA TACTCTCGCG TTCTTTATCT AGTTACATTC AATCTTTTGA
 201 TCCTCATAAA TCTTATCTTT CAAACCAAGA GGTTGCAGTT TTTCTACAGT
 251 CTCCGGAAAC AAAGAAACGT CTCTTAAAGA ATTATAAGGC AGGCAACTTT
 301 GCTATTTATC GCAACATCAA TCAATTAATT CATGAGAGTA TTCTTCGTGC
 351 CAGGCAGTGG AGAAACGAAT GGGTTAAGAA TCCAAAAGAG CTTGTATTGG
 401 AGGCATCCTC ATATCAGATA TCGAAGCAAC CTATGCAATG GAGCAAATCT
 451 TTAGACGAAG TGAAGCAGAG ACAACGCGCT CTACTCCTTT CCTATCTTTC
 501 TTTACATCTT GCTGGAGCTT CTTCCTCTCG TTATGAGGGT AAAGAAGAGC
 551 AGCTTGCTGC TCTGTGTCTA CGTCAAATCG AGAACCATGA GAATGTATAT
 601 TTAGGTATCA ACGATCATGG TGTTGCTATG GATCGGGATG AAGAAGCCTA
 651 CCAATTCCAT ATCCGTGTTG TTAAAGCTTT AGCTCATAGC TTAGATGCAC
 701 ATACGGCGTA TTTCAGTAAG GACGAAGCGT TGGCGATGCG AATCCAACTA
 751 GAAAAAGGCA TGTGTGGAAT TGGTGTTGTT CTGAAGGAAG ATATTGATGG
 801 AGTTGTTGTT AGAGAAATCA TTCCTGGGGG ACCTGCGGCT AAATCTGGGG
 851 ATCTTCAGCT TGGAGATATC ATCTATCGGG TGGATGGCAA GGATATCGAG
 901 CATCTTTCTT TCCGCGGTGT TTTAGATTGT TTACGTGGAG GTCATGGCTC
 951 TACTGTAGTC TTAGATATCC ATCGTGGGGA GAGCGATCAT ACGATCGCCT
1001 TGAGAAGGGA GAAAATCCTT TTAGAAGACC GTCGTGTGGA TGTTTCCTAT
1051 GAGCCTTATG GAGATGGTGT GATTGGGAAA GTTACGTTAC ATTCTTTTTA
1101 TGAAGGAGAA AATCAGGTTT CTAGTGAACA AGATCTACGT CGAGCGATTC
1151 AGGGATTAAA GGAGAAGAAC CTTCTTGGAT TAGTTTTAGA TATCCGAGAA
1201 AATACGGGTG GATTTTTATC TCAAGCGATC AAAGTTTCTG GTTTATTTAT
1251 GACCAATGGC GTTGTGGTTG TATCTCGCTA TGCTGATGGT ACCATGAAGT
1301 GCTACCGCAC AGTATCTCCT AAAAAATTCT ATGATGGTCC TTTGGCTATT
1351 TTAGTATCTA AAAGTTCCGC ATCAGCAGCG GAGATTGTAG CACAAACTCT
1401 CCAAGATTAT GGAGTTGCTT TAGTTGTTGG AGATGAGCAG ACCTATGGGA
1451 AGGGAACGAT TCAGCATCAA ACAATTACTG GAGATGCCTC TCAGGACGAT
1501 TGTTTTAAGG TTACTGTAGG GAAATATTAT TCCCCTTCTG GGAAATCGAC
1551 TCAACTTCAG GGAGTAAAAT CCGATATTTT AATTCCTTCT CTCTATGCTG
1601 AAGATCGTCT AGGAGAGCGT TTTCTAGAGC ATCCCTTACC TGCAGATTGC
1651 TGTGATAATG TACTTCACGA TCCTCTCACG GACTTGGATA CTCAAACACG
1701 TCCTTGGTTT CAAAAATACT ATCTTCCTAA TCTACAAAAG CAAGAGACTC
1751 TTTGGAGAGA GATGCTACCT CAGCTTACGA AAAACAGTGA GCAAAGGCTT
1801 TCTGAGAATT CGAATTTTCA GGCATTTTTG TCGCAGATAA AATCATCTGA
1851 AAAAACGGAC CTATCCTATG GTTCCAATGA TTTACAATTG GAAGAGTCGA
1901 TAAACATTTT GAAGGACATG ATTTTATTAC AACAGTGTAG AAAATAA
```

The PSORT algorithm predicts periplasmic (0.932).

The protein was expressed in *E.coli* and purified as a GST-fusion product (Figure 78A). The recombinant protein was used to immunise mice, whose sera were used in a Western blot (Figure 78B) and for FACS analysis.

These experiments show that cp6847 is a surface-exposed and immunoaccessible protein, and that it is a useful immunogen. These properties are not evident from the sequence alone.

**Example 79**

The following *C.pneumoniae* protein (PID 4376969) was expressed <SEQ ID 157; cp6969>:

```
  1 MRLFSLGTIY LFFSLALSSC CGYSILNSPY HLSSLGKSLL QERIFIAPIK
 51 EDPHGQLCSA LTYELSKRSF AISGRSSCAG YTLKVELLNG IDKNIGFTYA
101 PNKLGDKTHR HFIVSNEGRL SLSAKVQLIN NDTQEVLIDQ CVARESVDFD
151 FEPDLGTANA HEFALGQFEM HSEAIKSARR ILSIRLAETI AQQVYYDLF*
```

A predicted signal peptide is highlighted.

The cp6969 nucleotide sequence <SEQ ID 158> is:

```
  1  ATGAGATTGT TTTCTTTAGG CACGATTTAT CTTTTTTTTT CTCTAGCACT
 51  TTCGTCATGC TGTGGTTACT CTATTTTAAA CAGCCCGTAT CACTTATCGT
101  CTTTAGGTAA GTCTTTATTA CAGGAAAGAA TTTTCATTGC TCCCATAAAA
```

```
151  GAAGATCCTC ATGGTCAGCT CTGCTCAGCT CTAACTTATG AGCTTAGTAA
201  GCGTTCTTTT GCTATCTCTG GAAGGAGTTC TTGCGCAGGC TATACTCTTA
251  AAGTAGAGCT TCTGAATGGT ATTGACAAGA ATATAGGTTT TACGTATGCC
301  CCAAATAAAC TCGGAGATAA GACTCACAGG CATTTTATAG TCTCTAATGA
351  AGGCAGACTA TCACTATCTG CAAAAGTACA GCTTATCAAT AATGACACTC
401  AAGAAGTCCT TATAGACCAA TGTGTTGCTC GAGAGTCTGT AGACTTTGAC
451  TTTGAGCCTG ACTTAGGAAC AGCAAACGCT CATGAATTTG CTTTAGGCCA
501  ATTTGAAATG CATAGTGAAG CCATAAAAAG TGCTCGCCGT ATACTATCTA
551  TACGCCTAGC CGAGACGATT GCTCAACAGG TATACTATGA CCTTTTTTGA
```

The PSORT algorithm predicts inner membrane (0.126).

The protein was expressed in *E.coli* and purified as a GST-fusion product (Figure 79A). The recombinant protein was used to immunise mice, whose sera were used in a Western blot (Figure 79B) and for FACS analysis.

These experiments show that cp6969 is a surface-exposed and immunoaccessible protein, and that it is a useful immunogen. These properties are not evident from the sequence alone.

**Example 80**

The following *C.pneumoniae* protein (PID 4377109) was expressed <SEQ ID 159; cp7109>:

```
  1  MKKTCCQNYR SIGVVFSVVL FVLTTQTLFA GHFIDIGTSG LYSWARGVSG
 51  DGRVVVGYEG GNAFKYVDGE KFLLEGLVPR SEALVFKASY DGSVIIGISD
101  QDPSCRAVKW VNGALVDLGI FSEGMQSFAE GVSSDGKTIV GCLYSDDTET
151  NFAVKWDETG MVVLPNLPED RHSCAWDASE DGSVIVGDAM GSEEIAKAVY
201  WKDGEQHLLS NIPGAKRSSA HAVSKDGSFI VGEFISEENE VHAFVYHNGV
251  IKDIGTLGGD YSVATGVSRD GKVIVGHSTR TDGEYRAFKY VDGRMIDLGT
301  LGGSASFAFG VSDDGKTIVG KFETELGECH AFIYLDD*
```

A predicted signal peptide is highlighted.

The cp7109 nucleotide sequence <SEQ ID 160> is:

```
   1  ATGAAAAAGA CATGTTGCCA AAATTACAGA TCGATAGGCG TTGTGTTCTC
  51  TGTGGTACTT TTCGTTCTTA CAACACAGAC GCTGTTTGCA GGACATTTTA
 101  TTGATATTGG AACTTCTGGA TTATATTCTT GGGCTCGAGG TGTATCTGGA
 151  GATGGCCGCG TTGTCGTAGG TTATGAAGGT GGCAATGCAT TTAAATATGT
 201  TGATGGTGAG AAATTTCTGT TAGAAGGTTT GGTCCCGAGA TCCGAGGCCT
 251  TGGTATTTAA AGCTTCTTAT GATGGCTCTG TAATTATAGG AATCTCGGAT
 301  CAAGATCCGT CTTGCCGCGC TGTGAAGTGG GTAAACGGTG CACTTGTTGA
 351  TCTTGGAATA TTTTCTGAGG GAATGCAATC TTTTGCAGAG GGTGTTTCCA
 401  GTGATGGAAA GACGATTGTA GGGTGCCTAT ATAGTGATGA TACAGAGACA
 451  AACTTTGCTG TGAAGTGGGA TGAAACAGGA ATGGTTGTTC TCCCTAACTT
 501  ACCAGAAGAT CGACATTCTT GCGCTTGGGA TGCCTCTGAA GATGGCTCTG
 551  TGATTGTAGG GGACGCCATG GGTAGCGAGG AAATTGCCAA GGCAGTGTAC
 601  TGGAAGGACG GTGAACAACA TCTGCTTTCT AATATCCCAG GAGCTAAAAG
 651  ATCGTCAGCA CATGCAGTTT CTAAAGATGG ATCTTTTATC GTAGGCGAGT
 701  TCATCAGTGA AGAAAATGAA GTTCATGCCT TTGTTTATCA CAACGGTGTT
 751  ATCAAAGATA TCGGGACTTT AGGAGGAGAT TACTCTGTAG CAACTGGAGT
 801  TTCTAGGGAT GGTAAGGTCA TCGTGGGTCA TTCTACAAGA ACAGATGGTG
 851  AATACCGTGC ATTTAAATAT GTGGATGGAA GAATGATAGA TTTGGGGACT
 901  TTAGGAGGTT CAGCATCTTT TGCTTTTGGT GTTTCTGACG ATGGCAAAAC
 951  AATCGTAGGA AAATTTGAAA CAGAGCTAGG AGAATGTCAT GCCTTTATCT
1001  ACCTTGATGA TTAG
```

The PSORT algorithm predicts outer membrane (0.887).

The protein was expressed in *E.coli* and purified as a GST-fusion product (Figure 80A). The recombinant protein was used to immunise mice, whose sera were used in a Western blot (Figure 80B) and for FACS analysis.

These experiments show that cp7109 is a surface-exposed and immunoaccessible protein, and that it is a useful immunogen. These properties are not evident from the sequence alone.

### Example 81

The following *C.pneumoniae* protein (PID 4377110) was expressed <SEQ ID 161; cp7110>:

```
  1  MAAIKQILRS MLSQSSLWMV LFSLYSLSGY CYVITDKPED DFHSSSAVKW
 51  DHWGKTTLSR LSNKKASAKA VSGTGATTVG FIKDTWSRTY AVRWNYWGTK
101  ELPTSSWVKK SKATGISSDG SIIAGIVENE LSQSFAVTWK NNEMYLLPST
151  WAVQSKAYGI SSDGSVIVGS AKDAWSRTFA VKWTGHEAQV LPVGWAVKSV
201  ANSVSANGSI IVGSVQDASG ILYAVKWEGN TITHLGTLGG YSAIAKAVSN
251  NGKVIVGRSE TYYGEVHAFC HKNGVMSDLG TLGGSYSAAK GVSATGKVIV
301  GMSTTANGKL HAFKYVGGRM IDLGEYSWKE ACANAVSIDG EIIVGVQSE*
```

A predicted signal peptide is highlighted.

The cp7110 nucleotide sequence <SEQ ID 162> is:

```
   1  ATGGCAGCTA TAAAACAAAT TTTACGTTCT ATGCTATCTC AGAGTAGCTT
  51  ATGGATGGTC CTATTTTCAT TATATTCTCT ATCTGGTTAT TGCTATGTAA
 101  TTACAGACAA ACCAGAAGAT GACTTCCATT CTTCATCCGC AGTAAAATGG
 151  GATCATTGGG GAAAGACAAC TCTCTCAAGA TTATCAAATA AAAAAGCCTC
 201  TGCAAAAGCT GTTTCAGGAA CTGGTGCTAC AACTGTCGGC TTTATAAAAG
 251  ACACTTGGTC TCGAACATAC GCAGTAAGAT GGAATTATTG GGGGACCAAA
 301  GAACTCCCTA CCAGCTCATG GGTAAAAAAA TCAAAAGCAA CAGGAATCTC
 351  CTCTGATGGG TCTATAATCG CGGGGATTGT CGAGAATGAG CTTTCTCAAA
 401  GTTTCGCAGT CACATGGAAA AACAATGAAA TGTATTTGCT CCCTTCCACA
 451  TGGGCAGTGC AATCTAAAGC GTATGGAATT TCTTCTGATG GCTCTGTTAT
 501  TGTAGGGAGT GCTAAGGATG CTTGGTCGCG AACTTTCGCT GTGAAGTGGA
 551  CGGGACACGA GGCTCAGGTG TTACCAGTAG GCTGGGCTGT CAAATCTGTA
 601  GCGAATTCTG TATCTGCCAA TGGATCTATA ATTGTAGGGT CTGTACAAGA
 651  CGCCTCTGGA ATTCTTTATG CTGTAAAGTG GGAAGGGAAC ACTATTACAC
 701  ATCTAGGAAC TTTAGGAGGC TATTCTGCCA TTGCAAAAGC TGTATCCAAT
 751  AATGGCAAGG TCATTGTAGG GAGATCCGAA ACATATTATG GAGAGGTCCA
 801  TGCTTTCTGT CATAAGAATG GCGTCATGTC AGACCTCGGC ACCCTCGGAG
 851  GATCTTATTC TGCAGCTAAG GGAGTCTCTG CAACTGGAAA AGTTATTGTC
 901  GGTATGTCCA CAACAGCAAA TGGGAAATTG CATGCCTTTA AATATGTCGG
 951  TGGAAGAATG ATCGACTTAG GAGAGTATAG CTGGAAAGAA GCCTGTGCAA
1001  ACGCTGTTTC TATTGATGGA GAAATTATTG TTGGAGTCCA ATCAGAATAA
```

The PSORT algorithm predicts outer membrane (0.827).

The protein was expressed in *E.coli* and purified as a GST-fusion product (Figure 81A). The recombinant protein was used to immunise mice, whose sera were used in a Western blot (Figure 81B) and for FACS analysis.

These experiments show that cp7110 is a surface-exposed and immunoaccessible protein, and that it is a useful immunogen. These properties are not evident from the sequence alone.

Figure 191 shows a schematic representation of the structural relationships between of cp7105, cp7106, cp7107, cp7108, cp7109 and cp7110, each of which is identified herein. These six proteins may be grouped in a new family of related outer membrane-associated proteins. These proteins have a repeat structure in common (*cf.* the pmp family).

**Example 82**

The following *C.pneumoniae* protein (PID 4377127) was expressed <SEQ ID 163; cp7127>:

```
   1  MVFFRNSLLH LVALSGMLCC SSGVALTIAE KMASLEHSGR GADDYEGMAS

  51  FNANMREYSL QLSKLYEEAR KLRASGTEDE ALWKDLIRRI GEVRGYLREI
 101  EELWAAEIRE KGGNLEDYAL WNHPETTIYN LVTDYGTEDS IYLIPQEIGA
 151  IKIATLSKFV VPKESFEDCL TQILSRLGIG VRQVNSWIKE LYMMRKEGCS
 201  VAGVFSSRKD LEALPETAYI GFVLNSNVDA HTNQHVLKKF INPETTHVDV
 251  IAGRVWIFGS AGEVGELLKI YNFVQSESIR QEYRVIPLTK IDPGEMISIL
 301  NAAFREDLTK DVSEESLGLR VVPLQYQGRS LFLSGTAALV QQALTLIREL
 351  EEGIENPTDK TVFWYNVKHS DPQELAALLS QVHDVFSGEN KASVGAADGC
 401  GSQLNASIQI DTTVSSSAKD GSVKYGNFIA DSKTGTLIMV VEKEVLPRIQ
 451  MLLKKLDVPK KMVRIEVLLF ERKLAHEQKS GLNLLRLGEE VCKKGCSPSV
 501  SWAGGTGILE FLFKGSTGSS IVPGYDLAYQ FLMAQEDVRI NASPSVVTMN
 551  QTPARIAVVD EMSIAVSSDK DKAQYNRAQY GIMIKMLPVI NVGEEDGKSY
 601  ITLETDITFD TTGKNHDDRP DVTRRNITNK VRIADGETVI IGGLRCKQMS
 651  DSHDGIPFLG DIPGIGKLFG MSSTSDSLTE MFVFITPKIL ENPVEQQERK
 701  EEALLSSRPG EREEYYQALA ASEAAARAAH KKLEMFPASG VSLSQVERQE
 751  YDGC*
```

A predicted signal peptide is highlighted.

The cp7127 nucleotide sequence <SEQ ID 164> is:

```
   1  ATGGTTTTTT TCCGTAATTC TTTACTGCAT TTAGTTGCCC TATCCGGAAT
  51  GCTCTGTTGT TCTTCTGGAG TGGCTTTAAC GATAGCCGAG AAGATGGCTT
 101  CTTTAGAGCA CTCGGGGAGA GGAGCAGACG ATTATGAGGG GATGGCTTCG
 151  TTTAATGCCA ATATGAGGGA GTATAGCCTT CAGCTGAGCA AGTTGTATGA
 201  GGAAGCACGA AAGCTACGCG CTTCTGGAAC TGAGGATGAA GCTCTGTGGA
 251  AGGACTTAAT TCGACGGATT GGTGAGGTGC GAGGCTATCT TCGAGAGATC
 301  GAGGAGCTTT GGGCTGCAGA AATTCGTGAG AAAGGGGGCA ATCTCGAGGA
 351  CTACGCCCTC TGGAATCACC CAGAGACTAC GATTTACAAT CTTGTTACCG
 401  ATTACGGAAC CGAAGACTCT ATTTATTTGA TTCCTCAAGA AATCGGAGCG
 451  ATTAAAATCG CAACCTTATC GAAATTTGTA GTTCCTAAAG AGTCTTTCGA
 501  AGACTGTCTC ACTCAGATCC TATCTCGCTT AGGTATTGGC GTGCGTCAGG
 551  TCAATTCTTG GATTAAGGAA CTTTATATGA TGCGTAAGGA GGGCTGCAGT
 601  GTTGCTGGAG TTTTTTCCTC CAGAAAAGAT TTAGAGGCGC TCCCAGAAAC
 651  AGCCTATATT GGTTTTGTAT TGAATTCGAA CGTAGATGCG CATACCAATC
 701  AACATGTCTT AAAAAAGTTC ATTAACCCTG AAACAACGCA TGTAGATGTG
 751  ATTGCAGGAC GTGTGTGGAT TTTTGGTTCT GCGGGGGAAG TCGGCGAGCT
 801  TCTGAAGATT TATAATTTTG TGCAGTCGGA GAGCATACGT CAAGAGTATC
 851  GGGTGATTCC CTTAACTAAG ATCGATCCAG GGGAGATGAT TTCCATTCTC
 901  AACGCAGCAT TTCGTGAGGA TCTGACTAAA GATGTTAGTG AAGAATCTTT
 951  AGGCCTTCGT GTAGTTCCTT TACAGTATCA AGGGCGTTCG TTGTTTTTAA
1001  GTGGAACCGC GGCGTTAGTG CAGCAAGCGC TGACTCTCAT TCGAGAGCTT
1051  GAAGAAGGGA TTGAGAACCC TACGGATAAA ACAGTATTTT GGTATAACGT
1101  CAAGCACTCC GATCCCCAAG AGTTGGCGGC ATTGCTTTCC CAAGTCCATG
1151  ATGTCTTCTC TGGCGAGAAT AAGGCGAGTG TCGGAGCTGC AGATGGATGT
1201  GGGTCGCAAT TAAATGCCTC GATCCAAATT GATACTACAG TAAGTTCTTC
1251  TGCGAAAGAT GGCTCAGTGA AGTACGGAAA CTTCATCGCG GATTCTAAGA
1301  CAGGAACTCT GATTATGGTG GTTGAGAAAG AAGTTCTTCC ACGTATTCAG
1351  ATGCTACTTA AGAAACTAGA TGTCCCTAAA AAGATGGTCC GTATCGAGGT
1401  GCTGTTATTT GAAAGAAAAT TGGCACATGA GCAGAAATCT GGGTTAAATC
1451  TTCTACGTCT TGGTGAGGAA GTTTGTAAAA AAGGGTGCAG TCCTTCTGTG
1501  TCTTGGGCCG GGGGTACTGG CATACTAGAA TTTTTATTTA AAGGAAGTAC
1551  GGGATCTTCG ATAGTTCCTG GTTATGATCT CGCCTATCAA TTTTTAATGG
1601  CTCAAGAGGA CGTTCGGATT AATGCGAGTC CTTCTGTAGT TACTATGAAC
1651  CAAACCCCAG CACGGATTGC TGTTGTTGAT GAAATGTCAA TAGCGGTGTC
1701  TTCAGATAAA GATAAAGCGC AATACAATCG TGCGCAGTAC GGTATCATGA
1751  TAAAAATGCT CCCCGTAATT AATGTGGGAG AGGAAGACGG AAAAAGTTAC
1801  ATTACTTTAG AGACAGACAT CACCTTTGAT ACTACGGGAA AAAATCATGA
1851  TGATCGTCCT GATGTTACAA GGCGTAATAT TACTAATAAG GTGCGCATTG
1901  CTGACGGAGA GACTGTGATT ATTGGAGGTT TGCGTTGCAA ACAGATGTCA
1951  GATTCTCATG ATGGCATTCC TTTCCTTGGA GACATTCCTG GTATAGGGAA
2001  GTTATTTGGA ATGAGTTCCA CATCAGACAG TCTCACGGAG ATGTTTGTAT
2051  TTATCACTCC GAAGATCCTA GAAAATCCTG TAGAGCAACA AGAACGTAAA
2101  GAAGAAGCTT TACTCTCTTC GCGCCCTGGA GAGAGAGAAG AATACTATCA
2151  GGCTTTAGCA GCTAGTGAGG CTGCAGCACG AGCAGCTCAT AAAAAATTAG
2201  AGATGTTCCC GGCATCAGGA GTATCTTTAT CTCAGGTAGA GAGGCAAGAA
2251  TACGATGGCT GCTAG
```

The PSORT algorithm predicts periplasmic (0.920).

The protein was expressed in *E.coli* and purified as a GST-fusion product (Figure 82A) and also in his-tagged form. The recombinant proteins were used to immunise mice, whose sera were used in a Western blot (Figure 82B) and for FACS analysis.

These experiments show that cp7127 is a surface-exposed and immunoaccessible protein, and that it is a useful immunogen. These properties are not evident from the sequence alone.

## Example 83

The following *C.pneumoniae* protein (PID 4377133) was expressed <SEQ ID 165; cp7133>:

```
  1 MQPFIFTLLC LTSLVSLVAF DAANARKRCA CAQTIERGEN FFSIKRSACA
 51 EIEYQEKSRH ASAIERISKD KGKVTPKQIA KVATKKKQRY RLLQVPFSRP
101 PNNSRYNLYA LLSEPPECYS DTASWYAIFI RLLRRAYVDT GNVPPGSEYA
151 IANALISNKQ EILERGAQLG PDVIETLTLP EEQAEIFYKM LKGSSNSQSL
201 LNFLHYEEKS LGHCKLNLIF MDPLLLEAVL DHPDAYRETS LLRDGIWEAV
251 KRQEHAIQEH GQAAALELFK TRTDFRLELR DKMQLLLSRY DLLPLLNKKM
301 FDYTLGSAGD YLFLVDPDTK AISRCRCPSK SIKL
```

A predicted signal peptide is highlighted.

The cp7133 nucleotide sequence <SEQ ID 166> is:

```
   1 ATGCAACCTT TTATCTTTAC TTTACTGTGC TTGACATCTT TGGTTTCTTT
  51 AGTCGCCTTT GATGCTGCGA ATGCTCGTAA ACGTTGTGCC TGTGCTCAAA
 101 CTATAGAACG TGGAGAGAAC TTCTTTTCCA TAAAACGCTC TGCTTGTGCT
 151 GAAATCGAAT ATCAAGAAAA ATCTCGCCAC GCCTCAGCAA TTGAAAGAAT
 201 CTCAAAAGAT AAAGGCAAAG TCACTCCAAA GCAGATTGCG AAAGTAGCTA
 251 CTAAGAAAAA GCAAAGATAC CGTTTATTGC AGGTTCCTTT TTCAAGGCCT
 301 CCGAATAACT CAAGGTATAA CCTCTATGCT TTGCTTAGTG AACCTCCCGA
 351 ATGCTATAGC GATACAGCAT CATGGTATGC TATTTTTATT CGGTTACTTC
 401 GACGTGCTTA TGTAGACACG GGAAATGTAC CTCCTGGATC TGAGTATGCC
 451 ATCGCTAATG CTTTGATAAG TAACAAACAA GAGATTTTAG AGAGGGGAGC
 501 GCAGCTTGGA CCCGATGTTA TTGAAACTCT AACATTGCCT GAGGAACAAG
 551 CCGAGATTTT TTATAAAATG CTCAAAGGGT CGTCAAACTC TCAGTCGCTA
 601 CTGAATTTTC TGCATTATGA AGAGAAAAGC TTAGGCCACT GTAAGCTAAA
 651 TCTGATCTTC ATGGATCCCC TACTGTTAGA AGCTGTTCTA GATCATCCCG
 701 ATGCTTATAG GGAAACGTCG CTCCTGCGCG ATGGCATTTG GGAAGCGGTG
 751 AAGCGTCAAG AACATGCCAT CCAAGAACAT GGCCAGGCAG CTGCTTTGGA
 801 GCTTTTTAAA ACACGCACCG ACTTCCGCCT GGAGCTGCGA GATAAGATGC
 851 AGTTACTTCT AAGTCGATAC GATTTGCTCC CCTTATTAAA TAAAAAAATG
 901 TTCGACTACA CCTTAGGAAG TGCCGGAGAT TACTTATTTT TGGTAGACCC
 951 AGATACTAAG GCAATTTCTC GATGTCGCTG CCCTTCAAAG AGTATTAAAT
1001 TATAA
```

The PSORT algorithm predicts outer membrane (0.92).

The protein was expressed in *E.coli* and purified as a GST-fusion product (Figure 83A) and also in his-tagged form. The recombinant proteins were used to immunise mice, whose sera were used in a Western blot (Figure 83B) and for FACS analysis.

These experiments show that cp7133 is a surface-exposed and immunoaccessible protein, and that it is a useful immunogen. These properties are not evident from the sequence alone.

### Example 84

The following *C.pneumoniae* protein (PID 4377222) was expressed <SEQ ID 167; cp7222>:

```
  1 MNRRDMVITA VVVNAILLVA LFVTSKRIGV KDYDEGFRNF ASSKVTQAVV
 51 SEEKVIEKPV VAEVPSRPIA KETLAAQFIE SKPVIVTTPP VPVVSETPEV

101 PTVAVPPQPV RETVKEEQAP YATVVVKKGD FLERIARANH TTVAKLMQIN
151 DLTTTQLKIG QVIKVPTSQD VSNEKTPQTQ TANPENYYIV QEGDSPWTIA
201 LRNHIRLDDL LKMNDLDEYK ARRLKPGDQL RIR*
```

A predicted signal peptide is highlighted.

The cp7222 nucleotide sequence <SEQ ID 168> is:

```
  1  ATGAATCGTA GAGACATGGT AATAACAGCT GTCGTAGTGA ATGCTATATT
 51  GCTTGTGGCT CTTTTCGTCA CATCAAAGCG TATTGGCGTC AAGGACTATG
101  ACGAGGGATT CCGTAATTTT GCTTCTAGCA AGGTTACACA AGCAGTAGTT
151  TCAGAAGAAA AAGTCATAGA AAAGCCTGTA GTCGCAGAAG TGCCTAGCCG
201  TCCTATCGCT AAAGAGACTC TAGCTGCACA GTTTATTGAA AGTAAGCCGG
251  TTATTGTAAC CACACCACCC GTGCCTGTTG TTAGCGAAAC CCCAGAAGTG
301  CCTACTGTGG CAGTTCCGCC TCAGCCTGTT CGTGAGACAG TAAAAGAGGA
351  ACAAGCTCCT TATGCTACTG TTGTAGTGAA AAAAGGAGAT TTTCTCGAAC
401  GCATTGCGAG AGCAAATCAT ACTACCGTTG CAAAATTGAT GCAGATCAAT
451  GATCTTACCA CCACCCAACT TAAAATTGGT CAGGTCATCA AAGTCCCTAC
501  GTCTCAAGAT GTCAGCAACG AAAAAACTCC TCAAACACAG ACCGCAAACC
551  CTGAAAATTA TTATATCGTC CAAGAAGGGG ATAGCCCGTG GACAATAGCA
601  TTGCGTAACC ATATTCGATT GGATGATTTG CTAAAAATGA ATGATCTCGA
651  TGAATATAAA GCCCGGCGCC TTAAGCCTGG AGATCAGTTG CGCATACGTT
701  GA
```

The PSORT algorithm predicts periplasmic (0.935).

The protein was expressed in *E.coli* and purified as a GST-fusion product (Figure 84A) and also in his-tagged form. The recombinant proteins were used to immunise mice, whose sera were used in a Western blot (Figure 84B) and for FACS analysis.

These experiments show that cp7222 is a surface-exposed and immunoaccessible protein, and that it is a useful immunogen. These properties are not evident from the sequence alone.

**Example 85**

The following *C.pneumoniae* protein (PID 4377225) was expressed <SEQ ID 169; cp7225>:

```
  1  MKGTPQYHFI GIGGIGMSAL AHILLDRGYE VSGSDLYESY TIESLKAKGA
 51  RCFSGHDSSH VPHDAVVVYS SSIAPDNVEY LTAIQRSSRL LHRAELLSQL
101  MEGYESILVS GSHGKTGTSS LIRAIFQEAQ KDPSYAIGGL AANCLNGYSG
151  SSKIFVAEAD ESDGSLKHYT PRAVVITNID NEHLNNYAGN LDNLVQVIQD
201  FSRKVTDLNK VFYNGDCPIL KGNVQGISYG YSPECQLHIV SYNQKAWQSH
251  FSFTFLGQEY QDIELNLPGQ HNAANAAAAC GVALTFGIDI NIIRKALKKF
301  SGVHRRLERK NISESFLFLE DYAHHPVEVA HTLRSVRDAV GLRRVIAIFQ
351  PHRFSRLEEC LQTFPKAFQE ADEVILTDVY SAGESPRESI ILSDLAEQIR
401  KSSYVHCCYV PHGDIVDYLR NYIRIHDVCV SLGAGNIYTI GEALKDFNPK
451  KLSIGLVCGG KSCEHDISLL SAQHVSKYIS PEFYDVSYFI INRQGLWRTG
501  KDFPHLIEET QGDSPLSSEI ASALAKVDCL FPVLHGPFGE DGTIQGFFEI
551  LGKPYAGPSL SLAATAMDKL LTKRIASAVG VPVVPYQPLN LCFWKRNPEL
601  CIQNLIETFS FPMIVKTAHL GSSIGIFLVR DKEELQEKIS EAFLYDTDVF
651  VEESRLGSRE IEVSCIGHSS SWYCMAGPNE RCGASGFIDY QEKYGFDGID
701  CAKISFDLQL SQESLDCVRE LAERVYRAMQ GKGSARIDFF LDEEGNYWLS
751  EVNPIPGMTA ASPFLQAFVH AGWTQEQIVD HFIIDALHKF DKQQTIEQAF
801  TKEQDLVKR*
```

The cp7225 nucleotide sequence <SEQ ID 170> is:

```
  1  ATGAAGGGAA CTCCTCAGTA TCATTTTATC GGTATCGGTG GTATAGGAAT
 51  GAGCGCTTTA GCTCATATTT TGCTTGATCG TGGCTATGAG GTCTCTGGAA
101  GCGACTTATA TGAAAGCTAT ACGATCGAAA GCCTGAAAGC TAAAGGTGCG
151  AGGTGTTTCT CAGGCCATGA TTCCTCCCAT GTTCCTCATG ATGCCGTCGT
201  TGTTTATAGC TCAAGTATAG CCCCTGATAA TGTAGAGTAT CTTACCGCTA
251  TTCAAAGATC ATCACGTCTT CTTCATAGAG CAGAGCTCTT GAGTCAGCTT
301  ATGGAGGGTT ATGAAAGCAT TCTGGTTTCA GGAAGCCATG GGAAGACAGG
351  GACCTCATCT CTAATTCGAG CGATTTTCCA GGAAGCTCAG AAAGATCCCT
```

```
 401 CCTATGCTAT TGGAGGACTC GCTGCAAACT GCCTGAATGG GTATTCTGGA
 451 TCATCGAAAA TCTTCGTTGC CGAAGCCGAT GAAAGTGATG GGTCTTTAAA
 501 GCACTACACT CCCCGTGCAG TAGTCATTAC AAATATAGAT AATGAACATT
 551 TGAATAATTA CGCTGGGAAT CTTGATAACC TGGTTCAGGT AATCCAGGAC
 601 TTCTCTAGAA AAGTAACAGA TCTCAATAAG GTATTCTATA ACGGGGATTG
 651 TCCTATTTTG AAAGGAAATG TCCAAGGGAT TTCTTATGGA TATTCACCAG
 701 AATGTCAATT GCATATCGTT TCCTATAATC AAAAGGCATG GCAATCTCAC
 751 TTTTCCTTTA CCTTTTTAGG CCAGGAGTAT CAAGACATTG AGCTCAATCT
 801 CCCTGGACAA CATAACGCTG CAAATGCAGC AGCAGCCTGT GGAGTTGCTC
 851 TTACCTTTGG CATAGACATA AACATCATTC GAAAAGCTCT CAAAAAATTC
 901 TCGGGAGTTC ATCGACGTCT AGAAAGAAAA AATATATCCG AAAGCTTTCT
 951 TTTCTTAGAA GATTATGCTC ATCATCCTGT AGAGGTTGCA CATACCCTGC
1001 GCTCTGTGCG TGATGCTGTG GGTTTGCGAA GAGTCATCGC AATTTTTCAA
1051 CCACATCGAT TCTCTCGTTT AGAAGAGTGC TTACAAACCT TCCCCAAAGC
1101 TTTCCAAGAA GCTGATGAAG TCATACTTAC AGATGTCTAT AGTGCCGGAG
1151 AAAGTCCTAG AGAGTCTATC ATTCTTTCCG ACCTTGCGGA ACAGATTCGT
1201 AAGTCTTCTT ATGTCCATTG TTGTTATGTT CCCCATGGAG ACATCGTAGA
1251 TTATCTACGA AACTACATTC GCATTCATGA TGTCTGTGTT TCTCTAGGAG
1301 CTGGAAATAT CTATACTATT GGAGAGGCTT TAAAAGACTT TAACCCTAAA
1351 AAATTATCCA TAGGACTCGT CTGTGGAGGG AAATCTTGCG AACACGATAT
1401 TTCTCTACTT TCTGCTCAAC ATGTCTCTAA ATATATTTCT CCTGAATTCT
1451 ATGATGTGAG TTACTTCATC ATAAATCGTC AGGGCTTATG GAGAACAGGA
1501 AAGGATTTTC CTCATCTTAT TGAAGAGACT CAAGGGGATT CGCCACTTTC
1551 TTCTGAAATC GCTTCAGCTT TAGCAAAAGT CGACTGTTTG TTTCCCGTGC
1601 TCCATGGCCC ATTTGGAGAG GATGGTACGA TCCAGGGATT TTTTGAAATC
1651 TTAGGAAAAC CTTATGCCGG ACCCTCACTA TCTTTAGCAG CAACTGCAAT
1701 GGATAAGCTG TTAACAAAAC GAATTGCATC AGCAGTGGGT GTTCCTGTAG
1751 TCCCTTACCA ACCTTTAAAT CTCTGTTTCT GGAAACGCAA TCCAGAACTA
1801 TGTATTCAGA ATCTTATAGA GACATTTTCT TTCCCTATGA TTGTAAAAAC
1851 TGCACATTTG GGATCTAGTA TTGGGATATT TTTAGTCCGT GATAAAGAGG
1901 AATTACAAGA AAAGATCTCA GAAGCATTTC TATATGACAC GGATGTGTTT
1951 GTGGAGGAAA GTCGCTTAGG GTCTCGTGAA ATCGAAGTGT CCTGTATCGG
2001 CCATTCTTCT AGCTGGTATT GTATGGCAGG GCCTAATGAA CGCTGTGGTG
2051 CTAGTGGGTT TATTGATTAT CAAGAGAAAT ATGGATTTGA TGGCATAGAT
2101 TGCGCAAAGA TCTCTTTTGA TTTACAGCTC TCACAAGAAT CTTTAGATTG
2151 TGTTAGAGAA CTTGCAGAGC GTGTCTACCG AGCAATGCAA GGAAAAGGTT
2201 CAGCTCGAAT AGATTTTTTC TTGGATGAAG AGGGGAATTA TTGGTTGTCA
2251 GAGGTCAATC CTATTCCAGG AATGACAGCA GCTAGCCCAT TTTTACAAGC
2301 TTTTGTTCAC GCAGGATGGA CGCAAGAACA AATTGTAGAT CACTTTATTA
2351 TAGATGCTCT ACATAAGTTT GATAAGCAGC AGACTATCGA ACAGGCATTC
2401 ACTAAAGAAC AAGATTTAGT TAAAAGATAA
```

The PSORT algorithm predicts inner membrane (0.16).

The protein was expressed in *E.coli* and purified as a his-tag product (Figure 85A). The recombinant protein was used to immunise mice, whose sera were used in a Western blot (Figure 85B) and for FACS analysis.

These experiments show that cp7225 is a surface-exposed and immunoaccessible protein, and that it is a useful immunogen. These properties are not evident from the sequence alone.

## Example 86

The following *C.pneumoniae* protein (PID 4377248) was expressed <SEQ ID 171; cp7248>:

```
  1 MKFWLQGCAF VGCLLLTLPC CAARRRASGE NLQQTRPIAA ANLQWESYAE
 51 ALEHSKQDHK PICLFFTGSD WCMWCIKMQD QILQSSEFKH FAGVHLHMVE
101 VDFPQKNHQP EEQRQKNQEL KAQYKVTGFP ELVFIDAEGK QLARMGFEPG
151 GGAAYVSKVK SALKLR*
```

A predicted signal peptide is highlighted.

The cp7248 nucleotide sequence <SEQ ID 172> is:

```
  1 ATGAAATTTT GGTTGCAAGG ATGTGCTTTT GTCGGTTGTC TGCTATTGAC
 51 TTTACCTTGT TGTGCTGCAC GAAGACGTGC TTCTGGAGAA AATTTGCAAC
101 AAACTCGTCC TATAGCAGCT GCAAATCTAC AATGGGAGAG CTATGCAGAA
151 GCTCTTGAAC ATTCTAAACA AGATCACAAA CCTATTTGTC TTTTCTTTAC
201 AGGATCAGAC TGGTGTATGT GGTGCATAAA AATGCAAGAC CAGATTTTGC
251 AAAGCTCTGA GTTTAAGCAT TTTGCGGGTG TGCATCTGCA TATGGTTGAA
301 GTTGATTTCC CCCAAAAGAA TCATCAACCT GAAGAGCAGC GCCAAAAAAA
351 TCAAGAACTG AAAGCTCAAT ATAAAGTTAC AGGATTCCCC GAACTGGTCT
401 TCATAGATGC AGAAGGAAAA CAGCTTGCTC GCATGGGATT TGAGCCTGGT
451 GGTGGAGCTG CTTACGTAAG CAAGGTGAAG TCTGCTCTTA AACTACGTTA
501 A
```

The PSORT algorithm predicts periplasmic (0.932).

The protein was expressed in *E.coli* and purified as a GST-fusion product (Figure 86A) and also in his-tagged form. The recombinant proteins were used to immunise mice, whose sera were used in a Western blot (Figure 86B) and for FACS analysis.

The cp7248 protein was also identified in the 2D-PAGE experiment.

These experiments show that cp7248 is a surface-exposed and immunoaccessible protein, and that it is a useful immunogen. These properties are not evident from the sequence alone.

**Example 87**

The following *C.pneumoniae* protein (PID 4377249) was expressed <SEQ ID 173; cp7249>:

```
  1 MIPSPTPINF RDDTILETDP KPSLIMFSSK KTEIASERRK AHPTLFKVLG
 51 TIWNIVKFII SIILFLPLAL LWVLKKTCQF FILPSSIISQ SMSKTAVAIR
101 RMTFLSHIKQ LLSLKEISAA DRVVIQYDDL VVDSLAIKIP HALPHRWILY
151 SQGNSGLMEN LFDRGDSSLH QLAKATGSNL LVFNYPGIMS SKGEAKRENL
201 VKSYQACVRY LRDEETGPKA NQIIAFGYSL GTSVQAAALD REVTDGSDGT
251 SWIVVKDRGP RSLADVANQI CKPIASAIIK LVGWNIDSVK PSERLRCPEI
301 FIYNSNHDQE LISDGLFERE NCVATPFLEL PEVKTSGTKI PIPERDLLHL
351 NPLSPNVVDR LAAVISNYLD SENRKSQQPD *
```

The cp7249 nucleotide sequence <SEQ ID 174> is:

```
   1  ATGATCCCAT CCCCTACCCC AATAAACTTT CGTGATGATA CGATTCTAGA
  51  GACGGATCCA AAGCCGTCTT TAATCATGTT CTCTTCAAAA AAAACAGAGA
 101  TAGCTTCTGA AAGACGGAAG GCCCATCCCA CCTTATTTAA AGTTCTAGGA
 151  ACGATTTGGA ATATTGTGAA GTTTATTATC TCAATCATTC TGTTCCTTCC
 201  CTTAGCGTTA TTGTGGGTAC TCAAGAAAAC CTGTCAGTTT TTCATTCTCC
 251  CATCTTCTAT CATATCTCAG AGCATGTCAA AAACAGCTGT GGCAATTCGG
 301  CGAATGACCT TTCTGTCCCA TATTAAACAA CTCCTAAGCC TTAAGGAAAT
 351  CTCAGCTGCC GATCGTGTGG TTATACAATA TGACGATTTG GTGGTTGATA
 401  GCTTAGCTAT AAAGATACCT CATGCTCTTC CCCACAGGTG GATTCTTTAT
 451  TCTCAAGGAA ACTCTGGATT GATGGAAAAC CTGTTCGATC GGGGCGATTC
 501  CTCTCTACAC CAGCTAGCCA AAGCAACCGG CTCGAATCTT CTTGTGTTCA
 551  ACTATCCTGG AATTATGTCC AGCAAAGGAG AAGCGAAACG AGAAAATCTG
 601  GTTAAATCGT ATCAGGCATG CGTACGCTAC CTACGAGATG AAGAGACAGG
 651  TCCTAAAGCC AATCAAATCA TAGCTTTCGG ATACTCTTTG GGAACTAGTG
 701  TCCAAGCTGC TGCTCTAGAT CGTGAGGTCA CTGATGGCAG TGATGGAACT
 751  TCATGGATTG TTGTAAAAGA TCGGGGCCCT CGCTCTCTAG CAGATGTCGC
 801  GAATCAAATT TGTAAGCCCA TAGCTTCCGC GATTATAAAA CTCGTTGGTT
 851  GGAACATAGA CTCTGTGAAA CCTAGCGAAA GATTGCGTTG TCCCGAAATT
 901  TTCATTTACA ACTCTAATCA TGATCAAGAA CTCATTAGCG ACGGCCTCTT
 951  CGAAAGAGAA AATTGCGTAG CAACACCTTT TCTAGAGCTT CCTGAAGTAA
1001  AAACCTCGGG GACTAAAATT CCTATACCCG AAAGGGATCT TCTCCATCTA
1051  AATCCTCTCA GTCCAAATGT AGTAGACAGA TTAGCAGCAG TGATCTCTAA
1101  TTATTTAGAT TCTGAAAACA GAAAGTCTCA GCAACCTGAT TAA
```

The PSORT algorithm predicts inner membrane (0.571).

The protein was expressed in *E.coli* and purified as a GST-fusion product (Figure 87A). The recombinant protein was used to immunise mice, whose sera were used in a Western blot (Figure 87B) and for FACS analysis.

These experiments show that cp7249 is a surface-exposed and immunoaccessible protein, and that it is a useful immunogen. These properties are not evident from the sequence alone.

**Example 88**

The following *C.pneumoniae* protein (PID 4377261) was expressed <SEQ ID 175; cp7261>:

```
  1  MLPISILLFY VILGCLSAYI ADKKKRNVIG WFFAGAFFGF IGLVVLLLLP
 51  SRRNALEKPQ NDPFDNSDLF DDLKKSLAGN DEIPSSGDLQ EIVIDTEKWF
101  YLNKDRENVG PISFEELVVL LKGKTYPEEI WVWKKGMKDW QRVKDVPSLQ
151  QALKEASK*
```

The cp7261 nucleotide sequence <SEQ ID 176> is:

```
  1  ATGCTCCCTA TTTCGATTTT ATTATTTTAT GTGATTCTAG GTTGTCTATC
 51  TGCCTACATA GCAGATAAGA AAAAACGAAA TGTTATTGGC TGGTTTTTTG
101  CAGGAGCATT TTTTGGATTT ATTGGTCTAG TTGTCCTTCT TCTTCTTCCT
151  TCTCGTCGAA ACGCTTTAGA AAAGCCACAA AACGATCCTT TTGATAACTC
201  CGATCTTTTT GATGATTTGA AAAAAAGTTT AGCAGGTAAT GACGAGATAC
251  CCTCATCGGG AGATCTTCAA GAAATCGTTA TCGATACAGA GAAGTGGTTT
301  TATTTAAATA AAGATAGAGA AAACGTAGGT CCGATATCTT TTGAGGAGTT
351  GGTCGTACTT TTAAAGGGAA AAACGTATCC AGAAGAAATT TGGGTATGGA
401  AAAAGGGAAT GAAAGATTGG CAACGAGTGA AGGATGTTCC ATCACTACAA
451  CAGGCTTTGA AAGAAGCATC AAAATAA
```

The PSORT algorithm predicts inner membrane (0.848).

The protein was expressed in *E.coli* and purified as a GST-fusion product (Figure 88A). The recombinant protein was used to immunise mice, whose sera were used in a Western blot (Figure 88B) and for FACS analysis.

These experiments show that cp7261 is a surface-exposed and immunoaccessible protein, and that it is a useful immunogen. These properties are not evident from the sequence alone.

## Example 89

The following *C.pneumoniae* protein (PID 4377305) was expressed <SEQ ID 177; cp7305>:

```
  1 MEVYSFHPAV RTSFQHRVMA ALDAWFFLGG HRLKVVSLDS CNSGWAYQEL
 51 VSISTTEKVL KLLSYLLVPI VIIALLIRCL LHSNFRIDVE KERWLKIREL
101 GIDIESCKLP SSYVNQVSSF IWFEKDKSKR PRIDVDYHTL HSKDWVVFPI
151 VFQKIPKTSR FSYWFSQKET RKRDYVRNML DHVIGYLTSE GGEWLQYISK
201 TSYQSATSLD PERVLQYCLT DNQELQGEVQ RLLNEESATK SSGDKEVLLS
251 HVSDIICQCW WPKFLEVIQS PAFIEELVEE VSGKLNLDFL CLEKANTLDQ
301 ELRNSLLRAV VHHGSEGVDI KKVGAGLIIY TEAIQLQIPF SRS*
```

The cp7305 nucleotide sequence <SEQ ID 178> is:

```
   1 ATGGAAGTTT ATAGTTTTCA CCCTGCGGTA AGGACTTCGT TTCAGCACCG
  51 TGTAATGGCA GCACTAGATG CTTGGTTTTT TCTAGGAGGG CACCGTTTAA
 101 AAGTAGTTTC TCTAGATAGT TGTAACTCAG GTTGGGCGTA TCAAGAACTT
 151 GTGTCTATTT CAACGACAGA AAAAGTCTTG AAACTACTCT CTTACCTACT
 201 CGTACCGATT GTCATAATAG CTCTGTTAAT TCGTTGTCTT TTACATAGCA
 251 ATTTTAGGAT AGACGTAGAG AAGGAACGTT GGTTAAAAAT AAGGGAGTTA
 301 GGAATTGATA TAGAAAGCTG CAAACTCCCC AGTTCTTATG TAAACCAGGT
 351 TTCCTCGTTT ATTTGGTTTG AAAAAGATAA ATCCAAACGG CCACGTATTG
 401 ATGTAGATTA TCATACGCTA CATAGCAAAG ACTGGGTAGT TTTCCCTATC

 451 GTTTTTCAGA AAATTCCAAA GACCTCGCGT TTCAGTTATT GGTTCTCACA
 501 AAAAGAAACA AGGAAGAGGG ATTATGTGAG AAATATGCTG GACCACGTCA
 551 TTGGTTATCT AACGTCAGAA GGTGGGGAGT GGTTGCAGTA TATATCGAAA
 601 ACCTCTTATC AAAGCGCTAC TTCCTTGGAT CCTGAAAGAG TTCTTCAATA
 651 TTGCTTAACT GATAACCAGG AGCTCCAGGG AGAAGTGCAA CGTTTGCTTA
 701 ATGAGGAGAG TGCGACCAAA AGCTCTGGGG ATAAGGAAGT TTTGTTAAGT
 751 CATGTATCTG ACATTATTTG CCAGTGTTGG TGGCCAAAGT TTCTTGAAGT
 801 TATACAATCT CCGGCCTTTA TTGAAGAATT AGTAGAAGAA GTGAGTGGTA
 851 AACTTAATTT AGATTTTTTA TGCCTAGAAA AGGCTAATAC ATTAGATCAG
 901 GAGTTGAGAA ACAGTCTTCT AAGAGCAGTC GTACACCACG GTTCTGAAGG
 951 AGTTGATATT AAGAAAGTTG GTGCCGGCCT CATTATTTAT ACGGAAGCTA
1001 TTCAATTACA GATTCCCTTC TCAAGGAGTT AA
```

The PSORT algorithm predicts inner membrane (0.508).

The protein was expressed in *E.coli* and purified as a GST-fusion product (Figure 89A) and also as a double GST/his fusion. The recombinant proteins were used to immunise mice, whose sera were used in a Western blot (Figure 89B) and for FACS analysis.

These experiments show that cp7305 is a surface-exposed and immunoaccessible protein, and that it is a useful immunogen. These properties are not evident from the sequence alone.

## Example 90

The following *C.pneumoniae* protein (PID 4377347) was expressed <SEQ ID 179; cp7347>:

```
  1  MKKGKLGAIV FGLLFTSSVA GFSKDLTKDN AYQDLNVIEH LISLKYAPLP
 51  WKELLFGWDL SQQTQQARLQ LVLEEKPTTN YCQKVLSNYV RSLNDYHAGI
101  TFYRTESAYI PYVLKLSEDG HVFVVDVQTS QGDIYLGDEI LEVDGMGIRE
151  AIESLRFGRG SATDYSAAVR SLTSRSAAFG DAVPSGIAML KLRRPSGLIR
201  STPVRWRYTP EHIGDFSLVA PLIPEHKPQL PTQSCVLFRS GVNSQSSSSS
251  LFSSYMVPYF WEELRVQNKQ RFDSNHHIGS RNGFLPTFGP ILWEQDKGPY
301  RSYIFKAKDS QGNPHRIGFL RISSYVWTDL EGLEEDHKDS PWELFGEIID
351  HLEKETDALI IDQTHNPGGS VFYLYSLLSM LTDHPLDTPK HRMIFTQDEV
401  SSALHWQDLL EDVFTDEQAV AVLGETMEGY CMDMHAVASL QNFSQSVLSS
451  WVSGDINLSK PMPLLGFAQV RPHPKHQYTK PLFMLIDEDD FSCGDLAPAI
501  LKDNGRATLI GKPTAGAGGF VFQVTFPNRS GIKGLSLTGS LAVRKDGEFI
551  ENLGVAPHID LGFTSRDLQT SRFTDYVEAV KTIVLTSLSE NAKKSEEQTS
601  PQETPEVIRV SYPTTTSAS*
```

A predicted signal peptide is highlighted.

The cp7347 nucleotide sequence <SEQ ID 180> is:

```
   1  ATGAAAAAAG GGAAATTAGG AGCCATAGTT TTTGGCCTTC TATTTACAAG
  51  TAGTGTTGCT GGTTTTTCTA AGGATTTGAC TAAAGACAAC GCTTATCAAG
 101  ATTTAAATGT CATAGAGCAT TTAATATCGT TAAAATATGC TCCTTTACCA
 151  TGGAAGGAAC TATTATTTGG TTGGGATTTA TCTCAGCAAA CACAGCAAGC
 201  TCGCTTGCAA CTGGTCTTAG AAGAAAAACC AACAACCAAC TACTGCCAGA
 251  AGGTACTCTC TAACTACGTG AGATCATTAA ACGATTATCA TGCAGGGATT
 301  ACGTTTTATC GTACTGAAAG TGCGTATATC CCTTACGTAT TGAAGTTAAG
 351  TGAAGATGGT CATGTCTTTG TAGTCGACGT ACAGACTAGC CAAGGGGATA
 401  TTTACTTAGG GGATGAAATC CTTGAAGTAG ATGGAATGGG GATTCGTGAG
 451  GCTATCGAAA GCCTTCGCTT TGGACGAGGG AGTGCCACAG ACTATTCTGC
 501  TGCAGTTCGT TCCTTGACAT CGCGTTCCGC CGCTTTTGGA GATGCGGTTC
 551  CTTCAGGAAT TGCCATGTTG AAACTTCGCC GACCCAGTGG TTTGATCCGT
 601  TCGACACCGG TCCGTTGGCG TTATACTCCA GAGCATATCG GAGATTTTTC
 651  TTTAGTTGCT CCTTTGATTC CTGAACATAA ACCTCAATTA CCTACACAAA
 701  GTTGTGTGCT ATTCCGTTCC GGGGTAAATT CACAGTCTTC TAGTAGCTCT
 751  TTATTCAGTT CCTACATGGT GCCTTATTTC TGGGAAGAAT TGCGGGTTCA
 801  AAATAAGCAG CGTTTTGACA GTAATCACCA TATAGGGAGC CGTAATGGAT
 851  TTTTACCTAC GTTTGGTCCT ATTCTTTGGG AACAAGACAA GGGGCCCTAT
 901  CGTTCCTATA TCTTTAAAGC AAAAGATTCT CAGGGCAATC CCCATCGCAT
 951  AGGATTTTTA AGAATTTCTT CTTATGTTTG GACTGATTTA GAAGGACTTG
1001  AAGAGGATCA TAAGGATAGT CCTTGGGAGC TCTTTGGAGA GATCATCGAT

1051  CATTTGGAAA AAGAGACTGA TGCTTTGATT ATTGATCAGA CCCATAATCC
1101  TGGAGGCAGT GTTTTCTATC TCTATTCGTT ACTATCTATG TTAACAGATC
1151  ATCCTTTAGA TACTCCTAAA CATAGAATGA TTTTCACTCA GGATGAAGTC
1201  AGCTCGGCTT TGCACTGGCA AGATCTACTA GAAGATGTCT TCACAGATGA
1251  GCAGGCAGTT GCCGTGCTAG GGGAAACTAT GGAAGGATAT TGCATGGATA
1301  TGCATGCTGT AGCCTCTCTT CAAAACTTCT CTCAGAGTGT CCTTTCTTCC
1351  TGGGTTTCAG GTGATATTAA CCTTTCAAAA CCTATGCCTT TGCTAGGATT
1401  TGCACAGGTT CGACCTCATC CTAAACATCA ATATACTAAA CCTTTGTTTA
1451  TGTTGATAGA CGAGGATGAC TTCTCTTGTG GAGATTTAGC GCCTGCAATT
1501  TTGAAGGATA ATGGCCGCGC TACTCTCATT GGAAAGCCAA CAGCAGGAGC
1551  TGGAGGTTTT GTATTCCAAG TCACTTTCCC TAACCGTTCT GGAATTAAAG
1601  GTCTTTCTTT AACAGGATCT TTAGCTGTTA GGAAAGATGG TGAGTTTATT
1651  GAAAACTTAG GAGTGGCTCC TCATATTGAT TTAGGATTTA CCTCCAGGGA
1701  TTTGCAAACT TCCAGGTTTA CTGATTACGT TGAGGCAGTG AAAACTATAG
1751  TTTTAACTTC TTTGTCTGAG AACGCTAAGA AGAGTGAAGA GCAGACTTCT
1801  CCGCAAGAGA CGCCTGAAGT TATTCGAGTC TCTTATCCCA CAACGACTTC
1851  TGCTTCGTAA
```

The PSORT algorithm predicts periplasmic space (0.2497).

The protein was expressed in *E.coli* and purified as a GST-fusion product (Figure 90A) and also in his-tagged form. The recombinant proteins were used to immunise mice, whose sera were used in a Western blot (Figure 90B) and for FACS analysis.

These experiments show that cp7347 is a surface-exposed and immunoaccessible protein, and that it is a useful immunogen. These properties are not evident from the sequence alone.

**Example 91**

The following *C.pneumoniae* protein (PID 4377353) was expressed <SEQ ID 181; cp7353>:

```
  1 MNMPVPSAVP SANITLKEDS STVSTASGIL KTATGEVLVS CTALEGSSST
 51 DALISLALGQ IILATQQELL LQSTNVHQLL FLPPEVVELE IQVVDLLVQL
101 EHAETITSEP QETQTQSRSE QTLPQQSSSK QSALSPRSLK PEISDSKQQQ
151 ALQTPKDSAV RKHSEAPSPE TQARASLSQA SSSSQRSLPP QESAPERTLL
201 EQQKASSFSP LSQFSAEKQK EALTTSKSHE LYKERDQDRQ QREQHDRKHD
251 QEEDAESKKK KKKRGLGVEA VAEEPGENLD IAALIFSDQM RPPAEETSKK
301 ETTFKKKLPS PMSVFSRFIP SKNPLSVGSS IHGPIQTPKV ENVFLRFMKL
351 MARILGQAEA EANELYMRVK QRTDDVDTLT VLISKINNEK KDIDWSENEE
401 MKALLNRAKE IGVTIDKEKY TWTEEEKRLL KENVQMRKEN MEKITQMERT
451 DMQRHLQEIS QCHQARSNVL KLLKELMDTF IYNLRP*
```

The cp7353 nucleotide sequence <SEQ ID 182> is:

```
   1 ATGAATATGC CTGTTCCTTC TGCAGTTCCC TCTGCAAATA TAACTCTAAA
  51 AGAAGACAGC TCAACAGTTT CCACAGCCTC TGGAATATTA AAGACTGCAA
 101 CAGGTGAAGT CTTAGTCTCT TGTACAGCGC TAGAAGGAAG CTCTTCTACA
 151 GATGCTTTAA TTAGCTTAGC TTTAGGACAA ATCATTCTTG CGACCCAACA
 201 AGAACTGCTC TTACAAAGCA CAAATGTTCA TCAACTCCTC TTCCTCCCTC
 251 CTGAAGTTGT AGAATTAGAA ATCCAAGTTG TTGACTTGCT AGTGCAATTG
 301 GAACATGCAG AGACAATCAC AAGTGAACCA CAAGAAACAC AAACGCAAAG
 351 TAGGAGTGAG CAGACCCTCC CTCAACAAAG CAGCAGTAAA CAATCTGCTC
 401 TCTCCCCACG CTCCTTAAAA CCTGAAATTT CTGATTCTAA ACAACAGCAA
 451 GCTCTTCAAA CACCAAAAGA CTCTGCTGTA AGAAAACACA GCGAAGCACC
 501 GTCACCTGAG ACACAAGCTC GCGCTTCCTT ATCTCAGGCA AGCTCAAGTT
 551 CTCAGAGATC CTTACCTCCG CAAGAAAGTG CGCCAGAAAG AACACTATTA
 601 GAACAACAAA AAGCAAGCTC CTTCTCTCCT CTATCCCAGT TCTCTGCAGA
 651 GAAACAAAAA GAGGCCCTGA CGACCTCAAA ATCTCATGAA CTCTATAAAG
 701 AACGCGATCA AGATCGCCAA CAAAGAGAGC AGCACGACAG AAAGCACGAT
 751 CAGGAAGAAG ACGCTGAATC TAAAAAGAAA AAGAAGAAAC GTGGTCTCGG
 801 TGTAGAGGCA GTCGCTGAGG AACCCGGAGA AAATCTAGAT ATTGCCGCTT
 851 TAATCTTCTC AGATCAAATG CGACCTCCTG CTGAAGAAAC TTCTAAAAAA
 901 GAAACGACAT TCAAAAAGAA GCTACCTTCT CCAATGTCTG TGTTTAGCAG
 951 ATTCATCCCT AGTAAGAATC CGTTATCTGT AGGCTCTTCA ATACACGGGC
1001 CTATACAAAC TCCAAAAGTA GAAAATGTGT TCTTAAGGTT CATGAAGCTC

1051 ATGGCAAGAA TCTTAGGCCA AGCCGAAGCC GAAGCTAATG AACTCTACAT
1101 GCGAGTCAAA CAACGTACCG ATGATGTAGA CACACTCACA GTCCTTATCT
1151 CTAAGATCAA TAATGAAAAG AAAGACATTG ATTGGAGTGA AAATGAAGAG
1201 ATGAAAGCTC TTTTAAATCG AGCTAAAGAG ATTGGAGTCA CTATAGACAA
1251 AGAAAAATAT ACTTGGACAG AAGAGGAAAA AAGACTTCTA AAAGAGAATG
1301 TCCAAATGCG CAAAGAGAAT ATGGAGAAAA TCACTCAAAT GGAAAGGACG
1351 GACATGCAAA GGCACCTCCA AGAGATTTCT CAATGTCATC AAGCGCGCTC
1401 TAATGTATTG AAGTTATTGA AAGAACTTAT GGACACCTTC ATTTACAACC
1451 TACGCCCCTA A
```

The PSORT algorithm predicts cytoplasm (0.1308).

The protein was expressed in *E.coli* and purified as a GST-fusion product (Figure 91A). The recombinant protein was used to immunise mice, whose sera were used in a Western blot (Figure 91B) and for FACS analysis.

These experiments show that cp7353 is a surface-exposed and immunoaccessible protein, and that it is a useful immunogen. These properties are not evident from the sequence alone.

**Example 92**

The following *C.pneumoniae* protein (PID 4377408) was expressed <SEQ ID 183; cp7408>:

```
  1 MLKIQKKRMC VSVVITVGAI VGFFNSADAA PKKKKIPIQI LYSFTKVSSY
 51 LKNEDASTIF CVDVDRGLLQ HRYLGSPGWQ ETRRRQLFKS LENQSYGNER
101 LGEETLAIDI FRNKECLESE IPEQMEAILA NSSALVLGIS SFGITGIPAT
151 LHSLLRQNLS FQKRSIASES FLLKIDSAPS DASVFYKGVL FRGETAIVDA
201 LSQLFAQLDL SPKKIIFLGE DPEVVQAVGS ACIGWGMNFL GLVYYPAQES
251 LFSYVHPYST ATELQEAQGL QVISDEVAQL TLNALPKMN*
```

The cp7408 nucleotide sequence <SEQ ID 184> is:

```
  1 ATGTTGAAAA TCCAGAAAAA AAGAATGTGT GTCAGCGTAG TCATCACGGT
 51 AGGCGCCATA GTGGGGTTTT TCAATTCTGC AGACGCAGCA CCAAAGAAAA
101 AGAAGATCCC TATACAGATT CTCTACTCCT TTACTAAAGT CTCTTCCTAT
151 TTAAAAAACG AAGACGCAAG TACTATATTT TGCGTCGATG TGGATCGTGG
201 ACTTCTCCAG CATCGGTATT TAGGTAGTCC AGGATGGCAG GAAACCAGAC
251 GTCGGCAGTT ATTTAAATCC TTAGAAAATC AATCATACGG CAACGAACGT
301 TTAGGAGAAG AAACTCTTGC TATTGATATT TTCAGGAACA AAGAGTGCTT
351 GGAGAGCGAG ATCCCAGAGC AGATGGAAGC TATCCTTGCA AATTCCTCGG
401 CCTTGGTCTT AGGCATCTCT TCTTTTGGGA TCACAGGAAT TCCTGCGACT
451 TTGCATAGTT TGCTTCGACA GAATCTATCT TTCCAAAAAC GCTCTATAGC
501 ATCGGAGAGC TTCCTTTTAA AGATCGATAG TGCCCCCTCA GATGCCTCTG
551 TTTTTTATAA AGGCGTGCTT TTCCGCGGAG AGACTGCGAT CGTGGATGCG
601 TTAAGCCAAT TATTTGCCCA GCTCGATCTT TCTCCTAAAA AAATTATCTT
651 TCTAGGAGAA GACCCTGAGG TCGTTCAAGC TGTTGGGTCT GCTTGTATAG
701 GTTGGGGCAT GAACTTTTTA GGCCTGGTAT ACTATCCTGC TCAAGAAAGC
751 CTTTTTTCTT ATGTTCATCC TTACTCTACA GCAACGGAGC TCCAAGAAGC
801 ACAGGGTTTA CAAGTAATTT CAGATGAAGT CGCACAGCTT ACTTTAAACG
851 CTCTTCCGAA AATGAATTAA
```

The PSORT algorithm predicts inner membrane (0.123).

The protein was expressed in *E.coli* and purified as a his-tag product (Figure 92A). The recombinant protein was used to immunise mice, whose sera were used in a Western blot (Figure 92B) and for FACS analysis.

These experiments show that cp7408 is a surface-exposed and immunoaccessible protein, and that it is a useful immunogen. These properties are not evident from the sequence alone.

**Example 93**

The following *C.pneumoniae* protein (PID 4376424) was expressed <SEQ ID 185; cp6424>:

```
  1 MMHNIVVLSE EPGRSAFLGR TAFFPNKYPI AQGGVGIPST IGNLFTIWYC
 51 FYFYRAATPQ SDHPDGCGFI LLERLKELGA GFFYCDLRES NTTGFTLFFE
101 GSNKGVLKNH LFIRDE*
```

The cp6424 nucleotide sequence <SEQ ID 186> is:

```
  1 ATGATGCACA ATATTGTTGT TCTTAGTGAG GAACCTGGAC GAAGCGCTTT
 51 TCTTGGTAGG ACGGCATTTT TCCCTAATAA GTATCCAATA GCTCAGGGTG
101 GTGTTGGAAT ACCATCTACA ATAGGCAATC TCTTTACTAT ATGGTACTGT
151 TTCTATTTTT ATAGAGCTGC AACTCCACAA TCTGATCATC CTGACGGATG
201 TGGCTTTATT CTACTAGAAA GGCTTAAGGA GCTCGGTGCA GGGTTCTTTT
251 ATTGTGATCT TCGTGAGTCC AATACCACTG GCTTTACTCT TTTTTTTGAA
301 GGCTCCAATA AAGGTGTGTT AAAGAATCAC TTGTTTATTA GAGATGAGTA
351 A
```

The PSORT algorithm predicts cytoplasm (0.2502).

The protein was expressed in *E.coli* and purified as a GST-fusion product (Figure 93A) and also in his-tagged form. The recombinant proteins were used to immunise mice, whose sera were used in Western blots (Figure 93B) and for FACS analyses (Figure 93C; GST-fusion).

These experiments show that cp6424 is a surface-exposed and immunoaccessible protein, and that it is a useful immunogen. These properties are not evident from the sequence alone.

**Example 94**

The following *C.pneumoniae* protein (PID 4376449) was expressed <SEQ ID 187; cp6449>:

```
  1  VASETYPSQI LHAQREVRDA YFNQADCHPA RANQILEAKK ICLLDVYHTN
 51  HYSVFTFCVD NYPNLRFTFV SSKNNEMNGL SNPLDNVLVE AMVRRTHARN
101  LLAACKIRNI EVPRVVGLDL RSGILISKLE LKQPQFQSLT EDFVNHSTNQ
151  EEARVHQKHV LLISLILLCK QAVLESFQEK KRSS*
```

The cp6449 nucleotide sequence <SEQ ID 188> is:

```
  1  GTGGCGTCTG AAACGTATCC TTCTCAGATA TTGCACGCTC AGAGGGAAGT
 51  ACGTGATGCC TATTTTAATC AAGCGGATTG CCATCCTGCT CGGGCTAATC
101  AGATTCTCGA GGCTAAGAAA ATCTGTTTAT TAGATGTTTA TCATACTAAT
151  CATTATTCCG TATTTACTTT TTGTGTAGAT AATTATCCGA ATCTCCGCTT
201  TACATTTGTA TCTTCAAAAA ACAATGAGAT GAATGGCTTA TCTAATCCTC
251  TAGATAATGT TCTTGTAGAG GCTATGGTAC GTAGAACACA TGCAAGAAAC
301  CTACTTGCAG CGTGTAAAAT TCGAAATATT GAGGTTCCAA GGGTTGTTGG
351  GCTTGACCTA AGATCTGGGA TACTCATTTC GAAACTAGAA TTGAAGCAAC
401  CTCAGTTCCA AAGTTTAACA GAAGACTTCG TAAATCATTC CACAAATCAG
451  GAAGAAGCTC GCGTCCATCA AAAGCATGTG TTGCTAATTT CTTTAATTTT
501  ACTTTGCAAG CAGGCCGTTC TGGAATCATT CCAGGAAAAA AAGCGATCCT
551  CTTAA
```

The PSORT algorithm predicts inner membrane (0.2084).

The protein was expressed in *E.coli* and purified as a GST-fusion product (Figure 94A) and also in his-tagged form. The recombinant proteins were used to immunise mice, whose sera were used in Western blots (Figure 94B) and for FACS analyses (Figure 94C; GST-fusion).

These experiments show that cp6449 is a surface-exposed and immunoaccessible protein, and that it is a useful immunogen. These properties are not evident from the sequence alone.

**Example 95**

The following *C.pneumoniae* protein (PID 4376495) was expressed <SEQ ID 189; cp6495>:

```
MRELNAFELTQPEEYRNRWVLMPCLKCRFCRTQHAKVWSYRCVHEASLYEKNCFLTLTYDDKHLPQYGSLVKLHLQLFLKR
LRKMISPHKIRYFECGAYGTKLQRPHYHLLLS
```

The cp6495 nucleotide sequence <SEQ ID 190> is:

```
TTGCGAGAATTAAATGCTTTTGAATTAACTCAACCTGAAGAGTATCGAAACCGTTGGGTTTTGATGCCTTGTCTTAAGTGT
CGTTTTTGTAGAACGCAACATGCAAAAGTCTGGTCTTATCGTTGTGTCCATGAAGCTTCTTTGTATGAGAAAAATTGTTTT
CTTACTTTGACTTATGATGATAAGCATTTACCTCAGTATGGTTCGTTGGTAAAGCTGCATTTACAGCTGTTTCTTAAGAGA
TTAAGAAAGATGATTTCTCCTCATAAAATTCGTTATTTTGAATGTGGTGCGTATGGAACCAAATTACAAAGACCTCATTAT
CATCTACTTTTATCATGA
```

The PSORT algorithm predicts cytoplasmic (0.280).

The protein was expressed in *E.coli* and purified as a GST-fusion product (Figure 95A). The recombinant protein was used to immunise mice, whose sera were used in a Western blot (Figure 95B) and for FACS analysis (Figure 95C).

These experiments show that cp6495 is a surface-exposed and immunoaccessible protein, and that it is a useful immunogen. These properties are not evident from the sequence alone.

**Example 96**

The following *C.pneumoniae* protein (PID 4376506) was expressed <SEQ ID 191; cp6506>:

```
  1  MRRFLFLILS SLPLVAFSAD NFTILEEKQS PLSRVSIIFA LPGVTPVSFD
 51  GNCPIPWFSH SKKTLEGQRI YYSGDSFGKY FVVSALWPNK VSSAVVACNM
101  ILKHRVDLIL IIGSCYSRSQ DSRFGSVLVS KGYINYDADV RPFFERFEIP
151  DIKKSVFATS EVHREAILRG GEEFISTHKQ EIEELLKTHG YLKSTTKTEH
201  TLMEGLVATG ESFAMSRNYF LSLQKLYPEI HGFDSVSGAV SQVCYEYSIP
251  CLGVNILLPH PLESRSNEDW KHLQSEASKI YMDTLLKSVL KELCSSH*
```

The cp6506 nucleotide sequence <SEQ ID 192> is:

```
  1  ATGCGTCGTT TTCTGTTTCT TATTCTTAGC TCTCTTCCTT TGGTCGCATT
 51  CTCTGCTGAT AATTTCACTA TTCTAGAAGA AAAACAGAGT CCTTTAAGTC
101  GTGTAAGTAT TATTTTTGCT TTACCTGGGG TTACTCCCGT TTCTTTTGAT
151  GGTAATTGTC CTATTCCTTG GTTTTCTCAT AGTAAAAAGA CTCTAGAGGG
201  ACAGAGAATT TATTACTCTG GCGACTCCTT TGGGAAATAC TTTGTAGTTT
251  CTGCTCTTTG GCCTAATAAA GTTTCTTCAG CTGTTGTGGC TTGTAATATG
301  ATTCTTAAAC ATCGAGTGGA TCTTATTCTA ATTATAGGCT CGTGTTACTC
351  TAGGTCTCAA GATAGCCGTT TTGGCAGCGT CTTAGTTTCT AAAGGCTACA
401  TTAATTATGA TGCAGATGTG AGGCCTTTCT TTGAAAGATT TGAGATTCCA
451  GACATTAAAA AGAGTGTTTT TGCAACCAGT GAGGTTCATC GGGAGGCAAT
501  TCTTCGTGGA GGCGAAGAGT TTATTTCTAC CCATAAACAA GAAATCGAAG
551  AGCTTTTGAA GACTCATGGG TATTTGAAAT CAACAACCAA AACGGAGCAC
601  ACCTTAATGG AAGGTTTGGT TGCTACAGGC GAGTCTTTCG CGATGTCGCG
651  AAACTATTTT CTTTCCTTAC AAAAATTGTA TCCAGAGATT CATGGTTTTG
701  ATAGTGTCAG CGGCGCTGTT TCTCAGGTAT GCTATGAATA TAGCATTCCT
751  TGTTTAGGTG TGAATATCCT TCTCCCTCAT CCTTTAGAAT CACGGAGTAA
801  CGAGGATTGG AAGCATCTTC AAAGTGAGGC AAGTAAAATT TATATGGATA
851  CCTTGCTCAA GAGTGTATTA AAAGAACTCT GTTCTTCTCA TTAA
```

The PSORT algorithm predicts periplasmic space (0.571).

The protein was expressed in *E.coli* and purified as his-tag (Figure 96A) and GST-fusion (Figure 96B) products. The GST-fusion protein was used to immunise mice, whose sera were used in a Western blot (Figure 96C) and for FACS analysis (Figure 96D).

These experiments show that cp6506 is a surface-exposed and immunoaccessible protein, and that it is a useful immunogen. These properties are not evident from the sequence alone.

**Example 97**

The following *C.pneumoniae* protein (PID 4376882) was expressed <SEQ ID 193; cp6882>:

```
  1  MSLLNLPSSQ DSASEDSTSQ SQIFDPIRNR ELVSTPEEKV RQRLLSFLMH
 51  KLNYPKKLII IEKELKTLFP LLMRKGTLIP KRRPDILIIT PPTYTDAQGN
101  THNLGDPKPL LLIECKALAV NQNALKQLLS YNYSIGATCI AMAGKHSQVS
151  ALFNPKTQTL DFYPGLPEYS QLLNYFISLN L*
```

The cp6882 nucleotide sequence <SEQ ID 194> is:

```
  1  ATGTCCTTAT TGAACCTTCC CTCAAGCCAG GATTCTGCAT CTGAGGACTC
 51  CACATCGCAA TCTCAAATCT TCGATCCCAT TAGAAATCGG GAGTTAGTTT
101  CTACTCCCGA AGAAAAAGTC CGCCAAAGGT TGCTCTCCTT CCTAATGCAT
151  AAGCTGAACT ACCCTAAGAA ACTCATCATC ATAGAAAAAG AACTCAAAAC
201  TCTTTTTCCT CTGCTTATGC GTAAAGGAAC CCTAATCCCA AAACGCCGCC
251  CAGATATTCT CATCATCACT CCCCCCACAT ACACAGACGC ACAGGGAAAC
301  ACTCACAACC TAGGCGACCC AAAACCCCTG CTACTTATCG AATGTAAGGC
351  CTTAGCCGTA AACCAAAATG CACTCAAACA ACTCCTTAGC TATAACTACT
401  CTATCGGAGC CACCTGCATT GCTATGGCAG GGAAACACTC TCAAGTGTCA
451  GCTCTCTTCA ATCCAAAAAC ACAAACTCTT GATTTTTATC CTGGCCTCCC
501  AGAGTATTCC CAACTCCTAA ACTACTTTAT TTCTTTAAAC TTATAG
```

The PSORT algorithm predicts cytoplasm (0.362).

The protein was expressed in *E.coli* and purified as a GST-fusion product (Figure 97A). The protein was used to immunise mice, whose sera were used in a Western blot (Figure 97B) and for FACS analysis (Figure 97C).

These experiments show that cp6882 is a surface-exposed and immunoaccessible protein, and that it is a useful immunogen. These properties are not evident from the sequence alone.

**Example 98**

The following *C.pneumoniae* protein (PID 4376979) was expressed <SEQ ID 195; cp6979>:

```
  1  MSVNPSGNSK NDLWITGAHD QHPDVKESGV TSANLGSHRV TASGGRQGLL
 51  ARIKEAVTGF FSRMSFFRSG APRGSQQPSA PSADTVRSPL PGGDARATEG
101  AGRNLIKKGY QPGMKVTIPQ VPGGGAQRSS GSTTLKPTRP APPPPKTGGT
151  NAKRPATHGK GPAPQPPKTG GTNAKRAATH GKGPAPQPPK GILKQPGQSG
201  TSGKKRVSWS DED*
```

The cp6979 nucleotide sequence <SEQ ID 196> is:

```
  1  ATGTCTGTTA ATCCATCAGG AAATTCCAAG AACGATCTCT GGATTACGGG
 51  AGCTCATGAT CAGCATCCCG ATGTTAAAGA ATCCGGGGTT ACAAGTGCTA
101  ACCTAGGAAG TCATAGAGTG ACTGCCTCAG GAGGACGCCA AGGGTTATTA
151  GCACGAATCA AAGAAGCAGT AACCGGGTTT TTTAGTCGGA TGAGCTTCTT
201  CAGATCGGGA GCTCCAAGAG GTAGCCAACA ACCCTCTGCT CCATCTGCAG
251  ATACTGTACG TAGCCCGTTG CCGGGAGGGG ATGCTCGCGC TACCGAGGGA
301  GCTGGTAGGA ACTTAATTAA AAAAGGGTAC CAACCAGGGA TGAAAGTCAC
351  TATCCCACAG GTTCCTGGAG GAGGGGCCCA ACGTTCATCA GGTAGCACGA
401  CACTAAAGCC TACGCGTCCG GCACCCCCAC CTCCTAAAAC GGGTGGAACT
451  AATGCAAAAC GTCCGGCAAC GCACGGGAAG GGTCCAGCAC CCCAGCCTCC
501  TAAAACAGGT GGGACCAATG CTAAGCGCGC AGCAACGCAT GGGAAAGGTC
551  CAGCACCTCA ACCTCCTAAG GGCATTTTGA AACAGCCTGG GCAGTCTGGG
601  ACTTCAGGAA AGAAGCGTGT CAGCTGGTCT GACGAAGATT AA
```

The PSORT algorithm predicts cytoplasm (0.360).

The protein was expressed in *E.coli* and purified as a GST-fusion product (Figure 98A). The GST-fusion protein was used to immunise mice, whose sera were used in a Western blot (Figure 98B) and for FACS analysis (Figure 98C).

These experiments show that cp6979 is a surface-exposed and immunoaccessible protein, and that it is a useful immunogen. These properties are not evident from the sequence alone.

**Example 99**

The following *C.pneumoniae* protein (PID 4377028) was expressed <SEQ ID 197; cp7028>:

```
  1  MLLGFLCDCP CASWQCAAVA NCYDSVFMSR PEHKPNIPYI TKATRRGLRM
 51  KTLAYLASLK DARQLAYDFL KDPGSLARLA KALIAPKEAL QEGNLFFYGC
101  SNIEDILEEM RRPHRILLLG FSYCQKPKAC PEGRFNDACR YDPSHPTCAS
151  CSIGTMMRLN ARRYTTVIIP TFIDIAKHLH TLKKRYPGYQ ILFAVTACEL
201  SLKMFGDYAS VMNLKGVGIR LTGRICNTFK AFKLAERGVK PGVTILEEDG
251  FEVLARILTE YSSAPFPRDF CEIH*
```

The cp7028 nucleotide sequence <SEQ ID 198> is:

```
  1  ATGCTTCTAG GGTTTTTGTG TGACTGCCCC TGTGCTTCGT GGCAGTGTGC
 51  GGCCGTTGCT AATTGTTATG ATTCCGTATT TATGTCTAGA CCAGAGCACA
101  AACCTAATAT TCCTTATATT ACTAAAGCTA CAAGACGGGG TCTGCGTATG
151  AAGACGCTTG CTTATCTGGC CTCTTTAAAA GATGCTAGAC AGCTTGCCTA
201  TGATTTTCTG AAAGATCCTG GTTCTTTAGC TCGGTTAGCT AAGGCTTTGA
251  TAGCTCCTAA GGAGGCCTTA CAGGAGGGCA ACCTATTTTT TTATGGCTGT
301  AGTAATATTG AGGATATTTT AGAGGAGATG CGTCGTCCTC ATAGAATCCT
351  TTTGTTAGGA TTTTCTTATT GTCAAAAGCC TAAGGCATGT CCTGAAGGGC
401  GTTTCAATGA TGCTTGTCGG TATGATCCTT CACATCCTAC ATGTGCCTCA
451  TGTTCTATAG GGACCATGAT GCGGCTGAAT GCTCGTAGAT ACACTACTGT
501  GATCATCCCT ACATTTATAG ATATCGCAAA ACATTTACAC ACTTTAAAAA
551  AGCGCTACCC TGGATATCAA ATTCTCTTTG CAGTTACTGC TTGTGAACTT
601  TCCTTAAAAA TGTTTGGAGA TTATGCCTCC GTAATGAACT TAAAGGGTGT
651  GGGCATCAGA CTCACAGGAC GTATTTGCAA TACATTTAAG GCATTTAAAT
701  TAGCTGAGCG AGGAGTCAAA CCAGGAGTCA CTATCCTAGA AGAAGATGGC
751  TTTGAGGTAT TAGCAAGGAT TCTTACAGAA TACAGTAGCG CTCCTTTCCC
801  TAGAGACTTT TGTGAGATCC ATTAG
```

The PSORT algorithm predicts cytoplasm (0.1453).

The protein was expressed in *E.coli* and purified as a GST-fusion product (Figure 99A). The recombinant protein was used to immunise mice, whose sera were used in a Western blot (Figure 99B) and for FACS analysis (Figure 99C).

These experiments show that cp7028 is a surface-exposed and immunoaccessible protein, and that it is a useful immunogen. These properties are not evident from the sequence alone.

## Example 100

The following *C.pneumoniae* protein (PID 4377355) was expressed <SEQ ID 199; cp7355>:

```
  1  MKKVVTLSII FFATYCASEL SAVTVVAVPL SEAPGKIQVR PVVGLQFQEE
 51  QGSVPYSFYY PYDYGYYYPE TYGYTKNTGQ ESRECYTRFE DGTIFYECD*
```

The cp7355 nucleotide sequence <SEQ ID 200> is:

```
  1  ATGAAGAAAG TCGTAACACT ATCCATTATA TTTTTCGCAA CGTATTGTGC
 51  ATCAGAGCTT AGTGCTGTAA CTGTAGTGGC TGTGCCTTTA TCAGAGGCTC
101  CAGGGAAGAT TCAAGTTCGT CCCGTCGTTG GTCTGCAATT TCAAGAAGAA
151  CAGGGTTCTG TGCCCTATAG TTTTTATTAT CCTTATGACT ATGGGTATTA
201  CTATCCAGAG ACTTATGGCT ATACTAAAAA TACAGGTCAA GAAAGTCGCG
251  AATGTTATAC CCGATTTGAA GATGGCACAA TTTTTTATGA ATGCGATTAG
```

The PSORT algorithm predicts inner membrane (0.143).

The protein was expressed in *E.coli* and purified as a GST-fusion (Figure 100A) and a his-tag product. The proteins were used to immunise mice, whose sera were used in a Western blot (Figure 100B) and for FACS analysis (Figure 100C).

These experiments show that cp7355 is a surface-exposed and immunoaccessible protein, and that it is a useful immunogen. These properties are not evident from the sequence alone.

**Example 101**

The following *C.pneumoniae* protein (PID 4377380) was expressed <SEQ ID 201; cp7380>:

```
  1  VHYCERTLDP KYILKIALKL RQSLSLFFQN SQSLQRAYST PYSYYRIILQ
 51  KENKEKQALA RHKCISILEF FKNLLFVHLL SLSKNQREGC STDMAVVSTP
101  FFNRNLWYRL LSSRFSLWKS YCPRFFLDYL EAFGLLSDFL DHQAVIKFFE
151  LETHFSYYPV SGFVAPHQYL SLLQDRYFPI ASVMRTLDKD NFSLTPDLIH
201  DLLGHVPWLL HPSFSEFFIN MGRLFTKVIE KVQALPSKKQ RIQTLQSNLI
251  AIVRCFWFTV ESGLIENHEG RKAYGAVLIS SPQELGHAFI DNVRVLPLEL
301  DQIIRLPFNT STPQETLFSI RHFDELVELT SKLEWMLDQG LLESIPLYNQ
351  EKYLSGFEVL CQ*
```

The cp7380 nucleotide sequence <SEQ ID 202> is:

```
   1  GTGCACTACT GCGAGAGAAC CCTGGACCCA AAGTATATTC TGAAGATTGC
  51  TCTAAAGCTG AGACAATCAC TTTCCCTGTT CTTCCAGAAC AGCCAATCAC
 101  TCCAACGTGC ATACTCGACC CCATATTCCT ACTACCGAAT CATTCTACAA
 151  AAGGAAAATA AAGAGAAGCA AGCTTTAGCT CGACACAAAT GCATTTCTAT
 201  TTTAGAATTT TTCAAAAACT TACTCTTTGT TCATCTTCTG TCATTATCAA
 251  AGAATCAAAG GGAAGGTTGC TCCACTGATA TGGCTGTTGT AAGCACTCCC
 301  TTTTTTAATC GGAATTTATG GTATCGACTC CTTTCCTCAC GGTTTTCTCT
 351  ATGGAAAAGC TATTGTCCAA GATTTTTTCT TGATTACTTA GAAGCTTTCG
 401  GTCTCCTTTC TGATTTCTTA GACCATCAAG CAGTCATTAA ATTCTTCGAA
 451  TTAGAAACAC ATTTTTCCTA TTATCCCGTT TCAGGATTTG TAGCTCCCCA
 501  TCAATACTTG TCTCTGTTGC AGGACCGTTA CTTTCCCATT GCCTCTGTAA
 551  TGCGAACTCT CGATAAAGAT AATTTCTCCT TAACTCCTGA TCTCATCCAT
 601  GACCTTTTAG GGCACGTGCC TTGGCTTCTA CATCCCTCAT TTTCTGAATT
 651  TTTCATAAAC ATGGGAAGAC TCTTCACTAA AGTCATAGAA AAAGTACAAG
 701  CTCTTCCTAG TAAAAAACAA CGCATACAAA CCCTACAAAG CAATCTGATC
 751  GCTATTGTAC GCTGCTTTTG GTTTACTGTT GAAAGCGGAC TTATTGAAAA
 801  CCATGAAGGA AGAAAAGCAT ATGGAGCCGT TCTTATCAGT TCTCCTCAGG
 851  AACTTGGACA CGCTTTCATT GATAACGTAC GTGTTCTCCC TTTAGAATTG
 901  GATCAGATTA TTCGTCTTCC CTTCAATACA TCAACTCCAC AAGAGACTTT
 951  ATTTTCAATA AGACATTTTG ATGAACTGGT AGAACTCACT TCAAAATTAG
1001  AATGGATGCT CGACCAAGGT CTGTTAGAAT CAATTCCCCT TTACAATCAA
1051  GAGAAATATC TTTCTGGTTT TGAGGTACTT TGCCAATGA
```

The PSORT algorithm predicts inner membrane (0.1362).

The protein was expressed in *E.coli* and purified as a GST-fusion product (Figure 101A). The recombinant protein was used to immunise mice, whose sera were used in a Western blot (Figure 101B) and for FACS analysis (Figure 101C).

These experiments show that cp7380 is a surface-exposed and immunoaccessible protein, and that it is a useful immunogen. These properties are not evident from the sequence alone.

**Example 102**

The following *C.pneumoniae* protein (PID 4376904) was expressed <SEQ ID 203; cp6904>:

```
  1  MMNYEDAKLR GQAVAILYQI GAIKFGKHIL ASGEETPLYV DMRLVISSPE
 51  VLQTVATLIW RLRPSFNSSL LCGVPYTALT LATSISLKYN IPMVLRRKEL
101  QNVDPSDAIK VEGLFTPGQT CLVINDMVSS GKSIIETAVA LEENGLVVRE
151  ALVFLDRRKE ACQPLGPQGI KVSSVFTVPT LIKALIAYGK LSSGDLTLAN
201  KISEILEIES *
```

The cp6904 nucleotide sequence <SEQ ID 204> is:

```
  1 ATGATGAACT ACGAAGATGC AAAATTACGC GGTCAAGCTG TAGCAATTCT
 51 ATACCAAATC GGAGCTATAA AGTTCGGAAA ACATATTCTC GCTAGCGGAG
101 AAGAAACTCC TCTGTATGTA GATATGCGTC TTGTGATCTC CTCTCCAGAA
151 GTTCTCCAGA CAGTGGCAAC TCTTATTTGG CGCCTCCGCC CCTCATTCAA
201 TAGTAGCTTA CTCTGCGGAG TCCCTTATAC TGCTCTAACC CTAGCAACCT
251 CGATCTCTTT AAAATATAAC ATCCCTATGG TATTGCGAAG GAAGGAATTA
301 CAGAATGTAG ACCCCTCGGA CGCTATTAAA GTAGAAGGGT TATTTACTCC
351 AGGACAAACT TGTTTAGTCA TCAATGATAT GGTTTCCTCA GGAAAATCTA
401 TAATAGAGAC AGCAGTCGCA CTGGAAGAAA ATGGTCTGGT AGTTCGTGAA
451 GCATTGGTAT TCTTAGATCG TAGAAAAGAA GCGTGTCAAC CACTTGGTCC
501 ACAGGGAATA AAAGTCAGTT CGGTATTTAC TGTACCCACT CTGATAAAAG
551 CTTTGATCGC TTATGGGAAG CTAAGCAGTG GTGATCTAAC CCTGGCAAAC
601 AAAATTTCCG AAATTCTAGA AATTGAATCT TAA
```

The PSORT algorithm predicts cytoplasm (0.0358).

The protein was expressed in *E.coli* and purified as a his-tag product (Figure 102A). The recombinant protein was used to immunise mice, whose sera were used in a Western blot (Figure 102B) and for FACS analysis.

The cp6904 protein was also identified in the 2D-PAGE experiment.

These experiments show that cp6904 is a surface-exposed and immunoaccessible protein, and that it is a useful immunogen. These properties are not evident from the sequence alone.

**Example 103**

The following *C.pneumoniae* protein (PID 4376964) was expressed <SEQ ID 205; cp6964>:

```
 1 MKKLIALIGI FLVPIKGNTN KEHDAHATVL KAARAKYNLF FVQDVFPVHE
51 VIEPISPDCL VHYEGWV*
```

The cp6964 nucleotide sequence <SEQ ID 206> is:

```
  1 ATGAAAAAAT TGATTGCTTT GATAGGGATA TTTCTTGTTC CAATAAAAGG
 51 AAATACCAAT AAGGAACACG ACGCTCACGC GACTGTTTTA AAAGCGGCCA
101 GAGCAAAGTA TAATTTGTTC TTTGTTCAGG ATGTTTTCCC TGTACACGAA
151 GTTATCGAGC CTATTTCTCC CGATTGCCTG GTACATTATG AAGGGTGGGT
201 TTGA
```

The PSORT algorithm predicts inner membrane (0.091).

The protein was expressed in *E.coli* and purified as a GST-fusion product (Figure 103A) and also in his-tagged form. The recombinant proteins were used to immunise mice, whose sera were used in a Western blot (Figure 103B) and for FACS analysis (Figure 103C).

These experiments show that cp6964 is a surface-exposed and immunoaccessible protein, and that it is a useful immunogen. These properties are not evident from the sequence alone.

**Example 104**

The following *C.pneumoniae* protein (PID 4377387) was expressed <SEQ ID 207; cp7387>:

```
  1 LNFAKIDHNH LYLTCLGDLG VACPILSTDC LPNYSEKASH EVLVYSKFRC
 51 ISGEPSRLAT SGNDTYYSIV SLPIGLRYEV TSPSGRHDFN IDMHVAPKIG
101 AVLSHGTREA KEIPGSSKDY AFFSLTARES LMISEKLAMT FQVSEVIQNC
151 YSQCTKVTKT NLKEQYRHLS HNTGFELSVK SAF*
```

The cp7387 nucleotide sequence <SEQ ID 208> is:

```
  1  TTGAATTTTG CAAAGATTGA TCACAATCAT CTCTACCTTA CATGTTTGGG
 51  AGATCTTGGT GTAGCTTGTC CTATACTTTC TACAGATTGT CTACCTAATT
101  ATAGCGAGAA AGCATCTCAT GAGGTTCTTG TTTATAGTAA ATTTAGATGC
151  ATTTCTGGAG AGCCATCTCG ACTTGCAACT TCAGGAAATG ACACATATTA
201  TTCTATAGTA AGTTTACCTA TAGGACTCCG TTACGAAGTG ACTTCACCAT
251  CAGGACGTCA TGATTTCAAT ATTGATATGC ATGTAGCTCC AAAGATAGGT
301  GCAGTACTCT CTCATGGAAC ACGAGAGGCT AAAGAGATCC CAGGATCTTC
351  AAAAGACTAT GCATTTTTTA GCTTGACTGC TAGAGAAAGT TTAATGATTT
401  CTGAAAAGCT TGCGATGACT TTCCAAGTTA GCGAAGTTAT TCAGAATTGT
451  TATTCACAAT GTACTAAAGT AACGAAAACT AATTTAAAAG AACAGTATAG
501  GCACTTATCC CACAATACAG GGTTTGAGTT AAGCGTCAAG TCTGCATTCT
551  AA
```

The PSORT algorithm predicts inner membrane (0.043).

The protein was expressed in *E.coli* and purified as a his-tagged-fusion product (Figure 104A) and also as a GST-fusion (Figure 104B). The recombinant proteins were used to immunise mice, whose sera were used in a Western blot and for FACS analysis (Figure 104C; his-tagged).

These experiments show that cp7387 is a surface-exposed and immunoaccessible protein, and that it is a useful immunogen. These properties are not evident from the sequence alone.

## Example 105

The following *C.pneumoniae* protein (PID 4376281) was expressed <SEQ ID 209; cp6281>:

```
  1  MFLQFFHPIV FSDQSLSFLP YLGKSSGIIE KCSNIVEHYL HLGGDTSVII
 51  TGVSGATFLS VDHALPISKS EKIIKILSYI LILPLILALF IKIVLRIILF
101  FKYRGLILDV KKEDLKKTLT PDQENLSLPL PSPTTLKKIH ALHILVRSGK
151  TYNELIQEGF SFTKITDLGQ APSPKQDIGF SYNSLLPNFY FHSLVSVPNI
201  SGEERALNYH KEQQEEMAVK LKTMQACSFV FRSLHLPSMQ TKDKKAGFGL
251  LTFFPWKIYP L*
```

The cp6281 nucleotide sequence <SEQ ID 210> is:

```
  1  ATGTTTCTTC AGTTTTTTCA TCCTATAGTC TTCTCGGATC AGTCCTTATC
 51  TTTTCTTCCT TACCTAGGAA AAAGCTCTGG CATTATTGAA AAATGTTCCA
101  ATATCGTTGA ACACTATTTA CATTTGGGAG GAGACACTTC TGTTATCATC
151  ACAGGAGTTT CTGGAGCTAC CTTTCTATCT GTTGATCATG CCCTCCCAAT
201  CTCGAAATCT GAAAAAATAA TAAAAATTCT CTCCTATATT TTAATTCTTC
251  CTCTGATTCT AGCTCTCTTT ATTAAGATCG TTTTACGCAT TATCTTATTC
301  TTCAAGTATC GTGGTCTAAT CCTAGATGTT AAGAAGGAGG ATTTGAAAAA
351  AACACTTACA CCTGACCAAG AAAACCTCAG TCTTCCTTTA CCATCTCCTA
401  CAACATTAAA GAAAATTCAT GCGCTACACA TTTTAGTGCG TTCTGGAAAA
451  ACCTATAACG AGCTTATACA AGAAGGGTTT TCTTTCACTA AAATCACAGA
501  TCTTGGTCAA GCTCCTTCAC CAAAGCAAGA TATTGGCTTC TCTTATAATT
551  CCCTTCTCCC TAACTTCTAT TTTCATTCCT TGGTATCTGT TCCAAATATT
601  TCAGGCGAGG AACGGGCTCT TAATTATCAT AAAGAACAAC AAGAGGAAAT
651  GGCTGTTAAA TTAAAAACAA TGCAAGCGTG TTCTTTTGTC TTCCGATCCC
701  TGCATTTACC TTCAATGCAA ACGAAGGACA AAAAGGCTGG ATTTGGACTA
751  CTGACGTTTT TCCCTTGGAA AATCTACCCC CTATAA
```

The PSORT algorithm predicts inner membrane (0.5373).

The protein was expressed in *E.coli* and purified as a GST-fusion product (Figure 105A). The recombinant protein was used to immunise mice, whose sera were used in a Western blot (Figure 105B) and for FACS analysis.

These experiments show that cp6281 is a surface-exposed and immunoaccessible protein, and that it is a useful immunogen. These properties are not evident from the sequence alone.

**Example 106 and Example 107**

The following *C.pneumoniae* protein (PID 4376306) was expressed <SEQ ID 211; cp6306>:

```
  1  MGNHETYIHP GVLPSSHAQD VSRSTVYPSR SFIMRRMLMG WNFNRVPSKS
 51  SEQLMDGHRI PLIFFGKHHP TISILNVNRF SWLSIFYNGE RGF*
```

The cp6306 nucleotide sequence <SEQ ID 212> is:

```
  1  ATGGGAAACC ATGAGACCTA TATACATCCA GGAGTGCTCC CGAGTAGTCA
 51  TGCTCAGGAT GTTAGCAGAT CTACAGTTTA CCCCAGTCGA AGTTTTATCA
101  TGAGACGTAT GCTCATGGGC TGGAATTTCA ATCGTGTTCC CTCGAAGAGC
151  TCCGAGCAGT TAATGGATGG TCATCGCATA CCTCTTATAT TTTTTGGGAA
201  GCATCATCCT ACTATATCTA TTTTAAATGT CAATAGATTT TCTTGGCTCT
251  CCATTTTTTA CAATGGAGAA AGGGGGTTTT GA
```

The PSORT algorithm predicts cytoplasm (0.167).

The following *C.pneumoniae* protein (PID 4376434) was also expressed <SEQ ID 213; cp6434>:

```
  1  MSESINRSIH LEASTPFFIK LTNLCESRLV KITSLVISLL ALVGAGVTLV
 51  VLFVAGILPL LPVLILEIIL ITVLVLLFCL VLEPYLIEKP SKIKELPKVD
101  ELSVVETDST L*
```

The cp6434 nucleotide sequence <SEQ ID 214> is:

```
  1  ATGTCTGAAA GTATTAACAG AAGCATTCAT TTAGAAGCCT CTACACCATT
 51  TTTTATAAAA TTAACGAATC TCTGTGAAAG TAGATTAGTT AAGATCACTT
101  CTCTTGTTAT TTCTCTATTA GCTTTAGTGG GTGCGGGAGT CACTCTTGTG
151  GTTTTATTTG TAGCTGGGAT CCTTCCTTTA CTTCCTGTAC TCATCTTAGA
201  AATTATTTTA ATAACCGTCC TTGTCTTGCT TTTTTGTTTG GTATTGGAAC
251  CTTATTTAAT AGAAAAACCT AGTAAAATAA AGGAACTACC TAAAGTAGAC
301  GAGCTATCTG TAGTAGAAAC GGACAGTACT CTTTAA
```

The PSORT algorithm predicts inner membrane (0.6859).

The proteins were expressed in *E.coli* and purified as his-tag products (Figure 106A; 6306 = lanes 2-4; 6434 = lanes 8-10). The recombinant proteins were used to immunise mice, whose sera were used in Western blots (Figures 106B & 107) and for FACS analysis.

These experiments show that cp6306 & cp6434 are surface-exposed and immunoaccessible proteins, and that they are useful immunogens. These properties are not evident from the sequences alone.

**Example 108**

The following *C.pneumoniae* protein (PID 4377400) was expressed <SEQ ID 215; cp7400>:

```
  1  MRVMRFFCLF FLGFLGSFHC VAEDKGVDLF GVWDDNQITE CDDSYMTEGR
 51  EEVEKVVDA
```

The cp7400 nucleotide sequence <SEQ ID 216> is:

```
   1  GTGAGAGTTA TGAGATTTTT TTGTCTATTT TTTCTTGGGT TCCTAGGATC
  51  TTTTCATTGT GTTGCTGAAG ACAAGGGCGT GGATTTATTT GGAGTCTGGG
 101  ACGATAACCA AATTACAGAG TGTGACGATA GTTACATGAC AGAGGGTCGT
 151  GAAGAGGTTG AAAAGGTAGT GGACGCTTAG
```

The PSORT algorithm predicts periplasmic space (0.924).

The protein was expressed in *E.coli* and purified as a GST-fusion product (Figure 108A). The recombinant protein was used to immunise mice, whose sera were used in a Western blot (Figure 108B) and for FACS analysis.

These experiments show that cp7400 is a surface-exposed and immunoaccessible protein, and that it is a useful immunogen. These properties are not evident from the sequence alone.

**Example 109**

The following *C.pneumoniae* protein (PID 4376395) was expressed <SEQ ID 217; cp6395>:

```
   1  MENAMSSSFV YNGPSWILKT SVAQEVFKKH GKGIQVLLST SVMLFIGLGV
  51  CAFIFPQYLI VFVLTIALLM LAISLVLFLL IRSVRSSMVD RLWCSEKGYA
 101  LHQHENGPFL DVKRVQQILL RSPYIKVRAL WPSGDIPEDP SQAAVLLLSP
 151  WTFFSSVDVE ALLPSPQEKE GKYIDPVLPK LSRIERVSLL VFLSAFTLDD
 201  LNEQGVNPLM NNEEFLFFIN KKAREHGIQD LKHEIMSSLE KTGVPLDPSM
 251  SFQVSQAMFS VYRYLRQRDL TTSELRCFHL LSCFKGDVVH CLASFENPKD
 301  LADSDFLEAC KNVEWGEFIS ACEKALLKNP QGISIKDLKQ FLVR*
```

The cp6395 nucleotide sequence <SEQ ID 218> is:

```
   1  ATGGAGAATG CTATGTCATC ATCGTTTGTG TATAATGGGC CTTCGTGGAT
  51  TTTAAAAACG TCAGTAGCTC AGGAGGTATT TAAAAAGCAC GGTAAGGGGA
 101  TTCAGGTTCT CTTAAGTACT TCAGTGATGC TTTTTATAGG TCTTGGAGTC
 151  TGTGCCTTTA TATTTCCTCA ATATCTGATT GTTTTTGTTT TGACTATAGC
 201  TTTGCTTATG CTCGCTATAA GCTTGGTATT GTTTCTCTTA ATACGTTCTG
 251  TACGCTCTTC AATGGTAGAT CGTTTGTGGT GTTCTGAAAA AGGATATGCT
 301  CTTCATCAAC ATGAGAACGG GCCTTTTTTG GATGTGAAGC GTGTACAGCA
 351  AATTCTTCTA AGATCACCCT ATATTAAAGT TCGGGCTTTA TGGCCGTCTG
 401  GAGATATCCC TGAGGATCCT TCACAAGCTG CGGTTCTATT ACTTTCTCCT
 451  TGGACTTTCT TTTCATCCGT GGATGTAGAG GCTTTATTAC CGAGTCCTCA
 501  AGAAAAGGAG GGTAAGTATA TAGATCCTGT GCTGCCTAAG TTGTCTAGGA
 551  TAGAGAGAGT CTCACTTTTA GTGTTTTTGA GTGCATTTAC TTTGGATGAC
 601  TTAAACGAAC AGGGAGTCAA TCCTTTGATG AATAATGAGG AATTTTTATT
 651  TTTTATAAAT AAGAAAGCGC GTGAGCATGG GATTCAGGAT TTAAAACACG
 701  AGATTATGTC TTCGTTAGAG AAAACAGGAG TGCCATTAGA CCCCTCAATG
 751  AGTTTTCAAG TTTCACAAGC GATGTTTTCT GTATATCGCT ACTTGAGACA
 801  AAGGGATTTA ACGACTTCAG AATTAAGATG TTTTCACCTC TTAAGTTGTT
 851  TTAAAGGGGA TGTGGTTCAT TGTTTAGCTT CATTTGAAAA CCCTAAAGAT
 901  TTAGCAGATT CTGACTTTTT AGAAGCTTGT AAGAACGTGG AATGGGGTGA
 951  GTTTATTTCG GCATGTGAGA AGGCTCTTTT AAAGAATCCG CAAGGAATTT
1001  CCATTAAGGA TCTAAAACAA TTTTTAGTGA GGTAA
```

The PSORT algorithm predicts inner membrane (0.6307).

The protein was expressed in *E.coli* and purified as a GST-fusion product (Figure 109A). The recombinant protein was used to immunise mice, whose sera were used in a Western blot (Figure 109B) and for FACS analysis.

These experiments show that cp6395 is a surface-exposed and immunoaccessible protein, and that it is a useful immunogen. These properties are not evident from the sequence alone.

**Example 110**

The following *C.pneumoniae* protein (PID 4376396) was expressed <SEQ ID 219; cp6396>:

```
  1  MIEFAFVPHT SVTADRIEDR MACRMNKLST LAITSLCVLI SSVCIMIGIL
 51  CISGTVGTYA FVVGIIFSVL ALVACVFFLY FFYFSSEEFK CASSQEFRFL
101  PIPAVVSALR SYEYISQDAI NDVIKDTMQL STLSSLLDPE AFFLEFPYFN
151  SLIVNHSMKE ADRLSREAFL ILLGEITWKD CETKILPWLK DPNITPDDFW
201  KLLKDHFDLK DFKKRIATWI RKAYPEIRLP KKHCLDKSIY KGCCKFLLLS

251  ENDVQYQRLL HKVCYFSGEF PAMVLGLGSE VPMVLGLPKV PKDLTWEMFM
301  ENMPVLLQSK REGHWKISLE DVASL*
```

The cp6396 nucleotide sequence <SEQ ID 220> is:

```
  1  ATGATCGAGT TTGCTTTTGT TCCTCATACC TCCGTGACAG CGGATCGGAT
 51  TGAGGATCGC ATGGCCTGTC GCATGAACAA GTTGTCTACT TTAGCAATTA
101  CAAGTCTTTG TGTATTGATC AGTTCAGTTT GTATTATGAT TGGGATTTTA
151  TGCATTTCTG GAACGGTTGG GACCTATGCA TTTGTTGTAG GAATTATTTT
201  TTCTGTGCTT GCTTTGGTAG CATGTGTTTT CTTTCTTTAT TTCTTTTATT
251  TTTCTTCTGA GGAATTTAAG TGTGCTTCTT CGCAGGAGTT TCGTTTTTTG
301  CCTATACCAG CTGTGGTTTC TGCATTGCGT TCCTATGAAT ACATTTCTCA
351  GGACGCTATC AATGACGTTA TAAAAGATAC GATGCAGTTG TCTACCCTTT
401  CTTCTCTTTT AGATCCCGAA GCTTTTTTCT TAGAATTTCC TTATTTTAAC
451  TCTTTGATAG TGAATCATTC GATGAAGGAA GCGGATCGTT TGTCTCGAGA
501  GGCTTTTTTG ATTTTATTAG GTGAGATTAC TTGGAAGGAT TGTGAAACAA
551  AAATTTTGCC ATGGTTGAAA GATCCTAATA TCACTCCTGA TGATTTCTGG
601  AAGCTATTAA AAGACCATTT CGATTTAAAG GACTTTAAGA AGAGGATCGC
651  CACTTGGATA CGGAAGGCCT ATCCAGAAAT TAGATTACCG AAGAAGCATT
701  GTTTAGATAA GTCTATCTAT AAGGGGTGTT GTAAGTTTTT ATTACTTTCT
751  GAGAATGATG TGCAATATCA GAGGTTATTA CATAAGGTCT GTTATTTCTC
801  TGGGGAGTTT CCTGCCATGG TTTTAGGTTT GGGAAGTGAA GTGCCTATGG
851  TGTTAGGACT CCCTAAGGTT CCCAAGGATC TTACCTGGGA GATGTTTATG
901  GAAAATATGC CTGTTCTTCT GCAAAGCAAA AGAGAGGGGC ATTGGAAAAT
951  CTCCTTGGAA GACGTAGCCT CTCTTTAA
```

The PSORT algorithm predicts inner membrane (0.6095).

The protein was expressed in *E.coli* and purified as a GST-fusion product (Figure 110A). The recombinant protein was used to immunise mice, whose sera were used in a Western blot (Figure 110B) and for FACS analysis.

These experiments show that cp6396 is a surface-exposed and immunoaccessible protein, and that it is a useful immunogen. These properties are not evident from the sequence alone.

## Example 111

The following *C.pneumoniae* protein (PID 4376408) was expressed <SEQ ID 221; cp6408>:

```
  1  MNTSLKRPLK SHFDVVGSFL RPEHLKKTRE SLKEGSISLD QLMQIEDIAI
 51  QDLIKKQKAA GLSFITDGEF RRATWHYDFM WGFHGVGHHR ATEGVFFDGE
101  RAMIDDTYLT DKISVSHHPF VDHFKFVKAL EDEFTTAKQT LPAPAQFLKQ
151  MIFPNNIEVT RKFYPTNQEL IEDIVAGYRK VIRDLYDAGC RYLQLDDCTR
201  GGLVDPRVCS WYGIDEKGLQ DLIQQYLLIN NLVIADRPDD LVVNLHVCRG
251  NYHSKFFASG SYDFIAKPLF EQTNVDGYYL EFDHERSGDF SPLTFISGEK
301  TVCLGLVTSK TPTLENKDEV IARIHQAADY LPLERLSLSP QCGFASCEIG
351  NKLTEEEQWA KVALVKEISE EVWK*
```

The cp6408 nucleotide sequence <SEQ ID 222> is:

```
   1  ATGAATACTT CACTAAAAAG ACCTCTGAAA TCTCATTTTG ATGTTGTCGG
  51  TAGTTTTTTG CGTCCTGAGC ATTTAAAAAA AACTAGAGAA AGCCTTAAAG
 101  AAGGCTCTAT TTCTCTAGAT CAACTCATGC AAATTGAGGA TATCGCTATC
 151  CAAGATTTGA TCAAAAAACA AAAAGCAGCA GGTCTTTCTT TTATTACTGA
 201  TGGAGAATTC CGCAGAGCTA CGTGGCATTA CGACTTCATG TGGGGTTTTC
 251  ATGGCGTAGG TCACCACAGA GCTACAGAAG GAGTTTTCTT TGATGGAGAA
 301  CGCGCTATGA TCGATGATAC CTATCTGACA GACAAGATCT CTGTATCTCA
 351  CCACCCATTT GTGGATCACT TTAAATTTGT AAAAGCTCTA GAAGATGAAT
 401  TTACGACTGC AAAGCAAACT CTTCCTGCAC CGGCACAGTT TTTAAAGCAG
 451  ATGATCTTCC CTAATAATAT AGAGGTCACA CGTAAATTCT ATCCTACAAA
 501  TCAGGAGCTA ATTGAAGATA TTGTTGCAGG TTATCGTAAA GTCATTCGCG
 551  ATCTTTATGA TGCTGGCTGC CGCTATCTCC AATTAGATGA CTGTACTCGG
 601  GGAGGTTTAG TAGACCCTCG AGTCTGTTCG TGGTATGGTA TCGATGAAAA
 651  AGGTCTTCAA GATCTGATTC AACAATATCT TCTGATTAAT AATCTTGTAA
 701  TTGCAGATCG TCCCGATGAT CTAGTCGTTA ATTTACATGT ATGCCGTGGG
```

```
 751  AACTACCACT CAAAATTCTT TGCTAGTGGT AGTTATGACT TTATTGCAAA
 801  GCCCCTATTC GAACAAACAA ATGTAGACGG CTACTATTTA GAGTTTGATC
 851  ATGAGCGTTC TGGAGACTTC TCTCCTCTCA CCTTCATTTC TGGAGAAAAA
 901  ACTGTCTGCT TAGGTCTTGT TACCAGCAAA ACCCCTACAC TTGAAAATAA
 951  GGATGAGGTC ATTGCTCGCA TACATCAAGC AGCAGACTAC CTGCCCTTGG
1001  AAAGACTCTC TCTAAGTCCA CAGTGTGGTT TTGCTTCATG TGAAATAGGA
1051  AATAAATTAA CAGAAGAAGA GCAATGGGCT AAAGTTGCTC TAGTAAAAGA
1101  AATTTCCGAA GAAGTTTGGA AATAA
```

The PSORT algorithm predicts cytoplasm (0.2171).

The protein was expressed in *E.coli* and purified as a GST-fusion product (Figure 111A) and also as a his-tagged product. The his-tag protein was used to immunise mice, whose sera were used in a Western blot (Figure 111B) and for FACS analysis.

These experiments show that cp6408 is a surface-exposed and immunoaccessible protein, and that it is a useful immunogen. These properties are not evident from the sequence alone.

**Example 112**

The following *C.pneumoniae* protein (PID 4376430) was expressed <SEQ ID 223; cp6430>:

```
  1  MKLYSISSDV DTPWIFQLMS KVDSYLFLGG NRIKVVSIVM QEPNLIIGKV
 51  ENVRISTIVK ILKILSFLIF PLILIALALH YFLHAKYANH LLVSKILERA
101  PQYVPIPGRS GDTASHYKLT TLVPVSQKNL QAMGSNPLEV EAALRTTKPS
151  FFCVPAKYRQ IIISSHGIRF SLDLEQLADD INLDSVSWPT EYLNSTMDFC
201  SKADKRVIQN VQNLRTGTYI NSVGKRSLLK FMLQHLFIDG ITQENPEALP
251  NNTSGRLTLF PSVRYIYSHF TPQNPTIWPQ VFFRQGPLDE DRGGGFEILE
301  QLQELGVRFP ICPSQGPDNP NFQGFQGIRI YWEDSYQPNK EV*
```

The cp6430 nucleotide sequence <SEQ ID 224> is:

```
   1  ATGAAACTTT ATAGCATCTC TTCAGATGTA GATACACCTT GGATATTTCA
  51  GCTTATGTCA AAGGTAGATT CTTATCTTTT CTTAGGCGGG AATAGAATCA
 101  AGGTTGTATC TATAGTTATG CAAGAACCTA ACTTAATTAT TGGAAAAGTA
 151  GAAAACGTTC GGATCTCCAC AATAGTGAAA ATATTAAAGA TTTTATCCTT
 201  CTTAATCTTC CCTCTGATTT TAATCGCTTT AGCCCTACAC TATTTTCTAC
 251  ATGCTAAATA TGCTAATCAC TTACTTGTAT CTAAGATTTT AGAAAGAGCT
 301  CCTCAGTATG TGCCTATTCC TGGTCGTTCA GGAGACACGG CGTCTCATTA
 351  TAAATTAACA ACATTGGTTC CAGTATCCCA AAAAAATCTA CAAGCTATGG
 401  GATCAAATCC TCTAGAAGTT GAAGCGGCTC TTCGAACTAC AAAACCCTCT
 451  TTTTTCTGTG TACCTGCAAA ATACCGTCAG ATTATAATTT CAAGTCACGG
 501  CATTCGCTTT TCTTTAGATC TTGAACAACT TGCTGATGAC ATTAATTTAG
 551  ATTCGGTTTC CTGGCCTACG GAGTATCTTA ACTCTACTAT GGATTTTTGC
 601  AGCAAGGCAG ATAAACGTGT TATACAGAAT GTACAAAATC TGCGGACAGG
 651  AACTTACATA AATTCTGTAG GAAAGCGTAG CCTTTTAAAA TTCATGTTAC
 701  AGCACCTATT TATTGATGGG ATCACACAAG AAAACCCTGA AGCCCTTCCT
 751  AACAATACAT CTGGAAGACT GACTCTATTC CCTAGTGTTC GTTATATCTA
 801  TTCTCATTTT ACTCCACAAA ATCCTACAAT ATGGCCGCAA GTCTTTTTCA
 851  GACAAGGTCC TCTAGATGAA GATCGAGGAG GAGGATTTGA GATCTTAGAG
 901  CAATTACAAG AGTTAGGAGT TAGGTTTCCA ATTTGCCCCT CTCAAGGACC
 951  AGACAATCCT AATTTTCAAG GTTTTCAAGG GATTCGTATC TATTGGGAAG
1001  ATTCCTATCA ACCCAATAAG GAGGTTTAA
```

The PSORT algorithm predicts inner membrane (0.5140).

The protein was expressed in *E.coli* and purified as a GST-fusion product (Figure 112A). The recombinant protein was used to immunise mice, whose sera were used in a Western blot (Figure 112B) and for FACS analysis.

These experiments show that cp6430 is a surface-exposed and immunoaccessible protein, and that it is a useful immunogen. These properties are not evident from the sequence alone.

### Example 113

The following *C.pneumoniae* protein (PID 4376439) was expressed <SEQ ID 225; cp6439>:

```
  1  MSYDTLFKNL EKEDSVHKIC NEIFALVPRL NTIACTEAII KNLPKADIHV
 51  HLPGTITPQL AWILGVKNGF LKWSYNSWTN HRLLSPKNPH KQYSNIFRNF
101  QDICHEKDPD LSVLQYNILN YDFNSFDRVM ATVQGHRFPP GGIQNEEDLL
151  LIFNNYLQQC LDDTIVYTEV QQNIRLAHVL YPSLPEKHAR MKFYQILYRA
201  SQTFSKHGIT LRFLNCFNKT FAPQINTQEP AQEAVQWLQE VDSTFPGLFV
251  GIQSAGSESA PGACPKRLAS GYRNAYDSGF GCEAHAGEGI ETRTIFSSAK
301  VNPEGLIEIT RVTFSSLKRK QPSSLPIRVT CQLG*
```

The cp6439 nucleotide sequence <SEQ ID 226> is:

```
   1  ATGTCTTATG ATACGTTATT CAAGAATCTT GAAAAGGAAG ATTCTGTACA
  51  TAAGATATGC AATGAGATCT TTGCATTAGT ACCACGACTC AATACAATCG
 101  CTTGCACCGA AGCTATCATC AAAAACCTCC CCAAAGCAGA TATCCATGTA
 151  CACCTTCCTG GGACCATAAC ACCTCAATTA GCTTGGATTT TAGGTGTGAA
 201  AAATGGGTTC TTAAAATGGT CTTATAATTC TTGGACCAAT CATCGATTAC
 251  TTTCTCCTAA GAATCCTCAT AAACAATACT CCAATATTTT CCGAAACTTT
 301  CAAGATATCT GTCACGAAAA GGATCCGGAT TTAAGTGTAT TACAATATAA
 351  TATCTTAAAT TACGATTTTA ATAGCTTTGA TAGAGTGATG GCTACAGTAC
 401  AAGGACATCG CTTTCCTCCT GGAGGAATCC AAAATGAAGA AGACCTTCTT
 451  CTCATTTTCA ATAACTATCT CCAGCAATGT CTGGACGATA CTATCGTGTA
 501  TACTGAAGTA CAACAAAATA TCCGCCTTGC CCATGTTTTG TATCCTTCAT
 551  TACCTGAAAA GCACGCGCGT ATGAAGTTTT ATCAAATCTT GTATCGTGCT
 601  TCGCAAACGT TTTCAAAACA CGGGATTACT TTACGATTTT TAAACTGCTT
 651  CAATAAAACA TTTGCTCCAC AAATAAACAC ACAAGAACCT GCCCAAGAAG
 701  CTGTTCAATG GCTCCAAGAG GTTGATTCTA CATTTCCTGG TCTATTTGTA
 751  GGGATACAAT CCGCAGGATC AGAATCTGCG CCCGGAGCCT GTCCTAAGCG
 801  ATTAGCTTCT GGATATAGAA ATGCTTATGA CTCAGGGTTT GGTTGTGAAG
 851  CTCATGCTGG AGAAGGCATA GAGACCCGGA CTATTTTTTC GTCAGCTAAG
 901  GTAAATCCAG AGGGATTGAT CGAGATAACC CGAGTGACTT TCTCGTCTCT
 951  TAAACGAAAA CAGCCATCTA GTTTACCCAT AAGAGTTACT TGCCAGTTAG
1001  GATAA
```

The PSORT algorithm predicts cytoplasm (0.1628).

The protein was expressed in *E.coli* and purified as a GST-fusion product (Figure 113A). The recombinant protein was used to immunise mice, whose sera were used in a Western blot (Figure 113B) and for FACS analysis.

These experiments show that cp6439 is a surface-exposed and immunoaccessible protein, and that it is a useful immunogen. These properties are not evident from the sequence alone.

**Example 114**

The following *C.pneumoniae* protein (PID 4376440) was expressed <SEQ ID 227; cp6440>:

```
  1  LQSARRHLNT IFILDFGSQY TYVLAKQVRK LFVYCEVLPW NISVQCLKER
 51  APLGIILSGG PHSVYENKAP HLDPEIYKLG IPILAICYGM QLMARDFGGT
101  VSPGVGEFGY TPIHLYPCEL FKHIVDCESL DTEIRMSHRD HVTTIPEGFN
151  VIASTSQCSI SGIENTKQRL YGLQFHPEVS DSTPTGNKIL ETFVQEICSA
201  PTLWNPLYIQ QDLVSKIQDT VIEVFDEVAQ SLDVQWLAQG TIYSDVIESS
251  RSGHASEVIK SHHNVGGLPK NLKLKLVEPL RYLFKDEVRI LGEALGLSSY
301  LLDRHPFPGP GLTIRVIGEI LPEYLAILRR ADLIFIEELR KAKLYDKISQ
351  AFALFLPIKS VSVKGDCRSY GYTIALRAVE STDFMTGRWA YLPCDVLSSC
401  SSRIINEIPE VSRVVYDISD KPPATIEWE*
```

The cp6440 nucleotide sequence <SEQ ID 228> is:

```
  1  TTGCAGAGTG CAAGGAGACA TTTGAACACC ATATTTATTC TAGATTTTGG
 51  ATCTCAATAT ACTTATGTAT TAGCAAAGCA AGTGCGGAAG TTATTTGTAT
101  ATTGCGAAGT TCTTCCCTGG AATATCTCTG TGCAATGTTT AAAAGAAAGA
151  GCGCCTTTGG GGATCATTCT CTCAGGAGGT CCTCACTCTG TCTATGAAAA
```

```
 201  CAAGGCTCCA CATTTAGATC CTGAAATCTA TAAACTTGGC ATTCCAATTC
 251  TAGCTATTTG CTATGGCATG CAGCTTATGG CTAGAGATTT TGGAGGGACT
 301  GTAAGCCCTG GTGTAGGAGA ATTTGGATAT ACGCCCATCC ATCTGTATCC
 351  TTGTGAGCTC TTCAAACACA TCGTCGACTG CGAATCTCTA GACACAGAGA
 401  TTCGGATGAG CCATCGGGAT CATGTTACGA CAATTCCTGA AGGATTTAAT
 451  GTAATCGCAT CCACCTCACA ATGCTCGATC TCAGGAATAG AAAATACCAA
 501  ACAACGGTTG TACGGGCTGC AATTTCATCC CGAGGTTTCT GACTCCACTC
 551  CAACGGGAAA TAAGATTCTA GAAACTTTTG TTCAAGAGAT CTGTTCTGCT
 601  CCCACACTAT GGAATCCCTT GTATATTCAG CAAGACCTTG TAAGTAAAAT
 651  TCAAGATACC GTTATTGAAG TATTTGATGA AGTCGCTCAG TCATTAGACG
 701  TACAATGGTT AGCTCAAGGA ACCATCTACT CAGATGTTAT TGAGTCCTCA
 751  CGCTCTGGAC ATGCCTCCGA AGTAATAAAA TCACATCATA ATGTAGGGGG
 801  GCTTCCAAAA AATCTTAAGC TGAAGTTAGT CGAGCCCTTA CGTTATTTAT
 851  TTAAAGATGA AGTTCGAATT TTAGGAGAAG CCCTAGGACT TTCTAGCTAT
 901  CTCTTGGACA GGCATCCTTT TCCTGGACCT GGCTTGACAA TTCGTGTGAT
 951  TGGAGAGATC CTTCCTGAAT ATCTAGCCAT TTTACGACGG GCGGACCTCA
1001  TCTTTATAGA AGAGCTTAGG AAAGCAAAAC TCTACGATAA AATAAGCCAA
1051  GCCTTTGCTC TATTTCTTCC TATAAAATCA GTATCTGTAA AAGGAGATTG
1101  TAGAAGCTAT GGTTATACCA TAGCATTACG TGCTGTAGAA TCTACAGATT
1151  TCATGACAGG ACGATGGGCC TACCTTCCAT GCGATGTTCT CAGTTCTTGC
1201  TCATCGCGAA TTATTAATGA AATACCCGAG GTAAGCCGAG TGGTCTATGA
1251  TATTTCTGAC AAGCCACCAG CAACTATAGA ATGGGAATAG
```

The PSORT algorithm predicts cytoplasm (0.0481).

The protein was expressed in *E.coli* and purified as a GST-fusion product (Figure 114A) and also as a his-tagged product. The recombinant proteins were used to immunise mice, whose sera were used in a Western blot (Figure 114B) and for FACS analysis.

These experiments show that cp6440 is a surface-exposed and immunoaccessible protein, and that it is a useful immunogen. These properties are not evident from the sequence alone.

## Example 115

The following *C.pneumoniae* protein (PID 4376475) was expressed <SEQ ID 229; cp6475>:

```
  1  MNTYTFSPTL QKSFSLFLLE KLDSYFFFGG TRTQILVITP TNIRLAAKKR
 51  GCKVSTIEKI IKILSFILLP LVIIAFILRY FLHKKFDKQF LCIPKVISNE
101  DEALLGSRPQ AVEKAVREIS PAFFSIPRKY QLIRIDTPKD DAPSILFPIG
151  IEIILKDLCI DTLKQSNLFL KREMDFLGHP EEKALFDSIC SIEKDQEWMS
201  LESKKLLITH FLKYLFVSGI EQLNPGFNPE NGRGYFSEIS TAKIHFHQHG
251  RYGPIRSSGP IMKEI*
```

The cp6475 nucleotide sequence <SEQ ID 230> is:

```
  1  ATGAATACCT ATACCTTCTC TCCTACACTT CAGAAAAGCT TCAGCCTATT
 51  TCTTTTAGAA AAATTAGACT CTTACTTTTT CTTTGGAGGG ACTCGTACAC
101  AAATCTTAGT CATCACACCA ACCAATATTA GATTAGCAGC TAAAAAAAGA
151  GGGTGTAAGG TTTCTACTAT AGAAAAGATA ATCAAGATCC TCTCTTTTAT
201  CCTGCTGCCC CTAGTTATCA TTGCCTTTAT ACTTCGCTAT TTCTTACATA
251  AGAAATTCGA TAAACAGTTC TTGTGTATCC CAAAAGTCAT TTCTAACGAA
301  GACGAAGCTC TTCTTGGATC TAGACCACAA GCAGTTGAAA AAGCAGTTCG
351  AGAAATATCT CCAGCCTTCT TCTCTATACC AAGAAAATAC CAACTTATTA
401  GAATCGACAC TCCTAAAGAT GACGCTCCCT CAATCCTTTT CCCTATAGGC
451  ATAGAGATCA TTCTCAAAGA TTTATGTATT GATACACTCA AGCAATCTAA
501  TCTTTTCCTT AAAAGAGAAA TGGATTTCTT AGGTCATCCA GAAGAAAAAG
551  CATTATTCGA CTCGATATGT TCTATAGAAA AAGATCAAGA ATGGATGAGC
601  TTGGAAAGTA AAAAACTTTT AATCACGCAC TTCCTAAAGT ATCTCTTTGT
651  CTCTGGAATC GAACAACTAA ATCCAGGCTT TAACCCAGAG AATGGGCGTG
701  GGTATTTTTC AGAAATAAGT ACAGCAAAGA TCCATTTTCA TCAGCACGGT
751  CGATATGGGC CAATCCGTTC TTCGGGACCC ATCATGAAGG AAATATAA
```

The PSORT algorithm predicts inner membrane (0.5373).

The protein was expressed in *E.coli* and purified as a GST-fusion product (Figure 115A). The recombinant protein was used to immunise mice, whose sera were used in a Western blot (Figure 115B) and for FACS analysis.

These experiments show that cp6475 is a surface-exposed and immunoaccessible protein, and that it is a useful immunogen. These properties are not evident from the sequence alone.

**Example 116**

The following *C.pneumoniae* protein (PID 4376482) was expressed <SEQ ID 231; cp6482>:

```
  1  MLVELEALKR EFAHLKDQKP TSDQEITSLY QCLDHLEFVL LGLGQDKFLK
 51  ATEDEDVLFE SQKAIDAWNA LLTKARDVLG LGDIGAIYQT IEFLGAYLSK
101  VNRRAFCIAS EIHFLKTAIR DLNAYYLLDF RWPLCKIEEF VDWGNDCVEI
151  AKRKLCTFEK ETKELNESLL REEHAMEKCS IQDLQRKLSD IIIELHDVSL
201  FCFSKTPSQE EYQKDCLYQS RLRYLLLLYE YTLLCKTSTD FQEQARAKEE
251  FIREKFSLLE LEKGIKQTKE LEFAIAKSKL ERGCLVMRKY EAAAKHSLDS
301  MFEEETVKSP RKDTE*
```

The cp6482 nucleotide sequence <SEQ ID 232> is:

```
  1  ATGCTAGTAG AGTTAGAGGC TCTTAAAAGA GAGTTTGCGC ATTTAAAAGA
 51  CCAGAAGCCG ACAAGTGACC AAGAGATCAC TTCACTTTAT CAATGTTTGG
101  ATCATCTTGA ATTCGTTTTA CTCGGGCTGG GCCAGGACAA ATTTTTAAAG
151  GCTACGGAAG ATGAAGATGT GCTTTTTGAG TCTCAAAAAG CAATCGATGC
201  GTGGAATGCT TTATTGACAA AAGCCAGAGA TGTTTTAGGT CTTGGGGACA
251  TAGGTGCTAT CTATCAGACT ATAGAATTCT TGGGTGCCTA TTTATCAAAA
301  GTGAATCGGA GGGCTTTTTG TATTGCTTCG GAGATACATT TTCTAAAAAC
351  AGCAATCCGA GATTTGAATG CATATTACCT GTTAGATTTT AGATGGCCTC
401  TTTGCAAGAT AGAAGAGTTT GTGGATTGGG GGAATGATTG TGTTGAAATA
451  GCAAAGAGGA AGCTATGCAC TTTTGAAAAA GAAACCAAGG AGCTCAATGA
501  GAGCCTTCTT AGAGAGGAGC ATGCGATGGA GAAATGCTCG ATTCAAGATC
551  TGCAAAGGAA ACTTAGCGAC ATTATTATTG AATTGCATGA TGTTTCTCTT
601  TTTTGTTTTT CTAAGACTCC CAGTCAAGAG GAGTATCAAA AGGATTGTTT
651  GTATCAATCA CGATTGAGGT ACTTATTGTT GCTGTATGAG TATACATTGT
701  TATGTAAGAC ATCCACAGAT TTTCAAGAGC AGGCTAGGGC TAAAGAGGAG
751  TTCATTAGGG AGAAATTCAG CCTTCTAGAG CTCGAAAAGG GAATAAAACA
801  AACTAAAGAG CTTGAGTTTG CAATTGCTAA AAGTAAGTTA GAACGGGGCT
851  GTTTAGTTAT GAGGAAGTAT GAAGCTGCCG CTAAACATAG TTTAGATTCT
901  ATGTTCGAAG AAGAAACTGT GAAGTCGCCG CGGAAAGACA CAGAATAA
```

The PSORT algorithm predicts cytoplasm (0.4607).

The protein was expressed in *E.coli* and purified as a GST-fusion product (Figure 116A). The recombinant protein was used to immunise mice, whose sera were used in a Western blot (Figure 116B) and for FACS analysis.

These experiments show that cp6482 is a surface-exposed and immunoaccessible protein, and that it is a useful immunogen. These properties are not evident from the sequence alone.

**Example 117**

The following *C.pneumoniae* protein (PID 4376486) was expressed <SEQ ID 233; cp6486>:

```
  1  VVVVALFILG IFFLSGSLAF LVHTSCGVLL GAALPILCIG LVLLAVALIV
 51  FLCHKHKTRQ DLDYYDQDLD SLVIHKKEIP NDISELRVTF EKLQNLFQFH
101  TKDFSDLSQE LQGKFINCME KWLTLEDEVT KFLIVRDRFL ETRRNFTTFG
151  EQVKGIQSNI FDLHEEKSSL YLELYRLRKD LQVLLNFFLL PPGILKVDYD
201  EIEAIKGLFI RLTSRLDKLD VKAQERKKFI NEMSREFKEV EKAFDIVDRA
251  TKKLMDRAKK ESPARLFMGR TESLLEMKKN EEALKNQGLD PENLSHPELF
301  SPYQQLLILN YLNSEIVLHH YEFLISGTVT SGLTLEECEN RMRAASTGLN

351  ALLVRKLQFR GAIKSAYFEK LTEIEKELRS LQDVIKSLEL ELIHKIKDIV
401  TEET*
```

The cp6486 nucleotide sequence <SEQ ID 234> is:

```
   1  GTGGTGGTTG TCGCTTTATT TATCCTTGGG ATTTTCTTTT TATCTGGTTC
  51  TCTTGCATTC CTTGTTCATA CGTCTTGCGG AGTTCTTTTA GGAGCGGCGC
 101  TTCCCATACT TTGCATAGGT CTTGTTTTAT TGGCTGTAGC TCTTATTGTT
 151  TTCTTATGTC ACAAACACAA GACTCGTCAA GATTTAGATT ATTATGATCA
 201  AGATTTAGAT TCTTTGGTGA TTCATAAGAA AGAGATCCCC AATGACATCT
 251  CTGAGTTGCG GGTAACATTT GAAAAGTTGC AAAATCTGTT TCAGTTCCAT
 301  ACGAAAGATT TCTCTGATCT AAGCCAAGAG CTTCAGGGTA AATTTATCAA
 351  TTGCATGGAG AAATGGCTAA CTTTAGAAGA CGAAGTGACT AAATTTCTTA
 401  TTGTTCGAGA TAGATTTTTA GAAACCAGAA GAAATTTTAC CACTTTTGGA
 451  GAACAGGTTA AAGGGATCCA AAGCAATATT TTTGATTTGC ATGAGGAAAA
 501  GTCTTCATTA TATTTAGAAT TGTATAGGCT TAGGAAAGAC CTCCAAGTTC
 551  TATTAAATTT TTTTCTGCTC CCCCCAGGTA TACTCAAGGT AGATTATGAT
 601  GAAATTGAGG CTATCAAAGG TCTGTTTATA AGATTAACCT CTAGATTAGA
 651  TAAGCTTGAT GTGAAAGCTC AGGAACGTAA GAAGTTCATT AATGAAATGA
 701  GTAGGGAATT TAAAGAAGTA GAGAAAGCTT TTGATATTGT CGATAGGGCA
 751  ACAAAAAAGC TTATGGATAG AGCCAAGAAA GAAAGTCCGG CACGTCTTTT
 801  CATGGGTAGA ACTGAGTCTC TCTTAGAAAT GAAAAAAAAT GAAGAAGCCC
 851  TTAAAAATCA GGGGCTAGAT CCTGAAAATC TTTCCCATCC TGAACTTTTT
 901  AGTCCGTATC AACAGCTTTT AATTTTGAAT TATTTAAATA GCGAAATAGT
 951  TCTGCATCAT TATGAGTTCC TTATTTCTGG AACAGTAACT TCTGGCCTAA
1001  CTCTTGAAGA ATGTGAAAAT CGAATGAGGG CGGCTTCTAC TGGGTTGAAC
1051  GCCCTTCTGG TGCGTAAGCT CCAGTTCAGA GGTGCTATAA AATCTGCGTA
1101  TTTTGAAAAA CTCACAGAGA TTGAAAAAGA GTTACGATCA CTTCAAGACG
1151  TAATAAAGTC ATTGGAACTA GAACTGATCC ATAAGATAAA AGATATAGTG
1201  ACAGAAGAAA CTTAG
```

The PSORT algorithm predicts inner membrane (0.7474).

The protein was expressed in *E.coli* and purified as a GST-fusion product (Figure 117A). The recombinant protein was used to immunise mice, whose sera were used in a Western blot (Figure 117B) and for FACS analysis.

These experiments show that cp6486 is a surface-exposed and immunoaccessible protein, and that it is a useful immunogen. These properties are not evident from the sequence alone.

**Example 118**

The following *C.pneumoniae* protein (PID 4376526) was expressed <SEQ ID 235; cp6526>:

```
  1 MSPFKKIVNR LLCYISFQKE SRTLPIIIRE PRMTTKSLGS FNSVISKNKI
 51 HFISLGCSRN LVDSEVMLGI LLKAGYESTN EIEDADYLIL NTCAFLKSAR
101 DEAKDYLDHL IDVKKENAKI IVTGCMTSNH KDELKPWMSH IHYLLGSGDV
151 ENILSAIESR ESGEKISAKS YIEMGEVPRQ LSTPKHYAYL KVAEGCRKRC
201 AFCIIPSIKG KLRSKPLDQI LKEFRILVNK SVKEIILIAQ DLGDYGKDLS
251 TDRSSQLESL LHELLKEPGD YWLRMLYLYP DEVSDGIIDL MQSNPKLLPY
301 VDIPLQHIND RILKQMRRTT SREQILGFLE KLRAKVPQVY IRSSVIVGFP
351 GETQEEFQEL ADFIGEGWID NLGIFLYSQE ANTPAAELPD QIPEKVKESR
401 LKILSQIQKR NVDKHNQKLI GEKIEAVIDN YHPETNLLLT ARFYGQAPEV
451 DPCIIVNEAK LVSHFGERCF IEITGTAGYD LVGRVVKKSQ NQALLKTSKA
501 *
```

The cp6526 nucleotide sequence <SEQ ID 236> is:

```
   1 ATGAGTCCTT TTAAGAAAAT AGTAAATCGC TTACTATGCT ATATTTCTTT
  51 TCAAAAAGAA TCAAGAACTC TCCCAATCAT TATTAGAGAA CCTAGGATGA
 101 CAACAAAAAG TTTAGGATCT TTCAATTCAG TTATTTCCAA AAATAAAATT
 151 CATTTTATTA GTTTGGGATG CTCTCGGAAC CTTGTAGATA GCGAAGTCAT
 201 GCTAGGCATT CTTCTTAAGG CAGGTTACGA GTCTACTAAT GAAATTGAAG
 251 ATGCTGACTA TTTAATTTTA AATACCTGTG CGTTTTTAAA AAGTGCTAGA
 301 GATGAAGCTA AAGATTATCT AGACCATCTA ATTGATGTAA AAAAAGAGAA
 351 CGCTAAAATT ATTGTAACTG GATGCATGAC TTCCAACCAC AAAGATGAGC
 401 TTAAACCCTG GATGTCACAC ATCCATTACC TACTAGGTTC TGGGGATGTT
 451 GAGAATATTC TTTCTGCTAT TGAGTCTCGT GAATCTGGAG AAAAAATCTC
 501 TGCAAAGAGT TACATTGAGA TGGGAGAAGT TCCAAGACAG CTTTCCACAC
 551 CAAAACACTA TGCCTATTTA AAAGTTGCTG AGGGCTGTAG AAAACGTTGT
 601 GCTTTTTGTA TTATTCCTTC CATTAAAGGA AAGCTCCGCA GCAAACCTCT
 651 GGATCAAATT CTTAAAGAAT TCCGCATCCT TGTAAACAAG AGTGTGAAAG
 701 AGATTATATT GATAGCTCAA GACCTAGGAG ATTATGGAAA GGATCTCTCT
 751 ACAGACCGCA GTTCGCAGCT AGAATCACTA TTACATGAGT TACTGAAAGA
 801 GCCTGGTGAT TATTGGCTGC GGATGTTGTA TTTATATCCT GATGAAGTGA
 851 GTGATGGCAT TATAGATCTT ATGCAATCTA ATCCCAAACT TCTTCCCTAT
 901 GTAGATATTC CCTTACAGCA CATTAACGAC CGTATTTTAA AGCAAATGCG
 951 AAGAACGACT TCTAGGGAGC AAATCCTAGG ATTCCTAGAA AAATTACGTG
1001 CCAAGGTTCC TCAGGTCTAT ATCCGTTCTT CTGTTATTGT GGGTTTCCCC
1051 GGTGAAACTC AGGAAGAATT CCAGGAGTTA GCTGATTTTA TTGGTGAGGG
1101 TTGGATTGAT AATCTCGGAA TTTTCTTGTA CTCTCAAGAA GCGAATACCC
1151 CGGCAGCAGA ACTCCCTGAC CAGATACCAG AAAAAGTTAA AGAATCGAGG
1201 TTGAAAATTC TATCTCAAAT TCAGAAACGC AATGTGGATA AACATAATCA
1251 GAAGCTCATT GGGGAAAAAA TAGAAGCAGT TATTGATAAC TATCATCCTG
1301 AAACGAATCT TTTACTCACT GCAAGGTTCT ATGGACAAGC TCCTGAAGTG
1351 GACCCTTGTA TTATTGTAAA TGAGGCGAAG CTTGTTTCTC ATTTTGGAGA
1401 AAGATGCTTT ATAGAAATCA CAGGGACTGC TGGTTACGAC CTTGTAGGGC
1451 GTGTTGTAAA AAAATCTCAG AACCAAGCTT TGCTAAAAAC TAGCAAAGCT
1501 TAG
```

The PSORT algorithm predicts cytoplasm (0.1296).

The protein was expressed in *E.coli* and purified as a GST-fusion product (Figure 118A) and also as a his-tagged product. The recombinant proteins were used to immunise mice, whose sera were used in a Western blot (Figure 118B) and for FACS analysis.

These experiments show that cp6526 is a surface-exposed and immunoaccessible protein, and that it is a useful immunogen. These properties are not evident from the sequence alone.

**Example 119**

The following *C.pneumoniae* protein (PID 4376528) was expressed <SEQ ID 237; cp6528>:

```
  1  MKNNINNNEC YFKLDSTVDG DLLAANLKTF DTQAQGISST ETFSVQGNAT
 51  FKDQVSATGL TSGTTYNLNA QNFTSSQISI DFKNNRLSNC ALPKEDCDPV
101  PANYVRSPEY FFCSKPLIGD FDFNSGESYL PLTGSEYTLY QSRNVNSIFR
151  FIGWKQSTRE LTVGGNTAIQ FLAAGTYIVS FTVGKRWGWN NGWGGAIYIN
201  NGLGQVQCES TIYSGGGYAT IGTLGTSIYR ASVDVAPNPN DPNASDRYRA
251  GIFYLSNGGS SAGIGNYSFS LLYYPDDRG*
```

The cp6528 nucleotide sequence <SEQ ID 238> is:

```
  1  ATGAAAAACA ATATTAATAA TAATGAGTGC TATTTTAAAT TAGACTCAAC
 51  TGTAGATGGT GATTTGTTAG CAGCCAATCT CAAGACCTTT GATACACAGG
101  CCCAAGGAAT CTCATCGACT GAAACATTTT CTGTTCAGGG GAATGCAACA
151  TTTAAAGATC AAGTTTCAGC AACTGGATTA ACTTCAGGAA CTACTTATAA
201  TTTAAATGCA CAAAACTTTA CTTCCTCCCA AATCTCTATA GATTTTAAAA
251  ATAATCGTCT GAGTAATTGT GCATTGCCAA AAGAAGACTG CGATCCGGTG
301  CCAGCGAATT ATGTTCGTTC TCCCGAATAT TTTTTCTGTT CCAAGCCTCT
351  GATCGGAGAT TTTGATTTTA ACTCAGGGGA ATCTTATTTG CCTCTGACTG
401  GTTCGGAATA TACTCTATAT CAGTCACGTA ATGTAAATAG TATATTTCGT
451  TTTATAGGAT GGAAGCAAAG TACACGAGAA TTAACTGTAG GGGGAAATAC
501  TGCGATACAA TTTCTTGCAG CAGGAACCTA TATCGTTTCA TTTACTGTTG
551  GTAAACGGTG GGGATGGAAT AATGGTTGGG GAGGAGCCAT TTATATCAAT
601  AATGGTTTAG GACAAGTCCA ATGTGAAAGC ACGATTTATA GTGGTGGAGG
651  GTATGCAACA ATAGGTACAC TGGGGACCTC AATATATAGA GCCTCTGTAG
701  ATGTAGCTCC TAATCCTAAT GATCCGAATG CTTCGGATCG CTATAGAGCG
751  GGTATTTTCT ATCTCAGTAA CGGTGGTTCT AGTGCAGGTA TAGGGAATTA
801  CTCCTTTTCT CTTCTCTATT ATCCGGACGA TAGAGGGTAG
```

The PSORT algorithm predicts cytoplasm (0.1668).

The protein was expressed in *E.coli* and purified as a GST-fusion product (Figure 119A). The recombinant protein was used to immunise mice, whose sera were used in a Western blot (Figure 119B) and for FACS analysis.

These experiments show that cp6528 is a surface-exposed and immunoaccessible protein, and that it is a useful immunogen. These properties are not evident from the sequence alone.

**Example 120**

The following *C.pneumoniae* protein (PID 4376627) was expressed <SEQ ID 239; cp6627>:

```
  1  MKCSPLTLVP HIFLKNDCEC HRSCSLKIRT IARLILGLVL ALVSALSFVF
 51  LAAPISYAIG GTLALAAIVI LIITLVVALL AKSKVLPIPN ELQKIIYNRY
101  PKEVFYFVKT HSLTVNELKI FINCWKSGTD LPPNLHKKAE AFGIDILKSI
151  DLTLFPEFEE ILLQNCPLYW LSHFIDKTES VAGEIGLNKT QKVYGLLGPL
201  AFHKGYTTIF HSYTRPLLTL ISESQYKFLY SKASKNQWDS PSVKKTCEEI
251  FKELPHNMIF RKDVQGISQF LFLFFSHGIT WEQAQMIQLI NPDNWKMLCQ
301  FDKAGGHCSM ATFGGFLNTE TNMFDPVSSN YEPTVNFMTW KELKVLLEKV
351  KESPMHPASA LVQKICVNTT HHQNLLKRWQ FVRNTSSQWT SSLPQYAFHA
401  QTYKLEKKIE SSLPIRSSL*
```

The cp6627 nucleotide sequence <SEQ ID 240> is:

```
   1  ATGAAGTGTA GTCCTTTAAC ACTAGTTCCC CATATATTTT TAAAAAATGA
  51  CTGCGAATGT CATAGATCTT GTTCTTTAAA AATTAGGACA ATTGCCCGAC
 101  TCATTCTTGG GCTTGTTCTA GCTCTTGTTA GCGCACTTTC TTTTGTTTTC
 151  CTTGCTGCGC CGATTAGCTA TGCTATTGGA GGAACTTTAG CTTTAGCCGC
 201  TATCGTAATC TTGATTATAA CGCTAGTCGT AGCACTGCTA GCTAAATCAA
 251  AGGTTCTGCC CATCCCCAAC GAACTTCAGA AGATTATTTA CAATCGCTAT
 301  CCTAAAGAAG TCTTTTATTT CGTGAAAACA CACTCCCTGA CTGTTAACGA
 351  ATTAAAAATA TTTATTAATT GCTGGAAAAG CGGTACAGAC CTGCCTCCGA
 401  ATTTACATAA AAAAGCAGAG GCTTTCGGGA TCGATATTCT AAAATCTATA
 451  GATTTAACCC TGTTTCCAGA GTTCGAAGAG ATTCTTCTTC AAAACTGCCC
 501  GTTATACTGG CTCTCCCATT TTATAGACAA AACTGAATCT GTTGCTGGGG
 551  AAATCGGATT AAATAAAACA CAAAAAGTTT ATGGTTTACT TGGGCCCTTA
 601  GCGTTTCATA AAGGATATAC AACTATTTTC CACTCTTATA CACGCCCTCT
 651  ACTAACATTA ATCTCAGAAT CACAGTATAA GTTCCTATAT AGTAAAGCGT
 701  CTAAGAATCA ATGGGATTCT CCTTCTGTGA AAAAAACCTG CGAAGAAATA
 751  TTCAAGGAAC TCCCCCACAA TATGATTTTC CGGAAGGATG TTCAAGGAAT
 801  CTCACAATTC TTATTTCTTT TCTTTTCTCA TGGTATCACT TGGGAACAGG
 851  CTCAGATGAT TCAACTTATA AATCCTGATA ATTGGAAAAT GTTGTGTCAG
 901  TTTGATAAAG CAGGAGGCCA CTGTTCCATG GCAACATTTG GAGGCTTTTT
 951  GAATACTGAA ACAAATATGT TCGATCCAGT ATCCTCTAAC TATGAACCTA
1001  CAGTGAACTT CATGACGTGG AAAGAATTGA AGGTTTTACT AGAGAAAGTA
1051  AAAGAAAGTC CTATGCACCC AGCGAGTGCT CTTGTTCAGA AGATATGCGT
1101  AAATACAACG CACCATCAAA ATCTGTTAAA ACGATGGCAA TTTGTTCGTA
1151  ATACGAGTTC ACAATGGACA TCAAGCTTAC CTCAGTATGC TTTCCACGCC
1201  CAAACCTACA AACTAGAGAA AAAAATAGAA AGCAGTCTCC CTATACGATC
1251  TTCCCTATAA
```

The PSORT algorithm predicts inner membrane (0.7198).

The protein was expressed in *E.coli* and purified as a GST-fusion product (Figure 120A). The recombinant protein was used to immunise mice, whose sera were used in a Western blot (Figure 120B) and for FACS analysis.

These experiments show that cp6627 is a surface-exposed and immunoaccessible protein, and that it is a useful immunogen. These properties are not evident from the sequence alone.

**Example 121**

The following *C.pneumoniae* protein (PID 4376629) was expressed <SEQ ID 241; cp6629>:

```
  1  MSNITSPVIQ NNRSCNYYFE LKNSTTIHIV ISAILLCGAL IAFLCVAAPV
 51  SYILSGALLG LGLLIALIGV ILGIKKITPM ISSKEQVFPQ ELVNRIRAHY
101  PKFVSDFVSE AKPNLKDLIS FIDLLNQLHS EVGSSTNYNV SEELQQKIDT
151  FEGIARLKNE VRTASLKRLE SAASSRPLFP SLPKILQKVF PFFWLGEFIS
201  AGSKVVELHR VKKIGGSLEE DLSDYIKPEM LPTYWLIPLD FRPTNSSILN
251  LHTLVLARVL TRDVFQHLKY AALNGEWNLN HSDLNTMKQQ LFAKYHAAYQ
301  SYKHLSQPSL QEDEFYNLLL CIFKHRYSWK QMSLIKTVPA DLWENLCCLT
351  LDHTGRPQDM EFASLIGTLY TQGLIHKESE AFLSSLTLLS LDQFKTIRRQ
401  STNIAMFLEN LATHNSTFRS LPPITVHPLK RSVFSQPEED ESSLLIG*
```

The cp6629 nucleotide sequence <SEQ ID 242> is:

```
   1  ATGAGTAATA TAACCTCGCC AGTTATTCAA AATAATCGCT CTTGTAATTA
  51  TTATTTTGAA TTAAAGAATT CAACCACTAT TCATATTGTT ATCAGTGCCA
 101  TCTTACTCTG CGGAGCTTTG ATAGCTTTCT TGTGTGTAGC AGCTCCTGTT
 151  TCCTATATTC TAAGTGGCGC ATTGTTAGGA TTAGGATTAT TAATAGCCTT
 201  GATTGGTGTG ATTTTAGGAA TAAAAAAAAT CACGCCTATG ATTTCATCAA
 251  AAGAACAAGT ATTCCCCCAA GAACTCGTAA ATAGAATCAG GGCGCACTAT
 301  CCTAAATTTG TCTCTGATTT TGTTTCAGAA GCTAAACCAA ATCTTAAAGA
 351  TCTCATAAGT TTTATTGATC TTCTAAATCA ATTGCACTCT GAAGTTGGAT
 401  CATCTACAAA TTACAACGTA TCTGAAGAAC TACAACAGAA AATAGATACG
 451  TTCGAGGGTA TCGCACGCTT AAAAAATGAA GTCCGTACTG CTTCTCTTAA
 501  AAGACTTGAA AGCGCTGCTT CTTCCCGTCC CCTCTTCCCC TCTTTACCAA
 551  AAATCTTACA AAAGGTATTT CCATTTTTCT GGTTAGGAGA GTTTATTTCT
 601  GCAGGCAGCA AGGTTGTAGA GCTCCATCGA GTTAAGAAAA TTGGAGGCAG
 651  CCTCGAAGAA GACCTTAGTG ATTATATAAA ACCAGAGATG CTTCCTACCT
 701  ATTGGTTGAT TCCTTTAGAT TTTAGACCAA CAAATTCCTC TATTCTAAAT
 751  CTACACACAT TAGTTTTAGC TAGAGTCTTA ACTCGTGATG TTTTTCAACA
 801  TCTTAAGTAT GCAGCATTAA ATGGCGAGTG GAACCTGAAT CATAGTGATC
 851  TAAATACTAT GAAACAGCAG CTCTTTGCTA AATATCATGC GGCGTATCAA
 901  TCCTATAAAC ATCTATCTCA ACCCTCTCTT CAAGAGGATG AATTCTATAA
 951  CCTGCTCTTG TGTATTTTTA AGCATAGGTA CTCGTGGAAG CAGATGTCCT
1001  TAATAAAAAC AGTCCCGGCT GATTTATGGG AAAACCTCTG TTGCTTGACT
1051  TTAGACCATA CAGGACGACC CCAAGACATG GAATTTGCCT CTCTAATTGG
1101  TACTCTCTAC ACACAAGGCC TAATTCATAA AGAAAGCGAA GCATTTCTTT
1151  CTTCATTGAC ACTCCTTAGT TTAGATCAGT TTAAAACGAT CCGTCGTCAG
1201  TCAACCAATA TAGCGATGTT CCTTGAGAAT TTAGCAACTC ATAATTCCAC
1251  CTTTAGAAGC TTACCACCTA TAACAGTCCA TCCACTCAAG AGAAGCGTCT
1301  TCTCCCAACC TGAAGAAGAC GAGTCCTCCC TGCTGATAGG TTAG
```

The PSORT algorithm predicts inner membrane (0.5776).

The protein was expressed in *E.coli* and purified as a GST-fusion product (Figure 121A). The recombinant protein was used to immunise mice, whose sera were used in a Western blot (Figure 121B) and for FACS analysis.

These experiments show that cp6629 is a surface-exposed and immunoaccessible protein, and that it is a useful immunogen. These properties are not evident from the sequence alone.

**Example 122**

The following *C.pneumoniae* protein (PID 4376732) was expressed <SEQ ID 243; cp6732>:

```
  1  MEMMSPFQQP EQCHFDVVGS FLRPESLTRA RSDFEEGRIV YEQMRVVEDA
 51  AIRNLIKKQT EAGLIFFTDG EFRRYSWDFD FMWGFHGVDR RRDSNDPEIG
101  VYLKDKISVS KHPFIEHFEF VKTFEKGNAK AKQTIPSPSQ FFHEMIFAPN
151  LKNTRKFYPT NQELIDDIVF YYRQVIQDLY AAGCRNLQLD DCAWCRLLDI
201  RAPSWYGVDS HDRLQEILEQ FLWIHNLVMK DRPEDLFVSL HVCRGDYQAE
251  FFSRRAYDSI EEPLFAKTDV DSYHYYWALD DKYSGGAEPL AYVSGEKHVC
301  LGLISSNHSC IEDRDAVVSR IYEAASYIPL ERLSLSPQCG FASCEGDHRM

351  TEEEQWKKIA FVKEIAKEIW G*
```

The cp6732 nucleotide sequence <SEQ ID 244> is:

```
   1  ATGGAAATGA TGAGCCCATT CCAACAACCT GAGCAATGTC ATTTTGATGT
  51  TGTGGGAAGT TTCTTACGTC CTGAAAGTCT TACACGAGCA CGCTCTGATT
 101  TTGAAGAAGG AAGAATTGTC TATGAGCAGA TGCGAGTTGT CGAAGATGCT
 151  GCTATTCGTA ATCTCATAAA AAAGCAAACA GAAGCAGGTC TTATCTTTTT
 201  TACTGATGGG GAATTCCGTA GGTATAGTTG GGATTTCGAC TTTATGTGGG
 251  GATTCCATGG CGTGGATCGT CGCAGGGACT CTAATGACCC TGAAATTGGA
 301  GTGTATCTTA AAGATAAAAT CTCCGTATCA AAACATCCGT TTATAGAACA
 351  TTTCGAGTTT GTCAAAACTT TTGAGAAGGG AAATGCAAAA GCAAAACAAA
 401  CGATTCCTTC TCCATCACAA TTTTTCCATG AGATGATTTT TGCTCCTAAT
 451  CTGAAAAATA CTCGGAAGTT TTATCCTACG AATCAAGAGC TAATTGATGA
 501  TATTGTCTTT TATTATCGCC AAGTCATCCA AGATCTTTAT GCTGCAGGTT
 551  GTCGTAATTT GCAGTTGGAC GATTGTGCTT GGTGTCGCCT CTTGGATATA
 601  CGAGCGCCTT CTTGGTATGG TGTTGATTCT CATGACAGGT TGCAGGAAAT
 651  TTTAGAACAG TTTTTATGGA TCCATAATTT AGTGATGAAG GATAGACCCG
 701  AGGATCTTTT TGTAAGTCTG CATGTCTGTC GTGGTGATTA TCAGGCCGAG
 751  TTTTTCTCTA GACGAGCTTA TGATTCTATA GAGGAGCCTT TATTTGCTAA
 801  GACCGATGTG GATAGTTATC ACTATTATTG GGCTCTTGAT GATAAGTATT
 851  CAGGAGGTGC TGAGCCTTTA GCTTACGTCT CTGGAGAGAA ACACGTCTGC
 901  TTGGGATTGA TCTCCAGCAA CCATTCTTGT ATTGAAGATC GAGATGCTGT
 951  GGTTTCTCGT ATTTATGAAG CTGCGAGCTA CATTCCCTTA GAGAGACTTT
1001  CTTTGAGCCC GCAATGTGGG TTTGCTTCTT GTGAGGGAGA CCATAGAATG
1051  ACTGAAGAAG AACAGTGGAA GAAGATCGCC TTTGTGAAAG AGATTGCTAA
1101  AGAGATCTGG GGATAA
```

The PSORT algorithm predicts cytoplasm (0.2196).

The protein was expressed in *E.coli* and purified as a GST-fusion product (Figure 122A). The recombinant protein was used to immunise mice, whose sera were used in a Western blot (Figure 122B) and for FACS analysis.

These experiments show that cp6732 is a surface-exposed and immunoaccessible protein, and that it is a useful immunogen. These properties are not evident from the sequence alone.

**Example 123**

The following *C.pneumoniae* protein (PID 4376738) was expressed <SEQ ID 245; cp6738>:

```
  1  VWLRFLLLVS YDEKEKDVVV VCNHSEPNIL GLPPEAVSQL IEELSDEGYS
 51  YLNVVRCDLS GETTVQQRLL LNADEGRSMT VVISELPEGH PDIRNLQLAS
101  ERIFVSREKE AADAYASGCK VVAFDDEHLP WVSSHIAYAE EIREKQEQTM
151  QGSLTEEQLG ALLCNTVSTE KNLAFALDAV IKQSVWRFRN PDLFAYEREA
201  LEASVTDALV SYVSNLDMIP YTSSQGIVIE DSSIVRTSQE HTLIVNCAAF
251  DKLASQIEFL CPSDVLPISG KDPLISDDED EELNPKVSSA ADSKDKT*
```

The cp6738 nucleotide sequence <SEQ ID 246> is:

```
  1  GTGTGGCTGC GCTTTTTACT TTTAGTGTCC TATGATGAGA AGGAGAAAGA
 51  CGTAGTTGTC GTTTGTAATC ATTCTGAACC TAATATCCTC GGCCTGCCTC
101  CTGAAGCAGT CTCTCAGCTT ATTGAAGAGC TTAGCGATGA AGGCTATAGC
151  TATCTGAATG TAGTGCGTTG TGATCTCTCC GGGGAGACTA CGGTTCAACA
201  ACGTCTGCTA TTGAATGCCG ATGAAGGGAG ATCTATGACG GTGGTGATCT
251  CAGAGCTTCC TGAAGGGCAC CCCGATATTC GGAATTTGCA GTTGGCATCC
301  GAAAGAATTT TTGTTTCTCG TGAAAAAGAA GCTGCTGATG CCTATGCTTC
351  AGGATGTAAA GTGGTCGCTT TCGATGATGA GCATCTCCCT TGGGTCTCCA
401  GTCATATTGC CTACGCGGAG GAGATCAGAG AGAAACAAGA ACAAACAATG
451  CAAGGGTCTT TAACTGAAGA GCAGTTAGGA GCACTCCTCT GCAACACAGT
501  CTCCACAGAG AAAAATCTAG CCTTTGCTCT AGACGCCGTG ATAAAACAGT
551  CTGTGTGGAG ATTCCGCAAT CCGGATCTTT TTGCTTATGA GAGAGAAGCT
601  CTAGAGGCTT CAGTAACAGA TGCTTTAGTA TCTTACGTTT CAAATTTAGA
651  CATGATACCG TACACAAGTT CTCAGGGCAT AGTCATAGAA GATAGTAGTA
701  TCGTCCGTAC CTCTCAAGAG CATACACTCA TTGTGAACTG TGCAGCATTC
```

```
751  GATAAGTTAG CGAGCCAAAT AGAGTTCTTA TGCCCCAGTG ACGTGTTGCC
801  CATTTCTGGT AAAGACCCTT TGATTTCTGA TGATGAGGAT GAGGAACTGA
851  ATCCTAAAGT TTCATCTGCT GCAGACTCTA AAGATAAAAC CTAG
```

The PSORT algorithm predicts cytoplasm (0.1587).

The protein was expressed in *E.coli* and purified as a GST-fusion product (Figure 123A). The recombinant protein was used to immunise mice, whose sera were used in a Western blot (Figure 123B) and for FACS analysis.

These experiments show that cp6738 is a surface-exposed and immunoaccessible protein, and that it is a useful immunogen. These properties are not evident from the sequence alone.

**Example 124**

The following *C.pneumoniae* protein (PID 4376739) was expressed <SEQ ID 247; cp6739>:

```
  1  MTHCLHGWFS VVRHHFVQAF NFSRPLYSRI THFALGVIKA IPIVGHLVMG
 51  VDWLISHCFE RGVSHPGFPS DIAPILKVEK IAGRDHISRI ENQLKSLRKT
101  IEVEDLDKVH GQYQENPYAD MASSEVLKLD KGVHVSELGK AFSRVRNRIT
151  RSYSYAPTPQ LDSIAIVGID LVSPEEQENL VRLANEVIQL YPKSKTTLYL
201  LIDFNKEWVG DISSDKEKQL RSLGLHSEVQ CLSVLEPQGA EGEDTKHFDL
251  MVGCYGKDSY LREGKILQQA LGTSLGTVPW VNVMHTLPSR YRSRLSLPIN
301  TEKDKTELYK EISRTHHQLH TLGMGLGAQD SGLLLDRQRL HAPLSQGSHC
351  HSYLADLTHE ELKILLFSAF VDAKNISKKE LREVSLNFAN DTSVECGCAF
401  YF*
```

The cp6739 nucleotide sequence <SEQ ID 248> is:

```
   1  ATGACTCATT GCTTACATGG TTGGTTTTCT GTAGTTCGTC ATCACTTTGT
  51  GCAGGCGTTT AATTTCTCAC GTCCTTTATA TTCTCGAATT ACCCACTTCG
 101  CTTTAGGGGT GATTAAGGCC ATCCCCATTG TAGGGCATCT TGTTATGGGA
 151  GTCGATTGGT TGATCTCTCA TTGCTTCGAG AGGGGAGTCT CACACCCTGG
 201  GTTCCCTTCA GATATTGCTC CTATACTGAA AGTAGAAAAG ATCGCGGGCC
 251  GAGATCATAT TTCTAGAATC GAAAATCAGC TAAAGAGCCT TAGGAAAACT
 301  ATCGAGGTTG AAGATCTAGA TAAAGTCCAC GGGCAATATC AAGAGAATCC
 351  TTATGCAGAT ATGGCCTCTA GTGAGGTTCT TAAACTCGAT AAGGGAGTTC
 401  ATGTTAGCGA GCTTGGCAAA GCCTTTTCTA GAGTTCGCAA TCGCATCACC
 451  AGATCCTATA GTTATGCCCC TACTCCTCAG TTGGACTCTA TAGCTATTGT
 501  TGGTATAGAT CTCGTCAGTC CTGAAGAACA AGAGAATTTA GTACGCTTGG
 551  CGAATGAGGT CATTCAACTC TATCCCAAAT CAAAGACAAC TCTATATCTT
 601  CTTATCGATT TTAATAAGGA GTGGGTAGGG GATATCTCCT CTGATAAGGA
 651  AAAACAGCTC CGTTCTCTAG GTCTACATTC TGAAGTTCAG TGTCTTTCCG
 701  TCTTGGAACC TCAGGGTGCC GAGGGCGAAG ATACGAAACA CTTTGACCTT
 751  ATGGTCGGCT GTTATGGGAA GGATTCTTAC TTAAGGGAGG GTAAAATTTT
 801  ACAGCAGGCC CTAGGGACTT CGTTAGGTAC TGTTCCCTGG GTGAATGTTA
 851  TGCACACATT GCCATCTAGG TATAGATCTC GGCTTTCCTT ACCTATAAAT
 901  ACCGAAAAGG ATAAGACAGA GCTTTATAAA GAGATTTCTC GTACACACCA
 951  TCAGTTGCAT ACTTTGGGAA TGGGACTTGG AGCCCAGGAT TCAGGATTGC
1001  TCTTAGACCG GCAACGACTC CATGCTCCTT TATCTCAAGG GTCTCACTGC
1051  CATTCCTATC TTGCAGATCT CACCCATGAA GAGCTGAAAA TTTTGTTATT
1101  TTCAGCATTT GTGGATGCTA AGAACATAAG TAAGAAAGAG CTTCGTGAGG
1151  TATCTCTAAA TTTTGCTAAC GATACTTCCG TAGAGTGTGG CTGCGCTTTT
1201  TACTTTTAG
```

The PSORT algorithm predicts inner membrane (0.2190).

The protein was expressed in *E.coli* and purified as a GST-fusion product (Figure 124A). The recombinant protein was used to immunise mice, whose sera were used in a Western blot (Figure 124B) and for FACS analysis.

These experiments show that cp6739 is a surface-exposed and immunoaccessible protein, and that it is a useful immunogen. These properties are not evident from the sequence alone.

**Example 125**

The following *C.pneumoniae* protein (PID 4376741) was expressed <SEQ ID 249; cp6741>:

```
  1  MASCLSAWFS IVREHFYRAF DFSLPFCARI TEFVLGVIKG IPVVGHIIVG
 51  IEWLVSRYLE SFVTKPTFVS DVVSLLKTEK VAGRDHIARV VETLKRQRVA
101  VAPEDEDKVH GKIPVHPFGG IQPVEVLTLY PEVQDATLGL AFSKIRNRVR
151  QAYLQAPRPK LQKIYIIGND MNPFEVDDFL HLARLCNETQ RLYPDATISL
201  YLTASGGRNA MDKKNRKLLS DCELNPKIAC LDFNQGDVVK QATCDCWMVY
251  HGENDQGTLN QIQEELEKSG EETPWIHVGQ KPLSQSLWDF SPFSSLEMKG
301  DKEKALEYSE LEKEQLYSRL VYVGERSSVL SLGFGDSRSG ILMDPKRVHA
351  PLSEGHYCHS YLADLENPGL QKTILAAFLN PKELSSTILQ PISLNLILNS
401  KTYLRQHFGF FERMSRSDRN VVVVVCDSWW GTDWKEEPSF QHFIMELECR
451  GYSHFNIFAF RSNSMCVEER RILNESSQEK AFTMIFCEDS VSQGDIRCLH
501  LASEGMLCGK ECYAVDVYTS GCANFMMEEV LTLERESNLW NRKHGLWKRE
551  VRKQKQEAAL DQDESEIYVC NQLTAQQNFA CS*
```

The cp6741 nucleotide sequence <SEQ ID 250> is:

```
   1  ATGGCTTCTT GTTTATCTGC CTGGTTTTCT ATAGTTCGTG AGCACTTTTA
  51  TCGAGCCTTT GATTTTTCTT TGCCGTTTTG TGCTCGTATT ACGGAATTTG
 101  TATTAGGGGT CATCAAGGGG ATCCCTGTTG TGGGTCACAT TATTGTTGGG
 151  ATAGAGTGGC TCGTTTCTAG GTATTTAGAG AGTTTCGTGA CCAAGCCGAC
 201  ATTTGTCTCT GATGTGGTGA GTCTTCTGAA AACAGAGAAA GTTGCTGGTC
 251  GCGATCACAT TGCTCGTGTA GTGGAGACTT TGAAGAGGCA GAGAGTCGCT
 301  GTGGCTCCTG AAGATGAGGA TAAGGTCCAT GGGAAGATTC CTGTGCATCC
 351  TTTCGGGGGA ATCCAACCTG TAGAAGTTCT CACTCTCTAT CCCGAAGTTC
 401  AAGATGCAAC GTTAGGGCTT GCCTTCTCTA AAATTCGTAA TCGTGTAAGA
 451  CAGGCGTATT TGCAAGCTCC ACGGCCAAAA CTGCAGAAGA TTTACATCAT
 501  AGGAAACGAT ATGAATCCTT TTGAAGTTGA CGACTTCTTG CATCTAGCCC
 551  GTCTCTGTAA TGAAACTCAA AGACTCTATC CTGACGCTAC GATTTCTCTA
 601  TATCTAACAG CTTCTGGTGG TCGCAATGCT ATGGACAAAA AGAATCGGAA
 651  GTTACTTAGT GATTGCGAAC TAAACCCCAA GATTGCTTGT TTGGACTTTA
 701  ATCAGGGTGA TGTAGTCAAA CAAGCAACTT GTGACTGTTG GATGGTGTAT
 751  CATGGGGAGA ATGATCAAGG TACGTTGAAT CAGATTCAGG AAGAGTTAGA
 801  AAAGTCAGGG GAGGAAACCC CTTGGATTCA TGTGGGGCAA AAGCCTCTTT
 851  CACAATCCTT GTGGGATTTC TCTCCATTTT CATCTTTGGA GATGAAGGGA
 901  GATAAAGAGA AAGCTCTAGA GTACTCTGAA TTAGAAAAAG AACAGCTATA
 951  TTCTCGATTG GTATACGTAG GAGAGCGCTC TTCGGTTCTT AGTTTGGGGT
1001  TTGGAGATAG TCGGTCAGGG ATCTTGATGG ACCCAAAACG GGTGCATGCT
1051  CCCTTATCTG AAGGGCATTA TTGTCATTCC TACCTTGCAG ACTTAGAAAA
1101  TCCCGGGTTA CAAAAAACAA TTTTAGCGGC ATTTCTGAAT CCTAAGGAGT
1151  TGAGCAGTAC CATACTGCAA CCTATATCTC TAAATCTTAT CTTAAATAGC
1201  AAAACTTACT TAAGGCAGCA CTTTGGCTTT TTTGAGAGGA TGAGCAGAAG
1251  TGATCGCAAT GTGGTTGTCG TTGTATGTGA TTCTTGGTGG GGTACCGACT
1301  GGAAGGAGGA GCCAAGCTTC CAACACTTTA TTATGGAGCT AGAGTGTCGA
1351  GGGTATTCGC ACTTCAATAT TTTTGCCTTT AGATCTAATA GCATGTGTGT
1401  AGAAGAACGT AGGATCTTAA ATGAAAGTTC TCAAGAGAAA GCCTTTACCA
1451  TGATTTTCTG TGAGGATTCA GTATCTCAAG GAGATATCCG CTGTTTGCAT
1501  TTGGCGTCTG AAGGAATGCT TTGTGGTAAA GAGTGCTATG CTGTCGATGT
1551  CTATACGTCA GGATGCGCGA ACTTTATGAT GGAAGAAGTC TTAACTTTGG
1601  AGCGAGAATC TAATCTGTGG AATAGAAAGC ATGGTCTTTG GAAAAGAGAA
1651  GTTAGAAAAC AGAAACAAGA AGCTGCTTTG GATCAAGACG AGAGCGAGAT
1701  TTACGTTTGT AATCAGCTGA CGGCGCAACA GAACTTCGCT TGTTCTTGA
```

The PSORT algorithm predicts inner membrane (0.2869).

The protein was expressed in *E.coli* and purified as a GST-fusion product (Figure 125A). The recombinant protein was used to immunise mice, whose sera were used in a Western blot (Figure 125B) and for FACS analysis.

These experiments show that cp6741 is a surface-exposed and immunoaccessible protein, and that it is a useful immunogen. These properties are not evident from the sequence alone.

**Example 126**

The following *C.pneumoniae* protein (PID 4376742) was expressed <SEQ ID 251; cp6742>:

```
  1 LFVSNFIFFV VMPIPYISSW ISTVRQHFVK AFDFSRPFCS RVTNFALGVI
 51 KAIPIVGHIV MGMEWLVSSC VAGIITRSSF TSDVVQIVKT EKALGRDHIS
101 RVAEILQRER GTITPENQDK VHGKFPVCPF GRLKSEETLK LKPGEREGTL
151 DTVFSPIRTR VTRAYLQAPR PEIRTISIVG SKLKTPQDFS QFVSLANETQ
201 RLHPEALVCL YLTGLNRESQ MCDTTTAEKK QYLHNSGLDS RIQCKDSKED
251 DAGSPENPEL WIGYYSREQQ HNIDGQYIQQ CLGKSADPIP WIHVTEDTKD
301 FYYPPNFTSY SHTRQSTDPT SPPRLPESEG DKDSLYGQLS RSYHHEYMLG
351 LGLKPEDAGL LMDPDRIYAP LSQGHYCHSY LADIENEDLR TLVLSPFLDP
401 GNLSSEDLRP VAFNIARLPL ELDSLFFRLV AGQQEGRNIV TLAHGTPRPE
451 DLDPDSMNIL TRRLQMSGYS YLNIFSYKSR KMIVKERQFF GDRSEGKSFT
501 LILFEDPISA ADFRCLQLAA EGMVAKDLPS VADICASGCS CIQFSEMQSP
551 QAIEYRQWEA RVEDEAGEEA REPVIYSQDQ LSSMLTTQQN FVFSLDAVVK
601 QAIWRFRSKG LLTMERKALG EEFLTAIFSY LGSQERNENM GKRTTEEHEV
651 VISFEELDRM VQVLPAEVPA DSGNDPTRPV PNPDSNPDSS QNEGS*
```

The cp6742 nucleotide sequence <SEQ ID 252> is:

```
   1 TTGTTTGTTT CTAATTTTAT TTTTTTTGTT GTTATGCCAA TTCCCTATAT
  51 TTCTTCTTGG ATTTCTACCG TTCGACAGCA TTTTGTTAAG GCGTTTGATT
 101 TCTCTCGTCC CTTTTGTTCT AGGGTTACGA ATTTTGCTTT AGGGGTCATC
 151 AAGGCCATCC CTATTGTAGG ACATATTGTC ATGGGGATGG AGTGGTTAGT
 201 TTCTTCCTGT GTTGCCGGGA TTATTACTAG GTCCTCCTTT ACCTCAGATG
 251 TCGTTCAGAT TGTAAAGACT GAGAAGGCGT TAGGTCGAGA TCATATATCT
 301 CGAGTGGCGG AGATATTGCA AAGAGAAAGG GGGACCATAA CTCCTGAGAA
 351 TCAAGATAAG GTGCATGGGA AGTTTCCTGT CTGTCCTTTT GGTCGTTTAA
 401 AATCCGAGGA AACTTTAAAA CTTAAGCCGG GAGAAAGAGA GGGAACTTTA
 451 GATACTGTAT TTTCTCCGAT TCGCACGCGC GTGACTCGTG CGTACTTACA
 501 GGCCCCCCGA CCCGAAATAC GTACGATTTC TATTGTGGGT TCGAAACTTA
 551 AAACTCCTCA AGATTTCTCG CAATTTGTGA GTCTCGCGAA TGAAACGCAG
 601 AGACTGCATC CTGAAGCGTT AGTTTGTCTG TATTTGACAG GCTTGAATCG
 651 CGAATCTCAG ATGTGCGATA CAACTACTGC AGAGAAGAAG CAGTACCTAC
 701 ATAACTCAGG TCTCGACTCT AGAATCCAGT GCAAAGACAG TAAAGAAGAC
 751 GACGCTGGCT CTCCTGAAAA TCCCGAACTT TGGATTGGCT ATTATTCACG
 801 AGAGCAACAG CATAATATAG ACGGGCAGTA TATTCAGCAG TGTCTAGGGA
 851 AGAGTGCAGA TCCAATTCCT TGGATTCATG TTACTGAAGA CACAAAGGAT
 901 TTTTATTACC CACCAAACTT TACTTCATAC TCACATACAA GACAATCTAC
 951 AGACCCAACA TCGCCACCAA GACTCCCTGA AAGTGAGGGG GATAAGGATT
1001 CCTTGTACGG ACAACTGAGT CGATCGTATC ACCATGAGTA TATGCTTGGT
1051 TTGGGATTAA AACCAGAGGA TGCAGGACTC CTGATGGACC CGGATAGAAT
1101 CTATGCTCCT CTATCCCAAG GGCATTATTG TCATTCCTAC CTTGCGGATA
1151 TAGAAAATGA GGATCTACGA ACTTTAGTCC TTTCGCCTTT CCTAGATCCT
1201 GGCAATCTTA GTAGCGAGGA TCTTCGTCCT GTAGCATTCA ATATCGCTAG
1251 ATTGCCATTA GAATTGGACT CGTTATTTTT CCGCCTTGTT GCGGGTCAGC
1301 AAGAAGGGAG AAACATAGTT ACCCTTGCCC ACGGAACTCC TCGTCCAGAA
1351 GATCTTGATC CTGACTCAAT GAACATTCTG ACCAGAAGAT TACAAATGTC
1401 TGGATATAGC TATTTGAACA TTTTCTCCTA TAAATCACGG AAAATGATTG
1451 TAAAAGAACG TCAGTTCTTT GGAGATCGTT CTGAAGGGAA GTCTTTCACA
1501 TTGATCTTAT TTGAGGATCC CATTAGTGCA GCAGATTTCC GTTGTTTGCA
1551 GCTAGCTGCA GAAGGTATGG TTGCTAAGGA TCTCCCCAGC GTAGCAGATA
1601 TTTGTGCCTC TGGATGTTCC TGCATTCAGT TTTCTGAGAT GCAGAGTCCT
1651 CAGGCTATTG AATATAGACA ATGGGAGGCA CGTGTCGAAG ATGAAGCAGG
1701 AGAAGAAGCC AGAGAACCAG TAATTTATTC TCAGGATCAA TTGAGCAGCA
1751 TGCTCACTAC ACAACAGAAT TTTGTATTTT CTCTAGATGC TGTGGTAAAA
1801 CAGGCGATCT GGAGATTCCG TTCGAAAGGT CTTCTTACTA TGGAAAGAAA
1851 GGCACTAGGC GAGGAGTTCT TAACTGCGAT ATTTTCCTAT TTAGGGAGTC
1901 AGGAGCGTAA TGAGAATATG GGGAAAAGAA CTACCGAAGA ACATGAGGTC
1951 GTTATCAGCT TCGAAGAGCT AGATCGCATG GTGCAAGTCC TCCCAGCCGA
2001 AGTCCCTGCA GATTCAGGCA ATGATCCTAC GCGTCCCGTT CCTAATCCAG
2051 ATAGTAACCC TGATTCCTCG CAAAATGAAG GCAGTTAG
```

The PSORT algorithm predicts inner membrane (0.2338).
The protein was expressed in *E.coli* and purified as a GST-fusion product (Figure 126A). The recombinant protein was used to immunise mice, whose sera were used in a Western blot (Figure 126B) and for FACS analysis.
These experiments show that cp6742 is a surface-exposed and immunoaccessible protein, and that it is a useful immunogen. These properties are not evident from the sequence alone.

**Example 127**

The following *C.pneumoniae* protein (PID 4376744) was expressed <SEQ ID 253; cp6744>:

```
  1 VIQHLLNFAL EETPSISVQY QEQEKLSPCD HSPEIGKKKR WNKLESFSTY
 51 CSLFMSVKDH YKLNLGIQNS LSGWLLDPYR VCAPLSSPYS CPSYLLDLQN
101 KELRRSLLST FLDPKNLTSE TFRSVSINFG NSSFGQRWSE FLSRVLHDEK
151 EKHVAVVCND AKLLEEGLSP EALSLLEEDL RESGYSYLNI LSVSPEGVSK
201 VQERQILRRD LQGRSFTVMI TDLPLGSEDI RSLQLASDRI LVSSSLDAAD
251 ACASGCKVLV YENPNASWAQ ELENFYKQVE RRR*
```

The cp6744 nucleotide sequence <SEQ ID 254> is:

```
  1 GTGATACAAC ATCTTCTAAA CTTTGCTCTA GAAGAGACCC CTTCCATTTC
 51 CGTGCAATAC CAAGAACAAG AGAAGCTCTC TCCGTGCGAT CATTCCCCAG
101 AAATAGGTAA AAAGAAAAGA TGGAATAAGC TGGAATCCTT CTCCACGTAT
151 TGTTCTCTGT TTATGTCTGT TAAGGATCAT TATAAGCTGA ATCTAGGAAT
201 TCAGAATTCC CTGTCAGGGT GGCTTCTGGA TCCCTATAGG GTTTGCGCGC
251 CTTTATCTTC ACCGTACTCG TGTCCTTCCT ATCTTTTAGA TTTGCAAAAC
301 AAAGAGCTAC GTCGTTCCCT TCTGTCAACG TTTCTAGACC CTAAAAATCT
351 CACTAGCGAA ACATTCCGTT CTGTCTCTAT AAACTTTGGC AACTCTTCGT
401 TTGGACAGAG ATGGTCAGAG TTTCTATCTC GTGTTCTGCA CGACGAGAAA
451 GAAAAGCACG TAGCTGTTGT TTGTAATGAT GCAAAACTTC TGGAAGAAGG
501 ATTGTCCCCA GAGGCATTGT CTCTATTAGA AGAAGACTTA AGAGAATCAG
551 GGTATTCGTA TCTAAACATT CTCTCGGTGA GCCCCGAAGG AGTCTCCAAG
601 GTTCAGGAAC GTCAGATTCT AAGGCGAGAT CTCCAAGGAC GGTCCTTTAC
651 TGTCATGATT ACAGATCTTC CTTTAGGTAG CGAAGATATC CGTAGTTTAC
701 AATTAGCCTC GGATAGGATT TTAGTCTCCA GTTCTCTTGA TGCCGCGGAT
751 GCATGTGCTT CGGGATGTAA AGTCTTAGTC TACGAAAATC CAAATGCATC
801 CTGGGCTCAG GAATTGGAGA ACTTCTACAA ACAAGTTGAG AGAAGAAGGT
851 AG
```

The PSORT algorithm predicts cytoplasm (0.3833).
The protein was expressed in *E.coli* and purified as a GST-fusion product (Figure 127A). The recombinant protein was used to immunise mice, whose sera were used in a Western blot (Figure 127B) and for FACS analysis.
These experiments show that cp6744 is a surface-exposed and immunoaccessible protein, and that it is a useful immunogen. These properties are not evident from the sequence alone.

**Example 128**

The following *C.pneumoniae* protein (PID 4376745) was expressed <SEQ ID 255; cp6745>:

```
  1 VACPSISSWF TVVRQHFVNA FDFTHPVCSR ITNFALGIIK AIPVLGHIVM
 51 GIEWLISWIP RHTVRHGMFT SDVSSAIKVE QTRGHNCLAP LEAYLSSLRV
101 PISQEDLGKV HGRTPEDPFV DITPTEIVQL LPDEELSTVD EALQGVRSRL
151 TYAYRSVEKP MIQDLALVGF GLRDSADLIN FVRLANGVQN HYPHTKVKLY
201 LAKNLADVWD CEISEEEKGQ LRALGLDPKI ESISLTSAGL PSVPEVATVD
251 FMITCYGKDQ EVQDP*
```

The cp6745 nucleotide sequence <SEQ ID 256> is:

```
   1  GTGGCTTGTC CAAGTATTTC TTCTTGGTTT ACTGTCGTTC GACAGCATTT
  51  TGTAAACGCC TTTGATTTCA CCCATCCCGT TTGTTCTCGG ATTACAAATT
 101  TTGCTTTGGG GATCATTAAG GCAATTCCCG TATTAGGACA CATTGTCATG
 151  GGAATCGAGT GGTTGATTTC CTGGATTCCC AGACACACCG TTCGTCATGG
 201  AATGTTTACT TCTGATGTCT CTAGTGCTAT TAAAGTAGAA CAAACACGGG
 251  GTCATAATTG TTTAGCTCCC CTAGAAGCCT ATTTAAGTAG CTTGAGAGTC
 301  CCCATTTCCC AAGAAGATCT AGGCAAAGTA CACGGGAGAA CCCCAGAAGA
 351  TCCCTTCGTA GATATCACAC CCACAGAAAT TGTCCAACTT CTCCCTGATG
 401  AAGAACTCTC TACTGTAGAT GAGGCACTGC AAGGCGTTCG TAGTAGGTTA
 451  ACCTATGCCT ATAGGTCCGT AGAGAAACCT ATGATTCAAG ATCTTGCTCT
 501  TGTGGGTTTT GGTCTCCGAG ATTCTGCGGA CCTCATAAAT TTCGTGCGTC
 551  TTGCTAATGG CGTGCAGAAT CACTATCCCC ATACTAAAGT GAAGCTCTAT
 601  TTAGCGAAGA ACTTGGCAGA TGTCTGGGAC TGTGAAATTT CTGAAGAGGA
 651  AAAAGGGCAA CTCCGAGCTC TAGGTTTAGA CCCTAAAATA GAGAGTATAT
 701  CCCTTACGAG TGCAGGTCTT CCTTCAGTGC CAGAAGTCGC TACTGTCGAT
 751  TTTATGATTA CCTGTTACGG GAAAGATCAG GAAGTCCAAG ATCCCTAG
```

The PSORT algorithm predicts inner membrane (0.2253).

The protein was expressed in *E.coli* and purified as a GST-fusion product (Figure 128A). The recombinant protein was used to immunise mice, whose sera were used in a Western blot (Figure 128B) and for FACS analysis.

These experiments show that cp6745 is a surface-exposed and immunoaccessible protein, and that it is a useful immunogen. These properties are not evident from the sequence alone.

**Example 129**

The following *C.pneumoniae* protein (PID 4376747) was expressed <SEQ ID 257; cp6747>:

```
   1  MMKQGVGQDA KELYTFLSRG NEHYQPCLWF SLEEELGFLF DEKMLCAPLS
  51  EDHYCHSYLV DLVDQHLKDL ILSMFLDPQN ISAGELLKVS INVGDSFSPL
 101  QQKDFLSMVL RDETGKNVVV VFKGVLSLPA TQVCKLVEEL NSKDYSYLNI
 151  FSCHGDSSPQ LLFRKELEGT SGRYFTVICA LYLGDTDMRS LQLASERIMV
 201  SREFDLVDAY AARCKLLKID HTNWRPGTFS RHADFADAVD VSAGFNSREF
 251  KLITQANQGI LESGELPLPS KTFWEGFLAF CDRVTVTRHF IPMLDAAIKQ
 301  AVWTHKHPSL IDKECEALDL KTQCLPSIVS YLEYVTNSHE KTSKGPFIQK
 351  EIIADCSPLK EALFPGSDED VPSTSEDPSD DHPSDLEDS*
```

The cp6747 nucleotide sequence <SEQ ID 258> is:

```
   1  ATGATGAAAC AAGGAGTCGG GCAGGATGCT AAAGAGCTAT ACACATTTCT
  51  ATCTCGTGGG AATGAGCATT ACCAACCGTG TCTATGGTTC AGTCTCGAAG
 101  AGGAACTCGG ATTCCTTTTC GATGAAAAAA TGCTCTGCGC CCCTCTATCT
 151  GAGGATCACT ATTGCCACTC GTATCTTGTA GATCTAGTGG ATCAACATTT
 201  AAAGGATTTA ATATTATCGA TGTTTTTAGA TCCTCAGAAT ATCTCAGCAG
 251  GAGAACTCCT CAAGGTCTCT ATAAACGTTG GAGATTCTTT TTCTCCTCTA
 301  CAACAGAAAG ATTTCCTCTC GATGGTCTTA CGTGATGAAA CGGGAAAAAA
 351  CGTCGTCGTG GTTTTTAAAG GAGTTCTCTC CTTACCCGCA ACCCAAGTCT
 401  GCAAATTAGT AGAGGAATTG AACTCTAAGG ACTACTCCTA CCTCAATATA
 451  TTTTCTTGTC ACGGAGATAG TAGTCCTCAG CTTTTATTCC GTAAGGAATT
 501  AGAGGGAACT TCAGGGCGTT ATTTTACAGT GATTTGCGCT TTATATCTAG
 551  GGGATACAGA CATGCGTAGT TTACAACTTG CTTCTGAAAG GATCATGGTC
 601  TCTAGAGAGT TTGATCTTGT AGATGCCTAT GCTGCAAGAT GCAAGCTCTT
 651  GAAAATCGAT CATACAAATT GGAGACCTGG AACTTTCAGT CGCCACGCCG
 701  ATTTCGCAGA TGCTGTAGAC GTATCAGCAG GATTTAACTC AAGAGAATTT
 751  AAACTGATTA CGCAGGCGAA TCAAGGGATC CTAGAGTCTG GAGAACTCCC
 801  GCTCCCTTCA AAAACCTTCT GGGAAGGATT CTTAGCATTC TGTGATCGAG
 851  TGACTGTCAC GAGACACTTC ATTCCAATGT TAGACGCCGC TATAAAGCAA
 901  GCGGTATGGA CTCATAAACA TCCCAGCTTG ATAGATAAAG AGTGTGAAGC
 951  CCTAGACTTG AAAACACAGT GCTTGCCATC TATCGTATCG TACCTTGAAT
1001  ATGTCACAAA CTCTCACGAA AAAACATCGA AAGGCCCGTT CATACAAAAA
1051  GAGATTATCG CAGACTGTTC TCCTCTTAAA GAGGCGCTCT TCCCAGGTTC
```

```
1101  TGATGAAGAT GTTCCCTCTA CCTCTGAGGA TCCTTCAGAT GATCATCCTT
1151  CGGATCTTGA AGACTCTTAA
```

The PSORT algorithm predicts inner membrane (0.1447).

The protein was expressed in *E.coli* and purified as a GST-fusion product (Figure 129A) and also as a his-tagged product. The recombinant proteins were used to immunise mice, whose sera were used in a Western blot (Figure 129B) and for FACS analysis.

These experiments show that cp6747 is a surface-exposed and immunoaccessible protein, and that it is a useful immunogen. These properties are not evident from the sequence alone.

**Example 130**

The following *C.pneumoniae* protein (PID 4376756) was expressed <SEQ ID 259; cp6756>:

```
  1  MASGIGGSSG LGKIPPKDNG DRSRSPSPKG ELGSHEISLP PQEHGEEGAS
 51  GSSHIHSSSS FLPEDQESQS SSSAASSPGF FSRVRSGVDR ALKSFGNFFS
101  AESTSQARET RQAFVRLSKT ITADERRDVD SSSAAATEAR VAEDASVSGE
151  NPSQGVPETS SGPEPQRLFS LPSVKKQSGL GRLVQTVRDR IVLPSGAPPT
201  DSEPLSLYEL NLRLSSLRQE LSDIQSNDQL TPEEKAEATV TIQQLIQITE
251  FQCGYMEATQ SSVSLAEARF KGVETSDEIN SLCSELTDPE LQELMSDGDS
301  LQNLLDETAD DLEAALSHTR LSFSLDDNPT PIDNNPTLIS QEEPIYEEIG
351  GAADPQRTRE NWSTRLWNQI REALVSLLGM ILSILGSILH RLRIARHAAA
401  EAVGRCCTCR GEECTSSEED SMSVGSPSEI DETERTGSPH DVPRRNGSPR
451  EDSPLMNALV GWAHKHGAKT KESSESSTPE ISISAPIVRG WSQDSSVSFI
501  VMEDDHIFYD VPRRKDGIYD VPSSPRWSPA RELEEDVFGD YEVPITSAEP
551  SKDKNIYMTP RLATPAIYDL PSRPGSSGSS RSPSSDRVRS SSPNRRGVPL
601  PPVPSPAMSE EGSIYEDMSG ASGAGESDYE DMSRSPSPRG DLDEPIYANT
651  PEDNPFTQRN IDRILQERSG GASASPVEPI YDEIPWIHGR PPATLPRPEN
701  TLTNVSLRVS PGFGPEVRAA LLSESVSAVM VEAESIVPPT EPGDGESEYL
751  EPLGGLVATT KILLQKGWPR GESNA*
```

The cp6756 nucleotide sequence <SEQ ID 260> is:

```
   1 ATGGCATCAG GAATCGGAGG ATCTAGTGGA TTAGGAAAGA TTCCACCTAA
  51 AGATAATGGG GATAGAAGTC GATCGCCCTC TCCTAAGGGA GAACTTGGCA
 101 GCCACGAGAT TTCCCTGCCT CCTCAAGAAC ATGGAGAGGA AGGAGCTTCA
 151 GGATCTTCGC ATATACATAG CAGTTCCTCT TTTCTACCAG AAGATCAGGA
 201 GTCTCAGAGC TCTTCTTCGG CAGCTTCTAG CCCGGGATTT TTTTCTCGCG
 251 TACGTTCTGG GGTAGACAGG GCCTTAAAAT CATTTGGCAA CTTTTTTTCC
 301 GCAGAGTCTA CGAGTCAAGC GCGTGAAACG CGACAAGCTT TTGTTAGATT
 351 ATCAAAAACC ATCACCGCGG ATGAGAGACG GGATGTCGAT TCATCAAGTG
 401 CTGCTGCTAC AGAAGCCCGA GTGGCAGAGG ACGCGAGTGT TTCAGGCGAA
 451 AATCCTTCTC AGGGGGTTCC AGAAACCTCT TCTGGACCAG AACCTCAGCG
 501 TTTATTTTCT CTTCCTTCAG TAAAAAAACA GAGCGGTTTG GGTCGGTTGG
 551 TACAGACAGT TCGCGATCGC ATAGTACTTC CTAGTGGGGC TCCACCTACA
 601 GACAGCGAGC CTTTAAGTCT CTACGAGCTA AACCTCCGTT TGAGTAGTTT
 651 ACGTCAGGAG CTCTCTGACA TACAAAGTAA TGATCAGTTG ACTCCAGAGG
 701 AAAAAGCAGA AGCCACAGTT ACCATACAAC AGCTGATCCA AATTACAGAA
 751 TTCCAATGCG GCTATATGGA GGCAACACAA TCTTCGGTAT CTCTAGCAGA
 801 AGCTCGTTTT AAGGGGGTAG AAACTAGTGA TGAGATCAAT TCCCTCTGTT
 851 CAGAACTGAC AGATCCTGAG CTTCAAGAAC TCATGAGTGA TGGAGACTCT
 901 CTTCAAAACC TATTAGATGA GACTGCCGAC GATTTAGAAG CTGCTTTGTC
 951 CCATACTCGA TTGAGTTTTT CTTTAGACGA TAATCCAACT CCGATAGACA
1001 ATAATCCAAC TCTGATTTCT CAAGAAGAGC CTATTTATGA GGAAATCGGA
1051 GGAGCTGCAG ATCCTCAAAG AACTCGGGAA AACTGGTCTA CAAGATTATG
1101 GAATCAGATT CGCGAGGCTC TGGTTTCTCT TTTAGGAATG ATTTTAAGCA
1151 TTCTAGGGTC CATCTTGCAC AGGTTGCGTA TTGCTCGTCA TGCAGCTGCT
1201 GAAGCAGTGG GTCGTTGTTG CACGTGCCGA GGAGAAGAGT GTACTTCTTC
1251 TGAAGAGGAC TCGATGTCGG TGGGGTCTCC TTCAGAAATT GATGAAACTG
1301 AAAGAACGGG CTCTCCGCAT GACGTTCCAC GCAGAAATGG AAGTCCACGT
1351 GAAGATTCTC CATTGATGAA TGCCTTAGTA GGATGGGCAC ATAAGCACGG
1401 TGCTAAAACC AAGGAGAGTT CAGAATCAAG TACCCCGGAA ATTTCGATTT
1451 CTGCTCCCAT AGTGAGAGGT TGGAGTCAAG ACAGTTCCGT CAGTTTTATT
```

```
1501 GTTATGGAAG ATGATCATAT TTTCTATGAT GTTCCTCGTA GAAAAGATGG
1551 AATCTATGAC GTTCCTAGTT CCCCTAGATG GAGTCCTGCG CGAGAGTTGG
1601 AAGAGGATGT TTTTGGAGAT TATGAAGTTC CTATAACCTC TGCTGAACCA
1651 TCTAAAGACA AGAACATCTA CATGACACCT AGATTAGCAA CTCCTGCTAT
1701 CTATGATCTT CCTTCACGTC CAGGATCGTC TGGAAGCTCA CGTTCTCCGT
1751 CTTCAGATCG CGTACGAAGC AGCTCACCAA ATAGACGGGG TGTGCCTCTT
1801 CCTCCAGTTC CTTCACCTGC TATGAGTGAG GAGGGGAGCA TTTATGAGGA
1851 TATGAGCGGT GCTTCAGGTG CAGGTGAAAG TGATTATGAA GATATGAGCC
1901 GTTCCCCCTC TCCTAGAGGC GACTTGGATG AACCCATATA TGCTAATACT
1951 CCTGAAGATA ATCCATTTAC TCAGAGAAAT ATAGATAGAA TTTTACAGGA
2001 GAGGTCAGGC GGTGCTTCCG CTTCTCCTGT AGAGCCTATT TATGATGAGA
2051 TCCCATGGAT TCATGGCAGG CCCCCTGCTA CACTTCCAAG ACCCGAGAAT
2101 ACATTGACTA ATGTTTCGCT TAGAGTGAGC CCAGGGTTTG GACCAGAAGT
2151 AAGAGCCGCT TTGCTTAGCG AGAGCGTGAG TGCTGTTATG GTCGAAGCAG
2201 AGAGTATTGT TCCTCCAACA GAGCCGGGGG ACGGAGAATC AGAATATCTA
2251 GAGCCCTTAG GGGGACTTGT AGCTACAACG AAAATCTTAC TACAAAAAGG
2301 ATGGCCTCGT GGAGAGTCGA ATGCTTAG
```

The PSORT algorithm predicts inner membrane (0.3994).

The protein was expressed in *E.coli* and purified as a GST-fusion product (Figure 130A). The recombinant protein was used to immunise mice, whose sera were used in a Western blot (Figure 130B) and for FACS analysis.

These experiments show that cp6756 is a surface-exposed and immunoaccessible protein, and that it is a useful immunogen. These properties are not evident from the sequence alone.

### Example 131

The following *C.pneumoniae* protein (PID 4376761) was expressed <SEQ ID 261; cp6761>:

```
  1  MTVAEVKGTF KLVCLGCRVN QYEVQAYRDQ LTILGYQEVL DSEIPADLCI
 51  INTCAVTASA ESSGRHAVRQ LCRQNPTAHI VVTGCLGESD KEFFASLDRQ
101  CTLVSNKEKS RLIEKIFSYD TTFPEFKIHS FEGKSRAFIK VQDGCNSFCS
151  YCIIPYLRGR SVSRPAEKIL AEIAGVVDQG YREVVIAGIN VGDYCDGERS
201  LASLIEQVDR IPGIERIRIS SIDPDDITED LHRAITSSRH TCPSSHLVLQ
251  SGSNSILKRM NRKYSRGDFL DCVEKFRASD PRYAFTTDVI VGFPGESDQD
301  FEDTLRIIED VGFIKVHSFP FSARRRTKAY TFDNQIPNQV IYERKKYLAE
351  VAKRVGQKEM MKRLGETTEV LVEKVTGQVA TGHSPYFEKV SFPVVGTVAI
401  NTLVSVRLDR VEEEGLIGEI V*
```

The cp6761 nucleotide sequence <SEQ ID 262> is:

```
   1  ATGACGGTTG CGGAAGTCAA AGGAACATTT AAGCTGGTCT GTTTAGGCTG
  51  TCGGGTGAAT CAGTATGAGG TCCAAGCATA TCGCGACCAG TTGACTATCT
 101  TAGGTTACCA AGAGGTCCTG GATTCTGAAA TCCCTGCAGA TTTATGCATA
 151  ATCAATACGT GTGCTGTCAC AGCTTCTGCT GAGAGTTCGG GTCGTCATGC
 201  TGTGCGTCAG TTATGTCGTC AGAACCCTAC AGCACATATT GTTGTCACAG
 251  GTTGTTTGGG GGAATCTGAC AAAGAGTTTT TTGCTTCTTT GGATCGGCAA
 301  TGCACACTTG TTTCCAATAA AGAAAAATCC CGACTTATAG AAAAAATTTT
 351  TTCCTATGAT ACGACCTTCC CTGAGTTCAA GATCCATAGT TTTGAGGGAA
 401  AGTCTCGAGC TTTTATTAAA GTTCAAGATG GCTGTAATTC TTTTTGCTCG
 451  TACTGCATTA TTCCTTATTT GCGGGGGCGT TCGGTTTCTC GTCCTGCTGA
 501  GAAGATTTTA GCTGAAATCG CAGGGGTTGT AGACCAAGGA TATCGCGAAG
 551  TTGTAATTGC AGGAATTAAT GTTGGAGATT ATTGCGATGG AGAGCGTTCA
 601  TTAGCCTCTT TGATTGAACA GGTGGACCGG ATTCCTGGAA TTGAGAGGAT
 651  TCGAATTTCC TCTATAGATC CTGATGATAT CACTGAAGAT CTGCACCGTG
 701  CCATCACCTC ATCGCGTCAC ACTTGTCCTT CGTCACACCT TGTTCTTCAA
 751  TCGGGGTCGA ATTCAATTTT AAAGAGAATG AACCGGAAGT ATTCTCGCGG
 801  AGATTTTTTA GATTGTGTAG AGAAGTTCCG TGCTTCTGAT CCTCGCTATG
 851  CCTTTACTAC AGATGTGATT GTCGGATTTC CTGGAGAGAG TGATCAAGAT
 901  TTTGAAGATA CTTTGAGAAT TATTGAAGAT GTAGGCTTTA TTAAAGTGCA
 951  TAGTTTCCCT TTCAGTGCTC GTCGTCGTAC TAAGGCATAT ACTTTTGATA
1001  ATCAGATTCC CAATCAGGTG ATCTATGAGA GGAAGAAGTA TCTTGCTGAG
1051  GTTGCTAAGA GGGTAGGCCA GAAAGAGATG ATGAAGCGTT TAGGAGAGAC

1101  TACAGAGGTG CTTGTTGAGA AAGTAACGGG GCAGGTTGCT ACGGGTCACT
1151  CTCCTTATTT TGAAAAGGTT TCTTTCCCTG TTGTAGGAAC GGTAGCTATC
1201  AACACTCTAG TTTCTGTGCG TCTTGATAGG GTAGAGGAAG AAGGGCTGAT
1251  TGGGGAGATT GTATGA
```

The PSORT algorithm predicts inner membrane (0.1574).

The protein was expressed in *E.coli* and purified as a GST-fusion product (Figure 131A) and also as a his-tagged product. The recombinant proteins were used to immunise mice, whose sera were used in a Western blot (Figure 131B) and for FACS analysis.

These experiments show that cp6761 is a surface-exposed and immunoaccessible protein, and that it is a useful immunogen. These properties are not evident from the sequence alone.

**Example 132**

The following *C.pneumoniae* protein (PID 4376766) was expressed <SEQ ID 263; cp6766>:

```
  1  MATSVPVTSS TSVGEANSSN ERFTERTSRM YYAALVLGAL SCLIFIAMIV
 51  IFPQVGLWAV VLGFALGCLL LSLAIVFAVS GLVLGKTLEP SREATPPEIV
101  AQKEWTTQQD VLGNEYWRSE LISLFLRGDL HESLIVDSKD RSLDIDQSLQ
151  NILKLEPLST TLSLLKKDCV HINIILHLVR QWNLLGVDLS PEVTAHAEEL
201  LLFLIEEQYY SPDILKLIRY GDALQATSPL MDWADSGSFS VDADGVFSCR
251  REECSPEDAL AQFDLLLALE NPDRRFLKDS FLTYIWSSSF FEKFLHRHLE
301  SLQRKLPETA IDVARYEAQI QTFLSRYFQK LDLINAMSLD WGYNCAEGEK
351  CYESANQRLD NLFIAFSSSV PAMKRLFDKY GSVVRVDRRQ IREQILSNTE
401  ILENESGFLC SLYEYPLSYL IDWAVLLDCV RGTEISLEDQ ADYTVCLQGL
451  DSMLSQFASR LQSGQKVLNP RDVLSEQAAV MLVHGLAAQG VSFQGLKALM
501  YLTAVPQRMW LGALPLFESF PVFNRMKEFL GESLGD*
```

The cp6766 nucleotide sequence <SEQ ID 264> is:

```
   1  ATGGCAACCT CTGTTCCTGT AACTTCATCT ACTTCTGTAG GAGAGGCTAA
  51  CTCCTCCAAC GAAAGATTTA CTGAACGAAC ATCGCGAATG TATTACGCAG
 101  CTTTAGTCCT AGGGGCTTTG AGCTGTTTAA TTTTTATTGC TATGATTGTC
 151  ATTTTCCCAC AGGTCGGATT GTGGGCTGTG GTCCTCGGGT TTGCTCTTGG
 201  ATGTTTACTT TTAAGCTTAG CTATCGTTTT TGCTGTCTCC GGTCTCGTTT
 251  TAGGCAAGAC TTTAGAACCT AGTCGAGAAG CGACTCCTCC AGAAATTGTT
 301  GCGCAAAAGG AGTGGACTAC ACAACAAGAT GTCTTAGGGA ATGAGTATTG
 351  GCGTTCCGAG TTGATTTCCT TGTTCTTACG AGGGGATCTC CACGAATCTC
 401  TGATTGTTGA TTCTAAGGAT CGATCTTTAG ATATTGATCA GAGTTTACAA
 451  AATATATTGA AACTTGAGCC CCTATCTACG ACACTTTCGC TGTTAAAGAA
 501  AGATTGTGTC CACATCAATA TCATTTTACA TTTAGTGAGA CAGTGGAACT
 551  TACTGGGAGT GGATCTTAGT CCTGAAGTCA CTGCGCACGC CGAGGAACTT
 601  CTACTCTTTT TGATAGAAGA GCAGTATTAC TCTCCTGATA TTTTGAAATT
 651  GATTCGCTAC GGAGATGCTT TACAAGCAAC GTCTCCTTTG ATGGATTGGG
 701  CAGATTCAGG TTCCTTTAGT GTAGACGCAG ACGGGGTATT TAGCTGTCGC
 751  AGAGAAGAAT GTTCTCCTGA GGATGCTTTG GCGCAATTCG ATCTTCTTTT
 801  GGCGTTGGAA AATCCCGACA GACGCTTCTT AAAGGATTCT TTTCTTACCT
 851  ACATTTGGTC GTCTTCATTT TTTGAGAAGT TTTTACATCG CCATCTAGAG
 901  AGCTTGCAAA GAAAGCTCCC AGAGACAGCG ATCGATGTCG CCCGCTATGA
 951  AGCACAAATA CAAACATTTC TCTCTCGCTA TTTTCAGAAG CTCGATTTGA
1001  TAAACGCAAT GTCCTTAGAT TGGGGATATA ACTGTGCTGA GGGAGAAAAA
1051  TGTTATGAGA GCGCAAATCA AAGATTAGAC AACCTATTTA TTGCTTTTTC
1101  TTCTTCTGTT CCTGCTATGA AGCGGCTCTT TGACAAATAT GGTTCTGTGG
1151  TACGGGTAGA TCGTAGGCAG ATTCGTGAGC AGATTCTTTC GAACACTGAA
1201  ATCTTAGAAA ATGAGTCAGG GTTCCTCTGC AGTTTGTATG AATATCCTTT
1251  ATCCTATTTG ATAGATTGGG CTGTTTTGCT AGACTGTGTT CGCGGTACCG
1301  AAATCTCTCT AGAAGATCAG GCCGATTACA CCGTTTGTTT GCAAGGCTTG
1351  GATTCTATGT TATCTCAATT TGCGAGTCGT TTACAGTCTG GACAAAAAGT
1401  ATTGAATCCT AGAGATGTTT TAAGTGAACA GGCTGCGGTT ATGCTTGTTC
1451  ATGGCTTGGC AGCACAGGGC GTGTCGTTTC AAGGATTGAA AGCTTTGATG
1501  TATTTGACAG CCGTTCCCCA AAGAATGTGG TTAGGAGCAT TGCCTTTATT
1551  TGAATCTTTT CCTGTCTTTA ATCGGATGAA AGAATTTCTT GGGGAATCTC
1601  TGGGAGACTA G
```

The PSORT algorithm predicts inner membrane (0.6158).

The protein was expressed in *E.coli* and purified as a GST-fusion product (Figure 132A) and also as a his-tagged product. The recombinant proteins were used to immunise mice, whose sera were used in a Western blot (Figure 132B) and for FACS analysis.

These experiments show that cp6766 is a surface-exposed and immunoaccessible protein, and that it is a useful immunogen. These properties are not evident from the sequence alone.

**Example 133**

The following *C.pneumoniae* protein (PID 4376804) was expressed <SEQ ID 265; cp6804>:

```
  1  MSNQLQPCIS LGCVSYINSF PLSLQLIKRN DIRCVLAPPA DLLNLLIEGK
 51  LDVALTSSLG AISHNLGYVP GFGIAANQRI LSVNLYAAPT FFNSPQPRIA
101  ATLESRSSIG LLKVLCRHLW RIPTPHILRF ITTKVLRQTP ENYDGLLLIG
151  DAALQHPVLP GFVTYDLASG WYDLTKLPFV FALLLHSTSW KEHPLPNLAM
201  EEALQQFESS PEEVLKEAHQ HTGLPPSLLQ EYYALCQYRL GEEHYESFEK
251  FREYYGTLYQ QARL
```

The cp6804 nucleotide sequence <SEQ ID 266> is:

```
  1  ATGTCTAACC AACTCCAGCC ATGTATAAGC TTAGGCTGCG TAAGTTATAT
 51  TAATTCCTTT CCGCTGTCCC TACAACTCAT AAAAAGAAAC GATATTCGCT
101  GTGTTCTTGC TCCCCCTGCA GACCTCCTCA ACTTGCTAAT CGAAGGGAAA
151  CTCGATGTTG CTTTGACCTC ATCCCTAGGA GCTATCTCTC ATAACTTGGG
201  GTATGTCCCC GGCTTTGGAA TTGCAGCAAA CCAACGTATC CTCAGTGTAA
251  ACCTCTATGC AGCTCCCACT TTCTTTAACT CACCGCAACC TCGGATTGCC
301  GCAACTTTAG AAAGTCGCTC CTCTATAGGA CTCTTAAAAG TGCTTTGTCG
351  TCATCTCTGG CGCATCCCAA CTCCTCATAT CCTAAGATTC ATAACTACAA
401  AAGTACTCAG ACAAACCCCT GAAAATTATG ATGGCCTCCT CCTAATCGGA
451  GATGCAGCGC TACAACATCC TGTACTTCCT GGATTTGTAA CCTATGACCT
501  TGCCTCGGGG TGGTATGATC TTACAAAGCT ACCTTTTGTA TTTGCTCTTC
551  TTCTACACAG CACCTCTTGG AAAGAACATC CCCTACCCAA CCTTGCGATG
601  GAAGAAGCCC TCCAACAGTT CGAATCTTCA CCCGAAGAAG TCCTTAAAGA
651  AGCTCATCAA CATACAGGTC TGCCCCCTTC TCTTCTTCAA GAATACTATG
701  CCCTATGCCA GTACCGTCTA GGAGAAGAAC ACTACGAAAG CTTTGAAAAA
751  TTCCGGGAAT ATTATGGAAC CCTCTACCAA CAAGCCCGAC TGTAA
```

The PSORT algorithm predicts inner membrane (0.060).

The protein was expressed in *E.coli* and purified as a GST-fusion product (Figure 133A). The recombinant protein was used to immunise mice, whose sera were used in a Western blot (Figure 133B) and for FACS analysis.

These experiments show that cp6804 is a surface-exposed and immunoaccessible protein, and that it is a useful immunogen. These properties are not evident from the sequence alone.

**Example 134**

The following *C.pneumoniae* protein (PID 4376805) was expressed <SEQ ID 267; cp6805>:

```
  1  MSSLLSCGRI EPTRVTCSLK TYLEDTSQNQ LSTRLVRASV IFLCALLIIL
 51  VCVALSSLIP SIMALATSFT VMGLILFVMS LLGDVAIISY LTYSTVTSYR
101  QNKRAFEIHK PARSVYYEGV RHWDLGRSSL GTGEIPIVRT LFSPFQNHGL
151  NHALAAKIFL FMEHFSPEPP NEPLVDWACL IRDFRPHVSS LCFVIEKQGS
201  SLRTKEGNTI CEAFRSDYDA HFAMVDCYRL IHSKLIIEKM GLKNIDIIPS
251  VMVREDYPSR PGEGYREGLL RMYGGKGAL*
```

The cp6805 nucleotide sequence <SEQ ID 268> is:

```
  1  ATGTCATCAC TACTGAGCTG CGGAAGAATA GAGCCGACTC GGGTTACCTG
 51  TAGCTTAAAG ACGTATCTTG AGGATACGAG TCAGAATCAG TTGAGCACAC
101  GTCTAGTTCG GGCAAGTGTC ATCTTTTTAT GCGCATTGTT GATCATTTTG
151  GTTTGTGTGG CCCTCTCTAG TTTGATTCCA AGCATTATGG CCTTGGCGAC
201  CTCTTTTACG GTAATGGGGT TAATTCTTTT TGTGATGTCA CTTCTTGGTG
251  ACGTTGCAAT TATAAGTTAT CTTACTTATA GCACTGTTAC GAGTTACCGG
301  CAAAATAAGA GAGCTTTTGA GATTCACAAG CCCGCTCGCT CCGTTTACTA
351  CGAGGGGGTC CGCCATTGGG ATTTAGGACG ATCATCTTTA GGCACAGGCG
401  AGATTCCTAT AGTAAGGACG TTATTCTCTC CATTTCAGAA CCATGGTCTT
451  AACCATGCCT TAGCTGCTAA AATTTTCCTA TTTATGGAGC ATTTCAGCCC
501  TGAGCCACCG AACGAGCCTT TGGTGGATTG GGCCTGTTTG ATTCGGGATT
551  TTAGGCCTCA CGTCAGTTCT TTGTGCTTTG TTATTGAAAA ACAAGGGTCA
601  TCGCTGAGGA CTAAGGAAGG CAATACGATT TGTGAGGCTT TCCGCTCTGA
651  TTACGACGCC CATTTTGCTA TGGTAGATTG CTACCGGTTG ATCCACTCTA
701  AGTTGATTAT AGAGAAAATG GGATTGAAGA ATATCGATAT CATTCCGAGT
751  GTCATGGTTC GTGAAGATTA TCCTAGCCGT CCTGGGGAGG GCTATCGCGA
801  AGGCCTATTA CGTATGTATG GTGGCAAGGG GGCTCTGTGA
```

The PSORT algorithm predicts inner membrane (0.711).

The protein was expressed in *E.coli* and purified as a GST-fusion product (Figure 134A). The recombinant protein was used to immunise mice, whose sera were used in a Western blot (Figure 134B) and for FACS analysis.

These experiments show that cp6805 is a surface-exposed and immunoaccessible protein, and that it is a useful immunogen. These properties are not evident from the sequence alone.

**Example 135**

The following *C.pneumoniae* protein (PID 4376813) was expressed <SEQ ID 269; cp6813>:

```
  1  MSGPSRTESS QVSVLSYVPR DKEIAPKKQF TIAKISTLAI LASLALGALV
 51  AGISLTIVLG NPVFLALLIT TALFSVVTFL VYHQMTSKVS SNWQKVLEQN
101  FKPLGKAWQE KNVDCYSNEM QFYNNHLNPK FKVAIQTDAS QPFQPTFLTG
151  LRVIEKNQST GIIFNPVGPT NLIDNTATNL STILYSTLKD KSVWDTCKQR
201  EGGPAKGEDP FSPTEVRVVK LPNEALDQTF NLNLSSAEKK SILPTFLGHV
251  CGPKSEELPN QQEYYRQALL AYENCLKAAI ESHAAIVALP LFTSVYEVPP
301  EEILPKEGTF YWDNQTQAFC KRALLDAIQN TALRYPQRSL LVILQDPFNT
351  IESQSRSEE*
```

The cp6813 nucleotide sequence <SEQ ID 270> is:

```
   1  ATGTCAGGAC CCTCACGTAC TGAGAGCTCT CAAGTTTCTG TACTATCCTA
  51  TGTGCCTCGG GATAAAGAAA TTGCTCCTAA AAAACAGTTT ACCATAGCAA
 101  AAATATCCAC TCTTGCAATC CTAGCTTCTT TAGCTTTAGG AGCTTTGGTG
 151  GCTGGAATCT CTTTAACGAT AGTATTAGGG AACCCTGTAT TTTTGGCTCT
 201  TCTCATTACC ACGGCCCTCT TCTCAGTTGT AACCTTCTTA GTCTACCACC
 251  AAATGACCTC AAAGGTATCT TCTAACTGGC AGAAAGTTCT AGAGCAAAAC
 301  TTCAAGCCTT TGGGAAAAGC GTGGCAAGAA AAAAACGTAG ACTGCTACTC
 351  AAACGAGATG CAATTTTACA ATAATCACCT GAACCCTAAG TTCAAGGTAG
 401  CGATACAAAC AGATGCGTCT CAACCATTTC AGCCTACTTT CTTAACTGGA
 451  CTTAGAGTGA TCGAAAAAAA TCAATCCACA GGGATCATCT TTAATCCCGT
 501  AGGCCCAACG AATCTGATCG ACAACACTGC AACGAACCTC TCTACTATCC
 551  TTTACTCCAC CCTAAAAGAT AAAAGCGTGT GGGATACATG CAAGCAACGC
 601  GAAGGGGGTC CCGCAAAAGG AGAAGACCCC TTTTCCCCTA CCGAAGTGAG
 651  AGTAGTAAAA CTTCCAAACG AAGCTCTAGA TCAAACGTTT AATCTAAATT
 701  TAAGCTCTGC AGAAAAGAAA AGTATTCTTC CGACCTTTTT AGGCCACGTA
 751  TGCGGCCCTA AATCTGAAGA GTTACCAAAT CAGCAAGAAT ATTATCGCCA
 801  AGCTTTACTA GCGTACGAGA ACTGCCTTAA AGCAGCTATA GAAAGTCATG
 851  CAGCAATCGT TGCTCTTCCT CTCTTTACTT CGGTCTATGA AGTGCCTCCA
 901  GAAGAGATTC TTCCTAAAGA AGGCACTTTC TATTGGGACA ACCAAACTCA
 951  AGCGTTTTGC AAACGCGCTT TATTGGACGC TATTCAAAAT ACGGCCCTAC
1001  GCTATCCTCA AAGATCTTTA CTTGTTATAC TCCAAGATCC TTTTAATACT
1051  ATAGAATCAC AAAGTCGTTC TGAGGAGTAA
```

The PSORT algorithm predicts inner membrane (0.4291).

The protein was expressed in *E.coli* and purified as a GST-fusion product (Figure 135A). The recombinant protein was used to immunise mice, whose sera were used in a Western blot (Figure 135B) and for FACS analysis.

These experiments show that cp6813 is a surface-exposed and immunoaccessible protein, and that it is a useful immunogen. These properties are not evident from the sequence alone.

**Example 136**

The following *C.pneumoniae* protein (PID 4376844) was expressed <SEQ ID 271; cp6844>:

```
  1  MWRVVLRFLI IFILGRAVFP LRASESFSWE TSTCLTVLGI PFIDIILTTN
 51  EDFVAQCGLQ IGTISSTNNA KIKEIFLIYK EKFPEASISF KRKEPLNLSQ
101  SHLSDLGILC MRNGETYAEG MANKENGPAL KQPKDLRLVL RCPNQPDTLL
151  YSEKEAEKGI ETNTCLCNQG YTLLDGQLIL YGDSIEKFLK ETKRKNNHTL
201  VDLCDSQVVT TFLGRFWSLL NYVQVLFLSE DSAKILAGIP DLAQATQLLS
251  HTVPLLFIYT NDSIHIIEQG KESSFTYNQD LTEPILGFLF GYINRGSMEY
301  CFNCAQSSLG ET*
```

The cp6844 nucleotide sequence <SEQ ID 272> is:

```
  1 ATGTGGCGCG TTGTCCTCAG ATTCCTTATA ATTTTTATCT TGGGAAGAGC
 51 CGTCTTCCCT CTAAGAGCTT CAGAAAGCTT CTCCTGGGAA ACATCGACCT
101 GTTTAACAGT GCTAGGGATT CCTTTCATAG ATATTATCCT CACAACGAAT
151 GAGGACTTTG TTGCCCAGTG CGGCCTGCAA ATAGGAACCA TTTCTTCGAC
201 TAATAACGCA AAAATAAAAG AAATTTTTTT GATATATAAG GAAAAATTTC
251 CAGAAGCCTC TATCAGTTTC AAACGAAAAG AACCTCTAAA CCTTTCCCAA
301 TCCCATCTCT CCGATTTAGG TATTTTATGT ATGCGTAACG GAGAAACTTA
351 CGCTGAGGGA ATGGCAAATA AAGAAAACGG ACCCGCTCTA AAACAACCCA
401 AGGATCTAAG ATTAGTTTTA CGTTGTCCTA ACCAACCAGA TACCCTGCTC
451 TACTCGGAAA AAGAAGCAGA AAAGGGCATA GAAACAAATA CTTGCCTATG
501 CAATCAGGGA TACACACTCC TGGATGGGCA ATTGATTCTC TACGGGGATA
551 GTATAGAAAA GTTTCTGAAA GAGACCAAAA GAAAGAATAA CCACACGCTT
601 GTTGATCTTT GTGACTCACA AGTCGTGACC ACGTTCCTCG GTCGCTTTTG
651 GTCTCTTCTA AACTACGTTC AAGTTCTTTT CCTATCTGAA GACTCCGCTA
701 AAATTCTTGC GGGCATCCCA GACCTAGCTC AAGCTACGCA ATTGCTTTCC
751 CACACCGTAC CTTTGCTTTT TATTTATACC AACGATTCTA TTCACATCAT
801 AGAACAAGGC AAAGAAAGTA GTTTTACCTA TAACCAAGAT TTAACAGAGC
851 CCATTTTAGG ATTTCTCTTT GGTTACATAA ATCGCGGCTC TATGGAATAC
901 TGCTTTAATT GTGCACAGTC TTCATTAGGA GAAACCTAA
```

The PSORT algorithm predicts inner membrane (0.1786).

The protein was expressed in *E.coli* and purified as a GST-fusion product (Figure 136A) and also in his-tagged form. The recombinant proteins were used to immunise mice, whose sera were used in a Western blot (Figure 136B) and for FACS analysis.

These experiments show that cp6844 is a surface-exposed and immunoaccessible protein, and that it is a useful immunogen. These properties are not evident from the sequence alone.

### Example 137

The following *C.pneumoniae* protein (PID 4377201) was expressed <SEQ ID 273; cp7201>:

```
  1 VLVGICPSLY PEHPRSFYYR VSGDIGSRFD DRGFVNSGVE TLPYSSGSFG
 51 IFWISFTDPT FNFAIVNTFM RTAGINEVSR PMTQDTETSL IEMRDLSEQQ
101 EANNTDSLEQ EESLMGIVGH TVGGVSMTVT SSPNIFYRIQ TLLGLPETLA
151 EAEENPTFPN STIDSLAEIM MNLVRISDAV SIFWIFPIVD TTYNGVLLAV

201 CIGFFGINGI CSTFLMLTNP RSRRDRWRNL RIMVLCYRSL GSGMNLFDLS
251 NNVRMAARRH VTSCTVALYA MVTLFGWTVA IQDALQYGFP SVRDAFYRYC
301 LRHRYCLTQR NEDSLQTTGT RFQVTRTHLE DQQMVASILN LSVFGLFFGF
351 VGLMTTFGGL EISPSCRWDA ANNRTVGIF*
```

The cp7201 nucleotide sequence <SEQ ID 274> is:

```
   1 GTGCTCGTTG GTATCTGTCC TTCTCTATAT CCAGAACATC CTCGCTCCTT
  51 TTATTATCGT GTTTCTGGAG ATATAGGCTC CCGATTCGAC GATAGAGGAT
 101 TTGTAAACTC TGGAGTCGAA ACCCTGCCAT ACTCTTCAGG CAGCTTTGGG
 151 ATTTTTTGGA TCTCGTTTAC GGATCCCACA TTTAATTTTG CTATCGTAAA
 201 TACCTTTATG CGAACTGCAG GGATCAATGA AGTCTCTAGA CCCATGACAC
 251 AAGATACAGA AACTTCATTG ATAGAAATGA GAGACCTAAG TGAACAACAA
 301 GAAGCGAATA ACACAGATTC TTTAGAGCAA GAAGAGAGCT TAATGGGTAT
 351 TGTAGGACAT ACTGTGGGAG GAGTTTCCAT GACCGTGACC TCCAGTCCAA
 401 ATATCTTTTA TCGTATACAA ACACTTCTGG GACTGCCAGA GACTCTTGCA
 451 GAAGCTGAAG AAAATCCTAC CTTCCCAAAT TCTACTATAG ATAGCCTTGC
 501 AGAAATAATG ATGAACCTCG TAAGGATCTC TGATGCTGTC TCTATTTTCT
 551 GGATTTTTCC TATCGTAGAT ACTACATATA ATGGAGTTTT ATTAGCCGTC
 601 TGTATCGGCT TCTTCGGAAT CAATGGGATT TGTTCCACGT TCCTTATGCT
 651 TACGAATCCA CGCTCTCGTC GAGATAGATG GAGGAATTTA CGCATCATGG
 701 TTCTTTGCTA TCGTTCTTTG GGAAGCGGAA TGAATCTCTT TGATCTTAGC
 751 AATAATGTGC GCATGGCAGC ACGTAGGCAT GTGACATCAT GTACAGTAGC
 801 TCTCTATGCT ATGGTCACTC TATTTGGATG GACAGTAGCA ATACAAGATG
 851 CTTTGCAATA TGGTTTCCCT AGCGTTCGGG ATGCCTTCTA TAGATATTGC
 901 TTACGCCACA GATATTGCTT AACTCAAAGA AACGAAGACT CTCTGCAAAC
 951 TACAGGAACG CGCTTTCAGG TTACCCGTAC ACATCTAGAA GATCAACAGA
1001 TGGTGGCTTC TATTTTGAAT TTGAGTGTTT TTGGGCTCTT TTTTGGATTC
1051 GTAGGGCTAA TGACCACGTT TGGAGGATTA GAAATCTCAC CATCTTGTCG
1101 GTGGGATGCA GCAAATAACC GAACGGTAGG TATTTTTTAG
```

The PSORT algorithm predicts inner membrane (0.3102).

The protein was expressed in *E.coli* and purified as a GST-fusion product (Figure 137A). The recombinant protein was used to immunise mice, whose sera were used in a Western blot (Figure 137B) and for FACS analysis.

These experiments show that cp7201 is a surface-exposed and immunoaccessible protein, and that it is a useful immunogen. These properties are not evident from the sequence alone.

**Example 138**

The following *C.pneumoniae* protein (PID 4377251) was expressed <SEQ ID 275; cp7251>:

```
  1 MAPIHGSNAF VEDILHSHPS PQATYFSSTR AQKLHEFKDR HPVLTRIASV
 51 IIKIFKVLIG LIILPLGIYW LCQTLCTNSI LPSKNLLKIF KKQPNTKTLK
101 TNYLHALQDY SSKNRVASMR RVPILQDNVL IDTLEICLSQ APTNRWMLIS
151 LGSDCSLEEI ACKEIFDSWQ RFAKLIGANI LVYNYPGVMS STGSSSLKDL
201 ASAHNICTRY LKDKEQGPGA KEIITYGYSL GGLIQAEALR DQKIVANDDT
251 TWIAVKDRCP LFISPEGFHS CRRIGKLVAR LFGWGTKAVE RSQDLPCLEI
301 FLYPTDSLRR STVRQNKLLA PELTLAHAIK NSPYVQNKEF IEVRLSSDID
351 PIDSKTRVAL ATPILKKLS*
```

The cp7251 nucleotide sequence <SEQ ID 276> is:

```
  1 ATGGCTCCAA TTCACGGAAG TAATGCGTTT GTTGAGGATA TTTTACATTC
 51 CCACCCTTCT CCACAAGCGA CTTATTTTTC TTCAACACGC GCCCAAAAAC
101 TTCATGAGTT TAAAGACAGG CATCCCGTGC TTACACGGAT TGCTTCTGTA
151 ATTATTAAAA TTTTTAAAGT TCTGATAGGG CTGATCATCC TTCCCTTAGG
201 AATCTACTGG CTATGTCAAA CGCTTTGTAC AAACTCGATT CTCCCTTCCA
251 AGAATTTATT AAAAATTTTC AAGAAGCAAC CCAACACTAA AACCTTAAAA
301 ACTAATTATT TGCATGCTTT GCAAGATTAT TCCTCGAAAA ACCGCGTTGC
351 TTCCATGAGA CGAGTTCCTA TCCTCCAGGA TAATGTTCTC ATCGACACTT
401 TGGAAATATG CCTTTCACAA GCACCTACGA ATCGTTGGAT GCTCATTTCT
451 TTAGGAAGTG ACTGTAGCTT GGAAGAAATC GCTTGTAAGG AGATCTTTGA
```

```
 501 TTCTTGGCAA AGATTTGCCA AGTTGATAGG GGCCAATATA CTCGTTTATA
 551 ACTACCCCGG AGTCATGTCC AGCACAGGGA GCAGCAGCCT AAAGGACCTA
 601 GCATCAGCTC ATAATATTTG TACAAGATAC CTTAAAGATA AAGAACAGGG
 651 CCCTGGAGCA AAAGAAATCA TTACCTATGG GTACTCCCTA GGAGGTTTGA
 701 TACAAGCAGA AGCATTGCGA GACCAGAAGA TTGTTGCAAA CGATGATACT
 751 ACTTGGATAG CAGTCAAAGA TAGGTGTCCT CTCTTTATAT CTCCAGAAGG
 801 TTTCCACAGT TGCAGACGCA TAGGAAAGCT AGTAGCTCGT CTTTTTGGCT
 851 GGGGGACCAA AGCCGTAGAG AGAAGCCAAG ACCTTCCCTG CCTAGAAATT
 901 TTTCTCTATC CTACGGATTC CTTACGAAGA TCAACAGTCA GACAGAACAA
 951 GCTCTTAGCA CCTGAACTTA CTCTCGCTCA TGCGATAAAA AATAGTCCCT
1001 ATGTTCAAAA TAAAGAATTT ATAGAAGTAC GATTATCGTC TGATATCGAT
1051 CCCATCGACA GCAAAACAAG AGTGGCTCTT GCCACACCAA TTTTGAAAAA
1101 GCTCTCTTAG
```

The PSORT algorithm predicts inner membrane (0.4545).

The protein was expressed in *E.coli* and purified as a GST-fusion product (Figure 138A). The recombinant protein was used to immunise mice, whose sera were used in a Western blot (Figure 138B) and for FACS analysis.

These experiments show that cp7251 is a surface-exposed and immunoaccessible protein, and that it is a useful immunogen. These properties are not evident from the sequence alone.

**Example 139**

The following *C.pneumoniae* protein (PID 4377288) was expressed <SEQ ID 277; cp7288>:

```
  1 MHMSNPISLF SPAELIAKYN LIPKTSPIYP RRTELIILEE NACQTRLTNV
 51 AQVLHPSSLF SMSKKILNPC GCSGGPLCWV ILNILAFIIT SVLFIILLPV
101 NLIVAGLRLF MPLPPKKIVE DLSEPTTEET NEVIQPFIFA LQALLFEDNK
151 LRSFKIVEQS VGKAPLPNPF LNRLVAISPQ ESQEAMRKIP DLCSQLKKVL
201 KSLGVLTPEW KHMLKYFEGL KNEHDSNPDK KTFPILIKLL IEALTGKSSL
251 PKTPSTKEKM QAALFIASSC KTCKPTWGEV ITRSLNRLYS IANEGDNQLL
301 IWVQEFKERE LMSIQDGDDA EEYRFAAQQH GERYTEAIEQ VLRNESAAKL
351 QWHVINTMKF FHGKNLGLVT EHLQDTLGAL TLRQTTVDTH QGREDADLSA
401 ALFLNKYLNS GNQLVNSVFK SMQKADPETK ALIREFALDI LYASLRLPQT
451 SAHTEVFSTL LMDPETYEPN KACIAYLLYV LKIIEL*
```

The cp7288 nucleotide sequence <SEQ ID 278> is:

```
   1  ATGCATATGT CTAACCCCAT CTCTTTGTTT TCCCCTGCAG AGTTAATAGC
  51  AAAGTACAAT TTAATTCCAA AAACTTCGCC GATTTATCCT CGGAGGACGG
 101  AACTTATTAT CTTGGAAGAA AATGCGTGTC AAACACGCCT AACCAACGTG
 151  GCTCAGGTCC TACATCCTTC TAGCCTATTC AGTATGTCAA AAAAAATACT
 201  GAATCCCTGC GGGTGCTCTG GTGGTCCCTT ATGTTGGGTG ATTCTCAACA
 251  TCCTAGCATT TATTATTACT TCAGTACTGT TTATCATTCT TTTACCGGTG
 301  AATCTCATCG TAGCAGGTCT TCGTCTCTTC ATGCCTCTTC CCCCTAAAAA
 351  AATCGTAGAG GATTTAAGTG AACCTACTAC TGAAGAAACG AATGAGGTCA
 401  TTCAACCCTT CATTTTCGCT TTGCAAGCGT TGCTTTTTGA GGATAACAAA
 451  CTTCGCTCTT TTAAAATTGT TGAACAAAGT GTAGGCAAAG CACCCTTACC
 501  TAATCCCTTT TTAAATAGAC TAGTAGCAAT TTCGCCGCAA GAAAGCCAAG
 551  AAGCCATGCG GAAGATTCCG GATCTATGCT CACAACTGAA AAAAGTATTA
 601  AAGTCTCTAG GCGTGCTAAC TCCAGAATGG AAGCACATGC TGAAGTACTT
 651  TGAGGGACTG AAAAACGAAC ATGATAGTAA TCCTGATAAA AAGACGTTCC
 701  CAATATTGAT CAAGCTCCTC ATAGAAGCTC TTACTGGAAA GTCCTCTTTA
 751  CCCAAAACTC CTAGTACAAA GGAAAAAATG CAAGCGGCCT TATTTATTGC
 801  AAGTTCTTGC AAGACTTGTA AGCCGACTTG GGGAGAAGTC ATAACCAGAT
 851  CTCTTAACAG ACTCTATAGT ATAGCTAATG AAGGAGACAA TCAGCTTCTG
 901  ATTTGGGTTC AAGAGTTTAA AGAACGAGAG CTGATGTCCA TCCAAGATGG
 951  TGATGATGCT GAAGAGTATC GGTTTGCGGC TCAGCAACAC GGTGAGCGTT
1001  ACACAGAGGC AATAGAACAA GTTCTACGAA ACGAGTCAGC AGCCAAACTA
1051  CAATGGCATG TGATCAACAC TATGAAATTC TTCCATGGGA AAAATCTCGG
1101  TCTAGTTACA GAACACCTAC AAGATACTCT CGGCGCCCTA ACTTTACGTC
1151  AAACTACAGT GGACACACAT CAAGGCAGAG AAGACGCTGA TTTGTCAGCT
1201  GCTCTTTTCC TAAATAAGTA TTTAAATTCT GGAAATCAAC TTGTTAATAG

1251  CGTCTTTAAA TCCATGCAAA AAGCAGATCC AGAAACCAAA GCTTTAATCC
1301  GTGAGTTTGC TCTAGATATA TTATATGCAT CCTTACGGCT TCCTCAAACT
1351  TCCGCTCATA CCGAGGTCTT TTCTACACTC TTAATGGACC CAGAGACCTA
1401  TGAACCTAAT AAAGCTTGTA TCGCCTACTT GCTCTATGTA TTAAAGATCA
1451  TCGAACTATA A
```

The PSORT algorithm predicts inner membrane (0.5989).

The protein was expressed in *E.coli* and purified as a GST-fusion product (Figure 139A). The recombinant protein was used to immunise mice, whose sera were used in a Western blot (Figure 139B) and for FACS analysis.

These experiments show that cp7288 is a surface-exposed and immunoaccessible protein, and that it is a useful immunogen. These properties are not evident from the sequence alone.

**Example 140**

The following *C.pneumoniae* protein (PID 4377359) was expressed <SEQ ID 279; cp7359>:

```
  1  MPGSVSSPPL SPVIVRERVP SSSGSDLIQP HAVLKISILI FALVTILGIV
 51  LVVLSSALGA LPSLVLTVSG CIAIAVGLIG LGILVTRLIL STIRKVDAMG
101  YDAAVKEEQY LSRIRELESE NREIRDRNRA VEDQCAHLSE ENKDLRDPEY
151  LHGMTERLIA SLEIENQALV AENILLKDWN ASLSRDFRAY KQKFPLGALE
201  PWKEDIACIM EQNLFLKPEC IAMVKSLPLE TQRLFLYPKG FQSLVNRFAP
251  RSRFFQTPKY EYNSRNENED GKVAAVCARL KKEFFSAVLG ACSYEELGGI
301  CERAVALKET LPLPEAVYDT LVQEFPNLLT AESLWKEWCF YSYPYLRPYL
351  SVDYCKRLFV QLFEELCLKL FTTGSPEDQA LVRLFSYYRN HIPAVLASFG
401  LPPPETGGSV FVLLPKQENL LWSQIEVLAT RYLKDTFVRN SEWTGSFEMM
451  FSYNEMCKEI SEGRIRFAED YETRHSEEFP PSPLSEEGEG EEFLPPCSEE
501  EVSVLERPDL DVDSMWVWHP PVPKGPL*
```

The cp7359 nucleotide sequence <SEQ ID 280> is:

```
   1  ATGCCAGGTT CTGTGTCATC ACCTCCTTTG TCTCCTGTAA TTGTCCGTGA
  51  AAGGGTCCCA TCCTCTTCAG GATCCGACCT CATACAGCCT CATGCTGTTT
 101  TAAAGATCTC CATCCTAATT TTTGCGCTTG TGACAATTTT AGGAATTGTT
 151  CTTGTAGTGT TGTCTAGTGC TTTAGGAGCT CTTCCTAGTT TAGTTTTGAC
 201  GGTTTCTGGT TGTATTGCAA TAGCTGTAGG CCTGATTGGT TTAGGGATTC
 251  TTGTGACACG GCTGATTCTC TCTACGATCA GAAAAGTAGA TGCCATGGGT
 301  TATGATGCTG CGGTCAAAGA AGAGCAGTAT TTGTCACGTA TCAGAGAATT
 351  AGAGTCTGAA AATAGAGAGA TTAGAGATAG AAATCGTGCT GTCGAAGATC
 401  AGTGTGCCCA TTTATCCGAA GAGAACAAGG ACCTTAGGGA TCCCGAATAT
 451  CTACATGGAA TGACTGAAAG GCTCATTGCG AGCTTAGAAA TAGAGAATCA
 501  AGCTCTCGTA GCTGAGAACA TTCTTCTCAA AGACTGGAAT GCAAGCCTAT
 551  CTAGAGATTT CCGCGCATAT AAGCAAAAAT TTCCTCTTGG GGCATTAGAA
 601  CCCTGGAAAG AAGATATTGC ATGTATCATG GAACAAAATC TCTTTTTAAA
 651  ACCGGAATGT ATCGCGATGG TTAAGTCTCT TCCATTAGAG ACGCAACGGC
 701  TGTTTTTATA TCCAAAAGGA TTTCAGTCTT TAGTTAATCG ATTTGCTCCG
 751  CGGTCTCGCT TTTTCCAGAC TCCAAAGTAT GAATATAACA GTAGGAATGA
 801  AAATGAGGAC GGAAAGGTAG CCGCAGTGTG CGCCCGTTTG AAAAAAGAAT
 851  TCTTCAGTGC TGTTTTAGGA GCCTGTAGTT ACGAAGAACT AGGGGGCATT
 901  TGTGAAAGAG CAGTAGCACT TAAAGAGACG TTGCCATTGC CTGAAGCTGT
 951  CTATGATACC CTAGTTCAGG AGTTCCCAAA TCTTCTTACT GCTGAGAGTT
1001  TATGGAAAGA ATGGTGCTTC TATTCCTATC CCTACCTTCG TCCCTATCTT
1051  TCTGTGGATT ACTGTAAGAG GTTATTTGTA CAACTTTTTG AGGAACTCTG
1101  CCTAAAGCTT TTTACAACGG GATCTCCAGA AGACCAAGCT TTGGTTCGCC
1151  TTTTCTCTTA CTATAGGAAT CATATTCCCG CAGTCTTGGC CTCATTTGGT
1201  TTGCCCCCGC CTGAGACAGG GGGGTCTGTA TTTGTATTGC TACCAAAACA
1251  AGAAAACCTT CTTTGGAGTC AAATTGAGGT GCTGGCTACA AGGTATCTCA
1301  AAGATACCTT CGTGAGAAAC TCAGAATGGA CGGGCTCTTT CGAGATGATG
1351  TTTTCTTATA ACGAGATGTG TAAGGAGATC TCCGAAGGAA GGATTCGTTT
1401  TGCTGAAGAC TATGAAACGA GGCATTCCGA AGAATTCCCT CCTTCCCCTC
1451  TCTCTGAAGA AGGAGAGGGC GAAGAATTCC TTCCTCCTTG CTCTGAAGAA
1501  GAGGTTTCGG TTCTTGAGCG CCCAGATCTA GATGTAGACT CTATGTGGGT
1551  CTGGCATCCG CCGGTCCCTA AGGGACCTCT TTAA
```

The PSORT algorithm predicts inner membrane (0.7453).

The protein was expressed in *E.coli* and purified as a GST-fusion product (Figure 140A). The recombinant protein was used to immunise mice, whose sera were used in a Western blot (Figure 140B) and for FACS analysis.

These experiments show that cp7359 is a surface-exposed and immunoaccessible protein, and that it is a useful immunogen. These properties are not evident from the sequence alone.

**Example 141**

The following *C.pneumoniae* protein (PID 4377374) was expressed <SEQ ID 281; cp7374>:

```
  1  MDKQSSGNSG CIWHPFTQSA LDSTPIKIVR GEGAYLYAES GTRYLDAISS
 51  WWCNLHGHGH PYITKKLCEQ AQKLEHVIFA NFTHEPALEL VSKLAPLLPE
101  GLERFFFSDN GSTSIEIAMK IAVQYYYNQN KAKSHFVGLS NAYHGDTFGA
151  MSIAGTSPTT VPFHDLFLPS STIAAPYYGK EELAIAQAKT VFSESNIAAF
201  IYEPLLQGAG GMLMYNPEGL KEILKLAKHY GVLCIADEIL TGFGRTGPLF
251  ASEFTDIPPD IICLSKGLTG GYLPLALTVT TKEIHDAFVS QDRMKALLHG
301  HTFTGNPLGC SAALASLDLT LSPECLQQRQ MIERCHQEFQ EAHGSLWQRC
351  EVLGTVLALD YPAEATGYFS QYRDHLNRFF LERGVLLRPL GNTLYVLPPY
401  CIQEEDLRII YSHLQDALCL QPQ*
```

The cp7374 nucleotide sequence <SEQ ID 282> is:

```
   1  ATGGACAAGC AATCATCAGG GAATTCAGGG TGTATCTGGC ACCCCTTCAC
  51  TCAATCTGCA TTAGATTCTA CACCCATAAA GATTGTAAGG GGAGAAGGTG
 101  CTTACCTCTA TGCGGAATCA GGAACAAGAT ATCTTGATGC GATATCTTCA
 151  TGGTGGTGCA ACCTCCACGG TCATGGGCAT CCCTACATTA CAAAAAAATT
 201  ATGTGAGCAA GCACAGAAGT TAGAACATGT GATCTTCGCA AATTTCACCC
 251  ATGAACCGGC TCTAGAGCTC GTATCGAAAC TCGCTCCCCT CCTTCCTGAA
 301  GGTCTAGAAC GTTTCTTTTT CTCTGACAAC GGATCAACGT CTATCGAAAT
 351  AGCAATGAAA ATTGCTGTGC AATATTACTA CAATCAAAAC AAGGCTAAGA
 401  GCCATTTTGT TGGACTCAGC AATGCCTATC ACGGAGATAC ATTTGGAGCT
 451  ATGTCGATAG CTGGCACGAG CCCTACTACA GTTCCCTTTC ATGATCTTTT
 501  TCTTCCTTCC AGTACAATTG CTGCTCCCTA TTATGGCAAG GAAGAGCTTG
 551  CCATTGCCCA AGCAAAAACA GTCTTTTCTG AAAGCAATAT CGCAGCGTTT
 601  ATCTATGAGC CGCTATTGCA AGGTGCTGGA GGGATGTTAA TGTATAATCC
 651  CGAAGGCCTA AAGGAGATTC TCAAGCTTGC CAAGCATTAC GGGGTTCTCT
 701  GTATTGCTGA TGAAATTCTT ACTGGCTTTG GCCGTACGGG TCCACTGTTT
 751  GCTTCTGAAT TTACAGACAT TCCTCCTGAC ATTATCTGTC TTTCTAAAGG
 801  TCTTACAGGA GGCTATCTCC CTCTAGCCTT GACAGTAACC ACTAAAGAAA
 851  TTCATGATGC CTTTGTCTCC CAAGATCGGA TGAAGGCACT GCTTCATGGC
 901  CATACCTTCA CAGGAAATCC TTTAGGCTGT AGTGCTGCCC TCGCTTCTTT
 951  GGATCTCACC CTATCTCCAG AATGCCTACA ACAAAGGCAA ATGATAGAAC
1001  GGTGTCATCA AGAGTTTCAA GAAGCTCATG GTTCCCTATG GCAACGGTGT
1051  GAGGTTCTGG GCACGGTACT CGCTCTAGAT TACCCTGCAG AAGCTACAGG
1101  ATATTTTTCA CAATATAGAG ACCATCTCAA TCGCTTTTTC TTAGAACGTG
1151  GAGTCCTTCT TCGTCCTTTA GGGAACACAC TGTATGTGCT GCCCCCCTAC
1201  TGTATCCAAG AAGAAGATCT CCGGATTATT TATTCTCACC TACAGGATGC
1251  CCTATGTCTA CAACCACAGT AA
```

The PSORT algorithm predicts cytoplasm (0.2930).

The protein was expressed in *E.coli* and purified as a GST-fusion product (Figure 141A) and also in his-tagged form. The recombinant proteins were used to immunise mice, whose sera were used in a Western blot (Figure 141B) and for FACS analysis.

These experiments show that cp7374 is a surface-exposed and immunoaccessible protein, and that it is a useful immunogen. These properties are not evident from the sequence alone.

**Example 142**

The following *C.pneumoniae* protein (PID 4377377) was expressed <SEQ ID 283; cp7377>:

```
  1  MREETVSWSL EDIREIYHTP VFELIHKANA ILRSNFLHSE LQTCYLISIK
 51  TGGCVEDCAY CAQSSRYHTH VTPEPMMKIV DVVERAKRAV ELGATRVCLG
101  AAWRNAKDDR YFDRVLAMVK SITDLGAEVC CALGMLSEEQ AKKLYDAGLY
151  AYNHNLDSSP EFYETIITTR SYEDRLNTLD VVNKSGISTC CGGIVGMGES
201  EEDRIKLLHV LATRDHIPES VPVNLLWPID GTPLQDQPPI SFWEVLRTIA
251  TARVVFPRSM VRLAAGRAFL TVEQQTLCFL AGANSIFYGD KLLTVENNDI
301  DEDAEMIKLL GLIPRPSFGI ERGNPCYANN S*
```

The cp7377 nucleotide sequence <SEQ ID 284> is:

```
  1  ATGCGTGAAG AAACTGTATC CTGGTCATTA GAAGACATCC GCGAAATTTA
 51  TCACACTCCC GTATTTGAGC TGATTCACAA AGCCAATGCC ATATTGCGTA
101  GTAATTTCCT CCATTCAGAA CTGCAGACTT GCTATCTGAT TTCGATTAAA
151  ACTGGTGGAT GCGTTGAAGA TTGCGCCTAC TGTGCCCAAT CTTCCCGCTA
201  TCATACCCAC GTCACACCAG AACCTATGAT GAAAATTGTA GACGTTGTGG
251  AAAGGGCAAA ACGTGCTGTA GAGCTAGGCG CCACTCGTGT GTGTCTTGGG
301  GCTGCCTGGC GCAATGCTAA GGACGATCGA TACTTTGATA GAGTCCTCGC
351  TATGGTGAAA AGTATCACAG ATCTCGGAGC CGAGGTTTGT TGTGCTTTAG
401  GCATGCTCTC CGAAGAGCAA GCTAAAAAAC TGTATGATGC AGGACTTTAT
451  GCCTACAATC ATAATTTAGA CTCTTCTCCG GAATTCTATG AAACTATAAT
501  CACAACACGT TCTTATGAAG ATCGCCTCAA CACTCTTGAT GTAGTAAATA
551  AATCTGGCAT TAGTACATGC TGCGGTGGTA TTGTAGGTAT GGGAGAATCT
601  GAAGAAGACC GTATAAAGCT TCTTCATGTT CTTGCAACAA GAGATCATAT
651  CCCAGAATCC GTACCTGTAA ATTTACTTTG GCCGATTGAC GGCACGCCTT
701  TGCAAGACCA GCCTCCGATT TCTTTCTGGG AAGTCTTGCG AACCATAGCA
751  ACGGCACGGG TTGTTTTCCC CAGATCCATG GTACGACTTG CTGCAGGACG
801  CGCTTTCCTC ACAGTAGAAC AACAAACCTT ATGTTTTCTA GCCGGTGCCA
851  ACTCCATATT CTATGGAGAT AAACTGTTGA CTGTAGAAAA CAATGATATA
901  GATGAAGATG CTGAAATGAT CAAACTTTTA GGCTTAATCC CTCGCCCTTC
951  ATTTGGAATA GAAAGAGGTA ACCCATGTTA TGCCAACAAT TCCTAA
```

The PSORT algorithm predicts cytoplasm (0.2926).

The protein was expressed in *E.coli* and purified as a GST-fusion product (Figure 142A) and also in his-tagged form. The recombinant proteins were used to immunise mice, whose sera were used in a Western blot (Figure 142B) and for FACS analysis.

These experiments show that cp7377 is a surface-exposed and immunoaccessible protein, and that it is a useful immunogen. These properties are not evident from the sequence alone.

**Example 143**

The following *C.pneumoniae* protein (PID 4377407) was expressed <SEQ ID 285; cp7407>:

```
  1  MVCPNNSWFR MCGNFNCEWV EVTTTEETTR QSASDISEEA GSSGGAAPIT
 51  TQPTKITKVE KRVQFNTAQG DESTIHMIQE AGELVDSILS HRRTQGCTEY
101  CYDSYATGCG QRCGSFGRLI CGTYKACCLD REDNQVAGLV HECEQTHGPI
151  AVALAAKTMG LNLMELVEKN TILSEEQKNE FRQHCSEAKT QLYGTMQSLS
201  QNFFLEGVNS IRERGLDDSL VQAVLSFIAT RSWEKTIESE EASGTSSASN
251  STRIPACYIL NTSPLTTSRL SCGSRDARRP SSVGAEPQYV AKKYNDNGMA
301  RQLGKIQVTN LKTGDFSALG PFGLLIVKML NSFLLSASQS TSSILKHTGG
351  EICYTCPNFR DIVVLLMLAI GYCPANTDET SVVDIHMIDD PIMTIFYRLQ
401  YSYRTGKTSA SFLKKKPSLV RQESLDCPTP AESVPLMSSL EEEDENEDDD
451  EDGNLAYQQR ILECSGHLQT LFLGIKINKE *
```

The cp7407 nucleotide sequence <SEQ ID 286> is:

```
  1  ATGGTTTGCC CAAATAATTC TTGGTTCAGA ATGTGTGGAA ATTTCAACTG
 51  CGAATGGGTT GAAGTAACAA CAACAGAAGA AACAACGCGG CAATCGGCTT
101  CAGATATAAG CGAAGAAGCT GGTTCGAGTG GAGGAGCTGC TCCTATAACT
151  ACGCAACCTA CTAAAATTAC AAAAGTAGAG AAACGTGTCC AATTTAATAC
```

```
 201  TGCTCAAGGT GATGAAAGTA CAATACACAT GATCCAAGAA GCAGGAGAAT
 251  TGGTAGACTC CATTCTATCA CATAGACGAA CGCAAGGATG TACAGAGTAT
 301  TGTTATGACA GTTACGCAAC TGGATGTGGT CAGCGTTGCG GATCTTTTGG
 351  AAGACTCATT TGTGGAACGT ATAAAGCGTG TTGCTTAGAC AGAGAGGATA
 401  ATCAGGTTGC TGGACTTGTC CATGAATGCG AACAGACCCA TGGTCCTATT
 451  GCCGTTGCTT TAGCTGCTAA AACTATGGGC CTCAACTTAA TGGAACTTGT
 501  AGAAAAAAAC ACTATTTTGT CTGAAGAACA GAAAAATGAA TTTAGACAGC
 551  ATTGCTCGGA AGCTAAAACC CAACTCTATG GAACGATGCA GAGCCTTTCT
 601  CAAAACTTTT TCCTTGAAGG AGTCAACAGC ATTAGAGAAC GCGGTCTAGA
 651  CGATTCACTA GTCCAAGCCG TGCTAAGCTT TATTGCTACA AGGTCTTGGG
 701  AAAAAACTAT AGAATCAGAG GAAGCCTCAG GAACATCTTC TGCTTCTAAT
 751  TCTACACGCA TTCCTGCGTG CTATATCTTA AATACGAGCC CCTTAACGAC
 801  GTCACGCCTA TCCTGTGGAT CAAGAGATGC GCGACGCCCA TCTTCAGTCG
 851  GTGCAGAGCC CCAGTACGTA GCAAAAAAAT ACAATGACAA TGGCATGGCC
 901  AGACAATTAG GAAAAATCCA AGTCACCAAT CTAAAAACAG GAGATTTTTC
 951  AGCTTTAGGT CCTTTTGGTC TCCTGATTGT GAAAATGCTG AATAGCTTTC
1001  TCTTATCTGC ATCACAAAGC ACATCTTCTA TTCTAAAGCA CACAGGTGGA
1051  GAAATATGTT ATACGTGCCC AAATTTTCGT GATATCGTCG TTTTATTGAT
1101  GTTAGCGATT GGCTATTGCC CTGCAAATAC CGATGAGACA TCTGTCGTAG
1151  ATATACACAT GATAGATGAT CCGATTATGA CCATCTTCTA TCGACTACAA
1201  TACAGCTATA GAACAGGGAA AACTTCAGCA TCGTTTTTAA AAAAGAAACC
1251  CTCATTAGTA AGACAGGAAA GTCTTGATTG TCCTACCCCT GCAGAATCTG
1301  TCCCTCTCAT GTCAAGTCTC GAAGAAGAAG ATGAAAATGA AGATGATGAT
1351  GAGGATGGGA ATTTGGCGTA TCAACAGCGT ATCCTTGAAT GCTCGGGTCA
1401  TTTACAAACT CTATTTTTAG GGATAAAAAT AAACAAAGAA TAA
```

The PSORT algorithm predicts inner membrane (0.1319).

The protein was expressed in *E.coli* and purified as a GST-fusion product (Figure 143A). The recombinant protein was used to immunise mice, whose sera were used in a Western blot (Figure 143B) and for FACS analysis.

These experiments show that cp7407 is a surface-exposed and immunoaccessible protein, and that it is a useful immunogen. These properties are not evident from the sequence alone:

### Example 144

The following *C.pneumoniae* protein (PID 4376432) was expressed <SEQ ID 287; cp6432>:

```
  1  MTRSTIESSD SLCSRSFSQK LSVQTLKNLC ESRLMKITSL VIAFLTLIVG
 51  GALIALAGGG VLSFPLGLIL GSVLVLFSSI YLVSCCKFFT LKEMTMTCSV
101  KSKINIWFEK QRNKDIEKAL ENPDLFGENK RNVGNRSARN QLEMILHETD
151  GIILKRYMKG AKMYFYL*
```

The cp6432 nucleotide sequence <SEQ ID 288> is:

```
  1  ATGACTAGAA GTACTATTGA AAGCAGTGAT TCGCTATGCT CAAGGTCTTT
 51  TTCTCAAAAA TTAAGTGTCC AGACATTAAA AAATCTCTGT GAAAGTAGAT
101  TAATGAAGAT CACTTCTCTT GTGATTGCTT TCCTAACTCT AATTGTGGGG
151  GGTGCTCTTA TAGCTTTAGC AGGAGGGGGG GTTCTTTCTT TCCCTCTTGG
201  GCTAATCTTA GGAAGCGTAC TCGTTTTGTT TTCTTCTATC TATTTAGTCT
251  CTTGTTGTAA ATTTTTTACT TTAAAAGAGA TGACAATGAC CTGTAGTGTC
301  AAATCTAAAA TCAATATATG GTTTGAAAAG CAACGAAACA AAGACATCGA
351  AAAGGCATTA GAGAATCCAG ATCTCTTTGG AGAAAATAAG AGAAATGTTG
401  GAAATCGTTC GGCAAGAAAT CAACTAGAAA TGATCTTACA CGAGACTGAC
451  GGAATTATTT TGAAAAGATA TATGAAAGGA GCTAAAATGT ACTTTTATTT
501  ATGA
```

The PSORT algorithm predicts inner membrane (0.5394).

The protein was expressed in *E.coli* and purified as a his-tagged product (Figure 144A). The recombinant protein was used to immunise mice, whose sera were used in a Western blot (Figure 144B) and for FACS analysis.

These experiments show that cp6432 is a surface-exposed and immunoaccessible protein, and that it is a useful immunogen. These properties are not evident from the sequence alone.

**Example 145**

The following *C.pneumoniae* protein (PID 4376433) was expressed <SEQ ID 289; cp6433>:

```
  1  MNWVPKTIDH VDPESEIDIR KVVSCYKLIK ECQPEFRSLI SELLGVIRCG
 51  LRLLKRSKYQ EQARTVSDED APLFCLTRSY YQDGYLTPLR AGPRDLINHY
101  IHLRRRENPK HFFSPKHPCY YARLAFNESV CVYRELFDIE RLTKMYVEGD
151  YSKEQEKNLQ AILSFVKTLD EGKDFLIEHK DTDLIGRGFT DVFCT*
```

The cp6433 nucleotide sequence <SEQ ID 290> is:

```
  1  ATGAATTGGG TTCCAAAAAC AATAGACCAT GTAGATCCAG AATCAGAGAT
 51  AGATATACGT AAAGTCGTCT CCTGCTATAA GTTGATAAAA GAATGTCAAC
101  CTGAATTTCG ATCTCTTATA AGTGAATTAC TAGGAGTGAT TCGGTGTGGC
151  TTAAGACTAT TAAAACGTTC TAAGTATCAA GAACAGGCTA GAACTGTATC
201  TGATGAAGAT GCACCTCTTT TCTGCCTGAC TCGTTCTTAT TATCAAGATG
251  GTTATCTCAC GCCATTAAGA GCAGGACCTC GTGATCTTAT AAATCACTAT
301  ATACACTTGC GTCGCCGAGA GAATCCTAAG CATTTTTTCA GTCCTAAGCA
351  TCCATGTTAT TATGCTCGAT TGGCTTTTAA TGAGTCAGTG TGTGTCTATA
401  GAGAACTCTT TGATATAGAG CGACTTACAA AAATGTATGT CGAGGGTGAT
451  TATTCTAAAG AACAAGAGAA AAACCTACAG GCTATTCTTA GTTTTGTGAA
501  AACTCTAGAT GAAGGAAAGG ACTTTCTTAT TGAACATAAA GATACCGATC
551  TCATTGGGAG AGGTTTTACT GATGTGTTCT GCACTTAA
```

The PSORT algorithm predicts cytoplasm (0.4068).

The protein was expressed in *E.coli* and purified as a his-tagged product (Figure 145A). The recombinant protein was used to immunise mice, whose sera were used in a Western blot (Figure 145B) and for FACS analysis.

These experiments show that cp6433 is a surface-exposed and immunoaccessible protein, and that it is a useful immunogen. These properties are not evident from the sequence alone.

**Example 146**

The following *C.pneumoniae* protein (PID 4376643) was expressed <SEQ ID 291; cp6643>:

```
  1  MGYLPVSATD VLFESPAAPL INSANTQNQK LIELKGKQQA ESSPRTITSV
 51  ILEVLLVIGC CLIVLSLLAI RPALQFTLET GHPAAIAVLA VSGTILLVAV
101  IILFCFLAAV PFAAKKTYKY VKTVDDYASW HSHQQTPTLG TIFSGIVYAE
151  SQAQL*
```

The cp6643 nucleotide sequence <SEQ ID 292> is:

```
  1  ATGGGATATC TTCCAGTATC TGCTACGGAC GTTCTTTTTG AAAGTCCAGC
 51  CGCTCCCTTA ATCAATAGCG CAAACACACA AAATCAGAAA CTCATAGAAC
101  TCAAGGGGAA GCAGCAAGCT GAGTCTTCTC CACGGACAAT CACTTCTGTC
151  ATATTGGAAG TTCTCCTAGT GATCGGATGC TGCCTCATAG TTCTTAGTTT
201  ATTGGCAATC CGCCCTGCTC TGCAATTCAC TCTAGAAACT GGACATCCAG
251  CTGCCATTGC AGTCCTTGCT GTCTCAGGAA CAATTCTATT GGTGGCTGTT
301  ATCATCTTGT TTTGCTTTCT AGCAGCTGTG CCATTCGCTG CTAAGAAAAC
351  TTATAAATAT GTTAAGACGG TTGATGACTA TGCTTCTTGG CATTCTCATC
401  AGCAAACACC GACCCTAGGC ACTATCTTTT CAGGTATCGT CTATGCAGAA
451  TCCCAGGCGC AATTATAG
```

The PSORT algorithm predicts inner membrane (0.6859).

The protein was expressed in *E.coli* and purified as a his-tagged product (Figure 146A). The recombinant protein was used to immunise mice, whose sera were used in a Western blot (Figure 146B) and for FACS analysis.

These experiments show that cp6643 is a surface-exposed and immunoaccessible protein, and that it is a useful immunogen. These properties are not evident from the sequence alone.

**Example 147**

The following *C.pneumoniae* protein (PID 4376722) was expressed <SEQ ID 293; cp6722>:

```
  1  VSSTLNGVFP SSLPEESADL FITNKEIVAL GEKGNVFLTH SIPMHIAAIT
 51  ILVIVALAGI AIICLGCYSQ SILLIAVGIV LTILTLLCLQ ALVGFIKFIR
101  QLPQQLHTTV QFIREKIRPE SSLQLVTNAQ RKTTQDTLKL YEELCDLSQK
151  EFKLQSTLYQ KRFELSHKNE KTNQN*
```

The cp6722 nucleotide sequence <SEQ ID 294> is:

```
  1  GTGTCTAGTA CTTTAAACGG GGTATTTCCC TCATCCCTTC CGGAAGAGTC
 51  TGCTGATTTA TTCATTACGA ATAAGGAGAT CGTAGCTTTG GGGGAGAAGG
101  GCAATGTTTT TCTCACCCAC TCCATTCCTA TGCATATTGC TGCGATTACG
151  ATCTTAGTGA TTGTAGCTCT TGCTGGAATC GCTATTATCT GTTTGGGTTG
201  CTATAGCCAA AGCATTCTGT TGATTGCCGT TGGCATTGTT CTTACTATTT
251  TGACTCTTCT CTGCCTACAA GCCTTGGTAG GATTTATTAA ATTCATCCGG
301  CAGCTCCCTC AGCAGCTCCA TACGACAGTA CAATTTATCA GGGAGAAGAT
351  TCGACCTGAA TCCTCTCTAC AGCTTGTAAC CAATGCACAG AGAAAAACCA
401  CTCAAGATAC GCTAAAGTTA TACGAAGAAC TCTGCGACCT CTCACAAAAA
451  GAGTTCAAAC TGCAATCAAC TCTTTATCAA AAACGTTTTG AGCTTTCTCA
501  CAAGAATGAA AAGACAAATC AAAACTAG
```

The PSORT algorithm predicts inner membrane (0.6668).

The protein was expressed in *E.coli* and purified as a his-tagged product (Figure 147A). The recombinant protein was used to immunise mice, whose sera were used in a Western blot (Figure 147B) and for FACS analysis.

These experiments show that cp6722 is a surface-exposed and immunoaccessible protein, and that it is a useful immunogen. These properties are not evident from the sequence alone.

**Example 148**

The following *C.pneumoniae* protein (PID 4377253) was expressed <SEQ ID 295; cp7253>:

```
  1  MSELAPCSTG LQMVPHTQVH HALDTRRVIL TIAACLSLIA GIVLVGLGAA
 51  AILPSLFGVI GGMILILFSS IALIYLYKKT REVDQIALEP LPEMISKDQS
101  IIDFVKTRDY ASLEKKATFA YTHTHYYDGS MVFYREIPRF MLGSYLALRK
151  DMDRQALF*
```

The cp7253 nucleotide sequence <SEQ ID 296> is:

```
  1  ATGAGCGAGC TCGCCCCCTG CTCGACAGGA TTGCAGATGG TCCCCCATAC
 51  GCAGGTCCAT CATGCCCTTG ATACGCGGAG AGTCATTCTA ACGATAGCCG
101  CCTGTCTGTC TTTAATTGCA GGAATCGTGT TGGTTGGCTT AGGTGCTGCA
151  GCAATCCTGC CCTCGCTTTT TGGAGTCATT GGAGGAATGA TTCTTATTCT
201  GTTTTCTTCG ATCGCCCTCA TTTATTTATA CAAGAAGACA AGGGAGGTGG
251  ATCAGATTGC TCTGGAGCCT CTTCCTGAGA TGATTTCTAA AGATCAAAGC
301  ATTATAGATT TTGTAAAGAC ACGAGACTAT GCATCTTTAG AAAAGAAAGC
351  GACCTTTGCT TATACTCATA CTCATTATTA CGATGGAAGC ATGGTCTTCT
401  ATAGGGAGAT CCCTAGATTT ATGTTAGGCT CTTATCTCGC GCTTCGCAAA
451  GACATGGACC GCCAAGCTCT TTTTTGA
```

The PSORT algorithm predicts inner membrane (0.5394).

The protein was expressed in *E.coli* and purified as a his-tagged product (Figure 148A). The recombinant protein was used to immunise mice, whose sera were used in a Western blot (Figure 148B) and for FACS analysis.
These experiments show that cp7253 is a surface-exposed and immunoaccessible protein, and that it is a useful immunogen. These properties are not evident from the sequence alone.

**Example 149**

The following *C.pneumoniae* protein (PID 4376264) was expressed <SEQ ID 297; cp6264>:

```
  1  VISGLLFLLV RREVPTVRSE EIPRGVSVTP SEEPALEKAQ KEPETKKILD
 51  RLPKELDQLD TYIQEVFACL ERLKDPKYED RGLLTEAKEK LRVFDVVEKD
101  MMSEFLDIQR VLNEEAYYVE HCQDPLENIA YEIFSSQELR DYYCAGVCGY
151  LPSGDARADR LKRSVKEVMD RFMRVTWKSW EASVMLDHSY GVARELFKKA
201  VGVLEESVYK ILFKSYRDAF YECEKAKIQR DGRFKWL*
```

The cp6264 nucleotide sequence <SEQ ID 298> is:

```
  1  GTGATTTCGG GACTTCTATT CCTTCTAGTA AGACGAGAGG TTCCGACAGT
 51  ACGTTCAGAG GAAATTCCCA GAGGGGTTTC TGTGACCCCT TCTGAAGAGC
101  CTGCTCTAGA GAAGGCTCAA AAAGAACCGG AGACAAAGAA AATTTTAGAT
151  CGGTTGCCGA AGGAATTGGA TCAGTTAGAT ACGTATATTC AGGAAGTGTT
201  TGCATGTTTA GAGAGGCTGA AGGATCCTAA GTACGAAGAT CGAGGTCTTT
251  TAACAGAGGC GAAGGAGAAA CTTCGAGTTT TTGACGTTGT TGAGAAAGAT
301  ATGATGTCAG AGTTTTTAGA CATACAACGA GTGTTGAATG AGGAAGCATA
351  TTATGTAGAA CATTGTCAAG ATCCCCTAGA GAATATAGCC TACGAGATTT
401  TCTCTTCCCA AGAGCTTCGT GATTACTACT GTGCAGGGGT GTGTGGGTAT
451  TTGCCTTCTG GGGATGCTCG AGCGGATCGA TTAAAGAGAT CAGTTAAGGA
501  GGTAATGGAT CGCTTTATGA GGGTGACCTG GAAATCTTGG GAGGCATCAG
551  TCATGTTGGA TCATAGCTAT GGGGTAGCGC GAGAGTTATT CAAGAAGGCA
601  GTAGGAGTAC TAGAGGAGAG TGTCTATAAA ATTCTGTTTA AGAGCTATAG
651  AGATGCGTTT TATGAATGTG AGAAGGCAAA GATCCAGAGG GATGGGCGTT
701  TCAAATGGTT ATAG
```

The PSORT algorithm predicts cytoplasm (0.2817).
The protein was expressed in *E.coli* and purified as a his-tagged product (Figure 149A). The recombinant protein was used to immunise mice, whose sera were used in a Western blot (Figure 149B) and for FACS analysis.
These experiments show that cp6264 is a surface-exposed and immunoaccessible protein, and that it is a useful immunogen. These properties are not evident from the sequence alone.

**Example 150**

The following *C.pneumoniae* protein (PID 4376266) was expressed <SEQ ID 299; cp6266>:

```
  1  MLLLISGALF LTLGIPGLSA AISFGLGIGL SALGGVLMIS GLLCLLVKRE
 51  IPTVRPEEIP EGVSLAPSEE PALQAAQKTL AQLPKELDQL DTDIQEVFAC
101  LRKLKDSKYE SRSFLNDAKK ELRVFDFVVE DTLSEIFELR QIVAQEGWDL
151  NFLINGGRSL MMTAESESLD LFHVSKRLGY LPSGDVRGEG LKKSAKEIVA
201  RLMSLHCEIH KVAVAFDRNS YAMAEKAFAK ALGALEESVY RSLTQSYRDK
251  FLESERAKIP WNGHITWLRD DAKSGCAEKK LGMPRNVGRN LGKQSFG*
```

The cp6266 nucleotide sequence <SEQ ID 300> is:

```
  1  ATGCTCTTAC TGATTTCAGG AGCTCTCTTT CTGACGTTAG GGATTCCAGG
 51  ATTGAGTGCA GCAATTTCTT TTGGATTAGG CATCGGTCTC TCCGCATTAG
101  GAGGAGTGCT GATGATTTCG GGACTACTAT GTCTTTTAGT AAAACGAGAG
151  ATTCCGACAG TACGACCAGA AGAAATTCCT GAAGGGGTTT CGCTGGCTCC
```

```
201  TTCTGAGGAG CCAGCTCTAC AGGCAGCTCA GAAGACTTTA GCTCAGCTGC
251  CTAAGGAATT GGATCAGTTA GATACAGATA TTCAGGAAGT GTTCGCATGT
301  TTAAGAAAGC TGAAAGATTC TAAGTATGAA AGTCGAAGTT TTTTAAACGA
351  TGCTAAGAAG GAGCTTCGAG TTTTTGACTT TGTGGTTGAG GATACCCTCT
401  CGGAGATTTT CGAGTTGCGG CAGATTGTGG CTCAAGAGGG ATGGGATTTA
451  AACTTTTTGA TCAATGGGGG ACGAAGCCTC ATGATGACTG CAGAATCTGA
501  ATCGCTTGAT TTGTTTCATG TATCGAAGCG GCTAGGGTAT TTACCTTCTG
551  GGGATGTTCG AGGGGAGGGG TTAAAGAAAT CTGCGAAGGA GATAGTCGCT
601  CGTTTGATGA GCTTGCATTG CGAGATTCAC AAGGTGGCGG TAGCGTTTGA
651  TAGGAATTCC TATGCGATGG CAGAAAAGGC GTTTGCGAAA GCGTTGGGAG
701  CTTTAGAAGA GAGTGTGTAT CGGAGTCTGA CGCAGAGTTA TAGAGATAAA
751  TTTTTGGAGA GCGAGAGGGC GAAGATCCCA TGGAATGGGC ATATAACCTG
801  GTTAAGAGAT GATGCGAAGA GTGGGTGTGC TGAAAAGAAG CTCGGGATGC
851  CGAGGAACGT TGGAAGAAAT TTAGGAAAGC AGTCTTTTGG GTAG
```

The PSORT algorithm predicts inner membrane (0.3590).

The protein was expressed in *E.coli* and purified as a his-tag product (Figure 150A). The recombinant protein was used to immunise mice, whose sera were used in a Western blot (Figure 150) and for FACS analysis.

These experiments show that cp6266 is a surface-exposed and immunoaccessible protein and that they it is a useful immunogen. These properties are not evident from the sequence alone.

**Example 151**

The following *C.pneumoniae* protein (PID 4376895) was expressed <SEQ ID 301; cp6895>:

```
  1  MKIKKSFQYS LCQAKRFQNM LPNHFDPCLQ PVNLQLKQDR LAYGELIILL
 51  SKYQQKTFSS LLKEETCSLN RAKQHLLYKI LRDFNTMQHL RSLGLNGWGE
101  IPMSPCL*
```

The cp6895 nucleotide sequence <SEQ ID 302> is:

```
  1  ATGAAGATTA AAAAATCTTT TCAATACAGT TTATGCCAAG CAAAGAGATT
 51  TCAGAACATG CTGCCAAACC ACTTTGATCC ATGTTTGCAG CCAGTGAATT
101  TACAACTCAA ACAAGACAGA TTGGCATACG GGGAGCTCAT CATATTGCTA
151  TCTAAATATC AACAAAAGAC CTTTTCCTCT TTGTTGAAGG AAGAAACATG
201  TTCTCTTAAT CGTGCGAAGC AGCACTTATT GTATAAGATT TTGAGAGATT
251  TTAATACTAT GCAGCATCTA AGGTCCCTCG GATTAAATGG TTGGGGAGAG
301  ATCCCTATGA GTCCTTGCCT CTAA
```

The PSORT algorithm predicts cytoplasm (0.3264).

The protein was expressed in *E.coli* and purified as a his-tag product (Figure 151A). The recombinant protein was used to immunise mice, whose sera were used in a Western blot (Figure 151B) and for FACS analysis.

These experiments show that cp6895 is a surface-exposed and immunoaccessible protein and that it is a useful immunogen. These properties are not evident from the sequence alone.

**Example 152 and Example 153**

The following *C.pneumoniae* protein (PID 4376282) was expressed <SEQ ID 303; cp6282>:

```
  1 MSLLNLPSSQ DSASEDSTSQ SQIFDPIRNR ELVSTPEEKV RQRLLSFLMH
 51 KLNYPKKLII IEKELKTLFP LLMRKGTLIP KRRPDILIIT PPTYTDAQGN
101 THNLGDPKPL LLIECKALAV NQNALKQLLS YNYSIGATCI AMAGKHSQVS
151 ALFNPKTQTL DFYPGLPEYS QLLNYFISLN L*
```

The cp6282 nucleotide sequence <SEQ ID 304> is:

```
  1 ATGTCCTTAT TGAACCTTCC CTCAAGCCAG GATTCTGCAT CTGAGGACTC
 51 CACATCGCAA TCTCAAATCT TCGATCCCAT TAGAAATCGG GAGTTAGTTT
101 CTACTCCCGA AGAAAAAGTC CGCCAAAGGT TGCTCTCCTT CCTAATGCAT
151 AAGCTGAACT ACCCTAAGAA ACTCATCATC ATAGAAAAAG AACTCAAAAC
201 TCTTTTTCCT CTGCTTATGC GTAAAGGAAC CCTAATCCCA AAACGCCGCC
251 CAGATATTCT CATCATCACT CCCCCCACAT ACACAGACGC ACAGGGAAAC
301 ACTCACAACC TAGGCGACCC AAAACCCCTG CTACTTATCG AATGTAAGGC
351 CTTAGCCGTA AACCAAAATG CACTCAAACA ACTCCTTAGC TATAACTACT
401 CTATCGGAGC CACCTGCATT GCTATGGCAG GGAAACACTC TCAAGTGTCA
451 GCTCTCTTCA ATCCAAAAAC ACAAACTCTT GATTTTTATC CTGGCCTCCC
501 AGAGTATTCC CAACTCCTAA ACTACTTTAT TTCTTTAAAC TTATAG
```

The PSORT algorithm predicts cytoplasm (0.362).

The following *C.pneumoniae* protein (PID 4377373) was also expressed <SEQ ID 305; cp7373>:

```
  1 MSTTTVKHFI HTASRWEPVL KEIVASNYWH AQWINTLSFL ENSGAKKISA
 51 SEHPTEVKEE VLKHAAEEFR HGHYLKTQIS RISETSLPDY TSKNLLGGLL
101 TKYYLHLLDL RTCRVLENEY SLSGQTLKTA AYILVTYAIE LRASELYPLY
151 HDILKEAQSK ITVKSIILEE QGHLQEMERE LKDLPHGEEL LGYACQFEGE
201 LCLQFVERLE QMIFDPSSTF TKF*
```

The cp7373 nucleotide sequence <SEQ ID 306> is:

```
  1 ATGTCTACAA CCACAGTAAA ACACTTTATC CACACAGCCT CTCGTTGGGA
 51 GCCCGTTCTC AAAGAGATCG TAGCTTCCAA CTATTGGCAT GCACAATGGA
101 TAAATACCCT GTCCTTTTTA GAAAATAGTG GAGCAAAAAA AATCTCCGCA
151 AGTGAACATC CTACGGAGGT AAAGGAAGAA GTTTTAAAAC ATGCTGCTGA
201 AGAATTTCGT CATGGTCACT ATCTAAAAAC TCAGATTTCT AGAATCTCAG
251 AGACTTCTCT CCCTGACTAT ACATCTAAAA ATCTTCTGGG AGGCTTACTT
301 ACAAAATATT ACCTCCATCT TCTAGATTTA AGGACGTGCC GAGTACTGGA
351 AAATGAATAC TCCCTATCGG GACAAACGTT AAAAACTGCA GCGTATATTT
401 TAGTTACCTA CGCAATCGAA CTTCGTGCTT CTGAACTTTA TCCTCTGTAT
451 CACGATATTC TGAAAGAAGC TCAAAGTAAA ATAACGGTAA AATCCATTAT
501 CTTAGAAGAG CAAGGCCATC TGCAAGAGAT GGAACGTGAA CTTAAAGATC
551 TCCCCCACGG GGAGGAACTC TTAGGCTATG CTTGCCAATT CGAAGGGGAG
601 CTTTGCTTGC AGTTTGTAGA GAGATTAGAA CAAATGATCT TCGATCCTTC
651 CTCGACTTTT ACAAAGTTCT AG
```

The PSORT algorithm predicts cytoplasm (0.1069).

The proteins were expressed in *E.coli* and purified as his-tag products (Figure 152A; 6282 = lanes 8 & 9; 7373 = lanes 2-4). The recombinant proteins were used to immunise mice, whose sera were used in Western blots (Figures 152B & 153) and for FACS analysis.

These experiments show that cp6282 & cp7373 are surface-exposed and immunoaccessible proteins and that they are useful immunogens. These properties are not evident from the sequence alone.

Example 154 , Example 155 , Example 156 , **Example 157 and Example 158**

The following *C.pneumoniae* protein (PID 4376412) was expressed <SEQ ID 307; cp6412>:

```
  1  MSSSEVVFQT VHGLGFGGLS SKSVVPFKKS LSDAPRVVCS ILVLTLGLGA
 51  LVCGIAITCW CVPGVILMGG ICAIVLGAIS LALSLFWLWG LFSNCCGSKR
101  VLPGEGLLRD KLLDGGFSRA APSGMGLPGD GSPRASTPSC LEELQAEIQA
151  VTQAIDQMSD D*
```

The cp6412 nucleotide sequence <SEQ ID 308> is:

```
  1  ATGAGCAGTT CGGAAGTTGT TTTCCAGACA GTTCATGGCC TTGGCTTTGG
 51  TGGATTGTCT TCAAAAAGTG TTGTCCCTTT TAAGAAAAGT CTTTCGGATG
101  CGCCCCGTGT TGTGTGCTCG ATTTTAGTTT TGACTCTGGG GTTGGGAGCG
151  CTTGTTTGTG GTATTGCCAT TACTTGTTGG TGTGTCCCGG GAGTTATTTT
201  AATGGGGGGA ATTTGCGCTA TAGTTTTAGG TGCAATTTCT TTAGCTTTAA
251  GTCTATTTTG GTTGTGGGGT TTATTTTCTA ATTGTTGTGG TTCTAAGAGA
301  GTTTTACCGG GTGAGGGATT GCTACGGGAT AAGCTTTTAG ATGGTGGATT
351  TTCAAGAGCG GCACCTTCAG GAATGGGACT TCCGGGTGAT GGATCTCCAA
401  GAGCGTCAAC GCCATCTTGC CTAGAGGAAC TTCAAGCAGA GATACAGGCA
451  GTTACTCAAG CTATCGATCA GATGTCAGAT GATTGA
```

The PSORT algorithm predicts inner membrane (0.4864).

The following *C.pneumoniae* protein (PID 4376431) was also expressed <SEQ ID 309; cp6431>:

```
  1  LRAGGSLVTT YPKEGQRLRS PEQLRVLDDL VQSYPNHLHA IELDCGAIPQ
 51  DLIGATYIIT FADFSTYILS LRSYQANSPS DDTWGIWFGS IDDPVQAVIS
101  FLKDHGFALP STLAQDPLLC TNK*
```

The cp6431 nucleotide sequence <SEQ ID 310> is:

```
  1  TTGCGAGCAG GAGGTAGTCT TGTTACAACA TACCCTAAGG AAGGTCAGAG
 51  ATTGCGCTCC CCAGAACAGT TAAGAGTTCT GGATGATTTA GTGCAAAGCT
101  ATCCAAATCA CCTACATGCG ATTGAACTTG ATTGTGGTGC AATCCCTCAA
151  GATTTGATCG GAGCCACCTA TATCATCACG TTCGCCGATT TTTCCACCTA
201  TATTCTCTCT TTAAGAAGCT ACCAAGCCAA TTCTCCCTCC GATGATACAT
251  GGGGGATTTG GTTTGGATCT ATTGACGATC CTGTTCAAGC AGTCATATCA
301  TTTTTAAAAG ATCATGGATT TGCTCTTCCC TCGACCTTAG CTCAAGATCC
351  TTTGCTTTGT ACTAACAAGT AA
```

The PSORT algorithm predicts cytoplasm (0.2115).

The following *C.pneumoniae* protein (PID 4376443) was also expressed <SEQ ID 311; cp6443>:

```
  1  MIMTTISNSP SPALNPELSL IPPPTLVSSG TQTSLAYTIP AQGRRSTLRI
 51  ILDIFIIILG LATIISTFIV IFFLNGLNLL STPSIISSSC LIIVGLLFLI
101  MGLYFMISSL DQGLVGLLQK ELSQAEEREE EYIQEIEALR GAPRAESPTE
151  SPSTWL*
```

The cp6443 nucleotide sequence <SEQ ID 312> is:

```
  1 ATGATTATGA CTACTATATC TAACTCACCC TCCCCTGCAT TGAATCCCGA
 51 ACTTTCCCTT ATTCCTCCAC CAACACTTGT ATCTTCAGGT ACGCAAACAT
101 CTCTAGCTTA TACGATCCCC GCACAAGGAC GAAGATCCAC CCTACGTATT
151 ATATTAGATA TATTCATTAT CATTCTTGGT TTAGCTACGA TCATTTCTAC
201 CTTTATTGTT ATTTTCTTTT TAAATGGGCT GAACTTGCTC TCGACCCCAT
251 CTATTATCTC TTCGTCATGT TTAATCATTG TTGGATTGCT TTTTTTGATT
301 ATGGGGTTAT ATTTCATGAT CTCGAGTTTG GATCAGGGGC TTGTAGGCCT
351 TCTGCAAAAG GAACTCTCTC AAGCCGAAGA AAGAGAAGAA GAGTATATCC
401 AGGAAATCGA AGCTTTAAGA GGAGCTCCTA GAGCAGAATC TCCCACAGAG
451 TCTCCTAGTA CCTGGTTATG A
```

The PSORT algorithm predicts inner membrane (0.5585).

The following *C.pneumoniae* protein (PID 4376496) was also expressed <SEQ ID 313; cp6496>:

```
  1 MLIGRYSSDD QFTEATKNTP TIIKLGFVRD NLEGLTNPIS EIVSETSSSI
 51 KDSVLRSLPI LGSILGCARL YSTLSTNDPL DETQEKIWHT IFGALETLGL
101 GILILLFKII FVILHCIFHL VIGFCK*
```

The cp6496 nucleotide sequence <SEQ ID 314> is:

```
  1 ATGCTAATAG GCAGATACAG TAGTGATGAC CAATTCACTG AAGCAACAAA
 51 AAACACCCCA ACCATAATTA AGCTAGGTTT TGTTAGAGAT AATCTCGAGG
101 GATTAACGAA CCCTATCTCT GAAATCGTCT CGGAAACCTC CTCTTCTATT
151 AAAGATTCCG TTCTTCGCTC TCTTCCTATT TTAGGGTCCA TTTTAGGATG
201 CGCCCGACTT TACAGCACAC TCTCTACAAA TGATCCTCTT GACGAAACTC
251 AAGAAAAGAT TTGGCACACT ATATTTGGAG CCTTAGAAAC CTTAGGCTTA
301 GGGATTCTCA TCCTCTTATT TAAAATTATT TTTGTTATAT TACACTGCAT
351 ATTTCATCTA GTTATTGGGT TCTGCAAATA A
```

The PSORT algorithm predicts inner membrane (0.5989).

The following *C.pneumoniae* protein (PID 4376654) was also expressed <SEQ ID 315; cp6654>:

```
  1 MKTKMNSRKK AGQWAIFNSP TPGVSSTLVL AWTPWGYYDK DVQDILERKD
 51 PMSSSLSEKD SKEFLKNLFV DLLENGFTSV HIHAEEAFTP LDHTGKPHFK
101 RDNVYLPGKL LGALNEAAVQ ANVSADTQFT LFLTQDECNP FHDKKRG*
```

The cp6654 nucleotide sequence <SEQ ID 316> is:

```
  1 ATGAAAACTA AAATGAACTC TAGAAAAAAA GCAGGTCAAT GGGCAATTTT
 51 CAATTCTCCA ACTCCTGGTG TCAGTTCAAC TTTAGTTTTA GCATGGACTC
101 CTTGGGGTTA TTACGACAAG GATGTACAAG ATATCTTAGA AAGAAAAGAT
151 CCGATGAGCT CTTCGCTTTC TGAAAAAGAC TCAAAGGAGT TCTTGAAAAA
201 TCTGTTTGTA GATCTCTTAG AAAATGGCTT CACATCAGTA CATATTCACG
251 CAGAAGAAGC TTTCACTCCT CTTGATCATA CCGGGAAACC TCACTTTAAA
301 AGAGACAATG TGTACTTACC CGGAAAGTTG TTAGGCGCCT TGAATGAGGC
351 TGCGGTACAA GCCAATGTAA GTGCGGATAC TCAATTTACA TTGTTCCTTA
401 CTCAAGATGA GTGCAATCCT TTTCATGATA AGAAAAGAGG TTAA
```

The PSORT algorithm predicts cytoplasm (0.0730).

The proteins were expressed in *E.coli* and purified as his-tag products (Figure 154A; 6412 = lanes 2-3; 6431 = lanes 11-12; 6443 = lanes 5-6; 6496 = lanes 8-9; 6654 = lane 10; markers in lanes 1, 4, 7). The recombinant proteins were used to immunise mice, whose sera were used in Western blots (Figures 154B, 155, 156, 157 & 158) and for FACS analysis.

These experiments show that cp6412, cp6431, cp6443, cp6496 & cp6654 are surface-exposed and immunoaccessible proteins and that they are useful immunogens. These properties are not evident from their sequences alone.

**Example 159 and Example 160**

The following *C.pneumoniae* protein (PID 4376477) was expressed <SEQ ID 317; cp6477>:

```
  1  LLKFFLVCEE LCILTVATHR ALLETPLALS FFKELKTKYV YRAKDILQLH
 51  NYKGFTILNT SPLCS*
```

The cp6477 nucleotide sequence <SEQ ID 318> is:

```
  1  TTGCTAAAGT TCTTTCTAGT ATGTGAAGAG TTATGTATAC TTACTGTTGC
 51  TACACATAGA GCTCTCTTAG AAACTCCTTT AGCTCTATCA TTTTTTAAAG
101  AACTTAAGAC AAAATATGTC TACAGGGCGA AAGACATACT ACAACTACAT
151  AACTATAAAG GATTTACTAT CCTTAATACA TCACCGTTAT GTTCTTAA
```

The PSORT algorithm predicts inner membrane (0.128).

The following *C.pneumoniae* protein (PID 4376435) was also expressed <SEQ ID 319; cp6435>:

```
  1  LWSHFPRGFF MLPFCPTILL AKPFLNSENY GLERLAATVD SYFDLGQSQI
 51  VFLSKQDQGI TVEELSAKDR KFKPGSMNCT LYTEDPILPA HNSFSNCSDI
101  QMRTPISPIH *
```

The cp6435 nucleotide sequence <SEQ ID 320> is:

```
  1  TTGTGGTCGC ATTTCCCAAG AGGATTTTTT ATGCTCCCTT TTTGCCCTAC
 51  CATCCTTCTT GCTAAACCTT TTTTAAATAG CGAGAATTAC GGCTTAGAAC
101  GTTTAGCTGC AACCGTAGAT TCTTATTTTG ATCTGGGACA GTCTCAAATA
151  GTCTTCCTAA GCAAACAGGA TCAAGGAATC ACTGTGGAAG AATTGAGTGC
201  TAAAGATAGG AAATTCAAGC CAGGCTCTAT GAACTGTACA CTGTACACTG
251  AAGATCCTAT CTTACCTGCT CATAATTCCT TTAGTAATTG CTCTGATATT
301  CAAATGCGTA CTCCGATTAG CCCTATACAT TAA
```

The PSORT algorithm predicts periplasmic space (0.4044).

The proteins were expressed in *E.coli* and purified as his-tag products (Figure 159A; 6435 = lanes 2-4; 6477 = lanes 5-7). The recombinant proteins were used to immunise mice, whose sera were used in Western blots (Figures 159B & 160) and for FACS analysis.

These experiments show that cp6477 & cp6435 are surface-exposed and immunoaccessible proteins and that they are useful immunogens. These properties are not evident from the sequences alone.

**Example 161 and Example 162 and Example 163**

The following *C.pneumoniae* protein (PID 4376441) was expressed <SEQ ID 321; cp6441>:

```
  1  VEAGANVLVI DTAHAHSKGV FQTVLEIKSQ FPQISLVVGN LVTAEAAVSL
 51  AEIGVDAVKV GIGPGSICTT RIVSGVGYPQ ITAITNVAKA LKNSAVTVIA
101  DGRIRYSGDV VKALAAGADC VMLGSLLAGT DEAPGDIVSI DEKLFKRYRG
151  MGSLGAMKQG SADRYFQTQG QKKLVPGGVE GLVAYKGSVH DVLYQILGGI
201  RSGMGYVGAE TLKDLKTKAS FVRITESGRA ESHIHNIYKV QPTLNY
```

The cp6441 nucleotide sequence <SEQ ID 322> is:

```
  1 GTGGAAGCTG GAGCAAATGT TCTAGTCATT GACACAGCTC ATGCACACTC
 51 TAAAGGAGTA TTCCAAACAG TTTTAGAAAT AAAATCCCAG TTCCCACAAA
101 TTTCTTTAGT TGTAGGGAAT CTTGTTACAG CTGAAGCCGC AGTTTCCTTA
151 GCTGAGATTG GAGTTGACGC TGTAAAGGTA GGTATTGGCC CAGGATCTAT
201 CTGTACAACT AGAATCGTTT CAGGGGTCGG TTATCCACAA ATTACTGCCA
251 TTACAAACGT AGCAAAAGCT CTTAAAAACT CTGCCGTGAC TGTAATTGCT
301 GATGGGAGAA TCCGCTATTC TGGAGATGTG GTAAAAGCAT TAGCAGCAGG
351 AGCAGACTGT GTCATGCTAG GAAGTTTGCT TGCAGGGACT GATGAAGCTC
401 CTGGGGATAT CGTTTCTATC GATGAGAAGC TTTTTAAAAG GTACCGCGGC
451 ATGGGATCTT TAGGCGCTAT GAAACAAGGA AGTGCTGACC GGTATTTTCA
501 AACACAGGGA CAGAAAAAGC TGGTTCCTGG GGAGTTGAA GGACTAGTCG
551 CTTATAAAGG CTCTGTCCAC GATGTCCTCT ATCAAATTTT AGGAGGAATA
601 CGCTCAGGTA TGGGGTATGT TGGAGCTGAA ACTCTCAAAG ATTTAAAAAC
651 TAAGGCTTCC TTTGTTCGAA TTACTGAATC TGGAAGAGCT GAAAGTCATA
701 TTCATAATAT TTACAAAGTT CAACCAACCT TAAATTATTA A
```

The PSORT algorithm predicts bacterial inner membrane (0.132).

The following *C.pneumoniae* protein (PID 4376748) was also expressed <SEQ ID 323; cp6748>:

```
  1 LFSEGTALNL FRIFAPLRNR VTTEYSRARQ PDLHRIAIVY IGVLDSESSK
 51 ILERLISYMS CIYSESQMYL RFFMGKNVNQ SAVLSKLHVE NLHIRCGFFS
101 EDAVPESEPF DLSIYVHTDR SCPLPTKKRS SSWELQTVEL PESIYPQSEF
151 LLMRPRMLS*
```

The cp6748 nucleotide sequence <SEQ ID 324> is:

```
  1 TTGTTCTCTG AGGGGACAGC TCTAAATTTA TTTCGTATAT TTGCTCCACT
 51 ACGCAACCGT GTGACTACAG AATACAGTCG TGCTAGGCAA CCCGACCTAC
101 ATAGAATTGC CATCGTCTAT ATAGGAGTTC TCGATTCAGA AAGTTCCAAG
151 ATCCTAGAGC GGCTAATCTC TTATATGAGT TGTATCTATT CTGAATCGCA
201 AATGTATTTA AGATTCTTTA TGGGCAAGAA TGTAAATCAA AGTGCTGTAC
251 TCTCAAAATT ACATGTAGAA AATCTGCACA TCCGTTGTGG GTTTTTCAGC
301 GAGGATGCTG TTCCAGAGAG TGAGCCCTTC GATCTCTCCA TCTACGTGCA
351 CACAGATCGT AGCTGTCCTC TCCCTACGAA AAAACGGAGC AGCTCCTGGG
401 AACTCCAAAC TGTAGAACTC CCAGAGTCAA TATATCCACA GTCGGAATTC
451 CTATTGATGA GACCTCGAAT GCTTTCGTAG
```

The PSORT algorithm predicts cytoplasm (0.170).

The following *C.pneumoniae* protein (PID 4376881) was also expressed <SEQ TD 325; cp6881>:

```
  1 MRPHRKHVSS KSLALKQSAS THVEITTKAF RLSMPLKQLI LEKSDHLPPM
 51 ETIRVVLTSH KDKLGTEVHV VASHGKEILQ TKVHNANPYT AVINAFKKIR
101 TMANKHSNKR KDRTKHDLGL AAKEERIAIQ EEQEDRLSNE WLPVEGLDAW
151 DSLKTLGYVP ASAKKKISKK KMSIRMLSQD EAIRQLESAA ENFLIFLNEQ
201 EHKIQCIYKK HDGNYVLIEP SLKPGFCI*
```

The cp6881 nucleotide sequence <SEQ ID 326> is:

```
  1  ATGAGACCTC ATCGTAAACA CGTATCATCT AAAAGCTTAG CTTTAAAGCA
 51  ATCTGCATCA ACTCATGTAG AGATCACAAC AAAAGCCTTT CGTCTCTCTA
101  TGCCTCTAAA ACAGCTGATC CTAGAGAAAA GCGACCACCT CCCCCCTATG
151  GAAACAATCC GTGTGGTGCT AACCTCTCAT AAAGATAAGC TAGGCACCGA
201  GGTGCATGTT GTAGCTTCTC ATGGCAAAGA AATCCTTCAA ACTAAGGTTC
251  ATAACGCAAA CCCATACACT GCAGTGATCA ATGCTTTTAA GAAAATCCGC
301  ACCATGGCAA ATAAGCACTC CAATAAACGT AAAGACAGGA CAAAACATGA
351  TCTAGGTCTT GCAGCAAAAG AAGAACGTAT CGCAATACAG GAAGAACAAG
401  AAGATCGCCT TAGCAACGAG TGGCTTCCTG TCGAAGGCCT CGATGCCTGG
451  GATTCTCTAA AAACTCTTGG GTATGTTCCC GCATCAGCGA AAAAGAAGAT
501  CTCCAAGAAA AAGATGAGCA TTCGTATGCT ATCTCAAGAC GAGGCTATCC
551  GCCAGCTAGA GTCTGCCGCA GAAAACTTCC TGATCTTCTT GAACGAGCAA
601  GAGCATAAAA TCCAATGCAT TTATAAAAAA CATGACGGCA ACTATGTCCT
651  TATTGAACCT TCCCTCAAGC CAGGATTCTG CATCTGA
```

The PSORT algorithm predicts cytoplasm (0.249).

The proteins were expressed in *E.coli* and purified as his-tag products (Figure 161A; 6441= lanes 7-9; 6748 = lanes 2-3; 6881 = lanes 4-6). The recombinant protein was used to immunise mice, whose sera were used in Western blots (Figures 161B, 162 & 163) and for FACS analysis.

These experiments show that cp6441, cp6748 & cp6881 are surface-exposed and immunoaccessible proteins and that they are useful immunogens. These properties are not evident from the sequence alone.

**Example 164 and Example 165 Example 166**

The following *C.pneumoniae* protein (PID 4376444) was expressed <SEQ ID 327; cp6444>:

```
  1  MEQPNCVIQD TTTVLYALNS FDPRLSDDTH RLGKQSPLEA ENALGEFIEG
 51  LDTNSFPLEE VAIPILPGYH PKFYLSFIDR DDQGVHYEVL DGVFLKTVAA
101  CIIENSFLTD SMSPELLSEV KEALKR*
```

The cp6444 nucleotide sequence <SEQ ID 328> is:

```
  1  ATGGAGCAAC CCAATTGTGT GATTCAGGAT ACTACAACTG TTTTGTATGC
 51  CTTAAATAGC TTTGATCCTA GACTTAGTGA TGACACTCAC AGACTTGGGA
101  AGCAATCACC TCTTGAAGCA GAAAATGCTC TTGGAGAATT TATTGAAGGT
151  TTGGATACAA ATAGCTTTCC TTTAGAGGAA GTTGCCATTC CCATCCTGCC
201  AGGTTATCAC CCTAAGTTTT ATTTATCTTT CATAGATAGG GACGATCAAG
251  GTGTCCACTA TGAAGTTTTA GATGGCGTAT TTTTAAAGAC AGTCGCTGCT
301  TGTATTATAG AGAACTCCTT CTTAACTGAT TCTATGAGCC CGGAGCTTCT
351  CAGCGAAGTT AAGGAAGCTC TGAAACGATG A
```

The PSORT algorithm predicts cytoplasm (0.2031).

The following *C.pneumoniae* protein (PID 4376413) was also expressed <SEQ ID 329; cp6413>:

```
  1  MAVQSIKEAV TSAATSVGCV NCSREAIPAF NTEERATSIA RSVIAAIIAV
 51  VAISLLGLGL VVLAGCCPLG MAAGAITMLL GVALLAWAIL ITLRLLNIPK
101  AEIPSPGNNG EPNERNSATP PLEGGVAGEA GRGGGSPLTQ LDLNSGAGS*
```

The cp6413 nucleotide sequence <SEQ ID 330> is:

```
  1  ATGGCTGTTC AATCTATAAA AGAAGCCGTA ACATCAGCCG CAACATCAGT
```

```
 51  AGGATGTGTA AACTGTTCTA GAGAGGCTAT ACCAGCATTT AATACAGAGG
101  AGAGAGCAAC GAGTATTGCT AGATCTGTTA TAGCAGCTAT CATTGCTGTT
151  GTAGCTATCT CCTTACTCGG ACTAGGTCTT GTAGTTCTTG CTGGTTGCTG
201  TCCTTTAGGA ATGGCTGCGG GTGCTATAAC AATGCTGCTG GGTGTAGCAT
251  TATTAGCTTG GGCAATACTG ATTACTTTGA GACTGCTTAA TATACCTAAG
301  GCTGAAATAC CGAGTCCAGG GAACAACGGT GAGCCTAATG AAAGAAATTC
351  AGCAACTCCT CCTCTAGAGG GTGGTGTTGC AGGAGAAGCC GGTCGCGGCG
401  GGGGGTCACC TTTAACCCAA CTTGATCTCA ATTCAGGGGC GGGAAGTTAG
```

The PSORT algorithm predicts inner membrane (0.6180).

The following *C.pneumoniae* protein (PID 4377391) was also expressed <SEQ ID 331; cp7391>:

```
  1  MMLRVIELPL LPIKQALEKA FVQYNSYKAK LTKVEPCFRE SPAYITSEER
 51  LQSLDQTLER AYKEYQKRFQ EPSRLESEVS GCREHLREQV KQFETQGLDL
101  IKEELIFVSD VLFRKMVSCL VSTVHVPFME FYYEYFELHR LRLRAQWMAN
151  AEIYSKVRKA FPEMLKETLE KAKAPREEEY WLLCEERKSK EKRLILNKIE
201  AAQQRVKDLE PPPIKETGKQ KRKKEYSFFI RLKS*
```

The cp7391 nucleotide sequence <SEQ ID 332> is:

```
  1  ATGATGCTTC GTGTCATAGA GCTTCCACTA CTTCCTATAA AGCAAGCGTT
 51  GGAGAAGGCT TTTGTACAAT ATAATAGCTA CAAAGCGAAG TTAACCAAGG
101  TAGAACCTTG CTTTAGAGAG AGCCCTGCCT ATATAACTAG CGAAGAGCGA
151  CTCCAGAGTT TGGATCAGAC TTTAGAACGT GCGTACAAAG AGTACCAGAA
201  GAGATTCCAG GAGCCTTCAC GTTTGGAATC GGAAGTAAGT GGATGTAGAG
251  AGCATCTTAG AGAGCAGGTA AAACAATTTG AAACTCAAGG ACTAGACTTG
301  ATCAAAGAAG AGCTTATTTT TGTTAGTGAT GTGTTATTCC GAAAAATGGT
351  CAGTTGTCTA GTGTCGACAG TGCATGTTCC CTTTATGGAG TTTTATTATG
401  AGTATTTTGA GTTGCATAGA TTGAGGTTGC GGGCCCAATG GATGGCGAAT
451  GCCGAGATTT ATAGCAAAGT TAGAAAAGCA TTCCCAGAGA TGTTGAAGGA
501  GACCTTAGAA AAAGCTAAGG CTCCCAGAGA AGAAGAGTAT TGGTTACTTT
551  GCGAGGAGAG AAAGAGTAAG GAGAAGCGTT TGATTCTCAA CAAGATAGAG
601  GCAGCTCAGC AGCGGGTAAA AGATTTAGAA CCTCCTCCTA TTAAAGAGAC
651  AGGGAAACAG AAACGGAAGA AAGAATATTC GTTTTTCATT CGATTAAAAT
701  CGTGA
```

The PSORT algorithm predicts inner membrane (0.1489).

The proteins were expressed in *E.coli* and purified as his-tag and GST-fusion products (Figure 164A; 6444=lanes 11-12; 7391=lanes 2-3; 6413=lanes 4-6). The recombinant protein was used to immunise mice, whose sera were used in Western blots (Figures 164B, 165 & 166) and for FACS analysis.

These experiments show that cp6444, cp6413 & cp7391 are surface-exposed and immunoaccessible proteins and that they are useful immunogens. These properties are not evident from the sequence alone.

**Example 167 , Example 168 , Example 169 and Example 170**

The following *C.pneumoniae* protein (PID 4376463) was expressed <SEQ ID 333; cp6463>:

```
  1  MKKKVTIDEA LKEILRLEGA ATQEELCAKL LAQGFATTQS SVSRWLRKIQ
 51  AVKVAGERGA RYSLPSSTEK TTTRHLVLSI RHNASLIVIR TVPGSASWIA
101  ALLDQGLKDE ILGTLAGDDT IFVTPIDEGR LPLLMVSIAN LLQVFLD*
```

The cp6463 nucleotide sequence <SEQ ID 334> is:

```
  1  ATGAAAAAAA AAGTAACTAT AGATGAGGCT TTAAAAGAAA TTTTACGTCT
 51  TGAAGGAGCG GCAACTCAGG AGGAATTATG TGCAAAACTC TTAGCTCAAG
101  GTTTTGCTAC AACCCAGTCG TCTGTATCTC GTTGGCTACG AAAGATTCAG
151  GCTGTAAAGG TTGCTGGAGA GCGTGGTGCT CGTTATTCTT TACCCTCTTC
201  AACAGAGAAG ACCACGACCC GTCATTTGGT GCTCTCTATT CGCCATAACG
251  CCTCTCTTAT TGTAATTCGT ACGGTTCCTG GTTCAGCTTC TTGGATCGCT
301  GCTTTGTTAG ATCAAGGGCT CAAAGATGAA ATTCTTGGAA CTTTGGCAGG
351  AGATGACACG ATTTTTGTCA CTCCTATAGA TGAAGGGAGG CTCCCATTGT
401  TGATGGTTTC GATTGCAAAT TTACTGCAAG TTTTCTTGGA TTAA
```

The PSORT algorithm predicts inner membrane (0.1510).

The following *C.pneumoniae* protein (PID 4376540) was also expressed <SEQ ID 335; cp6540>:

```
  1  MSQCQSSSTS TWEWMKSFVP NWKNPTPPLS PIPSEDEFIL AYEPFVLPKT
 51  DPENAQANPP GTSTPNVENG IDDLNPLLGQ PNEQNNANNP GTSGSNPTSL
101  PAPERLPETE ENSQEEEQGS QNNEDLIG*
```

The cp6540 nucleotide sequence <SEQ ID 336> is:

```
  1  ATGTCTCAAT GTCAGAGTAG CAGTACATCT ACCTGGGAAT GGATGAAATC
 51  TTTTGTGCCA AACTGGAAGA ATCCAACTCC CCCCTTATCT CCTATACCTT
101  CTGAGGACGA ATTTATATTA GCATACGAGC CATTTGTTCT ACCGAAAACA
151  GATCCAGAAA ACGCACAAGC TAATCCTCCA GGCACATCTA CACCGAATGT
201  AGAAAACGGG ATCGATGATC TCAACCCTCT TCTGGGGCAA CCCAACGAAC
251  AAAACAATGC CAACAATCCA GGAACTTCTG GATCTAATCC TACATCTCTA
301  CCCGCCCCCG AACGACTCCC TGAAACTGAA GAGAACAGCC AAGAAGAAGA
351  ACAAGGATCT CAAAATAATG AGGATCTTAT AGGATAA
```

The PSORT algorithm predicts cytoplasm (0.3086).

The following *C.pneumoniae* protein (PID 4376743) was also expressed <SEQ ID 337; cp6743>:

```
  1  LREEGSVSFR EYFRAYMCDK IVAQKNFLFT LDAVIKQAGW RSQEKLNLFY
 51  VESQALGREI KVSLEEYIQS MVGILGSQRT KKSFKFSVDF TPLEQALQER
101  CSSDDDEDAT ATSTATGATA SPTDMHEDE*
```

The cp6743 nucleotide sequence <SEQ ID 338> is:

```
  1  TTGAGAGAAG AAGGTAGTGT TTCTTTCAGA GAATATTTCA GAGCCTATAT
 51  GTGTGATAAA ATCGTGGCAC AGAAGAACTT CTTATTTACT TTAGACGCTG
101  TAATTAAACA GGCCGGTTGG AGATCACAAG AGAAACTCAA TTTATTTTAT
151  GTTGAAAGTC AGGCTTTAGG AAGAGAAATC AAAGTCAGCT TAGAGGAATA
201  TATTCAGAGT ATGGTCGGGA TTTTGGGATC TCAGAGAACC AAGAAAAGCT
251  TTAAGTTTTC TGTCGACTTT ACCCCTTTAG AGCAGGCTCT ACAAGAAAGA
301  TGCTCTTCTG ATGATGACGA AGATGCAACA GCAACTTCGA CCGCTACAGG
351  GGCAACAGCA TCTCCGACTG ACATGCACGA AGATGAGTAA
```

The PSORT algorithm predicts cytoplasm (0.2769).

The following *C.pneumoniae* protein (PID 4377041) was also expressed <SEQ ID 339; cp7041>:

```
  1  MLMMLMMIIG ITGGSGAGKT TLTQNIKEIF GEDVSVICQD NYYKDRSHYT
 51  PEERANLIWD HPDAFDNDLL ISDIKRLKNN EIVQAPVFDF VLGNRSKTEI
101  ETIYPSKVIL VEGILVFENQ ELRDLMDIRI FVDTDADERI LRRMVRDVQE
151  QGDSVDCIMS RYLSMVKPMH EKFIEPTRKY ADIIVHGNYR QNVVTNILSQ
201  KIKNHLENAL ESDETYYMVN SK*
```

The cp7041 nucleotide sequence <SEQ ID 340> is:

```
  1  ATGTTGATGA TGCTTATGAT GATTATTGGA ATTACAGGAG GTTCTGGAGC
 51  TGGGAAAACC ACCCTAACCC AAAACATTAA AGAAATTTTC GGTGAGGATG
101  TGAGTGTTAT CTGCCAAGAT AATTATTACA AAGATAGATC TCATTATACT
151  CCTGAAGAAC GTGCCAATTT AATTTGGGAT CATCCGGACG CCTTTGATAA
201  TGACTTATTA ATTTCAGACA TAAAACGTCT AAAAAAATAAT GAGATTGTCC
251  AAGCCCCAGT TTTTGATTTT GTTTTAGGTA ATCGATCTAA AACGGAGATA
301  GAAACGATCT ATCCATCTAA AGTTATTCTT GTTGAAGGTA TTCTGGTCTT
351  TGAAAATCAA GAACTTAGAG ATCTTATGGA TATTAGGATC TTTGTAGACA
401  CCGATGCTGA TGAAAGGATA CTACGCCGTA TGGTTCGAGA TGTTCAAGAA
451  CAAGGAGATA GCGTGGACTG CATCATGTCT CGTTATCTTT CTATGGTAAA
501  GCCTATGCAT GAGAAATTTA TAGAGCCGAC TCGGAAATAT GCTGATATCA
551  TTGTACATGG AAATTACCGA CAAAACGTAG TAACAAATAT TTTGTCACAG
601  AAAATTAAAA ATCATTTAGA GAATGCCCTG GAAAGCGATG AGACGTATTA
651  TATGGTCAAC TCTAAGTAA
```

The PSORT algorithm predicts inner membrane (0.1022).

The proteins were expressed in *E.coli* and purified as his-tag products (Figure 167A; 6463 = lanes 2-4; 6540 = lanes 5-7; 6743 = lanes 8-9; 7041 = lanes 10-11). The recombinant proteins were used to immunise mice, whose sera were used in Western blots (Figures 167B, 168, 169 & 170) and for FACS analysis.

These experiments show that cp6463, cp6540, cp6743 & cp7041 are surface-exposed and immunoaccessible proteins and that they are useful immunogens. These properties are not evident from the sequence alone.

**Example 171 and Example 172 and Example 173**

The following *C.pneumoniae* protein (PID 4376632) was expressed <SEQ ID 341; cp6632>:

```
  1  VQLFQYMNES GWDWLCDFDS QGEGFQLSRL VGLLHSSWAL YEAKEQFYLP
 51  EVSLLTWEEL IEMQLLSKPT KHGVAKDLCN VFEKHFQRFR QYLGSLDLNQ
101  RFENTFLNYP KYHLDRE*
```

The cp6632 nucleotide sequence <SEQ ID 342> is:

```
  1  GTGCAATTAT TTCAATATAT GAATGAGTCC GGATGGGATT GGCTTTGTGA
 51  TTTTGATTCT CAAGGCGAGG GATTCCAGTT ATCACGTCTG GTTGGGCTGT
101  TACATTCGTC CTGGGCATTA TACGAAGCAA AAGAGCAATT TTACCTTCCT
151  GAGGTTTCTC TATTGACCTG GGAAGAACTG ATAGAAATGC AGTTATTAAG
201  CAAACCAACA AAACACGGGG TTGCAAAAGA TCTTTGTAAT GTATTTGAAA
251  AACACTTTCA AAGGTTTAGA CAGTACCTAG GTTCCTTAGA TCTAAATCAA
301  AGGTTCGAAA ATACCTTCTT GAATTATCCT AAATACCATT TAGATAGGGA
351  GTGA
```

The PSORT algorithm predicts cytoplasm (0.3627).

The following *C.pneumoniae* protein (PID 4376648) was also expressed <SEQ ID 343; cp6648>:

```
  1  MPVSSAPLPT SHRPSSGNLG LMEPNSKALK AKHQDKTTKT IKLLVKILVA
 51  ILVIEVLGII AAFFIPGTPP ICLIIILGGLI LTTVLCVLLL VIKLALVNKT
101  EGTTAEQQIK RKLSSKSIS*
```

The cp6648 nucleotide sequence <SEQ ID 344> is:

```
  1  ATGCCCGTGT CCTCAGCCCC CCTACCCACA AGCCACCGCC CTTCCTCTGG
 51  AAATCTAGGC CTCATGGAAC CAAATTCCAA AGCTCTAAAA GCAAAGCATC
101  AAGATAAAAC GACGAAGACG ATTAAACTTT TAGTTAAAAT CCTTGTTGCC
151  ATTCTAGTAA TAGAAGTTTT AGGAATAATT GCAGCTTTCT TTATTCCTGG
201  GACTCCTCCC ATCTGCTTGA TTATCCTAGG AGGCCTTATT CTTACAACAG
251  TACTCTGTGT GCTTCTTCTT GTTATAAAGC TTGCCCTTGT AAACAAAACC
301  GAAGGAACAA CTGCTGAACA GCAGATAAAA CGTAAACTCT CTTCTAAAAG
351  TATTTCTTAG
```

The PSORT algorithm predicts inner membrane (0.6074).

The following *C.pneumoniae* protein (PID 4376497) was also expressed <SEQ ID 345; cp6497>:

```
 1  MKPNSIIFLE NTKHYPDIFR EGFVRDRHGL MEASDWLLST EITIIRSILG
51  AIPILGNILG AGRLYSVWYT SDEDWKKQVV *
```

The cp6497 nucleotide sequence <SEQ ID 346> is:

```
  1  ATGAAGCCAA ATAGTATTAT TTTTTTAGAA AATACTAAGC ATTATCCCGA
 51  CATCTTTCGA GAAGGATTTG TTCGTGATCG TCATGGACTA ATGGAAGCCT
101  CGGATTGGTT ACTTTCTACG GAAATTACGA TCATTCGCTC CATTCTGGGA
151  GCTATCCCTA TTTTAGGAAA TATTCTTGGA GCCGGACGAC TCTATAGCGT

201  TTGGTATACA AGTGACGAAG ATTGGAAAAA ACAAGTGGTT TGA
```

The PSORT algorithm predicts inner membrane (0.145).

The proteins were expressed in *E.coli* and purified as his-tag products (Figure 171A; 6632 = lanes 5-7; 6648 = lanes 8-10; 6497 = lanes 2-4). The recombinant proteins were used to immunise mice, whose sera were used in Western blots (Figures 171B, 172, 173) and for FACS analysis.

These experiments show that cp6632, cp6648 and cp6497 are surface-exposed and immunoaccessible proteins and that they are useful immunogens. These properties are not evident from the sequence alone.

**Example 174 , Example 175 , Example 176 , Example 177 and Example 178**

The following *C.pneumoniae* protein (PID 4377200) was expressed <SEQ ID 347; cp7200>:

```
  1  MPVPIDNSSR NLQEVPESLE DLEQHAEESP THQSAESSSL QLSLASSAIS
 51  SRVEQLSSLV LGMENSDFSS LRDVPIFSAI YESSTHTPVP TPLVGVGYIN
101  GSQSGYYDTQ RESLHLSQLL GSRRVEVVYN QGNFMEASLL NLCPRRPRRD
151  PSPISLALLE LWEAFFLEHP PGSTFNPIFF W*
```

The cp7200 nucleotide sequence <SEQ ID 348> is:

```
  1  ATGCCCGTTC CTATAGATAA TTCCTCTCGC AACCTACAAG AAGTTCCAGA
 51  AAGCCTAGAA GACCTCGAAC AACACGCAGA AGAATCTCCT ACTCATCAAA
101  GTGCAGAAAG CAGTTCTTTG CAACTGTCTC TAGCCTCCTC AGCAATTTCT
151  AGTAGAGTAG AACAACTATC TTCCCTCGTC TTAGGAATGG AAAATTCAGA
201  TTTCTCCTCT TTAAGAGACG TTCCTATCTT CTCAGCTATC TACGAATCTT
251  CAACACACAC ACCTGTCCCC ACTCCTCTAG TTGGCGTGGG ATATATCAAC
301  GGAAGTCAAT CAGGATACTA CGATACACAA AGAGAATCTC TTCACCTCAG
351  CCAATTGTTA GGAAGCCGAA GAGTTGAAGT TGTCTATAAC CAAGGAAACT
401  TCATGGAGGC CTCTTTGCTA AATCTGTGCC CCAGAAGACC TCGAAGAGAT
451  CCCTCTCCAA TTTCTTTAGC TCTATTAGAG CTCTGGGAAG CATTTTTTTT
501  AGAACACCCC CCAGGTAGCA CTTTTAATCC AATATTTTTT TGGTAA
```

The PSORT algorithm predicts cytoplasm (0.3672).

The following *C.pneumoniae* protein (PID 4377235) was also expressed <SEQ ID 349; cp7235>:

```
  1  LNFVSTLTGS DFYAPVLEKL EEAFADTTGQ VILFSSSPDF IVHPIAQQLG
 51  ISSWYASCYR DQSAEQTIYK KCLTGDKKAQ ILSYIKKINQ ARSHTFSDHI
101  LDLPFLMLGE EKTVVRPQGR LKKMAKKYYW NIV*
```

The cp7235 nucleotide sequence <SEQ ID 350> is:

```
  1  TTGAATTTTG TATCGACTCT GACCGGCTCC GATTTTTATG CTCCTGTTTT
 51  AGAAAAACTA GAAGAAGCTT TTGCAGATAC CACAGGACAG GTGATCCTTT
101  TTTCTTCTTC TCCAGACTTT ATTGTCCACC CCATAGCGCA GCAACTCGGG
151  ATTAGTTCTT GGTATGCGTC GTGTTATCGC GATCAGTCTG CAGAACAGAC
201  GATCTATAAA AAATGTCTTA CAGGGGATAA AAAAGCGCAA ATTTTGAGTT
251  ATATTAAAAA AATTAATCAA GCAAGAAGCC ATACCTTCTC CGACCATATT
301  TTAGATCTTC CTTTTCTTAT GCTGGGAGAA GAGAAAACCG TCGTTCGCCC
351  TCAGGGACGA CTCAAGAAAA TGGCAAAAAA ATATTACTGG AATATCGTTT
401  AA
```

The PSORT algorithm predicts cytoplasm (0.3214).

The following *C.pneumoniae* protein (PID 4377268) was also expressed <SEQ ID 351; cp7268>:

```
  1  MMHRYFIPLL ALLIFSPSLV RAELQPSENR KGGWPTQLSC AEGSQLFCKF

 51  EAAYNNAIEE GKPGILVFFS ERPTPEFADL TNGSFSLSTP IAKGFNVVVL
101  CPGLISPLDF FHKMDPVILY MGSFLEMFPE VEAVSGPRLC YILIDEQGGA
151  QCQAVLPLET KN*
```

The cp7268 nucleotide sequence <SEQ ID 352> is:

```
  1  ATGATGCACC GTTATTTTAT TCCTTTATTA GCACTTCTCA TTTTCTCTCC
 51  TTCTTTAGTC AGGGCAGAGC TACAACCAAG TGAAAACAGA AAAGGGGGGT
101  GGCCTACACA ACTTTCCTGT GCAGAAGGTT CGCAACTCTT CTGTAAATTC
151  GAAGCTGCCT ATAATAATGC AATTGAGGAA GGGAAACCTG GGATTTTAGT
201  CTTTTTCTCT GAGCGACCCA CACCAGAATT TGCCGACTTA ACGAATGGTT
251  CATTTTCTCT CTCTACGCCA ATCGCCAAGG GCTTTAATGT CGTTGTGTTA
301  TGCCCCGGGC TTATCAGTCC CTTAGACTTT TTCCACAAAA TGGATCCTGT
351  GATTCTCTAT ATGGGAAGTT TTCTAGAGAT GTTCCCTGAA GTGGAGGCAG
401  TTAGTGGCCC TCGCTTATGT TATATCTTAA TAGATGAACA GGGTGGGGCT
451  CAATGTCAGG CTGTCCTGCC TTTAGAAACA AAGAATTAG
```

The PSORT algorithm predicts inner membrane (0.1235).

The following *C.pneumoniae* protein (PID 4377375) was also expressed <SEQ ID 353; cp7375>:

```
  1  MQRIIIVGID TGVGKTIVSA ILARALNAEY WKPIQAGNLE NSDSNIVHEL
 51  SGAYCHPEAY RLHKPLSPHK AAQIDNVSIE ESHICAPKTT SNLIIETSGG
101  FLSPCTSKRL QGDVFSSWSC SWILVSQAYL GSINHTCLTV EAMRSRNLNI
151  LGMVVNGYPE DEEHWLTQEI KLPIIGTLAK EKEITKTIIS CYAEQWKEVW
201  TSNHQGIQGV SGTPSLNLH*
```

The cp7375 nucleotide sequence <SEQ ID 354> is:

```
  1  ATGCAACGTA TCATCATTGT AGGAATCGAC ACTGGCGTAG GAAAAACCAT
 51  TGTCAGTGCT ATCCTTGCTA GAGCACTTAA CGCAGAATAC TGGAAACCTA
101  TACAAGCAGG GAATCTAGAA AATTCAGATA GCAATATTGT TCATGAGCTA
151  TCGGGAGCCT ACTGTCATCC CGAAGCTTAT CGATTGCATA AGCCCTTGTC
201  TCCACACAAG GCAGCGCAAA TCGATAATGT AAGTATCGAA GAGAGTCATA
251  TTTGTGCGCC AAAAACAACT TCGAATCTGA TTATTGAGAC TTCAGGAGGA
301  TTTTTATCCC CCTGCACATC AAAAAGACTT CAGGGAGATG TGTTTTCTTC
351  TTGGTCATGT TCTTGGATTT TAGTGAGCCA AGCATATCTC GGAAGTATCA
401  ATCACACCTG TTTAACGGTA GAAGCAATGC GCTCACGAAA CCTCAATATC
451  TTAGGTATGG TGGTAAATGG GTATCCAGAG GACGAAGAGC ACTGGCTAAC
501  TCAAGAAATC AAGCTTCCTA TAATCGGGAC TCTTGCCAAG GAAAAAGAAA
551  TCACAAAGAC AATCATAAGC TGTTATGCCG AACAATGGAA GGAAGTATGG
601  ACAAGCAATC ATCAGGGAAT TCAGGGTGTA TCTGGCACCC CTTCACTCAA
651  TCTGCATTAG
```

The PSORT algorithm predicts cytoplasm (0.0049).

The following *C.pneumoniae* protein (PID 4377388) was also expressed <SEQ ID 355; cp7388>:

```
  1  MQVLLSPQLP PPPQHSVGSI SSPSKLRVLA ITFLVFGMLL LISGALFLTL
 51  GIPGLSAAIS FGLGIGLSAL GGVLMISGLL CLLVKREIPT VRPEEIPEGV
101  SLAPSEEPAL QAAQKTLAQL PKELDQLDTD IQEVFACLRK LKDSKYESRS
151  FLNDAKKELR VFDFVVEDTL SEIFELRQIV AQEGWDLNFL INGGRSLMMT
201  AESESLDLFH VSKRLGYLPS GDVRGEGLKK SAKEIVARLM SLHCEIHKVA
251  VAFDRNSYAM AEKAFAKALG ALEESVYRSL TQSYRDKFLE SERAKIPWNG
301  HITWLRDDAK SGCAEKKLRD AEERWKKFRK AVFWVEEDGG FDINNLLGDW
351  GTVLDPYRQE RMDEITFHEL YEKTTFLKRL HRKCALAKTT FEKKRSKKNL
401  QAVEEANARR LKYVRDWYDQ EFQKAGERLE KLHALYPEVS VSIRENKIQE
451  TRSNLEKAYE AIEENYRCCV REQEDYWKEE EKREAEFRER GNKILSPEEL
501  ESSLEQFDHG LKNFSEKLME LEGHILKLQK EATAEVENKI LSDAESRLEI
551  VFEDVKEMPC RIEEIEKTLR MAELPLLPTK KAFEKACSQY NSCAEMLEKV
601  KPYCKESLAY VTSKERLVSL DEDLRRAYTE CQKRFQGDSG LESEVRACRE
651  QLRERIQEFE TQGLDLVEKE LLCVSSRLRN TECDCVSGVK KEAPPGKKFY
701  AQYYDEIYRV RVQSRWMTMS ERLREGVQAC NKMLKAGLSE EDKVLKEEEY
751  WLYREERKNK EKRLVGTKIV ATQQRVAAFE SIEVPEIPEA PEEKPSLLDK
801  ARSLFTREDH T
```

The cp7388 nucleotide sequence <SEQ ID 356> is:

```
  1  ATGCAAGTAC TTCTATCTCC GCAGCTACCC CCCCCCCCCC AACACTCTGT
 51  AGGGTCGATT TCTTCTCCAT CTAAACTTCG CGTTTTAGCG ATTACTTTTT
```

```
 101  TAGTTTTTGG TATGCTCTTA CTGATTTCAG GAGCTCTCTT TCTGACGTTA
 151  GGGATTCCAG GATTGAGTGC AGCAATTTCT TTTGGATTAG GCATCGGTCT
 201  CTCCGCATTA GGAGGAGTGC TGATGATTTC GGGACTACTA TGTCTTTTAG
 251  TAAAACGAGA GATTCCGACA GTACGACCAG AAGAAATTCC TGAAGGGGTT
 301  TCGCTGGCTC CTTCTGAGGA GCCAGCTCTA CAGGCAGCTC AGAAGACTTT
 351  AGCTCAGCTG CCTAAGGAAT TGGATCAGTT AGATACAGAT ATTCAGGAAG
 401  TGTTCGCATG TTTAAGAAAG CTGAAAGATT CTAAGTATGA AAGTCGAAGT
 451  TTTTTAAACG ATGCTAAGAA GGAGCTTCGA GTTTTTGACT TTGTGGTTGA
 501  GGATACCCTC TCGGAGATTT TCGAGTTGCG GCAGATTGTG GCTCAAGAGG
 551  GATGGGATTT AAACTTTTTG ATCAATGGGG GACGAAGCCT CATGATGACT
 601  GCAGAATCTG AATCGCTTGA TTTGTTTCAT GTATCGAAGC GGCTAGGGTA
 651  TTTACCTTCT GGGGATGTTC GAGGGGAGGG GTTAAAGAAA TCTGCGAAGG
 701  AGATAGTCGC TCGTTTGATG AGCTTGCATT GCGAGATTCA CAAGGTGGCG
 751  GTAGCGTTTG ATAGGAATTC CTATGCGATG GCAGAAAAGG CGTTTGCGAA
 801  AGCGTTGGGA GCTTTAGAAG AGAGTGTGTA TCGGAGTCTG ACGCAGAGTT
 851  ATAGAGATAA ATTTTTGGAG AGCGAGAGGG CGAAGATCCC ATGGAATGGG
 901  CATATAACCT GGTTAAGAGA TGATGCGAAG AGTGGGTGTG CTGAAAAGAA
 951  GCTTCGGGAT GCCGAGGAAC GTTGGAAGAA ATTTAGGAAA GCAGTCTTTT
1001  GGGTAGAAGA AGACGGGGGC TTTGACATCA ATAATCTCCT TGGAGACTGG
1051  GGGACAGTGC TTGATCCTTA TAGACAAGAG AGAATGGACG AGATAACGTT
1101  CCATGAGTTG TATGAAAAAA CTACGTTTTT GAAAAGACTG CACAGAAAGT
1151  GTGCGTTAGC GAAAACAACC TTTGAAAAGA AGAGATCTAA AAAGAATTTG
1201  CAGGCAGTCG AGGAGGCGAA TGCACGTAGG TTGAAATATG TAAGGGATTG
1251  GTATGATCAG GAGTTTCAGA AAGCAGGGGA GAGATTAGAG AAACTGCATG
1301  CTTTGTATCC TGAGGTTTCA GTCTCTATAA GAGAGAACAA AATACAAGAG
1351  ACGCGCTCTA ATTTAGAGAA AGCCTATGAG GCTATCGAAG AGAACTATCG
1401  TTGCTGTGTC CGAGAGCAAG AGGACTACTG GAAAGAAGAA GAGAAAAGGG
1451  AAGCGGAGTT TAGGGAGAGG GGAAACAAGA TTCTTTCTCC TGAGGAGCTG
1501  GAAAGTTCTT TGGAGCAATT CGACCATGGT TTGAAAAATT TTTCTGAGAA
1551  ATTAATGGAA TTGGAAGGGC ATATCTTAAA ACTTCAGAAA GAAGCCACAG
1601  CAGAGGTGGA GAATAAAATA CTTTCAGATG CAGAGAGCCG CCTTGAGATT
1651  GTATTTGAAG ATGTCAAGGA GATGCCCTGT CGAATTGAGG AGATAGAGAA
1701  GACGCTGCGT ATGGCGGAGC TGCCCCTACT TCCTACGAAG AAGGCGTTTG
1751  AGAAGGCCTG CTCACAATAT AATAGCTGCG CAGAGATGTT GGAGAAGGTG
1801  AAGCCTTACT GCAAGGAGAG CCTCGCCTAT GTGACTAGCA AAGAGCGTTT
1851  AGTGAGCTTG GATGAAGATT TACGACGAGC CTACACAGAG TGTCAGAAGA
1901  GATTCCAGGG GGATTCGGGT TTGGAGTCGG AAGTAAGAGC CTGTCGAGAG
1951  CAACTGCGAG AGCGGATCCA AGAGTTTGAA ACTCAAGGGC TGGACTTGGT
2001  GGAAAAAGAG TTGCTTTGTG TGAGTAGTAG ATTAAGAAAT ACAGAGTGCG
2051  ATTGTGTATC TGGTGTTAAG AAAGAAGCAC CTCCTGGTAA GAAGTTTTAT
2101  GCCCAGTATT ATGATGAGAT TTATCGAGTT AGAGTTCAAT CCCGATGGAT
2151  GACGATGTCT GAGAGATTGA GAGAGGGAGT TCAAGCATGC AACAAGATGT
2201  TGAAGGCAGG CCTAAGCGAA GAAGATAAGG TTCTTAAAGA AGAAGAGTAT
2251  TGGTTGTATC GAGAGGAGAG AAAGAATAAA GAGAAACGTT TGGTTGGTAC
2301  TAAGATAGTA GCAACGCAGC AGCGAGTTGC AGCATTTGAA TCCATAGAAG
2351  TTCCTGAGAT TCCTGAGGCC CCAGAGGAGA AACCGAGTTT GCTGGATAAA
2401  GCGCGTTCTT TATTTACTCG CGAGGACCAT ACCTAG
```

The PSORT algorithm predicts inner membrane (0.461).

The proteins were expressed in *E.coli* and purified as his-tag products (Figure 174: 7200=lanes 2-3; 7236=lanes 4-5; 7268=lanes 6-8; 7375=lanes 9-10; 7388=lanes 11-12). The recombinant proteins were used to immunise mice, whose sera were used in Western blots (Figures 174, 175, 176, 177 & 178) and for FACS analysis.

These experiments show that cp7200, cp7235, cp7268, cp7375 & cp7388 are surface-exposed and immunoaccessible proteins and that they are useful immunogens. These properties are not evident from the sequence alone.

## Example 179

The following *C.pneumoniae* protein (PID 4376723) was expressed <SEQ ID 357; cp6723>:

```
  1  MATSVAPSPV PESSPLSHAT EVLNLPNAYI TQPHPIPAAP WETFRSKLST
 51  KHTLCFALTL LLTLGGTISA GYAGYTGNWI ICGIGLGIIV LTLILALLLA
101  IPLKNKQTGT KLIDEISQDI SSIGSGFVQR YGLMFSTIKS VHLPELTTQN
151  QEKTRILNEI EAKKESIQNL ELKITECQNK LAQKQPKRKS SQKSFMRSIK
201  HLSKNPVILF DC*
```

The cp6723 nucleotide sequence <SEQ ID 358> is:

```
  1  ATGGCAACTT CCGTAGCCCC ATCACCAGTC CCCGAGAGCA GCCCTCTCTC
 51  TCATGCTACA GAAGTTCTCA ATCTTCCTAA TGCTTATATT ACGCAGCCTC
101  ATCCGATTCC AGCGGCTCCT TGGGAGACCT TTCGCTCCAA ACTTTCCACA
151  AAGCATACGC TCTGTTTTGC CTTAACACTA CTGTTAACCT TAGGGGGAAC
201  GATCTCAGCA GGTTACGCAG GATATACTGG AAACTGGATC ATCTGTGGCA
251  TCGGCTTGGG AATTATCGTA CTCACACTGA TTCTTGCTCT TCTTCTAGCA
301  ATCCCTCTTA AAAATAAGCA GACAGGAACA AAACTGATTG ATGAGATATC
351  TCAAGACATT TCCTCTATAG GATCAGGATT TGTTCAGAGA TACGGGTTGA
401  TGTTCTCTAC AATTAAAAGC GTGCATCTTC CAGAGCTGAC AACACAAAAT
451  CAAGAAAAAA CAAGAATTTT AAATGAAATT GAAGCGAAAA AGGAATCGAT
501  CCAAAATCTT GAGCTTAAAA TTACTGAGTG CCAAAACAAG TTAGCACAGA
551  AACAGCCGAA ACGGAAATCA TCTCAGAAAT CATTTATGCG TAGTATTAAG
601  CACCTCTCCA AGAACCCTGT AATTTTGTTC GATTGCTGA
```

The PSORT algorithm predicts inner membrane (0.6095).
The protein was expressed in *E.coli* and purified as a his-tag product (Figure 179A). The recombinant protein was used to immunise mice, whose sera were used in a Western blot (Figure 179B) and for FACS analysis.
These experiments show that cp6723 is a surface-exposed and immunoaccessible protein, and that it is a useful immunogen. These properties are not evident from the sequence alone.

## Example 180

The following *C.pneumoniae* protein (PID 4376749) was expressed <SEQ ID 359; cp6749>:

```
  1  MSYYFSLWYL KVQQHFQAAF DFTRSLCSRI SNFALGVIAL LPIIGQLYVG
 51  LDWLLSRIKK PEFPSDVDQI VRVEHVVGHD HRSRVEDILK RQRLSLEPRD
101  EGKVHGDLPS APFF*
```

The cp6749 nucleotide sequence <SEQ ID 360> is:

```
  1  ATGAGTTATT ACTTTTCTCT TTGGTATCTG AAGGTGCAAC AGCACTTTCA
 51  AGCAGCATTT GATTTTACTC GCTCCCTGTG TTCACGAATT TCTAATTTTG
101  CTTTGGGAGT GATTGCATTG CTTCCTATTA TTGGGCAGTT GTATGTAGGG
151  CTGGACTGGC TCCTCTCTAG GATAAAAAAG CCAGAATTTC CTTCCGATGT
201  GGATCAGATC GTGCGAGTAG AACACGTCGT GGGTCACGAC CATAGAAGTC
251  GAGTTGAAGA TATTCTAAAG AGACAAAGGC TCTCATTAGA GCCTAGAGAC
301  GAGGGGAAGG TTCACGGAGA TCTGCCTTCA GCTCCTTTTT TTTGA
```

The PSORT algorithm predicts inner membrane (0.2996).
The protein was expressed in *E.coli* and purified as a his-tag product (Figure 180A). The recombinant protein was used to immunise mice, whose sera were used in a Western blot (Figure 180B) and for FACS analysis.
These experiments show that cp6749 is a surface-exposed and immunoaccessible protein, and that it is a useful immunogen. These properties are not evident from the sequence alone.

## Example 181 , Example 182 , Example 183 , Example 184 and Example 185

The following *C.pneumoniae* protein (PID 4376301) was expressed <SEQ ID 361; cp6301>:

```
  1  LNQDLQNVYQ ECQKATGLES EVSAYRDHLR EQITEFETQG LDVIKEELLF
 51  VSSTLKSKLS YDPLIADIPC MKFYEEYYDG IDKARVQSRW LEKSERYRKA
101  KKGFQEMLKE GLFKEDQALK KAEYRLLREK RMNKEKLLIC NKIEAAQQRV
151  QEFGPSDS*
```

The cp6301 nucleotide sequence <SEQ ID 362> is:

```
  1  TTGAATCAGG ATTTACAAAA TGTATACCAA GAGTGCCAGA AGGCTACAGG
 51  TTTAGAATCG GAAGTGAGTG CATATAGAGA TCATCTTAGA GAGCAGATCA
101  CAGAGTTTGA AACTCAAGGG CTGGACGTGA TAAAAGAAGA ACTTCTTTTT
151  GTGAGTAGTA CTCTCAAAAG TAAATTGAGC TATGATCCAT TAATAGCAGA
201  CATTCCCTGT ATGAAGTTTT ATGAGGAGTA TTATGATGGC ATTGATAAAG
251  CGAGAGTTCA ATCCCGATGG CTGGAGAAGT CTGAGAGGTA TAGAAAGGCG
301  AAGAAGGGAT TCCAAGAGAT GCTGAAGGAA GGCCTATTCA AAGAAGATCA
351  GGCTTTGAAA AAAGCAGAGT ATAGATTACT TCGAGAGAAG AGAATGAATA
401  AGGAGAAGCT TTTGATTTGC AATAAGATAG AAGCAGCTCA GCAGCGAGTC
451  CAAGAATTTG GACCCTCGGA TTCATAA
```

The PSORT algorithm predicts cytoplasm (0.4621).

The following *C.pneumoniae* protein (PID 4376558) was also expressed <SEQ ID 363; cp6558>:

```
  1  MNIPAPQVPV IDEPVVNNTS SYGLSLKSSL RPITYLILAI LAIATLMSVL
 51  YFCGIISVGT FVLGMLIPLS VCSVLCVAYL FYQQSSIEKT KVFSITSPSV
101  FFSDEDLNLL LGREEDSVSA IDELLKNFPA DDFRRPKMLP YSNFLDEQGR
151  PNESREEDSH TSKIL*
```

The cp6558 nucleotide sequence <SEQ ID 364> is:

```
  1  ATGAACATAC CCGCTCCCCA AGTACCAGTC ATAGATGAGC CTGTAGTGAA
 51  CAACACAAGT AGCTATGGTC TTTCATTGAA AAGTAGTTTA AGACCGATTA
101  CTTATTTGAT TTTAGCTATC TTAGCTATAG CCACACTGAT GTCTGTTCTC
151  TACTTTTGTG GCATCATTAG TGTTGGGACG TTTGTTTTGG GCATGCTGAT
201  CCCTCTATCG GTCTGCTCTG TTCTTTGCGT TGCCTATTTA TTCTATCAGC
251  AATCTTCTAT AGAAAAGACT AAGGTCTTTT CTATAACCAG TCCTTCAGTA
301  TTTTTCTCTG ATGAGGATCT TAATTTACTC TTAGGTCGAG AAGAAGATTC
351  AGTGTCTGCA ATTGATGAAC TTCTTAAGAA CTTTCCAGCT GATGATTTCC
401  GTAGGCCGAA GATGCTTCCT TATTCAAATT TTCTAGATGA GCAGGGAAGG
451  CCTAATGAGA GTAGGGAAGA AGACTCTCAT ACTTCCAAGA TCTTATAA
```

The PSORT algorithm predicts inner membrane (0.4630).

The following *C.pneumoniae* protein (PID 4376630) was also expressed <SEQ ID 365; cp6630>:

```
  1  MSMTIVPHAL FKNHCECHST FPLSSRTIVR IAIASLFCIG ALAALGCLAP
 51  PVSYIVGSVL AFIAFVILSL VILALIFGEK KLPPTPRIIP DRFTHVIDEA
101  YGLSISAFVR EQQVTLAEFR QFSTALLCNI SPEEKIKQLP SELRSKVESF
151  GISRLAGDLE KNNWPIFEDL LSQTCPLYWL QKFISAGDPQ VCRDLGVPRE
201  CYGYYWLGPL GYSTAKATIF CKETHHILQQ LTKEDVLLLK NKALQEKWDT
251  DEVKAIVERI YTTYTARGTL KTEAGGLTKE TISKELLLLS LHGYSFDQLQ
301  LITQLPRDAW DWLCFVDNST AYNLQLCALV GALSSQNLLD ESSIDFDVNL
351  GLYVIQDLKE AVQAFSASDE PKKELGKFLL RHLSSVSKRL ESVLRQGLHR
401  IALEHGNARA RVYDVNFVTG ARIHRKTSIF FKD*
```

The cp6630 nucleotide sequence <SEQ ID 366> is:

```
   1  ATGAGCATGA CGATCGTTCC ACATGCTTTA TTTAAAAATC ATTGCGAGTG
  51  TCATTCTACC TTTCCTTTGA GTTCAAGGAC TATTGTAAGA ATAGCCATTG
 101  CCAGCCTCTT TTGTATAGGT GCATTAGCAG CTTTAGGCTG TTTGGCTCCT
 151  CCCGTTTCTT ATATTGTTGG GAGTGTTTTA GCTTTTATTG CCTTTGTCAT
 201  TCTTTCTTTA GTAATTTTAG CTTTGATTTT TGGAGAGAAG AAGCTTCCAC
```

```
 251  CAACACCAAG AATCATTCCT GATAGATTTA CTCACGTGAT AGATGAAGCT
 301  TATGGCCTTT CAATCTCTGC ATTTGTAAGA GAACAGCAGG TAACATTAGC
 351  CGAGTTTAGA CAATTTTCTA CTGCCCTGTT GTGTAACATA TCTCCTGAAG
 401  AGAAAATCAA ACAATTGCCT TCTGAATTGC GAAGTAAAGT AGAGAGTTTT
 451  GGTATTAGCA GGCTCGCAGG TGATTTAGAA AAGAATAATT GGCCAATATT
 501  TGAAGATCTT TTAAGCCAAA CCTGCCCGTT ATATTGGCTT CAGAAATTTA
 551  TATCAGCAGG AGATCCACAA GTTTGTAGAG ACCTAGGTGT CCCTAGAGAA
 601  TGTTATGGGT ACTATTGGCT AGGGCCTTTG GGATACAGTA CAGCTAAGGC
 651  TACAATTTTT TGTAAAGAGA CGCATCATAT TCTTCAACAA TTAACGAAAG
 701  AGGACGTTCT TTTATTAAAA AACAAGGCTC TTCAAGAGAA ATGGGATACT
 751  GATGAAGTCA AAGCAATTGT AGAGCGTATC TACACTACCT ATACGGCACG
 801  AGGAACTCTA AAGACCGAAG CAGGGGGACT TACAAAAGAG ACAATCAGTA
 851  AGGAATTGCT ATTGTTGAGC TTGCATGGCT ATTCTTTTGA TCAGCTACAG
 901  CTGATCACTC AACTTCCTAG AGATGCTTGG GATTGGCTGT GTTTTGTAGA
 951  TAACAGTACC GCATACAACC TTCAGCTTTG TGCTCTTGTA GGAGCTTTGT
1001  CATCCCAAAA TCTTCTTGAC GAATCTTCTA TCGATTTTGA TGTAAACCTA
1051  GGCCTGTATG TGATTCAGGA TCTAAAAGAA GCTGTTCAAG CATTTTCTGC
1101  TTCTGATGAG CCAAAGAAAG AACTAGGTAA ATTCTTGTTA AGGCATTTGA
1151  GTTCAGTTTC TAAGCGATTA GAGAGTGTAT TAAGACAGGG TCTTCACAGA
1201  ATAGCTCTAG AGCATGGAAA TGCCAGAGCT AGGGTTTATG ACGTCAATTT
1251  TGTAACAGGA GCTAGAATTC ATAGGAAGAC GAGTATCTTC TTTAAAGACT
1301  AA
```

The PSORT algorithm predicts inner membrane (0.7092).

The following *C.pneumoniae* protein (PID 4376633) was also expressed <SEQ ID 367; cp6633>:

```
   1  MVNIQPVYRN TQVNYSQATQ FSVCQPALSL IIVSVVAAVL AIVALVCSQS
  51  LLSIELGTAL VLVSLILFAS AMFMIYKMRQ EPKELLIPKK IMELIQEHYP
 101  SIVVDFIRDQ EVSIYEIHHL ISILNKTNVF DKAPVYLQEK LLQFGIEKFK
 151  DVHPSKLPNF EEILLQHCPL HWLGRLVYPM VSDVTPGTYG YYWCGPLGLY
 201  ENAPSLFERR SLLLLKKISF GEFALLEDGL KKNTWSSSEL VQIRQNLFTR
 251  YYADKEEVDE AELNADYEQF DSLLHLIFSH KLS*
```

The cp6633 nucleotide sequence <SEQ ID 368> is:

```
   1  ATGGTTAATA TACAGCCTGT GTATAGGAAT ACCCAAGTCA ACTATAGTCA
  51  GGCTACCCAA TTTTCGGTGT GCCAGCCAGC GCTTAGCCTG ATTATCGTTT
 101  CTGTTGTTGC TGCTGTACTC GCTATTGTAG CTTTGGTATG CAGTCAATCT
 151  CTTTTATCCA TAGAGTTAGG AACTGCTCTT GTTCTAGTTT CTCTTATTCT
 201  TTTTGCTTCT GCTATGTTTA TGATTTATAA GATGAGACAA GAACCTAAGG
 251  AGTTGCTGAT CCCTAAGAAA ATCATGGAAC TCATCCAAGA ACATTATCCA
 301  AGTATTGTTG TTGATTTTAT TAGAGATCAG GAGGTTTCCA TTTATGAGAT
 351  ACATCACTTG ATCTCTATTC TTAATAAGAC GAATGTTTTC GACAAAGCAC
 401  CAGTATATTT ACAAGAAAAA CTCTTACAGT TTGGCATTGA GAAGTTCAAA
 451  GATGTACATC CAAGTAAGCT CCCTAATTTT GAAGAAATTC TTCTACAGCA
 501  TTGCCCATTG CATTGGTTGG GACGTCTGGT ATATCCCATG GTATCGGATG
 551  TCACTCCAGG AACCTATGGA TACTATTGGT GTGGTCCTTT AGGACTGTAC
 601  GAGAACGCTC CCTCTCTTTT TGAACGTCGA TCTCTTCTAT TGTTAAAGAA
 651  AATTAGCTTT GGAGAGTTTG CTCTTTTAGA AGATGGTCTC AAGAAAAACA
 701  CGTGGAGTTC TTCGGAACTC GTTCAAATCA GACAAAACCT TTTTACAAGA
 751  TATTATGCTG ATAAAGAAGA GGTAGATGAA GCAGAGTTAA ACGCTGATTA
 801  CGAACAGTTT GATTCCCTCC TTCACCTTAT TTTTTCTCAC AAGCTCTCTT
 851  GA
```

The PSORT algorithm predicts inner membrane (0.7283).

The following *C.pneumoniae* protein (PID 4376642) was also expressed <SEQ ID 369; cp6642>:

```
  1 MATISPISLT VDHPLVDTKK KSCSNFDKIQ SRILLITAIF AVLVTIGTLL
 51 IGLLLNIPVI YFLTGISFIA VVLSNFILYK RATTLLKPRA CGKHKEIKPK
101 RVSTNLQYSS ISIAINRSKE NWEHQPKDLQ NLPAPSALLT DNPYEIWKAK
151 HSLFSLVSLL PGGNPEHLLI SASENLGKTL LIEETSQNAP ISSYVDTTPS
201 PKSLLNEAIQ ETRVEINTEL PAGDSGERLY WQPDFRGRVF LPQIPTTPEA
251 IYQYYYALYV TYIQTAINTN TQIIQIPLYS LREHLYSREL PPQSRMQQSL
301 AMITAVKYMA ELHPEYPLTI ACVERSLAQL PQESIEDLS*
```

The cp6642 nucleotide sequence <SEQ ID 370> is:

```
   1 ATGGCTACAA TCTCACCCAT ATCTTTAACT GTAGATCATC CCCTAGTAGA

  51 CACTAAAAAA AAATCCTGCA GCAACTTTGA TAAGATTCAG TCTCGAATTC
 101 TATTGATTAC TGCAATCTTT GCTGTCTTAG TTACTATAGG GACCCTACTT
 151 ATTGGTTTGC TTTTAAATAT TCCTGTTATC TATTTCCTCA CAGGAATTTC
 201 ATTTATTGCT GTTGTTCTTA GCAACTTTAT CCTTTATAAA CGAGCAACCA
 251 CCCTCTTAAA ACCGCGTGCT TGTGGCAAAC ACAAAGAAAT AAAACCAAAA
 301 AGGGTCTCCA CCAACCTACA GTATTCTTCT ATCTCTATCG CAATCAATCG
 351 TTCTAAAGAA AACTGGGAAC ACCAACCCAA GGACCTACAG AATCTCCCCG
 401 CACCCTCTGC ATTACTCACA GATAACCCTT ACGAGATATG GAAAGCTAAA
 451 CATTCACTGT TTTCCCTAGT ATCCCTCCTA CCGGGAGGCA ATCCAGAACA
 501 TCTCTTAATT TCAGCTTCCG AAAATTTAGG AAAGACTCTG TTAATTGAAG
 551 AAACCTCGCA AAATGCGCCT ATATCCTCCT ACGTAGATAC CACTCCCTCC
 601 CCAAAATCCT TGCTCAATGA GGCAATTCAG GAAACCAGGG TAGAAATAAA
 651 TACAGAACTC CCTGCGGGAG ATTCAGGAGA ACGTTTATAC TGGCAACCCG
 701 ATTTCCGAGG CCGCGTCTTC CTCCCACAAA TACCAACAAC TCCTGAAGCC
 751 ATCTACCAAT ACTACTATGC ACTCTATGTC ACTTATATCC AGACTGCGAT
 801 CAATACGAAC ACCCAAATTA TCCAAATCCC TTTATACAGC TTGAGGGAGC
 851 ATCTCTATTC TAGAGAATTG CCCCCGCAAT CAAGAATGCA ACAATCTTTG
 901 GCTATGATTA CAGCAGTAAA ATACATGGCC GAGCTGCACC CAGAATATCC
 951 GCTAACTATT GCTTGTGTTG AAAGATCCTT AGCCCAACTA CCTCAAGAAA
1001 GTATTGAGGA TCTCTCTTAG
```

The PSORT algorithm predicts inner membrane (0.5288).

The proteins were expressed in *E.coli* and purified as GST-fusion products. The recombinant proteins were used to immunise mice, whose sera were used in Western blots (Figures 181-185) and for FACS analysis.

These experiments show that cp6301, cp6558, cp6630, cp6633 and cp6642 are surface-exposed and immunoaccessible proteins, and that they are useful immunogens. These properties are not evident from their sequences alone.

**Example 186**

The following *C.pneumoniae* protein (PID 4376389) was expressed <SEQ ID 371; cp6389>:

```
  1 MSEVKPLFLK NDSFDLATQR FQNLINMLQE QAEIYNEYEE KNARVQNEIK
 51 EQKDFVKRCI EDFEARGLGV LKEELASLTR DFHDKAKAET SMLIECPCIG
101 FYYSIHQEEQ RQRQERLQKM AERYRDCKQV LEAVQVEQKD MISSRVVVDD
151 SYFEEEKEEQ KVDNRKKEQD *
```

The cp6389 nucleotide sequence <SEQ ID 372> is:

```
  1  ATGTCAGAAG TGAAGCCTTT GTTTTTAAAG AATGACTCTT TTGATTTGGC
 51  AACTCAGAGA TTCCAGAATC TAATTAACAT GCTACAAGAG CAAGCCGAGA
101  TATATAACGA GTATGAAGAA AAGAATGCTA GGGTTCAGAA TGAGATTAAG
151  GAGCAAAAGG ACTTTGTGAA AAGATGCATA GAGGACTTTG AAGCCAGAGG
201  ACTGGGGGTG CTAAAAGAAG AGCTTGCATC TTTGACGCGT GATTTCCATG
251  ATAAAGCAAA AGCAGAGACT TCTATGCTCA TTGAATGTCC TTGTATTGGT
301  TTTTATTATA GTATTCATCA GGAGGAACAA AGGCAAAGGC AAGAAAGGCT
351  TCAAAAGATG GCTGAGCGCT ATAGGGACTG TAAACAAGTC TTGGAGGCTG
401  TCCAGGTGGA GCAAAAAGAT ATGATATCTT CTAGAGTCGT TGTCGATGAC
451  AGCTACTTTG AAGAAGAAAA AGAAGAACAA AAGGTGGATA ACAGAAAGAA
501  AGAACAGGAC TAG
```

The PSORT algorithm predicts cytoplasm (0.3193).

The protein was expressed in *E.coli* and purified as a GST-fusion product (Figure 186A) and also in his-tagged form. The recombinant proteins were used to immunise mice, whose sera were used in a Western blot (Figure 186B) and for FACS analysis.

These experiments show that cp6389 is a surface-exposed and immunoaccessible protein, and that it is a useful immunogen. These properties are not evident from the sequence alone.

## Example 187

The following *C.pneumoniae* protein (PID 4376792) was expressed <SEQ ID 373; cp6792>:

```
  1  VLQEHFFLSE DVITLAQQLL GHKLITTHEG LITSGYIVET EAYRGPDDKA
 51  CHAYNYRKTQ RNRAMYLKGG SAYLYRCYGM HHLLNVVTGP EDIPHAVLIR
101  AILPDQGKEL MIQRRQWRDK PPHLLTNGPG KVCQALGISL ENNRQRLNTP
151  ALYISKEKIS GTLTATARIG IDYAQEYRDV PWRFLLSPED SGKVLS*
```

The cp6792 nucleotide sequence <SEQ ID 374> is:

```
  1  GTGCTACAAG AACATTTTTT TCTATCGGAA GATGTAATTA CACTAGCGCA
 51  ACAGCTTTTA GGACATAAAC TCATCACAAC ACATGAGGGT CTGATAACTT
101  CAGGTTACAT TGTAGAAACC GAAGCGTATC GTGGCCCTGA TGACAAAGCA
151  TGCCACGCCT ACAACTACAG AAAAACTCAG AGGAACAGAG CGATGTACCT
201  GAAAGGAGGC TCTGCTTACC TCTACCGTTG CTATGGCATG CATCACCTAT
251  TGAATGTTGT CACTGGACCT GAGGACATTC CCCATGCCGT CCTGATCCGG
301  GCCATCCTTC CTGATCAAGG CAAAGAACTT ATGATCCAAC GCCGCCAATG
351  GAGAGATAAA CCCCCACACC TTCTCACCAA TGGACCCGGA AAAGTGTGCC
401  AAGCTCTAGG AATCTCTTTG GAAAACAATA GGCAACGCCT AAATACCCCA
451  GCTCTCTATA TCAGCAAAGA AAAAATCTCT GGGACTCTAA CAGCAACTGC
501  CCGGATCGGC ATCGATTATG CTCAAGAGTA TCGTGATGTC CCATGGAGAT
551  TTCTCCTATC CCCAGAAGAT TCGGGAAAAG TTTTATCTTA A
```

The PSORT algorithm predicts cytoplasm (0.180).

The protein was expressed in *E.coli* and purified as a his-tagged product (Figure 187A; lanes 2-4). The recombinant protein was used to immunise mice, whose sera were used in a Western blot (Figure 187B) and for FACS analysis.

These experiments show that cp6792 is a surface-exposed and immunoaccessible protein, and that it is a useful immunogen. These properties are not evident from the sequence alone.

## Example 188

The following *C.pneumoniae* protein (PID 4376868) was expressed <SEQ ID 375; cp6868>:

```
  1  MVETVLHNFQ RYLSKYLYRV FRFPCRKKTF LSSHRVLARP SFPVDYCPGK
 51  IYDLQEIYEE LNAQLFQGAL RLQIGWFGRK ATRKGKSVVL GLFHENEQLI
101  RIHRSLDRQE IPRFFMEYLV YHEMVHSVVP REYSLSGRSI FHGKKFKEYE
151  QRFPLYDRAV AWEKANAYLL RGYKKRVGGG YGRA*
```

The cp6868 nucleotide sequence <SEQ ID 376> is:

```
  1  ATGGTTGAAA CAGTACTTCA TAATTTCCAA CGTTATCTGA GCAAGTATCT
 51  CTATAGGGTA TTTCGCTTCC CATGTCGTAA AAAGACGTTC CTATCTTCGC
101  ACAGGGTTCT TGCTCGTCCT TCATTCCCAG TAGACTACTG TCCGGGAAAG
151  ATCTATGATT TGCAGGAGAT CTATGAGGAA TTGAATGCGC AGTTATTTCA
201  AGGTGCACTG CGTTTACAGA TTGGTTGGTT CGGAAGGAAA GCTACCAGAA
251  AAGGCAAGAG TGTTGTCTTG GGATTGTTTC ATGAAAATGA ACAGTTAATT
301  CGAATTCATC GTTCTTTAGA TCGGCAGGAA ATCCCAAGAT TTTTTATGGA
351  ATATCTTGTG TATCATGAAA TGGTTCATAG TGTAGTCCCT AGAGAGTATT
401  CTCTATCGGG GCGTTCGATT TTTCATGGTA AAAAGTTTAA AGAATACGAA
451  CAACGTTTCC CCTTGTATGA TCGTGCTGTT GCTTGGGAAA AGGCAAACGC
501  TTATTTATTG CGAGGGTATA AAAAAAGAGT AGGTGGAGGA TATGGCAGGG
551  CATAG
```

The PSORT algorithm predicts bacterial cytoplasm (0.325).

The protein was expressed in *E.coli* and purified as a his-tag product (Figure 188A; lanes 2-3). The recombinant protein was used to immunise mice, whose sera were used in a Western blot (Figure 188B) and for FACS analysis.

These experiments show that cp6868 is a surface-exposed and immunoaccessible protein, and that it is a useful immunogen. These properties are not evident from the sequence alone.

### Example 189

The following *C.pneumoniae* protein (PID 4376894) was expressed <SEQ ID 377; cp6894>:

```
  1  MYKRCVLDKI LKGIVAGSLI LLYWSSDLLE RDIKSIKGNV RDIQEDIREI
 51  SRVVKQQQTS QAIPAAPGVM LAPKLVRDEA FALLFGDPSY PNLLSLDPYK
101  QQTLPELLGT NFHPHGILRT AHVGKPENLS PFNGFDYVVG FYDLCIPSLA
151  SPHVGKYEEF SPDLAVKIEE HLVEDGSGDK EFHIYLRPNV FWRPIDPKAL
201  PKHVQLDEVF QRPHPVTAHD IKFFYDAVMN PYVATMRAVA LRSCYEDVVS
251  VSVENDLKLV VRWKAHTVIN EEGKEERKVL YSAFSNTLSL QPLPRFVYQY
301  FANGEKIIED ENIDTYRTNS IWAQNFTMHW ANNYIVSCGA YYFAGMDDEK
351  IVFSRNPDFY DPLAALIDKR FVYFKESTDS LFQDFKTGKI DISYLPPNQR
401  DNFYSFMKSS AYNKQVAKGG AVRETVSADR AYTYIGWNCF SLFFQSRQVR
451  CAMNMAIDRE RIIEQCLDGQ GYTISGPFAS SSPSYNKQIE GWHYSPEEAA
501  RLLEEEGWID TDGDGIREKV IDGVIVPFRF RLCYYVKSVT AHTIADYVAT
551  ACKEIGIECS LLGLDMADLS QAFDEKNFDA LLMGWCLGIP PEDPRALWHS
601  EGAMEKGSAN VVGFHNEEAD KIIDRLSYEY DLKERNRLYH RFHEIIHEEA
651  PYAFLFSRHC SLLYKDYVKN IFVPTHRTDL IPEAQDETVN VTMVWLEKKE
701  DPCLSTS*
```

The cp6894 nucleotide sequence <SEQ ID 378> is:

```
   1 ATGTATAAAA GATGTGTGCT AGATAAAATT TTAAAGGGGA TTGTCGCCGG
  51 TTCTTTAATT TTGTTATACT GGTCCTCAGA CCTACTTGAA AGAGACATTA
 101 AGTCGATAAA AGGTAACGTA AGAGATATTC AAGAAGACAT TCGTGAAATC
 151 TCACGCGTAG TGAAACAACA GCAGACATCA CAAGCTATCC CTGCGGCACC
 201 TGGGGTGATG CTCGCTCCTA AGCTCGTCAG AGACGAAGCT TTTGCTCTAC
 251 TCTTTGGAGA TCCTAGTTAT CCTAATTTAC TTTCCCTAGA CCCCTATAAA
 301 CAGCAGACTC TTCCTGAACT TCTAGGAACA AATTTCCACC CTCATGGTAT
 351 CCTACGCACT GCCCATGTCG GAAAACCCGA AAATCTGAGC CCTTTTAATG
 401 GCTTTGATTA TGTCGTGGGC TTTTACGATC TCTGTATTCC TAGTTTAGCT
 451 TCTCCCCACG TAGGGAAATA CGAAGAATTT TCTCCAGATC TCGCTGTGAA
 501 AATAGAAGAA CATCTTGTTG AAGATGGTTC TGGGGATAAA GAGTTTCACA
 551 TCTATCTGAG GCCGAATGTT TTTTGGCGTC CTATAGATCC TAAGGCCCTT
 601 CCAAAACACG TTCAGTTAGA CGAAGTATTT CAACGTCCTC ATCCTGTGAC
 651 AGCTCATGAT ATTAAGTTTT TCTACGACGC TGTTATGAAC CCTTATGTAG
 701 CAACCATGCG AGCAGTGGCT CTGCGCTCTT GTTATGAAGA TGTGGTTTCT
 751 GTCTCAGTAG AAAACGATTT AAAATTAGTA GTCAGATGGA AAGCACACAC
 801 GGTAATCAAT GAAGAAGGAA AGGAAGAGCG CAAAGTGCTC TACTCTGCAT
 851 TTTCTAATAC CTTAAGCTTG CAGCCCCTCC CTAGATTTGT ATATCAGTAT
 901 TTTGCTAACG GGGAAAAAAT CATTGAAGAT GAGAATATCG ATACCTACCG
 951 AACCAATTCC ATTTGGGCGC AAAACTTCAC TATGCATTGG GCAAACAACT
1001 ATATTGTAAG TTGTGGAGCC TACTACTTTG CAGGGATGGA TGATGAGAAA
1051 ATCGTGTTTT CTAGAAATCC TGACTTCTAT GATCCTCTTG CGGCTCTTAT
1101 TGACAAGCGT TTCGTCTATT TTAAGGAAAG CACAGACTCC CTATTCCAAG
1151 ATTTTAAGAC AGGGAAAATA GACATCTCTT ACCTTCCACC CAACCAAAGA
1201 GATAATTTCT ATAGTTTTAT GAAAAGCTCC GCTTATAACA AACAGGTAGC
1251 TAAGGGAGGA GCCGTCCGTG AAACAGTCTC AGCAGATCGA GCATATACGT
1301 ACATAGGATG GAATTGCTTT TCATTATTTT TCCAAAGCCG ACAGGTGCGC
1351 TGTGCTATGA ACATGGCAAT CGATAGAGAG AGGATTATCG AACAGTGCTT
1401 GGATGGCCAA GGCTATACGA TTAGTGGGCC TTTTGCTTCG AGTTCTCCTT
1451 CTTATAATAA ACAGATCGAA GGGTGGCATT ATTCTCCAGA AGAAGCAGCT
1501 CGTCTCCTGG AAGAAGAGGG ATGGATAGAT ACCGATGGCG ATGGAATCCG
1551 AGAAAAAGTT ATCGATGGTG TGATTGTCCC GTTCCGTTTC CGTTTATGCT
1601 ATTATGTAAA GAGTGTCACC GCTCATACCA TTGCAGATTA CGTAGCTACT
1651 GCTTGTAAGG AAATCGGAAT CGAGTGTAGC CTTCTAGGAC TAGATATGGC
1701 CGATCTTTCG CAAGCTTTTG ATGAAAAGAA TTTCGATGCT CTTTTAATGG
1751 GATGGTGTTT AGGAATTCCT CCTGAGGATC CTAGGGCTTT ATGGCATTCT

1801 GAAGGGGCTA TGGAAAAGGG TTCAGCGAAT GTTGTAGGTT TCCATAATGA
1851 AGAAGCTGAT AAAATCATAG ACAGACTCAG CTACGAATAC GATCTGAAAG
1901 AACGTAATCG CCTGTACCAC CGTTTCCATG AAATTATTCA TGAGGAAGCT
1951 CCTTATGCTT TCTTGTTCTC ACGACATTGT TCCTTACTTT ATAAGGATTA
2001 TGTAAAAAAT ATTTTCGTAC CTACACATAG AACAGATTTA ATTCCTGAAG
2051 CTCAGGATGA GACTGTCAAC GTAACTATGG TATGGCTTGA GAAGAAGGAG
2101 GATCCGTGCT TAAGTACATC CTAA
```

The PSORT algorithm predicts inner membrane (0.162).

The protein was expressed in *E.coli* and purified as a his-tag product (Figure 189A) and also in GST/his form. The recombinant proteins were used to immunise mice, whose sera were used in a Western blot (Figure 189B) and for FACS analysis.

These experiments show that cp6894 is a surface-exposed and immunoaccessible protein, and that it is a useful immunogen. These properties are not evident from the sequence alone.

**Example 190**

The following *C.pneumoniae* protein (PID 4377193) was identified in the 2D-PAGE experiment <SEQ ID 379; cp7193>:

```
  1  MKRVIYKTIF CGLTLLTSLS SCSLDPKGYN LETKNSRDLN QESVILKENR
 51  ETPSLVKRLS RRSRRLFARR DQTQKDTLQV QANFKTYAEK ISEQDERDLS
101  FVVSSAAEKS SISLALSQGE IKDALYRIRE VHPLALIEAL AENPALIEGM
151  KKMQGRDWIW NLFLTQLSEV FSQAWSQGVI SEEDIAAFAS TLGLDSGTVA
201  SIVQGERWPE LVDIVIT*
```

A predicted leader peptide is underlined.

The cp7193 nucleotide sequence <SEQ ID 380> is:

```
  1  ATGAAAAGAG TCATTTATAA AACCATATTT TGCGGGTTAA CTTTACTTAC
 51  AAGTTTGAGT AGTTGTTCCC TGGATCCTAA AGGATATAAC CTAGAGACAA
101  AAAACTCGAG GGACTTAAAT CAAGAGTCTG TTATACTGAA GGAAAACCGT
151  GAAACACCTT CTCTTGTTAA GAGACTCTCT CGTCGTTCTC GAAGACTCTT
201  CGCTCGACGT GATCAAACTC AGAAGGATAC GCTGCAAGTG CAAGCTAACT
251  TTAAGACCTA CGCAGAAAAG ATTTCAGAGC AGGACGAAAG AGACCTTTCT
301  TTCGTTGTCT CGTCTGCTGC AGAAAAGTCT TCAATTTCGT TAGCTTTGTC
351  TCAGGGTGAA ATTAAGGATG CTTTGTACCG TATCCGAGAA GTCCACCCTC
401  TAGCTTTAAT AGAAGCTCTT GCTGAAAACC CTGCCTTGAT AGAAGGGATG
451  AAAAAGATGC AAGGCCGTGA TTGGATTTGG AATCTTTTCT TAACACAATT
501  AAGTGAAGTA TTTTCTCAAG CTTGGTCTCA AGGGGTTATC TCTGAAGAAG
551  ATATCGCCGC ATTTGCCTCC ACCTTAGGTT TGGACTCCGG GACCGTTGCG
601  TCCATTGTCC AAGGGGAAAG GTGGCCCGAG CTTGTGGATA TAGTGATAAC
651  TTAA
```

The PSORT algorithm predicts periplasmic (0.925).

This shows that cp7193 is an immunoaccessible protein in the EB and that it is a useful immunogen. These properties are not evident from the protein's sequence alone.

It will be appreciated that the invention has been described by way of example only and that modifications may be made whilst remaining within the spirit and scope of the invention.

**TABLE II - sequences of the primers used to amplify Cpn genes.**

| Orf ID | N-terminus final primer | C-terminus final primer |
|---|---|---|
| CP0014P | GCGTC CCG GGTCATATG AAGTCTTCTTTCCCCA | GCGT CTC GAG ATGAAAGAGTTTTTGCG |
| CP0015P | GCGTCCCGGGTCATATG TCAGCTCTGTTTTCTGA | GCGT CTC GAG GAATTGGTATTTTGCTC |
| CP0016P | GCGTCCCGGGTCATATG GCCGATCTCACATTAG | GCGT CTC GAG GTCCAAGTTAAGGTAGCA |
| CP0017P | GCGT CCG GGTCATATG GGTATCAAGGGAACTG | GCGT CTC GAG AAATCCGAATCTTCC |
| CP0019P | GCGTCCCGGGTCAT ATGCAAGACTCTCAAGACTATAG | GCGT CTC GAG AAATCGGTATTTACCC |
| CP6260P | GCGTC CCG GGT GCTAGCACTACGATTTCTTTAACCC | GCGT CTC GAG AAAACGAAATTTGCTTC |
| CP6397P | GCGTC CCG GGTCATATGTTTAAACTGCTAAAAAATCTATT | GCGT CTC GAG ATGAAAGAAGAGTCCTCG |
| CP6456P | GCGTC CCG GGT CATATG TCATCTCCTGTAAATAACA | GCGT CTC GAG CTGACCATCTCCTGTT |
| CP6466P | GCGTC CCG GGT CAT ATG TGCAAGGAGTCCAGT | GCGT CTC GAG ATTTTCCTTAGCATAACG |
| CP6467P | GCGTC CCG GGT CAT ATG TGTTCCCCATCCCAA | GCGT CTC GAG TAGTTTTTCTATAAAACGAAAGTCT |

(continued)

| Orf ID | N-terminus final primer | C-terminus final primer |
|---|---|---|
| CP6468P | GCGTC CCG GGT CAT ATG TGCTCCTCCTACTCTTC | GCGT CTC GAG GGGGAAATAGGTATATTTGA |
| CP6469P | GCGTC CCG GGT CAT ATG AGCTGCTCAAAGCAA | GCGT CTC GAG ACTTAAGATATCGATATTTTTGA |
| CP6552P | GCGTC CCG GGT CAT ATG TGCCATAAGGAAGATG | GCGT CTC GAG ACCATTGTCTTGAGTCAT |
| CP6567P | GCGTC CCG GGT CAT ATG ACCTCACCGATCCCC | GCGT CTC GAG AGAAGCCGGTAGAGGC |
| CP6576P | GCGTC CCG GGT CAT ATG ACTGAAAAAGTTAAAGAAGG | GCGT CTC GAG GAA CATGCCCCCTAA |
| CP6727P | GCGTC CCG GGT CATATGCTACATCCACTAATGGC | GCGT CTC GAG GAAAGAATAACGAGTTCC |
| CP6729P | GCGTC CCG GGT CAT ATGGCAGATGCTTCTTTATC | GCGT CTC GAG GAATGAGTATCTTAGCC |
| CP6731P | GCGTC CCG GGT CATATGGCTGTTGTTGAAATCAAT | GCGTC CAT GGC GGC CGC GAACTGGAACTTACCTCC |
| CP6736P | GCGTC CCG GGT GCT AGCGTAGAAGTTATCATGCCTT | GCGTC CAT GGC GGC CGC AAATCGTAATTTGCTTC |
| CP6737P | GCGT GGA TCC CAT ATG GAGACTAGACTCGGAGG | GCGT CTC GAG AAATGTGGATTTTAGTCC |
| CP6751P | GCGTC CCG GGT GCT AGC AATGAAGGTCTCCAACT | GCGT CTC GAG AAATCTCATTCTACTCGC |
| CP6752P | GCGTGA ATT CAT ATGTTCGGGATGACTCCT | GCGT CTC GAG GAATTTTAAGGTACTTCCTG |
| CP6753P | GCGTC CCG GGT GCT AGCACTCCCTACTCTCATAGAG | GCGT CTC GAG AAACTTAAAGGTCGTTC |
| CP6767P | GCGTC CCG GGT CAT ATG ATAAAACAAATAGGCCGT | GCGT CTC GAG TTCGTAAGCAACTTCAGA |
| CP6829P | GCGTC CCG GGT CAT ATG AAGCAGATGCGTCTTT | GCGTC CAT GGC GGC CGC GAAACTAAGGGAGAGGC |
| CP6830P | GCGTC CCG GGT CAT ATG GATCCCGCGTCTGTT | GCGTC CAT GGC GGC CGC GAATACAAACCGGATCC |
| CP6832P | GCGTC CCG GGT CAT ATG CATAAAGTAATAGTTTTCATTT | GCGT CTC GAG TAAACTAGAAAAAGTCGTC |
| CP6848P | GCGTC CCG GGT CAT ATG TCATCAAATCTACATCCC | GCGT CTC GAG AACGCGAGCTATTTTAC |
| CP6849P | GCGTC CCG GGT GCT AGC AGCGGGGGTATAGAG | GCGT CTC GAG ATACACGTGGGTATTTTC |
| CP6850P | GCGTC CCG GGT CAT ATG TGCCGCATTGTAGAT | GCGT CTC GAG CTGTTTGCATCTGCC |
| CP6854P | GCGTC CCG GGT GCT AGC TCAATAGCTATTGCAAG | GCGT CTC GAG TTATCGAAATGTCTTTG |
| CP6879P | GCGTC CCG GGT CAT ATG GCAACACCCGCTCAA | GCGTC CAT GGC GGC CGC TCCTTGAAATTGCTCTTGC |
| CP6894P | GCGTC CCG GGT CAT ATG TATAAAAGATGTGTGCTAGA | GCGT CTC GAG GGATGTACTTAAGCACG |

(continued)

| Orf ID | N-terminus final primer | C-terminus final primer |
|---|---|---|
| CP6900P | GCGTC CCG GGT CAT ATG AAGATAAAATTTTCTTGGAAG | GCGT AAG CTT GGGAAGACGATACCG |
| CP6952P | GCGTC CCG GGT CAT ATG CTCTCGGATCAATATATAGG | GCGT CTC GAG TCGAATTTCTTTTTTAGC |
| CP7034P | GCGTC CCG GGT CAT ATG AAAAAACAGGTATATCAATG | GCGT AAG CTT AAACGCTGAAATTATACC |
| CP7090P | GCGTC CCG GGT CAT ATG TGTAGCCTTTCCCCT | GCGT CTC GAG GCGTGCATGAATCTTA |
| CP7091P | GCGTC CCG GGT CAT ATG GAAGAATTAGAAGTTGTTGT | GCGT CTC GAG TAGTGTTCTCTTTATCGGT |
| CP7170P | GCGTC CCG GGT CAT ATG CTAGGGGCTGGAAACC | GCGT AAG CTT AAACTGCAGACCTGACG |
| CP7228P | GCGTC CCG GGT CAT ATG ACTGCTGTTCTTATTCTTACA | GCGT CTC GAG ATCTGAAAGCGGAGG |
| CP7249P | GCGTC CCG GGT CAT ATG ATCCCATCCCCTACC | GCGT CTC GAG ATCAGGTTGCTGAGACTT |
| CP7250P | GCGTC CCG GGT CAT ATG AATCTTTCAAACAGGTCT | GCGT CTC GAG ATTTTTTCTAGAGAGACTCTC |
| CP0018P | GTGCGT CATATG GCAACCACTCCACTAA | ACTCGCTA GCGGCCGC TAATGAGGTCCCCAG |
| CP6270P | GTGCGT CATATG AATTTATTAGGAGCTGCT | ACTCGCTA GCGGCCGC AAATTTGATTTTGCTACC |
| CP6735P | GTGCGT CATATG GCAGCACAAGTTGTATAT | ACTCGCTA GCGGCCGC TGGCGTAGAAGTGATC |
| CP6998P | GTGCGT CATATG TTGCCTGTAGGGAAC | ACTCGCTA GCGGCCGC GAATCTGAACTGACCAGA |
| CP7033P | GTGCGT CATATG GTTAATCCTATTGGTCCA | ACTCGCTA GCGGCCGC TTGGAGATAACCAGAATATA |
| CP7287P | GTGCGT CATATG TTACACAGCTCAGAACTAGA | ACTCGCTA GCGGCCGC GAAAATAATACGGATACCA |
| CP0010P | GTGCGT CATATG GCAACTGCTGAAAATATA | GCGT CTCGAG GAATTGGAACTTACCC |
| CP0468P | GTGCGT GCTAGC ATTTTTTATGACAAACTCTAT | GCGT CTCGAG AAATGTGCAATGACTCT |
| CP6272P | GTGCGT CATATG TTGACTCATCAAGAGGCT | GCGT CTCGAG GAAGGGAGGTTTTTTAGGT |
| CP6273P | GTGCGT CATATG ACATATCTGGAAGCTC | ACTCGCTA GCGGCCGC CTCCACAATTTTTATG |
| CP6362P | GTGCGT CATATG CCCTTTGATATTACTTATTATACA | GCGT CTCGAG TCGTTTCCAAATCCA |
| CP6372P | GTGCGT CATATG AAACAACACTATTCTCTAAATA | GCGT CTCGAG TTTCTTGTGGTTTTTCT |
| CP6390P | GTGCGT CATATG CGAGAGGTGCCTAAG | ACTCGCTA GCGGCCGC TCTCCTAGACAGCCTT |
| CP6402P | GTGCGT CATATG AATGTTGCGGATCTCCTTT | GCGT CTCGAG GAAGGGGTTGGCCGT |
| CP6446P | GTGCGT CATATG TGTAATCAAAAGCCCTCTT | GCGT CTCGAG GGGCTGAGGAGGAAC |

(continued)

| Orf ID | N-terminus final primer | C-terminus final primer |
|---|---|---|
| CP6520P | GTGCGT GCTAGC AAACACTACCTATCATTTTCT | GCGT CTCGAG CAGAAAGGCTTTTCTTT |
| CP6577P | GTGCGT CATATG AATTTAGGCTATGTTAATTTA | GCGT CTCGAG GTTTTGTTTTTTGAAAGA |
| CP6602P | GTGCGT CATATG GCAGCATCAGGAGGCA | GCGT CTCGAG TGACCAAGGATAGGGTTTAG |
| CP6607P | GTGCGT CATATG CCTCGTGGTGACACTTT | GCGT CTCGAG CGCTGCTTCTTGCTC |
| CP6615P | GTGCGT CATATG TGCTCTCAAAAAACGACAA | GCGT CTCGAG TGAAGAGGCGCCATC |
| CP6624P | GTGCGT CATATG GATGCGAAAATGGGA | GCGT CTCGAG TCTTTGACATTCAAGAGC |
| CP6672P | GTGCGT CATATG ATTCCTACCATGTTAATG | GCGT CTCGAG GTCATACAATTTCCTTATATA |
| CP6679P | GTGCGT CATATG TGCACTCACTTAGGCT | GCGT CTCGAG CGAGTAGTTAGCACAAAC |
| CP6717P | GTGCGT GCTAGC AAGACAATCGTAGCTTCA | ACTCGCTA GCGGCCGC GGCTGGCATATAGGT |
| CP6784P | GTGCGT GCTAGC AAATCAAGATGTTCTATTGATA | GCGT CTCGAG TCCAAAACAACCCTCT |
| CP6802P | GTGCGT CATATG TGCGTAAGTTATATTAATTCCTT | GCGT CTCGAG CAGTCGGGCTTGTTG |
| CP6847P | GTGCGT CATATG TCGGATCTTTTACGAG | GCGT CTCGAG TTTTCTACACTGTTGTAATAAA |
| CP6884P | GTGCGT CATATG AATCAGCTGCTTTCT | GCGT CTCGAG AGAGAAGGTAATTGTACC |
| CP6886P | GTGCGT CATATG TGTCTACTTATTATCTATCTCTAC | GCGT CTCGAG TTCAGAAAAATGGCT |
| CP6890P | GTGCGT CATATG TCCCCACGACGACAA | GCGT CTCGAG TCCTGCAGCATTTAGC |
| CP6960P | GTGCGT CATATG TGTGACGTACGGTCTA | ACTCGCTA GCGGCCGC TTCACCTTGATTTCCT |
| CP6968P | GTGCGT CATATG TGCGATGCAAAAC | ACTCGCTA GCGGCCGC GGAAGTATGCTTAGATATT |
| CP6969P | GTGCGT CATATG TGCTGTGGTTACTCTATT | ACTCGCTA GCGGCCGC AAAAAGGTCATAGTATACCT |
| CP7005P | GTGCGT CATATG AAAACTGTGATATTGAACA | GCGT CTCGAG CTGAGCTTCTATTTCTATTAT |
| CP7072P | GTGCGT CATATG CCCATTTATGGGAAA | GCGT CTCGAG GTTGAGCAAAGGTTTG |
| CP7101P | GTGCGT CATATG TATTCGTGTTACAGCAA | GCGT CTCGAG GAAAAATTCTTTAGGGAG |
| CP7102P | GTGCGT CATATG GCCGCTAAAGCAAAT | GCGT CTCGAG TGAAAATGAAAGGATGGT |
| CP7105P | GTGCGT GCTAGC AGTCTATATCAAAAATGGTG | GCGT CTCGAG ATCTTTCATTTGGTTATCT |
| CP7106P | GTGCGT CATATG AAAGATTTGGGGACTCT | GCGT CTCGAG GAATCCTAAGGCATACCTA |
| CP7107P | GTGCGT GCTAGC AGTATAGTCAGAAATTCTGCA | GCGT CTCGAG GAAGCTAAGATTATAGCTACTTT |
| CP7108P | GTGCGT GCTAGC GCGGCCCTTTCCA | ACTCGCTA GCGGCCGC TTTATGTATATGGAACAGATAGG |
| CP7109P | GTGCGT CATATG GGACATTTTATTGATATTG | ACTCGCTA GCGGCCGC ATCATCAAGGTAGATAAAG |

(continued)

| Orf ID | N-terminus final primer | C-terminus final primer |
|---|---|---|
| CP7110P | GTGCGT CATATG GGTTATTGCTATGTAATTACA | GCGT CTCGAG TTCTGATTGGACTCCA |
| CP7127P | GTGCGT CATATG GTGGCTTTAACGATAGC | ACTCGCTA GCGGCCG GCAGCCATCGTATTC |
| CP7130P | GTGCGT CATATG TTCAATATGCGAGG | GCGT CTCGAG CTTCTTATTTGAACTTTG |
| CP7140P | GTGCGT CATATG ACAGCCGGAGCAGCT | GCGT CTCGAG AGCACCCTCAATTTCATTG |
| CP7182P | GTGCGT CATATG GGATATGTTTTCTATGTGATC | GCGT CTCGAG GCTACTAAATCGAATCGA |
| CP6262P | GTGCGT CATATG ATCCCTGGATTAAGTTCA | ACTCGCTA GCGGCCGC TTCACTGGGAGCTTGA |
| CP6269P | GTGCGT CATATG TACCAGGAGAATCTAAGAT | ACTCGCTA GCGGCCGC GATTTTCTTCTTCAGCTC |
| CP6296P | GTGCGT CATATG GAGGAGGTGTCTGAGTAT | ACTCGCTA GCGGCCGC ATGTTTCTTTTTACTCTTTCT |
| CP6419P | GTGCGT CATATG GCTCCAGTCCGTGTT | GCGT CTCGAG AAGTGTTCGTTGGAAGT |
| CP6601P | GTGCGT CATATG AATAAGCTACTCAATTTCGT | GCGT CTCGAG GAAAATCTGAATTCTTCCT |
| CP6639P | GTGCGT CATATG TTAAATTCAAGCAATTCA | GCGT CTCGAG AGGAACTAAAACCTCATCT |
| CP6664P | GTGCGT GCTAGC GTTTTATTTCATGCTCAA | ACTCGCTA GCGGCCGC CTTAGAAAGACTATTTTCTAAGTA |
| CP6696P | GTGCGT CATATG TGCGTGATAATGGG | GCGT CTCGAG ATTCATCTTCGTAAAGAAT |
| CP6757P | GTGCGT CATATG GCAGTTGGTGGCGT | ACTCGCTA GCGGCCGC CTGTCCCTCTGGAGC |
| CP6790P | GTGCGT GCTAGC AGTGAACACAAAAAATCA | ACTCGCTA GCGGCCGC CTTATCGTCGTTATCAATA |
| CP6814P | GTGCGT CATATG CATGACGCACTTCTAAG | GCGT CTCGAG TACAGCTGCGCGA |
| CP6834P | GTGCGT CATATG GTTATGGGAACCTATATCG | GCGT CTCGAG TACATTTGTATTGATTTCAG |
| CP6878P | GTGCGT CATATG AACGTCCCTGATTCC | GCGT CTCGAG GCTAGCGGCTCTTTC |
| CP6892P | GTGCGT CATATG CAGAAGCATCCTTCCT | ACTCGCTA GCGGCCGC TCCTCTTTAGGAAATGG |
| CP6909P | GTGCGT CATATG TCCTCTTTAGGAAATGG | GCGT CTCGAG CAGTGCCAAGTAGGGA |
| CP7015P | GTGCGT CATATG GCAGTACGATTAATTGTTG | GCGT CTCGAG TTTATTGTAGTCTATTTTATATTTC |
| CP7035P | GTGCGT GCTAGC AGCAGAAAAGACAATGA | GCGT CTCGAG ATTTTGAGTGTCTTGCA |
| CP7073P | GTGCGT CATATG ATTACCATAAATCACGTG | GCGT CTCGAG TATCCATCGACTTATAGC |
| CP7085P | GTGCGT GCTAGC TGTATTTTCCCTTACGTA | ACTCGCTA GCGGCCGC GGATTCTGCATACTCTG |
| CP7092P | GTGCGT CATATG TCTCCTCTTCCTAAAAAA | GCGT CTCGAG GGATTCATTACTGACCA |
| CP7093P | GTGCGT CATATG AAATACCGCTTCACG | GCGT CTCGAG ATTCTGTAGGGCTACGT |
| CP7094P | GTGCGT CATATG GTACACTTCTCTCATAACCC | GCGT CTCGAG TAAGTTTGTATTGCGGTAT |
| CP7132P | GTGCGT CATATG TTGTTATTAGGGACTTTAGGA | GCGT CTCGAG TTTCCCAACCGCA |

(continued)

| Orf ID | N-terminus final primer | C-terminus final primer |
|---|---|---|
| CP7133P | GTGCGT CATATG GCTGCGAATGCTC | GCGT CTCGAG TAATTTAATACTCTTTGAAGG |
| CP7177P | GTGCGT CATATG CCTACTCAAGTTAAAACAGA | GCGT CTCGAG AAGTTTATATTTCAGCACTT |
| CP7184P | GTGCGT GCTAGC CATATAGGATTTTGCCA | GCGT CTCGAG GTACTTAGCAAAGCGAT |
| CP7206P | GTGCGT GCTAGC AAGAAGCTATATCACCCTA | GCGT CTCGAG CACACCGAGGAAAC |
| CP7222P | GTGCGT CATATG GTAGTTTCAGAAGAAAAAGTC | GCGT CTCGAG ACGTATGCGCAACTG |
| CP7223P | GTGCGT CATATG GAAGTATTAGACCGCTCT | GCGT CTCGAG CGAGAAAAAGCTTCC |
| CP7224P | GTGCGT CATATG ATGAAGAAAATTCGAAA | ACTCGCTA GCGGCCGC TAAGCATTCACAAATGA |
| CP7225P | GTGCGT CATATG CATATTTTGCTTGATCGT | GCGT CTCGAG TCTTTTAACTAAATCTTGTTCTT |
| CP7303P | GTGCGT CATATG CTTGTCTATTGTTTTGATCC | GCGT CTCGAG AAAATATACGGAACTCGC |
| CP7304P | GTGCGT GCTAGC GAAGTTTATAGTTTTTCCC | GCGT CTCGAG TTTTTGATTCCTTAAGAAG |
| CP7305P | GTGCGT CATATG GAAGTTTATAGTTTTCACCCT | GCGT CTCGAG ACTCCTTGAGAAGGGAA |
| CP7307P | GTGCGT CATATG CTTAATCATGCTAAAAAGC | ACTCGCTA GCGGCCGC CTCTTTTATTTTAGGAAGCT |
| CP7342P | GTGCGT CATATG AAAAAAAAATTTATTTTCTACT | ACTCGCTA GCGGCCGC CACACTCTGTTCTTCTG |
| CP7347P | GTGCGT CATATG TTTTCTAAGGATTTGACTAA | GCGT CTCGAG CGAAGCAGAAGTCGT |
| CP7353P | GTGCGT CATATG AATATGCCTGTTCCTTCT | GCGT CTCGAG GGGGCGTAGGTTGTA |
| CP7193P | GTGCGT CATATG TGTTCCCTGGATCCT | ACTCGCTA GCGGCCGC AGTTATCACTATATCCACAAG |
| CP7248P | GTGCGT GCTAGC CTTGAACATTCTAAACAAGAT | GCGT CTCGAG ACGTAGTTTAAGAGCAGACT |
| CP7261P | GTGCGT CATATG TGTCTATCTGCCTACATAG | GCGT CTCGAG TTTTGATGCTTCTTTCA |
| CP7280P | GTGCGT CATATG GACCAGAAAATTGAAAA | GCGT CTCGAG AGAGGTCTTCTGAGTGC |
| CP7302P | GTGCGT CATATG AATTTCCATTGTAGTGTAGT | GCGT CTCGAG GAACAGTTCGATTTGTG |
| CP7306P | GTGCGT CATATG CTTCCTTTATCAGGGCA | ACTCGCTA GCGGCCGC TTCTTCAGGTTTCAGG |
| CP7367P | GTGCGT GCTAGC CGTTATGCCGAGGTC | GCGT CTCGAG TTCGTGCATTTGGTG |
| CP7408P | GTGCGT CATATG TTGAAAATCCAGAAAAA | GCGT CTCGAG ATTCATTTTCGGAAGAG |
| CP7409P | GTGCGT CATATG AGACGTTATCTTTTCATGGT | GCGT CTCGAG CCCTTTGCTCTTTACATAG |
| CP6733P | GTGCGT ACTAGT TGTCACCTACAGTCACTAG | GCGT CTCGAG GAATCGGAGTTTGGTA |
| CP6728P | GTGCGT ACTAGT AAGTCCTCTGTCTCTTGG | GCGT CTCGAG GAAACAAAACTTAGAGCCC |

TABLE III - Proteins with best results in FACS analysis

| cp number | Molecular Weight (kDa) | | Fusion type |
|---|---|---|---|
| | Theoretical | Western Blot | |
| 6260 | 97.5 | 94; 70 | GST |
| 6270 | 87.5 | - | GST |
| 6272 | 78.0 | 90 | GST |
| 6273 | 58.6 | 74; 64; 50 | GST |
| 6296 | 31.1 | - | GST |
| 6390 | 88.9 | 102 | GST |
| 6456 | 42.5 | 89; 67,45 | GST |
| 6466 | 57.5 | 59; 56 | His |
| 6467 | 59.0 | 67 | GST |
| 6552 | 28.4 | 50; 27 | GST |
| 6576 | 86.0 | 79; 70; 62; 45 | GST |
| 6577 | 17.3 | 12 | GST |
| 6602 | 43.4 | 53; 42; 34 | GST |
| 6664 | 54.5 | 104; 45 | GST |
| 6696 | 47.9 | 95; 53 | GST |
| 6727 | 130.0-142.9 | 123; 61; 39 | His |
| 6729 | 94.8 | multiple bands | GST |
| 6731 | 95.5 | 97 | GST |
| 6733 | 97.1 | 104 | His |
| 6736 | 100.1 | 98; 93; 66; 60 | GST |
| 6737 | 101.2 | multiple bands | GST |
| 6751 | 100.2 | 95; 71 | GST |
| 6752 | 102.1 | 97; 48 | His |
| 6767 | 29.1 | 28 | GST |
| 6784 | 32.9 | 35 | GST |
| 6790 | 71.3 | multiple bands | His |
| 6802 | 29.7 | - | GST |
| 6814 | 29.6 | 28 | GST |
| 6830 | 177.4 | 174; 91; 13 | GST |
| 6849 | 57.3 | multiple bands | GST |
| 6850 | 7.4-9.4 | 61; 14; 8 | GST |
| 6854 | 42.2 | - | GST |
| 6878 | 40.4 | - | GST |
| 6900 | 28.0 | - | GST |
| 6960 | 25.6 | 75; 35 | GST |
| 6968 | 34.6 | 83; 53; 35 | GST |

(continued)

| cp number | Molecular Weight (kDa) | | Fusion type |
|---|---|---|---|
| | Theoretical | Western Blot | |
| 6998 | 39.3 | multiple bands | GST |
| 7033 | 68.2 | multiple bands | GST |
| 7101 | 113 | 105 | GST |
| 7102 | 63.4 | - | GST |
| 7105 | 29.2 | 30 | GST |
| 7106 | 39.5 | 72;46 | GST |
| 7107 | 71.4 | 67; 31 | His |
| 7108 | 35.9 | 35 | GST |
| 7111 | 46.1 | 51 | GST |
| 7132 | 17.9 | 57; 47; 17 | His |
| 7140 | 36.2-29.8 | 50; 38; 34 | GST |
| 7170 | 34.4 | 77; 33 | GST |
| 7224 | 39.4 | 40 | GST |
| 7287 | 167.3 | 180 | GST |
| 7306 | 50.1 | 50 | GST |

**TABLE IV - FACS-positive proteins not found in *C.trachomatis***

| | |
|---|---|
| cp7105 | cp6390 |
| cp7106 | cp6784 |
| cp7107 | cp6296 |
| cp7108 | |

**TABLE V - Proteins identified by MALDI-TOF following 2D electrophoresis**

| | | |
|---|---|---|
| cp6270 | cp6733 | cp6900 |
| cp6552 | cp6736 | cp6960 |
| cp6576 | cp6737 | cp6998 |
| cp6577 | cp6752 | cp7033 |
| cp6602 | cp6767 | cp7108 |
| cp6664 | cp6784 | cp7111 |
| cp6727 | cp6790 | cp7170 |
| cp6728 | cp6830 | cp7287 |
| cp6729 | cp6849 | cp7306 |

## Claims

1. A protein comprising an amino acid sequence selected from the group consisting of SEQ IDs 111, 1, 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93, 95, 97, 99, 101, 103, 105, 107, 109, 113, 115, 117, 119, 121, 123, 125, 127, 129, 131, 133, 135, 137, 139, 141, 143, 145, 147, 149, 151, 153, 155, 157, 159, 161, 163, 165, 167, 169, 171, 173, 175, 177, 179, 181, 183, 185, 187, 189, 191, 193, 195, 197, 199, 201, 203, 205, 207, 209, 211, 213, 215, 217, 219, 221, 223, 225, 227, 229, 231, 233, 235, 237, 239, 241, 243, 245, 247, 249, 251, 253, 255, 257, 259, 261, 263, 265, 267, 269, 271, 273, 275, 277, 279, 281, 283, 285, 287, 289, 291, 293, 295, 297, 299, 301, 303, 305, 307, 309, 311, 313, 315, 317, 319, 321, 323, 325, 327, 329, 331, 333, 335, 337, 339, 341, 343, 345, 347, 349, 351, 353, 355, 357, 359, 361, 363, 365, 367, 369, 371, 373, 375, & 377.

2. A protein having 50% or greater sequence identity to a protein according to claim 1.

3. A protein comprising a fragment of an amino acid sequence selected from the group consisting of SEQ IDs 111, 1, 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93, 95, 97, 99, 101, 103, 105, 107, 109, 113, 115, 117, 119, 121, 123, 125, 127, 129, 131, 133, 135, 137, 139, 141, 143, 145, 147, 149, 151, 153, 155, 157, 159, 161, 163, 165, 167, 169, 171, 173, 175, 177, 179, 181, 183, 185, 187, 189, 191, 193, 195, 197, 199, 201, 203, 205, 207, 209, 211, 213, 215, 217, 219, 221, 223, 225, 227, 229, 231, 233, 235, 237, 239, 241, 243, 245, 247, 249, 251, 253, 255, 257, 259, 261, 263, 265, 267, 269, 271, 273, 275, 277, 279, 281, 283, 285, 287, 289, 291, 293, 295, 297, 299, 301, 303, 305, 307, 309, 311, 313, 315, 317, 319, 321, 323, 325, 327, 329, 331, 333, 335, 337, 339, 341, 343, 345, 347, 349, 351, 353, 355, 357, 359, 361, 363, 365, 367, 369, 371, 373, 375, & 377.

4. A nucleic acid molecule which encodes a protein according to any one of claims 1 to 3.

5. A nucleic acid molecule according to claim 4, comprising a nucleotide sequence selected from the group consisting of SEQ IDs 112, 2, 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, 100, 102, 104, 106, 108, 110, 114, 116, 118, 120, 122, 124, 126, 128, 130, 132, 134, 136, 138, 140, 142, 144, 146, 148, 150, 152, 154, 156, 158, 160, 162, 164, 166, 168, 170, 172, 174, 176, 178, 180, 182, 184, 186, 188, 190, 192, 194, 196, 198, 200, 202, 204, 206, 208, 210, 212, 214, 216, 218, 220, 222, 224, 226, 228, 230, 232, 234, 236, 238, 240, 242, 244, 246, 248, 250, 252, 254, 256, 258, 260, 262, 264, 266, 268, 270, 272, 274, 276, 278, 280, 282, 284, 286, 288, 290, 292, 294, 296, 298, 300, 302, 304, 306, 308, 310, 312, 314, 316, 318, 320, 322, 324, 326, 328, 330, 332, 334, 336, 338, 340, 342, 344, 346, 348, 350, 352, 354, 356, 358, 360, 362, 364, 366, 368, 370, 372, 374, 376, & 378.

6. A nucleic acid molecule comprising a fragment of a nucleotide sequence selected from the group consisting of SEQ IDs 112, 2, 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, 98, 100, 102, 104, 106, 108, 110, 114, 116, 118, 120, 122, 124, 126, 128, 130, 132, 134, 136, 138, 140, 142, 144, 146, 148, 150, 152, 154, 156, 158, 160, 162, 164, 166, 168, 170, 172, 174, 176, 178, 180, 182, 184, 186, 188, 190, 192, 194, 196, 198, 200, 202, 204, 206, 208, 210, 212, 214, 216, 218, 220, 222, 224, 226, 228, 230, 232, 234, 236, 238, 240, 242, 244, 246, 248, 250, 252, 254, 256, 258, 260, 262, 264, 266, 268, 270, 272, 274, 276, 278, 280, 282, 284, 286, 288, 290, 292, 294, 296, 298, 300, 302, 304, 306, 308, 310, 312, 314, 316, 318, 320, 322, 324, 326, 328, 330, 332, 334, 336, 338, 340, 342, 344, 346, 348, 350, 352, 354, 356, 358, 360, 362, 364, 366, 368, 370, 372, 374, 376, & 378.

7. A nucleic acid molecule comprising a nucleotide sequence complementary to a nucleic acid molecule according to any one of claims 4 to 6.

8. A nucleic acid molecule comprising a nucleotide sequences having 50% or greater sequence identity to a nucleic acid molecule according to any one of claims 4 to 7.

9. A nucleic acid molecule which can hybridise to a nucleic acid molecule according to any one of claims 4 to 8 under high stringency conditions.

10. A composition comprising a protein or a nucleic acid molecule according to any preceding claim.

11. A composition according to claim 10 being a vaccine composition.

**12.** A composition according to claim 10 or claim 11 for use as a pharmaceutical.

**13.** The use of a composition according to claim 10 in the manufacture of a medicament for the treatment or prevention of infection due to *Chlamydia* bacteria, particularly *Chlamydia pneumoniae.*

## FIGURE 1

**FIG. 1A**

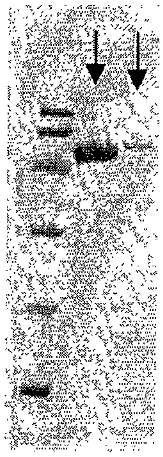

**FIG. 1B**

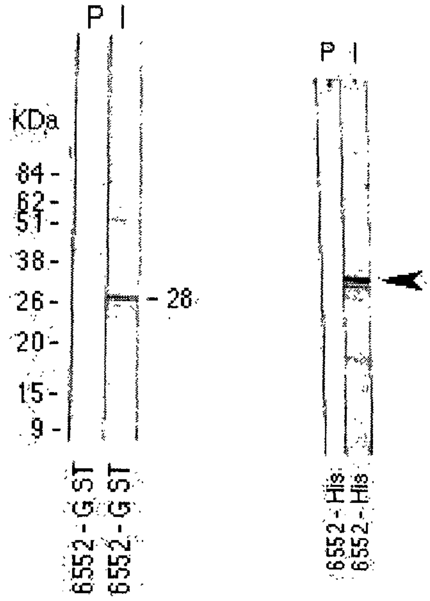


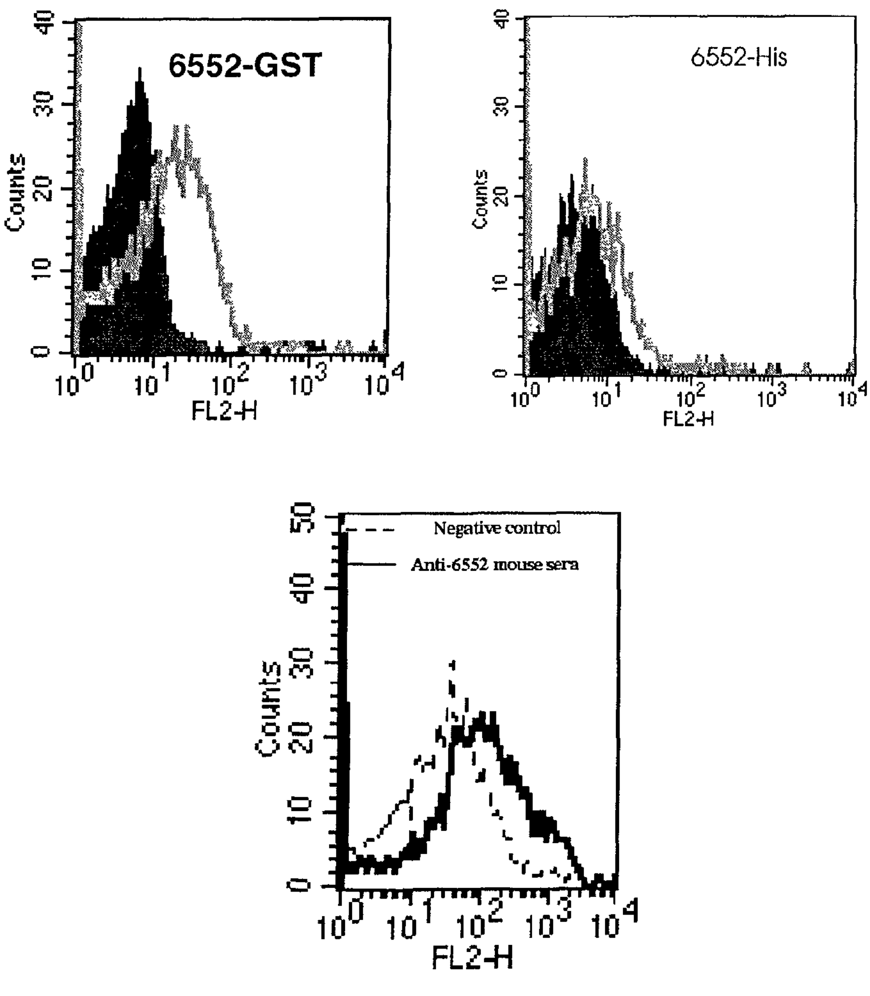


**FIG. 1C**

## FIGURE 2

**FIG. 2A**

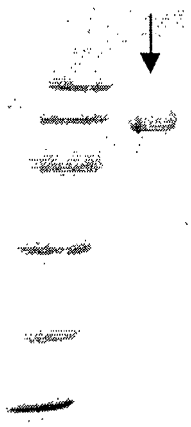

**FIG. 2B**

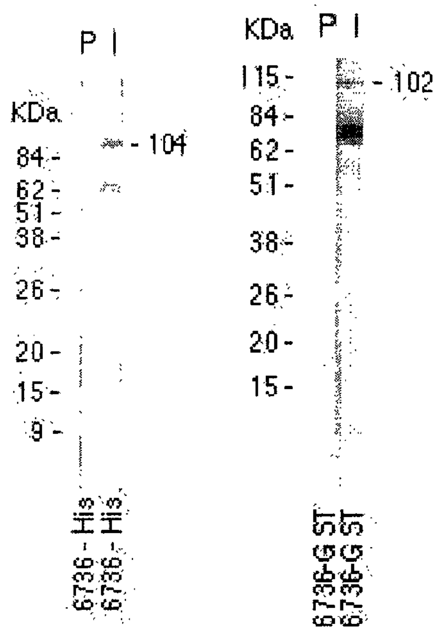


**FIG. 2C**

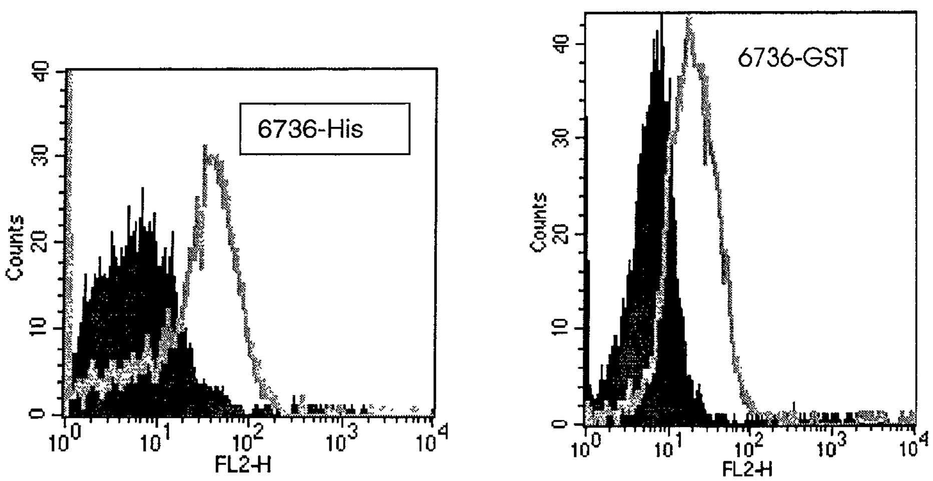

# FIGURE 3

**FIG. 3A**

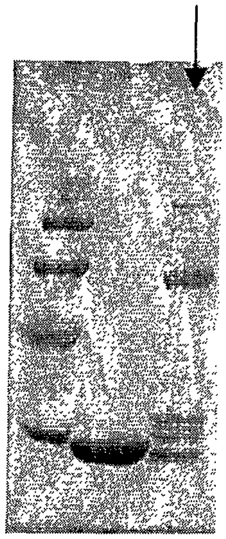

**FIG. 3B**

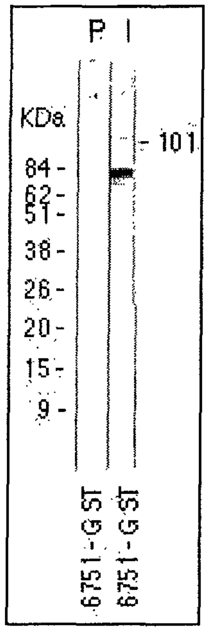


**FIG. 3C**

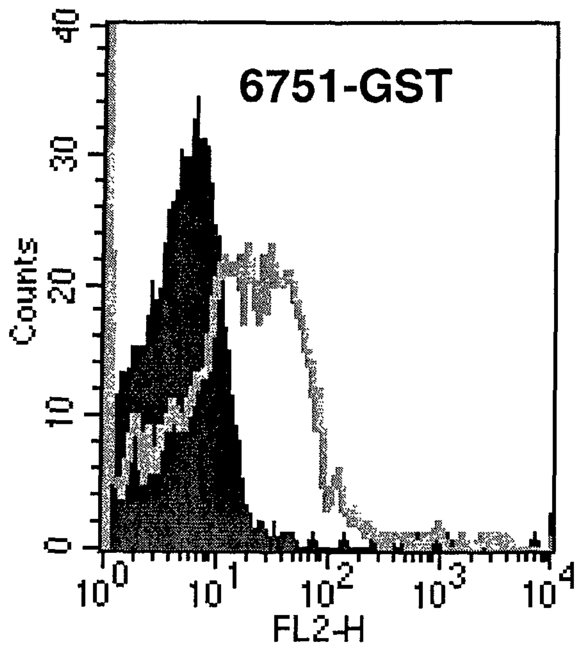

# FIGURE 4

**FIG. 4A**

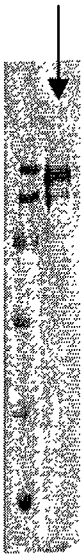

**FIG. 4B**

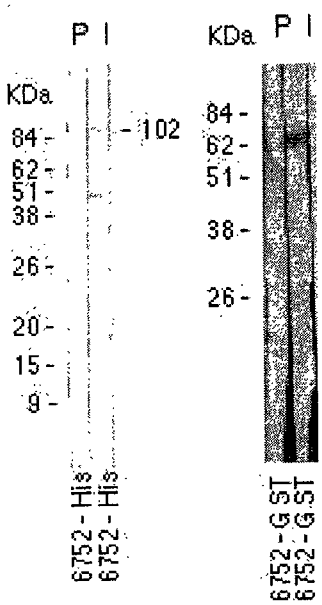


**FIG. 4C**

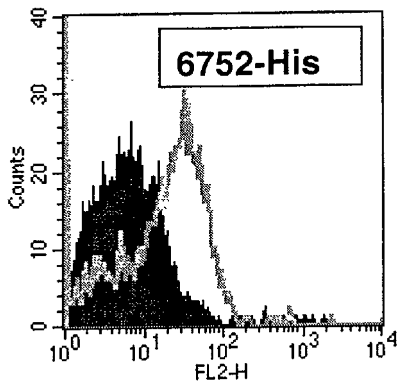

# FIGURE 5

**FIG. 5A**

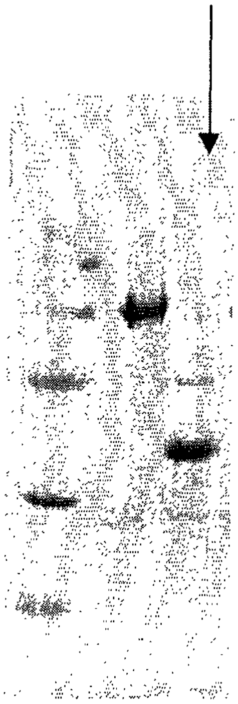

**FIG. 5B**

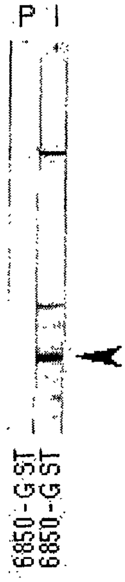


**FIG. 5C**

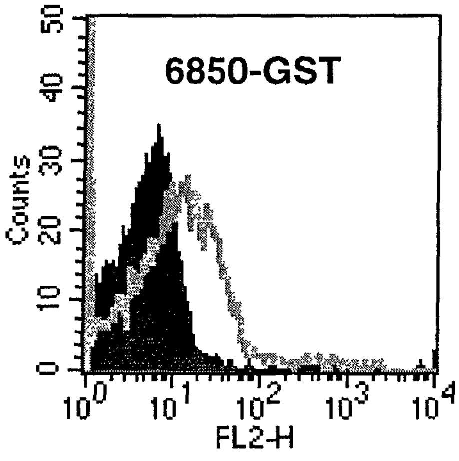

## FIGURE 6

**FIG. 6A**

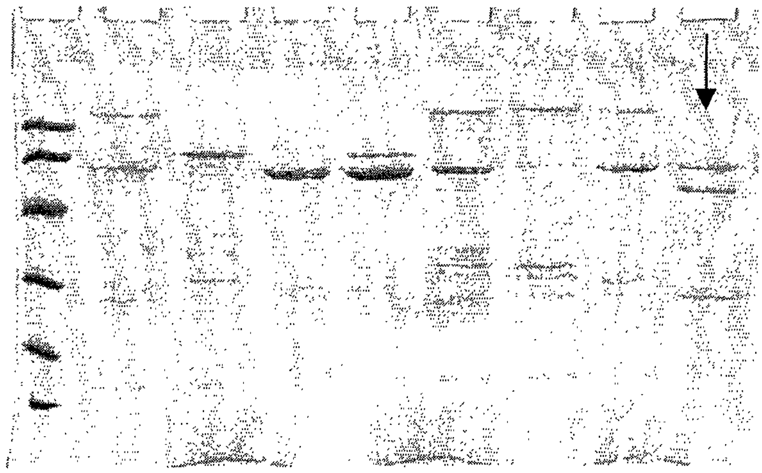

**FIG. 6B**

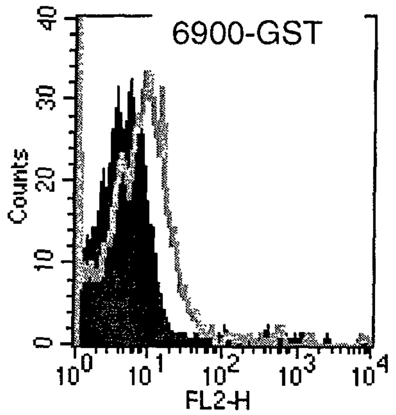


**FIG. 6C**

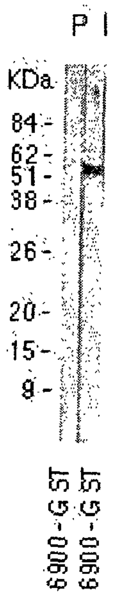

# FIGURE 7

*FIG. 7A*

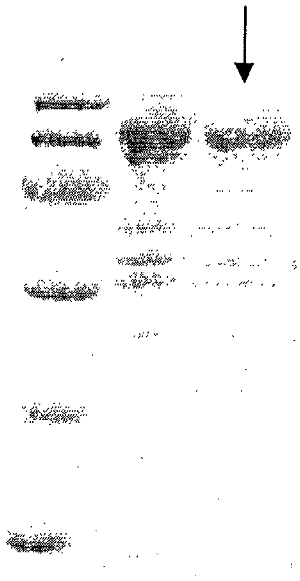

*FIG. 7B*

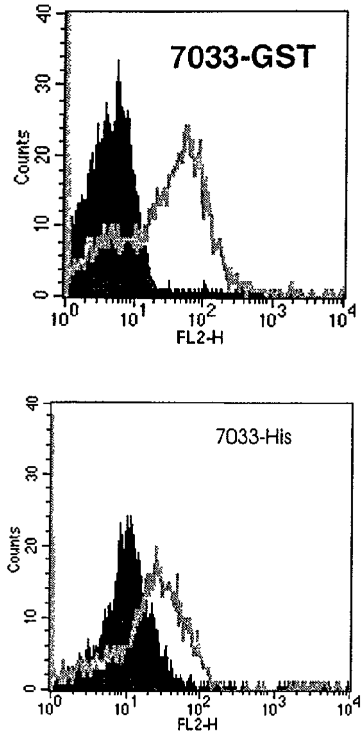


*FIG. 7C*

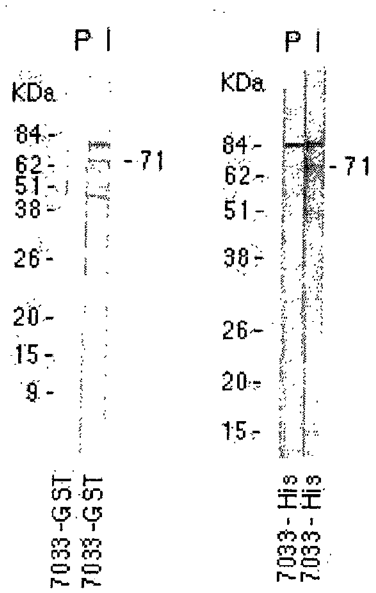

# FIGURE 8

FIG. 8A

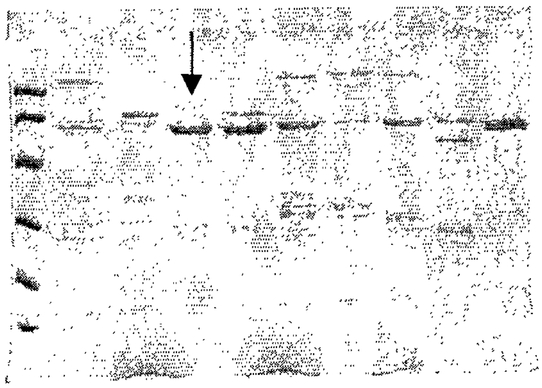

FIG. 8B

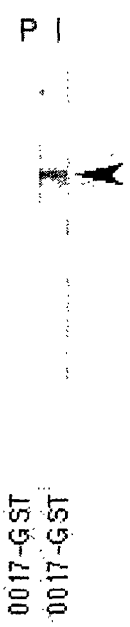


FIG. 8C

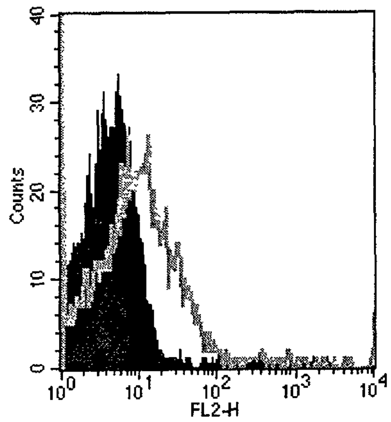

# FIGURE 9

FIG. 9A

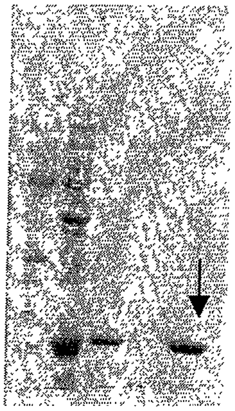

FIG. 9B

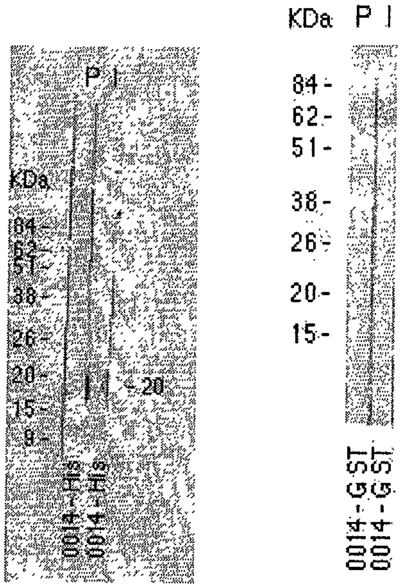


FIG. 9C

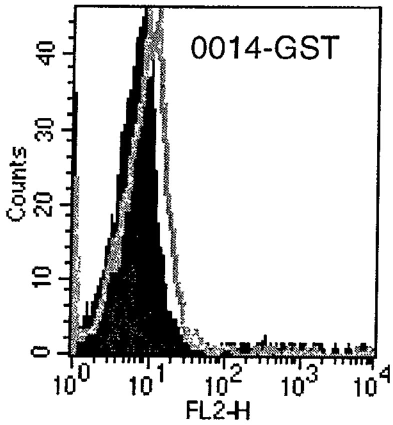

## FIGURE 10

**FIG. 10A**

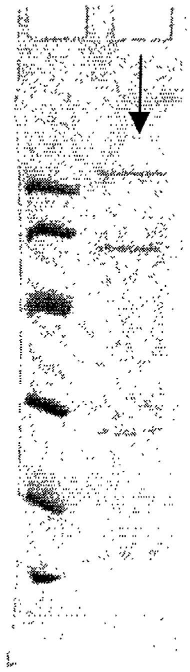

**FIG. 10B**

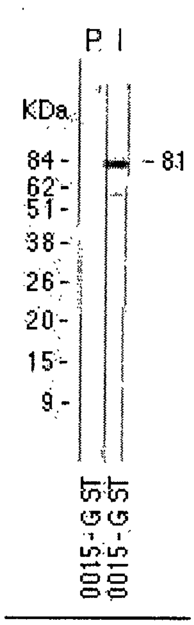

## FIGURE 11

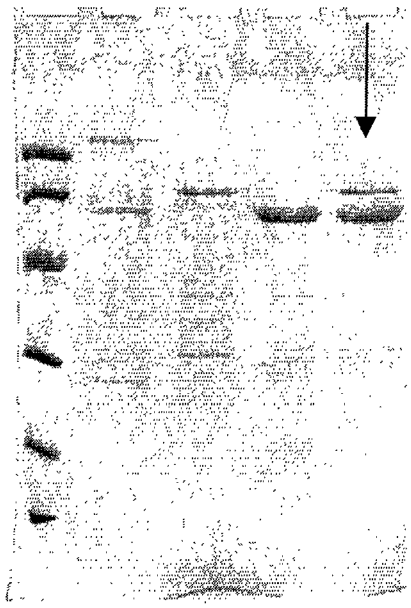

**FIG. 11A**

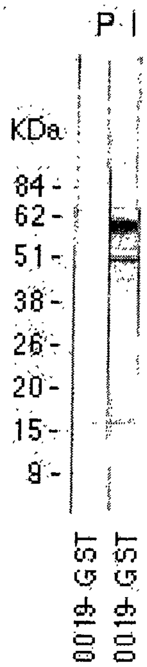


**FIG. 11B**

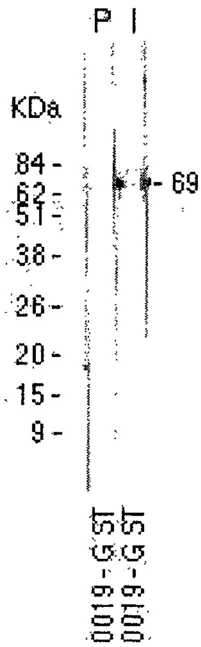


**FIG. 11C**

# FIGURE 12

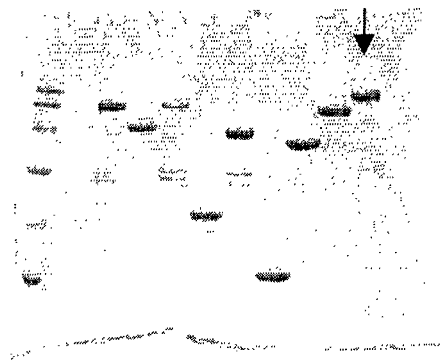

FIG. 12A

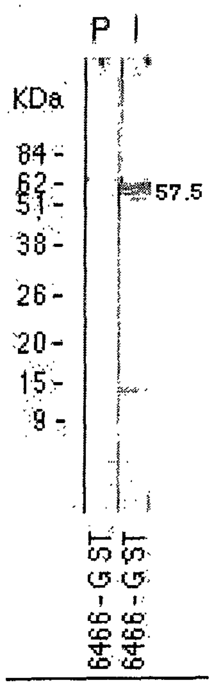


FIG. 12B

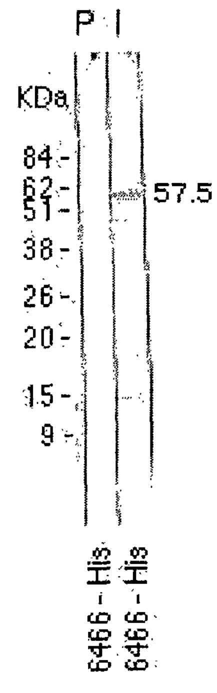


FIG. 12C

# FIGURE 13

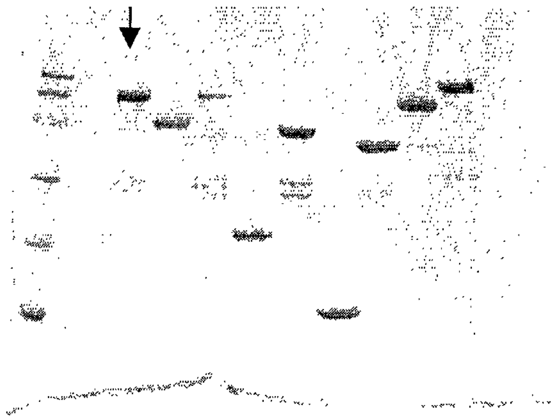

FIG. 13A

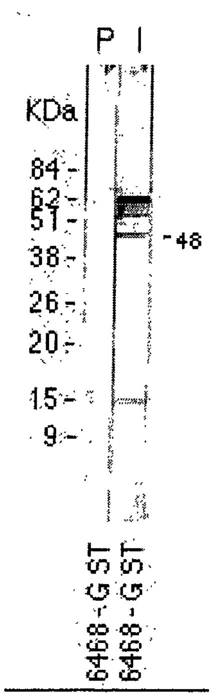


FIG. 13B

## FIGURE 14

FIG. 14A

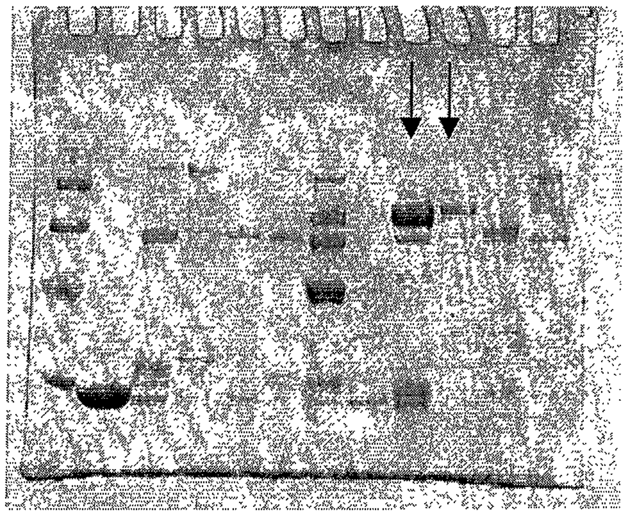

FIG. 14B

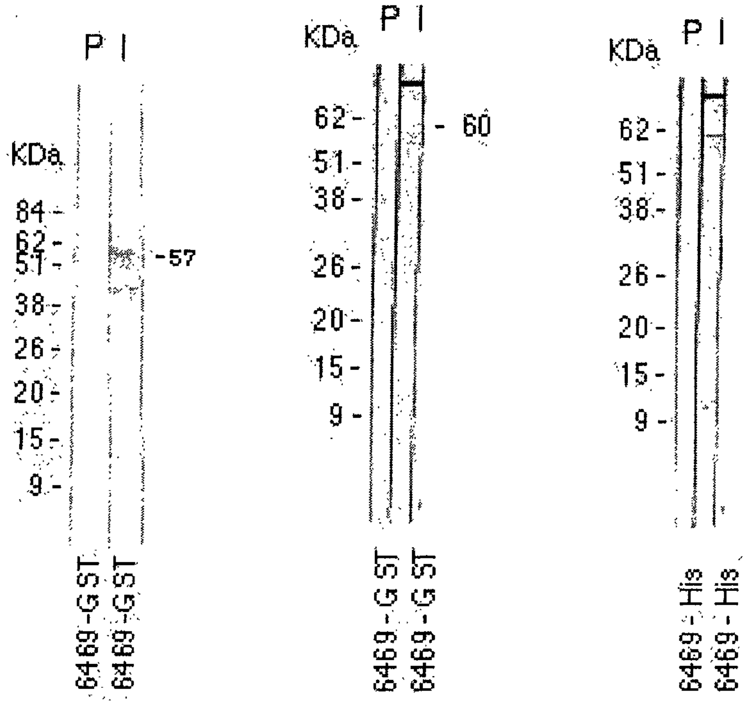

# FIGURE 15

***Fig. 15A***

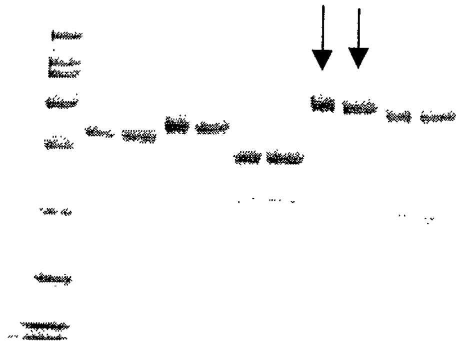

***Fig. 15B***

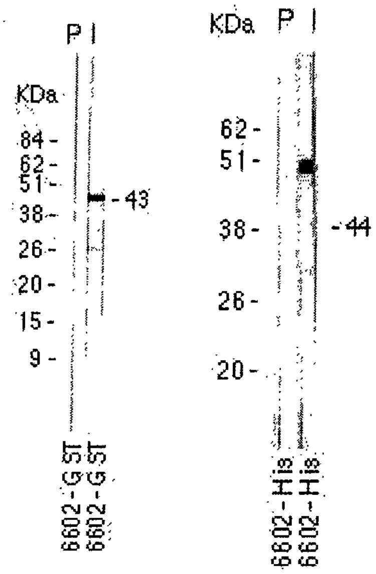


***Fig. 15C***

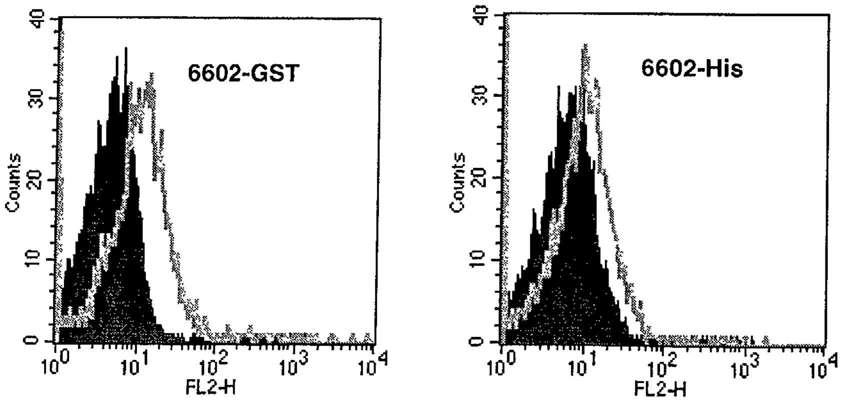

## FIGURE 16

**FIG. 16A**

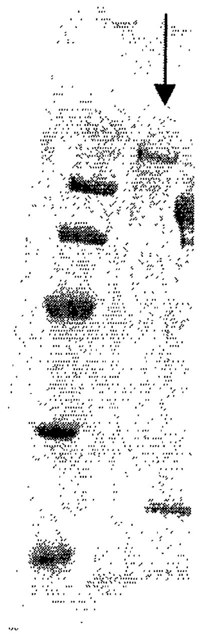

**FIG. 16B**

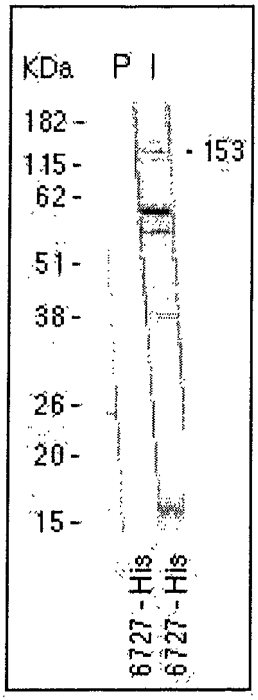


**FIG. 16C**

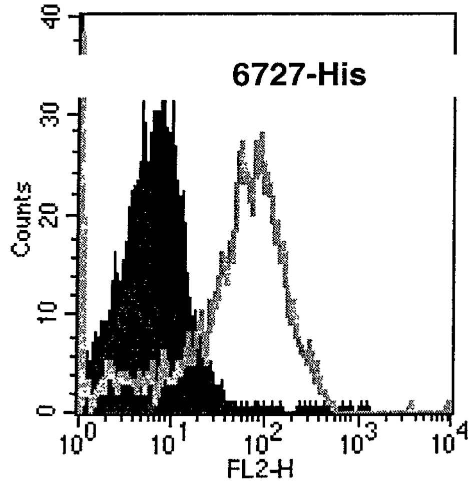

***FIGURE 17***

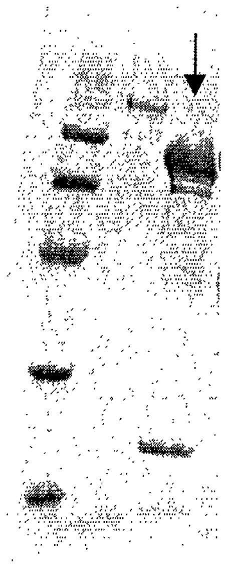

***Fig. 17A***

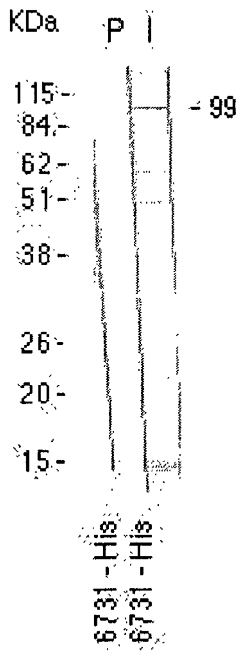


***Fig. 17B***

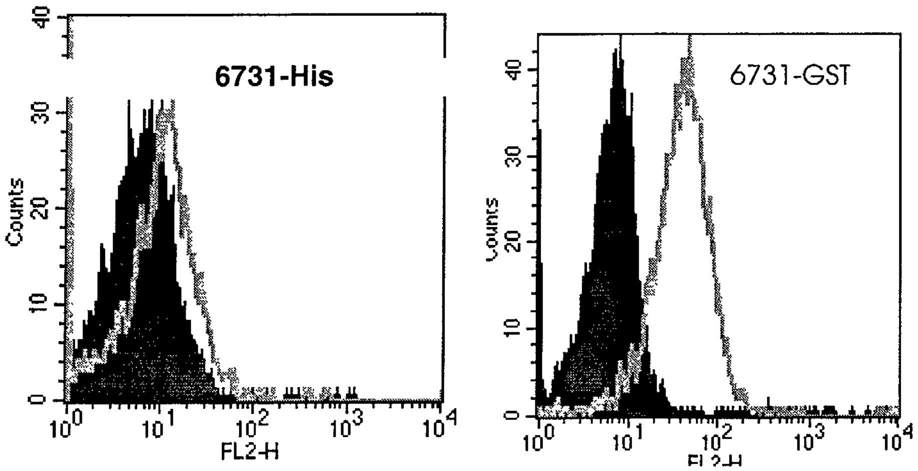


***Fig. 17C***

# ***FIGURE 18***

***FIG. 18A***

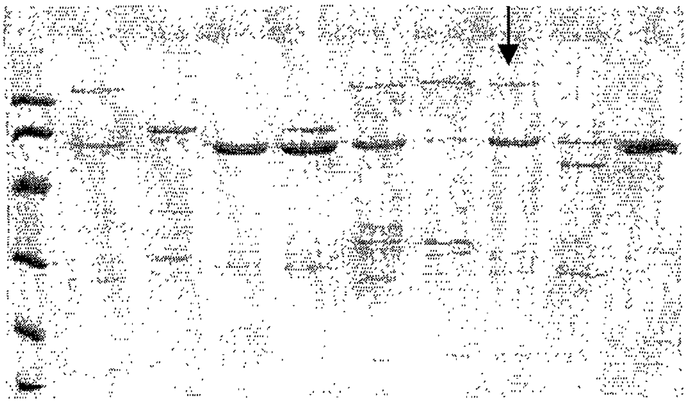

***FIG. 18B***

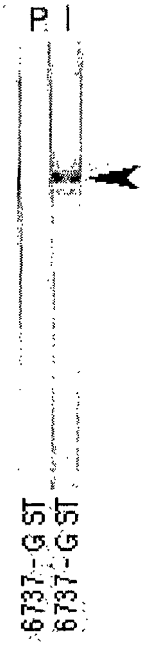


***FIG. 18C***

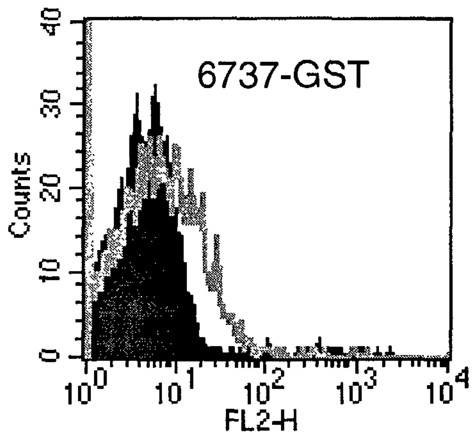

## FIGURE 19

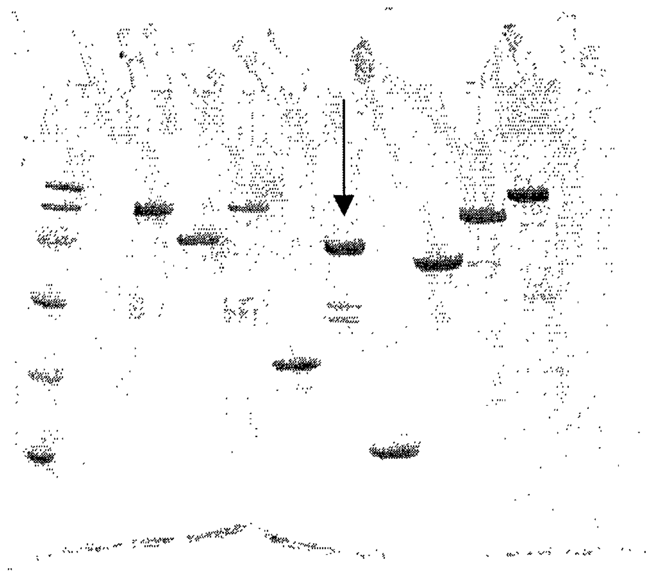

FIG. 19A

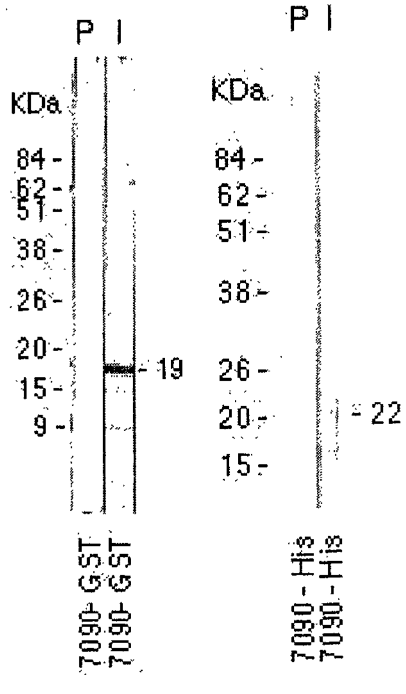


FIG. 19B

**FIGURE 20**

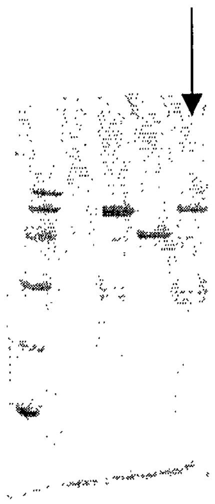

**FIG. 20A**

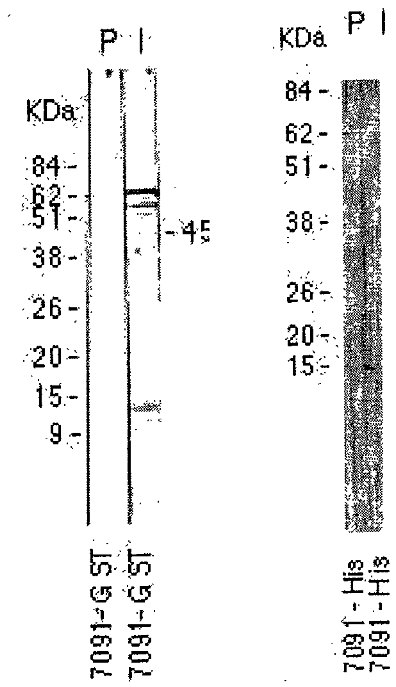


**FIG. 20B**

## FIGURE 21

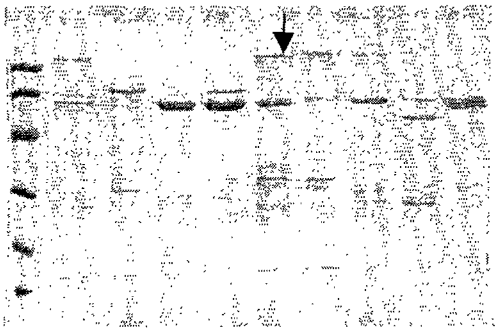

**FIG. 21A**

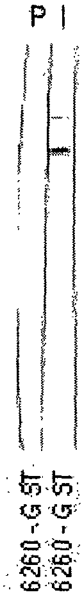


**FIG. 21B**

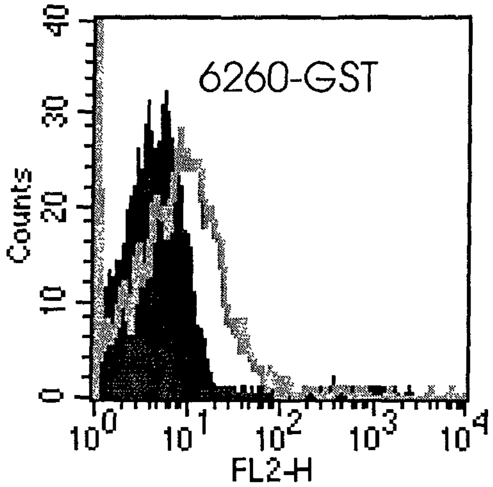


**FIG. 21C**

# FIGURE 22

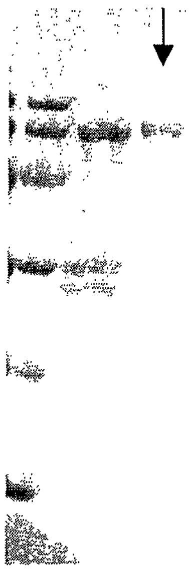

FIG. 22A

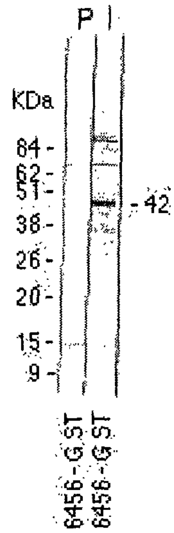


FIG. 22B

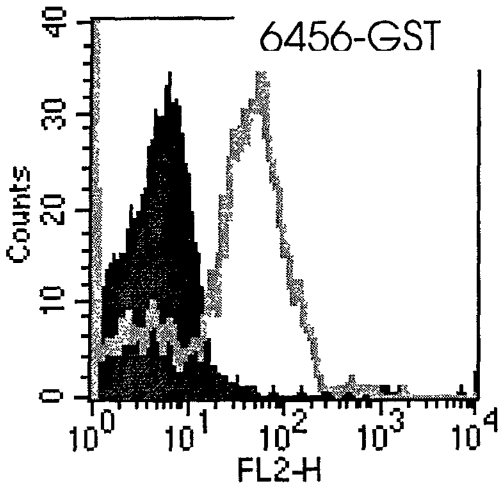


FIG. 22C

## FIGURE 23

FIG. 23A

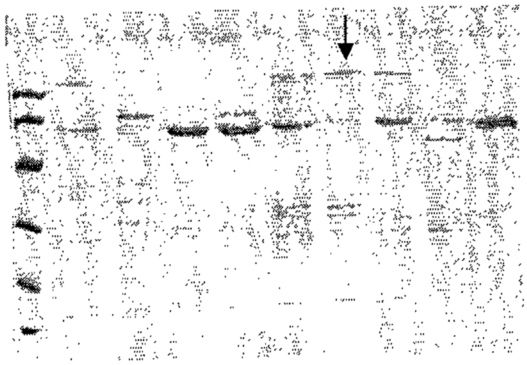

FIG. 23B

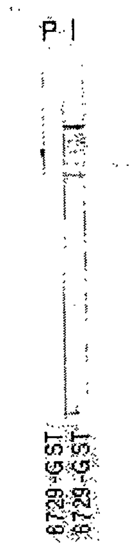


FIG. 23C

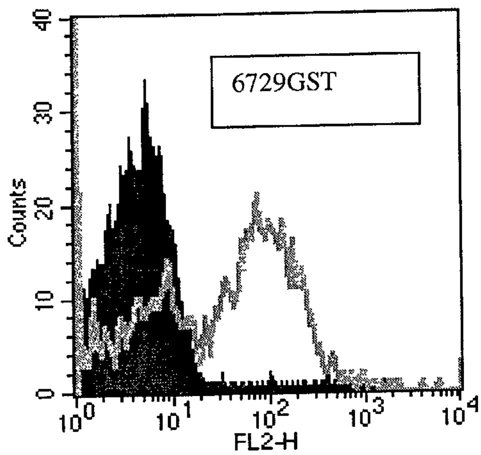

# FIGURE 24

FIG. 24A

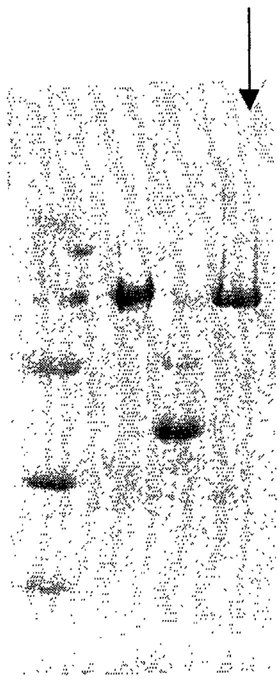

FIG. 24B

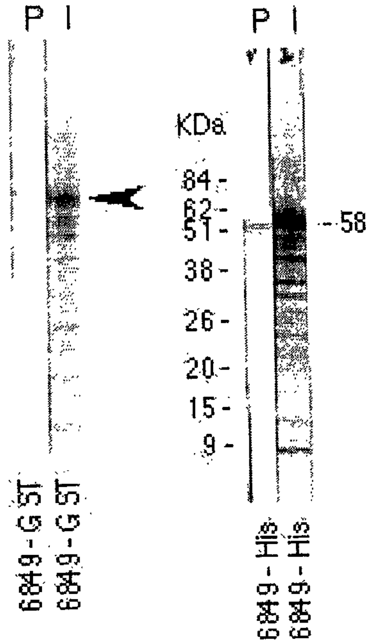


FIG. 24C

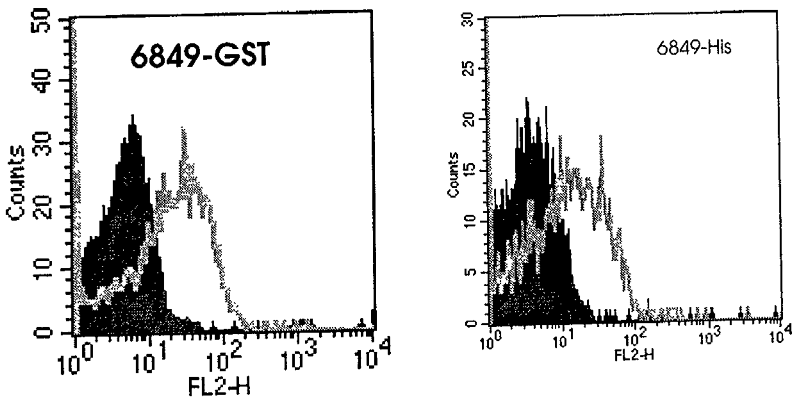

# *FIGURE 25*

**FIG. 25A**

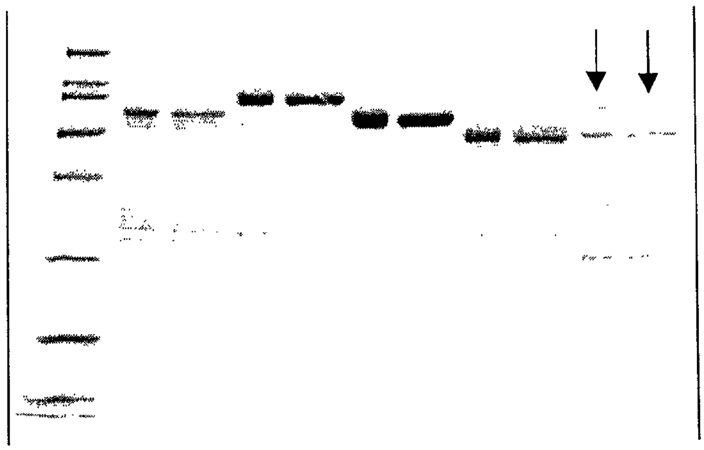

**FIG. 25B**

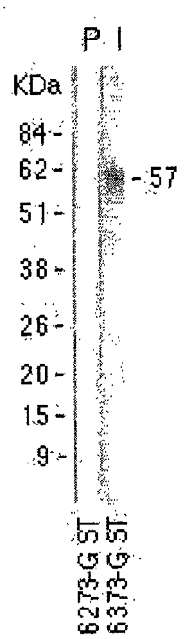


**FIG. 25C**

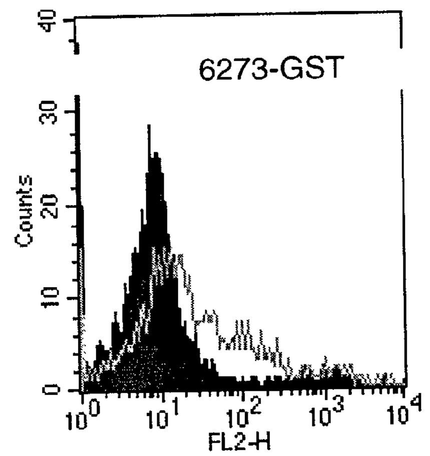

**FIGURE 26**

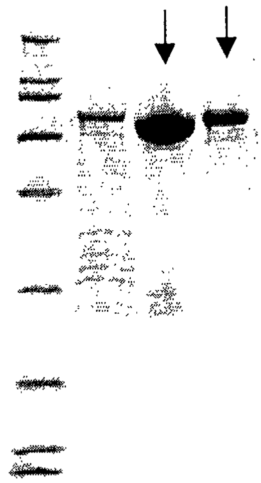

FIG. 26A

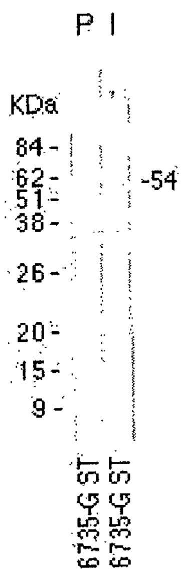


FIG. 26B

# FIGURE 27

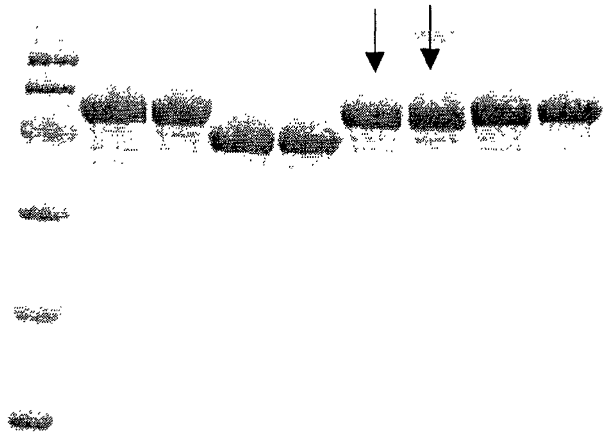

FIG. 27A

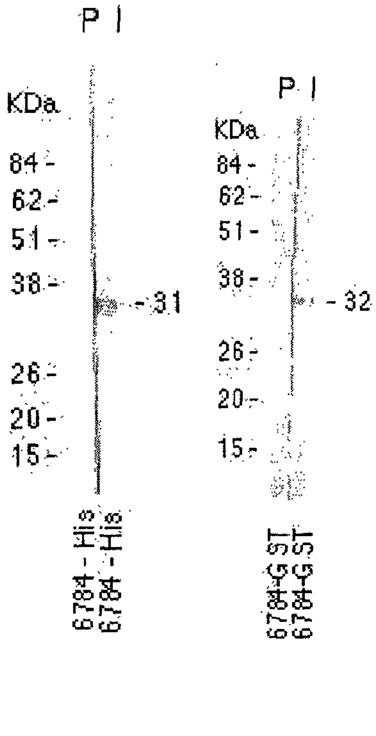


FIG. 27B

FIG. 27C

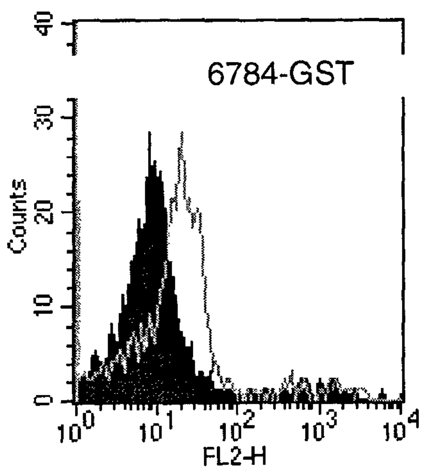

# FIGURE 28

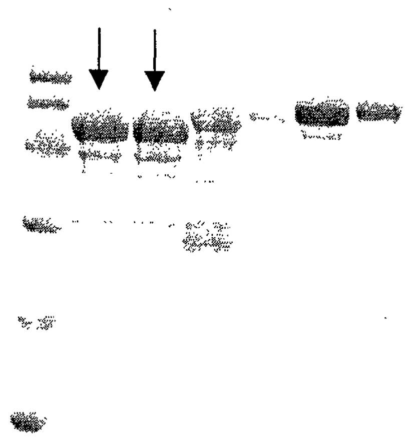

FIG. 28A

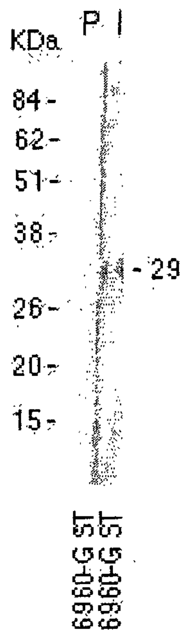


FIG. 28B

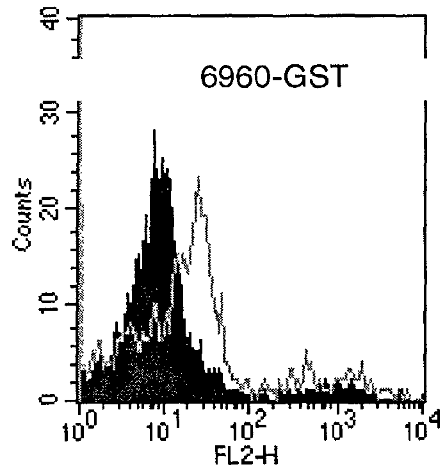


FIG. 28C

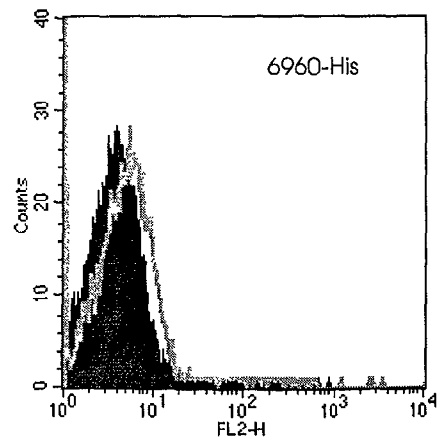

## FIGURE 29

**FIG. 29A**

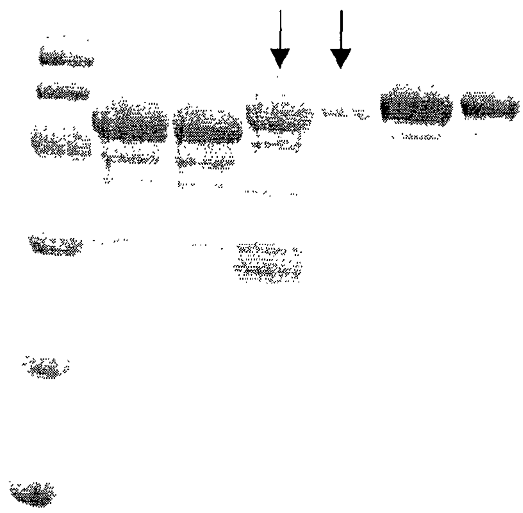

**FIG. 29B**

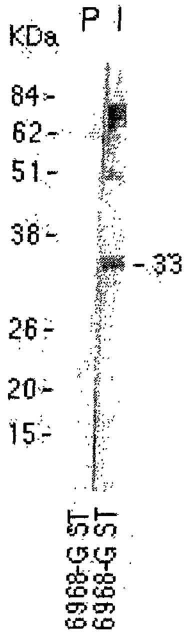


**FIG. 29C**

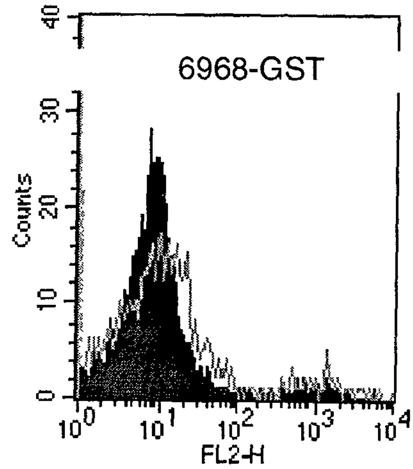

## FIGURE 30

**FIG. 30A**

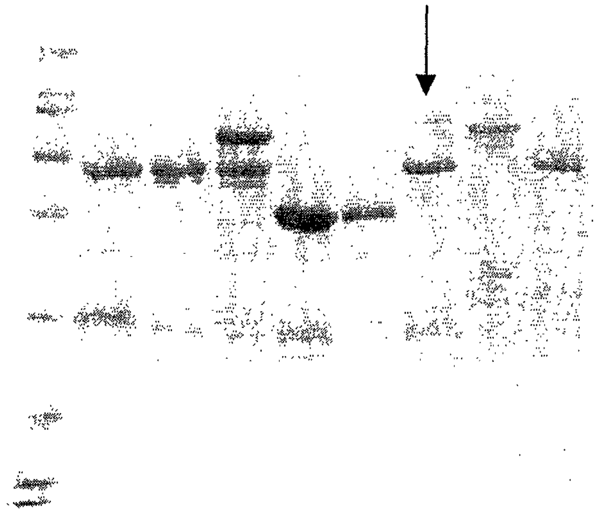

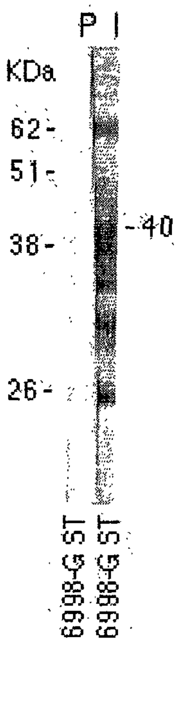


**FIG. 30B**

**FIG. 30C**

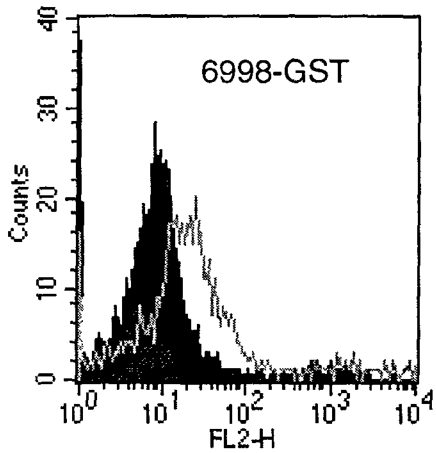

# FIGURE 31

**FIG. 31A**

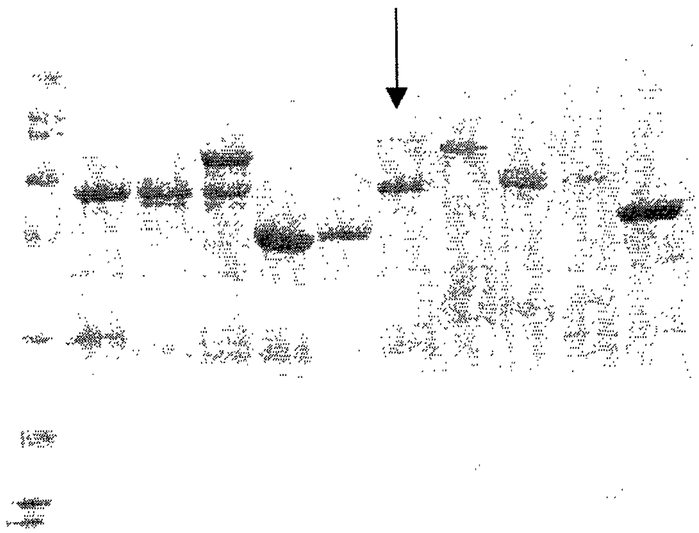

**FIG. 31B**

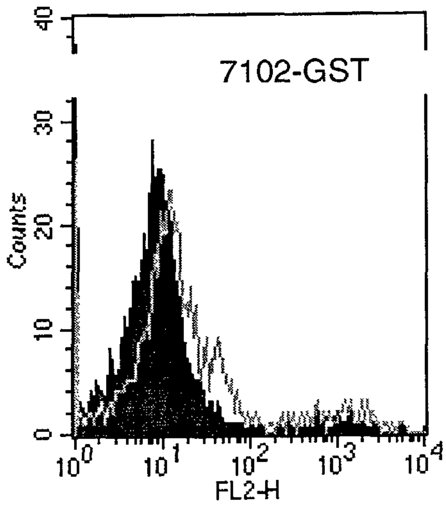

# FIGURE 32

FIG. 32A

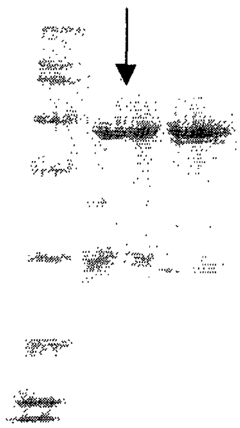

FIG. 32B

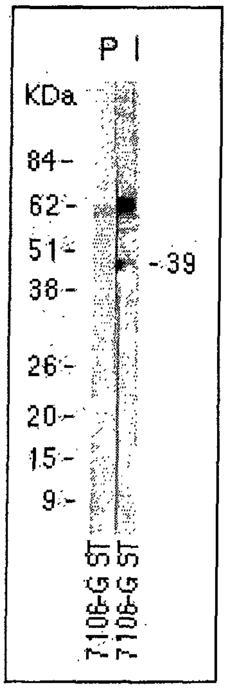


FIG. 32C

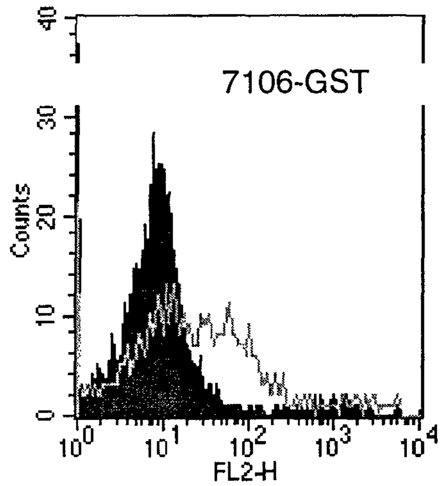

## FIGURE 33

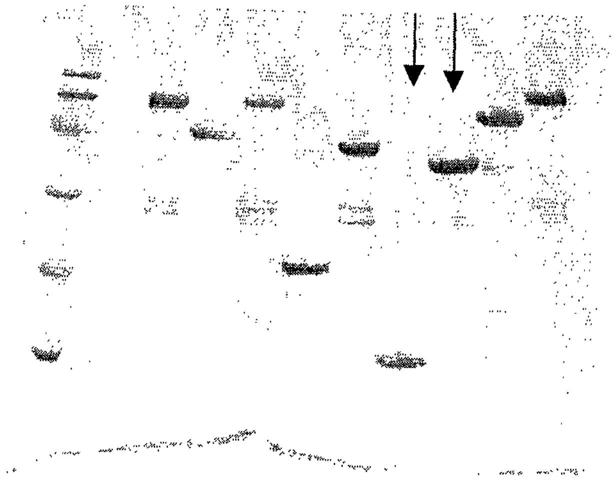

FIG. 33A

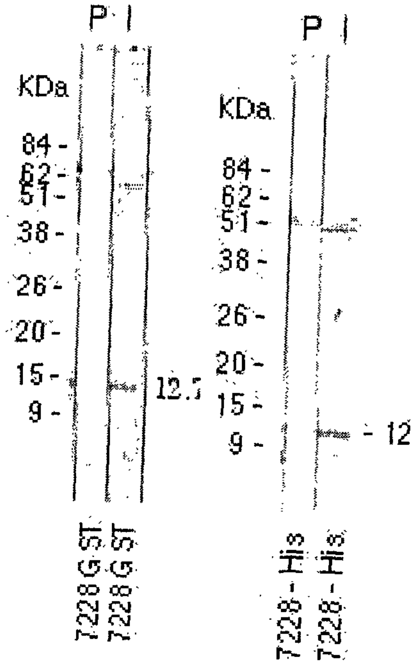


FIG. 33B

# FIGURE 34

FIG. 34A

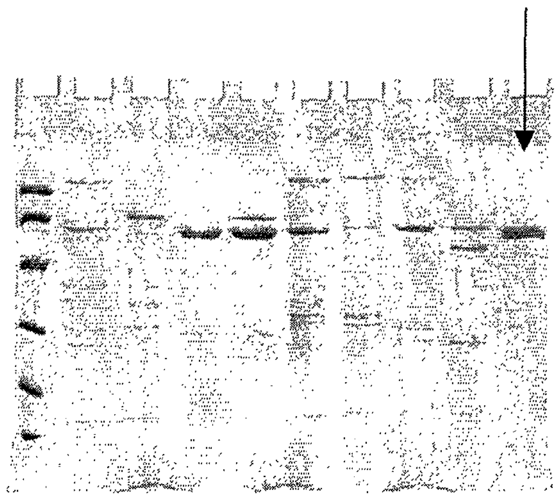

FIG. 34B

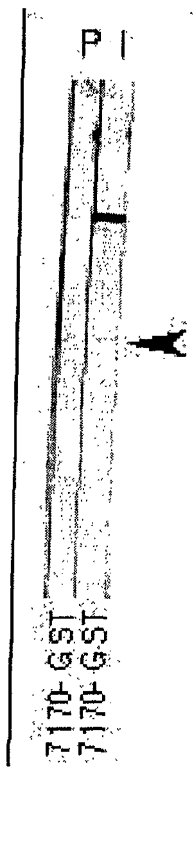


FIG. 34C

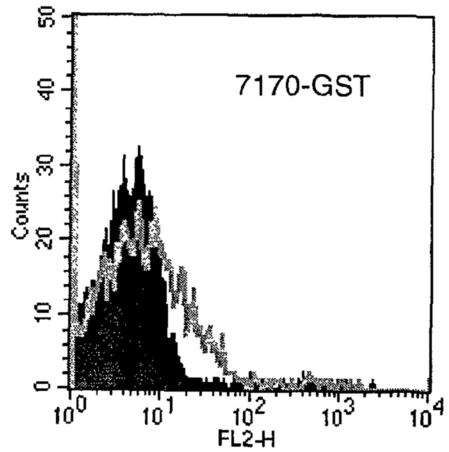

## FIGURE 35

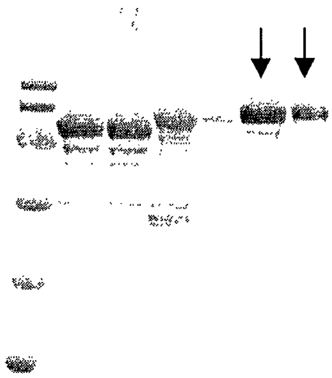

FIG. 35A

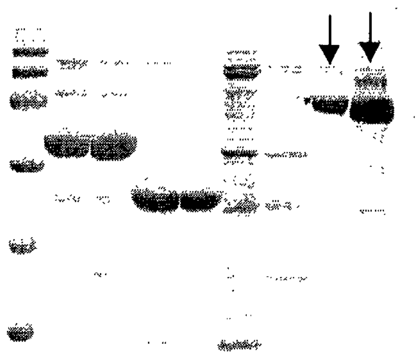

FIG. 35B

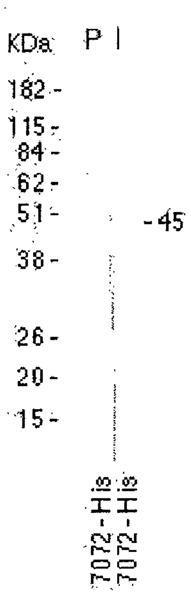


FIG. 35C

## FIGURE 36

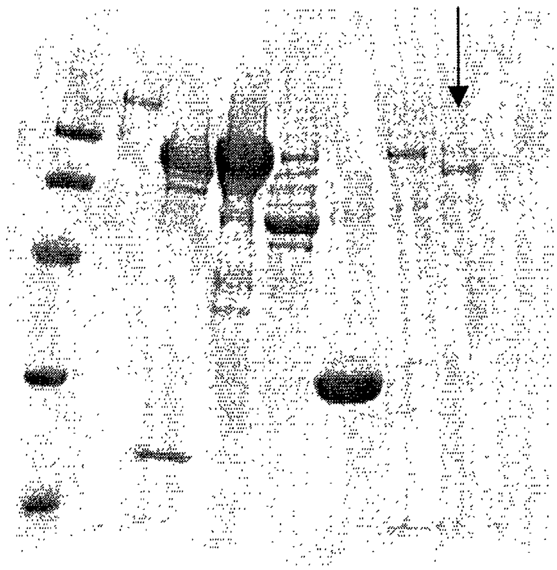

FIG. 36A

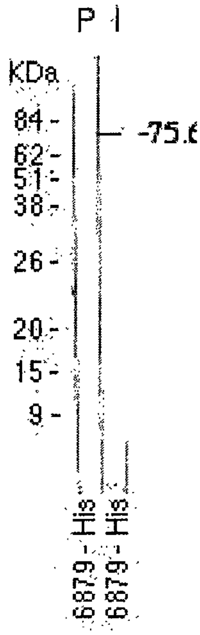


FIG. 36B

## FIGURE 37

FIG. 37A

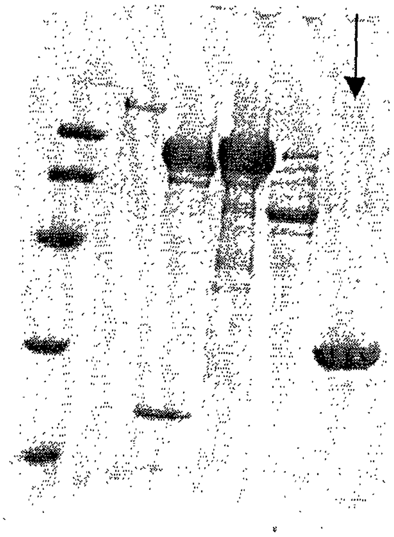

FIG. 37C

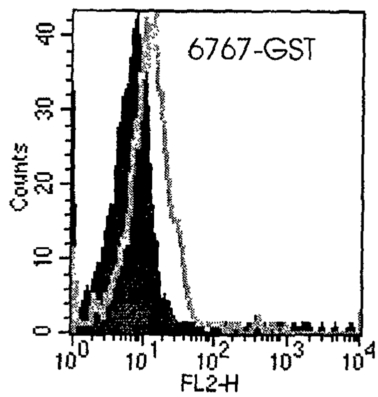


FIG. 37B

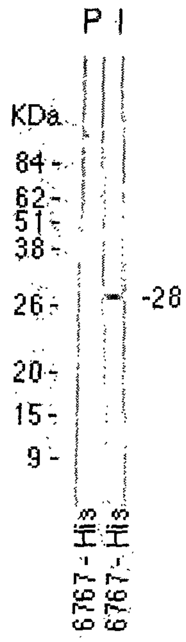


FIG. 37D

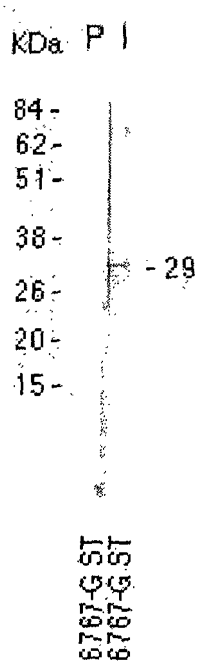

# FIGURE 38

**FIG. 38A**

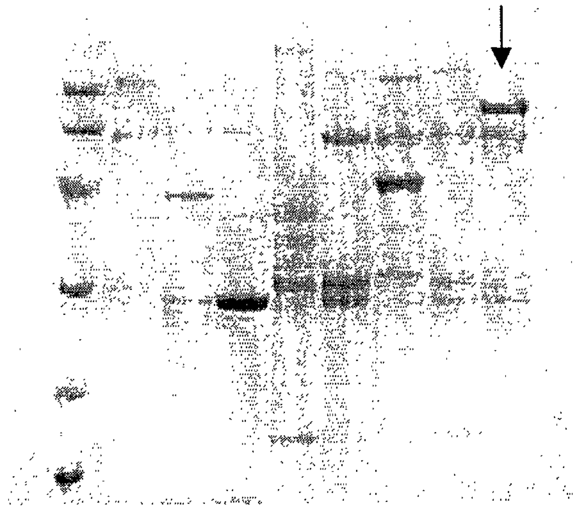

**FIG. 38B**

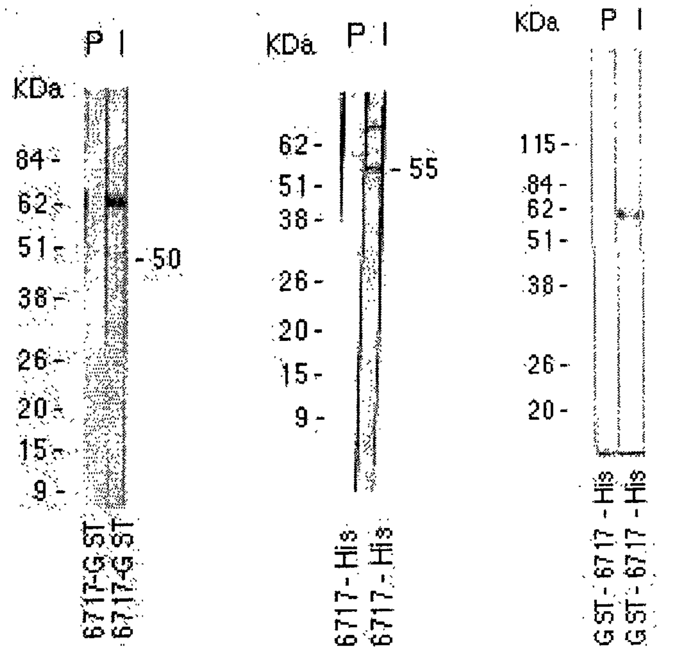

# FIGURE 39

**FIG. 39A**

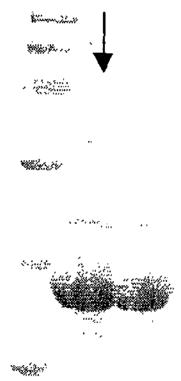

**FIG. 39B**

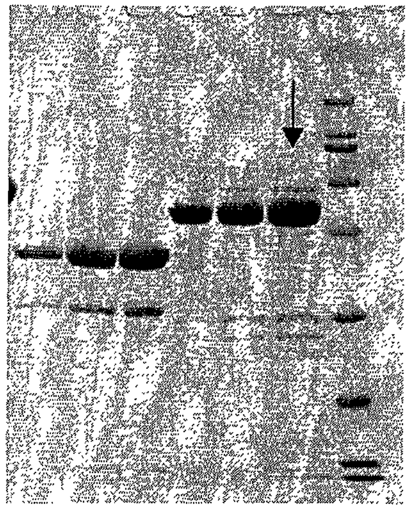

**FIG. 39C**

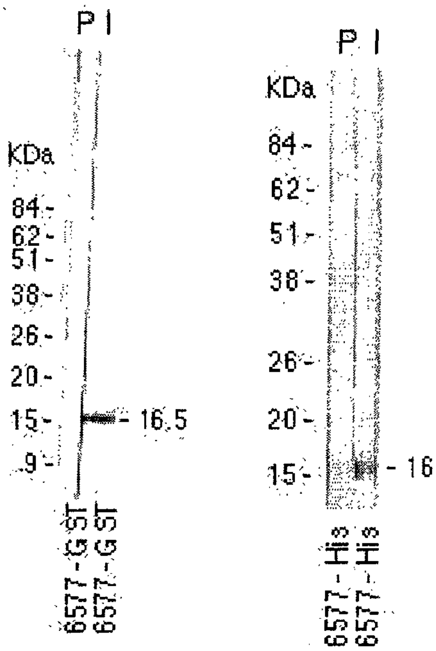


**FIG. 39D**

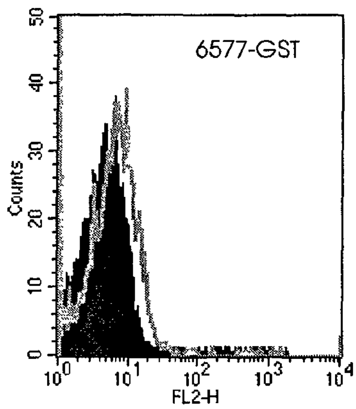

## FIGURE 40

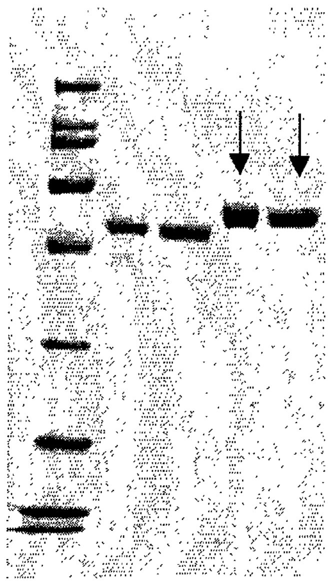

FIG. 40A

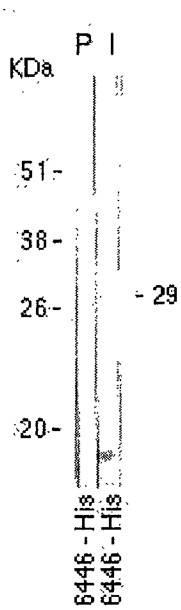


FIG. 40B

## FIGURE 41

FIG. 41A

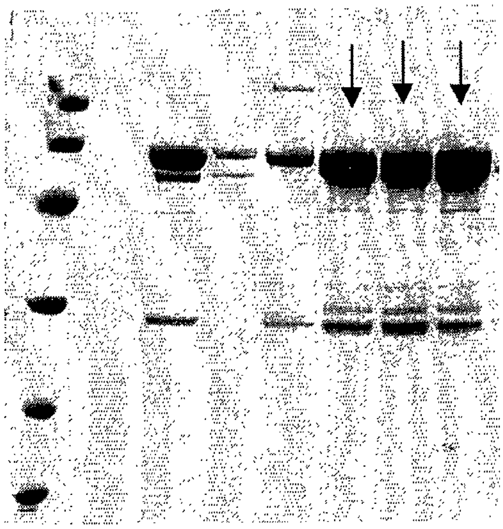

FIG. 41B

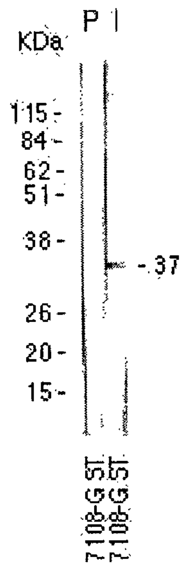


FIG. 41C

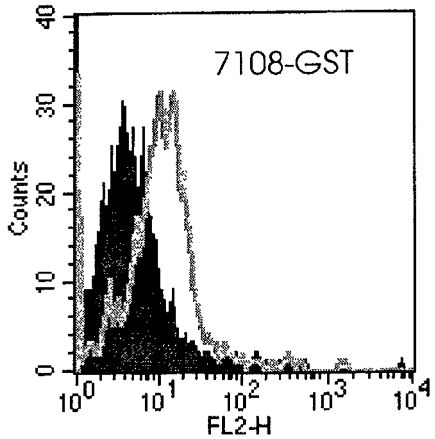

## FIGURE 42

**FIG. 42A**

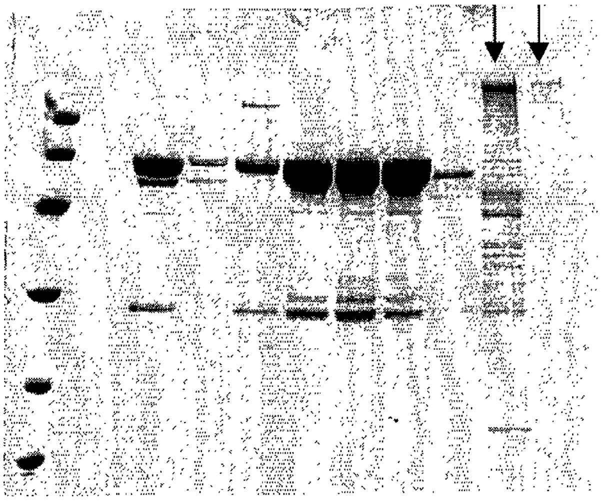

**FIG. 42B**

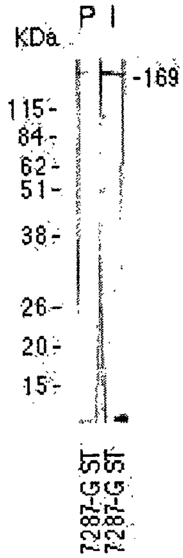


**FIG. 42C**

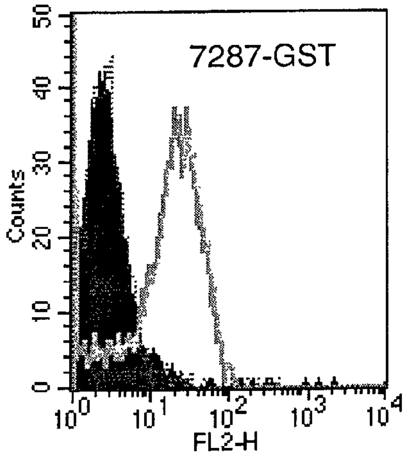

## FIGURE 43

FIG. 43A

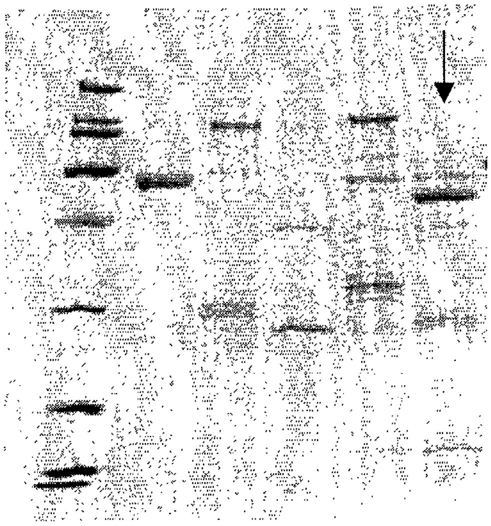

FIG. 43B

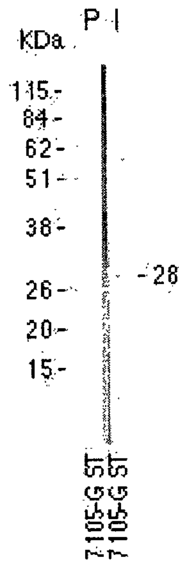


FIG. 43C

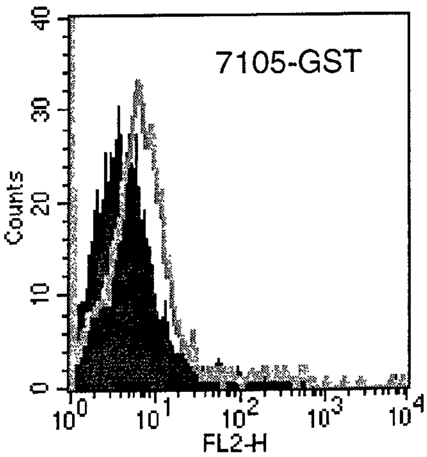

## FIGURE 44

FIG. 44A

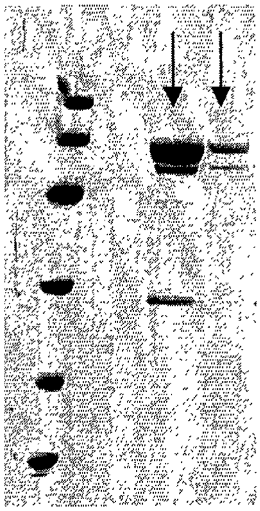

FIG. 44B

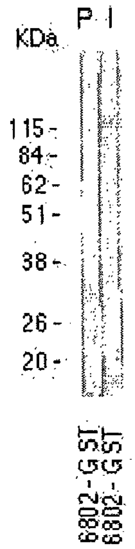


FIG. 44C

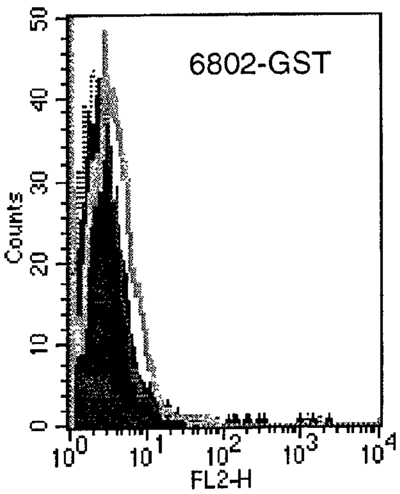

## FIGURE 45

FIG. 45A

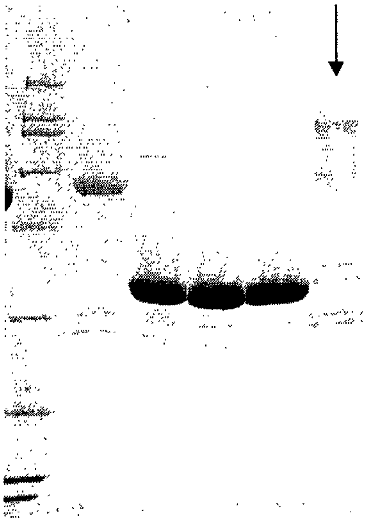

FIG. 45B

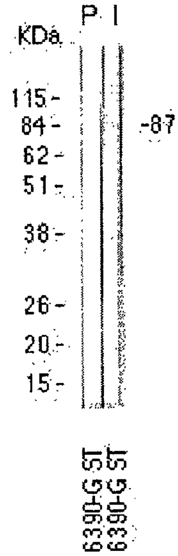


FIG. 45C

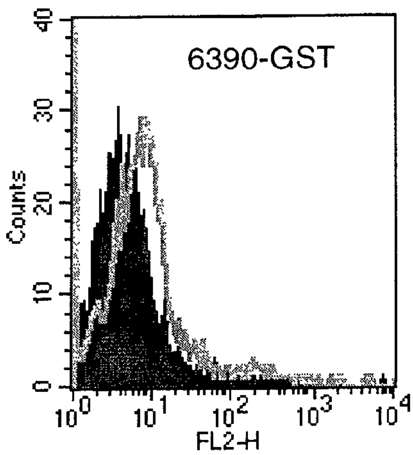

**FIGURE 46**

**FIG. 46A**

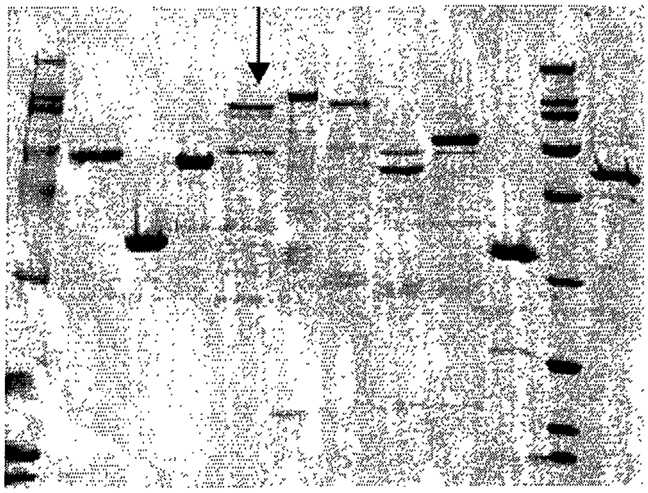

**FIG. 46B**

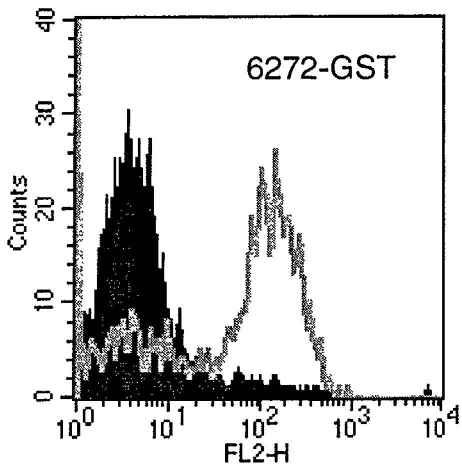

## FIGURE 47

FIG. 47A

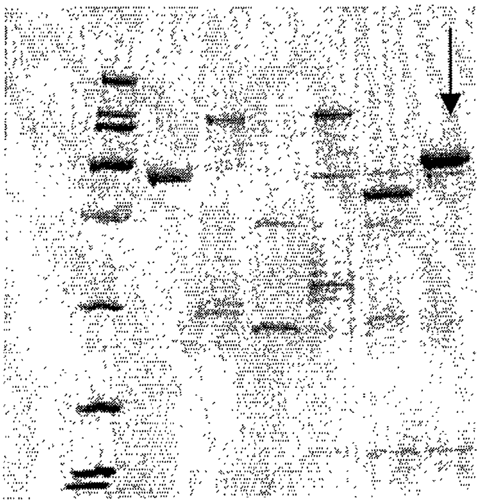

FIG. 47B

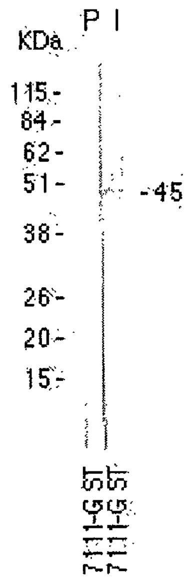


FIG. 47C

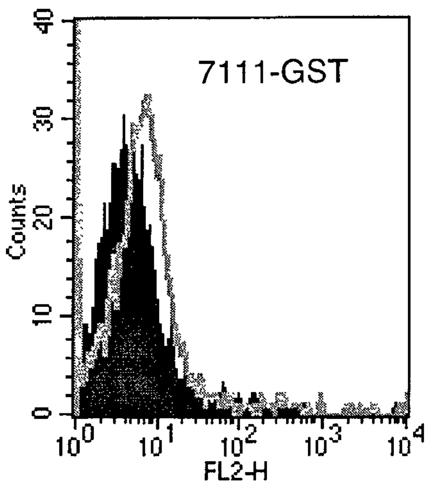

## FIGURE 48

FIG. 48A

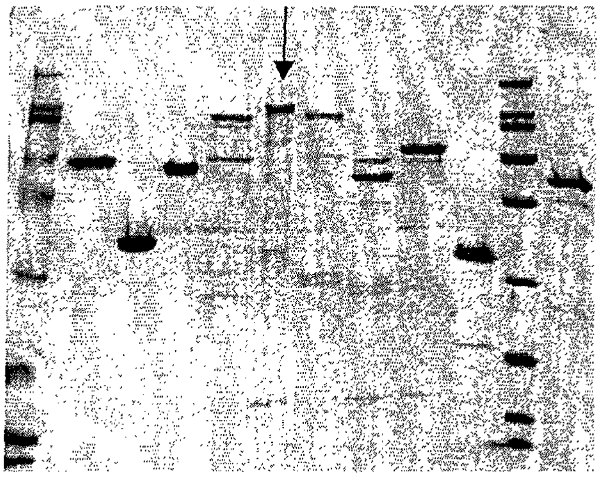

FIG. 48B

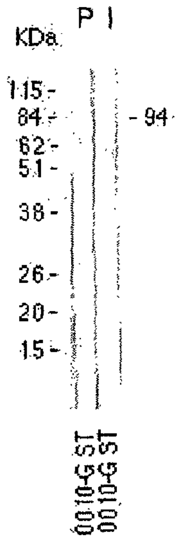


FIG. 48C

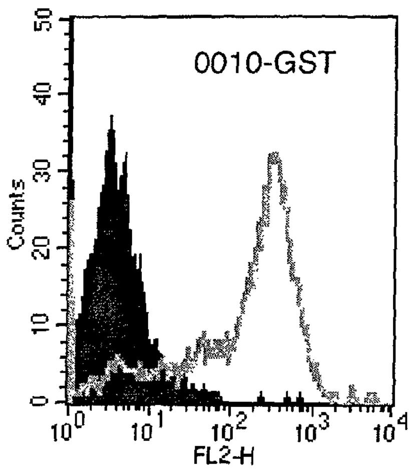

## FIGURE 49

FIG. 49A

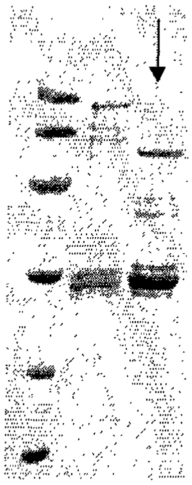

FIG. 49B

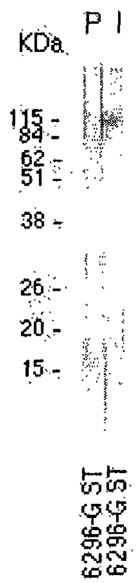


FIG. 49C

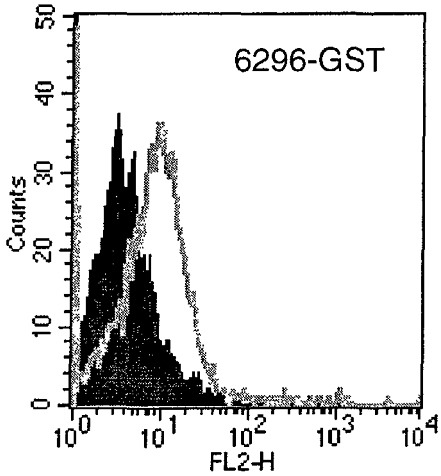

# FIGURE 50

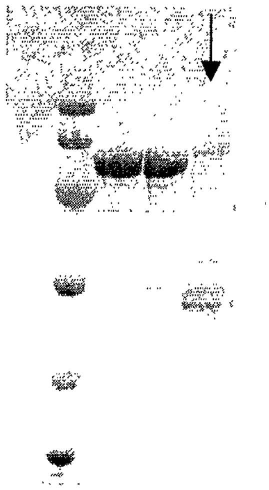

FIG. 50A

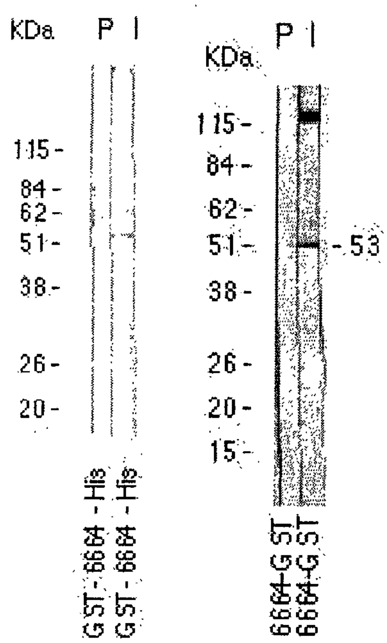


FIG. 50B

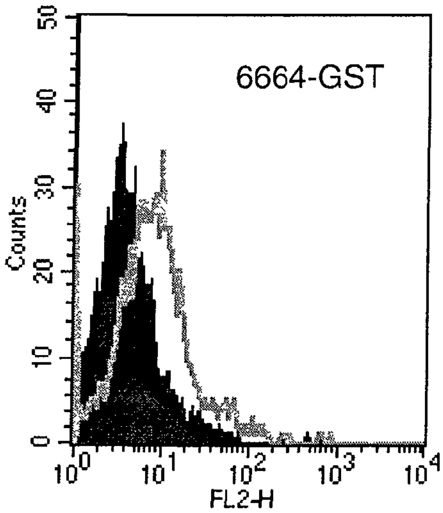


FIG. 50C

## FIGURE 51

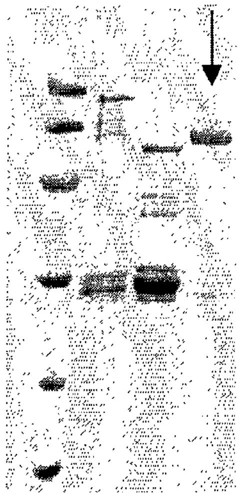

FIG. 51A

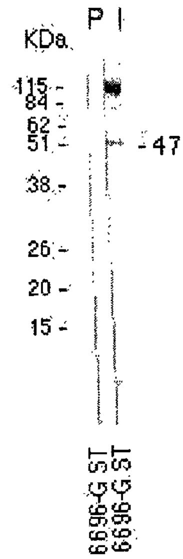


FIG. 51B

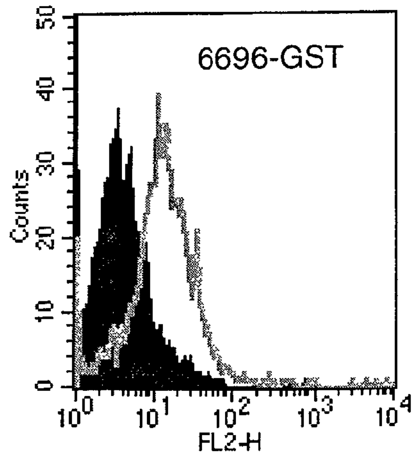


FIG. 51C

# *FIGURE 52*

*FIG. 52A*

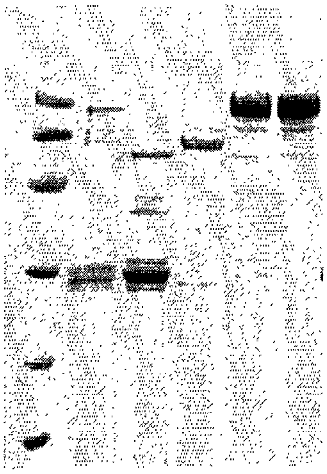

*FIG. 52B*

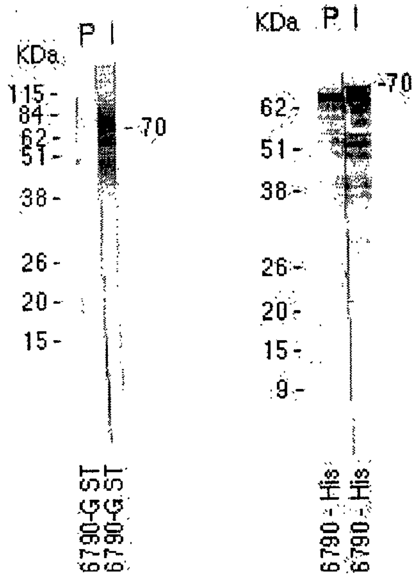


*FIG. 52C*

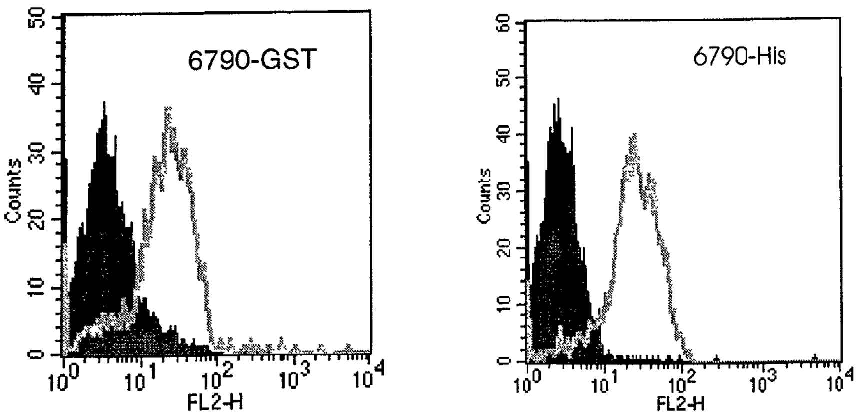

FIGURE 53

FIG. 53A

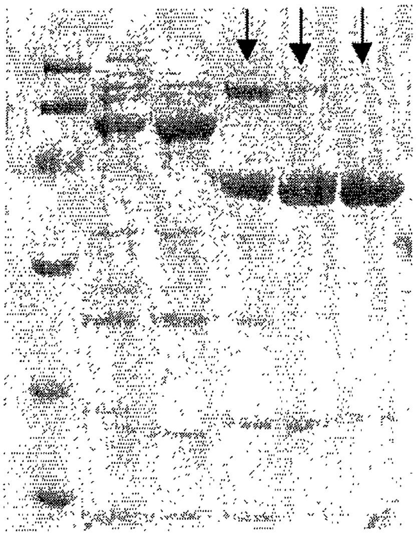

FIG. 53B

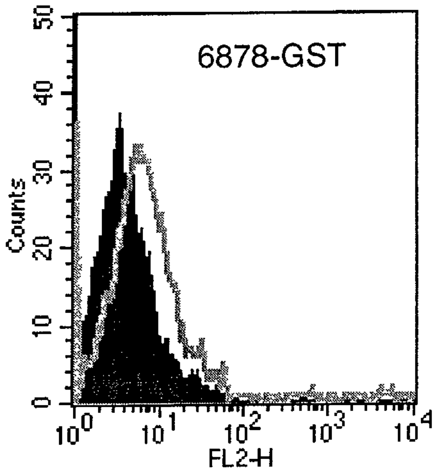

## FIGURE 54

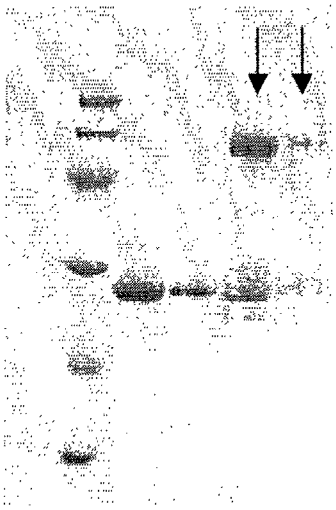

**FIG. 54A**

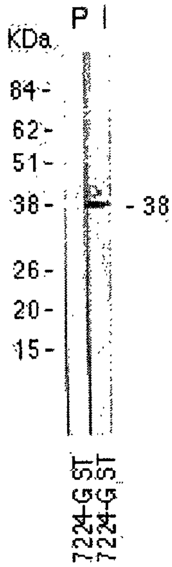


**FIG. 54B**

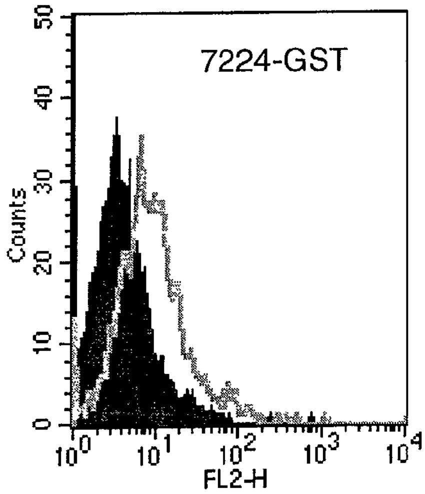


**FIG. 54C**

## FIGURE 55

FIG. 55A

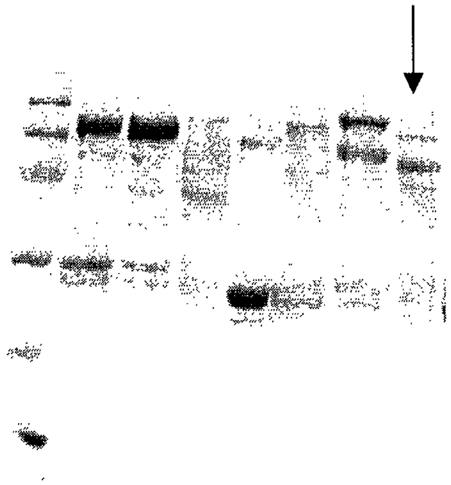

FIG. 55B

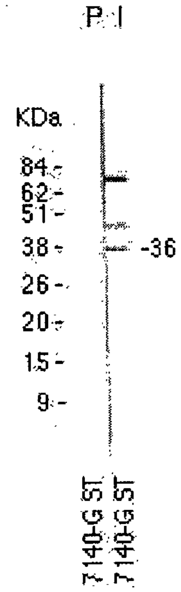


FIG. 55C

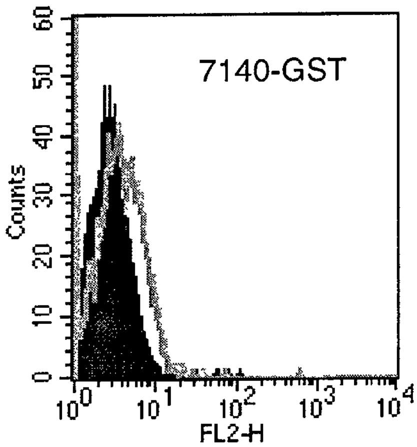

# FIGURE 56

FIG. 56A

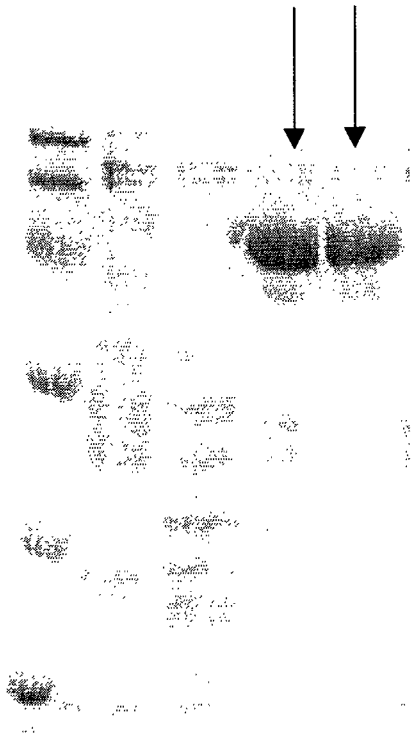

FIG. 56B

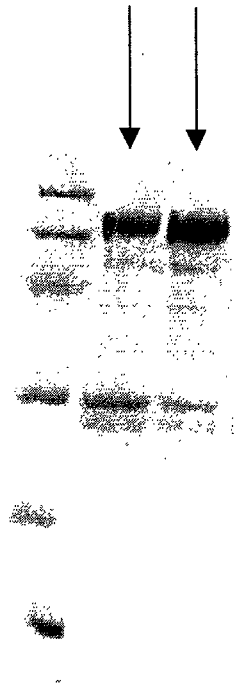

FIG. 56C

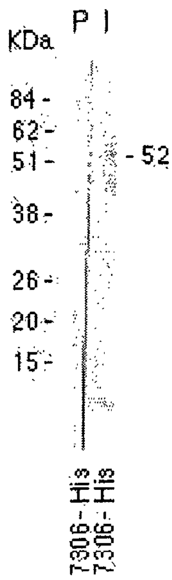


FIG. 56D

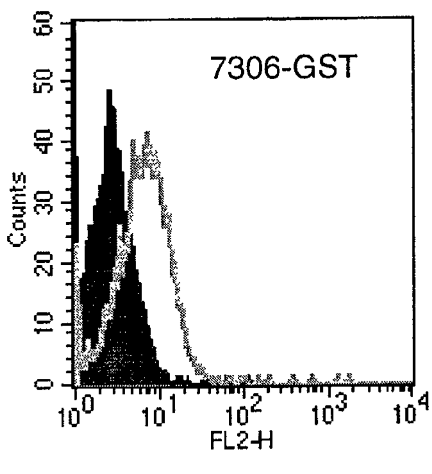

**FIGURE 57**

FIG. 57A

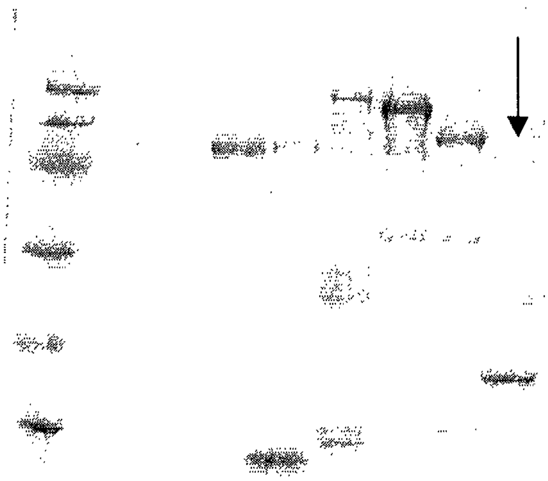

FIG. 57B

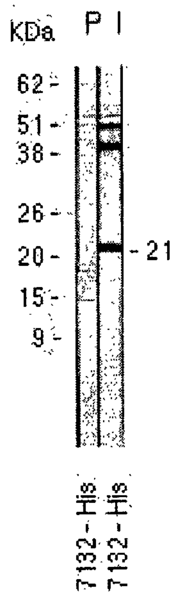


FIG. 57C

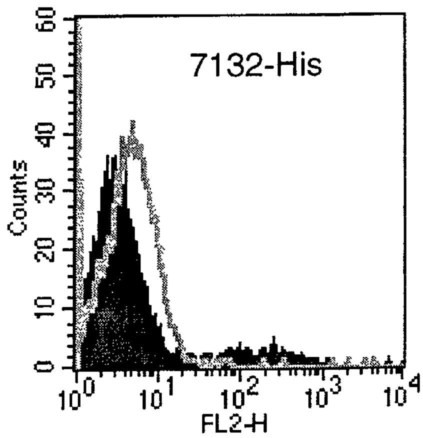

# FIGURE 58

**FIG. 58A**

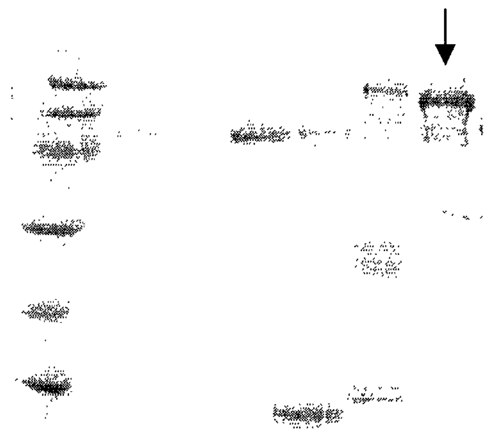

**FIG. 58B**

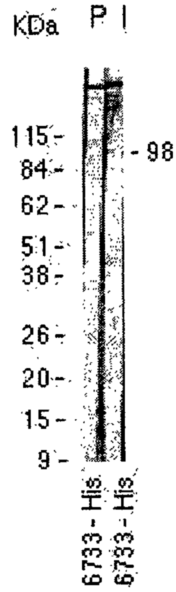


**FIG. 58C**

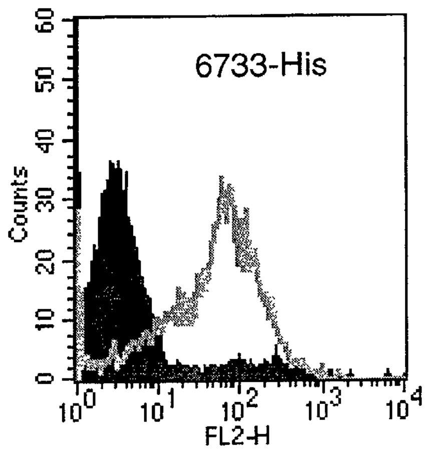

## FIGURE 59

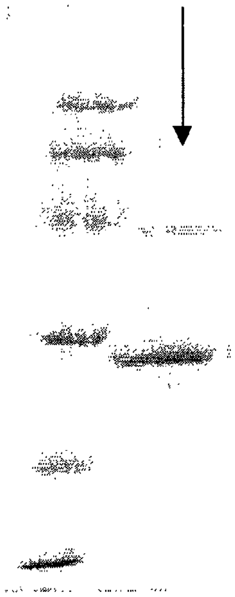

FIG. 59A

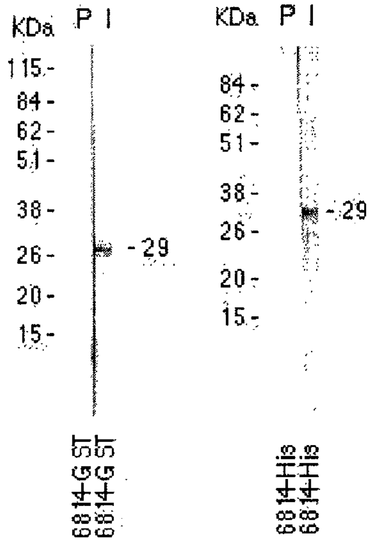


FIG. 59B

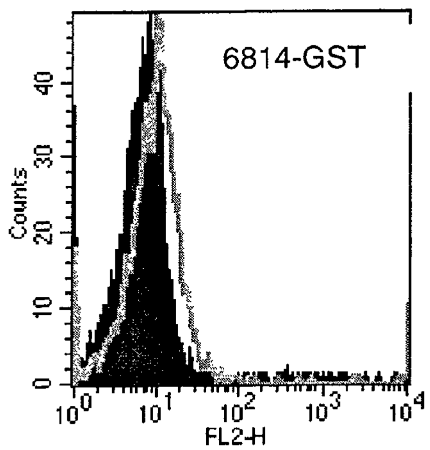


FIG. 59C

# FIGURE 60

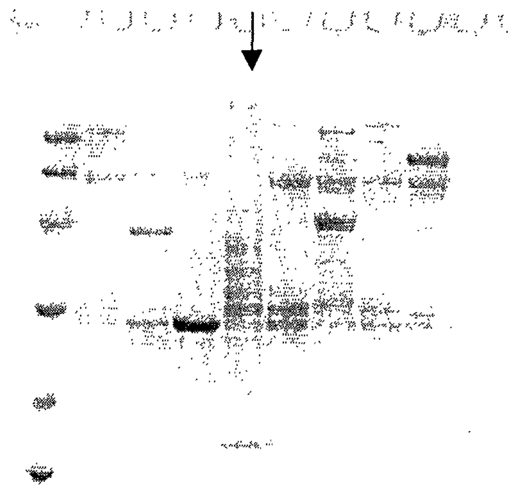

**FIG. 60A**

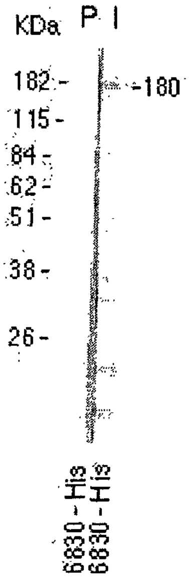


**FIG. 60B**

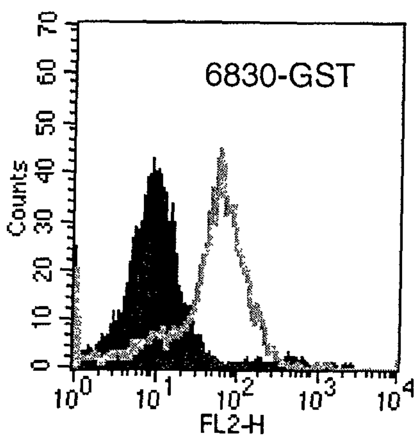


**FIG. 60C**

## FIGURE 61

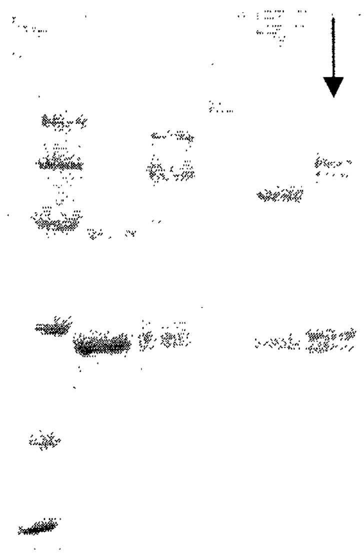

FIG. 61A

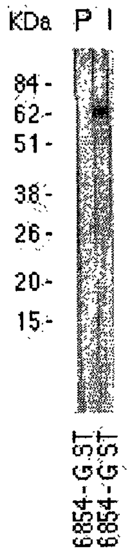


FIG. 61B

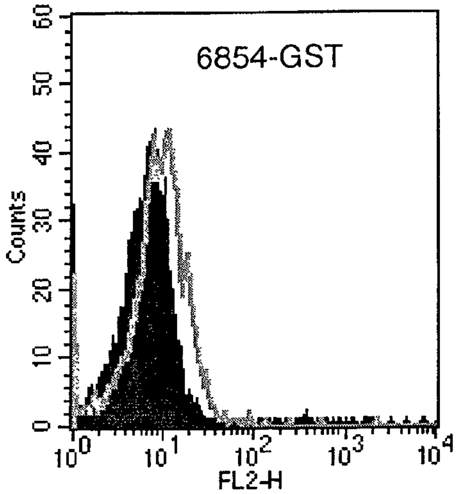


FIG. 61C

# FIGURE 62

**FIG. 62A**

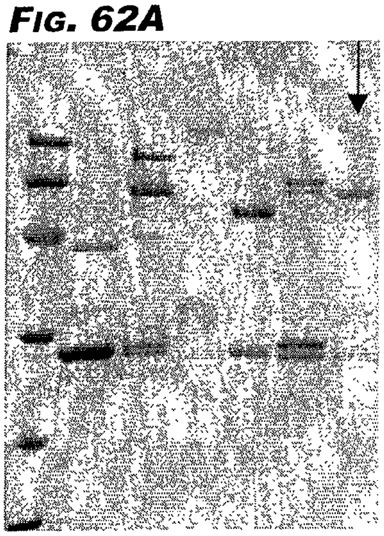

**FIG. 62C**

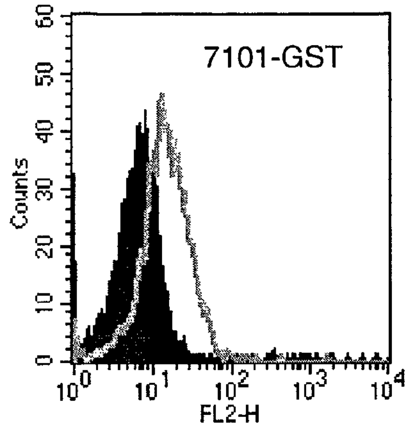


**FIG. 62B**

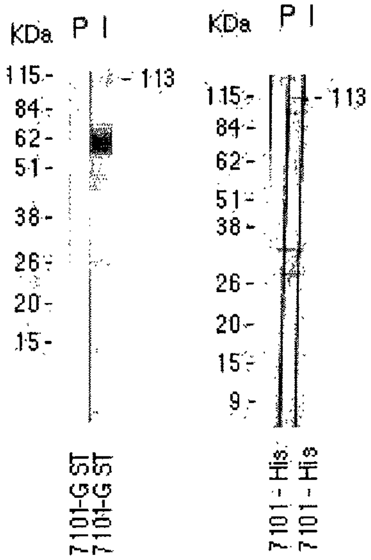

# FIGURE 63

**FIG. 63A**

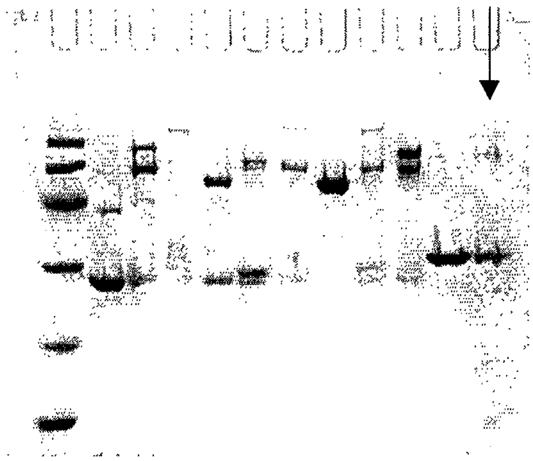

**FIG. 63B**

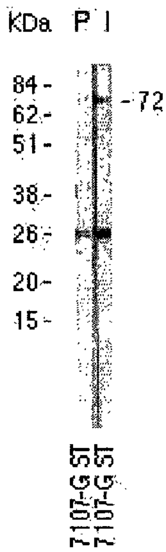


**FIG. 63C**

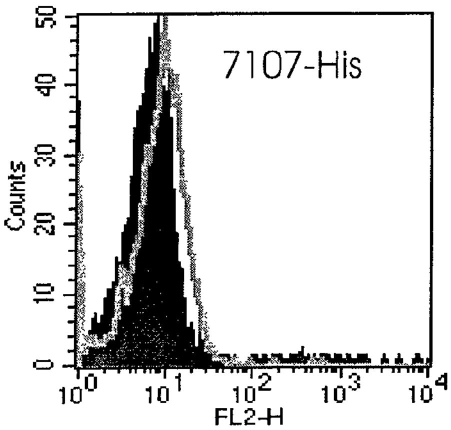

# FIGURE 64

FIG. 64A

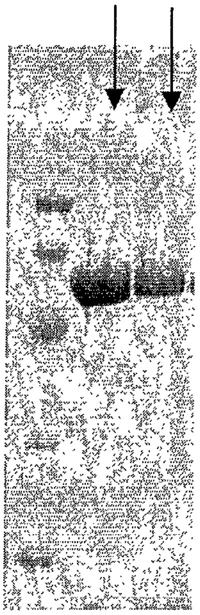

FIG. 64B

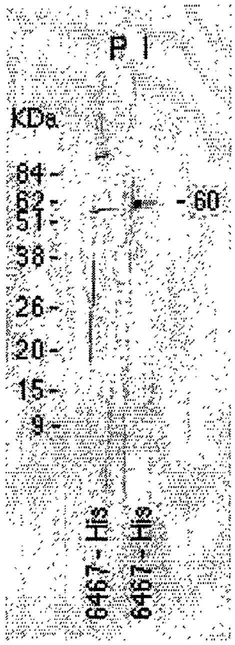


FIG. 64C

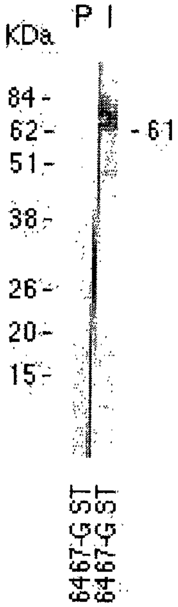


FIG. 64D

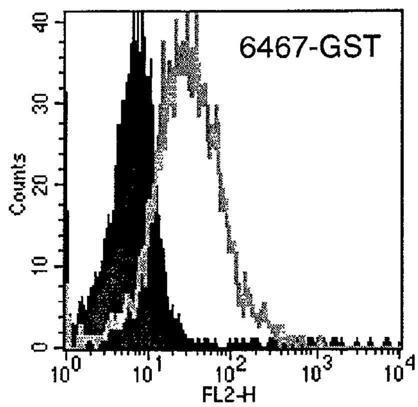

## FIGURE 65

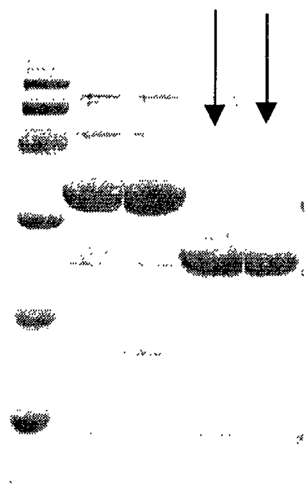

FIG. 65A

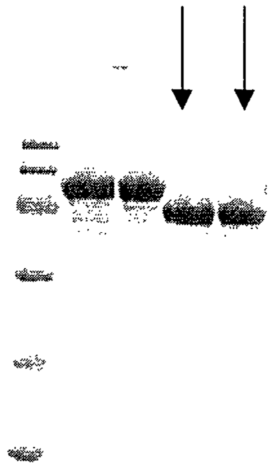

FIG. 65B

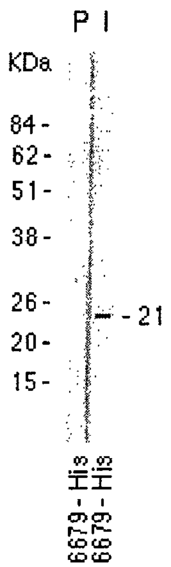


FIG. 65C

## FIGURE 66

FIG. 66A

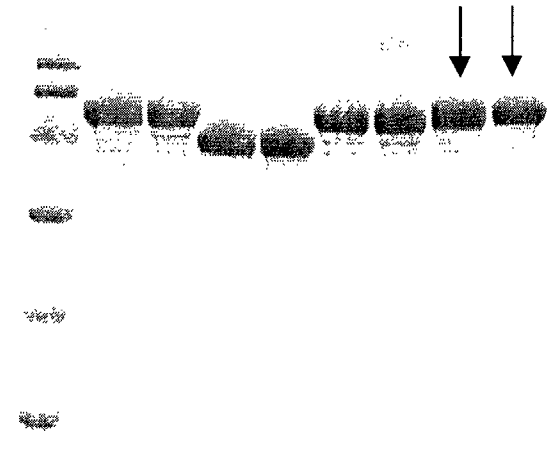

FIG. 66B

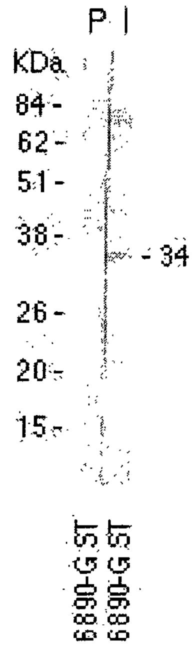

## FIGURE 67

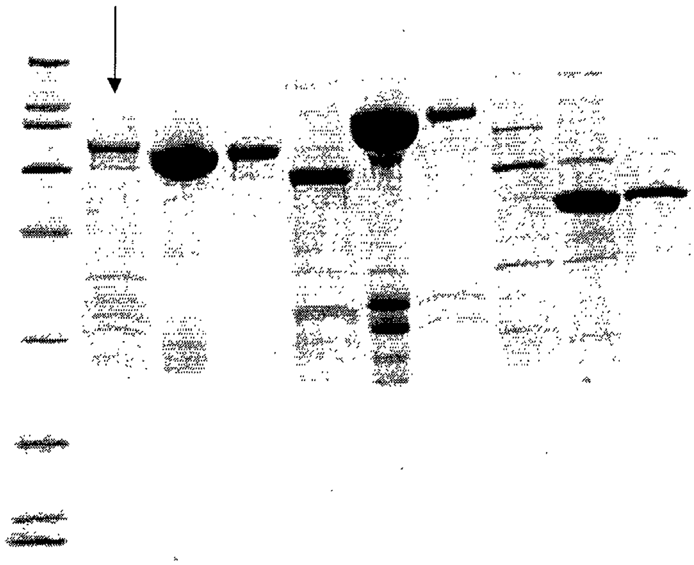

**FIG. 67A**

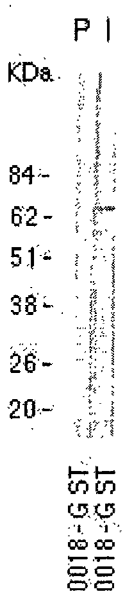


**FIG. 67B**

## FIGURE 68

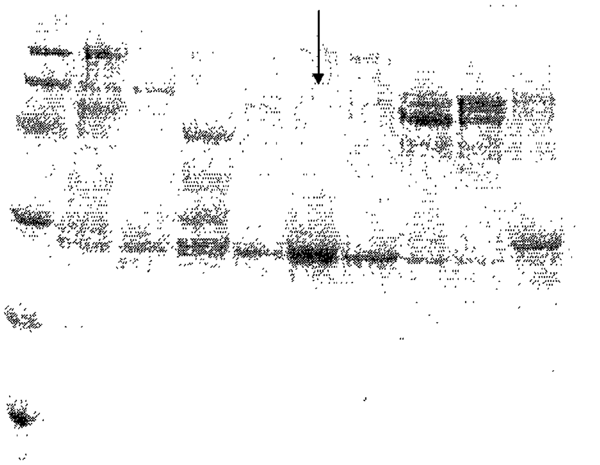

FIG. 68A

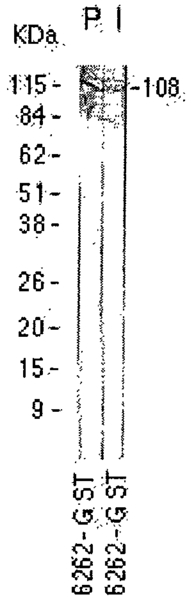


FIG. 68B

**FIGURE 69**

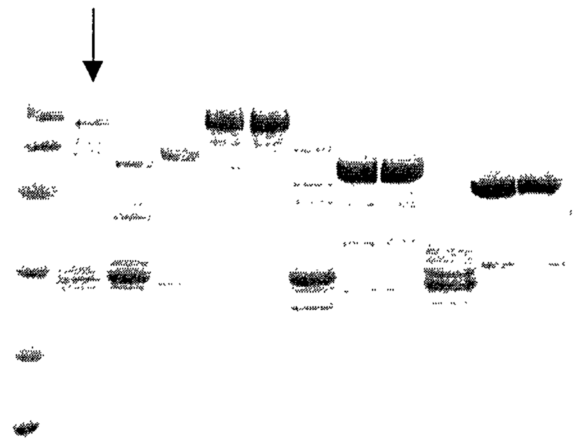

**Fig. 69A**

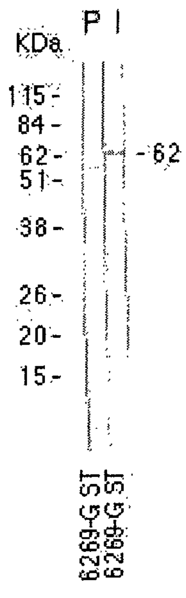


**Fig. 69B**

**FIGURE 70**

**FIG. 70A**

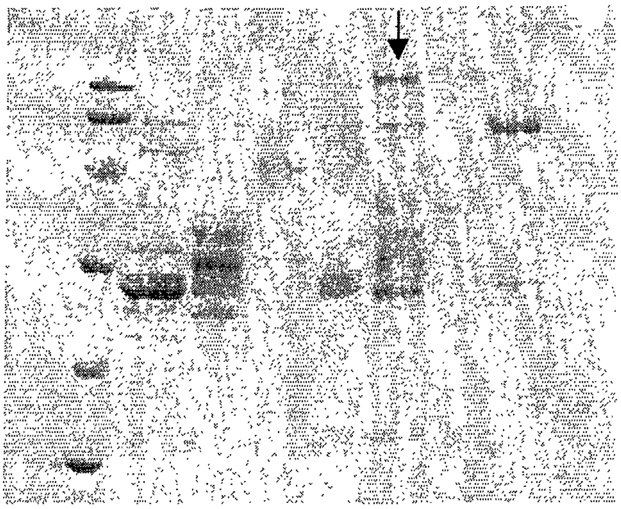

**FIG. 70B**

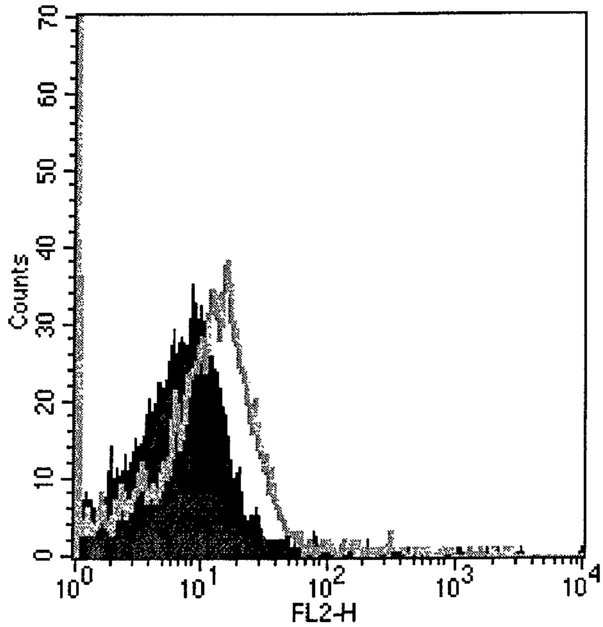

## FIGURE 71

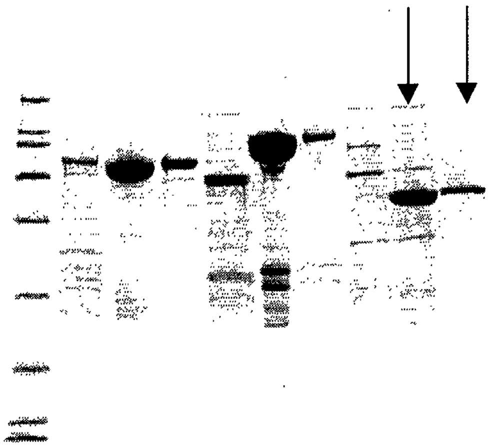

*FIG. 71A*

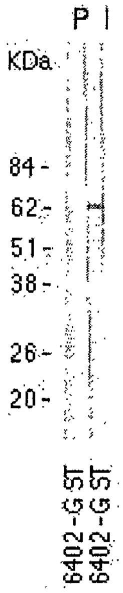


*FIG. 71B*

**FIGURE 72**

**FIG. 72A**

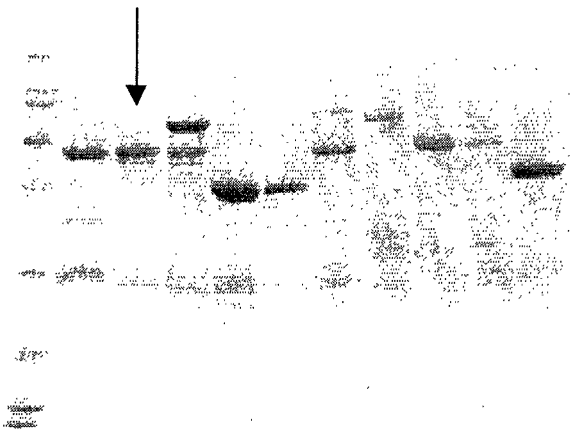

**FIG. 72B**

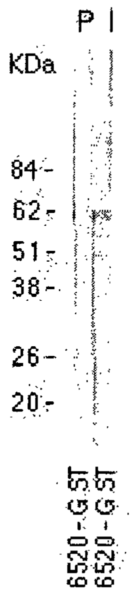

**FIGURE 73**

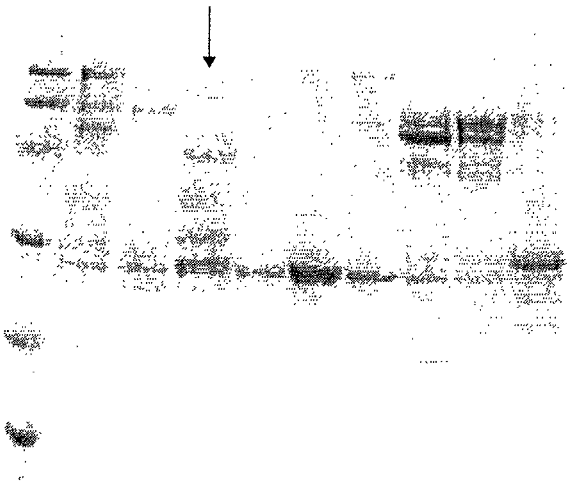

**FIG. 73A**

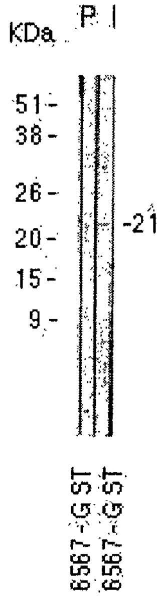


**FIG. 73B**

## FIGURE 74

FIG. 74A

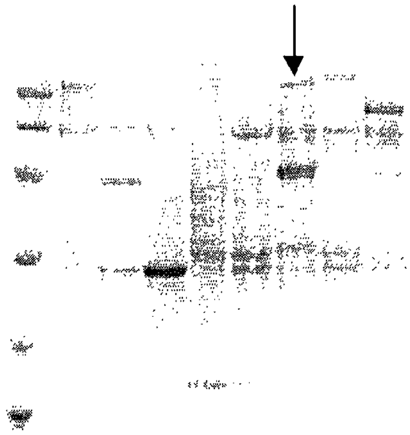

FIG. 74B

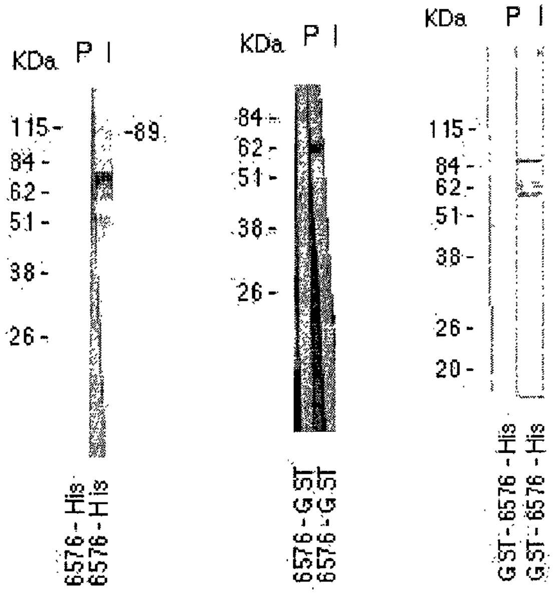


FIG. 74C

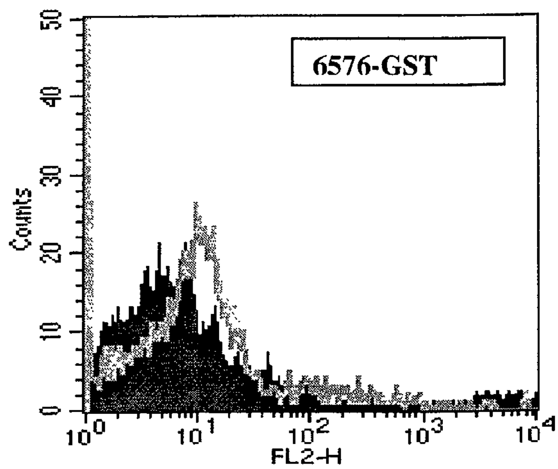

**FIGURE 75**

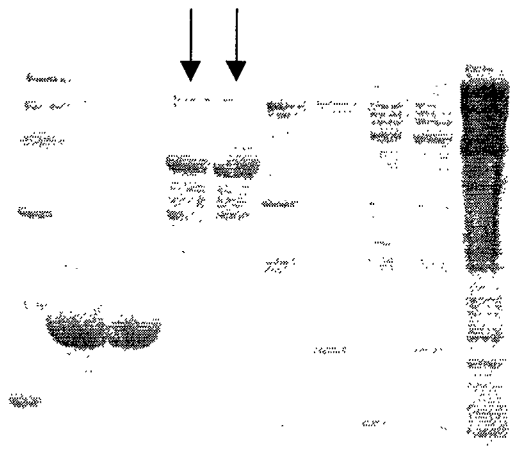

**FIG. 75A**

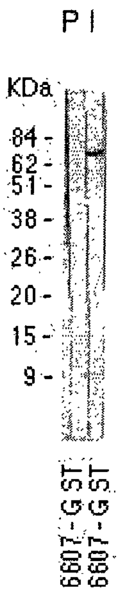


**FIG. 75B**

## FIGURE 76

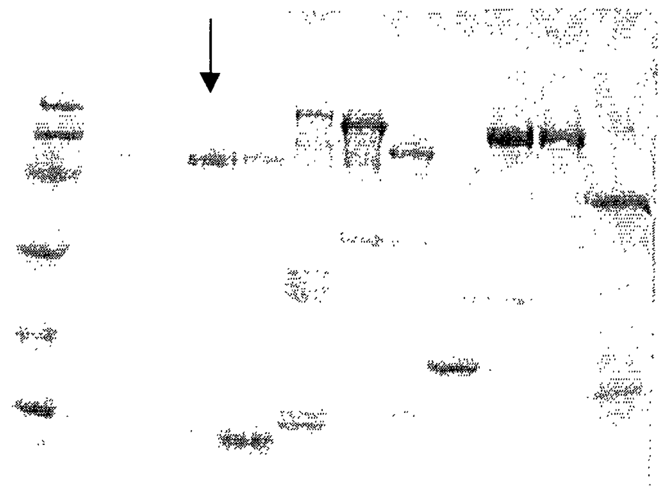

FIG. 76A

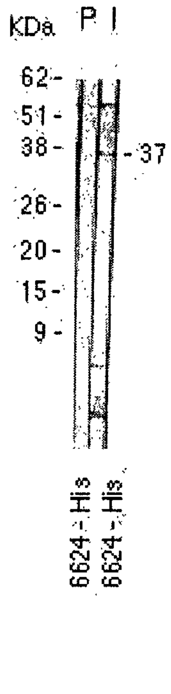


FIG. 76B

## FIGURE 77

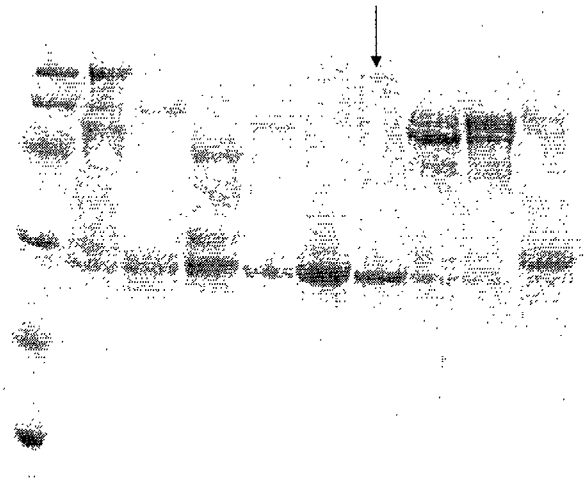

FIG. 77A

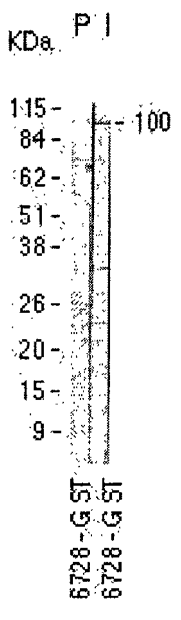


FIG. 77B

## FIGURE 78

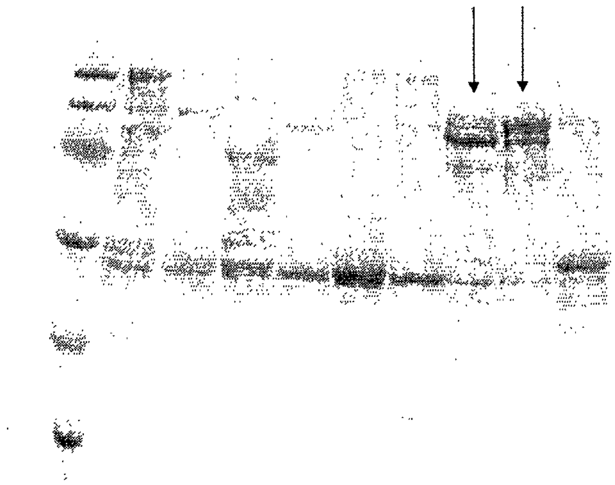

FIG. 78A

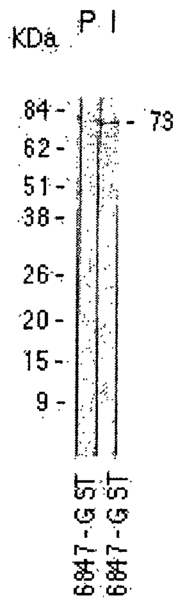


FIG. 78B

## FIGURE 79

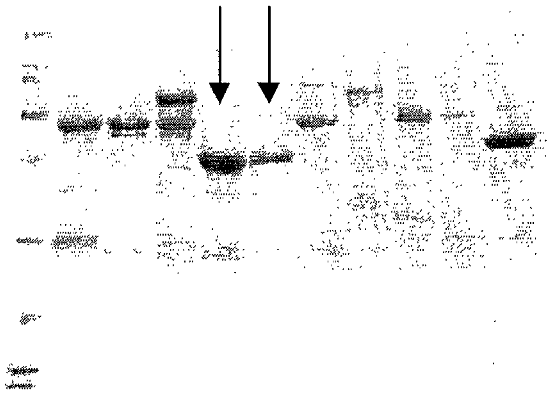

FIG. 79A

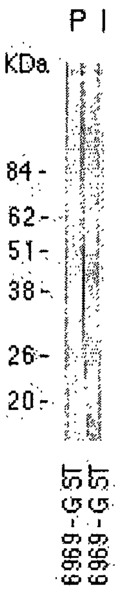


FIG. 79B

## FIGURE 80

**FIG. 80A**

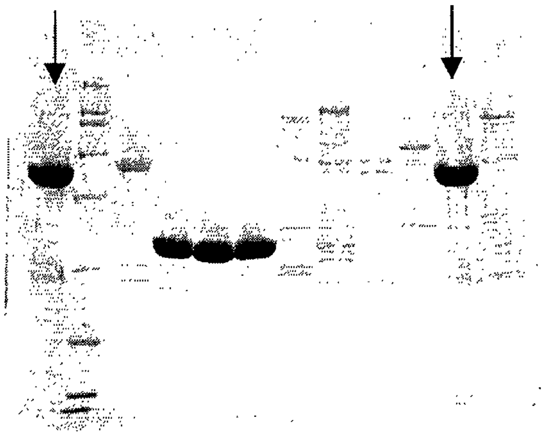

**FIG. 80B**

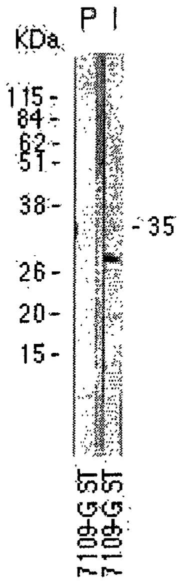

## FIGURE 81

FIG. 81A

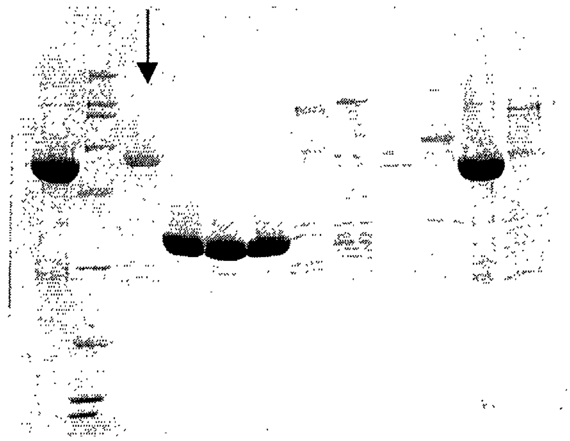

FIG. 81B

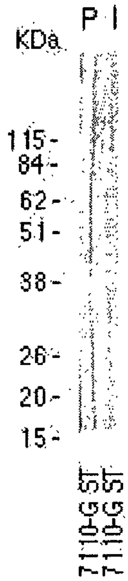

**FIGURE 82**

FIG. 82A

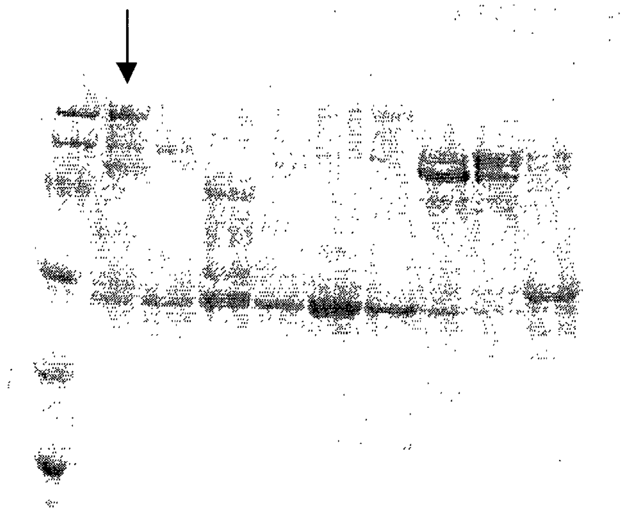

FIG. 82B

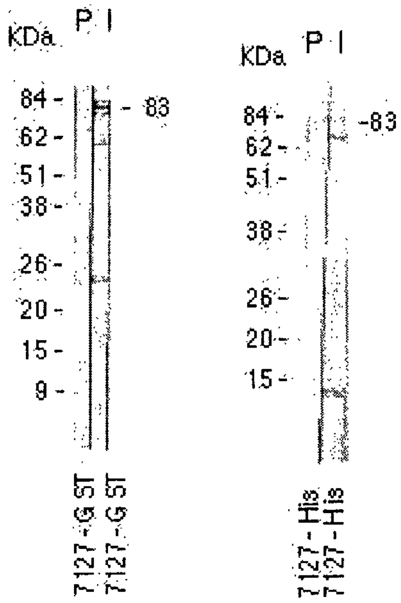

## FIGURE 83

FIG. 83A

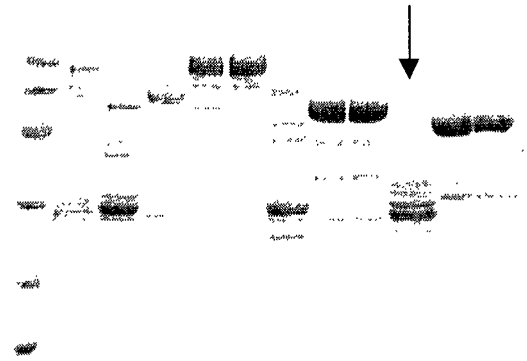

FIG. 83B

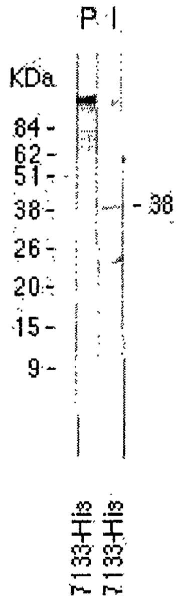

FIGURE 84

FIG. 84A

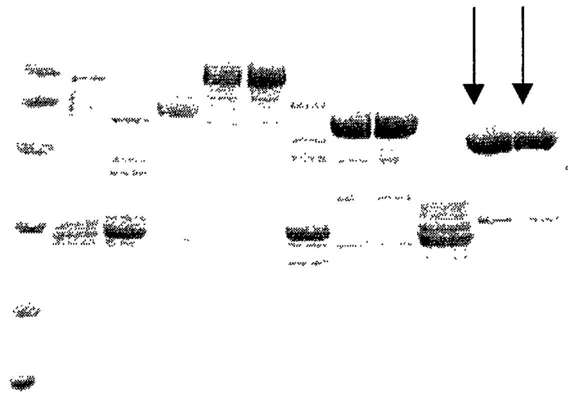

FIG. 84B

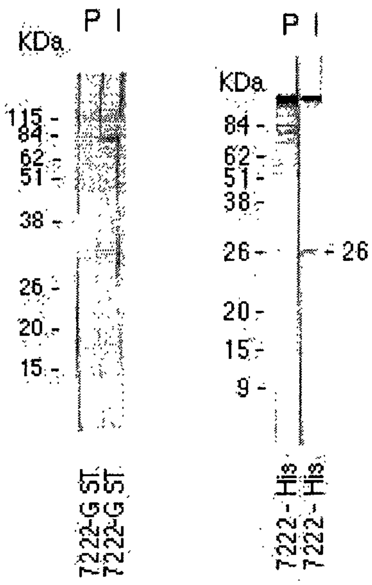

## FIGURE 85

**FIG. 85A**

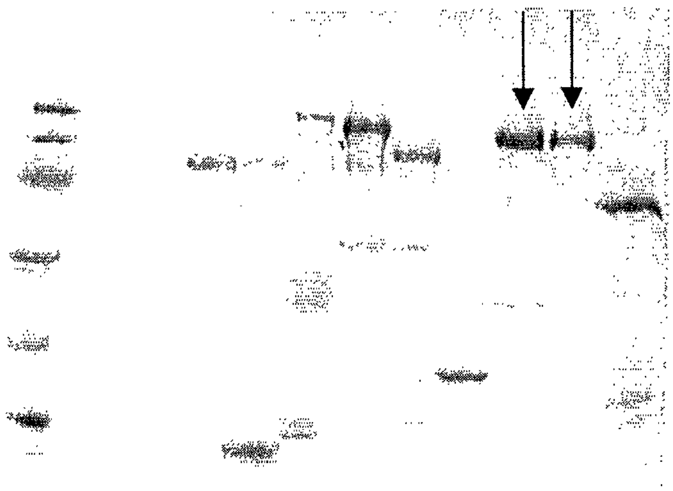

**FIG. 85B**

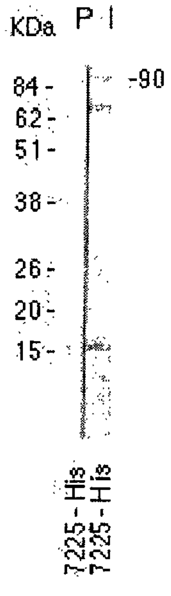

## FIGURE 86

**FIG. 86A**

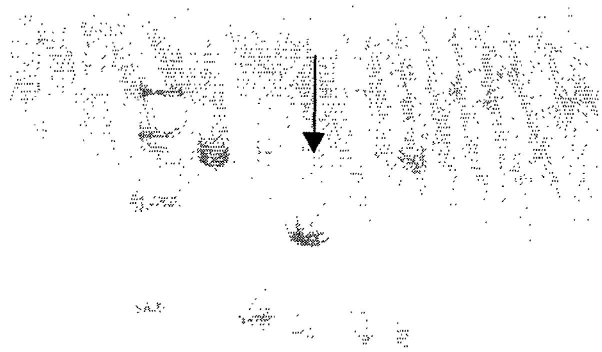

**FIG. 86B**

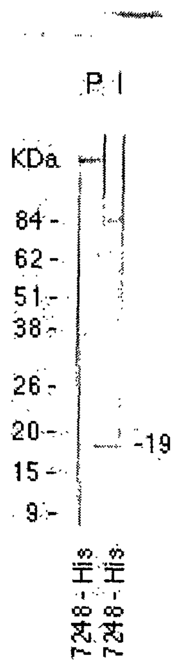

FIGURE 87

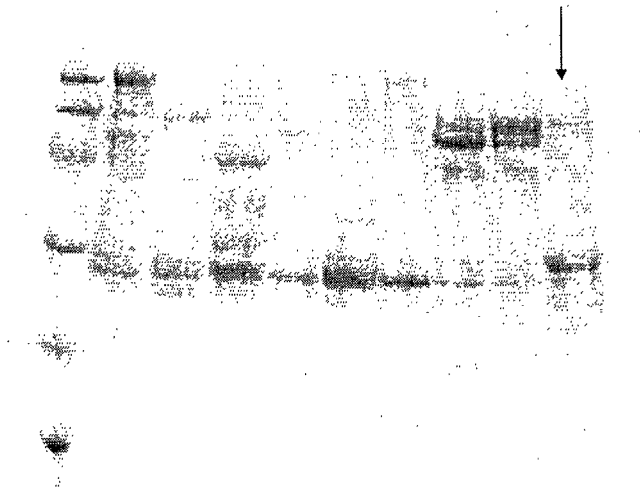

FIG. 87A

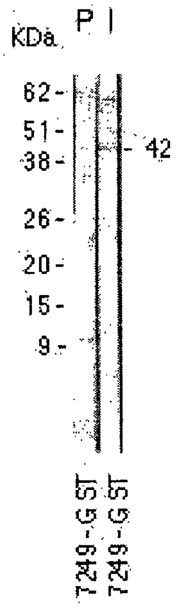


FIG. 87B

## FIGURE 88

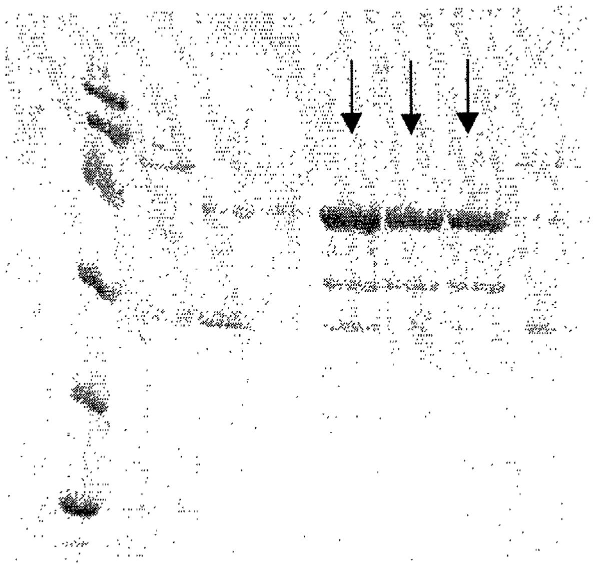

**FIG. 88A**

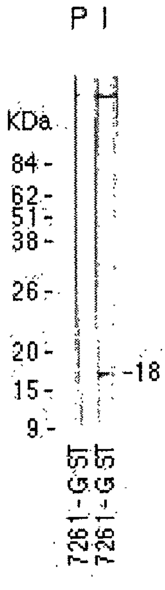


**FIG. 88B**

FIGURE 89

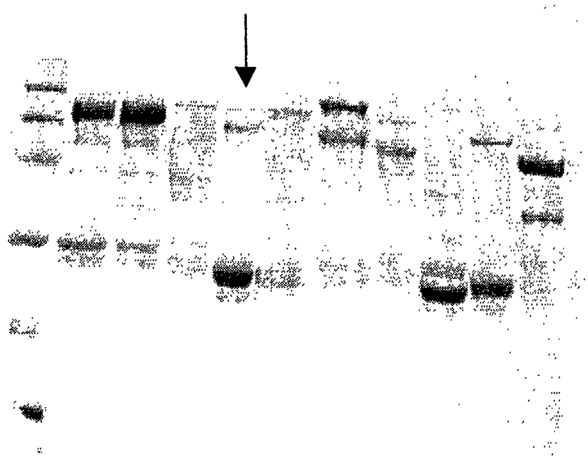

FIG. 89A

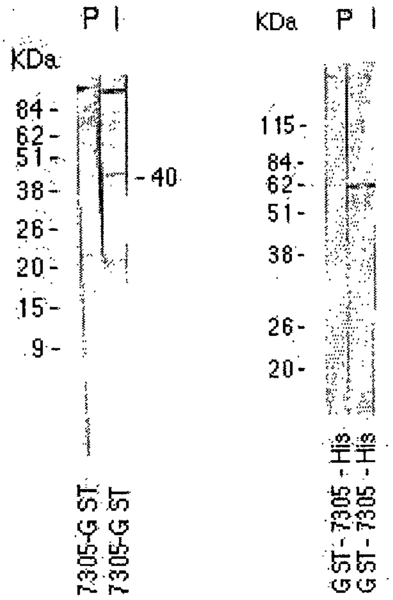


FIG. 89B

FIGURE 90

FIG. 90A

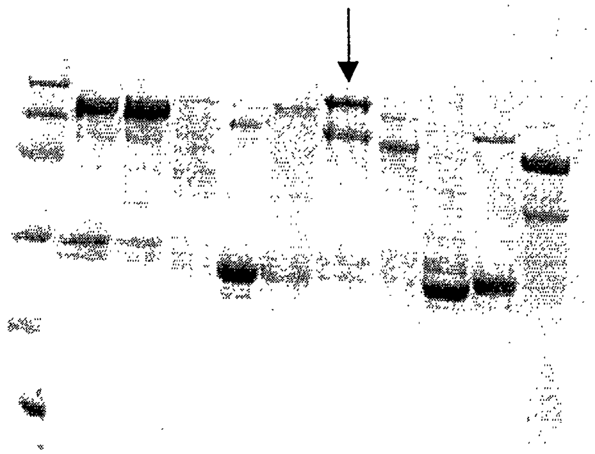

FIG. 90B

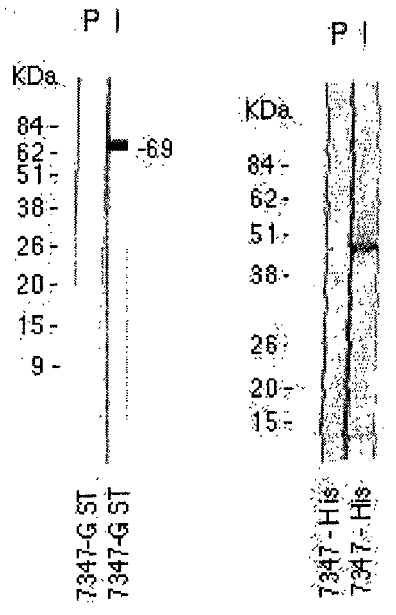

## FIGURE 91

FIG. 91A

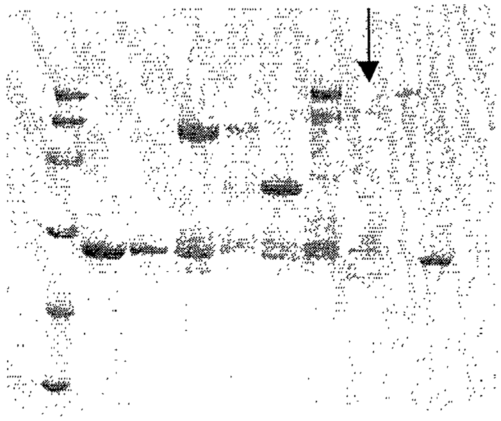

FIG. 91B

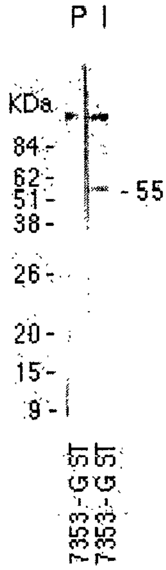

## FIGURE 92

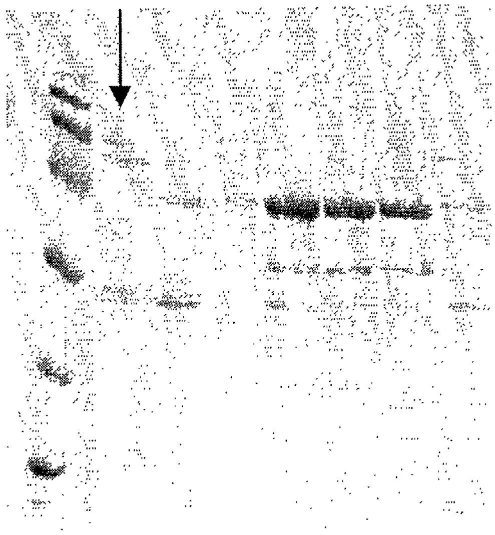

FIG. 92A

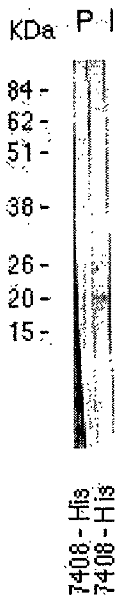


FIG. 92B

# FIGURE 93

FIG. 93A

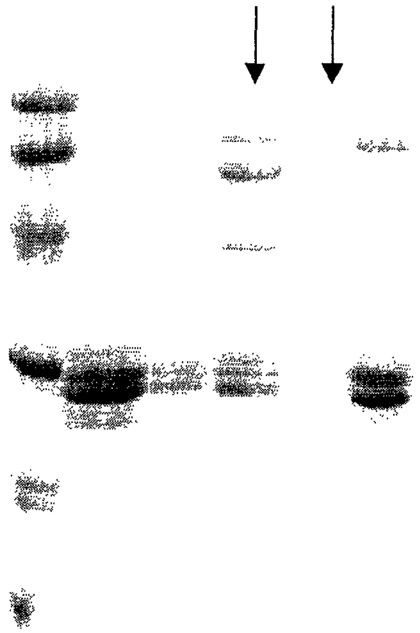

FIG. 93B

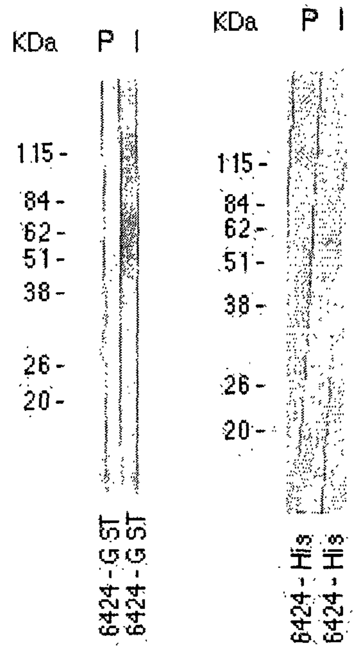


FIG. 93C

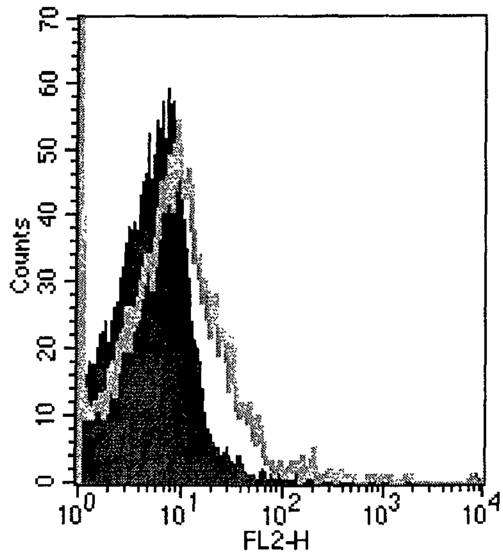

# FIGURE 94

FIG. 94A

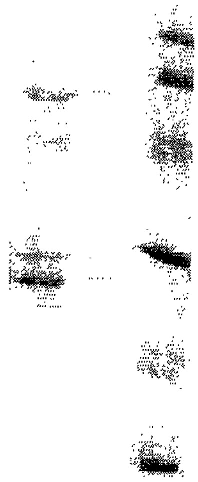

FIG. 94B

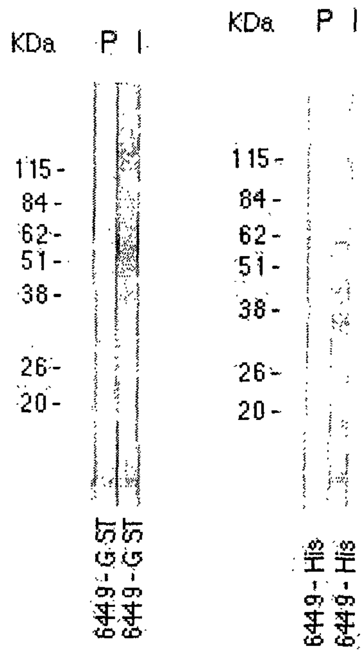


FIG. 94C

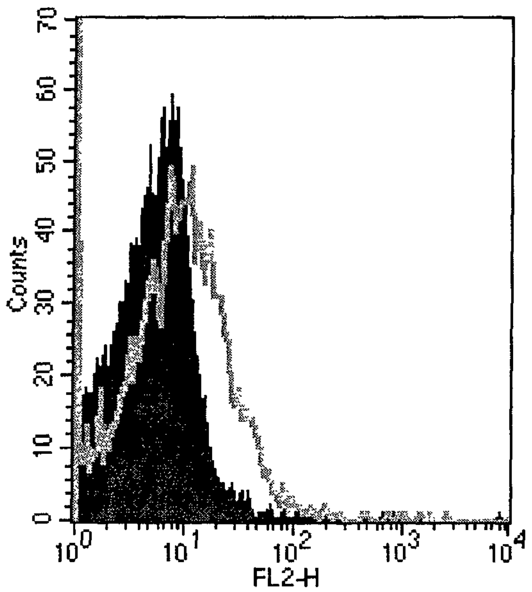

## *FIGURE 95*

**FIG. 95A**

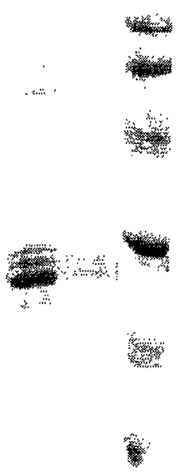

**FIG. 95B**

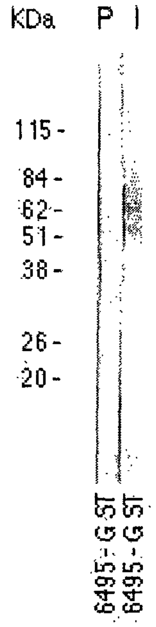


**FIG. 95C**

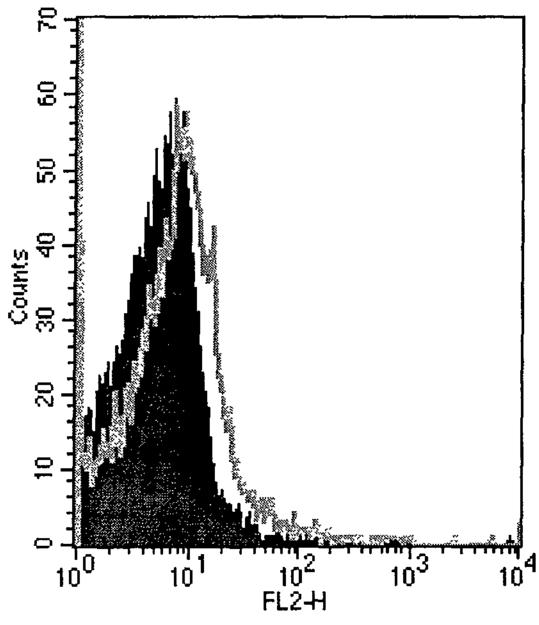

## FIGURE 96

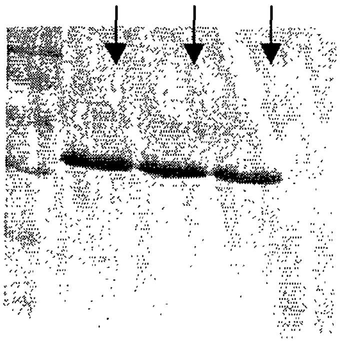

FIG. 96A

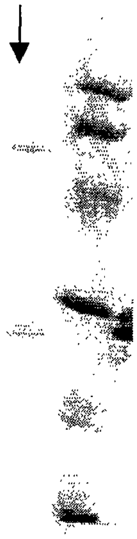

FIG. 96B

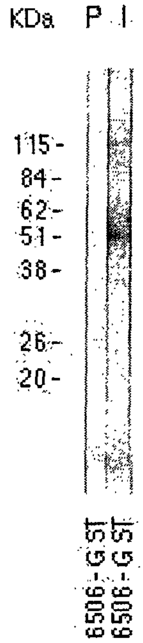


FIG. 96C

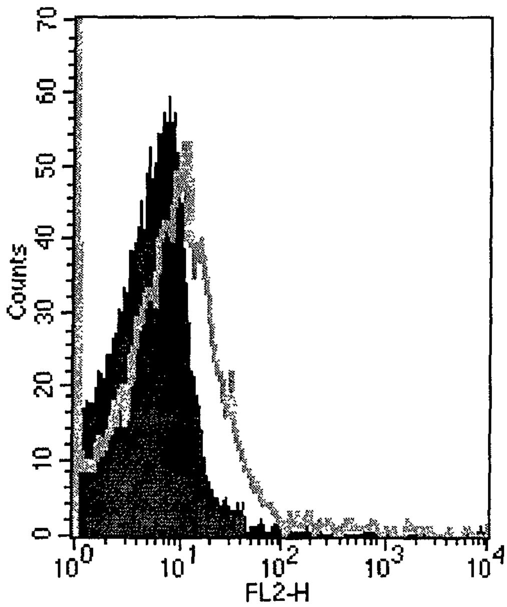


FIG. 96D

# FIGURE 97

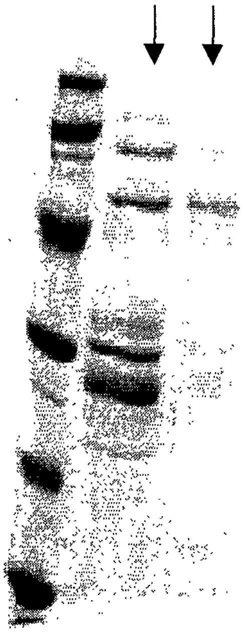

**FIG. 97A**

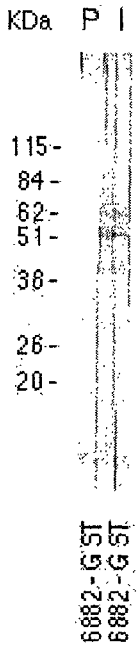


**FIG. 97B**

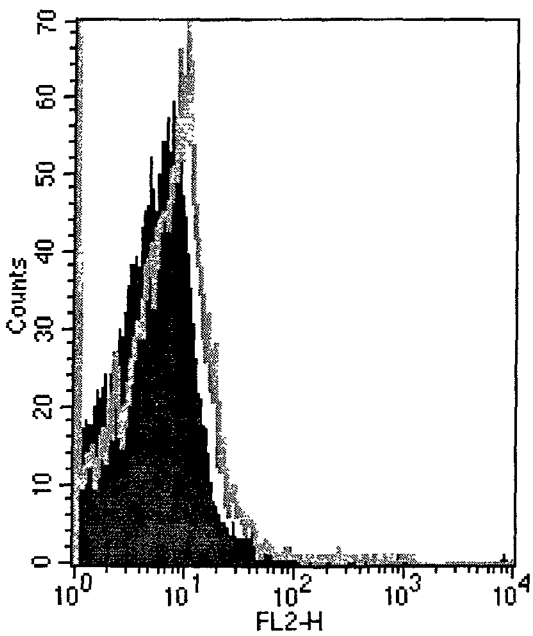


**FIG. 97C**

## FIGURE 98

FIG. 98A

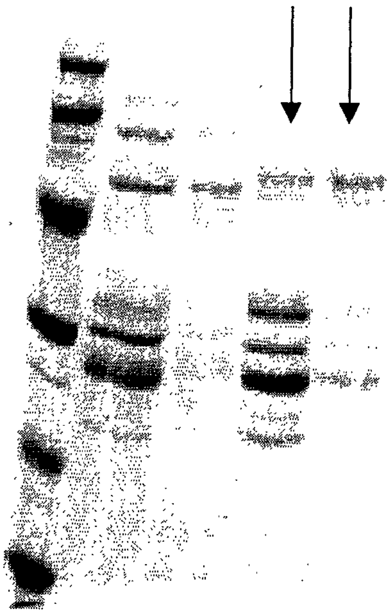

FIG. 98B

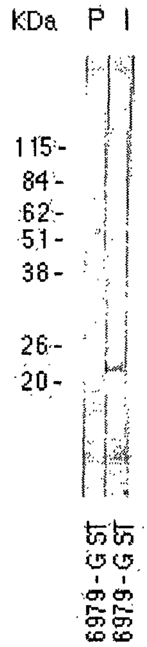


FIG. 98C

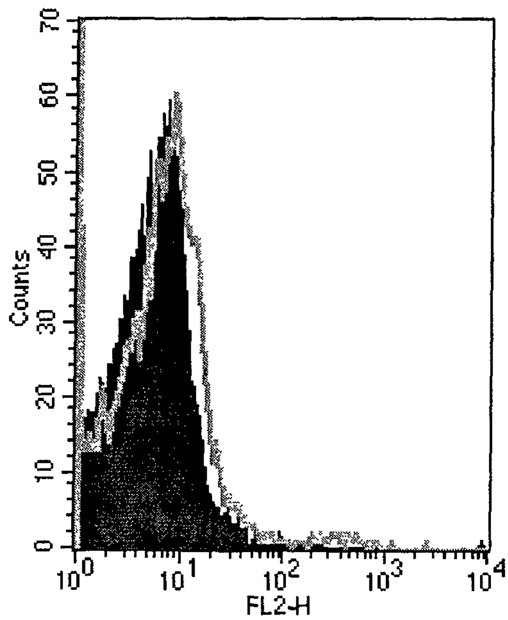

## FIGURE 99

**FIG. 99A**

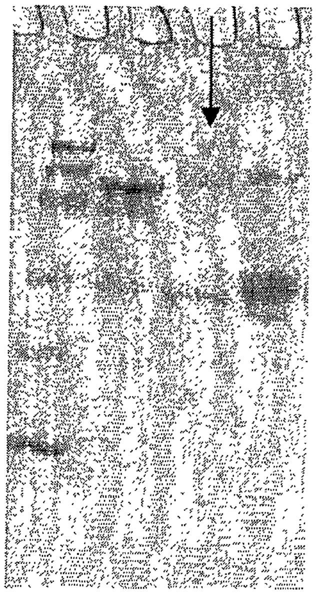

**FIG. 99B**

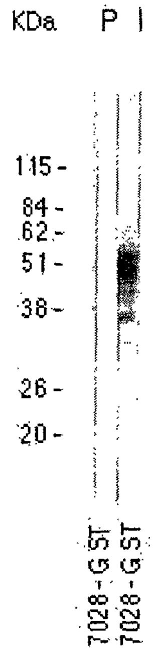


**FIG. 99C**

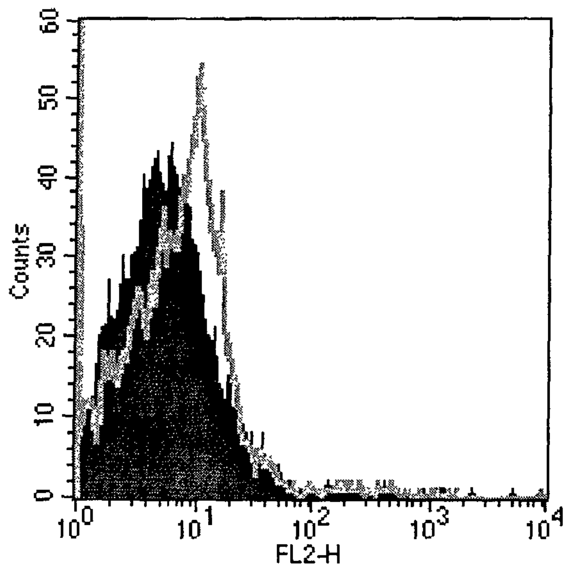

## FIGURE 100

*FIG. 100A*

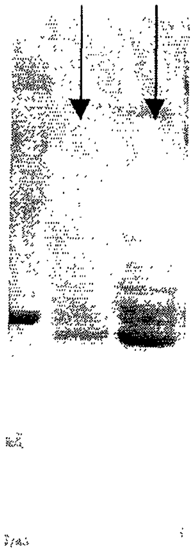

*FIG. 100B*

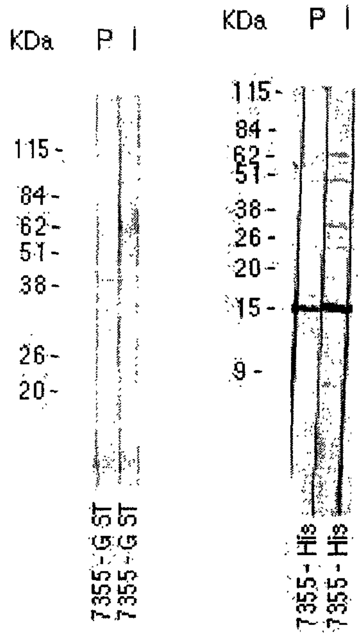


*FIG. 100C*

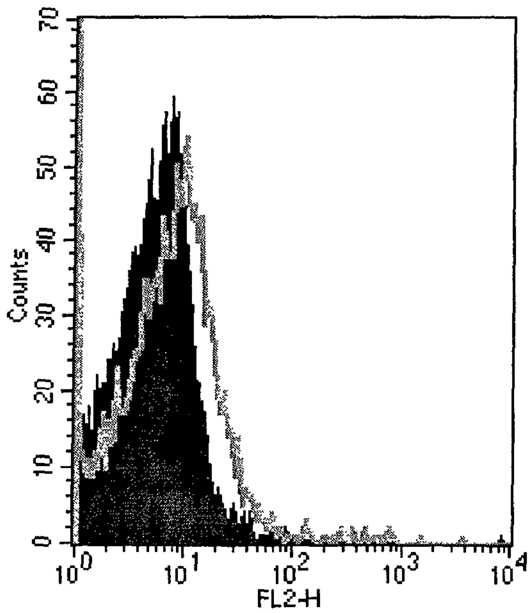

## FIGURE 101

**FIG. 101A**

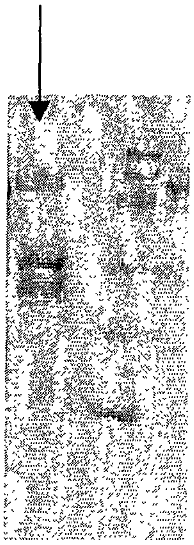

**FIG. 101B**

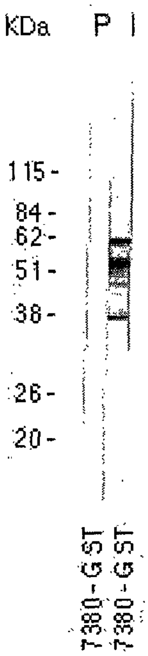


**FIG. 101C**

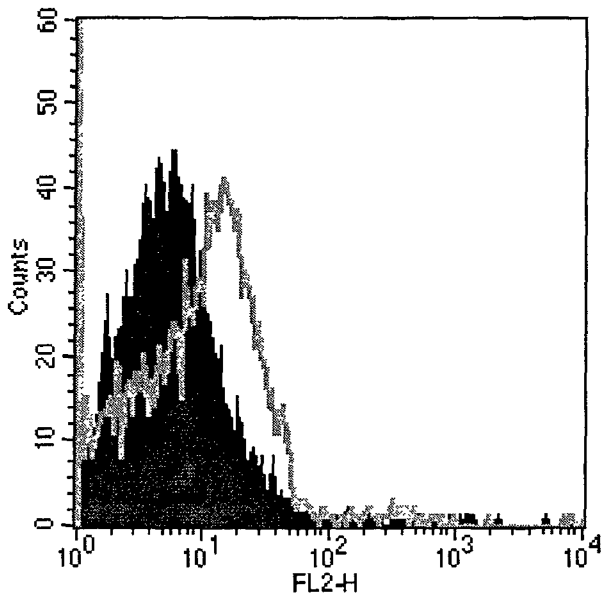

## FIGURE 102

FIG. 102A

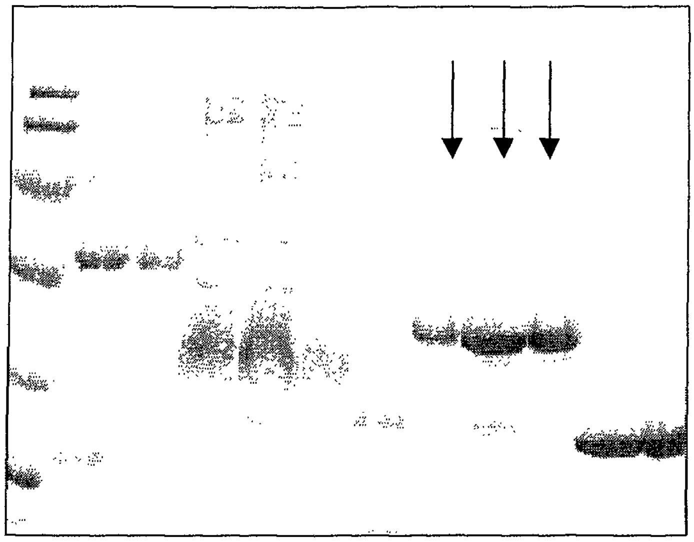

FIG. 102B

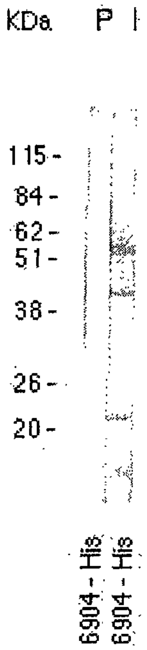

# ***FIGURE 103***

***FIG. 103A***

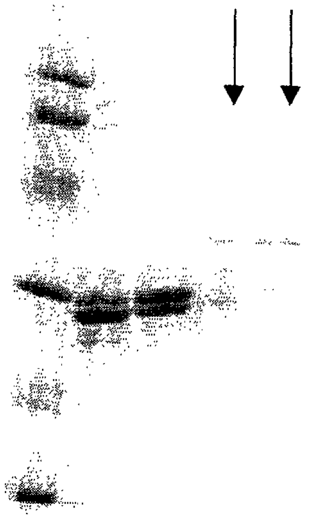

***FIG. 103C***

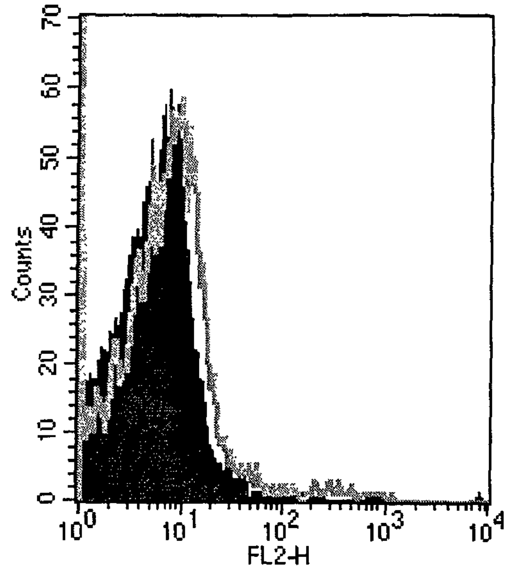


***FIG. 103B***

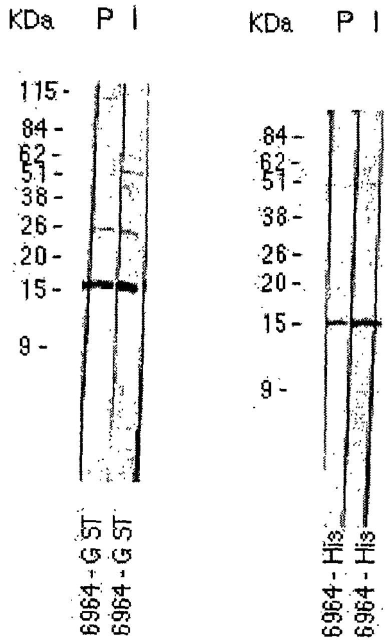

## FIGURE 104

FIG. 104A

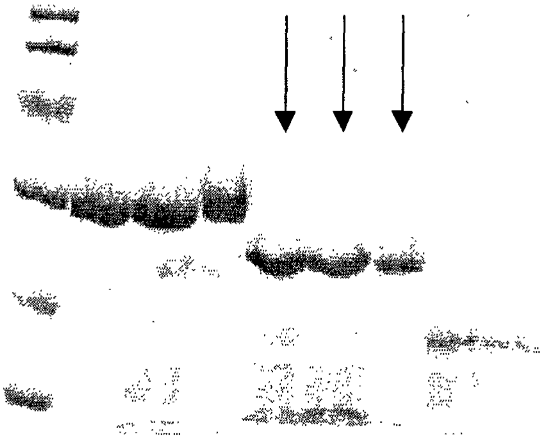

FIG. 104B

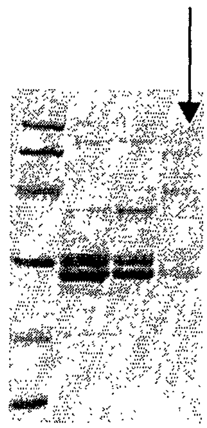

FIG. 104C

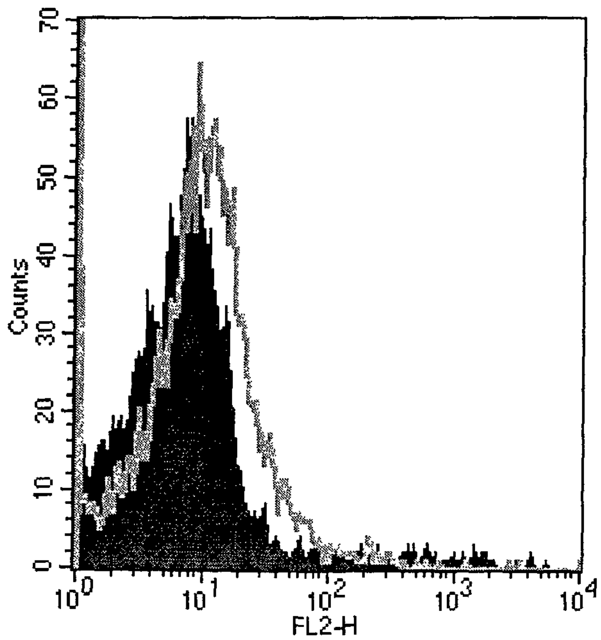

# FIGURE 105

FIG. 105A

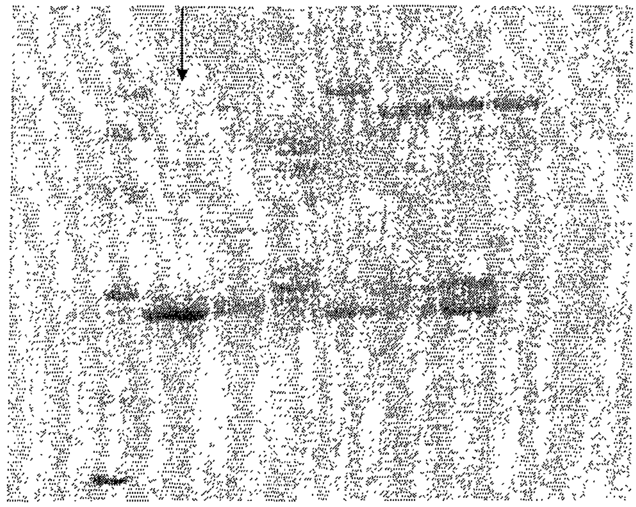

FIG. 105B

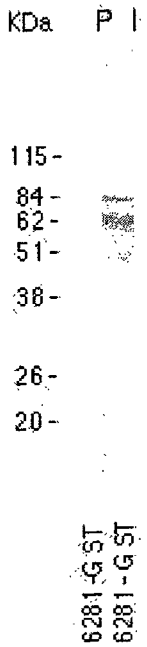

# FIGURE 106

Fig. 106A

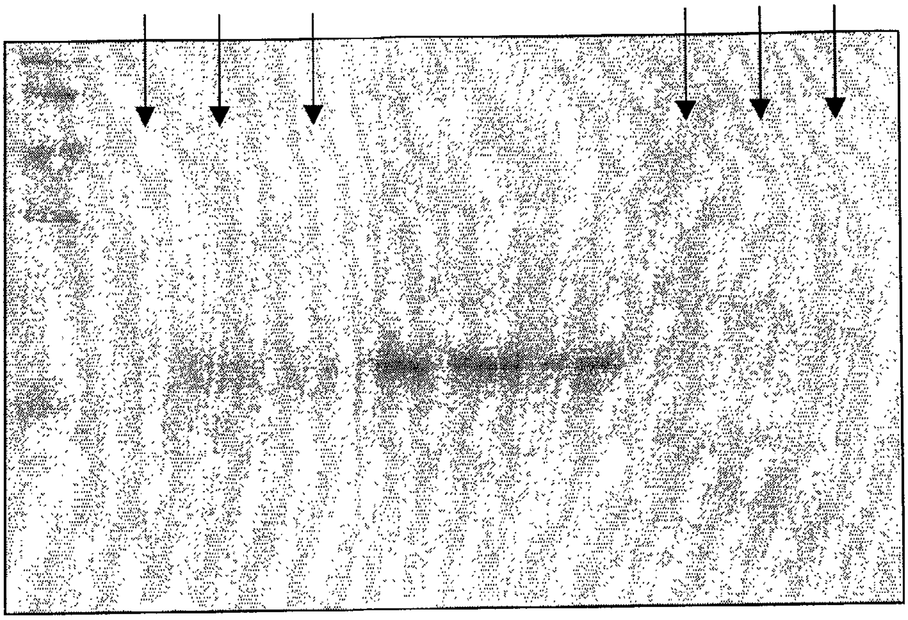

Fig. 106B

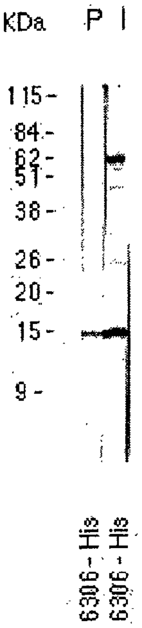


# FIGURE 107

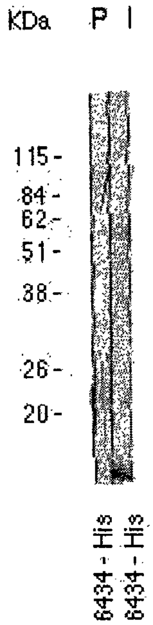

## FIGURE 108

FIG. 108A

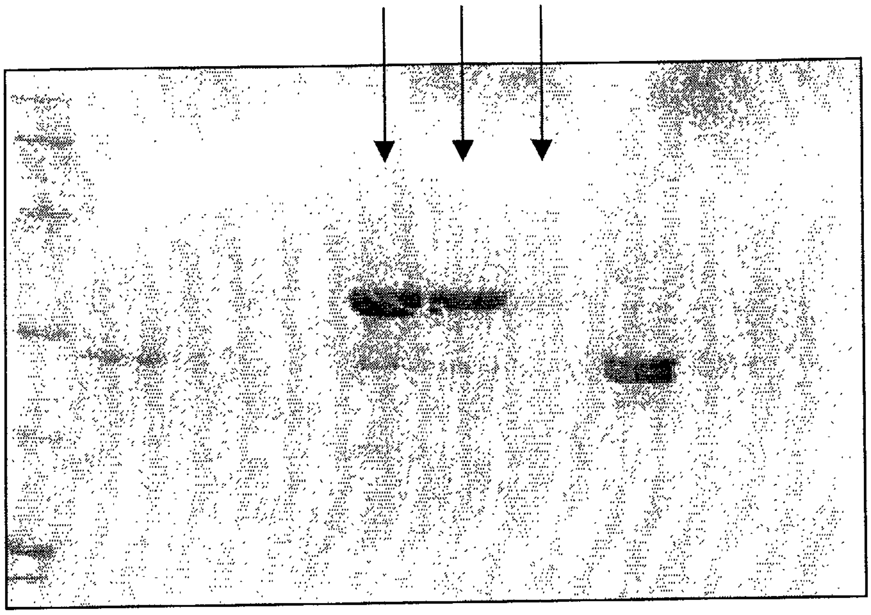

FIG. 108B

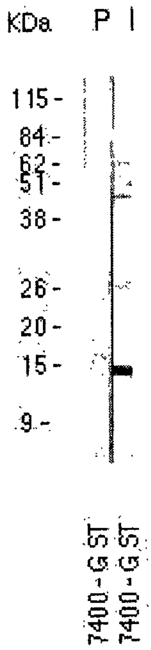

## FIGURE 109

FIG. 109A

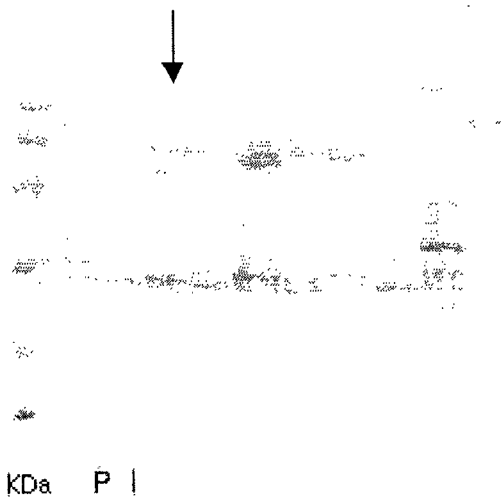


FIG. 109B

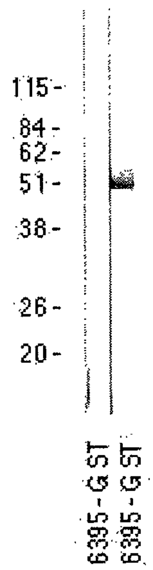

## FIGURE 110

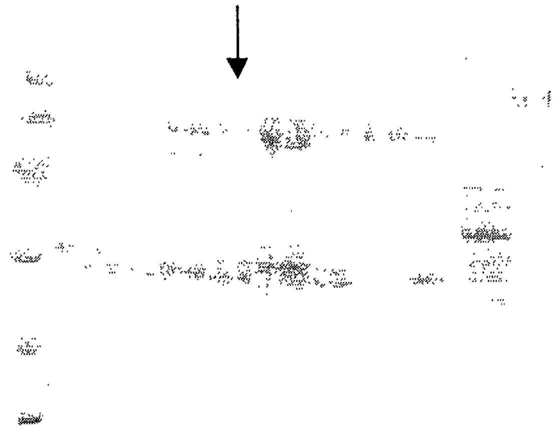

**FIG. 110A**

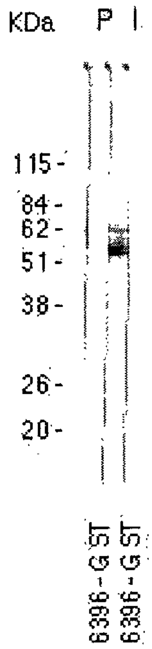


**FIG. 110B**

## FIGURE 111

FIG. 111A

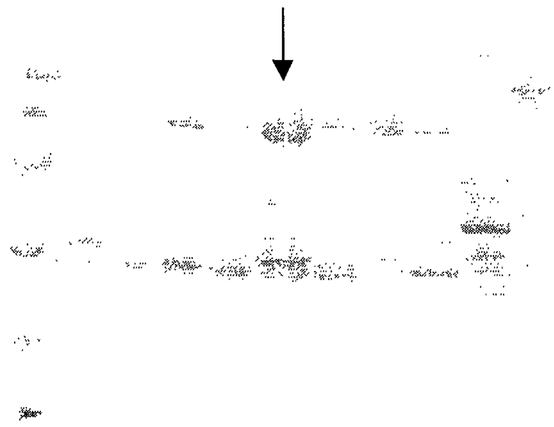

FIG. 111B

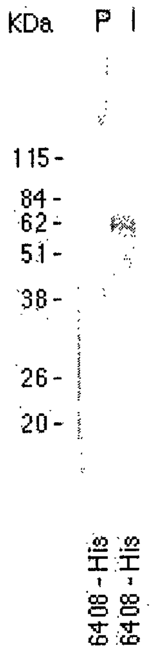

## FIGURE 112

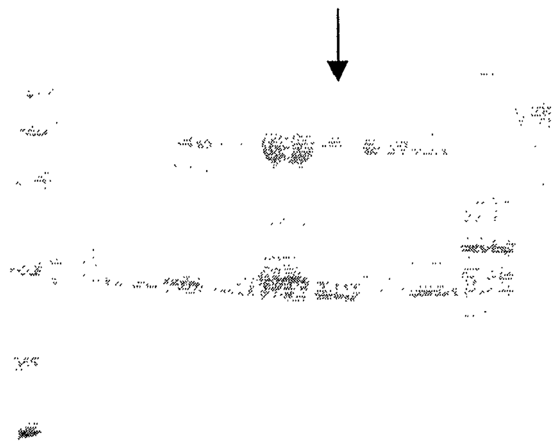

FIG. 112A

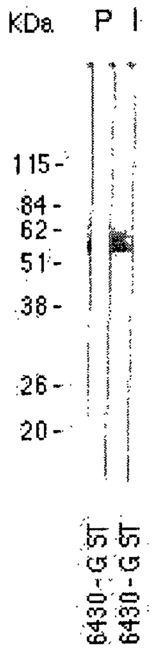


FIG. 112B

## FIGURE 113

FIG. 113A

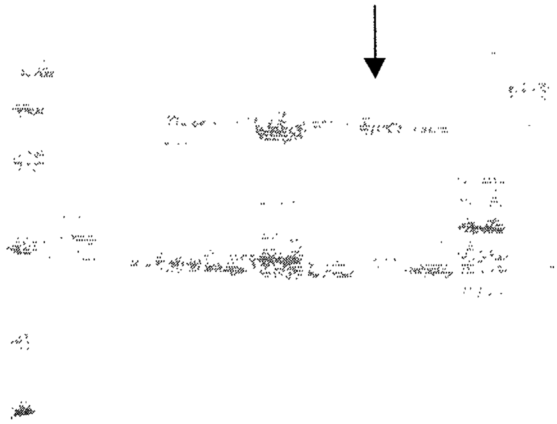

FIG. 113B

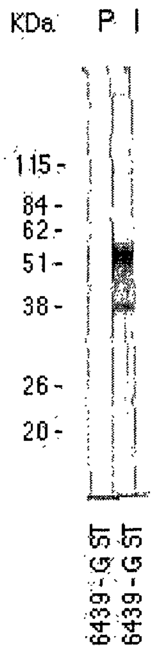

**FIGURE 114**

**FIG. 114A**

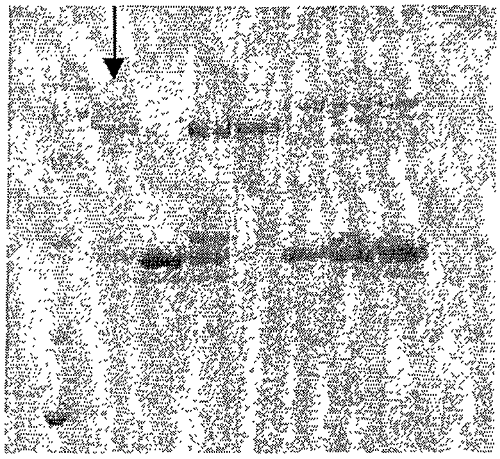

**FIG. 114B**

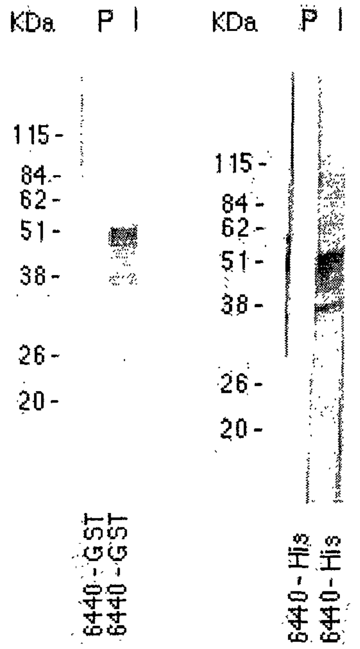

## FIGURE 115

FIG. 115A

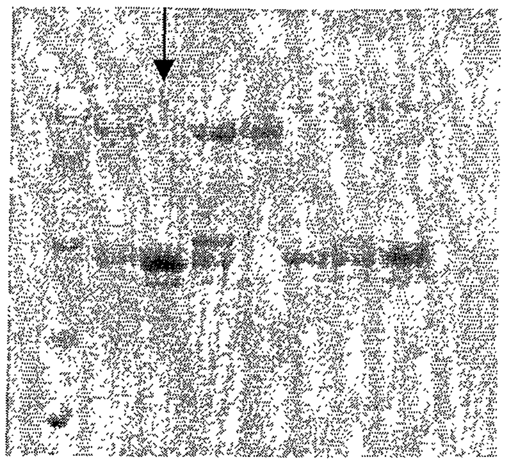

FIG. 115B

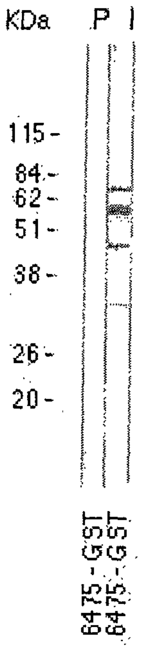

## FIGURE 116

FIG. 116A

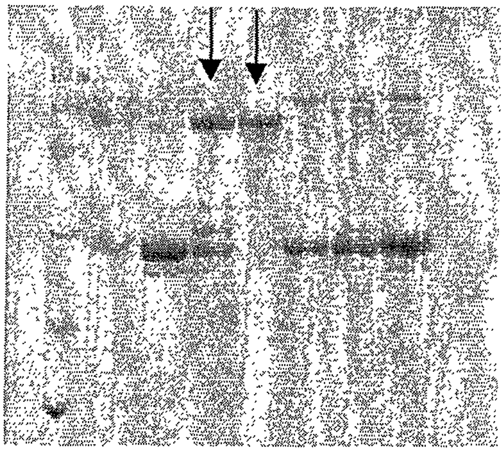

FIG. 116B

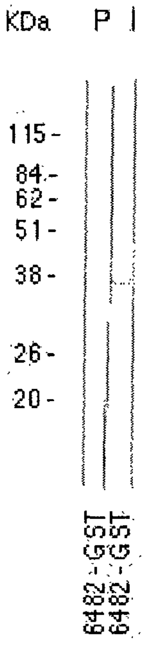

## FIGURE 117

*FIG. 117A*

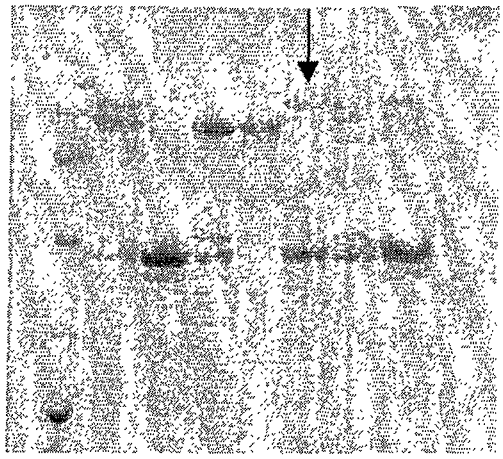

*FIG. 117B*

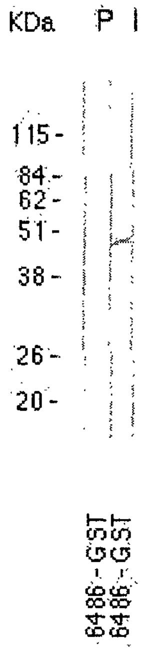

# FIGURE 118

FIG. 118A

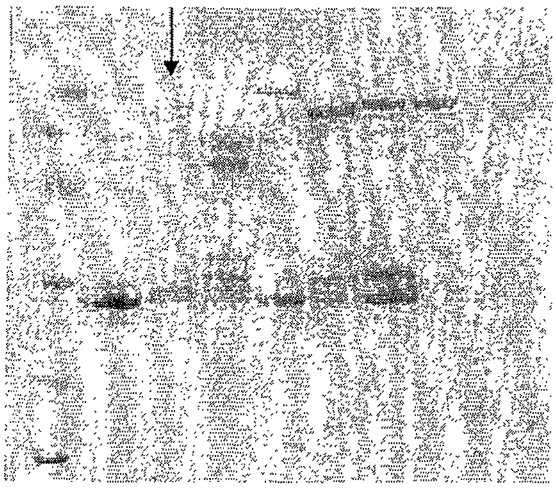

FIG. 118B

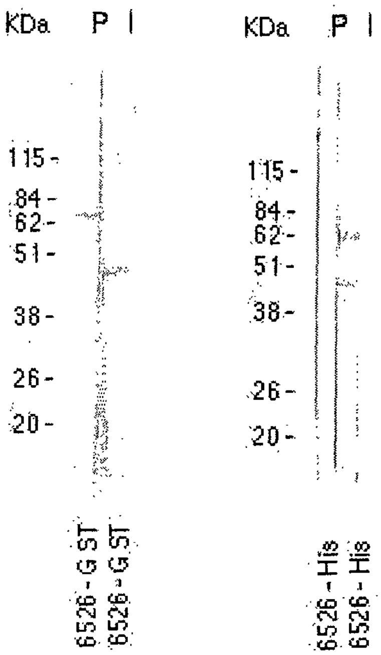

**FIGURE 119**

**FIG. 119A**

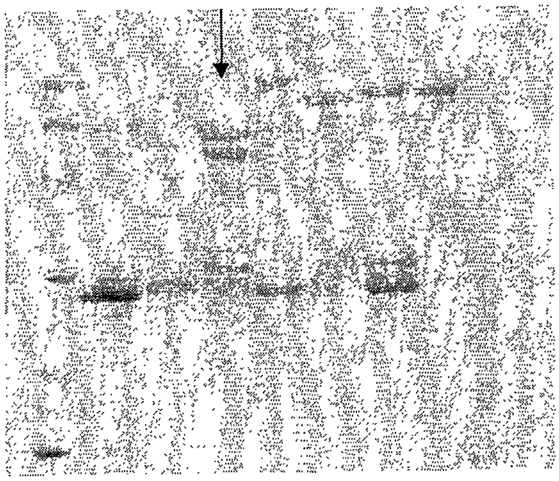

**FIG. 119B**

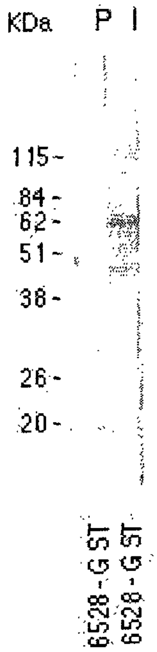

**FIGURE 120**

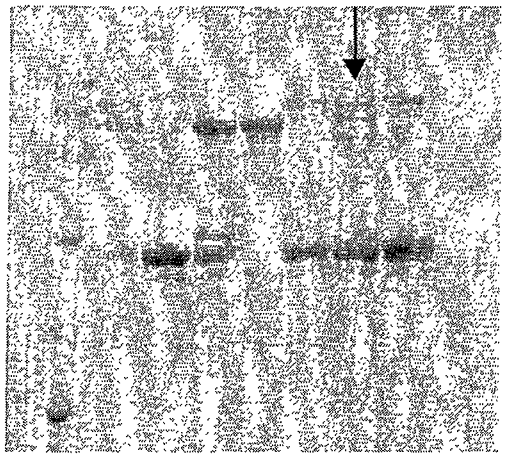

**FIG. 120A**

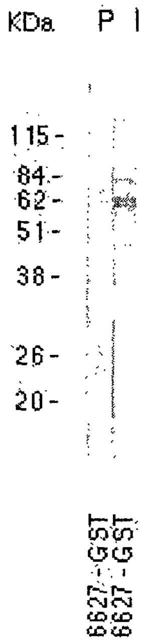


**FIG. 120B**

## FIGURE 121

**FIG. 121A**

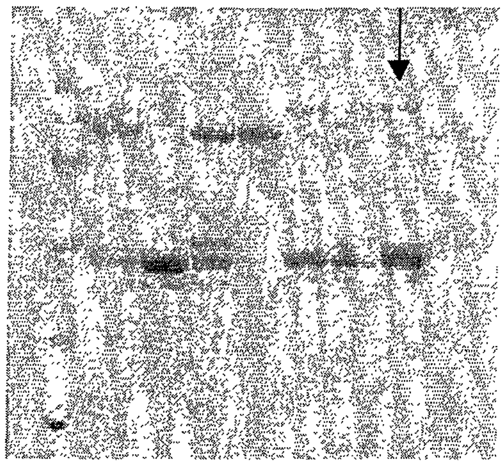

**FIG. 121B**

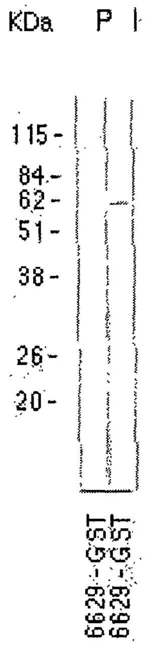

# FIGURE 122

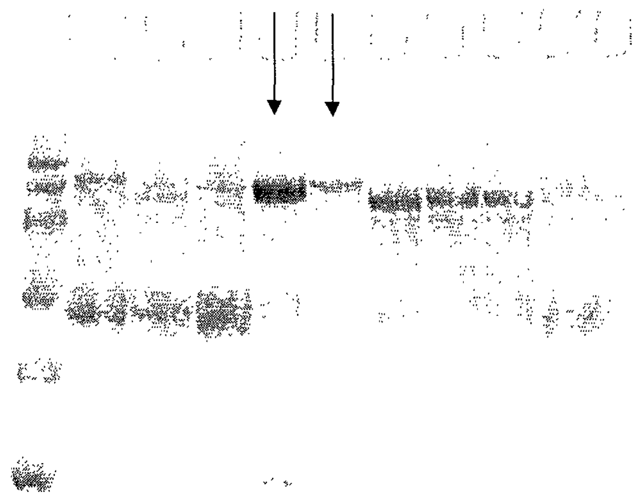

FIG. 122A

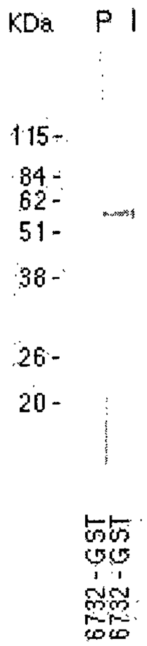


FIG. 122B

# FIGURE 123

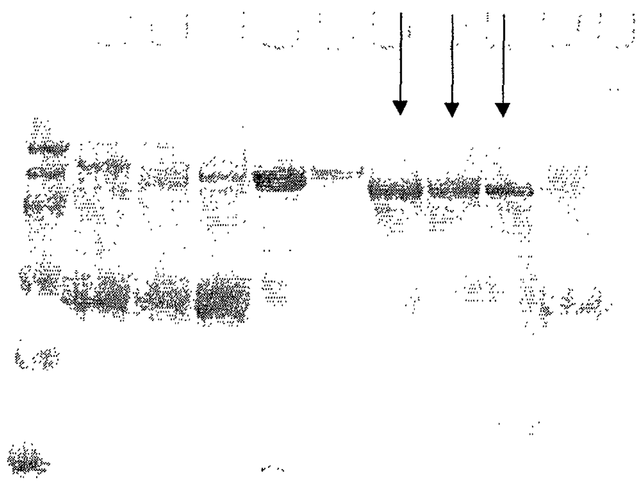

FIG. 123A

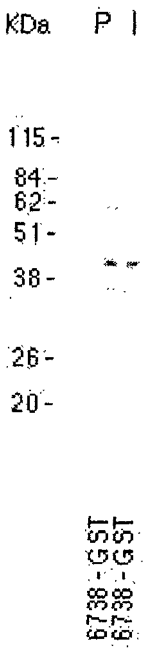


FIG. 123B

## FIGURE 124

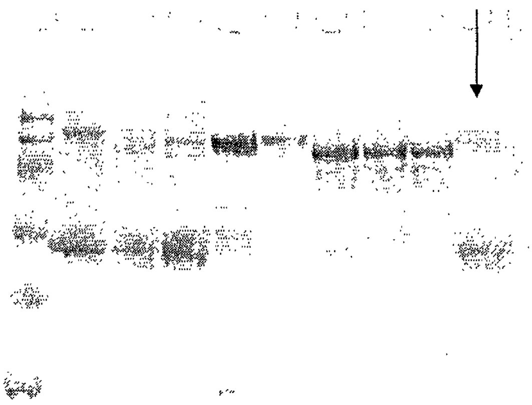

**FIG. 124A**

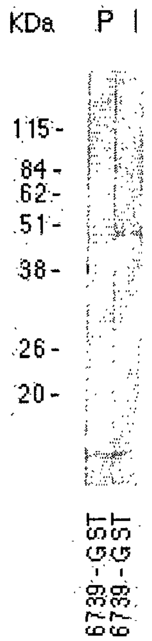


**FIG. 124B**

# FIGURE 125

**FIG. 125A**

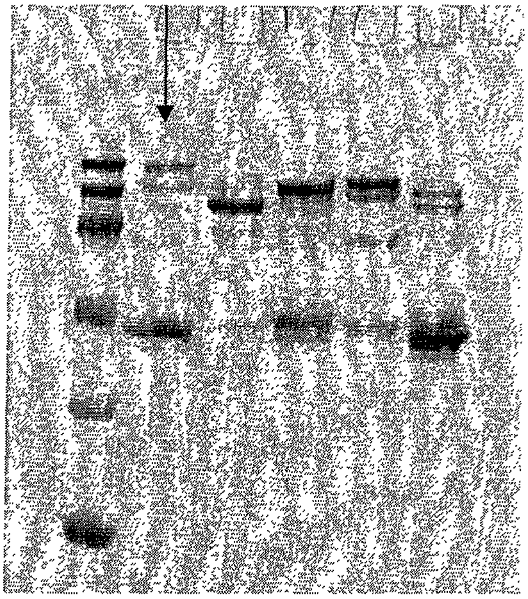

**FIG. 125B**

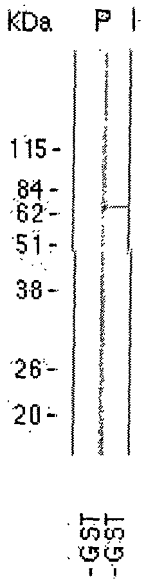

# FIGURE 126

FIG. 126A

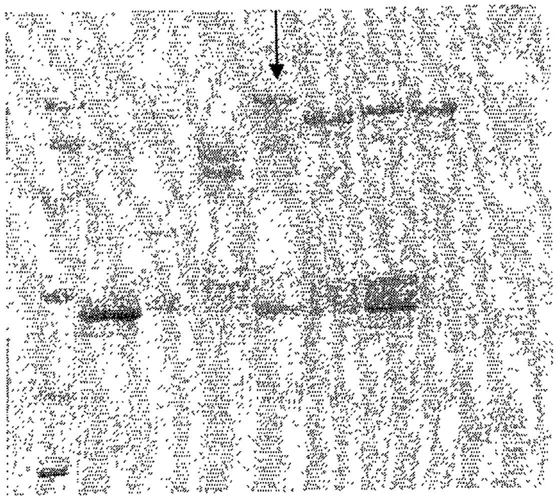

FIG. 126B

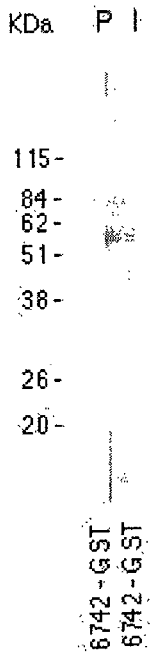

## FIGURE 127

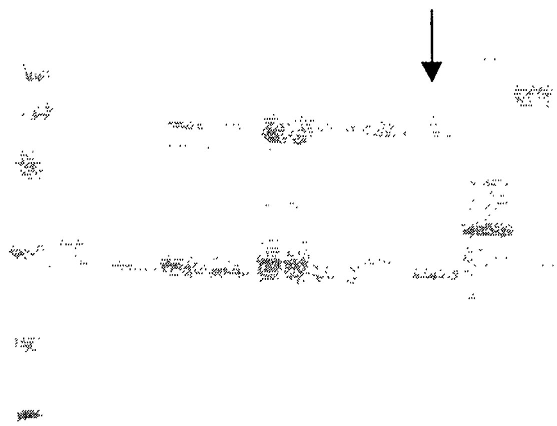

**FIG. 127A**

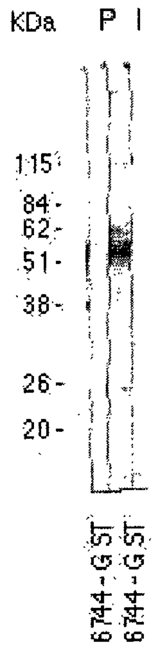


**FIG. 127B**

# FIGURE 128

FIG. 128A

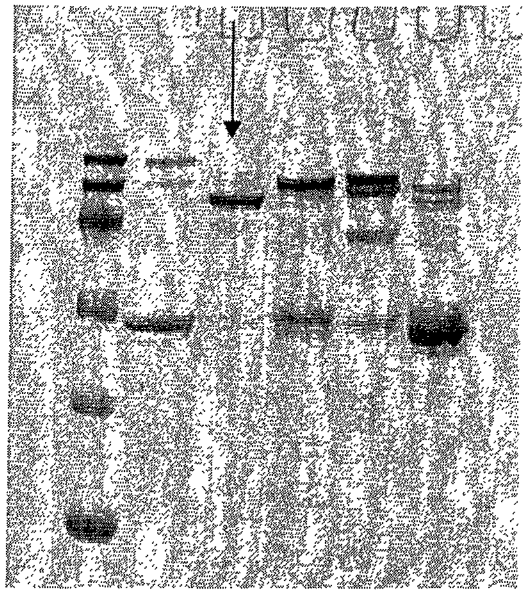

FIG. 128B

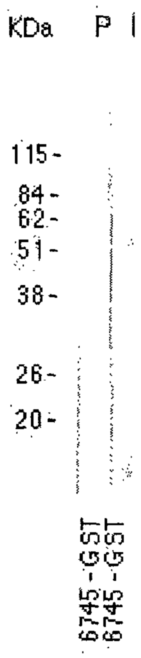

## FIGURE 129

FIG. 129A

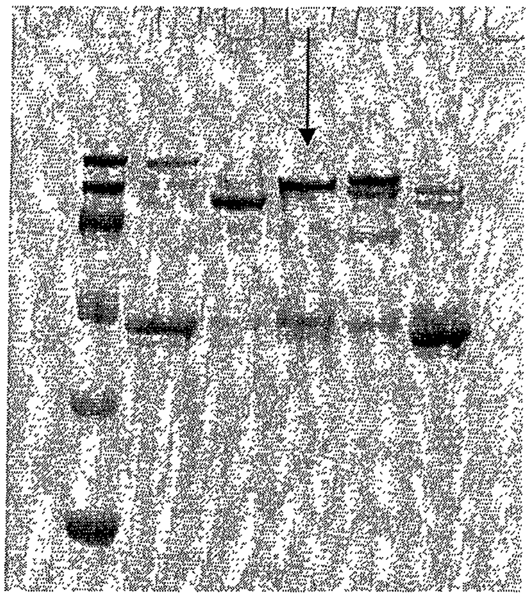

FIG. 129B

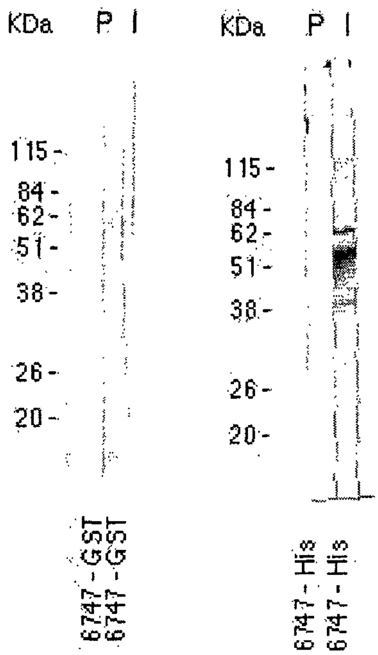

FIGURE 130

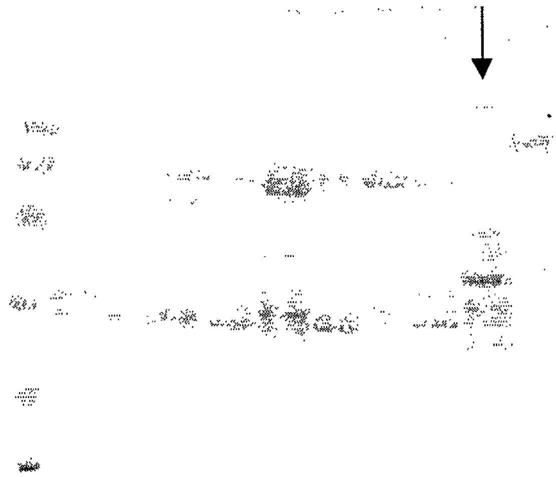

FIG. 130A

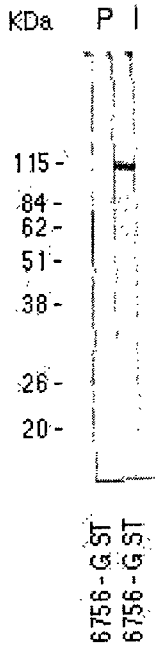


FIG. 130B

# FIGURE 131

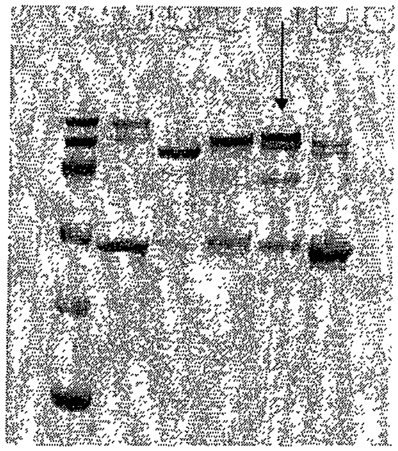

FIG. 131A

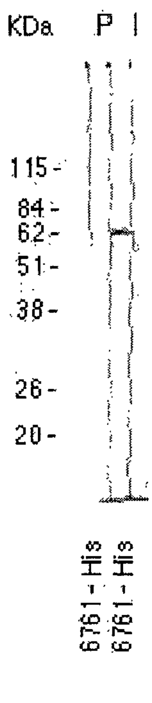


FIG. 131B

# FIGURE 132

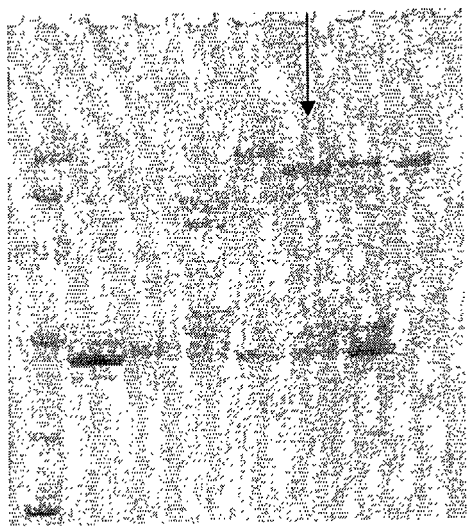

FIG. 132A

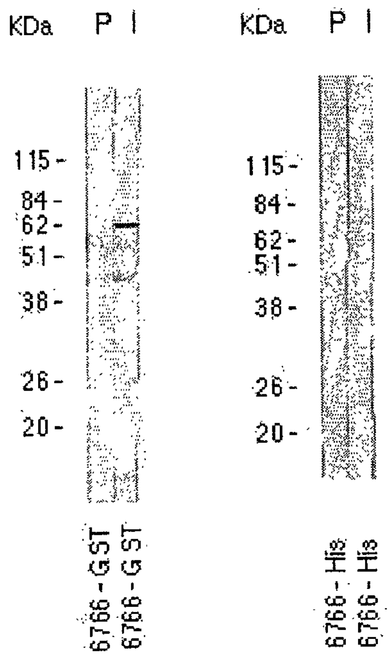


FIG. 132B

# FIGURE 133

**FIG. 133A**

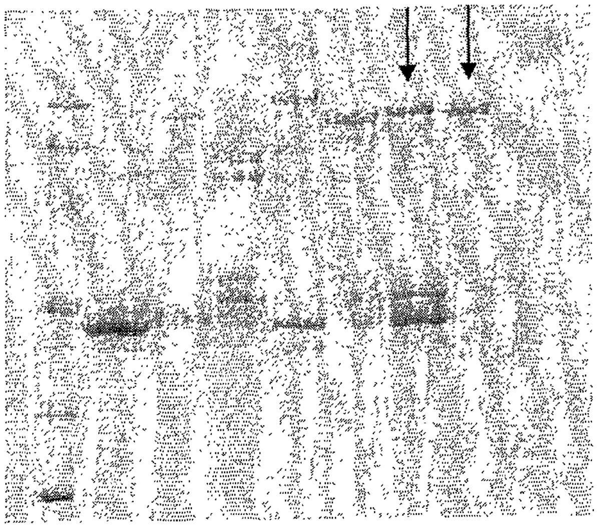

**FIG. 133B**

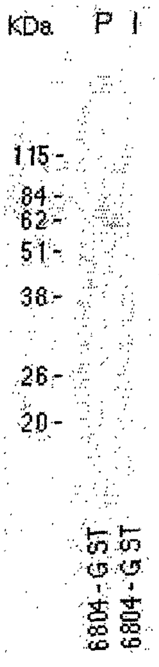

# FIGURE 134

FIG. 134A

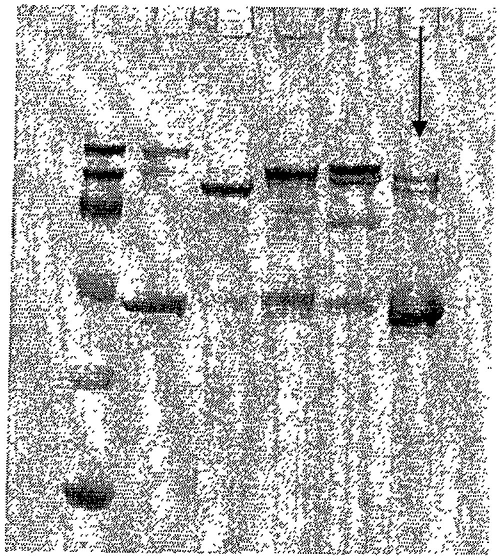

FIG. 134B

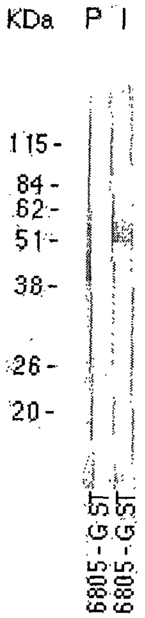

## FIGURE 135

**FIG. 135A**

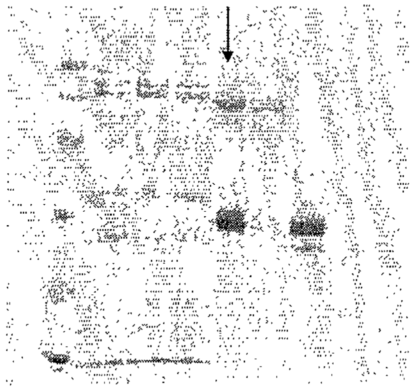

**FIG. 135B**

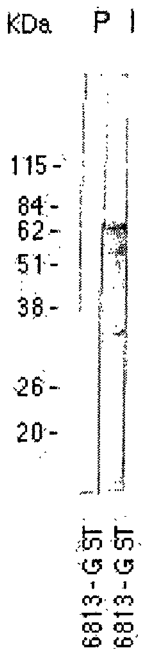

## FIGURE 136

**FIG. 136A**

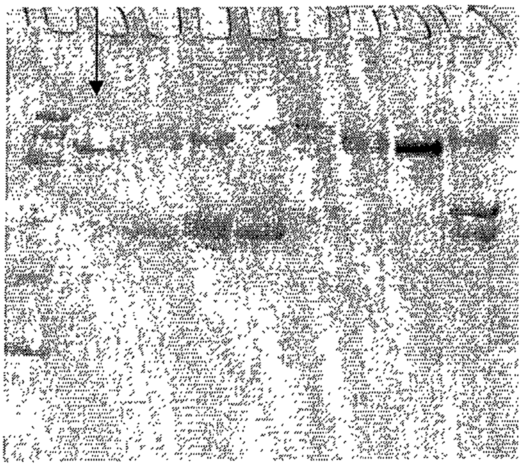

**FIG. 136B**

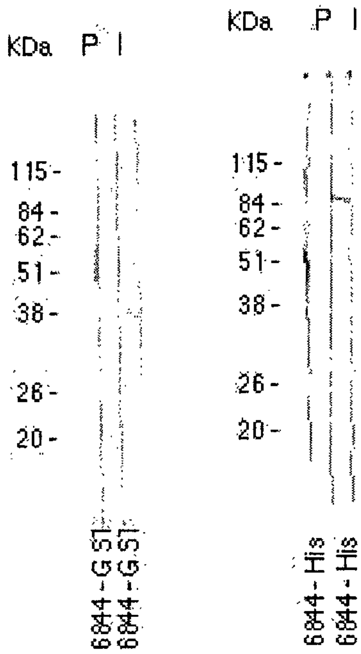

**FIGURE 137**

**FIG. 137A**

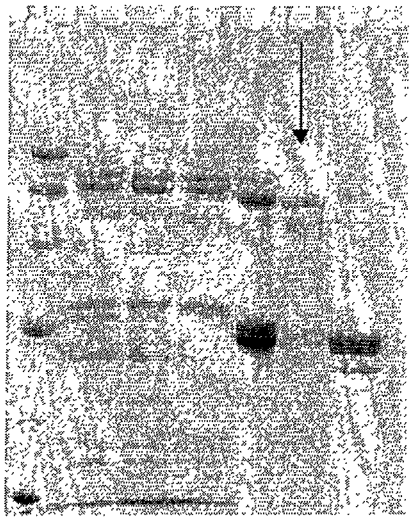

**FIG. 137B**

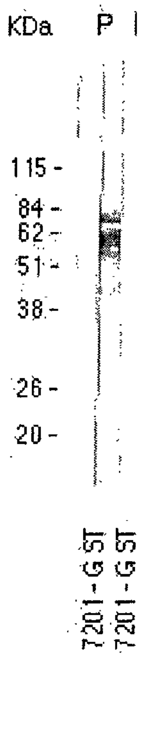

## FIGURE 138

**FIG. 138A**

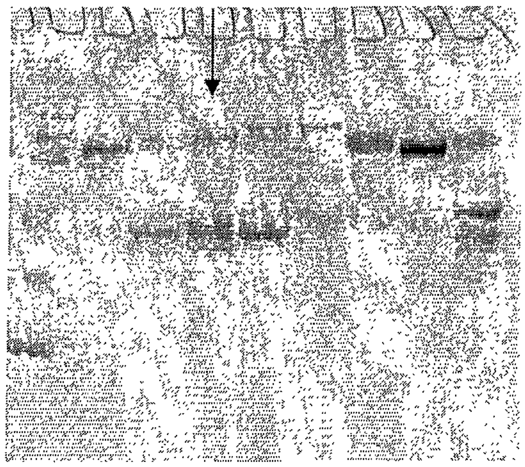

**FIG. 138B**

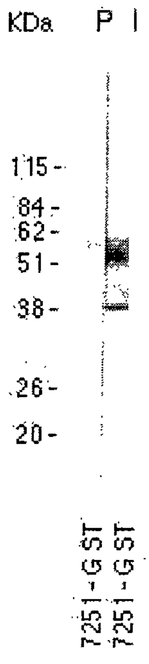

**FIGURE 139**

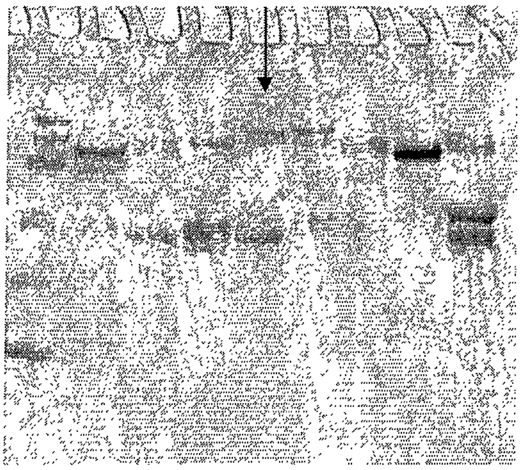

**FIG. 139A**

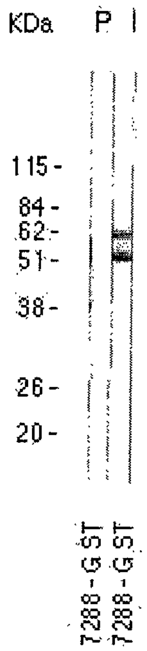


**FIG. 139B**

## FIGURE 140

FIG. 140A

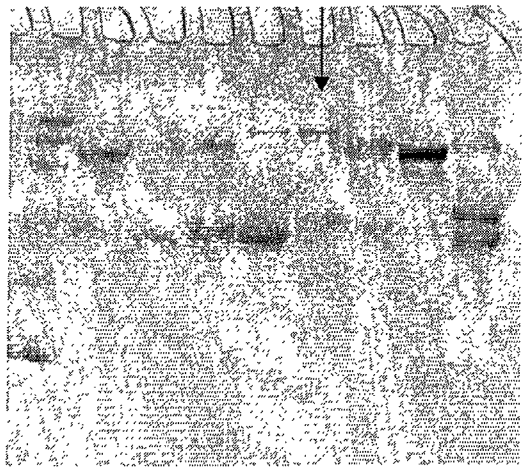

FIG. 140B

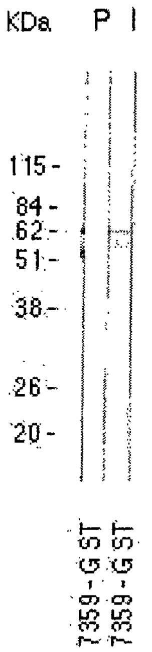

**FIGURE 141**

**FIG. 141A**

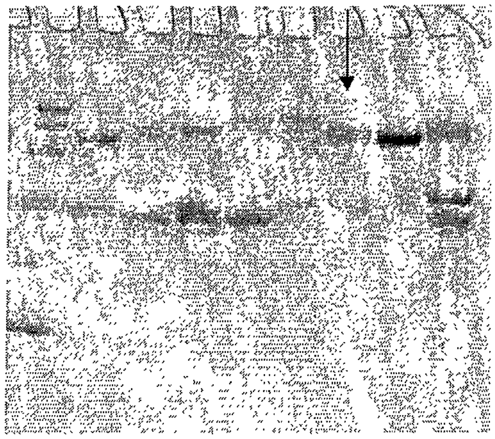

**FIG. 141B**

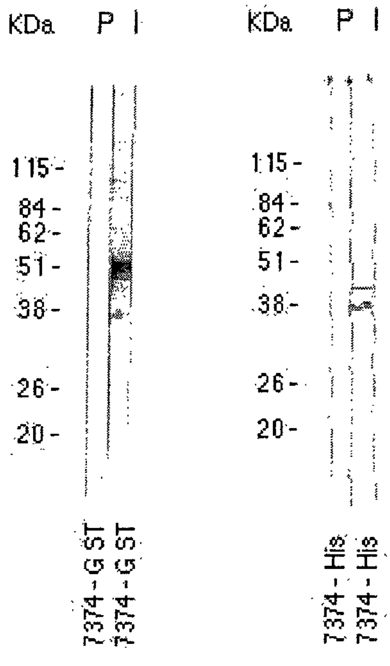

## FIGURE 142

**FIG. 142A**

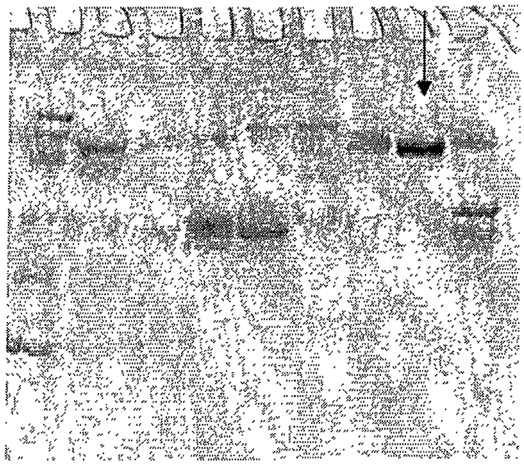

**FIG. 142B**

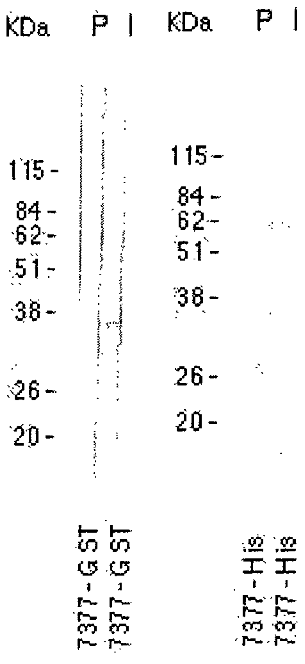

# FIGURE 143

FIG. 143A

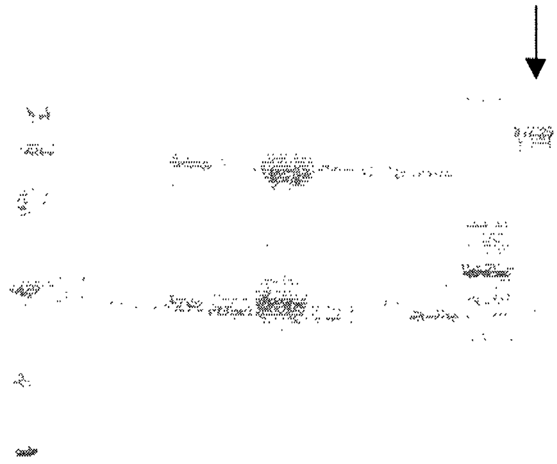

FIG. 143B

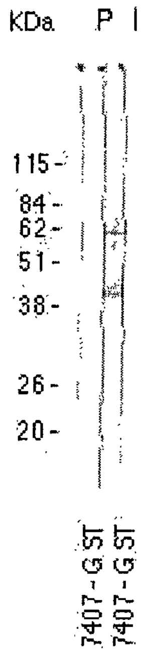

## FIGURE 144

FIG. 144A

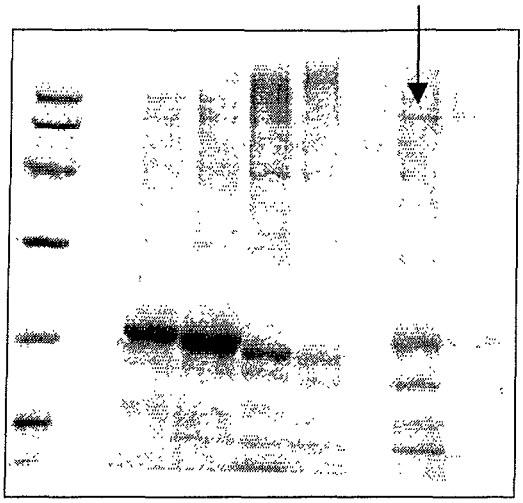

FIG. 144B

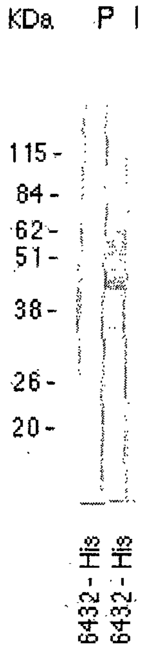

## FIGURE 145

**FIG. 145A**

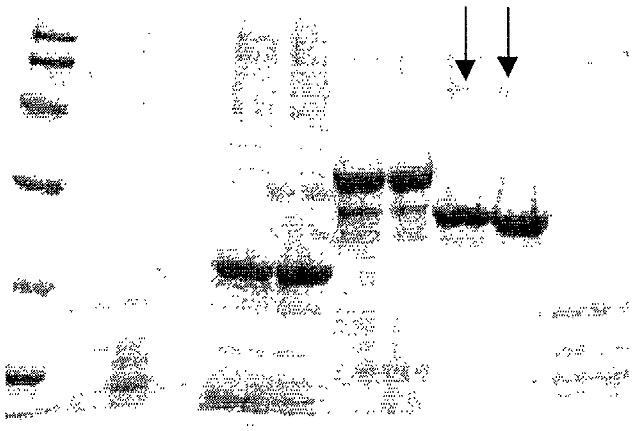

**FIG. 145B**

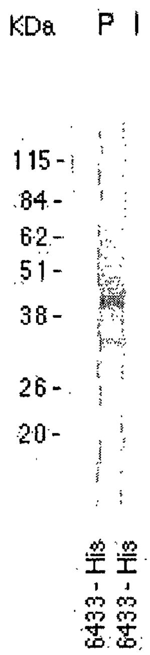

## FIGURE 146

FIG. 146A

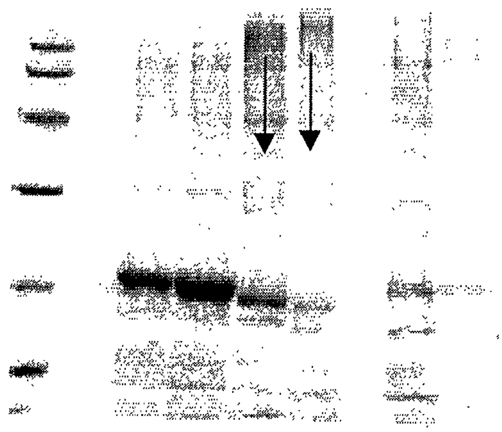

FIG. 146B

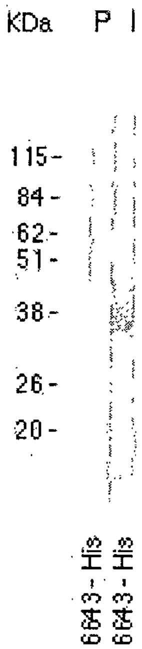

## FIGURE 147

FIG. 147A

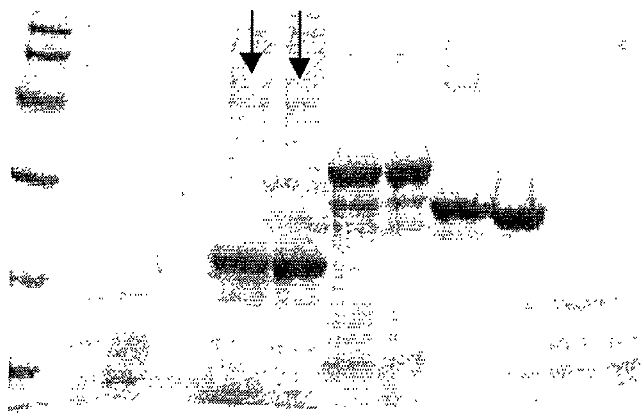

FIG. 147B

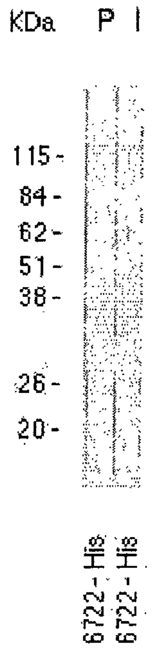

## FIGURE 148

FIG. 148A

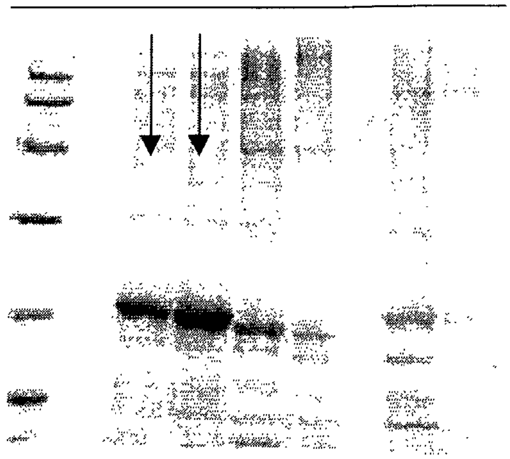

FIG. 148B

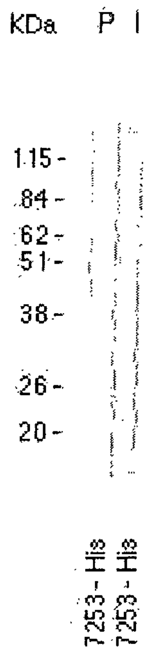

# FIGURE 149

FIG. 149A

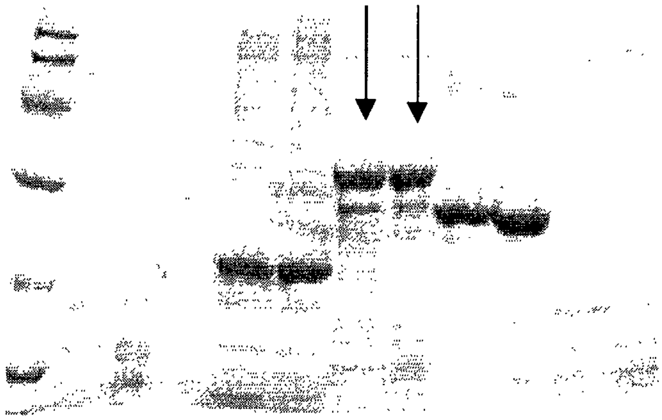

FIG. 149B

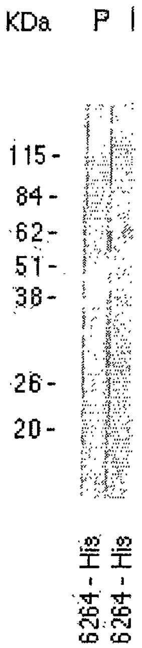

## FIGURE 150

FIG. 150A

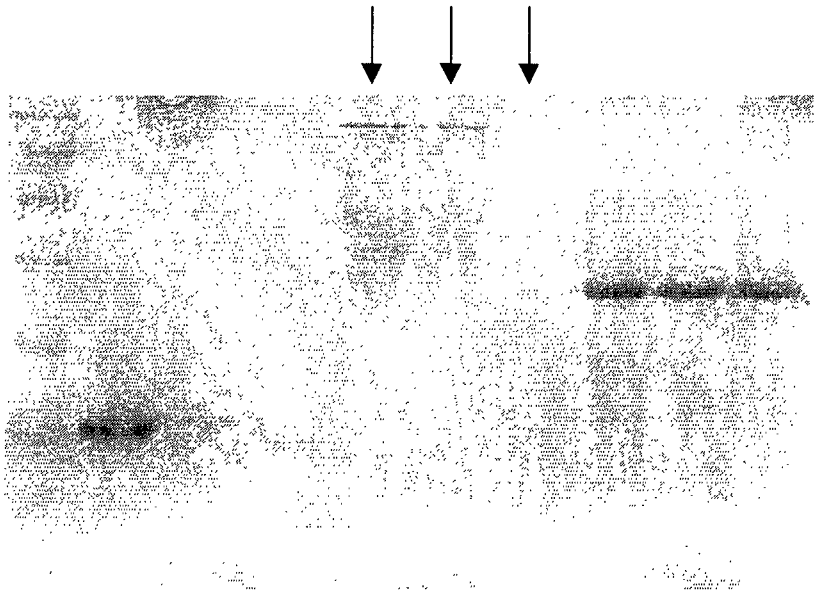

FIG. 150B

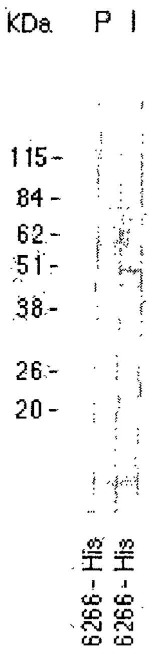

# FIGURE 151

FIG. 151A

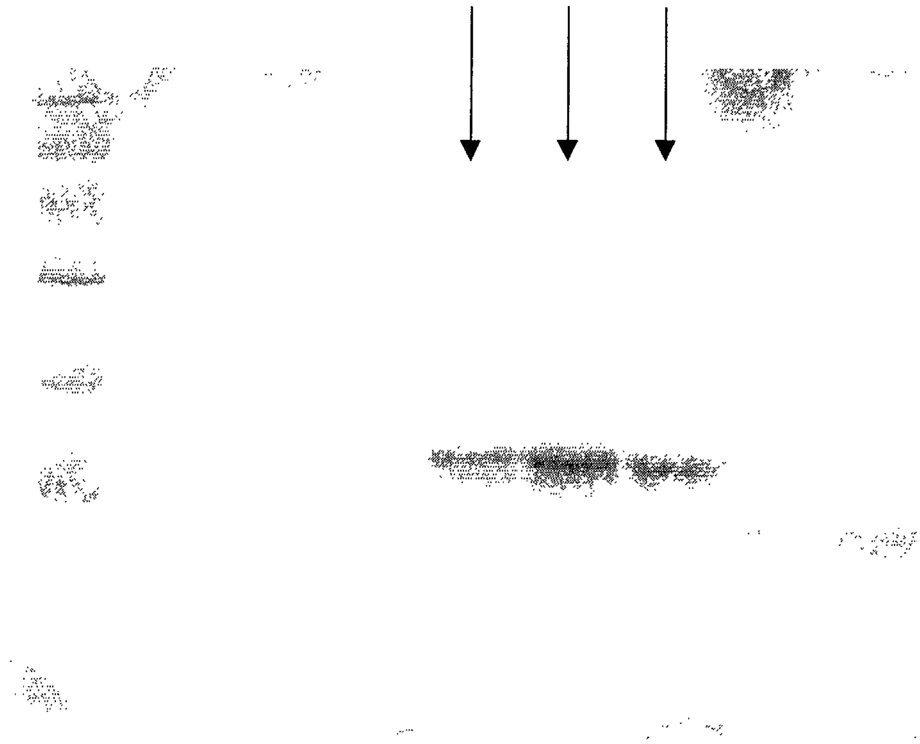

FIG. 151B

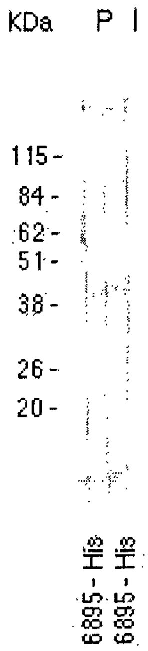

# FIGURE 152

FIG. 152A

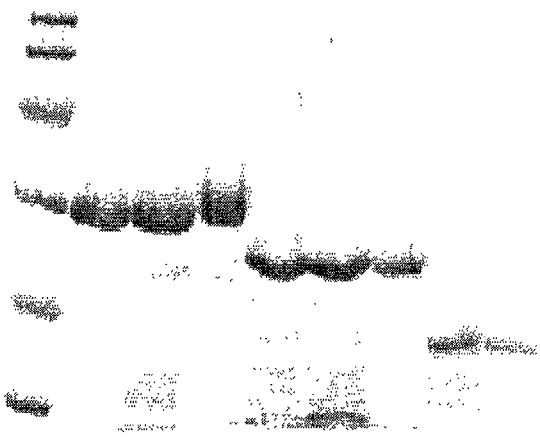

FIG. 152B

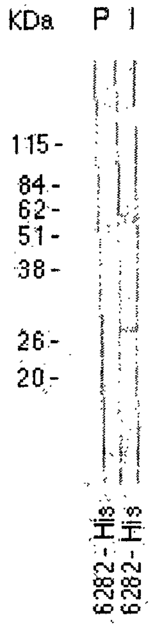


FIGURE 153

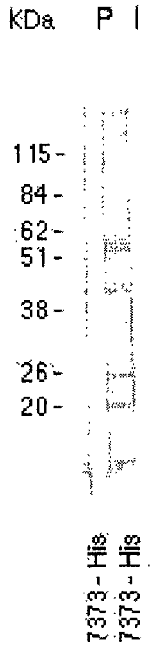

## FIGURE 154

**FIG. 154A**

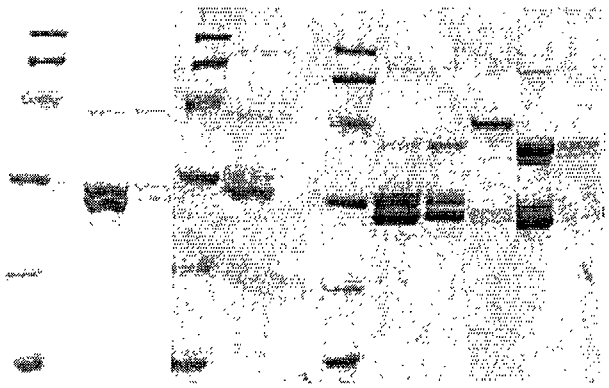

**FIG. 154B**

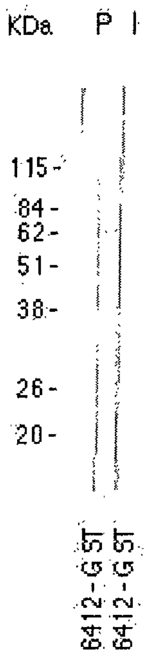


## FIGURE 155

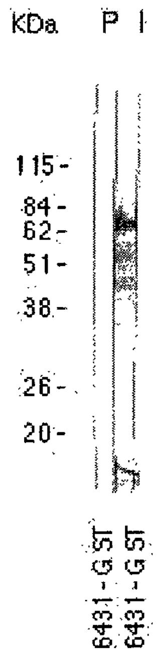

## FIGURE 156

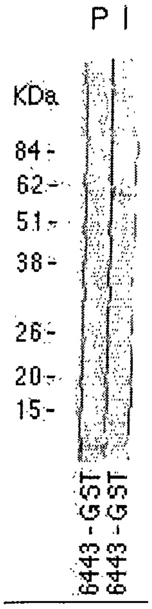


## FIGURE 157

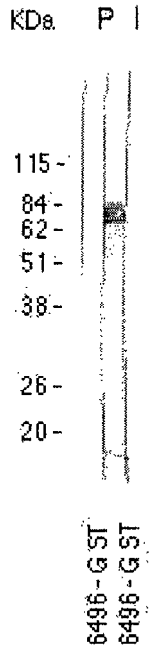


## FIGURE 158

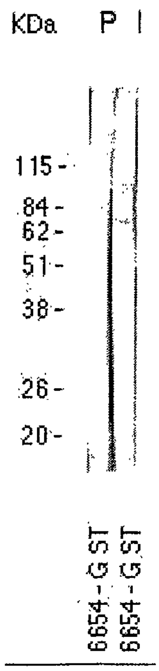

## FIGURE 159

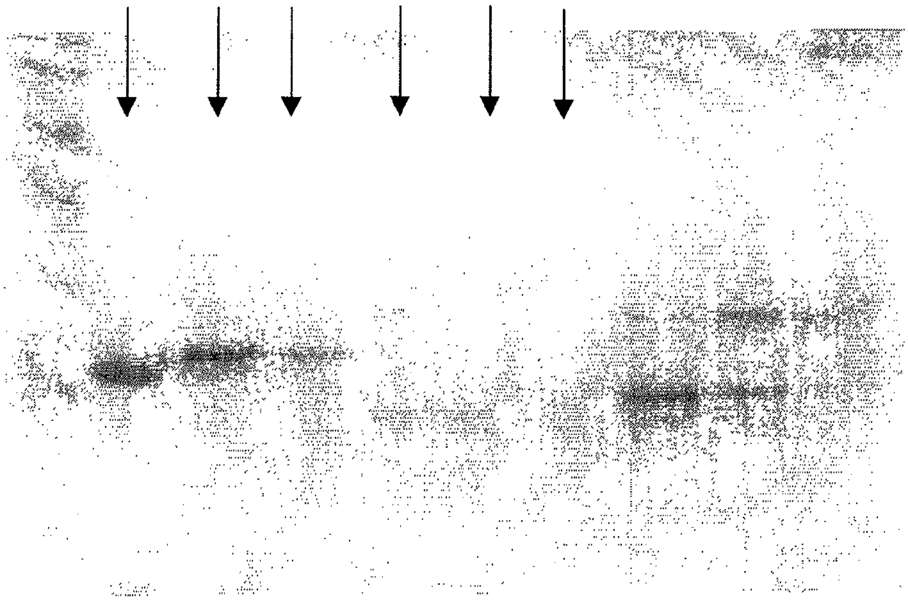

FIG. 159A

FIG. 159B

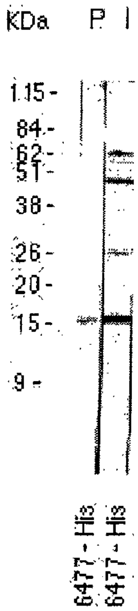


## FIGURE 160

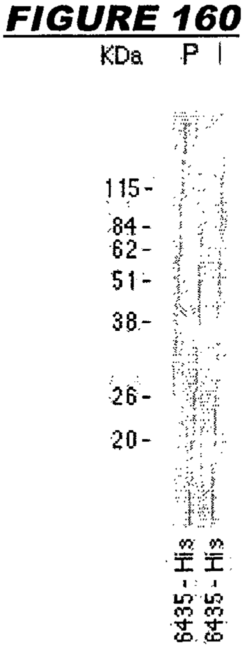

## FIGURE 161

FIG. 161A

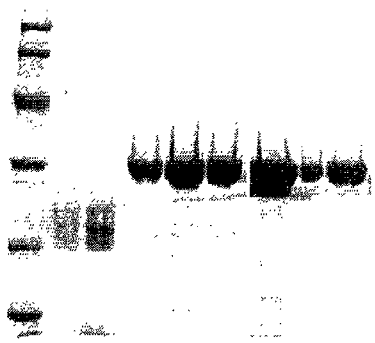

FIG. 161B

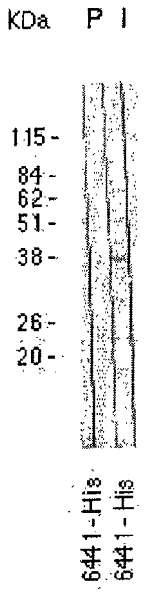


## FIGURE 162

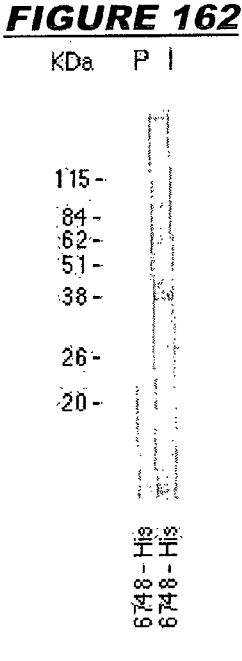


## FIGURE 163

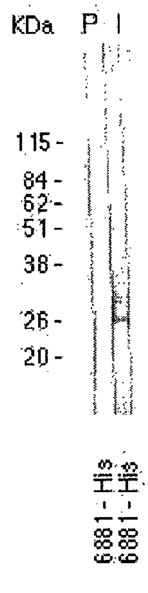

# FIGURE 164

FIG. 164A

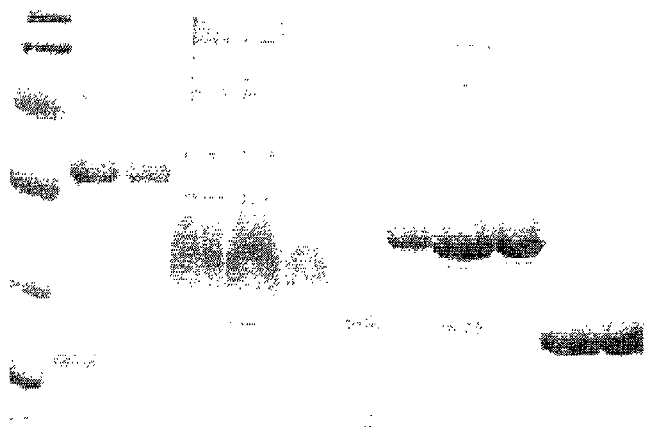

FIG. 164B

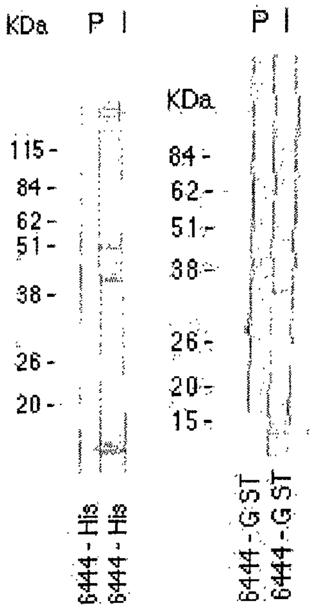


# FIGURE 165

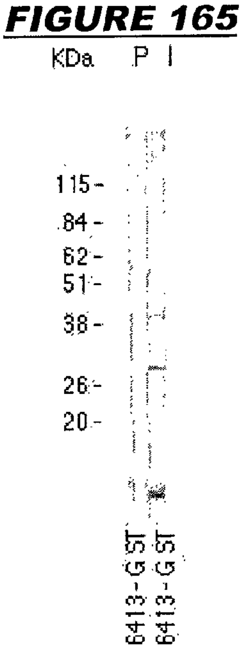


# FIGURE 166

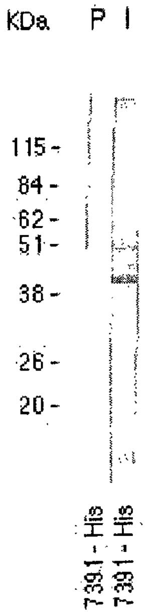

## FIGURE 167

**FIG. 167A**

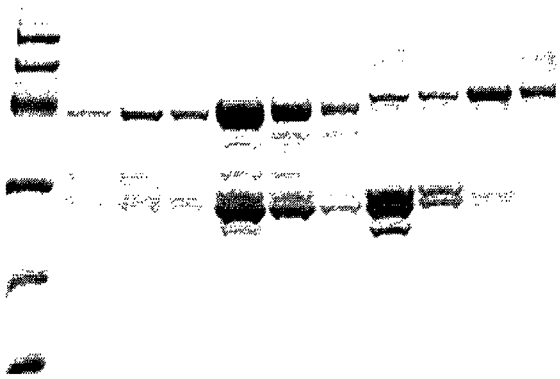

**FIG. 167B**

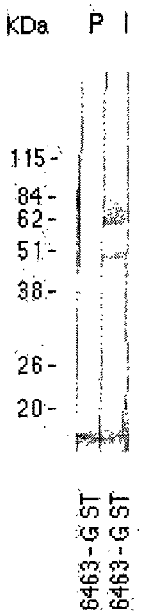


## FIGURE 168

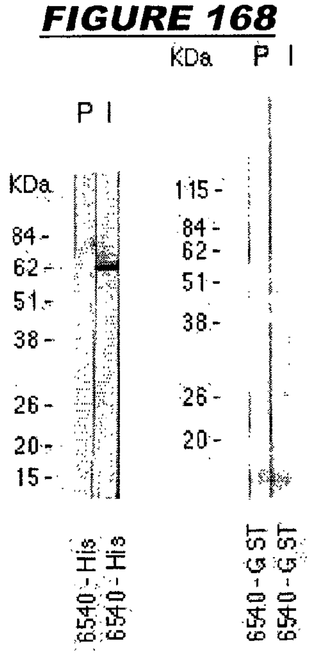

## FIGURE 169

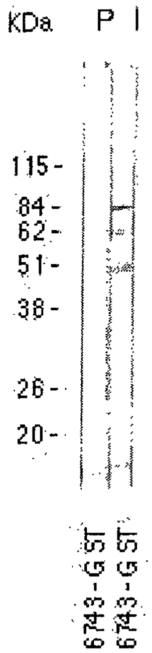


## FIGURE 170

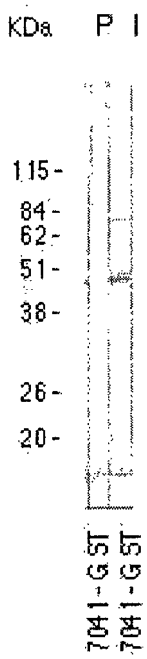

## FIGURE 171

**Fig. 171A**

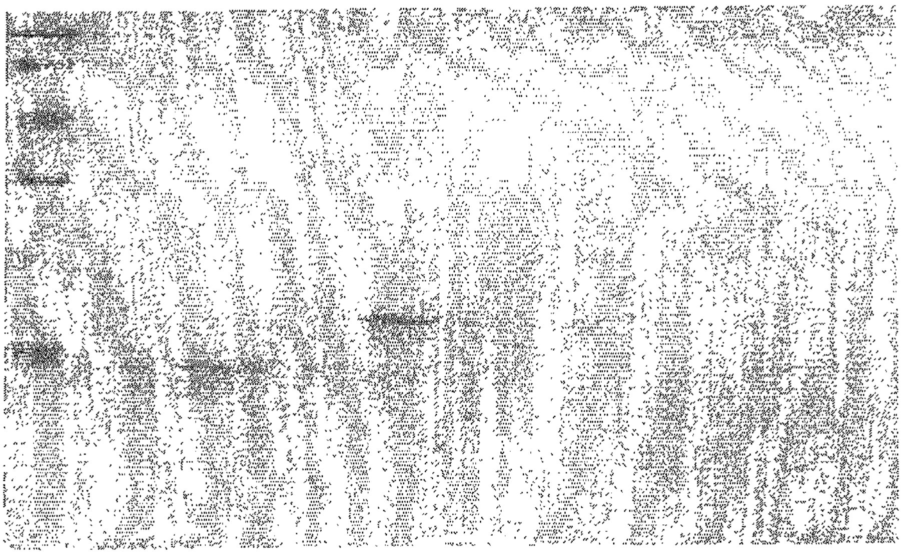

**Fig. 171B**

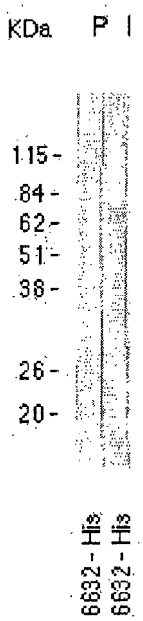


## FIGURE 172

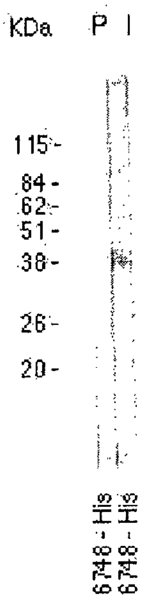


## FIGURE 173

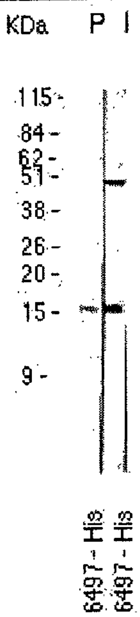

## FIGURE 174

**FIG. 174A**

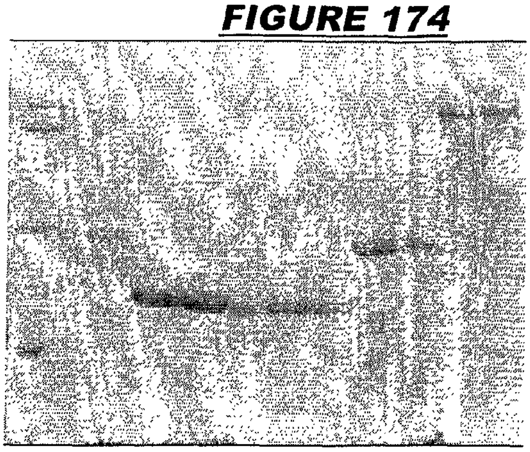

**FIG. 174B**

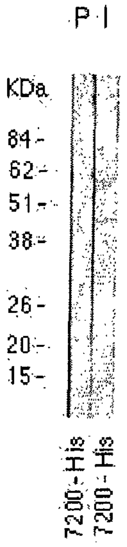


## FIGURE 175

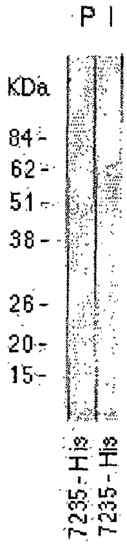


## FIGURE 176

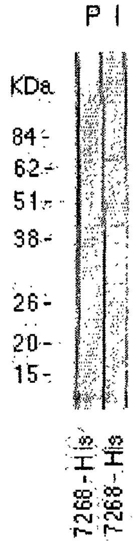


## FIGURE 177

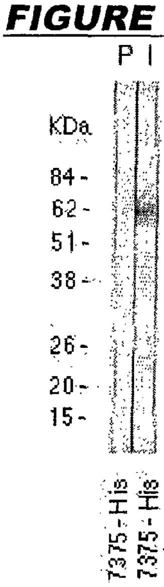


## FIGURE 178

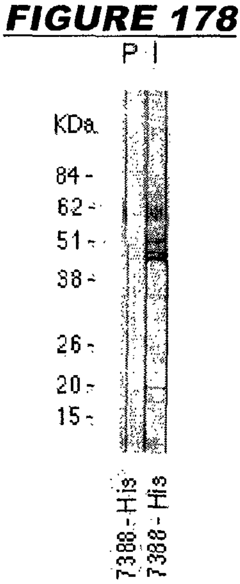

## FIGURE 179

FIG. 179A

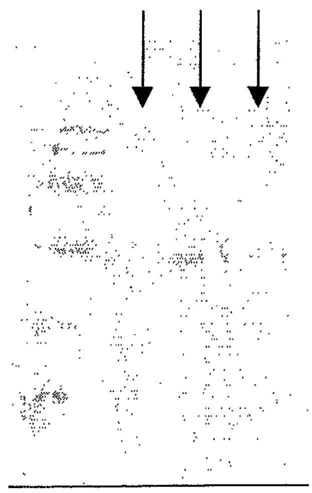

FIG. 179B

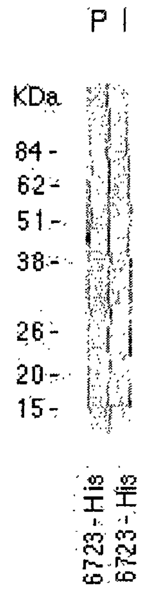

# FIGURE 180

FIG. 180A

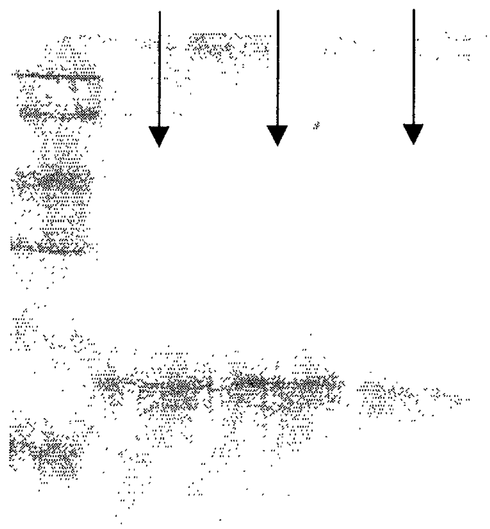

FIG. 180B

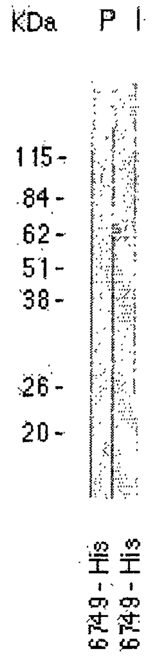

FIGURE 181

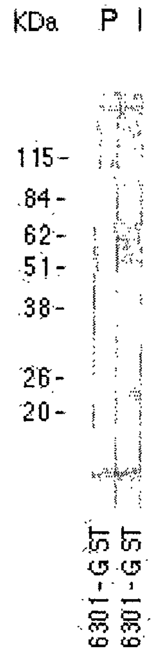


FIGURE 182

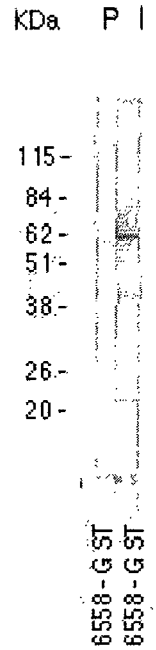


FIGURE 183

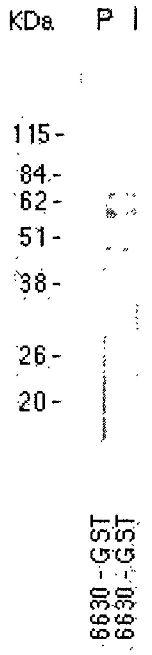


FIGURE 184

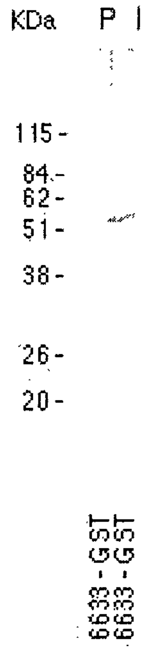

**FIGURE 185**

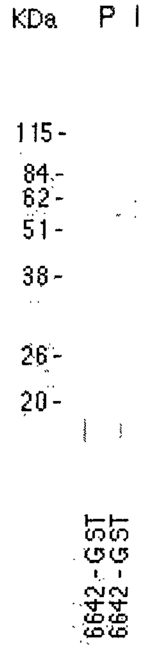

## FIGURE 186

FIG. 186A

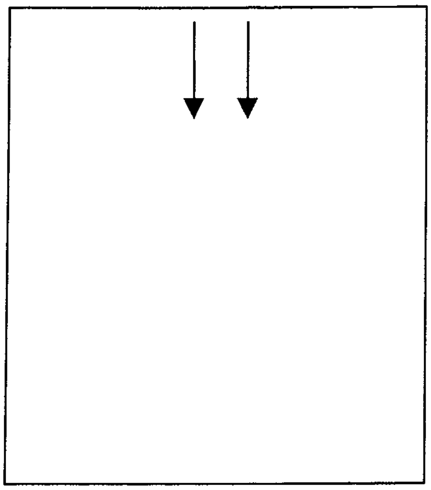

FIG. 186B

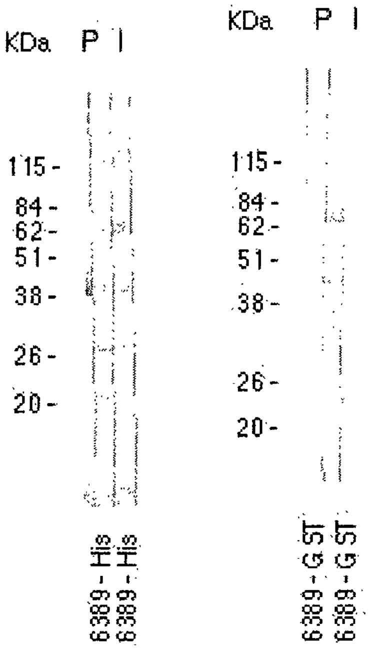

## FIGURE 187

FIG. 187A

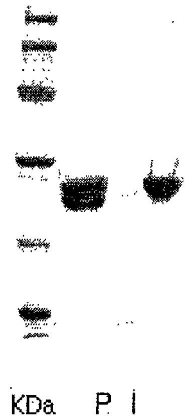


FIG. 187B

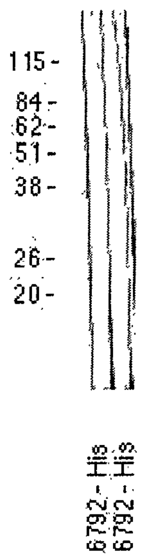

# FIGURE 188

FIG. 188A

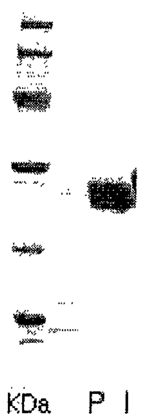


FIG. 188B

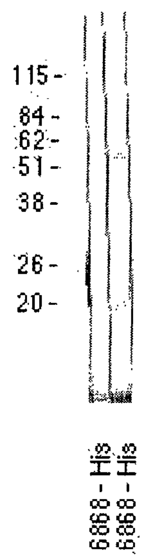

## FIGURE 189

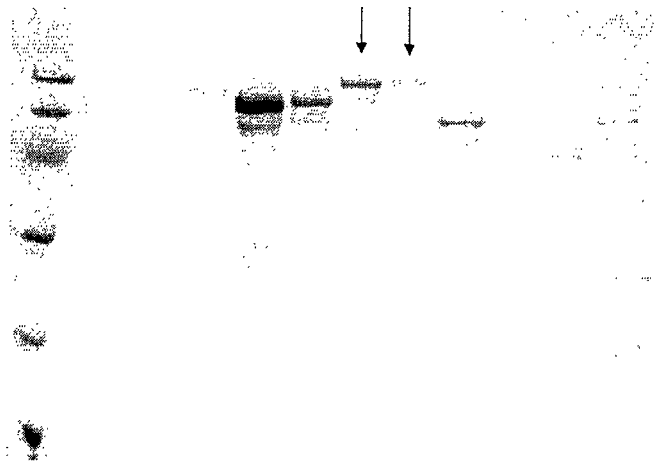

**FIG. 189A**

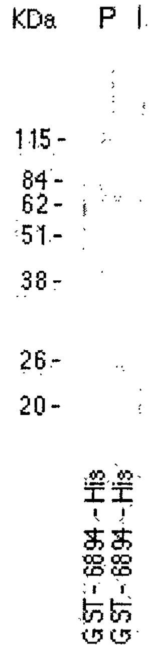


**FIG. 189B**

## *FIGURE 190*

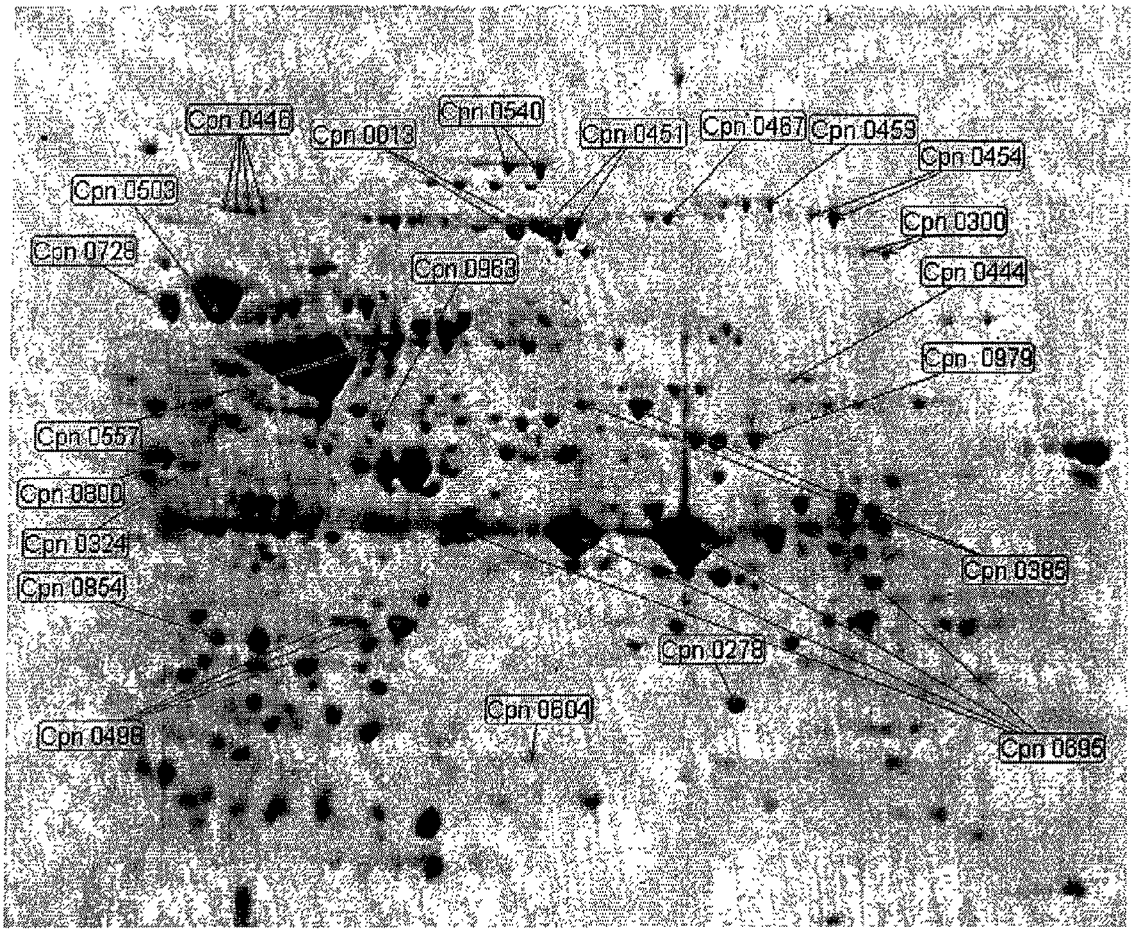


## *FIGURE 191*

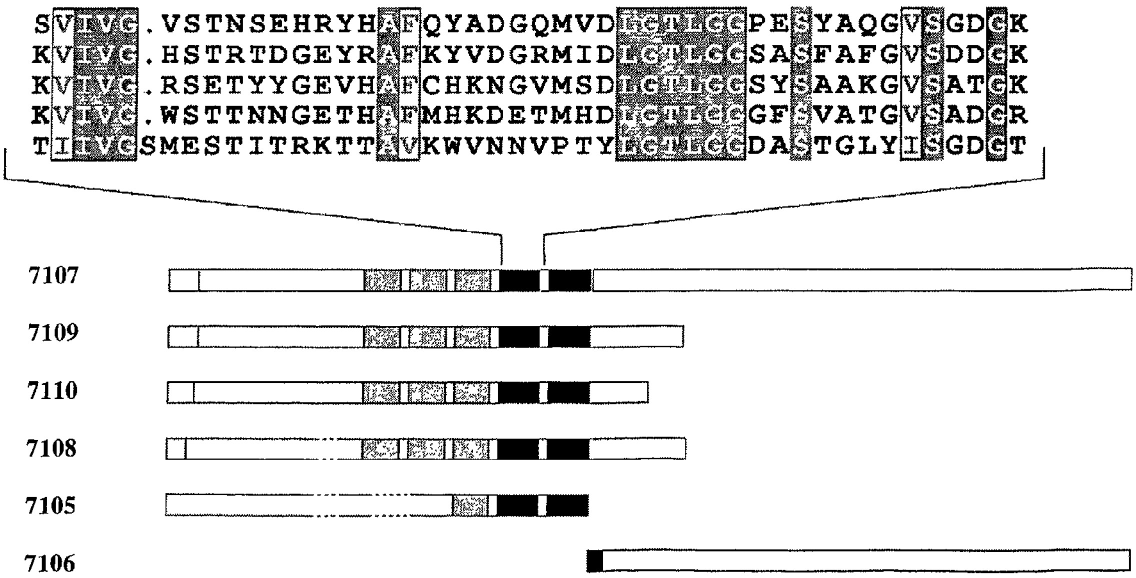

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 9927105 A
- WO 0027994 A
- US 5753235 A
- WO 89046699 A
- US 5693506 A
- US 5659122 A
- US 5608143 A
- US 4738921 A
- EP 0036776 A
- EP 0121775 A
- US 4689406 A
- US 4551433 A
- EP 0267851 A
- EP 0219237 A
- EP 0324647 A, Chey
- US 4336336 A
- EP 0244042 A
- EP 0127328 A
- EP 0036259 A
- EP 0063953 A
- WO 8404541 A
- EP 0136829 A
- EP 0136907 A
- US 4745056 A
- WO 84104541 A
- EP 0284044 A
- EP 0329203 A
- US 4876197 A
- US 4880734 A
- EP 0164556 A
- EP 0196056 A
- WO 88024066 A
- EP 0012873 A
- JP O62096086 B
- US 4588684 A
- EP 0060057 A
- US 4546083 A
- US 4870008 A
- EP 0324274 A
- WO 8902463 A
- US 4837148 A
- US 4929555 A
- WO 9820734 A
- WO 90114837 A
- WO 98120734 A
- US 5591624 A
- WO 9637626 A
- WO 9530763 A
- WO 92105266 A
- GB 2200651 A
- EP 0415731 A
- EP 0345242 A
- EP 0334301 A
- WO 8902468 A
- WO 8905349 A
- WO 8909271 A
- WO 9002806 A
- WO 9007936 A
- WO 9403622 A
- WO 9325698 A
- WO 9325234 A
- WO 9311230 A
- WO 9310218 A
- WO 9102805 A
- WO 9102825 A
- WO 9507994 A
- US 5219740 A
- US 4405712 A
- US 4861719 A
- US 4980289 A
- US 4777127 A
- WO 9307283 A
- WO 9306223 A
- WO 9307282 A
- WO 9412649 A
- WO 9303769 A
- WO 9319191 A
- WO 9428938 A
- WO 9511984 A
- WO 9500655 A
- WO 9527071 A
- WO 9529993 A
- WO 9534671 A
- WO 9605320 A
- WO 9408026 A
- WO 9411506 A
- WO 9424299 A
- WO 9514102 A
- WO 9524297 A
- WO 9502697 A
- WO 9428152 A
- WO 9509241 A
- WO 9525807 A
- WO 9505835 A
- WO 9418922 A
- WO 9509654 A
- WO 9309239 A
- US 5478745 A

- US 4797368 A, Carter
- US 5139941 A, Muzyczka
- US 5474935 A, Chartejee
- WO 94288157 A, Kotin
- US 5354678 A
- US 5173414 A
- US 5252479 A
- US 5288641 A
- EP 0176170 A, Roizman
- WO 9504139 A, Wistar
- WO 9009441 A
- WO 9207945 A
- EP 0453242 A
- US 5091309 A
- US 5217879 A
- WO 9210578 A
- US 08405627 B
- WO 9421792 A
- US SN08679640 A
- US 4603112 A
- US 4769330 A
- WO 8901973 A
- US 5166057 A
- EP 0386882 A
- EP 0440219 A
- US 08366787 B
- US 08240030 B
- US 08404796 B
- US 5149655 A
- US 5206152 A
- WO 9211033 A
- US 60023867 B
- WO 9011092 A
- US 5580859 A
- US 5422120 A
- WO 9513796 A
- WO 9423697 A
- WO 9114445 A
- EP 524968 A
- US SN60023867 A
- US 4762915 A
- EP 0524968 A
- WO 9314778 A
- US 9714465 W, Zuckermann
- US 4683195 A
- US 4683202 A


**Non-patent literature cited in the description**


- **Raulston.** *Mol Microbiol,* 1995, vol. 15, 607-616
- **Everett.** *Vet Microbiol,* 2000, vol. 75, 109-126
- **Kalman et al.** *Nature Genetics,* 1999, vol. 21, 385-389
- **Read et al.** *Nucleic Acids Res,* 2000, vol. 28, 1397-406
- **Stephens et al.** *Science,* 1998, vol. 282, 754-759
- **Kalayoglu et al.** *J. Infect. Dis.,* 1999, vol. 180, 780-90
- **Kalayoglu et al.** *Am. Heart J.,* 1999, vol. 138, 488-490
- **Shirai et al.** *J. Infect. Dis.,* 2000, vol. 181 (3), S524-S527
- **Sambrook.** Molecular Cloning; A Laboratory Manual. 1989
- MOLECULAR CLONING:A LABORATORY MANUAL. 2001
- DNA Cloning. 1985, vol. I, ii
- Oligonucleotide Synthesis. 1984
- Nucleic Acid Hybridization. 1984
- Transcription and Translation. 1984
- Animal Cell Culture. 1986
- Immobilized Cells and Enzymes. IRL Press, 1986
- **B. Perbal.** A Practical Guide to Molecular Cloning. 1984
- Methods in Enzymology series. Academic Press, Inc, vol. 154, 155
- Gene Transfer Vectors for Mammalian Cells. Cold Spring Harbor Laboratory, 1987
- Immunochemical Methods in Cell and Molecular Biology. Academic Press, 1987
- **Scopes.** Protein Purification: Principles and Practice. Springer-Verlag, 1987
- Handbook of Experimental Immunology. 1986, vol. I-IV
- Expression of Cloned Genes in Mammalian Cells. **Sambrook et al.** Molecular Cloning: A Laboratory Manual. 1989
- **Maniatis et al.** *Science,* 1987, vol. 236, 1237
- **Alberts et al.** Molecular Biology of the Cell. 1989
- **Dijkema et al.** *EMBO J.,* 1985, vol. 4, 761
- **Gorman et al.** *PNAS USA,* 1982, vol. 79, 6777
- **Boshart et al.** *Cell,* 1985, vol. 41, 521
- **Sassone-Corsi ; Borelli.** *Trends Genet.,* 1986, vol. 2, 215
- **Birnstiel et al.** *Cell,* 1985, vol. 41, 349
- Termination and 3' end processing of eukaryotic RNA. **Proudfoot ; Whitelaw.** Transcription and splicing. 1988
- **Proudfoot.** *Trends Biochem. Sci.,* 1989, vol. 14, 105
- Expression of cloned genes in cultured mammalian cells. **Sambrook et al.** Molecular Cloning: A Laboratory Manual. 1989
- **Gluzman.** *Cell,* 1981, vol. 23, 175
- **Kaufman et al.** *Mol. Cell. Biol.,* 1989, vol. 9, 946

- **Shimizu et al.** *Mol. Cell. Biol.,* 1986, vol. 6, 1074
- **Summers ; Smith.** *Texas Agricultural Experiment Station Bulletin,* 1987
- **Luckow ; Summers.** *Virology,* 1989, vol. 17, 31
- **Miller et al.** *Ann. Rev. Microbiol.,* 1988, vol. 42, 177
- The Regulation of Baculovirus Gene Expression. **Friesen et al.** The Molecular Biology of Baculoviruses
- **Vlak et al.** *J. Gen. Virol.,* 1988, vol. 69, 765
- **Carbonell et al.** *Gene,* 1988, vol. 73, 409
- **Maeda et al.** *Nature,* 1985, vol. 315, 592
- **Lebacq et al.** *Molec. Cell. Biol.,* 1988, vol. 8, 3129
- **Smith et al.** *Proc. Nat'l Acad. Sci. USA,* 1985, vol. 82, 8404
- **Miyajima et al.** *Gene,* 1987, vol. 58, 273
- **Martin et al.** *DNA,* 1988, vol. 7, 99
- **Smith et al.** *Mol. Cell. Biol.,* 1983, vol. 3, 2156
- **Miller et al.** *Bioessays,* 1989, vol. 4, 91
- Current Protocols in Microbiology. 1990, vol. 2
- **Carbonell et al.** *J. Virol.,* 1985, vol. 56, 153
- **Wright.** *Nature,* 1986, vol. 321, 718
- **Fraser et al.** *In Vitro Cell. Dev. Biol.,* 1989, vol. 25, 225
- **Zenk.** *Phytochemistry,* 1991, vol. 30, 3861-3863
- **Vaulcombe et al.** *Mol. Gen. Genet.,* 1987, vol. 209, 33-40
- **Chandler et al.** *Plant Molecular Biology,* 1984, vol. 3, 407-418
- **Rogers.** *J. Biol. Chem.,* 1985, vol. 260, 3731-3738
- **Rothstein et al.** *Gene,* 1987, vol. 55, 353-356
- **Whittier et al.** *Nucleic Acids Research,* 1987, vol. 15, 2515-2535
- **Wirsel et al.** *Molecular Microbiology,* 1989, vol. 3, 3-14
- **Yu et al.** *Gene,* 1992, vol. 122, 247-253
- **R.L. Jones ; J. MacMillin, Gibberellins.** Advanced Plant Physiology. Pitman Publishing Limited, 1984, 21-52
- **Sheen.** *Plant Cell,* 1990, vol. 2, 1027-1038
- **Maas et al.** *EMBO J.,* 1990, vol. 9, 3447-3452
- **Benkel ; Hickey.** *Proc. Natl. Acad. Sci.,* 1987, vol. 84, 1337-1339
- **Wilmink ; Dons.** *Plant Mol. Biol. Reptr,* 1993, vol. 11 (2), 165-185
- **Reed ; Maniatis.** *Cell,* 1985, vol. 41, 95-105
- **Crossway.** *Mol. Gen. Genet,* 1985, vol. 202, 179-185
- **Krens et al.** *Nature,* 1982, vol. 296, 72-74
- **Klein et al.** *Nature,* 1987, vol. 327, 70-73
- **Knudsen ; Muller.** *Planta,* 1991, vol. 185, 330-336
- **Fraley et al.** *Proc. Natl. Acad. Sci. USA,* 1982, vol. 79, 1859-1863
- **Fromm et al.** *Proc. Natl Acad. Sci. USA,* 1985, vol. 82, 5824
- **Raibaud et al.** *Annu. Rev. Genet.,* 1984, vol. 18, 173
- **Chang et al.** *Nature,* 1977, vol. 198, 1056
- **Goeddel et al.** *Nuc. Acids Res.,* 1980, vol. 8, 4057
- **Yelverton et al.** *Nucl. Acids Res.,* 1981, vol. 9, 731
- The cloning of interferon and other mistakes. **Weissmann.** Interferon 3. 1981
- **Shimatake et al.** *Nature,* 1981, vol. 292, 128
- **Amann et al.** *Gene,* 1983, vol. 25, 167
- **Boer et al.** *Proc. Natl. Acad. Sci.,* 1983, vol. 80, 21
- **Studier et al.** *J. Mol. Biol.,* 1986, vol. 189, 113
- **Tabor et al.** *Proc Natl. Acad. Sci.,* 1985, vol. 82, 1074
- **Shine et al.** *Nature,* 1975, vol. 254, 34
- Genetic signals and nucleotide sequences in messenger RNA. **Steitz et al.** Biological Regulation and Development: Gene Expression. 1979
- Expression of cloned genes in Escherichia coli. **Sambrook et al.** Molecular Cloning: A Laboratory Manual. 1989
- **Nagai et al.** *Nature,* 1984, vol. 309, 810
- **Jia et al.** *Gene,* 1987, vol. 60, 197
- **Allen et al.** *J. Biotechnol.,* 1987, vol. 5, 93
- **Makoff et al.** *J. Gen. Microbiol.,* 1989, vol. 135, 11
- **Miller et al.** *Bio/Technology,* 1989, vol. 7, 698
- **Masui et al.** *Experimental Manipulation of Gene Expression,* 1983
- **Ghrayeb et al.** *EMBO J.,* 1984, vol. 3, 2437
- **Oka et al.** *Proc. Natl. Acad. Sci.,* 1985, vol. 82, 7212
- **Palva et al.** *Proc. Natl. Acad. Sci. USA,* 1982, vol. 79, 5582
- **Davies et al.** *Annu. Rev. Microbiol.,* 1978, vol. 32, 469
- **Amann et al.** *Gene,* 1985, vol. 40, 183
- **Powell et al.** *Appl. Environ. Microbiol.,* 1988, vol. 54, 655
- **Masson et al.** *FEMS Microbiol. Lett.,* 1989, vol. 60, 273
- **Miller et al.** *Proc. Natl. Acad. Sci.,* 1988, vol. 85, 856
- **Wang et al.** *J. Bacteriol.,* 1990, vol. 172, 949
- **Cohen et al.** *Proc. Natl. Acad. Sci.,* 1973, vol. 69, 2110
- **Dower et al.** *Nucleic Acids Res.,* 1988, vol. 16, 6127

- An improved method for transformation of Escherichia coli with ColE1-derived plasmids. **Kushner.** Genetic Engineering: Proceedings of the International Symposium on Genetic Engineering. 1978
- **Mandel et al.** *J. Mol. Biol.,* 1970, vol. 53, 159
- **Taketo.** *Biochim. Biophys. Acta,* 1988, vol. 949, 318
- **Chassy et al.** *FEMS Microbiol. Lett.,* 1987, vol. 44, 173
- **Fiedler et al.** *Anal. Biochem,* 1988, vol. 170, 38
- **Augustin et al.** *FEMS Microbiol. Lett.,* 1990, vol. 66, 203
- **Barany et al.** *J. Bacteriol.,* 1980, vol. 144, 698
- Transformation of Streptococcus lactis by electroporation. **Harlander.** Streptococcal Genetics. 1987
- **Perry et al.** *Infect. Immun.,* 1981, vol. 32, 1295
- **Somkuti et al.** *Proc. 4th Evr. Cong. Biotechnology,* 1987, vol. 1, 412
- **Myanohara et al.** *Proc. Natl. Acad. Sci. USA,* 1983, vol. 80, 1
- **Cohen et al.** *Proc. Natl. Acad. Sci. USA,* 1980, vol. 77, 1078
- **Henikoff et al.** *Nature,* 1981, vol. 283, 835
- **Hollenberg et al.** *Curr. Topics Microbiol. Immunol.,* 1981, vol. 96, 119
- The Expression of Bacterial Antibiotic Resistance Genes in the Yeast Saccharomyces cerevisiae. **Hollenberg et al.** Plasmids of Medical, Environmental and Commercial Importance. 1979
- **Mercerau-Puigalon et al.** *Gene,* 1980, vol. 11, 163
- **Panthier et al.** *Curr. Genet,* 1980, vol. 2, 109
- **Botstein et al.** *Gene,* 1979, vol. 8, 17-24
- **Brake et al.** *Proc, Natl. Acad. Sci USA,* 1984, vol. 81, 4642-4646
- **Stinchcomb et al.** *J. Mol. Biol.,* 1982, vol. 158, 157
- **Orr-Weaver et al.** *Methods in Enzymol.,* 1983, vol. 101, 228-245
- **Rine et al.** *Proc. Natl. Acad. Sci. USA,* 1983, vol. 80, 6750
- **Butt et al.** *Microbiol, Rev.,* 1987, vol. 51, 351
- **Kurtz et al.** *Mol. Cell. Biol.,* 1986, vol. 6, 142
- **Kunze et al.** *J. Basic Microbiol.,* 1985, vol. 25, 141
- **Gleeson et al.** *J. Gen. Microbiol.,* 1986, vol. 132, 3459
- **Roggenkamp et al.** *Mol. Gen. Genet.,* 1986, vol. 202, 302
- **Das et al.** *J. Bacteriol.,* 1984, vol. 158, 1165
- **De Louvencourt et al.** *J. Bacteriol.,* 1983, vol. 154, 737
- **Van den Berg et al.** *Bio/Technology,* 1990, vol. 8, 135
- **Cregg et al.** *Mol. Cell. Biol.,* 1985, vol. 5, 3376
- **Hinnen et al.** *Proc. Natl. Acad. Sci. USA,* 1978, vol. 75, 1929
- **Ito et al.** *J. Bacteriol.,* 1983, vol. 153, 163
- **Beach ; Nurse.** *Nature,* 1981, vol. 300, 706
- **Davidow et al.** *Curr. Genet,* 1985, vol. 10, 380471
- **Gaillardin et al.** *Curr. Genet,* 1985, vol. 10, 49
- **De Louvencourt et al.** *J. Bacteriol.,* 1983, vol. 154, 1165
- **Van den et al.** *Bio/Technology,* 1990, vol. 8, 135
- **Davidow et al.** *Curr. Genet,* 1985, vol. 10, 39
- Remington's Pharmaceutical Sciences. Mack Pub. Co, 1991
- Vaccine design: the subunit and adjuvant approach. Plenum Press, 1995
- **Robinson ; Torres.** *Seminars in Immunology,* 1997, vol. 9, 271-283
- **Donnelly et al.** *Annu Rev Immunol,* 1997, vol. 15, 617-648
- **Jolly.** *Cancer Gene Therapy,* 1994, vol. 1, 51-64
- **Kimura.** *Human Gene Therapy,* 1994, vol. 5, 845-852
- **Connelly.** *Human Gene Therapy,* 1995, vol. 6, 185-193
- **Kaplitt.** *Nature Genetics,* 1994, vol. 6, 148-153
- **O'Neill.** *J. Virol.,* 1985, vol. 53, 160
- **Kelly.** *J. Virol.,* 1983, vol. 45, 291
- RNA Tumor Viruses. Cold Spring Harbor Laboratory, 1985
- **Hartley ; Rowe.** *J Virol,* 1976, vol. 19, 19-25
- **Vile.** *Cancer Res,* 1993, vol. 53, 3860-3864
- **Vile.** *Cancer Res,* 1993, vol. 53, 962-967
- **Ram.** *Cancer Res,* 1993, vol. 53, 83-88
- **Takamiya.** *J Neurosci Res,* 1992, vol. 33, 493-503
- **Baba.** *J Neurosurg,* 1993, vol. 79, 729-735
- **Mann.** *Cell,* 1983, vol. 33, 153
- **Cane.** *Proc Natl Acad Sci,* 1984, vol. 81, 6349
- **Miller.** *Human Gene Therapy,* 1990, 1
- **Berkner.** *Biotechniques,* 1988, vol. 6, 616
- **Rosenfeld.** *Science,* 1991, vol. 252, 431
- **Curiel.** *Hum. Gene Ther.,* 1992, vol. 3, 147-154
- **Nahreini.** *Gene,* 1993, vol. 124, 257-262
- **Samulski.** *J. Virol.,* 1987, vol. 61, 3096
- **Su.** *Human Gene Therapy,* 1996, vol. 7, 463-470
- **Geller.** *Science,* 1988, vol. 241, 1667-1669

- **Fink.** *Human Gene Therapy,* 1992, vol. 3, 11-19
- **Evans.** *Nature,* 1989, vol. 339, 385
- **Sabin.** *J. Biol. Standardization,* 1973, vol. 1, 115
- **Arnold.** *J Cell Biochem,* 1990, L401
- **Fisher-Hoch.** *Proc Natl Acad Sci,* 1989, vol. 86, 317
- **Flexner.** *Ann NY Acad Sci,* 1989, vol. 569, 86
- **Flexner.** *Vaccine,* 1990, vol. 8, 17
- **Mulligan.** *Nature,* 1979, vol. 277, 108
- **Madzak.** *J Gen Virol,* 1992, vol. 73, 1533
- **Enami.** *Proc Natl Acad Sci,* 1990, vol. 87, 3802-3805
- **Enami ; Palese.** *J Virol,* 1991, vol. 65, 2711-2713
- **Luytjes.** *Cell,* 1989, vol. 59, 110
- **McMichael.** *NEJ Med,* 1983, vol. 309, 13
- **Yap.** *Nature,* 1978, vol. 273, 238
- *Nature,* 1979, vol. 277, 108
- **Buchschacher.** *J. Virol,* 1992, vol. 66, 2731
- **Hamre.** *Proc Soc Exp Biol Med,* 1966, vol. 121, 190
- **Curiel.** *Hum Gene Ther,* 1992, vol. 3, 147-154
- **Wu.** *J Biol Chem,* 1989, vol. 264, 16985-16987
- **Philip.** *Mol Cell Biol,* 1994, vol. 14, 2411-2418
- **W offendin.** *Proc Natl Acad Sci,* 1994, vol. 91, 1581-1585
- **Wu ; Wu.** *J. Biol. Chem.,* 1987, vol. 262, 4429-4432
- **Hucked.** *Biochem Pharmacol,* 1990, vol. 40, 253-263
- **Plank.** *Bioconjugate Chem,* 1992, vol. 3, 533-539
- **Woffendin et al.** *Proc, Natl. Acad. Sci. USA,* 1994, vol. 91 (24), 11581-11585
- **Stryer.** Biochemistry. W.H. Freeman, 1975, 236-240
- **Szoka.** *Biochem Biophys Acta,* 1980, vol. 600, 1
- **Bayer.** *Biochem Biophys Acta,* 1979, vol. 550, 464
- **Rivnay.** *Meth Enzymol,* 1987, vol. 149, 119
- **Wang.** *Proc Natl Acad Sci,* 1987, vol. 84, 7851
- *Anal Biochem,* 1989, vol. 176, 420
- **Hug ; Sleight.** *Biochim. Biophys. Acta.,* 1991, vol. 1097, 1-17
- **Straubinger.** *Meth. Enzymol.,* 1983, vol. 101, 512-527
- **Felgner.** *Proc. Natl. Acad. Sci. USA,* 1987, vol. 84, 7413-7416
- **Malone.** *Proc. Natl. Acad. Sci. USA,* 1989, vol. 86, 6077-6081
- **Debs.** *J. Biol. Chem.,* 1990, vol. 265, 10189-10192
- **Szoka.** *Proc. Natl. Acad. Sci. USA,* 1978, vol. 75, 4194-4198
- **Straubinger.** *Meth. Immunol.,* 1983, vol. 101, 512-527
- **Papahadjopoulos.** *Biochim. Biophys. Acta,* 1975, vol. 394, 483
- **Wilson.** *Cell,* 1979, vol. 17, 77
- **Deamer ; Bangham.** *Biochim. Biophys. Acta,* 1976, vol. 443, 629
- **Ostro.** *Biochem. Biophys. Res. Commun.,* 1977, vol. 76, 836
- **Fraley.** *Proc. Natl. Acad. Sci. USA,* 1979, vol. 76, 3348
- **Enoch ; Strittmatter.** *Proc. Natl. Acad. Sci. USA,* 1979, vol. 76, 145
- **Fraley.** *J. Biol. Chem.,* 1980, vol. 255, 10431
- **Szoka ; Papahadjopoulos.** *Proc. Natl. Acad. Sci. USA,* 1978, vol. 75, 145
- **Schaefer-Ridder.** *Science,* 1982, vol. 215, 166
- **Breslow.** *Annu Rev. Biochem,* 1985, vol. 54, 699
- **Law.** *Adv. Exp Med. Biol.,* 1986, vol. 151, 162
- **Chen.** *J Biol Chem,* 1986, vol. 261, 12918
- **Kane.** *Proc Natl Acad Sci USA,* 1980, vol. 77, 2465
- **Utermann.** *Hum Genet,* 1984, vol. 65, 232
- *Meth. Enzymol.,* 1986, 128
- **Pitas.** *J. Biochem.,* 1980, vol. 255, 5454-5460
- **Mahey.** *J Clin. Invest,* 1979, vol. 64, 743-750
- **Atkinson.** *Annu Rev Biophys Chem,* 1986, vol. 15, 403
- **Radding.** *Biochim Biophys Acta,* 1958, vol. 30, 443
- **Meinkoth ; Wahl.** *Anal. Biochem.,* 1984, vol. 138, 267-284
- **Matteucci et al.** *J. Am. Chem. Soc.,* 1981, vol. 103, 3185
- **Urdea et al.** *Proc. Natl. Acad. Sci. USA,* 1983, vol. 80, 7461
- **Agrawal ; Iyer.** *Curr Opin Biotechnol,* 1995, vol. 6, 12-19
- **Agrawal.** *TIBTECH,* 1996, vol. 14, 376-387
- **Corey.** *TIBTECH,* 1997, vol. 15, 224-229
- **Buchardt et al.** *TIBTECH,* 1993, vol. 11, 384-386
- **Mullis et al.** *Meth. Enzymol.,* 1987, vol. 155, 335-350
- **Guan ; Dixon.** *Anal. Biochem.,* 1991, vol. 192, 262
- **Breslauer et al.** *PNAS USA,* 1986, vol. 83, 3746-50
- **Kubo ; Stephens.** *Mol. Microbiol.,* 2000, vol. 38, 772-780
- **Chevallet et al.** *Electrophor.,* 1998, vol. 19, 1901-9
- **Herbert et al.** *Electrophor.,* 1998, vol. 19, 845-51
- **Doherty et al.** *Electrophor.,* 1998, vol. 19, 355-63

- **Wilm et al.** *Nature,* 1996, vol. 379, 466-9

SEQUENCE LISTING

<110> NOVARTIS VACCINES AND DIAGNOSTICS S.R.L

<120> IMMUNISATION AGAINST CHLAMYDIA PNEUMONIAE

<130> P051927EP

<150> EP-01954278.6
<151> 2001-07-03

<150> PCT/IB01/01445
<151> 2001-07-03

<150> GB-0016363.4
<151> 2000-07-03

<150> GB-0017047.2
<151> 2000-07-11

<150> GB-0017983.8
<151> 2000-07-21

<150> GB-0019368.0
<151> 2000-08-07

<150> GB-0020440.4
<151> 2000-08-18

<150> GB-0022583.9
<151> 2000-09-14

<150> GB-0027549.5
<151> 2000-11-10

<150> GB-0031706.5
<151> 2000-12-22

<160> 664

<170> SeqWin99, version 1.02

<210> 1
<211> 272
<212> PRT
<213> Chlamydia pneumoniae

<400> 1
```
Met Lys Lys Lys Leu Ser Leu Leu Val Gly Leu Ile Phe Val Leu Ser
1               5                   10                  15

Ser Cys His Lys Glu Asp Ala Gln Asn Lys Ile Arg Ile Val Ala Ser
            20                  25                  30

Pro Thr Pro His Ala Glu Leu Leu Glu Ser Leu Gln Glu Glu Ala Lys
        35                  40                  45

Asp Leu Gly Ile Lys Leu Lys Ile Leu Pro Val Asp Asp Tyr Arg Ile
    50                  55                  60

Pro Asn Arg Leu Leu Leu Asp Lys Gln Val Asp Ala Asn Tyr Phe Gln
```

```
65                  70                  75                  80

His Gln Ala Phe Leu Asp Asp Glu Cys Glu Arg Tyr Asp Cys Lys Gly
                85                  90                  95

Glu Leu Val Val Ile Ala Lys Val His Leu Glu Pro Gln Ala Ile Tyr
            100                 105                 110

Ser Lys Lys His Ser Ser Leu Glu Arg Leu Lys Ser Gln Lys Lys Leu
        115                 120                 125

Thr Ile Ala Ile Pro Val Asp Arg Thr Asn Ala Gln Arg Ala Leu His
    130                 135                 140

Leu Leu Glu Glu Cys Gly Leu Ile Val Cys Lys Gly Pro Ala Asn Leu
145                 150                 155                 160

Asn Met Thr Ala Lys Asp Val Cys Gly Lys Glu Asn Arg Ser Ile Asn
                165                 170                 175

Ile Leu Glu Val Ser Ala Pro Leu Leu Val Gly Ser Leu Pro Asp Val
            180                 185                 190

Asp Ala Ala Val Ile Pro Gly Asn Phe Ala Ile Ala Ala Asn Leu Ser
        195                 200                 205

Pro Lys Lys Asp Ser Leu Cys Leu Glu Asp Leu Ser Val Ser Lys Tyr
    210                 215                 220

Thr Asn Leu Val Val Ile Arg Ser Glu Asp Val Gly Ser Pro Lys Met
225                 230                 235                 240

Ile Lys Leu Gln Lys Leu Phe Gln Ser Pro Ser Val Gln His Phe Phe
                245                 250                 255

Asp Thr Lys Tyr His Gly Asn Ile Leu Thr Met Thr Gln Asp Asn Gly
            260                 265                 270


<210>   2
<211>   819
<212>   DNA
<213>   Chlamydia pneumoniae

<400>   2
atgaaaaaaa aattatcatt acttgtaggt ttaattttg ttttgagttc ttgccataag   60
gaagatgctc agaataaaat acgtattgta gccagtccga cacctcatgc ggaattattg  120
gagagtttac aggaagaggc taaagatctt ggaatcaagc tgaaaatact tccagtagat  180
gattatcgta ttcctaatcg tttgcttttg gataaacaag tagatgcaaa ttactttcaa  240
catcaagctt ttcttgatga cgaatgcgag cgttatgatt gtaagggtga attagttgtt  300
atcgctaaag ttcatttgga acctcaagca atttattcta agaaacattc ttctttagag  360
cgcttaaaaa gccagaagaa actgactata gcgattcctg tggatcgtac gaatgctcag  420
cgtgctctac acttgttaga agagtgcgga ctcattgttt gcaaagggcc tgctaattta  480
aatatgacag ctaaagatgt ctgtgggaaa gaaaatagaa gtatcaacat attagaggtg  540
tcagctcctc ttcttgtcgg atctcttcct gacgttgatg ctgctgtcat tcctggaaat  600
tttgctatag cagcaaacct ttctccaaag aaagatagtc tttgtttaga ggatctttcg  660
gtatctaagt atacaaacct tgttgtcatt cgttctgaag acgtaggttc tcctaaaatg  720
ataaaattac agaagctgtt tcaatctcct tctgtacaac attttttga tacaaaatat  780
catgggaata ttttgacaat gactcaagac aatggttag                         819

<210>   3
```

<211> 973
<212> PRT
<213> Chlamydia pneumoniae

<400> 3

```
Met Lys Thr Ser Ile Arg Lys Phe Leu Ile Ser Thr Thr Leu Ala Pro
1               5                   10                  15

Cys Phe Ala Ser Thr Ala Phe Thr Val Glu Val Ile Met Pro Ser Glu
            20                  25                  30

Asn Phe Asp Gly Ser Ser Gly Lys Ile Phe Pro Tyr Thr Thr Leu Ser
        35                  40                  45

Asp Pro Arg Gly Thr Leu Cys Ile Phe Ser Gly Asp Leu Tyr Ile Ala
    50                  55                  60

Asn Leu Asp Asn Ala Ile Ser Arg Thr Ser Ser Ser Cys Phe Ser Asn
65                  70                  75                  80

Arg Ala Gly Ala Leu Gln Ile Leu Gly Lys Gly Gly Val Phe Ser Phe
                85                  90                  95

Leu Asn Ile Arg Ser Ser Ala Asp Gly Ala Ala Ile Ser Ser Val Ile
            100                 105                 110

Thr Gln Asn Pro Glu Leu Cys Pro Leu Ser Phe Ser Gly Phe Ser Gln
        115                 120                 125

Met Ile Phe Asp Asn Cys Glu Ser Leu Thr Ser Asp Thr Ser Ala Ser
    130                 135                 140

Asn Val Ile Pro His Ala Ser Ala Ile Tyr Ala Thr Thr Pro Met Leu
145                 150                 155                 160

Phe Thr Asn Asn Asp Ser Ile Leu Phe Gln Tyr Asn Arg Ser Ala Gly
                165                 170                 175

Phe Gly Ala Ala Ile Arg Gly Thr Ser Ile Thr Ile Glu Asn Thr Lys
            180                 185                 190

Lys Ser Leu Leu Phe Asn Gly Asn Gly Ser Ile Ser Asn Gly Gly Ala
        195                 200                 205

Leu Thr Gly Ser Ala Ala Ile Asn Leu Ile Asn Asn Ser Ala Pro Val
    210                 215                 220

Ile Phe Ser Thr Asn Ala Thr Gly Ile Tyr Gly Gly Ala Ile Tyr Leu
225                 230                 235                 240

Thr Gly Gly Ser Met Leu Thr Ser Gly Asn Leu Ser Gly Val Leu Phe
                245                 250                 255

Val Asn Asn Ser Ser Arg Ser Gly Gly Ala Ile Tyr Ala Asn Gly Asn
            260                 265                 270

Val Thr Phe Ser Asn Asn Ser Asp Leu Thr Phe Gln Asn Asn Thr Ala
        275                 280                 285

Ser Pro Gln Asn Ser Leu Pro Ala Pro Thr Pro Pro Pro Thr Pro Pro
    290                 295                 300
```

```
Ala Val Thr Pro Leu Leu Gly Tyr Gly Gly Ala Ile Phe Cys Thr Pro
305                 310                 315                 320

Pro Ala Thr Pro Pro Pro Thr Gly Val Ser Leu Thr Ile Ser Gly Glu
                325                 330                 335

Asn Ser Val Thr Phe Leu Glu Asn Ile Ala Ser Glu Gln Gly Gly Ala
            340                 345                 350

Leu Tyr Gly Lys Lys Ile Ser Ile Asp Ser Asn Lys Ser Thr Ile Phe
        355                 360                 365

Leu Gly Asn Thr Ala Gly Lys Gly Gly Ala Ile Ala Ile Pro Glu Ser
    370                 375                 380

Gly Glu Leu Ser Leu Ser Ala Asn Gln Gly Asp Ile Leu Phe Asn Lys
385                 390                 395                 400

Asn Leu Ser Ile Thr Ser Gly Thr Pro Thr Arg Asn Ser Ile His Phe
                405                 410                 415

Gly Lys Asp Ala Lys Phe Ala Thr Leu Gly Ala Thr Gln Gly Tyr Thr
            420                 425                 430

Leu Tyr Phe Tyr Asp Pro Ile Thr Ser Asp Asp Leu Ser Ala Ala Ser
        435                 440                 445

Ala Ala Ala Thr Val Val Val Asn Pro Lys Ala Ser Ala Asp Gly Ala
    450                 455                 460

Tyr Ser Gly Thr Ile Val Phe Ser Gly Glu Thr Leu Thr Ala Thr Glu
465                 470                 475                 480

Ala Ala Thr Pro Ala Asn Ala Thr Ser Thr Leu Asn Gln Lys Leu Glu
                485                 490                 495

Leu Glu Gly Gly Thr Leu Ala Leu Arg Asn Gly Ala Thr Leu Asn Val
            500                 505                 510

His Asn Phe Thr Gln Asp Glu Lys Ser Val Val Ile Met Asp Ala Gly
        515                 520                 525

Thr Thr Leu Ala Thr Thr Asn Gly Ala Asn Asn Thr Asp Gly Ala Ile
    530                 535                 540

Thr Leu Asn Lys Leu Val Ile Asn Leu Asp Ser Leu Asp Gly Thr Lys
545                 550                 555                 560

Ala Ala Val Val Asn Val Gln Ser Thr Asn Gly Ala Leu Thr Ile Ser
                565                 570                 575

Gly Thr Leu Gly Leu Val Lys Asn Ser Gln Asp Cys Cys Asp Asn His
            580                 585                 590

Gly Met Phe Asn Lys Asp Leu Gln Gln Val Pro Ile Leu Glu Leu Lys
        595                 600                 605

Ala Thr Ser Asn Thr Val Thr Thr Thr Asp Phe Ser Leu Gly Thr Asn
    610                 615                 620
```

```
Gly Tyr Gln Gln Ser Pro Tyr Gly Tyr Gln Gly Thr Trp Glu Phe Thr
625                 630                 635                 640

Ile Asp Thr Thr Thr His Thr Val Thr Gly Asn Trp Lys Lys Thr Gly
                645                 650                 655

Tyr Leu Pro His Pro Glu Arg Leu Ala Pro Leu Ile Pro Asn Ser Leu
            660                 665                 670

Trp Ala Asn Val Ile Asp Leu Arg Ala Val Ser Gln Ala Ser Ala Ala
        675                 680                 685

Asp Gly Glu Asp Val Pro Gly Lys Gln Leu Ser Ile Thr Gly Ile Thr
    690                 695                 700

Asn Phe Phe His Ala Asn His Thr Gly Asp Ala Arg Ser Tyr Arg His
705                 710                 715                 720

Met Gly Gly Gly Tyr Leu Ile Asn Thr Tyr Thr Arg Ile Thr Pro Asp
                725                 730                 735

Ala Ala Leu Ser Leu Gly Phe Gly Gln Leu Phe Thr Lys Ser Lys Asp
            740                 745                 750

Tyr Leu Val Gly His Gly His Ser Asn Val Tyr Phe Ala Thr Val Tyr
        755                 760                 765

Ser Asn Ile Thr Lys Ser Leu Phe Gly Ser Ser Arg Phe Phe Ser Gly
    770                 775                 780

Gly Thr Ser Arg Val Thr Tyr Ser Arg Ser Asn Glu Lys Val Lys Thr
785                 790                 795                 800

Ser Tyr Thr Lys Leu Pro Lys Gly Arg Cys Ser Trp Ser Asn Asn Cys
                805                 810                 815

Trp Leu Gly Glu Leu Glu Gly Asn Leu Pro Ile Thr Leu Ser Ser Arg
            820                 825                 830

Ile Leu Asn Leu Lys Gln Ile Ile Pro Phe Val Lys Ala Glu Val Ala
        835                 840                 845

Tyr Ala Thr His Gly Gly Ile Gln Glu Asn Thr Pro Glu Gly Arg Ile
    850                 855                 860

Phe Gly His Gly His Leu Leu Asn Val Ala Val Pro Val Gly Val Arg
865                 870                 875                 880

Phe Gly Lys Asn Ser His Asn Arg Pro Asp Phe Tyr Thr Ile Ile Val
                885                 890                 895

Ala Tyr Ala Pro Asp Val Tyr Arg His Asn Pro Asp Cys Asp Thr Thr
            900                 905                 910

Leu Pro Ile Asn Gly Ala Thr Trp Thr Ser Ile Gly Asn Asn Leu Thr
        915                 920                 925

Arg Ser Thr Leu Leu Val Gln Ala Ser Ser His Thr Ser Val Asn Asp
    930                 935                 940

Val Leu Glu Ile Phe Gly His Cys Gly Cys Asp Ile Arg Arg Thr Ser
```

945 950 955 960

Arg Gln Tyr Thr Leu Asp Ile Gly Ser Lys Leu Arg Phe
965 970

<210> 4
<211> 2922
<212> DNA
<213> Chlamydia pneumoniae

<400> 4
atgaaaacgt ctattcgtaa gttcttaatt tctaccacac tggcgccatg ttttgcttca 60
acagcgttta ctgtagaagt tatcatgcct tccgagaact ttgatggatc gagtgggaag 120
atttttcctt acacaacact ttctgatcct agagggacac tctgtatttt ttcaggggat 180
ctctacattg cgaatcttga taatgccata tccagaacct cttccagttg ctttagcaat 240
agggcgggag cactacaaat cttaggaaaa ggtggggttt tctccttctt aaatatccgt 300
tcttcagctg acggagccgc gattagtagt gtaatcaccc aaaatcctga actatgtccc 360
ttgagttttt caggatttag tcagatgatc ttcgataact gtgaatcttt gacttcagat 420
acctcagcga gtaatgtcat acctcacgca tcggcgattt acgctacaac gcccatgctc 480
tttacaaaca atgactccat actattccaa tacaaccgtt ctgcaggatt tggagctgcc 540
attcgaggca caagcatcac aatagaaaat acgaaaaaga gccttctctt taatggtaat 600
ggatccatct ctaatggagg ggccctcacg ggatctgcag cgatcaacct catcaacaat 660
agcgctcctg tgattttctc aacgaatgct acagggatct atggtggggc tatttacctt 720
accggaggat ctatgctcac ctctgggaac ctctcaggag tcttgttcgt taataatagc 780
tcgcgctcag gaggcgctat ctatgctaac ggaaatgtca cattttctaa taacagcgac 840
ctgactttcc aaaacaatac agcatctcca caaaactcct tacctgcacc tacacctcca 900
cctacaccac cagcagtcac tcctttgtta ggatatggag gcgccatctt ctgtactcct 960
ccagctaccc ccccaccaac aggtgttagc ctgactatat ctggagaaaa cagcgttaca 1020
ttcctagaaa acattgcctc cgaacaagga ggagccctct atggcaaaaa gatctctata 1080
gattctaata aatctacaat atttcttgga aatacagctg gaaaaggagg cgctattgct 1140
attcccgaat ctggggagct ctctctatcc gcaaatcaag gtgatatcct ctttaacaag 1200
aacctcagca tcactagtgg gacacctact cgcaatagta ttcacttcgg aaaagatgcc 1260
aagtttgcca ctctaggagc tacgcaaggc tataccctat acttctatga tccgattaca 1320
tctgatgatt tatctgctgc atccgcagcc gctactgtgg tcgtcaatcc caaagccagt 1380
gcagatggtg cgtattcagg gactattgtc ttttcaggag aaaccctcac tgctaccgaa 1440
gcagcaaccc ctgcaaatgc tacatctaca ttaaaccaaa agctagaact tgaaggcggt 1500
actctcgctt taagaaacgg tgctacctta aatgttcata acttcacgca agatgaaaag 1560
tccgtcgtca tcatggatgc agggaccaca ttagcaacta caaatggagc taataatact 1620
gacggtgcta tcaccttaaa caagcttgta atcaatctgg attctttgga tggcactaaa 1680
gcggctgtcg ttaatgtgca gagtaccaat ggagctctca ctatatccgg aactttagga 1740
cttgtgaaaa actctcaaga ttgctgtgac aaccacggga tgtttaataa agatttacag 1800
caagttccga ttttagaact caaagcgact tcaaatactg taaccactac ggacttcagt 1860
ctcggcacaa acggctatca gcaatctccc tatgggtatc aaggaacttg ggagtttacc 1920
atagacacga caacccatac ggtcacagga aattggaaaa aaaccggtta tcttcctcat 1980
ccggagcgtc ttgctcccct cattcctaat agcctatggg caaacgtcat agatttacga 2040
gctgtaagtc aagcgtcagc agctgatggc gaagatgtcc ctgggaagca actgagcatc 2100
acaggaatta caaatttctt ccatgcgaat cataccggtg atgcacgcag ctaccgccat 2160
atgggtggag gctacctcat caatacctac acacgcatca ctccagatgc tgcgttaagt 2220
ctaggttttg gacagctgtt tacaaaatct aaggattacc tcgtaggtca cggtcattct 2280
aacgtttatt tcgctacagt atactctaac atcaccaagt ctctgtttgg atcatcgaga 2340
ttcttctcag gaggcacttc tcgagttacc tatagccgta gcaatgagaa agtaaagact 2400
tcatatacaa aattgcctaa agggcgctgc tcttggagta acaattgctg gttaggagaa 2460
ctcgaaggga accttcccat cactctctct tctcgcatct taaacctcaa gcagatcatt 2520
ccctttgtaa aagctgaagt tgcttacgcg actcatgggg gcatccaaga aaataccccc 2580
gaggggagga tttttggaca cggtcatcta ctcaacgttg cagttcccgt aggcgtccgc 2640
tttggtaaaa attctcataa tcgaccagat ttttacacta taatcgtagc ctatgctcct 2700
gatgtctatc gtcacaatcc tgattgcgat acgacattac ctattaatgg agctacgtgg 2760
acctctatag ggaataatct aaccagaagt actttgctag tacaagcatc cagccatact 2820
tcagtaaatg atgttctaga gatcttcggg cactgtggat gtgatattcg cagaacctcc 2880
cgtcaatata ctctagatat aggaagcaaa ttacgatttt aa 2922

<210> 5
<211> 938
<212> PRT
<213> Chlamydia pneumoniae

<400> 5
Met Arg Phe Phe Cys Phe Gly Met Leu Leu Pro Phe Thr Phe Val Leu
1 5 10 15

Ala Asn Glu Gly Leu Gln Leu Pro Leu Glu Thr Tyr Ile Thr Leu Ser
20 25 30

Pro Glu Tyr Gln Ala Ala Pro Gln Val Gly Phe Thr His Asn Gln Asn
35 40 45

Gln Asp Leu Ala Ile Val Gly Asn His Asn Asp Phe Ile Leu Asp Tyr
50 55 60

Lys Tyr Tyr Arg Ser Asn Gly Gly Ala Leu Thr Cys Lys Asn Leu Leu
65 70 75 80

Ile Ser Glu Asn Ile Gly Asn Val Phe Phe Glu Lys Asn Val Cys Pro
85 90 95

Asn Ser Gly Gly Ala Ile Tyr Ala Ala Gln Asn Cys Thr Ile Ser Lys
100 105 110

Asn Gln Asn Tyr Ala Phe Thr Thr Asn Leu Val Ser Asp Asn Pro Thr
115 120 125

Ala Thr Ala Gly Ser Leu Leu Gly Gly Ala Leu Phe Ala Ile Asn Cys
130 135 140

Ser Ile Thr Asn Asn Leu Gly Gln Gly Thr Phe Val Asp Asn Leu Ala
145 150 155 160

Leu Asn Lys Gly Gly Ala Leu Tyr Thr Glu Thr Asn Leu Ser Ile Lys
165 170 175

Asp Asn Lys Gly Pro Ile Ile Ile Lys Gln Asn Arg Ala Leu Asn Ser
180 185 190

Asp Ser Leu Gly Gly Gly Ile Tyr Ser Gly Asn Ser Leu Asn Ile Glu
195 200 205

Gly Asn Ser Gly Ala Ile Gln Ile Thr Ser Asn Ser Ser Gly Ser Gly
210 215 220

Gly Gly Ile Phe Ser Thr Gln Thr Leu Thr Ile Ser Ser Asn Lys Lys
225 230 235 240

Leu Ile Glu Ile Ser Glu Asn Ser Ala Phe Ala Asn Asn Tyr Gly Ser
245 250 255

Asn Phe Asn Pro Gly Gly Gly Gly Leu Thr Thr Thr Phe Cys Thr Ile
260 265 270

Leu Asn Asn Arg Glu Gly Val Leu Phe Asn Asn Asn Gln Ser Gln Ser
275 280 285

Asn Gly Gly Ala Ile His Ala Lys Ser Ile Ile Ile Lys Glu Asn Gly

290 295 300

Pro Val Tyr Phe Leu Asn Asn Thr Ala Thr Arg Gly Gly Ala Leu Leu
305 310 315 320

Asn Leu Ser Ala Gly Ser Gly Asn Gly Ser Phe Ile Leu Ser Ala Asp
325 330 335

Asn Gly Asp Ile Ile Phe Asn Asn Asn Thr Ala Ser Lys His Ala Leu
340 345 350

Asn Pro Pro Tyr Arg Asn Ala Ile His Ser Thr Pro Asn Met Asn Leu
355 360 365

Gln Ile Gly Ala Arg Pro Gly Tyr Arg Val Leu Phe Tyr Asp Pro Ile
370 375 380

Glu His Glu Leu Pro Ser Ser Phe Pro Ile Leu Phe Asn Phe Glu Thr
385 390 395 400

Gly His Thr Gly Thr Val Leu Phe Ser Gly Glu His Val His Gln Asn
405 410 415

Phe Thr Asp Glu Met Asn Phe Phe Ser Tyr Leu Arg Asn Thr Ser Glu
420 425 430

Leu Arg Gln Gly Val Leu Ala Val Glu Asp Gly Ala Gly Leu Ala Cys
435 440 445

Tyr Lys Phe Phe Gln Arg Gly Gly Thr Leu Leu Leu Gly Gln Gly Ala
450 455 460

Val Ile Thr Thr Ala Gly Thr Ile Pro Thr Pro Ser Ser Thr Pro Thr
465 470 475 480

Thr Val Gly Ser Thr Ile Thr Leu Asn His Ile Ala Ile Asp Leu Pro
485 490 495

Ser Ile Leu Ser Phe Gln Ala Gln Ala Pro Lys Ile Trp Ile Tyr Pro
500 505 510

Thr Lys Thr Gly Ser Thr Tyr Thr Glu Asp Ser Asn Pro Thr Ile Thr
515 520 525

Ile Ser Gly Thr Leu Thr Leu Arg Asn Ser Asn Asn Glu Asp Pro Tyr
530 535 540

Asp Ser Leu Asp Leu Ser His Ser Leu Glu Lys Val Pro Leu Leu Tyr
545 550 555 560

Ile Val Asp Val Ala Ala Gln Lys Ile Asn Ser Ser Gln Leu Asp Leu
565 570 575

Ser Thr Leu Asn Ser Gly Glu His Tyr Gly Tyr Gln Gly Ile Trp Ser
580 585 590

Thr Tyr Trp Val Glu Thr Thr Thr Ile Thr Asn Pro Thr Ser Leu Leu
595 600 605

Gly Ala Asn Thr Lys His Lys Leu Leu Tyr Ala Asn Trp Ser Pro Leu
610 615 620

Gly Tyr Arg Pro His Pro Glu Arg Arg Gly Glu Phe Ile Thr Asn Ala
625 630 635 640

Leu Trp Gln Ser Ala Tyr Thr Ala Leu Ala Gly Leu His Ser Leu Ser
645 650 655

Ser Trp Asp Glu Glu Lys Gly His Ala Ala Ser Leu Gln Gly Ile Gly
660 665 670

Leu Leu Val His Gln Lys Asp Lys Asn Gly Phe Lys Gly Phe Arg Ser
675 680 685

His Met Thr Gly Tyr Ser Ala Thr Thr Glu Ala Thr Ser Ser Gln Ser
690 695 700

Pro Asn Phe Ser Leu Gly Phe Ala Gln Phe Phe Ser Lys Ala Lys Glu
705 710 715 720

His Glu Ser Gln Asn Ser Thr Ser Ser His His Tyr Phe Ser Gly Met
725 730 735

Cys Ile Glu Asn Thr Leu Phe Lys Glu Trp Ile Arg Leu Ser Val Ser
740 745 750

Leu Ala Tyr Met Phe Thr Ser Glu His Thr His Thr Met Tyr Gln Gly
755 760 765

Leu Leu Glu Gly Asn Ser Gln Gly Ser Phe His Asn His Thr Leu Ala
770 775 780

Gly Ala Leu Ser Cys Val Phe Leu Pro Gln Pro His Gly Glu Ser Leu
785 790 795 800

Gln Ile Tyr Pro Phe Ile Thr Ala Leu Ala Ile Arg Gly Asn Leu Ala
805 810 815

Ala Phe Gln Glu Ser Gly Asp His Ala Arg Glu Phe Ser Leu His Arg
820 825 830

Pro Leu Thr Asp Val Ser Leu Pro Val Gly Ile Arg Ala Ser Trp Lys
835 840 845

Asn His His Arg Val Pro Leu Val Trp Leu Thr Glu Ile Ser Tyr Arg
850 855 860

Ser Thr Leu Tyr Arg Gln Asp Pro Glu Leu His Ser Lys Leu Leu Ile
865 870 875 880

Ser Gln Gly Thr Trp Thr Thr Gln Ala Thr Pro Val Thr Tyr Asn Ala
885 890 895

Leu Gly Ile Lys Val Lys Asn Thr Met Gln Val Phe Pro Lys Val Thr
900 905 910

Leu Ser Leu Asp Tyr Ser Ala Asp Ile Ser Ser Ser Thr Leu Ser His
915 920 925

Tyr Leu Asn Val Ala Ser Arg Met Arg Phe
930 935

<210> 6
<211> 2817
<212> DNA
<213> Chlamydia pneumoniae

<400> 6
atgcgctttt tttgcttcgg aatgttgctt ccttttactt ttgtattggc taatgaaggt 60
ctccaacttc ctttggagac ctatattaca ttaagtcctg aatatcaagc agcccctcaa 120
gtagggttta ctcataacca aaatcaagat ctcgcaattg tcgggaatca caatgatttc 180
atcttggact ataagtacta tcggtcgaat ggaggtgctc ttacctgtaa gaatcttctg 240
atctctgaaa atatagggaa tgtcttcttt gagaagaatg tctgtcccaa ttctggcggg 300
gcaatttatg ctgctcaaaa ttgcacgatc tccaagaatc agaactatgc atttactaca 360
aacttggtct ctgacaatcc tacagccact gcgggatcac tattgggtgg agctctcttt 420
gccataaatt gctctattac taataaccta ggacagggaa ctttcgttga caatctcgct 480
ttaaataagg ggggtgccct ctatactgag acgaacttat ctattaaaga caataaaggc 540
ccgatcataa tcaagcagaa tcgggcacta aattcggaca gtttaggagg agggatttat 600
agtgggaact ctctaaatat agagggaaat tctggagcta tacagatcac aagcaactct 660
tcaggatctg gggaggcat attttctacc caaacactca cgatctcctc gaataaaaaa 720
ctcatagaaa tcagtgaaaa ttccgcgttc gcaaataact atggatcgaa cttcaatcca 780
ggaggaggag gtcttactac cacctttgc acgatattga acaaccgaga aggggtactc 840
tttaacaata accaaagcca gagcaacggt ggagccattc atgcgaaatc tatcattatc 900
aaagaaaatg gtcctgtata ctttttaaat aacactgcaa ctcgggggag ggctctcctc 960
aacttatcag caggttctgg aaacggaagc ttcatcttat ctgcagataa tggagatatt 1020
atctttaaca ataatacggc ctccaagcat gccctcaatc ctccatacag aaacgccatt 1080
cactcgactc ctaatatgaa tctgcaaata ggagcccgtc ccggctatcg agtgctgttc 1140
tatgatccca tagaacatga gctcccttcc tccttcccca tactctttaa tttcgaaacc 1200
ggtcatacag gtacagtttt attttcaggg gaacatgtac accagaactt taccgatgaa 1260
atgaatttct ttttcctattt aaggaacact tcggaactac gtcaaggagt ccttgctgtt 1320
gaagatggtg cggggctggc ctgctataag ttcttccaac gaggaggcac tctacttcta 1380
ggtcaaggtg cggtgatcac gacagcagga acgattccca caccatcctc aacaccaacg 1440
acagtaggaa gtactataac tttaaatcac attgccattg accttccttc tattctttct 1500
tttcaagctc aggctccaaa aatttggatt taccccacaa aaacaggatc tacctatact 1560
gaagattcca acccgacaat cacaatctca ggaactctca ccttacgcaa cagcaacaac 1620
gaagatccct acgatagtct ggatctctcg cactctcttg agaaagttcc ccttctttat 1680
attgtcgatg tcgctgcaca aaaaattaac tcttcgcaac tggatctatc cacattaaat 1740
tctggcgaac actatgggta tcaaggcatc tggtcgacct attgggtaga aactacaaca 1800
atcacgaacc ctacatctct actaggcgcg aatacaaaac acaagctgct ctatgcaaac 1860
tggtctcctc taggctaccg tcctcatccc gaacgtcgag gagaattcat tacgaatgcc 1920
ttgtggcaat cggcatatac ggctcttgca ggactccact ccctctcctc ctgggatgaa 1980
gagaagggtc atgcagcttc cctacaaggc attggtcttc tggttcatca aaaagacaaa 2040
aacggtttta agggatttcg tagtcatatg acaggttata gtgctaccac cgaagcaacc 2100
tcttctcaaa gtccgaattt ctctttagga tttgctcagt tcttctccaa agctaaagaa 2160
catgaatctc aaaatagcac gtcctctcac cactatttct ctggaatgtg catagaaaat 2220
actctcttca aagagtggat acgtctatct gtgtctcttg cttatatgtt tacctcggaa 2280
catacccata caatgtatca gggtctcctg gaagggaact ctcagggatc tttccacaac 2340
catacccttag caggggctct ctcctgtgtt ttcttacctc aacctcacgg cgagtccctg 2400
cagatctatc cctttattac tgccttagcc atccgaggaa atcttgctgc gtttcaagaa 2460
tctggagacc atgctcggga attttcccta caccgccccc taacggacgt ctccctccct 2520
gtaggaatcc gcgcttcttg gaagaaccac caccgagttc ccctagtctg gctcacagaa 2580
atttcctatc gctctactct ctataggcaa gatcctgaac tccactcgaa attactgatt 2640
agccaaggta cgtggacgac gcaggccact cctgtgacct acaatgcttt agggatcaaa 2700
gtgaaaaata ccatgcaggt gtttcctaaa gtcactctct ccttagatta ctctgcggat 2760
atttcttcct ccacgctgag tcactactta aacgtggcga gtagaatgag attttaa 2817

<210> 7
<211> 934
<212> PRT
<213> Chlamydia pneumoniae

<400> 7
Met Phe Gly Met Thr Pro Ala Val Tyr Ser Leu Gln Thr Asp Ser Leu

1 5 10 15

Glu Lys Phe Ala Leu Glu Arg Asp Glu Glu Phe Arg Thr Ser Phe Pro
20 25 30

Leu Leu Asp Ser Leu Ser Thr Leu Thr Gly Phe Ser Pro Ile Thr Thr
35 40 45

Phe Val Gly Asn Arg His Asn Ser Ser Gln Asp Ile Val Leu Ser Asn
50 55 60

Tyr Lys Ser Ile Asp Asn Ile Leu Leu Leu Trp Thr Ser Ala Gly Gly
65 70 75 80

Ala Val Ser Cys Asn Asn Phe Leu Leu Ser Asn Val Glu Asp His Ala
85 90 95

Phe Phe Ser Lys Asn Leu Ala Ile Gly Thr Gly Gly Ala Ile Ala Cys
100 105 110

Gln Gly Ala Cys Thr Ile Thr Lys Asn Arg Gly Pro Leu Ile Phe Phe
115 120 125

Ser Asn Arg Gly Leu Asn Asn Ala Ser Thr Gly Gly Glu Thr Arg Gly
130 135 140

Gly Ala Ile Ala Cys Asn Gly Asp Phe Thr Ile Ser Gln Asn Gln Gly
145 150 155 160

Thr Phe Tyr Phe Val Asn Asn Ser Val Asn Asn Trp Gly Gly Ala Leu
165 170 175

Ser Thr Asn Gly His Cys Arg Ile Gln Ser Asn Arg Ala Pro Leu Leu
180 185 190

Phe Phe Asn Asn Thr Ala Pro Ser Gly Gly Gly Ala Leu Arg Ser Glu
195 200 205

Asn Thr Thr Ile Ser Asp Asn Thr Arg Pro Ile Tyr Phe Lys Asn Asn
210 215 220

Cys Gly Asn Asn Gly Gly Ala Ile Gln Thr Ser Val Thr Val Ala Ile
225 230 235 240

Lys Asn Asn Ser Gly Ser Val Ile Phe Asn Asn Asn Thr Ala Leu Ser
245 250 255

Gly Ser Ile Asn Ser Gly Asn Gly Ser Gly Gly Ala Ile Tyr Thr Thr
260 265 270

Asn Leu Ser Ile Asp Asp Asn Pro Gly Thr Ile Leu Phe Asn Asn Asn
275 280 285

Tyr Cys Ile Arg Asp Gly Gly Ala Ile Cys Thr Gln Phe Leu Thr Ile
290 295 300

Lys Asn Ser Gly His Val Tyr Phe Thr Asn Asn Gln Gly Asn Trp Gly
305 310 315 320

Gly Ala Leu Met Leu Leu Gln Asp Ser Thr Cys Leu Leu Phe Ala Glu
325 330 335

```
Gln Gly Asn Ile Ala Phe Gln Asn Asn Glu Val Phe Leu Thr Thr Phe
            340                 345                 350

Gly Arg Tyr Asn Ala Ile His Cys Thr Pro Asn Ser Asn Leu Gln Leu
        355                 360                 365

Gly Ala Asn Lys Gly Tyr Thr Thr Ala Phe Phe Asp Pro Ile Glu His
    370                 375                 380

Gln His Pro Thr Thr Asn Pro Leu Ile Phe Asn Pro Asn Ala Asn His
385                 390                 395                 400

Gln Gly Thr Ile Leu Phe Ser Ser Ala Tyr Ile Pro Glu Ala Ser Asp
                405                 410                 415

Tyr Glu Asn Asn Phe Ile Ser Ser Ser Lys Asn Thr Ser Glu Leu Arg
            420                 425                 430

Asn Gly Val Leu Ser Ile Glu Asp Arg Ala Gly Trp Gln Phe Tyr Lys
        435                 440                 445

Phe Thr Gln Lys Gly Gly Ile Leu Lys Leu Gly His Ala Ala Ser Ile
    450                 455                 460

Ala Thr Thr Ala Asn Ser Glu Thr Pro Ser Thr Ser Val Gly Ser Gln
465                 470                 475                 480

Val Ile Ile Asn Asn Leu Ala Ile Asn Leu Pro Ser Ile Leu Ala Lys
                485                 490                 495

Gly Lys Ala Pro Thr Leu Trp Ile Arg Pro Leu Gln Ser Ser Ala Pro
            500                 505                 510

Phe Thr Glu Asp Asn Asn Pro Thr Ile Thr Leu Ser Gly Pro Leu Thr
        515                 520                 525

Leu Leu Asn Glu Glu Asn Arg Asp Pro Tyr Asp Ser Ile Asp Leu Ser
    530                 535                 540

Glu Pro Leu Gln Asn Ile His Leu Leu Ser Leu Ser Asp Val Thr Ala
545                 550                 555                 560

Arg His Ile Asn Thr Asp Asn Phe His Pro Glu Ser Leu Asn Ala Thr
                565                 570                 575

Glu His Tyr Gly Tyr Gln Gly Ile Trp Ser Pro Tyr Trp Val Glu Thr
            580                 585                 590

Ile Thr Thr Thr Asn Asn Ala Ser Ile Glu Thr Ala Asn Thr Leu Tyr
        595                 600                 605

Arg Ala Leu Tyr Ala Asn Trp Thr Pro Leu Gly Tyr Lys Val Asn Pro
    610                 615                 620

Glu Tyr Gln Gly Asp Leu Ala Thr Thr Pro Leu Trp Gln Ser Phe His
625                 630                 635                 640

Thr Met Phe Ser Leu Leu Arg Ser Tyr Asn Arg Thr Gly Asp Ser Asp
                645                 650                 655
```

```
Ile Glu Arg Pro Phe Leu Glu Ile Gln Gly Ile Ala Asp Gly Leu Phe
            660                 665                 670

Val His Gln Asn Ser Ile Pro Gly Ala Pro Gly Phe Arg Ile Gln Ser
        675                 680                 685

Thr Gly Tyr Ser Leu Gln Ala Ser Ser Glu Thr Ser Leu His Gln Lys
    690                 695                 700

Ile Ser Leu Gly Phe Ala Gln Phe Phe Thr Arg Thr Lys Glu Ile Gly
705                 710                 715                 720

Ser Ser Asn Asn Val Ser Ala His Asn Thr Val Ser Ser Leu Tyr Val
                725                 730                 735

Glu Leu Pro Trp Phe Gln Glu Ala Phe Ala Thr Ser Thr Val Leu Ala
            740                 745                 750

Tyr Gly Tyr Gly Asp His His Leu His Ser Leu His Pro Ser His Gln
        755                 760                 765

Glu Gln Ala Glu Gly Thr Cys Tyr Ser His Thr Leu Ala Ala Ala Ile
    770                 775                 780

Gly Cys Ser Phe Pro Trp Gln Gln Lys Ser Tyr Leu His Leu Ser Pro
785                 790                 795                 800

Phe Val Gln Ala Ile Ala Ile Arg Ser His Gln Thr Ala Phe Glu Glu
                805                 810                 815

Ile Gly Asp Asn Pro Arg Lys Phe Val Ser Gln Lys Pro Phe Tyr Asn
            820                 825                 830

Leu Thr Leu Pro Leu Gly Ile Gln Gly Lys Trp Gln Ser Lys Phe His
        835                 840                 845

Val Pro Thr Glu Trp Thr Leu Glu Leu Ser Tyr Gln Pro Val Leu Tyr
    850                 855                 860

Gln Gln Asn Pro Gln Ile Gly Val Thr Leu Leu Ala Ser Gly Gly Ser
865                 870                 875                 880

Trp Asp Ile Leu Gly His Asn Tyr Val Arg Asn Ala Leu Gly Tyr Lys
                885                 890                 895

Val His Asn Gln Thr Ala Leu Phe Arg Ser Leu Asp Leu Phe Leu Asp
            900                 905                 910

Tyr Gln Gly Ser Val Ser Ser Ser Thr Ser Thr His His Leu Gln Ala
        915                 920                 925

Gly Ser Thr Leu Lys Phe
    930

<210>   8
<211>   2805
<212>   DNA
<213>   Chlamydia pneumoniae

<400>   8
atgttcggga tgactcctgc agtgtatagt ttacaaacgg actcccttga aaagtttgct    60
```

```
ttagagaggg atgaagagtt tcgtacgagc tttcctctct tagactctct ctccactctt    120
acaggatttt ctccaataac tacgtttgtt ggaaatagac ataattcctc tcaagacatt    180
gtactttcta actacaagtc tattgataac atccttcttc tttggacatc ggctggggga    240
gctgtgtcct gtaataattt cttattatca aatgttgaag accatgcctt cttcagtaaa    300
aatctcgcga ttgggactgg aggcgcgatt gcttgccagg gagcctgcac aatcacgaag    360
aatagaggac cccttatttt tttcagcaat cgaggtctta acaatgcgag tacaggagga    420
gaaactcgtg gggtgcgat tgcctgtaat ggagacttca cgatttctca aaatcaaggg    480
actttctact ttgtcaacaa ttccgtcaac aactggggag gagccctctc caccaatgga    540
cactgccgca tccaaagcaa cagggcacct ctactctttt ttaacaatac agcccctagt    600
ggagggggtg cgcttcgtag tgaaaataca acgatctctg ataacacgcg tcctatttat    660
tttaagaaca actgtgggaa caatggcggg gccattcaaa caagcgttac tgttgcgata    720
aaaaataact ccgggtcggt gattttcaat aacaacacag cgttatctgg ttcgataaat    780
tcaggaaatg gttcaggagg ggcgatttat acaacaaacc tatccataga cgataacccct   840
ggaactattc ttttcaataa taactactgc attcgcgatg gcggagctat ctgtacacaa    900
tttttgacaa tcaaaaatag tggccacgta tatttcacca acaatcaagg aaactgggga    960
ggtgctctta tgctcctaca ggacagcacc tgcctactct tcgcggaaca aggaaatatc    1020
gcatttcaaa ataatgaggt tttcctcacc acatttggta gatacaacgc catacattgt    1080
acaccaaata gcaacttaca acttggagct aataaggggt atacgactgc tttttttgat    1140
cctatagaac accaacatcc aactacaaat cctctaatct ttaatcccaa tgcgaaccat    1200
cagggaacga tcttattttc ttcagcctat atcccagaag cttctgacta cgaaaataat    1260
ttcattagca gctcgaaaaa tacctctgaa cttcgcaatg gtgtcctctc tatcgaggat    1320
cgtgcgggat ggcaattcta taagttcact caaaaaggag gtatccttaa attagggcat    1380
gcggcgagta ttgcaacaac tgccaactct gagactccat caactagtgt aggctcccag    1440
gtcatcatta ataaccttgc gattaacctc ccctcgatct tagcaaaagg aaaagctcct    1500
accttgtgga tccgtcctct acaatctagt gctcctttca cagaggacaa taaccctaca    1560
attactttat caggtcctct gacactctta aatgaggaaa accgcgatcc ctacgacagt    1620
atagatctct ctgagccttt acaaaacatt catcttcttt ctttatcgga tgtaacagca    1680
cgtcatatca ataccgataa ctttcatcct gaaagcttaa atgcgactga gcattacggt    1740
tatcaaggca tctggtctcc ttattgggta gagacgataa caacaacaaa taacgcttct    1800
atagagacgg caaacaccct ctacagagct ctgtatgcca attggactcc cttaggatat    1860
aaggtcaatc ctgaatacca aggagatctt gctacgactc ccctatggca atcctttcat    1920
actatgttct ctctattaag aagttataat cgaactggtg attctgatat cgagaggcct    1980
ttcttagaaa ttcaagggat tgccgacggc ctctttgttc atcaaaatag catccccggg    2040
gctccaggat tccgtatcca atctacaggg tattccttac aagcatcctc cgaaacttct    2100
ttacatcaga aatctccttt aggttttgca cagttcttca cccgcactaa agaaatcgga    2160
tcaagcaaca acgtctcggc tcacaataca gtctcttcac tttatgttga gcttccgtgg    2220
ttccaagagg cctttgcaac atccacagtg ttagcgtatg gctatgggga ccatcacctc    2280
cacagcctac atccctcaca tcaagaacag gcagaaggga cgtgttatag ccatacatta    2340
gcagcagcta tcggctgttc tttcccttgg caacagaaat cctatcttca cctcagcccg    2400
ttcgttcagg caattgcaat acgttctcac caaacagcgt tcgaagagat tggtgacaat    2460
ccccgaaagt ttgtctctca aaagcctttc tataatctga ccttacctct aggaatccaa    2520
ggaaaatggc agtcaaaatt ccacgtacct acagaatgga ctctagaact ttcttaccaa    2580
ccggtactct atcaacaaaa tccccaaatc ggtgtcacgc tacttgcgag cggaggttcc    2640
tgggatatcc taggccataa ctatgttcgc aatgctttag ggtacaaagt ccacaatcaa    2700
actgcgctct tccgttctct cgatctattc ttggattacc aaggatcggt ctcctcctcg    2760
acatctacgc accatctcca agcaggaagt accttaaaat tctaa                    2805
```

<210> 9
<211> 90
<212> PRT
<213> Chlamydia pneumoniae

<400> 9

```
Met Lys Lys Ala Val Leu Ile Ala Ala Met Phe Cys Gly Val Val Ser
1               5                   10                  15

Leu Ser Ser Cys Cys Arg Ile Val Asp Cys Cys Phe Glu Asp Pro Cys
            20                  25                  30

Ala Pro Ser Ser Cys Asn Pro Cys Glu Val Ile Arg Lys Lys Glu Arg
        35                  40                  45
```

```
Ser Cys Gly Gly Asn Ala Cys Gly Ser Tyr Val Pro Ser Cys Ser Asn
    50                  55                  60

Pro Cys Gly Ser Thr Glu Cys Asn Ser Gln Ser Pro Gln Val Lys Gly
65                  70                  75                  80

Cys Thr Ser Pro Asp Gly Arg Cys Lys Gln
                85                  90
```

<210> 10
<211> 273
<212> DNA
<213> Chlamydia pneumoniae

<400> 10

```
atgaagaaag ctgttttaat tgctgcaatg ttttgtggag tagttagctt aagtagctgc    60
tgccgcattg tagattgttg ttttgaggat ccttgcgcac cctcttcttg caatccttgt   120
gaagtaataa gaaaaaaaga aagatcttgc ggcggtaatg cttgtgggtc ctacgttcct   180
tcttgttcta atccatgtgg ttcaacagag tgtaactctc aaagcccaca agttaaaggt   240
tgtacatcac ctgatggcag atgcaaacag taa                                273
```

<210> 11
<211> 250
<212> PRT
<213> Chlamydia pneumoniae

<400> 11

```
Met Lys Ile Lys Phe Ser Trp Lys Val Asn Phe Leu Ile Cys Leu Leu
1               5                   10                  15

Ala Val Gly Leu Ile Phe Phe Gly Cys Ser Arg Val Lys Arg Glu Val
            20                  25                  30

Leu Val Gly Arg Asp Ala Thr Trp Phe Pro Lys Gln Phe Gly Ile Tyr
        35                  40                  45

Thr Ser Asp Thr Asn Ala Phe Leu Asn Asp Leu Val Ser Glu Ile Asn
    50                  55                  60

Tyr Lys Glu Asn Leu Asn Ile Asn Ile Val Asn Gln Asp Trp Val His
65                  70                  75                  80

Leu Phe Glu Asn Leu Asp Asp Lys Lys Thr Gln Gly Ala Phe Thr Ser
                85                  90                  95

Val Leu Pro Thr Leu Glu Met Leu Glu His Tyr Gln Phe Ser Asp Pro
            100                 105                 110

Ile Leu Leu Thr Gly Pro Val Leu Val Val Ala Gln Asp Ser Pro Tyr
        115                 120                 125

Gln Ser Ile Glu Asp Leu Lys Gly Arg Leu Ile Gly Val Tyr Lys Phe
    130                 135                 140

Asp Ser Ser Val Leu Val Ala Gln Asn Ile Pro Asp Ala Val Ile Ser
145                 150                 155                 160

Leu Tyr Gln His Val Pro Ile Ala Leu Glu Ala Leu Thr Ser Asn Cys
                165                 170                 175
```

```
Tyr Asp Ala Leu Leu Ala Pro Val Ile Glu Val Thr Ala Leu Ile Glu
            180                 185                 190

Thr Ala Tyr Lys Gly Arg Leu Lys Ile Ile Ser Lys Pro Leu Asn Ala
        195                 200                 205

Asp Gly Leu Arg Leu Ala Ile Leu Lys Gly Thr Asn Gly Asp Leu Leu
    210                 215                 220

Glu Gly Phe Asn Ala Gly Leu Val Lys Thr Arg Arg Ser Gly Lys Tyr
225                 230                 235                 240

Asp Ala Ile Lys Gln Arg Tyr Arg Leu Pro
                245                 250

<210>   12
<211>   753
<212>   DNA
<213>   Chlamydia pneumoniae

<400>   12
gtgaagataa aattttcttg gaaggtaaat tttttaatat gtttactggc tgtgggactg    60
atctttttcg ggtgctctcg agtaaaaaga gaagttctcg taggtcgtga tgccacctgg   120
tttccaaaac aattcggcat ttatacatcc gataccaacg catttttaaa cgatcttgtt   180
tctgagatta actataaaga gaatctaaat attaatattg taaatcaaga ttgggtgcat   240
ctctttgaga atttagatga taaaaagacc caaggagcat ttacatctgt attgcctact   300
cttgagatgc tcgaacacta tcaattttct gatcccattt tactcacagg tcctgtcctt   360
gtcgtcgctc aagactctcc ttaccaatct atagaggatc ttaaaggtcg tcttattgga   420
gtgtataagt ttgactcttc agttcttgta gctcaaaata tccctgacgc tgtgattagc   480
ctctaccaac atgttccaat agcattggaa gccttaacat cgaattgtta cgacgctctt   540
ctagctcctg taattgaagt gaccgcgcta atagaaacag catataaagg aagactgaaa   600
attatttcaa aacccttaaa cgcagatggt ttgcggcttg caatactgaa agggacaaac   660
ggagatttgc ttgaagggtt taacgcagga cttgtgaaaa cacgacgctc aggaaaatac   720
gatgctataa aacagcggta tcgtcttccc taa                                753

<210>   13
<211>   651
<212>   PRT
<213>   Chlamydia pneumoniae

<400>   13
Met Val Asn Pro Ile Gly Pro Gly Pro Ile Asp Glu Thr Glu Arg Thr
1               5                   10                  15

Pro Pro Ala Asp Leu Ser Ala Gln Gly Leu Glu Ala Ser Ala Ala Asn
            20                  25                  30

Lys Ser Ala Glu Ala Gln Arg Ile Ala Gly Ala Glu Ala Lys Pro Lys
        35                  40                  45

Glu Ser Lys Thr Asp Ser Val Glu Arg Trp Ser Ile Leu Arg Ser Ala
    50                  55                  60

Val Asn Ala Leu Met Ser Leu Ala Asp Lys Leu Gly Ile Ala Ser Ser
65                  70                  75                  80

Asn Ser Ser Ser Ser Thr Ser Arg Ser Ala Asp Val Asp Ser Thr Thr
                85                  90                  95

Ala Thr Ala Pro Thr Pro Pro Pro Pro Thr Phe Asp Asp Tyr Lys Thr
            100                 105                 110
```

```
Gln Ala Gln Thr Ala Tyr Asp Thr Ile Phe Thr Ser Thr Ser Leu Ala
        115                 120                 125

Asp Ile Gln Ala Ala Leu Val Ser Leu Gln Asp Ala Val Thr Asn Ile
    130                 135                 140

Lys Asp Thr Ala Ala Thr Asp Glu Glu Thr Ala Ile Ala Ala Glu Trp
145                 150                 155                 160

Glu Thr Lys Asn Ala Asp Ala Val Lys Val Gly Ala Gln Ile Thr Glu
                165                 170                 175

Leu Ala Lys Tyr Ala Ser Asp Asn Gln Ala Ile Leu Asp Ser Leu Gly
            180                 185                 190

Lys Leu Thr Ser Phe Asp Leu Leu Gln Ala Ala Leu Leu Gln Ser Val
        195                 200                 205

Ala Asn Asn Asn Lys Ala Ala Glu Leu Leu Lys Glu Met Gln Asp Asn
    210                 215                 220

Pro Val Val Pro Gly Lys Thr Pro Ala Ile Ala Gln Ser Leu Val Asp
225                 230                 235                 240

Gln Thr Asp Ala Thr Ala Thr Gln Ile Glu Lys Asp Gly Asn Ala Ile
                245                 250                 255

Arg Asp Ala Tyr Phe Ala Gly Gln Asn Ala Ser Gly Ala Val Glu Asn
            260                 265                 270

Ala Lys Ser Asn Asn Ser Ile Ser Asn Ile Asp Ser Ala Lys Ala Ala
        275                 280                 285

Ile Ala Thr Ala Lys Thr Gln Ile Ala Glu Ala Gln Lys Lys Phe Pro
    290                 295                 300

Asp Ser Pro Ile Leu Gln Glu Ala Glu Gln Met Val Ile Gln Ala Glu
305                 310                 315                 320

Lys Asp Leu Lys Asn Ile Lys Pro Ala Asp Gly Ser Asp Val Pro Asn
                325                 330                 335

Pro Gly Thr Thr Val Gly Gly Ser Lys Gln Gln Gly Ser Ser Ile Gly
            340                 345                 350

Ser Ile Arg Val Ser Met Leu Leu Asp Asp Ala Glu Asn Glu Thr Ala
        355                 360                 365

Ser Ile Leu Met Ser Gly Phe Arg Gln Met Ile His Met Phe Asn Thr
    370                 375                 380

Glu Asn Pro Asp Ser Gln Ala Ala Gln Gln Glu Leu Ala Ala Gln Ala
385                 390                 395                 400

Arg Ala Ala Lys Ala Ala Gly Asp Asp Ser Ala Ala Ala Ala Leu Ala
                405                 410                 415

Asp Ala Gln Lys Ala Leu Glu Ala Ala Leu Gly Lys Ala Gly Gln Gln
            420                 425                 430
```

```
Gln Gly Ile Leu Asn Ala Leu Gly Gln Ile Ala Ser Ala Ala Val Val
        435                 440                 445

Ser Ala Gly Val Pro Pro Ala Ala Ala Ser Ser Ile Gly Ser Ser Val
    450                 455                 460

Lys Gln Leu Tyr Lys Thr Ser Lys Ser Thr Gly Ser Asp Tyr Lys Thr
465                 470                 475                 480

Gln Ile Ser Ala Gly Tyr Asp Ala Tyr Lys Ser Ile Asn Asp Ala Tyr
                485                 490                 495

Gly Arg Ala Arg Asn Asp Ala Thr Arg Asp Val Ile Asn Asn Val Ser
            500                 505                 510

Thr Pro Ala Leu Thr Arg Ser Val Pro Arg Ala Arg Thr Glu Ala Arg
        515                 520                 525

Gly Pro Glu Lys Thr Asp Gln Ala Leu Ala Arg Val Ile Ser Gly Asn
    530                 535                 540

Ser Arg Thr Leu Gly Asp Val Tyr Ser Gln Val Ser Ala Leu Gln Ser
545                 550                 555                 560

Val Met Gln Ile Ile Gln Ser Asn Pro Gln Ala Asn Asn Glu Glu Ile
                565                 570                 575

Arg Gln Lys Leu Thr Ser Ala Val Thr Lys Pro Pro Gln Phe Gly Tyr
            580                 585                 590

Pro Tyr Val Gln Leu Ser Asn Asp Ser Thr Gln Lys Phe Ile Ala Lys
        595                 600                 605

Leu Glu Ser Leu Phe Ala Glu Gly Ser Arg Thr Ala Ala Glu Ile Lys
    610                 615                 620

Ala Leu Ser Phe Glu Thr Asn Ser Leu Phe Ile Gln Gln Val Leu Val
625                 630                 635                 640

Asn Ile Gly Ser Leu Tyr Ser Gly Tyr Leu Gln
                645                 650

<210>   14
<211>   1956
<212>   DNA
<213>   Chlamydia pneumoniae

<400>   14
atggttaatc ctattggtcc aggtcctata gacgaaacag aacgcacacc tcccgcagat     60
ctttctgctc aaggattgga ggcgagtgca gcaaataaga gtgcggaagc tcaaagaata    120
gcaggtgcgg aagctaagcc taaagaatct aagaccgatt ctgtagagcg atggagcatc    180
ttgcgttctg cagtgaatgc tctcatgagt ctggcagata agctgggtat tgcttctagt    240
aacagctcgt cttctactag cagatctgca gacgtggact caacgacagc gaccgcacct    300
acgcctcctc cacccacgtt tgatgattat aagactcaag cgcaaacagc ttacgatact    360
atctttacct caacatcact agctgacata caggctgctt tggtgagcct ccaggatgct    420
gtcactaata taaaggatac agcggctact gatgaggaaa ccgcaatcgc tgcggagtgg    480
gaaactaaga atgccgatgc agttaaagtt ggcgcgcaaa ttacagaatt agcgaaatat    540
gcttcggata accaagcgat tcttgactct ttaggtaaac tgacttcctt cgacctctta    600
caggctgctc ttctccaatc tgtagcaaac aataacaaag cagctgagct tcttaaagag    660
atgcaagata acccagtagt cccagggaaa acgcctgcaa ttgctcaatc tttagttgat    720
cagacagatg ctacagcgac acagatagag aaagatggaa atgcgattag ggatgcatat    780
```

```
tttgcaggac agaacgctag tggagctgta gaaaatgcta aatctaataa cagtataagc    840
aacatagatt cagctaaagc agcaatcgct actgctaaga cacaaatagc tgaagctcag    900
aaaaagttcc ccgactctcc aattcttcaa gaagcggaac aaatggtaat acaggctgag    960
aaagatctta aaaatatcaa acctgcagat ggttctgatg ttccaaatcc aggaactaca   1020
gttggaggct ccaagcaaca aggaagtagt attggtagta ttcgtgtttc catgctgtta   1080
gatgatgctg aaaatgagac cgcttccatt ttgatgtctg ggtttcgtca gatgattcac   1140
atgttcaata cggaaaatcc tgattctcaa gctgcccaac aggagctcgc agcacaagct   1200
agagcagcga aagccgctgg agatgacagt gctgctgcag cgctggcaga tgctcagaaa   1260
gctttagaag cggctctagg taaagctggg caacaacagg gcatactcaa tgctttagga   1320
cagatcgctt ctgctgctgt tgtgagcgca ggagttcctc ccgctgcagc aagttctata   1380
gggtcatctg taaaacagct ttacaagacc tcaaaatcta caggttctga ttataaaaca   1440
cagatatcag caggttatga tgcttacaaa tccatcaatg atgcctatgg tagggcacga   1500
aatgatgcga ctcgtgatgt gataaacaat gtaagtaccc ccgctctcac acgatccgtt   1560
cctagagcac gaacagaagc tcgaggacca gaaaaaacag atcaagccct cgctagggtg   1620
atttctggca atagcagaac tcttggagat gtctatagtc aagtttcggc actacaatct   1680
gtaatgcaga tcatccagtc gaatcctcaa gcgaataatg aggagatcag acaaaagctt   1740
acatcggcag tgacaaagcc tccacagttt ggctatcctt atgtgcaact ttctaatgac   1800
tctacacaga agttcatagc taaattagaa agtttgtttg ctgaaggatc taggacagca   1860
gctgaaataa aagcactttc ctttgaaacg aactccttgt ttattcagca ggtgctggtc   1920
aatatcggct ctctatattc tggttatctc caataa                             1956

<210>   15
<211>   354
<212>   PRT
<213>   Chlamydia pneumoniae

<400>   15
Met Gly Ile Lys Gly Thr Gly Ile Ile Val Trp Val Asp Asp Ala Thr
1               5                   10                  15

Ala Lys Thr Lys Asn Ala Thr Leu Thr Trp Thr Lys Thr Gly Tyr Lys
            20                  25                  30

Pro Asn Pro Glu Arg Gln Gly Pro Leu Val Pro Asn Ser Leu Trp Gly
        35                  40                  45

Ser Phe Val Asp Val Arg Ser Ile Gln Ser Leu Met Asp Arg Ser Thr
    50                  55                  60

Ser Ser Leu Ser Ser Ser Thr Asn Leu Trp Val Ser Gly Ile Ala Asp
65                  70                  75                  80

Phe Leu His Glu Asp Gln Lys Gly Asn Gln Arg Ser Tyr Arg His Ser
                85                  90                  95

Ser Ala Gly Tyr Ala Leu Gly Gly Gly Phe Phe Thr Ala Ser Glu Asn
            100                 105                 110

Phe Phe Asn Phe Ala Phe Cys Gln Leu Phe Gly Tyr Asp Lys Asp His
        115                 120                 125

Leu Val Ala Lys Asn His Thr His Val Tyr Ala Gly Ala Met Ser Tyr
    130                 135                 140

Arg His Leu Gly Glu Ser Lys Thr Leu Ala Lys Ile Leu Ser Gly Asn
145                 150                 155                 160

Ser Asp Ser Leu Pro Phe Val Phe Asn Ala Arg Phe Ala Tyr Gly His
                165                 170                 175

Thr Asp Asn Asn Met Thr Thr Lys Tyr Thr Gly Tyr Ser Pro Val Lys
```

180 185 190

Gly Ser Trp Gly Asn Asp Ala Phe Gly Ile Glu Cys Gly Gly Ala Ile
195 200 205

Pro Val Val Ala Ser Gly Arg Arg Ser Trp Val Asp Thr His Thr Pro
210 215 220

Phe Leu Asn Leu Glu Met Ile Tyr Ala His Gln Asn Asp Phe Lys Glu
225 230 235 240

Asn Gly Thr Glu Gly Arg Ser Phe Gln Ser Glu Asp Leu Phe Asn Leu
245 250 255

Ala Val Pro Val Gly Ile Lys Phe Glu Lys Phe Ser Asp Lys Ser Thr
260 265 270

Tyr Asp Leu Ser Ile Ala Tyr Val Pro Asp Val Ile Arg Asn Asp Pro
275 280 285

Gly Cys Thr Thr Thr Leu Met Val Ser Gly Asp Ser Trp Ser Thr Cys
290 295 300

Gly Thr Ser Leu Ser Arg Gln Ala Leu Leu Val Arg Ala Gly Asn His
305 310 315 320

His Ala Phe Ala Ser Asn Phe Glu Val Phe Ser Gln Phe Glu Val Glu
325 330 335

Leu Arg Gly Ser Ser Arg Ser Tyr Ala Ile Asp Leu Gly Gly Arg Phe
340 345 350

Gly Phe

<210> 16
<211> 1065
<212> DNA
<213> Chlamydia pneumoniae

<400> 16
atgggtatca agggaactgg aataattgtt tgggtcgacg atgcaactgc aaaaacaaaa 60
aatgctacct taacttggac taaaacagga tacaagccga atccagaacg tcagggacct 120
ttggttccta atagcctgtg ggttcttttt gtcgatgtcc gctccattca gagcctcatg 180
gaccggagca caagttcgtt atcttcgtca acaaatttgt gggtatcagg aatcgcggac 240
tttttgcatg aagatcagaa aggaaaccaa cgtagttatc gtcattctag cgcgggttat 300
gcattaggag gaggattctt cacggcttct gaaaatttct ttaattttgc tttttgtcag 360
ctttttggct acgacaagga ccatcttgtg gctaagaacc atacccatgt atatgcaggg 420
gcaatgagtt accgacacct cggagagtct aagaccctcg ctaagatttt gtcaggaaat 480
tctgactccc taccttttgt cttcaatgct cggtttgctt atggccatac cgacaataac 540
atgaccacaa agtacactgg ctattctcct gttaagggaa gctggggaaa tgatgccttc 600
ggtatagaat gtggaggagc tatcccggta gttgcttcag gacgtcggtc ttgggtggat 660
acccacacgc catttctaaa cctagagatg atctatgcac atcagaatga ctttaaggaa 720
aacggcacag aaggccgttc tttccaaagt gaagacctct tcaatctagc ggttcctgta 780
gggataaaat ttgagaaatt ctccgataag tctacgtatg atctctccat agcttacgtt 840
cccgatgtga ttcgtaatga tccaggctgc acgacaactc ttatggtttc tggggattct 900
tggtcgacat gtggtacaag cttgtctaga caagctcttc ttgtacgtgc tggaaatcat 960
catgcctttg cttcaaactt tgaagttttc agtcagtttg aagtcgagtt gcgaggttct 1020
tctcgtagct atgctatcga tcttggagga agattcggat tttaa 1065

<210> 17

<211> 177
<212> PRT
<213> Chlamydia pneumoniae

<400> 17
Met Lys Ser Ser Phe Pro Lys Phe Val Phe Ser Thr Phe Ala Ile Phe
1 5 10 15

Pro Leu Ser Met Ile Ala Thr Glu Thr Val Leu Asp Ser Ser Ala Ser
20 25 30

Phe Asp Gly Asn Lys Asn Gly Asn Phe Ser Val Arg Glu Ser Gln Glu
35 40 45

Asp Ala Gly Thr Thr Tyr Leu Phe Lys Gly Asn Val Thr Leu Glu Asn
50 55 60

Ile Pro Gly Thr Gly Thr Ala Ile Thr Lys Ser Cys Phe Asn Asn Thr
65 70 75 80

Lys Gly Asp Leu Thr Phe Thr Gly Asn Gly Asn Ser Leu Leu Phe Gln
85 90 95

Thr Val Asp Ala Gly Thr Val Ala Gly Ala Ala Val Asn Ser Ser Val
100 105 110

Val Asp Lys Ser Thr Thr Phe Ile Gly Phe Ser Ser Leu Ser Phe Ile
115 120 125

Ala Ser Pro Gly Ser Ser Ile Thr Thr Gly Lys Gly Ala Val Ser Cys
130 135 140

Ser Thr Gly Ser Leu Ser Leu Thr Lys Met Ser Val Cys Ser Ser Ala
145 150 155 160

Lys Thr Phe Gln Arg Ile Met Ala Val Leu Ser Pro Gln Lys Leu Phe
165 170 175

His

<210> 18
<211> 534
<212> DNA
<213> Chlamydia pneumoniae

<400> 18
atgaagtctt ctttccccaa gtttgtattt tctacatttg ctattttccc tttgtctatg 60
attgctaccg agacagtttt ggattcaagt gcgagtttcg atgggaataa aaatggtaat 120
tttcagttc gtgagagtca ggaagatgct ggaactacct acctatttaa gggaaatgtc 180
actctagaaa atattcctgg aacaggcaca gcaatcacaa aaagctgttt taacaacact 240
aagggcgatt tgactttcac aggtaacggg aactctctat tgttccaaac ggtggatgca 300
gggactgtag caggggctgc tgttaacagc agcgtggtag ataaatctac cacgtttata 360
gggttttctt cgctatcttt tattgcgtct cctggaagtt cgataactac cggcaaagga 420
gccgttagct gctctacggg tagcttgagt ttgacaaaaa tgtcagtttg ctcttcagca 480
aaaacttttc aacggataat ggcggtgcta tcaccgcaaa aactctttca ttaa 534

<210> 19
<211> 746
<212> PRT
<213> Chlamydia pneumoniae

```
<400>  19
Met Ser Ala Leu Phe Ser Glu Asn Thr Ser Ser Lys Lys Gly Gly Ala
1               5                   10                  15

Ile Gln Thr Ser Asp Ala Leu Thr Ile Thr Gly Asn Gln Gly Glu Val
            20                  25                  30

Ser Phe Ser Asp Asn Thr Ser Ser Asp Ser Gly Ala Ala Ile Phe Thr
        35                  40                  45

Glu Ala Ser Val Thr Ile Ser Asn Asn Ala Lys Val Ser Phe Ile Asp
    50                  55                  60

Asn Lys Val Thr Gly Ala Ser Ser Ser Thr Thr Gly Asp Met Ser Gly
65                  70                  75                  80

Gly Ala Ile Cys Ala Tyr Lys Thr Ser Thr Asp Thr Lys Val Thr Leu
                85                  90                  95

Thr Gly Asn Gln Met Leu Leu Phe Ser Asn Asn Thr Ser Thr Thr Ala
            100                 105                 110

Gly Gly Ala Ile Tyr Val Lys Lys Leu Glu Leu Ala Ser Gly Gly Leu
        115                 120                 125

Thr Leu Phe Ser Arg Asn Ser Val Asn Gly Gly Thr Ala Pro Lys Gly
    130                 135                 140

Gly Ala Ile Ala Ile Glu Asp Ser Gly Glu Leu Ser Leu Ser Ala Asp
145                 150                 155                 160

Ser Gly Asp Ile Val Phe Leu Gly Asn Thr Val Thr Ser Thr Thr Pro
                165                 170                 175

Gly Thr Asn Arg Ser Ser Ile Asp Leu Gly Thr Ser Ala Lys Met Thr
            180                 185                 190

Ala Leu Arg Ser Ala Ala Gly Arg Ala Ile Tyr Phe Tyr Asp Pro Ile
        195                 200                 205

Thr Thr Gly Ser Ser Thr Thr Val Thr Asp Val Leu Lys Val Asn Glu
    210                 215                 220

Thr Pro Ala Asp Ser Ala Leu Gln Tyr Thr Gly Asn Ile Ile Phe Thr
225                 230                 235                 240

Gly Glu Lys Leu Ser Glu Thr Glu Ala Ala Asp Ser Lys Asn Leu Thr
                245                 250                 255

Ser Lys Leu Leu Gln Pro Val Thr Leu Ser Gly Gly Thr Leu Ser Leu
            260                 265                 270

Lys His Gly Val Thr Leu Gln Thr Gln Ala Phe Thr Gln Gln Ala Asp
        275                 280                 285

Ser Arg Leu Glu Met Asp Val Gly Thr Thr Leu Glu Pro Ala Asp Thr
    290                 295                 300

Ser Thr Ile Asn Asn Leu Val Ile Asn Ile Ser Ser Ile Asp Gly Ala
305                 310                 315                 320
```

Lys Lys Ala Lys Ile Glu Thr Lys Ala Thr Ser Lys Asn Leu Thr Leu
325 330 335

Ser Gly Thr Ile Thr Leu Leu Asp Pro Thr Gly Thr Phe Tyr Glu Asn
340 345 350

His Ser Leu Arg Asn Pro Gln Ser Tyr Asp Ile Leu Glu Leu Lys Ala
355 360 365

Ser Gly Thr Val Thr Ser Thr Ala Val Thr Pro Asp Pro Ile Met Gly
370 375 380

Glu Lys Phe His Tyr Gly Tyr Gln Gly Thr Trp Gly Pro Ile Val Trp
385 390 395 400

Gly Thr Gly Ala Ser Thr Thr Ala Thr Phe Asn Trp Thr Lys Thr Gly
405 410 415

Tyr Ile Pro Asn Pro Glu Arg Ile Gly Ser Leu Val Pro Asn Ser Leu
420 425 430

Trp Asn Ala Phe Ile Asp Ile Ser Ser Leu His Tyr Leu Met Glu Thr
435 440 445

Ala Asn Glu Gly Leu Gln Gly Asp Arg Ala Phe Trp Cys Ala Gly Leu
450 455 460

Ser Asn Phe Phe His Lys Asp Ser Thr Lys Thr Arg Arg Gly Phe Arg
465 470 475 480

His Leu Ser Gly Gly Tyr Val Ile Gly Gly Asn Leu His Thr Cys Ser
485 490 495

Asp Lys Ile Leu Ser Ala Ala Phe Cys Gln Leu Phe Gly Arg Asp Arg
500 505 510

Asp Tyr Phe Val Ala Lys Asn Gln Gly Thr Val Tyr Gly Gly Thr Leu
515 520 525

Tyr Tyr Gln His Asn Glu Thr Tyr Ile Ser Leu Pro Cys Lys Leu Arg
530 535 540

Pro Cys Ser Leu Ser Tyr Val Pro Thr Glu Ile Pro Val Leu Phe Ser
545 550 555 560

Gly Asn Leu Ser Tyr Thr His Thr Asp Asn Asp Leu Lys Thr Lys Tyr
565 570 575

Thr Thr Tyr Pro Thr Val Lys Gly Ser Trp Gly Asn Asp Ser Phe Ala
580 585 590

Leu Glu Phe Gly Gly Arg Ala Pro Ile Cys Leu Asp Glu Ser Ala Leu
595 600 605

Phe Glu Gln Tyr Met Pro Phe Met Lys Leu Gln Phe Val Tyr Ala His
610 615 620

Gln Glu Gly Phe Lys Glu Gln Gly Thr Glu Ala Arg Glu Phe Gly Ser
625 630 635 640

```
Ser Arg Leu Val Asn Leu Ala Leu Pro Ile Gly Ile Arg Phe Asp Lys
                645                 650                 655

Glu Ser Asp Cys Gln Asp Ala Thr Tyr Asn Leu Thr Leu Gly Tyr Thr
            660                 665                 670

Val Asp Leu Val Arg Ser Asn Pro Asp Cys Thr Thr Thr Leu Arg Ile
        675                 680                 685

Ser Gly Asp Ser Trp Lys Thr Phe Gly Thr Asn Leu Ala Arg Gln Ala
    690                 695                 700

Leu Val Leu Arg Ala Gly Asn His Phe Cys Phe Asn Ser Asn Phe Glu
705                 710                 715                 720

Ala Phe Ser Gln Phe Ser Phe Glu Leu Arg Gly Ser Ser Arg Asn Tyr
                725                 730                 735

Asn Val Asp Leu Gly Ala Lys Tyr Gln Phe
            740                 745

<210>   20
<211>   2241
<212>   DNA
<213>   Chlamydia pneumoniae

<400>   20
atgtcagctc tgttttctga aaatacctcc tcaaagaaag gcggagccat tcagacttcc    60
gatgccctta ccattactgg aaaccaaggg gaagtctctt tttctgacaa tacttcttcg   120
gattctggag ctgcaatttt tacagaagcc tcggtgacta tttctaataa tgctaaagtt   180
tcctttattg acaataaggt cacaggagcg agctcctcaa caacgggggga tatgtcagga   240
ggtgctatct gtgcttataa aactagtaca gatactaagg tcaccctcac tggaaatcag   300
atgttactct tcagcaacaa tacatcgaca acagcgggag gagctatcta tgtgaaaaag   360
ctcgaactgg cttccggagg acttacccta ttcagtagaa atagtgtcaa tggaggtaca   420
gctcctaaag gtggagccat agctatcgaa gatagtgggg aattgagttt atccgccgat   480
agtggtgaca ttgtcttttt agggaataca gtcacttcta ctactcctgg gacgaataga   540
agtagtatcg acttaggaac gagtgcaaag atgacagctt tgcgttctgc tgctggtaga   600
gccatctact tctatgatcc cataactaca ggatcatcca caacagttac agatgtctta   660
aaagttaatg agactccggc agattctgca ctacaatata cagggaacat catcttcaca   720
ggagaaaagt tatcagagac agaggccgca gattctaaaa atcttacttc gaagctacta   780
cagcctgtaa ctctttcagg aggtactcta tctttaaaac atggagtgac tctgcagact   840
caggcattca ctcaacaggc agattctcgt ctcgaaatgg acgtaggaac tactctagaa   900
cctgctgata ctagcaccat aaacaatttg gtcattaaca tcagttctat agacggtgca   960
aagaaggcaa aaatagaaac caaagctacg tcaaaaaatc tgactttatc tggaaccatc  1020
actttattgg acccgacggg cacgttttat gaaaatcata gtttaagaaa tcctcagtcc  1080
tacgacatct tagagctcaa agcttctgga actgtaacaa gcaccgcagt gactccagat  1140
cctataatgg gtgagaaatt ccattacggc tatcagggaa cttgggggcc caattgtttgg  1200
gggacagggg cttctacgac tgcaaccttc aactggacta aaactggcta tattcctaat  1260
cccgagcgta tcggctcttt agtccctaat agcttatgga atgcatttat agatattagc  1320
tctctccatt atcttatgga gactgcaaac gaagggttgc agggagaccg tgctttttgg  1380
tgtgctggat tatctaactt cttccataag gatagtacaa aaacacgacg cgggtttcgc  1440
catttgagtg gcggttatgt cataggagga aacctacata cttgttcaga taagattctt  1500
agtgctgcat tttgtcagct ctttggaaga gatagagact actttgtagc taagaatcaa  1560
ggtacagtct acggaggaac tctctattac cagcacaacg aaacctatat ctctcttcct  1620
tgcaaactac ggccttgttc gttgtcttat gttcctacag agattcctgt tctcttttca  1680
ggaaacctta gctacaccca tacggataac gatctgaaaa ccaagtatac aacatatcct  1740
actgttaaag gaagctgggg gaatgatagt ttcgctttag aattcggtgg aagagctccg  1800
atttgcttag atgaaagtgc tctatttgag cagtacatgc ccttcatgaa attgcagttt  1860
gtctatgcac atcaggaagg ttttaaagaa cagggaacag aagctcgtga atttggaagt  1920
agccgtcttg tgaatcttgc cttacctatc gggatccgat ttgataagga atcagactgc  1980
caagatgcaa cgtacaatct aactcttggt tatactgtgg atcttgttcg tagtaacccc  2040
```

```
gactgtacga caacactgcg aattagcggt gattcttgga aaaccttcgg tacgaatttg   2100
gcaagacaag ctttagtcct tcgtgcaggg aaccattttt gctttaactc aaattttgaa   2160
gcctttagcc aattttcttt tgaattgcgt gggtcatctc gcaattacaa tgtagactta   2220
ggagcaaaat accaattcta a                                             2241

<210>  21
<211>  395
<212>  PRT
<213>  Chlamydia pneumoniae

<400>  21
Leu Gln Asp Ser Gln Asp Tyr Ser Phe Val Lys Leu Ser Pro Gly Ala
1               5                   10                  15

Gly Gly Thr Ile Ile Thr Gln Asp Ala Ser Gln Lys Pro Leu Glu Val
            20                  25                  30

Ala Pro Ser Arg Pro His Tyr Gly Tyr Gln Gly His Trp Asn Val Gln
        35                  40                  45

Val Ile Pro Gly Thr Gly Thr Gln Pro Ser Gln Ala Asn Leu Glu Trp
    50                  55                  60

Val Arg Thr Gly Tyr Leu Pro Asn Pro Glu Arg Gln Gly Ser Leu Val
65                  70                  75                  80

Pro Asn Ser Leu Trp Gly Ser Phe Val Asp Gln Arg Ala Ile Gln Glu
                85                  90                  95

Ile Met Val Asn Ser Ser Gln Ile Leu Cys Gln Glu Arg Gly Val Trp
            100                 105                 110

Gly Ala Gly Ile Ala Asn Phe Leu His Arg Asp Lys Ile Asn Glu His
        115                 120                 125

Gly Tyr Arg His Ser Gly Val Gly Tyr Leu Val Gly Val Gly Thr His
    130                 135                 140

Ala Phe Ser Asp Ala Thr Ile Asn Ala Ala Phe Cys Gln Leu Phe Ser
145                 150                 155                 160

Arg Asp Lys Asp Tyr Val Val Ser Lys Asn His Gly Thr Ser Tyr Ser
                165                 170                 175

Gly Val Val Phe Leu Glu Asp Thr Leu Glu Phe Arg Ser Pro Gln Gly
            180                 185                 190

Phe Tyr Thr Asp Ser Ser Ser Glu Ala Cys Cys Asn Gln Val Val Thr
        195                 200                 205

Ile Asp Met Gln Leu Ser Tyr Ser His Arg Asn Asn Asp Met Lys Thr
    210                 215                 220

Lys Tyr Thr Thr Tyr Pro Glu Ala Gln Gly Ser Trp Ala Asn Asp Val
225                 230                 235                 240

Phe Gly Leu Glu Phe Gly Ala Thr Thr Tyr Tyr Tyr Pro Asn Ser Thr
                245                 250                 255

Phe Leu Phe Asp Tyr Tyr Ser Pro Phe Leu Arg Leu Gln Cys Thr Tyr
            260                 265                 270
```

Ala His Gln Glu Asp Phe Lys Glu Thr Gly Gly Glu Val Arg His Phe
275 280 285

Thr Ser Gly Asp Leu Phe Asn Leu Ala Val Pro Ile Gly Val Lys Phe
290 295 300

Glu Arg Phe Ser Asp Cys Lys Arg Gly Ser Tyr Glu Leu Thr Leu Ala
305 310 315 320

Tyr Val Pro Asp Val Ile Arg Lys Asp Pro Lys Ser Thr Ala Thr Leu
325 330 335

Ala Ser Gly Ala Thr Trp Ser Thr His Gly Asn Asn Leu Ser Arg Gln
340 345 350

Gly Leu Gln Leu Arg Leu Gly Asn His Cys Leu Ile Asn Pro Gly Ile
355 360 365

Glu Val Phe Ser His Gly Ala Ile Glu Leu Arg Gly Ser Ser Arg Asn
370 375 380

Tyr Asn Ile Asn Leu Gly Gly Lys Tyr Arg Phe
385 390 395

<210> 22
<211> 1188
<212> DNA
<213> Chlamydia pneumoniae

<400> 22
ttgcaagact ctcaagacta tagctttgta aagttatctc caggagcggg agggactata 60
attactcaag atgcttctca gaagcctctt gaagtagctc cttctagacc acattatggc 120
tatcaaggac attggaatgt gcaagtcatc ccaggaacgg gaactcaacc gagccaggca 180
aatttagaat gggtgcggac aggatacctt ccgaatcccg aacggcaagg atctttagtt 240
cccaatagcc tgtggggttc ttttgttgat cagcgtgcta tccaagaaat catggtaaat 300
agtagccaaa tcttatgtca ggaacgggga gtctggggag ctggaattgc taatttccta 360
catagagata aaattaatga gcacggctat cgccatagcg gtgtcggtta tcttgtggga 420
gttggcactc atgctttttc tgatgctacg ataaatgcgg ctttttgcca gctcttcagt 480
agagataaag actacgtagt atccaaaaat catggaacta gctactcagg ggtcgtattt 540
cttgaggata ccctagagtt tagaagtcca cagggattct atactgatag ctcctcagaa 600
gcttgctgta accaagtcgt cactatagat atgcagttgt cttacagcca tagaaataat 660
gatatgaaaa ccaaatacac gacatatcca gaagctcagg gatcttgggc aaatgatgtt 720
tttggtcttg agtttggagc gactacatac tactacccta acagtacttt tttatttgat 780
tactactctc cgtttctcag gctgcagtgc acctatgctc accaggaaga cttcaaagag 840
acaggaggtg aggttcgtca ctttactagc ggagatcttt tcaatttagc agttcctatt 900
ggcgtgaagt ttgagagatt ttcagactgt aaaaggggat cttatgaact tacccttgct 960
tatgttcctg atgtgattcg caaagatccc aagagcacgg caacattggc tagtggagct 1020
acgtggagca cccacggaaa caatctctcc agacaaggat tacaactgcg tttagggaac 1080
cactgtctca taaatcctgg aattgaggtg ttcagtcacg gagctattga attgcgggga 1140
tcctctcgta attataacat caatctcggg ggtaaatacc gattttaa 1188

<210> 23
<211> 532
<212> PRT
<213> Chlamydia pneumoniae

<400> 23
Met Arg Lys Ile Ser Val Gly Ile Cys Ile Thr Ile Leu Leu Ser Leu
1 5 10 15

Ser Val Val Leu Gln Gly Cys Lys Glu Ser Ser His Ser Ser Thr Ser
20 25 30

Arg Gly Glu Leu Ala Ile Asn Ile Arg Asp Glu Pro Arg Ser Leu Asp
35 40 45

Pro Arg Gln Val Arg Leu Leu Ser Glu Ile Ser Leu Val Lys His Ile
50 55 60

Tyr Glu Gly Leu Val Gln Glu Asn Asn Leu Ser Gly Asn Ile Glu Pro
65 70 75 80

Ala Leu Ala Glu Asp Tyr Ser Leu Ser Ser Asp Gly Leu Thr Tyr Thr
85 90 95

Phe Lys Leu Lys Ser Ala Phe Trp Ser Asn Gly Asp Pro Leu Thr Ala
100 105 110

Glu Asp Phe Ile Glu Ser Trp Lys Gln Val Ala Thr Gln Glu Val Ser
115 120 125

Gly Ile Tyr Ala Phe Ala Leu Asn Pro Ile Lys Asn Val Arg Lys Ile
130 135 140

Gln Glu Gly His Leu Ser Ile Asp His Phe Gly Val His Ser Pro Asn
145 150 155 160

Glu Ser Thr Leu Val Val Thr Leu Glu Ser Pro Thr Ser His Phe Leu
165 170 175

Lys Leu Leu Ala Leu Pro Val Phe Phe Pro Val His Lys Ser Gln Arg
180 185 190

Thr Leu Gln Ser Lys Ser Leu Pro Ile Ala Ser Gly Ala Phe Tyr Pro
195 200 205

Lys Asn Ile Lys Gln Lys Gln Trp Ile Lys Leu Ser Lys Asn Pro His
210 215 220

Tyr Tyr Asn Gln Ser Gln Val Glu Thr Lys Thr Ile Thr Ile His Phe
225 230 235 240

Ile Pro Asp Ala Asn Thr Ala Ala Lys Leu Phe Asn Gln Gly Lys Leu
245 250 255

Asn Trp Gln Gly Pro Pro Trp Gly Glu Arg Ile Pro Gln Glu Thr Leu
260 265 270

Ser Asn Leu Gln Ser Lys Gly His Leu His Ser Phe Asp Val Ala Gly
275 280 285

Thr Ser Trp Leu Thr Phe Asn Ile Asn Lys Phe Pro Leu Asn Asn Met
290 295 300

Lys Leu Arg Glu Ala Leu Ala Ser Ala Leu Asp Lys Glu Ala Leu Val
305 310 315 320

Ser Thr Ile Phe Leu Gly Arg Ala Lys Thr Ala Asp His Leu Leu Pro
325 330 335

Thr Asn Ile His Ser Tyr Pro Glu His Gln Lys Gln Glu Met Ala Gln

340 345 350

Arg Gln Ala Tyr Ala Lys Lys Leu Phe Lys Glu Ala Leu Glu Glu Leu
355 360 365

Gln Ile Thr Ala Lys Asp Leu Glu His Leu Asn Leu Ile Phe Pro Val
370 375 380

Ser Ser Ser Ala Ser Ser Leu Leu Val Gln Leu Ile Arg Glu Gln Trp
385 390 395 400

Lys Glu Ser Leu Gly Phe Ala Ile Pro Ile Val Gly Lys Glu Phe Ala
405 410 415

Leu Leu Gln Ala Asp Leu Ser Ser Gly Asn Phe Ser Leu Ala Thr Gly
420 425 430

Gly Trp Phe Ala Asp Phe Ala Asp Pro Met Ala Phe Leu Thr Ile Phe
435 440 445

Ala Tyr Pro Ser Gly Val Pro Pro Tyr Ala Ile Asn His Lys Asp Phe
450 455 460

Leu Glu Ile Leu Gln Asn Ile Glu Gln Glu Gln Asp His Gln Lys Arg
465 470 475 480

Ser Glu Leu Val Ser Gln Ala Ser Leu Tyr Leu Glu Thr Phe His Ile
485 490 495

Ile Glu Pro Ile Tyr His Asp Ala Phe Gln Phe Ala Met Asn Lys Lys
500 505 510

Leu Ser Asn Leu Gly Val Ser Pro Thr Gly Val Val Asp Phe Arg Tyr
515 520 525

Ala Lys Glu Asn
530

<210> 24
<211> 1599
<212> DNA
<213> Chlamydia pneumoniae

<400> 24
atgcgcaaga tatcagtggg aatctgtatc accattctcc ttagcctctc cgtagtcctc 60
caaggctgca aggagtccag tcactcctct acatctcggg gagaactcgc tattaatata 120
agagatgaac cccgttcttt agatccaaga caagtgcgac ttctttcaga aatcagcctt 180
gtcaaacata tctatgaggg attagttcaa gaaaataatc tttcaggaaa tatagagcct 240
gctcttgcag aagactactc tctttcctcg gacggactca cttatacttt taaactgaaa 300
tcagcttttt ggagtaatgg cgaccccttta acagctgaag actttataga atcttggaaa 360
caagtagcta ctcaagaagt ctcaggaatc tatgcttttg ccttgaatcc aattaaaaat 420
gtacgaaaga tccaagaggg acacctctcc atagaccatt ttggagtgca ctctcctaat 480
gaatctacac ttgttgttac cctggaatcc ccaacctcgc atttcttaaa actttttagct 540
cttccagtct ttttccccgt tcataaatct caaagaaccc tgcaatccaa atctctacct 600
atagcaagcg gagctttcta tcctaaaaat atcaaacaaa aacaatggat aaaactctca 660
aaaaaccctc actactataa tcaaagtcag gtggaaacta aaacgattac gattcacttc 720
attcccgatg caaacacagc agcaaaacta tttaatcagg gaaaactcaa ttggcaagga 780
cctccttggg gagaacgcat tcctcaagaa accctatcca atttacagtc taagggggcac 840
ttacactctt ttgatgtcgc aggaacctca tggctcacct tcaatatcaa taaattcccc 900
ctcaacaata tgaagcttag agaagcctta gcatcagcct tagataagga agctcttgtc 960
tcaactatat tcttaggccg tgcaaaaact gccgatcatc tcctacctac aaatattcat 1020

```
agctatcccg aacatcaaaa acaagagatg gcacaacgcc aagcttacgc taaaaaactc    1080
tttaaagaag ctttagaaga actccaaatc actgctaaag atctcgaaca tcttaatctt    1140
atctttcccg tttcctcgtc agcaagttct ttactagtcc aacttatacg agaacagtgg    1200
aaagaaagtt tagggttcgc tatccctatt gtcggaaagg aatttgctct tctccaagca    1260
gacctatctt cagggaactt ctctttagct acaggaggat ggttcgcaga ctttgctgat    1320
cctatggcat ttctaacgat ctttgcttat ccatcaggag ttcctcctta tgcaatcaac    1380
cataaggact tcctagaaat tctacaaaac atagaacaag agcaagatca ccaaaaacgc    1440
tcggaattag tgtcgcaagc ttctctttac ctagagacct ttcatattat tgagccgatc    1500
taccacgacg catttcaatt tgctatgaat aaaaaacttt ctaatctagg agtctcacca    1560
acaggagttg tggacttccg ttatgctaag gaaaattag                          1599
```

<210> 25
<211> 435
<212> PRT
<213> Chlamydia pneumoniae

<400> 25

```
Met Phe Ser Arg Trp Ile Thr Leu Phe Leu Leu Phe Ile Ser Leu Thr
1               5                   10                  15

Gly Cys Ser Ser Tyr Ser Ser Lys His Lys Gln Ser Leu Ile Ile Pro
            20                  25                  30

Ile His Asp Asp Pro Val Ala Phe Ser Pro Glu Gln Ala Lys Arg Ala
        35                  40                  45

Met Asp Leu Ser Ile Ala Gln Leu Leu Phe Asp Gly Leu Thr Arg Glu
    50                  55                  60

Thr His Arg Glu Ser Asn Asp Leu Glu Leu Ala Ile Ala Ser Arg Tyr
65                  70                  75                  80

Thr Val Ser Glu Asp Phe Cys Ser Tyr Thr Phe Phe Ile Lys Asp Ser
                85                  90                  95

Ala Leu Trp Ser Asp Gly Thr Pro Ile Thr Ser Glu Asp Ile Arg Asn
            100                 105                 110

Ala Trp Glu Tyr Ala Gln Glu Asn Ser Pro His Ile Gln Ile Phe Gln
        115                 120                 125

Gly Leu Asn Phe Ser Thr Pro Ser Ser Asn Ala Ile Thr Ile His Leu
    130                 135                 140

Asp Ser Pro Asn Pro Asp Phe Pro Lys Leu Leu Ala Phe Pro Ala Phe
145                 150                 155                 160

Ala Ile Phe Lys Pro Glu Asn Pro Lys Leu Phe Ser Gly Pro Tyr Thr
                165                 170                 175

Leu Val Glu Tyr Phe Pro Gly His Asn Ile His Leu Lys Lys Asn Pro
            180                 185                 190

Asn Tyr Tyr Asp Tyr His Cys Val Ser Ile Asn Ser Ile Lys Leu Leu
        195                 200                 205

Ile Ile Pro Asp Ile Tyr Thr Ala Ile His Leu Leu Asn Arg Gly Lys
    210                 215                 220

Val Asp Trp Val Gly Gln Pro Trp His Gln Gly Ile Pro Trp Glu Leu
225                 230                 235                 240
```

His Lys Gln Ser Gln Tyr His Tyr Tyr Thr Tyr Pro Val Glu Gly Ala
245 250 255

Phe Trp Leu Cys Leu Asn Thr Lys Ser Pro His Leu Asn Asp Leu Gln
260 265 270

Asn Arg His Arg Leu Ala Thr Cys Ile Asp Lys Arg Ser Ile Ile Glu
275 280 285

Glu Ala Leu Gln Gly Thr Gln Gln Pro Ala Glu Thr Leu Ser Arg Gly
290 295 300

Ala Pro Gln Pro Asn Gln Tyr Lys Lys Gln Lys Pro Leu Thr Pro Gln
305 310 315 320

Glu Lys Leu Val Leu Thr Tyr Pro Ser Asp Ile Leu Arg Cys Gln Arg
325 330 335

Ile Ala Glu Ile Leu Lys Glu Gln Trp Lys Ala Ala Gly Ile Asp Leu
340 345 350

Ile Leu Glu Gly Leu Glu Tyr His Leu Phe Val Asn Lys Arg Lys Val
355 360 365

Gln Asp Tyr Ala Ile Ala Thr Gln Thr Gly Val Ala Tyr Tyr Pro Gly
370 375 380

Ala Asn Leu Ile Ser Glu Glu Asp Lys Leu Leu Gln Asn Phe Glu Ile
385 390 395 400

Ile Pro Ile Tyr Tyr Leu Ser Tyr Asp Tyr Leu Thr Gln Asp Phe Ile
405 410 415

Glu Gly Val Ile Tyr Asn Ala Ser Gly Ala Val Asp Leu Lys Tyr Thr
420 425 430

Tyr Phe Pro
435

<210> 26
<211> 1308
<212> DNA
<213> Chlamydia pneumoniae

<400> 26
atgttttcac gatggatcac cctcttttta ttattcatta gccttactgg atgctcctcc 60
tactcttcaa aacataaaca atctttaatt attcccatac atgacgaccc tgtagctttt 120
tctcctgaac aagcaaaacg ggccatggac ctttctattg cccaacttct ttttgatggt 180
ctgactagag aaactcatcg cgaatccaat gatttggaat tagcgattgc cagtcgctat 240
acagtctctg aagacttttg ctcttatacg ttctttatca aagacagcgc tttatggagc 300
gacggaacac caatcacctc cgaagatatc cgtaacgctt gggagtatgc acaggagaac 360
tctccccaca tacagatctt ccaaggactt aacttctcaa ctccttcatc aaatgcaatt 420
acgattcatc tcgactcgcc caaccccgat ttcctaagc ttcttgcctt tcctgcattt 480
gctatcttta aaccagaaaa cccgaagctc tttagcggtc cgtatactct tgtagagtat 540
ttcccagggc ataacattca tttaaagaaa aaccctaact attacgacta ccactgcgtc 600
tccatcaact ccatcaaact gctcattatt cctgatatat atacagccat ccacctccta 660
aacagaggca aggtggactg ggtaggacaa ccctggcatc aagggattcc ttgggagctc 720
cataaacaat cgcaatatca ctactacacc tatcctgtag aaggtgcctt ctggctttgt 780
ctaaatacaa aatccccaca cttaaatgat cttcaaaaca gacatagact cgctacttgt 840
attgataaac gttctatcat tgaagaagct cttcaaggaa cccaacaacc agcggaaaca 900

```
ctgtcccgag gagctccaca accaaatcaa tataaaaaac aaaagcctct aactccacaa    960
gaaaaactcg tgcttaccta tccctcagat attctaagat gccaacgcat agcagaaatc   1020
ttaaaggaac aatggaaagc tgctggaata gatttaatcc ttgaaggact cgaataccat   1080
ctgtttgtta acaaacgaaa agtccaagac tacgccatag caacacagac tggagttgct   1140
tattacccag gagcaaatct aatttctgaa gaagacaagc tcctgcaaaa ctttgagatt   1200
atcccgatct actatctgag ctatgactat ctcactcaag attttataga gggagtaatc   1260
tataatgctt ctggagctgt agatctcaaa tatacctatt tcccctag                1308

<210>  27
<211>  528
<212>  PRT
<213>  Chlamydia pneumoniae

<400>  27
Met Lys Met His Arg Leu Lys Pro Thr Leu Lys Ser Leu Ile Pro Asn
1               5                   10                  15

Leu Leu Phe Leu Leu Leu Thr Leu Ser Ser Cys Ser Lys Gln Lys Gln
            20                  25                  30

Glu Pro Leu Gly Lys His Leu Val Ile Ala Met Ser His Asp Leu Ala
        35                  40                  45

Asp Leu Asp Pro Arg Asn Ala Tyr Leu Ser Arg Asp Ala Ser Leu Ala
    50                  55                  60

Lys Ala Leu Tyr Glu Gly Leu Thr Arg Glu Thr Asp Gln Gly Ile Ala
65                  70                  75                  80

Leu Ala Leu Ala Glu Ser Tyr Thr Leu Ser Lys Asp His Lys Val Tyr
                85                  90                  95

Thr Phe Lys Leu Arg Pro Ser Val Trp Ser Asp Gly Thr Pro Leu Thr
            100                 105                 110

Ala Tyr Asp Phe Glu Lys Ser Ile Lys Gln Leu Tyr Phe Glu Glu Phe
        115                 120                 125

Ser Pro Ser Ile His Thr Leu Leu Gly Val Ile Lys Asn Ser Ser Ala
    130                 135                 140

Ile His Asn Ala Gln Lys Ser Leu Glu Thr Leu Gly Ile Gln Ala Lys
145                 150                 155                 160

Asp Asp Leu Thr Leu Val Ile Thr Leu Glu Gln Pro Phe Pro Tyr Phe
                165                 170                 175

Leu Thr Leu Ile Ala Arg Pro Val Phe Ser Pro Val His His Thr Leu
            180                 185                 190

Arg Glu Ser Tyr Lys Lys Gly Thr Pro Pro Ser Thr Tyr Ile Ser Asn
        195                 200                 205

Gly Pro Phe Val Leu Lys Lys His Glu His Gln Asn Tyr Leu Ile Leu
    210                 215                 220

Glu Lys Asn Pro His Tyr Tyr Asp His Glu Ser Val Lys Leu Asp Arg
225                 230                 235                 240

Val Thr Leu Lys Ile Ile Pro Asp Ala Ser Thr Ala Thr Lys Leu Phe
                245                 250                 255
```

```
Lys Ser Lys Ser Ile Asp Trp Ile Gly Ser Pro Trp Ser Ala Pro Ile
            260                 265                 270

Ser Asn Glu Asp Gln Lys Val Leu Ser Gln Glu Lys Ile Leu Thr Tyr
        275                 280                 285

Ser Val Ser Ser Thr Thr Leu Leu Ile Tyr Asn Leu Gln Lys Pro Leu
    290                 295                 300

Ile Gln Asn Lys Ala Leu Arg Lys Ala Ile Ala His Ala Ile Asp Arg
305                 310                 315                 320

Lys Ser Ile Leu Arg Leu Val Pro Ser Gly Gln Glu Ala Val Thr Leu
                325                 330                 335

Val Pro Pro Asn Leu Ser Gln Leu Asn Leu Gln Lys Glu Ile Ser Thr
            340                 345                 350

Glu Glu Arg Gln Thr Lys Ala Arg Ala Tyr Phe Gln Glu Ala Lys Glu
        355                 360                 365

Thr Leu Ser Glu Lys Glu Leu Ala Glu Leu Ser Ile Leu Tyr Pro Ile
    370                 375                 380

Asp Ser Ser Asn Ser Ser Ile Ile Ala Gln Glu Ile Gln Arg Gln Leu
385                 390                 395                 400

Lys Asp Thr Leu Gly Leu Lys Ile Lys Ile Gln Gly Met Glu Tyr His
                405                 410                 415

Cys Phe Leu Lys Lys Arg Arg Gln Gly Asp Phe Phe Ile Ala Thr Gly
            420                 425                 430

Gly Trp Ile Ala Glu Tyr Val Ser Pro Val Ala Phe Leu Ser Ile Leu
        435                 440                 445

Gly Asn Pro Arg Asp Leu Thr Gln Trp Arg Asn Ser Asp Tyr Glu Lys
    450                 455                 460

Thr Leu Glu Lys Leu Tyr Leu Pro His Ala Tyr Lys Glu Asn Leu Lys
465                 470                 475                 480

Arg Ala Glu Met Ile Ile Glu Glu Glu Thr Pro Ile Ile Pro Leu Tyr
                485                 490                 495

His Gly Lys Tyr Ile Tyr Ala Ile His Pro Lys Ile Gln Asn Thr Phe
            500                 505                 510

Gly Ser Leu Leu Gly His Thr Asp Leu Lys Asn Ile Asp Ile Leu Ser
        515                 520                 525
```

<210> 28
<211> 1587
<212> DNA
<213> Chlamydia pneumoniae

<400> 28
```
atgaagatgc ataggcttaa acctacctta aaaagtctga tccctaatct tcttttctta    60
ttgctcactc tttcaagctg ctcaaagcaa aaacaagaac ccttaggaaa acatctcgtt   120
```

```
attgcgatga gccatgatct cgccgaccta gatcctcgca atgcctattt aagcagagat    180
gcttccctag caaaagccct ctatgaagga ctgacaagag aaactgatca aggaatcgca    240
ctggctcttg cagaaagtta taccctgtca aaagatcata aggtctatac ctttaaactc    300
agaccttctg tgtggagcga tggcactcca ctcactgctt atgactttga aaaatctata    360
aaacaactgt acttcgaaga attttcacct tccatacata ctttactcgg cgtgattaaa    420
aattcttcgg caatccacaa tgctcaaaaa tctctggaaa ctcttgggat acaggcaaaa    480
gatgatctta ctttggtgat taccctagag caacctttcc catactttct cacacttatc    540
gctcgccccg tattctcccc tgttcatcac acccttaggg aatcctataa gaaaggaaca    600
cccccatcca catacatctc caatgggccc tttgtcttaa aaaaacatga acaccaaaac    660
tacttaattt tagaaaaaaa tcctcactac tatgatcatg aatcagtaaa gttagaccga    720
gtcaccttaa aaattatccc agacgcctcc acagccacga aacttttcaa aagtaaatct    780
atagattgga ttggctcacc ttggagcgct ccgatatcta acgaagacca aaaagttctc    840
tcccaagaaa agattcttac ctattctgtt tcaagcacca cccttcttat ctataacctg    900
caaaaacctc taatacaaaa taaagccctc aggaaagcca ttgctcatgc tattgataga    960
aaatctatct taagactcgt gccttcagga caagaagctg taactctagt tcccccaaat   1020
ctttcacaac tcaatcttca aaaagagatc tcaacagaag aacgacaaac aaaagccaga   1080
gcatattttc aagaagctaa agaaacactt tctgaaaaag aactcgcaga actcagcatc   1140
ctctatccta tagattcctc gaattcctcc atcatagctc aagaaatcca aagacaactt   1200
aaagatacct taggattgaa aatcaaaatc caaggcatgg agtaccactg ctttttaaag   1260
aaacgtcgtc aaggagattt cttcatagcg acaggaggat ggattgcgga atacgtaagc   1320
cccgtagcct tcctatctat tctaggcaac cccagagacc tcacacaatg gagaaacagt   1380
gattacgaaa agactttaga gaaactctat ctccctcatg cctacaaaga gaatttaaaa   1440
cgcgcagaaa tgataataga agaagaaacc ccgattatcc ccctgtatca cggcaaatat   1500
atttacgcta tacatcctaa aatccagaat acattcggat ctcttctagg ccacacagat   1560
ctcaaaaata tcgatatctt aagttag                                        1587
```

<210> 29
<211> 399
<212> PRT
<213> Chlamydia pneumoniae

<400> 29

```
Met Ala Ala Ser Gly Gly Thr Gly Gly Leu Gly Gly Thr Gln Gly Val
1               5                   10                  15

Asn Leu Ala Ala Val Glu Ala Ala Ala Ala Lys Ala Asp Ala Ala Glu
            20                  25                  30

Val Val Ala Ser Gln Glu Gly Ser Glu Met Asn Met Ile Gln Gln Ser
        35                  40                  45

Gln Asp Leu Thr Asn Pro Ala Ala Ala Thr Arg Thr Lys Lys Lys Glu
    50                  55                  60

Glu Lys Phe Gln Thr Leu Glu Ser Arg Lys Lys Gly Glu Ala Gly Lys
65                  70                  75                  80

Ala Glu Lys Lys Ser Glu Ser Thr Glu Glu Lys Pro Asp Thr Asp Leu
                85                  90                  95

Ala Asp Lys Tyr Ala Ser Gly Asn Ser Glu Ile Ser Gly Gln Glu Leu
            100                 105                 110

Arg Gly Leu Arg Asp Ala Ile Gly Asp Asp Ala Ser Pro Glu Asp Ile
        115                 120                 125

Leu Ala Leu Val Gln Glu Lys Ile Lys Asp Pro Ala Leu Gln Ser Thr
    130                 135                 140

Ala Leu Asp Tyr Leu Val Gln Thr Thr Pro Pro Ser Gln Gly Lys Leu
145                 150                 155                 160
```

```
Lys Glu Ala Leu Ile Gln Ala Arg Asn Thr His Thr Glu Gln Phe Gly
                165                 170                 175

Arg Thr Ala Ile Gly Ala Lys Asn Ile Leu Phe Ala Ser Gln Glu Tyr
            180                 185                 190

Ala Asp Gln Leu Asn Val Ser Pro Ser Gly Leu Arg Ser Leu Tyr Leu
        195                 200                 205

Glu Val Thr Gly Asp Thr His Thr Cys Asp Gln Leu Leu Ser Met Leu
    210                 215                 220

Gln Asp Arg Tyr Thr Tyr Gln Asp Met Ala Ile Val Ser Ser Phe Leu
225                 230                 235                 240

Met Lys Gly Met Ala Thr Glu Leu Lys Arg Gln Gly Pro Tyr Val Pro
                245                 250                 255

Ser Ala Gln Leu Gln Val Leu Met Thr Glu Thr Arg Asn Leu Gln Ala
            260                 265                 270

Val Leu Thr Ser Tyr Asp Tyr Phe Glu Ser Arg Val Pro Ile Leu Leu
        275                 280                 285

Asp Ser Leu Lys Ala Glu Gly Ile Gln Thr Pro Ser Asp Leu Asn Phe
    290                 295                 300

Val Lys Val Ala Glu Ser Tyr His Lys Ile Ile Asn Asp Lys Phe Pro
305                 310                 315                 320

Thr Ala Ser Lys Val Glu Arg Glu Val Arg Asn Leu Ile Gly Asp Asp
                325                 330                 335

Val Asp Ser Val Thr Gly Val Leu Asn Leu Phe Phe Ser Ala Leu Arg
            340                 345                 350

Gln Thr Ser Ser Arg Leu Phe Ser Ser Ala Asp Lys Arg Gln Gln Leu
        355                 360                 365

Gly Ala Met Ile Ala Asn Ala Leu Asp Ala Val Asn Ile Asn Asn Glu
    370                 375                 380

Asp Tyr Pro Lys Ala Ser Asp Phe Pro Lys Pro Tyr Pro Trp Ser
385                 390                 395

<210>   30
<211>   1200
<212>   DNA
<213>   Chlamydia pneumoniae

<400>   30
atggcagcat caggaggcac aggtggttta ggaggcactc agggtgtcaa ccttgcagct    60
gtagaagctg cagctgcaaa agcagatgca gcagaagttg tagccagcca agaaggttct   120
gagatgaaca tgattcaaca atctcaggac ctgacaaatc ccgcagcagc aacacgcacg   180
aaaaaaaagg aagagaagtt tcaaactcta gaatctcgga aaaaaggaga agctggaaag   240
gctgagaaaa aatctgaatc tacagaagag aagcctgaca cagatcttgc tgataagtat   300
gcttctggga attctgaaat ctctggtcaa gaacttcgcg gcctgcgtga tgcaatagga   360
gacgatgctt ctccagaaga cattcttgct cttgtacaag agaaaattaa agacccagct   420
ctgcaatcca cagctttgga ctacctggtt caaacgactc caccctccca aggtaaatta   480
aaagaagcgc ttatccaagc aaggaatact catacggagc aattcggacg aactgctatt   540
```

```
ggtgcgaaaa acatcttatt tgcctctcaa gaatatgcag accaactgaa tgtttctcct   600
tcagggcttc gctctttgta cttagaagtg actggagaca cacatacctg tgatcagcta   660
ctttctatgc ttcaagaccg ctatacctac caagatatgg ctattgtcag ctcctttcta   720
atgaaaggaa tggcaacaga attaaaaagg cagggtccct acgtacccag tgcgcaacta   780
caagttctca tgacagaaac tcgtaacctg caagcagttc ttacctcgta cgattacttt   840
gaaagtcgcg ttcctatttt actcgatagc ttaaaagctg agggaatcca aactccttct   900
gatctaaact ttgtgaaggt agctgagtcc taccataaaa tcattaacga taagttccca   960
acagcatcta aagtagaacg agaagtccgc aatctcatag gagacgatgt tgattctgtg  1020
accggtgtct tgaacttatt cttttctgct ttacgtcaaa cgtcgtcacg cctttttctct 1080
tcagcagaca aacgtcagca attaggagct atgattgcta atgctttaga tgctgtaaat  1140
ataaacaatg aagattatcc caaagcatca gacttcccta aaccctatcc ttggtcatga  1200
```

<210> 31
<211> 1407
<212> PRT
<213> Chlamydia pneumoniae

<400> 31

```
Met Lys Tyr Ser Leu Pro Trp Leu Leu Thr Ser Ser Ala Leu Val Phe
1               5                   10                  15

Ser Leu His Pro Leu Met Ala Ala Asn Thr Asp Leu Ser Ser Ser Asp
            20                  25                  30

Asn Tyr Glu Asn Gly Ser Ser Gly Ser Ala Ala Phe Thr Ala Lys Glu
        35                  40                  45

Thr Ser Asp Ala Ser Gly Thr Thr Tyr Thr Leu Thr Ser Asp Val Ser
    50                  55                  60

Ile Thr Asn Val Ser Ala Ile Thr Pro Ala Asp Lys Ser Cys Phe Thr
65                  70                  75                  80

Asn Thr Gly Gly Ala Leu Ser Phe Val Gly Ala Asp His Ser Leu Val
                85                  90                  95

Leu Gln Thr Ile Ala Leu Thr His Asp Gly Ala Ala Ile Asn Asn Thr
            100                 105                 110

Asn Thr Ala Leu Ser Phe Ser Gly Phe Ser Ser Leu Leu Ile Asp Ser
        115                 120                 125

Ala Pro Ala Thr Gly Thr Ser Gly Gly Lys Gly Ala Ile Cys Val Thr
    130                 135                 140

Asn Thr Glu Gly Gly Thr Ala Thr Phe Thr Asp Asn Ala Ser Val Thr
145                 150                 155                 160

Leu Gln Lys Asn Thr Ser Glu Lys Asp Gly Ala Ala Val Ser Ala Tyr
                165                 170                 175

Ser Ile Asp Leu Ala Lys Thr Thr Thr Ala Ala Leu Leu Asp Gln Asn
            180                 185                 190

Thr Ser Thr Lys Asn Gly Gly Ala Leu Cys Ser Thr Ala Asn Thr Thr
        195                 200                 205

Val Gln Gly Asn Ser Gly Thr Val Thr Phe Ser Ser Asn Thr Ala Thr
    210                 215                 220

Asp Lys Gly Gly Gly Ile Tyr Ser Lys Glu Lys Asp Ser Thr Leu Asp
```

225 230 235 240

Ala Asn Thr Gly Val Val Thr Phe Lys Ser Asn Thr Ala Lys Thr Gly
245 250 255

Gly Ala Trp Ser Ser Asp Asp Asn Leu Ala Leu Thr Gly Asn Thr Gln
260 265 270

Val Leu Phe Gln Glu Asn Lys Thr Thr Gly Ser Ala Ala Gln Ala Asn
275 280 285

Asn Pro Glu Gly Cys Gly Gly Ala Ile Cys Cys Tyr Leu Ala Thr Ala
290 295 300

Thr Asp Lys Thr Gly Leu Ala Ile Ser Gln Asn Gln Glu Met Ser Phe
305 310 315 320

Thr Ser Asn Thr Thr Thr Ala Asn Gly Gly Ala Ile Tyr Ala Thr Lys
325 330 335

Cys Thr Leu Asp Gly Asn Thr Thr Leu Thr Phe Asp Gln Asn Thr Ala
340 345 350

Thr Ala Gly Cys Gly Gly Ala Ile Tyr Thr Glu Thr Glu Asp Phe Ser
355 360 365

Leu Lys Gly Ser Thr Gly Thr Val Thr Phe Ser Thr Asn Thr Ala Lys
370 375 380

Thr Gly Gly Ala Leu Tyr Ser Lys Gly Asn Ser Ser Leu Thr Gly Asn
385 390 395 400

Thr Asn Leu Leu Phe Ser Gly Asn Lys Ala Thr Gly Pro Ser Asn Ser
405 410 415

Ser Ala Asn Gln Glu Gly Cys Gly Gly Ala Ile Leu Ala Phe Ile Asp
420 425 430

Ser Gly Ser Val Ser Asp Lys Thr Gly Leu Ser Ile Ala Asn Asn Gln
435 440 445

Glu Val Ser Leu Thr Ser Asn Ala Ala Thr Val Ser Gly Gly Ala Ile
450 455 460

Tyr Ala Thr Lys Cys Thr Leu Thr Gly Asn Gly Ser Leu Thr Phe Asp
465 470 475 480

Gly Asn Thr Ala Gly Thr Ser Gly Gly Ala Ile Tyr Thr Glu Thr Glu
485 490 495

Asp Phe Thr Leu Thr Gly Ser Thr Gly Thr Val Thr Phe Ser Thr Asn
500 505 510

Thr Ala Lys Thr Gly Gly Ala Leu Tyr Ser Lys Gly Asn Asn Ser Leu
515 520 525

Ser Gly Asn Thr Asn Leu Leu Phe Ser Gly Asn Lys Ala Thr Gly Pro
530 535 540

Ser Asn Ser Ser Ala Asn Gln Glu Gly Cys Gly Gly Ala Ile Leu Ser
545 550 555 560

```
Phe Leu Glu Ser Ala Ser Val Ser Thr Lys Lys Gly Leu Trp Ile Glu
                565                 570                 575

Asp Asn Glu Asn Val Ser Leu Ser Gly Asn Thr Ala Thr Val Ser Gly
            580                 585                 590

Gly Ala Ile Tyr Ala Thr Lys Cys Ala Leu His Gly Asn Thr Thr Leu
        595                 600                 605

Thr Phe Asp Gly Asn Thr Ala Glu Thr Ala Gly Gly Ala Ile Tyr Thr
    610                 615                 620

Glu Thr Glu Asp Phe Thr Leu Thr Gly Ser Thr Gly Thr Val Thr Phe
625                 630                 635                 640

Ser Thr Asn Thr Ala Lys Thr Ala Gly Ala Leu His Thr Lys Gly Asn
                645                 650                 655

Thr Ser Phe Thr Lys Asn Lys Ala Leu Val Phe Ser Gly Asn Ser Ala
            660                 665                 670

Thr Ala Thr Ala Thr Thr Thr Thr Asp Gln Glu Gly Cys Gly Gly Ala
        675                 680                 685

Ile Leu Cys Asn Ile Ser Glu Ser Asp Ile Ala Thr Lys Ser Leu Thr
    690                 695                 700

Leu Thr Glu Asn Glu Ser Leu Ser Phe Ile Asn Asn Thr Ala Lys Arg
705                 710                 715                 720

Ser Gly Gly Gly Ile Tyr Ala Pro Lys Cys Val Ile Ser Gly Ser Glu
                725                 730                 735

Ser Ile Asn Phe Asp Gly Asn Thr Ala Glu Thr Ser Gly Gly Ala Ile
            740                 745                 750

Tyr Ser Lys Asn Leu Ser Ile Thr Ala Asn Gly Pro Val Ser Phe Thr
        755                 760                 765

Asn Asn Ser Gly Gly Lys Gly Gly Ala Ile Tyr Ile Ala Asp Ser Gly
    770                 775                 780

Glu Leu Ser Leu Glu Ala Ile Asp Gly Asp Ile Thr Phe Ser Gly Asn
785                 790                 795                 800

Arg Ala Thr Glu Gly Thr Ser Thr Pro Asn Ser Ile His Leu Gly Ala
                805                 810                 815

Gly Ala Lys Ile Thr Lys Leu Ala Ala Ala Pro Gly His Thr Ile Tyr
            820                 825                 830

Phe Tyr Asp Pro Ile Thr Met Glu Ala Pro Ala Ser Gly Gly Thr Ile
        835                 840                 845

Glu Glu Leu Val Ile Asn Pro Val Val Lys Ala Ile Val Pro Pro Pro
    850                 855                 860

Gln Pro Lys Asn Gly Pro Ile Ala Ser Val Pro Val Val Pro Val Ala
865                 870                 875                 880
```

```
Pro Ala Asn Pro Asn Thr Gly Thr Ile Val Phe Ser Ser Gly Lys Leu
                885                 890                 895

Pro Ser Gln Asp Ala Ser Ile Pro Ala Asn Thr Thr Thr Ile Leu Asn
            900                 905                 910

Gln Lys Ile Asn Leu Ala Gly Gly Asn Val Val Leu Lys Glu Gly Ala
        915                 920                 925

Thr Leu Gln Val Tyr Ser Phe Thr Gln Gln Pro Asp Ser Thr Val Phe
    930                 935                 940

Met Asp Ala Gly Thr Thr Leu Glu Thr Thr Thr Thr Asn Asn Thr Asp
945                 950                 955                 960

Gly Ser Ile Asp Leu Lys Asn Leu Ser Val Asn Leu Asp Ala Leu Asp
                965                 970                 975

Gly Lys Arg Met Ile Thr Ile Ala Val Asn Ser Thr Ser Gly Gly Leu
            980                 985                 990

Lys Ile Ser Gly Asp Leu Lys Phe His Asn Asn Glu Gly Ser Phe Tyr
        995                 1000                1005

Asp Asn Pro Gly Leu Lys Ala Asn Leu Asn Leu Pro Phe Leu Asp Leu
    1010                1015                1020

Ser Ser Thr Ser Gly Thr Val Asn Leu Asp Asp Phe Asn Pro Ile Pro
1025                1030                1035                1040

Ser Ser Met Ala Ala Pro Asp Tyr Gly Tyr Gln Gly Ser Trp Thr Leu
                1045                1050                1055

Val Pro Lys Val Gly Ala Gly Gly Lys Val Thr Leu Val Ala Glu Trp
            1060                1065                1070

Gln Ala Leu Gly Tyr Thr Pro Lys Pro Glu Leu Arg Ala Thr Leu Val
        1075                1080                1085

Pro Asn Ser Leu Trp Asn Ala Tyr Val Asn Ile His Ser Ile Gln Gln
    1090                1095                1100

Glu Ile Ala Thr Ala Met Ser Asp Ala Pro Ser His Pro Gly Ile Trp
1105                1110                1115                1120

Ile Gly Gly Ile Gly Asn Ala Phe His Gln Asp Lys Gln Lys Glu Asn
                1125                1130                1135

Ala Gly Phe Arg Leu Ile Ser Arg Gly Tyr Ile Val Gly Gly Ser Met
            1140                1145                1150

Thr Thr Pro Gln Glu Tyr Thr Phe Ala Val Ala Phe Ser Gln Leu Phe
        1155                1160                1165

Gly Lys Ser Lys Asp Tyr Val Val Ser Asp Ile Lys Ser Gln Val Tyr
    1170                1175                1180

Ala Gly Ser Leu Cys Ala Gln Ser Ser Tyr Val Ile Pro Leu His Ser
1185                1190                1195                1200

Ser Leu Arg Arg His Val Leu Ser Lys Val Leu Pro Glu Leu Pro Gly
```

1205 1210 1215

Glu Thr Pro Leu Val Leu His Gly Gln Val Ser Tyr Gly Arg Asn His
1220 1225 1230

His Asn Met Thr Thr Lys Leu Ala Asn Asn Thr Gln Gly Lys Ser Asp
1235 1240 1245

Trp Asp Ser His Ser Phe Ala Val Glu Val Gly Gly Ser Leu Pro Val
1250 1255 1260

Asp Leu Asn Tyr Arg Tyr Leu Thr Ser Tyr Ser Pro Tyr Val Lys Leu
1265 1270 1275 1280

Gln Val Val Ser Val Asn Gln Lys Gly Phe Gln Glu Val Ala Ala Asp
1285 1290 1295

Pro Arg Ile Phe Asp Ala Ser His Leu Val Asn Val Ser Ile Pro Met
1300 1305 1310

Gly Leu Thr Phe Lys His Glu Ser Ala Lys Pro Pro Ser Ala Leu Leu
1315 1320 1325

Leu Thr Leu Gly Tyr Ala Val Asp Ala Tyr Arg Asp His Pro His Cys
1330 1335 1340

Leu Thr Ser Leu Thr Asn Gly Thr Ser Trp Ser Thr Phe Ala Thr Asn
1345 1350 1355 1360

Leu Ser Arg Gln Ala Phe Phe Ala Glu Ala Ser Gly His Leu Lys Leu
1365 1370 1375

Leu His Gly Leu Asp Cys Phe Ala Ser Gly Ser Cys Glu Leu Arg Ser
1380 1385 1390

Ser Ser Arg Ser Tyr Asn Ala Asn Cys Gly Thr Arg Tyr Ser Phe
1395 1400 1405

<210> 32
<211> 4224
<212> DNA
<213> Chlamydia pneumoniae

<400> 32
atgaaatatt ctttaccttg gctacttacc tcttcggctt tagttttctc cctacatcca 60
ctaatggctg ctaacacgga tctctcatca tccgataact atgaaaatgg tagtagtggt 120
agcgcagcat tcactgccaa ggaaacttcg gatgcttcag gaactaccta cactctcact 180
agcgatgttt ctattacgaa tgtatctgca attactcctg cagataaaag ctgttttaca 240
aacacaggag gagcattgag ttttgttgga gctgatcact cattggttct gcaaaccata 300
gcgcttacgc atgatggtgc tgcaattaac aataccaaca cagctctttc tttctcagga 360
ttctcgtcac tcttaatcga ctcagctcca gcaacaggaa cttcgggcgg caagggtgct 420
atttgtgtga caaatacaga gggaggtact gcgactttta ctgacaatgc cagtgtcacc 480
ctccaaaaaa atacttcaga aaaagatgga gctgcagttt ctgcctacag catcgatctt 540
gctaagacta cgacagcagc tctcttagat caaaatacta gcacaaaaaa tggcggggcc 600
ctctgtagta cagcaaacac tacagtccaa ggaaactcag gaacggtgac cttctcctca 660
aatactgcta cagataaagg tgggggggatc tactcaaaag aaaaggatag cacgctagat 720
gccaatacag gagtcgttac cttcaaatct aatactgcaa agacgggggg tgcttggagc 780
tctgatgaca atcttgctct taccggcaac actcaagtac tttttcagga aaataaaaca 840
accggctcag cagcacaggc aaataacccg gaaggttgtg gtggggcaat ctgttgttat 900
cttgctacag caacagacaa aactggatta gccatttctc agaatcaaga aatgagcttc 960
actagtaata caacaactgc gaatggtgga gcgatctacg ctactaaatg tactctggat 1020

ggaaacacaa ctcttacctt cgatcagaat actgcgacag caggatgtgg cggagctatc 1080
tatacagaaa ctgaagattt ttctcttaag ggaagtacgg gaaccgtgac cttcagcaca 1140
aatacagcaa agacaggcgg cgccttatat tctaaaggaa acagctcgct gactggaaat 1200
accaacctgc tcttttcagg gaacaaagct acgggcccga gtaattcttc agcaaatcaa 1260
gagggttgcg gtggggcaat cctagccttt attgattcag gatccgtaag cgataaaaca 1320
ggactatcga ttgcaaacaa ccaagaagtc agcctcacta gtaatgctgc aacagtaagt 1380
ggtggtgcga tctatgctac caaatgtact ctaactggaa acggctccct gacctttgac 1440
ggcaatactg ctggaacttc aggaggggcg atctatacag aaactgaaga ttttactctt 1500
acaggaagta caggaaccgt gaccttcagc acaaatacag caaagacagg cggcgcctta 1560
tattctaaag gcaacaactc tctgtctggt aataccaacc tgctcttttc agggaacaaa 1620
gctacgggcc cgagtaattc ttcagcaaat caagagggtt gcggtggggc aatcctatcg 1680
tttcttgagt cagcatctgt aagtactaaa aaaggactct ggattgaaga taacgaaaac 1740
gtgagtctct ctggtaatac tgcaacagta agtggcggtg cgatctatgc gaccaagtgt 1800
gctctgcatg gaaacacgac tcttaccttt gatggcaata ctgccgaaac tgcaggagga 1860
gcgatctata cagaaaccga agattttact cttacgggaa gtacgggaac cgtgaccttc 1920
agcacaaata cagcaaagac agcaggggct ctacatacta aaggaaatac ttcctttacc 1980
aaaaataagg ctcttgtatt ttctggaaat tcagcaacag caacagcaac aacaactaca 2040
gatcaagaag gttgtggtgg agcgatcctc tgtaatatct cagagtctga catagctaca 2100
aaaagcttaa ctcttactga aaatgagagt ttaagtttca ttaacaatac ggcaaaaaga 2160
agtggtggtg gtatttatgc tcctaagtgt gtaatctcag gcagtgaatc cataaacttt 2220
gatggcaata ctgctgaaac ttcgggagga gcgatttatt cgaaaaacct ttcgattaca 2280
gctaacggtc ctgtctcctt taccaataat tctggaggca agggaggcgc catttatata 2340
gccgatagcg gagaactttc cttagaggct attgatgggg atattacttt ctcagggaac 2400
cgagcgactg agggaacttc aactcccaac tcgatccatt taggtgcagg ggctaagatc 2460
actaagcttg cagcagctcc tggtcatacg atttattttt atgatcctat tacgatggaa 2520
gctcctgcat ctggaggaac aatagaggag ttagtcatca atcctgttgt caaagctatt 2580
gttcctcctc cccaaccaaa aaatggtcct atagcttcag tgcctgtagt ccctgtagca 2640
cctgcaaacc caaacacggg aactatagta ttttcttctg gaaaactccc cagtcaagat 2700
gcctcgattc ctgcaaatac taccaccata ctgaaccaga agatcaactt agcaggagga 2760
aatgtcgttt taaaagaagg agccacccta caagtatatt ccttcacaca gcagcctgat 2820
tctacagtat tcatggatgc aggaacgacc ttagagacca cgacaactaa caatacagat 2880
ggcagcatcg atctaaagaa tctctctgta aatctggatg ctttagatgg caagcgtatg 2940
ataacgattg ccgtaaacag cacaagtggg ggattaaaaa tctcagggga tctgaaattc 3000
cataacaatg aaggaagttt ctatgacaat cctgggttga aagcaaactt aaatcttcct 3060
ttcttagatc tttcttctac ttcaggaact gtaaatttag acgacttcaa tccgattcct 3120
tctagcatgg ctgctccgga ttatgggtat caagggagtt ggactctggt tcctaaagta 3180
ggagctggag ggaaggtgac tttggtcgcg gaatggcaag cgttaggata cactcctaaa 3240
ccagagcttc gtgcgacttt agttcctaat agcctttgga atgcttatgt aaacatccat 3300
tctatacagc aggagatcgc cactgcgatg tcggacgctc cctcacatcc agggatttgg 3360
attggaggta ttggcaacgc cttccatcaa gacaagcaaa aggaaaatgc aggattccgt 3420
ttgatttcca gaggttatat tgttggtggc agcatgacca cccctcaaga atataccttt 3480
gctgttgcat tcagccaact ctttggcaaa tctaaggatt acgtagtctc ggatattaaa 3540
tctcaagtct atgcaggatc tctctgtgct cagagctctt atgtcattcc cctgcatagc 3600
tcattacgtc gccacgtcct ctctaaggtc cttccagagc tcccaggaga aactcccctt 3660
gttctccatg gtcaagtttc ctatggaaga aaccaccata atatgacgac aaagcttgcg 3720
aacaacacac aagggaaatc agactgggac agccatagct tcgctgttga agtcggtggt 3780
tctcttcctg tagatctaaa ctacagatac cttaccagct actctcccta tgtgaaactc 3840
caagttgtga gtgtaaatca aaaaggattc caagaggttg ctgctgatcc acgtatcttt 3900
gacgctagcc atctggtcaa cgtgtctatc cctatgggac tcaccttcaa acacgaatca 3960
gcaaagcccc ccagtgcttt gcttcttact ttaggttacg ctgtagatgc ttaccgggat 4020
caccctcact gcctgacctc cttaacaaat ggcacctcgt ggtctacgtt tgctacaaac 4080
ttatcacgac aagctttctt tgctgaggct tctggacatc tgaagttact tcatggtctt 4140
gactgcttcg cttctggaag ttgtgaactg cgcagctcct caagaagcta taatgcaaac 4200
tgtggaactc gttattcttt ctaa 4224

<210> 33
<211> 928
<212> PRT
<213> Chlamydia pneumoniae

<400> 33

```
Met Lys Ser Ser Leu His Trp Phe Leu Ile Ser Ser Ser Leu Ala Leu
1               5                   10                  15

Pro Leu Ser Leu Asn Phe Ser Ala Phe Ala Ala Val Val Glu Ile Asn
            20                  25                  30

Leu Gly Pro Thr Asn Ser Phe Ser Gly Pro Gly Thr Tyr Thr Pro Pro
        35                  40                  45

Ala Gln Thr Thr Asn Ala Asp Gly Thr Ile Tyr Asn Leu Thr Gly Asp
    50                  55                  60

Val Ser Ile Thr Asn Ala Gly Ser Pro Thr Ala Leu Thr Ala Ser Cys
65                  70                  75                  80

Phe Lys Glu Thr Thr Gly Asn Leu Ser Phe Gln Gly His Gly Tyr Gln
                85                  90                  95

Phe Leu Leu Gln Asn Ile Asp Ala Gly Ala Asn Cys Thr Phe Thr Asn
            100                 105                 110

Thr Ala Ala Asn Lys Leu Leu Ser Phe Ser Gly Phe Ser Tyr Leu Ser
        115                 120                 125

Leu Ile Gln Thr Thr Asn Ala Thr Thr Gly Thr Gly Ala Ile Lys Ser
    130                 135                 140

Thr Gly Ala Cys Ser Ile Gln Ser Asn Tyr Ser Cys Tyr Phe Gly Gln
145                 150                 155                 160

Asn Phe Ser Asn Asp Asn Gly Gly Ala Leu Gln Gly Ser Ser Ile Ser
                165                 170                 175

Leu Ser Leu Asn Pro Asn Leu Thr Phe Ala Lys Asn Lys Ala Thr Gln
            180                 185                 190

Lys Gly Gly Ala Leu Tyr Ser Thr Gly Gly Ile Thr Ile Asn Asn Thr
        195                 200                 205

Leu Asn Ser Ala Ser Phe Ser Glu Asn Thr Ala Ala Asn Asn Gly Gly
    210                 215                 220

Ala Ile Tyr Thr Glu Ala Ser Ser Phe Ile Ser Ser Asn Lys Ala Ile
225                 230                 235                 240

Ser Phe Ile Asn Asn Ser Val Thr Ala Thr Ser Ala Thr Gly Gly Ala
                245                 250                 255

Ile Tyr Cys Ser Ser Thr Ser Ala Pro Lys Pro Val Leu Thr Leu Ser
            260                 265                 270

Asp Asn Gly Glu Leu Asn Phe Ile Gly Asn Thr Ala Ile Thr Ser Gly
        275                 280                 285

Gly Ala Ile Tyr Thr Asp Asn Leu Val Leu Ser Ser Gly Gly Pro Thr
    290                 295                 300

Leu Phe Lys Asn Asn Ser Ala Ile Asp Thr Ala Ala Pro Leu Gly Gly
305                 310                 315                 320

Ala Ile Ala Ile Ala Asp Ser Gly Ser Leu Ser Leu Ser Ala Leu Gly
```

325 330 335

Gly Asp Ile Thr Phe Glu Gly Asn Thr Val Val Lys Gly Ala Ser Ser
340 345 350

Ser Gln Thr Thr Thr Arg Asn Ser Ile Asn Ile Gly Asn Thr Asn Ala
355 360 365

Lys Ile Val Gln Leu Arg Ala Ser Gln Gly Asn Thr Ile Tyr Phe Tyr
370 375 380

Asp Pro Ile Thr Thr Ser Ile Thr Ala Ala Leu Ser Asp Ala Leu Asn
385 390 395 400

Leu Asn Gly Pro Asp Leu Ala Gly Asn Pro Ala Tyr Gln Gly Thr Ile
405 410 415

Val Phe Ser Gly Glu Lys Leu Ser Glu Ala Glu Ala Ala Glu Ala Asp
420 425 430

Asn Leu Lys Ser Thr Ile Gln Gln Pro Leu Thr Leu Ala Gly Gly Gln
435 440 445

Leu Ser Leu Lys Ser Gly Val Thr Leu Val Ala Lys Ser Phe Ser Gln
450 455 460

Ser Pro Gly Ser Thr Leu Leu Met Asp Ala Gly Thr Thr Leu Glu Thr
465 470 475 480

Ala Asp Gly Ile Thr Ile Asn Asn Leu Val Leu Asn Val Asp Ser Leu
485 490 495

Lys Glu Thr Lys Lys Ala Thr Leu Lys Ala Thr Gln Ala Ser Gln Thr
500 505 510

Val Thr Leu Ser Gly Ser Leu Ser Leu Val Asp Pro Ser Gly Asn Val
515 520 525

Tyr Glu Asp Val Ser Trp Asn Asn Pro Gln Val Phe Ser Cys Leu Thr
530 535 540

Leu Thr Ala Asp Asp Pro Ala Asn Ile His Ile Thr Asp Leu Ala Ala
545 550 555 560

Asp Pro Leu Glu Lys Asn Pro Ile His Trp Gly Tyr Gln Gly Asn Trp
565 570 575

Ala Leu Ser Trp Gln Glu Asp Thr Ala Thr Lys Ser Lys Ala Ala Thr
580 585 590

Leu Thr Trp Thr Lys Thr Gly Tyr Asn Pro Asn Pro Glu Arg Arg Gly
595 600 605

Thr Leu Val Ala Asn Thr Leu Trp Gly Ser Phe Val Asp Val Arg Ser
610 615 620

Ile Gln Gln Leu Val Ala Thr Lys Val Arg Gln Ser Gln Glu Thr Arg
625 630 635 640

Gly Ile Trp Cys Glu Gly Ile Ser Asn Phe Phe His Lys Asp Ser Thr
645 650 655

```
Lys Ile Asn Lys Gly Phe Arg His Ile Ser Ala Gly Tyr Val Val Gly
            660                 665                 670

Ala Thr Thr Thr Leu Ala Ser Asp Asn Leu Ile Thr Ala Ala Phe Cys
        675                 680                 685

Gln Leu Phe Gly Lys Asp Arg Asp His Phe Ile Asn Lys Asn Arg Ala
    690                 695                 700

Ser Ala Tyr Ala Ala Ser Leu His Leu Gln His Leu Ala Thr Leu Ser
705                 710                 715                 720

Ser Pro Ser Leu Leu Arg Tyr Leu Pro Gly Ser Glu Ser Glu Gln Pro
                725                 730                 735

Val Leu Phe Asp Ala Gln Ile Ser Tyr Ile Tyr Ser Lys Asn Thr Met
            740                 745                 750

Lys Thr Tyr Tyr Thr Gln Ala Pro Lys Gly Glu Ser Ser Trp Tyr Asn
        755                 760                 765

Asp Gly Cys Ala Leu Glu Leu Ala Ser Ser Leu Pro His Thr Ala Leu
    770                 775                 780

Ser His Glu Gly Leu Phe His Ala Tyr Phe Pro Phe Ile Lys Val Glu
785                 790                 795                 800

Ala Ser Tyr Ile His Gln Asp Ser Phe Lys Glu Arg Asn Thr Thr Leu
                805                 810                 815

Val Arg Ser Phe Asp Ser Gly Asp Leu Ile Asn Val Ser Val Pro Ile
            820                 825                 830

Gly Ile Thr Phe Glu Arg Phe Ser Arg Asn Glu Arg Ala Ser Tyr Glu
        835                 840                 845

Ala Thr Val Ile Tyr Val Ala Asp Val Tyr Arg Lys Asn Pro Asp Cys
    850                 855                 860

Thr Thr Ala Leu Leu Ile Asn Asn Thr Ser Trp Lys Thr Thr Gly Thr
865                 870                 875                 880

Asn Leu Ser Arg Gln Ala Gly Ile Gly Arg Ala Gly Ile Phe Tyr Ala
                885                 890                 895

Phe Ser Pro Asn Leu Glu Val Thr Ser Asn Leu Ser Met Glu Ile Arg
            900                 905                 910

Gly Ser Ser Arg Ser Tyr Asn Ala Asp Leu Gly Gly Lys Phe Gln Phe
        915                 920                 925


<210>   34
<211>   2787
<212>   DNA
<213>   Chlamydia pneumoniae

<400>   34
atgaaatcct ctcttcattg gtttttaatc tcgtcatctt tagcacttcc cttgtcacta    60
aatttctctg cgtttgctgc tgttgttgaa atcaatctag gacctaccaa tagcttctct   120
```

```
ggaccaggaa cctacactcc tccagcccaa acaacaaatg cagatggaac tatctataat    180
ctaacagggg atgtctcaat caccaatgca ggatctccga cagctctaac cgcttcctgc    240
tttaaagaaa ctactgggaa tctttctttc caaggccacg gctaccaatt tctcctacaa    300
aatatcgatg cgggagcgaa ctgtaccttt accaatacag ctgcaaataa gcttctctcc    360
ttttcaggat tctcctattt gtcactaata caaaccacga atgctaccac aggaacagga    420
gccatcaagt ccacaggagc ttgttctatt cagtcgaact atagttgcta ctttggccaa    480
aacttttcta atgacaatgg aggcgccctc caaggcagct ctatcagtct atcgctaaac    540
cccaacctaa cgtttgccaa aaacaaagca acgcaaaaag gggtgccct ctattccacg    600
ggagggatta caattaacaa tacgttaaac tcagcatcat tttctgaaaa taccgcggcg    660
aacaatggcg gagccattta cacggaagct agcagtttta ttagcagcaa caaagcaatt    720
agctttataa acaatagtgt gaccgcaacc tcagctacag gggagccat ttactgtagt    780
agtacatcag cccccaaacc agtcttaact ctatcagaca acggggaact gaactttata    840
ggaaatacag caattactag tggtggggcg atttatactg acaatctagt tctttcttct    900
ggaggaccta cgctttttaa aaacaactct gctatagata ctgcagctcc cttaggagga    960
gcaattgcga ttgctgactc tggatctttg agtctttcgg ctcttggtgg agacatcact    1020
tttgaaggaa acacagtagt caaaggagct tcttcgagtc agaccactac cagaaattct    1080
attaacatcg gaaacaccaa tgctaagatt gtacagctgc gagcctctca aggcaatact    1140
atctacttct atgatcctat aacaactagc atcactgcag ctctctcaga tgctctaaac    1200
ttaaatggtc ctgaccttgc agggaatcct gcatatcaag gaaccatcgt attttctgga    1260
gagaagctct cggaagcaga agctgcagaa gctgataatc tcaaatctac aattcagcaa    1320
cctctaactc ttgcgggagg gcaactctct cttaaatcag gagtcactct agttgctaag    1380
tccttttcgc aatctccggg ctctaccctc ctcatggatg cagggaccac attagaaacc    1440
gctgatggga tcactatcaa taatcttgtt ctcaatgtag attccttaaa agagaccaag    1500
aaggctacgc taaaagcaac acaagcaagt cagacagtca ctttatctgg atcgctctct    1560
cttgtagatc cttctggaaa tgtctacgaa gatgtctctt ggaataaccc tcaagtcttt    1620
tcttgtctca ctcttactgc tgacgacccc gcgaatattc acatcacaga cttagctgct    1680
gatcccctag aaaaaaatcc tatccattgg ggataccaag ggaattgggc attatcttgg    1740
caagaggata ctgcgactaa atccaaagca gcgactctta cctggacaaa aacaggatac    1800
aatccgaatc ctgagcgtcg tggaacctta gttgctaaca cgctatgggg atcctttgtt    1860
gatgtgcgct ccatacaaca gcttgtagcc actaaagtac gccaatctca agaaactcgc    1920
ggcatctggt gtgaagggat ctcgaacttc ttccataaag atagcacgaa gataaataaa    1980
ggttttcgcc acataagtgc aggttatgtt gtaggagcga ctacaacatt agcttctgat    2040
aatcttatca ctgcagcctt ctgccaatta ttcgggaaag atagagatca ctttataaat    2100
aaaaatagag cttctgccta tgcagcttct ctccatctcc agcatctagc gaccttgtct    2160
tctccaagct tgttacgcta ccttcctgga tctgaaagtg agcagcctgt cctctttgat    2220
gctcagatca gctatatcta tagtaaaaat actatgaaaa cctattacac ccaagcacca    2280
aagggagaga gctcgtggta taatgacggt tgcgctctgg aacttgcgag ctccctacca    2340
cacactgctt taagccatga gggtctcttc cacgcgtatt ttcctttcat caaagtagaa    2400
gcttcgtaca tacaccaaga tagcttcaaa gaacgtaata ctaccttggt acgatctttc    2460
gatagcggtg atttaattaa cgtctctgtg cctattggaa ttaccttcga gagattctcg    2520
agaaacgagc gtgcgtctta cgaagctact gtcatctacg ttgccgatgt ctatcgtaag    2580
aatcctgact gcacgacagc tctcctaatc aacaatacct cgtggaaaac tacaggaacg    2640
aatctctcaa gacaagctgg tatcggaaga gcagggatct tttatgcctt ctctccaaat    2700
cttgaggtca caagtaacct atctatggaa attcgtggat cttcacgcag ctacaatgca    2760
gatcttggag gtaagttcca gttctaa                                        2787
```

<210> 35
<211> 978
<212> PRT
<213> Chlamydia pneumoniae

<400> 35

```
Met Pro Leu Ser Phe Lys Ser Ser Ser Phe Cys Leu Leu Ala Cys Leu
1               5                   10                  15

Cys Ser Ala Ser Cys Ala Phe Ala Glu Thr Arg Leu Gly Gly Asn Phe
            20                  25                  30

Val Pro Pro Ile Thr Asn Gln Gly Glu Glu Ile Leu Leu Thr Ser Asp
        35                  40                  45
```

```
Phe Val Cys Ser Asn Phe Leu Gly Ala Ser Phe Ser Ser Ser Phe Ile
    50                  55                  60

Asn Ser Ser Ser Asn Leu Ser Leu Leu Gly Lys Gly Leu Ser Leu Thr
65                  70                  75                  80

Phe Thr Ser Cys Gln Ala Pro Thr Asn Ser Asn Tyr Ala Leu Leu Ser
                85                  90                  95

Ala Ala Glu Thr Leu Thr Phe Lys Asn Phe Ser Ser Ile Asn Phe Thr
            100                 105                 110

Gly Asn Gln Ser Thr Gly Leu Gly Gly Leu Ile Tyr Gly Lys Asp Ile
        115                 120                 125

Val Phe Gln Ser Ile Lys Asp Leu Ile Phe Thr Thr Asn Arg Val Ala
    130                 135                 140

Tyr Ser Pro Ala Ser Val Thr Thr Ser Ala Thr Pro Ala Ile Thr Thr
145                 150                 155                 160

Val Thr Thr Gly Ala Ser Ala Leu Gln Pro Thr Asp Ser Leu Thr Val
                165                 170                 175

Glu Asn Ile Ser Gln Ser Ile Lys Phe Phe Gly Asn Leu Ala Asn Phe
            180                 185                 190

Gly Ser Ala Ile Ser Ser Ser Pro Thr Ala Val Val Lys Phe Ile Asn
        195                 200                 205

Asn Thr Ala Thr Met Ser Phe Ser His Asn Phe Thr Ser Ser Gly Gly
    210                 215                 220

Gly Val Ile Tyr Gly Gly Ser Ser Leu Leu Phe Glu Asn Asn Ser Gly
225                 230                 235                 240

Cys Ile Ile Phe Thr Ala Asn Ser Cys Val Asn Ser Leu Lys Gly Val
                245                 250                 255

Thr Pro Ser Ser Gly Thr Tyr Ala Leu Gly Ser Gly Gly Ala Ile Cys
            260                 265                 270

Ile Pro Thr Gly Thr Phe Glu Leu Lys Asn Asn Gln Gly Lys Cys Thr
        275                 280                 285

Phe Ser Tyr Asn Gly Thr Pro Asn Asp Ala Gly Ala Ile Tyr Ala Glu
    290                 295                 300

Thr Cys Asn Ile Val Gly Asn Gln Gly Ala Leu Leu Leu Asp Ser Asn
305                 310                 315                 320

Thr Ala Ala Arg Asn Gly Gly Ala Ile Cys Ala Lys Val Leu Asn Ile
                325                 330                 335

Gln Gly Arg Gly Pro Ile Glu Phe Ser Arg Asn Arg Ala Glu Lys Gly
            340                 345                 350

Gly Ala Ile Phe Ile Gly Pro Ser Val Gly Asp Pro Ala Lys Gln Thr
        355                 360                 365

Ser Thr Leu Thr Ile Leu Ala Ser Glu Gly Asp Ile Ala Phe Gln Gly
```

```
    370                 375                 380

Asn Met Leu Asn Thr Lys Pro Gly Ile Arg Asn Ala Ile Thr Val Glu
385                 390                 395                 400

Ala Gly Gly Glu Ile Val Ser Leu Ser Ala Gln Gly Gly Ser Arg Leu
                405                 410                 415

Val Phe Tyr Asp Pro Ile Thr His Ser Leu Pro Thr Thr Ser Pro Ser
            420                 425                 430

Asn Lys Asp Ile Thr Ile Asn Ala Asn Gly Ala Ser Gly Ser Val Val
        435                 440                 445

Phe Thr Ser Lys Gly Leu Ser Ser Thr Glu Leu Leu Leu Pro Ala Asn
    450                 455                 460

Thr Thr Thr Ile Leu Leu Gly Thr Val Lys Ile Ala Ser Gly Glu Leu
465                 470                 475                 480

Lys Ile Thr Asp Asn Ala Val Val Asn Val Leu Gly Phe Ala Thr Gln
                485                 490                 495

Gly Ser Gly Gln Leu Thr Leu Gly Ser Gly Gly Thr Leu Gly Leu Ala
            500                 505                 510

Thr Pro Thr Gly Ala Pro Ala Ala Val Asp Phe Thr Ile Gly Lys Leu
        515                 520                 525

Ala Phe Asp Pro Phe Ser Phe Leu Lys Arg Asp Phe Val Ser Ala Ser
    530                 535                 540

Val Asn Ala Gly Thr Lys Asn Val Thr Leu Thr Gly Ala Leu Val Leu
545                 550                 555                 560

Asp Glu His Asp Val Thr Asp Leu Tyr Asp Met Val Ser Leu Gln Thr
                565                 570                 575

Pro Val Ala Ile Pro Ile Ala Val Phe Lys Gly Ala Thr Val Thr Lys
            580                 585                 590

Thr Gly Phe Pro Asp Gly Glu Ile Ala Thr Pro Ser His Tyr Gly Tyr
        595                 600                 605

Gln Gly Lys Trp Ser Tyr Thr Trp Ser Arg Pro Leu Leu Ile Pro Ala
    610                 615                 620

Pro Asp Gly Gly Phe Pro Gly Gly Pro Ser Pro Ser Ala Asn Thr Leu
625                 630                 635                 640

Tyr Ala Val Trp Asn Ser Asp Thr Leu Val Arg Ser Thr Tyr Ile Leu
                645                 650                 655

Asp Pro Glu Arg Tyr Gly Glu Ile Val Ser Asn Ser Leu Trp Ile Ser
            660                 665                 670

Phe Leu Gly Asn Gln Ala Phe Ser Asp Ile Leu Gln Asp Val Leu Leu
        675                 680                 685

Ile Asp His Pro Gly Leu Ser Ile Thr Ala Lys Ala Leu Gly Ala Tyr
    690                 695                 700
```

```
Val Glu His Thr Pro Arg Gln Gly His Glu Gly Phe Ser Gly Arg Tyr
705                 710                 715                 720

Gly Gly Tyr Gln Ala Ala Leu Ser Met Asn Tyr Thr Asp His Thr Thr
                725                 730                 735

Leu Gly Leu Ser Phe Gly Gln Leu Tyr Gly Lys Thr Asn Ala Asn Pro
            740                 745                 750

Tyr Asp Ser Arg Cys Ser Glu Gln Met Tyr Leu Leu Ser Phe Phe Gly
        755                 760                 765

Gln Phe Pro Ile Val Thr Gln Lys Ser Glu Ala Leu Ile Ser Trp Lys
    770                 775                 780

Ala Ala Tyr Gly Tyr Ser Lys Asn His Leu Asn Thr Thr Tyr Leu Arg
785                 790                 795                 800

Pro Asp Lys Ala Pro Lys Ser Gln Gly Gln Trp His Asn Asn Ser Tyr
                805                 810                 815

Tyr Val Leu Ile Ser Ala Glu His Pro Phe Leu Asn Trp Cys Leu Leu
            820                 825                 830

Thr Arg Pro Leu Ala Gln Ala Trp Asp Leu Ser Gly Phe Ile Ser Ala
        835                 840                 845

Glu Phe Leu Gly Gly Trp Gln Ser Lys Phe Thr Glu Thr Gly Asp Leu
    850                 855                 860

Gln Arg Ser Phe Ser Arg Gly Lys Gly Tyr Asn Val Ser Leu Pro Ile
865                 870                 875                 880

Gly Cys Ser Ser Gln Trp Phe Thr Pro Phe Lys Lys Ala Pro Ser Thr
                885                 890                 895

Leu Thr Ile Lys Leu Ala Tyr Lys Pro Asp Ile Tyr Arg Val Asn Pro
            900                 905                 910

His Asn Ile Val Thr Val Val Ser Asn Gln Glu Ser Thr Ser Ile Ser
        915                 920                 925

Gly Ala Asn Leu Arg Arg His Gly Leu Phe Val Gln Ile His Asp Val
    930                 935                 940

Val Asp Leu Thr Glu Asp Thr Gln Ala Phe Leu Asn Tyr Thr Phe Asp
945                 950                 955                 960

Gly Lys Asn Gly Phe Thr Asn His Arg Val Ser Thr Gly Leu Lys Ser
                965                 970                 975

Thr Phe
```

<210> 36
<211> 2937
<212> DNA
<213> Chlamydia pneumoniae

<400> 36

```
atgcctcttt ctttcaaatc ttcatctttt tgtctacttg cctgtttatg tagtgcaagt    60
tgcgcgtttg ctgagactag actcggaggg aactttgttc ctccaattac gaatcagggt   120
gaagagatct tactcacttc agattttgtt tgttcaaact tcttgggggc gagtttttca   180
agttccttta tcaatagttc cagcaatctc tccttattag ggaagggcct ttccttaacg   240
tttacctctt gtcaagctcc tacaaatagt aactatgcgc tactttctgc cgcagagact   300
ctgaccttca agaatttttc ttctataaac tttacaggga accaatcgac aggacttggc   360
ggcctcatct acggaaaaga tattgttttc caatctatca aagatttgat cttcactacg   420
aaccgtgttg cctattctcc agcatctgta actacgtcgg caactcccgc aatcactaca   480
gtaactacag gagcctctgc tctccaacct acagactcac tcactgtcga aaacatatcc   540
caatcgatca agttttttgg gaaccttgcc aacttcggct ctgcaattag cagttctccc   600
acggcagtcg ttaaattcat caataacacc gctaccatga gcttctccca taactttact   660
tcgtcaggag gcggcgtgat ttatggagga agctctctcc tttttgaaaa caattctgga   720
tgcatcatct tcaccgccaa ctcctgtgtg aacagcttaa aggcgtcac ccccttcatca   780
ggaacctatg ctttaggaag tggcggagcc atctgcatcc ctacgggaac tttcgaatta   840
aaaaacaatc aggggaagtg caccttctct tataatggta caccaaatga tgcgggtgcg   900
atctacgccg aaacctgcaa catcgtaggg aaccagggtg ccttgctcct agatagcaac   960
actgcagcga gaaatggcgg agccatctgt gctaaagtgc tcaatattca aggacgcggt  1020
cctattgaat tctctagaaa ccgcgcggag aagggtggag ctattttcat aggcccctct  1080
gttggagacc ctgcgaagca aacatcgaca cttacgattt tggcttccga aggtgatatt  1140
gcgttccaag gaaacatgct caatacaaaa cctggaatcc gcaatgccat cactgtagaa  1200
gcagggggag agattgtgtc tctatctgca caaggaggct cacgtcttgt attttatgat  1260
cccattacac atagcctccc aaccacaagt ccgtctaata aagacattac aatcaacgct  1320
aatggcgctt caggatctgt agtctttaca agtaagggac tctcctctac agaactcctg  1380
ttgcctgcca acacgacaac tatacttcta ggaacagtca agatcgctag tggagaactg  1440
aagattactg acaatgcggt tgtcaatgtt cttggcttcg ctactcaggg ctcaggtcag  1500
cttaccctgg gctctggagg aaccttaggg ctggcaacac ccacgggagc acctgccgct  1560
gtagacttta cgattggaaa gttagcattc gatccttttt ccttcctaaa aagagatttt  1620
gtttcagcat cagtaaatgc aggcacaaaa aacgtcactt taacaggagc tctggttctt  1680
gatgaacatg acgttacaga tctttatgat atggtgtcat tacaaactcc agtagcaatt  1740
cctatcgctg ttttcaaagg agcaaccgtt actaagacag gatttcctga tggggagatt  1800
gcgactccaa gccactacgg ctaccaagga aagtggtcct acacatggtc ccgtcccctg  1860
ttaattccag ctcctgatgg aggatttcct ggaggtccct ctcctagcgc aaatactctc  1920
tatgctgtat ggaattcaga cactctcgtg cgttctacct atatcttaga tcccgagcgt  1980
tacggagaaa ttgtcagcaa cagcttatgg atttccttct taggaaatca ggcattctct  2040
gatattctcc aagatgttct tttgatagat catcccgggt tgtccataac cgcgaaagct  2100
ttaggagcct atgtcgaaca cacaccaaga caaggacatg agggcttttc aggtcgctat  2160
ggaggctacc aagctgcgct atctatgaac tacacggacc acactacgtt aggactttct  2220
ttcgggcagc tttatggaaa aactaacgcc aacccctacg attcacgttg ctcagaacaa  2280
atgtatttac tctcgttctt tggtcaattc cctatcgtga ctcaaaagag cgaggcctta  2340
atttcctgga aagcagctta tggttattcc aaaaatcacc taaataccac ctacctcaga  2400
cctgacaaag ctccaaaatc tcaagggcaa tggcataaca atagttacta tgttcttatt  2460
tctgcagaac atcctttcct aaactggtgt cttcttacaa gacctctggc tcaagcttgg  2520
gatctttcag gttttatttc cgcagaattc ctaggtggtt ggcaaagtaa gttcacagaa  2580
actggagatc tgcaacgtag ctttagtaga ggtaaagggt acaatgtttc cctaccgata  2640
ggatgttctt ctcaatggtt cacaccattt aagaaggctc cttctacact gaccatcaaa  2700
cttgcctaca agcctgatat ctatcgtgtc aaccctcaca atattgtgac tgtcgtctca  2760
aaccaagaga gcacttcgat ctcaggagca aatctacgcc gccacggttt gtttgtacaa  2820
atccatgatg tagtagatct caccgaggac actcaggcct ttctaaacta tacctttgac  2880
gggaaaaatg gatttacaaa ccaccgagtg tctacaggac taaaatccac attttaa     2937
```

<210> 37
<211> 192
<212> PRT
<213> Chlamydia pneumoniae

<400> 37

```
Met Asn Ile His Ser Leu Trp Lys Leu Cys Thr Leu Leu Ala Leu Leu
1               5                   10                  15

Ala Leu Pro Ala Cys Ser Leu Ser Pro Asn Tyr Gly Trp Glu Asp Ser
            20                  25                  30
```

Cys Asn Thr Cys His His Thr Arg Arg Lys Lys Pro Ser Ser Phe Gly
35 40 45

Phe Val Pro Leu Tyr Thr Glu Glu Asp Phe Asn Pro Asn Phe Thr Phe
50 55 60

Gly Glu Tyr Asp Ser Lys Glu Glu Lys Gln Tyr Lys Ser Ser Gln Val
65 70 75 80

Ala Ala Phe Arg Asn Ile Thr Phe Ala Thr Asp Ser Tyr Thr Ile Lys
85 90 95

Gly Glu Glu Asn Leu Ala Ile Leu Thr Asn Leu Val His Tyr Met Lys
100 105 110

Lys Asn Pro Lys Ala Thr Leu Tyr Ile Glu Gly His Thr Asp Glu Arg
115 120 125

Gly Ala Ala Ser Tyr Asn Leu Ala Leu Gly Ala Arg Arg Ala Asn Ala
130 135 140

Ile Lys Glu His Leu Arg Lys Gln Gly Ile Ser Ala Asp Arg Leu Ser
145 150 155 160

Thr Ile Ser Tyr Gly Lys Glu His Pro Leu Asn Ser Gly His Asn Glu
165 170 175

Leu Ala Trp Gln Gln Asn Arg Arg Thr Glu Phe Lys Ile His Ala Arg
180 185 190

<210> 38
<211> 579
<212> DNA
<213> Chlamydia pneumoniae

<400> 38
atgaatatac attccctatg gaaactttgt actttattgg ctttacttgc attgccagca 60
tgtagccttt cccctaatta tggctgggag gattcctgta atacatgcca tcatacaaga 120
cgaaaaaagc cttcttcttt tggctttgtt cctctctata ccgaagagga ctttaaccct 180
aattttacct tcggtgagta tgattccaaa gaagaaaaac aatacaagtc aagccaagtt 240
gcagcatttc gtaatatcac ctttgctaca gacagctata caattaaagg tgaagagaac 300
cttgcgattc tcacgaactt ggttcactac atgaagaaaa acccgaaagc tacactgtac 360
attgaagggc atactgacga gcgtggagct gcatcctata accttgcttt aggagcacga 420
cgagccaatg cgattaaaga gcatctccga aagcagggaa tctctgcaga tcgtctatct 480
actatttcct acggaaaaga acatccttta aattcgggac acaacgaact agcatggcaa 540
caaaatcgcc gtacagagtt taagattcat gcacgctaa 579

<210> 39
<211> 431
<212> PRT
<213> Chlamydia pneumoniae

<400> 39
Met Leu Arg Gln Leu Cys Phe Gln Val Phe Phe Phe Cys Phe Ala Ser
1 5 10 15

Leu Val Tyr Ala Glu Glu Leu Glu Val Val Val Arg Ser Glu His Ile
20 25 30

Thr Leu Pro Ile Glu Val Ser Cys Gln Thr Asp Thr Lys Asp Pro Lys
35 40 45

Ile Gln Lys Tyr Leu Ser Ser Leu Thr Glu Ile Phe Cys Lys Asp Ile
50 55 60

Ala Leu Gly Asp Cys Leu Gln Pro Thr Ala Ala Ser Lys Glu Ser Ser
65 70 75 80

Ser Pro Leu Ala Ile Ser Leu Arg Leu His Val Pro Gln Leu Ser Val
85 90 95

Val Leu Leu Gln Ser Ser Lys Thr Pro Gln Thr Leu Cys Ser Phe Thr
100 105 110

Ile Ser Gln Asn Leu Ser Val Asp Arg Gln Lys Ile His His Ala Ala
115 120 125

Asp Thr Val His Tyr Ala Leu Thr Gly Ile Pro Gly Ile Ser Ala Gly
130 135 140

Lys Ile Val Phe Ala Leu Ser Ser Leu Gly Lys Asp Gln Lys Leu Lys
145 150 155 160

Gln Gly Glu Leu Trp Thr Thr Asp Tyr Asp Gly Lys Asn Leu Ala Pro
165 170 175

Leu Thr Thr Glu Cys Ser Leu Ser Ile Thr Pro Lys Trp Val Gly Val
180 185 190

Gly Ser Asn Phe Pro Tyr Leu Tyr Val Ser Tyr Lys Tyr Gly Val Pro
195 200 205

Lys Ile Phe Leu Gly Ser Leu Glu Asn Thr Glu Gly Lys Lys Val Leu
210 215 220

Pro Leu Lys Gly Asn Gln Leu Met Pro Thr Phe Ser Pro Arg Lys Lys
225 230 235 240

Leu Leu Ala Phe Val Ala Asp Thr Tyr Gly Asn Pro Asp Leu Phe Ile
245 250 255

Gln Pro Phe Ser Leu Thr Ser Gly Pro Met Gly Arg Pro Arg Arg Leu
260 265 270

Leu Asn Glu Asn Phe Gly Thr Gln Gly Asn Pro Ser Phe Asn Pro Glu
275 280 285

Gly Ser Gln Leu Val Phe Ile Ser Asn Lys Asp Gly Arg Pro Arg Leu
290 295 300

Tyr Ile Met Ser Leu Asp Pro Glu Pro Gln Ala Pro Arg Leu Leu Thr
305 310 315 320

Lys Lys Tyr Arg Asn Ser Ser Cys Pro Ala Trp Ser Pro Asp Gly Lys
325 330 335

Lys Ile Ala Phe Cys Ser Val Ile Lys Gly Val Arg Gln Ile Cys Ile
340 345 350

Tyr Asp Leu Ser Ser Gly Glu Asp Tyr Gln Leu Thr Thr Ser Pro Thr

355 360 365

Asn Lys Glu Ser Pro Ser Trp Ala Ile Asp Ser Arg His Leu Val Phe
370 375 380

Ser Ala Gly Asn Ala Glu Glu Ser Glu Leu Tyr Leu Ile Ser Leu Val
385 390 395 400

Thr Lys Lys Thr Asn Lys Ile Ala Ile Gly Val Gly Glu Lys Arg Phe
405 410 415

Pro Ser Trp Gly Ala Phe Pro Gln Gln Pro Ile Lys Arg Thr Leu
420 425 430

<210> 40
<211> 1296
<212> DNA
<213> Chlamydia pneumoniae

<400> 40
atgttacggc aactatgctt ccaagttttt ttcttttgct tcgcatcgct agtctatgct 60
gaagaattag aagttgttgt ccgttccgaa catatcacgc tccctattga ggtctcttgc 120
cagaccgata cgaaagatcc aaaaatacag aaatacctca gctcgctaac ggagatattt 180
tgcaaggaca ttgccctagg agattgtcta caacccacag cggcttctaa agaatcgtca 240
tctcctttag caatatcttt acggttgcat gtacctcagc tatctgtagt gcttttacag 300
tcttcaaaaa ctcctcaaac cttatgttct tttactattt ctcaaaatct ttctgtagat 360
cgtcaaaaaa tccatcacgc tgctgataca gttcattacg ccctcacagg gattcctgga 420
atcagtgctg ggaaaattgt ttttgctcta agttctttag gaaaagatca aaagctcaag 480
caaggagaat tatggactac agattacgat gggaaaaacc tcgccccttt aaccacagaa 540
tgttcgctct ctataactcc aaaatgggtg ggtgtgggat caaattttcc ctatctctat 600
gtttcgtata agtatggtgt gcctaaaatt tttcttggtt ccctagagaa cactgaaggt 660
aaaaaagtcc ttccgttaaa aggcaaccaa ctcatgccta cgttttctcc aagaaaaaag 720
cttttagctt tcgttgctga tacgtatgga aatcctgatt tatttattca accgttctca 780
ctaacttcag gacctatggg tcgcccacgt cgcctcctta atgagaattt cgggactcaa 840
gggaatccct ccttcaaccc tgaaggatcc cagcttgtct ttatatcgaa caaagacggc 900
cgtccgcgtc tttatattat gtccctcgat cctgaacccc aagcacctcg cttgctgaca 960
aaaaaataca gaaatagcag ttgccctgca tggtctccag atggtaaaaa aatagccttc 1020
tgctctgtaa ttaaaggggt gcgacaaatt tgtatttacg atctctcctc tggagaggat 1080
taccaactca ctacgtctcc cacaaataaa gagagtcctt cttgggctat agacagccgt 1140
catcttgtct ttagtgcggg gaatgctgaa gaatcagagt tatatttaat cagtctagtc 1200
accaaaaaaa ctaacaaaat tgctatagga gtaggagaaa aacggttccc ctcctggggt 1260
gctttccctc agcaaccgat aaagagaaca ctatga 1296

<210> 41
<211> 922
<212> PRT
<213> Chlamydia pneumoniae

<400> 41
Met Arg Phe Ser Leu Cys Gly Phe Pro Leu Val Phe Ser Phe Thr Leu
1 5 10 15

Leu Ser Val Phe Asp Thr Ser Leu Ser Ala Thr Thr Ile Ser Leu Thr
20 25 30

Pro Glu Asp Ser Phe His Gly Asp Ser Gln Asn Ala Glu Arg Ser Tyr
35 40 45

Asn Val Gln Ala Gly Asp Val Tyr Ser Leu Thr Gly Asp Val Ser Ile
50 55 60

Ser Asn Val Asp Asn Ser Ala Leu Asn Lys Ala Cys Phe Asn Val Thr
65 70 75 80

Ser Gly Ser Val Thr Phe Ala Gly Asn His His Gly Leu Tyr Phe Asn
85 90 95

Asn Ile Ser Ser Gly Thr Thr Lys Glu Gly Ala Val Leu Cys Cys Gln
100 105 110

Asp Pro Gln Ala Thr Ala Arg Phe Ser Gly Phe Ser Thr Leu Ser Phe
115 120 125

Ile Gln Ser Pro Gly Asp Ile Lys Glu Gln Gly Cys Leu Tyr Ser Lys
130 135 140

Asn Ala Leu Met Leu Leu Asn Asn Tyr Val Val Arg Phe Glu Gln Asn
145 150 155 160

Gln Ser Lys Thr Lys Gly Gly Ala Ile Ser Gly Ala Asn Val Thr Ile
165 170 175

Val Gly Asn Tyr Asp Ser Val Ser Phe Tyr Gln Asn Ala Ala Thr Phe
180 185 190

Gly Gly Ala Ile His Ser Ser Gly Pro Leu Gln Ile Ala Val Asn Gln
195 200 205

Ala Glu Ile Arg Phe Ala Gln Asn Thr Ala Lys Asn Gly Ser Gly Gly
210 215 220

Ala Leu Tyr Ser Asp Gly Asp Ile Asp Ile Asp Gln Asn Ala Tyr Val
225 230 235 240

Leu Phe Arg Glu Asn Glu Ala Leu Thr Thr Ala Ile Gly Lys Gly Gly
245 250 255

Ala Val Cys Cys Leu Pro Thr Ser Gly Ser Ser Thr Pro Val Pro Ile
260 265 270

Val Thr Phe Ser Asp Asn Lys Gln Leu Val Phe Glu Arg Asn His Ser
275 280 285

Ile Met Gly Gly Gly Ala Ile Tyr Ala Arg Lys Leu Ser Ile Ser Ser
290 295 300

Gly Gly Pro Thr Leu Phe Ile Asn Asn Ile Ser Tyr Ala Asn Ser Gln
305 310 315 320

Asn Leu Gly Gly Ala Ile Ala Ile Asp Thr Gly Gly Glu Ile Ser Leu
325 330 335

Ser Ala Glu Lys Gly Thr Ile Thr Phe Gln Gly Asn Arg Thr Ser Leu
340 345 350

Pro Phe Leu Asn Gly Ile His Leu Leu Gln Asn Ala Lys Phe Leu Lys
355 360 365

Leu Gln Ala Arg Asn Gly Tyr Ser Ile Glu Phe Tyr Asp Pro Ile Thr
370 375 380

Ser Glu Ala Asp Gly Ser Thr Gln Leu Asn Ile Asn Gly Asp Pro Lys

385 390 395 400

Asn Lys Glu Tyr Thr Gly Thr Ile Leu Phe Ser Gly Glu Lys Ser Leu
405 410 415

Ala Asn Asp Pro Arg Asp Phe Lys Ser Thr Ile Pro Gln Asn Val Asn
420 425 430

Leu Ser Ala Gly Tyr Leu Val Ile Lys Glu Gly Ala Glu Val Thr Val
435 440 445

Ser Lys Phe Thr Gln Ser Pro Gly Ser His Leu Val Leu Asp Leu Gly
450 455 460

Thr Lys Leu Ile Ala Ser Lys Glu Asp Ile Ala Ile Thr Gly Leu Ala
465 470 475 480

Ile Asp Ile Asp Ser Leu Ser Ser Ser Ser Thr Ala Ala Val Ile Lys
485 490 495

Ala Asn Thr Ala Asn Lys Gln Ile Ser Val Thr Asp Ser Ile Glu Leu
500 505 510

Ile Ser Pro Thr Gly Asn Ala Tyr Glu Asp Leu Arg Met Arg Asn Ser
515 520 525

Gln Thr Phe Pro Leu Leu Ser Leu Glu Pro Gly Ala Gly Gly Ser Val
530 535 540

Thr Val Thr Ala Gly Asp Phe Leu Pro Val Ser Pro His Tyr Gly Phe
545 550 555 560

Gln Gly Asn Trp Lys Leu Ala Trp Thr Gly Thr Gly Asn Lys Val Gly
565 570 575

Glu Phe Phe Trp Asp Lys Ile Asn Tyr Lys Pro Arg Pro Glu Lys Glu
580 585 590

Gly Asn Leu Val Pro Asn Ile Leu Trp Gly Asn Ala Val Asp Val Arg
595 600 605

Ser Leu Met Gln Val Gln Glu Thr His Ala Ser Ser Leu Gln Thr Asp
610 615 620

Arg Gly Leu Trp Ile Asp Gly Ile Gly Asn Phe Phe His Val Ser Ala
625 630 635 640

Ser Glu Asp Asn Ile Arg Tyr Arg His Asn Ser Gly Gly Tyr Val Leu
645 650 655

Ser Val Asn Asn Glu Ile Thr Pro Lys His Tyr Thr Ser Met Ala Phe
660 665 670

Ser Gln Leu Phe Ser Arg Asp Lys Asp Tyr Ala Val Ser Asn Asn Glu
675 680 685

Tyr Arg Met Tyr Leu Gly Ser Tyr Leu Tyr Gln Tyr Thr Thr Ser Leu
690 695 700

Gly Asn Ile Phe Arg Tyr Ala Ser Arg Asn Pro Asn Val Asn Val Gly
705 710 715 720

```
Ile Leu Ser Arg Arg Phe Leu Gln Asn Pro Leu Met Ile Phe His Phe
                725                 730                 735

Leu Cys Ala Tyr Gly His Ala Thr Asn Asp Met Lys Thr Asp Tyr Ala
            740                 745                 750

Asn Phe Pro Met Val Lys Asn Ser Trp Arg Asn Asn Cys Trp Ala Ile
        755                 760                 765

Glu Cys Gly Gly Ser Met Pro Leu Leu Val Phe Glu Asn Gly Arg Leu
    770                 775                 780

Phe Gln Gly Ala Ile Pro Phe Met Lys Leu Gln Leu Val Tyr Ala Tyr
785                 790                 795                 800

Gln Gly Asp Phe Lys Glu Thr Thr Ala Asp Gly Arg Arg Phe Ser Asn
                805                 810                 815

Gly Ser Leu Thr Ser Ile Ser Val Pro Leu Gly Ile Arg Phe Glu Lys
            820                 825                 830

Leu Ala Leu Ser Gln Asp Val Leu Tyr Asp Phe Ser Phe Ser Tyr Ile
        835                 840                 845

Pro Asp Ile Phe Arg Lys Asp Pro Ser Cys Glu Ala Ala Leu Val Ile
    850                 855                 860

Ser Gly Asp Ser Trp Leu Val Pro Ala Ala His Val Ser Arg His Ala
865                 870                 875                 880

Phe Val Gly Ser Gly Thr Gly Arg Tyr His Phe Asn Asp Tyr Thr Glu
                885                 890                 895

Leu Leu Cys Arg Gly Ser Ile Glu Cys Arg Pro His Ala Arg Asn Tyr
            900                 905                 910

Asn Ile Asn Cys Gly Ser Lys Phe Arg Phe
        915                 920

<210>   42
<211>   2769
<212>   DNA
<213>   Chlamydia pneumoniae

<400>   42
atgcgatttt cgctctgcgg atttcctcta gtttttttctt ttacattgct ctcagtcttc    60
gacacttctt tgagtgctac tacgatttct ttaaccccag aagatagttt tcatggagat   120
agtcagaatg cagaacgttc ttataatgtt caagctgggg atgtctatag ccttactggt   180
gatgtctcaa tatctaacgt cgataactct gcattaaata aagcctgctt caatgtgacc   240
tcaggaagtg tgacgttcgc aggaaatcat catgggttat attttaataa tatttcctca   300
ggaactacaa aggaaggggc tgtactttgt tgccaagatc ctcaagcaac ggcacgtttt   360
tctgggttct ccacgctctc ttttattcag agccccggag atattaaaga acagggatgt   420
ctctattcaa aaaatgcact tatgctctta aacaattatg tagtgcgttt tgaacaaaac   480
caaagtaaga ctaaaggcgg agctattagt ggggcgaatg ttactatagt aggcaactac   540
gattccgtct ctttctatca gaatgcagcc acttttggag gtgctatcca ttcttcaggt   600
cccctacaga ttgcagtaaa tcaggcagag ataagatttg cacaaaatac tgccaagaat   660
ggttctggag ggggctttgta ctccgatggt gatattgata ttgatcagaa tgcttatgtt   720
ctatttcgag aaaatgaggc attgactact gctataggta agggaggggc tgtctgttgt   780
cttcccactt caggaagtag tactccagtt cctattgtga ctttctctga caataaacag   840
ttagtctttg aaagaaacca ttccataatg ggtggcggag ccatttatgc taggaaactt   900
```

```
agcatctctt caggaggtcc tactctattt atcaataata tatcatatgc aaattcgcaa    960
aatttaggtg gagctattgc cattgatact ggaggggaga tcagtttatc agcagagaaa   1020
ggaacaatta cattccaagg aaaccggacg agcttaccgt ttttgaatgg catccatctt   1080
ttacaaaatg ctaaattcct gaaattacag gcgagaaatg gatactctat agaattttat   1140
gatcctatta cttctgaagc agatgggtct acccaattga atatcaacgg agatcctaaa   1200
aataaagagt acacagggac catactcttt tctggagaaa agagtctagc aaacgatcct   1260
agggatttta aatctacaat ccctcagaac gtcaacctgt ctgcaggata cttagttatt   1320
aaagaggggg ccgaagtcac agtttcaaaa ttcacgcagt ctccaggatc gcatttagtt   1380
ttagatttag gaaccaaact gatagcctct aaggaagaca ttgccatcac aggcctcgcg   1440
atagatatag atagcttaag ctcatcctca acagcagctg ttattaaagc aaacaccgca   1500
aataaacaga tatccgtgac ggactctata gaacttatct cgcctactgg caatgcctat   1560
gaagatctca gaatgagaaa ttcacagacg ttccctctgc tctctttaga gcctggagcc   1620
gggggtagtg tgactgtaac tgctggagat ttcctaccgg taagtcccca ttatggtttt   1680
caaggcaatt ggaaattagc ttggacagga actggaaaca aagttggaga attcttctgg   1740
gataaaataa attataagcc tagacctgaa aaagaaggaa atttagttcc taatatcttg   1800
tgggggaatg ctgtagatgt cagatcctta atgcaggttc aagagaccca tgcatcgagc   1860
ttacagacag atcgagggct gtggatcgat ggaattggga atttcttcca tgtatctgcc   1920
tccgaagaca atataaggta ccgtcataac agcggtggat atgttctatc tgtaaataat   1980
gagatcacac ctaagcacta tacttcgatg gcattttccc aactctttag tagagacaag   2040
gactatgcgg tttccaacaa cgaatacaga atgtatttag gatcgtatct ctatcaatat   2100
acaacctccc tagggaatat tttccgttat gcttcgcgta accctaatgt aaacgtcggg   2160
attctctcaa gaaggtttct tcaaaatcct cttatgattt ttcatttttt gtgtgcttat   2220
ggtcatgcca ccaatgatat gaaaacagac tacgcaaatt tccctatggt gaaaaacagc   2280
tggagaaaca attgttgggc tatagagtgc ggagggagca tgcctctatt ggtatttgag   2340
aacggaagac ttttccaagg tgccatccca tttatgaaac tacaattagt ttatgcttat   2400
cagggagatt tcaaagagac gactgcagat ggccgtagat ttagtaatgg gagtttaaca   2460
tcgatttctg tacctctagg catacgcttt gagaagctgg cactttctca ggatgtactc   2520
tatgacttta gtttctccta tattcctgat attttccgta aggatccctc atgtgaagct   2580
gctctggtga ttagcggaga ctcctggctt gttccggcag cacacgtatc aagacatgct   2640
tttgtaggga gtggaacggg tcggtatcac tttaacgact atactgagct cttatgtcga   2700
ggaagtatag aatgccgccc ccatgctagg aattataata taaactgtgg aagcaaattt   2760
cgtttttag                                                           2769
```

<210> 43
<211> 390
<212> PRT
<213> Chlamydia pneumoniae

<400> 43

```
Met Ser Ser Pro Val Asn Asn Thr Pro Ser Ala Pro Asn Ile Pro Ile
1               5                   10                  15

Pro Ala Pro Thr Thr Pro Gly Ile Pro Thr Thr Lys Pro Arg Ser Ser
            20                  25                  30

Phe Ile Glu Lys Val Ile Ile Val Ala Lys Tyr Ile Leu Phe Ala Ile
        35                  40                  45

Ala Ala Thr Ser Gly Ala Leu Gly Thr Ile Leu Gly Leu Ser Gly Ala
    50                  55                  60

Leu Thr Pro Gly Ile Gly Ile Ala Leu Leu Val Ile Phe Phe Val Ser
65                  70                  75                  80

Met Val Leu Leu Gly Leu Ile Leu Lys Asp Ser Ile Ser Gly Gly Glu
                85                  90                  95

Glu Arg Arg Leu Arg Glu Glu Val Ser Arg Phe Thr Ser Glu Asn Gln
            100                 105                 110

Arg Leu Thr Val Ile Thr Thr Thr Leu Glu Thr Glu Val Lys Asp Leu
```

```
        115                 120                 125

Lys Ala Ala Lys Asp Gln Leu Thr Leu Glu Ile Glu Ala Phe Arg Asn
    130                 135                 140

Glu Asn Gly Asn Leu Lys Thr Thr Ala Glu Asp Leu Glu Glu Gln Val
145                 150                 155                 160

Ser Lys Leu Ser Glu Gln Leu Glu Ala Leu Glu Arg Ile Asn Gln Leu
                165                 170                 175

Ile Gln Ala Asn Ala Gly Asp Ala Gln Glu Ile Ser Ser Glu Leu Lys
            180                 185                 190

Lys Leu Ile Ser Gly Trp Asp Ser Lys Val Val Glu Gln Ile Asn Thr
        195                 200                 205

Ser Ile Gln Ala Leu Lys Val Leu Leu Gly Gln Glu Trp Val Gln Glu
    210                 215                 220

Ala Gln Thr His Val Lys Ala Met Gln Glu Gln Ile Gln Ala Leu Gln
225                 230                 235                 240

Ala Glu Ile Leu Gly Met His Asn Gln Ser Thr Ala Leu Gln Lys Ser
                245                 250                 255

Val Glu Asn Leu Leu Val Gln Asp Gln Ala Leu Thr Arg Val Val Gly
            260                 265                 270

Glu Leu Leu Glu Ser Glu Asn Lys Leu Ser Gln Ala Cys Ser Ala Leu
        275                 280                 285

Arg Gln Glu Ile Glu Lys Leu Ala Gln His Glu Thr Ser Leu Gln Gln
    290                 295                 300

Arg Ile Asp Ala Met Leu Ala Gln Glu Gln Asn Leu Ala Glu Gln Val
305                 310                 315                 320

Thr Ala Leu Glu Lys Met Lys Gln Glu Ala Gln Lys Ala Glu Ser Glu
                325                 330                 335

Phe Ile Ala Cys Val Arg Asp Arg Thr Phe Gly Arg Arg Glu Thr Pro
            340                 345                 350

Pro Pro Thr Thr Pro Val Val Glu Gly Asp Glu Ser Gln Glu Glu Asp
        355                 360                 365

Glu Gly Gly Thr Pro Pro Val Ser Gln Pro Ser Ser Pro Val Asp Arg
    370                 375                 380

Ala Thr Gly Asp Gly Gln
385                 390

<210>   44
<211>   1173
<212>   DNA
<213>   Chlamydia pneumoniae

<400>   44
atgtcatctc ctgtaaataa cacaccctca gcaccaaaca ttccaatacc agcgcccacg     60
actccaggta ttcctacaac aaaacctcgt tctagtttca ttgaaaaggt tatcattgta    120
```

```
gctaagtaca tactatttgc aattgcagcc acatcaggag cactcggaac aattctaggt   180
ctatctggag cgctaacccc aggaataggt attgcccttc ttgttatctt ctttgtttct   240
atggtgcttt taggtttaat ccttaaagat tctataagtg gaggagaaga acgcaggctc   300
agagaagagg tctctcgatt tacaagtgag aatcaacggt tgacagtcat aaccacaaca   360
cttgagactg aagtaaagga tttaaaagca gctaaagatc aacttacact tgaaatcgaa   420
gcatttagaa atgaaaacgg taatttaaaa acaactgctg aggacttaga agagcaggtt   480
tctaaactta gcgaacaatt agaagcacta gagcgaatta atcaacttat ccaagcaaac   540
gctggagatg ctcaagaaat ttcgtctgaa ctaaagaaat taataagcgg ttgggattcc   600
aaagttgttg aacagataaa tacttctatt caagcattga aagtgttatt gggtcaagag   660
tgggtgcaag aggctcaaac acacgttaaa gcaatgcaag agcaaattca agcattgcaa   720
gctgaaattc taggaatgca caatcaatct acagcattgc aaaagtcagt tgagaatcta   780
ttagtacaag atcaagctct aacaagagta gtaggtgagt tgttagagtc tgagaacaag   840
ctaagccaag cttgttctgc gctacgtcaa gaaatagaaa agttggccca acatgaaaca   900
tctttgcaac aacgtattga tgcgatgcta gcccaagagc aaaatttggc agagcaggtc   960
acagcccttg aaaaaatgaa acaagaagct cagaaggctg agtccgagtt cattgcttgt   1020
gtacgtgatc gaactttcgg acgtcgtgaa acacctccac caacaacacc tgtagttgaa   1080
ggtgatgaaa gtcaagaaga agacgaagga ggtactcccc cagtatcaca accatcttca   1140
cccgtagata gagcaacagg agatggtcag taa                               1173
```

<210> 45
<211> 930
<212> PRT
<213> Chlamydia pneumoniae

<400> 45

```
Met Lys Ile Pro Leu His Lys Leu Leu Ile Ser Ser Thr Leu Val Thr
1               5                   10                  15

Pro Ile Leu Leu Ser Ile Ala Thr Tyr Gly Ala Asp Ala Ser Leu Ser
            20                  25                  30

Pro Thr Asp Ser Phe Asp Gly Ala Gly Gly Ser Thr Phe Thr Pro Lys
        35                  40                  45

Ser Thr Ala Asp Ala Asn Gly Thr Asn Tyr Val Leu Ser Gly Asn Val
    50                  55                  60

Tyr Ile Asn Asp Ala Gly Lys Gly Thr Ala Leu Thr Gly Cys Cys Phe
65                  70                  75                  80

Thr Glu Thr Thr Gly Asp Leu Thr Phe Thr Gly Lys Gly Tyr Ser Phe
                85                  90                  95

Ser Phe Asn Thr Val Asp Ala Gly Ser Asn Ala Gly Ala Ala Ala Ser
            100                 105                 110

Thr Thr Ala Asp Lys Ala Leu Thr Phe Thr Gly Phe Ser Asn Leu Ser
        115                 120                 125

Phe Ile Ala Ala Pro Gly Thr Thr Val Ala Ser Gly Lys Ser Thr Leu
    130                 135                 140

Ser Ser Ala Gly Ala Leu Asn Leu Thr Asp Asn Gly Thr Ile Leu Phe
145                 150                 155                 160

Ser Gln Asn Val Ser Asn Glu Ala Asn Asn Asn Gly Gly Ala Ile Thr
                165                 170                 175

Thr Lys Thr Leu Ser Ile Ser Gly Asn Thr Ser Ser Ile Thr Phe Thr
            180                 185                 190
```

Ser Asn Ser Ala Lys Lys Leu Gly Gly Ala Ile Tyr Ser Ser Ala Ala
195 200 205

Ala Ser Ile Ser Gly Asn Thr Gly Gln Leu Val Phe Met Asn Asn Lys
210 215 220

Gly Glu Thr Gly Gly Gly Ala Leu Gly Phe Glu Ala Ser Ser Ser Ile
225 230 235 240

Thr Gln Asn Ser Ser Leu Phe Phe Ser Gly Asn Thr Ala Thr Asp Ala
245 250 255

Ala Gly Lys Gly Gly Ala Ile Tyr Cys Glu Lys Thr Gly Glu Thr Pro
260 265 270

Thr Leu Thr Ile Ser Gly Asn Lys Ser Leu Thr Phe Ala Glu Asn Ser
275 280 285

Ser Val Thr Gln Gly Gly Ala Ile Cys Ala His Gly Leu Asp Leu Ser
290 295 300

Ala Ala Gly Pro Thr Leu Phe Ser Asn Asn Arg Cys Gly Asn Thr Ala
305 310 315 320

Ala Gly Lys Gly Gly Ala Ile Ala Ile Ala Asp Ser Gly Ser Leu Ser
325 330 335

Leu Ser Ala Asn Gln Gly Asp Ile Thr Phe Leu Gly Asn Thr Leu Thr
340 345 350

Ser Thr Ser Ala Pro Thr Ser Thr Arg Asn Ala Ile Tyr Leu Gly Ser
355 360 365

Ser Ala Lys Ile Thr Asn Leu Arg Ala Ala Gln Gly Gln Ser Ile Tyr
370 375 380

Phe Tyr Asp Pro Ile Ala Ser Asn Thr Thr Gly Ala Ser Asp Val Leu
385 390 395 400

Thr Ile Asn Gln Pro Asp Ser Asn Ser Pro Leu Asp Tyr Ser Gly Thr
405 410 415

Ile Val Phe Ser Gly Glu Lys Leu Ser Ala Asp Glu Ala Lys Ala Ala
420 425 430

Asp Asn Phe Thr Ser Ile Leu Lys Gln Pro Leu Ala Leu Ala Ser Gly
435 440 445

Thr Leu Ala Leu Lys Gly Asn Val Glu Leu Asp Val Asn Gly Phe Thr
450 455 460

Gln Thr Glu Gly Ser Thr Leu Leu Met Gln Pro Gly Thr Lys Leu Lys
465 470 475 480

Ala Asp Thr Glu Ala Ile Ser Leu Thr Lys Leu Val Val Asp Leu Ser
485 490 495

Ala Leu Glu Gly Asn Lys Ser Val Ser Ile Glu Thr Ala Gly Ala Asn
500 505 510

Lys Thr Ile Thr Leu Thr Ser Pro Leu Val Phe Gln Asp Ser Ser Gly

515 520 525

Asn Phe Tyr Glu Ser His Thr Ile Asn Gln Ala Phe Thr Gln Pro Leu
530 535 540

Val Val Phe Thr Ala Ala Thr Ala Ala Ser Asp Ile Tyr Ile Asp Ala
545 550 555 560

Leu Leu Thr Ser Pro Val Gln Thr Pro Glu Pro His Tyr Gly Tyr Gln
565 570 575

Gly His Trp Glu Ala Thr Trp Ala Asp Thr Ser Thr Ala Lys Ser Gly
580 585 590

Thr Met Thr Trp Val Thr Thr Gly Tyr Asn Pro Asn Pro Glu Arg Arg
595 600 605

Ala Ser Val Val Pro Asp Ser Leu Trp Ala Ser Phe Thr Asp Ile Arg
610 615 620

Thr Leu Gln Gln Ile Met Thr Ser Gln Ala Asn Ser Ile Tyr Gln Gln
625 630 635 640

Arg Gly Leu Trp Ala Ser Gly Thr Ala Asn Phe Phe His Lys Asp Lys
645 650 655

Ser Gly Thr Asn Gln Ala Phe Arg His Lys Ser Tyr Gly Tyr Ile Val
660 665 670

Gly Gly Ser Ala Glu Asp Phe Ser Glu Asn Ile Phe Ser Val Ala Phe
675 680 685

Cys Gln Leu Phe Gly Lys Asp Lys Asp Leu Phe Ile Val Glu Asn Thr
690 695 700

Ser His Asn Tyr Leu Ala Ser Leu Tyr Leu Gln His Arg Ala Phe Leu
705 710 715 720

Gly Gly Leu Pro Met Pro Ser Phe Gly Ser Ile Thr Asp Met Leu Lys
725 730 735

Asp Ile Pro Leu Ile Leu Asn Ala Gln Leu Ser Tyr Ser Tyr Thr Lys
740 745 750

Asn Asp Met Asp Thr Arg Tyr Thr Ser Tyr Pro Glu Ala Gln Gly Ser
755 760 765

Trp Thr Asn Asn Ser Gly Ala Leu Glu Leu Gly Gly Ser Leu Ala Leu
770 775 780

Tyr Leu Pro Lys Glu Ala Pro Phe Phe Gln Gly Tyr Phe Pro Phe Leu
785 790 795 800

Lys Phe Gln Ala Val Tyr Ser Arg Gln Gln Asn Phe Lys Glu Ser Gly
805 810 815

Ala Glu Ala Arg Ala Phe Asp Asp Gly Asp Leu Val Asn Cys Ser Ile
820 825 830

Pro Val Gly Ile Arg Leu Glu Lys Ile Ser Glu Asp Glu Lys Asn Asn
835 840 845

Phe Glu Ile Ser Leu Ala Tyr Ile Gly Asp Val Tyr Arg Lys Asn Pro
850 855 860

Arg Ser Arg Thr Ser Leu Met Val Ser Gly Ala Ser Trp Thr Ser Leu
865 870 875 880

Cys Lys Asn Leu Ala Arg Gln Ala Phe Leu Ala Ser Ala Gly Ser His
885 890 895

Leu Thr Leu Ser Pro His Val Glu Leu Ser Gly Glu Ala Ala Tyr Glu
900 905 910

Leu Arg Gly Ser Ala His Ile Tyr Asn Val Asp Cys Gly Leu Arg Tyr
915 920 925

Ser Phe
930

<210> 46
<211> 2793
<212> DNA
<213> Chlamydia pneumoniae

<400> 46
atgaaaatac ccttgcacaa actcctgatc tcttcgactc ttgtcactcc cattctattg 60
agcattgcaa cttacggagc agatgcttct ttatccccta cagatagctt tgatggagcg 120
ggcggctcta catttactcc aaaatctaca gcagatgcca atggaacgaa ctatgtctta 180
tcaggaaatg tctatataaa cgatgctggg aaaggcacag cattaacagg ctgctgcttt 240
acagaaacta cgggtgatct gacatttact ggaaagggat actcattttc attcaacacg 300
gtagatgcgg gttcgaatgc aggagctgcg gcaagcacaa ctgctgataa agccctaaca 360
ttcacaggat ttctaacct ttccttcatt gcagctcctg gaactacagt tgcttcagga 420
aaaagtactt taagttctgc aggagcctta aatcttaccg ataatggaac gattctcttt 480
agccaaaacg tctccaatga agctaataac aatggcggag cgatcaccac aaaaactctt 540
tctatttctg ggaatacctc ttctataacc ttcactagta atagcgcaaa aaaattaggt 600
ggagcgatct atagctctgc ggctgcaagt atttcaggaa acaccggcca gttagtcttt 660
atgaataata aaggagaaac tgggggtggg gctctgggct ttgaagccag ctcctcgatt 720
actcaaaata gctccctttt cttctctgga aacactgcaa cagatgctgc aggcaagggc 780
ggggccattt attgtgaaaa aacaggagag actcctactc ttactatctc tggaaataaa 840
agtctgacct tcgccgagaa ctcttcagta actcaaggcg gagcaatctg tgcccatggt 900
ctagatcttt ccgctgctgg ccctacccta ttttcaaata atagatgcgg gaacacagct 960
gcaggcaagg gcggcgctat tgcaattgcc gactctggat ctttaagtct ctctgcaaat 1020
caaggagaca tcacgttcct tggcaacact ctaacctcaa cctccgcgcc aacatcgaca 1080
cggaatgcta tctacctggg atcgtcagca aaaattacga acttaagggc agcccaaggc 1140
caatctatct atttctatga tccgattgca tctaacacca caggagcttc agacgttctg 1200
accatcaacc aaccggatag caactcgcct ttagattatt caggaacgat tgtattttct 1260
ggggaaaagc tctctgcaga tgaagcgaaa gctgctgata acttcacatc tatattaaag 1320
caaccattgg ctctagcctc tggaacctta gcactcaaag gaaatgtcga gttagatgtc 1380
aatggtttca cacagactga aggctctaca ctcctcatgc aaccaggaac aaagctcaaa 1440
gcagatactg aagctatcag tcttaccaaa cttgtcgttg atctttctgc cttagaggga 1500
aataagagtg tgtccattga aacagcagga gccaacaaaa ctataactct aacctctcct 1560
cttgttttcc aagatagtag cggcaatttt tatgaaagcc atacgataaa ccaagccttc 1620
acgcagcctt tggtggtatt cactgctgct actgctgcta gcgatattta tatcgatgcg 1680
cttctcactt ctccagtaca aactccagaa cctcattacg ggtatcaggg acattgggaa 1740
gccacttggg cagacacatc aactgcaaaa tcaggaacta tgacttgggt aactacgggc 1800
tacaacccta atcctgagcg tagagcttcc gtagttcccg attcattatg ggcatccttt 1860
actgacattc gcactctaca gcagatcatg acatctcaag cgaatagtat ctatcagcaa 1920
cgaggactct gggcatcagg aactgcgaat ttcttccata aggataaatc aggaactaac 1980
caagcattcc gacataaaag ctacggctat attgttggag gaagtgctga agatttttct 2040
gaaaatatct tcagtgtagc tttctgccag ctcttcggta aagataaaga cctgtttata 2100
gttgaaaata cctctcataa ctatttagcg tcgctatacc tgcaacatcg agcattccta 2160

```
ggaggacttc ccatgccctc atttggaagt atcaccgaca tgctgaaaga tattcctctc   2220
attttgaatg cccagctaag ctacagctac actaaaaatg atatggatac tcgctatact   2280
tcctatcctg aagctcaagg ctcttggacc aataactctg ggctctaga gctcggagga    2340
tctctggctc tatatctccc taaagaagca ccgttcttcc agggatattt ccccttctta   2400
aagttccagg cagtctacag ccgccaacaa aactttaaag agagtggcgc tgaagcccgt   2460
gcttttgatg atggagacct agtgaactgc tctatccctg tcggcattcg gttagaaaaa   2520
atctccgaag atgaaaaaaa taatttcgag atttctctag cctacattgg tgatgtgtat   2580
cgtaaaaatc cccgttcgcg tacttctcta atggtcagtg gagcctcttg gacttcgcta   2640
tgtaaaaacc tcgcacgaca agccttctta gcaagtgctg gaagccatct gactctctcc   2700
cctcatgtag aactctctgg ggaagctgct tatgagcttc gtggctcagc acacatctac   2760
aatgtagatt gtgggctaag atactcattc tag                                2793
```

<210> 47
<211> 556
<212> PRT
<213> Chlamydia pneumoniae

<400> 47

```
Met Ser Lys Leu Ile Arg Arg Val Val Thr Val Leu Ala Leu Thr Ser
1               5                   10                  15

Met Ala Ser Cys Phe Ala Ser Gly Gly Ile Glu Ala Ala Val Ala Glu
            20                  25                  30

Ser Leu Ile Thr Lys Ile Val Ala Ser Ala Glu Thr Lys Pro Ala Pro
        35                  40                  45

Val Pro Met Thr Ala Lys Lys Val Arg Leu Val Arg Arg Asn Lys Gln
    50                  55                  60

Pro Val Glu Gln Lys Ser Arg Gly Ala Phe Cys Asp Lys Glu Phe Tyr
65                  70                  75                  80

Pro Cys Glu Glu Gly Arg Cys Gln Pro Val Glu Ala Gln Gln Glu Ser
                85                  90                  95

Cys Tyr Gly Arg Leu Tyr Ser Val Lys Val Asn Asp Asp Cys Asn Val
            100                 105                 110

Glu Ile Cys Gln Ser Val Pro Glu Tyr Ala Thr Val Gly Ser Pro Tyr
        115                 120                 125

Pro Ile Glu Ile Leu Ala Ile Gly Lys Lys Asp Cys Val Asp Val Val
    130                 135                 140

Ile Thr Gln Gln Leu Pro Cys Glu Ala Glu Phe Val Ser Ser Asp Pro
145                 150                 155                 160

Glu Thr Thr Pro Thr Ser Asp Gly Lys Leu Val Trp Lys Ile Asp Arg
                165                 170                 175

Leu Gly Ala Gly Asp Lys Cys Lys Ile Thr Val Trp Val Lys Pro Leu
            180                 185                 190

Lys Glu Gly Cys Cys Phe Thr Ala Ala Thr Val Cys Ala Cys Pro Glu
        195                 200                 205

Leu Arg Ser Tyr Thr Lys Cys Gly Gln Pro Ala Ile Cys Ile Lys Gln
    210                 215                 220

Glu Gly Pro Asp Cys Ala Cys Leu Arg Cys Pro Val Cys Tyr Lys Ile
```

```
225                 230                 235                 240

Glu Val Val Asn Thr Gly Ser Ala Ile Ala Arg Asn Val Thr Val Asp
                245                 250                 255

Asn Pro Val Pro Asp Gly Tyr Ser His Ala Ser Gly Gln Arg Val Leu
            260                 265                 270

Ser Phe Asn Leu Gly Asp Met Arg Pro Gly Asp Lys Lys Val Phe Thr
        275                 280                 285

Val Glu Phe Cys Pro Gln Arg Arg Gly Gln Ile Thr Asn Val Ala Thr
    290                 295                 300

Val Thr Tyr Cys Gly Gly His Lys Cys Ser Ala Asn Val Thr Thr Val
305                 310                 315                 320

Val Asn Glu Pro Cys Val Gln Val Asn Ile Ser Gly Ala Asp Trp Ser
                325                 330                 335

Tyr Val Cys Lys Pro Val Glu Tyr Ser Ile Ser Val Ser Asn Pro Gly
            340                 345                 350

Asp Leu Val Leu His Asp Val Val Ile Gln Asp Thr Leu Pro Ser Gly
        355                 360                 365

Val Thr Val Leu Glu Ala Pro Gly Gly Glu Ile Cys Cys Asn Lys Val
    370                 375                 380

Val Trp Arg Ile Lys Glu Met Cys Pro Gly Glu Thr Leu Gln Phe Lys
385                 390                 395                 400

Leu Val Val Lys Ala Gln Val Pro Gly Arg Phe Thr Asn Gln Val Ala
                405                 410                 415

Val Thr Ser Glu Ser Asn Cys Gly Thr Cys Thr Ser Cys Ala Glu Thr
            420                 425                 430

Thr Thr His Trp Lys Gly Leu Ala Ala Thr His Met Cys Val Leu Asp
        435                 440                 445

Thr Asn Asp Pro Ile Cys Val Gly Glu Asn Thr Val Tyr Arg Ile Cys
    450                 455                 460

Val Thr Asn Arg Gly Ser Ala Glu Asp Thr Asn Val Ser Leu Ile Leu
465                 470                 475                 480

Lys Phe Ser Lys Glu Leu Gln Pro Ile Ala Ser Ser Gly Pro Thr Lys
                485                 490                 495

Gly Thr Ile Ser Gly Asn Thr Val Val Phe Asp Ala Leu Pro Lys Leu
            500                 505                 510

Gly Ser Lys Glu Ser Val Glu Phe Ser Val Thr Leu Lys Gly Ile Ala
        515                 520                 525

Pro Gly Asp Ala Arg Gly Glu Ala Ile Leu Ser Ser Asp Thr Leu Thr
    530                 535                 540

Ser Pro Val Ser Asp Thr Glu Asn Thr His Val Tyr
545                 550                 555
```

<210> 48
<211> 1671
<212> DNA
<213> Chlamydia pneumoniae

<400> 48
atgtccaaac tcatcagacg agtagttacg gtccttgcgc taacgagtat ggcgagttgc 60
tttgccagcg ggggtataga ggccgctgta gcagagtctc tgattactaa gatcgtcgct 120
agtgcggaaa caaagccagc acctgttcct atgacagcga agaaggttag acttgtccgt 180
agaaataaac aaccagttga acaaaaaagc cgtggtgctt tttgtgataa agaattttat 240
ccctgtgaag agggacgatg tcaacctgta gaggctcagc aagagtcttg ctacggaaga 300
ttgtattctg taaaagtaaa cgatgattgc aacgtagaaa tttgccagtc cgttccagaa 360
tacgctactg taggatctcc ttaccctatt gaaatccttg ctataggcaa aaaagattgt 420
gttgatgttg tgattacaca acagctacct tgcgaagctg aattcgtaag cagtgatcca 480
gaaacaactc ctacaagtga tgggaaatta gtctggaaaa tcgatcgcct gggtgcagga 540
gataaatgca aaattactgt atgggtaaaa cctcttaaag aaggttgctg cttcacagct 600
gctactgtat gtgcttgccc agagctccgt tcttatacta aatgcggtca accagccatt 660
tgtattaagc aagaaggacc tgactgtgct tgcctaagat gccctgtatg ctacaaaatc 720
gaagtagtga acacaggatc tgctattgcc cgtaacgtaa ctgtagataa tcctgttccc 780
gatggctatt ctcatgcatc tggtcaaaga gttctctctt ttaacttagg agacatgaga 840
cctggcgata aaaaggtatt tacagttgag ttctgccctc aaagaagagg tcaaatcact 900
aacgttgcta ctgtaactta ctgcggtgga cacaaatgtt ctgcaaatgt aactacagtt 960
gttaatgagc cttgtgtaca agtaaatatc tctggtgctg attggtctta cgtatgtaaa 1020
cctgtggagt actctatctc agtatcgaat cctggagact tggttcttca tgatgtcgtg 1080
atccaagata cactcccttc tggtgttaca gtactcgaag ctcctggtgg agagatctgc 1140
tgtaataaag ttgtttggcg tattaaagaa atgtgcccag gagaaaccct ccagtttaaa 1200
cttgtagtga aagctcaagt tcctggaaga ttcacaaatc aagttgcagt aactagtgag 1260
tctaactgcg gaacatgtac atcttgcgca gaaacaacaa cacattggaa aggtcttgca 1320
gctacccata tgtgcgtatt agacacaaat gatcctatct gtgtaggaga aaatactgtc 1380
tatcgtatct gtgtaactaa ccgtggttct gctgaagata ctaacgtatc tttaatcttg 1440
aagttctcaa aagaacttca gccaatagct tcttcaggtc caactaaagg aacgatttca 1500
ggtaataccg ttgttttcga cgctttacct aaactcggtt ctaaggaatc tgtagagttt 1560
tctgttacct tgaaaggtat tgctcccgga gatgctcgcg gcgaagctat tctttcttct 1620
gatacactga cttcaccagt atcagacaca gaaaataccc acgtgtatta a 1671

<210> 49
<211> 568
<212> PRT
<213> Chlamydia pneumoniae

<400> 49
Met Gly Leu Phe His Leu Thr Leu Phe Gly Leu Leu Leu Cys Ser Leu
1 5 10 15

Pro Ile Ser Leu Val Ala Lys Phe Pro Glu Ser Val Gly His Lys Ile
20 25 30

Leu Tyr Ile Ser Thr Gln Ser Thr Gln Gln Ala Leu Ala Thr Tyr Leu
35 40 45

Glu Ala Leu Asp Ala Tyr Gly Asp His Asp Phe Phe Val Leu Arg Lys
50 55 60

Ile Gly Glu Asp Tyr Leu Lys Gln Ser Ile His Ser Ser Asp Pro Gln
65 70 75 80

Thr Arg Lys Ser Thr Ile Ile Gly Ala Gly Leu Ala Gly Ser Ser Glu
85 90 95

Ala Leu Asp Val Leu Ser Gln Ala Met Glu Thr Ala Asp Pro Leu Gln

100 105 110

Gln Leu Leu Val Leu Ser Ala Val Ser Gly His Leu Gly Lys Thr Ser
115 120 125

Asp Asp Leu Leu Phe Lys Ala Leu Ala Ser Pro Tyr Pro Val Ile Arg
130 135 140

Leu Glu Ala Ala Tyr Arg Leu Ala Asn Leu Lys Asn Thr Lys Val Ile
145 150 155 160

Asp His Leu His Ser Phe Ile His Lys Leu Pro Glu Glu Ile Gln Cys
165 170 175

Leu Ser Ala Ala Ile Phe Leu Arg Leu Glu Thr Glu Glu Ser Asp Ala
180 185 190

Tyr Ile Arg Asp Leu Leu Ala Ala Lys Lys Ser Ala Ile Arg Ser Ala
195 200 205

Thr Ala Leu Gln Ile Gly Glu Tyr Gln Gln Lys Arg Phe Leu Pro Thr
210 215 220

Leu Arg Asn Leu Leu Thr Ser Ala Ser Pro Gln Asp Gln Glu Ala Ile
225 230 235 240

Leu Tyr Ala Leu Gly Lys Leu Lys Asp Gly Gln Ser Tyr Tyr Asn Ile
245 250 255

Lys Lys Gln Leu Gln Lys Pro Asp Val Asp Val Thr Leu Ala Ala Ala
260 265 270

Gln Ala Leu Ile Ala Leu Gly Lys Glu Glu Asp Ala Leu Pro Val Ile
275 280 285

Lys Lys Gln Ala Leu Glu Glu Arg Pro Arg Ala Leu Tyr Ala Leu Arg
290 295 300

His Leu Pro Ser Glu Ile Gly Ile Pro Ile Ala Leu Pro Ile Phe Leu
305 310 315 320

Lys Thr Lys Asn Ser Glu Ala Lys Leu Asn Val Ala Leu Ala Leu Leu
325 330 335

Glu Leu Gly Cys Asp Thr Pro Lys Leu Leu Glu Tyr Ile Thr Glu Arg
340 345 350

Leu Val Gln Pro His Tyr Asn Glu Thr Leu Ala Leu Ser Phe Ser Lys
355 360 365

Gly Arg Thr Leu Gln Asn Trp Lys Arg Val Asn Ile Ile Val Pro Gln
370 375 380

Asp Pro Gln Glu Arg Glu Arg Leu Leu Ser Thr Thr Arg Gly Leu Glu
385 390 395 400

Glu Gln Ile Leu Thr Phe Leu Phe Arg Leu Pro Lys Glu Ala Tyr Leu
405 410 415

Pro Cys Ile Tyr Lys Leu Leu Ala Ser Gln Lys Thr Gln Leu Ala Thr
420 425 430

Thr Ala Ile Ser Phe Leu Ser His Thr Ser His Gln Glu Ala Leu Asp
435 440 445

Leu Leu Phe Gln Ala Ala Lys Leu Pro Gly Glu Pro Ile Ile Arg Ala
450 455 460

Tyr Ala Asp Leu Ala Ile Tyr Asn Leu Thr Lys Asp Pro Glu Lys Lys
465 470 475 480

Arg Ser Leu His Asp Tyr Ala Lys Lys Leu Ile Gln Glu Thr Leu Leu
485 490 495

Phe Val Asp Thr Glu Asn Gln Arg Pro His Pro Ser Met Pro Tyr Leu
500 505 510

Arg Tyr Gln Val Thr Pro Glu Ser Arg Thr Lys Leu Met Leu Asp Ile
515 520 525

Leu Glu Thr Leu Ala Thr Ser Lys Ser Ser Glu Asp Ile Arg Leu Leu
530 535 540

Ile Gln Leu Met Thr Glu Gly Asp Ala Lys Asn Phe Pro Val Leu Ala
545 550 555 560

Gly Leu Leu Ile Lys Ile Val Glu
565

<210> 50
<211> 1707
<212> DNA
<213> Chlamydia pneumoniae

<400> 50
atgggactat tccatctaac tctctttgga cttttattgt gtagtcttcc catttctctt 60
gttgctaaat tccctgagtc tgtaggtcat aagatccttt atataagtac gcaatctaca 120
cagcaggcct tagcaacata tctggaagct ctagatgcct acggtgatca tgacttcttc 180
gttttaagaa aaatcggaga agactatctc aagcaaagca tccactcctc agatccgcaa 240
actagaaaaa gcaccatcat tggagcaggc ctggcgggat cttcagaagc cttggacgtg 300
ctctcccaag ctatggaaac tgcagacccc ctgcagcagc tactggtttt atcggcagtc 360
tcaggacatc ttgggaaaac ttctgacgac ttactgttta aagctttagc atctccctat 420
cctgtcatcc gcttagaagc cgcctataga cttgctaatt tgaagaacac taaagtcatt 480
gatcatctac attctttcat tcataagctt cccgaagaaa tccaatgcct atctgcggca 540
atattcctac gcttggagac tgaagaatct gatgcttata ttcgggatct cttagctgcc 600
aagaaaagcg cgattcggag tgccacagct ttgcagatcg gagaatacca acaaaaacgc 660
tttcttccga cacttaggaa tttgctaacg agtgcgtctc ctcaagatca agaagctatt 720
ctttatgctt tagggaagct taaggatggt cagagctact acaatataaa aaagcaattg 780
cagaagcctg atgtggatgt cactttagca gcagctcaag ctttaattgc tttggggaaa 840
gaagaggacg ctcttcccgt gataaaaaag caagcacttg aggagcggcc tcgagccctg 900
tatgccttac ggcatctacc ctctgagata gggattccga ttgccctgcc gatattccta 960
aaaactaaga acagcgaagc caagttgaat gtagctttag ctctcttaga gttagggtgt 1020
gacaccccta aactactgga atacattacc gaaaggcttg tccaaccaca ttataatgag 1080
actctagcct tgagtttctc taaggggcgt actttacaaa attggaagcg ggtgaacatc 1140
atagtccctc aagatcccca ggagagggaa aggttgctct ccacaacccg aggtcttgaa 1200
gagcagatcc ttacgtttct cttccgccta cctaaagaag cttacctccc ctgtatttat 1260
aagcttttgg cgagtcagaa aactcagctt gccactactg cgatttcttt tttaagtcac 1320
acctcacatc aggaagcctt agatctactt ttccaagctg cgaagcttcc tggagaacct 1380
atcatccgcg cctatgcaga tcttgctatt tataatctca ccaaagatcc tgaaaaaaaa 1440
cgttctctcc atgattatgc aaaaaagcta attcaggaaa ccttgttatt tgtggacacg 1500
gaaaaccaaa gaccccatcc cagcatgccc tatctacgtt atcaggtcac cccagaaagc 1560
cgtacgaagc tcatgttgga tattctagag acactagcca cctcgaagtc ttccgaagat 1620

```
atccgtttat tgatacaact gatgacggaa ggagatgcaa aaaatttccc agtccttgca   1680
ggcttactca taaaaattgt ggagtaa                                        1707

<210>   51
<211>   514
<212>   PRT
<213>   Chlamydia pneumoniae

<400>   51
Met Thr Ile Leu Arg Asn Phe Leu Thr Cys Ser Ala Leu Phe Leu Ala
1               5                   10                  15

Leu Pro Ala Ala Ala Gln Val Val Tyr Leu His Glu Ser Asp Gly Tyr
            20                  25                  30

Asn Gly Ala Ile Asn Asn Lys Ser Leu Glu Pro Lys Ile Thr Cys Tyr
        35                  40                  45

Pro Glu Gly Thr Ser Tyr Ile Phe Leu Asp Asp Val Arg Ile Ser Asn
    50                  55                  60

Val Lys His Asp Gln Glu Asp Ala Gly Val Phe Ile Asn Arg Ser Gly
65                  70                  75                  80

Asn Leu Phe Phe Met Gly Asn Arg Cys Asn Phe Thr Phe His Asn Leu
                85                  90                  95

Met Thr Glu Gly Phe Gly Ala Ala Ile Ser Asn Arg Val Gly Asp Thr
            100                 105                 110

Thr Leu Thr Leu Ser Asn Phe Ser Tyr Leu Ala Phe Thr Ser Ala Pro
        115                 120                 125

Leu Leu Pro Gln Gly Gln Gly Ala Ile Tyr Ser Leu Gly Ser Val Met
    130                 135                 140

Ile Glu Asn Ser Glu Glu Val Thr Phe Cys Gly Asn Tyr Ser Ser Trp
145                 150                 155                 160

Ser Gly Ala Ala Ile Tyr Thr Pro Tyr Leu Leu Gly Ser Lys Ala Ser
                165                 170                 175

Arg Pro Ser Val Asn Leu Ser Gly Asn Arg Tyr Leu Val Phe Arg Asp
            180                 185                 190

Asn Val Ser Gln Gly Tyr Gly Gly Ala Ile Ser Thr His Asn Leu Thr
        195                 200                 205

Leu Thr Thr Arg Gly Pro Ser Cys Phe Glu Asn Asn His Ala Tyr His
    210                 215                 220

Asp Val Asn Ser Asn Gly Gly Ala Ile Ala Ile Ala Pro Gly Gly Ser
225                 230                 235                 240

Ile Ser Ile Ser Val Lys Ser Gly Asp Leu Ile Phe Lys Gly Asn Thr
                245                 250                 255

Ala Ser Gln Asp Gly Asn Thr Ile His Asn Ser Ile His Leu Gln Ser
            260                 265                 270

Gly Ala Gln Phe Lys Asn Leu Arg Ala Val Ser Glu Ser Gly Val Tyr
```

```
        275                 280                 285

Phe Tyr Asp Pro Ile Ser His Ser Glu Ser His Lys Ile Thr Asp Leu
    290                 295                 300

Val Ile Asn Ala Pro Glu Gly Lys Glu Thr Tyr Glu Gly Thr Ile Ser
305                 310                 315                 320

Phe Ser Gly Leu Cys Leu Asp Asp His Glu Val Cys Ala Glu Asn Leu
                325                 330                 335

Thr Ser Thr Ile Leu Gln Asp Val Thr Leu Ala Gly Gly Thr Leu Ser
            340                 345                 350

Leu Ser Asp Gly Val Thr Leu Gln Leu His Ser Phe Lys Gln Glu Ala
        355                 360                 365

Ser Ser Thr Leu Thr Met Ser Pro Gly Thr Thr Leu Leu Cys Ser Gly
    370                 375                 380

Asp Ala Arg Val Gln Asn Leu His Ile Leu Ile Glu Asp Thr Asp Asn
385                 390                 395                 400

Phe Val Pro Val Arg Ile Arg Ala Glu Asp Lys Asp Ala Leu Val Ser
                405                 410                 415

Leu Glu Lys Leu Lys Val Ala Phe Glu Ala Tyr Trp Ser Val Tyr Asp
            420                 425                 430

Phe Pro Gln Phe Lys Glu Ala Phe Thr Ile Pro Leu Leu Glu Leu Leu
        435                 440                 445

Gly Pro Ser Phe Asp Ser Leu Leu Leu Gly Glu Thr Thr Leu Glu Arg
    450                 455                 460

Thr Gln Val Thr Thr Glu Asn Asp Ala Val Arg Gly Phe Trp Ser Leu
465                 470                 475                 480

Ser Trp Glu Glu Tyr Pro Pro Ser Leu Asp Lys Asp Arg Arg Ile Thr
                485                 490                 495

Pro Thr Lys Lys Thr Val Phe Leu Thr Trp Asn Pro Glu Ile Thr Ser
            500                 505                 510

Thr Pro


<210>  52
<211>  1545
<212>  DNA
<213>  Chlamydia pneumoniae

<400>  52
atgaccatac ttcgaaattt tcttacctgc tcggctttat tcctcgctct ccctgcagca    60
gcacaagttg tatatcttca tgaaagtgat ggttataacg gtgctatcaa taataaaagc   120
ttagaaccta aaattacctg ttatccagaa ggaacttctt acatctttct agatgacgtg   180
aggatttcca acgttaagca tgatcaagaa gatgctgggg tttttataaa tcgatctggg   240
aatctttttt tcatgggcaa ccgttgcaac ttcacttttc acaaccttat gaccgagggt   300
tttggcgctg ccatttcgaa ccgcgttgga gacaccactc tcactctctc taatttttct   360
tacttagcgt tcacctcagc acctctacta cctcaaggac aaggagcgat ttatagtctt   420
ggttccgtga tgatcgaaaa tagtgaggaa gtgactttct gtgggaacta ctcttcgtgg   480
```

```
agtggagctg cgatttatac tccctacctt ttaggttcta aggcgagtcg tccttcagta   540
aatctcagcg ggaaccgcta cctggtgttt agagacaatg tgagccaagg ttatggcggc   600
gccatatcta cccacaatct cacactcacg actcgaggac cttcgtgttt tgaaaataat   660
catgcttatc atgacgtgaa tagtaatgga ggagccattg ccattgctcc tggaggatcg   720
atctctatat ccgtgaaaag cggagatctc atcttcaaag gaaatacagc atcacaagac   780
ggaaatacaa tacacaactc catccatctg caatctggag cacagtttaa gaacctacgt   840
gctgtttcag aatccggagt ttatttctat gatcctataa gccatagcga gtcgcataaa   900
attacagatc ttgtaatcaa tgctcctgaa ggaaaggaaa cttatgaagg aacaattagc   960
ttctcaggac tatgcctgga tgatcatgaa gtttgtgcgg aaaatcttac ttccacaatc  1020
ctacaagatg tcacattagc aggaggaact ctctctctat cggatggggt taccttgcaa  1080
ctgcattctt ttaagcagga agcaagctct acgcttacta tgtctccagg aaccactctg  1140
ctctgctcag gagatgctcg ggttcagaat ctgcacatcc tgattgaaga taccgacaac  1200
tttgttcctg taaggattcg cgccgaggac aaggatgctc ttgtctcatt agaaaaactt  1260
aaagttgcct ttgaggctta ttggtccgtc tatgactttc ctcaatttaa ggaagccttt  1320
acgattcctc ttcttgaact tctagggcct tcttttgaca gtcttctcct aggggagacc  1380
actttggaga gaacccaagt cacaacagag aatgacgccg ttcgaggttt ctggtcccta  1440
agctgggaag agtacccccc ttctctggat aaagacagaa ggatcacacc aactaagaaa  1500
actgttttcc tcacttggaa tcctgagatc acttctacgc cataa                  1545
```

<210> 53
<211> 317
<212> PRT
<213> Chlamydia pneumoniae

<400> 53

```
Met Asn Arg Arg Lys Ala Arg Trp Val Val Ala Leu Phe Ala Met Thr
1               5                   10                  15

Ala Leu Ile Ser Val Gly Cys Cys Pro Trp Ser Gln Ala Lys Ser Arg
            20                  25                  30

Cys Ser Ile Asp Lys Tyr Ile Pro Val Val Asn Arg Leu Leu Glu Val
        35                  40                  45

Cys Gly Leu Pro Glu Ala Glu Asn Val Glu Asp Leu Ile Glu Ser Ser
    50                  55                  60

Ser Ala Trp Val Leu Thr Pro Glu Glu Arg Phe Ser Gly Glu Leu Val
65                  70                  75                  80

Ser Ile Cys Gln Val Lys Asp Glu His Ala Phe Tyr Asn Asp Leu Ser
                85                  90                  95

Leu Leu His Met Thr Gln Ala Val Pro Ser Tyr Ser Ala Thr Tyr Asp
            100                 105                 110

Cys Ala Val Val Phe Gly Gly Pro Leu Pro Ala Leu Arg Gln Arg Leu
        115                 120                 125

Asp Phe Leu Val Arg Glu Trp Gln Arg Gly Val Arg Phe Lys Lys Ile
    130                 135                 140

Val Phe Leu Cys Gly Glu Arg Gly Arg Tyr Gln Ser Ile Glu Glu Gln
145                 150                 155                 160

Glu His Phe Phe Asp Ser Arg Tyr Asn Pro Phe Pro Thr Glu Glu Asn
                165                 170                 175

Trp Glu Ser Gly Asn Arg Val Thr Pro Ser Ser Glu Glu Glu Ile Ala
            180                 185                 190
```

```
Lys Phe Val Trp Met Gln Met Leu Leu Pro Arg Ala Trp Arg Asp Ser
        195                 200                 205

Thr Ser Gly Val Arg Val Thr Phe Leu Leu Ala Lys Pro Glu Glu Asn
    210                 215                 220

Arg Val Val Ala Asn Arg Lys Asp Thr Leu Leu Leu Phe Arg Ser Tyr
225                 230                 235                 240

Gln Glu Ala Phe Pro Gly Arg Val Leu Phe Val Ser Ser Gln Pro Phe
                245                 250                 255

Ile Gly Leu Asp Ala Cys Arg Val Gly Gln Phe Phe Lys Gly Glu Ser
            260                 265                 270

Tyr Asp Leu Ala Gly Pro Gly Phe Ala Gln Gly Val Leu Lys Tyr His
        275                 280                 285

Trp Ala Pro Arg Ile Cys Leu His Thr Leu Ala Glu Trp Leu Lys Glu
    290                 295                 300

Thr Asn Gly Cys Leu Asn Ile Ser Glu Gly Cys Phe Gly
305                 310                 315

<210>   54
<211>   954
<212>   DNA
<213>   Chlamydia pneumoniae

<400>   54
atgaatagaa gaaaagcaag atgggtagtg gcattgttcg caatgacggc gctcatttct     60
gttgggtgtt gtccttggtc acaagcgaaa tcaagatgtt ctattgataa gtatattcct    120
gtagtcaatc gtttactaga agtttgtgga cttcctgaag ctgagaatgt tgaggattta    180
atcgagtcct cgtctgcttg ggtactgact cctgaagaac gtttttctgg agagttagtc    240
tctatctgtc aggttaaaga tgagcatgct ttctataacg atttgtcttt attacatatg    300
actcaggctg tgccttcgta ttctgcaacg tatgattgtg ctgtagtttt tggcgggcct    360
ttgccagcgc tacgtcagcg cttagatttt ttggtgcgag agtggcagcg tggcgtgcgc    420
tttaagaaaa tcgtttttct atgtggagag cgagggcgct atcagtctat tgaagaacaa    480
gagcatttct ttgattctcg gtacaatcct ttccctactg aagagaactg ggaatctggt    540
aaccgagtta ctccctcttc tgaagaagag attgccaaat ttgtttggat gcaaatgctt    600
ttacctagag catggcgaga tagtacttca ggagtcagag tgacatttct tctagcaaag    660
ccagaggaaa atcgtgtggt tgcgaatcgt aaggacacct tacttttatt ccgttcttat    720
caagaagcgt ttccgggacg cgtgttattt gtaagtagtc aacccttttat cggtttagat    780
gcttgcaggg tcgggcagtt tttcaaaggg gaaagctatg atcttgctgg acctggattt    840
gctcaaggag tcttgaagta tcattgggct ccaaggattt gtctacatac tttagcggaa    900
tggttaaagg aaacgaacgg ctgcttaaat atttcagagg gttgttttgg atga          954

<210>   55
<211>   258
<212>   PRT
<213>   Chlamydia pneumoniae

<400>   55
Met Asn Arg Arg Trp Asn Leu Val Leu Ala Thr Val Ala Leu Ala Leu
1               5                   10                  15

Ser Val Ala Ser Cys Asp Val Arg Ser Lys Asp Lys Asp Lys Asp Gln
            20                  25                  30

Gly Ser Leu Val Glu Tyr Lys Asp Asn Lys Asp Thr Asn Asp Ile Glu
        35                  40                  45
```

Leu Ser Asp Asn Gln Lys Leu Ser Arg Thr Phe Gly His Leu Leu Ala
    50                  55                  60

Arg Gln Leu Arg Lys Ser Glu Asp Met Phe Phe Asp Ile Ala Glu Val
65                  70                  75                  80

Ala Lys Gly Leu Gln Ala Glu Leu Val Cys Lys Ser Ala Pro Leu Thr
                85                  90                  95

Glu Thr Glu Tyr Glu Glu Lys Met Ala Glu Val Gln Lys Leu Val Phe
            100                 105                 110

Glu Lys Lys Ser Lys Glu Asn Leu Ser Leu Ala Glu Lys Phe Leu Lys
        115                 120                 125

Glu Asn Ser Lys Asn Ala Gly Val Val Glu Val Gln Pro Ser Lys Leu
    130                 135                 140

Gln Tyr Lys Ile Ile Lys Glu Gly Ala Gly Lys Ala Ile Ser Gly Lys
145                 150                 155                 160

Pro Ser Ala Leu Leu His Tyr Lys Gly Ser Phe Ile Asn Gly Gln Val
                165                 170                 175

Phe Ser Ser Ser Glu Gly Asn Asn Glu Pro Ile Leu Leu Pro Leu Gly
            180                 185                 190

Gln Thr Ile Pro Gly Phe Ala Leu Gly Met Gln Gly Met Lys Glu Gly
        195                 200                 205

Glu Thr Arg Val Leu Tyr Ile His Pro Asp Leu Ala Tyr Gly Thr Ala
    210                 215                 220

Gly Gln Leu Pro Pro Asn Ser Leu Leu Ile Phe Glu Ile Asn Leu Ile
225                 230                 235                 240

Gln Ala Ser Ala Asp Glu Val Ala Ala Val Pro Gln Glu Gly Asn Gln
                245                 250                 255

Gly Glu

<210> 56
<211> 777
<212> DNA
<213> Chlamydia pneumoniae

<400> 56
atgaacagac ggtggaattt agttttagca acagtagctc tggcactctc cgtcgcttct 60
tgtgacgtac ggtctaagga taaagacaag gatcaggggt cgttagtgga atataaagat 120
aacaaagata ccaatgacat agaattatcc gataatcaaa agttatccag aacatttggt 180
catttattag cacgccaatt acgcaagtca gaagatatgt tttttgatat tgcagaagtg 240
gctaaggggt tgcaggcgga attggtttgt aaaagtgctc ctttaacaga aacagagtat 300
gaagaaaaaa tggctgaagt acagaagttg gtttttgaaa aaaaatcaaa agaaaatctt 360
tcattggcag aaaaattctt aaaagaaaat agcaagaacg ctggtgttgt tgaagtgcaa 420
ccaagtaaat tgcaatacaa aattattaaa gaaggtgcag ggaaagcaat ttcaggtaaa 480
ccttcagctc tattgcacta caagggttcc ttcatcaatg gccaagtatt tagcagttca 540
gaaggcaaca atgagcctat cttgcttcct ctaggccaaa caattcctgg ttttgcttta 600
ggtatgcagg gcatgaaaga aggagaaact cgagttctct acatccatcc tgatcttgct 660
tacggaaccg caggacaact tcctccaaac tctttattaa tttttgaaat taacttgatt 720

caggcttcag cagatgaagt tgctgctgta ccccaagaag gaaatcaagg tgaatga 777

<210> 57
<211> 318
<212> PRT
<213> Chlamydia pneumoniae

<400> 57
Met Lys Phe Leu Leu Tyr Val Pro Leu Leu Leu Val Leu Val Ser Thr
1 5 10 15

Gly Cys Asp Ala Lys Pro Val Ser Phe Glu Pro Phe Ser Gly Lys Leu
20 25 30

Ser Thr Gln Arg Phe Glu Pro Gln His Ser Ala Glu Glu Tyr Phe Ser
35 40 45

Gln Gly Gln Glu Phe Leu Lys Lys Gly Asn Phe Arg Lys Ala Leu Leu
50 55 60

Cys Phe Gly Ile Ile Thr His His Phe Pro Arg Asp Ile Leu Arg Asn
65 70 75 80

Gln Ala Gln Tyr Leu Ile Gly Val Cys Tyr Phe Thr Gln Asp His Pro
85 90 95

Asp Leu Ala Asp Lys Ala Phe Ala Ser Tyr Leu Gln Leu Pro Asp Ala
100 105 110

Glu Tyr Ser Glu Glu Leu Phe Gln Met Lys Tyr Ala Ile Ala Gln Arg
115 120 125

Phe Ala Gln Gly Lys Arg Lys Arg Ile Cys Arg Leu Glu Gly Phe Pro
130 135 140

Lys Leu Met Asn Ala Asp Glu Asp Ala Leu Arg Ile Tyr Asp Glu Ile
145 150 155 160

Leu Thr Ala Phe Pro Ser Lys Asp Leu Gly Ala Gln Ala Leu Tyr Ser
165 170 175

Lys Ala Ala Leu Leu Ile Val Lys Asn Asp Leu Thr Glu Ala Thr Lys
180 185 190

Thr Leu Lys Lys Leu Thr Leu Gln Phe Pro Leu His Ile Leu Ser Ser
195 200 205

Glu Ala Phe Val Arg Leu Ser Glu Ile Tyr Leu Gln Gln Ala Lys Lys
210 215 220

Glu Pro His Asn Leu Gln Tyr Leu His Phe Ala Lys Leu Asn Glu Glu
225 230 235 240

Ala Met Lys Lys Gln His Pro Asn His Pro Leu Asn Glu Val Val Ser
245 250 255

Ala Asn Val Gly Ala Met Arg Glu His Tyr Ala Arg Gly Leu Tyr Ala
260 265 270

Thr Gly Arg Phe Tyr Glu Lys Lys Lys Lys Ala Glu Ala Ala Asn Ile
275 280 285

Tyr Tyr Arg Thr Ala Ile Thr Asn Tyr Pro Asp Thr Leu Leu Val Ala
290 295 300

Lys Cys Gln Lys Arg Leu Asp Arg Ile Ser Lys His Thr Ser
305 310 315

<210> 58
<211> 957
<212> DNA
<213> Chlamydia pneumoniae

<400> 58
atgaaatttc tattatacgt tccacttctt cttgttctcg tatctacggg gtgcgatgca 60
aaacctgttt cttttgagcc cttttcagga aagctttcca cccagcgttt tgagcctcag 120
cactctgctg aagaatattt ttctcaggga caggaattct taaaaaaagg aaatttcaga 180
aaagctttac tatgctttgg aatcattacg catcacttcc ctagggacat cttgcgtaat 240
caagcacagt atcttatagg agtctgttac ttcacgcagg atcacccaga tttagcagac 300
aaggcatttg catcttactt acaacttcct gatgcggagt actctgaaga gttgttccag 360
atgaaatatg cgattgctca aagatttgct caagggaagc gtaaacggat ttgtcgatta 420
gagggcttcc caaaactaat gaatgctgat gaagatgcgc tacgcattta tgacgagatt 480
ctaacagcgt ttcctagtaa agacttagga gctcaggccc tctatagtaa agctgcgtta 540
cttattgtaa aaaacgatct tacagaagcc accaaaacct taaaaaaact cacgttacaa 600
tttcctctac atattttatc ttcagaggcc tttgtacgtt tatcggaaat ctatttacag 660
caagctaaga aagagcctca caatcttcaa tatcttcatt ttgcaaagct taatgaagag 720
gcaatgaaaa agcagcatcc taaccatcct ctgaatgagg ttgtttctgc taatgttgga 780
gctatgcggg aacattatgc tcgaggtttg tatgccacag gtcgtttcta tgagaagaag 840
aaaaaagccg aggctgcgaa tatctattac cgcactgcga ttacaaacta cccagacact 900
ttattagtgg ctaaatgtca aaagcgtcta gatagaatat ctaagcatac ttcctaa 957

<210> 59
<211> 389
<212> PRT
<213> Chlamydia pneumoniae

<400> 59
Met Lys Lys Leu Leu Lys Ser Ala Leu Leu Ser Ala Ala Phe Ala Gly
1 5 10 15

Ser Val Gly Ser Leu Gln Ala Leu Pro Val Gly Asn Pro Ser Asp Pro
20 25 30

Ser Leu Leu Ile Asp Gly Thr Ile Trp Glu Gly Ala Ala Gly Asp Pro
35 40 45

Cys Asp Pro Cys Ala Thr Trp Cys Asp Ala Ile Ser Leu Arg Ala Gly
50 55 60

Phe Tyr Gly Asp Tyr Val Phe Asp Arg Ile Leu Lys Val Asp Ala Pro
65 70 75 80

Lys Thr Phe Ser Met Gly Ala Lys Pro Thr Gly Ser Ala Ala Ala Asn
85 90 95

Tyr Thr Thr Ala Val Asp Arg Pro Asn Pro Ala Tyr Asn Lys His Leu
100 105 110

His Asp Ala Glu Trp Phe Thr Asn Ala Gly Phe Ile Ala Leu Asn Ile
115 120 125

Trp Asp Arg Phe Asp Val Phe Cys Thr Leu Gly Ala Ser Asn Gly Tyr

```
        130                 135                 140

    Ile Arg Gly Asn Ser Thr Ala Phe Asn Leu Val Gly Leu Phe Gly Val
    145                 150                 155                 160

    Lys Gly Thr Thr Val Asn Ala Asn Glu Leu Pro Asn Val Ser Leu Ser
                    165                 170                 175

    Asn Gly Val Val Glu Leu Tyr Thr Asp Thr Ser Phe Ser Trp Ser Val
                180                 185                 190

    Gly Ala Arg Gly Ala Leu Trp Glu Cys Gly Cys Ala Thr Leu Gly Ala
            195                 200                 205

    Glu Phe Gln Tyr Ala Gln Ser Lys Pro Lys Val Glu Glu Leu Asn Val
        210                 215                 220

    Ile Cys Asn Val Ser Gln Phe Ser Val Asn Lys Pro Lys Gly Tyr Lys
    225                 230                 235                 240

    Gly Val Ala Phe Pro Leu Pro Thr Asp Ala Gly Val Ala Thr Ala Thr
                    245                 250                 255

    Gly Thr Lys Ser Ala Thr Ile Asn Tyr His Glu Trp Gln Val Gly Ala
                260                 265                 270

    Ser Leu Ser Tyr Arg Leu Asn Ser Leu Val Pro Tyr Ile Gly Val Gln
            275                 280                 285

    Trp Ser Arg Ala Thr Phe Asp Ala Asp Asn Ile Arg Ile Ala Gln Pro
        290                 295                 300

    Lys Leu Pro Thr Ala Val Leu Asn Leu Thr Ala Trp Asn Pro Ser Leu
    305                 310                 315                 320

    Leu Gly Asn Ala Thr Ala Leu Ser Thr Thr Asp Ser Phe Ser Asp Phe
                    325                 330                 335

    Met Gln Ile Val Ser Cys Gln Ile Asn Lys Phe Lys Ser Arg Lys Ala
                340                 345                 350

    Cys Gly Val Thr Val Gly Ala Thr Leu Val Asp Ala Asp Lys Trp Ser
            355                 360                 365

    Leu Thr Ala Glu Ala Arg Leu Ile Asn Glu Arg Ala Ala His Val Ser
        370                 375                 380

    Gly Gln Phe Arg Phe
    385

    <210>   60
    <211>   1170
    <212>   DNA
    <213>   Chlamydia pneumoniae

    <400>   60
    atgaaaaaac tcttaaagtc ggcgttatta tccgccgcat ttgctggttc tgttggctcc     60
    ttacaagcct tgcctgtagg gaacccttct gatccaagct tattaattga tggtacaata    120
    tgggaaggtg ctgcaggaga tccttgcgat ccttgcgcta cttggtgcga cgctattagc    180
    ttacgtgctg gattttacgg agactatgtt ttcgaccgta tcttaaaagt agatgcacct    240
    aaaacatttt ctatgggagc caagcctact ggatccgctg ctgcaaacta tactactgcc    300
```

```
gtagatagac ctaacccggc ctacaataag catttacacg atgcagagtg gttcactaat   360
gcaggcttca ttgccttaaa catttgggat cgctttgatg ttttctgtac tttaggagct   420
tctaatggtt acattagagg aaactctaca gcgttcaatc tcgttggttt attcggagtt   480
aaaggtacta ctgtaaatgc aaatgaacta ccaaacgttt ctttaagtaa cggagttgtt   540
gaactttaca cagacacctc tttctcttgg agcgtaggcg ctcgtggagc cttatgggaa   600
tgcggttgtg caactttggg agctgaattc caatatgcac agtccaaacc taaagttgaa   660
gaacttaatg tgatctgtaa cgtatcgcaa ttctctgtaa acaaacccaa gggctataaa   720
ggcgttgctt tcccccttgcc aacagacgct ggcgtagcaa cagctactgg aacaaagtct   780
gcgaccatca attatcatga atggcaagta ggagcctctc tatcttacag actaaactct   840
ttagtgccat acattggagt acaatggtct cgagcaactt ttgatgctga taacatccgc   900
attgctcagc caaaactacc tacagctgtt ttaaacttaa ctgcatggaa cccttcttta   960
ctaggaaatg ccacagcatt gtctactact gattcgttct cagacttcat gcaaattgtt   1020
tcctgtcaga tcaacaagtt taaatctaga aaagcttgtg gagttactgt aggagctact   1080
ttagttgatg ctgataaatg gtcacttact gcagaagctc gtttaattaa cgagagagct   1140
gctcacgtat ctggtcagtt cagattctaa                                     1170
```

<210> 61
<211> 604
<212> PRT
<213> Chlamydia pneumoniae

<400> 61

```
Met Lys His Thr Phe Thr Lys Arg Val Leu Phe Phe Phe Phe Leu Val
1               5                   10                  15

Ile Pro Ile Pro Leu Leu Leu Asn Leu Met Val Val Gly Phe Phe Ser
            20                  25                  30

Phe Ser Ala Ala Lys Ala Asn Leu Val Gln Val Leu His Thr Arg Ala
        35                  40                  45

Thr Asn Leu Ser Ile Glu Phe Glu Lys Lys Leu Thr Ile His Lys Leu
    50                  55                  60

Phe Leu Asp Arg Leu Ala Asn Thr Leu Ala Leu Lys Ser Tyr Ala Ser
65                  70                  75                  80

Pro Ser Ala Glu Pro Tyr Ala Gln Ala Tyr Asn Glu Met Met Ala Leu
                85                  90                  95

Ser Asn Thr Asp Phe Ser Leu Cys Leu Ile Asp Pro Phe Asp Gly Ser
            100                 105                 110

Val Arg Thr Lys Asn Pro Gly Asp Pro Phe Ile Arg Tyr Leu Lys Gln
        115                 120                 125

His Pro Glu Met Lys Lys Lys Leu Ser Ala Ala Val Gly Lys Ala Phe
    130                 135                 140

Leu Leu Thr Ile Pro Gly Lys Pro Leu Leu His Tyr Leu Ile Leu Val
145                 150                 155                 160

Glu Asp Val Ala Ser Trp Asp Ser Thr Thr Thr Ser Gly Leu Leu Val
                165                 170                 175

Ser Phe Tyr Pro Met Ser Phe Leu Gln Lys Asp Leu Phe Gln Ser Leu
            180                 185                 190

His Ile Thr Lys Gly Asn Ile Cys Leu Val Asn Lys Tyr Gly Glu Val
        195                 200                 205
```

```
Leu Phe Cys Ala Gln Asp Ser Glu Ser Ser Phe Val Phe Ser Leu Asp
    210                 215                 220

Leu Pro Asn Leu Pro Gln Phe Gln Ala Arg Ser Pro Ser Ala Ile Glu
225                 230                 235                 240

Ile Glu Lys Ala Ser Gly Ile Leu Gly Gly Glu Asn Leu Ile Thr Val
                245                 250                 255

Ser Ile Asn Lys Lys Arg Tyr Leu Gly Leu Val Leu Asn Lys Ile Pro
            260                 265                 270

Ile Gln Gly Thr Tyr Thr Leu Ser Leu Val Pro Val Ser Asp Leu Ile
        275                 280                 285

Gln Ser Ala Leu Lys Val Pro Leu Asn Ile Cys Phe Phe Tyr Val Leu
    290                 295                 300

Ala Phe Leu Leu Met Trp Trp Ile Phe Ser Lys Ile Asn Thr Lys Leu
305                 310                 315                 320

Asn Lys Pro Leu Gln Glu Leu Thr Phe Cys Met Glu Ala Ala Trp Arg
                325                 330                 335

Gly Asn His Asn Val Arg Phe Glu Pro Gln Pro Tyr Gly Tyr Glu Phe
            340                 345                 350

Asn Glu Leu Gly Asn Ile Phe Asn Cys Thr Leu Leu Leu Leu Leu Asn
        355                 360                 365

Ser Ile Glu Lys Ala Asp Ile Asp Tyr His Ser Gly Glu Lys Leu Gln
    370                 375                 380

Lys Glu Leu Gly Ile Leu Ser Ser Leu Gln Ser Ala Leu Leu Ser Pro
385                 390                 395                 400

Asp Phe Pro Thr Phe Pro Lys Val Thr Phe Ser Ser Gln His Leu Arg
                405                 410                 415

Arg Arg Gln Leu Ser Gly His Phe Asn Gly Trp Thr Val Gln Asp Gly
            420                 425                 430

Gly Asp Thr Leu Leu Gly Ile Ile Gly Leu Ala Gly Asp Ile Gly Leu
        435                 440                 445

Pro Ser Tyr Leu Tyr Ala Leu Ser Ala Arg Ser Leu Phe Leu Ala Tyr
    450                 455                 460

Ala Ser Ser Asp Val Ser Leu Gln Lys Ile Ser Lys Asp Thr Ala Asp
465                 470                 475                 480

Ser Phe Ser Lys Thr Thr Glu Gly Asn Glu Ala Val Val Ala Met Thr
                485                 490                 495

Phe Ile Lys Tyr Val Glu Lys Asp Arg Ser Leu Glu Leu Leu Ser Leu
            500                 505                 510

Ser Glu Gly Ala Pro Thr Met Phe Leu Gln Arg Gly Glu Ser Phe Val
        515                 520                 525

Arg Leu Pro Leu Glu Thr His Gln Ala Leu Gln Pro Gly Asp Arg Leu
```

```
    530                 535                 540

Ile Cys Leu Thr Gly Gly Glu Asp Ile Leu Lys Tyr Phe Ser Gln Leu
545                 550                 555                 560

Pro Ile Glu Glu Leu Leu Lys Asp Pro Leu Asn Pro Leu Asn Thr Glu
                565                 570                 575

Asn Leu Ile Asp Ser Leu Thr Met Met Leu Asn Asn Glu Thr Glu His
            580                 585                 590

Ser Ala Asp Gly Thr Leu Thr Ile Leu Ser Phe Ser
        595                 600

<210>   62
<211>   1815
<212>   DNA
<213>   Chlamydia pneumoniae

<400>   62
atgaaacata cctttaccaa gcgtgttcta ttttttttct ttttagtgat tcccattccc    60
ctactcctca atcttatggt cgtaggtttt ttctcatttt ctgccgctaa agcaaattta   120
gtacaggtcc tccatacccg tgctacgaac ttaagtatag aattcgaaaa aaaactgacg   180
atacacaagc ttttcctcga tagacttgcc aacacattag ccttaaaatc ctatgcatct   240
ccttctgcag agccctatgc acaggcatac aatgagatga tggcactctc caatacagac   300
ttttccttat gccttataga tccctttgat ggatctgtaa ggacgaaaaa tcctggagac   360
cctttcattc gctatctaaa acagcatcct gaaatgaaga aaaagctatc cgcagctgta   420
gggaaagcct ttttattgac cattccaggt aaaccacttt tacattatct tattctagtt   480
gaagatgtcg catcttggga ttctacaacg acttcaggac tgcttgtaag tttctatccc   540
atgtcttttt tacagaaaga tttattccaa tccttacaca tcaccaaagg aaatatctgc   600
cttgtaaata agtatggcga ggtcctcttc tgtgctcagg acagtgaatc ttcttttgta   660
ttttctctag atctccctaa tttaccgcaa ttccaagcaa gaagcccctc tgccatagaa   720
attgagaaag cttctggaat tcttggtggg gagaacctaa tcacagtgag tatcaacaag   780
aaacgctacc taggattggt actgaataaa attcctatcc aagggaccta cactctatct   840
ttagttccag tttctgatct catccaatcc gccttgaaag ttcctctcaa tatttgtttt   900
ttctatgtac ttgctttcct cctcatgtgg tggattttct ctaagatcaa caccaaactt   960
aacaagcctc ttcaagaact gaccttctgt atggaagctg cctggcgagg aaaccataac  1020
gtgaggtttg aaccccagcc ttacggttat gaattcaatg aactaggaaa tattttcaat  1080
tgcactctcc tactcttatt gaattccatt gagaaagcag atatcgatta ccattcaggc  1140
gaaaaattac aaaaagaatt agggatttta tcttcactac aaagtgcgtt actaagtccg  1200
gatttcccta cgttccctaa agttaccttt agttcccaac atctccggag aaggcaactt  1260
tccggtcatt ttaatggttg gacagttcaa gatggtggcg ataccctttt agggatcata  1320
gggctcgctg gcgatattgg tcttccttcc tatctctatg ctttatccgc acggagtctt  1380
tttcttgcct atgcttcctc ggacgtttcg ttacaaaaaa tcagcaagga tactgccgac  1440
agcttctcaa aaacaacaga aggcaatgag gctgtagttg ctatgacttt cattaaatat  1500
gtagaaaaag atcgatctct agagctcctc tcgttaagcg agggagctcc taccatgttt  1560
ctacaacgag gagaatcttt cgtacgtctc cccttagaga ctcaccaagc tctacagcct  1620
ggagatcggt tgatctgcct cactggagga gaagacatcc tcaagtactt ttctcagctt  1680
cctattgaag agctcttaaa agatcctta aaccctctaa atacagagaa tcttattgat  1740
tctctaacca tgatgttaaa caacgaaacc gaacattctg cagatggaac tctgaccatc  1800
ctttcatttt cataa                                                   1815

<210>   63
<211>   356
<212>   PRT
<213>   Chlamydia pneumoniae

<400>   63
Met Lys Asp Leu Gly Thr Leu Gly Gly Thr Ser Ser Thr Ala Lys Thr
1               5                   10                  15
```

```
Val Ser Pro Asp Gly Lys Val Ile Met Gly Arg Ser Gln Ile Ala Asp
            20                  25                  30

Gly Ser Trp His Ala Phe Met Cys His Thr Asp Phe Ser Ser Asn Asn
        35                  40                  45

Val Leu Phe Asp Leu Asp Asn Thr Tyr Lys Thr Leu Arg Glu Asn Gly
    50                  55                  60

Arg Gln Leu Asn Ser Ile Phe Asn Leu Gln Asn Met Met Leu Gln Arg
65                  70                  75                  80

Ala Ser Asp His Glu Phe Thr Glu Phe Gly Arg Ser Asn Ile Ala Leu
                85                  90                  95

Gly Ala Gly Leu Tyr Val Asn Ala Leu Gln Asn Leu Pro Ser Asn Leu
            100                 105                 110

Ala Ala Gln Tyr Phe Gly Ile Ala Tyr Lys Ile Arg Pro Lys Tyr Arg
        115                 120                 125

Leu Gly Val Phe Leu Asp His Asn Phe Ser Ser His Val Pro Asn Asn
    130                 135                 140

Phe Asn Val Ser His Asn Arg Leu Trp Met Gly Ala Phe Ile Gly Trp
145                 150                 155                 160

Gln Asp Ser Asp Ala Leu Gly Ser Ser Val Lys Val Ser Phe Gly Tyr
                165                 170                 175

Gly Lys Gln Lys Ala Thr Ile Thr Arg Glu Gln Leu Glu Asn Thr Glu
            180                 185                 190

Ala Gly Ser Gly Glu Ser His Phe Glu Gly Val Ala Ala Gln Ile Glu
        195                 200                 205

Gly Arg Tyr Gly Lys Ser Leu Gly Gly His Val Arg Val Gln Pro Phe
    210                 215                 220

Leu Gly Leu Gln Phe Val His Ile Thr Arg Lys Glu Tyr Thr Glu Asn
225                 230                 235                 240

Ala Val Gln Phe Pro Val His Tyr Asp Pro Ile Asp Tyr Ser Thr Gly
                245                 250                 255

Val Val Tyr Leu Gly Ile Gly Ser His Ile Ala Leu Val Asp Ser Leu
            260                 265                 270

His Val Gly Thr Arg Met Gly Met Glu Gln Asn Phe Ala Ala His Thr
        275                 280                 285

Asp Arg Phe Ser Gly Ser Ile Ala Ser Ile Gly Asn Phe Val Phe Glu
    290                 295                 300

Lys Leu Asp Val Thr His Thr Arg Ala Phe Ala Glu Met Arg Val Asn
305                 310                 315                 320

Tyr Glu Leu Pro Tyr Leu Gln Ser Leu Asn Leu Ile Leu Arg Val Asn
                325                 330                 335

Gln Gln Pro Leu Gln Gly Val Met Gly Phe Ser Ser Asp Leu Arg Tyr
```

```
            340                 345                 350

Ala Leu Gly Phe
        355

<210>   64
<211>   1071
<212>   DNA
<213>   Chlamydia pneumoniae

<400>   64
atgaaagatt tggggactct tgggggtacc tcttctacag caaaaacagt gtccccagat    60
ggtaaagtga tcatgggtag atcacaaatt gctgatggca gttggcacgc atttatgtgt   120
catacggatt tctcctctaa taatgtactc tttgatctcg ataatacgta taaaactcta   180
agagaaaatg gccgtcagct aaattccata ttcaacctac aaaatatgat gttacagaga   240
gcctcagatc atgagttcac agagtttgga aggagtaaca tcgctcttgg tgccgggctt   300
tatgtgaatg ccttgcagaa tctccctagc aatttagcag cacaatattt tggaatcgca   360
tacaaaatac gtcctaaata tcgtttgggg gtgtttttgg accataattt cagctcccac   420
gttcctaata attttaacgt aagccacaat agactctgga tgggagcctt tattggatgg   480
caggattctg atgctctagg atctagtgtc aaggtgtctt tcggatatgg aaaacaaaaa   540
gccacgatta caagagagca attagagaat acagaagccg ggagtgggga gagccatttt   600
gaaggggtcg ctgctcagat agaagggcgg tatggtaaga gcctcggagg acatgtcagg   660
gtccagcctt tcctaggact gcagtttgtc cacattacaa ggaaagaata taccgaaaat   720
gcagtgcaat ttcctgtaca ctatgatcct atagactatt ctacaggtgt agtgtattta   780
ggaattggat ctcatattgc acttgtagat tctttacatg taggcacacg catgggaatg   840
gagcaaaact ttgcagccca tacggacagg ttctcaggat ctatagcgtc tattggaaac   900
tttgtgtttg aaaagcttga tgtgactcac acaagggcat ttgcggaaat gcgtgtcaac   960
tatgagcttc cctatctaca gtctctgaat cttattctac gagttaatca acagcctcta  1020
caaggggtta tgggattttc cagtgatctt aggtatgcct taggattcta a           1071

<210>   65
<211>   112
<212>   PRT
<213>   Chlamydia pneumoniae

<400>   65
Met Thr Ala Val Leu Ile Leu Thr Ser Phe Pro Ser Glu Glu Ser Ala
1               5                   10                  15

Arg Ser Leu Ala Arg His Leu Ile Thr Glu Arg Leu Ala Ser Cys Val
            20                  25                  30

His Val Phe Pro Lys Gly Thr Ser Thr Tyr Leu Trp Glu Gly Lys Leu
        35                  40                  45

Cys Glu Ser Glu Glu His His Ile Gln Ile Lys Ser Ile Asp Ile Arg
    50                  55                  60

Phe Ser Glu Ile Cys Leu Ala Ile Gln Glu Phe Ser Gly Tyr Glu Val
65                  70                  75                  80

Pro Glu Val Leu Leu Phe Pro Ile Glu Asn Gly Asp Pro Arg Tyr Leu
                85                  90                  95

Asn Trp Leu Thr Ile Leu Ser Tyr Pro Glu Lys Pro Pro Leu Ser Asp
            100                 105                 110


<210>   66
<211>   339
<212>   DNA
```

<213> Chlamydia pneumoniae

<400> 66
```
atgactgctg ttcttattct tacatctttc ccttcggagg aaagtgctcg ctccttagct   60
agacatctga ttacagagcg tcttgcttcc tgtgtgcatg tattccctaa aggcacatcg  120
acatatctat gggaaggcaa gctatgtgag tctgaagaac atcatataca aatcaaatcg  180
atagacatac gcttctcgga aatttgtctt gctattcagg agttctctgg ctatgaggtt  240
cctgaagtct tactatttcc tattgaaaat ggggatccga ggtacttgaa ttggttaacg  300
attctcagct atccagagaa gcctccgctt tcagattag                          339
```

<210> 67
<211> 344
<212> PRT
<213> Chlamydia pneumoniae

<400> 67
```
Met Asn Ser Lys Met Leu Lys His Leu Arg Leu Ala Thr Leu Ser Phe
1               5                   10                  15

Ser Met Phe Phe Gly Ile Val Ser Ser Pro Ala Val Tyr Ala Leu Gly
            20                  25                  30

Ala Gly Asn Pro Ala Ala Pro Val Leu Pro Gly Val Asn Pro Glu Gln
        35                  40                  45

Thr Gly Trp Cys Ala Phe Gln Leu Cys Asn Ser Tyr Asp Leu Phe Ala
    50                  55                  60

Ala Leu Ala Gly Ser Leu Lys Phe Gly Phe Tyr Gly Asp Tyr Val Phe
65                  70                  75                  80

Ser Glu Ser Ala His Ile Thr Asn Val Pro Val Ile Thr Ser Val Thr
                85                  90                  95

Thr Ser Gly Thr Gly Thr Thr Pro Thr Ile Thr Ser Thr Thr Lys Asn
            100                 105                 110

Val Asp Phe Asp Leu Asn Asn Ser Ser Ile Ser Ser Ser Cys Val Phe
        115                 120                 125

Ala Thr Ile Ala Leu Gln Glu Thr Ser Pro Ala Ala Ile Pro Leu Leu
    130                 135                 140

Asp Ile Ala Phe Thr Ala Arg Val Gly Gly Leu Lys Gln Tyr Tyr Arg
145                 150                 155                 160

Leu Pro Leu Asn Ala Tyr Arg Asp Phe Thr Ser Asn Pro Leu Asn Ala
                165                 170                 175

Glu Ser Glu Val Thr Asp Gly Leu Ile Glu Val Gln Ser Asp Tyr Gly
            180                 185                 190

Ile Val Trp Gly Leu Ser Leu Gln Lys Val Leu Trp Lys Asp Gly Val
        195                 200                 205

Ser Phe Val Gly Val Ser Ala Asp Tyr Arg His Gly Ser Ser Pro Ile
    210                 215                 220

Asn Tyr Ile Ile Val Tyr Asn Lys Ala Asn Pro Glu Ile Tyr Phe Asp
225                 230                 235                 240
```

```
Ala Thr Asp Gly Asn Leu Ser Tyr Lys Glu Trp Ser Ala Ser Ile Gly
                245                 250                 255

Ile Ser Thr Tyr Leu Asn Asp Tyr Val Leu Pro Tyr Ala Ser Val Ser
            260                 265                 270

Ile Gly Asn Thr Ser Arg Lys Ala Pro Ser Asp Ser Phe Thr Glu Leu
        275                 280                 285

Glu Lys Gln Phe Thr Asn Phe Lys Phe Lys Ile Arg Lys Ile Thr Asn
    290                 295                 300

Phe Asp Arg Val Asn Phe Cys Phe Gly Thr Thr Cys Cys Ile Ser Asn
305                 310                 315                 320

Asn Phe Tyr Tyr Ser Val Glu Gly Arg Trp Gly Tyr Gln Arg Ala Ile
                325                 330                 335

Asn Ile Thr Ser Gly Leu Gln Phe
            340
```

<210> 68
<211> 1035
<212> DNA
<213> Chlamydia pneumoniae

<400> 68

```
atgaatagca agatgctaaa acatttacgt ttagcaaccc tttccttctc tatgttcttc     60
gggattgtat cttctcccgc agtatatgcc ctaggggctg gaaaccctgc agctccagta    120
ctcccaggtg tgaatcctga gcaaacggga tggtgtgcct tccaactttg taatagttac    180
gatctttttg ctgctcttgc aggaagcctc aaatttgggt tctatggaga ttatgtcttc    240
tcagaaagtg cccatattac caatgtccct gtcattacct ccgttacgac ttcaggcaca    300
ggaacaacgc caaccattac ctctacaact aaaaacgtag actttgatct taacaacagc    360
tccatcagct cgagctgtgt ttttgcaacc atagctctac aggaaacatc cccagctgcc    420
attccccttt tagatatagc cttcactgca cgtgtcggag gacttaagca gtactaccgc    480
ctccctctca atgcttacag agacttcact tcaaatcctt taaatgcaga atctgaagtt    540
acagatggtc tcattgaagt ccagtcagac tatggaattg tctggggtct gagtttacaa    600
aaagtattgt ggaaagatgg agtgtctttt gtaggggtga gcgctgacta ccgtcacggt    660
tccagtccca tcaactatat catcgtttac aacaaggcca accccgagat ctatttcgat    720
gctactgatg gaaacctaag ctataaagaa tggtctgcaa gcatcggcat ctctacgtat    780
cttaatgact atgtgcttcc ctatgcatcc gtatctatag gaaatacttc aagaaaagct    840
ccttctgata gcttcacaga actcgaaaag caatttacga attttaaatt taaaattcgt    900
aaaatcacaa acttcgacag agtaaacttc tgcttcggaa ctacctgctg catctcaaat    960
aacttctact atagtgtaga aggccgttgg ggatatcagc gtgctatcaa cattacgtca   1020
ggtctgcagt tttag                                                     1035
```

<210> 69
<211> 428
<212> PRT
<213> Chlamydia pneumoniae

<400> 69

```
Met Asp Ile Lys Lys Leu Phe Cys Leu Phe Leu Cys Ser Ser Leu Ile
1               5                   10                  15

Ala Met Ser Pro Ile Tyr Gly Lys Thr Gly Asp Tyr Glu Lys Leu Thr
            20                  25                  30

Leu Thr Gly Ile Asn Ile Ile Asp Arg Asn Gly Leu Ser Glu Thr Ile
        35                  40                  45
```

```
Cys Ser Lys Glu Lys Leu Lys Lys Tyr Thr Lys Val Asp Phe Leu Ala
    50                  55                  60

Pro Gln Pro Tyr Gln Lys Val Met Arg Met Tyr Lys Asn Lys Arg Gly
65                  70                  75                  80

Asp Asn Val Ser Cys Leu Thr Ala Tyr His Thr Asn Gly Gln Ile Lys
                85                  90                  95

Gln Tyr Leu Glu Cys Leu Asn Asn Arg Ala Tyr Gly Arg Tyr Arg Glu
            100                 105                 110

Trp His Val Asn Gly Asn Ile Lys Ile Gln Ala Glu Val Ile Gly Gly
        115                 120                 125

Ile Ala Asp Leu His Pro Ser Ala Glu Ser Gly Trp Leu Phe Asp Gln
    130                 135                 140

Thr Thr Phe Ala Tyr Asn Asp Glu Gly Ile Leu Glu Ala Ala Ile Val
145                 150                 155                 160

Tyr Glu Lys Gly Leu Leu Glu Gly Ser Ser Val Tyr Tyr His Thr Asn
                165                 170                 175

Gly Asn Ile Trp Lys Glu Cys Pro Tyr His Lys Gly Val Pro Gln Gly
            180                 185                 190

Lys Phe Leu Thr Tyr Thr Ser Ser Gly Lys Leu Leu Lys Glu Gln Asn
        195                 200                 205

Tyr Gln Gln Gly Lys Arg His Gly Leu Ser Ile Arg Tyr Ser Glu Asp
    210                 215                 220

Ser Glu Glu Asp Val Leu Ala Trp Glu Glu Tyr His Glu Gly Arg Leu
225                 230                 235                 240

Leu Lys Ala Glu Tyr Leu Asp Pro Gln Thr His Glu Ile Tyr Ala Thr
                245                 250                 255

Ile His Glu Gly Asn Gly Ile Gln Ala Ile Tyr Gly Lys Tyr Ala Val
            260                 265                 270

Ile Glu Thr Arg Ala Phe Tyr Arg Gly Glu Pro Tyr Gly Lys Val Thr
        275                 280                 285

Arg Phe Asp Asn Ser Gly Thr Gln Ile Val Gln Thr Tyr Asn Leu Leu
    290                 295                 300

Gln Gly Ala Lys His Gly Glu Glu Phe Phe Phe Tyr Pro Glu Thr Gly
305                 310                 315                 320

Lys Pro Lys Leu Leu Leu Asn Trp His Glu Gly Ile Leu Asn Gly Ile
                325                 330                 335

Val Lys Thr Trp Tyr Pro Gly Gly Thr Leu Glu Ser Cys Lys Glu Leu
            340                 345                 350

Val Asn Asn Lys Lys Ser Gly Leu Leu Thr Ile Tyr Tyr Pro Glu Gly
        355                 360                 365

Gln Ile Met Ala Thr Glu Glu Tyr Asp Asn Asp Leu Leu Ile Lys Gly
```

```
    370                 375                 380

Glu Tyr Phe Arg Pro Gly Asp Arg His Pro Tyr Ser Lys Ile Asp Arg
385                 390                 395                 400

Gly Cys Gly Thr Ala Val Phe Phe Ser Ser Ala Gly Thr Ile Thr Lys
                405                 410                 415

Lys Ile Pro Tyr Gln Asp Gly Lys Pro Leu Leu Asn
            420                 425

<210>   70
<211>   1287
<212>   DNA
<213>   Chlamydia pneumoniae

<400>   70
atggatataa aaaaactctt ttgcttattt ctatgttctt ctctaattgc catgagtccc     60
atttatggga aaacaggtga ctatgagaaa ctcaccctta cagggatcaa tatcattgat    120
agaaacggcc tgtcagaaac tatttgctct aaagagaagc taaagaaata caccaaggta    180
gactttcttg ctccccagcc ctatcaaaag gtcatgagga tgtataaaaa caaacgcgga    240
gataacgttt cttgtttaac agcctatcac actaacgggc aaattaagca gtacctggag    300
tgtctcaata atcgtgctta tggaagatat cgtgaatggc acgtcaacgg gaatatcaaa    360
atccaagctg aggttatcgg aggtattgcg gatcttcatc cctcagcaga gtctggctgg    420
ctatttgatc aaactacatt tgcctataat gatgaaggta tcttagaagc cgctatcgtc    480
tatgaaaaag ggctgctcga aggatcttcg gtgtattacc atactaatgg gaatatttgg    540
aaagagtgtc cctatcataa gggagttcct caaggtaaat tcctgacata cacatcttcg    600
gggaaactgc tcaaagaaca gaattaccaa caaggcaaaa gacacggtct ttcgattcgc    660
tacagcgaag attccgaaga agatgtttta gcctgggaag aatatcatga gggacgactc    720
ctaaaagcag agtacttaga tcctcaaact cacgaaatct atgcgactat acacgaaggg    780
aacggcattc aagcaatcta cggcaagtat gccgttatag aaactagggc attttaccga    840
ggggaacctt atggaaaagt taccagattc gacaactccg gaacacagat tgtccaaacg    900
tataaccttt tgcaaggcgc gaagcacgga gaagaatttt tcttttatcc tgagacaggg    960
aaacccaagc tgcttcttaa ttggcatgaa ggaattttaa atgggatagt aaaaacttgg   1020
tatcccggag gaaccttaga aagttgtaaa gaactcgtaa ataacaaaaa atccgggtta   1080
ctgaccattt actaccctga aggacagatc atggcgaccg aagagtatga taatgatctt   1140
ctaattaaag gagagtactt ccgccctgga gaccgtcatc cctactctaa aatagatcgt   1200
ggttgtggga ctgcagtatt tttctcgtcg gcgggaacta ttactaaaaa aatcccctat   1260
caggacggca aacctttgct caactag                                       1287

<210>   71
<211>   651
<212>   PRT
<213>   Chlamydia pneumoniae

<400>   71
Met Ala Thr Pro Ala Gln Lys Ser Pro Thr Phe Gln Asp Pro Ser Phe
1               5                   10                  15

Val Arg Glu Leu Gly Ser Asn His Pro Val Phe Ser Pro Leu Thr Leu
            20                  25                  30

Glu Glu Arg Gly Glu Met Ala Ile Ala Arg Val Gln Gln Cys Gly Trp
        35                  40                  45

Asn His Thr Ile Val Lys Val Ser Leu Ile Ile Leu Ala Leu Leu Thr
    50                  55                  60

Ile Leu Gly Gly Gly Leu Leu Val Gly Leu Leu Pro Ala Val Pro Met
65                  70                  75                  80
```

```
Phe Ile Gly Thr Gly Leu Ile Ala Leu Gly Ala Val Ile Phe Ala Leu
                85                  90                  95

Ala Leu Ile Leu Cys Leu Tyr Asp Ser Gln Gly Leu Pro Glu Glu Leu
            100                 105                 110

Pro Pro Val Pro Glu Pro Gln Gln Ile Gln Ile Glu Asp Leu Arg Asn
        115                 120                 125

Glu Thr Arg Glu Val Leu Glu Gly Thr Leu Leu Glu Val Leu Leu Lys
    130                 135                 140

Asp Arg Asp Ala Lys Asp Pro Ala Val Pro Gln Val Val Val Asp Cys
145                 150                 155                 160

Glu Lys Arg Leu Gly Met Leu Asp Arg Lys Leu Arg Arg Glu Glu Glu
                165                 170                 175

Ile Leu Tyr Arg Ser Thr Ala His Leu Lys Asp Glu Glu Arg Tyr Glu
            180                 185                 190

Phe Leu Leu Glu Leu Leu Glu Met Arg Ser Leu Val Ala Asp Arg Leu
        195                 200                 205

Glu Phe Asn Arg Arg Ser Tyr Glu Arg Phe Val Gln Gly Ile Met Thr
    210                 215                 220

Val Arg Ser Glu Glu Gly Glu Lys Glu Ile Ser Arg Leu Gln Asp Leu
225                 230                 235                 240

Ile Ser Leu Gln Gln Gln Thr Val Gln Asp Leu Arg Ser Arg Ile Asp
                245                 250                 255

Asp Glu Gln Lys Arg Cys Trp Thr Ala Leu Gln Arg Ile Asn Gln Ser
            260                 265                 270

Gln Lys Asp Ile Gln Arg Ala His Asp Arg Glu Ala Ser Gln Arg Ala
        275                 280                 285

Cys Glu Gly Thr Glu Met Asp Cys Ala Glu Arg Gln Gln Leu Glu Lys
    290                 295                 300

Asp Leu Arg Arg Gln Leu Lys Ser Met Gln Glu Trp Ile Glu Met Arg
305                 310                 315                 320

Gly Thr Ile His Gln Gln Glu Lys Ala Trp Arg Lys Gln Asn Ala Lys
                325                 330                 335

Leu Glu Arg Leu Gln Glu Asp Leu Arg Leu Thr Gly Ile Ala Phe Asp
            340                 345                 350

Glu Gln Ser Leu Phe Tyr Arg Glu Tyr Lys Glu Lys Tyr Leu Ser Gln
        355                 360                 365

Lys Leu Asp Met Gln Lys Ile Leu Gln Glu Val Asn Ala Glu Lys Ser
    370                 375                 380

Glu Lys Ala Cys Leu Glu Ser Leu Val His Asp Tyr Glu Lys Gln Leu
385                 390                 395                 400

Glu Gln Lys Asp Ala Asn Leu Lys Lys Ala Ala Ala Val Trp Glu Glu
```

```
                405                 410                 415

Glu Leu Gly Lys Gln Gln Gln Glu Asp Tyr Glu Gln Thr Gln Glu Ile
            420                 425                 430

Arg Arg Leu Ser Thr Phe Ile Leu Glu Tyr Gln Asp Ser Leu Arg Glu
        435                 440                 445

Ala Glu Lys Val Glu Lys Asp Phe Gln Glu Leu Gln Gln Arg Tyr Ser
    450                 455                 460

Arg Leu Gln Glu Glu Lys Gln Val Lys Glu Lys Ile Leu Glu Glu Ser
465                 470                 475                 480

Met Asn His Phe Ala Asp Leu Phe Glu Lys Ala Gln Lys Glu Asn Met
                485                 490                 495

Ala Tyr Lys Lys Lys Leu Ala Asp Leu Glu Gly Ala Ala Ala Pro Thr
            500                 505                 510

Glu Ile Gly Glu Asp Asp Asp Trp Val Leu Thr Asp Ser Ala Ser Leu
        515                 520                 525

Ser Gln Lys Lys Ile Arg Glu Leu Val Glu Glu Asn Gln Glu Leu Leu
    530                 535                 540

Lys Ala Leu Ala Phe Lys Ser Asn Glu Leu Thr Gln Leu Val Ala Asp
545                 550                 555                 560

Ala Val Glu Ala Glu Lys Glu Ile Ser Lys Leu Arg Glu His Ile Glu
                565                 570                 575

Glu Gln Lys Glu Gly Leu Arg Ala Leu Asp Lys Met His Ala Gln Ala
            580                 585                 590

Ile Lys Asp Cys Glu Ala Ala Gln Arg Lys Cys Cys Asp Leu Glu Ser
        595                 600                 605

Leu Leu Ser Pro Val Arg Glu Asp Ala Gly Met Arg Phe Glu Leu Glu
    610                 615                 620

Val Glu Leu Gln Arg Leu Gln Glu Glu Asn Ala Gln Leu Arg Ala Glu
625                 630                 635                 640

Val Glu Arg Leu Glu Gln Glu Gln Phe Gln Gly
                645                 650

<210>   72
<211>   1956
<212>   DNA
<213>   Chlamydia pneumoniae

<400>   72
atggcaacac ccgctcaaaa atcccctaca tttcaagatc ctagttttgt aagagagcta    60
ggcagtaacc accctgtctt ttccccgcta acgcttgagg aaagagggga gatggcaata   120
gctcgagtcc agcagtgtgg atggaatcat acaattgtta aggtaagtct tattattctt   180
gctcttctta ctattttagg gggaggatta ctcgtaggat tgctgccagc agttcctatg   240
tttattggaa caggtctgat tgctttggga gccgttatat ttgctttggc tttgatttta   300
tgtctttatg attctcaggg ccttcctgag gaactccctc cggttcctga accacaacaa   360
attcagattg aagatttaag aaacgagacc agagaagttc ttgaagggac tcttttagag   420
gttctcttaa aggatagaga cgctaaggac cctgcggtgc cccaggtggt tgtagactgt   480
```

```
gaaaagcgtc ttggaatgtt ggatcgtaag ctgcgacgtg aagaggagat tctgtatcgc     540
tcgacggccc atcttaaaga cgaggaaagg tatgagttct tgctggagct cttggaaatg     600
cgtagtctgg ttgccgatcg gctagaattt aaccgtagaa gttatgagcg atttgttcaa     660
ggaattatga cagttagatc agaggagggg gaaaaagaga tttctcgtct acaagatcta     720
atcagtttgc agcagcagac ggtgcaagat ttaaggagtc ggatcgatga cgagcagaag     780
agatgctgga cggctttaca acgtattaac caatctcaga aggatataca acgggctcat     840
gatcgcgagg cttcgcagcg tgcctgtgag ggcacagaga tggattgtgc agaacgccag     900
caactggaga aggatttaag gagacagctg aaatctatgc aggagtggat tgagatgagg     960
ggcacaatcc atcaacaaga gaaggcttgg cgtaagcaga atgccaaatt agaaagatta    1020
caagaggatc tgagacttac tgggattgct tttgacgaac aatctctgtt ctatcgcgaa    1080
tataaagaga aatatctgag tcagaaacta gatatgcaaa agattttaca ggaagtcaac    1140
gcagagaaaa gtgagaaggc ttgcttagag agtctggtcc atgactatga gaagcagctc    1200
gaacaaaaag atgctaatct gaagaaagca gcagctgttt gggaagaaga attagggaag    1260
cagcaacagg aagactacga acaaacccaa gaaattagac gtctgagtac attcattctt    1320
gagtaccagg acagtctgcg tgaggcagaa aaagttgaga aagatttcca agagctacaa    1380
caaaggtata gccgtcttca agaggagaaa caggtaaaag aaaaaatctt agaagaaagt    1440
atgaatcatt ttgccgatct ctttgagaag gctcaaaagg aaaacatggc ctacaagaag    1500
aagttagcgg atttagaggg tgccgctgct cctactgaga tcggtgagga cgatgactgg    1560
gtactcacag attctgcttc tctcagccag aagaagatcc gcgaactcgt ggaagagaat    1620
caagaactcc tgaaagcact tgcatttaaa tctaacgaat tgactcaact ggttgccgat    1680
gctgtagaag ctgaaaaaga aatcagcaag cttcgagaac acatagaaga gcagaaagaa    1740
ggattacgag ctcttgataa gatgcatgca caagcgatca aagattgcga agctgctcag    1800
agaaaatgct gtgaccttga gagccttctc tctcctgttc gagaagatgc tggaatgaga    1860
tttgagctag aggtcgagct tcaaagattg caagaagaaa atgcacagct tagagcggag    1920
gttgaaagac tagagcaaga gcaatttcaa ggataa                              1956
```

<210> 73
<211> 259
<212> PRT
<213> Chlamydia pneumoniae

<400> 73

```
Met Ile Lys Gln Ile Gly Arg Phe Phe Arg Ala Phe Ile Phe Ile Met
1               5                   10                  15

Pro Leu Ser Leu Thr Ser Cys Glu Ser Lys Ile Asp Arg Asn Arg Ile
            20                  25                  30

Trp Ile Val Gly Thr Asn Ala Thr Tyr Pro Pro Phe Glu Tyr Val Asp
        35                  40                  45

Ala Gln Gly Glu Val Val Gly Phe Asp Ile Asp Leu Ala Lys Ala Ile
    50                  55                  60

Ser Glu Lys Leu Gly Lys Gln Leu Glu Val Arg Glu Phe Ala Phe Asp
65                  70                  75                  80

Ala Leu Ile Leu Asn Leu Lys Lys His Arg Ile Asp Ala Ile Leu Ala
                85                  90                  95

Gly Met Ser Ile Thr Pro Ser Arg Gln Lys Glu Ile Ala Leu Leu Pro
            100                 105                 110

Tyr Tyr Gly Asp Glu Val Gln Glu Leu Met Val Val Ser Lys Arg Ser
        115                 120                 125

Leu Glu Thr Pro Val Leu Pro Leu Thr Gln Tyr Ser Ser Val Ala Val
    130                 135                 140

Gln Thr Gly Thr Phe Gln Glu His Tyr Leu Leu Ser Gln Pro Gly Ile
145                 150                 155                 160
```

```
Cys Val Arg Ser Phe Asp Ser Thr Leu Glu Val Ile Met Glu Val Arg
                165                 170                 175

Tyr Gly Lys Ser Pro Val Ala Val Leu Glu Pro Ser Val Gly Arg Val
            180                 185                 190

Val Leu Lys Asp Phe Pro Asn Leu Val Ala Thr Arg Leu Glu Leu Pro
        195                 200                 205

Pro Glu Cys Trp Val Leu Gly Cys Gly Leu Gly Val Ala Lys Asp Arg
    210                 215                 220

Pro Glu Glu Ile Gln Thr Ile Gln Gln Ala Ile Thr Asp Leu Lys Ser
225                 230                 235                 240

Glu Gly Val Ile Gln Ser Leu Thr Lys Lys Trp Gln Leu Ser Glu Val
                245                 250                 255

Ala Tyr Glu


<210>   74
<211>   780
<212>   DNA
<213>   Chlamydia pneumoniae

<400>   74
atgataaaac aaataggccg ttttttaga gcatttattt ttataatgcc tttatcttta    60
acaagttgtg agtctaaaat cgatcgaaat cgcatctgga ttgtaggtac gaatgctaca   120
tatcctcctt ttgagtatgt ggatgctcag ggggaagttg taggtttcga tatagatttg   180
gcaaaggcaa ttagtgaaaa acttggcaag caattggaag ttagagaatt cgctttcgat   240
gctttaattt taaatttaaa aaaacatcgt atcgatgcaa ttttagcagg aatgtccatt   300
actccttcgc gtcagaagga aatcgccctg cttccctatt atggcgatga ggttcaagag   360
ctgatggtgg tttctaagcg gtctttagag acccctgtgc ttcccctaac acagtattct   420
tctgttgctg ttcagacagg aacgtttcag gagcattatc ttttatctca gcccggaatt   480
tgtgtccgtt cttttgatag caccttggag gtgattatgg aagttcgtta tgggaaatct   540
ccggttgccg ttctagaacc ctcggtagga cgtgtcgttc ttaaagactt ccctaatctt   600
gttgcaacaa gattagagct ccctcctgaa tgttgggtgt tgggctgtgg tctcggcgta   660
gctaaagatc gtcctgaaga aatacaaacg attcaacaag cgattacaga tttaaagagc   720
gaaggggtga ttcaatcttt aaccaagaaa tggcaacttt ctgaagttgc ttacgaatag   780

<210>   75
<211>   476
<212>   PRT
<213>   Chlamydia pneumoniae

<400>   75
Met Met Ser Arg Leu Arg Phe Arg Leu Ala Ala Leu Gly Ile Phe Phe
1               5                   10                  15

Ile Leu Leu Val Pro Asn Ser Val Ser Ala Lys Thr Ile Val Ala Ser
            20                  25                  30

Asp Lys Glu Lys Val Gly Val Leu Val Tyr Asp Asn Ser Val Glu Ala
        35                  40                  45

Phe Gln Gln Ile Leu Asp Cys Ile Asp His Ala Asn Phe Tyr Val Glu
    50                  55                  60

Leu Cys Pro Cys Met Thr Gly Gly Arg Thr Leu Lys Glu Met Val Asp
```

65 70 75 80

His Leu Glu Ala Arg Met Asp Leu Val Pro Glu Leu Cys Ser Tyr Ile
85 90 95

Ile Ile Gln Pro Thr Phe Thr Asp Ala Glu Asp Gln Lys Leu Leu Lys
100 105 110

Ala Leu Lys Glu Arg His Pro Asn Arg Phe Phe Tyr Val Phe Thr Gly
115 120 125

Cys Pro Pro Ser Thr Ser Ile Leu Ala Pro Asn Val Ile Glu Met His
130 135 140

Ile Lys Leu Ser Ile Ile Asp Gly Lys Tyr Cys Ile Leu Gly Gly Thr
145 150 155 160

Asn Phe Glu Glu Phe Met Cys Thr Pro Gly Asp Glu Val Pro Glu Lys
165 170 175

Val Asp Asn Pro Arg Leu Phe Val Ser Gly Val Arg Arg Pro Leu Ala
180 185 190

Phe Arg Asp Gln Asp Ile Met Leu Arg Ser Thr Ala Phe Gly Leu Gln
195 200 205

Leu Arg Glu Glu Tyr His Lys Gln Phe Ala Met Trp Asp Tyr Tyr Ala
210 215 220

His His Met Trp Phe Ile Asp Asn Pro Glu Gln Phe Ala Gly Ala Cys
225 230 235 240

Pro Pro Leu Thr Leu Glu Gln Ala Glu Glu Thr Val Phe Pro Gly Phe
245 250 255

Asp Lys His Glu Asp Leu Val Leu Val Asp Ser Ser Lys Ile Arg Ile
260 265 270

Val Leu Gly Gly Pro His Asp Lys Gln Pro Asn Pro Val Thr Gln Glu
275 280 285

Tyr Leu Lys Leu Ile Gln Gly Ala Arg Ser Ser Val Lys Leu Ala His
290 295 300

Met Tyr Phe Ile Pro Lys Asp Glu Leu Leu Asn Ala Leu Val Asp Val
305 310 315 320

Ser His Asn His Gly Val His Leu Ser Leu Ile Thr Asn Gly Cys His
325 330 335

Glu Leu Ser Pro Ala Ile Thr Gly Pro Tyr Ala Trp Gly Asn Arg Ile
340 345 350

Asn Tyr Phe Ala Leu Leu Tyr Gly Lys Arg Tyr Pro Leu Trp Lys Lys
355 360 365

Trp Phe Cys Glu Lys Leu Lys Pro Tyr Glu Arg Val Ser Ile Tyr Glu
370 375 380

Phe Ala Ile Trp Glu Thr Gln Leu His Lys Lys Cys Met Ile Ile Asp
385 390 395 400

Asp Glu Ile Phe Val Ile Gly Ser Tyr Asn Phe Gly Lys Lys Ser Asp
405 410 415

Ala Phe Asp Tyr Glu Ser Ile Val Val Ile Glu Ser Pro Glu Val Ala
420 425 430

Ala Lys Ala Asn Lys Val Phe Asn Lys Asp Ile Gly Leu Ser Ile Pro
435 440 445

Val Ser His Gly Asp Ile Phe Ser Trp Tyr Phe His Ser Val His His
450 455 460

Thr Leu Gly His Leu Gln Leu Thr Tyr Met Pro Ala
465 470 475

<210> 76
<211> 1431
<212> DNA
<213> Chlamydia pneumoniae

<400> 76
atgatgagtc ggttgcgttt tcgcttggca gctcttggaa tatttttat tttgctggtt 60
cctaattctg tttcagcaaa gacaatcgta gcttcagaca aggagaaggt tggagttctt 120
gtttatgaca atagtgtaga ggcctttcaa cagatattgg attgcataga tcatgcaaat 180
ttttatgtag aactgtgtcc ctgcatgaca ggaggccgaa cgcttaaaga gatggtagat 240
cacctcgagg ctcgtatgga tctggttcca gagctctgta gctatatcat tatccaaccc 300
acgtttaccg atgctgaaga ccaaaaatta ctcaaagctc tcaaagaacg tcatcccaac 360
cggtttttct acgtttttac agggtgccca ccctcaacaa gcatcctcgc tcctaatgtc 420
attgaaatgc atatcaaact ttctatcatc gatgggaaat attgtatttt aggtggtacc 480
aattttgaag agtttatgtg cactccaggg gatgaggttc ctgagaaagt ggataaccca 540
cgtttatttg tcagtggagt gcgtcggccc ctagcatttc gtgatcagga tatcatgttg 600
cgttctacag cattcggttt gcagctcaga gaagaatatc ataagcaatt tgctatgtgg 660
gactactatg cacatcatat gtggttcatt gataatcctg aacagtttgc aggcgcctgt 720
cctccactga ctttagaaca agccgaggag acagtatttc ctggatttga caaacatgaa 780
gatcttgttc ttgtcgactc ttccaagatc aggatagttt taggtggtcc ccacgataag 840
caacccaatc ctgtgactca agaatatttg aaacttatcc agggagctag atcttctgtg 900
aagcttgctc acatgtattt catccctaag gacgagcttt taaatgctct tgtcgacgtt 960
tctcataatc acggtgttca tctgagttta attacgaacg gctgtcatga attaagtcct 1020
gcaattacag gaccctatgc ttggggaaac cgtattaact atttcgcctt gctctatggg 1080
aaacggtatc ctctttggaa aaaatggttt tgcgaaaagc taaaacctta tgagcgggtt 1140
tctatttatg agtttgctat ttgggaaacg cagttgcaca agaagtgtat gattatcgat 1200
gatgaaattt ttgtgatcgg aagttataat tttggaaaga aaagtgatgc ctttgattac 1260
gaaagtattg tagttatcga atctccagaa gtcgctgcaa aagctaacaa agtcttcaat 1320
aaagatatcg gattgtcgat tcctgtaagt catggcgaca ttttctcttg gtatttccat 1380
tccgtacacc acactttggg acatttgcag ctgacctata tgccagccta g 1431

<210> 77
<211> 171
<212> PRT
<213> Chlamydia pneumoniae

<400> 77
Met Lys Lys Leu Leu Phe Ser Thr Phe Leu Leu Val Leu Gly Ser Thr
1 5 10 15

Ser Ala Ala His Ala Asn Leu Gly Tyr Val Asn Leu Lys Arg Cys Leu
20 25 30

Glu Glu Ser Asp Leu Gly Lys Lys Glu Thr Glu Glu Leu Glu Ala Met
35 40 45

Lys Gln Gln Phe Val Lys Asn Ala Glu Lys Ile Glu Glu Glu Leu Thr
    50                  55                  60

Ser Ile Tyr Asn Lys Leu Gln Asp Glu Asp Tyr Met Glu Ser Leu Ser
65                  70                  75                  80

Asp Ser Ala Ser Glu Glu Leu Arg Lys Lys Phe Glu Asp Leu Ser Gly
                85                  90                  95

Glu Tyr Asn Ala Tyr Gln Ser Gln Tyr Tyr Gln Ser Ile Asn Gln Ser
            100                 105                 110

Asn Val Lys Arg Ile Gln Lys Leu Ile Gln Glu Val Lys Ile Ala Ala
        115                 120                 125

Glu Ser Val Arg Ser Lys Glu Lys Leu Glu Ala Ile Leu Asn Glu Glu
    130                 135                 140

Ala Val Leu Ala Ile Ala Pro Gly Thr Asp Lys Thr Thr Glu Ile Ile
145                 150                 155                 160

Ala Ile Leu Asn Glu Ser Phe Lys Lys Gln Asn
                165                 170

<210> 78
<211> 516
<212> DNA
<213> Chlamydia pneumoniae

<400> 78
atgaaaaaat tattattttc tacatttctt cttgttttag gatcaacaag cgcagctcat 60
gcaaatttag gctatgttaa tttaaagcga tgtcttgaag aatccgatct aggtaaaaag 120
gaaactgaag aattggaagc tatgaaacag cagtttgtaa aaaatgctga gaaaatagaa 180
gaagaactca cttctattta taataagttg caagatgaag attacatgga aagcctatcg 240
gattctgcct ctgaagagtt gcgaaagaaa ttcgaagatc tttcaggaga gtacaatgcg 300
taccagtctc agtactatca atctatcaat caaagtaatg taaaacgcat tcaaaaactc 360
attcaagaag taaaaatagc tgcagaatca gtgcggtcca aagaaaaact agaagctatc 420
cttaatgaag aagctgtctt agcaatagca cctgggactg ataaaacaac cgaaattatt 480
gctattctta acgaatcttt caaaaaacaa aactag 516

<210> 79
<211> 284
<212> PRT
<213> Chlamydia pneumoniae

<400> 79
Met Lys Gln Pro Met Ser Leu Ile Phe Ser Ser Val Cys Leu Gly Leu
1               5                   10                  15

Gly Leu Gly Ser Leu Ser Ser Cys Asn Gln Lys Pro Ser Trp Asn Tyr
            20                  25                  30

His Asn Thr Ser Thr Ser Glu Glu Phe Phe Val His Gly Asn Lys Ser
        35                  40                  45

Val Ser Gln Leu Pro His Tyr Pro Ser Ala Phe Arg Thr Thr Gln Ile
    50                  55                  60

Phe Ser Glu Glu His Asn Asp Pro Tyr Val Val Ala Lys Thr Asp Glu
65                  70                  75                  80

Glu Ser Arg Lys Ile Trp Arg Glu Ile His Lys Asn Leu Lys Ile Lys
85 90 95

Gly Ser Tyr Ile Pro Ile Ser Thr Tyr Gly Ser Leu Met His Pro Lys
100 105 110

Ser Ala Ala Leu Thr Leu Lys Thr Tyr Arg Pro His Pro Ile Trp Ile
115 120 125

Asn Gly Tyr Glu Arg Ser Phe Asn Ile Asp Thr Gly Lys Tyr Leu Lys
130 135 140

Asn Gly Ser Arg Arg Arg Thr Ser His Asp Gly Pro Lys Asn Arg Ala
145 150 155 160

Val Leu Asn Leu Ile Lys Ser Ser Gly Arg Arg Cys Asn Ala Ile Gly
165 170 175

Leu Glu Met Thr Glu Glu Asp Phe Val Ile Ala Arg Arg Arg Glu Gly
180 185 190

Val Tyr Ser Leu Tyr Pro Val Glu Val Cys Ser Tyr Pro Gln Gly Asn
195 200 205

Pro Phe Val Ile Ala Tyr Ala Trp Ile Ala Asp Glu Ser Ala Cys Ser
210 215 220

Lys Glu Val Leu Pro Val Lys Gly Tyr Tyr Ser Leu Val Trp Glu Ser
225 230 235 240

Val Ser Ser Ser Asp Ser Leu Asn Ala Phe Gly Asp Ser Phe Ala Glu
245 250 255

Asp Tyr Leu Arg Ser Thr Phe Leu Ala Asn Gly Thr Ser Ile Leu Cys
260 265 270

Val His Glu Ser Tyr Lys Lys Val Pro Pro Gln Pro
275 280

<210> 80
<211> 855
<212> DNA
<213> Chlamydia pneumoniae

<400> 80
atgaaacagc ccatgtctct tatcttttca agtgtatgtt taggattagg tcttggatct 60
ctttcctcct gtaatcaaaa gccctcttgg aattatcaca acacttcaac gagcgaagaa 120
ttctttgttc atggaaataa gagtgtttcg caactgcctc attatccttc tgcatttcgt 180
acgactcaaa tcttttctga agagcacaat gatccttatg tcgtagctaa gactgatgaa 240
gagtctcgta aaatttggag agaaatccat aaaaatctca aatcaaagg ttcttacatt 300
cccatatcga cttatggaag tctgatgcac ccaaaatcag cagctcttac attaaaaacg 360
tatcgtccac atcctatttg gataaatgga tacgagcgtt cttttaatat agacacagga 420
aagtacttaa aaaacggaag tcgccgtaga acttctcacg atggtccgaa aaatcgagct 480
gtactgaatc tcattaaatc ttcgggacga cgctgtaatg ctataggcct tgagatgaca 540
gaagaagact ttgtaatagc tagaaggcga gaaggtgttt atagcctgta tcccgttgaa 600
gtgtgctcgt atcctcaggg gaatcctttt gtcattgctt atgcctggat tgcagatgag 660
agtgcttgct caaaagaggt cctacctgta aaagggtact attctttagt ctgggaaagc 720
gtttcttcct ctgattctct gaatgctttt ggagattcct ttgcagagga ctacctcaga 780
agcacgtttt tagcaaacgg aacttctata ctctgtgttc atgaaagcta taagaaagtt 840
cctcctcagc cctaa 855

```
<210>   81
<211>   365
<212>   PRT
<213>   Chlamydia pneumoniae

<400>   81
Met Ser Lys Lys Ile Lys Val Leu Gly His Leu Thr Leu Cys Thr Leu
1               5                   10                  15

Phe Arg Gly Val Leu Cys Ala Ala Ala Leu Ser Asn Ile Gly Tyr Ala
            20                  25                  30

Ser Thr Ser Gln Glu Ser Pro Tyr Gln Lys Ser Ile Glu Asp Trp Lys
        35                  40                  45

Gly Tyr Thr Phe Thr Asp Leu Glu Leu Leu Ser Lys Glu Gly Trp Ser
    50                  55                  60

Glu Ala His Ala Val Ser Gly Asn Gly Ser Arg Ile Val Gly Ala Ser
65                  70                  75                  80

Gly Ala Gly Gln Gly Ser Val Thr Ala Val Ile Trp Glu Ser His Leu
                85                  90                  95

Ile Lys His Leu Gly Thr Leu Gly Gly Glu Ala Ser Ser Ala Glu Gly
            100                 105                 110

Ile Ser Lys Asp Gly Glu Val Val Val Gly Trp Ser Asp Thr Arg Glu
        115                 120                 125

Gly Tyr Thr His Ala Phe Val Phe Asp Gly Arg Asp Met Lys Asp Leu
    130                 135                 140

Gly Thr Leu Gly Ala Thr Tyr Ser Val Ala Arg Gly Val Ser Gly Asp
145                 150                 155                 160

Gly Ser Ile Ile Val Gly Val Ser Ala Thr Ala Arg Gly Glu Asp Tyr
                165                 170                 175

Gly Trp Gln Val Gly Val Lys Trp Glu Lys Gly Lys Ile Lys Gln Leu
            180                 185                 190

Lys Leu Leu Pro Gln Gly Leu Trp Ser Glu Ala Asn Ala Ile Ser Glu
        195                 200                 205

Asp Gly Thr Val Ile Val Gly Arg Gly Glu Ile Ser Arg Asn His Ile
    210                 215                 220

Val Ala Val Lys Trp Asn Lys Asn Ala Val Tyr Ser Leu Gly Thr Leu
225                 230                 235                 240

Gly Gly Ser Val Ala Ser Ala Glu Ala Ile Ser Ala Asn Gly Lys Val
                245                 250                 255

Ile Val Gly Trp Ser Thr Thr Asn Asn Gly Glu Thr His Ala Phe Met
            260                 265                 270

His Lys Asp Glu Thr Met His Asp Leu Gly Thr Leu Gly Gly Gly Phe
        275                 280                 285
```

```
Ser Val Ala Thr Gly Val Ser Ala Asp Gly Arg Ala Ile Val Gly Phe
    290                 295                 300

Ser Ala Val Lys Thr Gly Glu Ile His Ala Phe Tyr Tyr Ala Glu Gly
305                 310                 315                 320

Glu Met Glu Asp Leu Thr Thr Leu Gly Gly Glu Glu Ala Arg Val Phe
                325                 330                 335

Asp Ile Ser Ser Glu Gly Asn Asp Ile Ile Gly Ser Ile Lys Thr Asp
            340                 345                 350

Ala Gly Ala Glu Arg Ala Tyr Leu Phe His Ile His Lys
        355                 360                 365

<210>   82
<211>   1098
<212>   DNA
<213>   Chlamydia pneumoniae

<400>   82
atgagtaaga agataaaggt tctaggtcat ttgacgctct gcactctgtt tagaggagtg    60
ctgtgtgcag cggccctttc caacatagga tatgcgagta cttctcagga atcaccatat   120
cagaagtcta tagaagactg gaaagggtat acctttacag atcttgagtt actgagtaag   180
gaagggtggt ctgaagctca tgcagtttct ggaaatggca gtagaattgt aggagcttcg   240
ggagctggcc aaggtagtgt gactgctgtc atatgggaaa gtcacctgat aaaacatctc   300
ggcactttag gtggcgaggc ttcatctgca gagggaattt caaaggatgg agaggtggtc   360
gttgggtggt cagatactag agagggatat actcatgcct ttgtcttcga cggtagagat   420
atgaaagatc tcggtactct aggagctacc tattctgtag caaggggtgt ttctggagat   480
ggtagtatca tcgtaggagt ctctgcaact gctcgtggag aggattacgg atggcaagtt   540
ggtgtcaagt gggaaaaagg gaaaatcaaa caattgaagt tgttgcctca aggtctctgg   600
tctgaggcga atgcaatctc tgaggatggt acggtgattg tcgggagagg ggaaatctct   660
cgcaatcaca tcgttgctgt aaaatggaat aaaaatgctg tgtatagttt ggggactctc   720
ggaggtagtg tcgcttcagc agaggctata tcggcaaatg ggaaagtaat tgtaggatgg   780
tccacgacta ataatggtga gactcatgcc tttatgcaca aagatgagac aatgcacgat   840
ctcggcactc taggaggagg tttttctgtc gcaactggag tttctgctga tgggagagcc   900
atcgtaggat tttcagcagt gaagaccgga gaaattcatg ctttttacta tgcagaagga   960
gaaatggagg atttaacaac tttgggaggg gaagaagctc gagtgttcga catatctagc  1020
gaaggaaacg atatcattgg ctctataaaa actgacgctg gagctgaacg cgcctatctg  1080
ttccatatac ataaataa                                                1098

<210>   83
<211>   1609
<212>   PRT
<213>   Chlamydia pneumoniae

<400>   83
Met Val Ala Lys Lys Thr Val Arg Ser Tyr Arg Ser Ser Phe Ser His
1               5                   10                  15

Ser Val Ile Val Ala Ile Leu Ser Ala Gly Ile Ala Phe Glu Ala His
            20                  25                  30

Ser Leu His Ser Ser Glu Leu Asp Leu Gly Val Phe Asn Lys Gln Phe
        35                  40                  45

Glu Glu His Ser Ala His Val Glu Glu Ala Gln Thr Ser Val Leu Lys
    50                  55                  60

Gly Ser Asp Pro Val Asn Pro Ser Gln Lys Glu Ser Glu Lys Val Leu
65                  70                  75                  80
```

```
Tyr Thr Gln Val Pro Leu Thr Gln Gly Ser Ser Gly Glu Ser Leu Asp
                85                  90                  95

Leu Ala Asp Ala Asn Phe Leu Glu His Phe Gln His Leu Phe Glu Glu
            100                 105                 110

Thr Thr Val Phe Gly Ile Asp Gln Lys Leu Val Trp Ser Asp Leu Asp
        115                 120                 125

Thr Arg Asn Phe Ser Gln Pro Thr Gln Glu Pro Asp Thr Ser Asn Ala
    130                 135                 140

Val Ser Glu Lys Ile Ser Ser Asp Thr Lys Glu Asn Arg Lys Asp Leu
145                 150                 155                 160

Glu Thr Glu Asp Pro Ser Lys Lys Ser Gly Leu Lys Glu Val Ser Ser
                165                 170                 175

Asp Leu Pro Lys Ser Pro Glu Thr Ala Val Ala Ala Ile Ser Glu Asp
            180                 185                 190

Leu Glu Ile Ser Glu Asn Ile Ser Ala Arg Asp Pro Leu Gln Gly Leu
        195                 200                 205

Ala Phe Phe Tyr Lys Asn Thr Ser Ser Gln Ser Ile Ser Glu Lys Asp
    210                 215                 220

Ser Ser Phe Gln Gly Ile Ile Phe Ser Gly Ser Gly Ala Asn Ser Gly
225                 230                 235                 240

Leu Gly Phe Glu Asn Leu Lys Ala Pro Lys Ser Gly Ala Ala Val Tyr
                245                 250                 255

Ser Asp Arg Asp Ile Val Phe Glu Asn Leu Val Lys Gly Leu Ser Phe
            260                 265                 270

Ile Ser Cys Glu Ser Leu Glu Asp Gly Ser Ala Ala Gly Val Asn Ile
        275                 280                 285

Val Val Thr His Cys Gly Asp Val Thr Leu Thr Asp Cys Ala Thr Gly
    290                 295                 300

Leu Asp Leu Glu Ala Leu Arg Leu Val Lys Asp Phe Ser Arg Gly Gly
305                 310                 315                 320

Ala Val Phe Thr Ala Arg Asn His Glu Val Gln Asn Asn Leu Ala Gly
                325                 330                 335

Gly Ile Leu Ser Val Val Gly Asn Lys Gly Ala Ile Val Val Glu Lys
            340                 345                 350

Asn Ser Ala Glu Lys Ser Asn Gly Gly Ala Phe Ala Cys Gly Ser Phe
        355                 360                 365

Val Tyr Ser Asn Asn Glu Asn Thr Ala Leu Trp Lys Glu Asn Gln Ala
    370                 375                 380

Leu Ser Gly Gly Ala Ile Ser Ser Ala Ser Asp Ile Asp Ile Gln Gly
385                 390                 395                 400
```

```
Asn Cys Ser Ala Ile Glu Phe Ser Gly Asn Gln Ser Leu Ile Ala Leu
                405                 410                 415

Gly Glu His Ile Gly Leu Thr Asp Phe Val Gly Gly Gly Ala Leu Ala
            420                 425                 430

Ala Gln Gly Thr Leu Thr Leu Arg Asn Asn Ala Val Val Gln Cys Val
        435                 440                 445

Lys Asn Thr Ser Lys Thr His Gly Gly Ala Ile Leu Ala Gly Thr Val
    450                 455                 460

Asp Leu Asn Glu Thr Ile Ser Glu Val Ala Phe Lys Gln Asn Thr Ala
465                 470                 475                 480

Ala Leu Thr Gly Gly Ala Leu Ser Ala Asn Asp Lys Val Ile Ile Ala
                485                 490                 495

Asn Asn Phe Gly Glu Ile Leu Phe Glu Gln Asn Glu Val Arg Asn His
            500                 505                 510

Gly Gly Ala Ile Tyr Cys Gly Cys Arg Ser Asn Pro Lys Leu Glu Gln
        515                 520                 525

Lys Asp Ser Gly Glu Asn Ile Asn Ile Ile Gly Asn Ser Gly Ala Ile
    530                 535                 540

Thr Phe Leu Lys Asn Lys Ala Ser Val Leu Glu Val Met Thr Gln Ala
545                 550                 555                 560

Glu Asp Tyr Ala Gly Gly Gly Ala Leu Trp Gly His Asn Val Leu Leu
                565                 570                 575

Asp Ser Asn Ser Gly Asn Ile Gln Phe Ile Gly Asn Ile Gly Gly Ser
            580                 585                 590

Thr Phe Trp Ile Gly Glu Tyr Val Gly Gly Gly Ala Ile Leu Ser Thr
        595                 600                 605

Asp Arg Val Thr Ile Ser Asn Asn Ser Gly Asp Val Val Phe Lys Gly
    610                 615                 620

Asn Lys Gly Gln Cys Leu Ala Gln Lys Tyr Val Ala Pro Gln Glu Thr
625                 630                 635                 640

Ala Pro Val Glu Ser Asp Ala Ser Ser Thr Asn Lys Asp Glu Lys Ser
                645                 650                 655

Leu Asn Ala Cys Ser His Gly Asp His Tyr Pro Pro Lys Thr Val Glu
            660                 665                 670

Glu Glu Val Pro Pro Ser Leu Leu Glu Glu His Pro Val Val Ser Ser
        675                 680                 685

Thr Asp Ile Arg Gly Gly Gly Ala Ile Leu Ala Gln His Ile Phe Ile
    690                 695                 700

Thr Asp Asn Thr Gly Asn Leu Arg Phe Ser Gly Asn Leu Gly Gly Gly
705                 710                 715                 720

Glu Glu Ser Ser Thr Val Gly Asp Leu Ala Ile Val Gly Gly Gly Ala
```

725 730 735

Leu Leu Ser Thr Asn Glu Val Asn Val Cys Ser Asn Gln Asn Val Val
740 745 750

Phe Ser Asp Asn Val Thr Ser Asn Gly Cys Asp Ser Gly Gly Ala Ile
755 760 765

Leu Ala Lys Lys Val Asp Ile Ser Ala Asn His Ser Val Glu Phe Val
770 775 780

Ser Asn Gly Ser Gly Lys Phe Gly Gly Ala Val Cys Ala Leu Asn Glu
785 790 795 800

Ser Val Asn Ile Thr Asp Asn Gly Ser Ala Val Ser Phe Ser Lys Asn
805 810 815

Arg Thr Arg Leu Gly Gly Ala Gly Val Ala Ala Pro Gln Gly Ser Val
820 825 830

Thr Ile Cys Gly Asn Gln Gly Asn Ile Ala Phe Lys Glu Asn Phe Val
835 840 845

Phe Gly Ser Glu Asn Gln Arg Ser Gly Gly Gly Ala Ile Ile Ala Asn
850 855 860

Ser Ser Val Asn Ile Gln Asp Asn Ala Gly Asp Ile Leu Phe Val Ser
865 870 875 880

Asn Ser Thr Gly Ser Tyr Gly Gly Ala Ile Phe Val Gly Ser Leu Val
885 890 895

Ala Ser Glu Gly Ser Asn Pro Arg Thr Leu Thr Ile Thr Gly Asn Ser
900 905 910

Gly Asp Ile Leu Phe Ala Lys Asn Ser Thr Gln Thr Ala Ala Ser Leu
915 920 925

Ser Glu Lys Asp Ser Phe Gly Gly Gly Ala Ile Tyr Thr Gln Asn Leu
930 935 940

Lys Ile Val Lys Asn Ala Gly Asn Val Ser Phe Tyr Gly Asn Arg Ala
945 950 955 960

Pro Ser Gly Ala Gly Val Gln Ile Ala Asp Gly Gly Thr Val Cys Leu
965 970 975

Glu Ala Phe Gly Gly Asp Ile Leu Phe Glu Gly Asn Ile Asn Phe Asp
980 985 990

Gly Ser Phe Asn Ala Ile His Leu Cys Gly Asn Asp Ser Lys Ile Val
995 1000 1005

Glu Leu Ser Ala Val Gln Asp Lys Asn Ile Ile Phe Gln Asp Ala Ile
1010 1015 1020

Thr Tyr Glu Glu Asn Thr Ile Arg Gly Leu Pro Asp Lys Asp Val Ser
1025 1030 1035 1040

Pro Leu Ser Ala Pro Ser Leu Ile Phe Asn Ser Lys Pro Gln Asp Asp
1045 1050 1055

```
Ser Ala Gln His His Glu Gly Thr Ile Arg Phe Ser Arg Gly Val Ser
            1060                1065                1070

Lys Ile Pro Gln Ile Ala Ala Ile Gln Glu Gly Thr Leu Ala Leu Ser
        1075                1080                1085

Gln Asn Ala Glu Leu Trp Leu Ala Gly Leu Lys Gln Glu Thr Gly Ser
    1090                1095                1100

Ser Ile Val Leu Ser Ala Gly Ser Ile Leu Arg Ile Phe Asp Ser Gln
1105                1110                1115                1120

Val Asp Ser Ser Ala Pro Leu Pro Thr Glu Asn Lys Glu Glu Thr Leu
                1125                1130                1135

Val Ser Ala Gly Val Gln Ile Asn Met Ser Ser Pro Thr Pro Asn Lys
            1140                1145                1150

Asp Lys Ala Val Asp Thr Pro Val Leu Ala Asp Ile Ile Ser Ile Thr
        1155                1160                1165

Val Asp Leu Ser Ser Phe Val Pro Glu Gln Asp Gly Thr Leu Pro Leu
    1170                1175                1180

Pro Pro Glu Ile Ile Ile Pro Lys Gly Thr Lys Leu His Ser Asn Ala
1185                1190                1195                1200

Ile Asp Leu Lys Ile Ile Asp Pro Thr Asn Val Gly Tyr Glu Asn His
                1205                1210                1215

Ala Leu Leu Ser Ser His Lys Asp Ile Pro Leu Ile Ser Leu Lys Thr
            1220                1225                1230

Ala Glu Gly Met Thr Gly Thr Pro Thr Ala Asp Ala Ser Leu Ser Asn
        1235                1240                1245

Ile Lys Ile Asp Val Ser Leu Pro Ser Ile Thr Pro Ala Thr Tyr Gly
    1250                1255                1260

His Thr Gly Val Trp Ser Glu Ser Lys Met Glu Asp Gly Arg Leu Val
1265                1270                1275                1280

Val Gly Trp Gln Pro Thr Gly Tyr Lys Leu Asn Pro Glu Lys Gln Gly
                1285                1290                1295

Ala Leu Val Leu Asn Asn Leu Trp Ser His Tyr Thr Asp Leu Arg Ala
            1300                1305                1310

Leu Lys Gln Glu Ile Phe Ala His His Thr Ile Ala Gln Arg Met Glu
        1315                1320                1325

Leu Asp Phe Ser Thr Asn Val Trp Gly Ser Gly Leu Gly Val Val Glu
    1330                1335                1340

Asp Cys Gln Asn Ile Gly Glu Phe Asp Gly Phe Lys His His Leu Thr
1345                1350                1355                1360

Gly Tyr Ala Leu Gly Leu Asp Thr Gln Leu Val Glu Asp Phe Leu Ile
                1365                1370                1375
```

Gly Gly Cys Phe Ser Gln Phe Phe Gly Lys Thr Glu Ser Gln Ser Tyr
1380 1385 1390

Lys Ala Lys Asn Asp Val Lys Ser Tyr Met Gly Ala Ala Tyr Ala Gly
1395 1400 1405

Ile Leu Ala Gly Pro Trp Leu Ile Lys Gly Ala Phe Val Tyr Gly Asn
1410 1415 1420

Ile Asn Asn Asp Leu Thr Thr Asp Tyr Gly Thr Leu Gly Ile Ser Thr
1425 1430 1435 1440

Gly Ser Trp Ile Gly Lys Gly Phe Ile Ala Gly Thr Ser Ile Asp Tyr
1445 1450 1455

Arg Tyr Ile Val Asn Pro Arg Arg Phe Ile Ser Ala Ile Val Ser Thr
1460 1465 1470

Val Val Pro Phe Val Glu Ala Glu Tyr Val Arg Ile Asp Leu Pro Glu
1475 1480 1485

Ile Ser Glu Gln Gly Lys Glu Val Arg Thr Phe Gln Lys Thr Arg Phe
1490 1495 1500

Glu Asn Val Ala Ile Pro Phe Gly Phe Ala Leu Glu His Ala Tyr Ser
1505 1510 1515 1520

Arg Gly Ser Arg Ala Glu Val Asn Ser Val Gln Leu Ala Tyr Val Phe
1525 1530 1535

Asp Val Tyr Arg Lys Gly Pro Val Ser Leu Ile Thr Leu Lys Asp Ala
1540 1545 1550

Ala Tyr Ser Trp Lys Ser Tyr Gly Val Asp Ile Pro Cys Lys Ala Trp
1555 1560 1565

Lys Ala Arg Leu Ser Asn Asn Thr Glu Trp Asn Ser Tyr Leu Ser Thr
1570 1575 1580

Tyr Leu Ala Phe Asn Tyr Glu Trp Arg Glu Asp Leu Ile Ala Tyr Asp
1585 1590 1595 1600

Phe Asn Gly Gly Ile Arg Ile Ile Phe
1605

<210> 84
<211> 4830
<212> DNA
<213> Chlamydia pneumoniae

<400> 84
atggtagcga aaaaaacagt acgatcttat aggtcttcat tttctcattc cgtaatagta 60
gcaatattgt cagcaggcat tgcttttgaa gcacattcct tacacagctc agaactagat 120
ttaggtgtat tcaataaaca gtttgaggaa cattctgctc atgttgaaga ggctcaaaca 180
tctgttttaa agggatcaga tcctgtaaat ccctctcaga aagaatccga gaaggttttg 240
tacactcaag tgcctcttac ccaaggaagc tctggagaga gtttggatct cgccgatgct 300
aatttcttag agcattttca gcatcttttt gaagagacta cagtatttgg tatcgatcaa 360
aagctggttt ggtcagattt agatactagg aatttttccc aacccactca agaacctgat 420
acaagtaatg ctgtaagtga gaaaatctcc tcagatacca aagagaatag aaaagaccta 480
gagactgaag atccttcaaa aaaaagtggc cttaaagaag tttcatcaga tctccctaaa 540
agtcctgaaa ctgcagtagc agctatttct gaagatcttg aaatctcaga aaacatttca 600

```
gcaagagatc ctcttcaggg tttagcattt ttttataaaa atacatcttc tcagtctatc   660
tctgaaaagg attcttcatt tcaaggaatt atcttttctg gttcaggagc taattcaggg   720
ctaggttttg aaaatcttaa ggcgccgaaa tctggggctg cagtttattc tgatcgagat   780
attgtttttg aaaatcttgt taaaggattg agttttatat cttgtgaatc tttagaagat   840
ggctctgccg caggtgtaaa cattgttgtg acccattgtg gtgatgtaac tctcactgat   900
tgtgccactg gtttagacct tgaagcttta cgtctggtta aagatttttc tcgtggagga   960
gctgttttca ctgctcgcaa ccatgaagtg caaaataacc ttgcaggtgg aattctatcc   1020
gttgtaggca ataaaggagc tattgttgta gagaaaaata gtgctgagaa gtccaatgga   1080
ggagcttttg cttgcggaag ttttgtttac agtaacaacg aaaacaccgc cttgtggaaa   1140
gaaaatcaag cattatcagg aggagccata tcctcagcaa gtgatattga tattcaaggg   1200
aactgtagcg ctattgaatt ttcaggaaac cagtctctaa ttgctcttgg agagcatata   1260
gggcttacag attttgtagg tggaggagct ttagctgctc aagggacgct taccttaaga   1320
aataatgcag tagtgcaatg tgttaaaaac acttctaaaa cacatggtgg agctatttta   1380
gcaggtactg ttgatctcaa cgaaacaatt agcgaagttg cctttaagca gaatacagca   1440
gctctaactg gaggtgcttt aagtgcaaat gataaggtta taattgcaaa taactttgga   1500
gaaattcttt ttgagcaaaa cgaagtgagg aatcacggag gagccattta ttgtggatgt   1560
cgatctaatc ctaagttaga acaaaaggat tctggagaga acatcaatat tattggaaac   1620
tccggagcta tcactttttt aaaaaataag gcttctgttt tagaagtgat gacacaagct   1680
gaagattatg ctggtggagg cgctttatgg gggcataatg ttcttctaga ttccaatagt   1740
gggaatattc aatttatagg aaatataggt ggaagtacct tctggatagg agaatatgtc   1800
ggtggtggtg cgattctctc tactgataga gtgacaattt ctaataactc tggagatgtt   1860
gtttttaaag gaaacaaagg ccaatgtctt gctcaaaaat atgtagctcc tcaagaaaca   1920
gctcccgtgg aatcagatgc ttcatctaca aataaagacg agaagagcct taatgcttgt   1980
agtcatggag atcattatcc tcctaaaact gtagaagagg aagtgccacc ttcattgtta   2040
gaagaacatc ctgttgtttc ttcgacagat attcgtggtg gtggggccat tctagctcaa   2100
catatcttta ttacagataa tacaggaaat ctgagattct ctgggaacct tggtggtggt   2160
gaagagtctt ctactgtcgg tgatttagct atcgtaggag gaggtgcttt gctttctact   2220
aatgaagtta atgtttgcag taaccaaaat gttgtttttt ctgataacgt gacttcaaat   2280
ggttgtgatt caggggggagc tattttagct aaaaaagtag atatctccgc gaaccactcg   2340
gttgaatttg tctctaatgg ttcagggaaa ttcggtggtg ccgtttgcgc tttaaacgaa   2400
tcagtaaaca ttacggacaa tggctcggca gtatcattct ctaaaaatag aacacgtctt   2460
ggcggtgctg gagttgcagc tcctcaaggc tctgtaacga tttgtggaaa tcagggaaac   2520
atagcattta aagagaactt tgtttttggc tctgaaaatc aaagatcagg tggaggagct   2580
atcattgcta actcttctgt aaatattcag gataacgcag gagatatcct atttgtaagt   2640
aactctacgg gatcttatgg aggtgctatt tttgtaggat ctttggttgc ttctgaaggc   2700
agcaacccac gaacgcttac aattacaggc aacagtgggg atatcctatt tgctaaaaat   2760
agcacgcaaa cagccgcttc tttatcagaa aaagattcct ttggtggagg ggccatctat   2820
acacaaaacc tcaaaattgt aaagaatgca gggaacgttt ctttctatgg caacagagct   2880
cctagtggtg ctggtgtcca aattgcagac ggaggaactg tttgtttaga ggcttttgga   2940
ggagatatct tatttgaagg gaatatcaat tttgatggga gtttcaatgc gattcactta   3000
tgcgggaatg actcaaaaat cgtagagctt tctgctgttc aagataaaaa tattattttc   3060
caagatgcaa ttacttatga agagaacaca attcgtggct tgccagataa agatgtcagt   3120
cctttaagtg ccccttcatt aatttttaac tccaagccac aagatgacag cgctcaacat   3180
catgaaggga cgatacggtt ttctcgaggg gtatctaaaa ttcctcagat tgctgctata   3240
caagagggaa ccttagcttt atcacaaaac gcagagcttt ggttggcagg acttaaacag   3300
gaaacaggaa gttctatcgt attgtctgcg ggatctattc tccgtatttt tgattcccag   3360
gttgatagca gtgcgcctct tcctacagaa aataaagagg agactcttgt ttctgccgga   3420
gttcaaatta acatgagctc tcctacaccc aataaagata aagctgtaga tactccagta   3480
cttgcagata tcataagtat tactgtagat ttgtcttcat ttgttcctga gcaagacgga   3540
actcttcctc ttcctcctga aattatcatt cctaagggaa caaaattaca ttctaatgcc   3600
atagatctta agattataga tcctaccaat gtgggatatg aaaatcatgc tcttctaagt   3660
tctcataaag atattccatt aatttctctt aagacagcgg aaggaatgac agggacgcct   3720
acagcagatg cttctctatc taatataaaa atagatgtat ctttaccttc gatcacacca   3780
gcaacgtatg gtcacacagg agtttggtct gaaagtaaaa tggaagatgg aagacttgta   3840
gtcggttggc aacctacggg atataagtta aatcctgaga agcaaggggc tctagttttg   3900
aataatctct ggagtcatta tacagatctt agagctctta agcaggagat ctttgctcat   3960
catacgatag ctcaaagaat ggagttagat ttctcgacaa atgtctgggg atcaggatta   4020
ggtgttgttg aagattgtca gaacatcgga gagtttgatg ggttcaaaca tcatctcaca   4080
gggtatgccc taggcttgga tacacaacta gttgaagact tcttaattgg aggatgtttc   4140
tcacagttct ttggtaaaac tgaaagccaa tcctacaaag ctaagaacga tgtgaagagt   4200
tatatgggag ctgcttatgc ggggatttta gcaggtcctt ggttaataaa aggagctttt   4260
```

```
gtttacggta atataaacaa cgatttgact acagattacg gtactttagg tatttcaaca    4320
ggttcatgga taggaaaagg gtttatcgca ggcacaagca ttgattaccg ctatattgta    4380
aatcctcgac ggtttatatc ggcaatcgta tccacagtgg ttccttttgt agaagccgag    4440
tatgtccgta tagatcttcc agaaattagc gaacagggta aagaggttag aacgttccaa    4500
aaaactcgtt ttgagaatgt cgccattcct tttggatttg ctttagaaca tgcttattcg    4560
cgtggctcac gtgctgaagt gaacagtgta cagcttgctt acgtctttga tgtatatcgt    4620
aagggacctg tctctttgat tacactcaag gatgctgctt attcttggaa gagttatggg    4680
gtagatattc cttgtaaagc ttggaaggct cgcttgagca ataatacgga atggaattca    4740
tatttaagta cgtatttagc gtttaattat gaatggagag aagatctgat agcttatgac    4800
ttcaatggtg gtatccgtat tattttctag                                     4830

<210>   85
<211>   277
<212>   PRT
<213>   Chlamydia pneumoniae

<400>   85
Met Ser Leu Tyr Gln Lys Trp Trp Asn Ser Gln Leu Lys Lys Ser Leu
1               5                   10                  15

Cys Tyr Ser Thr Val Ala Ala Leu Ile Phe Met Ile Pro Ser Gln Glu
            20                  25                  30

Ser Phe Ala Asp Ser Leu Ile Asp Leu Asn Leu Gly Leu Asp Pro Ser
        35                  40                  45

Val Glu Cys Leu Ser Gly Asp Gly Ala Phe Ser Val Gly Tyr Phe Thr
    50                  55                  60

Lys Ala Gly Ser Thr Pro Val Glu Tyr Gln Pro Phe Lys Tyr Asp Val
65                  70                  75                  80

Ser Lys Lys Thr Phe Thr Ile Leu Ser Val Glu Thr Ala Asn Gln Ser
                85                  90                  95

Gly Tyr Ala Tyr Gly Ile Ser Tyr Asp Gly Thr Ile Thr Val Gly Thr
            100                 105                 110

Cys Ser Leu Gly Ala Gly Lys Tyr Asn Gly Ala Lys Trp Ser Ala Asp
        115                 120                 125

Gly Thr Leu Thr Pro Leu Thr Gly Ile Thr Gly Gly Thr Ser His Thr
    130                 135                 140

Glu Ala Arg Ala Ile Ser Lys Asp Thr Gln Val Ile Glu Gly Phe Ser
145                 150                 155                 160

Tyr Asp Ala Ser Gly Gln Pro Lys Ala Val Gln Trp Ala Ser Gly Ala
                165                 170                 175

Thr Thr Val Thr Gln Leu Ala Asp Ile Ser Gly Gly Ser Arg Ser Ser
            180                 185                 190

Tyr Ala Tyr Ala Ile Ser Asp Asp Gly Thr Ile Ile Val Gly Ser Met
        195                 200                 205

Glu Ser Thr Ile Thr Arg Lys Thr Thr Ala Val Lys Trp Val Asn Asn
    210                 215                 220

Val Pro Thr Tyr Leu Gly Thr Leu Gly Gly Asp Ala Ser Thr Gly Leu
225                 230                 235                 240
```

```
Tyr Ile Ser Gly Asp Gly Thr Val Ile Val Gly Ala Ala Asn Thr Ala
                245                 250                 255

Thr Val Thr Asn Gly Asn Gln Glu Ser His Ala Tyr Met Tyr Lys Asp
            260                 265                 270

Asn Gln Met Lys Asp
        275

<210>   86
<211>   834
<212>   DNA
<213>   Chlamydia pneumoniae

<400>   86
gtgagtctat atcaaaaatg gtggaacagt cagttaaaga agagcctctg ctattcgact    60
gttgctgctc taatatttat gattccttct caagaatcct ttgcagatag tcttatagat   120
ttaaatttag gtttagatcc ttcggtcgaa tgtctgtcag gagatggtgc attttctgtt   180
gggtatttta ctaaggcggg atcgactccc gtagaatatc agccgtttaa atacgacgta   240
tctaagaaga cattcacaat cctttccgta gaaacggcaa atcagagcgg ctatgcttac   300
ggaatctcct acgatggcac gatcactgta ggaacgtgta gcctaggtgc aggaaaatat   360
aacggcgcaa aatggagtgc ggatggcact ttaacacccct taactggaat cacggggggg   420
acgtcacata cggaagcgcg tgcgatttct aaggatactc aggtgatcga gggtttctca   480
tatgatgctt cagggcaacc caaggctgtg cagtgggcaa gcggagcgac tacagtaaca   540
caattagcag atatttcagg aggctctaga agctcttatg cgtatgctat atctgatgat   600
ggcacgatta ttgttgggtc tatggagagc acgataacaa ggaaaactac agctgtaaaa   660
tgggtaaata atgttcctac gtatctggga accttaggag gagatgcttc tacaggtctt   720
tatatttctg gagacggcac cgtgattgta ggtgcggcaa atacagcaac tgtaaccaat   780
gggaatcagg aatcccacgc ctatatgtat aaagataacc aaatgaaaga ttga         834

<210>   87
<211>   264
<212>   PRT
<213>   Chlamydia pneumoniae

<400>   87
Met Ser Asn Gln Leu Gln Pro Cys Ile Ser Leu Gly Cys Val Ser Tyr
1               5                   10                  15

Ile Asn Ser Phe Pro Leu Ser Leu Gln Leu Ile Lys Arg Asn Asp Ile
            20                  25                  30

Arg Cys Val Leu Ala Pro Pro Ala Asp Leu Leu Asn Leu Leu Ile Glu
        35                  40                  45

Gly Lys Leu Asp Val Ala Leu Thr Ser Ser Leu Gly Ala Ile Ser His
    50                  55                  60

Asn Leu Gly Tyr Val Pro Gly Phe Gly Ile Ala Ala Asn Gln Arg Ile
65                  70                  75                  80

Leu Ser Val Asn Leu Tyr Ala Ala Pro Thr Phe Phe Asn Ser Pro Gln
                85                  90                  95

Pro Arg Ile Ala Ala Thr Leu Glu Ser Arg Ser Ser Ile Gly Leu Leu
            100                 105                 110

Lys Val Leu Cys Arg His Leu Trp Arg Ile Pro Thr Pro His Ile Leu
        115                 120                 125
```

```
Arg Phe Ile Thr Thr Lys Val Leu Arg Gln Thr Pro Glu Asn Tyr Asp
    130                 135                 140

Gly Leu Leu Leu Ile Gly Asp Ala Ala Leu Gln His Pro Val Leu Pro
145                 150                 155                 160

Gly Phe Val Thr Tyr Asp Leu Ala Ser Gly Trp Tyr Asp Leu Thr Lys
                165                 170                 175

Leu Pro Phe Val Phe Ala Leu Leu Leu His Ser Thr Ser Trp Lys Glu
            180                 185                 190

His Pro Leu Pro Asn Leu Ala Met Glu Glu Ala Leu Gln Gln Phe Glu
        195                 200                 205

Ser Ser Pro Glu Glu Val Leu Lys Glu Ala His Gln His Thr Gly Leu
    210                 215                 220

Pro Pro Ser Leu Leu Gln Glu Tyr Tyr Ala Leu Cys Gln Tyr Arg Leu
225                 230                 235                 240

Gly Glu Glu His Tyr Glu Ser Phe Glu Lys Phe Arg Glu Tyr Tyr Gly
                245                 250                 255

Thr Leu Tyr Gln Gln Ala Arg Leu
            260
```

<210> 88
<211> 795
<212> DNA
<213> Chlamydia pneumoniae

<400> 88

```
atgtctaacc aactccagcc atgtataagc ttaggctgcg taagttatat taattccttt   60
ccgctgtccc tacaactcat aaaaagaaac gatattcgct gtgttcttgc tccccctgca  120
gacctcctca acttgctaat cgaagggaaa ctcgatgttg ctttgacctc atccctagga  180
gctatctctc ataacttggg gtatgtcccc ggctttggaa ttgcagcaaa ccaacgtatc  240
ctcagtgtaa acctctatgc agctcccact ttctttaact caccgcaacc tcggattgcc  300
gcaactttag aaagtcgctc ctctatagga ctcttaaaag tgctttgtcg tcatctctgg  360
cgcatcccaa ctcctcatat cctaagattc ataactacaa aagtactcag acaaacccct  420
gaaaattatg atggcctcct cctaatcgga gatgcagcgc tacaacatcc tgtacttcct  480
ggatttgtaa cctatgacct tgcctcgggg tggtatgatc ttacaaagct accttttgta  540
tttgctcttc ttctacacag cacctcttgg aaagaacatc ccctacccaa ccttgcgatg  600
gaagaagccc tccaacagtt cgaatcttca cccgaagaag tccttaaaga agctcatcaa  660
catacaggtc tgccccccttc tcttcttcaa gaatactatg ccctatgcca gtaccgtcta  720
ggagaagaac actacgaaag ctttgaaaaa ttccgggaat attatggaac cctctaccaa  780
caagcccgac tgtaa                                                   795
```

<210> 89
<211> 759
<212> PRT
<213> Chlamydia pneumoniae

<400> 89

```
Met Val Phe Ser Tyr Tyr Cys Met Gly Leu Phe Phe Phe Ser Gly Ala
1               5                   10                  15

Ile Ser Ser Cys Gly Leu Leu Val Ser Leu Gly Val Gly Leu Gly Leu
            20                  25                  30

Ser Val Leu Gly Val Leu Leu Leu Leu Leu Ala Gly Leu Leu Leu Phe
```

```
        35                  40                  45

Lys Ile Gln Ser Met Leu Arg Glu Val Pro Lys Ala Pro Asp Leu Leu
    50                  55                  60

Asp Leu Glu Asp Ala Ser Glu Arg Leu Arg Val Lys Ala Ser Arg Ser
65                  70                  75                  80

Leu Ala Ser Leu Pro Lys Glu Ile Ser Gln Leu Glu Ser Tyr Ile Arg
                85                  90                  95

Ser Ala Ala Asn Asp Leu Asn Thr Ile Lys Thr Trp Pro His Lys Asp
            100                 105                 110

Gln Arg Leu Val Glu Thr Val Ser Arg Lys Leu Glu Arg Leu Ala Ala
        115                 120                 125

Ala Gln Asn Tyr Met Ile Ser Glu Leu Cys Glu Ile Ser Glu Ile Leu
    130                 135                 140

Glu Glu Glu Glu His His Leu Ile Leu Ala Gln Glu Ser Leu Glu Trp
145                 150                 155                 160

Ile Gly Lys Ser Leu Phe Ser Thr Phe Leu Asp Met Glu Ser Phe Leu
                165                 170                 175

Asn Leu Ser His Leu Ser Glu Val Arg Pro Tyr Leu Ala Val Asn Asp
            180                 185                 190

Pro Arg Leu Leu Glu Ile Thr Glu Glu Ser Trp Glu Val Val Ser His
        195                 200                 205

Phe Ile Asn Val Thr Ser Ala Phe Lys Lys Ala Gln Ile Leu Phe Lys
    210                 215                 220

Asn Asn Glu His Ser Arg Met Lys Lys Lys Leu Glu Ser Val Gln Glu
225                 230                 235                 240

Leu Leu Glu Thr Phe Ile Tyr Lys Ser Leu Lys Arg Ser Tyr Arg Glu
                245                 250                 255

Leu Gly Cys Leu Ser Glu Lys Met Arg Ile Ile His Asp Asn Pro Leu
            260                 265                 270

Phe Pro Trp Val Gln Asp Gln Gln Lys Tyr Ala His Ala Lys Asn Glu
        275                 280                 285

Phe Gly Glu Ile Ala Arg Cys Leu Glu Glu Phe Glu Lys Thr Phe Phe
    290                 295                 300

Trp Leu Asp Glu Glu Cys Ala Ile Ser Tyr Met Asp Cys Trp Asp Phe
305                 310                 315                 320

Leu Asn Glu Ser Ile Gln Asn Lys Lys Ser Arg Val Asp Arg Asp Tyr
                325                 330                 335

Ile Ser Thr Lys Lys Ile Ala Leu Lys Asp Arg Ala Arg Thr Tyr Ala
            340                 345                 350

Lys Val Leu Leu Glu Glu Asn Pro Thr Thr Glu Gly Lys Ile Asp Leu
        355                 360                 365
```

```
Gln Asp Ala Gln Arg Ala Phe Glu Arg Gln Ser Gln Glu Phe Tyr Thr
    370                 375                 380

Leu Glu His Thr Glu Thr Lys Val Arg Leu Glu Ala Leu Gln Gln Cys
385                 390                 395                 400

Phe Ser Asp Leu Arg Glu Ala Thr Asn Val Arg Gln Val Arg Phe Thr
                405                 410                 415

Asn Ser Glu Asn Ala Asn Asp Leu Lys Glu Ser Phe Glu Lys Ile Asp
            420                 425                 430

Lys Glu Arg Val Arg Tyr Gln Lys Glu Gln Arg Leu Tyr Trp Glu Thr
        435                 440                 445

Ile Asp Arg Asn Glu Gln Glu Leu Arg Glu Glu Ile Gly Glu Ser Leu
    450                 455                 460

Arg Leu Gln Asn Arg Arg Lys Gly Tyr Arg Ala Gly Tyr Asp Ala Gly
465                 470                 475                 480

Arg Leu Lys Gly Leu Leu Arg Gln Trp Lys Lys Asn Leu Arg Asp Val
                485                 490                 495

Glu Ala His Leu Glu Asp Ala Thr Met Asp Phe Glu His Glu Val Ser
            500                 505                 510

Lys Ser Glu Leu Cys Ser Val Arg Ala Arg Leu Glu Val Leu Glu Glu
        515                 520                 525

Glu Leu Met Asp Met Ser Pro Lys Val Ala Asp Ile Glu Glu Leu Leu
    530                 535                 540

Ser Tyr Glu Glu Arg Cys Ile Leu Pro Ile Arg Glu Asn Leu Glu Arg
545                 550                 555                 560

Ala Tyr Leu Gln Tyr Asn Lys Cys Ser Glu Ile Leu Ser Lys Ala Lys
                565                 570                 575

Phe Phe Phe Pro Glu Asp Glu Gln Leu Leu Val Ser Glu Ala Asn Leu
            580                 585                 590

Arg Glu Val Gly Ala Gln Leu Lys Gln Val Gln Gly Lys Cys Gln Glu
        595                 600                 605

Arg Ala Gln Lys Phe Ala Ile Phe Glu Lys His Ile Gln Glu Gln Lys
    610                 615                 620

Ser Leu Ile Lys Glu Gln Val Arg Ser Phe Asp Leu Ala Gly Val Gly
625                 630                 635                 640

Phe Leu Lys Ser Glu Leu Leu Ser Ile Ala Cys Asn Leu Tyr Ile Lys
                645                 650                 655

Ala Val Val Lys Glu Ser Ile Pro Val Asp Val Pro Cys Met Gln Leu
            660                 665                 670

Tyr Tyr Ser Tyr Tyr Glu Asp Asn Glu Ala Val Val Arg Asn Arg Leu
        675                 680                 685
```

```
Leu Asn Met Thr Glu Arg Tyr Gln Asn Phe Lys Arg Ser Leu Asn Ser
    690                 695                 700

Ile Gln Phe Asn Gly Asp Val Leu Leu Arg Asp Pro Val Tyr Gln Pro
705                 710                 715                 720

Glu Gly His Glu Thr Arg Leu Lys Glu Arg Glu Leu Gln Glu Thr Thr
                725                 730                 735

Leu Ser Cys Lys Lys Leu Lys Val Ala Gln Asp Arg Leu Ser Glu Leu
            740                 745                 750

Glu Ser Arg Leu Ser Arg Arg
        755

<210>   90
<211>   2280
<212>   DNA
<213>   Chlamydia pneumoniae

<400>   90
ttggtattct catactattg catgggatta ttttttttct ctggagctat ttctagttgt   60
ggtcttttag tgtctctagg agttggttta ggacttagtg ttttaggagt acttttactt  120
ctcttagcag gtcttttgct ttttaagatc caaagtatgc ttcgagaggt gcctaaggct  180
cctgatctat tagatttaga agatgcaagt gaacggctta gagtaaaggc tagccgttct  240
ttagcaagcc tcccgaagga aatcagtcag ctagagagct acattcgttc tgcagctaat  300
gatctaaata caattaagac ttggccgcat aaagatcaaa gactcgtcga gaccgtgtca  360
cgaaaattag agcgtctggc agctgctcaa aactatatga tttctgaact ctgcgagatt  420
agtgagattc ttgaggaaga ggagcatcat ctaattttgg ctcaggaatc tctagaatgg  480
ataggtaaga gtctattttc taccttttctg gacatggaat ctttttttaaa tttgagccat  540
ctatctgaag tgcgtccgta cttagctgta aatgatccta gattattaga aattaccgaa  600
gaatcttggg aagtagtgag tcatttcata aatgtaacgt ctgcttttaa gaaagctcag  660
attcttttta agaacaacga acattctcgg atgaagaaga agttagaaag tgttcaagag  720
ttactggaaa catttattta taagagttta aagagaagtt atcgagaatt aggatgctta  780
agtgaaaaga tgagaatcat tcacgacaat cctctcttcc cttgggtgca agatcagcag  840
aagtatgctc atgctaagaa tgaatttgga gagattgcgc ggtgtttaga ggagtttgaa  900
aagacgttct tctggttgga tgaggagtgt gctatttctt acatggactg ttgggatttt  960
ctaaatgagt ctattcagaa taagaagtcc agagtagatc gagattatat atccacgaag 1020
aaaattgcat taaaggatag agcccgcact tatgctaagg ttcttttaga agagaatccg 1080
actacagagg gtaaaataga tttgcaagac gctcaaagag cctttgagcg tcaaagtcag 1140
gagttttata cactagagca tacggaaaca aaggtgagac tagaagcact tcaacagtgc 1200
ttctcggatc ttagggaggc gacgaacgta aggcaagtta ggtttacaaa ttctgaaaat 1260
gcgaatgatt taaaggagag tttcgagaag atagataaag agcgtgtgcg atatcaaaaa 1320
gagcaaaggc tctattggga aacaatagat cgcaatgagc aagagcttag ggaagagatt 1380
ggggagtcgc ttcgtttaca aaatcggaga aaagggtata gggctggata tgatgctggg 1440
cgtttaaaag gtttgttgcg tcagtggaag aaaaatctcc gcgatgtgga agcccacctt 1500
gaagatgcaa ctatggattt tgagcatgaa gtaagcaaga gcgaattgtg cagtgttcgg 1560
gcgaggctcg aggttctaga agaagagctg atggatatgt ctcctaaagt tgcggatata 1620
gaagagttgt tgtcctatga agagcgttgt attcttccta ttagggaaaa tttagaaagg 1680
gcatacctcc aatataataa gtgttctgaa attttatcca aggcaaagtt cttctttccg 1740
gaagacgagc aattgctagt ttcggaagcg aatctaagag aggtgggtgc ccagttaaaa 1800
caagtacagg gaaaatgtca agagagggcc caaaagttcg caatatttga aaagcatatt 1860
caggagcaga aaagccttat taaagagcaa gtgcggagtt ttgatctagc gggagttggg 1920
tttttaaaga gtgagcttct tagtattgct tgtaaccttt atataaaggc ggttgttaag 1980
gagtctatac cagttgatgt gccttgtatg cagttatatt atagttatta cgaagataat 2040
gaagctgtag tgcgaaaccg cctttttaaat atgacggaga ggtatcaaaa ttttaaaagg 2100
agtttgaatt ccatacaatt taatggtgac gttcttttac gggatccggt ctatcaacct 2160
gaaggtcatg agaccaggct aaaggaacgg gagctacaag aaacaacttt gtcttgtaag 2220
aaattaaaag tggctcaaga tcgtctttct gaattagagt caaggctgtc taggagatag 2280

<210>   91
```

```
<211>   695
<212>   PRT
<213>   Chlamydia pneumoniae

<400>   91
Met Lys Arg Cys Phe Leu Phe Leu Ala Ser Phe Val Leu Met Gly Ser
1               5                   10                  15

Ser Ala Asp Ala Leu Thr His Gln Glu Ala Val Lys Lys Lys Asn Ser
            20                  25                  30

Tyr Leu Ser His Phe Lys Ser Val Ser Gly Ile Val Thr Ile Glu Asp
        35                  40                  45

Gly Val Leu Asn Ile His Asn Asn Leu Arg Ile Gln Ala Asn Lys Val
    50                  55                  60

Tyr Val Glu Asn Thr Val Gly Gln Ser Leu Lys Leu Val Ala His Gly
65                  70                  75                  80

Asn Val Met Val Asn Tyr Arg Ala Lys Thr Leu Val Cys Asp Tyr Leu
                85                  90                  95

Glu Tyr Tyr Glu Asp Thr Asp Ser Cys Leu Leu Thr Asn Gly Arg Phe
            100                 105                 110

Ala Met Tyr Pro Trp Phe Leu Gly Gly Ser Met Ile Thr Leu Thr Pro
        115                 120                 125

Glu Thr Ile Val Ile Arg Lys Gly Tyr Ile Ser Thr Ser Glu Gly Pro
    130                 135                 140

Lys Lys Asp Leu Cys Leu Ser Gly Asp Tyr Leu Glu Tyr Ser Ser Asp
145                 150                 155                 160

Ser Leu Leu Ser Ile Gly Lys Thr Thr Leu Arg Val Cys Arg Ile Pro
                165                 170                 175

Ile Leu Phe Leu Pro Pro Phe Ser Ile Met Pro Met Glu Ile Pro Lys
            180                 185                 190

Pro Pro Ile Asn Phe Arg Gly Gly Thr Gly Gly Phe Leu Gly Ser Tyr
        195                 200                 205

Leu Gly Met Ser Tyr Ser Pro Ile Ser Arg Lys His Phe Ser Ser Thr
    210                 215                 220

Phe Phe Leu Asp Ser Phe Phe Lys His Gly Val Gly Met Gly Phe Asn
225                 230                 235                 240

Leu His Cys Ser Gln Lys Gln Val Pro Glu Asn Val Phe Asn Met Lys
                245                 250                 255

Ser Tyr Tyr Ala His Arg Leu Ala Ile Asp Met Ala Glu Ala His Asp
            260                 265                 270

Arg Tyr Arg Leu His Gly Asp Phe Cys Phe Thr His Lys His Val Asn
        275                 280                 285

Phe Ser Gly Glu Tyr His Leu Ser Asp Ser Trp Glu Thr Val Ala Asp
    290                 295                 300
```

```
Ile Phe Pro Asn Asn Phe Met Leu Lys Asn Thr Gly Pro Thr Arg Val
305                 310                 315                 320

Asp Cys Thr Trp Asn Asp Asn Tyr Phe Glu Gly Tyr Leu Thr Ser Ser
                325                 330                 335

Val Lys Val Asn Ser Phe Gln Asn Ala Asn Gln Glu Leu Pro Tyr Leu
            340                 345                 350

Thr Leu Arg Gln Tyr Pro Ile Ser Ile Tyr Asn Thr Gly Val Tyr Leu
        355                 360                 365

Glu Asn Ile Val Glu Cys Gly Tyr Leu Asn Phe Ala Phe Ser Asp His
    370                 375                 380

Ile Val Gly Glu Asn Phe Ser Ser Leu Arg Leu Ala Ala Arg Pro Lys
385                 390                 395                 400

Leu His Lys Thr Val Pro Leu Pro Ile Gly Thr Leu Ser Ser Thr Leu
                405                 410                 415

Gly Ser Ser Leu Ile Tyr Tyr Ser Asp Val Pro Glu Ile Ser Ser Arg
            420                 425                 430

His Ser Gln Leu Ser Ala Lys Leu Gln Leu Asp Tyr Arg Phe Leu Leu
        435                 440                 445

His Lys Ser Tyr Ile Gln Arg Arg His Ile Ile Glu Pro Phe Val Thr
    450                 455                 460

Phe Ile Thr Glu Thr Arg Pro Leu Ala Lys Asn Glu Asp His Tyr Ile
465                 470                 475                 480

Phe Ser Ile Gln Asp Ala Phe His Ser Leu Asn Leu Leu Lys Ala Gly
                485                 490                 495

Ile Asp Thr Ser Val Leu Ser Lys Thr Asn Pro Arg Phe Pro Arg Ile
            500                 505                 510

His Ala Lys Leu Trp Thr Thr His Ile Leu Ser Asn Thr Glu Ser Lys
        515                 520                 525

Pro Thr Phe Pro Lys Thr Ala Cys Glu Leu Ser Leu Pro Phe Gly Lys
    530                 535                 540

Lys Asn Thr Val Ser Leu Asp Ala Glu Trp Ile Trp Lys Lys His Cys
545                 550                 555                 560

Trp Asp His Met Asn Ile Arg Trp Glu Trp Ile Gly Asn Asp Asn Val
                565                 570                 575

Ala Met Thr Leu Glu Ser Leu His Arg Ser Lys Tyr Ser Leu Ile Lys
            580                 585                 590

Cys Asp Arg Glu Asn Phe Ile Leu Asp Val Ser Arg Pro Ile Asp Gln
        595                 600                 605

Leu Leu Asp Ser Pro Leu Ser Asp His Arg Asn Leu Ile Leu Gly Lys
    610                 615                 620
```

Leu Phe Val Arg Pro His Pro Cys Trp Asn Tyr Arg Leu Ser Leu Arg
625 630 635 640

Tyr Gly Trp His Arg Gln Asp Thr Pro Asn Tyr Leu Glu Tyr Gln Met
645 650 655

Ile Leu Gly Thr Lys Ile Phe Glu His Trp Gln Leu Tyr Gly Val Tyr
660 665 670

Glu Arg Arg Glu Ala Asp Ser Arg Phe Phe Phe Phe Leu Lys Leu Asp
675 680 685

Lys Pro Lys Lys Pro Pro Phe
690 695

<210> 92
<211> 2088
<212> DNA
<213> Chlamydia pneumoniae

<400> 92
atgaaacgtt gcttcttatt tctagcttcc tttgttctta tgggttcctc agctgatgct 60
ttgactcatc aagaggctgt gaaaaagaaa aactcctatc ttagtcactt taagagtgtt 120
tctgggattg tgaccatcga agatggggta ttgaatatcc ataacaacct gcggatacaa 180
gccaataaag tgtatgtaga aaatactgtg ggtcaaagcc tgaagcttgt cgcacatggc 240
aatgttatgg tgaactatag ggcaaaaacc ctagtttgtg attacctaga gtattacgaa 300
gatacagact cttgtcttct tactaatgga agattcgcga tgtatccttg gtttctaggg 360
gggtctatga tcactctaac cccagaaacc atagtcattc ggaagggata tatctctacc 420
tccgagggtc ccaaaaaaga cctgtgcctc tccggagatt acctggaata ttcttcagat 480
agtcttcttt ctatagggaa gacaacatta agggtgtgtc gcattccgat acttttctta 540
cctccatttt ctatcatgcc tatggagatc cctaagcctc cgataaactt tcgaggagga 600
acaggaggat ttctgggatc ctatttgggg atgagctact cgcccgattc taggaagcat 660
ttctcctcga cattttttctt ggatagcttt ttcaagcatg gcgtcggcat gggattcaac 720
ctccattgtt ctcagaagca ggttcctgag aatgtcttca atatgaaaag ctattatgcc 780
caccgccttg ctatcgatat ggcagaagct catgatcgct atcgcctaca cggagatttc 840
tgcttcacgc ataagcatgt aaattttttct ggagaatacc atctcagcga tagttgggaa 900
actgttgctg acattttccc caacaacttc atgttgaaaa atacaggccc cacacgtgtc 960
gattgcactt ggaatgacaa ctattttgaa gggtatctca cctcttctgt taaggtaaac 1020
tctttccaaa atgccaacca agagctccct tatttaacat taaggcagta cccgatttct 1080
atttataata cgggagtgta ccttgaaaac atcgtagaat gtgggtattt aaactttgct 1140
tttagcgatc atatcgttgg cgagaatttc tcttcactac gtcttgctgc gcgccctaag 1200
ctccataaaa ctgtgcctct acctatagga acgctctcct ccaccctagg gagttctctg 1260
atttactata gcgatgttcc tgagatctcc tcgcgccata gtcagctttc cgcgaagcta 1320
caacttgatt atcgctttct attacataag tcctacattc aaagacgcca tattatagag 1380
ccgttcgtta ccttcattac agagactcgt cctctagcta agaatgaaga tcattatatc 1440
ttttctattc aagatgcctt tcactcctta aaccttctga aagcgggtat agatacctcg 1500
gtactgagta agactaaccc tcgattcccg agaatccatg cgaagctgtg gactacccac 1560
atcttgagca atacagaaag caaacccacg tttcccaaaa ctgcatgcga gctatctcta 1620
ccttttggaa agaaaaatac agtctcctta gatgctgaat ggatttggaa aaagcactgt 1680
tgggatcaca tgaacatacg ttgggagtgg atcggaaatg acaatgtggc tatgactcta 1740
gaatccctgc atagaagcaa atacagcctg attaagtgtg acagggagaa cttcatttta 1800
gatgtcagcc gtcccattga ccagctttta gactcccctc tctctgatca taggaatctc 1860
attttaggga aattatttgt acgacctcat ccctgttgga attaccgctt atccttacgc 1920
tatggctggc atcgccagga cactccgaac tacctagaat accagatgat tctaggacg 1980
aagatcttcg aacattggca gctctatggg gtgtatgaac gccgagaagc agatagtcga 2040
tttttcttct tcttaaagct cgacaaacct aaaaaacctc ccttctaa 2088

<210> 93
<211> 428
<212> PRT
<213> Chlamydia pneumoniae

<400> 93

```
Met Phe Glu Ala Val Ile Ala Asp Ile Gln Ala Arg Glu Ile Leu Asp
1               5                   10                  15

Ser Arg Gly Tyr Pro Thr Leu His Val Lys Val Thr Thr Ser Thr Gly
            20                  25                  30

Ser Val Gly Glu Ala Arg Val Pro Ser Gly Ala Ser Thr Gly Lys Lys
        35                  40                  45

Glu Ala Leu Glu Phe Arg Asp Thr Asp Ser Pro Arg Tyr Gln Gly Lys
    50                  55                  60

Gly Val Leu Gln Ala Val Lys Asn Val Lys Glu Ile Leu Phe Pro Leu
65                  70                  75                  80

Val Lys Gly Cys Ser Val Tyr Glu Gln Ser Leu Ile Asp Ser Leu Met
                85                  90                  95

Met Asp Ser Asp Gly Ser Pro Asn Lys Glu Thr Leu Gly Ala Asn Ala
            100                 105                 110

Ile Leu Gly Val Ser Leu Ala Thr Ala His Ala Ala Ala Ala Thr Leu
        115                 120                 125

Arg Arg Pro Leu Tyr Arg Tyr Leu Gly Gly Cys Phe Ala Cys Ser Leu
    130                 135                 140

Pro Cys Pro Met Met Asn Leu Ile Asn Gly Gly Met His Ala Asp Asn
145                 150                 155                 160

Gly Leu Glu Phe Gln Glu Phe Met Ile Arg Pro Ile Gly Ala Ser Ser
                165                 170                 175

Ile Lys Glu Ala Val Asn Met Gly Ala Asp Val Phe His Thr Leu Lys
            180                 185                 190

Lys Leu Leu His Glu Arg Gly Leu Ser Thr Gly Val Gly Asp Glu Gly
        195                 200                 205

Gly Phe Ala Pro Asn Leu Ala Ser Asn Glu Glu Ala Leu Glu Leu Leu
    210                 215                 220

Leu Leu Ala Ile Glu Lys Ala Gly Phe Thr Pro Gly Lys Asp Ile Ser
225                 230                 235                 240

Leu Ala Leu Asp Cys Ala Ala Ser Ser Phe Tyr Asn Val Lys Thr Gly
                245                 250                 255

Thr Tyr Asp Gly Arg His Tyr Glu Glu Gln Ile Ala Ile Leu Ser Asn
            260                 265                 270

Leu Cys Asp Arg Tyr Pro Ile Asp Ser Ile Glu Asp Gly Leu Ala Glu
        275                 280                 285

Glu Asp Tyr Asp Gly Trp Ala Leu Leu Thr Glu Val Leu Gly Glu Lys
    290                 295                 300

Val Gln Ile Val Gly Asp Asp Leu Phe Val Thr Asn Pro Glu Leu Ile
305                 310                 315                 320
```

Leu Glu Gly Ile Ser Asn Gly Leu Ala Asn Ser Val Leu Ile Lys Pro
325 330 335

Asn Gln Ile Gly Thr Leu Thr Glu Thr Val Tyr Ala Ile Lys Leu Ala
340 345 350

Gln Met Ala Gly Tyr Thr Thr Ile Ile Ser His Arg Ser Gly Glu Thr
355 360 365

Thr Asp Thr Thr Ile Ala Asp Leu Ala Val Ala Phe Asn Ala Gly Gln
370 375 380

Ile Lys Thr Gly Ser Leu Ser Arg Ser Glu Arg Val Ala Lys Tyr Asn
385 390 395 400

Arg Leu Met Glu Ile Glu Glu Glu Leu Gly Ser Glu Ala Ile Phe Thr
405 410 415

Asp Ser Asn Val Phe Ser Tyr Glu Asp Ser Glu Glu
420 425

<210> 94
<211> 1287
<212> DNA
<213> Chlamydia pneumoniae

<400> 94
atgtttgaag ctgtcattgc cgatatccag gctagggaaa tcttggattc tcgcgggtat 60
cccactttac atgttaaagt aaccactagc acaggttctg ttggagaagc tcgggttcct 120
tcaggagcat ccacagggaa aaaagaagcc ttagagtttc gtgatacaga ttctcctcgt 180
tatcaaggca aaggggtttt gcaagctgta aaaaacgtaa aagaaattct tttcccctc 240
gtcaagggat gtagtgttta tgagcaatcc ttaattgatt ctctgatgat ggattctgac 300
ggctctccga acaaagaaac tctaggggcc aatgctattt taggagtctc tctagctaca 360
gcacatgcag cagcagcaac actacgcaga cctctgtatc gttatttagg agggtgtttt 420
gcctgcagtc ttccctgtcc tatgatgaat ctgatcaatg gaggcatgca tgccgataac 480
ggcttggagt tccaagaatt tatgatccgt cctattggag cctcttccat caaagaagct 540
gtcaacatgg gtgctgacgt tttcatactt ttgaaaaaat tactccatga aagaggctta 600
tctactggag tgggtgacga aggaggcttc gccccgaatc ttgcttctaa tgaagaagct 660
ctagagctcc tattgctggc tattgaaaaa gcaggcttta ctccaggaaa agatatatcg 720
ctagccttag actgcgcagc atcctcattc tataacgtaa aaacaggcac gtatgatggg 780
aggcactatg aagagcaaat cgcaatcctt tctaatttat gtgatcgcta tcctatagac 840
tccatagaag atggtcttgc tgaagaagac tatgacgggt gggccttgtt aactgaagtt 900
cttggagaaa aagtacagat tgtgggtgat gacctatttg ttacaaatcc ggaattaata 960
ttagagggta ttagcaatgg attagcgaac tctgtgttga ttaaaccaaa tcagataggg 1020
acgcttactg aaacagtgta tgctatcaag cttgcgcaaa tggctggcta tactacaatt 1080
atttctcatc gctcaggaga aactacggac actacgattg cagatcttgc tgttgccttc 1140
aacgccggtc aaatcaaaac aggctcttta tcacgttctg agcgtgttgc aaaatacaat 1200
agactcatgg aaattgaaga agagcttgga tccgaagcaa ttttcacaga ttctaatgta 1260
ttttcttacg aggattctga ggaatag 1287

<210> 95
<211> 928
<212> PRT
<213> Chlamydia pneumoniae

<400> 95
Met Lys Ser Gln Phe Ser Trp Leu Val Leu Ser Ser Thr Leu Ala Cys
1 5 10 15

Phe Thr Ser Cys Ser Thr Val Phe Ala Ala Thr Ala Glu Asn Ile Gly

```
            20                  25                  30
Pro Ser Asp Ser Phe Asp Gly Ser Thr Asn Thr Gly Thr Tyr Thr Pro
        35                  40                  45
Lys Asn Thr Thr Thr Gly Ile Asp Tyr Thr Leu Thr Gly Asp Ile Thr
    50                  55                  60
Leu Gln Asn Leu Gly Asp Ser Ala Ala Leu Thr Lys Gly Cys Phe Ser
65                  70                  75                  80
Asp Thr Thr Glu Ser Leu Ser Phe Ala Gly Lys Gly Tyr Ser Leu Ser
                85                  90                  95
Phe Leu Asn Ile Lys Ser Ser Ala Glu Gly Ala Ala Leu Ser Val Thr
            100                 105                 110
Thr Asp Lys Asn Leu Ser Leu Thr Gly Phe Ser Ser Leu Thr Phe Leu
        115                 120                 125
Ala Ala Pro Ser Ser Val Ile Thr Thr Pro Ser Gly Lys Gly Ala Val
    130                 135                 140
Lys Cys Gly Gly Asp Leu Thr Phe Asp Asn Asn Gly Thr Ile Leu Phe
145                 150                 155                 160
Lys Gln Asp Tyr Cys Glu Glu Asn Gly Gly Ala Ile Ser Thr Lys Asn
                165                 170                 175
Leu Ser Leu Lys Asn Ser Thr Gly Ser Ile Ser Phe Glu Gly Asn Lys
            180                 185                 190
Ser Ser Ala Thr Gly Lys Lys Gly Gly Ala Ile Cys Ala Thr Gly Thr
        195                 200                 205
Val Asp Ile Thr Asn Asn Thr Ala Pro Thr Leu Phe Ser Asn Asn Ile
    210                 215                 220
Ala Glu Ala Ala Gly Gly Ala Ile Asn Ser Thr Gly Asn Cys Thr Ile
225                 230                 235                 240
Thr Gly Asn Thr Ser Leu Val Phe Ser Glu Asn Ser Val Thr Ala Thr
                245                 250                 255
Ala Gly Asn Gly Gly Ala Leu Ser Gly Asp Ala Asp Val Thr Ile Ser
            260                 265                 270
Gly Asn Gln Ser Val Thr Phe Ser Gly Asn Gln Ala Val Ala Asn Gly
        275                 280                 285
Gly Ala Ile Tyr Ala Lys Lys Leu Thr Leu Ala Ser Gly Gly Gly Gly
    290                 295                 300
Val Ser Pro Phe Leu Thr Ile Ile Val Gln Gly Thr Thr Ala Gly Asn
305                 310                 315                 320
Gly Gly Ala Ile Ser Ile Leu Ala Ala Gly Glu Cys Ser Leu Ser Ala
                325                 330                 335
Glu Ala Gly Asp Ile Thr Phe Asn Gly Asn Ala Ile Val Ala Thr Thr
            340                 345                 350
```

Pro Gln Thr Thr Lys Arg Asn Ser Ile Asp Ile Gly Ser Thr Ala Lys
355 360 365

Ile Thr Asn Leu Arg Ala Ile Ser Gly His Ser Ile Phe Phe Tyr Asp
370 375 380

Pro Ile Thr Ala Asn Thr Ala Ala Asp Ser Thr Asp Thr Leu Asn Leu
385 390 395 400

Asn Lys Ala Asp Ala Gly Asn Ser Thr Asp Tyr Ser Gly Ser Ile Val
405 410 415

Phe Ser Gly Glu Lys Leu Ser Glu Asp Glu Ala Lys Val Ala Asp Asn
420 425 430

Leu Thr Ser Thr Leu Lys Gln Pro Val Thr Leu Thr Ala Gly Asn Leu
435 440 445

Val Leu Lys Arg Gly Val Thr Leu Asp Thr Lys Gly Phe Thr Gln Thr
450 455 460

Ala Gly Ser Ser Val Ile Met Asp Ala Gly Thr Thr Leu Lys Ala Ser
465 470 475 480

Thr Glu Glu Val Thr Leu Thr Gly Leu Ser Ile Pro Val Asp Ser Leu
485 490 495

Gly Glu Gly Lys Lys Val Val Ile Ala Ala Ser Ala Ala Ser Lys Asn
500 505 510

Val Ala Leu Ser Gly Pro Ile Leu Leu Leu Asp Asn Gln Gly Asn Ala
515 520 525

Tyr Glu Asn His Asp Leu Gly Lys Thr Gln Asp Phe Ser Phe Val Gln
530 535 540

Leu Ser Ala Leu Gly Thr Ala Thr Thr Thr Asp Val Pro Ala Val Pro
545 550 555 560

Thr Val Ala Thr Pro Thr His Tyr Gly Tyr Gln Gly Thr Trp Gly Met
565 570 575

Thr Trp Val Asp Asp Thr Ala Ser Thr Pro Lys Thr Lys Thr Ala Thr
580 585 590

Leu Ala Trp Thr Asn Thr Gly Tyr Leu Pro Asn Pro Glu Arg Gln Gly
595 600 605

Pro Leu Val Pro Asn Ser Leu Trp Gly Ser Phe Ser Asp Ile Gln Ala
610 615 620

Ile Gln Gly Val Ile Glu Arg Ser Ala Leu Thr Leu Cys Ser Asp Arg
625 630 635 640

Gly Phe Trp Ala Ala Gly Val Ala Asn Phe Leu Asp Lys Asp Lys Lys
645 650 655

Gly Glu Lys Arg Lys Tyr Arg His Lys Ser Gly Gly Tyr Ala Ile Gly
660 665 670

```
Gly Ala Ala Gln Thr Cys Ser Glu Asn Leu Ile Ser Phe Ala Phe Cys
        675                 680                 685

Gln Leu Phe Gly Ser Asp Lys Asp Phe Leu Val Ala Lys Asn His Thr
    690                 695                 700

Asp Thr Tyr Ala Gly Ala Phe Tyr Ile Gln His Ile Thr Glu Cys Ser
705                 710                 715                 720

Gly Phe Ile Gly Cys Leu Leu Asp Lys Leu Pro Gly Ser Trp Ser His
                725                 730                 735

Lys Pro Leu Val Leu Glu Gly Gln Leu Ala Tyr Ser His Val Ser Asn
            740                 745                 750

Asp Leu Lys Thr Lys Tyr Thr Ala Tyr Pro Glu Val Lys Gly Ser Trp
        755                 760                 765

Gly Asn Asn Ala Phe Asn Met Met Leu Gly Ala Ser Ser His Ser Tyr
    770                 775                 780

Pro Glu Tyr Leu His Cys Phe Asp Thr Tyr Ala Pro Tyr Ile Lys Leu
785                 790                 795                 800

Asn Leu Thr Tyr Ile Arg Gln Asp Ser Phe Ser Glu Lys Gly Thr Glu
                805                 810                 815

Gly Arg Ser Phe Asp Asp Ser Asn Leu Phe Asn Leu Ser Leu Pro Ile
            820                 825                 830

Gly Val Lys Phe Glu Lys Phe Ser Asp Cys Asn Asp Phe Ser Tyr Asp
        835                 840                 845

Leu Thr Leu Ser Tyr Val Pro Asp Leu Ile Arg Asn Asp Pro Lys Cys
    850                 855                 860

Thr Thr Ala Leu Val Ile Ser Gly Ala Ser Trp Glu Thr Tyr Ala Asn
865                 870                 875                 880

Asn Leu Ala Arg Gln Ala Leu Gln Val Arg Ala Gly Ser His Tyr Ala
                885                 890                 895

Phe Ser Pro Met Phe Glu Val Leu Gly Gln Phe Val Phe Glu Val Arg
            900                 905                 910

Gly Ser Ser Arg Ile Tyr Asn Val Asp Leu Gly Gly Lys Phe Gln Phe
        915                 920                 925


<210>   96
<211>   2787
<212>   DNA
<213>   Chlamydia pneumoniae

<400>   96
atgaaatcgc aattttcctg gttagtgctc tcttcgacat tggcatgttt tactagttgt     60
tccactgttt ttgctgcaac tgctgaaaat ataggcccct ctgatagctt tgacggaagt    120
actaacacag gcacctatac tcctaaaaat acgactactg gaatagacta tactctgaca    180
ggagatataa ctctgcaaaa ccttggggat tcggcagctt taacgaaggg ttgtttttct    240
gacactacgg aatctttaag ctttgccggt aaggggtact cactttcttt tttaaatatt    300
aagtctagtg ctgaaggcgc agcactttct gttacaactg ataaaaatct gtcgctaaca    360
```

```
ggattttcga gtcttacttt cttagcggcc ccatcatcgg taatcacaac cccctcagga   420
aaaggtgcag ttaaatgtgg aggggatctt acatttgata acaatggaac tattttattt   480
aaacaagatt actgtgagga aaatggcgga gccatttcta ccaagaatct ttctttgaaa   540
aacagcacgg gatcgatttc ttttgaaggg aataaatcga gcgcaacagg gaaaaaaggt   600
ggggctattt gtgctactgg tactgtagat attacaaata atacggctcc taccctcttc   660
tcgaacaata ttgctgaagc tgcaggtgga gctataaata gcacaggaaa ctgtacaatt   720
acagggaata cgtctcttgt attttctgaa aatagtgtga cagcgaccgc aggaaatgga   780
ggagctcttt ctggagatgc cgatgttacc atatctggga atcagagtgt aactttctca   840
ggaaaccaag ctgtagctaa tggcggagcc atttatgcta agaagcttac actggcttcc   900
gggggggggg gggtatctcc ttttctaaca ataatagtcc aaggtaccac tgcaggtaat   960
ggtggagcca tttctatact ggcagctgga gagtgtagtc tttcagcaga agcaggggac   1020
attaccttca atgggaatgc cattgttgca actacaccac aaactacaaa aagaaattct   1080
attgacatag gatctactgc aaagatcacg aatttacgtg caatatctgg gcatagcatc   1140
tttttctacg atccgattac tgctaatacg gctgcggatt ctacagatac tttaaatctc   1200
aataaggctg atgcaggtaa tagtacagat tatagtgggt cgattgtttt ttctggtgaa   1260
aagctctctg aagatgaagc aaaagttgca gacaacctca cttctacgct gaagcagcct   1320
gtaactctaa ctgcaggaaa tttagtactt aaacgtggtg tcactctcga tacgaaaggc   1380
tttactcaga ccgcgggttc ctctgttatt atggatgcgg gcacaacgtt aaaagcaagt   1440
acagaggagg tcactttaac aggtctttcc attcctgtag actctttagg cgagggtaag   1500
aaagttgtaa ttgctgcttc tgcagcaagt aaaaatgtag cccttagtgg tccgattctt   1560
cttttggata accaagggaa tgcttatgaa aatcacgact taggaaaaac tcaagacttt   1620
tcatttgtgc agctctctgc tctgggtact gcaacaacta cagatgttcc agcggttcct   1680
acagtagcaa ctcctacgca ctatgggtat caaggtactt ggggaatgac ttgggttgat   1740
gataccgcaa gcactccaaa gactaagaca gcgacattag cttggaccaa tacaggctac   1800
cttccgaatc ctgagcgtca aggaccttta gttcctaata gcctttgggg atctttttca   1860
gacatccaag cgattcaagg tgtcatagag agaagtgctt tgactctttg ttcagatcga   1920
ggcttctggg ctgcgggagt cgccaatttc ttagataaag ataagaaagg ggaaaaacgc   1980
aaataccgtc ataaatctgg tggatatgct atcggaggtg cagcgcaaac ttgttctgaa   2040
aacttaatta gctttgcctt ttgccaactc tttggtagcg ataaagattt cttagtcgct   2100
aaaaatcata ctgataccta tgcaggagcc ttctatatcc aacacattac agaatgtagt   2160
gggttcatag gttgtctctt agataaactt cctggctctt ggagtcataa acccctcgtt   2220
ttagaagggc agctcgctta tagccacgtc agtaatgatc tgaagacaaa gtatactgcg   2280
tatcctgagg tgaaaggttc ttggggggaat aatgctttta acatgatgtt gggagcttct   2340
tctcattctt atcctgaata cctgcattgt tttgatacct atgctccata catcaaactg   2400
aatctgacct atatacgtca ggacagcttc tcggagaaag gtacagaagg aagatctttt   2460
gatgacagca acctcttcaa tttatctttg cctatagggg tgaagtttga gaagttctct   2520
gattgtaatg actttttctta tgatctgact ttatcctatg ttcctgatct tatccgcaat   2580
gatcccaaat gcactacagc acttgtaatc agcggagcct cttgggaaac ttatgccaat   2640
aacttagcac gacaggcctt gcaagtgcgt gcaggcagtc actacgcctt ctctcctatg   2700
tttgaagtgc tcggccagtt tgtctttgaa gttcgtggat cctcacggat ttataatgta   2760
gatcttgggg gtaagttcca attctag                                       2787

<210>   97
<211>   260
<212>   PRT
<213>   Chlamydia pneumoniae

<400>   97
Met Glu Glu Val Ser Glu Tyr Leu Gln Gln Val Glu Asn Gln Leu Glu
1               5                   10                  15

Ser Cys Ser Lys Arg Leu Thr Lys Met Glu Thr Phe Ala Leu Gly Val
            20                  25                  30

Arg Leu Glu Ala Lys Glu Glu Ile Glu Ser Ile Ile Leu Ser Asp Val
        35                  40                  45

Val Asn Arg Phe Glu Val Leu Cys Arg Asp Ile Glu Asp Met Leu Ser
    50                  55                  60

Arg Val Glu Glu Ile Glu Arg Met Leu Arg Met Ala Glu Leu Pro Leu
```

65 70 75 80

Leu Pro Ile Lys Glu Ala Leu Thr Lys Ala Phe Val Gln His Asn Ser
85 90 95

Cys Lys Glu Lys Leu Thr Lys Val Glu Pro Tyr Phe Lys Glu Ser Pro
100 105 110

Ala Tyr Leu Thr Ser Glu Glu Arg Leu Gln Ser Leu Asn Gln Thr Leu
115 120 125

Gln Arg Ala Tyr Lys Glu Ser Gln Lys Val Ser Gly Leu Glu Ser Glu
130 135 140

Val Arg Ala Cys Arg Glu Gln Leu Lys Asp Gln Val Arg Gln Phe Glu
145 150 155 160

Thr Gln Gly Val Ser Leu Ile Lys Glu Glu Ile Leu Phe Val Thr Ser
165 170 175

Thr Phe Arg Thr Lys Phe Ser Tyr His Ser Phe Arg Leu His Val Pro
180 185 190

Cys Met Arg Leu Tyr Glu Glu Tyr Tyr Asp Asp Ile Asp Leu Glu Arg
195 200 205

Thr Arg Ala Arg Trp Met Ala Met Ser Glu Arg Tyr Arg Asp Ala Phe
210 215 220

Gln Ala Phe Gln Glu Met Leu Lys Glu Gly Leu Val Glu Glu Ala Gln
225 230 235 240

Ala Leu Arg Glu Thr Glu Tyr Trp Leu Tyr Arg Glu Glu Arg Lys Ser
245 250 255

Lys Lys Lys His
260

<210> 98
<211> 783
<212> DNA
<213> Chlamydia pneumoniae

<400> 98
atggaggagg tgtctgagta tcttcagcaa gtagaaaatc agttggaatc ctgttccaag 60
cgattaacca agatggaaac ttttgcctta ggtgtgaggt tggaagctaa agaagagata 120
gagtctatca tactttctga tgtagtgaac cgttttgagg ttttatgtag agatattgaa 180
gatatgctat ctcgagtcga ggagatagag cggatgttac gtatggcgga gcttcctcta 240
cttcctataa aagaagcgct taccaaggct tttgtacaac ataacagctg taaagagaag 300
ttaaccaagg tagagcctta ctttaaagag agccctgcat atctaactag tgaagagcga 360
ttgcagagtt tgaatcagac tttacaacgt gcgtacaaag agtcccaaaa ggtttcaggt 420
ttagaatcgg aagtgagagc ctgtcgagag cagcttaaag atcaagtaag acagtttgaa 480
actcaaggag tgagcttgat aaaagaagag attctctttg tgactagtac ctttagaact 540
aaatttagct atcattcatt tcgattacat gttccttgca tgaggttgta tgaggagtat 600
tatgatgaca ttgatctaga gagaactcga gctcgatgga tggcgatgtc tgagaggtat 660
agagatgctt ttcaggcatt ccaggagatg ttgaaggaag gcctagttga agaagctcag 720
gctcttagag aaaccgagta ctggttatat cgagaggaga gaaagagtaa aaagaaacat 780
tga 783

<210> 99
<211> 499

<212> PRT
<213> Chlamydia pneumoniae

<400> 99
```
Met Val Leu Phe His Ala Gln Ala Ser Gly Arg Asn Arg Val Lys Ala
1               5                   10                  15

Asp Ala Ile Val Leu Pro Phe Trp His Phe Lys Asp Ala Lys Asn Ala
            20                  25                  30

Ala Ser Phe Glu Ala Glu Phe Glu Pro Ser Tyr Leu Pro Ala Leu Glu
        35                  40                  45

Asn Phe Gln Gly Lys Thr Gly Glu Ile Glu Leu Leu Tyr Ser Ser Pro
    50                  55                  60

Lys Ala Lys Glu Lys Arg Ile Val Leu Leu Gly Leu Gly Lys Asn Glu
65                  70                  75                  80

Glu Leu Thr Ser Asp Val Val Phe Gln Thr Tyr Ala Thr Leu Thr Arg
                85                  90                  95

Val Leu Arg Lys Ala Lys Cys Ser Thr Val Asn Ile Ile Leu Pro Thr
            100                 105                 110

Ile Ser Glu Leu Arg Leu Ser Ala Glu Glu Phe Leu Val Gly Leu Ser
        115                 120                 125

Ser Gly Ile Leu Ser Leu Asn Tyr Asp Tyr Pro Arg Tyr Asn Lys Val
    130                 135                 140

Asp Arg Asn Leu Glu Thr Pro Leu Ser Lys Val Thr Val Ile Gly Ile
145                 150                 155                 160

Val Pro Lys Met Ala Asp Ala Ile Phe Arg Lys Glu Ala Ala Ile Phe
                165                 170                 175

Glu Gly Val Tyr Leu Thr Arg Asp Leu Val Asn Arg Asn Ala Asp Glu
            180                 185                 190

Ile Thr Pro Lys Lys Leu Ala Glu Val Ala Leu Asn Leu Gly Lys Glu
        195                 200                 205

Phe Pro Ser Ile Asp Thr Lys Val Leu Gly Lys Asp Ala Ile Ala Lys
    210                 215                 220

Glu Lys Met Gly Leu Leu Leu Ala Val Ser Lys Gly Ser Cys Val Asp
225                 230                 235                 240

Pro His Phe Ile Val Val Arg Tyr Gln Gly Arg Pro Lys Ser Lys Asp
                245                 250                 255

His Thr Val Leu Ile Gly Lys Gly Val Thr Phe Asp Ser Gly Gly Leu
            260                 265                 270

Asp Leu Lys Pro Gly Lys Ser Met Leu Thr Met Lys Glu Asp Met Ala
        275                 280                 285

Gly Gly Ala Thr Val Leu Gly Ile Leu Ser Ala Leu Ala Val Leu Glu
    290                 295                 300
```

```
Leu Pro Ile Asn Val Thr Gly Ile Ile Pro Ala Thr Glu Asn Ala Ile
305                 310                 315                 320

Asp Gly Ala Ser Tyr Lys Met Gly Asp Val Tyr Val Gly Met Ser Gly
                325                 330                 335

Leu Ser Val Glu Ile Cys Ser Thr Asp Ala Glu Gly Arg Leu Ile Leu
            340                 345                 350

Ala Asp Ala Ile Thr Tyr Ala Leu Lys Tyr Cys Lys Pro Thr Arg Ile
        355                 360                 365

Ile Asp Phe Ala Thr Leu Thr Gly Ala Met Val Val Ser Leu Gly Glu
    370                 375                 380

Glu Val Ala Gly Phe Phe Ser Asn Asn Asp Val Leu Ala Glu Asp Leu
385                 390                 395                 400

Leu Glu Ala Ser Ala Glu Thr Ser Glu Pro Leu Trp Arg Leu Pro Leu
                405                 410                 415

Val Lys Lys Tyr Asp Lys Thr Leu His Ser Asp Ile Ala Asp Met Lys
            420                 425                 430

Asn Leu Gly Ser Asn Arg Ala Gly Ala Ile Thr Ala Ala Leu Phe Leu
        435                 440                 445

Gln Arg Phe Leu Glu Glu Ser Ser Val Ala Trp Ala His Leu Asp Ile
    450                 455                 460

Ala Gly Thr Ala Tyr His Glu Lys Glu Glu Asp Arg Tyr Pro Lys Tyr
465                 470                 475                 480

Ala Ser Gly Phe Gly Val Arg Ser Ile Leu Tyr Tyr Leu Glu Asn Ser
                485                 490                 495

Leu Ser Lys


<210>   100
<211>   1500
<212>   DNA
<213>   Chlamydia pneumoniae

<400>   100
gtggttttat ttcatgctca agcctctggg cgtaatcgtg ttaaggcaga tgctatagtc    60
ctgccctttt ggcattttaa ggatgcaaaa aatgcagctt cttttgaagc cgagtttgaa   120
ccctcgtatc tccccgcttt agaaaacttt caaggaaaaa ccggggagat tgaactcctt   180
tatagtagtc ctaaagctaa ggaaaaacgc attgtcctct taggcttagg gaaaaatgaa   240
gagctcacct ctgatgttgt tttccaaacc tatgcgacac taactcgtgt cttacgtaaa   300
gcaaagtgtt ccacagtcaa tatcatctta cctacaattt ctgaattgcg gctttctgcc   360
gaagaattct tagtggggtt gtcctcagga attttgtcat taaactatga ctacccacgt   420
tataataagg tagatcgtaa tcttgaaact cctctttcta aagtcacggt tatcggtatc   480
gttcccaaaa tggcggatgc tatctttagg aaagaagcag ccatttcga aggcgtatat    540
ctcactcgag atcttgtgaa caggaatgct gatgaaatta cccctaagaa attggcagag   600
gttgctctga atctgggaaa agagttccct agtattgata ctaaggtctt gggaaaagat   660
gccatcgcca aagagaaaat gggactccta ttggctgttt ccaagggttc ttgtgtggat   720
ccacacttta tcgttgtccg ttatcaagga cgtcctaagt ctaaagatca caccgtcttg   780
atagggaaag gggtcacttt tgactctgga ggtttagacc tcaagcctgg aaaatccatg   840
cttactatga aagaagacat ggcaggtggg gctacagtcc tcgggattct ctcggcgtta   900
gcagttttag agcttcctat aaatgtcacg gggatcattc ctgctacaga gaatgctatc   960
```

```
gatggcgcct cctataaaat gggagatgtc tatgtaggaa tgtcggggct ttctgttgag   1020
atttgtagta ccgatgctga gggacgtctt atcctcgctg atgcgattac atatgcttta   1080
aaatattgta aaccgacacg tattatagat tttgcaactc taacaggagc tatggtagtc   1140
tctctaggag aagaggttgc aggtttcttt tccaataacg atgttttagc tgaagatctt   1200
ttagaggcgt cagccgaaac ctccgagccg ttatggagac ttcctctagt taagaagtat   1260
gataaaacat tgcattctga tattgctgat atgaaaaatc taggcagtaa ccgtgcaggg   1320
gctattacag cagcattatt cttgcagaga tttttggaag aatcttcggt agcttgggca   1380
catcttgata ttgcaggtac tgcatatcat gaaaaagaag aagaccgtta tccaaaatat   1440
gcttcaggtt ttggtgttcg ttctattctt tattacttag aaaatagtct ttctaagtag   1500
```

<210> 101
<211> 426
<212> PRT
<213> Chlamydia pneumoniae

<400> 101

```
Met Thr Leu Ile Phe Val Ile Ile Ile Val Trp Cys Asn Ala Phe Leu
1               5                   10                  15

Ile Lys Leu Cys Val Ile Met Gly Leu Gln Ser Arg Leu Gln His Cys
            20                  25                  30

Ile Glu Val Ser Gln Asn Ser Asn Phe Asp Ser Gln Val Lys Gln Phe
        35                  40                  45

Ile Tyr Ala Cys Gln Asp Lys Thr Leu Arg Gln Ser Val Leu Lys Ile
    50                  55                  60

Phe Arg Tyr His Pro Leu Leu Lys Ile His Asp Ile Ala Arg Ala Val
65                  70                  75                  80

Tyr Leu Leu Met Ala Leu Glu Glu Gly Glu Asp Leu Gly Leu Ser Phe
                85                  90                  95

Leu Asn Val Gln Gln Tyr Pro Ser Gly Ala Val Glu Leu Phe Ser Cys
            100                 105                 110

Gly Gly Phe Pro Trp Lys Gly Leu Pro Tyr Pro Ala Glu His Ala Glu
        115                 120                 125

Phe Gly Leu Leu Leu Leu Gln Ile Ala Glu Phe Tyr Glu Glu Ser Gln
    130                 135                 140

Ala Tyr Val Ser Lys Met Ser His Phe Gln Gln Ala Leu Phe Asp His
145                 150                 155                 160

Gln Gly Ser Val Phe Pro Ser Leu Trp Ser Gln Glu Asn Ser Arg Leu
                165                 170                 175

Leu Lys Glu Lys Thr Thr Leu Ser Gln Ser Phe Leu Phe Gln Leu Gly
            180                 185                 190

Met Gln Ile His Pro Glu Tyr Ser Leu Glu Asp Pro Ala Leu Gly Phe
        195                 200                 205

Trp Met Gln Arg Thr Arg Ser Ser Ser Ala Phe Val Ala Ala Ser Gly
    210                 215                 220

Cys Gln Ser Ser Leu Gly Ala Tyr Ser Ser Gly Asp Val Gly Val Ile
225                 230                 235                 240
```

```
Ala Tyr Gly Pro Cys Ser Gly Asp Ile Ser Asp Cys Tyr Tyr Phe Gly
                245                 250                 255

Cys Cys Gly Ile Ala Lys Glu Phe Val Cys Gln Lys Ser His Gln Thr
            260                 265                 270

Thr Glu Ile Ser Phe Leu Thr Ser Thr Gly Lys Pro His Pro Arg Asn
        275                 280                 285

Thr Gly Phe Ser Tyr Leu Arg Asp Ser Tyr Val His Leu Pro Ile Arg
    290                 295                 300

Cys Lys Ile Thr Ile Ser Asp Lys Gln Tyr Arg Val His Ala Ala Leu
305                 310                 315                 320

Ala Glu Ala Thr Ser Ala Met Thr Phe Ser Ile Phe Cys Lys Gly Lys
                325                 330                 335

Asn Cys Gln Val Val Asp Gly Pro Arg Leu Arg Ser Cys Ser Leu Asp
            340                 345                 350

Ser Tyr Lys Gly Pro Gly Asn Asp Ile Met Ile Leu Gly Glu Asn Asp
        355                 360                 365

Ala Ile Asn Ile Val Ser Ala Ser Pro Tyr Met Glu Ile Phe Ala Leu
    370                 375                 380

Gln Gly Lys Glu Lys Phe Trp Asn Ala Asp Phe Leu Ile Asn Ile Pro
385                 390                 395                 400

Tyr Lys Glu Glu Gly Val Met Leu Ile Phe Glu Lys Lys Val Thr Ser
                405                 410                 415

Glu Lys Gly Arg Phe Phe Thr Lys Met Asn
            420                 425

<210>   102
<211>   1281
<212>   DNA
<213>   Chlamydia pneumoniae

<400>   102
ttgactctaa tttttgttat tattatcgtt tggtgcaatg cttttctgat caaattgtgc     60
gtgataatgg ggctgcaatc caggttacaa cattgtatag aagtgtccca gaattcgaac    120
tttgattcac aagtaaaaca gtttatctat gcgtgccaag ataagacatt aaggcagtct    180
gtactcaaga ttttccgcta ccatccttta ctaaaaattc atgatattgc tcgggccgtc    240
tatcttttga tggccttaga agaaggcgag gatttaggct taagcttttt aaatgtacag    300
cagtaccctt caggtgctgt agaactgttt tcttgtgggg gatttccttg gaaaggatta    360
ccttatcctg cagaacatgc ggaatttggc ctactcctgt tacagatcgc agagttttat    420
gaagagagtc aggcatacgt ctctaaaatg agtcattttc aacaggcact ctttgatcac    480
caagggagcg tctttccctc tctctggagc caggagaact ctcgactcct aaaagaaaag    540
acaactctta gccaatcgtt tctcttccaa ttaggaatgc aaattcaccc agaatacagt    600
cttgaggatc ctgcactagg gttctggatg caaagaacgc gttcttcatc cgcttttgta    660
gccgcttcag gatgtcaaag tagcttggga gcgtattcct caggggatgt cggtgttatc    720
gcttatggac cttgctctgg agacattagt gattgttatt attttggatg ttgtggaatc    780
gctaaagagt tcgtgtgcca aaaatctcac caaactacag agatttcttt tctcacctct    840
acaggaaagc ctcatcccag aaatacggga ttttcctacc ttcgagattc ctatgtacat    900
ctgccgatcc gctgtaagat cactatttcc gacaagcaat atcgcgtgca cgctgcgttg    960
gctgaggcca cctctgccat gacgttttct attttctgta aggggaagaa ttgtcaggtt   1020
gttgacggcc ctcgcttgcg ctcctgttcc ctagattctt ataaaggtcc cggaaacgac   1080
attatgattc ttggggaaaa tgacgcaatc aacattgttt ctgcaagtcc ctatatggaa   1140
```

```
atttttgctt tgcaaggcaa agaaaaattt tggaatgcag actttttgat taatattcct    1200
tacaaagaag agggcgtcat gttaattttt gaaaaaaaag tgacctctga gaaaggaaga    1260
ttctttacga agatgaatta a                                              1281

<210>  103
<211>  660
<212>  PRT
<213>  Chlamydia pneumoniae

<400>  103
Met Ser Glu His Lys Lys Ser Ser Lys Ile Ile Gly Ile Asp Leu Gly
1               5                   10                  15

Thr Thr Asn Ser Cys Val Ser Val Met Glu Gly Gly Gln Ala Lys Val
            20                  25                  30

Ile Thr Ser Ser Glu Gly Thr Arg Thr Thr Pro Ser Ile Val Ala Phe
        35                  40                  45

Lys Gly Asn Glu Lys Leu Val Gly Ile Pro Ala Lys Arg Gln Ala Val
    50                  55                  60

Thr Asn Pro Glu Lys Thr Leu Gly Ser Thr Lys Arg Phe Ile Gly Arg
65                  70                  75                  80

Lys Tyr Ser Glu Val Ala Ser Glu Ile Gln Thr Val Pro Tyr Thr Val
                85                  90                  95

Thr Ser Gly Ser Lys Gly Asp Ala Val Phe Glu Val Asp Gly Lys Gln
            100                 105                 110

Tyr Thr Pro Glu Glu Ile Gly Ala Gln Ile Leu Met Lys Met Lys Glu
        115                 120                 125

Thr Ala Glu Ala Tyr Leu Gly Glu Thr Val Thr Glu Ala Val Ile Thr
    130                 135                 140

Val Pro Ala Tyr Phe Asn Asp Ser Gln Arg Ala Ser Thr Lys Asp Ala
145                 150                 155                 160

Gly Arg Ile Ala Gly Leu Asp Val Lys Arg Ile Ile Pro Glu Pro Thr
                165                 170                 175

Ala Ala Ala Leu Ala Tyr Gly Ile Asp Lys Val Gly Asp Lys Lys Ile
            180                 185                 190

Ala Val Phe Asp Leu Gly Gly Gly Thr Phe Asp Ile Ser Ile Leu Glu
        195                 200                 205

Ile Gly Asp Gly Val Phe Glu Val Leu Ser Thr Asn Gly Asp Thr Leu
    210                 215                 220

Leu Gly Gly Asp Asp Phe Asp Glu Val Ile Ile Lys Trp Met Ile Glu
225                 230                 235                 240

Glu Phe Lys Lys Gln Glu Gly Ile Asp Leu Ser Lys Asp Asn Met Ala
                245                 250                 255

Leu Gln Arg Leu Lys Asp Ala Ala Glu Lys Ala Lys Ile Glu Leu Ser
            260                 265                 270
```

```
Gly Val Ser Ser Thr Glu Ile Asn Gln Pro Phe Ile Thr Met Asp Ala
        275                 280                 285

Gln Gly Pro Lys His Leu Ala Leu Thr Leu Thr Arg Ala Gln Phe Glu
    290                 295                 300

Lys Leu Ala Ala Ser Leu Ile Glu Arg Thr Lys Ser Pro Cys Ile Lys
305                 310                 315                 320

Ala Leu Ser Asp Ala Lys Leu Ser Ala Lys Asp Ile Asp Asp Val Leu
                325                 330                 335

Leu Val Gly Gly Met Ser Arg Met Pro Ala Val Gln Glu Thr Val Lys
            340                 345                 350

Glu Leu Phe Gly Lys Glu Pro Asn Lys Gly Val Asn Pro Asp Glu Val
        355                 360                 365

Val Ala Ile Gly Ala Ala Ile Gln Gly Gly Val Leu Gly Gly Glu Val
    370                 375                 380

Lys Asp Val Leu Leu Leu Asp Val Ile Pro Leu Ser Leu Gly Ile Glu
385                 390                 395                 400

Thr Leu Gly Gly Val Met Thr Thr Leu Val Glu Arg Asn Thr Thr Ile
                405                 410                 415

Pro Thr Gln Lys Lys Gln Ile Phe Ser Thr Ala Ala Asp Asn Gln Pro
            420                 425                 430

Ala Val Thr Ile Val Val Leu Gln Gly Glu Arg Pro Met Ala Lys Asp
        435                 440                 445

Asn Lys Glu Ile Gly Arg Phe Asp Leu Thr Asp Ile Pro Pro Ala Pro
    450                 455                 460

Arg Gly His Pro Gln Ile Glu Val Ser Phe Asp Ile Asp Ala Asn Gly
465                 470                 475                 480

Ile Phe His Val Ser Ala Lys Asp Val Ala Ser Gly Lys Glu Gln Lys
                485                 490                 495

Ile Arg Ile Glu Ala Ser Ser Gly Leu Gln Glu Asp Glu Ile Gln Arg
            500                 505                 510

Met Val Arg Asp Ala Glu Ile Asn Lys Glu Glu Asp Lys Lys Arg Arg
        515                 520                 525

Glu Ala Ser Asp Ala Lys Asn Glu Ala Asp Ser Met Ile Phe Arg Ala
    530                 535                 540

Glu Lys Ala Ile Lys Asp Tyr Lys Glu Gln Ile Pro Glu Thr Leu Val
545                 550                 555                 560

Lys Glu Ile Glu Glu Arg Ile Glu Asn Val Arg Asn Ala Leu Lys Asp
                565                 570                 575

Asp Ala Pro Ile Glu Lys Ile Lys Glu Val Thr Glu Asp Leu Ser Lys
            580                 585                 590

His Met Gln Lys Ile Gly Glu Ser Met Gln Ser Gln Ser Ala Ser Ala
```

595 600 605

Ala Ala Ser Ser Ala Ala Asn Ala Lys Gly Gly Pro Asn Ile Asn Thr
610 615 620

Glu Asp Leu Lys Lys His Ser Phe Ser Thr Lys Pro Pro Ser Asn Asn
625 630 635 640

Gly Ser Ser Glu Asp His Ile Glu Glu Ala Asp Val Glu Ile Ile Asp
645 650 655

Asn Asp Asp Lys
660

<210> 104
<211> 1983
<212> DNA
<213> Chlamydia pneumoniae

<400> 104
atgagtgaac acaaaaaatc aagcaaaatt ataggtatag acttaggcac aacaaactcc 60
tgcgtatctg ttatggaagg aggacaagct aaagtaatta catcatccga aggaacaaga 120
accacgccat cgatcgttgc cttcaaaggt aatgagaaat tagtggggat tccagcaaaa 180
cgtcaagcag tgacaaatcc agaaaaaact ctcggctcta caaaacgctt tattggccgt 240
aagtactctg aagtagcttc ggaaatccaa accgttcctt atacagtcac ctccggatct 300
aaaggtgatg ccgttttcga agttgatggc aaacaataca ctccagaaga aattggcgca 360
caaatcttaa tgaaaatgaa agagacagca gaagcttatc taggcgaaac tgtcacagaa 420
gcagtgatca ccgtccccgc atacttcaat gattctcaac gagcatccac aaaagatgct 480
ggacgcattg caggtctaga tgtaaaacgt atcattccag aacctaccgc agcagctctt 540
gcctacggaa tcgataaagt cggtgataaa aaaatcgctg tcttcgacct tggtggagga 600
acttttgata tctccatcct agaaatcggt gatggcgtct tcgaagttct atctacaaat 660
ggagatactc tcctcggtgg agacgacttt gatgaagtca ttatcaaatg gatgatcgaa 720
gaattcaaaa aacaagaagg cattgatctt agcaaagata atatggcctt acaaagactt 780
aaagatgctg ctgagaaagc aaaaatagaa ctttcaggag tctcttccac agaaatcaat 840
cagccattca tcacaatgga tgcacaagga cctaaacacc ttgcattgac actcacacgt 900
gcgcaattcg agaaactcgc agcctctcta atcgaaagaa caaaatctcc atgcatcaaa 960
gcactcagtg acgcaaaact ttccgctaag gatatcgatg atgttctctt agttggaggt 1020
atgtcaagaa tgcccgcagt gcaagaaact gtaaaagaac tcttcggcaa agagcctaat 1080
aaaggagtca accccgacga agttgttgct attggagccg caattcaagg tggtgttctt 1140
ggcggagaag ttaaggatgt tctacttcta gacgttatcc ccctatctct gggtatcgaa 1200
actctaggag gcgtcatgac gactctggta gagagaaata ctacaatccc tacacagaaa 1260
aaacaaatct tctccacagc tgctgataac cagcctgcgg ttaccatcgt agttctccaa 1320
ggagagcgtc ccatggccaa agataacaag gaaatcggaa gattcgatct tacagatatc 1380
cctccggctc ctcgaggcca tcctcaaatc gaagtctcct tcgatatcga tgcaaacgga 1440
attttccatg tctcagctaa agatgttgcc agcggtaaag aacagaaaat tcgtatcgaa 1500
gcaagctcag gacttcaaga agatgaaatc caaagaatgg ttcgagatgc cgaaattaat 1560
aaggaagaag ataaaaaacg tcgtgaagct tcagatgcta aaaatgaagc cgatagcatg 1620
atcttcagag ccgaaaaagc tattaaagat tataaggagc aaattcctga aactttagtt 1680
aaagaaatcg aagagcgaat cgaaaacgtg cgcaacgcac tcaaagatga cgctcctatt 1740
gaaaaaatta aagaggttac tgaagaccta agcaagcata tgcaaaaaat tggagagtct 1800
atgcaatcgc agtctgcatc agcagcagca tcatcggcag ccaatgctaa aggtggacct 1860
aacatcaata cagaagattt gaaaaaacat agtttcagta cgaagcctcc ttcaaataac 1920
ggttcttcag aagaccatat cgaagaagct gatgtagaaa ttattgataa cgacgataag 1980
taa 1983

<210> 105
<211> 364
<212> PRT
<213> Chlamydia pneumoniae

<400> 105

Met Asn Val Pro Asp Ser Lys Asn Leu His Pro Pro Ala Tyr Glu Leu
1               5                   10                  15

Leu Glu Ile Lys Ala Arg Ile Thr Gln Ser Tyr Lys Glu Ala Ser Ala
            20                  25                  30

Ile Leu Thr Ala Ile Pro Asp Gly Ile Leu Leu Leu Ser Glu Thr Gly
        35                  40                  45

His Phe Leu Ile Cys Asn Ser Gln Ala Arg Glu Ile Leu Gly Ile Asp
    50                  55                  60

Glu Asn Leu Glu Ile Leu Asn Arg Ser Phe Thr Asp Val Leu Pro Asp
65                  70                  75                  80

Thr Cys Leu Gly Phe Ser Ile Gln Glu Ala Leu Glu Ser Leu Lys Val
                85                  90                  95

Pro Lys Thr Leu Arg Leu Ser Leu Cys Lys Glu Ser Lys Glu Lys Glu
            100                 105                 110

Val Glu Leu Phe Ile Arg Lys Asn Glu Ile Ser Gly Tyr Leu Phe Ile
        115                 120                 125

Gln Ile Arg Asp Arg Ser Asp Tyr Lys Gln Leu Glu Asn Ala Ile Glu
    130                 135                 140

Arg Tyr Lys Asn Ile Ala Glu Leu Gly Lys Met Thr Ala Thr Leu Ala
145                 150                 155                 160

His Glu Ile Arg Asn Pro Leu Ser Gly Ile Val Gly Phe Ala Ser Ile
                165                 170                 175

Leu Lys Lys Glu Ile Ser Ser Pro Arg His Gln Arg Met Leu Ser Ser
            180                 185                 190

Ile Ile Ser Gly Thr Arg Ser Leu Asn Asn Leu Val Ser Ser Met Leu
        195                 200                 205

Glu Tyr Thr Lys Ser Gln Pro Leu Asn Leu Lys Ile Ile Asn Leu Gln
    210                 215                 220

Asp Phe Phe Ser Ser Leu Ile Pro Leu Leu Ser Val Ser Phe Pro Asn
225                 230                 235                 240

Cys Lys Phe Val Arg Glu Gly Ala Gln Pro Leu Phe Arg Ser Ile Asp
                245                 250                 255

Pro Asp Arg Met Asn Ser Val Val Trp Asn Leu Val Lys Asn Ala Val
            260                 265                 270

Glu Thr Gly Asn Ser Pro Ile Thr Leu Thr Leu His Thr Ser Gly Asp
        275                 280                 285

Ile Ser Val Thr Asn Pro Gly Thr Ile Pro Ser Glu Ile Met Asp Lys
    290                 295                 300

Leu Phe Thr Pro Phe Phe Thr Thr Lys Arg Glu Gly Asn Gly Leu Gly
305                 310                 315                 320

Leu Ala Glu Ala Gln Lys Ile Ile Arg Leu His Gly Gly Asp Ile Gln

325 330 335

Leu Lys Thr Ser Asp Ser Ala Val Ser Phe Phe Ile Ile Ile Pro Glu
340 345 350

Leu Leu Ala Ala Leu Pro Lys Glu Arg Ala Ala Ser
355 360

<210> 106
<211> 1091
<212> DNA
<213> Chlamydia pneumoniae

<400> 106
atgaacgtcc ctgattccaa gaacctccat cctcctgcat acgaactcct agagatcaag 60
gctcgcatca cacaatctta taaagaagcg agtgctatac tgacagcgat tcctgatggt 120
atcctattac tttctgaaac aggacacttt cttatctgca attcacaagc acgtgaaatt 180
ctaggaattg atgaaaatct agaaattctt aatagatcct ttaccgatgt tctccccgat 240
acgtgtcttg gattttctat tcaagaggct cttgaatctc taaaagtccc taaaactctt 300
agactctctc tctgtaaaga atctaaagaa aaagaagtgg aactcttcat ccgtaaaaac 360
gagatcagtg gatacctgtt tatccaaatc cgcgatcggt ccgactataa acaactagaa 420
aacgctatag aaagatataa aaatatcgca gaacttggga aaatgacggc taccctagct 480
cacgaaatcc gcaatccgct aagtggaatc gttggatttg cctctatcct aaagaaagag 540
atttcctctc ctcgccacca acgaatgctc tcctcaatca tctccggcac aaggtctcta 600
aataaccttg tctcttctat gttagaatat acaaaatcac aaccgttgaa cctaaagatt 660
ataaatttac aagacttctt ctcttctctt atccctctgc tctccgtctc tttcccgaat 720
tgcaagtttg taagagaggg cgcacaacct ctattcagat ctatagatcc tgatcggatg 780
aacagtgtcg tttggaacct agtgaaaaat gctgtagaaa cagggaactc tccgatcact 840
ctgaccctgc atacatcggg agacatctcg gtaacgaacc ccggaacgat tccttccgag 900
atcatggaca agctcttcac tccattcttc acaacaaaga gagagggaaa tggtttggga 960
cttgctgaag ctcaaaaaat tataagactc catggaggag atatccaatt aaaaacaagc 1020
gactccgccg ttagcttctt cataatcatc cccgaacttc tagcggccct acccaaagaa 1080
agagccgcta g 1091

<210> 107
<211> 357
<212> PRT
<213> Chlamydia pneumoniae

<400> 107
Met Met Lys Lys Ile Arg Lys Val Ala Leu Ala Val Gly Gly Ser Gly
1 5 10 15

Gly His Ile Val Pro Ala Leu Ser Val Lys Glu Ala Phe Ser Arg Glu
20 25 30

Gly Ile Asp Val Leu Leu Leu Gly Lys Gly Leu Lys Asn His Pro Ser
35 40 45

Leu Gln Gln Gly Ile Ser Tyr Arg Glu Ile Pro Ser Gly Leu Pro Thr
50 55 60

Val Leu Asn Pro Ile Lys Ile Met Ser Arg Thr Leu Ser Leu Cys Ser
65 70 75 80

Gly Tyr Leu Lys Ala Arg Lys Glu Leu Lys Ile Phe Asp Pro Asp Leu
85 90 95

Val Ile Gly Phe Gly Ser Tyr His Ser Leu Pro Val Leu Leu Ala Gly
100 105 110

```
Leu Ser His Lys Ile Pro Leu Phe Leu His Glu Gln Asn Leu Val Pro
        115                 120                 125

Gly Lys Val Asn Gln Leu Phe Ser Arg Tyr Ala Arg Gly Ile Gly Val
    130                 135                 140

Asn Phe Ser Pro Val Thr Lys His Phe Arg Cys Pro Ala Glu Glu Val
145                 150                 155                 160

Phe Leu Pro Lys Arg Ser Phe Ser Leu Gly Ser Pro Met Met Lys Arg
                165                 170                 175

Cys Thr Asn His Thr Pro Thr Ile Cys Val Val Gly Gly Ser Gln Gly
            180                 185                 190

Ala Gln Ile Leu Asn Thr Cys Val Pro Gln Ala Leu Val Lys Leu Val
        195                 200                 205

Asn Lys Tyr Pro Asn Met Tyr Val His His Ile Val Gly Pro Lys Ser
    210                 215                 220

Asp Val Met Lys Val Gln His Val Tyr Asn Arg Gly Glu Val Leu Cys
225                 230                 235                 240

Cys Val Lys Pro Phe Glu Glu Gln Leu Leu Asp Val Leu Leu Ala Ala
                245                 250                 255

Asp Leu Val Ile Ser Arg Ala Gly Ala Thr Ile Leu Glu Glu Ile Leu
            260                 265                 270

Trp Ala Lys Val Pro Gly Ile Leu Ile Pro Tyr Pro Gly Ala Tyr Gly
        275                 280                 285

His Gln Glu Val Asn Ala Lys Phe Phe Val Asp Val Leu Glu Gly Gly
    290                 295                 300

Thr Met Ile Leu Glu Lys Glu Leu Thr Glu Lys Leu Leu Val Glu Lys
305                 310                 315                 320

Val Thr Phe Ala Leu Asp Ser His Asn Arg Glu Lys Gln Arg Asn Ser
                325                 330                 335

Leu Ala Ala Tyr Ser Gln Gln Arg Ser Thr Lys Thr Phe His Ala Phe
            340                 345                 350

Ile Cys Glu Cys Leu
        355

<210>   108
<211>   1074
<212>   DNA
<213>   Chlamydia pneumoniae

<400>   108
atgatgaaga aaattcgaaa agtagccttg gctgtaggag gttcaggagg ccacattgtc    60
ccagctctct cggtaaagga agctttttct cgtgaaggaa tagacgtatt actactaggg   120
aaaggtctca agaaccatcc ttctttgcaa cagggaatca gctatcggga aatcccctca   180
ggacttccta cagtccttaa tcccataaag atcatgagca ggaccctttc tctatgttca   240
ggatacctga aagcaagaaa ggaacttaaa atttttgacc ctgacctggt cataggattt   300
gggagctacc actctcttcc cgtgttgctc gcaggactgt cccataaaat tcccttattt   360
ctacacgaac aaaatctagt tcctggaaaa gtaaatcaat tgttttcccg ctatgctcga   420
```

```
ggtattggag tgaatttctc ccccgttact aaacacttcc gctgccccgc agaagaggtc    480
ttccttccta aacgaagctt ctccttagga agccctatga tgaagcgatg tacaaatcat    540
acccctacaa tctgtgttgt tggaggttct cagggagcac agatattaaa tacttgtgtt    600
ccccaagctc ttgtcaagct agtcaataag tacccaaata tgtacgtcca tcatattgta    660
ggacctaaaa gtgatgttat gaaggtgcaa catgtttaca atcgtggaga ggtcctctgc    720
tgtgtgaagc cgttcgaaga gcaactccta gatgtcttgc ttgccgcaga tttggtcatc    780
agtagggcag gagccacaat tttagaagaa attctttggg caaaagttcc cggaatttta    840
attccctatc caggagctta tggacatcag gaagttaatg ctaaattctt tgtagacgtc    900
ttagaagggg gaactatgat cctagaaaaa gaattaacag agaagctatt agtagaaaaa    960
gtaacgtttg ctttagactc ccataacaga gaaaaacaac gcaattccct agcggcgtat   1020
agtcagcaaa ggtcaacaaa aacattccat gcattcattt gtgaatgctt atag         1074
```

<210> 109
<211> 335
<212> PRT
<213> Chlamydia pneumoniae

<400> 109

```
Met Val Arg Arg Ser Ile Ser Phe Cys Leu Phe Phe Leu Met Thr Leu
1               5                   10                  15

Leu Cys Cys Thr Ser Cys Asn Ser Arg Ser Leu Ile Val His Gly Leu
            20                  25                  30

Pro Gly Arg Glu Ala Asn Glu Ile Val Val Leu Leu Val Ser Lys Gly
        35                  40                  45

Val Ala Ala Gln Lys Leu Pro Gln Ala Ala Ala Ala Thr Ala Gly Ala
    50                  55                  60

Ala Thr Glu Gln Met Trp Asp Ile Ala Val Pro Ser Ala Gln Ile Thr
65                  70                  75                  80

Glu Ala Leu Ala Ile Leu Asn Gln Ala Gly Leu Pro Arg Met Lys Gly
                85                  90                  95

Thr Ser Leu Leu Asp Leu Phe Ala Lys Gln Gly Leu Val Pro Ser Glu
            100                 105                 110

Leu Gln Glu Lys Ile Arg Tyr Gln Glu Gly Leu Ser Glu Gln Met Ala
        115                 120                 125

Ser Thr Ile Arg Lys Met Asp Gly Val Val Asp Ala Ser Val Gln Ile
    130                 135                 140

Ser Phe Thr Thr Glu Asn Glu Asp Asn Leu Pro Leu Thr Ala Ser Val
145                 150                 155                 160

Tyr Ile Lys His Arg Gly Val Leu Asp Asn Pro Asn Ser Ile Met Val
                165                 170                 175

Ser Lys Ile Lys Arg Leu Ile Ala Ser Ala Val Pro Gly Leu Val Pro
            180                 185                 190

Glu Asn Val Ser Val Val Ser Asp Arg Ala Ala Tyr Ser Asp Ile Thr
        195                 200                 205

Ile Asn Gly Pro Trp Gly Leu Thr Glu Glu Ile Asp Tyr Val Ser Val
    210                 215                 220

Trp Gly Ile Ile Leu Ala Lys Ser Ser Leu Thr Lys Phe Arg Leu Ile
```

225 230 235 240

Phe Tyr Val Leu Ile Leu Ile Leu Phe Val Ile Ser Cys Gly Leu Leu
245 250 255

Trp Val Ile Trp Lys Thr His Thr Leu Ile Met Thr Met Gly Gly Thr
260 265 270

Lys Gly Phe Phe Asn Pro Thr Pro Tyr Thr Lys Asn Ala Leu Glu Ala
275 280 285

Lys Lys Ala Glu Gly Ala Ala Ala Asp Lys Glu Lys Lys Glu Asp Ala
290 295 300

Asp Ser Gln Gly Glu Ser Lys Asn Ala Glu Thr Ser Asp Lys Asp Ser
305 310 315 320

Ser Asp Lys Asp Ala Pro Glu Gly Ser Asn Glu Ile Glu Gly Ala
325 330 335

<210> 110
<211> 1008
<212> DNA
<213> Chlamydia pneumoniae

<400> 110
atggttcgtc gatctatttc tttttgcttg ttctttctaa tgacattgct gtgctgtaca 60
agctgtaaca gcaggtctct aattgtgcac ggtcttcctg gcagagaagc gaatgagatt 120
gtggtgcttt tggtaagcaa aggggtggct gcacaaaaat tgcctcaagc tgcagcggct 180
acagccggag cagctactga gcaaatgtgg gatatcgcgg ttccgtcagc acaaatcaca 240
gaggcccttg ccattctaaa tcaagcgggt cttccacgta tgaaagggac aagcctgtta 300
gatcttttg caaaacaagg tcttgttcct tccgagcttc aggaaaaaat ccgttatcaa 360
gaaggcttat cagaacagat ggcctctacg attagaaaaa tggatggcgt tgtcgatgcc 420
tcagtacaga tttccttcac tacagaaaat gaagataatc ttcctttaac agcctctgtg 480
tatattaagc atcgaggggt tttggacaat ccgaacagca ttatggtttc caaaattaag 540
cgccttattg caagtgctgt tccaggactt gtgccagaga acgtctctgt agtgagcgat 600
cgcgcagctt atagtgatat tacaattaat ggtccttggg gattaacaga agaaatcgat 660
tatgtttctg tttggggtat tattcttgcg aagtcttcgc tcaccaaatt ccgtctcatt 720
ttttatgtct tgattctcat tttatttgtt atttcttgtg gtctcctttg ggtcatttgg 780
aaaactcata ctctcattat gactatggga ggtacaaaag ggttcttcaa ccctacacca 840
tatacaaaga atgccttgga agccaagaaa gccgagggag cagctgctga caaagagaaa 900
aaagaagatg cagattcaca gggggaaagc aaaaatgcgg aaaccagtga taaagactct 960
agtgataaag atgctccaga aggaagcaat gaaattgagg gtgcttag 1008

<210> 111
<211> 488
<212> PRT
<213> Chlamydia pneumoniae

<400> 111
Met Ile Thr Lys Gln Leu Arg Ser Trp Leu Ala Val Leu Val Gly Ser
1 5 10 15

Ser Leu Leu Ala Leu Pro Leu Ser Gly Gln Ala Val Gly Lys Lys Glu
20 25 30

Ser Arg Val Ser Glu Leu Pro Gln Asp Val Leu Leu Lys Glu Ile Ser
35 40 45

Gly Gly Phe Ser Lys Val Ala Thr Lys Ala Thr Pro Ala Val Val Tyr
50 55 60

```
Ile Glu Ser Phe Pro Lys Ser Gln Ala Val Thr His Pro Ser Pro Gly
65                  70                  75                  80

Arg Arg Gly Pro Tyr Glu Asn Pro Phe Asp Tyr Phe Asn Asp Glu Phe
                85                  90                  95

Phe Asn Arg Phe Phe Gly Leu Pro Ser Gln Arg Glu Lys Pro Gln Ser
            100                 105                 110

Lys Glu Ala Val Arg Gly Thr Gly Phe Leu Val Ser Pro Asp Gly Tyr
        115                 120                 125

Ile Val Thr Asn Asn His Val Val Glu Asp Thr Gly Lys Ile His Val
    130                 135                 140

Thr Leu His Asp Gly Gln Lys Tyr Pro Ala Thr Val Ile Gly Leu Asp
145                 150                 155                 160

Pro Lys Thr Asp Leu Ala Val Ile Lys Ile Lys Ser Gln Asn Leu Pro
                165                 170                 175

Tyr Leu Ser Phe Gly Asn Ser Asp His Leu Lys Val Gly Asp Trp Ala
            180                 185                 190

Ile Ala Ile Gly Asn Pro Phe Gly Leu Gln Ala Thr Val Thr Val Gly
        195                 200                 205

Val Ile Ser Ala Lys Gly Arg Asn Gln Leu His Ile Ala Asp Phe Glu
    210                 215                 220

Asp Phe Ile Gln Thr Asp Ala Ala Ile Asn Pro Gly Asn Ser Gly Gly
225                 230                 235                 240

Pro Leu Leu Asn Ile Asp Gly Gln Val Ile Gly Val Asn Thr Ala Ile
                245                 250                 255

Val Ser Gly Ser Gly Gly Tyr Ile Gly Ile Gly Phe Ala Ile Pro Ser
            260                 265                 270

Leu Met Ala Asn Arg Ile Ile Asp Gln Leu Ile Arg Asp Gly Gln Val
        275                 280                 285

Thr Arg Gly Phe Leu Gly Val Thr Leu Gln Pro Ile Asp Ala Glu Leu
    290                 295                 300

Ala Ala Cys Tyr Lys Leu Glu Lys Val Tyr Gly Ala Leu Val Thr Asp
305                 310                 315                 320

Val Val Lys Gly Ser Pro Ala Asp Lys Ala Gly Leu Lys Gln Glu Asp
                325                 330                 335

Val Ile Ile Ala Tyr Asn Gly Lys Glu Val Asp Ser Leu Ser Met Phe
            340                 345                 350

Arg Asn Ala Val Ser Leu Met Asn Pro Asp Thr Arg Ile Val Leu Lys
        355                 360                 365

Val Val Arg Glu Gly Lys Val Ile Glu Ile Pro Val Thr Val Ser Gln
    370                 375                 380
```

Ala Pro Lys Glu Asp Gly Met Ser Ala Leu Gln Arg Val Gly Ile Arg
385 390 395 400

Val Gln Asn Leu Thr Pro Glu Thr Ala Lys Lys Leu Gly Ile Ala Pro
405 410 415

Glu Thr Lys Gly Ile Leu Ile Ile Ser Val Glu Pro Gly Ser Val Ala
420 425 430

Ala Ser Ser Gly Ile Ala Pro Gly Gln Leu Ile Leu Ala Val Asn Arg
435 440 445

Gln Lys Val Ser Ser Ile Glu Asp Leu Asn Arg Thr Leu Lys Asp Ser
450 455 460

Asn Asn Glu Asn Ile Leu Leu Met Val Ser Gln Gly Asp Val Ile Arg
465 470 475 480

Phe Ile Ala Leu Lys Pro Glu Glu
485

<210> 112
<211> 1467
<212> DNA
<213> Chlamydia pneumoniae

<400> 112
atgataacta agcaattgcg ttcgtggcta gctgtacttg ttggttcaag tctgctagct 60
cttcctttat cagggcaagc tgtcgggaaa aaagaatctc gagtttccga gctgcctcaa 120
gacgttcttc ttaaagagat ctcgggaggg ttttctaagg tcgctaccaa ggcgactccc 180
gctgttgtgt acatagaaag tttcccaaag agccaggctg taacacatcc ttctcctgga 240
cgccgtgggc cttatgaaaa tccttttgat tattttaatg atgagttttt caatcgtttt 300
tttggtctac cttcacagag ggaaaaacct caaagtaaag aggcggttcg aggaacaggt 360
ttcctagtat ctccagatgg ctatattgtg actaataacc atgttgtcga agatacaggt 420
aagattcacg taactcttca tgatgggcaa aagtacccag caactgtaat cggactcgat 480
cctaaaacag accttgcagt cattaaaatt aaatcccaaa acctcccgta tctttctttt 540
ggaaactccg accacttaaa agtcggagat tgggcaattg caattggaaa tcccttcggt 600
cttcaagcta cggtcaccgt aggtgtcatc agtgctaaag gaagaaatca actccacatt 660
gcagattttg aagattttat tcagacagat gctgcgatta atccaggcaa ctctggaggc 720
cctcttctaa atattgatgg acaggtcatc ggtgttaata ctgccattgt cagtggtagt 780
ggtggctata ttggaatcgg gtttgcgatt cctagcctta tggcaaatag aatcatagat 840
cagctgattc gtgatggtca agttacccga ggattcttag gagtgacttt acaacctata 900
gatgcggaac tcgctgcttg ctacaaactc gaaaaggttt atggcgcttt agtcacagat 960
gttgttaaag gatctccagc agataaagca gggctaaaac aagaagatgt gatcattgct 1020
tataatggga aagaagtcga ttcactgagt atgttccgta atgctgtttc tttaatgaat 1080
ccagatacac gtattgttct aaaggtagtt cgtgaaggaa aggttatcga aatacccgtg 1140
acagtttctc aagctccaaa agaagatgga atgtcggctt tacagcgtgt gggaatccgt 1200
gtgcaaaacc taactcctga aactgctaag aagctgggaa ttgctccaga gactaaaggc 1260
attttgatta taagtgttga accagggtct gtagcagctt cttcaggaat tgctcctggt 1320
cagctgatcc ttgctgtgaa tagacaaaaa gtatcttcga ttgaagatct gaatagaacg 1380
ttaaaagatt ctaacaatga gaatattctt cttatggttt ctcaaggaga tgttattcgc 1440
ttcattgccc tgaaacctga agaataa 1467

<210> 113
<211> 180
<212> PRT
<213> Chlamydia pneumoniae

<400> 113
Met Cys Asn Ser Ile Ala Met Lys Lys Gln Lys Arg Gly Phe Val Leu
1 5 10 15

Met Glu Leu Leu Met Ser Phe Thr Leu Ile Ala Leu Leu Leu Gly Thr
20 25 30

Leu Gly Phe Trp Tyr Arg Lys Ile Tyr Thr Val Gln Lys Gln Lys Glu
35 40 45

Arg Ile Tyr Asn Phe Tyr Ile Glu Glu Ser Arg Ala Tyr Lys Gln Leu
50 55 60

Arg Thr Leu Phe Ser Met Ser Leu Ser Ser Ser Tyr Glu Glu Pro Gly
65 70 75 80

Ser Leu Phe Ser Leu Ile Phe Asp Arg Gly Val Tyr Arg Asp Pro Lys
85 90 95

Leu Ala Gly Ala Val Arg Ala Ser Leu His His Asp Thr Lys Asp Gln
100 105 110

Arg Leu Glu Leu Arg Ile Cys Asn Ile Lys Asp Gln Ser Tyr Phe Glu
115 120 125

Thr Gln Arg Leu Leu Ser His Val Thr His Val Val Leu Ser Phe Gln
130 135 140

Arg Asn Pro Asp Pro Glu Lys Leu Pro Glu Thr Ile Ala Leu Thr Ile
145 150 155 160

Thr Arg Glu Pro Lys Ala Tyr Pro Pro Arg Thr Leu Thr Tyr Gln Phe
165 170 175

Ala Val Gly Lys
180

<210> 114
<211> 543
<212> DNA
<213> Chlamydia pneumoniae

<400> 114
atgtgtaact ctatagctat gaaaaagcaa aagcgtggct ttgtgcttat ggaattactc 60
atgtcgttca ctctaattgc tttgttatta gggactttag gattttggta tcggaaaatt 120
tatactgtac aaaagcaaaa agaacgtatt tataactttt atatcgaaga aagccgagcc 180
tacaagcagc tcagaaccct gtttagcatg tccttgtctt catcttacga ggagcctgga 240
tcattatttt ctttaatctt tgatcggggt gtttatcgag atcctaagct ggcaggtgcg 300
gtacgagctt ctctccatca tgacaccaag gatcagagat tggaacttcg tatttgtaat 360
attaaggatc agtcttactt tgaaacacag cgactgctct cccacgtgac ccatgttgta 420
ctttccttcc agagaaatcc tgatcctgaa aaacttcctg aaacaattgc tttaactata 480
acacgggaac ctaaagcata tcctccaagg acgttaacat accaatttgc ggttgggaaa 540
taa 543

<210> 115
<211> 928
<212> PRT
<213> Chlamydia pneumoniae

<400> 115
Met Lys Thr Ser Ile Pro Trp Val Leu Val Ser Ser Val Leu Ala Phe
1 5 10 15

Ser Cys His Leu Gln Ser Leu Ala Asn Glu Glu Leu Leu Ser Pro Asp

```
            20                  25                  30

Asp Ser Phe Asn Gly Asn Ile Asp Ser Gly Thr Phe Thr Pro Lys Thr
        35                  40                  45

Ser Ala Thr Thr Tyr Ser Leu Thr Gly Asp Val Phe Phe Tyr Glu Pro
    50                  55                  60

Gly Lys Gly Thr Pro Leu Ser Asp Ser Cys Phe Lys Gln Thr Thr Asp
65                  70                  75                  80

Asn Leu Thr Phe Leu Gly Asn Gly His Ser Leu Thr Phe Gly Phe Ile
                85                  90                  95

Asp Ala Gly Thr His Ala Gly Ala Ala Ala Ser Thr Thr Ala Asn Lys
            100                 105                 110

Asn Leu Thr Phe Ser Gly Phe Ser Leu Leu Ser Phe Asp Ser Ser Pro
        115                 120                 125

Ser Thr Thr Val Thr Thr Gly Gln Gly Thr Leu Ser Ser Ala Gly Gly
    130                 135                 140

Val Asn Leu Glu Asn Ile Arg Lys Leu Val Val Ala Gly Asn Phe Ser
145                 150                 155                 160

Thr Ala Asp Gly Gly Ala Ile Lys Gly Ala Ser Phe Leu Leu Thr Gly
                165                 170                 175

Thr Ser Gly Asp Ala Leu Phe Ser Asn Asn Ser Ser Ser Thr Lys Gly
            180                 185                 190

Gly Ala Ile Ala Thr Thr Ala Gly Ala Arg Ile Ala Asn Asn Thr Gly
        195                 200                 205

Tyr Val Arg Phe Leu Ser Asn Ile Ala Ser Thr Ser Gly Gly Ala Ile
    210                 215                 220

Asp Asp Glu Gly Thr Ser Ile Leu Ser Asn Asn Lys Phe Leu Tyr Phe
225                 230                 235                 240

Glu Gly Asn Ala Ala Lys Thr Thr Gly Gly Ala Ile Cys Asn Thr Lys
                245                 250                 255

Ala Ser Gly Ser Pro Glu Leu Ile Ile Ser Asn Asn Lys Thr Leu Ile
            260                 265                 270

Phe Ala Ser Asn Val Ala Glu Thr Ser Gly Gly Ala Ile His Ala Lys
        275                 280                 285

Lys Leu Ala Leu Ser Ser Gly Gly Phe Thr Glu Phe Leu Arg Asn Asn
    290                 295                 300

Val Ser Ser Ala Thr Pro Lys Gly Gly Ala Ile Ser Ile Asp Ala Ser
305                 310                 315                 320

Gly Glu Leu Ser Leu Ser Ala Glu Thr Gly Asn Ile Thr Phe Val Arg
                325                 330                 335

Asn Thr Leu Thr Thr Thr Gly Ser Thr Asp Thr Pro Lys Arg Asn Ala
            340                 345                 350
```

```
Ile Asn Ile Gly Ser Asn Gly Lys Phe Thr Glu Leu Arg Ala Ala Lys
        355                 360                 365

Asn His Thr Ile Phe Phe Tyr Asp Pro Ile Thr Ser Glu Gly Thr Ser
    370                 375                 380

Ser Asp Val Leu Lys Ile Asn Asn Gly Ser Ala Gly Ala Leu Asn Pro
385                 390                 395                 400

Tyr Gln Gly Thr Ile Leu Phe Ser Gly Glu Thr Leu Thr Ala Asp Glu
                405                 410                 415

Leu Lys Val Ala Asp Asn Leu Lys Ser Ser Phe Thr Gln Pro Val Ser
            420                 425                 430

Leu Ser Gly Gly Lys Leu Leu Leu Gln Lys Gly Val Thr Leu Glu Ser
        435                 440                 445

Thr Ser Phe Ser Gln Glu Ala Gly Ser Leu Leu Gly Met Asp Ser Gly
    450                 455                 460

Thr Thr Leu Ser Thr Thr Ala Gly Ser Ile Thr Ile Thr Asn Leu Gly
465                 470                 475                 480

Ile Asn Val Asp Ser Leu Gly Leu Lys Gln Pro Val Ser Leu Thr Ala
                485                 490                 495

Lys Gly Ala Ser Asn Lys Val Ile Val Ser Gly Lys Leu Asn Leu Ile
            500                 505                 510

Asp Ile Glu Gly Asn Ile Tyr Glu Ser His Met Phe Ser His Asp Gln
        515                 520                 525

Leu Phe Ser Leu Leu Lys Ile Thr Val Asp Ala Asp Val Asp Thr Asn
    530                 535                 540

Val Asp Ile Ser Ser Leu Ile Pro Val Pro Ala Glu Asp Pro Asn Ser
545                 550                 555                 560

Glu Tyr Gly Phe Gln Gly Gln Trp Asn Val Asn Trp Thr Thr Asp Thr
                565                 570                 575

Ala Thr Asn Thr Lys Glu Ala Thr Ala Thr Trp Thr Lys Thr Gly Phe
            580                 585                 590

Val Pro Ser Pro Glu Arg Lys Ser Ala Leu Val Cys Asn Thr Leu Trp
        595                 600                 605

Gly Val Phe Thr Asp Ile Arg Ser Leu Gln Gln Leu Val Glu Ile Gly
    610                 615                 620

Ala Thr Gly Met Glu His Lys Gln Gly Phe Trp Val Ser Ser Met Thr
625                 630                 635                 640

Asn Phe Leu His Lys Thr Gly Asp Glu Asn Arg Lys Gly Phe Arg His
                645                 650                 655

Thr Ser Gly Gly Tyr Val Ile Gly Gly Ser Ala His Thr Pro Lys Asp
            660                 665                 670
```

Asp Leu Phe Thr Phe Ala Phe Cys His Leu Phe Ala Arg Asp Lys Asp
    675                 680                 685

Cys Phe Ile Ala His Asn Asn Ser Arg Thr Tyr Gly Gly Thr Leu Phe
    690                 695                 700

Phe Lys His Ser His Thr Leu Gln Pro Gln Asn Tyr Leu Arg Leu Gly
705                 710                 715                 720

Arg Ala Lys Phe Ser Glu Ser Ala Ile Glu Lys Phe Pro Arg Glu Ile
                725                 730                 735

Pro Leu Ala Leu Asp Val Gln Val Ser Phe Ser His Ser Asp Asn Arg
            740                 745                 750

Met Glu Thr His Tyr Thr Ser Leu Pro Glu Ser Glu Gly Ser Trp Ser
        755                 760                 765

Asn Glu Cys Ile Ala Gly Gly Ile Gly Leu Asp Leu Pro Phe Val Leu
    770                 775                 780

Ser Asn Pro His Pro Leu Phe Lys Thr Phe Ile Pro Gln Met Lys Val
785                 790                 795                 800

Glu Met Val Tyr Val Ser Gln Asn Ser Phe Phe Glu Ser Ser Ser Asp
                805                 810                 815

Gly Arg Gly Phe Ser Ile Gly Arg Leu Leu Asn Leu Ser Ile Pro Val
            820                 825                 830

Gly Ala Lys Phe Val Gln Gly Asp Ile Gly Asp Ser Tyr Thr Tyr Asp
        835                 840                 845

Leu Ser Gly Phe Phe Val Ser Asp Val Tyr Arg Asn Asn Pro Gln Ser
    850                 855                 860

Thr Ala Thr Leu Val Met Ser Pro Asp Ser Trp Lys Ile Arg Gly Gly
865                 870                 875                 880

Asn Leu Ser Arg Gln Ala Phe Leu Leu Arg Gly Ser Asn Asn Tyr Val
                885                 890                 895

Tyr Asn Ser Asn Cys Glu Leu Phe Gly His Tyr Ala Met Glu Leu Arg
            900                 905                 910

Gly Ser Ser Arg Asn Tyr Asn Val Asp Val Gly Thr Lys Leu Arg Phe
        915                 920                 925

<210> 116
<211> 2787
<212> DNA
<213> Chlamydia pneumoniae

<400> 116
atgaagactt cgattccttg ggttttagtt tcctccgtgt tagctttctc atgtcaccta 60
cagtcactag ctaacgagga acttttatca cctgatgata gctttaatgg aaatatcgat 120
tcaggaacgt ttactccaaa aacttcagcc acaacatatt ctctaacagg agatgtcttc 180
ttttacgagc ctggaaaagg cactccctta tctgacagtt gttttaagca aaccacggac 240
aatcttacct tcttggggaa cggtcatagc ttaacgtttg gctttataga tgctggcact 300
catgcaggtg ctgctgcatc tacaacagca aataagaatc ttaccttctc agggttttcc 360

```
ttactgagtt ttgattcctc tcctagcaca acggttacta caggtcaggg aacgctttcc    420
tcagcaggag gcgtaaattt agaaaatatt cgtaaacttg tagttgctgg gaatttttct    480
actgcagatg gtggagctat caaaggagcg tctttccttt taactggcac ttctggagat    540
gctcttttta gtaacaactc ttcatcaaca aagggaggag caattgctac tacagcaggc    600
gctcgcatag caaataacac aggttatgtt agattcctat ctaacatagc gtctacgtca    660
ggaggcgcta tcgatgatga aggcacgtcg atactatcga acaacaaatt tctatatttt    720
gaagggaatg cagcgaaaac tactggcggt gcgatctgca acaccaaggc gagtggatct    780
cctgaactga taatctctaa caataagact ctgatctttg cttcaaacgt agcagaaaca    840
agcggtggcg ccatccatgc taaaaagcta gccctttcct ctggaggctt tacagagttt    900
ctacgaaata atgtctcatc agcaactcct aagggggggtg ctatcagcat cgatgcctca    960
ggagagctca gtctttctgc agagacagga aacattacct ttgtaagaaa tacccttaca   1020
acaaccggaa gtaccgatac tcctaaacgt aatgcgatca acataggaag taacgggaaa   1080
ttcacggaat tacgggctgc taaaaatcat acaattttct tctatgatcc catcacttca   1140
gaaggaacct catcagacgt attgaagata aataacggct ctgcgggagc tctcaatcca   1200
tatcaaggaa cgattctatt ttctggagaa accctaacag cagatgaact taaagttgct   1260
gacaatttaa aatcttcatt cacgcagcca gtctccctat ccggaggaaa gttattgcta   1320
caaaagggag tcactttaga gagcacgagc ttctctcaag aggccggttc tctcctcggc   1380
atggattcag gaacgacatt atcaactaca gctgggagta ttacaatcac gaacctagga   1440
atcaatgttg actccttagg tcttaagcag cccgtcagcc taacagcaaa aggtgcttca   1500
aataaagtga tcgtatctgg gaagctcaac ctgattgata ttgaagggaa catttatgaa   1560
agtcatatgt tcagccatga ccagctcttc tctctattaa aatcacggt tgatgctgat    1620
gttgatacta acgttgacat cagcagcctt atccctgttc ctgctgagga tcctaattca   1680
gaatacggat tccaaggaca atggaatgtt aattggacta cggatacagc tacaaataca   1740
aaagaggcca cggcaacttg gaccaaaaca ggatttgttc ccagccccga aagaaaatct   1800
gcgttagtat gcaataccct atggggagtc tttactgaca ttcgctctct gcaacagctt   1860
gtagagatcg gcgcaactgg tatggaacac aaacaaggtt tctgggtttc ctccatgacg   1920
aacttcctgc ataagactgg agatgaaaat cgcaaaggct tccgtcatac ctctggaggc   1980
tacgtcatcg gtggaagtgc tcacactcct aaagacgacc tatttacctt tgcgttctgc   2040
catctctttg ctagagacaa agattgtttt atcgctcaca acaactctag aacctacggt   2100
ggaactttat tcttcaagca ctctcatacc ctacaacccc aaaactattt gagattagga   2160
agagcaaagt tttctgaatc agctatagaa aaattcccta gggaaattcc cctagccttg   2220
gatgtccaag tttcgttcag ccattcagac aaccgtatgg aaacgcacta tacctcattg   2280
ccagaatccg aaggttcttg gagcaacgag tgtatagctg gtggtatcgg cctagacctt   2340
ccttttgttc tttccaaccc acatcctctt ttcaagacct tcattccaca gatgaaagtc   2400
gaaatggttt atgtatcaca aaatagcttc ttcgaaagct ctagtgatgg ccgtggtttt   2460
agtattggaa ggctgcttaa cctctcgatt cctgtgggtg cgaaattcgt gcagggggat   2520
atcggagatt cctacaccta tgatctctca ggattctttg tttccgatgt ctatcgtaac   2580
aatccccaat ctacagcgac tcttgtgatg agcccagact cttggaaaat tcgcggtggc   2640
aatctttcaa gacaggcatt tttactgagg ggtagcaaca actacgtcta caactccaat   2700
tgtgagctct tcggacatta cgctatggaa ctccgtggat cttcaaggaa ctacaatgta   2760
gatgttggta ccaaactccg attctag                                       2787
```

<210> 117
<211> 254
<212> PRT
<213> Chlamydia pneumoniae

<400> 117

```
Met His Asp Ala Leu Leu Ser Ile Leu Ala Ile Gln Glu Leu Asp Ile
1               5                   10                  15

Lys Met Ile Arg Leu Met Arg Val Lys Lys Glu His Gln Lys Glu Leu
            20                  25                  30

Ala Lys Val Gln Ser Leu Lys Ser Asp Ile Arg Arg Lys Val Gln Glu
        35                  40                  45

Lys Glu Leu Glu Met Glu Asn Leu Lys Thr Gln Ile Arg Asp Gly Glu
    50                  55                  60

Asn Arg Ile Gln Glu Ile Ser Glu Gln Ile Asn Lys Leu Glu Asn Gln
```

65 70 75 80

Gln Ala Ala Val Lys Lys Met Asp Glu Phe Asn Ala Leu Thr Gln Glu
85 90 95

Met Thr Thr Ala Asn Lys Glu Arg Arg Ser Leu Glu His Gln Leu Ser
100 105 110

Asp Leu Met Asp Lys Gln Ala Gly Gly Glu Asp Leu Ile Val Ser Leu
115 120 125

Lys Glu Ser Leu Ala Ser Thr Glu Asn Ser Ser Ser Val Ile Glu Lys
130 135 140

Glu Ile Phe Glu Ser Ile Lys Lys Ile Asn Glu Glu Gly Lys Ala Leu
145 150 155 160

Leu Glu Gln Arg Thr Glu Leu Lys His Ala Thr Asn Pro Glu Leu Leu
165 170 175

Ser Ile Tyr Glu Arg Leu Leu Asn Asn Lys Lys Asp Arg Val Val Val
180 185 190

Pro Ile Glu Asn Arg Val Cys Ser Gly Cys His Ile Val Leu Thr Pro
195 200 205

Gln His Glu Asn Leu Val Arg Lys Lys Asp Arg Leu Ile Phe Cys Glu
210 215 220

His Cys Ser Arg Ile Leu Tyr Trp Gln Glu Ser Gln Val Asn Ala Gln
225 230 235 240

Glu Asn Ser Thr Ala Lys Arg Arg Arg Arg Arg Ala Ala Val
245 250

<210> 118
<211> 765
<212> DNA
<213> Chlamydia pneumoniae

<400> 118
atgcatgacg cacttctaag catttttggct attcaagagc ttgatattaa aatgattcgc 60
cttatgcgcg taaagaaaga acatcagaaa gaattggcta aagtccaatc tttaaaaagt 120
gatattcgta gaaaagttca ggaaaaagaa ctcgaaatgg agaatttgaa aactcaaatt 180
cgagatggag agaatcgcat ccaagagatt tctgaacaaa tcaataaatt agaaaatcag 240
caagctgctg taaaaaaaat ggatgagttt aacgctctta cccaagaaat gactacagca 300
aacaaagaac gtcgctcttt agagcaccag cttagcgatc tcatggataa gcaagctgga 360
ggcgaagacc ttattgtctc tctaaaagaa agcttagctt ctacagaaaa tagtagcagt 420
gtcattgaaa aagaaatttt tgaaagcatc aaaaagatta atgaagaagg caaagctttg 480
cttgaacaac ggacagagtt aaagcatgcg acgaatcccg aactactcag catctatgag 540
cgtctattaa acaataaaaa agatcgcgtt gttgttccta ttgaaaatcg tgtctgcagt 600
ggttgtcata ttgttctaac tcctcaacac gaaaatcttg taagaaagaa agaccgactc 660
attttttgcg aacattgctc tcgaattctc tattggcaag aatcccaagt caatgctcag 720
gaaaattcca cagcaaaacg tcgtcgtcgt cgcgcagctg tataa 765

<210> 119
<211> 1723
<212> PRT
<213> Chlamydia pneumoniae

<400> 119

Met Lys Trp Leu Pro Ala Thr Ala Val Phe Ala Ala Val Leu Pro Ala
1 5 10 15

Leu Thr Ala Phe Gly Asp Pro Ala Ser Val Glu Ile Ser Thr Ser His
20 25 30

Thr Gly Ser Gly Asp Pro Thr Ser Asp Ala Ala Leu Thr Gly Phe Thr
35 40 45

Gln Ser Ser Thr Glu Thr Asp Gly Thr Thr Tyr Thr Ile Val Gly Asp
50 55 60

Ile Thr Phe Ser Thr Phe Thr Asn Ile Pro Val Pro Val Val Thr Pro
65 70 75 80

Asp Ala Asn Asp Ser Ser Ser Asn Ser Ser Lys Gly Gly Ser Ser Ser
85 90 95

Ser Gly Ala Thr Ser Leu Ile Arg Ser Ser Asn Leu His Ser Asp Phe
100 105 110

Asp Phe Thr Lys Asp Ser Val Leu Asp Leu Tyr His Leu Phe Phe Pro
115 120 125

Ser Ala Ser Asn Thr Leu Asn Pro Ala Leu Leu Ser Ser Ser Ser Ser
130 135 140

Gly Gly Ser Ser Ser Ser Ser Ser Ser Ser Ser Ser Gly Ser Ala Ser
145 150 155 160

Ala Val Val Ala Ala Asp Pro Lys Gly Gly Ala Ala Phe Tyr Ser Asn
165 170 175

Glu Ala Asn Gly Thr Leu Thr Phe Thr Thr Asp Ser Gly Asn Pro Gly
180 185 190

Ser Leu Thr Leu Gln Asn Leu Lys Met Thr Gly Asp Gly Ala Ala Ile
195 200 205

Tyr Ser Lys Gly Pro Leu Val Phe Thr Gly Leu Lys Asn Leu Thr Phe
210 215 220

Thr Gly Asn Glu Ser Gln Lys Ser Gly Gly Ala Ala Tyr Thr Glu Gly
225 230 235 240

Ala Leu Thr Thr Gln Ala Ile Val Glu Ala Val Thr Phe Thr Gly Asn
245 250 255

Thr Ser Ala Gly Gln Gly Gly Ala Ile Tyr Val Lys Glu Ala Thr Leu
260 265 270

Phe Asn Ala Leu Asp Ser Leu Lys Phe Glu Lys Asn Thr Ser Gly Gln
275 280 285

Ala Gly Gly Gly Ile Tyr Thr Glu Ser Thr Leu Thr Ile Ser Asn Ile
290 295 300

Thr Lys Ser Ile Glu Phe Ile Ser Asn Lys Ala Ser Val Pro Ala Pro
305 310 315 320

Ala Pro Glu Pro Thr Ser Pro Ala Pro Ser Ser Leu Ile Asn Ser Thr

```
                325                 330                 335

Thr Ile Asp Thr Ser Thr Leu Gln Thr Arg Ala Ala Ser Ala Thr Pro
            340                 345                 350

Ala Val Ala Pro Val Ala Ala Val Thr Pro Thr Pro Ile Ser Thr Gln
        355                 360                 365

Glu Thr Ala Gly Asn Gly Gly Ala Ile Tyr Ala Lys Gln Gly Ile Ser
    370                 375                 380

Ile Ser Thr Phe Lys Asp Leu Thr Phe Lys Ser Asn Ser Ala Ser Val
385                 390                 395                 400

Asp Ala Thr Leu Thr Val Asp Ser Ser Thr Ile Gly Glu Ser Gly Gly
                405                 410                 415

Ala Ile Phe Ala Ala Asp Ser Ile Gln Ile Gln Gln Cys Thr Gly Thr
            420                 425                 430

Thr Leu Phe Ser Gly Asn Thr Ala Asn Lys Ser Gly Gly Gly Ile Tyr
        435                 440                 445

Ala Val Gly Gln Val Thr Leu Glu Asp Ile Ala Asn Leu Lys Met Thr
    450                 455                 460

Asn Asn Thr Cys Lys Gly Glu Gly Gly Ala Ile Tyr Thr Lys Lys Ala
465                 470                 475                 480

Leu Thr Ile Asn Asn Gly Ala Ile Leu Thr Thr Phe Ser Gly Asn Thr
                485                 490                 495

Ser Thr Asp Asn Gly Gly Ala Ile Phe Ala Val Gly Gly Ile Thr Leu
            500                 505                 510

Ser Asp Leu Val Glu Val Arg Phe Ser Lys Asn Lys Thr Gly Asn Tyr
        515                 520                 525

Ser Ala Pro Ile Thr Lys Ala Ala Ser Asn Thr Ala Pro Val Val Ser
    530                 535                 540

Ser Ser Thr Thr Ala Ala Ser Pro Ala Val Pro Ala Ala Ala Ala Ala
545                 550                 555                 560

Pro Val Thr Asn Ala Ala Lys Gly Gly Ala Leu Tyr Ser Thr Glu Gly
                565                 570                 575

Leu Thr Val Ser Gly Ile Thr Ser Ile Leu Ser Phe Glu Asn Asn Glu
            580                 585                 590

Cys Gln Asn Gln Gly Gly Gly Ala Tyr Val Thr Lys Thr Phe Gln Cys
        595                 600                 605

Ser Asp Ser His Arg Leu Gln Phe Thr Ser Asn Lys Ala Ala Asp Glu
    610                 615                 620

Gly Gly Gly Leu Tyr Cys Gly Asp Asp Val Thr Leu Thr Asn Leu Thr
625                 630                 635                 640

Gly Lys Thr Leu Phe Gln Glu Asn Ser Ser Glu Lys His Gly Gly Gly
                645                 650                 655
```

Leu Ser Leu Ala Ser Gly Lys Ser Leu Thr Met Thr Ser Leu Glu Ser
660 665 670

Phe Cys Leu Asn Ala Asn Thr Ala Lys Glu Asn Gly Gly Gly Ala Asn
675 680 685

Val Pro Glu Asn Ile Val Leu Thr Phe Thr Tyr Thr Pro Thr Pro Asn
690 695 700

Glu Pro Ala Pro Val Gln Gln Pro Val Tyr Gly Glu Ala Leu Val Thr
705 710 715 720

Gly Asn Thr Ala Thr Lys Ser Gly Gly Gly Ile Tyr Thr Lys Asn Ala
725 730 735

Ala Phe Ser Asn Leu Ser Ser Val Thr Phe Asp Gln Asn Thr Ser Ser
740 745 750

Glu Asn Gly Gly Ala Leu Leu Thr Gln Lys Ala Ala Asp Lys Thr Asp
755 760 765

Cys Ser Phe Thr Tyr Ile Thr Asn Val Asn Ile Thr Asn Asn Thr Ala
770 775 780

Thr Gly Asn Gly Gly Gly Ile Ala Gly Gly Lys Ala His Phe Asp Arg
785 790 795 800

Ile Asp Asn Leu Thr Val Gln Ser Asn Gln Ala Lys Lys Gly Gly Gly
805 810 815

Val Tyr Leu Glu Asp Ala Leu Ile Leu Glu Lys Val Ile Thr Gly Ser
820 825 830

Val Ser Gln Asn Thr Ala Thr Glu Ser Gly Gly Gly Ile Tyr Ala Lys
835 840 845

Asp Ile Gln Leu Gln Ala Leu Pro Gly Ser Phe Thr Ile Thr Asp Asn
850 855 860

Lys Val Glu Thr Ser Leu Thr Thr Ser Thr Asn Leu Tyr Gly Gly Gly
865 870 875 880

Ile Tyr Ser Ser Gly Ala Val Thr Leu Thr Asn Ile Ser Gly Thr Phe
885 890 895

Gly Ile Thr Gly Asn Ser Val Ile Asn Thr Ala Thr Ser Gln Asp Ala
900 905 910

Asp Ile Gln Gly Gly Gly Ile Tyr Ala Thr Thr Ser Leu Ser Ile Asn
915 920 925

Gln Cys Asn Thr Pro Ile Leu Phe Ser Asn Asn Ser Ala Ala Thr Lys
930 935 940

Lys Thr Ser Thr Thr Lys Gln Ile Ala Gly Gly Ala Ile Phe Ser Ala
945 950 955 960

Ala Val Thr Ile Glu Asn Asn Ser Gln Pro Ile Ile Phe Leu Asn Asn
965 970 975

Ser Ala Lys Ser Glu Ala Thr Thr Ala Ala Thr Ala Gly Asn Lys Asp
980 985 990

Ser Cys Gly Gly Ala Ile Ala Ala Asn Ser Val Thr Leu Thr Asn Asn
995 1000 1005

Pro Glu Ile Thr Phe Lys Gly Asn Tyr Ala Glu Thr Gly Gly Ala Ile
1010 1015 1020

Gly Cys Ile Asp Leu Thr Asn Gly Ser Pro Pro Arg Lys Val Ser Ile
1025 1030 1035 1040

Ala Asp Asn Gly Ser Val Leu Phe Gln Asp Asn Ser Ala Leu Asn Arg
1045 1050 1055

Gly Gly Ala Ile Tyr Gly Glu Thr Ile Asp Ile Ser Arg Thr Gly Ala
1060 1065 1070

Thr Phe Ile Gly Asn Ser Ser Lys His Asp Gly Ser Ala Ile Cys Cys
1075 1080 1085

Ser Thr Ala Leu Thr Leu Ala Pro Asn Ser Gln Leu Ile Phe Glu Asn
1090 1095 1100

Asn Lys Val Thr Glu Thr Thr Ala Thr Thr Lys Ala Ser Ile Asn Asn
1105 1110 1115 1120

Leu Gly Ala Ala Ile Tyr Gly Asn Asn Glu Thr Ser Asp Val Thr Ile
1125 1130 1135

Ser Leu Ser Ala Glu Asn Gly Ser Ile Phe Phe Lys Asn Asn Leu Cys
1140 1145 1150

Thr Ala Thr Asn Lys Tyr Cys Ser Ile Ala Gly Asn Val Lys Phe Thr
1155 1160 1165

Ala Ile Glu Ala Ser Ala Gly Lys Ala Ile Ser Phe Tyr Asp Ala Val
1170 1175 1180

Asn Val Ser Thr Lys Glu Thr Asn Ala Gln Glu Leu Lys Leu Asn Glu
1185 1190 1195 1200

Lys Ala Thr Ser Thr Gly Thr Ile Leu Phe Ser Gly Glu Leu His Glu
1205 1210 1215

Asn Lys Ser Tyr Ile Pro Gln Lys Val Thr Phe Ala His Gly Asn Leu
1220 1225 1230

Ile Leu Gly Lys Asn Ala Glu Leu Ser Val Val Ser Phe Thr Gln Ser
1235 1240 1245

Pro Gly Thr Thr Ile Thr Met Gly Pro Gly Ser Val Leu Ser Asn His
1250 1255 1260

Ser Lys Glu Ala Gly Gly Ile Ala Ile Asn Asn Val Ile Ile Asp Phe
1265 1270 1275 1280

Ser Glu Ile Val Pro Thr Lys Asp Asn Ala Thr Val Ala Pro Pro Thr
1285 1290 1295

Leu Lys Leu Val Ser Arg Thr Asn Ala Asp Ser Lys Asp Lys Ile Asp

1300 1305 1310

Ile Thr Gly Thr Val Thr Leu Leu Asp Pro Asn Gly Asn Leu Tyr Gln
1315 1320 1325

Asn Ser Tyr Leu Gly Glu Asp Arg Asp Ile Thr Leu Phe Asn Ile Asp
1330 1335 1340

Asn Ser Ala Ser Gly Ala Val Thr Ala Thr Asn Val Thr Leu Gln Gly
1345 1350 1355 1360

Asn Leu Gly Ala Lys Lys Gly Tyr Leu Gly Thr Trp Asn Leu Asp Pro
1365 1370 1375

Asn Ser Ser Gly Ser Lys Ile Ile Leu Lys Trp Thr Phe Asp Lys Tyr
1380 1385 1390

Leu Arg Trp Pro Tyr Ile Pro Arg Asp Asn His Phe Tyr Ile Asn Ser
1395 1400 1405

Ile Trp Gly Ala Gln Asn Ser Leu Val Thr Val Lys Gln Gly Ile Leu
1410 1415 1420

Gly Asn Met Leu Asn Asn Ala Arg Phe Glu Asp Pro Ala Phe Asn Asn
1425 1430 1435 1440

Phe Trp Ala Ser Ala Ile Gly Ser Phe Leu Arg Lys Glu Val Ser Arg
1445 1450 1455

Asn Ser Asp Ser Phe Thr Tyr His Gly Arg Gly Tyr Thr Ala Ala Val
1460 1465 1470

Asp Ala Lys Pro Arg Gln Glu Phe Ile Leu Gly Ala Ala Phe Ser Gln
1475 1480 1485

Val Phe Gly His Ala Glu Ser Glu Tyr His Leu Asp Asn Tyr Lys His
1490 1495 1500

Lys Gly Ser Gly His Ser Thr Gln Ala Ser Leu Tyr Ala Gly Asn Ile
1505 1510 1515 1520

Phe Tyr Phe Pro Ala Ile Arg Ser Arg Pro Ile Leu Phe Gln Gly Val
1525 1530 1535

Ala Thr Tyr Gly Tyr Met Gln His Asp Thr Thr Thr Tyr Tyr Pro Ser
1540 1545 1550

Ile Glu Glu Lys Asn Met Ala Asn Trp Asp Ser Ile Ala Trp Leu Phe
1555 1560 1565

Asp Leu Arg Phe Ser Val Asp Leu Lys Glu Pro Gln Pro His Ser Thr
1570 1575 1580

Ala Arg Leu Thr Phe Tyr Thr Glu Ala Glu Tyr Thr Arg Ile Arg Gln
1585 1590 1595 1600

Glu Lys Phe Thr Glu Leu Asp Tyr Asp Pro Arg Ser Phe Ser Ala Cys
1605 1610 1615

Ser Tyr Gly Asn Leu Ala Ile Pro Thr Gly Phe Ser Val Asp Gly Ala
1620 1625 1630

```
Leu Ala Trp Arg Glu Ile Ile Leu Tyr Asn Lys Val Ser Ala Ala Tyr
        1635                1640                1645

Leu Pro Val Ile Leu Arg Asn Asn Pro Lys Ala Thr Tyr Glu Val Leu
    1650                1655                1660

Ser Thr Lys Glu Lys Gly Asn Val Val Asn Val Leu Pro Thr Arg Asn
1665                1670                1675                1680

Ala Ala Arg Ala Glu Val Ser Ser Gln Ile Tyr Leu Gly Ser Tyr Trp
                1685                1690                1695

Thr Leu Tyr Gly Thr Tyr Thr Ile Asp Ala Ser Met Asn Thr Leu Val
            1700                1705                1710

Gln Met Ala Asn Gly Gly Ile Arg Phe Val Phe
        1715                1720

<210>   120
<211>   5172
<212>   DNA
<213>   Chlamydia pneumoniae

<400>   120
atgaagtggc taccagctac agctgttttt gctgccgtac tccccgcact aacagccttc     60
ggagatcccg cgtctgttga aataagtacc agccatacag gatccgggga tcctacaagc    120
gacgctgcct taacaggatt tacacaaagt tccacagaaa ctgacggtac tacctatacc    180
attgtcggtg atatcacctt ctctactttt acgaatattc ctgttcccgt agtaactcca    240
gacgccaacg atagttccag caatagctct aaaggaggaa gtagcagtag tggagctaca    300
tctctaatcc gatcctcaaa cctacactcc gattttgatt ttacaaaaga tagcgtgtta    360
gacctctatc accttttctt tccttcagct tcaaatactc tcaatcctgc actccttttct    420
tccagtagca gcggtggatc ctcgagcagc agtagctcct catcatctgg aagtgcatct    480
gctgttgttg ctgcggaccc aaaaggaggc gctgcctttt atagtaacga ggctaacgga    540
actttaacct tcactacaga ctctggaaat cccggctccc tgactcttca gaatcttaaa    600
atgaccggag atggagccgc catctactcg aagggtcctc tagtatttac tggtttaaaa    660
aatctaacct ttacaggaaa tgaatctcag aaatctggag gtgctgccta tactgaaggc    720
gcactcacaa cacaagcaat cgttgaagcc gtaacttttta ctggcaacac ctcggcaggg    780
caaggaggcg ctatctatgt taaagaagct accctattca atgctctaga cagcctcaaa    840
tttgaaaaaa acacttctgg gcaagctggt ggtggaatct atacagagtc tacgctcaca    900
atctcgaaca tcacaaaatc tattgaattt atctctaata aagcttctgt ccctgccccc    960
gctcctgagc ccacctctcc ggctccaagt agcttaataa attctacaac gatcgatacc   1020
tcgactctcc aaacccgagc agcatccgca actccagcag tggctcctgt tgctgccgta   1080
actccaacac caatctctac tcaagagacc gcaggaaatg gaggcgctat ctatgctaaa   1140
caaggtattt cgatatccac gtttaaagat ctgaccttca agtctaactc tgcatcggta   1200
gatgccaccc ttactgtcga ttctagcact attggagaat ctggaggtgc tatctttgca   1260
gcagactcta tacaaatcca acagtgcacg ggaaccacct tattcagtgg caatactgcc   1320
aataagtctg gtgggggtat ttacgctgta ggacaagtca ccctagaaga tatagcgaat   1380
ctgaagatga ccaacaacac ctgtaaaggt gaaggtggag ccatctacac taaaaaggct   1440
ttaactatca acaacggtgc cattctcact acattttctg gaaatacatc gacagataat   1500
ggtggggcta tttttgctgt aggtggcatc actctctctg atcttgtaga agtccgcttt   1560
agtaaaaata agaccggaaa ttattccgct cctattacca aagcggctag caacacagct   1620
cctgtagttt ctagctctac aactgctgca tctcctgcgg tccctgctgc cgctgcagca   1680
cctgttacaa acgcagcaaa aggaggggct ttatatagta cagaaggact gactgtatct   1740
ggaatcacat cgatattgtc gtttgaaaac aacgaatgcc agaatcaagg aggtggggct   1800
tacgttacta aaaccttcca gtgttccgat tctcatcgcc tccagtttac tagtaataaa   1860
gcagcagatg aaggcggggg cctgtattgt ggtgacgatg tcacgctaac gaacctgaca   1920
gggaaaacac tatttcaaga gaatagcagt gagaaacatg gaggtgggct ctctctcgcc   1980
tcaggaaaat ctctgactat gacatcgtta gagagcttct gcttaaatgc aaatacagca   2040
aaggaaaacg gaggcggtgc gaatgtccct gaaaatattg tactcacctt cacctatact   2100
cccactccaa atgaacctgc gcctgtgcag cagcccgtgt atggagaagc tcttgttact   2160
```

```
ggaaatacag ccacaaaaag tggtgggggc atttacacga aaaatgcggc cttctcaaat   2220
ttatcttctg taacttttga tcaaaatacc tcttcagaaa atggtggtgc cttacttacc   2280
caaaaagctg cagataaaac ggactgttct ttcacctata ttacaaatgt caatatcacc   2340
aacaatacag ctacaggaaa tggtgggggc attgctgggg gaaaagcaca tttcgatcgc   2400
attgataatc ttacagtcca aagcaaccaa gcaaagaaag gtggtggggt ttatcttgaa   2460
gatgccctca tcctggaaaa ggttattaca ggttctgtct cacaaaatac agctacagaa   2520
agtggtgggg gtatctacgc taaggatatt caactacaag ctctacctgg aagcttcaca   2580
attaccgata ataaagtcga aactagtctt actactagca ctaatttata tggtgggggc   2640
atctattcca gtggagctgt cacgctaacc aatatatctg gaacctttgg cattacagga   2700
aactctgtta tcaatacagc gacatcccag gatgcagata tacaaggtgg gggcatttat   2760
gcaaccacgt ctctctcaat aaatcaatgt aatacaccca ttctatttag caacaactct   2820
gctgccacta aaaaaacatc aacaacaaag caaattgctg gtggggctat cttctccgct   2880
gcagtaacta tcgagaataa ctctcagccc attattttct taaataattc cgcaaagtcg   2940
gaagcaacta cagcagcaac tgcaggaaat aaagatagct gtggaggagc cattgcagct   3000
aactctgtta ctttaacaaa taaccctgaa ataaccttta aaggaaatta tgcagaaact   3060
ggaggagcga ttggctgtat tgatcttact aatggctcac ctccccgtaa agtctctatt   3120
gcagacaacg gttctgtcct ttttcaagac aactctgcgt taaatcgcgg aggcgctatc   3180
tatggagaga ctatcgatat ctccaggaca ggtgcgactt tcatcggtaa ctcttcaaaa   3240
catgatggaa gtgcaatttg ctgttcaaca gccctaactc ttgcgccaaa ctcccaactt   3300
atctttgaaa acaataaggt tacggaaacc acagccacta caaaagcttc cataaataat   3360
ttaggagctg caatttatgg aaataatgag actagtgacg tcactatctc tttatcagct   3420
gagaatggaa gtattttctt taaaaacaat ctatgcacag caacaaacaa atactgcagt   3480
attgctggaa acgtaaaatt tacagcaata gaagcttcag cagggaaagc tatatctttc   3540
tatgatgcag ttaacgtttc caccaaagaa acaaatgctc aagagctaaa attaaatgaa   3600
aaagcgacaa gtacaggaac gattctattt tctggggaac ttcacgaaaa taaatcctat   3660
attccacaga aagtcacttt cgcacatggg aatctcattc taggtaaaaa tgcagaactt   3720
agcgtagttt cctttaccca atctccaggc accacaatca ctatgggccc aggatcggtt   3780
ctttccaacc atagcaaaga agcaggagga atcgctataa acaatgtcat cattgatttt   3840
agtgaaatcg ttcctactaa agataatgca acagtagctc cacccactct taaattagta   3900
tcgagaacta atgcagatag taaagataag attgatatta caggaactgt gactcttcta   3960
gatcctaatg gcaacttata tcaaaattct tatcttggtg aagaccgcga tatcactctt   4020
ttcaatatag acaattctgc aagtggggca gttacagcca cgaatgtcac ccttcaaggg   4080
aatttaggag ctaaaaaagg atatttagga acctggaatt tggatccaaa ttcctcgggt   4140
tcaaaaatta ttctaaaatg gacctttgac aaatacctgc gctggcccta catccctaga   4200
gacaaccact tctacatcaa ctctatttgg ggagcacaaa actctttagt gactgtgaaa   4260
caagggatct tagggaacat gttgaacaat gcaaggtttg aagatcctgc tttcaacaac   4320
ttctgggctt cggctatagg atctttcctt aggaaagaag tatctcgaaa ttctgactca   4380
ttcacctatc atggcagagg ctataccgct gctgtggatg ccaaacctcg ccaagaattt   4440
attttaggag ctgccttcag tcaggttttt ggtcacgccg agtctgaata tcaccttgac   4500
aactataagc ataaaggctc aggtcactct acacaagcat ctctttatgc tggcaatatc   4560
ttctattttc ctgcgatacg gtctcggcct attctattcc aaggtgtggc gacctatggt   4620
tatatgcaac atgacaccac aacctactat ccttctattg aagaaaaaaa tatggcaaac   4680
tgggatagca ttgcttggtt atttgatctg cgtttcagtg tggatcttaa agaacctcaa   4740
cctcactcta cagcaaggct taccttctat acagaagctg agtataccag aattcgccag   4800
gagaaattca cagagctaga ctatgatcct agatctttct ctgcatgctc ttatggaaac   4860
ttagcaattc ctactggatt ctctgtagac ggagcattag cttggcgtga gattattcta   4920
tataataaag tatcagctgc gtacctccct gtgattctca ggaataatcc aaaagcgacc   4980
tatgaagttc tctctacaaa agaaaagggc aacgtagtca acgttctccc tacaagaaac   5040
gcagctcgtg cagaggtgag ctctcaaatt tatcttggaa gttactggac actctacggc   5100
acgtatacta ttgatgcttc aatgaatact ttagtgcaaa tggccaacgg agggatccgg   5160
tttgtattct ag                                                       5172
```

<210> 121
<211> 373
<212> PRT
<213> Chlamydia pneumoniae

<400> 121

```
Met Ser Ile Ala Ile Ala Arg Glu Gln Tyr Ala Ala Ile Leu Asp Met
1               5                   10                  15
```

His Pro Lys Pro Ser Ile Ala Met Phe Ser Ser Glu Gln Ala Arg Thr
20 25 30

Ser Trp Glu Lys Arg Gln Ala His Pro Tyr Leu Tyr Arg Leu Leu Glu
35 40 45

Ile Ile Trp Gly Val Val Lys Phe Leu Leu Gly Leu Ile Phe Phe Ile
50 55 60

Pro Leu Gly Leu Phe Trp Val Leu Gln Lys Ile Cys Gln Asn Phe Ile
65 70 75 80

Leu Leu Gly Ala Gly Gly Trp Ile Phe Arg Pro Ile Cys Arg Asp Ser
85 90 95

Asn Leu Leu Arg Gln Ala Tyr Ala Ala Arg Leu Phe Ser Ala Ser Phe
100 105 110

Gln Asp His Val Ser Ser Val Arg Arg Val Cys Leu Gln Tyr Asp Glu
115 120 125

Val Phe Ile Asp Gly Leu Glu Leu Arg Leu Pro Asn Ala Lys Pro Asp
130 135 140

Arg Trp Met Leu Ile Ser Asn Gly Asn Ser Asp Cys Leu Glu Tyr Arg
145 150 155 160

Thr Val Leu Gln Gly Glu Lys Asp Trp Ile Phe Arg Ile Ala Glu Glu
165 170 175

Ser Gln Ser Asn Ile Leu Ile Phe Asn Tyr Pro Gly Val Met Lys Ser
180 185 190

Gln Gly Asn Ile Thr Arg Asn Asn Val Val Lys Ser Tyr Gln Ala Cys
195 200 205

Val Arg Tyr Leu Arg Asp Glu Pro Ala Gly Pro Gln Ala Arg Gln Ile
210 215 220

Val Ala Tyr Gly Tyr Ser Leu Gly Ala Ser Val Gln Ala Glu Ala Leu
225 230 235 240

Ser Lys Glu Ile Ala Asp Gly Ser Asp Ser Val Arg Trp Phe Val Val
245 250 255

Lys Asp Arg Gly Ala Arg Ser Thr Gly Ala Val Ala Lys Gln Phe Ile
260 265 270

Gly Ser Leu Gly Val Trp Leu Ala Asn Leu Thr His Trp Asn Ile Asn
275 280 285

Ser Glu Lys Arg Ser Lys Asp Leu His Cys Pro Glu Leu Phe Ile Tyr
290 295 300

Gly Lys Asp Ser Gln Gly Asn Leu Ile Gly Asp Gly Leu Phe Lys Lys
305 310 315 320

Glu Thr Cys Phe Ala Ala Pro Phe Leu Asp Pro Lys Asn Leu Glu Glu
325 330 335

Cys Ser Gly Lys Lys Ile Pro Val Ala Gln Thr Gly Leu Arg His Asp

340 345 350

His Ile Leu Ser Asp Asp Val Ile Lys Glu Val Ala Gly His Ile Gln
355 360 365

Arg His Phe Asp Asn
370

<210> 122
<211> 1121
<212> DNA
<213> Chlamydia pneumoniae

<400> 122
atgtcaatag ctattgcaag ggaacaatac gcagctatat tggatatgca tcctaaacct 60
tcgatcgcca tgttttcttc ggagcaggcg agaacttctt gggagaaacg acaggctcat 120
ccttaccttt atcgtcttct tgagatcata tggggtgttg tgaaatttct tctcggctta 180
atcttcttta ttcccttggg tcttttctgg gtccttcaga agatatgtca gaattttatt 240
cttcttggtg caggagggtg gatttttaga cccatatgca gggactctaa tttattgcga 300
caagcttacg ccgcgcgtct tttctccgct tcattccaag atcatgtctc ctctgtgcga 360
agggtttgct tacagtatga cgaggtcttt attgacggat tggagttacg tcttcccaat 420
gctaagccag atcgatggat gttaatctcc aatggaaact ccgattgctt agagtatagg 480
acagtgctgc aaggggaaaa ggactggata ttccgtattg ctgaagagtc tcaatccaac 540
attttaatct tcaattaccc aggagtcatg aagagccaag ggaatataac aagaaacaat 600
gtagtcaaat cttatcaagc atgcgtacgc tatcttagag atgaacccgc aggacctcag 660
gcgcgtcaaa tcgttgctta tggctattct ttaggagcta gtgttcaagc cgaagcatta 720
agtaaagaga tcgcagacgg aagtgatagc gtccgttggt ttgtcgttaa agatcgagga 780
gctcgctcta caggagccgt tgctaaacag tttattggaa gtctaggagt ttggctggcg 840
aatcttaccc attggaatat taattctgaa aagagaagca aggacttgca ttgcccagaa 900
ctctttattt atggcaagga ttcccaaggt aatcttatcg ggatggattt gttcaaaaaa 960
gagacgtgct tcgcagcacc atttttagat cctaaaaact tggaagagtg ttcagggaag 1020
aaaatccctg tagctcagac cggtctaaga cacgatcata tcctttccga tgatgtgatt 1080
aaagaagttg caggtcatat tcaaagacat ttcgataatt a 1121

<210> 123
<211> 980
<212> PRT
<213> Chlamydia pneumoniae

<400> 123
Met Tyr Ser Cys Tyr Ser Lys Gly Ile Ser His Asn Tyr Leu Leu His
1 5 10 15

Pro Met Ser Arg Leu Asp Ile Phe Val Phe Asp Ser Leu Ile Ala Asn
20 25 30

Gln Asp Gln Asn Leu Leu Glu Glu Ile Phe Cys Ser Glu Asp Thr Val
35 40 45

Leu Phe Lys Ala Tyr Arg Thr Thr Ala Leu Gln Ser Pro Leu Ala Ala
50 55 60

Lys Asn Leu Asn Ile Ala Arg Lys Val Ala Asn Tyr Ile Leu Ala Asp
65 70 75 80

Asn Gly Glu Ile Asp Thr Val Lys Leu Val Glu Ala Ile His His Leu
85 90 95

Ser Gln Cys Thr Tyr Pro Leu Gly Pro His Arg His Asn Glu Ala Gln
100 105 110

```
Asp Arg Glu His Leu Leu Lys Met Leu Lys Ala Leu Lys Glu Asn Pro
        115                 120                 125

Lys Leu Lys Glu Ser Ile Lys Thr Leu Phe Val Pro Ser Tyr Ser Thr
    130                 135                 140

Ile Gln Asn Leu Ile Arg His Thr Leu Ala Leu Asn Pro Gln Thr Ile
145                 150                 155                 160

Leu Ser Thr Ile His Val Arg Gln Ala Ala Leu Thr Ala Leu Phe Thr
                165                 170                 175

Tyr Leu Arg Gln Asp Val Gly Ser Cys Phe Ala Thr Ala Pro Ala Ile
            180                 185                 190

Leu Ile His Gln Glu Tyr Pro Glu Arg Phe Leu Lys Asp Leu Asn Asp
        195                 200                 205

Leu Ile Ser Ser Gly Lys Leu Ser Arg Ile Val Asn Gln Arg Glu Ile
    210                 215                 220

Ala Val Pro Ile Asn Leu Ser Gly Cys Ile Gly Glu Leu Phe Lys Pro
225                 230                 235                 240

Leu Arg Ile Leu Asp Leu Tyr Pro Asp Pro Leu Val Lys Leu Ser Ser
                245                 250                 255

Ser Pro Gly Leu Lys Lys Ala Phe Ser Ala Ala Asn Leu Ile Glu Thr
            260                 265                 270

Leu Gly Asp Ser Glu Ala Gln Ile Gln Gln Leu Leu Ser His Gln Tyr
        275                 280                 285

Leu Met Gln Lys Leu Gln Asn Val His Glu Thr Leu Thr Ala Asn Asp
    290                 295                 300

Ile Ile Lys Ser Thr Leu Leu His Tyr Tyr Gln Leu Gln Glu Ser Thr
305                 310                 315                 320

Val Arg Ala Ile Phe Phe Lys Glu Gly Leu Phe Ser Lys Glu Gln Val
                325                 330                 335

Ala Phe Ser Thr Gln His Pro Arg Glu Leu Ser Glu Ile Gln Arg Val
            340                 345                 350

Tyr His Tyr Leu His Ala Tyr Glu Glu Ala Lys Ser Ala Phe Ile His
        355                 360                 365

Asp Thr Gln Asn Pro Leu Leu Lys Ala Trp Glu Tyr Thr Leu Ala Thr
    370                 375                 380

Leu Ala Asp Ala Ser Gln Pro Thr Ile Ser Asn His Ile Arg Leu Ala
385                 390                 395                 400

Leu Gly Trp Lys Ser Glu Asp Pro His Ser Leu Val Ser Leu Val Thr
                405                 410                 415

His Phe Val Glu Glu Glu Val Glu Asn Ile Arg Ile Leu Val Gln Gln
            420                 425                 430

Cys Glu Gln Thr Tyr His Glu Ala Arg Ser Gln Leu Glu Tyr Ile Glu
```

435 440 445

Gly Arg Met Arg Asn Pro Leu Asn Asn Gln Asp Ser Gln Ile Leu Thr
450 455 460

Met Asp His Met Arg Phe Arg Gln Glu Leu Asn Lys Ala Leu Tyr Glu
465 470 475 480

Trp Asp Ser Ala Gln Glu Lys Ala Lys Lys Phe Leu His Leu Pro Glu
485 490 495

Phe Leu Leu Ser Phe Tyr Thr Lys Gln Ile Pro Leu Tyr Phe Arg Ser
500 505 510

Ser Tyr Asp Ala Phe Ile Gln Glu Phe Ala His Leu Tyr Ala Asn Ala
515 520 525

Pro Ala Gly Phe Arg Ile Leu Phe Thr His Gly Arg Thr His Pro Asn
530 535 540

Thr Trp Ser Pro Ile Tyr Ser Ile Asn Glu Phe Ile Arg Phe Leu Ser
545 550 555 560

Glu Phe Phe Thr Ser Thr Glu Ser Glu Leu Leu Gly Lys His Ala Val
565 570 575

Ile Asn Leu Glu Lys Glu Thr Ser Arg Leu Val His Asn Ile Thr Ala
580 585 590

Met Leu His Thr Asp Val Phe Gln Glu Ala Leu Leu Thr Arg Ile Leu
595 600 605

Glu Ala Tyr Gln Leu Pro Val Pro Pro Ser Ile Leu Asn His Leu Asp
610 615 620

Gln Leu Ser Gln Thr Pro Trp Val Tyr Val Ser Gly Gly Thr Val Asp
625 630 635 640

Thr Leu Leu Leu Asp Tyr Phe Glu Ser Ser Glu Pro Leu Thr Leu Thr
645 650 655

Glu Lys His Pro Glu Asn Pro His Glu Leu Ala Ala Phe Tyr Ala Asp
660 665 670

Ala Leu Lys Asp Leu Pro Thr Gly Ile Lys Ser Tyr Leu Glu Glu Gly
675 680 685

Ser His Ser Leu Leu Ser Ser Ser Pro Thr His Val Phe Ser Ile Ile
690 695 700

Ala Gly Ser Pro Leu Phe Arg Glu Ala Trp Asp Asn Asp Trp Tyr Ser
705 710 715 720

Tyr Thr Trp Leu Arg Asp Val Trp Val Lys Gln His Gln Asp Phe Leu
725 730 735

Gln Asp Thr Ile Leu Pro Gln Leu Ser Ile Tyr Ala Phe Ile Glu Asn
740 745 750

Phe Cys Asn Lys Tyr Ala Leu Gln His Val Val His Asp Phe His Asp
755 760 765

Phe Cys Ser Asp His Ser Leu Thr Leu Pro Glu Leu Tyr Asp Lys Gly
770 775 780

Ser Arg Phe Leu Ser Ser Leu Phe Thr Lys Asp Lys Thr Val Ala Leu
785 790 795 800

Ile Tyr Ile Arg Arg Leu Leu Tyr Leu Met Val Arg Glu Val Pro Tyr
805 810 815

Val Ser Glu Gln Gln Leu Pro Glu Val Leu Asp Asn Val Ser Ser Tyr
820 825 830

Leu Gly Ile Ser Ser Arg Ile Thr Tyr Glu Lys Phe Arg Ser Leu Ile
835 840 845

Glu Glu Thr Ile Pro Lys Met Thr Leu Leu Ser Ser Ala Asp Leu Arg
850 855 860

His Ile Tyr Lys Gly Leu Leu Met Gln Ser Tyr Gln Lys Ile Tyr Thr
865 870 875 880

Glu Glu Asp Thr Tyr Leu Arg Leu Thr Thr Ala Met Arg His His Asn
885 890 895

Leu Ala Tyr Pro Ala Pro Leu Leu Phe Ala Asp Ser Asn Trp Pro Ser
900 905 910

Ile Tyr Phe Gly Phe Ile Leu Asn Pro Gly Thr Thr Glu Ile Asp Leu
915 920 925

Trp Lys Phe Asn Tyr Ala Gly Leu Gln Gly Gln Pro Leu Asp Asn Ile
930 935 940

Gln Glu Leu Phe Ala Thr Ser Arg Pro Trp Thr Leu Tyr Ala Asn Pro
945 950 955 960

Ile Asp Tyr Gly Met Pro Pro Pro Pro Gly Tyr Arg Ser Arg Leu Pro
965 970 975

Lys Glu Phe Phe
980

<210> 124
<211> 2943
<212> DNA
<213> Chlamydia pneumoniae

<400> 124
atgtattcgt gttacagcaa aggaatatcc cataactatc ttctacatcc tatgtcacgt 60
ttggatattt ttgttttcga ttctctgatc gcaaaccagg atcaaaatct tcttgaggaa 120
attttctgtt ctgaagacac agttttattt aaagcctacc gtactacggc tctacaatcc 180
cctctagctg ctaagaacct aaatatcgcc cgtaaagtcg caaattatat cttagctgac 240
aatggggaaa tcgatacagt aaagcttgtc gaagccattc accatctctc acaatgtacc 300
tatcctttag ggcctcatcg ccataatgaa gctcaagatc gtgaacacct ccttaaaatg 360
ctaaaagctc taaaggaaaa tcctaaatta aaagaaagca tcaaaactct ctttgtccct 420
tcatactcta caatccaaaa cctaattcgc catacactag cattgaatcc acagacaatt 480
ctctctacga ttcatgtgcg tcaagcagca ctcacagcgc tcttcaccta ccttcggcaa 540
gatgtaggtt cctgttttgc tacggctcct gccattctca ttcaccaaga atatccagaa 600
cgattcctta aagatctcaa tgatctcatt agcagtggca aactctctag aatcgtaaac 660
caaagggaaa ttgcggttcc tataaacctt tcgggatgca ttggagagct attcaagcct 720

```
ttaaggattc tagatcttta tcctgatcct ctggttaagc tctcctcatc tccaggactc    780
aaaaaagcct tttctgctgc caatcttatt gaaactcttg gggattctga agcacaaatc    840
caacagttgc tctcgcatca atatttgatg caaaaactac aaaatgtcca tgagacctta    900
actgctaacg acattatcaa atcgacactt ctgcactact atcagctcca agaaagtact    960
gtacgagcta ttttcttcaa agaagggttg ttcagcaaag aacaagtggc attctcgacg   1020
caacacccca gagagctctc agaaatacaa cgggtatacc actacttaca tgcctatgaa   1080
gaagcaaaat ctgcttttat ccatgacact caaaatccct tactgaaagc ctgggagtat   1140
actttagcga ctcttgcgga tgctagccaa cctaccatct caaaccatat ccgccttgcc   1200
ttaggatgga aagtgaaga ccctcacagt cttgtatctc tagttacaca ctttgttgaa    1260
gaggaagtag aaaacatccg aattttagtc caacaatgtg aacagaccta tcacgaagca   1320
cgctcccaac tagaatatat tgaagggcgg atgcgcaacc cactaaataa tcaagacagt   1380
cagattttga cgatggatca catgcgcttc cgtcaagaac tcaataaagc tctttatgag   1440
tgggatagtg ctcaagaaaa ggcaaagaaa tttctacatc ttcctgaatt cttactttct   1500
ttctatacaa agcaaattcc cttatacttt cgtagttctt acgatgcctt cattcaagaa   1560
tttgctcatc tctatgctaa tgctcccgct ggcttccgta ttcttttcac gcatggacgc   1620
acccatccga acacatggtc ccccatctat tcgattaatg aatttatacg ttttctttct   1680
gaattcttca cctccacaga gtcagaactt ctggggaaac atgccgtgat caatttagag   1740
aaagaaacat ctcggctcgt ccacaacatc actgccatgc tacacacgga tgttttccaa   1800
gaagctctcc ttacaagaat tttagaagcc tatcagcttc ctgtgcctcc ctccatctta   1860
aaccacttag atcagctgtc acaaactccc tgggtttatg tttctggagg aacagtggac   1920
actcttcttt tggattattt tgaaagctca gaacctctga cacttacaga aaagcatcct   1980
gaaaatcctc atgagcttgc agctttctac gcagacgccc ttaaagatct ccctacagga   2040
attaaaagtt atctagaaga aggatcccac tctctactta gctcatcacc cacccacgtt   2100
ttctctataa tcgcaggatc tcctttattt cgggaagctt gggataatga ttggtacagc   2160
tatacctggc ttcgtgatgt ctgggtgaaa caacaccaag atttccttca agatactata   2220
ttacctcagc taagtatcta tgctttcata gagaattttt gtaacaaata tgctttgcaa   2280
catgtagttc atgactttca tgatttctgc tccgaccact ccttgactct tccggagctc   2340
tatgacaaag gatcgcgttt tctaagctcc ttattcacca aagataagac cgtagctctt   2400
atctatatac gccgtcttct ctaccttatg gtccgtgaag tcccttatgt ttcagaacaa   2460
cagcttccag aagtcttaga taacgtctct tcatatctcg ggatttcctc tcgtattacc   2520
tatgagaaat tccgctccct gatagaggaa accatcccta aaatgacctt actctcctca   2580
gcagacctga ggcatatcta taaaggtctc ctcatgcaaa gttatcaaaa gatctacacc   2640
gaagaagata cgtacctccg cctcaccacg gcaatgaggc atcataatct tgcctatccc   2700
gctcctttgc tctttgcaga cagtaactgg ccttctattt attttggatt catcctaaat   2760
ccaggaacca cagagatcga tctttggaaa tttaactatg cagggctgca aggacagcct   2820
cttgacaata tccaggagct gttcgcaacg tcaagaccct ggaccctcta tgcaaatcct   2880
atagattatg gcatgccacc gcctccaggc taccgcagcc gcctccctaa agaatttttc   2940
tag                                                                 2943

<210>   125
<211>   674
<212>   PRT
<213>   Chlamydia pneumoniae

<400>   125
Met Ser Ile Val Arg Asn Ser Ala Leu Pro Leu Pro Cys Leu Ser Arg
1               5                   10                  15

Ser Glu Thr Phe Lys Lys Val Arg Ser His Met Lys Phe Met Lys Val
            20                  25                  30

Leu Thr Pro Trp Ile Tyr Arg Lys Asp Leu Trp Val Thr Ala Phe Leu
        35                  40                  45

Leu Thr Ala Ile Pro Gly Ser Phe Ala His Thr Leu Val Asp Ile Ala
    50                  55                  60

Gly Glu Pro Arg His Ala Ala Gln Ala Thr Gly Val Ser Gly Asp Gly
65                  70                  75                  80

Lys Ile Val Ile Gly Met Lys Val Pro Asp Asp Pro Phe Ala Ile Thr
```

```
                85                  90                  95

Val Gly Phe Gln Tyr Ile Asp Gly His Leu Gln Pro Leu Glu Ala Val
            100                 105                 110

Arg Pro Gln Cys Ser Val Tyr Pro Asn Gly Ile Thr Pro Asp Gly Thr
        115                 120                 125

Val Ile Val Gly Thr Asn Tyr Ala Ile Gly Met Gly Ser Val Ala Val
    130                 135                 140

Lys Trp Val Asn Gly Lys Val Ser Glu Leu Pro Met Leu Pro Asp Thr
145                 150                 155                 160

Leu Asp Ser Val Ala Ser Ala Val Ser Ala Asp Gly Arg Val Ile Gly
                165                 170                 175

Gly Asn Arg Asn Ile Asn Leu Gly Ala Ser Val Ala Val Lys Trp Glu
            180                 185                 190

Asp Asp Val Ile Thr Gln Leu Pro Ser Leu Pro Asp Ala Met Asn Ala
        195                 200                 205

Cys Val Asn Gly Ile Ser Ser Asp Gly Ser Ile Ile Val Gly Thr Met
    210                 215                 220

Val Asp Val Ser Trp Arg Asn Thr Ala Val Gln Trp Ile Gly Asp Gln
225                 230                 235                 240

Leu Ser Val Ile Gly Thr Leu Gly Gly Thr Thr Ser Val Ala Ser Ala
                245                 250                 255

Ile Ser Thr Asp Gly Thr Val Ile Val Gly Gly Ser Glu Asn Ala Asp
            260                 265                 270

Ser Gln Thr His Ala Tyr Ala Tyr Lys Asn Gly Val Met Ser Asp Ile
        275                 280                 285

Gly Thr Leu Gly Gly Phe Tyr Ser Leu Ala His Ala Val Ser Ser Asp
    290                 295                 300

Gly Ser Val Ile Val Gly Val Ser Thr Asn Ser Glu His Arg Tyr His
305                 310                 315                 320

Ala Phe Gln Tyr Ala Asp Gly Gln Met Val Asp Leu Gly Thr Leu Gly
                325                 330                 335

Gly Pro Glu Ser Tyr Ala Gln Gly Val Ser Gly Asp Gly Lys Val Ile
            340                 345                 350

Val Gly Arg Ala Gln Val Pro Ser Gly Asp Trp His Ala Phe Leu Cys
        355                 360                 365

Pro Phe Gln Ala Pro Ser Pro Ala Pro Val His Gly Gly Ser Thr Val
    370                 375                 380

Val Thr Ser Gln Asn Pro Arg Gly Met Val Asp Ile Asn Ala Thr Tyr
385                 390                 395                 400

Ser Ser Leu Lys Asn Ser Gln Gln Gln Leu Gln Arg Leu Leu Ile Gln
                405                 410                 415
```

His Ser Ala Lys Val Glu Ser Val Ser Ser Gly Ala Pro Ser Phe Thr
420 425 430

Ser Val Lys Gly Ala Ile Ser Lys Gln Ser Pro Ala Val Gln Asn Asp
435 440 445

Val Gln Lys Gly Thr Phe Leu Ser Tyr Arg Ser Gln Val His Gly Asn
450 455 460

Val Gln Asn Gln Gln Leu Leu Thr Gly Ala Phe Met Asp Trp Lys Leu
465 470 475 480

Ala Ser Ala Pro Lys Cys Gly Phe Lys Val Ala Leu His Tyr Gly Ser
485 490 495

Gln Asp Ala Leu Val Glu Arg Ala Ala Leu Pro Tyr Thr Glu Gln Gly
500 505 510

Leu Gly Ser Ser Val Leu Ser Gly Phe Gly Gly Gln Val Gln Gly Arg
515 520 525

Tyr Asp Phe Asn Leu Gly Glu Thr Val Val Leu Gln Pro Phe Met Gly
530 535 540

Ile Gln Val Leu His Leu Ser Arg Glu Gly Tyr Ser Glu Lys Asn Val
545 550 555 560

Arg Phe Pro Val Ser Tyr Asp Ser Val Ala Tyr Ser Ala Ala Thr Ser
565 570 575

Phe Met Gly Ala His Val Phe Ala Ser Leu Ser Pro Lys Met Ser Thr
580 585 590

Ala Ala Thr Leu Gly Val Glu Arg Asp Leu Asn Ser His Ile Asp Glu
595 600 605

Phe Lys Gly Ser Val Ser Ala Met Gly Asn Phe Val Leu Glu Asn Ser
610 615 620

Thr Val Ser Val Leu Arg Pro Phe Ala Ser Leu Ala Met Tyr Tyr Asp
625 630 635 640

Val Arg Gln Gln Gln Leu Val Thr Leu Ser Val Val Met Asn Gln Gln
645 650 655

Pro Leu Thr Gly Thr Leu Ser Leu Val Ser Gln Ser Ser Tyr Asn Leu
660 665 670

Ser Phe

<210> 126
<211> 2025
<212> DNA
<213> Chlamydia pneumoniae

<400> 126
atgagtatag tcagaaattc tgcattgcca cttccgtgtt taagcagatc cgaaaccttt 60
aaaaaagtta ggtcgcatat gaaatttatg aaagtcctta ctccatggat ttatcgaaaa 120
gatctttggg taacagcatt cttactgaca gcaattccag gatcttttgc acatactctt 180

```
gttgatatag caggagaacc tcggcatgct gctcaagcaa caggagtttc tggagatggt    240
aaaattgtta taggaatgaa agttccggat gatccttttg ctataactgt aggatttcaa    300
tatattgatg ggcatttgca acccttagag gcagtacgtc ctcaatgctc tgtataccct    360
aatggtataa ccccggacgg aacggttatt gtgggtacaa actatgccat cgggatgggt    420
agtgttgctg tgaaatgggt aaatggcaag gtttctgaac ttcccatgct ccctgacacc    480
ctcgattctg tagcatcggc agtttctgca gatggaagag tgattggagg gaatagaaat    540
ataaatcttg gcgcttctgt tgctgtgaaa tgggaggacg acgtgattac acaacttcct    600
tctcttcctg atgctatgaa tgcttgtgtt aacggaattt cttcagatgg ttctataatt    660
gtaggaacca tggtagacgt gtcatggaga aataccgcag tacaatggat cggggatcag    720
ctctctgtta ttgggacttt aggaggaact acttctgttg ctagtgcaat ctcaacagat    780
ggcactgtga ttgtaggagg ttctgaaaat gcagattctc agactcatgc ctatgcttat    840
aaaaacggtg ttatgagcga tatagggacc ctcggaggtt tttattcttt agcacatgca    900
gtatcttcag atggttctgt gattgtagga gtatccacga actctgagca tagatatcat    960
gcattccaat atgctgatgg acagatggta gatttaggaa ctttaggagg gcctgaatct   1020
tatgctcaag gtgtgtctgg agatggaaag gtaattgtgg gtagagcaca agtaccatct   1080
ggagattggc atgcgttcct atgtcctttc caagctccga gccctgctcc tgtccatggg   1140
ggaagcactg tcgtaactag ccagaatcca cgtggaatgg tagatatcaa tgctacgtac   1200
tcctctttga aaaatagcca acaacaacta caaagattgc ttatccagca tagtgcaaaa   1260
gttgaaagtg tatcctcagg agcaccatct tttacaagtg tgaaaggtgc gatctcaaaa   1320
cagagccctg cagtgcaaaa tgatgtacag aaagggacgt ttttaagtta ccgttcccaa   1380
gttcatggaa acgtgcagaa tcagcaattg ctcacaggag cttttatgga ctggaaactc   1440
gcttcagctc ctaaatgcgg ctttaaagta gctctccact atggctctca agatgctctc   1500
gtagaacgtg cagctcttcc ttacacagaa caaggcttag gaagcagtgt cttgtcaggt   1560
tttggaggac aagttcaagg acgctatgac tttaatttag gagaaactgt tgttctgcaa   1620
ccctttatgg gcattcaagt tctccaccta agtagagaag ggtattctga gaagaatgtt   1680
cgatttcctg taagctatga ttctgtagcc tactcagcag ctactagctt tatgggtgcg   1740
catgtatttg cctccctaag ccctaaaatg agtacagcag caactttagg tgtggagaga   1800
gatctgaatt cacatataga tgaatttaag ggatccgtct ctgctatggg aaactttgtc   1860
ttggaaaatt ctacagtgag tgttttaaga ccttttgctt ctcttgctat gtactatgac   1920
gtaagacaac agcaactcgt gacgttgtca gtagttatga atcaacaacc cttaacaggc   1980
acactaagct tagtaagcca aagtagctat aatcttagct tctaa                    2025
```

<210> 127
<211> 527
<212> PRT
<213> Chlamydia pneumoniae

<400> 127

```
Met Leu Arg Phe Phe Ala Val Phe Ile Ser Thr Leu Trp Leu Ile Thr
1               5                   10                  15

Ser Gly Cys Ser Pro Ser Gln Ser Ser Lys Gly Ile Phe Val Val Asn
            20                  25                  30

Met Lys Glu Met Pro Arg Ser Leu Asp Pro Gly Lys Thr Arg Leu Ile
        35                  40                  45

Ala Asp Gln Thr Leu Met Arg His Leu Tyr Glu Gly Leu Val Glu Glu
    50                  55                  60

His Ser Gln Asn Gly Glu Ile Lys Pro Ala Leu Ala Glu Ser Tyr Thr
65                  70                  75                  80

Ile Ser Glu Asp Gly Thr Arg Tyr Thr Phe Lys Ile Lys Asn Ile Leu
                85                  90                  95

Trp Ser Asn Gly Asp Pro Leu Thr Ala Gln Asp Phe Val Ser Ser Trp
            100                 105                 110

Lys Glu Ile Leu Lys Glu Asp Ala Ser Ser Val Tyr Leu Tyr Ala Phe
        115                 120                 125
```

Leu Pro Ile Lys Asn Ala Arg Ala Ile Phe Asp Asp Thr Glu Ser Pro
130 135 140

Glu Asn Leu Gly Val Arg Ala Leu Asp Lys Arg His Leu Glu Ile Gln
145 150 155 160

Leu Glu Thr Pro Cys Ala His Phe Leu His Phe Leu Thr Leu Pro Ile
165 170 175

Phe Phe Pro Val His Glu Thr Leu Arg Asn Tyr Ser Thr Ser Phe Glu
180 185 190

Glu Met Pro Ile Thr Cys Gly Ala Phe Arg Pro Val Ser Leu Glu Lys
195 200 205

Gly Leu Arg Leu His Leu Glu Lys Asn Pro Met Tyr His Asn Lys Ser
210 215 220

Arg Val Lys Leu His Lys Ile Ile Val Gln Phe Ile Ser Asn Ala Asn
225 230 235 240

Thr Ala Ala Ile Leu Phe Lys His Lys Lys Leu Asp Trp Gln Gly Pro
245 250 255

Pro Trp Gly Glu Pro Ile Pro Pro Glu Ile Ser Ala Ser Leu His Gln
260 265 270

Asp Asp Gln Leu Phe Ser Leu Pro Gly Ala Ser Thr Thr Trp Leu Leu
275 280 285

Phe Asn Ile Gln Lys Lys Pro Trp Asn Asn Ala Lys Leu Arg Lys Ala
290 295 300

Leu Ser Leu Ala Ile Asp Lys Asp Met Leu Thr Lys Val Val Tyr Gln
305 310 315 320

Gly Leu Ala Glu Pro Thr Asp His Ile Leu His Pro Arg Leu Tyr Pro
325 330 335

Gly Thr Tyr Pro Glu Arg Lys Arg Gln Asn Glu Arg Ile Leu Glu Ala
340 345 350

Gln Gln Leu Phe Glu Glu Ala Leu Asp Glu Leu Gln Met Thr Arg Glu
355 360 365

Asp Leu Glu Lys Glu Thr Leu Thr Phe Ser Thr Phe Ser Phe Ser Tyr
370 375 380

Gly Arg Ile Cys Gln Met Leu Arg Glu Gln Trp Lys Lys Val Leu Lys
385 390 395 400

Phe Thr Ile Pro Ile Val Gly Gln Glu Phe Phe Thr Ile Gln Lys Asn
405 410 415

Phe Leu Glu Gly Asn Tyr Ser Leu Thr Val Asn Gln Trp Thr Ala Ala
420 425 430

Phe Ile Asp Pro Met Ser Tyr Leu Met Ile Phe Ala Asn Pro Gly Gly
435 440 445

Ile Ser Pro Tyr His Leu Gln Asp Ser His Phe Gln Thr Leu Leu Ile
450 455 460

Lys Ile Thr Gln Glu His Lys Lys His Leu Arg Asn Gln Leu Ile Ile
465 470 475 480

Glu Ala Leu Asp Tyr Leu Glu His Cys His Ile Leu Glu Pro Leu Cys
485 490 495

His Pro Asn Leu Arg Ile Ala Leu Asn Lys Asn Ile Lys Asn Phe Asn
500 505 510

Leu Phe Val Arg Arg Thr Ser Asp Phe Arg Phe Ile Glu Lys Leu
515 520 525

<210> 128
<211> 1584
<212> DNA
<213> Chlamydia pneumoniae

<400> 128
atgctccgtt tcttcgctgt atttatatca actctttggc tcattacctc aggatgttcc 60
ccatcccaat cctctaaagg aatttttgtg gtaaatatga aggaaatgcc acgctccttg 120
gatcctggaa aaactcgtct cattgcagac caaactctaa tgcgtcatct atatgaagga 180
ctcgtcgaag aacattccca aaatggagag attaaaccag cccttgcaga aagctacacc 240
atctccgaag acgggactcg gtacacattt aaaatcaaaa acatcctttg gagtaacgga 300
gaccctctga cagctcaaga ctttgtctcc tcttggaagg aaatcctaaa ggaagatgcg 360
tcctccgtat atctctatgc gtttttacct atcaaaaatg ctcgggcaat ctttgatgat 420
actgagtctc cagaaaatct aggagtccga gctttagata agcgtcatct cgaaattcag 480
ttagaaactc cctgcgcgca tttcctacat ttcttgactc ttcctatttt tttccctgtt 540
catgaaactc tgcgaaacta tagcacctct tttgaagaga tgcccattac ctgcggtgct 600
ttccgccctg tgtctctaga aaaaggcctg agactccatc tagagaaaaa ccctatgtac 660
cataataaaa gccgtgtgaa actacataaa attattgtac agtttatctc aaacgctaac 720
actgcagcca ttctattcaa acataagaaa ttagattggc aaggacctcc ttggggagaa 780
cctatccctc cagaaatctc agcttctcta catcaagatg accagctctt ttctcttccg 840
ggcgcttcga ctacatggtt actctttaat atacaaaaaa aaccttggaa caatgctaaa 900
ttacgcaagg cattgagcct tgcaatagac aaagatatgt taaccaaagt ggtataccaa 960
ggtcttgcag aacctacaga tcatatccta catccaagac tttatccagg gacctatccc 1020
gaacggaaaa gacaaaacga aagaattctt gaggctcaac aactctttga agaagctcta 1080
gacgaacttc aaatgacacg cgaagatcta gaaaaggaaa ctttgacttt ctcaaccttt 1140
tctttttctt acggaaggat ttgccaaatg ctaagagaac aatggaagaa agtcttaaaa 1200
tttactatcc ctatagtagg ccaagagttt ttcacaatac aaaaaaactt cctagagggg 1260
aactattccc taaccgtgaa ccaatggacc gcagcattta ttgatccgat gtcttatctc 1320
atgatctttg ccaatcctgg aggaatttcc ccctatcacc tccaagattc acactttcaa 1380
actcttctca taaagatcac tcaagaacat aaaaaacacc tacgaaatca gcttattatt 1440
gaagcccttg actatttaga acactgtcac attctcgaac cactatgtca tccaaatctt 1500
cgaattgctt tgaacaaaaa cattaaaaac tttaatcttt ttgttcgacg aacttcagac 1560
tttcgtttta tagaaaaact atag 1584

<210> 129
<211> 215
<212> PRT
<213> Chlamydia pneumoniae

<400> 129
Met Arg Lys Met Leu Val Leu Leu Ala Ser Leu Gly Leu Leu Ser Pro
1 5 10 15

Thr Leu Ser Ser Cys Thr His Leu Gly Ser Ser Gly Ser Tyr His Pro
20 25 30

Lys Leu Tyr Thr Ser Gly Ser Lys Thr Lys Gly Val Ile Ala Met Leu
35 40 45

Pro Val Phe His Arg Pro Gly Lys Ser Leu Glu Pro Leu Pro Trp Asn
50 55 60

Leu Gln Gly Glu Phe Thr Glu Glu Ile Ser Lys Arg Phe Tyr Ala Ser
65 70 75 80

Glu Lys Val Phe Leu Ile Lys His Asn Ala Ser Pro Gln Thr Val Ser
85 90 95

Gln Phe Tyr Ala Pro Ile Ala Asn Arg Leu Pro Glu Thr Ile Ile Glu
100 105 110

Gln Phe Leu Pro Ala Glu Phe Ile Val Ala Thr Glu Leu Leu Glu Gln
115 120 125

Lys Thr Gly Lys Glu Ala Gly Val Asp Ser Val Thr Ala Ser Val Arg
130 135 140

Val Arg Val Phe Asp Ile Arg His His Lys Ile Ala Leu Ile Tyr Gln
145 150 155 160

Glu Ile Ile Glu Cys Ser Gln Pro Leu Thr Thr Leu Val Asn Asp Tyr
165 170 175

His Arg Tyr Gly Trp Asn Ser Lys His Phe Asp Ser Thr Pro Met Gly
180 185 190

Leu Met His Ser Arg Leu Phe Arg Glu Val Val Ala Arg Val Glu Gly
195 200 205

Tyr Val Cys Ala Asn Tyr Ser
210 215

<210> 130
<211> 648
<212> DNA
<213> Chlamydia pneumoniae

<400> 130
atgcgaaaaa tgttggtatt attggcatct ttaggacttc tatccccaac cctatccagc 60
tgcactcact taggctcttc aggaagttat catcctaagc tatacacttc agggagcaaa 120
actaaaggtg tgattgcgat gcttcctgta tttcatcgcc caggaaagag tcttgaacct 180
ttaccttgga acctccaagg agaatttact gaagagatca gcaaaaggtt ttatgcttcg 240
gaaaaggtct tcctgatcaa gcacaatgct tcacctcaga cagtctctca gttctatgct 300
ccgattgcga atcgtctacc cgaaacaatt attgagcaat ttcttcctgc agaattcatt 360
gttgctacag aactgttaga acaaaagaca gggaaagaag caggtgtcga ttctgtaaca 420
gcgtctgtac gtgttcgcgt tttgatatc cgtcatcata aaatagctct catttatcaa 480
gagattatcg aatgcagcca gcctttaact accctagtca atgattatca tcgctatggc 540
tggaactcaa aacattttga ttcaacgccc atgggcttaa tgcatagccg tcttttccgc 600
gaagttgttg ccagagttga gggctatgtt tgtgctaact actcgtag 648

<210> 131
<211> 323
<212> PRT
<213> Chlamydia pneumoniae

<400> 131
Met Lys Gln Leu Leu Phe Cys Val Cys Val Phe Ala Met Ser Cys Ser

```
1               5                   10                  15
Ala Tyr Ala Ser Pro Arg Arg Gln Asp Pro Ser Val Met Lys Glu Thr
            20                  25                  30
Phe Arg Asn Asn Tyr Gly Ile Ile Val Ser Gly Gln Glu Trp Val Lys
        35                  40                  45
Arg Gly Ser Asp Gly Thr Ile Thr Lys Val Leu Lys Asn Gly Ala Thr
    50                  55                  60
Leu His Glu Val Tyr Ser Gly Gly Leu Leu His Gly Glu Ile Thr Leu
65                  70                  75                  80
Thr Phe Pro His Thr Thr Ala Leu Asp Val Val Gln Ile Tyr Asp Gln
                85                  90                  95
Gly Arg Leu Val Ser Arg Lys Thr Phe Phe Val Asn Gly Leu Pro Ser
            100                 105                 110
Gln Glu Glu Leu Phe Asn Glu Asp Gly Thr Phe Val Leu Thr Arg Trp
        115                 120                 125
Pro Asp Asn Asn Asp Ser Asp Thr Ile Thr Lys Pro Tyr Phe Ile Glu
    130                 135                 140
Thr Thr Tyr Gln Gly His Val Ile Glu Gly Ser Tyr Thr Ser Phe Asn
145                 150                 155                 160
Gly Lys Tyr Ser Ser Ser Ile His Asn Gly Glu Gly Val Arg Ser Val
                165                 170                 175
Phe Ser Ser Asn Asn Ile Leu Leu Ser Glu Glu Thr Phe Asn Glu Gly
            180                 185                 190
Val Met Val Lys Tyr Thr Thr Phe Tyr Pro Asn Arg Asp Pro Glu Ser
        195                 200                 205
Ile Thr His Tyr Gln Asn Gly Gln Pro His Gly Leu Arg Leu Thr Tyr
    210                 215                 220
Leu Gln Gly Gly Ile Pro Asn Thr Ile Glu Glu Trp Arg Tyr Gly Phe
225                 230                 235                 240
Gln Asp Gly Thr Thr Ile Val Phe Lys Asn Gly Cys Lys Thr Ser Glu
                245                 250                 255
Ile Ala Tyr Val Lys Gly Val Lys Glu Gly Leu Glu Leu Arg Tyr Asn
            260                 265                 270
Glu Gln Glu Ile Val Ala Glu Glu Val Ser Trp Arg Asn Asp Phe Leu
        275                 280                 285
His Gly Glu Arg Lys Ile Tyr Ala Gly Gly Ile Gln Lys His Glu Trp
    290                 295                 300
Tyr Tyr Arg Gly Arg Ser Val Ser Lys Ala Lys Phe Glu Arg Leu Asn
305                 310                 315                 320
Ala Ala Gly
```

<210> 132
<211> 972
<212> DNA
<213> Chlamydia pneumoniae

<400> 132
atgaaacaat tacttttctg tgtttgcgta tttgctatgt catgttctgc ttacgcatcc 60
ccacgacgac aagatccttc tgttatgaag gaaacattcc gaaataatta tggcattatt 120
gtttccggtc aagaatgggt aaagcgtggt tctgacggca ccatcaccaa agtactcaaa 180
aatggagcta ccctgcatga agtttattct ggaggcctcc ttcatgggga aattacctta 240
acgtttcccc ataccacagc attggacgtt gttcaaatct atgatcaagg tagactcgtt 300
tctcgcaaaa cctttttgt gaacggtctt ccatctcaag aagagctgtt caatgaagat 360
ggcacgtttg tcctcacacg atggccggac aacaacgaca gtgataccat cacaaagcct 420
tacttcatag aaacgacata tcaagggcat gtcatagaag gaagttatac ttcctttaat 480
gggaaatact cctcatccat ccacaatgga gagggagttc gttctgtgtt ctcctccaat 540
aacatccttc tttctgaaga gaccttcaat gaaggtgtca tggtgaaata taccacattc 600
tatccgaatc gcgatcccga atcgattact cattatcaaa atggacagcc tcacggctta 660
cggctaacat atctacaagg tggcatcccc aatacgatag aggagtggcg ttatggcttt 720
caagacggaa cgaccatcgt atttaaaaat ggttgtaaga catctgagat cgcttatgtt 780
aagggagtga aagaaggttt agaactgcgc tacaatgaac aggaaattgt agctgaagaa 840
gtttcttggc gtaatgattt tctgcatgga gaacgtaaga tctatgctgg aggaatccaa 900
aagcatgaat ggtattaccg cgggagatct gtatctaaag ccaaattcga gcggctaaat 960
gctgcaggat ag 972

<210> 133
<211> 494
<212> PRT
<213> Chlamydia pneumoniae

<400> 133
Met Lys Thr Ser Val Ser Met Leu Leu Ala Leu Leu Cys Ser Gly Ala
1 5 10 15

Ser Ser Ile Val Leu His Ala Ala Thr Thr Pro Leu Asn Pro Glu Asp
20 25 30

Gly Phe Ile Gly Glu Gly Asn Thr Asn Thr Phe Ser Pro Lys Ser Thr
35 40 45

Thr Asp Ala Ala Gly Thr Thr Tyr Ser Leu Thr Gly Glu Val Leu Tyr
50 55 60

Ile Asp Pro Gly Lys Gly Gly Ser Ile Thr Gly Thr Cys Phe Val Glu
65 70 75 80

Thr Ala Gly Asp Leu Thr Phe Leu Gly Asn Gly Asn Thr Leu Lys Phe
85 90 95

Leu Ser Val Asp Ala Gly Ala Asn Ile Ala Val Ala His Val Gln Gly
100 105 110

Ser Lys Asn Leu Ser Phe Thr Asp Phe Leu Ser Leu Val Ile Thr Glu
115 120 125

Ser Pro Lys Ser Ala Val Thr Thr Gly Lys Gly Ser Leu Val Ser Leu
130 135 140

Gly Ala Val Gln Leu Gln Asp Ile Asn Thr Leu Val Leu Thr Ser Asn
145 150 155 160

```
Ala Ser Val Glu Asp Gly Gly Val Ile Lys Gly Asn Ser Cys Leu Ile
                165                 170                 175

Gln Gly Ile Lys Asn Ser Ala Ile Phe Gly Gln Asn Thr Ser Ser Lys
            180                 185                 190

Lys Gly Gly Ala Ile Ser Thr Thr Gln Gly Leu Thr Ile Glu Asn Asn
        195                 200                 205

Leu Gly Thr Leu Lys Phe Asn Glu Asn Lys Ala Val Thr Ser Gly Gly
    210                 215                 220

Ala Leu Asp Leu Gly Ala Ala Ser Thr Phe Thr Ala Asn His Glu Leu
225                 230                 235                 240

Ile Phe Ser Gln Asn Lys Thr Ser Gly Asn Ala Ala Asn Gly Gly Ala
                245                 250                 255

Ile Asn Cys Ser Gly Asp Leu Thr Phe Thr Asp Asn Thr Ser Leu Leu
            260                 265                 270

Leu Gln Glu Asn Ser Thr Met Gln Asp Gly Gly Ala Leu Cys Ser Thr
        275                 280                 285

Gly Thr Ile Ser Ile Thr Gly Ser Asp Ser Ile Asn Val Ile Gly Asn
    290                 295                 300

Thr Ser Gly Gln Lys Gly Gly Ala Ile Ser Ala Ala Ser Leu Lys Ile
305                 310                 315                 320

Leu Gly Gly Gln Gly Gly Ala Leu Phe Ser Asn Asn Val Val Thr His
                325                 330                 335

Ala Thr Pro Leu Gly Gly Ala Ile Phe Ile Asn Thr Gly Gly Ser Leu
            340                 345                 350

Gln Leu Phe Thr Gln Gly Gly Asp Ile Val Phe Glu Gly Asn Gln Val
        355                 360                 365

Thr Thr Thr Ala Pro Asn Ala Thr Thr Lys Arg Asn Val Ile His Leu
    370                 375                 380

Glu Ser Thr Ala Lys Trp Thr Gly Leu Ala Ala Ser Gln Gly Asn Ala
385                 390                 395                 400

Ile Tyr Phe Tyr Asp Pro Ile Thr Thr Asn Asp Thr Gly Ala Ser Asp
                405                 410                 415

Asn Leu Arg Ile Asn Glu Val Ser Ala Asn Gln Lys Leu Ser Gly Ser
            420                 425                 430

Ile Val Phe Ser Gly Glu Arg Leu Ser Thr Ala Glu Ala Ile Ala Glu
        435                 440                 445

Asn Leu Thr Ser Arg Ile Asn Gln Pro Val Thr Leu Val Glu Gly Ser
    450                 455                 460

Leu Val Leu Lys Gln Gly Val Thr Leu Ile Thr Gln Gly Phe Ser Gln
465                 470                 475                 480

Glu Pro Glu Ser Thr Leu Leu Leu Asp Leu Gly Thr Ser Leu
```

485 490

<210> 134
<211> 1485
<212> DNA
<213> Chlamydia pneumoniae

<400> 134
atgaagactt cagtttctat gttgttggcc ctgctttgct cgggggctag ctctattgta 60
ctccatgccg caaccactcc actaaatcct gaagatgggt ttattgggga gggcaataca 120
aatacttttt ctccgaaatc tacaacggat gctgcaggaa ctacctactc tctcacagga 180
gaggttctgt atatagatcc ggggaaaggt ggttcaatta caggaacttg ctttgtagaa 240
actgctggcg atcttacatt tttaggtaat ggaaataccc taaagttcct gtcggtagat 300
gcaggtgcta atatcgcggt tgctcatgta caaggaagta agaatttaag cttcacagat 360
ttcctttctc tggtgatcac agaatctcca aaatccgctg ttactacagg aaaaggtagc 420
ctagtcagtt taggtgcagt ccaactgcaa gatataaaca ctctagttct tacaagcaat 480
gcctctgtcg aagatggtgg cgtgattaaa ggaaactcct gcttgattca gggaatcaaa 540
aatagtgcga tttttggaca aaatacatct tcgaaaaaag gaggggcgat ctccacgact 600
caaggactta ccatagagaa taacttaggg acgctaaagt tcaatgaaaa caaagcagtg 660
acctcaggag gcgccttaga tttaggagcc gcgtctacat tcactgcgaa ccatgagttg 720
atattttcac aaaataagac ttctgggaat gctgcaaatg gcggagccat aaattgctca 780
ggggacctta catttactga taacacttct ttgttacttc aagaaaatag cacaatgcag 840
gatggtggag ctttgtgtag cacaggaacc ataagcatta ccggtagtga ttctatcaat 900
gtgataggaa atacttcagg acaaaaagga ggagcgattt ctgcagcttc tctcaagatt 960
ttgggagggc agggaggcgc tctctttttct aataacgtag tgactcatgc cacccctcta 1020
ggaggtgcca tttttatcaa cacaggagga tccttgcagc tcttcactca aggaggggat 1080
atcgtattcg aggggaatca ggtcactaca acagctccaa atgctaccac taagagaaat 1140
gtaattcacc tcgagagcac cgcgaagtgg acgggacttg ctgcaagtca aggtaacgct 1200
atctatttct atgatcccat taccaccaac gatacgggag caagcgataa cttacgtatc 1260
aatgaggtca gtgcaaatca aaagctctcg ggatctatag tattttctgg agagagattg 1320
tcgacagcag aagctatagc tgaaaatctt acttcgagga tcaaccagcc tgtcacttta 1380
gtagagggga gcttagtact taaacaggga gtgaccttga tcacacaagg attctcgcag 1440
gagccagaat ccacgcttct tttggatctg ggacctcat tataa 1485

<210> 135
<211> 964
<212> PRT
<213> Chlamydia pneumoniae

<400> 135
Met Arg Lys Leu Arg Ile Leu Ala Ile Val Leu Ile Ala Leu Ser Ile
1 5 10 15

Ile Leu Ile Ala Gly Gly Val Val Leu Leu Thr Val Ala Ile Pro Gly
20 25 30

Leu Ser Ser Val Ile Ser Ser Pro Ala Gly Met Gly Ala Cys Ala Leu
35 40 45

Gly Cys Val Met Leu Ala Leu Gly Ile Asp Val Leu Leu Lys Lys Arg
50 55 60

Glu Val Pro Ile Val Leu Ala Ser Val Thr Thr Thr Pro Gly Thr Gly
65 70 75 80

Ser Pro Arg Ser Gly Ile Ser Ile Ser Gly Ala Asp Ser Thr Ile Arg
85 90 95

Ser Leu Pro Thr Tyr Leu Leu Asp Glu Gly His Pro Gln Ser Met Arg
100 105 110

```
Lys Leu Arg Ile Leu Ala Ile Val Leu Ile Val Phe Ser Ile Ile Leu
        115                 120                 125

Ile Ala Ser Gly Val Val Leu Leu Thr Val Ala Ile Pro Gly Leu Ser
    130                 135                 140

Ser Val Ile Ser Ser Pro Ala Gly Met Gly Ala Cys Ala Leu Gly Cys
145                 150                 155                 160

Val Met Leu Ala Leu Gly Ile Asp Val Leu Leu Lys Lys Arg Glu Val
                165                 170                 175

Pro Ile Val Leu Ala Ser Val Thr Thr Thr Pro Gly Thr Gly Ser Pro
            180                 185                 190

Arg Ser Gly Ile Ser Ile Ser Gly Ala Asp Ser Thr Ile Arg Ser Leu
        195                 200                 205

Pro Thr Tyr Pro Leu Asp Glu Gly His Pro Gln Ser Met Arg Lys Leu
    210                 215                 220

Arg Ile Leu Ala Ile Val Leu Ile Val Phe Ser Ile Ile Leu Ile Ala
225                 230                 235                 240

Ser Gly Val Val Leu Leu Thr Val Ala Ile Pro Gly Leu Ser Ser Ile
                245                 250                 255

Ile Ser Ser Pro Ala Glu Met Gly Ala Cys Ala Leu Gly Cys Val Met
            260                 265                 270

Leu Ala Leu Gly Ile Asp Val Leu Leu Lys Lys Arg Glu Val Pro Ile
        275                 280                 285

Val Val Pro Ala Pro Ile Pro Glu Glu Val Val Ile Asp Asp Ile Asp
    290                 295                 300

Glu Glu Ser Ile Arg Leu Gln Gln Glu Ala Glu Ala Ala Leu Ala Arg
305                 310                 315                 320

Leu Pro Glu Glu Met Ser Ala Phe Glu Gly Tyr Ile Lys Val Val Glu
                325                 330                 335

Ser His Leu Glu Asn Met Lys Ser Leu Pro Tyr Asp Gly His Gly Leu
            340                 345                 350

Glu Glu Lys Thr Lys His Gln Ile Arg Val Val Arg Ser Ser Leu Lys
        355                 360                 365

Ala Met Val Pro Glu Phe Leu Asp Ile Arg Arg Ile Phe Glu Glu Glu
    370                 375                 380

Glu Phe Phe Phe Leu Ser Ala Arg Lys Arg Leu Ile Asp Leu Ala Thr
385                 390                 395                 400

Thr Leu Val Glu Arg Lys Ile Leu Thr Glu Gln Leu Glu Arg Asn Asn
                405                 410                 415

Leu Arg Lys Ala Phe Ser Tyr Leu Tyr Gln Asp Ser Ile Phe Lys Lys
            420                 425                 430

Ile Ile Asp Asn Phe Glu Lys Leu Ala Trp Lys Phe Met Ile Leu Ser
```

```
        435                 440                 445

Lys Ser Ile Cys Arg Phe Thr Ile Ile Phe Glu Asn His Glu His Gly
    450                 455                 460

Val Ala Lys Ser Leu Leu His Lys Asn Ala Val Leu Leu Glu Lys Val
465                 470                 475                 480

Ile Tyr Arg Ser Leu Gln Lys Ser Tyr Arg Asp Ile Gly Met Ser Ser
                485                 490                 495

Ala Lys Met Lys Ile Leu His Gly Asn Pro Phe Phe Ser Leu Glu Asp
            500                 505                 510

Asn Lys Lys Thr Ile Met Lys Glu His Ala Glu Met Leu Glu Ser Leu
        515                 520                 525

Ser Ser Tyr Arg Lys Val Phe Leu Ala Leu Ser Asp Glu Asn Val Val
    530                 535                 540

Asp Thr Pro Ser Asp Pro Lys Lys Trp Asp Leu Ser Gly Ile Pro Cys
545                 550                 555                 560

Arg Asp Ala Leu Ser Glu Ile Ser Arg Asp Glu Gln Trp Gln Lys Lys
                565                 570                 575

Ala His Leu Lys His Gln Glu Ser Leu Tyr Thr Gln Ala Arg Asp Arg
            580                 585                 590

Leu Thr Asp Gln Ser Ser Lys Glu Asn Gln Lys Glu Leu Glu Lys Ala
        595                 600                 605

Glu Gln Glu Tyr Ile Ser Ser Trp Glu Arg Val Lys Lys Phe Glu Ile
    610                 615                 620

Glu Arg Val Gln Glu Arg Ile Arg Ala Ile Gln Lys Leu Tyr Pro Asn
625                 630                 635                 640

Ile Leu Glu Arg Glu Glu Glu Thr Thr Gly Gln Glu Thr Val Thr Pro
                645                 650                 655

Thr Val Gln Gly Thr Thr Ala Ser Ser Asp Leu Thr Asp Ile Leu Gly
            660                 665                 670

Arg Ile Glu Val Ser Ser Arg Glu Asp Asn Gln Asn Gln Glu Ser Cys
        675                 680                 685

Val Lys Val Leu Arg Ser His Glu Val Glu Met Ser Trp Glu Val Lys
    690                 695                 700

Gln Glu Tyr Gly Pro Lys Lys Lys Glu Phe Gln Asp Gln Met Gly Ser
705                 710                 715                 720

Leu Glu Arg Phe Phe Thr Glu His Ile Glu Glu Leu Glu Val Leu Gln
                725                 730                 735

Lys Asp Tyr Ser Lys His Leu Ser Tyr Phe Lys Lys Val Asn Asn Lys
            740                 745                 750

Lys Glu Val Gln Tyr Ala Lys Phe Arg Leu Lys Val Leu Glu Ser Asp
        755                 760                 765
```

```
Leu Glu Gly Ile Leu Ala Gln Thr Glu Ser Ala Glu Ser Leu Leu Thr
    770                 775                 780

Gln Glu Glu Leu Pro Ile Leu Ala Thr Arg Gly Ala Leu Glu Lys Ala
785                 790                 795                 800

Val Phe Lys Gly Ser Leu Cys Cys Ala Leu Ala Ser Lys Ala Lys Pro
                805                 810                 815

Tyr Phe Glu Glu Asp Pro Arg Phe Gln Asp Ser Asp Thr Gln Leu Arg
            820                 825                 830

Ala Leu Thr Leu Arg Leu Gln Glu Ala Lys Ala Ser Leu Glu Glu Glu
        835                 840                 845

Ile Lys Arg Phe Ser Asn Leu Glu Asn Asp Ile Ala Glu Glu Arg Arg
    850                 855                 860

Leu Leu Lys Glu Ser Lys Gln Thr Phe Glu Arg Ala Gly Leu Gly Val
865                 870                 875                 880

Leu Arg Glu Ile Ala Val Glu Ser Thr Tyr Asp Leu Arg Ser Leu Thr
                885                 890                 895

Asn Thr Trp Glu Gly Thr Pro Glu Ser Glu Lys Val Tyr Phe Ser Met
            900                 905                 910

Tyr Leu Asn Tyr Tyr Asn Glu Glu Lys Arg Arg Ala Lys Thr Arg Leu
        915                 920                 925

Val Glu Met Thr Gln Arg Tyr Arg Asp Phe Lys Met Ala Leu Glu Ala
    930                 935                 940

Met Gln Phe Asn Glu Glu Ala Leu Leu Gln Glu Glu Leu Ser Ile Gln
945                 950                 955                 960

Ala Pro Ser Glu


<210>   136
<211>   2895
<212>   DNA
<213>   Chlamydia pneumoniae

<400>   136
atgaggaaac ttcgtattct tgcgatcgtt ctcatagctt tgagcattat tttgattgca     60
ggtggtgtgg tattgcttac tgtagcgatc cctggattaa gttcagtcat ttcttccccg    120
gcagggatgg gtgcctgtgc tttgggatgt gtgatgcttg ctttagggat cgatgttctt    180
ctgaagaaac gagaagtccc tatagttctc gcatctgtaa ctacgacacc aggaactggc    240
agccctagaa gtggtatttc tatttcagga gctgatagca ccatacgttc tcttcctacg    300
tatctcttgg acgagggaca tccacaatcc atgaggaaac ttcgtattct tgcgatcgtt    360
ctcatagttt ttagcattat tttgattgca agtggtgtgg tattgcttac tgtagcgatc    420
cctggattaa gttcagtcat ttcttccccg gcagggatgg gtgcctgtgc tttgggatgt    480
gtgatgcttg ctttagggat cgatgttctt ctgaagaaac gagaagtccc tatagttctc    540
gcatctgtaa ctacgacacc aggaactggc agccctagaa gtggtatttc tatttcagga    600
gctgatagca ccatacgttc tcttcctacg tatcccttgg acgagggaca tccacaatcc    660
atgaggaaac ttcgtattct tgcgatcgtt ctcatagttt ttagcattat tttgattgca    720
agtggtgtgg tattgcttac tgtagcgatc cctggattaa gctcgatcat ttcttcccca    780
gcggagatgg gtgcttgtgc tttgggatgt gtgatgcttg ctttggggat cgacgttctt    840
ctgaagaaac gagaagtccc tatagtagtt cccgcaccta ttcctgaaga agtcgtcata    900
```

```
gatgatatag atgaagagag tatacggctg cagcaggaag ctgaagccgc tttagcaaga    960
cttcctgagg agatgagtgc atttgaaggt tacataaaag ttgtcgagag tcatttggag   1020
aacatgaaaa gcctgcctta tgatggtcat gggctagaag agaaaacgaa acatcagata   1080
agagtcgtca gatcttcttt gaaggctatg gttccagaat tttagatat cagaagaatt    1140
tttgaagaag aagagttctt ttttctctca gctcgcaaac gacttataga tttagctact   1200
actttagtag agagaaaaat tttaacagag caacttgagc gcaataattt aaggaaagcg   1260
ttttcttatt tatatcagga ctcaattttt aaaaaaatta ttgataactt cgagaagtta   1320
gcatggaaat ttatgatttt gagtaaatca atttgtcgat ttacaattat ttttgaaaat   1380
catgaacatg gtgtagcaaa gagcctgtta cacaagaatg cagtgttact ggagaaggta   1440
atctatagga gtttgcaaaa aagctataga gatataggca tgtcatctgc aaagatgaaa   1500
atcttgcacg gcaacccttt tttctctttg gaagataata aaaagacgat aatgaaagaa   1560
cacgcagaga tgcttgaaag tctcagtagc tataggaagg tatttttagc tctatctgat   1620
gagaacgttg tagatacacc tagcgatcca aagaaatggg atttgtcagg aatcccctgt   1680
agggacgcgt tgtctgagat ttctcgtgat gaacagtggc agaagaaagc acatctaaag   1740
catcaagagt ccctctatac gcaagctagg gatcgtttaa cagaccagag ctctaaagaa   1800
aatcagaaag agttagagaa agctgaacaa gagtacatat cttcttggga acgggttaaa   1860
aaatttgaga ttgagagagt acaggagagg atacgggcaa ttcaaaagct ttatcctaat   1920
atcctcgaga gagaagaaga aaccacaggt caggagactg tgactccaac tgttcaaggg   1980
acgacggctt catccgattt aacagatatt ttaggaagaa tagaggtctc cagtagggag   2040
gataatcaga atcaagagtc ttgtgtaaaa gtcttaagaa gtcatgaggt agaaatgagc   2100
tgggaagtca aacaagagta tggccctaag aaaaaagaat ttcaggatca aatgggttct   2160
ttagagaggt tttttacaga gcatattgaa gagttagaag tattacagaa ggactactct   2220
aaacacttgt cttattttaa aaaagtaaac aataagaaag aggttcaata tgcgaagttt   2280
aggttgaagg ttttagagtc agatttagaa gggattctag ctcagactga gagtgctgag   2340
agtctgttaa ctcaagaaga acttccgatt cttgcaactc ggggagcctt agagaaagct   2400
gttttcaaag ggagtctatg ttgcgcgcta gcaagcaaag caaaacccta ttttgaagag   2460
gatcccagat tccaagattc tgatacgcaa ttgcgagctc tgactctaag gttacaggag   2520
gctaaggcaa gcctggaaga agagataaag agattttcaa atcttgagaa cgatattgca   2580
gaggaaagac gccttcttaa agagagcaag cagacgttcg aaagagcagg tttaggggtt   2640
ctccgagaaa ttgcagtcga gtctacttat gatttgcgtt ccttaacaaa tacatgggaa   2700
gggaccccag agagtgagaa ggtctatttt agcatgtatc ttaattatta caacgaagag   2760
aaacgtaggg ctaaaacaag attggttgaa atgacacaga ggtatagaga ttttaaaatg   2820
gccttggaag ctatgcagtt taatgaagaa gcccttttgc aagaggaact ctctattcaa   2880
gctcccagtg aataa                                                    2895
```

<210> 137
<211> 523
<212> PRT
<213> Chlamydia pneumoniae

<400> 137

```
Met Tyr Gln Glu Asn Leu Arg Leu Leu Glu Arg Leu Leu Tyr Asn Ser
1               5                   10                  15

Val Gln Lys Ser Tyr Ala Asp Arg Leu Phe Ser Tyr Glu Lys Thr Lys
            20                  25                  30

Met Val His Asp Thr Pro Leu Ile Pro Trp Glu Glu Asp Lys Glu Lys
        35                  40                  45

Cys Ala Glu Ala Glu Lys Ala Phe Leu Glu Gln Gln Lys Ile Leu Leu
    50                  55                  60

Asp Tyr Gly Lys Ser Ile Phe Trp Leu Asn Glu Asn Asp Glu Ile Asn
65                  70                  75                  80

Leu Asn Asp Pro Trp Ser Trp Gly Leu Asn Thr Val Arg Thr Arg Lys
                85                  90                  95

Val Phe Gln Glu Val Asp Asp Ser Glu Arg Trp Asn His Lys Val Leu
            100                 105                 110
```

Ile Gln Lys Leu Glu Asp Asp Tyr Glu Lys Leu Leu Glu Glu Ser Ser
115 120 125

Lys Glu Ser Thr Glu Ala Asn Lys Lys Leu Leu Ser Asp Leu Val Asp
130 135 140

Arg Leu Glu Asp Ala Lys Thr Lys Phe Phe Leu Lys Lys Gln Glu Glu
145 150 155 160

Val Glu Thr Arg Val Lys Asp Leu Arg Ala Arg Tyr Gly Gly Thr Val
165 170 175

Asp Pro Lys Gln Asp Thr Glu Ala Lys Lys Lys Val Glu Leu Glu Ala
180 185 190

Ser Leu Glu Thr Phe Leu Asp Ser Ile Glu Ser Glu Leu Val Gln Cys
195 200 205

Leu Glu Asp Gln Asp Ile Tyr Trp Lys Glu Gln Asp Val Lys Asp Leu
210 215 220

Ala Arg Thr Gln Glu Leu Glu Glu Gln Asp Ile Glu Ala Lys Arg Glu
225 230 235 240

Glu Ala Ala Glu Asp Leu Arg Ser Leu Asn Glu Arg Leu Lys Lys Ser
245 250 255

Lys Thr Met Leu Asp Arg Ala Lys Trp His Ile Glu Asn Ala Glu Asp
260 265 270

Ser Ile Thr Trp Trp Thr Ser Gln Ile Glu Met Lys Asp Met Lys Ala
275 280 285

Arg Leu Lys Ile Leu Lys Glu Asp Ile Thr Ser Val Leu Pro Glu Ile
290 295 300

Asp Glu Ile Glu Thr Cys Leu Ser Leu Glu Glu Leu Pro Leu Leu Thr
305 310 315 320

Thr Arg Glu Leu Leu Thr Lys Ser Tyr Leu Lys Phe Lys Ile Cys Ser
325 330 335

Glu Thr Leu Leu Lys Met Thr Ser Val Phe Glu Asn Asn Ile Tyr Val
340 345 350

Gln Glu Tyr Glu Val Gln Leu Gln Asn Leu Gly Phe Lys Leu Gln Gly
355 360 365

Ile Ser Gln Arg Phe Gly Lys Lys Gln Asp Asp Phe Ala Asn Leu Glu
370 375 380

Glu Gln Val Ala Leu Gln Lys Lys Arg Leu Arg Glu Leu Thr Gln Asn
385 390 395 400

Phe Glu Ile Gln Gly Phe Asn Phe Met Lys Glu Asp Phe Lys Ala Ala
405 410 415

Ala Lys Asp Leu Tyr Ile Arg Ser Thr Ala Glu Gln Lys Met Asn Phe
420 425 430

Asp Val Pro Cys Met Glu Leu Phe Arg Arg Tyr His Glu Glu Val Asn
435 440 445

Lys Pro Leu Leu Glu Leu Met Tyr Asn Cys Ala Asp Ser Tyr Arg Asp
450 455 460

Ala Lys Lys Lys Leu Cys Ser Leu Arg Leu Asp Glu Lys Glu Leu Leu
465 470 475 480

Gln Lys Glu Ile Lys Lys Glu Glu Phe Tyr Gln Lys Lys Gln Gln Arg
485 490 495

His Ala Asp Arg Ser Arg His Thr Thr Tyr Gln Lys Leu Arg Ile Ala
500 505 510

Glu Glu Leu Ala Leu Glu Leu Lys Lys Lys Ile
515 520

<210> 138
<211> 1572
<212> DNA
<213> Chlamydia pneumoniae

<400> 138
atgtaccagg agaatctaag attgttggaa aggcttcttt ataatagtgt tcaaaagagc 60
tatgcggatc ggctgttttc ctatgaaaag acaaagatgg tgcacgatac tccgctgatt 120
ccttgggaag aggataagga aaaatgtgct gaagctgaga aagctttctt agagcaacag 180
aagattctcc tagattatgg aaaatctatc ttttggctga atgagaacga tgagatcaat 240
ttaaacgatc cttggagttg ggtcttaat acggtgagga ctaggaaagt attccaagag 300
gttgacgaca gtgaacgttg gaatcataag gtactcattc aaaaactcga ggacgattat 360
gagaaacttc tagaggaaag ttcaaaagag tctactgaag caaataagaa gcttttatct 420
gacttagtag atcgtcttga agatgctaag acaaaatttt tcctgaagaa acaggaggag 480
gtggagactc gcgttaagga tcttagagct cgatatggag gcacagtaga tcctaagcag 540
gatacggaag ctaagaagaa agtcgaattg gaggctagct tagaaacctt tttagattcc 600
atcgaatcag agctagtaca gtgtttagaa gatcaagata tatattggaa agaacaggat 660
gtcaaagatc tagcacgtac gcaagagctc gaggaacaag atattgaagc gaagagggaa 720
gaagctgccg aagacctaag aagtcttaat gagcgtttaa agaagtcaaa aactatgtta 780
gatagggcta aatggcatat tgaaaatgct gaggacagta ttacctggtg gactagtcag 840
atagaaatga aggatatgaa agcaagactg aagatcttaa aagaagatat aacaagtgtt 900
ctacctgaaa tagatgagat tgaaacgtgt ttaagcttag aggagcttcc tttgcttacg 960
accagggaac tcttaactaa gtcctaccta aagtttaaga tttgttcgga aacactatta 1020
aaaatgactt ctgtgtttga gaacaatatc tatgttcagg agtacgaggt tcagctgcaa 1080
aatctagggt ttaagttaca aggtatatct cagagattcg gaaagaaaca agacgatttt 1140
gcgaatctag aggaacaggt tgctttgcaa aagaaacgac tcagagagct cactcagaat 1200
tttgaaatac aaggattcaa tttcatgaaa gaagatttta aggcagccgc taaagatctt 1260
tatataagaa gtacagctga acaaaagatg aactttgatg tgccttgcat ggagctcttc 1320
cgtaggtatc atgaggaggt caacaagccg cttcttgagt tgatgtacaa ttgtgcagac 1380
agttatagag atgctaagaa aaagctttgc tctctacgtc ttgatgaaaa agagttatta 1440
caaaaagaaa tcaagaaaga ggaattttat caaaagaaac aacaaaggca tgcagataga 1500
tcacgtcata ctacgtatca aaagctacga attgctgaag agcttgctct tgagctgaag 1560
aagaaaatct aa 1572

<210> 139
<211> 841
<212> PRT
<213> Chlamydia pneumoniae

<400> 139
Met Lys Ile Pro Leu Arg Phe Leu Leu Ile Ser Leu Val Pro Thr Leu
1 5 10 15

```
Ser Met Ser Asn Leu Leu Gly Ala Ala Thr Thr Glu Glu Leu Ser Ala
            20                  25                  30

Ser Asn Ser Phe Asp Gly Thr Thr Ser Thr Thr Ser Phe Ser Ser Lys
        35                  40                  45

Thr Ser Ser Ala Thr Asp Gly Thr Asn Tyr Val Phe Lys Asp Ser Val
    50                  55                  60

Val Ile Glu Asn Val Pro Lys Thr Gly Glu Thr Gln Ser Thr Ser Cys
65                  70                  75                  80

Phe Lys Asn Asp Ala Ala Ala Gly Asp Leu Asn Phe Leu Gly Gly Gly
                85                  90                  95

Phe Ser Phe Thr Phe Ser Asn Ile Asp Ala Thr Thr Ala Ser Gly Ala
            100                 105                 110

Ala Ile Gly Ser Glu Ala Ala Asn Lys Thr Val Thr Leu Ser Gly Phe
        115                 120                 125

Ser Ala Leu Ser Phe Leu Lys Ser Pro Ala Ser Thr Val Thr Asn Gly
    130                 135                 140

Leu Gly Ala Ile Asn Val Lys Gly Asn Leu Ser Leu Leu Asp Asn Asp
145                 150                 155                 160

Lys Val Leu Ile Gln Asp Asn Phe Ser Thr Gly Asp Gly Gly Ala Ile
                165                 170                 175

Asn Cys Ala Gly Ser Leu Lys Ile Ala Asn Asn Lys Ser Leu Ser Phe
            180                 185                 190

Ile Gly Asn Ser Ser Ser Thr Arg Gly Gly Ala Ile His Thr Lys Asn
        195                 200                 205

Leu Thr Leu Ser Ser Gly Gly Glu Thr Leu Phe Gln Gly Asn Thr Ala
    210                 215                 220

Pro Thr Ala Ala Gly Lys Gly Gly Ala Ile Ala Ile Ala Asp Ser Gly
225                 230                 235                 240

Thr Leu Ser Ile Ser Gly Asp Ser Gly Asp Ile Ile Phe Glu Gly Asn
                245                 250                 255

Thr Ile Gly Ala Thr Gly Thr Val Ser His Ser Ala Ile Asp Leu Gly
            260                 265                 270

Thr Ser Ala Lys Ile Thr Ala Leu Arg Ala Ala Gln Gly His Thr Ile
        275                 280                 285

Tyr Phe Tyr Asp Pro Ile Thr Val Thr Gly Ser Thr Ser Val Ala Asp
    290                 295                 300

Ala Leu Asn Ile Asn Ser Pro Asp Thr Gly Asp Asn Lys Glu Tyr Thr
305                 310                 315                 320

Gly Thr Ile Val Phe Ser Gly Glu Lys Leu Thr Glu Ala Glu Ala Lys
                325                 330                 335

Asp Glu Lys Asn Arg Thr Ser Lys Leu Leu Gln Asn Val Ala Phe Lys
```

340 345 350

Asn Gly Thr Val Val Leu Lys Gly Asp Val Val Leu Ser Ala Asn Gly
355 360 365

Phe Ser Gln Asp Ala Asn Ser Lys Leu Ile Met Asp Leu Gly Thr Ser
370 375 380

Leu Val Ala Asn Thr Glu Ser Ile Glu Leu Thr Asn Leu Glu Ile Asn
385 390 395 400

Ile Asp Ser Leu Arg Asn Gly Lys Lys Ile Lys Leu Ser Ala Ala Thr
405 410 415

Ala Gln Lys Asp Ile Arg Ile Asp Arg Pro Val Val Leu Ala Ile Ser
420 425 430

Asp Glu Ser Phe Tyr Gln Asn Gly Phe Leu Asn Glu Asp His Ser Tyr
435 440 445

Asp Gly Ile Leu Glu Leu Asp Ala Gly Lys Asp Ile Val Ile Ser Ala
450 455 460

Asp Ser Arg Ser Ile Asp Ala Val Gln Ser Pro Tyr Gly Tyr Gln Gly
465 470 475 480

Lys Trp Thr Ile Asn Trp Ser Thr Asp Asp Lys Lys Ala Thr Val Ser
485 490 495

Trp Ala Lys Gln Ser Phe Asn Pro Thr Ala Glu Gln Glu Ala Pro Leu
500 505 510

Val Pro Asn Leu Leu Trp Gly Ser Phe Ile Asp Val Arg Ser Phe Gln
515 520 525

Asn Phe Ile Glu Leu Gly Thr Glu Gly Ala Pro Tyr Glu Lys Arg Phe
530 535 540

Trp Val Ala Gly Ile Ser Asn Val Leu His Arg Ser Gly Arg Glu Asn
545 550 555 560

Gln Arg Lys Phe Arg His Val Ser Gly Gly Ala Val Val Gly Ala Ser
565 570 575

Thr Arg Met Pro Gly Gly Asp Thr Leu Ser Leu Gly Phe Ala Gln Leu
580 585 590

Phe Ala Arg Asp Lys Asp Tyr Phe Met Asn Thr Asn Phe Ala Lys Thr
595 600 605

Tyr Ala Gly Ser Leu Arg Leu Gln His Asp Ala Ser Leu Tyr Ser Val
610 615 620

Val Ser Ile Leu Leu Gly Glu Gly Gly Leu Arg Glu Ile Leu Leu Pro
625 630 635 640

Tyr Val Ser Lys Thr Leu Pro Cys Ser Phe Tyr Gly Gln Leu Ser Tyr
645 650 655

Gly His Thr Asp His Arg Met Lys Thr Glu Ser Leu Pro Pro Pro Pro
660 665 670

Pro Thr Leu Ser Thr Asp His Thr Ser Trp Gly Gly Tyr Val Trp Ala
675 680 685

Gly Glu Leu Gly Thr Arg Val Ala Val Glu Asn Thr Ser Gly Arg Gly
690 695 700

Phe Phe Gln Glu Tyr Thr Pro Phe Val Lys Val Gln Ala Val Tyr Ala
705 710 715 720

Arg Gln Asp Ser Phe Val Glu Leu Gly Ala Ile Ser Arg Asp Phe Ser
725 730 735

Asp Ser His Leu Tyr Asn Leu Ala Ile Pro Leu Gly Ile Lys Leu Glu
740 745 750

Lys Arg Phe Ala Glu Gln Tyr Tyr His Val Val Ala Met Tyr Ser Pro
755 760 765

Asp Val Cys Arg Ser Asn Pro Lys Cys Thr Thr Thr Leu Leu Ser Asn
770 775 780

Gln Gly Ser Trp Lys Thr Lys Gly Ser Asn Leu Ala Arg Gln Ala Gly
785 790 795 800

Ile Val Gln Ala Ser Gly Phe Arg Ser Leu Gly Ala Ala Ala Glu Leu
805 810 815

Phe Gly Asn Phe Gly Phe Glu Trp Arg Gly Ser Ser Arg Ser Tyr Asn
820 825 830

Val Asp Ala Gly Ser Lys Ile Lys Phe
835 840

<210> 140
<211> 2526
<212> DNA
<213> Chlamydia pneumoniae

<400> 140
atgaagattc cactccgctt tttattgata tcattagtac ctacgctttc tatgtcgaat 60
ttattaggag ctgctactac cgaagagtta tcggctagca atagcttcga tggaactaca 120
tcaacaacaa gcttttctag taaaacatca tcggctacag atggcaccaa ttatgttttt 180
aaagattctg tagttataga aaatgtaccc aaaacagggg aaactcagtc tactagttgt 240
tttaaaaatg acgctgcagc tggagatcta aatttcttag gaggggatt ttctttcaca 300
tttagcaata tcgatgcaac cacggcttct ggagctgcta ttggaagtga agcagctaat 360
aagacagtca cgttatcagg attttcggca ctttcttttc ttaaatcccc agcaagtaca 420
gtgactaatg gattgggagc tatcaatgtt aaagggaatt taagcctatt ggataatgat 480
aaggtattga ttcaggacaa tttctcaaca ggagatggcg gagcaattaa ttgtgcaggc 540
tccttgaaga tcgcaaacaa taagtccctt tcttttattg gaaatagttc ttcaacacgt 600
ggcggagcga ttcataccaa aaacctcaca ctatcttctg gtggggaaac tctatttcag 660
gggaatacag cgcctacggc tgctggtaaa ggaggtgcta tcgcgattgc agactctggc 720
accctatcca tttctggaga cagtggcgac attatctttg aaggcaatac gataggagct 780
acaggaaccg tctctcatag tgctattgat ttaggaacta gcgctaagat aactgcgtta 840
cgtgctgcgc aaggacatac gatatacttt tatgatccga ttactgtaac aggatcgaca 900
tctgttgctg atgctctcaa tattaatagc cctgatactg gagataacaa agagtatacg 960
ggaaccatag tcttttctgg agagaagctc acggaggcag aagctaaaga tgagaagaac 1020
cgcacttcta aattacttca aaatgttgct tttaaaaatg ggactgtagt tttaaaaggt 1080
gatgtcgttt taagtgcgaa cggtttctct caggatgcaa actctaagtt gattatggat 1140
ttagggacgt cgttggttgc aaacaccgaa agtatcgagt taacgaattt ggaaattaat 1200
atagactctc tcaggaacgg gaaaaagata aaactcagtg ctgccacagc tcagaaagat 1260

```
attcgtatag atcgtcctgt tgtactggca attagcgatg agagtttttta tcaaaatggc   1320
tttttgaatg aggaccattc ctatgatggg attcttgagt tagatgctgg gaaagacatc   1380
gtgatttctg cagattctcg cagtatagat gctgtacaat ctccgtatgg ctatcaggga   1440
aagtggacga tcaattggtc tactgatgat aagaaagcta cggtttcttg ggcgaagcag   1500
agttttaatc ccactgctga gcaggaggct ccgttagttc ctaatcttct ttggggttct   1560
tttatagatg ttcgttcctt ccagaatttt atagagctag gtactgaagg tgctccttac   1620
gaaaagagat tttgggttgc aggcatttcc aatgttttgc ataggagcgg tcgtgaaaat   1680
caaaggaaat tccgtcatgt gagtggaggt gctgtagtag gtgctagcac gaggatgccg   1740
ggtggtgata ccttgtctct gggttttgct cagctctttg cgcgtgacaa agactacttt   1800
atgaatacca atttcgcaaa gacctacgca ggatctttac gtttgcagca cgatgcttcc   1860
ctatactctg tggtgagtat ccttttagga gagggaggac tccgcgagat cctgttgcct   1920
tatgtttcca agactctgcc gtgctctttc tatgggcagc ttagctacgg ccatacggat   1980
catcgcatga agaccgagtc tctacccccc cccccccga cgctctcgac ggatcatact   2040
tcttggggag gatatgtctg ggctggagag ctgggaactc gagttgctgt tgaaaatacc   2100
agcggcagag gattttttcca agagtacact ccatttgtaa aagtccaagc tgtttacgct   2160
cgccaagata gctttgtaga actaggagct atcagtcgtg attttagtga ttcgcatctt   2220
tataaccttg cgattcctct tggaatcaag ttagagaaac ggtttgcaga gcaatattat   2280
catgttgtag cgatgtattc tccagatgtt tgtcgtagta accccaaatg tacgactacc   2340
ctactttcca accaagggag ttggaagacc aaaggttcga acttagcaag acaggctggt   2400
attgttcagg cctcaggttt tcgatctttg ggagctgcag cagagctttt cgggaacttt   2460
ggctttgaat ggcggggatc ttctcgtagc tataatgtag atgcgggtag caaaatcaaa   2520
ttttag                                                               2526
```

<210> 141
<211> 251
<212> PRT
<213> Chlamydia pneumoniae

<400> 141

```
Met Asn Val Ala Asp Leu Leu Ser His Leu Glu Thr Leu Leu Ser Ser
1               5                   10                  15

Lys Ile Phe Gln Asp Tyr Gly Pro Asn Gly Leu Gln Val Gly Asp Pro
            20                  25                  30

Gln Thr Pro Val Lys Lys Ile Ala Val Ala Val Thr Ala Asp Leu Glu
        35                  40                  45

Thr Ile Lys Gln Ala Val Ala Ala Glu Ala Asn Val Leu Ile Val His
    50                  55                  60

His Gly Ile Phe Trp Lys Gly Met Pro Tyr Pro Ile Thr Gly Met Ile
65                  70                  75                  80

His Lys Arg Ile Gln Leu Leu Ile Glu His Asn Ile Gln Leu Ile Ala
                85                  90                  95

Tyr His Leu Pro Leu Asp Ala His Pro Thr Leu Gly Asn Asn Trp Arg
            100                 105                 110

Val Ala Leu Asp Leu Asn Trp His Asp Leu Lys Pro Phe Gly Ser Ser
        115                 120                 125

Leu Pro Tyr Leu Gly Val Gln Gly Ser Phe Ser Pro Ile Asp Ile Asp
    130                 135                 140

Ser Phe Ile Asp Leu Leu Ser Gln Tyr Tyr Gln Ala Pro Leu Lys Gly
145                 150                 155                 160

Ser Ala Leu Gly Gly Pro Ser Arg Val Ser Ser Ala Ala Leu Ile Ser
                165                 170                 175
```

Gly Gly Ala Tyr Arg Glu Leu Ser Ser Ala Ala Thr Ser Gln Val Asp
180 185 190

Cys Phe Ile Thr Gly Asn Phe Asp Glu Pro Ala Trp Ser Thr Ala Leu
195 200 205

Glu Ser Asn Ile Asn Phe Leu Ala Phe Gly His Thr Ala Thr Glu Lys
210 215 220

Val Gly Pro Lys Ser Leu Ala Glu His Leu Lys Ser Glu Phe Pro Ile
225 230 235 240

Ser Thr Thr Phe Ile Asp Thr Ala Asn Pro Phe
245 250

<210> 142
<211> 756
<212> DNA
<213> Chlamydia pneumoniae

<400> 142
atgaatgttg cggatctcct ttctcatctt gagactcttc tctcatcaaa aatatttcag 60
gattatggac ccaacggact tcaagttgga gatccccaaa ctccggtaaa gaaaatcgct 120
gttgcagtta ccgcagatct agaaaccata aaacaagctg ttgcggccga agcaaacgtt 180
ctcattgtac accacggaat tttttggaaa ggtatgccct atcctattac cggcatgatc 240
cataagcgca tccaattact aatagaacac aatatccaac tcattgccta ccaccttcct 300
ttggatgctc accctacctt aggaaataac tggagagttg ccctggatct aaattggcat 360
gacttgaagc cctttggttc ttccctccct tatttaggag tgcaaggctc tttctctcct 420
atcgatatag attctttcat tgacctgtta tctcaatatt accaagctcc cctaaaagga 480
tctgccttgg gcggcccctc tagagtctcc tcagcagctc tgatctcagg aggagcttat 540
agagaactct cttcggcagc cacgtcccaa gtcgattgct tcatcacagg aaattttgat 600
gaacctgcat ggtcgacagc tctagaaagc aatatcaact tcctagcatt tggacataca 660
gccacagaaa aagtaggtcc aaaatctctt gcagagcatc taaaaagcga atttcctatt 720
tccacaacct ttatagatac ggccaacccc ttctaa 756

<210> 143
<211> 285
<212> PRT
<213> Chlamydia pneumoniae

<400> 143
Met Lys His Tyr Leu Ser Phe Ser Pro Ser Ala Asp Phe Phe Ser Lys
1 5 10 15

Gln Gly Ala Ile Glu Thr Gln Val Leu Phe Gly Glu Arg Val Leu Val
20 25 30

Lys Gly Ser Thr Cys Tyr Ala Tyr Ser Gln Leu Phe His Asn Glu Leu
35 40 45

Leu Trp Lys Pro Tyr Pro Gly His Ser Phe Arg Ser Thr Leu Val Pro
50 55 60

Cys Thr Pro Glu Phe His Ile His Pro Asn Val Ser Val Val Ser Val
65 70 75 80

Asp Ala Phe Leu Asp Pro Trp Gly Ile Pro Leu Pro Phe Gly Thr Leu
85 90 95

Leu His Val Asn Ser Gln Asn Thr Val Ile Phe Pro Lys Asp Ile Leu

```
            100                 105                 110

Asn His Met Asn Thr Ile Trp Gly Ser Gly Thr Pro Gln Cys Asp Pro
        115                 120                 125

Arg His Leu Arg Arg Leu Asn Tyr Asn Phe Phe Ala Glu Leu Leu Ile
    130                 135                 140

Lys Asp Ala Asp Leu Leu Leu Asn Phe Pro Tyr Val Trp Gly Gly Arg
145                 150                 155                 160

Ser Val His Glu Ser Leu Glu Lys Pro Gly Val Asp Cys Ser Gly Phe
                165                 170                 175

Ile Asn Ile Leu Tyr Gln Ala Gln Gly Tyr Asn Val Pro Arg Asn Ala
            180                 185                 190

Ala Asp Gln Tyr Ala Asp Cys His Trp Ile Ser Ser Phe Glu Asn Leu
        195                 200                 205

Pro Ser Gly Gly Leu Ile Phe Leu Tyr Pro Lys Glu Glu Lys Arg Ile
    210                 215                 220

Ser His Val Met Leu Lys Gln Asp Ser Ser Thr Leu Ile His Ala Ser
225                 230                 235                 240

Gly Gly Gly Lys Lys Val Glu Tyr Phe Ile Leu Glu Gln Asp Gly Lys
                245                 250                 255

Phe Leu Asp Ser Thr Tyr Leu Phe Phe Arg Asn Asn Gln Arg Gly Arg
            260                 265                 270

Ala Phe Phe Gly Ile Pro Arg Lys Arg Lys Ala Phe Leu
        275                 280                 285

<210>   144
<211>   858
<212>   DNA
<213>   Chlamydia pneumoniae

<400>   144
atgaaacact acctatcatt ttctccttct gctgattttt tctctaaaca gggtgctatt    60
gaaactcaag tcctttttgg agagcgcgtc ttagtcaaag ggagcacctg ctatgcatat   120
tcccaattat tccacaatga gctgttatgg aagccctatc caggtcatag ctttcgttct   180
accctagtcc cctgcactcc tgaatttcat atccatccaa atgtttctgt ggtttctgtg   240
gatgcatttt tagatccttg ggggatccct cttccttttg gaactttact ccatgtgaat   300
tctcaaaata ccgttatttt ccctaaggat attctcaatc atatgaacac catctggggc   360
tccggcacac ctcaatgcga tcctagacat ctacgtcgtc taaattataa cttctttgct   420
gaacttttaa ttaaagacgc agacctttta ctgaactttc cctatgtatg gggaggacgg   480
tctgtacacg aaagtctgga aaagccgggt gttgattgtt cgggatttat caatatcctt   540
taccaggcac agggatacaa cgtccctaga aacgctgcag atcaatatgc ggattgtcat   600
tggatctcta gctttgagaa ccttccttct ggtgggttaa tatttcttta ccctaaagaa   660
gaaaagcgta tttctcatgt tatgttgaaa caggatagtt ccaccctcat tcatgcttct   720
ggtggaggga aaaaagtgga gtatttcatt ttagaacaag atgggaagtt tttagattcg   780
acttatctat tttttagaaa taatcagagg ggacgggcat tttttgggat ccctagaaaa   840
agaaaagcct ttctgtaa                                                 858

<210>   145
<211>   203
<212>   PRT
<213>   Chlamydia pneumoniae
```

<400> 145
Met Thr Ser Pro Ile Pro Phe Gln Ser Ser Gly Asp Ala Ser Phe Leu
1 5 10 15

Ala Glu Gln Pro Gln Gln Leu Pro Ser Thr Ser Glu Ser Gln Leu Val
20 25 30

Thr Gln Leu Leu Thr Met Met Lys His Thr Gln Ala Leu Ser Glu Thr
35 40 45

Val Leu Gln Gln Gln Arg Asp Arg Leu Pro Thr Ala Ser Ile Ile Leu
50 55 60

Gln Val Gly Gly Ala Pro Thr Gly Gly Ala Gly Ala Pro Phe Gln Pro
65 70 75 80

Gly Pro Ala Asp Asp His His His Pro Ile Pro Pro Pro Val Val Pro
85 90 95

Ala Gln Ile Glu Thr Glu Ile Thr Thr Ile Arg Ser Glu Leu Gln Leu
100 105 110

Met Arg Ser Thr Leu Gln Gln Ser Thr Lys Gly Ala Arg Thr Gly Val
115 120 125

Leu Val Val Thr Ala Ile Leu Met Thr Ile Ser Leu Leu Ala Ile Ile
130 135 140

Ile Ile Ile Leu Ala Val Leu Gly Phe Thr Gly Val Leu Pro Gln Val
145 150 155 160

Ala Leu Leu Met Gln Gly Glu Thr Asn Leu Ile Trp Ala Met Val Ser
165 170 175

Gly Ser Ile Ile Cys Phe Ile Ala Leu Ile Gly Thr Leu Gly Leu Ile
180 185 190

Leu Thr Asn Lys Asn Thr Pro Leu Pro Ala Ser
195 200

<210> 146
<211> 612
<212> DNA
<213> Chlamydia pneumoniae

<400> 146
atgacctcac cgatcccctt tcagtctagt ggcgatgcct ctttccttgc cgagcagcca 60
cagcaactcc cgtctacttc tgaatctcag ctagtaactc aattgctaac catgatgaag 120
catactcaag cattatccga aacggttctt caacaacaac gcgatcgatt accaaccgca 180
tctattatcc ttcaagtagg aggagctcct acaggaggag cgggtgcgcc ttttcaacca 240
ggaccggcag atgatcatca tcatcccata ccgccgcctg ttgtaccagc tcaaatagaa 300
acagaaatca ccactataag atccgagtta cagctcatgc gatctactct acaacaaagc 360
acaaaaggag ctcgtacagg agttctagtg gttactgcaa tcttaatgac gatctcctta 420
ttggctatta ttatcataat actagctgtg cttggattta cgggcgtctt gcctcaagta 480
gctttattga tgcagggtga aacaaatctg atttgggcta tggtgagcgg ttctattatt 540
tgctttattg cgctaattgg aactctagga ttaattttaa caaataagaa cacgcctcta 600
ccggcttctt aa 612

<210> 147
<211> 790

<212> PRT
<213> Chlamydia pneumoniae

<400> 147

```
Met Leu Ile Met Arg Asn Lys Val Ile Leu Gln Ile Ser Ile Leu Ala
1               5                   10                  15

Leu Ile Gln Thr Pro Leu Thr Leu Phe Ser Thr Glu Lys Val Lys Glu
            20                  25                  30

Gly His Val Val Val Asp Ser Ile Thr Ile Ile Thr Glu Gly Glu Asn
        35                  40                  45

Ala Ser Asn Lys His Pro Leu Pro Lys Leu Lys Thr Arg Ser Gly Ala
    50                  55                  60

Leu Phe Ser Gln Leu Asp Phe Asp Glu Asp Leu Arg Ile Leu Ala Lys
65                  70                  75                  80

Glu Tyr Asp Ser Val Glu Pro Lys Val Glu Phe Ser Glu Gly Lys Thr
                85                  90                  95

Asn Ile Ala Leu His Leu Ile Ala Lys Pro Ser Ile Arg Asn Ile His
            100                 105                 110

Ile Ser Gly Asn Gln Val Val Pro Glu His Lys Ile Leu Lys Thr Leu
        115                 120                 125

Gln Ile Tyr Arg Asn Asp Leu Phe Glu Arg Glu Lys Phe Leu Lys Gly
    130                 135                 140

Leu Asp Asp Leu Arg Thr Tyr Tyr Leu Lys Arg Gly Tyr Phe Ala Ser
145                 150                 155                 160

Ser Val Asp Tyr Ser Leu Glu His Asn Gln Glu Lys Gly His Ile Asp
                165                 170                 175

Val Leu Ile Lys Ile Asn Glu Gly Pro Cys Gly Lys Ile Lys Gln Leu
            180                 185                 190

Thr Phe Ser Gly Ile Ser Arg Ser Glu Lys Ser Asp Ile Gln Glu Phe
        195                 200                 205

Ile Gln Thr Lys Gln His Ser Thr Thr Thr Ser Trp Phe Thr Gly Ala
    210                 215                 220

Gly Leu Tyr His Pro Asp Ile Val Glu Gln Asp Ser Leu Ala Ile Thr
225                 230                 235                 240

Asn Tyr Leu His Asn Asn Gly Tyr Ala Asp Ala Ile Val Asn Ser His
                245                 250                 255

Tyr Asp Leu Asp Asp Lys Gly Asn Ile Leu Leu Tyr Met Asp Ile Asp
            260                 265                 270

Arg Gly Ser Arg Tyr Thr Leu Gly His Val His Ile Gln Gly Phe Glu
        275                 280                 285

Val Leu Pro Lys Arg Leu Ile Glu Lys Gln Ser Gln Val Gly Pro Asn
    290                 295                 300
```

Asp Leu Tyr Cys Pro Asp Lys Ile Trp Asp Gly Ala His Lys Ile Lys
305 310 315 320

Gln Thr Tyr Ala Lys Tyr Gly Tyr Ile Asn Thr Asn Val Asp Val Leu
325 330 335

Phe Ile Pro His Ala Thr Arg Pro Ile Tyr Asp Val Thr Tyr Glu Val
340 345 350

Ser Glu Gly Ser Pro Tyr Lys Val Gly Leu Ile Lys Ile Thr Gly Asn
355 360 365

Thr His Thr Lys Ser Asp Val Ile Leu His Glu Thr Ser Leu Phe Pro
370 375 380

Gly Asp Thr Phe Asn Arg Leu Lys Leu Glu Asp Thr Glu Gln Arg Leu
385 390 395 400

Arg Asn Thr Gly Tyr Phe Gln Ser Val Ser Val Tyr Thr Val Arg Ser
405 410 415

Gln Leu Asp Pro Met Gly Asn Ala Asp Gln Tyr Arg Asp Ile Phe Val
420 425 430

Glu Val Lys Glu Thr Thr Thr Gly Asn Leu Gly Leu Phe Leu Gly Phe
435 440 445

Ser Ser Leu Asp Asn Leu Phe Gly Gly Ile Glu Leu Ser Glu Ser Asn
450 455 460

Phe Asp Leu Phe Gly Ala Arg Asn Ile Phe Ser Lys Gly Phe Arg Cys
465 470 475 480

Leu Arg Gly Gly Gly Glu His Leu Phe Leu Lys Ala Asn Phe Gly Asp
485 490 495

Lys Val Thr Asp Tyr Thr Leu Lys Trp Thr Lys Pro His Phe Leu Asn
500 505 510

Thr Pro Trp Ile Leu Gly Ile Glu Leu Asp Lys Ser Ile Asn Arg Ala
515 520 525

Leu Ser Lys Asp Tyr Ala Val Gln Thr Tyr Gly Gly Asn Val Ser Thr
530 535 540

Thr Tyr Ile Leu Asn Glu His Leu Lys Tyr Gly Leu Phe Tyr Arg Gly
545 550 555 560

Ser Gln Thr Ser Leu His Glu Lys Arg Lys Phe Leu Leu Gly Pro Asn
565 570 575

Ile Asp Ser Asn Lys Gly Phe Val Ser Ala Ala Gly Val Asn Leu Asn
580 585 590

Tyr Asp Ser Val Asp Ser Pro Arg Thr Pro Thr Thr Gly Ile Arg Gly
595 600 605

Gly Val Thr Phe Glu Val Ser Gly Leu Gly Gly Thr Tyr His Phe Thr
610 615 620

Lys Leu Ser Leu Asn Ser Ser Ile Tyr Arg Lys Leu Thr Arg Lys Gly

625 630 635 640

Ile Leu Lys Ile Lys Gly Glu Ala Gln Phe Ile Lys Pro Tyr Ser Asn
645 650 655

Thr Thr Ala Glu Gly Val Pro Val Ser Glu Arg Phe Phe Leu Gly Gly
660 665 670

Glu Thr Thr Val Arg Gly Tyr Lys Ser Phe Ile Ile Gly Pro Lys Tyr
675 680 685

Ser Ala Thr Glu Pro Gln Gly Gly Leu Ser Ser Leu Leu Ile Ser Glu
690 695 700

Glu Phe Gln Tyr Pro Leu Ile Arg Gln Pro Asn Ile Ser Ala Phe Val
705 710 715 720

Phe Leu Asp Ser Gly Phe Val Gly Leu Gln Glu Tyr Lys Ile Ser Leu
725 730 735

Lys Asp Leu Arg Ser Ser Ala Gly Phe Gly Leu Arg Phe Asp Val Met
740 745 750

Asn Asn Val Pro Val Met Leu Gly Phe Gly Trp Pro Phe Arg Pro Thr
755 760 765

Glu Thr Leu Asn Gly Glu Lys Ile Asp Val Ser Gln Arg Phe Phe Phe
770 775 780

Ala Leu Gly Gly Met Phe
785 790

<210> 148
<211> 2373
<212> DNA
<213> Chlamydia pneumoniae

<400> 148
atgctcatca tgcgaaataa agttatcttg caaatatcta ttctagcgtt aatccaaacc 60
cctttaactt tattttctac tgaaaaagtt aaagaaggcc atgtggtggt agactctatc 120
acaatcataa cggaaggaga aaatgcttca aataaacatc ccttacccaa attaaagacc 180
agaagtgggg ctcttttttc tcaattagat tttgatgaag acttgagaat tctagctaaa 240
gaatacgact ctgttgagcc taaagtagaa ttttctgaag ggaaaactaa catagccctt 300
cacctaatag ctaaaccctc aattcgaaat attcatatct caggaaatca agtcgttcct 360
gaacataaaa ttcttaaaac cctacaaatt taccgtaatg atctctttga acgagaaaaa 420
tttcttaagg gtcttgatga tctaagaacg tattatctca agcgaggata tttcgcatcc 480
agtgtagact acagtctgga acacaatcaa gaaaaaggtc acatcgatgt tttaattaaa 540
atcaatgaag gtccttgcgg gaaaattaaa cagcttacgt tctcaggaat ctctcgatca 600
gaaaaatcag atatccaaga atttattcaa accaagcagc actctacaac tacaagttgg 660
tttactggag ctggactcta tcacccagat attgttgaac aagatagctt ggcaattacg 720
aattacctac ataataacgg gtacgctgat gctatagtca actctcacta tgaccttgac 780
gacaaaggga atattcttct ttacatggat attgatcgag ggtcgcgata taccttagga 840
cacgtccata tccaagggtt tgaggttttg ccaaaacgcc ttatagaaaa gcaatcccaa 900
gtcggcccca atgatcttta ttgccccgat aaaatatggg atggggctca taagatcaaa 960
caaacttatg caaagtatgg ctacatcaat accaatgtag acgttctctt catccctcac 1020
gcaacccgcc ctatttatga tgtaacttat gaggtaagtg aagggtctcc ttataaagtt 1080
gggttaatta aaattactgg gaatacccat acaaaatctg acgttatttt acacgaaacc 1140
agtctcttcc caggagatac attcaatcgc ttaaagctag aagatactga gcaacgttta 1200
agaaatacag gctacttcca aagcgttagt gtctatacag ttcgttctca acttgatcct 1260
atgggcaatg cggatcaata ccgagatatt tttgtagaag tcaaagaaac aacaacagga 1320
aacttaggct tattcttagg atttagttct cttgacaatc tttttggagg aattgaacta 1380

```
tctgaaagta attttgatct atttggagct agaaatatat tttctaaagg ttttcgttgt   1440
ctaagaggcg gtggagaaca tctattctta aaagccaact tcggggacaa agtcacagac   1500
tatactttga agtggaccaa acctcatttt ctaaacactc cttggatttt aggaattgaa   1560
ttagataaat caattaacag agcattatct aaagattatg ctgtccaaac ctatggcggg   1620
aacgtcagca caacgtatat cttgaacgaa cacctgaaat acggtctatt ttatcgagga   1680
agtcaaacga gtttacatga aaaacgtaag ttcctcctag ggccaaatat agacagcaat   1740
aaaggatttg tctctgctgc aggtgtcaac ttgaattacg attctgtaga tagtcctaga   1800
actccaacta cagggattcg cggggggtg actttgagg tttctggttt gggaggaact   1860
tatcatttta caaaactctc tttaaacagc tctatctata gaaaacttac gcgtaaaggt   1920
attttgaaaa tcaaagggga agctcaattt attaaaccct atagcaatac tacagctgaa   1980
ggagttcctg tcagtgagcg cttcttccta ggtggagaga ctacagttcg gggatataaa   2040
tcctttatta tcggtccaaa atactctgct acagaacctc agggaggact ctcttcgctc   2100
cttatttcag aagagtttca ataccctctc atcagacaac ctaatattag tgcctttgta   2160
ttcttagact caggttttgt cggtttacaa gagtataaga tttcgttaaa agatctacgt   2220
agtagtgctg gatttggtct gcgcttcgat gtaatgaata atgttcctgt tatgttagga   2280
tttggttggc ccttccgtcc aaccgagact ttgaatggag aaaaaattga tgtatctcag   2340
cgattcttct ttgctttagg gggcatgttc taa                                2373
```

<210> 149
<211> 353
<212> PRT
<213> Chlamydia pneumoniae

<400> 149

```
Met Asn Lys Arg Gln Lys Asp Lys Leu Lys Ile Cys Val Ile Ile Ser
1               5                   10                  15

Thr Leu Ile Leu Val Gly Ile Phe Ala Arg Ala Pro Arg Gly Asp Thr
            20                  25                  30

Phe Lys Thr Phe Leu Lys Ser Glu Glu Ala Ile Ile Tyr Ser Asn Gln
        35                  40                  45

Cys Asn Glu Asp Met Arg Lys Ile Leu Cys Asp Ala Ile Glu His Ala
    50                  55                  60

Asp Glu Glu Ile Phe Leu Arg Ile Tyr Asn Leu Ser Glu Pro Lys Ile
65                  70                  75                  80

Gln Gln Ser Leu Thr Arg Gln Ala Gln Ala Lys Asn Lys Val Thr Ile
                85                  90                  95

Tyr Tyr Gln Lys Phe Lys Ile Pro Gln Ile Leu Lys Gln Ala Ser Asn
            100                 105                 110

Val Thr Leu Val Glu Gln Pro Pro Ala Gly Arg Lys Leu Met His Gln
        115                 120                 125

Lys Ala Leu Ser Ile Asp Lys Lys Asp Ala Trp Leu Gly Ser Ala Asn
    130                 135                 140

Tyr Thr Asn Leu Ser Leu Arg Leu Asp Asn Asn Leu Ile Leu Gly Met
145                 150                 155                 160

His Ser Ser Glu Leu Cys Asp Leu Ile Ile Thr Asn Thr Ser Gly Asp
                165                 170                 175

Phe Ser Ile Lys Asp Gln Thr Gly Lys Tyr Phe Val Leu Pro Gln Asp
            180                 185                 190

Arg Lys Ile Ala Ile Gln Ala Val Leu Glu Lys Ile Gln Thr Ala Gln
```

```
        195                 200                 205

Lys Thr Ile Gln Val Ala Met Phe Ala Leu Thr His Ser Glu Ile Ile
    210                 215                 220

Gln Ala Leu His Gln Ala Lys Gln Arg Gly Ile His Val Asp Ile Ile
225                 230                 235                 240

Ile Asp Arg Ser His Ser Lys Leu Thr Phe Lys Gln Leu Arg Gln Leu
                245                 250                 255

Asn Ile Asn Lys Asp Phe Val Ser Ile Asn Thr Ala Pro Cys Thr Leu
            260                 265                 270

His His Lys Phe Ala Val Ile Asp Asn Lys Thr Leu Leu Ala Gly Ser
        275                 280                 285

Ile Asn Trp Ser Lys Gly Arg Phe Ser Leu Asn Asp Glu Ser Leu Ile
    290                 295                 300

Ile Leu Glu Asn Leu Thr Lys Gln Gln Asn Gln Lys Leu Arg Met Ile
305                 310                 315                 320

Trp Lys Asp Leu Ala Lys His Ser Glu His Pro Thr Val Asp Asp Glu
                325                 330                 335

Glu Lys Glu Ile Ile Glu Lys Ser Leu Pro Val Glu Glu Gln Glu Ala
            340                 345                 350

Ala


<210>  150
<211>  1062
<212>  DNA
<213>  Chlamydia pneumoniae

<400>  150
atgaataaaa gacaaaaaga taaattaaaa atctgtgtta ttattagcac gttgatttta    60
gtaggaattt ttgcaagagc tcctcgtggt gacactttta agactttttt aaagtctgaa   120
gaagctatca tctactcaaa tcaatgcaat gaggacatgc gtaaaattct atgcgatgct   180
atagaacacg ctgatgaaga gatcttccta cgtatttata acctctcaga acccaagatc   240
caacagagtt taactcgaca agctcaagca aaaaacaaag ttacgatcta ctatcaaaaa   300
tttaaaattc cccaaatctt aaagcaagcc agcaatgtaa ctttagtcga gcaacctcca   360
gcagggcgta aactgatgca tcaaaaagct ctttccatag ataagaaaga tgcttggcta   420
ggatctgcga actacaccaa tctttctcta cgtttagata ataatctcat tctaggaatg   480
catagctcgg agctctgtga tctcattatc acaaatacct ctggagactt ttctataaag   540
gatcaaacag gaaagtattt tgttcttcct caagatcgta aaattgcaat acaagctgta   600
ctcgaaaaaa tccagacagc tcagaaaacc atccaagttg ctatgtttgc tctgacccac   660
tcggagatta ttcaagcctt acatcaagca aaacaacgag gaatccatgt agatattatc   720
attgatagaa gtcatagcaa acttactttt aagcaattac gacaattaaa tatcaataaa   780
gactttgttt ctataaatac cgcaccctgt actcttcacc ataagtttgc agttatagat   840
aataaaactc tacttgcagg atctataaat tggtctaaag gaagattctc cttaaatgat   900
gaaagcttga tcatactgga aaacctgacc aaacaacaaa atcagaaact tcgaatgatt   960
tggaaagatc tagctaagca ttcagaacat cctacagtag acgatgaaga aaaagaaatt  1020
atagaaaaaa gtcttccagt agaagagcaa gaagcagcgt ga                     1062

<210>  151
<211>  327
<212>  PRT
<213>  Chlamydia pneumoniae
```

<400> 151

```
Met Asp Ala Lys Met Gly Tyr Ile Phe Lys Val Met Arg Trp Ile Phe
1               5                   10                  15

Cys Phe Val Ala Cys Gly Ile Thr Phe Gly Cys Thr Asn Ser Gly Phe
            20                  25                  30

Gln Asn Ala Asn Ser Arg Pro Cys Ile Leu Ser Met Asn Arg Met Ile
        35                  40                  45

His Asp Cys Val Glu Arg Val Val Gly Asn Arg Leu Ala Thr Ala Val
    50                  55                  60

Leu Ile Lys Gly Ser Leu Asp Pro His Ala Tyr Glu Met Val Lys Gly
65                  70                  75                  80

Asp Lys Asp Lys Ile Ala Gly Ser Ala Val Ile Phe Cys Asn Gly Leu
                85                  90                  95

Gly Leu Glu His Thr Leu Ser Leu Arg Lys His Leu Glu Asn Asn Pro
            100                 105                 110

Asn Ser Val Lys Leu Gly Glu Arg Leu Ile Ala Arg Gly Ala Phe Val
        115                 120                 125

Pro Leu Glu Glu Asp Gly Ile Cys Asp Pro His Ile Trp Met Asp Leu
    130                 135                 140

Ser Ile Trp Lys Glu Ala Val Ile Glu Ile Thr Glu Val Leu Ile Glu
145                 150                 155                 160

Lys Phe Pro Glu Trp Ser Ala Glu Phe Lys Ala Asn Ser Glu Glu Leu
                165                 170                 175

Val Cys Glu Met Ser Ile Leu Asp Ser Trp Ala Lys Gln Cys Leu Ser
            180                 185                 190

Thr Ile Pro Glu Asn Leu Arg Tyr Leu Val Ser Gly His Asn Ala Phe
        195                 200                 205

Ser Tyr Phe Thr Arg Arg Tyr Leu Ala Thr Pro Glu Glu Val Ala Ser
    210                 215                 220

Gly Ala Trp Arg Ser Arg Cys Ile Ser Pro Glu Gly Leu Ser Pro Glu
225                 230                 235                 240

Ala Gln Ile Ser Val Arg Asp Ile Met Ala Val Val Asp Tyr Ile Asn
                245                 250                 255

Glu His Asp Val Ser Val Val Phe Pro Glu Asp Thr Leu Asn Gln Asp
            260                 265                 270

Ala Leu Lys Lys Ile Val Ser Ser Leu Lys Lys Ser His Leu Val Arg
        275                 280                 285

Leu Ala Gln Lys Pro Leu Tyr Ser Asp Asn Val Asp Asp Asn Tyr Phe
    290                 295                 300

Ser Thr Phe Lys His Asn Val Cys Leu Ile Thr Glu Glu Leu Gly Gly
305                 310                 315                 320
```

```
Val Ala Leu Glu Cys Gln Arg
                325

<210>   152
<211>   984
<212>   DNA
<213>   Chlamydia pneumoniae

<400>   152
atggatgcga aaatgggata tatatttaaa gtgatgcgtt ggattttctg tttcgtggca    60
tgtggtataa cttttggatg taccaattct gggtttcaga atgcaaattc acgtccttgt   120
atactatcca tgaatcgcat gattcatgat tgtgttgaaa gagtcgtggg gaataggctt   180
gctaccgctg ttttgatcaa aggatcctta gaccctcatg cgtatgagat ggttaaaggg   240
gataaggaca agattgctgg aagtgccgta atttttgta acggcctggg tcttgagcat    300
acattaagtt tgcggaagca tttagaaaat aatcccaata gtgtcaagtt aggggagcgg   360
ttgatagcgc gtggggcctt tgttcctcta gaagaagacg gtatttgcga tcctcatatc   420
tggatggatc tttctatttg gaaggaagct gtcatagaaa ttacagaagt tctcattgaa   480
aagttccctg aatggtctgc tgaatttaaa gcaaatagtg aggaacttgt ttgtgaaatg   540
tctattttag attcttgggc gaaacaatgc ttgagcacaa ttcctgaaaa tttacggtat   600
cttgtctcag gtcataatgc gttcagttac tttacacgtc gctatttagc tactcctgaa   660
gaagtggctt ccggagcatg gaggtctcgt tgtatttctc ctgagggtct atctccagaa   720
gctcaaatca gtgttcgtga tattatggcg gttgtagatt atattaatga gcatgatgtc   780
agtgtggttt tccctgagga tactctgaac caagatgcgt tgaaaaaaat tgtttcttct   840
ctgaagaaaa gtcatttagt tcgtctagct caaaaaccat tgtatagtga taatgtggac   900
gacaattatt ttagcacctt taaacataat gtctgcctta tcacagaaga attaggaggg   960
gtggctcttg aatgtcaaag atga                                          984

<210>   153
<211>   936
<212>   PRT
<213>   Chlamydia pneumoniae

<400>   153
Met Lys Ser Ser Val Ser Trp Leu Phe Phe Ser Ser Ile Pro Leu Phe
1               5                   10                  15

Ser Ser Leu Ser Ile Val Ala Ala Glu Val Thr Leu Asp Ser Ser Asn
            20                  25                  30

Asn Ser Tyr Asp Gly Ser Asn Gly Thr Thr Phe Thr Val Phe Ser Thr
        35                  40                  45

Thr Asp Ala Ala Ala Gly Thr Thr Tyr Ser Leu Leu Ser Asp Val Ser
    50                  55                  60

Phe Gln Asn Ala Gly Ala Leu Gly Ile Pro Leu Ala Ser Gly Cys Phe
65                  70                  75                  80

Leu Glu Ala Gly Gly Asp Leu Thr Phe Gln Gly Asn Gln His Ala Leu
                85                  90                  95

Lys Phe Ala Phe Ile Asn Ala Gly Ser Ser Ala Gly Thr Val Ala Ser
            100                 105                 110

Thr Ser Ala Ala Asp Lys Asn Leu Leu Phe Asn Asp Phe Ser Arg Leu
        115                 120                 125

Ser Ile Ile Ser Cys Pro Ser Leu Leu Leu Ser Pro Thr Gly Gln Cys
    130                 135                 140
```

Ala Leu Lys Ser Val Gly Asn Leu Ser Leu Thr Gly Asn Ser Gln Ile
145 150 155 160

Ile Phe Thr Gln Asn Phe Ser Ser Asp Asn Gly Gly Val Ile Asn Thr
165 170 175

Lys Asn Phe Leu Leu Ser Gly Thr Ser Gln Phe Ala Ser Phe Ser Arg
180 185 190

Asn Gln Ala Phe Thr Gly Lys Gln Gly Gly Val Val Tyr Ala Thr Gly
195 200 205

Thr Ile Thr Ile Glu Asn Ser Pro Gly Ile Val Ser Phe Ser Gln Asn
210 215 220

Leu Ala Lys Gly Ser Gly Gly Ala Leu Tyr Ser Thr Asp Asn Cys Ser
225 230 235 240

Ile Thr Asp Asn Phe Gln Val Ile Phe Asp Gly Asn Ser Ala Trp Glu
245 250 255

Ala Ala Gln Ala Gln Gly Gly Ala Ile Cys Cys Thr Thr Thr Asp Lys
260 265 270

Thr Val Thr Leu Thr Gly Asn Lys Asn Leu Ser Phe Thr Asn Asn Thr
275 280 285

Ala Leu Thr Tyr Gly Gly Ala Ile Ser Gly Leu Lys Val Ser Ile Ser
290 295 300

Ala Gly Gly Pro Thr Leu Phe Gln Ser Asn Ile Ser Gly Ser Ser Ala
305 310 315 320

Gly Gln Gly Gly Gly Gly Ala Ile Asn Ile Ala Ser Ala Gly Glu Leu
325 330 335

Ala Leu Ser Ala Thr Ser Gly Asp Ile Thr Phe Asn Asn Asn Gln Val
340 345 350

Thr Asn Gly Ser Thr Ser Thr Arg Asn Ala Ile Asn Ile Ile Asp Thr
355 360 365

Ala Lys Val Thr Ser Ile Arg Ala Ala Thr Gly Gln Ser Ile Tyr Phe
370 375 380

Tyr Asp Pro Ile Thr Asn Pro Gly Thr Ala Ala Ser Thr Asp Thr Leu
385 390 395 400

Asn Leu Asn Leu Ala Asp Ala Asn Ser Glu Ile Glu Tyr Gly Gly Ala
405 410 415

Ile Val Phe Ser Gly Glu Lys Leu Ser Pro Thr Glu Lys Ala Ile Ala
420 425 430

Ala Asn Val Thr Ser Thr Ile Arg Gln Pro Ala Val Leu Ala Arg Gly
435 440 445

Asp Leu Val Leu Arg Asp Gly Val Thr Val Thr Phe Lys Asp Leu Thr
450 455 460

Gln Ser Pro Gly Ser Arg Ile Leu Met Asp Gly Gly Thr Thr Leu Ser

465 470 475 480

Ala Lys Glu Ala Asn Leu Ser Leu Asn Gly Leu Ala Val Asn Leu Ser
485 490 495

Ser Leu Asp Gly Thr Asn Lys Ala Ala Leu Lys Thr Glu Ala Ala Asp
500 505 510

Lys Asn Ile Ser Leu Ser Gly Thr Ile Ala Leu Ile Asp Thr Glu Gly
515 520 525

Ser Phe Tyr Glu Asn His Asn Leu Lys Ser Ala Ser Thr Tyr Pro Leu
530 535 540

Leu Glu Leu Thr Thr Ala Gly Ala Asn Gly Thr Ile Thr Leu Gly Ala
545 550 555 560

Leu Ser Thr Leu Thr Leu Gln Glu Pro Glu Thr His Tyr Gly Tyr Gln
565 570 575

Gly Asn Trp Gln Leu Ser Trp Ala Asn Ala Thr Ser Ser Lys Ile Gly
580 585 590

Ser Ile Asn Trp Thr Arg Thr Gly Tyr Ile Pro Ser Pro Glu Arg Lys
595 600 605

Ser Asn Leu Pro Leu Asn Ser Leu Trp Gly Asn Phe Ile Asp Ile Arg
610 615 620

Ser Ile Asn Gln Leu Ile Glu Thr Lys Ser Ser Gly Glu Pro Phe Glu
625 630 635 640

Arg Glu Leu Trp Leu Ser Gly Ile Ala Asn Phe Phe Tyr Arg Asp Ser
645 650 655

Met Pro Thr Arg His Gly Phe Arg His Ile Ser Gly Gly Tyr Ala Leu
660 665 670

Gly Ile Thr Ala Thr Thr Pro Ala Glu Asp Gln Leu Thr Phe Ala Phe
675 680 685

Cys Gln Leu Phe Ala Arg Asp Arg Asn His Ile Thr Gly Lys Asn His
690 695 700

Gly Asp Thr Tyr Gly Ala Ser Leu Tyr Phe His His Thr Glu Gly Leu
705 710 715 720

Phe Asp Ile Ala Asn Phe Leu Trp Gly Lys Ala Thr Arg Ala Pro Trp
725 730 735

Val Leu Ser Glu Ile Ser Gln Ile Ile Pro Leu Ser Phe Asp Ala Lys
740 745 750

Phe Ser Tyr Leu His Thr Asp Asn His Met Lys Thr Tyr Tyr Thr Asp
755 760 765

Asn Ser Ile Ile Lys Gly Ser Trp Arg Asn Asp Ala Phe Cys Ala Asp
770 775 780

Leu Gly Ala Ser Leu Pro Phe Val Ile Ser Val Pro Tyr Leu Leu Lys
785 790 795 800

Glu Val Glu Pro Phe Val Lys Val Gln Tyr Ile Tyr Ala His Gln Gln
805 810 815

Asp Phe Tyr Glu Arg His Ala Glu Gly Arg Ala Phe Asn Lys Ser Glu
820 825 830

Leu Ile Asn Val Glu Ile Pro Ile Gly Val Thr Phe Glu Arg Asp Ser
835 840 845

Lys Ser Glu Lys Gly Thr Tyr Asp Leu Thr Leu Met Tyr Ile Leu Asp
850 855 860

Ala Tyr Arg Arg Asn Pro Lys Cys Gln Thr Ser Leu Ile Ala Ser Asp
865 870 875 880

Ala Asn Trp Met Ala Tyr Gly Thr Asn Leu Ala Arg Gln Gly Phe Ser
885 890 895

Val Arg Ala Ala Asn His Phe Gln Val Asn Pro His Met Glu Ile Phe
900 905 910

Gly Gln Phe Ala Phe Glu Val Arg Ser Ser Ser Arg Asn Tyr Asn Thr
915 920 925

Asn Leu Gly Ser Lys Phe Cys Phe
930 935

<210> 154
<211> 2811
<212> DNA
<213> Chlamydia pneumoniae

<400> 154
atgaagtcct ctgtctcttg gttgttcttt tcttcaatcc cgctcttttc atcgctctct 60
atagtcgcgg cagaggtgac cttagatagc agcaataata gctatgatgg atctaacgga 120
actaccttca cggtcttttc cactacggac gctgctgcag gaactaccta ttccttactt 180
tccgacgtat cctttcaaaa tgcaggggct ttaggaattc ccttagcctc aggatgcttc 240
ctagaagcgg gcggcgatct tactttccaa ggaaatcaac atgcactgaa gtttgcattt 300
atcaatgcgg gctctagcgc tggaactgta gccagtacct cagcagcaga taagaatctt 360
ctctttaatg atttttctag actctctatt atctcttgtc cctctcttct tctctctcct 420
actggacaat gtgctttaaa atctgtgggg aatctatctc taactggcaa ttcccaaatt 480
atatttactc agaacttctc gtcagataac ggcggtgtta tcaatacgaa aaacttctta 540
ttatcaggga catctcagtt tgcgagcttt tcgagaaacc aagccttcac agggaagcaa 600
ggcggtgtag tttacgctac aggaactata actatcgaga acagccctgg gatagtttcc 660
ttctctcaaa acctagcgaa aggatctggc ggtgctctgt acagcactga caactgttcg 720
attacagata actttcaagt gatctttgac ggcaatagtg cttgggaagc cgctcaagct 780
cagggcgggg ctatttgttg cactacgaca gataaaacag tgactcttac tgggaacaaa 840
aacctctctt tcacaaataa tacagcattg acatatggcg gagccatctc tggactcaag 900
gtcagtattt ccgctggagg tcctactcta tttcaaagta atatctcagg aagtagcgcc 960
ggtcagggag gaggaggagc gatcaatata gcatctgctg gggaactcgc tctctctgct 1020
acttctggag atattacctt caataacaac caagtcacca acggaagcac aagtacaaga 1080
aacgcaataa atatcattga taccgctaaa gtcacatcga tacgagctgc tacggggcaa 1140
tctatctatt tctatgatcc catcacaaat ccaggaaccg cagcttctac cgacacattg 1200
aacttaaact tagcagatgc gaacagtgag atcgagtatg ggggtgcgat tgtcttttct 1260
ggagaaaagc tttcccctac agaaaaagca atcgctgcaa acgtcacctc tactatccga 1320
caacctgcag tattagcgcg gggagatctt gtacttcgtg atggagtcac cgtaactttc 1380
aaggatctga ctcaaagtcc aggatcccgc atcttaatgg atgggggac tacacttagt 1440
gctaaagagg caaatctttc gcttaatggc ttagcagtaa atctctcctc tttagatgga 1500
accaacaagg cagctttaaa aacagaagct gcagataaaa atatcagcct atcgggaacg 1560
attgcgctta ttgacacgga agggtcattc tatgagaatc ataacttaaa aagtgctagt 1620

```
acctatcctc ttcttgaact taccaccgca ggagccaacg gaacgattac tctgggagct   1680
ctttctaccc tgactcttca agaacctgaa acccactacg ggtatcaagg aaactggcag   1740
ttgtcttggg caaatgcaac atcctcaaaa ataggaagca tcaactggac ccgtacagga   1800
tacattccta gtcctgagag aaaaagtaat ctccctctaa atagcttatg ggaaactttt   1860
atagatatac gctcgatcaa tcagcttata gaaaccaagt ccagtgggga gccttttgag   1920
cgtgagctat ggctttcagg aattgcgaat ttcttctata gagattctat gcccacccgc   1980
catggtttcc gccatatcag cggggggttat gcactaggga tcacagcaac aactcctgcc   2040
gaggatcagc ttacttttgc cttctgccag ctctttgcta gagatcgcaa tcatattaca   2100
ggtaagaacc acggagatac ttacggtgcc tctttgtatt tccaccatac agaagggctc   2160
ttcgacatcg ccaatttcct ctggggaaaa gcaacccgag ctccctgggt gctctctgag   2220
atctcccaga tcattccttt atcgttcgat gctaaattca gttatctcca tacagacaac   2280
cacatgaaga catattatac cgataactct atcatcaagg gttcttggag aaacgatgcc   2340
ttctgtgcag atcttggagc tagcctgcct tttgttattt ccgttccgta tcttctgaaa   2400
gaagtcgaac cttttgtcaa agtacagtat atctatgcgc atcagcaaga cttctacgag   2460
cgtcatgctg aaggacgcgc tttcaataaa agcgagctta tcaacgtaga gattcctata   2520
ggcgtcacct tcgaaagaga ctcaaaatca gaaaagggaa cttacgatct tactcttatg   2580
tatatactcg atgcttaccg acgcaatcct aaatgtcaaa cttccctaat agctagcgat   2640
gctaactgga tggcctatgg taccaacctc gcacgacaag gttttctgt tcgtgctgcg   2700
aaccatttcc aagtgaaccc ccacatggaa atcttcggtc aattcgcttt tgaagtacga   2760
agttcttcac gaaattataa tacaaaccta ggctctaagt tttgtttcta g            2811
```

<210> 155
<211> 648
<212> PRT
<213> Chlamydia pneumoniae

<400> 155

```
Met Phe Val Met Lys Lys Leu Val Arg Leu Cys Val Val Leu Leu Ser
1               5                   10                  15

Leu Leu Pro Asn Val Leu Phe Ser Ser Asp Leu Leu Arg Glu Glu Gly
            20                  25                  30

Ile Lys Lys Met Met Asp Lys Leu Ile Glu Tyr His Val Asp Ala Gln
        35                  40                  45

Glu Val Ser Thr Asp Ile Leu Ser Arg Ser Leu Ser Ser Tyr Ile Gln
    50                  55                  60

Ser Phe Asp Pro His Lys Ser Tyr Leu Ser Asn Gln Glu Val Ala Val
65                  70                  75                  80

Phe Leu Gln Ser Pro Glu Thr Lys Lys Arg Leu Leu Lys Asn Tyr Lys
                85                  90                  95

Ala Gly Asn Phe Ala Ile Tyr Arg Asn Ile Asn Gln Leu Ile His Glu
            100                 105                 110

Ser Ile Leu Arg Ala Arg Gln Trp Arg Asn Glu Trp Val Lys Asn Pro
        115                 120                 125

Lys Glu Leu Val Leu Glu Ala Ser Ser Tyr Gln Ile Ser Lys Gln Pro
    130                 135                 140

Met Gln Trp Ser Lys Ser Leu Asp Glu Val Lys Gln Arg Gln Arg Ala
145                 150                 155                 160

Leu Leu Leu Ser Tyr Leu Ser Leu His Leu Ala Gly Ala Ser Ser Ser
                165                 170                 175

Arg Tyr Glu Gly Lys Glu Glu Gln Leu Ala Ala Leu Cys Leu Arg Gln
```

180 185 190

Ile Glu Asn His Glu Asn Val Tyr Leu Gly Ile Asn Asp His Gly Val
195 200 205

Ala Met Asp Arg Asp Glu Glu Ala Tyr Gln Phe His Ile Arg Val Val
210 215 220

Lys Ala Leu Ala His Ser Leu Asp Ala His Thr Ala Tyr Phe Ser Lys
225 230 235 240

Asp Glu Ala Leu Ala Met Arg Ile Gln Leu Glu Lys Gly Met Cys Gly
245 250 255

Ile Gly Val Val Leu Lys Glu Asp Ile Asp Gly Val Val Val Arg Glu
260 265 270

Ile Ile Pro Gly Gly Pro Ala Ala Lys Ser Gly Asp Leu Gln Leu Gly
275 280 285

Asp Ile Ile Tyr Arg Val Asp Gly Lys Asp Ile Glu His Leu Ser Phe
290 295 300

Arg Gly Val Leu Asp Cys Leu Arg Gly Gly His Gly Ser Thr Val Val
305 310 315 320

Leu Asp Ile His Arg Gly Glu Ser Asp His Thr Ile Ala Leu Arg Arg
325 330 335

Glu Lys Ile Leu Leu Glu Asp Arg Arg Val Asp Val Ser Tyr Glu Pro
340 345 350

Tyr Gly Asp Gly Val Ile Gly Lys Val Thr Leu His Ser Phe Tyr Glu
355 360 365

Gly Glu Asn Gln Val Ser Ser Glu Gln Asp Leu Arg Arg Ala Ile Gln
370 375 380

Gly Leu Lys Glu Lys Asn Leu Leu Gly Leu Val Leu Asp Ile Arg Glu
385 390 395 400

Asn Thr Gly Gly Phe Leu Ser Gln Ala Ile Lys Val Ser Gly Leu Phe
405 410 415

Met Thr Asn Gly Val Val Val Val Ser Arg Tyr Ala Asp Gly Thr Met
420 425 430

Lys Cys Tyr Arg Thr Val Ser Pro Lys Lys Phe Tyr Asp Gly Pro Leu
435 440 445

Ala Ile Leu Val Ser Lys Ser Ser Ala Ser Ala Ala Glu Ile Val Ala
450 455 460

Gln Thr Leu Gln Asp Tyr Gly Val Ala Leu Val Val Gly Asp Glu Gln
465 470 475 480

Thr Tyr Gly Lys Gly Thr Ile Gln His Gln Thr Ile Thr Gly Asp Ala
485 490 495

Ser Gln Asp Asp Cys Phe Lys Val Thr Val Gly Lys Tyr Tyr Ser Pro
500 505 510

```
Ser Gly Lys Ser Thr Gln Leu Gln Gly Val Lys Ser Asp Ile Leu Ile
        515                 520                 525

Pro Ser Leu Tyr Ala Glu Asp Arg Leu Gly Glu Arg Phe Leu Glu His
    530                 535                 540

Pro Leu Pro Ala Asp Cys Cys Asp Asn Val Leu His Asp Pro Leu Thr
545                 550                 555                 560

Asp Leu Asp Thr Gln Thr Arg Pro Trp Phe Gln Lys Tyr Tyr Leu Pro
                565                 570                 575

Asn Leu Gln Lys Gln Glu Thr Leu Trp Arg Glu Met Leu Pro Gln Leu
            580                 585                 590

Thr Lys Asn Ser Glu Gln Arg Leu Ser Glu Asn Ser Asn Phe Gln Ala
        595                 600                 605

Phe Leu Ser Gln Ile Lys Ser Ser Glu Lys Thr Asp Leu Ser Tyr Gly
    610                 615                 620

Ser Asn Asp Leu Gln Leu Glu Glu Ser Ile Asn Ile Leu Lys Asp Met
625                 630                 635                 640

Ile Leu Leu Gln Gln Cys Arg Lys
                645

<210>   156
<211>   1947
<212>   DNA
<213>   Chlamydia pneumoniae

<400>   156
atgttcgtaa tgaaaaaact tgtccgtcta tgcgtagttc ttctttcttt acttccgaat     60
gtattatttt cttcggatct tttacgagaa gagggcatca aaaagatgat ggacaagctg    120
atcgagtatc atgtcgatgc tcaagaggtt tctacggata tactctcgcg ttctttatct    180
agttacattc aatcttttga tcctcataaa tcttatcttt caaaccaaga ggttgcagtt    240
tttctacagt ctccggaaac aaagaaacgt ctcttaaaga attataaggc aggcaacttt    300
gctatttatc gcaacatcaa tcaattaatt catgagagta ttcttcgtgc caggcagtgg    360
agaaacgaat gggttaagaa tccaaaagag cttgtattgg aggcatcctc atatcagata    420
tcgaagcaac ctatgcaatg gagcaaatct ttagacgaag tgaagcagag acaacgcgct    480
ctactccttt cctatctttc tttacatctt gctggagctt cttcctctcg ttatgagggt    540
aaagaagagc agcttgctgc tctgtgtcta cgtcaaatcg agaaccatga gaatgtatat    600
ttaggtatca acgatcatgg tgttgctatg gatcgggatg aagaagccta ccaattccat    660
atccgtgttg ttaaagcttt agctcatagc ttagatgcac atacggcgta tttcagtaag    720
gacgaagcgt tggcgatgcg aatccaacta gaaaaaggca tgtgtggaat tggtgttgtt    780
ctgaaggaag atattgatgg agttgttgtt agagaaatca ttcctggggg acctgcggct    840
aaatctgggg atcttcagct tggagatatc atctatcggg tggatggcaa ggatatcgag    900
catctttctt tccgcggtgt tttagattgt ttacgtggag gtcatggctc tactgtagtc    960
ttagatatcc atcgtgggga gagcgatcat acgatcgcct tgagaaggga gaaaatcctt   1020
ttagaagacc gtcgtgtgga tgtttcctat gagccttatg gagatggtgt gattgggaaa   1080
gttacgttac attcttttta tgaaggagaa aatcaggttt ctagtgaaca agatctacgt   1140
cgagcgattc agggattaaa ggagaagaac cttcttggat tagttttaga tatccgagaa   1200
aatacgggtg gatttttatc tcaagcgatc aaagtttctg gtttatttat gaccaatggc   1260
gttgtggttg tatctcgcta tgctgatggt accatgaagt gctaccgcac agtatctcct   1320
aaaaaattct atgatggtcc tttggctatt ttagtatcta aaagttccgc atcagcagcg   1380
gagattgtag cacaaactct ccaagattat ggagttgctt tagttgttgg agatgagcag   1440
acctatggga agggaacgat tcagcatcaa acaattactg gagatgcctc tcaggacgat   1500
tgttttaagg ttactgtagg gaaatattat tccccttctg ggaaatcgac tcaacttcag   1560
ggagtaaaat ccgatatttt aattccttct ctctatgctg aagatcgtct aggagagcgt   1620
```

```
tttctagagc atcccttacc tgcagattgc tgtgataatg tacttcacga tcctctcacg   1680
gacttggata ctcaaacacg tccttggttt caaaaatact atcttcctaa tctacaaaag   1740
caagagactc tttggagaga gatgctacct cagcttacga aaaacagtga gcaaaggctt   1800
tctgagaatt cgaattttca ggcatttttg tcgcagataa aatcatctga aaaaacggac   1860
ctatcctatg gttccaatga tttacaattg gaagagtcga taaacatttt gaaggacatg   1920
attttattac aacagtgtag aaaataa                                         1947

<210>   157
<211>   199
<212>   PRT
<213>   Chlamydia pneumoniae

<400>   157
Met Arg Leu Phe Ser Leu Gly Thr Ile Tyr Leu Phe Phe Ser Leu Ala
1               5                   10                  15

Leu Ser Ser Cys Cys Gly Tyr Ser Ile Leu Asn Ser Pro Tyr His Leu
            20                  25                  30

Ser Ser Leu Gly Lys Ser Leu Leu Gln Glu Arg Ile Phe Ile Ala Pro
        35                  40                  45

Ile Lys Glu Asp Pro His Gly Gln Leu Cys Ser Ala Leu Thr Tyr Glu
    50                  55                  60

Leu Ser Lys Arg Ser Phe Ala Ile Ser Gly Arg Ser Ser Cys Ala Gly
65                  70                  75                  80

Tyr Thr Leu Lys Val Glu Leu Leu Asn Gly Ile Asp Lys Asn Ile Gly
                85                  90                  95

Phe Thr Tyr Ala Pro Asn Lys Leu Gly Asp Lys Thr His Arg His Phe
            100                 105                 110

Ile Val Ser Asn Glu Gly Arg Leu Ser Leu Ser Ala Lys Val Gln Leu
        115                 120                 125

Ile Asn Asn Asp Thr Gln Glu Val Leu Ile Asp Gln Cys Val Ala Arg
    130                 135                 140

Glu Ser Val Asp Phe Asp Phe Glu Pro Asp Leu Gly Thr Ala Asn Ala
145                 150                 155                 160

His Glu Phe Ala Leu Gly Gln Phe Glu Met His Ser Glu Ala Ile Lys
                165                 170                 175

Ser Ala Arg Arg Ile Leu Ser Ile Arg Leu Ala Glu Thr Ile Ala Gln
            180                 185                 190

Gln Val Tyr Tyr Asp Leu Phe
        195

<210>   158
<211>   600
<212>   DNA
<213>   Chlamydia pneumoniae

<400>   158
atgagattgt tttctttagg cacgatttat cttttttttt ctctagcact ttcgtcatgc   60
tgtggttact ctattttaaa cagcccgtat cacttatcgt ctttaggtaa gtctttatta   120
caggaaagaa ttttcattgc tcccataaaa gaagatcctc atggtcagct ctgctcagct   180
```

```
ctaacttatg agcttagtaa gcgttctttt gctatctctg gaaggagttc ttgcgcaggc   240
tatactctta aagtagagct tctgaatggt attgacaaga atataggttt tacgtatgcc   300
ccaaataaac tcggagataa gactcacagg cattttatag tctctaatga aggcagacta   360
tcactatctg caaaagtaca gcttatcaat aatgacactc aagaagtcct tatagaccaa   420
tgtgttgctc gagagtctgt agactttgac tttgagcctg acttaggaac agcaaacgct   480
catgaatttg ctttaggcca atttgaaatg catagtgaag ccataaaaag tgctcgccgt   540
atactatcta tacgcctagc cgagacgatt gctcaacagg tatactatga cctttttga    600

<210>   159
<211>   337
<212>   PRT
<213>   Chlamydia pneumoniae

<400>   159
Met Lys Lys Thr Cys Cys Gln Asn Tyr Arg Ser Ile Gly Val Val Phe
1               5                   10                  15

Ser Val Val Leu Phe Val Leu Thr Thr Gln Thr Leu Phe Ala Gly His
            20                  25                  30

Phe Ile Asp Ile Gly Thr Ser Gly Leu Tyr Ser Trp Ala Arg Gly Val
        35                  40                  45

Ser Gly Asp Gly Arg Val Val Val Gly Tyr Glu Gly Gly Asn Ala Phe
    50                  55                  60

Lys Tyr Val Asp Gly Glu Lys Phe Leu Leu Glu Gly Leu Val Pro Arg
65                  70                  75                  80

Ser Glu Ala Leu Val Phe Lys Ala Ser Tyr Asp Gly Ser Val Ile Ile
                85                  90                  95

Gly Ile Ser Asp Gln Asp Pro Ser Cys Arg Ala Val Lys Trp Val Asn
            100                 105                 110

Gly Ala Leu Val Asp Leu Gly Ile Phe Ser Glu Gly Met Gln Ser Phe
        115                 120                 125

Ala Glu Gly Val Ser Ser Asp Gly Lys Thr Ile Val Gly Cys Leu Tyr
    130                 135                 140

Ser Asp Asp Thr Glu Thr Asn Phe Ala Val Lys Trp Asp Glu Thr Gly
145                 150                 155                 160

Met Val Val Leu Pro Asn Leu Pro Glu Asp Arg His Ser Cys Ala Trp
                165                 170                 175

Asp Ala Ser Glu Asp Gly Ser Val Ile Val Gly Asp Ala Met Gly Ser
            180                 185                 190

Glu Glu Ile Ala Lys Ala Val Tyr Trp Lys Asp Gly Glu Gln His Leu
        195                 200                 205

Leu Ser Asn Ile Pro Gly Ala Lys Arg Ser Ser Ala His Ala Val Ser
    210                 215                 220

Lys Asp Gly Ser Phe Ile Val Gly Glu Phe Ile Ser Glu Glu Asn Glu
225                 230                 235                 240

Val His Ala Phe Val Tyr His Asn Gly Val Ile Lys Asp Ile Gly Thr
                245                 250                 255
```

Leu Gly Gly Asp Tyr Ser Val Ala Thr Gly Val Ser Arg Asp Gly Lys
260 265 270

Val Ile Val Gly His Ser Thr Arg Thr Asp Gly Glu Tyr Arg Ala Phe
275 280 285

Lys Tyr Val Asp Gly Arg Met Ile Asp Leu Gly Thr Leu Gly Gly Ser
290 295 300

Ala Ser Phe Ala Phe Gly Val Ser Asp Asp Gly Lys Thr Ile Val Gly
305 310 315 320

Lys Phe Glu Thr Glu Leu Gly Glu Cys His Ala Phe Ile Tyr Leu Asp
325 330 335

Asp

<210> 160
<211> 1014
<212> DNA
<213> Chlamydia pneumoniae

<400> 160
atgaaaaaga catgttgcca aaattacaga tcgataggcg ttgtgttctc tgtggtactt 60
ttcgttctta caacacagac gctgtttgca ggacatttta ttgatattgg aacttctgga 120
ttatattctt gggctcgagg tgtatctgga gatggccgcg ttgtcgtagg ttatgaaggt 180
ggcaatgcat ttaaatatgt tgatggtgag aaatttctgt tagaaggttt ggtcccgaga 240
tccgaggcct tggtatttaa agcttcttat gatggctctg taattatagg aatctcggat 300
caagatccgt cttgccgcgc tgtgaagtgg gtaaacggtg cacttgttga tcttggaata 360
ttttctgagg gaatgcaatc ttttgcagag ggtgtttcca gtgatggaaa gacgattgta 420
gggtgcctat atagtgatga tacagagaca aactttgctg tgaagtggga tgaaacagga 480
atggttgttc tccctaactt accagaagat cgacattctt gcgcttggga tgcctctgaa 540
gatggctctg tgattgtagg ggacgccatg ggtagcgagg aaattgccaa ggcagtgtac 600
tggaaggacg gtgaacaaca tctgctttct aatatcccag gagctaaaag atcgtcagca 660
catgcagttt ctaaagatgg atcttttatc gtaggcgagt tcatcagtga agaaaatgaa 720
gttcatgcct ttgtttatca caacggtgtt atcaaagata tcgggacttt aggaggagat 780
tactctgtag caactggagt ttctagggat ggtaaggtca tcgtgggtca ttctacaaga 840
acagatggtg aataccgtgc atttaaatat gtggatggaa gaatgataga tttggggact 900
ttaggaggtt cagcatcttt tgcttttggt gtttctgacg atggcaaaac aatcgtagga 960
aaatttgaaa cagagctagg agaatgtcat gcctttatct accttgatga ttag 1014

<210> 161
<211> 349
<212> PRT
<213> Chlamydia pneumoniae

<400> 161
Met Ala Ala Ile Lys Gln Ile Leu Arg Ser Met Leu Ser Gln Ser Ser
1 5 10 15

Leu Trp Met Val Leu Phe Ser Leu Tyr Ser Leu Ser Gly Tyr Cys Tyr
20 25 30

Val Ile Thr Asp Lys Pro Glu Asp Asp Phe His Ser Ser Ser Ala Val
35 40 45

Lys Trp Asp His Trp Gly Lys Thr Thr Leu Ser Arg Leu Ser Asn Lys
50 55 60

Lys Ala Ser Ala Lys Ala Val Ser Gly Thr Gly Ala Thr Thr Val Gly
65 70 75 80

Phe Ile Lys Asp Thr Trp Ser Arg Thr Tyr Ala Val Arg Trp Asn Tyr
85 90 95

Trp Gly Thr Lys Glu Leu Pro Thr Ser Ser Trp Val Lys Lys Ser Lys
100 105 110

Ala Thr Gly Ile Ser Ser Asp Gly Ser Ile Ile Ala Gly Ile Val Glu
115 120 125

Asn Glu Leu Ser Gln Ser Phe Ala Val Thr Trp Lys Asn Asn Glu Met
130 135 140

Tyr Leu Leu Pro Ser Thr Trp Ala Val Gln Ser Lys Ala Tyr Gly Ile
145 150 155 160

Ser Ser Asp Gly Ser Val Ile Val Gly Ser Ala Lys Asp Ala Trp Ser
165 170 175

Arg Thr Phe Ala Val Lys Trp Thr Gly His Glu Ala Gln Val Leu Pro
180 185 190

Val Gly Trp Ala Val Lys Ser Val Ala Asn Ser Val Ser Ala Asn Gly
195 200 205

Ser Ile Ile Val Gly Ser Val Gln Asp Ala Ser Gly Ile Leu Tyr Ala
210 215 220

Val Lys Trp Glu Gly Asn Thr Ile Thr His Leu Gly Thr Leu Gly Gly
225 230 235 240

Tyr Ser Ala Ile Ala Lys Ala Val Ser Asn Asn Gly Lys Val Ile Val
245 250 255

Gly Arg Ser Glu Thr Tyr Tyr Gly Glu Val His Ala Phe Cys His Lys
260 265 270

Asn Gly Val Met Ser Asp Leu Gly Thr Leu Gly Gly Ser Tyr Ser Ala
275 280 285

Ala Lys Gly Val Ser Ala Thr Gly Lys Val Ile Val Gly Met Ser Thr
290 295 300

Thr Ala Asn Gly Lys Leu His Ala Phe Lys Tyr Val Gly Gly Arg Met
305 310 315 320

Ile Asp Leu Gly Glu Tyr Ser Trp Lys Glu Ala Cys Ala Asn Ala Val
325 330 335

Ser Ile Asp Gly Glu Ile Ile Val Gly Val Gln Ser Glu
340 345

<210> 162
<211> 1050
<212> DNA
<213> Chlamydia pneumoniae

<400> 162
atggcagcta taaaacaaat tttacgttct atgctatctc agagtagctt atggatggtc 60

```
ctattttcat tatattctct atctggttat tgctatgtaa ttacagacaa accagaagat    120
gacttccatt cttcatccgc agtaaaatgg gatcattggg gaaagacaac tctctcaaga    180
ttatcaaata aaaaagcctc tgcaaaagct gtttcaggaa ctggtgctac aactgtcggc    240
tttataaaag acacttggtc tcgaacatac gcagtaagat ggaattattg ggggaccaaa    300
gaactcccta ccagctcatg ggtaaaaaaa tcaaaagcaa caggaatctc ctctgatggg    360
tctataatcg cggggattgt cgagaatgag ctttctcaaa gtttcgcagt cacatggaaa    420
aacaatgaaa tgtatttgct cccttccaca tgggcagtgc aatctaaagc gtatggaatt    480
tcttctgatg gctctgttat tgtagggagt gctaaggatg cttggtcgcg aactttcgct    540
gtgaagtgga cgggacacga ggctcaggtg ttaccagtag gctgggctgt caaatctgta    600
gcgaattctg tatctgccaa tggatctata attgtagggt ctgtacaaga cgcctctgga    660
attctttatg ctgtaaagtg ggaagggaac actattacac atctaggaac tttaggaggc    720
tattctgcca ttgcaaaagc tgtatccaat aatggcaagg tcattgtagg gagatccgaa    780
acatattatg gagaggtcca tgctttctgt cataagaatg gcgtcatgtc agacctcggc    840
accctcggag gatcttattc tgcagctaag ggagtctctg caactggaaa agttattgtc    900
ggtatgtcca caacagcaaa tgggaaattg catgccttta aatatgtcgg tggaagaatg    960
atcgacttag gagagtatag ctggaaagaa gcctgtgcaa acgctgtttc tattgatgga   1020
gaaattattg ttggagtcca atcagaataa                                     1050
```

```
<210>  163
<211>  754
<212>  PRT
<213>  Chlamydia pneumoniae

<400>  163
Met Val Phe Phe Arg Asn Ser Leu Leu His Leu Val Ala Leu Ser Gly
1               5                   10                  15

Met Leu Cys Cys Ser Ser Gly Val Ala Leu Thr Ile Ala Glu Lys Met
            20                  25                  30

Ala Ser Leu Glu His Ser Gly Arg Gly Ala Asp Asp Tyr Glu Gly Met
        35                  40                  45

Ala Ser Phe Asn Ala Asn Met Arg Glu Tyr Ser Leu Gln Leu Ser Lys
    50                  55                  60

Leu Tyr Glu Glu Ala Arg Lys Leu Arg Ala Ser Gly Thr Glu Asp Glu
65                  70                  75                  80

Ala Leu Trp Lys Asp Leu Ile Arg Arg Ile Gly Glu Val Arg Gly Tyr
                85                  90                  95

Leu Arg Glu Ile Glu Glu Leu Trp Ala Ala Glu Ile Arg Glu Lys Gly
            100                 105                 110

Gly Asn Leu Glu Asp Tyr Ala Leu Trp Asn His Pro Glu Thr Thr Ile
        115                 120                 125

Tyr Asn Leu Val Thr Asp Tyr Gly Thr Glu Asp Ser Ile Tyr Leu Ile
    130                 135                 140

Pro Gln Glu Ile Gly Ala Ile Lys Ile Ala Thr Leu Ser Lys Phe Val
145                 150                 155                 160

Val Pro Lys Glu Ser Phe Glu Asp Cys Leu Thr Gln Ile Leu Ser Arg
                165                 170                 175

Leu Gly Ile Gly Val Arg Gln Val Asn Ser Trp Ile Lys Glu Leu Tyr
            180                 185                 190

Met Met Arg Lys Glu Gly Cys Ser Val Ala Gly Val Phe Ser Ser Arg
```

```
        195                 200                 205

Lys Asp Leu Glu Ala Leu Pro Glu Thr Ala Tyr Ile Gly Phe Val Leu
    210                 215                 220

Asn Ser Asn Val Asp Ala His Thr Asn Gln His Val Leu Lys Lys Phe
225                 230                 235                 240

Ile Asn Pro Glu Thr Thr His Val Asp Val Ile Ala Gly Arg Val Trp
                245                 250                 255

Ile Phe Gly Ser Ala Gly Glu Val Gly Glu Leu Leu Lys Ile Tyr Asn
            260                 265                 270

Phe Val Gln Ser Glu Ser Ile Arg Gln Glu Tyr Arg Val Ile Pro Leu
        275                 280                 285

Thr Lys Ile Asp Pro Gly Glu Met Ile Ser Ile Leu Asn Ala Ala Phe
    290                 295                 300

Arg Glu Asp Leu Thr Lys Asp Val Ser Glu Glu Ser Leu Gly Leu Arg
305                 310                 315                 320

Val Val Pro Leu Gln Tyr Gln Gly Arg Ser Leu Phe Leu Ser Gly Thr
                325                 330                 335

Ala Ala Leu Val Gln Gln Ala Leu Thr Leu Ile Arg Glu Leu Glu Glu
            340                 345                 350

Gly Ile Glu Asn Pro Thr Asp Lys Thr Val Phe Trp Tyr Asn Val Lys
        355                 360                 365

His Ser Asp Pro Gln Glu Leu Ala Ala Leu Leu Ser Gln Val His Asp
    370                 375                 380

Val Phe Ser Gly Glu Asn Lys Ala Ser Val Gly Ala Ala Asp Gly Cys
385                 390                 395                 400

Gly Ser Gln Leu Asn Ala Ser Ile Gln Ile Asp Thr Thr Val Ser Ser
                405                 410                 415

Ser Ala Lys Asp Gly Ser Val Lys Tyr Gly Asn Phe Ile Ala Asp Ser
            420                 425                 430

Lys Thr Gly Thr Leu Ile Met Val Val Glu Lys Glu Val Leu Pro Arg
        435                 440                 445

Ile Gln Met Leu Leu Lys Lys Leu Asp Val Pro Lys Lys Met Val Arg
    450                 455                 460

Ile Glu Val Leu Leu Phe Glu Arg Lys Leu Ala His Glu Gln Lys Ser
465                 470                 475                 480

Gly Leu Asn Leu Leu Arg Leu Gly Glu Glu Val Cys Lys Lys Gly Cys
                485                 490                 495

Ser Pro Ser Val Ser Trp Ala Gly Gly Thr Gly Ile Leu Glu Phe Leu
            500                 505                 510

Phe Lys Gly Ser Thr Gly Ser Ser Ile Val Pro Gly Tyr Asp Leu Ala
        515                 520                 525
```

```
Tyr Gln Phe Leu Met Ala Gln Glu Asp Val Arg Ile Asn Ala Ser Pro
    530                 535                 540

Ser Val Val Thr Met Asn Gln Thr Pro Ala Arg Ile Ala Val Val Asp
545                 550                 555                 560

Glu Met Ser Ile Ala Val Ser Ser Asp Lys Asp Lys Ala Gln Tyr Asn
                565                 570                 575

Arg Ala Gln Tyr Gly Ile Met Ile Lys Met Leu Pro Val Ile Asn Val
            580                 585                 590

Gly Glu Glu Asp Gly Lys Ser Tyr Ile Thr Leu Glu Thr Asp Ile Thr
        595                 600                 605

Phe Asp Thr Thr Gly Lys Asn His Asp Asp Arg Pro Asp Val Thr Arg
    610                 615                 620

Arg Asn Ile Thr Asn Lys Val Arg Ile Ala Asp Gly Glu Thr Val Ile
625                 630                 635                 640

Ile Gly Gly Leu Arg Cys Lys Gln Met Ser Asp Ser His Asp Gly Ile
                645                 650                 655

Pro Phe Leu Gly Asp Ile Pro Gly Ile Gly Lys Leu Phe Gly Met Ser
            660                 665                 670

Ser Thr Ser Asp Ser Leu Thr Glu Met Phe Val Phe Ile Thr Pro Lys
        675                 680                 685

Ile Leu Glu Asn Pro Val Glu Gln Gln Glu Arg Lys Glu Glu Ala Leu
    690                 695                 700

Leu Ser Ser Arg Pro Gly Glu Arg Glu Glu Tyr Tyr Gln Ala Leu Ala
705                 710                 715                 720

Ala Ser Glu Ala Ala Ala Arg Ala Ala His Lys Lys Leu Glu Met Phe
                725                 730                 735

Pro Ala Ser Gly Val Ser Leu Ser Gln Val Glu Arg Gln Glu Tyr Asp
            740                 745                 750

Gly Cys


<210>   164
<211>   2265
<212>   DNA
<213>   Chlamydia pneumoniae

<400>   164
atggtttttt tccgtaattc tttactgcat ttagttgccc tatccggaat gctctgttgt    60
tcttctggag tggctttaac gatagccgag aagatggctt ctttagagca ctcggggaga   120
ggagcagacg attatgaggg gatggcttcg tttaatgcca atatgaggga gtatagcctt   180
cagctgagca agttgtatga ggaagcacga aagctacgcg cttctggaac tgaggatgaa   240
gctctgtgga aggacttaat tcgacggatt ggtgaggtgc gaggctatct tcgagagatc   300
gaggagcttt gggctgcaga aattcgtgag aaagggggca atctcgagga ctacgccctc   360
tggaatcacc cagagactac gatttacaat cttgttaccg attacggaac cgaagactct   420
atttatttga ttcctcaaga aatcggagcg attaaaatcg caaccttatc gaaatttgta   480
gttcctaaag agtctttcga agactgtctc actcagatcc tatctcgctt aggtattggc   540
```

```
gtgcgtcagg tcaattcttg gattaaggaa ctttatatga tgcgtaagga gggctgcagt    600
gttgctggag ttttttcctc cagaaaagat ttagaggcgc tcccagaaac agcctatatt    660
ggttttgtat tgaattcgaa cgtagatgcg cataccaatc aacatgtctt aaaaaagttc    720
attaaccctg aaacaacgca tgtagatgtg attgcaggac gtgtgtggat tttggttctt    780
gcgggggaag tcggcgagct tctgaagatt tataattttg tgcagtcgga gagcatacgt    840
caagagtatc gggtgattcc cttaactaag atcgatccag gggagatgat ttccattctc    900
aacgcagcat ttcgtgagga tctgactaaa gatgttagtg aagaatcttt aggccttcgt    960
gtagttcctt tacagtatca agggcgttcg ttgtttttaa gtggaaccgc ggcgttagtg   1020
cagcaagcgc tgactctcat tcgagagctt gaagaaggga ttgagaaccc tacggataaa   1080
acagtatttt ggtataacgt caagcactcc gatccccaag agttggcggc attgctttcc   1140
caagtccatg atgtcttctc tggcgagaat aaggcgagtg tcggagctgc agatggatgt   1200
gggtcgcaat taaatgcctc gatccaaatt gatactacag taagttcttc tgcgaaagat   1260
ggctcagtga agtacggaaa cttcatcgcg gattctaaga caggaactct gattatggtg   1320
gttgagaaag aagttcttcc acgtattcag atgctactta agaaactaga tgtccctaaa   1380
aagatggtcc gtatcgaggt gctgttattt gaaagaaaat tggcacatga gcagaaatct   1440
gggttaaatc ttctacgtct tggtgaggaa gtttgtaaaa aagggtgcag tccttctgtg   1500
tcttgggccg ggggtactgg catactagaa tttttattta aaggaagtac gggatcttcg   1560
atagttcctg gttatgatct cgcctatcaa tttttaatgg ctcaagagga cgttcggatt   1620
aatgcgagtc cttctgtagt tactatgaac caaaccccag cacggattgc tgttgttgat   1680
gaaatgtcaa tagcggtgtc ttcagataaa gataaagcgc aatacaatcg tgcgcagtac   1740
ggtatcatga taaaaatgct ccccgtaatt aatgtgggag aggaagacgg aaaaagttac   1800
attactttag agacagacat cacctttgat actacgggaa aaaatcatga tgatcgtcct   1860
gatgttacaa ggcgtaatat tactaataag gtgcgcattg ctgacggaga gactgtgatt   1920
attggaggtt tgcgttgcaa acagatgtca gattctcatg atggcattcc tttccttgga   1980
gacattcctg gtatagggaa gttatttgga atgagttcca catcagacag tctcacggag   2040
atgtttgtat ttatcactcc gaagatccta gaaaatcctg tagagcaaca agaacgtaaa   2100
gaagaagctt tactctcttc gcgccctgga gagagagaag aatactatca ggctttagca   2160
gctagtgagg ctgcagcacg agcagctcat aaaaaattag agatgttccc ggcatcagga   2220
gtatctttat ctcaggtaga gaggcaagaa tacgatggct gctag                   2265
```

<210> 165
<211> 334
<212> PRT
<213> Chlamydia pneumoniae

<400> 165

```
Met Gln Pro Phe Ile Phe Thr Leu Leu Cys Leu Thr Ser Leu Val Ser
1               5                   10                  15

Leu Val Ala Phe Asp Ala Ala Asn Ala Arg Lys Arg Cys Ala Cys Ala
            20                  25                  30

Gln Thr Ile Glu Arg Gly Glu Asn Phe Phe Ser Ile Lys Arg Ser Ala
        35                  40                  45

Cys Ala Glu Ile Glu Tyr Gln Glu Lys Ser Arg His Ala Ser Ala Ile
    50                  55                  60

Glu Arg Ile Ser Lys Asp Lys Gly Lys Val Thr Pro Lys Gln Ile Ala
65                  70                  75                  80

Lys Val Ala Thr Lys Lys Lys Gln Arg Tyr Arg Leu Leu Gln Val Pro
                85                  90                  95

Phe Ser Arg Pro Pro Asn Asn Ser Arg Tyr Asn Leu Tyr Ala Leu Leu
            100                 105                 110

Ser Glu Pro Pro Glu Cys Tyr Ser Asp Thr Ala Ser Trp Tyr Ala Ile
        115                 120                 125

Phe Ile Arg Leu Leu Arg Arg Ala Tyr Val Asp Thr Gly Asn Val Pro
```

130 135 140

Pro Gly Ser Glu Tyr Ala Ile Ala Asn Ala Leu Ile Ser Asn Lys Gln
145 150 155 160

Glu Ile Leu Glu Arg Gly Ala Gln Leu Gly Pro Asp Val Ile Glu Thr
165 170 175

Leu Thr Leu Pro Glu Glu Gln Ala Glu Ile Phe Tyr Lys Met Leu Lys
180 185 190

Gly Ser Ser Asn Ser Gln Ser Leu Leu Asn Phe Leu His Tyr Glu Glu
195 200 205

Lys Ser Leu Gly His Cys Lys Leu Asn Leu Ile Phe Met Asp Pro Leu
210 215 220

Leu Leu Glu Ala Val Leu Asp His Pro Asp Ala Tyr Arg Glu Thr Ser
225 230 235 240

Leu Leu Arg Asp Gly Ile Trp Glu Ala Val Lys Arg Gln Glu His Ala
245 250 255

Ile Gln Glu His Gly Gln Ala Ala Ala Leu Glu Leu Phe Lys Thr Arg
260 265 270

Thr Asp Phe Arg Leu Glu Leu Arg Asp Lys Met Gln Leu Leu Leu Ser
275 280 285

Arg Tyr Asp Leu Leu Pro Leu Leu Asn Lys Lys Met Phe Asp Tyr Thr
290 295 300

Leu Gly Ser Ala Gly Asp Tyr Leu Phe Leu Val Asp Pro Asp Thr Lys
305 310 315 320

Ala Ile Ser Arg Cys Arg Cys Pro Ser Lys Ser Ile Lys Leu
325 330

<210> 166
<211> 1005
<212> DNA
<213> Chlamydia pneumoniae

<400> 166
atgcaacctt ttatctttac tttactgtgc ttgacatctt tggtttcttt agtcgccttt 60
gatgctgcga atgctcgtaa acgttgtgcc tgtgctcaaa ctatagaacg tggagagaac 120
ttcttttcca taaaacgctc tgcttgtgct gaaatcgaat atcaagaaaa atctcgccac 180
gcctcagcaa ttgaaagaat ctcaaaagat aaaggcaaag tcactccaaa gcagattgcg 240
aaagtagcta ctaagaaaaa gcaaagatac cgtttattgc aggttccttt ttcaaggcct 300
ccgaataact caaggtataa cctctatgct ttgcttagtg aacctcccga atgctatagc 360
gatacagcat catggtatgc tatttttatt cggttacttc gacgtgctta tgtagacacg 420
ggaaatgtac ctcctggatc tgagtatgcc atcgctaatg ctttgataag taacaaacaa 480
gagattttag agaggggagc gcagcttgga cccgatgtta ttgaaactct aacattgcct 540
gaggaacaag ccgagatttt ttataaaatg ctcaaagggt cgtcaaactc tcagtcgcta 600
ctgaatttc tgcattatga agagaaaagc ttaggccact gtaagctaaa tctgatcttc 660
atggatcccc tactgttaga agctgttcta gatcatcccg atgcttatag ggaaacgtcg 720
ctcctgcgcg atggcatttg ggaagcggtg aagcgtcaag aacatgccat ccaagaacat 780
ggccaggcag ctgctttgga gctttttaaa acacgcaccg acttccgcct ggagctgcga 840
gataagatgc agttacttct aagtcgatac gatttgctcc ccttattaaa taaaaaaatg 900
ttcgactaca ccttaggaag tgccggagat tacttatttt tggtagaccc agatactaag 960
gcaatttctc gatgtcgctg cccttcaaag agtattaaat tataa 1005

<210> 167
<211> 233
<212> PRT
<213> Chlamydia pneumoniae

<400> 167
Met Asn Arg Arg Asp Met Val Ile Thr Ala Val Val Val Asn Ala Ile
1 5 10 15

Leu Leu Val Ala Leu Phe Val Thr Ser Lys Arg Ile Gly Val Lys Asp
20 25 30

Tyr Asp Glu Gly Phe Arg Asn Phe Ala Ser Ser Lys Val Thr Gln Ala
35 40 45

Val Val Ser Glu Glu Lys Val Ile Glu Lys Pro Val Val Ala Glu Val
50 55 60

Pro Ser Arg Pro Ile Ala Lys Glu Thr Leu Ala Ala Gln Phe Ile Glu
65 70 75 80

Ser Lys Pro Val Ile Val Thr Thr Pro Pro Val Pro Val Val Ser Glu
85 90 95

Thr Pro Glu Val Pro Thr Val Ala Val Pro Pro Gln Pro Val Arg Glu
100 105 110

Thr Val Lys Glu Glu Gln Ala Pro Tyr Ala Thr Val Val Val Lys Lys
115 120 125

Gly Asp Phe Leu Glu Arg Ile Ala Arg Ala Asn His Thr Thr Val Ala
130 135 140

Lys Leu Met Gln Ile Asn Asp Leu Thr Thr Thr Gln Leu Lys Ile Gly
145 150 155 160

Gln Val Ile Lys Val Pro Thr Ser Gln Asp Val Ser Asn Glu Lys Thr
165 170 175

Pro Gln Thr Gln Thr Ala Asn Pro Glu Asn Tyr Tyr Ile Val Gln Glu
180 185 190

Gly Asp Ser Pro Trp Thr Ile Ala Leu Arg Asn His Ile Arg Leu Asp
195 200 205

Asp Leu Leu Lys Met Asn Asp Leu Asp Glu Tyr Lys Ala Arg Arg Leu
210 215 220

Lys Pro Gly Asp Gln Leu Arg Ile Arg
225 230

<210> 168
<211> 702
<212> DNA
<213> Chlamydia pneumoniae

<400> 168
atgaatcgta gagacatggt aataacagct gtcgtagtga atgctatatt gcttgtggct 60
cttttcgtca catcaaagcg tattggcgtc aaggactatg acgagggatt ccgtaatttt 120
gcttctagca aggttacaca agcagtagtt tcagaagaaa aagtcataga aaagcctgta 180

```
gtcgcagaag tgcctagccg tcctatcgct aaagagactc tagctgcaca gtttattgaa    240
agtaagccgg ttattgtaac cacaccaccc gtgcctgttg ttagcgaaac cccagaagtg    300
cctactgtgg cagttccgcc tcagcctgtt cgtgagacag taaaagagga acaagctcct    360
tatgctactg ttgtagtgaa aaaaggagat tttctcgaac gcattgcgag agcaaatcat    420
actaccgttg caaaattgat gcagatcaat gatcttacca ccacccaact taaaattggt    480
caggtcatca aagtccctac gtctcaagat gtcagcaacg aaaaaactcc tcaaacacag    540
accgcaaacc ctgaaaatta ttatatcgtc caagaagggg atagcccgtg gacaatagca    600
ttgcgtaacc atattcgatt ggatgatttg ctaaaaatga atgatctcga tgaatataaa    660
gcccggcgcc ttaagcctgg agatcagttg cgcatacgtt ga                        702

<210>   169
<211>   809
<212>   PRT
<213>   Chlamydia pneumoniae

<400>   169
Met Lys Gly Thr Pro Gln Tyr His Phe Ile Gly Ile Gly Gly Ile Gly
1               5                   10                  15

Met Ser Ala Leu Ala His Ile Leu Leu Asp Arg Gly Tyr Glu Val Ser
            20                  25                  30

Gly Ser Asp Leu Tyr Glu Ser Tyr Thr Ile Glu Ser Leu Lys Ala Lys
        35                  40                  45

Gly Ala Arg Cys Phe Ser Gly His Asp Ser Ser His Val Pro His Asp
    50                  55                  60

Ala Val Val Val Tyr Ser Ser Ser Ile Ala Pro Asp Asn Val Glu Tyr
65                  70                  75                  80

Leu Thr Ala Ile Gln Arg Ser Ser Arg Leu Leu His Arg Ala Glu Leu
                85                  90                  95

Leu Ser Gln Leu Met Glu Gly Tyr Glu Ser Ile Leu Val Ser Gly Ser
            100                 105                 110

His Gly Lys Thr Gly Thr Ser Ser Leu Ile Arg Ala Ile Phe Gln Glu
        115                 120                 125

Ala Gln Lys Asp Pro Ser Tyr Ala Ile Gly Gly Leu Ala Ala Asn Cys
    130                 135                 140

Leu Asn Gly Tyr Ser Gly Ser Ser Lys Ile Phe Val Ala Glu Ala Asp
145                 150                 155                 160

Glu Ser Asp Gly Ser Leu Lys His Tyr Thr Pro Arg Ala Val Val Ile
                165                 170                 175

Thr Asn Ile Asp Asn Glu His Leu Asn Asn Tyr Ala Gly Asn Leu Asp
            180                 185                 190

Asn Leu Val Gln Val Ile Gln Asp Phe Ser Arg Lys Val Thr Asp Leu
        195                 200                 205

Asn Lys Val Phe Tyr Asn Gly Asp Cys Pro Ile Leu Lys Gly Asn Val
    210                 215                 220

Gln Gly Ile Ser Tyr Gly Tyr Ser Pro Glu Cys Gln Leu His Ile Val
225                 230                 235                 240
```

```
Ser Tyr Asn Gln Lys Ala Trp Gln Ser His Phe Ser Phe Thr Phe Leu
                245                 250                 255

Gly Gln Glu Tyr Gln Asp Ile Glu Leu Asn Leu Pro Gly Gln His Asn
            260                 265                 270

Ala Ala Asn Ala Ala Ala Ala Cys Gly Val Ala Leu Thr Phe Gly Ile
        275                 280                 285

Asp Ile Asn Ile Ile Arg Lys Ala Leu Lys Lys Phe Ser Gly Val His
    290                 295                 300

Arg Arg Leu Glu Arg Lys Asn Ile Ser Glu Ser Phe Leu Phe Leu Glu
305                 310                 315                 320

Asp Tyr Ala His His Pro Val Glu Val Ala His Thr Leu Arg Ser Val
                325                 330                 335

Arg Asp Ala Val Gly Leu Arg Arg Val Ile Ala Ile Phe Gln Pro His
            340                 345                 350

Arg Phe Ser Arg Leu Glu Glu Cys Leu Gln Thr Phe Pro Lys Ala Phe
        355                 360                 365

Gln Glu Ala Asp Glu Val Ile Leu Thr Asp Val Tyr Ser Ala Gly Glu
    370                 375                 380

Ser Pro Arg Glu Ser Ile Ile Leu Ser Asp Leu Ala Glu Gln Ile Arg
385                 390                 395                 400

Lys Ser Ser Tyr Val His Cys Cys Tyr Val Pro His Gly Asp Ile Val
                405                 410                 415

Asp Tyr Leu Arg Asn Tyr Ile Arg Ile His Asp Val Cys Val Ser Leu
            420                 425                 430

Gly Ala Gly Asn Ile Tyr Thr Ile Gly Glu Ala Leu Lys Asp Phe Asn
        435                 440                 445

Pro Lys Lys Leu Ser Ile Gly Leu Val Cys Gly Gly Lys Ser Cys Glu
    450                 455                 460

His Asp Ile Ser Leu Leu Ser Ala Gln His Val Ser Lys Tyr Ile Ser
465                 470                 475                 480

Pro Glu Phe Tyr Asp Val Ser Tyr Phe Ile Ile Asn Arg Gln Gly Leu
                485                 490                 495

Trp Arg Thr Gly Lys Asp Phe Pro His Leu Ile Glu Glu Thr Gln Gly
            500                 505                 510

Asp Ser Pro Leu Ser Ser Glu Ile Ala Ser Ala Leu Ala Lys Val Asp
        515                 520                 525

Cys Leu Phe Pro Val Leu His Gly Pro Phe Gly Glu Asp Gly Thr Ile
    530                 535                 540

Gln Gly Phe Phe Glu Ile Leu Gly Lys Pro Tyr Ala Gly Pro Ser Leu
545                 550                 555                 560

Ser Leu Ala Ala Thr Ala Met Asp Lys Leu Leu Thr Lys Arg Ile Ala
```

```
                565                 570                 575

Ser Ala Val Gly Val Pro Val Val Pro Tyr Gln Pro Leu Asn Leu Cys
            580                 585                 590

Phe Trp Lys Arg Asn Pro Glu Leu Cys Ile Gln Asn Leu Ile Glu Thr
        595                 600                 605

Phe Ser Phe Pro Met Ile Val Lys Thr Ala His Leu Gly Ser Ser Ile
    610                 615                 620

Gly Ile Phe Leu Val Arg Asp Lys Glu Glu Leu Gln Glu Lys Ile Ser
625                 630                 635                 640

Glu Ala Phe Leu Tyr Asp Thr Asp Val Phe Val Glu Glu Ser Arg Leu
                645                 650                 655

Gly Ser Arg Glu Ile Glu Val Ser Cys Ile Gly His Ser Ser Ser Trp
            660                 665                 670

Tyr Cys Met Ala Gly Pro Asn Glu Arg Cys Gly Ala Ser Gly Phe Ile
        675                 680                 685

Asp Tyr Gln Glu Lys Tyr Gly Phe Asp Gly Ile Asp Cys Ala Lys Ile
    690                 695                 700

Ser Phe Asp Leu Gln Leu Ser Gln Glu Ser Leu Asp Cys Val Arg Glu
705                 710                 715                 720

Leu Ala Glu Arg Val Tyr Arg Ala Met Gln Gly Lys Gly Ser Ala Arg
                725                 730                 735

Ile Asp Phe Phe Leu Asp Glu Glu Gly Asn Tyr Trp Leu Ser Glu Val
            740                 745                 750

Asn Pro Ile Pro Gly Met Thr Ala Ala Ser Pro Phe Leu Gln Ala Phe
        755                 760                 765

Val His Ala Gly Trp Thr Gln Glu Gln Ile Val Asp His Phe Ile Ile
    770                 775                 780

Asp Ala Leu His Lys Phe Asp Lys Gln Gln Thr Ile Glu Gln Ala Phe
785                 790                 795                 800

Thr Lys Glu Gln Asp Leu Val Lys Arg
                805

<210>   170
<211>   2430
<212>   DNA
<213>   Chlamydia pneumoniae

<400>   170
atgaagggaa ctcctcagta tcattttatc ggtatcggtg gtataggaat gagcgcttta     60
gctcatattt tgcttgatcg tggctatgag gtctctggaa gcgacttata tgaaagctat    120
acgatcgaaa gcctgaaagc taaaggtgcg aggtgtttct caggccatga ttcctcccat    180
gttcctcatg atgccgtcgt tgtttatagc tcaagtatag cccctgataa tgtagagtat    240
cttaccgcta ttcaaagatc atcacgtctt cttcatagag cagagctctt gagtcagctt    300
atggagggtt atgaaagcat tctggtttca ggaagccatg ggaagacagg gacctcatct    360
ctaattcgag cgattttcca ggaagctcag aaagatccct cctatgctat tggaggactc    420
gctgcaaact gcctgaatgg gtattctgga tcatcgaaaa tcttcgttgc cgaagccgat    480
```

```
gaaagtgatg ggtctttaaa gcactacact ccccgtgcag tagtcattac aaatatagat   540
aatgaacatt tgaataatta cgctgggaat cttgataacc tggttcaggt aatccaggac   600
ttctctagaa aagtaacaga tctcaataag gtattctata acggggattg tcctattttg   660
aaaggaaatg tccaagggat ttcttatgga tattcaccag aatgtcaatt gcatatcgtt   720
tcctataatc aaaaggcatg gcaatctcac ttttccttta cctttttagg ccaggagtat   780
caagacattg agctcaatct ccctggacaa cataacgctg caaatgcagc agcagcctgt   840
ggagttgctc ttacctttgg catagacata aacatcattc gaaaagctct caaaaaattc   900
tcgggagttc atcgacgtct agaaagaaaa aatatatccg aaagctttct tttcttagaa   960
gattatgctc atcatcctgt agaggttgca cataccctgc gctctgtgcg tgatgctgtg  1020
ggtttgcgaa gagtcatcgc aatttttcaa ccacatcgat tctctcgttt agaagagtgc  1080
ttacaaacct tccccaaagc tttccaagaa gctgatgaag tcatacttac agatgtctat  1140
agtgccggag aaagtcctag agagtctatc attctttccg accttgcgga acagattcgt  1200
aagtcttctt atgtccattg ttgttatgtt ccccatggag acatcgtaga ttatctacga  1260
aactacattc gcattcatga tgtctgtgtt tctctaggag ctggaaatat ctatactatt  1320
ggagaggctt taaaagactt taaccctaaa aaattatcca taggactcgt ctgtggaggg  1380
aaatcttgcg aacacgatat ttctctactt tctgctcaac atgtctctaa atatatttct  1440
cctgaattct atgatgtgag ttacttcatc ataaatcgtc agggcttatg gagaacagga  1500
aaggattttc ctcatcttat tgaagagact caaggggatt cgccactttc ttctgaaatc  1560
gcttcagctt tagcaaaagt cgactgtttg tttcccgtgc tccatggccc atttggagag  1620
gatggtacga tccagggatt ttttgaaatc ttaggaaaac cttatgccgg accctcacta  1680
tctttagcag caactgcaat ggataagctg ttaacaaaac gaattgcatc agcagtgggt  1740
gttcctgtag tcccttacca acctttaaat ctctgtttct ggaaacgcaa tccagaacta  1800
tgtattcaga atcttataga gacattttct ttccctatga ttgtaaaaac tgcacatttg  1860
ggatctagta ttgggatatt tttagtccgt gataaagagg aattacaaga aaagatctca  1920
gaagcatttc tatatgacac ggatgtgttt gtggaggaaa gtcgcttagg gtctcgtgaa  1980
atcgaagtgt cctgtatcgg ccattcttct agctggtatt gtatggcagg gcctaatgaa  2040
cgctgtggtg ctagtgggtt tattgattat caagagaaat atggatttga tggcatagat  2100
tgcgcaaaga tctcttttga tttacagctc tcacaagaat ctttagattg tgttagagaa  2160
cttgcagagc gtgtctaccg agcaatgcaa ggaaaaggtt cagctcgaat agattttttc  2220
ttggatgaag aggggaatta ttggttgtca gaggtcaatc ctattccagg aatgacagca  2280
gctagcccat ttttacaagc ttttgttcac gcaggatgga cgcaagaaca aattgtagat  2340
cactttatta tagatgctct acataagttt gataagcagc agactatcga acaggcattc  2400
actaaagaac aagatttagt taaaagataa                                    2430
```

<210> 171
<211> 166
<212> PRT
<213> Chlamydia pneumoniae

<400> 171

```
Met Lys Phe Trp Leu Gln Gly Cys Ala Phe Val Gly Cys Leu Leu Leu
1               5                   10                  15

Thr Leu Pro Cys Cys Ala Ala Arg Arg Arg Ala Ser Gly Glu Asn Leu
            20                  25                  30

Gln Gln Thr Arg Pro Ile Ala Ala Ala Asn Leu Gln Trp Glu Ser Tyr
        35                  40                  45

Ala Glu Ala Leu Glu His Ser Lys Gln Asp His Lys Pro Ile Cys Leu
    50                  55                  60

Phe Phe Thr Gly Ser Asp Trp Cys Met Trp Cys Ile Lys Met Gln Asp
65                  70                  75                  80

Gln Ile Leu Gln Ser Ser Glu Phe Lys His Phe Ala Gly Val His Leu
                85                  90                  95

His Met Val Glu Val Asp Phe Pro Gln Lys Asn His Gln Pro Glu Glu
            100                 105                 110
```

Gln Arg Gln Lys Asn Gln Glu Leu Lys Ala Gln Tyr Lys Val Thr Gly
115 120 125

Phe Pro Glu Leu Val Phe Ile Asp Ala Glu Gly Lys Gln Leu Ala Arg
130 135 140

Met Gly Phe Glu Pro Gly Gly Gly Ala Ala Tyr Val Ser Lys Val Lys
145 150 155 160

Ser Ala Leu Lys Leu Arg
165

<210> 172
<211> 501
<212> DNA
<213> Chlamydia pneumoniae

<400> 172
atgaaatttt ggttgcaagg atgtgctttt gtcggttgtc tgctattgac tttaccttgt 60
tgtgctgcac gaagacgtgc ttctggagaa aatttgcaac aaactcgtcc tatagcagct 120
gcaaatctac aatgggagag ctatgcagaa gctcttgaac attctaaaca agatcacaaa 180
cctatttgtc ttttctttac aggatcagac tggtgtatgt ggtgcataaa aatgcaagac 240
cagattttgc aaagctctga gtttaagcat tttgcgggtg tgcatctgca tatggttgaa 300
gttgatttcc cccaaaagaa tcatcaacct gaagagcagc gccaaaaaaa tcaagaactg 360
aaagctcaat ataaagttac aggattcccc gaactggtct tcatagatgc agaaggaaaa 420
cagcttgctc gcatgggatt tgagcctggt ggtggagctg cttacgtaag caaggtgaag 480
tctgctctta aactacgtta a 501

<210> 173
<211> 380
<212> PRT
<213> Chlamydia pneumoniae

<400> 173
Met Ile Pro Ser Pro Thr Pro Ile Asn Phe Arg Asp Asp Thr Ile Leu
1 5 10 15

Glu Thr Asp Pro Lys Pro Ser Leu Ile Met Phe Ser Ser Lys Lys Thr
20 25 30

Glu Ile Ala Ser Glu Arg Arg Lys Ala His Pro Thr Leu Phe Lys Val
35 40 45

Leu Gly Thr Ile Trp Asn Ile Val Lys Phe Ile Ile Ser Ile Ile Leu
50 55 60

Phe Leu Pro Leu Ala Leu Leu Trp Val Leu Lys Lys Thr Cys Gln Phe
65 70 75 80

Phe Ile Leu Pro Ser Ser Ile Ile Ser Gln Ser Met Ser Lys Thr Ala
85 90 95

Val Ala Ile Arg Arg Met Thr Phe Leu Ser His Ile Lys Gln Leu Leu
100 105 110

Ser Leu Lys Glu Ile Ser Ala Ala Asp Arg Val Val Ile Gln Tyr Asp
115 120 125

Asp Leu Val Val Asp Ser Leu Ala Ile Lys Ile Pro His Ala Leu Pro
130 135 140

```
His Arg Trp Ile Leu Tyr Ser Gln Gly Asn Ser Gly Leu Met Glu Asn
145                 150                 155                 160

Leu Phe Asp Arg Gly Asp Ser Ser Leu His Gln Leu Ala Lys Ala Thr
                165                 170                 175

Gly Ser Asn Leu Leu Val Phe Asn Tyr Pro Gly Ile Met Ser Ser Lys
            180                 185                 190

Gly Glu Ala Lys Arg Glu Asn Leu Val Lys Ser Tyr Gln Ala Cys Val
        195                 200                 205

Arg Tyr Leu Arg Asp Glu Glu Thr Gly Pro Lys Ala Asn Gln Ile Ile
    210                 215                 220

Ala Phe Gly Tyr Ser Leu Gly Thr Ser Val Gln Ala Ala Ala Leu Asp
225                 230                 235                 240

Arg Glu Val Thr Asp Gly Ser Asp Gly Thr Ser Trp Ile Val Val Lys
                245                 250                 255

Asp Arg Gly Pro Arg Ser Leu Ala Asp Val Ala Asn Gln Ile Cys Lys
            260                 265                 270

Pro Ile Ala Ser Ala Ile Ile Lys Leu Val Gly Trp Asn Ile Asp Ser
        275                 280                 285

Val Lys Pro Ser Glu Arg Leu Arg Cys Pro Glu Ile Phe Ile Tyr Asn
    290                 295                 300

Ser Asn His Asp Gln Glu Leu Ile Ser Asp Gly Leu Phe Glu Arg Glu
305                 310                 315                 320

Asn Cys Val Ala Thr Pro Phe Leu Glu Leu Pro Glu Val Lys Thr Ser
                325                 330                 335

Gly Thr Lys Ile Pro Ile Pro Glu Arg Asp Leu Leu His Leu Asn Pro
            340                 345                 350

Leu Ser Pro Asn Val Val Asp Arg Leu Ala Ala Val Ile Ser Asn Tyr
        355                 360                 365

Leu Asp Ser Glu Asn Arg Lys Ser Gln Gln Pro Asp
    370                 375                 380

<210>  174
<211>  1143
<212>  DNA
<213>  Chlamydia pneumoniae

<400>  174
atgatcccat cccctacccc aataaacttt cgtgatgata cgattctaga gacggatcca    60
aagccgtctt taatcatgtt ctcttcaaaa aaaacagaga tagcttctga aagacggaag   120
gcccatccca ccttatttaa agttctagga acgatttgga atattgtgaa gtttattatc   180
tcaatcattc tgttccttcc cttagcgtta ttgtgggtac tcaagaaaac ctgtcagttt   240
ttcattctcc catcttctat catatctcag agcatgtcaa aaacagctgt ggcaattcgg   300
cgaatgacct ttctgtccca tattaaacaa ctcctaagcc ttaaggaaat ctcagctgcc   360
gatcgtgtgg ttatacaata tgacgatttg gtggttgata gcttagctat aaagatacct   420
catgctcttc cccacaggtg gattctttat tctcaaggaa actctggatt gatggaaaac   480
ctgttcgatc ggggcgattc ctctctacac cagctagcca aagcaaccgg ctcgaatctt   540
cttgtgttca actatcctgg aattatgtcc agcaaaggag aagcgaaacg agaaaatctg   600
```

gttaaatcgt atcaggcatg cgtacgctac ctacgagatg aagagacagg tcctaaagcc 660
aatcaaatca tagctttcgg atactctttg ggaactagtg tccaagctgc tgctctagat 720
cgtgaggtca ctgatggcag tgatggaact tcatggattg ttgtaaaaga tcggggccct 780
cgctctctag cagatgtcgc gaatcaaatt tgtaagccca tagcttccgc gattataaaa 840
ctcgttggtt ggaacataga ctctgtgaaa cctagcgaaa gattgcgttg tcccgaaatt 900
ttcatttaca actctaatca tgatcaagaa ctcattagcg acggcctctt cgaaagagaa 960
aattgcgtag caacaccttt tctagagctt cctgaagtaa aaacctcggg gactaaaatt 1020
cctatacccg aaagggatct tctccatcta aatcctctca gtccaaatgt agtagacaga 1080
ttagcagcag tgatctctaa ttatttagat tctgaaaaca gaaagtctca gcaacctgat 1140
taa 1143

<210> 175
<211> 158
<212> PRT
<213> Chlamydia pneumoniae

<400> 175
Met Leu Pro Ile Ser Ile Leu Leu Phe Tyr Val Ile Leu Gly Cys Leu
1 5 10 15

Ser Ala Tyr Ile Ala Asp Lys Lys Lys Arg Asn Val Ile Gly Trp Phe
20 25 30

Phe Ala Gly Ala Phe Phe Gly Phe Ile Gly Leu Val Val Leu Leu Leu
35 40 45

Leu Pro Ser Arg Arg Asn Ala Leu Glu Lys Pro Gln Asn Asp Pro Phe
50 55 60

Asp Asn Ser Asp Leu Phe Asp Asp Leu Lys Lys Ser Leu Ala Gly Asn
65 70 75 80

Asp Glu Ile Pro Ser Ser Gly Asp Leu Gln Glu Ile Val Ile Asp Thr
85 90 95

Glu Lys Trp Phe Tyr Leu Asn Lys Asp Arg Glu Asn Val Gly Pro Ile
100 105 110

Ser Phe Glu Glu Leu Val Val Leu Leu Lys Gly Lys Thr Tyr Pro Glu
115 120 125

Glu Ile Trp Val Trp Lys Lys Gly Met Lys Asp Trp Gln Arg Val Lys
130 135 140

Asp Val Pro Ser Leu Gln Gln Ala Leu Lys Glu Ala Ser Lys
145 150 155

<210> 176
<211> 477
<212> DNA
<213> Chlamydia pneumoniae

<400> 176
atgctcccta tttcgatttt attattttat gtgattctag gttgtctatc tgcctacata 60
gcagataaga aaaaacgaaa tgttattggc tggtttttttg caggagcatt ttttggattt 120
attggtctag ttgtccttct tcttcttcct tctcgtcgaa acgctttaga aaagccacaa 180
aacgatcctt ttgataactc cgatcttttt gatgatttga aaaaaagttt agcaggtaat 240
gacgagatac cctcatcggg agatcttcaa gaaatcgtta tcgatacaga gaagtggttt 300
tatttaaata aagatagaga aaacgtaggt ccgatatctt ttgaggagtt ggtcgtactt 360
ttaaagggaa aaacgtatcc agaagaaatt tgggtatgga aaaagggaat gaaagattgg 420
caacgagtga aggatgttcc atcactacaa caggctttga aagaagcatc aaaataa 477

<210> 177
<211> 343
<212> PRT
<213> Chlamydia pneumoniae

<400> 177

```
Met Glu Val Tyr Ser Phe His Pro Ala Val Arg Thr Ser Phe Gln His
1               5                   10                  15

Arg Val Met Ala Ala Leu Asp Ala Trp Phe Phe Leu Gly Gly His Arg
            20                  25                  30

Leu Lys Val Val Ser Leu Asp Ser Cys Asn Ser Gly Trp Ala Tyr Gln
        35                  40                  45

Glu Leu Val Ser Ile Ser Thr Thr Glu Lys Val Leu Lys Leu Leu Ser
    50                  55                  60

Tyr Leu Leu Val Pro Ile Val Ile Ile Ala Leu Leu Ile Arg Cys Leu
65                  70                  75                  80

Leu His Ser Asn Phe Arg Ile Asp Val Glu Lys Glu Arg Trp Leu Lys
                85                  90                  95

Ile Arg Glu Leu Gly Ile Asp Ile Glu Ser Cys Lys Leu Pro Ser Ser
            100                 105                 110

Tyr Val Asn Gln Val Ser Ser Phe Ile Trp Phe Glu Lys Asp Lys Ser
        115                 120                 125

Lys Arg Pro Arg Ile Asp Val Asp Tyr His Thr Leu His Ser Lys Asp
    130                 135                 140

Trp Val Val Phe Pro Ile Val Phe Gln Lys Ile Pro Lys Thr Ser Arg
145                 150                 155                 160

Phe Ser Tyr Trp Phe Ser Gln Lys Glu Thr Arg Lys Arg Asp Tyr Val
                165                 170                 175

Arg Asn Met Leu Asp His Val Ile Gly Tyr Leu Thr Ser Glu Gly Gly
            180                 185                 190

Glu Trp Leu Gln Tyr Ile Ser Lys Thr Ser Tyr Gln Ser Ala Thr Ser
        195                 200                 205

Leu Asp Pro Glu Arg Val Leu Gln Tyr Cys Leu Thr Asp Asn Gln Glu
    210                 215                 220

Leu Gln Gly Glu Val Gln Arg Leu Leu Asn Glu Glu Ser Ala Thr Lys
225                 230                 235                 240

Ser Ser Gly Asp Lys Glu Val Leu Leu Ser His Val Ser Asp Ile Ile
                245                 250                 255

Cys Gln Cys Trp Trp Pro Lys Phe Leu Glu Val Ile Gln Ser Pro Ala
            260                 265                 270

Phe Ile Glu Glu Leu Val Glu Glu Val Ser Gly Lys Leu Asn Leu Asp
        275                 280                 285
```

Phe Leu Cys Leu Glu Lys Ala Asn Thr Leu Asp Gln Glu Leu Arg Asn
290 295 300

Ser Leu Leu Arg Ala Val Val His His Gly Ser Glu Gly Val Asp Ile
305 310 315 320

Lys Lys Val Gly Ala Gly Leu Ile Ile Tyr Thr Glu Ala Ile Gln Leu
325 330 335

Gln Ile Pro Phe Ser Arg Ser
340

<210> 178
<211> 1032
<212> DNA
<213> Chlamydia pneumoniae

<400> 178
atggaagttt atagttttca ccctgcggta aggacttcgt ttcagcaccg tgtaatggca 60
gcactagatg cttggttttt tctaggaggg caccgtttaa aagtagtttc tctagatagt 120
tgtaactcag gttgggcgta tcaagaactt gtgtctattt caacgacaga aaaagtcttg 180
aaactactct cttacctact cgtaccgatt gtcataatag ctctgttaat tcgttgtctt 240
ttacatagca attttaggat agacgtagag aaggaacgtt ggttaaaaat aagggagtta 300
ggaattgata tagaaagctg caaactcccc agttcttatg taaaccaggt ttcctcgttt 360
atttggtttg aaaaagataa atccaaacgg ccacgtattg atgtagatta tcatacgcta 420
catagcaaag actgggtagt tttccctatc gtttttcaga aaattccaaa gacctcgcgt 480
ttcagttatt ggttctcaca aaaagaaaca aggaagaggg attatgtgag aaatatgctg 540
gaccacgtca ttggttatct aacgtcagaa ggtggggagt ggttgcagta tatatcgaaa 600
acctcttatc aaagcgctac ttccttggat cctgaaagag ttcttcaata ttgcttaact 660
gataaccagg agctccaggg agaagtgcaa cgtttgctta atgaggagag tgcgaccaaa 720
agctctgggg ataaggaagt tttgttaagt catgtatctg acattatttg ccagtgttgg 780
tggccaaagt ttcttgaagt tatacaatct ccggccttta ttgaagaatt agtagaagaa 840
gtgagtggta aacttaattt agatttttta tgcctagaaa aggctaatac attagatcag 900
gagttgagaa acagtcttct aagagcagtc gtacaccacg gttctgaagg agttgatatt 960
aagaaagttg gtgccggcct cattatttat acggaagcta ttcaattaca gattcccttc 1020
tcaaggagtt aa 1032

<210> 179
<211> 619
<212> PRT
<213> Chlamydia pneumoniae

<400> 179
Met Lys Lys Gly Lys Leu Gly Ala Ile Val Phe Gly Leu Leu Phe Thr
1 5 10 15

Ser Ser Val Ala Gly Phe Ser Lys Asp Leu Thr Lys Asp Asn Ala Tyr
20 25 30

Gln Asp Leu Asn Val Ile Glu His Leu Ile Ser Leu Lys Tyr Ala Pro
35 40 45

Leu Pro Trp Lys Glu Leu Leu Phe Gly Trp Asp Leu Ser Gln Gln Thr
50 55 60

Gln Gln Ala Arg Leu Gln Leu Val Leu Glu Glu Lys Pro Thr Thr Asn
65 70 75 80

Tyr Cys Gln Lys Val Leu Ser Asn Tyr Val Arg Ser Leu Asn Asp Tyr
85 90 95

His Ala Gly Ile Thr Phe Tyr Arg Thr Glu Ser Ala Tyr Ile Pro Tyr
100 105 110

Val Leu Lys Leu Ser Glu Asp Gly His Val Phe Val Val Asp Val Gln
115 120 125

Thr Ser Gln Gly Asp Ile Tyr Leu Gly Asp Glu Ile Leu Glu Val Asp
130 135 140

Gly Met Gly Ile Arg Glu Ala Ile Glu Ser Leu Arg Phe Gly Arg Gly
145 150 155 160

Ser Ala Thr Asp Tyr Ser Ala Ala Val Arg Ser Leu Thr Ser Arg Ser
165 170 175

Ala Ala Phe Gly Asp Ala Val Pro Ser Gly Ile Ala Met Leu Lys Leu
180 185 190

Arg Arg Pro Ser Gly Leu Ile Arg Ser Thr Pro Val Arg Trp Arg Tyr
195 200 205

Thr Pro Glu His Ile Gly Asp Phe Ser Leu Val Ala Pro Leu Ile Pro
210 215 220

Glu His Lys Pro Gln Leu Pro Thr Gln Ser Cys Val Leu Phe Arg Ser
225 230 235 240

Gly Val Asn Ser Gln Ser Ser Ser Ser Ser Leu Phe Ser Ser Tyr Met
245 250 255

Val Pro Tyr Phe Trp Glu Glu Leu Arg Val Gln Asn Lys Gln Arg Phe
260 265 270

Asp Ser Asn His His Ile Gly Ser Arg Asn Gly Phe Leu Pro Thr Phe
275 280 285

Gly Pro Ile Leu Trp Glu Gln Asp Lys Gly Pro Tyr Arg Ser Tyr Ile
290 295 300

Phe Lys Ala Lys Asp Ser Gln Gly Asn Pro His Arg Ile Gly Phe Leu
305 310 315 320

Arg Ile Ser Ser Tyr Val Trp Thr Asp Leu Glu Gly Leu Glu Glu Asp
325 330 335

His Lys Asp Ser Pro Trp Glu Leu Phe Gly Glu Ile Ile Asp His Leu
340 345 350

Glu Lys Glu Thr Asp Ala Leu Ile Ile Asp Gln Thr His Asn Pro Gly
355 360 365

Gly Ser Val Phe Tyr Leu Tyr Ser Leu Leu Ser Met Leu Thr Asp His
370 375 380

Pro Leu Asp Thr Pro Lys His Arg Met Ile Phe Thr Gln Asp Glu Val
385 390 395 400

Ser Ser Ala Leu His Trp Gln Asp Leu Leu Glu Asp Val Phe Thr Asp
405 410 415

Glu Gln Ala Val Ala Val Leu Gly Glu Thr Met Glu Gly Tyr Cys Met

420 425 430

Asp Met His Ala Val Ala Ser Leu Gln Asn Phe Ser Gln Ser Val Leu
435 440 445

Ser Ser Trp Val Ser Gly Asp Ile Asn Leu Ser Lys Pro Met Pro Leu
450 455 460

Leu Gly Phe Ala Gln Val Arg Pro His Pro Lys His Gln Tyr Thr Lys
465 470 475 480

Pro Leu Phe Met Leu Ile Asp Glu Asp Asp Phe Ser Cys Gly Asp Leu
485 490 495

Ala Pro Ala Ile Leu Lys Asp Asn Gly Arg Ala Thr Leu Ile Gly Lys
500 505 510

Pro Thr Ala Gly Ala Gly Gly Phe Val Phe Gln Val Thr Phe Pro Asn
515 520 525

Arg Ser Gly Ile Lys Gly Leu Ser Leu Thr Gly Ser Leu Ala Val Arg
530 535 540

Lys Asp Gly Glu Phe Ile Glu Asn Leu Gly Val Ala Pro His Ile Asp
545 550 555 560

Leu Gly Phe Thr Ser Arg Asp Leu Gln Thr Ser Arg Phe Thr Asp Tyr
565 570 575

Val Glu Ala Val Lys Thr Ile Val Leu Thr Ser Leu Ser Glu Asn Ala
580 585 590

Lys Lys Ser Glu Glu Gln Thr Ser Pro Gln Glu Thr Pro Glu Val Ile
595 600 605

Arg Val Ser Tyr Pro Thr Thr Thr Ser Ala Ser
610 615

<210> 180
<211> 1860
<212> DNA
<213> Chlamydia pneumoniae

<400> 180
atgaaaaaag ggaaattagg agccatagtt tttggccttc tatttacaag tagtgttgct 60
ggtttttcta aggatttgac taaagacaac gcttatcaag atttaaatgt catagagcat 120
ttaatatcgt taaaatatgc tcctttacca tggaaggaac tattatttgg ttgggattta 180
tctcagcaaa cacagcaagc tcgcttgcaa ctggtcttag aagaaaaacc aacaaccaac 240
tactgccaga aggtactctc taactacgtg agatcattaa acgattatca tgcagggatt 300
acgttttatc gtactgaaag tgcgtatatc ccttacgtat tgaagttaag tgaagatggt 360
catgtctttg tagtcgacgt acagactagc caaggggata tttacttagg ggatgaaatc 420
cttgaagtag atggaatggg gattcgtgag gctatcgaaa gccttcgctt tggacgaggg 480
agtgccacag actattctgc tgcagttcgt tccttgacat cgcgttccgc cgcttttgga 540
gatgcggttc cttcaggaat tgccatgttg aaacttcgcc gacccagtgg tttgatccgt 600
tcgacaccgg tccgttggcg ttatactcca gagcatatcg gagatttttc tttagttgct 660
cctttgattc ctgaacataa acctcaatta cctacacaaa gttgtgtgct attccgttcc 720
ggggtaaatt cacagtcttc tagtagctct ttattcagtt cctacatggt gccttatttc 780
tgggaagaat tgcgggttca aaataagcag cgttttgaca gtaatcacca tatagggagc 840
cgtaatggat ttttacctac gtttggtcct attctttggg aacaagacaa ggggccctat 900
cgttcctata tctttaaagc aaaagattct cagggcaatc cccatcgcat aggattttta 960
agaatttctt cttatgtttg gactgattta gaaggacttg aagaggatca taaggatagt 1020

```
ccttgggagc tctttggaga gatcatcgat catttggaaa aagagactga tgctttgatt   1080
attgatcaga cccataatcc tggaggcagt gttttctatc tctattcgtt actatctatg   1140
ttaacagatc atcctttaga tactcctaaa catagaatga ttttcactca ggatgaagtc   1200
agctcggctt tgcactggca agatctacta gaagatgtct tcacagatga gcaggcagtt   1260
gccgtgctag gggaaactat ggaaggatat tgcatggata tgcatgctgt agcctctctt   1320
caaaacttct ctcagagtgt cctttcttcc tgggtttcag gtgatattaa cctttcaaaa   1380
cctatgcctt tgctaggatt tgcacaggtt cgacctcatc ctaaacatca atatactaaa   1440
cctttgttta tgttgataga cgaggatgac ttctcttgtg gagatttagc gcctgcaatt   1500
ttgaaggata atggccgcgc tactctcatt ggaaagccaa cagcaggagc tggaggtttt   1560
gtattccaag tcactttccc taaccgttct ggaattaaag gtctttcttt aacaggatct   1620
ttagctgtta ggaaagatgg tgagtttatt gaaaacttag gagtggctcc tcatattgat   1680
ttaggattta cctccaggga tttgcaaact tccaggttta ctgattacgt tgaggcagtg   1740
aaaactatag ttttaacttc tttgtctgag aacgctaaga agagtgaaga gcagacttct   1800
ccgcaagaga cgcctgaagt tattcgagtc tcttatccca caacgacttc tgcttcgtaa   1860
```

<210> 181
<211> 486
<212> PRT
<213> Chlamydia pneumoniae

<400> 181

```
Met Asn Met Pro Val Pro Ser Ala Val Pro Ser Ala Asn Ile Thr Leu
1               5                   10                  15

Lys Glu Asp Ser Ser Thr Val Ser Thr Ala Ser Gly Ile Leu Lys Thr
            20                  25                  30

Ala Thr Gly Glu Val Leu Val Ser Cys Thr Ala Leu Glu Gly Ser Ser
        35                  40                  45

Ser Thr Asp Ala Leu Ile Ser Leu Ala Leu Gly Gln Ile Ile Leu Ala
    50                  55                  60

Thr Gln Gln Glu Leu Leu Leu Gln Ser Thr Asn Val His Gln Leu Leu
65                  70                  75                  80

Phe Leu Pro Pro Glu Val Val Glu Leu Glu Ile Gln Val Val Asp Leu
                85                  90                  95

Leu Val Gln Leu Glu His Ala Glu Thr Ile Thr Ser Glu Pro Gln Glu
            100                 105                 110

Thr Gln Thr Gln Ser Arg Ser Glu Gln Thr Leu Pro Gln Gln Ser Ser
        115                 120                 125

Ser Lys Gln Ser Ala Leu Ser Pro Arg Ser Leu Lys Pro Glu Ile Ser
    130                 135                 140

Asp Ser Lys Gln Gln Gln Ala Leu Gln Thr Pro Lys Asp Ser Ala Val
145                 150                 155                 160

Arg Lys His Ser Glu Ala Pro Ser Pro Glu Thr Gln Ala Arg Ala Ser
                165                 170                 175

Leu Ser Gln Ala Ser Ser Ser Ser Gln Arg Ser Leu Pro Pro Gln Glu
            180                 185                 190

Ser Ala Pro Glu Arg Thr Leu Leu Glu Gln Gln Lys Ala Ser Ser Phe
        195                 200                 205

Ser Pro Leu Ser Gln Phe Ser Ala Glu Lys Gln Lys Glu Ala Leu Thr
```

```
        210                 215                 220

Thr Ser Lys Ser His Glu Leu Tyr Lys Glu Arg Asp Gln Asp Arg Gln
225                 230                 235                 240

Gln Arg Glu Gln His Asp Arg Lys His Asp Gln Glu Glu Asp Ala Glu
                245                 250                 255

Ser Lys Lys Lys Lys Lys Lys Arg Gly Leu Gly Val Glu Ala Val Ala
            260                 265                 270

Glu Glu Pro Gly Glu Asn Leu Asp Ile Ala Ala Leu Ile Phe Ser Asp
        275                 280                 285

Gln Met Arg Pro Pro Ala Glu Glu Thr Ser Lys Lys Glu Thr Thr Phe
    290                 295                 300

Lys Lys Lys Leu Pro Ser Pro Met Ser Val Phe Ser Arg Phe Ile Pro
305                 310                 315                 320

Ser Lys Asn Pro Leu Ser Val Gly Ser Ser Ile His Gly Pro Ile Gln
                325                 330                 335

Thr Pro Lys Val Glu Asn Val Phe Leu Arg Phe Met Lys Leu Met Ala
            340                 345                 350

Arg Ile Leu Gly Gln Ala Glu Ala Glu Ala Asn Glu Leu Tyr Met Arg
        355                 360                 365

Val Lys Gln Arg Thr Asp Asp Val Asp Thr Leu Thr Val Leu Ile Ser
    370                 375                 380

Lys Ile Asn Asn Glu Lys Lys Asp Ile Asp Trp Ser Glu Asn Glu Glu
385                 390                 395                 400

Met Lys Ala Leu Leu Asn Arg Ala Lys Glu Ile Gly Val Thr Ile Asp
                405                 410                 415

Lys Glu Lys Tyr Thr Trp Thr Glu Glu Glu Lys Arg Leu Leu Lys Glu
            420                 425                 430

Asn Val Gln Met Arg Lys Glu Asn Met Glu Lys Ile Thr Gln Met Glu
        435                 440                 445

Arg Thr Asp Met Gln Arg His Leu Gln Glu Ile Ser Gln Cys His Gln
    450                 455                 460

Ala Arg Ser Asn Val Leu Lys Leu Leu Lys Glu Leu Met Asp Thr Phe
465                 470                 475                 480

Ile Tyr Asn Leu Arg Pro
                485
```

<210> 182
<211> 1461
<212> DNA
<213> Chlamydia pneumoniae

<400> 182

```
atgaatatgc ctgttccttc tgcagttccc tctgcaaata taactctaaa agaagacagc    60
tcaacagttt ccacagcctc tggaatatta aagactgcaa caggtgaagt cttagtctct   120
```

```
tgtacagcgc tagaaggaag ctcttctaca gatgctttaa ttagcttagc tttaggacaa   180
atcattcttg cgacccaaca agaactgctc ttacaaagca caaatgttca tcaactcctc   240
ttcctccctc ctgaagttgt agaattagaa atccaagttg ttgacttgct agtgcaattg   300
gaacatgcag agacaatcac aagtgaacca caagaaacac aaacgcaaag taggagtgag   360
cagaccctcc ctcaacaaag cagcagtaaa caatctgctc tctccccacg ctccttaaaa   420
cctgaaattt ctgattctaa acaacagcaa gctcttcaaa caccaaaaga ctctgctgta   480
agaaaacaca gcgaagcacc gtcacctgag acacaagctc gcgcttcctt atctcaggca   540
agctcaagtt ctcagagatc cttacctccg caagaaagtg cgccagaaag aacactatta   600
gaacaacaaa aagcaagctc cttctctcct ctatcccagt tctctgcaga gaaacaaaaa   660
gaggccctga cgacctcaaa atctcatgaa ctctataaag aacgcgatca agatcgccaa   720
caaagagagc agcacgacag aaagcacgat caggaagaag acgctgaatc taaaaagaaa   780
aagaagaaac gtggtctcgg tgtagaggca gtcgctgagg aacccggaga aaatctagat   840
attgccgctt taatcttctc agatcaaatg cgacctcctg ctgaagaaac ttctaaaaaa   900
gaaacgacat tcaaaaagaa gctaccttct ccaatgtctg tgtttagcag attcatccct   960
agtaagaatc cgttatctgt aggctcttca atacacgggc ctatacaaac tccaaaagta   1020
gaaaatgtgt tcttaaggtt catgaagctc atggcaagaa tcttaggcca agccgaagcc   1080
gaagctaatg aactctacat gcgagtcaaa caacgtaccg atgatgtaga cacactcaca   1140
gtccttatct ctaagatcaa taatgaaaag aaagacattg attggagtga aaatgaagag   1200
atgaaagctc ttttaaatcg agctaaagag attggagtca ctatagacaa agaaaaatat   1260
acttggacag aagaggaaaa aagacttcta aaagagaatg tccaaatgcg caaagagaat   1320
atggagaaaa tcactcaaat ggaaaggacg gacatgcaaa ggcacctcca agagatttct   1380
caatgtcatc aagcgcgctc taatgtattg aagttattga aagaacttat ggacaccttc   1440
atttacaacc tacgccccta a                                             1461
```

<210> 183
<211> 289
<212> PRT
<213> Chlamydia pneumoniae

<400> 183

```
Met Leu Lys Ile Gln Lys Lys Arg Met Cys Val Ser Val Val Ile Thr
1               5                   10                  15

Val Gly Ala Ile Val Gly Phe Phe Asn Ser Ala Asp Ala Ala Pro Lys
            20                  25                  30

Lys Lys Lys Ile Pro Ile Gln Ile Leu Tyr Ser Phe Thr Lys Val Ser
        35                  40                  45

Ser Tyr Leu Lys Asn Glu Asp Ala Ser Thr Ile Phe Cys Val Asp Val
    50                  55                  60

Asp Arg Gly Leu Leu Gln His Arg Tyr Leu Gly Ser Pro Gly Trp Gln
65                  70                  75                  80

Glu Thr Arg Arg Arg Gln Leu Phe Lys Ser Leu Glu Asn Gln Ser Tyr
                85                  90                  95

Gly Asn Glu Arg Leu Gly Glu Glu Thr Leu Ala Ile Asp Ile Phe Arg
            100                 105                 110

Asn Lys Glu Cys Leu Glu Ser Glu Ile Pro Glu Gln Met Glu Ala Ile
        115                 120                 125

Leu Ala Asn Ser Ser Ala Leu Val Leu Gly Ile Ser Ser Phe Gly Ile
    130                 135                 140

Thr Gly Ile Pro Ala Thr Leu His Ser Leu Leu Arg Gln Asn Leu Ser
145                 150                 155                 160

Phe Gln Lys Arg Ser Ile Ala Ser Glu Ser Phe Leu Leu Lys Ile Asp
```

165 170 175

Ser Ala Pro Ser Asp Ala Ser Val Phe Tyr Lys Gly Val Leu Phe Arg
180 185 190

Gly Glu Thr Ala Ile Val Asp Ala Leu Ser Gln Leu Phe Ala Gln Leu
195 200 205

Asp Leu Ser Pro Lys Lys Ile Ile Phe Leu Gly Glu Asp Pro Glu Val
210 215 220

Val Gln Ala Val Gly Ser Ala Cys Ile Gly Trp Gly Met Asn Phe Leu
225 230 235 240

Gly Leu Val Tyr Tyr Pro Ala Gln Glu Ser Leu Phe Ser Tyr Val His
245 250 255

Pro Tyr Ser Thr Ala Thr Glu Leu Gln Glu Ala Gln Gly Leu Gln Val
260 265 270

Ile Ser Asp Glu Val Ala Gln Leu Thr Leu Asn Ala Leu Pro Lys Met
275 280 285

Asn

<210> 184
<211> 870
<212> DNA
<213> Chlamydia pneumoniae

<400> 184
atgttgaaaa tccagaaaaa aagaatgtgt gtcagcgtag tcatcacggt aggcgccata 60
gtggggtttt tcaattctgc agacgcagca ccaaagaaaa agaagatccc tatacagatt 120
ctctactcct ttactaaagt ctcttcctat ttaaaaaacg aagacgcaag tactatattt 180
tgcgtcgatg tggatcgtgg acttctccag catcggtatt taggtagtcc aggatggcag 240
gaaaccagac gtcggcagtt atttaaatcc ttagaaaatc aatcatacgg caacgaacgt 300
ttaggagaag aaactcttgc tattgatatt ttcaggaaca aagagtgctt ggagagcgag 360
atcccagagc agatggaagc tatccttgca aattcctcgg ccttggtctt aggcatctct 420
tcttttggga tcacaggaat tcctgcgact ttgcatagtt tgcttcgaca gaatctatct 480
ttccaaaaac gctctatagc atcggagagc ttccttttaa agatcgatag tgccccctca 540
gatgcctctg ttttttataa aggcgtgctt ttccgcggag agactgcgat cgtggatgcg 600
ttaagccaat tatttgccca gctcgatctt tctcctaaaa aaattatctt tctaggagaa 660
gaccctgagg tcgttcaagc tgttgggtct gcttgtatag gttggggcat gaacttttta 720
ggcctggtat actatcctgc tcaagaaagc ctttttttctt atgttcatcc ttactctaca 780
gcaacggagc tccaagaagc acagggttta caagtaattt cagatgaagt cgcacagctt 840
actttaaacg ctcttccgaa aatgaattaa 870

<210> 185
<211> 116
<212> PRT
<213> Chlamydia pneumoniae

<400> 185
Met Met His Asn Ile Val Val Leu Ser Glu Glu Pro Gly Arg Ser Ala
1 5 10 15

Phe Leu Gly Arg Thr Ala Phe Phe Pro Asn Lys Tyr Pro Ile Ala Gln
20 25 30

Gly Gly Val Gly Ile Pro Ser Thr Ile Gly Asn Leu Phe Thr Ile Trp

35 40 45

Tyr Cys Phe Tyr Phe Tyr Arg Ala Ala Thr Pro Gln Ser Asp His Pro
50 55 60

Asp Gly Cys Gly Phe Ile Leu Leu Glu Arg Leu Lys Glu Leu Gly Ala
65 70 75 80

Gly Phe Phe Tyr Cys Asp Leu Arg Glu Ser Asn Thr Thr Gly Phe Thr
85 90 95

Leu Phe Phe Glu Gly Ser Asn Lys Gly Val Leu Lys Asn His Leu Phe
100 105 110

Ile Arg Asp Glu
115

<210> 186
<211> 351
<212> DNA
<213> Chlamydia pneumoniae

<400> 186
atgatgcaca atattgttgt tcttagtgag gaacctggac gaagcgcttt tcttggtagg 60
acggcatttt tccctaataa gtatccaata gctcagggtg gtgttggaat accatctaca 120
ataggcaatc tctttactat atggtactgt ttctattttt atagagctgc aactccacaa 180
tctgatcatc ctgacggatg tggctttatt ctactagaaa ggcttaagga gctcggtgca 240
gggttctttt attgtgatct tcgtgagtcc aataccactg gctttactct ttttttgaa 300
ggctccaata aaggtgtgtt aaagaatcac ttgtttatta gagatgagta a 351

<210> 187
<211> 184
<212> PRT
<213> Chlamydia pneumoniae

<400> 187
Val Ala Ser Glu Thr Tyr Pro Ser Gln Ile Leu His Ala Gln Arg Glu
1 5 10 15

Val Arg Asp Ala Tyr Phe Asn Gln Ala Asp Cys His Pro Ala Arg Ala
20 25 30

Asn Gln Ile Leu Glu Ala Lys Lys Ile Cys Leu Leu Asp Val Tyr His
35 40 45

Thr Asn His Tyr Ser Val Phe Thr Phe Cys Val Asp Asn Tyr Pro Asn
50 55 60

Leu Arg Phe Thr Phe Val Ser Ser Lys Asn Asn Glu Met Asn Gly Leu
65 70 75 80

Ser Asn Pro Leu Asp Asn Val Leu Val Glu Ala Met Val Arg Arg Thr
85 90 95

His Ala Arg Asn Leu Leu Ala Ala Cys Lys Ile Arg Asn Ile Glu Val
100 105 110

Pro Arg Val Val Gly Leu Asp Leu Arg Ser Gly Ile Leu Ile Ser Lys
115 120 125

Leu Glu Leu Lys Gln Pro Gln Phe Gln Ser Leu Thr Glu Asp Phe Val

```
    130                 135                 140

Asn His Ser Thr Asn Gln Glu Glu Ala Arg Val His Gln Lys His Val
145                 150                 155                 160

Leu Leu Ile Ser Leu Ile Leu Leu Cys Lys Gln Ala Val Leu Glu Ser
                165                 170                 175

Phe Gln Glu Lys Lys Arg Ser Ser
            180

<210>   188
<211>   555
<212>   DNA
<213>   Chlamydia pneumoniae

<400>   188
gtggcgtctg aaacgtatcc ttctcagata ttgcacgctc agagggaagt acgtgatgcc   60
tattttaatc aagcggattg ccatcctgct cgggctaatc agattctcga ggctaagaaa   120
atctgtttat tagatgttta tcatactaat cattattccg tatttacttt ttgtgtagat   180
aattatccga atctccgctt tacatttgta tcttcaaaaa acaatgagat gaatggctta   240
tctaatcctc tagataatgt tcttgtagag gctatggtac gtagaacaca tgcaagaaac   300
ctacttgcag cgtgtaaaat tcgaaatatt gaggttccaa gggttgttgg gcttgaccta   360
agatctggga tactcatttc gaaactagaa ttgaagcaac ctcagttcca aagtttaaca   420
gaagacttcg taaatcattc cacaaatcag gaagaagctc gcgtccatca aaagcatgtg   480
ttgctaattt ctttaatttt actttgcaag caggccgttc tggaatcatt ccaggaaaaa   540
aagcgatcct cttaa                                                    555

<210>   189
<211>   113
<212>   PRT
<213>   Chlamydia pneumoniae

<400>   189
Met Arg Glu Leu Asn Ala Phe Glu Leu Thr Gln Pro Glu Glu Tyr Arg
1               5                   10                  15

Asn Arg Trp Val Leu Met Pro Cys Leu Lys Cys Arg Phe Cys Arg Thr
            20                  25                  30

Gln His Ala Lys Val Trp Ser Tyr Arg Cys Val His Glu Ala Ser Leu
        35                  40                  45

Tyr Glu Lys Asn Cys Phe Leu Thr Leu Thr Tyr Asp Asp Lys His Leu
    50                  55                  60

Pro Gln Tyr Gly Ser Leu Val Lys Leu His Leu Gln Leu Phe Leu Lys
65                  70                  75                  80

Arg Leu Arg Lys Met Ile Ser Pro His Lys Ile Arg Tyr Phe Glu Cys
                85                  90                  95

Gly Ala Tyr Gly Thr Lys Leu Gln Arg Pro His Tyr His Leu Leu Leu
            100                 105                 110

Ser


<210>   190
<211>   342
<212>   DNA
```

<213> Chlamydia pneumoniae

<400> 190
```
ttgcgagaat taaatgcttt tgaattaact caacctgaag agtatcgaaa ccgttgggtt    60
ttgatgcctt gtcttaagtg tcgtttttgt agaacgcaac atgcaaaagt ctggtcttat   120
cgttgtgtcc atgaagcttc tttgtatgag aaaaattgtt ttcttacttt gacttatgat   180
gataagcatt tacctcagta tggttcgttg gtaaagctgc atttacagct gtttcttaag   240
agattaagaa agatgatttc tcctcataaa attcgttatt ttgaatgtgg tgcgtatgga   300
accaaattac aaagacctca ttatcatcta cttttatcat ga                      342
```

<210> 191
<211> 297
<212> PRT
<213> Chlamydia pneumoniae

<400> 191
```
Met Arg Arg Phe Leu Phe Leu Ile Leu Ser Ser Leu Pro Leu Val Ala
1               5                   10                  15

Phe Ser Ala Asp Asn Phe Thr Ile Leu Glu Glu Lys Gln Ser Pro Leu
            20                  25                  30

Ser Arg Val Ser Ile Ile Phe Ala Leu Pro Gly Val Thr Pro Val Ser
        35                  40                  45

Phe Asp Gly Asn Cys Pro Ile Pro Trp Phe Ser His Ser Lys Lys Thr
    50                  55                  60

Leu Glu Gly Gln Arg Ile Tyr Tyr Ser Gly Asp Ser Phe Gly Lys Tyr
65                  70                  75                  80

Phe Val Val Ser Ala Leu Trp Pro Asn Lys Val Ser Ser Ala Val Val
                85                  90                  95

Ala Cys Asn Met Ile Leu Lys His Arg Val Asp Leu Ile Leu Ile Ile
            100                 105                 110

Gly Ser Cys Tyr Ser Arg Ser Gln Asp Ser Arg Phe Gly Ser Val Leu
        115                 120                 125

Val Ser Lys Gly Tyr Ile Asn Tyr Asp Ala Asp Val Arg Pro Phe Phe
    130                 135                 140

Glu Arg Phe Glu Ile Pro Asp Ile Lys Lys Ser Val Phe Ala Thr Ser
145                 150                 155                 160

Glu Val His Arg Glu Ala Ile Leu Arg Gly Gly Glu Glu Phe Ile Ser
                165                 170                 175

Thr His Lys Gln Glu Ile Glu Glu Leu Leu Lys Thr His Gly Tyr Leu
            180                 185                 190

Lys Ser Thr Thr Lys Thr Glu His Thr Leu Met Glu Gly Leu Val Ala
        195                 200                 205

Thr Gly Glu Ser Phe Ala Met Ser Arg Asn Tyr Phe Leu Ser Leu Gln
    210                 215                 220

Lys Leu Tyr Pro Glu Ile His Gly Phe Asp Ser Val Ser Gly Ala Val
225                 230                 235                 240
```

```
Ser Gln Val Cys Tyr Glu Tyr Ser Ile Pro Cys Leu Gly Val Asn Ile
                245                 250                 255

Leu Leu Pro His Pro Leu Glu Ser Arg Ser Asn Glu Asp Trp Lys His
            260                 265                 270

Leu Gln Ser Glu Ala Ser Lys Ile Tyr Met Asp Thr Leu Leu Lys Ser
        275                 280                 285

Val Leu Lys Glu Leu Cys Ser Ser His
    290                 295

<210>  192
<211>  894
<212>  DNA
<213>  Chlamydia pneumoniae

<400>  192
atgcgtcgtt ttctgtttct tattcttagc tctcttcctt tggtcgcatt ctctgctgat    60
aatttcacta ttctagaaga aaaacagagt cctttaagtc gtgtaagtat tatttttgct   120
ttacctgggg ttactcccgt ttcttttgat ggtaattgtc ctattccttg gttttctcat   180
agtaaaaaga ctctagaggg acagagaatt tattactctg gcgactcctt tgggaaatac   240
tttgtagttt ctgctctttg gcctaataaa gtttcttcag ctgttgtggc ttgtaatatg   300
attcttaaac atcgagtgga tcttattcta attataggct cgtgttactc taggtctcaa   360
gatagccgtt ttggcagcgt cttagtttct aaaggctaca ttaattatga tgcagatgtg   420
aggcctttct ttgaaagatt tgagattcca gacattaaaa agagtgtttt tgcaaccagt   480
gaggttcatc gggaggcaat tcttcgtgga ggcgaagagt ttatttctac ccataaacaa   540
gaaatcgaag agcttttgaa gactcatggg tatttgaaat caacaaccaa aacggagcac   600
accttaatgg aaggtttggt tgctacaggc gagtctttcg cgatgtcgcg aaactatttt   660
ctttccttac aaaaattgta tccagagatt catggttttg atagtgtcag cggcgctgtt   720
tctcaggtat gctatgaata tagcattcct tgtttaggtg tgaatatcct tctccctcat   780
cctttagaat cacggagtaa cgaggattgg aagcatcttc aaagtgaggc aagtaaaatt   840
tatatggata ccttgctcaa gagtgtatta aaagaactct gttcttctca ttaa         894

<210>  193
<211>  181
<212>  PRT
<213>  Chlamydia pneumoniae

<400>  193
Met Ser Leu Leu Asn Leu Pro Ser Ser Gln Asp Ser Ala Ser Glu Asp
1               5                   10                  15

Ser Thr Ser Gln Ser Gln Ile Phe Asp Pro Ile Arg Asn Arg Glu Leu
            20                  25                  30

Val Ser Thr Pro Glu Glu Lys Val Arg Gln Arg Leu Leu Ser Phe Leu
        35                  40                  45

Met His Lys Leu Asn Tyr Pro Lys Lys Leu Ile Ile Ile Glu Lys Glu
    50                  55                  60

Leu Lys Thr Leu Phe Pro Leu Leu Met Arg Lys Gly Thr Leu Ile Pro
65                  70                  75                  80

Lys Arg Arg Pro Asp Ile Leu Ile Ile Thr Pro Pro Thr Tyr Thr Asp
                85                  90                  95

Ala Gln Gly Asn Thr His Asn Leu Gly Asp Pro Lys Pro Leu Leu Leu
            100                 105                 110
```

Ile Glu Cys Lys Ala Leu Ala Val Asn Gln Asn Ala Leu Lys Gln Leu
115 120 125

Leu Ser Tyr Asn Tyr Ser Ile Gly Ala Thr Cys Ile Ala Met Ala Gly
130 135 140

Lys His Ser Gln Val Ser Ala Leu Phe Asn Pro Lys Thr Gln Thr Leu
145 150 155 160

Asp Phe Tyr Pro Gly Leu Pro Glu Tyr Ser Gln Leu Leu Asn Tyr Phe
165 170 175

Ile Ser Leu Asn Leu
180

<210> 194
<211> 546
<212> DNA
<213> Chlamydia pneumoniae

<400> 194
atgtccttat tgaaccttcc ctcaagccag gattctgcat ctgaggactc cacatcgcaa 60
tctcaaatct tcgatcccat tagaaatcgg gagttagttt ctactcccga agaaaaagtc 120
cgccaaaggt tgctctcctt cctaatgcat aagctgaact accctaagaa actcatcatc 180
atagaaaaag aactcaaaac tctttttcct ctgcttatgc gtaaaggaac cctaatccca 240
aaacgccgcc cagatattct catcatcact ccccccacat acacagacgc acagggaaac 300
actcacaacc taggcgaccc aaaacccctg ctacttatcg aatgtaaggc cttagccgta 360
aaccaaaatg cactcaaaca actccttagc tataactact ctatcggagc cacctgcatt 420
gctatggcag ggaaacactc tcaagtgtca gctctcttca atccaaaaac acaaactctt 480
gatttttatc ctggcctccc agagtattcc caactcctaa actactttat ttctttaaac 540
ttatag 546

<210> 195
<211> 213
<212> PRT
<213> Chlamydia pneumoniae

<400> 195
Met Ser Val Asn Pro Ser Gly Asn Ser Lys Asn Asp Leu Trp Ile Thr
1 5 10 15

Gly Ala His Asp Gln His Pro Asp Val Lys Glu Ser Gly Val Thr Ser
20 25 30

Ala Asn Leu Gly Ser His Arg Val Thr Ala Ser Gly Gly Arg Gln Gly
35 40 45

Leu Leu Ala Arg Ile Lys Glu Ala Val Thr Gly Phe Phe Ser Arg Met
50 55 60

Ser Phe Phe Arg Ser Gly Ala Pro Arg Gly Ser Gln Gln Pro Ser Ala
65 70 75 80

Pro Ser Ala Asp Thr Val Arg Ser Pro Leu Pro Gly Gly Asp Ala Arg
85 90 95

Ala Thr Glu Gly Ala Gly Arg Asn Leu Ile Lys Lys Gly Tyr Gln Pro
100 105 110

Gly Met Lys Val Thr Ile Pro Gln Val Pro Gly Gly Gly Ala Gln Arg
115 120 125

```
Ser Ser Gly Ser Thr Thr Leu Lys Pro Thr Arg Pro Ala Pro Pro Pro
    130                 135                 140

Pro Lys Thr Gly Gly Thr Asn Ala Lys Arg Pro Ala Thr His Gly Lys
145                 150                 155                 160

Gly Pro Ala Pro Gln Pro Pro Lys Thr Gly Gly Thr Asn Ala Lys Arg
                165                 170                 175

Ala Ala Thr His Gly Lys Gly Pro Ala Pro Gln Pro Pro Lys Gly Ile
            180                 185                 190

Leu Lys Gln Pro Gly Gln Ser Gly Thr Ser Gly Lys Lys Arg Val Ser
        195                 200                 205

Trp Ser Asp Glu Asp
    210
```

<210> 196
<211> 642
<212> DNA
<213> Chlamydia pneumoniae

<400> 196

```
atgtctgtta atccatcagg aaattccaag aacgatctct ggattacggg agctcatgat    60
cagcatcccg atgttaaaga atccggggtt acaagtgcta acctaggaag tcatagagtg   120
actgcctcag gaggacgcca agggttatta gcacgaatca aagaagcagt aaccgggttt   180
tttagtcgga tgagcttctt cagatcggga gctccaagag gtagccaaca accctctgct   240
ccatctgcag atactgtacg tagcccgttg ccgggagggg atgctcgcgc taccgaggga   300
gctggtagga acttaattaa aaaagggtac caaccaggga tgaaagtcac tatcccacag   360
gttcctggag gaggggccca acgttcatca ggtagcacga cactaaagcc tacgcgtccg   420
gcacccccac ctcctaaaac gggtggaact aatgcaaaac gtccggcaac gcacgggaag   480
ggtccagcac cccagcctcc taaaacaggt gggaccaatg ctaagcgcgc agcaacgcat   540
gggaaaggtc cagcacctca acctcctaag ggcattttga aacagcctgg gcagtctggg   600
acttcaggaa agaagcgtgt cagctggtct gacgaagatt aa                      642
```

<210> 197
<211> 274
<212> PRT
<213> Chlamydia pneumoniae

<400> 197

```
Met Leu Leu Gly Phe Leu Cys Asp Cys Pro Cys Ala Ser Trp Gln Cys
1               5                   10                  15

Ala Ala Val Ala Asn Cys Tyr Asp Ser Val Phe Met Ser Arg Pro Glu
            20                  25                  30

His Lys Pro Asn Ile Pro Tyr Ile Thr Lys Ala Thr Arg Arg Gly Leu
        35                  40                  45

Arg Met Lys Thr Leu Ala Tyr Leu Ala Ser Leu Lys Asp Ala Arg Gln
    50                  55                  60

Leu Ala Tyr Asp Phe Leu Lys Asp Pro Gly Ser Leu Ala Arg Leu Ala
65                  70                  75                  80

Lys Ala Leu Ile Ala Pro Lys Glu Ala Leu Gln Glu Gly Asn Leu Phe
                85                  90                  95
```

Phe Tyr Gly Cys Ser Asn Ile Glu Asp Ile Leu Glu Glu Met Arg Arg
100 105 110

Pro His Arg Ile Leu Leu Leu Gly Phe Ser Tyr Cys Gln Lys Pro Lys
115 120 125

Ala Cys Pro Glu Gly Arg Phe Asn Asp Ala Cys Arg Tyr Asp Pro Ser
130 135 140

His Pro Thr Cys Ala Ser Cys Ser Ile Gly Thr Met Met Arg Leu Asn
145 150 155 160

Ala Arg Arg Tyr Thr Thr Val Ile Ile Pro Thr Phe Ile Asp Ile Ala
165 170 175

Lys His Leu His Thr Leu Lys Lys Arg Tyr Pro Gly Tyr Gln Ile Leu
180 185 190

Phe Ala Val Thr Ala Cys Glu Leu Ser Leu Lys Met Phe Gly Asp Tyr
195 200 205

Ala Ser Val Met Asn Leu Lys Gly Val Gly Ile Arg Leu Thr Gly Arg
210 215 220

Ile Cys Asn Thr Phe Lys Ala Phe Lys Leu Ala Glu Arg Gly Val Lys
225 230 235 240

Pro Gly Val Thr Ile Leu Glu Glu Asp Gly Phe Glu Val Leu Ala Arg
245 250 255

Ile Leu Thr Glu Tyr Ser Ser Ala Pro Phe Pro Arg Asp Phe Cys Glu
260 265 270

Ile His

<210> 198
<211> 825
<212> DNA
<213> Chlamydia pneumoniae

<400> 198
atgcttctag ggtttttgtg tgactgcccc tgtgcttcgt ggcagtgtgc ggccgttgct 60
aattgttatg attccgtatt tatgtctaga ccagagcaca aacctaatat tccttatatt 120
actaaagcta caagacgggg tctgcgtatg aagacgcttg cttatctggc ctctttaaaa 180
gatgctagac agcttgccta tgattttctg aaagatcctg gttctttagc tcggttagct 240
aaggctttga tagctcctaa ggaggcctta caggagggca acctattttt ttatggctgt 300
agtaatattg aggatatttt agaggagatg cgtcgtcctc atagaatcct tttgttagga 360
ttttcttatt gtcaaaagcc taaggcatgt cctgaagggc gtttcaatga tgcttgtcgg 420
tatgatcctt cacatcctac atgtgcctca tgttctatag ggaccatgat gcggctgaat 480
gctcgtagat acactactgt gatcatccct acatttatag atatcgcaaa acatttacac 540
actttaaaaa agcgctaccc tggatatcaa attctctttg cagttactgc ttgtgaactt 600
tccttaaaaa tgtttggaga ttatgcctcc gtaatgaact taaagggtgt gggcatcaga 660
ctcacaggac gtatttgcaa tacatttaag gcatttaaat tagctgagcg aggagtcaaa 720
ccaggagtca ctatcctaga agaagatggc tttgaggtat tagcaaggat tcttacagaa 780
tacagtagcg ctcctttccc tagagacttt tgtgagatcc attag 825

<210> 199
<211> 99
<212> PRT
<213> Chlamydia pneumoniae

<400> 199
Met Lys Lys Val Val Thr Leu Ser Ile Ile Phe Phe Ala Thr Tyr Cys
1 5 10 15

Ala Ser Glu Leu Ser Ala Val Thr Val Val Ala Val Pro Leu Ser Glu
20 25 30

Ala Pro Gly Lys Ile Gln Val Arg Pro Val Val Gly Leu Gln Phe Gln
35 40 45

Glu Glu Gln Gly Ser Val Pro Tyr Ser Phe Tyr Tyr Pro Tyr Asp Tyr
50 55 60

Gly Tyr Tyr Tyr Pro Glu Thr Tyr Gly Tyr Thr Lys Asn Thr Gly Gln
65 70 75 80

Glu Ser Arg Glu Cys Tyr Thr Arg Phe Glu Asp Gly Thr Ile Phe Tyr
85 90 95

Glu Cys Asp

<210> 200
<211> 300
<212> DNA
<213> Chlamydia pneumoniae

<400> 200
atgaagaaag tcgtaacact atccattata tttttcgcaa cgtattgtgc atcagagctt 60
agtgctgtaa ctgtagtggc tgtgccttta tcagaggctc cagggaagat tcaagttcgt 120
cccgtcgttg gtctgcaatt tcaagaagaa cagggttctg tgccctatag tttttattat 180
ccttatgact atgggtatta ctatccagag acttatggct atactaaaaa tacaggtcaa 240
gaaagtcgcg aatgttatac ccgatttgaa gatggcacaa ttttttatga atgcgattag 300

<210> 201
<211> 362
<212> PRT
<213> Chlamydia pneumoniae

<400> 201
Val His Tyr Cys Glu Arg Thr Leu Asp Pro Lys Tyr Ile Leu Lys Ile
1 5 10 15

Ala Leu Lys Leu Arg Gln Ser Leu Ser Leu Phe Phe Gln Asn Ser Gln
20 25 30

Ser Leu Gln Arg Ala Tyr Ser Thr Pro Tyr Ser Tyr Tyr Arg Ile Ile
35 40 45

Leu Gln Lys Glu Asn Lys Glu Lys Gln Ala Leu Ala Arg His Lys Cys
50 55 60

Ile Ser Ile Leu Glu Phe Phe Lys Asn Leu Leu Phe Val His Leu Leu
65 70 75 80

Ser Leu Ser Lys Asn Gln Arg Glu Gly Cys Ser Thr Asp Met Ala Val
85 90 95

Val Ser Thr Pro Phe Phe Asn Arg Asn Leu Trp Tyr Arg Leu Leu Ser
100 105 110

```
Ser Arg Phe Ser Leu Trp Lys Ser Tyr Cys Pro Arg Phe Phe Leu Asp
        115                 120                 125

Tyr Leu Glu Ala Phe Gly Leu Leu Ser Asp Phe Leu Asp His Gln Ala
    130                 135                 140

Val Ile Lys Phe Phe Glu Leu Glu Thr His Phe Ser Tyr Tyr Pro Val
145                 150                 155                 160

Ser Gly Phe Val Ala Pro His Gln Tyr Leu Ser Leu Leu Gln Asp Arg
                165                 170                 175

Tyr Phe Pro Ile Ala Ser Val Met Arg Thr Leu Asp Lys Asp Asn Phe
            180                 185                 190

Ser Leu Thr Pro Asp Leu Ile His Asp Leu Leu Gly His Val Pro Trp
        195                 200                 205

Leu Leu His Pro Ser Phe Ser Glu Phe Phe Ile Asn Met Gly Arg Leu
    210                 215                 220

Phe Thr Lys Val Ile Glu Lys Val Gln Ala Leu Pro Ser Lys Lys Gln
225                 230                 235                 240

Arg Ile Gln Thr Leu Gln Ser Asn Leu Ile Ala Ile Val Arg Cys Phe
                245                 250                 255

Trp Phe Thr Val Glu Ser Gly Leu Ile Glu Asn His Glu Gly Arg Lys
            260                 265                 270

Ala Tyr Gly Ala Val Leu Ile Ser Ser Pro Gln Glu Leu Gly His Ala
        275                 280                 285

Phe Ile Asp Asn Val Arg Val Leu Pro Leu Glu Leu Asp Gln Ile Ile
    290                 295                 300

Arg Leu Pro Phe Asn Thr Ser Thr Pro Gln Glu Thr Leu Phe Ser Ile
305                 310                 315                 320

Arg His Phe Asp Glu Leu Val Glu Leu Thr Ser Lys Leu Glu Trp Met
                325                 330                 335

Leu Asp Gln Gly Leu Leu Glu Ser Ile Pro Leu Tyr Asn Gln Glu Lys
            340                 345                 350

Tyr Leu Ser Gly Phe Glu Val Leu Cys Gln
        355                 360

<210>   202
<211>   1089
<212>   DNA
<213>   Chlamydia pneumoniae

<400>   202
gtgcactact gcgagagaac cctggaccca aagtatattc tgaagattgc tctaaagctg    60
agacaatcac tttccctgtt cttccagaac agccaatcac tccaacgtgc atactcgacc   120
ccatattcct actaccgaat cattctacaa aaggaaaata aagagaagca agctttagct   180
cgacacaaat gcatttctat tttagaattt ttcaaaaact tactctttgt tcatcttctg   240
tcattatcaa agaatcaaag ggaaggttgc tccactgata tggctgttgt aagcactccc   300
ttttttaatc ggaatttatg gtatcgactc ctttcctcac ggttttctct atggaaaagc   360
```

```
tattgtccaa gatttttttct tgattactta gaagctttcg gtctcctttc tgatttctta     420
gaccatcaag cagtcattaa attcttcgaa ttagaaacac atttttccta ttatcccgtt     480
tcaggatttg tagctcccca tcaatacttg tctctgttgc aggaccgtta ctttcccatt     540
gcctctgtaa tgcgaactct cgataaagat aatttctcct taactcctga tctcatccat     600
gacctttttag ggcacgtgcc ttggcttcta catccctcat tttctgaatt tttcataaac     660
atgggaagac tcttcactaa agtcatagaa aaagtacaag ctcttcctag taaaaaaacaa     720
cgcatacaaa ccctacaaag caatctgatc gctattgtac gctgcttttg gtttactgtt     780
gaaagcggac ttattgaaaa ccatgaagga agaaaagcat atggagccgt tcttatcagt     840
tctcctcagg aacttggaca cgctttcatt gataacgtac gtgttctccc tttagaattg     900
gatcagatta ttcgtcttcc cttcaataca tcaactccac aagagacttt attttcaata     960
agacattttg atgaactggt agaactcact tcaaaattag aatggatgct cgaccaaggt    1020
ctgttagaat caattcccct ttacaatcaa gagaaatatc tttctggttt tgaggtactt    1080
tgccaatga                                                            1089
```

<210>   203
<211>   210
<212>   PRT
<213>   Chlamydia pneumoniae

<400>   203

```
Met Met Asn Tyr Glu Asp Ala Lys Leu Arg Gly Gln Ala Val Ala Ile
1               5                   10                  15

Leu Tyr Gln Ile Gly Ala Ile Lys Phe Gly Lys His Ile Leu Ala Ser
            20                  25                  30

Gly Glu Glu Thr Pro Leu Tyr Val Asp Met Arg Leu Val Ile Ser Ser
        35                  40                  45

Pro Glu Val Leu Gln Thr Val Ala Thr Leu Ile Trp Arg Leu Arg Pro
    50                  55                  60

Ser Phe Asn Ser Ser Leu Leu Cys Gly Val Pro Tyr Thr Ala Leu Thr
65                  70                  75                  80

Leu Ala Thr Ser Ile Ser Leu Lys Tyr Asn Ile Pro Met Val Leu Arg
                85                  90                  95

Arg Lys Glu Leu Gln Asn Val Asp Pro Ser Asp Ala Ile Lys Val Glu
            100                 105                 110

Gly Leu Phe Thr Pro Gly Gln Thr Cys Leu Val Ile Asn Asp Met Val
        115                 120                 125

Ser Ser Gly Lys Ser Ile Ile Glu Thr Ala Val Ala Leu Glu Glu Asn
    130                 135                 140

Gly Leu Val Val Arg Glu Ala Leu Val Phe Leu Asp Arg Arg Lys Glu
145                 150                 155                 160

Ala Cys Gln Pro Leu Gly Pro Gln Gly Ile Lys Val Ser Ser Val Phe
                165                 170                 175

Thr Val Pro Thr Leu Ile Lys Ala Leu Ile Ala Tyr Gly Lys Leu Ser
            180                 185                 190

Ser Gly Asp Leu Thr Leu Ala Asn Lys Ile Ser Glu Ile Leu Glu Ile
        195                 200                 205

Glu Ser
    210
```

```
<210>   204
<211>   633
<212>   DNA
<213>   Chlamydia pneumoniae

<400>   204
atgatgaact acgaagatgc aaaattacgc ggtcaagctg tagcaattct ataccaaatc   60
ggagctataa agttcggaaa acatattctc gctagcggag aagaaactcc tctgtatgta  120
gatatgcgtc ttgtgatctc ctctccagaa gttctccaga cagtggcaac tcttatttgg  180
cgcctccgcc cctcattcaa tagtagctta ctctgcggag tcccttatac tgctctaacc  240
ctagcaacct cgatctcttt aaaatataac atccctatgg tattgcgaag gaaggaatta  300
cagaatgtag acccctcgga cgctattaaa gtagaagggt tatttactcc aggacaaact  360
tgtttagtca tcaatgatat ggtttcctca ggaaaatcta taatagagac agcagtcgca  420
ctggaagaaa atggtctggt agttcgtgaa gcattggtat tcttagatcg tagaaaagaa  480
gcgtgtcaac cacttggtcc acagggaata aaagtcagtt cggtatttac tgtacccact  540
ctgataaaag ctttgatcgc ttatgggaag ctaagcagtg gtgatctaac cctggcaaac  600
aaaatttccg aaattctaga aattgaatct taa                               633

<210>   205
<211>   67
<212>   PRT
<213>   Chlamydia pneumoniae

<400>   205
Met Lys Lys Leu Ile Ala Leu Ile Gly Ile Phe Leu Val Pro Ile Lys
1               5                   10                  15

Gly Asn Thr Asn Lys Glu His Asp Ala His Ala Thr Val Leu Lys Ala
            20                  25                  30

Ala Arg Ala Lys Tyr Asn Leu Phe Phe Val Gln Asp Val Phe Pro Val
        35                  40                  45

His Glu Val Ile Glu Pro Ile Ser Pro Asp Cys Leu Val His Tyr Glu
    50                  55                  60

Gly Trp Val
65

<210>   206
<211>   204
<212>   DNA
<213>   Chlamydia pneumoniae

<400>   206
atgaaaaaat tgattgcttt gatagggata tttcttgttc caataaaagg aaataccaat   60
aaggaacacg acgctcacgc gactgtttta aaagcggcca gagcaaagta taatttgttc  120
tttgttcagg atgttttccc tgtacacgaa gttatcgagc ctatttctcc cgattgcctg  180
gtacattatg aagggtgggt ttga                                         204

<210>   207
<211>   183
<212>   PRT
<213>   Chlamydia pneumoniae

<400>   207
Leu Asn Phe Ala Lys Ile Asp His Asn His Leu Tyr Leu Thr Cys Leu
1               5                   10                  15

Gly Asp Leu Gly Val Ala Cys Pro Ile Leu Ser Thr Asp Cys Leu Pro
```

20 25 30

Asn Tyr Ser Glu Lys Ala Ser His Glu Val Leu Val Tyr Ser Lys Phe
35 40 45

Arg Cys Ile Ser Gly Glu Pro Ser Arg Leu Ala Thr Ser Gly Asn Asp
50 55 60

Thr Tyr Tyr Ser Ile Val Ser Leu Pro Ile Gly Leu Arg Tyr Glu Val
65 70 75 80

Thr Ser Pro Ser Gly Arg His Asp Phe Asn Ile Asp Met His Val Ala
85 90 95

Pro Lys Ile Gly Ala Val Leu Ser His Gly Thr Arg Glu Ala Lys Glu
100 105 110

Ile Pro Gly Ser Ser Lys Asp Tyr Ala Phe Phe Ser Leu Thr Ala Arg
115 120 125

Glu Ser Leu Met Ile Ser Glu Lys Leu Ala Met Thr Phe Gln Val Ser
130 135 140

Glu Val Ile Gln Asn Cys Tyr Ser Gln Cys Thr Lys Val Thr Lys Thr
145 150 155 160

Asn Leu Lys Glu Gln Tyr Arg His Leu Ser His Asn Thr Gly Phe Glu
165 170 175

Leu Ser Val Lys Ser Ala Phe
180

<210> 208
<211> 552
<212> DNA
<213> Chlamydia pneumoniae

<400> 208
ttgaattttg caaagattga tcacaatcat ctctacctta catgtttggg agatcttggt 60
gtagcttgtc ctatactttc tacagattgt ctacctaatt atagcgagaa agcatctcat 120
gaggttcttg tttatagtaa atttagatgc atttctggag agccatctcg acttgcaact 180
tcaggaaatg acacatatta ttctatagta agtttaccta taggactccg ttacgaagtg 240
acttcaccat caggacgtca tgatttcaat attgatatgc atgtagctcc aaagataggt 300
gcagtactct ctcatggaac acgagaggct aaagagatcc caggatcttc aaaagactat 360
gcattttta gcttgactgc tagagaaagt ttaatgattt ctgaaaagct tgcgatgact 420
ttccaagtta gcgaagttat tcagaattgt tattcacaat gtactaaagt aacgaaaact 480
aatttaaaag aacagtatag gcacttatcc cacaatacag ggtttgagtt aagcgtcaag 540
tctgcattct aa 552

<210> 209
<211> 261
<212> PRT
<213> Chlamydia pneumoniae

<400> 209
Met Phe Leu Gln Phe Phe His Pro Ile Val Phe Ser Asp Gln Ser Leu
1 5 10 15

Ser Phe Leu Pro Tyr Leu Gly Lys Ser Ser Gly Ile Ile Glu Lys Cys
20 25 30

Ser Asn Ile Val Glu His Tyr Leu His Leu Gly Gly Asp Thr Ser Val
35 40 45

Ile Ile Thr Gly Val Ser Gly Ala Thr Phe Leu Ser Val Asp His Ala
50 55 60

Leu Pro Ile Ser Lys Ser Glu Lys Ile Ile Lys Ile Leu Ser Tyr Ile
65 70 75 80

Leu Ile Leu Pro Leu Ile Leu Ala Leu Phe Ile Lys Ile Val Leu Arg
85 90 95

Ile Ile Leu Phe Phe Lys Tyr Arg Gly Leu Ile Leu Asp Val Lys Lys
100 105 110

Glu Asp Leu Lys Lys Thr Leu Thr Pro Asp Gln Glu Asn Leu Ser Leu
115 120 125

Pro Leu Pro Ser Pro Thr Thr Leu Lys Lys Ile His Ala Leu His Ile
130 135 140

Leu Val Arg Ser Gly Lys Thr Tyr Asn Glu Leu Ile Gln Glu Gly Phe
145 150 155 160

Ser Phe Thr Lys Ile Thr Asp Leu Gly Gln Ala Pro Ser Pro Lys Gln
165 170 175

Asp Ile Gly Phe Ser Tyr Asn Ser Leu Leu Pro Asn Phe Tyr Phe His
180 185 190

Ser Leu Val Ser Val Pro Asn Ile Ser Gly Glu Glu Arg Ala Leu Asn
195 200 205

Tyr His Lys Glu Gln Gln Glu Glu Met Ala Val Lys Leu Lys Thr Met
210 215 220

Gln Ala Cys Ser Phe Val Phe Arg Ser Leu His Leu Pro Ser Met Gln
225 230 235 240

Thr Lys Asp Lys Lys Ala Gly Phe Gly Leu Leu Thr Phe Phe Pro Trp
245 250 255

Lys Ile Tyr Pro Leu
260

<210> 210
<211> 786
<212> DNA
<213> Chlamydia pneumoniae

<400> 210
atgtttcttc agtttttca tcctatagtc ttctcggatc agtccttatc ttttcttcct 60
tacctaggaa aaagctctgg cattattgaa aaatgttcca atatcgttga acactattta 120
catttgggag gagacacttc tgttatcatc acaggagttt ctggagctac ctttctatct 180
gttgatcatg ccctcccaat ctcgaaatct gaaaaaataa taaaaattct ctcctatatt 240
ttaattcttc ctctgattct agctctcttt attaagatcg ttttacgcat tatcttattc 300
ttcaagtatc gtggtctaat cctagatgtt aagaaggagg atttgaaaaa aacacttaca 360
cctgaccaag aaaacctcag tcttccttta ccatctccta caacattaaa gaaaattcat 420
gcgctacaca ttttagtgcg ttctggaaaa acctataacg agcttataca agaagggttt 480
tctttcacta aaatcacaga tcttggtcaa gctccttcac caaagcaaga tattggcttc 540
tcttataatt cccttctccc taacttctat tttcattcct tggtatctgt tccaaatatt 600

```
tcaggcgagg aacgggctct taattatcat aaagaacaac aagaggaaat ggctgttaaa   660
ttaaaaacaa tgcaagcgtg ttcttttgtc ttccgatccc tgcatttacc ttcaatgcaa   720
acgaaggaca aaaaggctgg atttggacta ctgacgtttt tcccttggaa aatctacccc   780
ctataa                                                              786

<210>   211
<211>   93
<212>   PRT
<213>   Chlamydia pneumoniae

<400>   211
Met Gly Asn His Glu Thr Tyr Ile His Pro Gly Val Leu Pro Ser Ser
1               5                   10                  15

His Ala Gln Asp Val Ser Arg Ser Thr Val Tyr Pro Ser Arg Ser Phe
            20                  25                  30

Ile Met Arg Arg Met Leu Met Gly Trp Asn Phe Asn Arg Val Pro Ser
        35                  40                  45

Lys Ser Ser Glu Gln Leu Met Asp Gly His Arg Ile Pro Leu Ile Phe
    50                  55                  60

Phe Gly Lys His His Pro Thr Ile Ser Ile Leu Asn Val Asn Arg Phe
65                  70                  75                  80

Ser Trp Leu Ser Ile Phe Tyr Asn Gly Glu Arg Gly Phe
                85                  90

<210>   212
<211>   282
<212>   DNA
<213>   Chlamydia pneumoniae

<400>   212
atgggaaacc atgagaccta tatacatcca ggagtgctcc cgagtagtca tgctcaggat   60
gttagcagat ctacagttta ccccagtcga agttttatca tgagacgtat gctcatgggc   120
tggaatttca atcgtgttcc ctcgaagagc tccgagcagt taatggatgg tcatcgcata   180
cctcttatat tttttgggaa gcatcatcct actatatcta ttttaaatgt caatagattt   240
tcttggctct ccattttta caatggagaa aggggtttt ga                        282

<210>   213
<211>   111
<212>   PRT
<213>   Chlamydia pneumoniae

<400>   213
Met Ser Glu Ser Ile Asn Arg Ser Ile His Leu Glu Ala Ser Thr Pro
1               5                   10                  15

Phe Phe Ile Lys Leu Thr Asn Leu Cys Glu Ser Arg Leu Val Lys Ile
            20                  25                  30

Thr Ser Leu Val Ile Ser Leu Leu Ala Leu Val Gly Ala Gly Val Thr
        35                  40                  45

Leu Val Val Leu Phe Val Ala Gly Ile Leu Pro Leu Leu Pro Val Leu
    50                  55                  60

Ile Leu Glu Ile Ile Leu Ile Thr Val Leu Val Leu Leu Phe Cys Leu
65                  70                  75                  80
```

```
Val Leu Glu Pro Tyr Leu Ile Glu Lys Pro Ser Lys Ile Lys Glu Leu
                85                  90                  95

Pro Lys Val Asp Glu Leu Ser Val Val Glu Thr Asp Ser Thr Leu
            100                 105                 110

<210>   214
<211>   336
<212>   DNA
<213>   Chlamydia pneumoniae

<400>   214
atgtctgaaa gtattaacag aagcattcat ttagaagcct ctacaccatt ttttataaaa    60
ttaacgaatc tctgtgaaag tagattagtt aagatcactt ctcttgttat ttctctatta   120
gctttagtgg gtgcgggagt cactcttgtg gttttatttg tagctgggat ccttccttta   180
cttcctgtac tcatcttaga aattatttta ataaccgtcc ttgtcttgct tttttgtttg   240
gtattggaac cttatttaat agaaaaacct agtaaaataa aggaactacc taaagtagac   300
gagctatctg tagtagaaac ggacagtact ctttaa                             336

<210>   215
<211>   59
<212>   PRT
<213>   Chlamydia pneumoniae

<400>   215
Met Arg Val Met Arg Phe Phe Cys Leu Phe Phe Leu Gly Phe Leu Gly
1               5                   10                  15

Ser Phe His Cys Val Ala Glu Asp Lys Gly Val Asp Leu Phe Gly Val
            20                  25                  30

Trp Asp Asp Asn Gln Ile Thr Glu Cys Asp Asp Ser Tyr Met Thr Glu
        35                  40                  45

Gly Arg Glu Glu Val Glu Lys Val Val Asp Ala
    50                  55

<210>   216
<211>   180
<212>   DNA
<213>   Chlamydia pneumoniae

<400>   216
gtgagagtta tgagattttt ttgtctattt tttcttgggt tcctaggatc ttttcattgt    60
gttgctgaag acaagggcgt ggatttattt ggagtctggg acgataacca aattacagag   120
tgtgacgata gttacatgac agagggtcgt gaagaggttg aaaaggtagt ggacgcttag   180

<210>   217
<211>   344
<212>   PRT
<213>   Chlamydia pneumoniae

<400>   217
Met Glu Asn Ala Met Ser Ser Ser Phe Val Tyr Asn Gly Pro Ser Trp
1               5                   10                  15

Ile Leu Lys Thr Ser Val Ala Gln Glu Val Phe Lys Lys His Gly Lys
            20                  25                  30

Gly Ile Gln Val Leu Leu Ser Thr Ser Val Met Leu Phe Ile Gly Leu
```

```
        35                  40                  45

Gly Val Cys Ala Phe Ile Phe Pro Gln Tyr Leu Ile Val Phe Val Leu
    50                  55                  60

Thr Ile Ala Leu Leu Met Leu Ala Ile Ser Leu Val Leu Phe Leu Leu
65                  70                  75                  80

Ile Arg Ser Val Arg Ser Ser Met Val Asp Arg Leu Trp Cys Ser Glu
                85                  90                  95

Lys Gly Tyr Ala Leu His Gln His Glu Asn Gly Pro Phe Leu Asp Val
            100                 105                 110

Lys Arg Val Gln Gln Ile Leu Leu Arg Ser Pro Tyr Ile Lys Val Arg
        115                 120                 125

Ala Leu Trp Pro Ser Gly Asp Ile Pro Glu Asp Pro Ser Gln Ala Ala
    130                 135                 140

Val Leu Leu Leu Ser Pro Trp Thr Phe Phe Ser Ser Val Asp Val Glu
145                 150                 155                 160

Ala Leu Leu Pro Ser Pro Gln Glu Lys Glu Gly Lys Tyr Ile Asp Pro
                165                 170                 175

Val Leu Pro Lys Leu Ser Arg Ile Glu Arg Val Ser Leu Leu Val Phe
            180                 185                 190

Leu Ser Ala Phe Thr Leu Asp Asp Leu Asn Glu Gln Gly Val Asn Pro
        195                 200                 205

Leu Met Asn Asn Glu Glu Phe Leu Phe Phe Ile Asn Lys Lys Ala Arg
    210                 215                 220

Glu His Gly Ile Gln Asp Leu Lys His Glu Ile Met Ser Ser Leu Glu
225                 230                 235                 240

Lys Thr Gly Val Pro Leu Asp Pro Ser Met Ser Phe Gln Val Ser Gln
                245                 250                 255

Ala Met Phe Ser Val Tyr Arg Tyr Leu Arg Gln Arg Asp Leu Thr Thr
            260                 265                 270

Ser Glu Leu Arg Cys Phe His Leu Leu Ser Cys Phe Lys Gly Asp Val
        275                 280                 285

Val His Cys Leu Ala Ser Phe Glu Asn Pro Lys Asp Leu Ala Asp Ser
    290                 295                 300

Asp Phe Leu Glu Ala Cys Lys Asn Val Glu Trp Gly Glu Phe Ile Ser
305                 310                 315                 320

Ala Cys Glu Lys Ala Leu Leu Lys Asn Pro Gln Gly Ile Ser Ile Lys
                325                 330                 335

Asp Leu Lys Gln Phe Leu Val Arg
            340
```

<210> 218
<211> 1035

<212> DNA
<213> Chlamydia pneumoniae

<400> 218

```
atggagaatg ctatgtcatc atcgtttgtg tataatgggc cttcgtggat tttaaaaacg   60
tcagtagctc aggaggtatt taaaaagcac ggtaaggga  ttcaggttct cttaagtact   120
tcagtgatgc tttttatagg tcttggagtc tgtgccttta tatttcctca atatctgatt   180
gtttttgttt tgactatagc tttgcttatg ctcgctataa gcttggtatt gtttctctta   240
atacgttctg tacgctcttc aatggtagat cgtttgtggt gttctgaaaa aggatatgct   300
cttcatcaac atgagaacgg gcctttttg  gatgtgaagc gtgtacagca aattcttcta   360
agatcaccct atattaaagt tcgggcttta tggccgtctg gagatatccc tgaggatcct   420
tcacaagctg cggttctatt actttctcct tggactttct tttcatccgt ggatgtagag   480
gctttattac cgagtcctca agaaaaggag ggtaagtata tagatcctgt gctgcctaag   540
ttgtctagga tagagagagt ctcactttta gtgtttttga gtgcatttac tttggatgac   600
ttaaacgaac agggagtcaa tcctttgatg aataatgagg aatttttatt ttttataaat   660
aagaaagcgc gtgagcatgg gattcaggat ttaaaacacg agattatgtc ttcgttagag   720
aaaacaggag tgccattaga cccctcaatg agttttcaag tttcacaagc gatgttttct   780
gtatatcgct acttgagaca aagggattta acgacttcag aattaagatg ttttcacctc   840
ttaagttgtt ttaaagggga tgtggttcat tgtttagctt catttgaaaa ccctaaagat   900
ttagcagatt ctgacttttt agaagcttgt aagaacgtgg aatggggtga gtttatttcg   960
gcatgtgaga aggctctttt aaagaatccg caaggaattt ccattaagga tctaaaacaa   1020
tttttagtga ggtaa                                                    1035
```

<210> 219
<211> 325
<212> PRT
<213> Chlamydia pneumoniae

<400> 219

```
Met Ile Glu Phe Ala Phe Val Pro His Thr Ser Val Thr Ala Asp Arg
1               5                   10                  15

Ile Glu Asp Arg Met Ala Cys Arg Met Asn Lys Leu Ser Thr Leu Ala
            20                  25                  30

Ile Thr Ser Leu Cys Val Leu Ile Ser Ser Val Cys Ile Met Ile Gly
        35                  40                  45

Ile Leu Cys Ile Ser Gly Thr Val Gly Thr Tyr Ala Phe Val Val Gly
    50                  55                  60

Ile Ile Phe Ser Val Leu Ala Leu Val Ala Cys Val Phe Phe Leu Tyr
65                  70                  75                  80

Phe Phe Tyr Phe Ser Ser Glu Glu Phe Lys Cys Ala Ser Ser Gln Glu
                85                  90                  95

Phe Arg Phe Leu Pro Ile Pro Ala Val Val Ser Ala Leu Arg Ser Tyr
            100                 105                 110

Glu Tyr Ile Ser Gln Asp Ala Ile Asn Asp Val Ile Lys Asp Thr Met
        115                 120                 125

Gln Leu Ser Thr Leu Ser Ser Leu Leu Asp Pro Glu Ala Phe Phe Leu
    130                 135                 140

Glu Phe Pro Tyr Phe Asn Ser Leu Ile Val Asn His Ser Met Lys Glu
145                 150                 155                 160

Ala Asp Arg Leu Ser Arg Glu Ala Phe Leu Ile Leu Leu Gly Glu Ile
                165                 170                 175
```

```
Thr Trp Lys Asp Cys Glu Thr Lys Ile Leu Pro Trp Leu Lys Asp Pro
            180                 185                 190

Asn Ile Thr Pro Asp Asp Phe Trp Lys Leu Leu Lys Asp His Phe Asp
        195                 200                 205

Leu Lys Asp Phe Lys Lys Arg Ile Ala Thr Trp Ile Arg Lys Ala Tyr
    210                 215                 220

Pro Glu Ile Arg Leu Pro Lys Lys His Cys Leu Asp Lys Ser Ile Tyr
225                 230                 235                 240

Lys Gly Cys Cys Lys Phe Leu Leu Leu Ser Glu Asn Asp Val Gln Tyr
                245                 250                 255

Gln Arg Leu Leu His Lys Val Cys Tyr Phe Ser Gly Glu Phe Pro Ala
            260                 265                 270

Met Val Leu Gly Leu Gly Ser Glu Val Pro Met Val Leu Gly Leu Pro
        275                 280                 285

Lys Val Pro Lys Asp Leu Thr Trp Glu Met Phe Met Glu Asn Met Pro
    290                 295                 300

Val Leu Leu Gln Ser Lys Arg Glu Gly His Trp Lys Ile Ser Leu Glu
305                 310                 315                 320

Asp Val Ala Ser Leu
                325
```

<210> 220
<211> 978
<212> DNA
<213> Chlamydia pneumoniae

<400> 220

```
atgatcgagt ttgcttttgt tcctcatacc tccgtgacag cggatcggat tgaggatcgc    60
atggcctgtc gcatgaacaa gttgtctact ttagcaatta caagtctttg tgtattgatc   120
agttcagttt gtattatgat tgggatttta tgcatttctg gaacggttgg gacctatgca   180
tttgttgtag gaattatttt ttctgtgctt gctttggtag catgtgtttt ctttctttat   240
ttcttttatt tttcttctga ggaatttaag tgtgcttctt cgcaggagtt tcgttttttg   300
cctataccag ctgtggtttc tgcattgcgt tcctatgaat acatttctca ggacgctatc   360
aatgacgtta taaaagatac gatgcagttg tctacccttt cttctctttt agatcccgaa   420
gctttttctt tagaatttcc ttattttaac tctttgatag tgaatcattc gatgaaggaa   480
gcggatcgtt tgtctcgaga ggctttttttg attttattag gtgagattac ttggaaggat   540
tgtgaaacaa aaattttgcc atggttgaaa gatcctaata tcactcctga tgatttctgg   600
aagctattaa aagaccattt cgatttaaag gactttaaga agaggatcgc cacttggata   660
cggaaggcct atccagaaat tagattaccg aagaagcatt gtttagataa gtctatctat   720
aaggggtgtt gtaagttttt attactttct gagaatgatg tgcaatatca gaggttatta   780
cataaggtct gttatttctc tggggagttt cctgccatgg ttttaggttt gggaagtgaa   840
gtgcctatgg tgttaggact ccctaaggtt cccaaggatc ttacctggga gatgtttatg   900
gaaaatatgc ctgttcttct gcaaagcaaa agagaggggc attggaaaat ctccttggaa   960
gacgtagcct ctctttaa                                                   978
```

<210> 221
<211> 374
<212> PRT
<213> Chlamydia pneumoniae

<400> 221

```
Met Asn Thr Ser Leu Lys Arg Pro Leu Lys Ser His Phe Asp Val Val
1               5                   10                  15

Gly Ser Phe Leu Arg Pro Glu His Leu Lys Lys Thr Arg Glu Ser Leu
            20                  25                  30

Lys Glu Gly Ser Ile Ser Leu Asp Gln Leu Met Gln Ile Glu Asp Ile
        35                  40                  45

Ala Ile Gln Asp Leu Ile Lys Lys Gln Lys Ala Ala Gly Leu Ser Phe
    50                  55                  60

Ile Thr Asp Gly Glu Phe Arg Arg Ala Thr Trp His Tyr Asp Phe Met
65                  70                  75                  80

Trp Gly Phe His Gly Val Gly His His Arg Ala Thr Glu Gly Val Phe
                85                  90                  95

Phe Asp Gly Glu Arg Ala Met Ile Asp Asp Thr Tyr Leu Thr Asp Lys
            100                 105                 110

Ile Ser Val Ser His His Pro Phe Val Asp His Phe Lys Phe Val Lys
        115                 120                 125

Ala Leu Glu Asp Glu Phe Thr Thr Ala Lys Gln Thr Leu Pro Ala Pro
    130                 135                 140

Ala Gln Phe Leu Lys Gln Met Ile Phe Pro Asn Asn Ile Glu Val Thr
145                 150                 155                 160

Arg Lys Phe Tyr Pro Thr Asn Gln Glu Leu Ile Glu Asp Ile Val Ala
                165                 170                 175

Gly Tyr Arg Lys Val Ile Arg Asp Leu Tyr Asp Ala Gly Cys Arg Tyr
            180                 185                 190

Leu Gln Leu Asp Asp Cys Thr Arg Gly Gly Leu Val Asp Pro Arg Val
        195                 200                 205

Cys Ser Trp Tyr Gly Ile Asp Glu Lys Gly Leu Gln Asp Leu Ile Gln
    210                 215                 220

Gln Tyr Leu Leu Ile Asn Asn Leu Val Ile Ala Asp Arg Pro Asp Asp
225                 230                 235                 240

Leu Val Val Asn Leu His Val Cys Arg Gly Asn Tyr His Ser Lys Phe
                245                 250                 255

Phe Ala Ser Gly Ser Tyr Asp Phe Ile Ala Lys Pro Leu Phe Glu Gln
            260                 265                 270

Thr Asn Val Asp Gly Tyr Tyr Leu Glu Phe Asp His Glu Arg Ser Gly
        275                 280                 285

Asp Phe Ser Pro Leu Thr Phe Ile Ser Gly Glu Lys Thr Val Cys Leu
    290                 295                 300

Gly Leu Val Thr Ser Lys Thr Pro Thr Leu Glu Asn Lys Asp Glu Val
305                 310                 315                 320

Ile Ala Arg Ile His Gln Ala Ala Asp Tyr Leu Pro Leu Glu Arg Leu
```

325 330 335

Ser Leu Ser Pro Gln Cys Gly Phe Ala Ser Cys Glu Ile Gly Asn Lys
340 345 350

Leu Thr Glu Glu Glu Gln Trp Ala Lys Val Ala Leu Val Lys Glu Ile
355 360 365

Ser Glu Glu Val Trp Lys
370

<210> 222
<211> 1125
<212> DNA
<213> Chlamydia pneumoniae

<400> 222
```
atgaatactt cactaaaaag acctctgaaa tctcattttg atgttgtcgg tagtttttttg   60
cgtcctgagc atttaaaaaa aactagagaa agccttaaag aaggctctat ttctctagat   120
caactcatgc aaattgagga tatcgctatc caagatttga tcaaaaaaca aaaagcagca   180
ggtctttctt ttattactga tggagaattc cgcagagcta cgtggcatta cgacttcatg   240
tggggttttc atggcgtagg tcaccacaga gctacagaag gagttttctt tgatggagaa   300
cgcgctatga tcgatgatac ctatctgaca gacaagatct ctgtatctca ccacccattt   360
gtggatcact ttaaatttgt aaaagctcta gaagatgaat ttacgactgc aaagcaaact   420
cttcctgcac cggcacagtt tttaaagcag atgatcttcc ctaataatat agaggtcaca   480
cgtaaattct atcctacaaa tcaggagcta attgaagata ttgttgcagg ttatcgtaaa   540
gtcattcgcg atctttatga tgctggctgc cgctatctcc aattagatga ctgtactcgg   600
ggaggtttag tagaccctcg agtctgttcg tggtatggta tcgatgaaaa aggtcttcaa   660
gatctgattc aacaatatct tctgattaat aatcttgtaa ttgcagatcg tcccgatgat   720
ctagtcgtta atttacatgt atgccgtggg aactaccact caaaattctt tgctagtggt   780
agttatgact ttattgcaaa gcccctattc gaacaaacaa atgtagacgg ctactattta   840
gagtttgatc atgagcgttc tggagacttc tctcctctca ccttcatttc tggagaaaaa   900
actgtctgct taggtcttgt taccagcaaa acccctacac ttgaaaataa ggatgaggtc   960
attgctcgca tacatcaagc agcagactac ctgcccttgg aaagactctc tctaagtcca  1020
cagtgtggtt ttgcttcatg tgaaatagga aataaattaa cagaagaaga gcaatgggct  1080
aaagttgctc tagtaaaaga aatttccgaa gaagtttgga aataa                  1125
```

<210> 223
<211> 342
<212> PRT
<213> Chlamydia pneumoniae

<400> 223
Met Lys Leu Tyr Ser Ile Ser Ser Asp Val Asp Thr Pro Trp Ile Phe
1 5 10 15

Gln Leu Met Ser Lys Val Asp Ser Tyr Leu Phe Leu Gly Gly Asn Arg
20 25 30

Ile Lys Val Val Ser Ile Val Met Gln Glu Pro Asn Leu Ile Ile Gly
35 40 45

Lys Val Glu Asn Val Arg Ile Ser Thr Ile Val Lys Ile Leu Lys Ile
50 55 60

Leu Ser Phe Leu Ile Phe Pro Leu Ile Leu Ile Ala Leu Ala Leu His
65 70 75 80

Tyr Phe Leu His Ala Lys Tyr Ala Asn His Leu Leu Val Ser Lys Ile
85 90 95

```
Leu Glu Arg Ala Pro Gln Tyr Val Pro Ile Pro Gly Arg Ser Gly Asp
            100                 105                 110

Thr Ala Ser His Tyr Lys Leu Thr Thr Leu Val Pro Val Ser Gln Lys
        115                 120                 125

Asn Leu Gln Ala Met Gly Ser Asn Pro Leu Glu Val Glu Ala Ala Leu
    130                 135                 140

Arg Thr Thr Lys Pro Ser Phe Phe Cys Val Pro Ala Lys Tyr Arg Gln
145                 150                 155                 160

Ile Ile Ile Ser Ser His Gly Ile Arg Phe Ser Leu Asp Leu Glu Gln
                165                 170                 175

Leu Ala Asp Asp Ile Asn Leu Asp Ser Val Ser Trp Pro Thr Glu Tyr
            180                 185                 190

Leu Asn Ser Thr Met Asp Phe Cys Ser Lys Ala Asp Lys Arg Val Ile
        195                 200                 205

Gln Asn Val Gln Asn Leu Arg Thr Gly Thr Tyr Ile Asn Ser Val Gly
    210                 215                 220

Lys Arg Ser Leu Leu Lys Phe Met Leu Gln His Leu Phe Ile Asp Gly
225                 230                 235                 240

Ile Thr Gln Glu Asn Pro Glu Ala Leu Pro Asn Asn Thr Ser Gly Arg
                245                 250                 255

Leu Thr Leu Phe Pro Ser Val Arg Tyr Ile Tyr Ser His Phe Thr Pro
            260                 265                 270

Gln Asn Pro Thr Ile Trp Pro Gln Val Phe Phe Arg Gln Gly Pro Leu
        275                 280                 285

Asp Glu Asp Arg Gly Gly Gly Phe Glu Ile Leu Glu Gln Leu Gln Glu
    290                 295                 300

Leu Gly Val Arg Phe Pro Ile Cys Pro Ser Gln Gly Pro Asp Asn Pro
305                 310                 315                 320

Asn Phe Gln Gly Phe Gln Gly Ile Arg Ile Tyr Trp Glu Asp Ser Tyr
                325                 330                 335

Gln Pro Asn Lys Glu Val
            340
```

<210> 224
<211> 1029
<212> DNA
<213> Chlamydia pneumoniae

<400> 224

```
atgaaacttt atagcatctc ttcagatgta gatacacctt ggatatttca gcttatgtca    60
aaggtagatt cttatctttt cttaggcggg aatagaatca aggttgtatc tatagttatg   120
caagaaccta acttaattat tggaaaagta gaaaacgttc ggatctccac aatagtgaaa   180
atattaaaga ttttatcctt cttaatcttc cctctgattt taatcgcttt agccctacac   240
tattttctac atgctaaata tgctaatcac ttacttgtat ctaagatttt agaaagagct   300
cctcagtatg tgcctattcc tggtcgttca ggagacacgg cgtctcatta taaattaaca   360
acattggttc cagtatccca aaaaaatcta caagctatgg gatcaaatcc tctagaagtt   420
```

```
gaagcggctc ttcgaactac aaaaccctct tttttctgtg tacctgcaaa ataccgtcag    480
attataattt caagtcacgg cattcgcttt tctttagatc ttgaacaact tgctgatgac    540
attaatttag attcggtttc ctggcctacg gagtatctta actctactat ggatttttgc    600
agcaaggcag ataaacgtgt tatacagaat gtacaaaatc tgcggacagg aacttacata    660
aattctgtag gaaagcgtag ccttttaaaa ttcatgttac agcacctatt tattgatggg    720
atcacacaag aaaaccctga agcccttcct aacaatacat ctggaagact gactctattc    780
cctagtgttc gttatatcta ttctcatttt actccacaaa atcctacaat atggccgcaa    840
gtctttttca gacaaggtcc tctagatgaa gatcgaggag gaggatttga gatcttagag    900
caattacaag agttaggagt taggtttcca atttgcccct ctcaaggacc agacaatcct    960
aattttcaag gttttcaagg gattcgtatc tattgggaag attcctatca acccaataag   1020
gaggtttaa                                                           1029
```

<210> 225
<211> 334
<212> PRT
<213> Chlamydia pneumoniae

<400> 225

```
Met Ser Tyr Asp Thr Leu Phe Lys Asn Leu Glu Lys Glu Asp Ser Val
1               5                   10                  15

His Lys Ile Cys Asn Glu Ile Phe Ala Leu Val Pro Arg Leu Asn Thr
            20                  25                  30

Ile Ala Cys Thr Glu Ala Ile Ile Lys Asn Leu Pro Lys Ala Asp Ile
        35                  40                  45

His Val His Leu Pro Gly Thr Ile Thr Pro Gln Leu Ala Trp Ile Leu
    50                  55                  60

Gly Val Lys Asn Gly Phe Leu Lys Trp Ser Tyr Asn Ser Trp Thr Asn
65                  70                  75                  80

His Arg Leu Leu Ser Pro Lys Asn Pro His Lys Gln Tyr Ser Asn Ile
                85                  90                  95

Phe Arg Asn Phe Gln Asp Ile Cys His Glu Lys Asp Pro Asp Leu Ser
            100                 105                 110

Val Leu Gln Tyr Asn Ile Leu Asn Tyr Asp Phe Asn Ser Phe Asp Arg
        115                 120                 125

Val Met Ala Thr Val Gln Gly His Arg Phe Pro Pro Gly Gly Ile Gln
    130                 135                 140

Asn Glu Glu Asp Leu Leu Leu Ile Phe Asn Asn Tyr Leu Gln Gln Cys
145                 150                 155                 160

Leu Asp Asp Thr Ile Val Tyr Thr Glu Val Gln Gln Asn Ile Arg Leu
                165                 170                 175

Ala His Val Leu Tyr Pro Ser Leu Pro Glu Lys His Ala Arg Met Lys
            180                 185                 190

Phe Tyr Gln Ile Leu Tyr Arg Ala Ser Gln Thr Phe Ser Lys His Gly
        195                 200                 205

Ile Thr Leu Arg Phe Leu Asn Cys Phe Asn Lys Thr Phe Ala Pro Gln
    210                 215                 220

Ile Asn Thr Gln Glu Pro Ala Gln Glu Ala Val Gln Trp Leu Gln Glu
```

225 230 235 240

Val Asp Ser Thr Phe Pro Gly Leu Phe Val Gly Ile Gln Ser Ala Gly
245 250 255

Ser Glu Ser Ala Pro Gly Ala Cys Pro Lys Arg Leu Ala Ser Gly Tyr
260 265 270

Arg Asn Ala Tyr Asp Ser Gly Phe Gly Cys Glu Ala His Ala Gly Glu
275 280 285

Gly Ile Glu Thr Arg Thr Ile Phe Ser Ser Ala Lys Val Asn Pro Glu
290 295 300

Gly Leu Ile Glu Ile Thr Arg Val Thr Phe Ser Ser Leu Lys Arg Lys
305 310 315 320

Gln Pro Ser Ser Leu Pro Ile Arg Val Thr Cys Gln Leu Gly
325 330

<210> 226
<211> 1005
<212> DNA
<213> Chlamydia pneumoniae

<400> 226
atgtcttatg atacgttatt caagaatctt gaaaaggaag attctgtaca taagatatgc 60
aatgagatct ttgcattagt accacgactc aatacaatcg cttgcaccga agctatcatc 120
aaaaacctcc ccaaagcaga tatccatgta caccttcctg ggaccataac acctcaatta 180
gcttggattt taggtgtgaa aaatgggttc ttaaaatggt cttataattc ttggaccaat 240
catcgattac tttctcctaa gaatcctcat aaacaatact ccaatatttt ccgaaacttt 300
caagatatct gtcacgaaaa ggatccggat ttaagtgtat tacaatataa tatcttaaat 360
tacgatttta atagctttga tagagtgatg gctacagtac aaggacatcg ctttcctcct 420
ggaggaatcc aaaatgaaga agaccttctt ctcattttca ataactatct ccagcaatgt 480
ctggacgata ctatcgtgta tactgaagta caacaaaata tccgccttgc ccatgttttg 540
tatccttcat tacctgaaaa gcacgcgcgt atgaagtttt atcaaatctt gtatcgtgct 600
tcgcaaacgt tttcaaaaca cgggattact ttacgatttt taaactgctt caataaaaca 660
tttgctccac aaataaacac acaagaacct gcccaagaag ctgttcaatg gctccaagag 720
gttgattcta catttcctgg tctatttgta gggatacaat ccgcaggatc agaatctgcg 780
cccggagcct gtcctaagcg attagcttct ggatatagaa atgcttatga ctcagggttt 840
ggttgtgaag ctcatgctgg agaaggcata gagacccgga ctatttttc gtcagctaag 900
gtaaatccag agggattgat cgagataacc cgagtgactt tctcgtctct taaacgaaaa 960
cagccatcta gtttacccat aagagttact tgccagttag gataa 1005

<210> 227
<211> 429
<212> PRT
<213> Chlamydia pneumoniae

<400> 227
Leu Gln Ser Ala Arg Arg His Leu Asn Thr Ile Phe Ile Leu Asp Phe
1 5 10 15

Gly Ser Gln Tyr Thr Tyr Val Leu Ala Lys Gln Val Arg Lys Leu Phe
20 25 30

Val Tyr Cys Glu Val Leu Pro Trp Asn Ile Ser Val Gln Cys Leu Lys
35 40 45

Glu Arg Ala Pro Leu Gly Ile Ile Leu Ser Gly Gly Pro His Ser Val
50 55 60

```
Tyr Glu Asn Lys Ala Pro His Leu Asp Pro Glu Ile Tyr Lys Leu Gly
65                  70                  75                  80

Ile Pro Ile Leu Ala Ile Cys Tyr Gly Met Gln Leu Met Ala Arg Asp
                85                  90                  95

Phe Gly Gly Thr Val Ser Pro Gly Val Gly Glu Phe Gly Tyr Thr Pro
            100                 105                 110

Ile His Leu Tyr Pro Cys Glu Leu Phe Lys His Ile Val Asp Cys Glu
        115                 120                 125

Ser Leu Asp Thr Glu Ile Arg Met Ser His Arg Asp His Val Thr Thr
    130                 135                 140

Ile Pro Glu Gly Phe Asn Val Ile Ala Ser Thr Ser Gln Cys Ser Ile
145                 150                 155                 160

Ser Gly Ile Glu Asn Thr Lys Gln Arg Leu Tyr Gly Leu Gln Phe His
                165                 170                 175

Pro Glu Val Ser Asp Ser Thr Pro Thr Gly Asn Lys Ile Leu Glu Thr
            180                 185                 190

Phe Val Gln Glu Ile Cys Ser Ala Pro Thr Leu Trp Asn Pro Leu Tyr
        195                 200                 205

Ile Gln Gln Asp Leu Val Ser Lys Ile Gln Asp Thr Val Ile Glu Val
    210                 215                 220

Phe Asp Glu Val Ala Gln Ser Leu Asp Val Gln Trp Leu Ala Gln Gly
225                 230                 235                 240

Thr Ile Tyr Ser Asp Val Ile Glu Ser Ser Arg Ser Gly His Ala Ser
                245                 250                 255

Glu Val Ile Lys Ser His His Asn Val Gly Gly Leu Pro Lys Asn Leu
            260                 265                 270

Lys Leu Lys Leu Val Glu Pro Leu Arg Tyr Leu Phe Lys Asp Glu Val
        275                 280                 285

Arg Ile Leu Gly Glu Ala Leu Gly Leu Ser Ser Tyr Leu Leu Asp Arg
    290                 295                 300

His Pro Phe Pro Gly Pro Gly Leu Thr Ile Arg Val Ile Gly Glu Ile
305                 310                 315                 320

Leu Pro Glu Tyr Leu Ala Ile Leu Arg Arg Ala Asp Leu Ile Phe Ile
                325                 330                 335

Glu Glu Leu Arg Lys Ala Lys Leu Tyr Asp Lys Ile Ser Gln Ala Phe
            340                 345                 350

Ala Leu Phe Leu Pro Ile Lys Ser Val Ser Val Lys Gly Asp Cys Arg
        355                 360                 365

Ser Tyr Gly Tyr Thr Ile Ala Leu Arg Ala Val Glu Ser Thr Asp Phe
    370                 375                 380
```

Met Thr Gly Arg Trp Ala Tyr Leu Pro Cys Asp Val Leu Ser Ser Cys
385 390 395 400

Ser Ser Arg Ile Ile Asn Glu Ile Pro Glu Val Ser Arg Val Val Tyr
405 410 415

Asp Ile Ser Asp Lys Pro Pro Ala Thr Ile Glu Trp Glu
420 425

<210> 228
<211> 1290
<212> DNA
<213> Chlamydia pneumoniae

<400> 228
ttgcagagtg caaggagaca tttgaacacc atatttattc tagattttgg atctcaatat 60
acttatgtat tagcaaagca agtgcggaag ttatttgtat attgcgaagt tcttccctgg 120
aatatctctg tgcaatgttt aaaagaaaga gcgcctttgg ggatcattct ctcaggaggt 180
cctcactctg tctatgaaaa caaggctcca catttagatc ctgaaatcta taaacttggc 240
attccaattc tagctatttg ctatggcatg cagcttatgg ctagagattt tggagggact 300
gtaagccctg gtgtaggaga atttggatat acgcccatcc atctgtatcc ttgtgagctc 360
ttcaaacaca tcgtcgactg cgaatctcta gacacagaga ttcggatgag ccatcgggat 420
catgttacga caattcctga aggatttaat gtaatcgcat ccacctcaca atgctcgatc 480
tcaggaatag aaaataccaa acaacggttg tacgggctgc aatttcatcc cgaggtttct 540
gactccactc caacgggaaa taagattcta gaaacttttg ttcaagagat ctgttctgct 600
cccacactat ggaatccctt gtatattcag caagaccttg taagtaaaat tcaagatacc 660
gttattgaag tatttgatga agtcgctcag tcattagacg tacaatggtt agctcaagga 720
accatctact cagatgttat tgagtcctca cgctctggac atgcctccga agtaataaaa 780
tcacatcata atgtaggggg gcttccaaaa aatcttaagc tgaagttagt cgagccctta 840
cgttatttat ttaaagatga agttcgaatt ttaggagaag ccctaggact ttctagctat 900
ctcttggaca ggcatccttt tcctggacct ggcttgacaa ttcgtgtgat tggagagatc 960
cttcctgaat atctagccat tttacgacgg gcggacctca tctttataga agagcttagg 1020
aaagcaaaac tctacgataa aataagccaa gcctttgctc tatttcttcc tataaaatca 1080
gtatctgtaa aaggagattg tagaagctat ggttatacca tagcattacg tgctgtagaa 1140
tctacagatt tcatgacagg acgatgggcc taccttccat gcgatgttct cagttcttgc 1200
tcatcgcgaa ttattaatga aatacccgag gtaagccgag tggtctatga tatttctgac 1260
aagccaccag caactataga atgggaatag 1290

<210> 229
<211> 265
<212> PRT
<213> Chlamydia pneumoniae

<400> 229
Met Asn Thr Tyr Thr Phe Ser Pro Thr Leu Gln Lys Ser Phe Ser Leu
1 5 10 15

Phe Leu Leu Glu Lys Leu Asp Ser Tyr Phe Phe Phe Gly Gly Thr Arg
20 25 30

Thr Gln Ile Leu Val Ile Thr Pro Thr Asn Ile Arg Leu Ala Ala Lys
35 40 45

Lys Arg Gly Cys Lys Val Ser Thr Ile Glu Lys Ile Ile Lys Ile Leu
50 55 60

Ser Phe Ile Leu Leu Pro Leu Val Ile Ile Ala Phe Ile Leu Arg Tyr
65 70 75 80

Phe Leu His Lys Lys Phe Asp Lys Gln Phe Leu Cys Ile Pro Lys Val
85 90 95

```
Ile Ser Asn Glu Asp Glu Ala Leu Leu Gly Ser Arg Pro Gln Ala Val
            100                 105                 110

Glu Lys Ala Val Arg Glu Ile Ser Pro Ala Phe Phe Ser Ile Pro Arg
        115                 120                 125

Lys Tyr Gln Leu Ile Arg Ile Asp Thr Pro Lys Asp Asp Ala Pro Ser
    130                 135                 140

Ile Leu Phe Pro Ile Gly Ile Glu Ile Ile Leu Lys Asp Leu Cys Ile
145                 150                 155                 160

Asp Thr Leu Lys Gln Ser Asn Leu Phe Leu Lys Arg Glu Met Asp Phe
                165                 170                 175

Leu Gly His Pro Glu Glu Lys Ala Leu Phe Asp Ser Ile Cys Ser Ile
            180                 185                 190

Glu Lys Asp Gln Glu Trp Met Ser Leu Glu Ser Lys Lys Leu Leu Ile
        195                 200                 205

Thr His Phe Leu Lys Tyr Leu Phe Val Ser Gly Ile Glu Gln Leu Asn
    210                 215                 220

Pro Gly Phe Asn Pro Glu Asn Gly Arg Gly Tyr Phe Ser Glu Ile Ser
225                 230                 235                 240

Thr Ala Lys Ile His Phe His Gln His Gly Arg Tyr Gly Pro Ile Arg
                245                 250                 255

Ser Ser Gly Pro Ile Met Lys Glu Ile
            260                 265
```

<210> 230
<211> 798
<212> DNA
<213> Chlamydia pneumoniae

<400> 230

```
atgaatacct ataccttctc tcctacactt cagaaaagct tcagcctatt tcttttagaa    60
aaattagact cttacttttt ctttggaggg actcgtacac aaatcttagt catcacacca   120
accaatatta gattagcagc taaaaaaaga gggtgtaagg tttctactat agaaaagata   180
atcaagatcc tctcttttat cctgctgccc ctagttatca ttgcctttat acttcgctat   240
ttcttacata agaaattcga taaacagttc ttgtgtatcc caaaagtcat ttctaacgaa   300
gacgaagctc ttcttggatc tagaccacaa gcagttgaaa aagcagttcg agaaatatct   360
ccagccttct tctctatacc aagaaaatac caacttatta gaatcgacac tcctaaagat   420
gacgctccct caatcctttt ccctataggc atagagatca ttctcaaaga tttatgtatt   480
gatacactca agcaatctaa tcttttcctt aaaagagaaa tggatttctt aggtcatcca   540
gaagaaaaag cattattcga ctcgatatgt tctatagaaa aagatcaaga atggatgagc   600
ttggaaagta aaaaactttt aatcacgcac ttcctaaagt atctctttgt ctctggaatc   660
gaacaactaa atccaggctt taacccagag aatgggcgtg ggtatttttc agaaataagt   720
acagcaaaga tccattttca tcagcacggt cgatatgggc caatccgttc ttcgggaccc   780
atcatgaagg aaatataa                                                 798
```

<210> 231
<211> 315
<212> PRT
<213> Chlamydia pneumoniae

<400> 231

Met Leu Val Glu Leu Glu Ala Leu Lys Arg Glu Phe Ala His Leu Lys
1 5 10 15

Asp Gln Lys Pro Thr Ser Asp Gln Glu Ile Thr Ser Leu Tyr Gln Cys
20 25 30

Leu Asp His Leu Glu Phe Val Leu Leu Gly Leu Gly Gln Asp Lys Phe
35 40 45

Leu Lys Ala Thr Glu Asp Glu Asp Val Leu Phe Glu Ser Gln Lys Ala
50 55 60

Ile Asp Ala Trp Asn Ala Leu Leu Thr Lys Ala Arg Asp Val Leu Gly
65 70 75 80

Leu Gly Asp Ile Gly Ala Ile Tyr Gln Thr Ile Glu Phe Leu Gly Ala
85 90 95

Tyr Leu Ser Lys Val Asn Arg Arg Ala Phe Cys Ile Ala Ser Glu Ile
100 105 110

His Phe Leu Lys Thr Ala Ile Arg Asp Leu Asn Ala Tyr Tyr Leu Leu
115 120 125

Asp Phe Arg Trp Pro Leu Cys Lys Ile Glu Glu Phe Val Asp Trp Gly
130 135 140

Asn Asp Cys Val Glu Ile Ala Lys Arg Lys Leu Cys Thr Phe Glu Lys
145 150 155 160

Glu Thr Lys Glu Leu Asn Glu Ser Leu Leu Arg Glu Glu His Ala Met
165 170 175

Glu Lys Cys Ser Ile Gln Asp Leu Gln Arg Lys Leu Ser Asp Ile Ile
180 185 190

Ile Glu Leu His Asp Val Ser Leu Phe Cys Phe Ser Lys Thr Pro Ser
195 200 205

Gln Glu Glu Tyr Gln Lys Asp Cys Leu Tyr Gln Ser Arg Leu Arg Tyr
210 215 220

Leu Leu Leu Leu Tyr Glu Tyr Thr Leu Leu Cys Lys Thr Ser Thr Asp
225 230 235 240

Phe Gln Glu Gln Ala Arg Ala Lys Glu Glu Phe Ile Arg Glu Lys Phe
245 250 255

Ser Leu Leu Glu Leu Glu Lys Gly Ile Lys Gln Thr Lys Glu Leu Glu
260 265 270

Phe Ala Ile Ala Lys Ser Lys Leu Glu Arg Gly Cys Leu Val Met Arg
275 280 285

Lys Tyr Glu Ala Ala Ala Lys His Ser Leu Asp Ser Met Phe Glu Glu
290 295 300

Glu Thr Val Lys Ser Pro Arg Lys Asp Thr Glu
305 310 315

<210> 232

<211> 948
<212> DNA
<213> Chlamydia pneumoniae

<400> 232
atgctagtag agttagaggc tcttaaaaga gagtttgcgc atttaaaaga ccagaagccg 60
acaagtgacc aagagatcac ttcactttat caatgtttgg atcatcttga attcgtttta 120
ctcgggctgg gccaggacaa atttttaaag gctacggaag atgaagatgt gctttttgag 180
tctcaaaaag caatcgatgc gtggaatgct ttattgacaa aagccagaga tgttttaggt 240
cttggggaca taggtgctat ctatcagact atagaattct tgggtgccta tttatcaaaa 300
gtgaatcgga gggctttttg tattgcttcg gagatacatt ttctaaaaac agcaatccga 360
gatttgaatg catattacct gttagatttt agatggcctc tttgcaagat agaagagttt 420
gtggattggg ggaatgattg tgttgaaata gcaaagagga agctatgcac ttttgaaaaa 480
gaaaccaagg agctcaatga gagccttctt agagaggagc atgcgatgga gaaatgctcg 540
attcaagatc tgcaaaggaa acttagcgac attattattg aattgcatga tgtttctctt 600
ttttgttttt ctaagactcc cagtcaagag gagtatcaaa aggattgttt gtatcaatca 660
cgattgaggt acttattgtt gctgtatgag tatacattgt tatgtaagac atccacagat 720
tttcaagagc aggctagggc taaagaggag ttcattaggg agaaattcag ccttctagag 780
ctcgaaaagg gaataaaaca aactaaagag cttgagtttg caattgctaa aagtaagtta 840
gaacggggct gtttagttat gaggaagtat gaagctgccg ctaaacatag tttagattct 900
atgttcgaag aagaaactgt gaagtcgccg cggaaagaca cagaataa 948

<210> 233
<211> 404
<212> PRT
<213> Chlamydia pneumoniae

<400> 233

Val Val Val Val Ala Leu Phe Ile Leu Gly Ile Phe Phe Leu Ser Gly
1 5 10 15

Ser Leu Ala Phe Leu Val His Thr Ser Cys Gly Val Leu Leu Gly Ala
20 25 30

Ala Leu Pro Ile Leu Cys Ile Gly Leu Val Leu Leu Ala Val Ala Leu
35 40 45

Ile Val Phe Leu Cys His Lys His Lys Thr Arg Gln Asp Leu Asp Tyr
50 55 60

Tyr Asp Gln Asp Leu Asp Ser Leu Val Ile His Lys Lys Glu Ile Pro
65 70 75 80

Asn Asp Ile Ser Glu Leu Arg Val Thr Phe Glu Lys Leu Gln Asn Leu
85 90 95

Phe Gln Phe His Thr Lys Asp Phe Ser Asp Leu Ser Gln Glu Leu Gln
100 105 110

Gly Lys Phe Ile Asn Cys Met Glu Lys Trp Leu Thr Leu Glu Asp Glu
115 120 125

Val Thr Lys Phe Leu Ile Val Arg Asp Arg Phe Leu Glu Thr Arg Arg
130 135 140

Asn Phe Thr Thr Phe Gly Glu Gln Val Lys Gly Ile Gln Ser Asn Ile
145 150 155 160

Phe Asp Leu His Glu Glu Lys Ser Ser Leu Tyr Leu Glu Leu Tyr Arg
165 170 175

```
Leu Arg Lys Asp Leu Gln Val Leu Leu Asn Phe Phe Leu Leu Pro Pro
            180                 185                 190

Gly Ile Leu Lys Val Asp Tyr Asp Glu Ile Glu Ala Ile Lys Gly Leu
        195                 200                 205

Phe Ile Arg Leu Thr Ser Arg Leu Asp Lys Leu Asp Val Lys Ala Gln
    210                 215                 220

Glu Arg Lys Lys Phe Ile Asn Glu Met Ser Arg Glu Phe Lys Glu Val
225                 230                 235                 240

Glu Lys Ala Phe Asp Ile Val Asp Arg Ala Thr Lys Lys Leu Met Asp
                245                 250                 255

Arg Ala Lys Lys Glu Ser Pro Ala Arg Leu Phe Met Gly Arg Thr Glu
            260                 265                 270

Ser Leu Leu Glu Met Lys Lys Asn Glu Glu Ala Leu Lys Asn Gln Gly
        275                 280                 285

Leu Asp Pro Glu Asn Leu Ser His Pro Glu Leu Phe Ser Pro Tyr Gln
    290                 295                 300

Gln Leu Leu Ile Leu Asn Tyr Leu Asn Ser Glu Ile Val Leu His His
305                 310                 315                 320

Tyr Glu Phe Leu Ile Ser Gly Thr Val Thr Ser Gly Leu Thr Leu Glu
                325                 330                 335

Glu Cys Glu Asn Arg Met Arg Ala Ala Ser Thr Gly Leu Asn Ala Leu
            340                 345                 350

Leu Val Arg Lys Leu Gln Phe Arg Gly Ala Ile Lys Ser Ala Tyr Phe
        355                 360                 365

Glu Lys Leu Thr Glu Ile Glu Lys Glu Leu Arg Ser Leu Gln Asp Val
    370                 375                 380

Ile Lys Ser Leu Glu Leu Glu Leu Ile His Lys Ile Lys Asp Ile Val
385                 390                 395                 400

Thr Glu Glu Thr


<210>   234
<211>   1215
<212>   DNA
<213>   Chlamydia pneumoniae

<400>   234
gtggtggttg tcgctttatt tatccttggg attttctttt tatctggttc tcttgcattc     60
cttgttcata cgtcttgcgg agttctttta ggagcggcgc ttcccatact ttgcataggt    120
cttgttttat tggctgtagc tcttattgtt ttcttatgtc acaaacacaa gactcgtcaa    180
gatttagatt attatgatca agatttagat tctttggtga ttcataagaa agagatcccc    240
aatgacatct ctgagttgcg ggtaacattt gaaaagttgc aaaatctgtt tcagttccat    300
acgaaagatt tctctgatct aagccaagag cttcagggta aatttatcaa ttgcatggag    360
aaatggctaa ctttagaaga cgaagtgact aaatttctta ttgttcgaga tagattttta    420
gaaaccagaa gaaattttac cacttttgga gaacaggtta aagggatcca aagcaatatt    480
tttgatttgc atgaggaaaa gtcttcatta tatttagaat tgtataggct taggaaagac    540
ctccaagttc tattaaattt ttttctgctc cccccaggta tactcaaggt agattatgat    600
```

```
gaaattgagg ctatcaaagg tctgtttata agattaacct ctagattaga taagcttgat   660
gtgaaagctc aggaacgtaa gaagttcatt aatgaaatga gtagggaatt taaagaagta   720
gagaaagctt ttgatattgt cgatagggca acaaaaaagc ttatggatag agccaagaaa   780
gaaagtccgg cacgtctttt catgggtaga actgagtctc tcttagaaat gaaaaaaaat   840
gaagaagccc ttaaaaatca ggggctagat cctgaaaatc tttcccatcc tgaacttttt   900
agtccgtatc aacagctttt aattttgaat tatttaaata gcgaaatagt tctgcatcat   960
tatgagttcc ttatttctgg aacagtaact tctggcctaa ctcttgaaga atgtgaaaat  1020
cgaatgaggg cggcttctac tgggttgaac gcccttctgg tgcgtaagct ccagttcaga  1080
ggtgctataa aatctgcgta ttttgaaaaa ctcacagaga ttgaaaaaga gttacgatca  1140
cttcaagacg taataaagtc attggaacta gaactgatcc ataagataaa agatatagtg  1200
acagaagaaa cttag                                                   1215
```

<210> 235
<211> 500
<212> PRT
<213> Chlamydia pneumoniae

<400> 235

```
Met Ser Pro Phe Lys Lys Ile Val Asn Arg Leu Leu Cys Tyr Ile Ser
1               5                   10                  15

Phe Gln Lys Glu Ser Arg Thr Leu Pro Ile Ile Ile Arg Glu Pro Arg
            20                  25                  30

Met Thr Thr Lys Ser Leu Gly Ser Phe Asn Ser Val Ile Ser Lys Asn
        35                  40                  45

Lys Ile His Phe Ile Ser Leu Gly Cys Ser Arg Asn Leu Val Asp Ser
    50                  55                  60

Glu Val Met Leu Gly Ile Leu Leu Lys Ala Gly Tyr Glu Ser Thr Asn
65                  70                  75                  80

Glu Ile Glu Asp Ala Asp Tyr Leu Ile Leu Asn Thr Cys Ala Phe Leu
                85                  90                  95

Lys Ser Ala Arg Asp Glu Ala Lys Asp Tyr Leu Asp His Leu Ile Asp
            100                 105                 110

Val Lys Lys Glu Asn Ala Lys Ile Ile Val Thr Gly Cys Met Thr Ser
        115                 120                 125

Asn His Lys Asp Glu Leu Lys Pro Trp Met Ser His Ile His Tyr Leu
    130                 135                 140

Leu Gly Ser Gly Asp Val Glu Asn Ile Leu Ser Ala Ile Glu Ser Arg
145                 150                 155                 160

Glu Ser Gly Glu Lys Ile Ser Ala Lys Ser Tyr Ile Glu Met Gly Glu
                165                 170                 175

Val Pro Arg Gln Leu Ser Thr Pro Lys His Tyr Ala Tyr Leu Lys Val
            180                 185                 190

Ala Glu Gly Cys Arg Lys Arg Cys Ala Phe Cys Ile Ile Pro Ser Ile
        195                 200                 205

Lys Gly Lys Leu Arg Ser Lys Pro Leu Asp Gln Ile Leu Lys Glu Phe
    210                 215                 220

Arg Ile Leu Val Asn Lys Ser Val Lys Glu Ile Ile Leu Ile Ala Gln
```

225 230 235 240

Asp Leu Gly Asp Tyr Gly Lys Asp Leu Ser Thr Asp Arg Ser Ser Gln
245 250 255

Leu Glu Ser Leu Leu His Glu Leu Leu Lys Glu Pro Gly Asp Tyr Trp
260 265 270

Leu Arg Met Leu Tyr Leu Tyr Pro Asp Glu Val Ser Asp Gly Ile Ile
275 280 285

Asp Leu Met Gln Ser Asn Pro Lys Leu Leu Pro Tyr Val Asp Ile Pro
290 295 300

Leu Gln His Ile Asn Asp Arg Ile Leu Lys Gln Met Arg Arg Thr Thr
305 310 315 320

Ser Arg Glu Gln Ile Leu Gly Phe Leu Glu Lys Leu Arg Ala Lys Val
325 330 335

Pro Gln Val Tyr Ile Arg Ser Ser Val Ile Val Gly Phe Pro Gly Glu
340 345 350

Thr Gln Glu Glu Phe Gln Glu Leu Ala Asp Phe Ile Gly Glu Gly Trp
355 360 365

Ile Asp Asn Leu Gly Ile Phe Leu Tyr Ser Gln Glu Ala Asn Thr Pro
370 375 380

Ala Ala Glu Leu Pro Asp Gln Ile Pro Glu Lys Val Lys Glu Ser Arg
385 390 395 400

Leu Lys Ile Leu Ser Gln Ile Gln Lys Arg Asn Val Asp Lys His Asn
405 410 415

Gln Lys Leu Ile Gly Glu Lys Ile Glu Ala Val Ile Asp Asn Tyr His
420 425 430

Pro Glu Thr Asn Leu Leu Leu Thr Ala Arg Phe Tyr Gly Gln Ala Pro
435 440 445

Glu Val Asp Pro Cys Ile Ile Val Asn Glu Ala Lys Leu Val Ser His
450 455 460

Phe Gly Glu Arg Cys Phe Ile Glu Ile Thr Gly Thr Ala Gly Tyr Asp
465 470 475 480

Leu Val Gly Arg Val Val Lys Lys Ser Gln Asn Gln Ala Leu Leu Lys
485 490 495

Thr Ser Lys Ala
500

<210> 236
<211> 1503
<212> DNA
<213> Chlamydia pneumoniae

<400> 236
atgagtcctt ttaagaaaat agtaaatcgc ttactatgct atatttcttt tcaaaaagaa 60
tcaagaactc tcccaatcat tattagagaa cctaggatga caacaaaaag tttaggatct 120

```
ttcaattcag ttatttccaa aaataaaatt cattttatta gtttgggatg ctctcggaac   180
cttgtagata gcgaagtcat gctaggcatt cttcttaagg caggttacga gtctactaat   240
gaaattgaag atgctgacta tttaatttta aatacctgtg cgtttttaaa aagtgctaga   300
gatgaagcta aagattatct agaccatcta attgatgtaa aaaaagagaa cgctaaaatt   360
attgtaactg gatgcatgac ttccaaccac aaagatgagc ttaaaccctg gatgtcacac   420
atccattacc tactaggttc tggggatgtt gagaatattc tttctgctat tgagtctcgt   480
gaatctggag aaaaaatctc tgcaaagagt tacattgaga tgggagaagt tccaagacag   540
ctttccacac caaaacacta tgcctattta aaagttgctg agggctgtag aaaacgttgt   600
gctttttgta ttattccttc cattaaagga aagctccgca gcaaacctct ggatcaaatt   660
cttaaagaat tccgcatcct tgtaaacaag agtgtgaaag agattatatt gatagctcaa   720
gacctaggag attatggaaa ggatctctct acagaccgca gttcgcagct agaatcacta   780
ttacatgagt tactgaaaga gcctggtgat tattggctgc ggatgttgta tttatatcct   840
gatgaagtga gtgatggcat tatagatctt atgcaatcta atcccaaact tcttccctat   900
gtagatattc ccttacagca cattaacgac cgtattttaa agcaaatgcg aagaacgact   960
tctagggagc aaatcctagg attcctagaa aaattacgtg ccaaggttcc tcaggtctat  1020
atccgttctt ctgttattgt gggtttcccc ggtgaaactc aggaagaatt ccaggagtta  1080
gctgatttta ttggtgaggg ttggattgat aatctcggaa ttttcttgta ctctcaagaa  1140
gcgaataccc cggcagcaga actccctgac cagataccag aaaaagttaa agaatcgagg  1200
ttgaaaattc tatctcaaat tcagaaacgc aatgtggata aacataatca gaagctcatt  1260
ggggaaaaaa tagaagcagt tattgataac tatcatcctg aaacgaatct tttactcact  1320
gcaaggttct atggacaagc tcctgaagtg gacccttgta ttattgtaaa tgaggcgaag  1380
cttgtttctc attttggaga aagatgcttt atagaaatca cagggactgc tggttacgac  1440
cttgtagggc gtgttgtaaa aaaatctcag aaccaagctt tgctaaaaac tagcaaagct  1500
tag                                                                1503
```

<210> 237
<211> 279
<212> PRT
<213> Chlamydia pneumoniae

<400> 237

```
Met Lys Asn Asn Ile Asn Asn Asn Glu Cys Tyr Phe Lys Leu Asp Ser
1               5                   10                  15

Thr Val Asp Gly Asp Leu Leu Ala Ala Asn Leu Lys Thr Phe Asp Thr
            20                  25                  30

Gln Ala Gln Gly Ile Ser Ser Thr Glu Thr Phe Ser Val Gln Gly Asn
        35                  40                  45

Ala Thr Phe Lys Asp Gln Val Ser Ala Thr Gly Leu Thr Ser Gly Thr
    50                  55                  60

Thr Tyr Asn Leu Asn Ala Gln Asn Phe Thr Ser Ser Gln Ile Ser Ile
65                  70                  75                  80

Asp Phe Lys Asn Asn Arg Leu Ser Asn Cys Ala Leu Pro Lys Glu Asp
                85                  90                  95

Cys Asp Pro Val Pro Ala Asn Tyr Val Arg Ser Pro Glu Tyr Phe Phe
            100                 105                 110

Cys Ser Lys Pro Leu Ile Gly Asp Phe Asp Phe Asn Ser Gly Glu Ser
        115                 120                 125

Tyr Leu Pro Leu Thr Gly Ser Glu Tyr Thr Leu Tyr Gln Ser Arg Asn
    130                 135                 140

Val Asn Ser Ile Phe Arg Phe Ile Gly Trp Lys Gln Ser Thr Arg Glu
145                 150                 155                 160
```

Leu Thr Val Gly Gly Asn Thr Ala Ile Gln Phe Leu Ala Ala Gly Thr
165 170 175

Tyr Ile Val Ser Phe Thr Val Gly Lys Arg Trp Gly Trp Asn Asn Gly
180 185 190

Trp Gly Gly Ala Ile Tyr Ile Asn Asn Gly Leu Gly Gln Val Gln Cys
195 200 205

Glu Ser Thr Ile Tyr Ser Gly Gly Gly Tyr Ala Thr Ile Gly Thr Leu
210 215 220

Gly Thr Ser Ile Tyr Arg Ala Ser Val Asp Val Ala Pro Asn Pro Asn
225 230 235 240

Asp Pro Asn Ala Ser Asp Arg Tyr Arg Ala Gly Ile Phe Tyr Leu Ser
245 250 255

Asn Gly Gly Ser Ser Ala Gly Ile Gly Asn Tyr Ser Phe Ser Leu Leu
260 265 270

Tyr Tyr Pro Asp Asp Arg Gly
275

<210> 238
<211> 840
<212> DNA
<213> Chlamydia pneumoniae

<400> 238
atgaaaaaca atattaataa taatgagtgc tattttaaat tagactcaac tgtagatggt 60
gatttgttag cagccaatct caagaccttt gatacacagg cccaaggaat ctcatcgact 120
gaaacatttt ctgttcaggg gaatgcaaca tttaaagatc aagtttcagc aactggatta 180
acttcaggaa ctacttataa tttaaatgca caaaacttta cttcctccca aatctctata 240
gattttaaaa ataatcgtct gagtaattgt gcattgccaa aagaagactg cgatccggtg 300
ccagcgaatt atgttcgttc tcccgaatat tttttctgtt ccaagcctct gatcggagat 360
tttgatttta actcagggga atcttatttg cctctgactg gttcggaata tactctatat 420
cagtcacgta atgtaaatag tatatttcgt tttataggat ggaagcaaag tacacgagaa 480
ttaactgtag ggggaaatac tgcgatacaa tttcttgcag caggaaccta tatcgtttca 540
tttactgttg gtaaacggtg ggatggaat aatggttggg gaggagccat ttatatcaat 600
aatggtttag gacaagtcca atgtgaaagc acgatttata gtggtggagg gtatgcaaca 660
ataggtacac tggggacctc aatatataga gcctctgtag atgtagctcc taatcctaat 720
gatccgaatg cttcggatcg ctatagagcg ggtattttct atctcagtaa cggtggttct 780
agtgcaggta tagggaatta ctcctttct cttctctatt atccggacga tagagggtag 840

<210> 239
<211> 419
<212> PRT
<213> Chlamydia pneumoniae

<400> 239
Met Lys Cys Ser Pro Leu Thr Leu Val Pro His Ile Phe Leu Lys Asn
1 5 10 15

Asp Cys Glu Cys His Arg Ser Cys Ser Leu Lys Ile Arg Thr Ile Ala
20 25 30

Arg Leu Ile Leu Gly Leu Val Leu Ala Leu Val Ser Ala Leu Ser Phe
35 40 45

Val Phe Leu Ala Ala Pro Ile Ser Tyr Ala Ile Gly Gly Thr Leu Ala

```
    50                  55                  60

Leu Ala Ala Ile Val Ile Leu Ile Ile Thr Leu Val Val Ala Leu Leu
65                  70                  75                  80

Ala Lys Ser Lys Val Leu Pro Ile Pro Asn Glu Leu Gln Lys Ile Ile
                85                  90                  95

Tyr Asn Arg Tyr Pro Lys Glu Val Phe Tyr Phe Val Lys Thr His Ser
            100                 105                 110

Leu Thr Val Asn Glu Leu Lys Ile Phe Ile Asn Cys Trp Lys Ser Gly
        115                 120                 125

Thr Asp Leu Pro Pro Asn Leu His Lys Lys Ala Glu Ala Phe Gly Ile
    130                 135                 140

Asp Ile Leu Lys Ser Ile Asp Leu Thr Leu Phe Pro Glu Phe Glu Glu
145                 150                 155                 160

Ile Leu Leu Gln Asn Cys Pro Leu Tyr Trp Leu Ser His Phe Ile Asp
                165                 170                 175

Lys Thr Glu Ser Val Ala Gly Glu Ile Gly Leu Asn Lys Thr Gln Lys
            180                 185                 190

Val Tyr Gly Leu Leu Gly Pro Leu Ala Phe His Lys Gly Tyr Thr Thr
        195                 200                 205

Ile Phe His Ser Tyr Thr Arg Pro Leu Leu Thr Leu Ile Ser Glu Ser
    210                 215                 220

Gln Tyr Lys Phe Leu Tyr Ser Lys Ala Ser Lys Asn Gln Trp Asp Ser
225                 230                 235                 240

Pro Ser Val Lys Lys Thr Cys Glu Glu Ile Phe Lys Glu Leu Pro His
                245                 250                 255

Asn Met Ile Phe Arg Lys Asp Val Gln Gly Ile Ser Gln Phe Leu Phe
            260                 265                 270

Leu Phe Phe Ser His Gly Ile Thr Trp Glu Gln Ala Gln Met Ile Gln
        275                 280                 285

Leu Ile Asn Pro Asp Asn Trp Lys Met Leu Cys Gln Phe Asp Lys Ala
    290                 295                 300

Gly Gly His Cys Ser Met Ala Thr Phe Gly Gly Phe Leu Asn Thr Glu
305                 310                 315                 320

Thr Asn Met Phe Asp Pro Val Ser Ser Asn Tyr Glu Pro Thr Val Asn
                325                 330                 335

Phe Met Thr Trp Lys Glu Leu Lys Val Leu Leu Glu Lys Val Lys Glu
            340                 345                 350

Ser Pro Met His Pro Ala Ser Ala Leu Val Gln Lys Ile Cys Val Asn
        355                 360                 365

Thr Thr His His Gln Asn Leu Leu Lys Arg Trp Gln Phe Val Arg Asn
    370                 375                 380
```

Thr Ser Ser Gln Trp Thr Ser Ser Leu Pro Gln Tyr Ala Phe His Ala
385 390 395 400

Gln Thr Tyr Lys Leu Glu Lys Lys Ile Glu Ser Ser Leu Pro Ile Arg
405 410 415

Ser Ser Leu

<210> 240
<211> 1260
<212> DNA
<213> Chlamydia pneumoniae

<400> 240
```
atgaagtgta gtcctttaac actagttccc catatatttt taaaaaatga ctgcgaatgt   60
catagatctt gttctttaaa aattaggaca attgcccgac tcattcttgg gcttgttcta  120
gctcttgtta gcgcactttc ttttgttttc cttgctgcgc cgattagcta tgctattgga  180
ggaactttag ctttagccgc tatcgtaatc ttgattataa cgctagtcgt agcactgcta  240
gctaaatcaa aggttctgcc catccccaac gaacttcaga agattattta caatcgctat  300
cctaaagaag tcttttattt cgtgaaaaca cactccctga ctgttaacga attaaaaata  360
tttattaatt gctggaaaag cggtacagac ctgcctccga atttacataa aaaagcagag  420
gctttcggga tcgatattct aaaatctata gatttaaccc tgtttccaga gttcgaagag  480
attcttcttc aaaactgccc gttatactgg ctctcccatt ttatagacaa aactgaatct  540
gttgctgggg aaatcggatt aaataaaaca caaaaagttt atggtttact tgggccctta  600
gcgtttcata aaggatatac aactattttc cactcttata cacgccctct actaacatta  660
atctcagaat cacagtataa gttcctatat agtaaagcgt ctaagaatca atgggattct  720
ccttctgtga aaaaaacctg cgaagaaata ttcaaggaac tcccccacaa tatgattttc  780
cggaaggatg ttcaaggaat ctcacaattc ttatttcttt tcttttctca tggtatcact  840
tgggaacagg ctcagatgat tcaacttata aatcctgata attggaaaat gttgtgtcag  900
tttgataaag caggaggcca ctgttccatg gcaacatttg gaggcttttt gaatactgaa  960
acaaatatgt tcgatccagt atcctctaac tatgaaccta cagtgaactt catgacgtgg 1020
aaagaattga aggttttact agagaaagta aaagaaagtc ctatgcaccc agcgagtgct 1080
cttgttcaga agatatgcgt aaatacaacg caccatcaaa atctgttaaa acgatggcaa 1140
tttgttcgta atacgagttc acaatggaca tcaagcttac ctcagtatgc tttccacgcc 1200
caaacctaca aactagagaa aaaaatagaa agcagtctcc ctatacgatc ttccctataa 1260
```

<210> 241
<211> 447
<212> PRT
<213> Chlamydia pneumoniae

<400> 241
Met Ser Asn Ile Thr Ser Pro Val Ile Gln Asn Asn Arg Ser Cys Asn
1 5 10 15

Tyr Tyr Phe Glu Leu Lys Asn Ser Thr Thr Ile His Ile Val Ile Ser
20 25 30

Ala Ile Leu Leu Cys Gly Ala Leu Ile Ala Phe Leu Cys Val Ala Ala
35 40 45

Pro Val Ser Tyr Ile Leu Ser Gly Ala Leu Leu Gly Leu Gly Leu Leu
50 55 60

Ile Ala Leu Ile Gly Val Ile Leu Gly Ile Lys Lys Ile Thr Pro Met
65 70 75 80

Ile Ser Ser Lys Glu Gln Val Phe Pro Gln Glu Leu Val Asn Arg Ile
85 90 95

Arg Ala His Tyr Pro Lys Phe Val Ser Asp Phe Val Ser Glu Ala Lys
100 105 110

Pro Asn Leu Lys Asp Leu Ile Ser Phe Ile Asp Leu Leu Asn Gln Leu
115 120 125

His Ser Glu Val Gly Ser Ser Thr Asn Tyr Asn Val Ser Glu Glu Leu
130 135 140

Gln Gln Lys Ile Asp Thr Phe Glu Gly Ile Ala Arg Leu Lys Asn Glu
145 150 155 160

Val Arg Thr Ala Ser Leu Lys Arg Leu Glu Ser Ala Ala Ser Ser Arg
165 170 175

Pro Leu Phe Pro Ser Leu Pro Lys Ile Leu Gln Lys Val Phe Pro Phe
180 185 190

Phe Trp Leu Gly Glu Phe Ile Ser Ala Gly Ser Lys Val Val Glu Leu
195 200 205

His Arg Val Lys Lys Ile Gly Gly Ser Leu Glu Glu Asp Leu Ser Asp
210 215 220

Tyr Ile Lys Pro Glu Met Leu Pro Thr Tyr Trp Leu Ile Pro Leu Asp
225 230 235 240

Phe Arg Pro Thr Asn Ser Ser Ile Leu Asn Leu His Thr Leu Val Leu
245 250 255

Ala Arg Val Leu Thr Arg Asp Val Phe Gln His Leu Lys Tyr Ala Ala
260 265 270

Leu Asn Gly Glu Trp Asn Leu Asn His Ser Asp Leu Asn Thr Met Lys
275 280 285

Gln Gln Leu Phe Ala Lys Tyr His Ala Ala Tyr Gln Ser Tyr Lys His
290 295 300

Leu Ser Gln Pro Ser Leu Gln Glu Asp Glu Phe Tyr Asn Leu Leu Leu
305 310 315 320

Cys Ile Phe Lys His Arg Tyr Ser Trp Lys Gln Met Ser Leu Ile Lys
325 330 335

Thr Val Pro Ala Asp Leu Trp Glu Asn Leu Cys Cys Leu Thr Leu Asp
340 345 350

His Thr Gly Arg Pro Gln Asp Met Glu Phe Ala Ser Leu Ile Gly Thr
355 360 365

Leu Tyr Thr Gln Gly Leu Ile His Lys Glu Ser Glu Ala Phe Leu Ser
370 375 380

Ser Leu Thr Leu Leu Ser Leu Asp Gln Phe Lys Thr Ile Arg Arg Gln
385 390 395 400

Ser Thr Asn Ile Ala Met Phe Leu Glu Asn Leu Ala Thr His Asn Ser
405 410 415

Thr Phe Arg Ser Leu Pro Pro Ile Thr Val His Pro Leu Lys Arg Ser
420 425 430

Val Phe Ser Gln Pro Glu Glu Asp Glu Ser Ser Leu Leu Ile Gly
435 440 445

<210> 242
<211> 1344
<212> DNA
<213> Chlamydia pneumoniae

<400> 242
atgagtaata taacctcgcc agttattcaa aataatcgct cttgtaatta ttattttgaa 60
ttaaagaatt caaccactat tcatattgtt atcagtgcca tcttactctg cggagctttg 120
atagctttct tgtgtgtagc agctcctgtt tcctatattc taagtggcgc attgttagga 180
ttaggattat taatagcctt gattggtgtg attttaggaa taaaaaaaat cacgcctatg 240
atttcatcaa aagaacaagt attcccccaa gaactcgtaa atagaatcag ggcgcactat 300
cctaaatttg tctctgattt tgtttcagaa gctaaaccaa atcttaaaga tctcataagt 360
tttattgatc ttctaaatca attgcactct gaagttggat catctacaaa ttacaacgta 420
tctgaagaac tacaacagaa aatagatacg ttcgagggta tcgcacgctt aaaaaatgaa 480
gtccgtactg cttctcttaa aagacttgaa agcgctgctt cttcccgtcc cctcttcccc 540
tctttaccaa aaatcttaca aaaggtattt ccatttttct ggttaggaga gtttatttct 600
gcaggcagca aggttgtaga gctccatcga gttaagaaaa ttggaggcag cctcgaagaa 660
gaccttagtg attatataaa accagagatg cttcctacct attggttgat tcctttagat 720
tttagaccaa caaattcctc tattctaaat ctacacacat tagttttagc tagagtctta 780
actcgtgatg tttttcaaca tcttaagtat gcagcattaa atggcgagtg gaacctgaat 840
catagtgatc taaatactat gaaacagcag ctctttgcta aatatcatgc ggcgtatcaa 900
tcctataaac atctatctca accctctctt caagaggatg aattctataa cctgctcttg 960
tgtattttta agcataggta ctcgtggaag cagatgtcct taataaaaac agtcccggct 1020
gatttatggg aaaacctctg ttgcttgact ttagaccata caggacgacc ccaagacatg 1080
gaatttgcct ctctaattgg tactctctac acacaaggcc taattcataa agaaagcgaa 1140
gcatttcttt cttcattgac actccttagt ttagatcagt ttaaaacgat ccgtcgtcag 1200
tcaaccaata tagcgatgtt ccttgagaat ttagcaactc ataattccac ctttagaagc 1260
ttaccaccta taacagtcca tccactcaag agaagcgtct tctcccaacc tgaagaagac 1320
gagtcctccc tgctgatagg ttag 1344

<210> 243
<211> 371
<212> PRT
<213> Chlamydia pneumoniae

<400> 243
Met Glu Met Met Ser Pro Phe Gln Gln Pro Glu Gln Cys His Phe Asp
1 5 10 15

Val Val Gly Ser Phe Leu Arg Pro Glu Ser Leu Thr Arg Ala Arg Ser
20 25 30

Asp Phe Glu Glu Gly Arg Ile Val Tyr Glu Gln Met Arg Val Val Glu
35 40 45

Asp Ala Ala Ile Arg Asn Leu Ile Lys Lys Gln Thr Glu Ala Gly Leu
50 55 60

Ile Phe Phe Thr Asp Gly Glu Phe Arg Arg Tyr Ser Trp Asp Phe Asp
65 70 75 80

Phe Met Trp Gly Phe His Gly Val Asp Arg Arg Arg Asp Ser Asn Asp
85 90 95

Pro Glu Ile Gly Val Tyr Leu Lys Asp Lys Ile Ser Val Ser Lys His

```
            100                 105                 110

Pro Phe Ile Glu His Phe Glu Phe Val Lys Thr Phe Glu Lys Gly Asn
        115                 120                 125

Ala Lys Ala Lys Gln Thr Ile Pro Ser Pro Ser Gln Phe Phe His Glu
    130                 135                 140

Met Ile Phe Ala Pro Asn Leu Lys Asn Thr Arg Lys Phe Tyr Pro Thr
145                 150                 155                 160

Asn Gln Glu Leu Ile Asp Asp Ile Val Phe Tyr Tyr Arg Gln Val Ile
                165                 170                 175

Gln Asp Leu Tyr Ala Ala Gly Cys Arg Asn Leu Gln Leu Asp Asp Cys
            180                 185                 190

Ala Trp Cys Arg Leu Leu Asp Ile Arg Ala Pro Ser Trp Tyr Gly Val
        195                 200                 205

Asp Ser His Asp Arg Leu Gln Glu Ile Leu Glu Gln Phe Leu Trp Ile
    210                 215                 220

His Asn Leu Val Met Lys Asp Arg Pro Glu Asp Leu Phe Val Ser Leu
225                 230                 235                 240

His Val Cys Arg Gly Asp Tyr Gln Ala Glu Phe Phe Ser Arg Arg Ala
                245                 250                 255

Tyr Asp Ser Ile Glu Glu Pro Leu Phe Ala Lys Thr Asp Val Asp Ser
            260                 265                 270

Tyr His Tyr Tyr Trp Ala Leu Asp Asp Lys Tyr Ser Gly Gly Ala Glu
        275                 280                 285

Pro Leu Ala Tyr Val Ser Gly Glu Lys His Val Cys Leu Gly Leu Ile
    290                 295                 300

Ser Ser Asn His Ser Cys Ile Glu Asp Arg Asp Ala Val Val Ser Arg
305                 310                 315                 320

Ile Tyr Glu Ala Ala Ser Tyr Ile Pro Leu Glu Arg Leu Ser Leu Ser
                325                 330                 335

Pro Gln Cys Gly Phe Ala Ser Cys Glu Gly Asp His Arg Met Thr Glu
            340                 345                 350

Glu Glu Gln Trp Lys Lys Ile Ala Phe Val Lys Glu Ile Ala Lys Glu
        355                 360                 365

Ile Trp Gly
    370

<210>   244
<211>   1116
<212>   DNA
<213>   Chlamydia pneumoniae

<400>   244
atggaaatga tgagcccatt ccaacaacct gagcaatgtc attttgatgt tgtgggaagt     60
ttcttacgtc ctgaaagtct tacacgagca cgctctgatt ttgaagaagg aagaattgtc    120
```

```
tatgagcaga tgcgagttgt cgaagatgct gctattcgta atctcataaa aaagcaaaca    180
gaagcaggtc ttatcttttt tactgatggg gaattccgta ggtatagttg ggatttcgac    240
tttatgtggg gattccatgg cgtggatcgt cgcagggact ctaatgaccc tgaaattgga    300
gtgtatctta aagataaaat ctccgtatca aaacatccgt ttatagaaca tttcgagttt    360
gtcaaaactt ttgagaaggg aaatgcaaaa gcaaaacaaa cgattccttc tccatcacaa    420
tttttccatg agatgatttt tgctcctaat ctgaaaaata ctcggaagtt ttatcctacg    480
aatcaagagc taattgatga tattgtcttt tattatcgcc aagtcatcca agatctttat    540
gctgcaggtt gtcgtaattt gcagttggac gattgtgctt ggtgtcgcct cttggatata    600
cgagcgcctt cttggtatgg tgttgattct catgacaggt tgcaggaaat tttagaacag    660
tttttatgga tccataattt agtgatgaag gatagacccg aggatctttt tgtaagtctg    720
catgtctgtc gtggtgatta tcaggccgag tttttctcta gacgagctta tgattctata    780
gaggagcctt tatttgctaa gaccgatgtg gatagttatc actattattg ggctcttgat    840
gataagtatt caggaggtgc tgagccttta gcttacgtct ctggagagaa acacgtctgc    900
ttgggattga tctccagcaa ccattcttgt attgaagatc gagatgctgt ggtttctcgt    960
atttatgaag ctgcgagcta cattccctta gagagacttt ctttgagccc gcaatgtggg   1020
tttgcttctt gtgagggaga ccatagaatg actgaagaag aacagtggaa gaagatcgcc   1080
tttgtgaaag agattgctaa agagatctgg ggataa                              1116
```

<210> 245
<211> 297
<212> PRT
<213> Chlamydia pneumoniae

<400> 245

```
Val Trp Leu Arg Phe Leu Leu Leu Val Ser Tyr Asp Glu Lys Glu Lys
1               5                   10                  15

Asp Val Val Val Val Cys Asn His Ser Glu Pro Asn Ile Leu Gly Leu
            20                  25                  30

Pro Pro Glu Ala Val Ser Gln Leu Ile Glu Glu Leu Ser Asp Glu Gly
        35                  40                  45

Tyr Ser Tyr Leu Asn Val Val Arg Cys Asp Leu Ser Gly Glu Thr Thr
    50                  55                  60

Val Gln Gln Arg Leu Leu Leu Asn Ala Asp Glu Gly Arg Ser Met Thr
65                  70                  75                  80

Val Val Ile Ser Glu Leu Pro Glu Gly His Pro Asp Ile Arg Asn Leu
                85                  90                  95

Gln Leu Ala Ser Glu Arg Ile Phe Val Ser Arg Glu Lys Glu Ala Ala
            100                 105                 110

Asp Ala Tyr Ala Ser Gly Cys Lys Val Val Ala Phe Asp Asp Glu His
        115                 120                 125

Leu Pro Trp Val Ser Ser His Ile Ala Tyr Ala Glu Glu Ile Arg Glu
    130                 135                 140

Lys Gln Glu Gln Thr Met Gln Gly Ser Leu Thr Glu Glu Gln Leu Gly
145                 150                 155                 160

Ala Leu Leu Cys Asn Thr Val Ser Thr Glu Lys Asn Leu Ala Phe Ala
                165                 170                 175

Leu Asp Ala Val Ile Lys Gln Ser Val Trp Arg Phe Arg Asn Pro Asp
            180                 185                 190

Leu Phe Ala Tyr Glu Arg Glu Ala Leu Glu Ala Ser Val Thr Asp Ala
```

```
        195                 200                 205

Leu Val Ser Tyr Val Ser Asn Leu Asp Met Ile Pro Tyr Thr Ser Ser
    210                 215                 220

Gln Gly Ile Val Ile Glu Asp Ser Ser Ile Val Arg Thr Ser Gln Glu
225                 230                 235                 240

His Thr Leu Ile Val Asn Cys Ala Ala Phe Asp Lys Leu Ala Ser Gln
                245                 250                 255

Ile Glu Phe Leu Cys Pro Ser Asp Val Leu Pro Ile Ser Gly Lys Asp
            260                 265                 270

Pro Leu Ile Ser Asp Asp Glu Asp Glu Glu Leu Asn Pro Lys Val Ser
        275                 280                 285

Ser Ala Ala Asp Ser Lys Asp Lys Thr
    290                 295

<210>   246
<211>   894
<212>   DNA
<213>   Chlamydia pneumoniae

<400>   246
gtgtggctgc gctttttact tttagtgtcc tatgatgaga aggagaaaga cgtagttgtc    60
gtttgtaatc attctgaacc taatatcctc ggcctgcctc ctgaagcagt ctctcagctt   120
attgaagagc ttagcgatga aggctatagc tatctgaatg tagtgcgttg tgatctctcc   180
ggggagacta cggttcaaca acgtctgcta ttgaatgccg atgaagggag atctatgacg   240
gtggtgatct cagagcttcc tgaagggcac cccgatattc ggaatttgca gttggcatcc   300
gaaagaattt ttgtttctcg tgaaaaagaa gctgctgatg cctatgcttc aggatgtaaa   360
gtggtcgctt tcgatgatga gcatctccct tgggtctcca gtcatattgc ctacgcggag   420
gagatcagag agaaacaaga acaaacaatg caagggtctt taactgaaga gcagttagga   480
gcactcctct gcaacacagt ctccacagag aaaaatctag cctttgctct agacgccgtg   540
ataaaacagt ctgtgtggag attccgcaat ccggatcttt ttgcttatga gagagaagct   600
ctagaggctt cagtaacaga tgctttagta tcttacgttt caaatttaga catgataccg   660
tacacaagtt ctcagggcat agtcatagaa gatagtagta tcgtccgtac ctctcaagag   720
catacactca ttgtgaactg tgcagcattc gataagttag cgagccaaat agagttctta   780
tgccccagtg acgtgttgcc catttctggt aaagaccctt tgatttctga tgatgaggat   840
gaggaactga atcctaaagt ttcatctgct gcagactcta aagataaaac ctag         894

<210>   247
<211>   402
<212>   PRT
<213>   Chlamydia pneumoniae

<400>   247
Met Thr His Cys Leu His Gly Trp Phe Ser Val Val Arg His His Phe
1               5                   10                  15

Val Gln Ala Phe Asn Phe Ser Arg Pro Leu Tyr Ser Arg Ile Thr His
            20                  25                  30

Phe Ala Leu Gly Val Ile Lys Ala Ile Pro Ile Val Gly His Leu Val
        35                  40                  45

Met Gly Val Asp Trp Leu Ile Ser His Cys Phe Glu Arg Gly Val Ser
    50                  55                  60

His Pro Gly Phe Pro Ser Asp Ile Ala Pro Ile Leu Lys Val Glu Lys
```

```
65                  70                  75                  80

Ile Ala Gly Arg Asp His Ile Ser Arg Ile Glu Asn Gln Leu Lys Ser
                85                  90                  95

Leu Arg Lys Thr Ile Glu Val Glu Asp Leu Asp Lys Val His Gly Gln
            100                 105                 110

Tyr Gln Glu Asn Pro Tyr Ala Asp Met Ala Ser Ser Glu Val Leu Lys
        115                 120                 125

Leu Asp Lys Gly Val His Val Ser Glu Leu Gly Lys Ala Phe Ser Arg
    130                 135                 140

Val Arg Asn Arg Ile Thr Arg Ser Tyr Ser Tyr Ala Pro Thr Pro Gln
145                 150                 155                 160

Leu Asp Ser Ile Ala Ile Val Gly Ile Asp Leu Val Ser Pro Glu Glu
                165                 170                 175

Gln Glu Asn Leu Val Arg Leu Ala Asn Glu Val Ile Gln Leu Tyr Pro
            180                 185                 190

Lys Ser Lys Thr Thr Leu Tyr Leu Leu Ile Asp Phe Asn Lys Glu Trp
        195                 200                 205

Val Gly Asp Ile Ser Ser Asp Lys Glu Lys Gln Leu Arg Ser Leu Gly
    210                 215                 220

Leu His Ser Glu Val Gln Cys Leu Ser Val Leu Glu Pro Gln Gly Ala
225                 230                 235                 240

Glu Gly Glu Asp Thr Lys His Phe Asp Leu Met Val Gly Cys Tyr Gly
                245                 250                 255

Lys Asp Ser Tyr Leu Arg Glu Gly Lys Ile Leu Gln Gln Ala Leu Gly
            260                 265                 270

Thr Ser Leu Gly Thr Val Pro Trp Val Asn Val Met His Thr Leu Pro
        275                 280                 285

Ser Arg Tyr Arg Ser Arg Leu Ser Leu Pro Ile Asn Thr Glu Lys Asp
    290                 295                 300

Lys Thr Glu Leu Tyr Lys Glu Ile Ser Arg Thr His His Gln Leu His
305                 310                 315                 320

Thr Leu Gly Met Gly Leu Gly Ala Gln Asp Ser Gly Leu Leu Leu Asp
                325                 330                 335

Arg Gln Arg Leu His Ala Pro Leu Ser Gln Gly Ser His Cys His Ser
            340                 345                 350

Tyr Leu Ala Asp Leu Thr His Glu Glu Leu Lys Ile Leu Leu Phe Ser
        355                 360                 365

Ala Phe Val Asp Ala Lys Asn Ile Ser Lys Lys Glu Leu Arg Glu Val
    370                 375                 380

Ser Leu Asn Phe Ala Asn Asp Thr Ser Val Glu Cys Gly Cys Ala Phe
385                 390                 395                 400
```

Tyr Phe

<210> 248
<211> 1209
<212> DNA
<213> Chlamydia pneumoniae

<400> 248
atgactcatt gcttacatgg ttggttttct gtagttcgtc atcactttgt gcaggcgttt 60
aatttctcac gtcctttata ttctcgaatt acccacttcg ctttaggggt gattaaggcc 120
atccccattg tagggcatct tgttatggga gtcgattggt tgatctctca ttgcttcgag 180
aggggagtct cacaccctgg gttcccttca gatattgctc ctatactgaa agtagaaaag 240
atcgcgggcc gagatcatat ttctagaatc gaaaatcagc taaagagcct taggaaaact 300
atcgaggttg aagatctaga taaagtccac gggcaatatc aagagaatcc ttatgcagat 360
atggcctcta gtgaggttct taaactcgat aagggagttc atgttagcga gcttggcaaa 420
gcctttteta gagttcgcaa tcgcatcacc agatcctata gttatgcccc tactcctcag 480
ttggactcta tagctattgt tggtatagat ctcgtcagtc ctgaagaaca agagaattta 540
gtacgcttgg cgaatgaggt cattcaactc tatcccaaat caaagacaac tctatatctt 600
cttatcgatt ttaataagga gtgggtaggg gatatctcct ctgataagga aaaacagctc 660
cgttctctag gtctacattc tgaagttcag tgtctttccg tcttggaacc tcagggtgcc 720
gagggcgaag atacgaaaca ctttgacctt atggtcggct gttatgggaa ggattcttac 780
ttaagggagg gtaaaatttt acagcaggcc ctagggactt cgttaggtac tgttccctgg 840
gtgaatgtta tgcacacatt gccatctagg tatagatctc ggctttcctt acctataaat 900
accgaaaagg ataagacaga gctttataaa gagatttctc gtacacacca tcagttgcat 960
actttgggaa tgggacttgg agcccaggat tcaggattgc tcttagaccg gcaacgactc 1020
catgctcctt tatctcaagg gtctcactgc cattcctatc ttgcagatct cacccatgaa 1080
gagctgaaaa ttttgttatt ttcagcattt gtggatgcta agaacataag taagaaagag 1140
cttcgtgagg tatctctaaa ttttgctaac gatacttccg tagagtgtgg ctgcgctttt 1200
tacttttag 1209

<210> 249
<211> 582
<212> PRT
<213> Chlamydia pneumoniae

<400> 249
Met Ala Ser Cys Leu Ser Ala Trp Phe Ser Ile Val Arg Glu His Phe
1 5 10 15

Tyr Arg Ala Phe Asp Phe Ser Leu Pro Phe Cys Ala Arg Ile Thr Glu
20 25 30

Phe Val Leu Gly Val Ile Lys Gly Ile Pro Val Val Gly His Ile Ile
35 40 45

Val Gly Ile Glu Trp Leu Val Ser Arg Tyr Leu Glu Ser Phe Val Thr
50 55 60

Lys Pro Thr Phe Val Ser Asp Val Val Ser Leu Leu Lys Thr Glu Lys
65 70 75 80

Val Ala Gly Arg Asp His Ile Ala Arg Val Val Glu Thr Leu Lys Arg
85 90 95

Gln Arg Val Ala Val Ala Pro Glu Asp Glu Asp Lys Val His Gly Lys
100 105 110

Ile Pro Val His Pro Phe Gly Gly Ile Gln Pro Val Glu Val Leu Thr
115 120 125

```
Leu Tyr Pro Glu Val Gln Asp Ala Thr Leu Gly Leu Ala Phe Ser Lys
    130                 135                 140

Ile Arg Asn Arg Val Arg Gln Ala Tyr Leu Gln Ala Pro Arg Pro Lys
145                 150                 155                 160

Leu Gln Lys Ile Tyr Ile Ile Gly Asn Asp Met Asn Pro Phe Glu Val
                165                 170                 175

Asp Asp Phe Leu His Leu Ala Arg Leu Cys Asn Glu Thr Gln Arg Leu
            180                 185                 190

Tyr Pro Asp Ala Thr Ile Ser Leu Tyr Leu Thr Ala Ser Gly Gly Arg
        195                 200                 205

Asn Ala Met Asp Lys Lys Asn Arg Lys Leu Leu Ser Asp Cys Glu Leu
    210                 215                 220

Asn Pro Lys Ile Ala Cys Leu Asp Phe Asn Gln Gly Asp Val Val Lys
225                 230                 235                 240

Gln Ala Thr Cys Asp Cys Trp Met Val Tyr His Gly Glu Asn Asp Gln
                245                 250                 255

Gly Thr Leu Asn Gln Ile Gln Glu Glu Leu Glu Lys Ser Gly Glu Glu
            260                 265                 270

Thr Pro Trp Ile His Val Gly Gln Lys Pro Leu Ser Gln Ser Leu Trp
        275                 280                 285

Asp Phe Ser Pro Phe Ser Ser Leu Glu Met Lys Gly Asp Lys Glu Lys
    290                 295                 300

Ala Leu Glu Tyr Ser Glu Leu Glu Lys Glu Gln Leu Tyr Ser Arg Leu
305                 310                 315                 320

Val Tyr Val Gly Glu Arg Ser Ser Val Leu Ser Leu Gly Phe Gly Asp
                325                 330                 335

Ser Arg Ser Gly Ile Leu Met Asp Pro Lys Arg Val His Ala Pro Leu
            340                 345                 350

Ser Glu Gly His Tyr Cys His Ser Tyr Leu Ala Asp Leu Glu Asn Pro
        355                 360                 365

Gly Leu Gln Lys Thr Ile Leu Ala Ala Phe Leu Asn Pro Lys Glu Leu
    370                 375                 380

Ser Ser Thr Ile Leu Gln Pro Ile Ser Leu Asn Leu Ile Leu Asn Ser
385                 390                 395                 400

Lys Thr Tyr Leu Arg Gln His Phe Gly Phe Phe Glu Arg Met Ser Arg
                405                 410                 415

Ser Asp Arg Asn Val Val Val Val Val Cys Asp Ser Trp Trp Gly Thr
            420                 425                 430

Asp Trp Lys Glu Glu Pro Ser Phe Gln His Phe Ile Met Glu Leu Glu
        435                 440                 445
```

```
Cys Arg Gly Tyr Ser His Phe Asn Ile Phe Ala Phe Arg Ser Asn Ser
    450                 455                 460

Met Cys Val Glu Glu Arg Arg Ile Leu Asn Glu Ser Ser Gln Glu Lys
465                 470                 475                 480

Ala Phe Thr Met Ile Phe Cys Glu Asp Ser Val Ser Gln Gly Asp Ile
                485                 490                 495

Arg Cys Leu His Leu Ala Ser Glu Gly Met Leu Cys Gly Lys Glu Cys
            500                 505                 510

Tyr Ala Val Asp Val Tyr Thr Ser Gly Cys Ala Asn Phe Met Met Glu
        515                 520                 525

Glu Val Leu Thr Leu Glu Arg Glu Ser Asn Leu Trp Asn Arg Lys His
    530                 535                 540

Gly Leu Trp Lys Arg Glu Val Arg Lys Gln Lys Gln Glu Ala Ala Leu
545                 550                 555                 560

Asp Gln Asp Glu Ser Glu Ile Tyr Val Cys Asn Gln Leu Thr Ala Gln
                565                 570                 575

Gln Asn Phe Ala Cys Ser
            580

<210>   250
<211>   1749
<212>   DNA
<213>   Chlamydia pneumoniae

<400>   250
atggcttctt gtttatctgc ctggttttct atagttcgtg agcactttta tcgagccttt   60
gatttttctt tgccgttttg tgctcgtatt acggaatttg tattaggggt catcaagggg   120
atccctgttg tgggtcacat tattgttggg atagagtggc tcgtttctag gtatttagag   180
agtttcgtga ccaagccgac atttgtctct gatgtggtga gtcttctgaa aacagagaaa   240
gttgctggtc gcgatcacat tgctcgtgta gtggagactt tgaagaggca gagagtcgct   300
gtggctcctg aagatgagga taaggtccat gggaagattc ctgtgcatcc tttcgggga   360
atccaacctg tagaagttct cactctctat cccgaagttc aagatgcaac gttagggctt   420
gccttctcta aaattcgtaa tcgtgtaaga caggcgtatt tgcaagctcc acggccaaaa   480
ctgcagaaga tttacatcat aggaaacgat atgaatcctt ttgaagttga cgacttcttg   540
catctagccc gtctctgtaa tgaaactcaa agactctatc ctgacgctac gatttctcta   600
tatctaacag cttctggtgg tcgcaatgct atggacaaaa agaatcggaa gttacttagt   660
gattgcgaac taaaccccaa gattgcttgt ttggacttta atcagggtga tgtagtcaaa   720
caagcaactt gtgactgttg gatggtgtat catggggaga atgatcaagg tacgttgaat   780
cagattcagg aagagttaga aaagtcaggg gaggaaaccc cttggattca tgtggggcaa   840
aagcctcttt cacaatcctt gtgggatttc tctccatttt catctttgga gatgaaggga   900
gataaagaga aagctctaga gtactctgaa ttagaaaaag aacagctata ttctcgattg   960
gtatacgtag gagagcgctc ttcggttctt agtttggggt ttggagatag tcggtcaggg   1020
atcttgatgg acccaaaacg ggtgcatgct cccttatctg aagggcatta ttgtcattcc   1080
taccttgcag acttagaaaa tcccgggtta caaaaaacaa ttttagcggc atttctgaat   1140
cctaaggagt tgagcagtac catactgcaa cctatatctc taaatcttat cttaaatagc   1200
aaaacttact taaggcagca ctttggcttt tttgagagga tgagcagaag tgatcgcaat   1260
gtggttgtcg ttgtatgtga ttcttggtgg ggtaccgact ggaaggagga gccaagcttc   1320
caacacttta ttatggagct agagtgtcga gggtattcgc acttcaatat ttttgccttt   1380
agatctaata gcatgtgtgt agaagaacgt aggatcttaa atgaaagttc tcaagagaaa   1440
gcctttacca tgattttctg tgaggattca gtatctcaag gagatatccg ctgtttgcat   1500
ttggcgtctg aaggaatgct ttgtggtaaa gagtgctatg ctgtcgatgt ctatacgtca   1560
ggatgcgcga actttatgat ggaagaagtc ttaactttgg agcgagaatc taatctgtgg   1620
aatagaaagc atggtctttg gaaaagagaa gttagaaaac agaaacaaga agctgctttg   1680
```

```
gatcaagacg agagcgagat ttacgtttgt aatcagctga cggcgcaaca gaacttcgct   1740
tgttcttga                                                           1749

<210>  251
<211>  695
<212>  PRT
<213>  Chlamydia pneumoniae

<400>  251
Leu Phe Val Ser Asn Phe Ile Phe Phe Val Val Met Pro Ile Pro Tyr
1               5                   10                  15

Ile Ser Ser Trp Ile Ser Thr Val Arg Gln His Phe Val Lys Ala Phe
            20                  25                  30

Asp Phe Ser Arg Pro Phe Cys Ser Arg Val Thr Asn Phe Ala Leu Gly
        35                  40                  45

Val Ile Lys Ala Ile Pro Ile Val Gly His Ile Val Met Gly Met Glu
    50                  55                  60

Trp Leu Val Ser Ser Cys Val Ala Gly Ile Ile Thr Arg Ser Ser Phe
65                  70                  75                  80

Thr Ser Asp Val Val Gln Ile Val Lys Thr Glu Lys Ala Leu Gly Arg
                85                  90                  95

Asp His Ile Ser Arg Val Ala Glu Ile Leu Gln Arg Glu Arg Gly Thr
            100                 105                 110

Ile Thr Pro Glu Asn Gln Asp Lys Val His Gly Lys Phe Pro Val Cys
        115                 120                 125

Pro Phe Gly Arg Leu Lys Ser Glu Glu Thr Leu Lys Leu Lys Pro Gly
    130                 135                 140

Glu Arg Glu Gly Thr Leu Asp Thr Val Phe Ser Pro Ile Arg Thr Arg
145                 150                 155                 160

Val Thr Arg Ala Tyr Leu Gln Ala Pro Arg Pro Glu Ile Arg Thr Ile
                165                 170                 175

Ser Ile Val Gly Ser Lys Leu Lys Thr Pro Gln Asp Phe Ser Gln Phe
            180                 185                 190

Val Ser Leu Ala Asn Glu Thr Gln Arg Leu His Pro Glu Ala Leu Val
        195                 200                 205

Cys Leu Tyr Leu Thr Gly Leu Asn Arg Glu Ser Gln Met Cys Asp Thr
    210                 215                 220

Thr Thr Ala Glu Lys Lys Gln Tyr Leu His Asn Ser Gly Leu Asp Ser
225                 230                 235                 240

Arg Ile Gln Cys Lys Asp Ser Lys Glu Asp Asp Ala Gly Ser Pro Glu
                245                 250                 255

Asn Pro Glu Leu Trp Ile Gly Tyr Tyr Ser Arg Glu Gln Gln His Asn
            260                 265                 270

Ile Asp Gly Gln Tyr Ile Gln Gln Cys Leu Gly Lys Ser Ala Asp Pro
```

```
        275                 280                 285

Ile Pro Trp Ile His Val Thr Glu Asp Thr Lys Asp Phe Tyr Tyr Pro
    290                 295                 300

Pro Asn Phe Thr Ser Tyr Ser His Thr Arg Gln Ser Thr Asp Pro Thr
305                 310                 315                 320

Ser Pro Pro Arg Leu Pro Glu Ser Glu Gly Asp Lys Asp Ser Leu Tyr
                325                 330                 335

Gly Gln Leu Ser Arg Ser Tyr His His Glu Tyr Met Leu Gly Leu Gly
            340                 345                 350

Leu Lys Pro Glu Asp Ala Gly Leu Leu Met Asp Pro Asp Arg Ile Tyr
        355                 360                 365

Ala Pro Leu Ser Gln Gly His Tyr Cys His Ser Tyr Leu Ala Asp Ile
    370                 375                 380

Glu Asn Glu Asp Leu Arg Thr Leu Val Leu Ser Pro Phe Leu Asp Pro
385                 390                 395                 400

Gly Asn Leu Ser Ser Glu Asp Leu Arg Pro Val Ala Phe Asn Ile Ala
                405                 410                 415

Arg Leu Pro Leu Glu Leu Asp Ser Leu Phe Phe Arg Leu Val Ala Gly
            420                 425                 430

Gln Gln Glu Gly Arg Asn Ile Val Thr Leu Ala His Gly Thr Pro Arg
        435                 440                 445

Pro Glu Asp Leu Asp Pro Asp Ser Met Asn Ile Leu Thr Arg Arg Leu
    450                 455                 460

Gln Met Ser Gly Tyr Ser Tyr Leu Asn Ile Phe Ser Tyr Lys Ser Arg
465                 470                 475                 480

Lys Met Ile Val Lys Glu Arg Gln Phe Phe Gly Asp Arg Ser Glu Gly
                485                 490                 495

Lys Ser Phe Thr Leu Ile Leu Phe Glu Asp Pro Ile Ser Ala Ala Asp
            500                 505                 510

Phe Arg Cys Leu Gln Leu Ala Ala Glu Gly Met Val Ala Lys Asp Leu
        515                 520                 525

Pro Ser Val Ala Asp Ile Cys Ala Ser Gly Cys Ser Cys Ile Gln Phe
    530                 535                 540

Ser Glu Met Gln Ser Pro Gln Ala Ile Glu Tyr Arg Gln Trp Glu Ala
545                 550                 555                 560

Arg Val Glu Asp Glu Ala Gly Glu Glu Ala Arg Glu Pro Val Ile Tyr
                565                 570                 575

Ser Gln Asp Gln Leu Ser Ser Met Leu Thr Thr Gln Gln Asn Phe Val
            580                 585                 590

Phe Ser Leu Asp Ala Val Val Lys Gln Ala Ile Trp Arg Phe Arg Ser
        595                 600                 605
```

```
Lys Gly Leu Leu Thr Met Glu Arg Lys Ala Leu Gly Glu Glu Phe Leu
    610                 615                 620

Thr Ala Ile Phe Ser Tyr Leu Gly Ser Gln Glu Arg Asn Glu Asn Met
625                 630                 635                 640

Gly Lys Arg Thr Thr Glu Glu His Glu Val Val Ile Ser Phe Glu Glu
                645                 650                 655

Leu Asp Arg Met Val Gln Val Leu Pro Ala Glu Val Pro Ala Asp Ser
            660                 665                 670

Gly Asn Asp Pro Thr Arg Pro Val Pro Asn Pro Asp Ser Asn Pro Asp
        675                 680                 685

Ser Ser Gln Asn Glu Gly Ser
    690                 695

<210>   252
<211>   2088
<212>   DNA
<213>   Chlamydia pneumoniae

<400>   252
ttgtttgttt ctaattttat ttttttgtt gttatgccaa ttccctatat ttcttcttgg    60
atttctaccg ttcgacagca ttttgttaag gcgtttgatt tctctcgtcc cttttgttct   120
agggttacga attttgcttt aggggtcatc aaggccatcc ctattgtagg acatattgtc   180
atggggatgg agtggttagt ttcttcctgt gttgccggga ttattactag gtcctccttt   240
acctcagatg tcgttcagat tgtaaagact gagaaggcgt taggtcgaga tcatatatct   300
cgagtggcgg agatattgca aagagaaagg gggaccataa ctcctgagaa tcaagataag   360
gtgcatggga agtttcctgt ctgtcctttt ggtcgtttaa aatccgagga aactttaaaa   420
cttaagccgg gagaaagaga gggaacttta gatactgtat tttctccgat tcgcacgcgc   480
gtgactcgtg cgtacttaca ggccccccga cccgaaatac gtacgatttc tattgtgggt   540
tcgaaactta aaactcctca agatttctcg caatttgtga gtctcgcgaa tgaaacgcag   600
agactgcatc ctgaagcgtt agtttgtctg tatttgacag gcttgaatcg cgaatctcag   660
atgtgcgata caactactgc agagaagaag cagtacctac ataactcagg tctcgactct   720
agaatccagt gcaaagacag taaagaagac gacgctggct ctcctgaaaa tcccgaactt   780
tggattggct attattcacg agagcaacag cataatatag acgggcagta tattcagcag   840
tgtctaggga agagtgcaga tccaattcct tggattcatg ttactgaaga cacaaaggat   900
ttttattacc caccaaactt tacttcatac tcacatacaa gacaatctac agacccaaca   960
tcgccaccaa gactccctga aagtgagggg gataaggatt ccttgtacgg acaactgagt  1020
cgatcgtatc accatgagta tatgcttggt ttgggattaa aaccagagga tgcaggactc  1080
ctgatggacc cggatagaat ctatgctcct ctatcccaag ggcattattg tcattcctac  1140
cttgcggata tagaaaatga ggatctacga actttagtcc tttcgccttt cctagatcct  1200
ggcaatctta gtagcgagga tcttcgtcct gtagcattca atatcgctag attgccatta  1260
gaattggact cgttattttt ccgccttgtt gcgggtcagc aagaagggag aaacatagtt  1320
acccttgccc acggaactcc tcgtccagaa gatcttgatc ctgactcaat gaacattctg  1380
accagaagat tacaaatgtc tggatatagc tatttgaaca ttttctccta taaatcacgg  1440
aaaatgattg taaaagaacg tcagttcttt ggagatcgtt ctgaagggaa gtctttcaca  1500
ttgatcttat ttgaggatcc cattagtgca gcagatttcc gttgtttgca gctagctgca  1560
gaaggtatgg ttgctaagga tctccccagc gtagcagata tttgtgcctc tggatgttcc  1620
tgcattcagt tttctgagat gcagagtcct caggctattg aatatagaca atgggaggca  1680
cgtgtcgaag atgaagcagg agaagaagcc agagaaccag taatttattc tcaggatcaa  1740
ttgagcagca tgctcactac acaacagaat tttgtatttt ctctagatgc tgtggtaaaa  1800
caggcgatct ggagattccg ttcgaaaggt cttcttacta tggaaagaaa ggcactaggc  1860
gaggagttct taactgcgat attttcctat ttagggagtc aggagcgtaa tgagaatatg  1920
ggaaaagaa ctaccgaaga acatgaggtc gttatcagct tcgaagagct agatcgcatg  1980
gtgcaagtcc tcccagccga agtccctgca gattcaggca atgatcctac gcgtcccgtt  2040
cctaatccag atagtaaccc tgattcctcg caaaatgaag gcagttag               2088
```

<210> 253
<211> 283
<212> PRT
<213> Chlamydia pneumoniae

<400> 253

```
Val Ile Gln His Leu Leu Asn Phe Ala Leu Glu Glu Thr Pro Ser Ile
1               5                   10                  15

Ser Val Gln Tyr Gln Glu Gln Glu Lys Leu Ser Pro Cys Asp His Ser
            20                  25                  30

Pro Glu Ile Gly Lys Lys Lys Arg Trp Asn Lys Leu Glu Ser Phe Ser
        35                  40                  45

Thr Tyr Cys Ser Leu Phe Met Ser Val Lys Asp His Tyr Lys Leu Asn
    50                  55                  60

Leu Gly Ile Gln Asn Ser Leu Ser Gly Trp Leu Leu Asp Pro Tyr Arg
65                  70                  75                  80

Val Cys Ala Pro Leu Ser Ser Pro Tyr Ser Cys Pro Ser Tyr Leu Leu
                85                  90                  95

Asp Leu Gln Asn Lys Glu Leu Arg Arg Ser Leu Leu Ser Thr Phe Leu
            100                 105                 110

Asp Pro Lys Asn Leu Thr Ser Glu Thr Phe Arg Ser Val Ser Ile Asn
        115                 120                 125

Phe Gly Asn Ser Ser Phe Gly Gln Arg Trp Ser Glu Phe Leu Ser Arg
    130                 135                 140

Val Leu His Asp Glu Lys Glu Lys His Val Ala Val Val Cys Asn Asp
145                 150                 155                 160

Ala Lys Leu Leu Glu Glu Gly Leu Ser Pro Glu Ala Leu Ser Leu Leu
                165                 170                 175

Glu Glu Asp Leu Arg Glu Ser Gly Tyr Ser Tyr Leu Asn Ile Leu Ser
            180                 185                 190

Val Ser Pro Glu Gly Val Ser Lys Val Gln Glu Arg Gln Ile Leu Arg
        195                 200                 205

Arg Asp Leu Gln Gly Arg Ser Phe Thr Val Met Ile Thr Asp Leu Pro
    210                 215                 220

Leu Gly Ser Glu Asp Ile Arg Ser Leu Gln Leu Ala Ser Asp Arg Ile
225                 230                 235                 240

Leu Val Ser Ser Ser Leu Asp Ala Ala Asp Ala Cys Ala Ser Gly Cys
                245                 250                 255

Lys Val Leu Val Tyr Glu Asn Pro Asn Ala Ser Trp Ala Gln Glu Leu
            260                 265                 270

Glu Asn Phe Tyr Lys Gln Val Glu Arg Arg Arg
        275                 280
```

<210> 254

<211> 852
<212> DNA
<213> Chlamydia pneumoniae

<400> 254

```
gtgatacaac atcttctaaa ctttgctcta gaagagaccc cttccatttc cgtgcaatac    60
caagaacaag agaagctctc tccgtgcgat cattccccag aaataggtaa aaagaaaaga   120
tggaataagc tggaatcctt ctccacgtat tgttctctgt ttatgtctgt taaggatcat   180
tataagctga atctaggaat tcagaattcc ctgtcagggt ggcttctgga tccctatagg   240
gtttgcgcgc ctttatcttc accgtactcg tgtccttcct atcttttaga tttgcaaaac   300
aaagagctac gtcgttccct tctgtcaacg tttctagacc ctaaaaatct cactagcgaa   360
acattccgtt ctgtctctat aaactttggc aactcttcgt ttggacagag atggtcagag   420
tttctatctc gtgttctgca cgacgagaaa gaaaagcacg tagctgttgt ttgtaatgat   480
gcaaaacttc tggaagaagg attgtcccca gaggcattgt ctctattaga agaagactta   540
agagaatcag ggtattcgta tctaaacatt ctctcggtga gccccgaagg agtctccaag   600
gttcaggaac gtcagattct aaggcgagat ctccaaggac ggtcctttac tgtcatgatt   660
acagatcttc ctttaggtag cgaagatatc cgtagtttac aattagcctc ggataggatt   720
ttagtctcca gttctcttga tgccgcggat gcatgtgctt cgggatgtaa agtcttagtc   780
tacgaaaatc caaatgcatc ctgggctcag gaattggaga acttctacaa acaagttgag   840
agaagaaggt ag                                                        852
```

<210> 255
<211> 265
<212> PRT
<213> Chlamydia pneumoniae

<400> 255

```
Val Ala Cys Pro Ser Ile Ser Ser Trp Phe Thr Val Val Arg Gln His
1               5                   10                  15

Phe Val Asn Ala Phe Asp Phe Thr His Pro Val Cys Ser Arg Ile Thr
            20                  25                  30

Asn Phe Ala Leu Gly Ile Ile Lys Ala Ile Pro Val Leu Gly His Ile
        35                  40                  45

Val Met Gly Ile Glu Trp Leu Ile Ser Trp Ile Pro Arg His Thr Val
    50                  55                  60

Arg His Gly Met Phe Thr Ser Asp Val Ser Ser Ala Ile Lys Val Glu
65                  70                  75                  80

Gln Thr Arg Gly His Asn Cys Leu Ala Pro Leu Glu Ala Tyr Leu Ser
                85                  90                  95

Ser Leu Arg Val Pro Ile Ser Gln Glu Asp Leu Gly Lys Val His Gly
            100                 105                 110

Arg Thr Pro Glu Asp Pro Phe Val Asp Ile Thr Pro Thr Glu Ile Val
        115                 120                 125

Gln Leu Leu Pro Asp Glu Glu Leu Ser Thr Val Asp Glu Ala Leu Gln
    130                 135                 140

Gly Val Arg Ser Arg Leu Thr Tyr Ala Tyr Arg Ser Val Glu Lys Pro
145                 150                 155                 160

Met Ile Gln Asp Leu Ala Leu Val Gly Phe Gly Leu Arg Asp Ser Ala
                165                 170                 175

Asp Leu Ile Asn Phe Val Arg Leu Ala Asn Gly Val Gln Asn His Tyr
```

180 185 190

Pro His Thr Lys Val Lys Leu Tyr Leu Ala Lys Asn Leu Ala Asp Val
195 200 205

Trp Asp Cys Glu Ile Ser Glu Glu Glu Lys Gly Gln Leu Arg Ala Leu
210 215 220

Gly Leu Asp Pro Lys Ile Glu Ser Ile Ser Leu Thr Ser Ala Gly Leu
225 230 235 240

Pro Ser Val Pro Glu Val Ala Thr Val Asp Phe Met Ile Thr Cys Tyr
245 250 255

Gly Lys Asp Gln Glu Val Gln Asp Pro
260 265

<210> 256
<211> 798
<212> DNA
<213> Chlamydia pneumoniae

<400> 256
gtggcttgtc caagtatttc ttcttggttt actgtcgttc gacagcattt tgtaaacgcc 60
tttgatttca cccatcccgt ttgttctcgg attacaaatt ttgctttggg gatcattaag 120
gcaattcccg tattaggaca cattgtcatg ggaatcgagt ggttgatttc ctggattccc 180
agacacaccg ttcgtcatgg aatgtttact tctgatgtct ctagtgctat taaagtagaa 240
caaacacggg gtcataattg tttagctccc ctagaagcct atttaagtag cttgagagtc 300
cccatttccc aagaagatct aggcaaagta cacgggagaa ccccagaaga tcccttcgta 360
gatatcacac ccacagaaat tgtccaactt ctccctgatg aagaactctc tactgtagat 420
gaggcactgc aaggcgttcg tagtaggtta acctatgcct ataggtccgt agagaaacct 480
atgattcaag atcttgctct tgtgggtttt ggtctccgag attctgcgga cctcataaat 540
ttcgtgcgtc ttgctaatgg cgtgcagaat cactatcccc atactaaagt gaagctctat 600
ttagcgaaga acttggcaga tgtctgggac tgtgaaattt ctgaagagga aaaagggcaa 660
ctccgagctc taggtttaga ccctaaaata gagagtatat cccttacgag tgcaggtctt 720
ccttcagtgc cagaagtcgc tactgtcgat tttatgatta cctgttacgg gaaagatcag 780
gaagtccaag atccctag 798

<210> 257
<211> 389
<212> PRT
<213> Chlamydia pneumoniae

<400> 257
Met Met Lys Gln Gly Val Gly Gln Asp Ala Lys Glu Leu Tyr Thr Phe
1 5 10 15

Leu Ser Arg Gly Asn Glu His Tyr Gln Pro Cys Leu Trp Phe Ser Leu
20 25 30

Glu Glu Glu Leu Gly Phe Leu Phe Asp Glu Lys Met Leu Cys Ala Pro
35 40 45

Leu Ser Glu Asp His Tyr Cys His Ser Tyr Leu Val Asp Leu Val Asp
50 55 60

Gln His Leu Lys Asp Leu Ile Leu Ser Met Phe Leu Asp Pro Gln Asn
65 70 75 80

Ile Ser Ala Gly Glu Leu Leu Lys Val Ser Ile Asn Val Gly Asp Ser
85 90 95

```
Phe Ser Pro Leu Gln Gln Lys Asp Phe Leu Ser Met Val Leu Arg Asp
            100                 105                 110

Glu Thr Gly Lys Asn Val Val Val Val Phe Lys Gly Val Leu Ser Leu
        115                 120                 125

Pro Ala Thr Gln Val Cys Lys Leu Val Glu Glu Leu Asn Ser Lys Asp
    130                 135                 140

Tyr Ser Tyr Leu Asn Ile Phe Ser Cys His Gly Asp Ser Ser Pro Gln
145                 150                 155                 160

Leu Leu Phe Arg Lys Glu Leu Glu Gly Thr Ser Gly Arg Tyr Phe Thr
                165                 170                 175

Val Ile Cys Ala Leu Tyr Leu Gly Asp Thr Asp Met Arg Ser Leu Gln
            180                 185                 190

Leu Ala Ser Glu Arg Ile Met Val Ser Arg Glu Phe Asp Leu Val Asp
        195                 200                 205

Ala Tyr Ala Ala Arg Cys Lys Leu Leu Lys Ile Asp His Thr Asn Trp
    210                 215                 220

Arg Pro Gly Thr Phe Ser Arg His Ala Asp Phe Ala Asp Ala Val Asp
225                 230                 235                 240

Val Ser Ala Gly Phe Asn Ser Arg Glu Phe Lys Leu Ile Thr Gln Ala
                245                 250                 255

Asn Gln Gly Ile Leu Glu Ser Gly Glu Leu Pro Leu Pro Ser Lys Thr
            260                 265                 270

Phe Trp Glu Gly Phe Leu Ala Phe Cys Asp Arg Val Thr Val Thr Arg
        275                 280                 285

His Phe Ile Pro Met Leu Asp Ala Ala Ile Lys Gln Ala Val Trp Thr
    290                 295                 300

His Lys His Pro Ser Leu Ile Asp Lys Glu Cys Glu Ala Leu Asp Leu
305                 310                 315                 320

Lys Thr Gln Cys Leu Pro Ser Ile Val Ser Tyr Leu Glu Tyr Val Thr
                325                 330                 335

Asn Ser His Glu Lys Thr Ser Lys Gly Pro Phe Ile Gln Lys Glu Ile
            340                 345                 350

Ile Ala Asp Cys Ser Pro Leu Lys Glu Ala Leu Phe Pro Gly Ser Asp
        355                 360                 365

Glu Asp Val Pro Ser Thr Ser Glu Asp Pro Ser Asp Asp His Pro Ser
    370                 375                 380

Asp Leu Glu Asp Ser
385

<210>   258
<211>   1170
<212>   DNA
```

<213> Chlamydia pneumoniae

<400> 258
atgatgaaac aaggagtcgg gcaggatgct aaagagctat acacatttct atctcgtggg 60
aatgagcatt accaaccgtg tctatggttc agtctcgaag aggaactcgg attccttttc 120
gatgaaaaaa tgctctgcgc ccctctatct gaggatcact attgccactc gtatcttgta 180
gatctagtgg atcaacattt aaaggattta atattatcga tgtttttaga tcctcagaat 240
atctcagcag gagaactcct caaggtctct ataaacgttg gagattcttt ttctcctcta 300
caacagaaag atttcctctc gatggtctta cgtgatgaaa cgggaaaaaa cgtcgtcgtg 360
gtttttaaag gagttctctc cttacccgca acccaagtct gcaaattagt agaggaattg 420
aactctaagg actactccta cctcaatata ttttcttgtc acggagatag tagtcctcag 480
cttttattcc gtaaggaatt agagggaact tcagggcgtt attttacagt gatttgcgct 540
ttatatctag gggatacaga catgcgtagt ttacaacttg cttctgaaag gatcatggtc 600
tctagagagt ttgatcttgt agatgcctat gctgcaagat gcaagctctt gaaaatcgat 660
catacaaatt ggagacctgg aactttcagt cgccacgccg atttcgcaga tgctgtagac 720
gtatcagcag gatttaactc aagagaattt aaactgatta cgcaggcgaa tcaagggatc 780
ctagagtctg gagaactccc gctcccttca aaaaccttct gggaaggatt cttagcattc 840
tgtgatcgag tgactgtcac gagacacttc attccaatgt tagacgccgc tataaagcaa 900
gcggtatgga ctcataaaca tcccagcttg atagataaag agtgtgaagc cctagacttg 960
aaaacacagt gcttgccatc tatcgtatcg taccttgaat atgtcacaaa ctctcacgaa 1020
aaaacatcga aaggcccgtt catacaaaaa gagattatcg cagactgttc tcctcttaaa 1080
gaggcgctct tcccaggttc tgatgaagat gttccctcta cctctgagga tccttcagat 1140
gatcatcctt cggatcttga agactcttaa 1170

<210> 259
<211> 775
<212> PRT
<213> Chlamydia pneumoniae

<400> 259
Met Ala Ser Gly Ile Gly Gly Ser Ser Gly Leu Gly Lys Ile Pro Pro
1               5                   10                  15

Lys Asp Asn Gly Asp Arg Ser Arg Ser Pro Ser Pro Lys Gly Glu Leu
            20                  25                  30

Gly Ser His Glu Ile Ser Leu Pro Pro Gln Glu His Gly Glu Glu Gly
        35                  40                  45

Ala Ser Gly Ser Ser His Ile His Ser Ser Ser Ser Phe Leu Pro Glu
    50                  55                  60

Asp Gln Glu Ser Gln Ser Ser Ser Ser Ala Ala Ser Ser Pro Gly Phe
65                  70                  75                  80

Phe Ser Arg Val Arg Ser Gly Val Asp Arg Ala Leu Lys Ser Phe Gly
                85                  90                  95

Asn Phe Phe Ser Ala Glu Ser Thr Ser Gln Ala Arg Glu Thr Arg Gln
            100                 105                 110

Ala Phe Val Arg Leu Ser Lys Thr Ile Thr Ala Asp Glu Arg Arg Asp
        115                 120                 125

Val Asp Ser Ser Ser Ala Ala Ala Thr Glu Ala Arg Val Ala Glu Asp
    130                 135                 140

Ala Ser Val Ser Gly Glu Asn Pro Ser Gln Gly Val Pro Glu Thr Ser
145                 150                 155                 160

Ser Gly Pro Glu Pro Gln Arg Leu Phe Ser Leu Pro Ser Val Lys Lys

165 170 175

Gln Ser Gly Leu Gly Arg Leu Val Gln Thr Val Arg Asp Arg Ile Val
180 185 190

Leu Pro Ser Gly Ala Pro Pro Thr Asp Ser Glu Pro Leu Ser Leu Tyr
195 200 205

Glu Leu Asn Leu Arg Leu Ser Ser Leu Arg Gln Glu Leu Ser Asp Ile
210 215 220

Gln Ser Asn Asp Gln Leu Thr Pro Glu Glu Lys Ala Glu Ala Thr Val
225 230 235 240

Thr Ile Gln Gln Leu Ile Gln Ile Thr Glu Phe Gln Cys Gly Tyr Met
245 250 255

Glu Ala Thr Gln Ser Ser Val Ser Leu Ala Glu Ala Arg Phe Lys Gly
260 265 270

Val Glu Thr Ser Asp Glu Ile Asn Ser Leu Cys Ser Glu Leu Thr Asp
275 280 285

Pro Glu Leu Gln Glu Leu Met Ser Asp Gly Asp Ser Leu Gln Asn Leu
290 295 300

Leu Asp Glu Thr Ala Asp Asp Leu Glu Ala Ala Leu Ser His Thr Arg
305 310 315 320

Leu Ser Phe Ser Leu Asp Asp Asn Pro Thr Pro Ile Asp Asn Asn Pro
325 330 335

Thr Leu Ile Ser Gln Glu Glu Pro Ile Tyr Glu Glu Ile Gly Gly Ala
340 345 350

Ala Asp Pro Gln Arg Thr Arg Glu Asn Trp Ser Thr Arg Leu Trp Asn
355 360 365

Gln Ile Arg Glu Ala Leu Val Ser Leu Leu Gly Met Ile Leu Ser Ile
370 375 380

Leu Gly Ser Ile Leu His Arg Leu Arg Ile Ala Arg His Ala Ala Ala
385 390 395 400

Glu Ala Val Gly Arg Cys Cys Thr Cys Arg Gly Glu Glu Cys Thr Ser
405 410 415

Ser Glu Glu Asp Ser Met Ser Val Gly Ser Pro Ser Glu Ile Asp Glu
420 425 430

Thr Glu Arg Thr Gly Ser Pro His Asp Val Pro Arg Arg Asn Gly Ser
435 440 445

Pro Arg Glu Asp Ser Pro Leu Met Asn Ala Leu Val Gly Trp Ala His
450 455 460

Lys His Gly Ala Lys Thr Lys Glu Ser Ser Glu Ser Ser Thr Pro Glu
465 470 475 480

Ile Ser Ile Ser Ala Pro Ile Val Arg Gly Trp Ser Gln Asp Ser Ser
485 490 495

```
Val Ser Phe Ile Val Met Glu Asp Asp His Ile Phe Tyr Asp Val Pro
            500                 505                 510

Arg Arg Lys Asp Gly Ile Tyr Asp Val Pro Ser Ser Pro Arg Trp Ser
        515                 520                 525

Pro Ala Arg Glu Leu Glu Glu Asp Val Phe Gly Asp Tyr Glu Val Pro
    530                 535                 540

Ile Thr Ser Ala Glu Pro Ser Lys Asp Lys Asn Ile Tyr Met Thr Pro
545                 550                 555                 560

Arg Leu Ala Thr Pro Ala Ile Tyr Asp Leu Pro Ser Arg Pro Gly Ser
                565                 570                 575

Ser Gly Ser Ser Arg Ser Pro Ser Ser Asp Arg Val Arg Ser Ser Ser
            580                 585                 590

Pro Asn Arg Arg Gly Val Pro Leu Pro Pro Val Pro Ser Pro Ala Met
        595                 600                 605

Ser Glu Glu Gly Ser Ile Tyr Glu Asp Met Ser Gly Ala Ser Gly Ala
    610                 615                 620

Gly Glu Ser Asp Tyr Glu Asp Met Ser Arg Ser Pro Ser Pro Arg Gly
625                 630                 635                 640

Asp Leu Asp Glu Pro Ile Tyr Ala Asn Thr Pro Glu Asp Asn Pro Phe
                645                 650                 655

Thr Gln Arg Asn Ile Asp Arg Ile Leu Gln Glu Arg Ser Gly Gly Ala
            660                 665                 670

Ser Ala Ser Pro Val Glu Pro Ile Tyr Asp Glu Ile Pro Trp Ile His
        675                 680                 685

Gly Arg Pro Pro Ala Thr Leu Pro Arg Pro Glu Asn Thr Leu Thr Asn
    690                 695                 700

Val Ser Leu Arg Val Ser Pro Gly Phe Gly Pro Glu Val Arg Ala Ala
705                 710                 715                 720

Leu Leu Ser Glu Ser Val Ser Ala Val Met Val Glu Ala Glu Ser Ile
                725                 730                 735

Val Pro Pro Thr Glu Pro Gly Asp Gly Glu Ser Glu Tyr Leu Glu Pro
            740                 745                 750

Leu Gly Gly Leu Val Ala Thr Thr Lys Ile Leu Leu Gln Lys Gly Trp
        755                 760                 765

Pro Arg Gly Glu Ser Asn Ala
    770                 775
```

<210> 260
<211> 2328
<212> DNA
<213> Chlamydia pneumoniae

<400> 260

```
atggcatcag gaatcggagg atctagtgga ttaggaaaga ttccacctaa agataatggg   60
gatagaagtc gatcgccctc tcctaaggga gaacttggca gccacgagat ttccctgcct   120
cctcaagaac atggagagga aggagcttca ggatcttcgc atatacatag cagttcctct   180
tttctaccag aagatcagga gtctcagagc tcttcttcgg cagcttctag cccgggattt   240
ttttctcgcg tacgttctgg ggtagacagg gccttaaaat catttggcaa cttttttttcc  300
gcagagtcta cgagtcaagc gcgtgaaacg cgacaagctt ttgttagatt atcaaaaacc   360
atcaccgcgg atgagagacg ggatgtcgat tcatcaagtg ctgctgctac agaagcccga   420
gtggcagagg acgcgagtgt ttcaggcgaa aatccttctc aggggttcc agaaacctct    480
tctggaccag aacctcagcg tttattttct cttccttcag taaaaaaaca gagcggtttg   540
ggtcggttgg tacagacagt tcgcgatcgc atagtacttc ctagtggggc tccacctaca   600
gacagcgagc ctttaagtct ctacgagcta aacctccgtt tgagtagttt acgtcaggag   660
ctctctgaca tacaaagtaa tgatcagttg actccagagg aaaaagcaga agccacagtt   720
accatacaac agctgatcca aattacagaa ttccaatgcg gctatatgga ggcaacacaa   780
tcttcggtat ctctagcaga agctcgtttt aaggggggtag aaactagtga tgagatcaat  840
tccctctgtt cagaactgac agatcctgag cttcaagaac tcatgagtga tggagactct   900
cttcaaaacc tattagatga gactgccgac gatttagaag ctgctttgtc ccatactcga   960
ttgagttttt ctttagacga taatccaact ccgatagaca ataatccaac tctgatttct   1020
caagaagagc ctatttatga ggaaatcgga ggagctgcag atcctcaaag aactcgggaa   1080
aactggtcta caagattatg gaatcagatt cgcgaggctc tggtttctct tttaggaatg   1140
attttaagca ttctagggtc catcttgcac aggttgcgta ttgctcgtca tgcagctgct   1200
gaagcagtgg gtcgttgttg cacgtgccga ggagaagagt gtacttcttc tgaagaggac   1260
tcgatgtcgg tggggtctcc ttcagaaatt gatgaaactg aaagaacggg ctctccgcat   1320
gacgttccac gcagaaatgg aagtccacgt gaagattctc cattgatgaa tgccttagta   1380
ggatgggcac ataagcacgg tgctaaaacc aaggagagtt cagaatcaag taccccggaa   1440
atttcgattt ctgctcccat agtgagaggt tggagtcaag acagttccgt cagttttatt   1500
gttatggaag atgatcatat tttctatgat gttcctcgta gaaaagatgg aatctatgac   1560
gttcctagtt cccctagatg gagtcctgcg cgagagttgg aagaggatgt tttggagat    1620
tatgaagttc ctataacctc tgctgaacca tctaaagaca agaacatcta catgacacct   1680
agattagcaa ctcctgctat ctatgatctt ccttcacgtc caggatcgtc tggaagctca   1740
cgttctccgt cttcagatcg cgtacgaagc agctcaccaa atagacgggg tgtgcctctt   1800
cctccagttc cttcacctgc tatgagtgag gaggggagca tttatgagga tatgagcggt   1860
gcttcaggtg caggtgaaag tgattatgaa gatatgagcc gttcccccctc tcctagaggc  1920
gacttggatg aacccatata tgctaatact cctgaagata atccatttac tcagagaaat   1980
atagatagaa ttttacagga gaggtcaggc ggtgcttccg cttctcctgt agagcctatt   2040
tatgatgaga tcccatggat tcatggcagg ccccctgcta cacttccaag acccgagaat   2100
acattgacta atgtttcgct tagagtgagc ccagggtttg gaccagaagt aagagccgct   2160
ttgcttagcg agagcgtgag tgctgttatg gtcgaagcag agagtattgt tcctccaaca   2220
gagccggggg acggagaatc agaatatcta gagcccttag gggacttgt agctacaacg    2280
aaaatcttac tacaaaaagg atggcctcgt ggagagtcga atgcttag                2328
```

<210> 261
<211> 421
<212> PRT
<213> Chlamydia pneumoniae

<400> 261

```
Met Thr Val Ala Glu Val Lys Gly Thr Phe Lys Leu Val Cys Leu Gly
1               5                   10                  15

Cys Arg Val Asn Gln Tyr Glu Val Gln Ala Tyr Arg Asp Gln Leu Thr
            20                  25                  30

Ile Leu Gly Tyr Gln Glu Val Leu Asp Ser Glu Ile Pro Ala Asp Leu
        35                  40                  45

Cys Ile Ile Asn Thr Cys Ala Val Thr Ala Ser Ala Glu Ser Ser Gly
    50                  55                  60

Arg His Ala Val Arg Gln Leu Cys Arg Gln Asn Pro Thr Ala His Ile
65                  70                  75                  80
```

Val Val Thr Gly Cys Leu Gly Glu Ser Asp Lys Glu Phe Phe Ala Ser
85 90 95

Leu Asp Arg Gln Cys Thr Leu Val Ser Asn Lys Glu Lys Ser Arg Leu
100 105 110

Ile Glu Lys Ile Phe Ser Tyr Asp Thr Thr Phe Pro Glu Phe Lys Ile
115 120 125

His Ser Phe Glu Gly Lys Ser Arg Ala Phe Ile Lys Val Gln Asp Gly
130 135 140

Cys Asn Ser Phe Cys Ser Tyr Cys Ile Ile Pro Tyr Leu Arg Gly Arg
145 150 155 160

Ser Val Ser Arg Pro Ala Glu Lys Ile Leu Ala Glu Ile Ala Gly Val
165 170 175

Val Asp Gln Gly Tyr Arg Glu Val Val Ile Ala Gly Ile Asn Val Gly
180 185 190

Asp Tyr Cys Asp Gly Glu Arg Ser Leu Ala Ser Leu Ile Glu Gln Val
195 200 205

Asp Arg Ile Pro Gly Ile Glu Arg Ile Arg Ile Ser Ser Ile Asp Pro
210 215 220

Asp Asp Ile Thr Glu Asp Leu His Arg Ala Ile Thr Ser Ser Arg His
225 230 235 240

Thr Cys Pro Ser Ser His Leu Val Leu Gln Ser Gly Ser Asn Ser Ile
245 250 255

Leu Lys Arg Met Asn Arg Lys Tyr Ser Arg Gly Asp Phe Leu Asp Cys
260 265 270

Val Glu Lys Phe Arg Ala Ser Asp Pro Arg Tyr Ala Phe Thr Thr Asp
275 280 285

Val Ile Val Gly Phe Pro Gly Glu Ser Asp Gln Asp Phe Glu Asp Thr
290 295 300

Leu Arg Ile Ile Glu Asp Val Gly Phe Ile Lys Val His Ser Phe Pro
305 310 315 320

Phe Ser Ala Arg Arg Arg Thr Lys Ala Tyr Thr Phe Asp Asn Gln Ile
325 330 335

Pro Asn Gln Val Ile Tyr Glu Arg Lys Lys Tyr Leu Ala Glu Val Ala
340 345 350

Lys Arg Val Gly Gln Lys Glu Met Met Lys Arg Leu Gly Glu Thr Thr
355 360 365

Glu Val Leu Val Glu Lys Val Thr Gly Gln Val Ala Thr Gly His Ser
370 375 380

Pro Tyr Phe Glu Lys Val Ser Phe Pro Val Val Gly Thr Val Ala Ile
385 390 395 400

Asn Thr Leu Val Ser Val Arg Leu Asp Arg Val Glu Glu Glu Gly Leu

405 410 415

Ile Gly Glu Ile Val
420

<210> 262
<211> 1266
<212> DNA
<213> Chlamydia pneumoniae

<400> 262
```
atgacggttg cggaagtcaa aggaacattt aagctggtct gtttaggctg tcgggtgaat    60
cagtatgagg tccaagcata tcgcgaccag ttgactatct taggttacca agaggtcctg   120
gattctgaaa tccctgcaga tttatgcata atcaatacgt gtgctgtcac agcttctgct   180
gagagttcgg gtcgtcatgc tgtgcgtcag ttatgtcgtc agaaccctac agcacatatt   240
gttgtcacag gttgtttggg ggaatctgac aaagagtttt ttgcttcttt ggatcggcaa   300
tgcacacttg tttccaataa agaaaaatcc cgacttatag aaaaaatttt ttcctatgat   360
acgaccttcc ctgagttcaa gatccatagt tttgagggaa agtctcgagc ttttattaaa   420
gttcaagatg gctgtaattc tttttgctcg tactgcatta ttccttattt gcgggggcgt   480
tcggtttctc gtcctgctga gaagatttta gctgaaatcg caggggttgt agaccaagga   540
tatcgcgaag ttgtaattgc aggaattaat gttggagatt attgcgatgg agagcgttca   600
ttagcctctt tgattgaaca ggtggaccgg attcctggaa ttgagaggat tcgaatttcc   660
tctatagatc ctgatgatat cactgaagat ctgcaccgtg ccatcacctc atcgcgtcac   720
acttgtcctt cgtcacacct tgttcttcaa tcggggtcga attcaatttt aaagagaatg   780
aaccggaagt attctcgcgg agattttttta gattgtgtag agaagttccg tgcttctgat   840
cctcgctatg cctttactac agatgtgatt gtcggatttc ctggagagag tgatcaagat   900
tttgaagata ctttgagaat tattgaagat gtaggcttta ttaaagtgca tagtttccct   960
ttcagtgctc gtcgtcgtac taaggcatat acttttgata atcagattcc caatcaggtg  1020
atctatgaga ggaagaagta tcttgctgag gttgctaaga gggtaggcca gaaagagatg  1080
atgaagcgtt taggagagac tacagaggtg cttgttgaga aagtaacggg gcaggttgct  1140
acgggtcact ctccttattt tgaaaaggtt tctttccctg ttgtaggaac ggtagctatc  1200
aacactctag tttctgtgcg tcttgatagg gtagaggaag aagggctgat tggggagatt  1260
gtatga                                                             1266
```

<210> 263
<211> 536
<212> PRT
<213> Chlamydia pneumoniae

<400> 263
```
Met Ala Thr Ser Val Pro Val Thr Ser Ser Thr Ser Val Gly Glu Ala
1               5                   10                  15

Asn Ser Ser Asn Glu Arg Phe Thr Glu Arg Thr Ser Arg Met Tyr Tyr
            20                  25                  30

Ala Ala Leu Val Leu Gly Ala Leu Ser Cys Leu Ile Phe Ile Ala Met
        35                  40                  45

Ile Val Ile Phe Pro Gln Val Gly Leu Trp Ala Val Val Leu Gly Phe
    50                  55                  60

Ala Leu Gly Cys Leu Leu Leu Ser Leu Ala Ile Val Phe Ala Val Ser
65                  70                  75                  80

Gly Leu Val Leu Gly Lys Thr Leu Glu Pro Ser Arg Glu Ala Thr Pro
                85                  90                  95

Pro Glu Ile Val Ala Gln Lys Glu Trp Thr Thr Gln Gln Asp Val Leu
            100                 105                 110
```

Gly Asn Glu Tyr Trp Arg Ser Glu Leu Ile Ser Leu Phe Leu Arg Gly
115 120 125

Asp Leu His Glu Ser Leu Ile Val Asp Ser Lys Asp Arg Ser Leu Asp
130 135 140

Ile Asp Gln Ser Leu Gln Asn Ile Leu Lys Leu Glu Pro Leu Ser Thr
145 150 155 160

Thr Leu Ser Leu Leu Lys Lys Asp Cys Val His Ile Asn Ile Ile Leu
165 170 175

His Leu Val Arg Gln Trp Asn Leu Leu Gly Val Asp Leu Ser Pro Glu
180 185 190

Val Thr Ala His Ala Glu Glu Leu Leu Leu Phe Leu Ile Glu Glu Gln
195 200 205

Tyr Tyr Ser Pro Asp Ile Leu Lys Leu Ile Arg Tyr Gly Asp Ala Leu
210 215 220

Gln Ala Thr Ser Pro Leu Met Asp Trp Ala Asp Ser Gly Ser Phe Ser
225 230 235 240

Val Asp Ala Asp Gly Val Phe Ser Cys Arg Arg Glu Glu Cys Ser Pro
245 250 255

Glu Asp Ala Leu Ala Gln Phe Asp Leu Leu Leu Ala Leu Glu Asn Pro
260 265 270

Asp Arg Arg Phe Leu Lys Asp Ser Phe Leu Thr Tyr Ile Trp Ser Ser
275 280 285

Ser Phe Phe Glu Lys Phe Leu His Arg His Leu Glu Ser Leu Gln Arg
290 295 300

Lys Leu Pro Glu Thr Ala Ile Asp Val Ala Arg Tyr Glu Ala Gln Ile
305 310 315 320

Gln Thr Phe Leu Ser Arg Tyr Phe Gln Lys Leu Asp Leu Ile Asn Ala
325 330 335

Met Ser Leu Asp Trp Gly Tyr Asn Cys Ala Glu Gly Glu Lys Cys Tyr
340 345 350

Glu Ser Ala Asn Gln Arg Leu Asp Asn Leu Phe Ile Ala Phe Ser Ser
355 360 365

Ser Val Pro Ala Met Lys Arg Leu Phe Asp Lys Tyr Gly Ser Val Val
370 375 380

Arg Val Asp Arg Arg Gln Ile Arg Glu Gln Ile Leu Ser Asn Thr Glu
385 390 395 400

Ile Leu Glu Asn Glu Ser Gly Phe Leu Cys Ser Leu Tyr Glu Tyr Pro
405 410 415

Leu Ser Tyr Leu Ile Asp Trp Ala Val Leu Leu Asp Cys Val Arg Gly
420 425 430

Thr Glu Ile Ser Leu Glu Asp Gln Ala Asp Tyr Thr Val Cys Leu Gln

```
        435                 440                 445

Gly Leu Asp Ser Met Leu Ser Gln Phe Ala Ser Arg Leu Gln Ser Gly
    450                 455                 460

Gln Lys Val Leu Asn Pro Arg Asp Val Leu Ser Glu Gln Ala Ala Val
465                 470                 475                 480

Met Leu Val His Gly Leu Ala Ala Gln Gly Val Ser Phe Gln Gly Leu
                485                 490                 495

Lys Ala Leu Met Tyr Leu Thr Ala Val Pro Gln Arg Met Trp Leu Gly
            500                 505                 510

Ala Leu Pro Leu Phe Glu Ser Phe Pro Val Phe Asn Arg Met Lys Glu
        515                 520                 525

Phe Leu Gly Glu Ser Leu Gly Asp
    530                 535
```

<210> 264
<211> 1611
<212> DNA
<213> Chlamydia pneumoniae

<400> 264

```
atggcaacct ctgttcctgt aacttcatct acttctgtag gagaggctaa ctcctccaac   60
gaaagattta ctgaacgaac atcgcgaatg tattacgcag ctttagtcct aggggctttg  120
agctgtttaa tttttattgc tatgattgtc attttcccac aggtcggatt gtgggctgtg  180
gtcctcgggt ttgctcttgg atgtttactt ttaagcttag ctatcgtttt tgctgtctcc  240
ggtctcgttt taggcaagac tttagaacct agtcgagaag cgactcctcc agaaattgtt  300
gcgcaaaagg agtggactac acaacaagat gtcttaggga atgagtattg gcgttccgag  360
ttgatttcct tgttcttacg aggggatctc cacgaatctc tgattgttga ttctaaggat  420
cgatctttag atattgatca gagtttacaa aatatattga aacttgagcc cctatctacg  480
acactttcgc tgttaaagaa agattgtgtc cacatcaata tcattttaca tttagtgaga  540
cagtggaact tactgggagt ggatcttagt cctgaagtca ctgcgcacgc cgaggaactt  600
ctactctttt tgatagaaga gcagtattac tctcctgata ttttgaaatt gattcgctac  660
ggagatgctt tacaagcaac gtctcctttg atggattggg cagattcagg ttcctttagt  720
gtagacgcag acggggtatt tagctgtcgc agagaagaat gttctcctga ggatgctttg  780
gcgcaattcg atcttctttt ggcgttggaa aatcccgaca gacgcttctt aaaggattct  840
tttcttacct acatttggtc gtcttcattt tttgagaagt tttacatcgg ccatctagag  900
agcttgcaaa gaaagctccc agagacagcg atcgatgtcg cccgctatga agcacaaata  960
caaacatttc tctctcgcta ttttcagaag ctcgatttga taaacgcaat gtccttagat 1020
tggggatata actgtgctga gggagaaaaa tgttatgaga gcgcaaatca aagattagac 1080
aacctattta ttgctttttc ttcttctgtt cctgctatga agcggctctt tgacaaatat 1140
ggttctgtgg tacgggtaga tcgtaggcag attcgtgagc agattctttc gaacactgaa 1200
atcttagaaa atgagtcagg gttcctctgc agtttgtatg aatatccttt atcctatttg 1260
atagattggg ctgttttgct agactgtgtt cgcggtaccg aaatctctct agaagatcag 1320
gccgattaca ccgtttgttt gcaaggcttg gattctatgt tatctcaatt tgcgagtcgt 1380
ttacagtctg gacaaaaagt attgaatcct agagatgttt taagtgaaca ggctgcggtt 1440
atgcttgttc atggcttggc agcacagggc gtgtcgtttc aaggattgaa agctttgatg 1500
tatttgacag ccgttcccca aagaatgtgg ttaggagcat tgcctttatt tgaatctttt 1560
cctgtcttta atcggatgaa agaatttctt ggggaatctc tgggagacta g          1611
```

<210> 265
<211> 264
<212> PRT
<213> Chlamydia pneumoniae

<400> 265

```
Met Ser Asn Gln Leu Gln Pro Cys Ile Ser Leu Gly Cys Val Ser Tyr
```

```
1               5                   10                  15

Ile Asn Ser Phe Pro Leu Ser Leu Gln Leu Ile Lys Arg Asn Asp Ile
            20                  25                  30

Arg Cys Val Leu Ala Pro Pro Ala Asp Leu Leu Asn Leu Leu Ile Glu
        35                  40                  45

Gly Lys Leu Asp Val Ala Leu Thr Ser Ser Leu Gly Ala Ile Ser His
    50                  55                  60

Asn Leu Gly Tyr Val Pro Gly Phe Gly Ile Ala Ala Asn Gln Arg Ile
65                  70                  75                  80

Leu Ser Val Asn Leu Tyr Ala Ala Pro Thr Phe Phe Asn Ser Pro Gln
                85                  90                  95

Pro Arg Ile Ala Ala Thr Leu Glu Ser Arg Ser Ser Ile Gly Leu Leu
            100                 105                 110

Lys Val Leu Cys Arg His Leu Trp Arg Ile Pro Thr Pro His Ile Leu
        115                 120                 125

Arg Phe Ile Thr Thr Lys Val Leu Arg Gln Thr Pro Glu Asn Tyr Asp
    130                 135                 140

Gly Leu Leu Leu Ile Gly Asp Ala Ala Leu Gln His Pro Val Leu Pro
145                 150                 155                 160

Gly Phe Val Thr Tyr Asp Leu Ala Ser Gly Trp Tyr Asp Leu Thr Lys
                165                 170                 175

Leu Pro Phe Val Phe Ala Leu Leu Leu His Ser Thr Ser Trp Lys Glu
            180                 185                 190

His Pro Leu Pro Asn Leu Ala Met Glu Glu Ala Leu Gln Gln Phe Glu
        195                 200                 205

Ser Ser Pro Glu Glu Val Leu Lys Glu Ala His Gln His Thr Gly Leu
    210                 215                 220

Pro Pro Ser Leu Leu Gln Glu Tyr Tyr Ala Leu Cys Gln Tyr Arg Leu
225                 230                 235                 240

Gly Glu Glu His Tyr Glu Ser Phe Glu Lys Phe Arg Glu Tyr Tyr Gly
                245                 250                 255

Thr Leu Tyr Gln Gln Ala Arg Leu
            260
```

<210> 266
<211> 795
<212> DNA
<213> Chlamydia pneumoniae

<400> 266

```
atgtctaacc aactccagcc atgtataagc ttaggctgcg taagttatat taattccttt    60
ccgctgtccc tacaactcat aaaaagaaac gatattcgct gtgttcttgc tccccctgca   120
gacctcctca acttgctaat cgaagggaaa ctcgatgttg ctttgacctc atccctagga   180
gctatctctc ataacttggg gtatgtcccc ggctttggaa ttgcagcaaa ccaacgtatc   240
ctcagtgtaa acctctatgc agctcccact ttctttaact caccgcaacc tcggattgcc   300
```

```
gcaactttag aaagtcgctc ctctatagga ctcttaaaag tgctttgtcg tcatctctgg   360
cgcatcccaa ctcctcatat cctaagattc ataactacaa aagtactcag acaaacccct   420
gaaaattatg atggcctcct cctaatcgga gatgcagcgc tacaacatcc tgtacttcct   480
ggatttgtaa cctatgacct tgcctcgggg tggtatgatc ttacaaagct accttttgta   540
tttgctcttc ttctacacag cacctcttgg aaagaacatc ccctacccaa ccttgcgatg   600
gaagaagccc tccaacagtt cgaatcttca cccgaagaag tccttaaaga agctcatcaa   660
catacaggtc tgccccttc tcttcttcaa gaatactatg ccctatgcca gtaccgtcta   720
ggagaagaac actacgaaag ctttgaaaaa ttccgggaat attatggaac cctctaccaa   780
caagcccgac tgtaa                                                    795
```

<210> 267
<211> 279
<212> PRT
<213> Chlamydia pneumoniae

<400> 267

```
Met Ser Ser Leu Leu Ser Cys Gly Arg Ile Glu Pro Thr Arg Val Thr
1               5                   10                  15

Cys Ser Leu Lys Thr Tyr Leu Glu Asp Thr Ser Gln Asn Gln Leu Ser
            20                  25                  30

Thr Arg Leu Val Arg Ala Ser Val Ile Phe Leu Cys Ala Leu Leu Ile
        35                  40                  45

Ile Leu Val Cys Val Ala Leu Ser Ser Leu Ile Pro Ser Ile Met Ala
    50                  55                  60

Leu Ala Thr Ser Phe Thr Val Met Gly Leu Ile Leu Phe Val Met Ser
65                  70                  75                  80

Leu Leu Gly Asp Val Ala Ile Ile Ser Tyr Leu Thr Tyr Ser Thr Val
                85                  90                  95

Thr Ser Tyr Arg Gln Asn Lys Arg Ala Phe Glu Ile His Lys Pro Ala
            100                 105                 110

Arg Ser Val Tyr Tyr Glu Gly Val Arg His Trp Asp Leu Gly Arg Ser
        115                 120                 125

Ser Leu Gly Thr Gly Glu Ile Pro Ile Val Arg Thr Leu Phe Ser Pro
    130                 135                 140

Phe Gln Asn His Gly Leu Asn His Ala Leu Ala Ala Lys Ile Phe Leu
145                 150                 155                 160

Phe Met Glu His Phe Ser Pro Glu Pro Pro Asn Glu Pro Leu Val Asp
                165                 170                 175

Trp Ala Cys Leu Ile Arg Asp Phe Arg Pro His Val Ser Ser Leu Cys
            180                 185                 190

Phe Val Ile Glu Lys Gln Gly Ser Ser Leu Arg Thr Lys Glu Gly Asn
        195                 200                 205

Thr Ile Cys Glu Ala Phe Arg Ser Asp Tyr Asp Ala His Phe Ala Met
    210                 215                 220

Val Asp Cys Tyr Arg Leu Ile His Ser Lys Leu Ile Ile Glu Lys Met
225                 230                 235                 240
```

Gly Leu Lys Asn Ile Asp Ile Ile Pro Ser Val Met Val Arg Glu Asp
245 250 255

Tyr Pro Ser Arg Pro Gly Glu Gly Tyr Arg Glu Gly Leu Leu Arg Met
260 265 270

Tyr Gly Gly Lys Gly Ala Leu
275

<210> 268
<211> 840
<212> DNA
<213> Chlamydia pneumoniae

<400> 268
atgtcatcac tactgagctg cggaagaata gagccgactc gggttacctg tagcttaaag 60
acgtatcttg aggatacgag tcagaatcag ttgagcacac gtctagttcg ggcaagtgtc 120
atctttttat gcgcattgtt gatcattttg gtttgtgtgg ccctctctag tttgattcca 180
agcattatgg ccttggcgac ctcttttacg gtaatggggt taattctttt tgtgatgtca 240
cttcttggtg acgttgcaat tataagttat cttacttata gcactgttac gagttaccgg 300
caaaataaga gagcttttga gattcacaag cccgctcgct ccgtttacta cgagggggtc 360
cgccattggg atttaggacg atcatcttta ggcacaggcg agattcctat agtaaggacg 420
ttattctctc catttcagaa ccatggtctt aaccatgcct tagctgctaa aattttccta 480
tttatggagc atttcagccc tgagccaccg aacgagcctt tggtggattg ggcctgtttg 540
attcgggatt ttaggcctca cgtcagttct ttgtgctttg ttattgaaaa acaagggtca 600
tcgctgagga ctaaggaagg caatacgatt tgtgaggctt tccgctctga ttacgacgcc 660
cattttgcta tggtagattg ctaccggttg atccactcta agttgattat agagaaaatg 720
ggattgaaga atatcgatat cattccgagt gtcatggttc gtgaagatta tcctagccgt 780
cctggggagg gctatcgcga aggcctatta cgtatgtatg gtggcaaggg ggctctgtga 840

<210> 269
<211> 359
<212> PRT
<213> Chlamydia pneumoniae

<400> 269
Met Ser Gly Pro Ser Arg Thr Glu Ser Ser Gln Val Ser Val Leu Ser
1 5 10 15

Tyr Val Pro Arg Asp Lys Glu Ile Ala Pro Lys Lys Gln Phe Thr Ile
20 25 30

Ala Lys Ile Ser Thr Leu Ala Ile Leu Ala Ser Leu Ala Leu Gly Ala
35 40 45

Leu Val Ala Gly Ile Ser Leu Thr Ile Val Leu Gly Asn Pro Val Phe
50 55 60

Leu Ala Leu Leu Ile Thr Thr Ala Leu Phe Ser Val Val Thr Phe Leu
65 70 75 80

Val Tyr His Gln Met Thr Ser Lys Val Ser Ser Asn Trp Gln Lys Val
85 90 95

Leu Glu Gln Asn Phe Lys Pro Leu Gly Lys Ala Trp Gln Glu Lys Asn
100 105 110

Val Asp Cys Tyr Ser Asn Glu Met Gln Phe Tyr Asn Asn His Leu Asn
115 120 125

Pro Lys Phe Lys Val Ala Ile Gln Thr Asp Ala Ser Gln Pro Phe Gln

130 135 140

Pro Thr Phe Leu Thr Gly Leu Arg Val Ile Glu Lys Asn Gln Ser Thr
145 150 155 160

Gly Ile Ile Phe Asn Pro Val Gly Pro Thr Asn Leu Ile Asp Asn Thr
165 170 175

Ala Thr Asn Leu Ser Thr Ile Leu Tyr Ser Thr Leu Lys Asp Lys Ser
180 185 190

Val Trp Asp Thr Cys Lys Gln Arg Glu Gly Gly Pro Ala Lys Gly Glu
195 200 205

Asp Pro Phe Ser Pro Thr Glu Val Arg Val Val Lys Leu Pro Asn Glu
210 215 220

Ala Leu Asp Gln Thr Phe Asn Leu Asn Leu Ser Ser Ala Glu Lys Lys
225 230 235 240

Ser Ile Leu Pro Thr Phe Leu Gly His Val Cys Gly Pro Lys Ser Glu
245 250 255

Glu Leu Pro Asn Gln Gln Glu Tyr Tyr Arg Gln Ala Leu Leu Ala Tyr
260 265 270

Glu Asn Cys Leu Lys Ala Ala Ile Glu Ser His Ala Ala Ile Val Ala
275 280 285

Leu Pro Leu Phe Thr Ser Val Tyr Glu Val Pro Pro Glu Glu Ile Leu
290 295 300

Pro Lys Glu Gly Thr Phe Tyr Trp Asp Asn Gln Thr Gln Ala Phe Cys
305 310 315 320

Lys Arg Ala Leu Leu Asp Ala Ile Gln Asn Thr Ala Leu Arg Tyr Pro
325 330 335

Gln Arg Ser Leu Leu Val Ile Leu Gln Asp Pro Phe Asn Thr Ile Glu
340 345 350

Ser Gln Ser Arg Ser Glu Glu
355

<210> 270
<211> 1080
<212> DNA
<213> Chlamydia pneumoniae

<400> 270
atgtcaggac cctcacgtac tgagagctct caagtttctg tactatccta tgtgcctcgg 60
gataaagaaa ttgctcctaa aaaacagttt accatagcaa aaatatccac tcttgcaatc 120
ctagcttctt tagctttagg agctttggtg gctggaatct ctttaacgat agtattaggg 180
aaccctgtat ttttggctct tctcattacc acggccctct tctcagttgt aaccttctta 240
gtctaccacc aaatgacctc aaaggtatct tctaactggc agaaagttct agagcaaaac 300
ttcaagcctt tgggaaaagc gtggcaagaa aaaaacgtag actgctactc aaacgagatg 360
caattttaca ataatcacct gaaccctaag ttcaaggtag cgatacaaac agatgcgtct 420
caaccatttc agcctacttt cttaactgga cttagagtga tcgaaaaaaa tcaatccaca 480
gggatcatct ttaatcccgt aggcccaacg aatctgatcg acaacactgc aacgaacctc 540
tctactatcc tttactccac cctaaaagat aaaagcgtgt gggatacatg caagcaacgc 600
gaagggggtc ccgcaaaagg agaagacccc ttttccccta ccgaagtgag agtagtaaaa 660

```
cttccaaacg aagctctaga tcaaacgttt aatctaaatt taagctctgc agaaaagaaa    720
agtattcttc cgaccttttt aggccacgta tgcggcccta aatctgaaga gttaccaaat    780
cagcaagaat attatcgcca agctttacta gcgtacgaga actgccttaa agcagctata    840
gaaagtcatg cagcaatcgt tgctcttcct ctctttactt cggtctatga agtgcctcca    900
gaagagattc ttcctaaaga aggcactttc tattgggaca accaaactca agcgttttgc    960
aaacgcgctt tattggacgc tattcaaaat acggccctac gctatcctca aagatcttta   1020
cttgttatac tccaagatcc ttttaatact atagaatcac aaagtcgttc tgaggagtaa   1080

<210>   271
<211>   312
<212>   PRT
<213>   Chlamydia pneumoniae

<400>   271
Met Trp Arg Val Val Leu Arg Phe Leu Ile Ile Phe Ile Leu Gly Arg
1               5                   10                  15

Ala Val Phe Pro Leu Arg Ala Ser Glu Ser Phe Ser Trp Glu Thr Ser
            20                  25                  30

Thr Cys Leu Thr Val Leu Gly Ile Pro Phe Ile Asp Ile Ile Leu Thr
        35                  40                  45

Thr Asn Glu Asp Phe Val Ala Gln Cys Gly Leu Gln Ile Gly Thr Ile
    50                  55                  60

Ser Ser Thr Asn Asn Ala Lys Ile Lys Glu Ile Phe Leu Ile Tyr Lys
65                  70                  75                  80

Glu Lys Phe Pro Glu Ala Ser Ile Ser Phe Lys Arg Lys Glu Pro Leu
                85                  90                  95

Asn Leu Ser Gln Ser His Leu Ser Asp Leu Gly Ile Leu Cys Met Arg
            100                 105                 110

Asn Gly Glu Thr Tyr Ala Glu Gly Met Ala Asn Lys Glu Asn Gly Pro
        115                 120                 125

Ala Leu Lys Gln Pro Lys Asp Leu Arg Leu Val Leu Arg Cys Pro Asn
    130                 135                 140

Gln Pro Asp Thr Leu Leu Tyr Ser Glu Lys Glu Ala Glu Lys Gly Ile
145                 150                 155                 160

Glu Thr Asn Thr Cys Leu Cys Asn Gln Gly Tyr Thr Leu Leu Asp Gly
                165                 170                 175

Gln Leu Ile Leu Tyr Gly Asp Ser Ile Glu Lys Phe Leu Lys Glu Thr
            180                 185                 190

Lys Arg Lys Asn Asn His Thr Leu Val Asp Leu Cys Asp Ser Gln Val
        195                 200                 205

Val Thr Thr Phe Leu Gly Arg Phe Trp Ser Leu Leu Asn Tyr Val Gln
    210                 215                 220

Val Leu Phe Leu Ser Glu Asp Ser Ala Lys Ile Leu Ala Gly Ile Pro
225                 230                 235                 240

Asp Leu Ala Gln Ala Thr Gln Leu Leu Ser His Thr Val Pro Leu Leu
                245                 250                 255
```

Phe Ile Tyr Thr Asn Asp Ser Ile His Ile Ile Glu Gln Gly Lys Glu
260 265 270

Ser Ser Phe Thr Tyr Asn Gln Asp Leu Thr Glu Pro Ile Leu Gly Phe
275 280 285

Leu Phe Gly Tyr Ile Asn Arg Gly Ser Met Glu Tyr Cys Phe Asn Cys
290 295 300

Ala Gln Ser Ser Leu Gly Glu Thr
305 310

<210> 272
<211> 939
<212> DNA
<213> Chlamydia pneumoniae

<400> 272
atgtggcgcg ttgtcctcag attccttata atttttatct tgggaagagc cgtcttccct 60
ctaagagctt cagaaagctt ctcctgggaa acatcgacct gtttaacagt gctagggatt 120
cctttcatag atattatcct cacaacgaat gaggactttg ttgcccagtg cggcctgcaa 180
ataggaacca tttcttcgac taataacgca aaaataaaag aaattttttt gatatataag 240
gaaaaatttc cagaagcctc tatcagtttc aaacgaaaag aacctctaaa cctttcccaa 300
tcccatctct ccgatttagg tattttatgt atgcgtaacg gagaaactta cgctgaggga 360
atggcaaata aagaaaacgg acccgctcta aaacaaccca aggatctaag attagtttta 420
cgttgtccta accaaccaga taccctgctc tactcggaaa aagaagcaga aaagggcata 480
gaaacaaata cttgcctatg caatcaggga tacacactcc tggatgggca attgattctc 540
tacggggata gtatagaaaa gtttctgaaa gagaccaaaa gaaagaataa ccacacgctt 600
gttgatcttt gtgactcaca agtcgtgacc acgttcctcg gtcgcttttg gtctcttcta 660
aactacgttc aagttctttt cctatctgaa gactccgcta aaattcttgc gggcatccca 720
gacctagctc aagctacgca attgctttcc cacaccgtac ctttgctttt tatttatacc 780
aacgattcta ttcacatcat agaacaaggc aaagaaagta gttttaccta taaccaagat 840
ttaacagagc ccatttttagg atttctcttt ggttacataa atcgcggctc tatggaatac 900
tgctttaatt gtgcacagtc ttcattagga gaaacctaa 939

<210> 273
<211> 379
<212> PRT
<213> Chlamydia pneumoniae

<400> 273
Val Leu Val Gly Ile Cys Pro Ser Leu Tyr Pro Glu His Pro Arg Ser
1 5 10 15

Phe Tyr Tyr Arg Val Ser Gly Asp Ile Gly Ser Arg Phe Asp Asp Arg
20 25 30

Gly Phe Val Asn Ser Gly Val Glu Thr Leu Pro Tyr Ser Ser Gly Ser
35 40 45

Phe Gly Ile Phe Trp Ile Ser Phe Thr Asp Pro Thr Phe Asn Phe Ala
50 55 60

Ile Val Asn Thr Phe Met Arg Thr Ala Gly Ile Asn Glu Val Ser Arg
65 70 75 80

Pro Met Thr Gln Asp Thr Glu Thr Ser Leu Ile Glu Met Arg Asp Leu
85 90 95

Ser Glu Gln Gln Glu Ala Asn Asn Thr Asp Ser Leu Glu Gln Glu Glu

```
            100                 105                 110

Ser Leu Met Gly Ile Val Gly His Thr Val Gly Gly Val Ser Met Thr
        115                 120                 125

Val Thr Ser Ser Pro Asn Ile Phe Tyr Arg Ile Gln Thr Leu Leu Gly
    130                 135                 140

Leu Pro Glu Thr Leu Ala Glu Ala Glu Glu Asn Pro Thr Phe Pro Asn
145                 150                 155                 160

Ser Thr Ile Asp Ser Leu Ala Glu Ile Met Met Asn Leu Val Arg Ile
                165                 170                 175

Ser Asp Ala Val Ser Ile Phe Trp Ile Phe Pro Ile Val Asp Thr Thr
            180                 185                 190

Tyr Asn Gly Val Leu Leu Ala Val Cys Ile Gly Phe Phe Gly Ile Asn
        195                 200                 205

Gly Ile Cys Ser Thr Phe Leu Met Leu Thr Asn Pro Arg Ser Arg Arg
    210                 215                 220

Asp Arg Trp Arg Asn Leu Arg Ile Met Val Leu Cys Tyr Arg Ser Leu
225                 230                 235                 240

Gly Ser Gly Met Asn Leu Phe Asp Leu Ser Asn Asn Val Arg Met Ala
                245                 250                 255

Ala Arg Arg His Val Thr Ser Cys Thr Val Ala Leu Tyr Ala Met Val
            260                 265                 270

Thr Leu Phe Gly Trp Thr Val Ala Ile Gln Asp Ala Leu Gln Tyr Gly
        275                 280                 285

Phe Pro Ser Val Arg Asp Ala Phe Tyr Arg Tyr Cys Leu Arg His Arg
    290                 295                 300

Tyr Cys Leu Thr Gln Arg Asn Glu Asp Ser Leu Gln Thr Thr Gly Thr
305                 310                 315                 320

Arg Phe Gln Val Thr Arg Thr His Leu Glu Asp Gln Gln Met Val Ala
                325                 330                 335

Ser Ile Leu Asn Leu Ser Val Phe Gly Leu Phe Phe Gly Phe Val Gly
            340                 345                 350

Leu Met Thr Thr Phe Gly Gly Leu Glu Ile Ser Pro Ser Cys Arg Trp
        355                 360                 365

Asp Ala Ala Asn Asn Arg Thr Val Gly Ile Phe
    370                 375
```

<210> 274
<211> 1140
<212> DNA
<213> Chlamydia pneumoniae

<400> 274

```
gtgctcgttg gtatctgtcc ttctctatat ccagaacatc ctcgctcctt ttattatcgt    60
gtttctggag atataggctc ccgattcgac gatagaggat ttgtaaactc tggagtcgaa   120
```

```
accctgccat actcttcagg cagctttggg attttttgga tctcgtttac ggatcccaca   180
tttaattttg ctatcgtaaa tacctttatg cgaactgcag ggatcaatga agtctctaga   240
cccatgacac aagatacaga aacttcattg atagaaatga gagacctaag tgaacaacaa   300
gaagcgaata acacagattc tttagagcaa gaagagagct taatgggtat tgtaggacat   360
actgtgggag gagtttccat gaccgtgacc tccagtccaa atatctttta tcgtatacaa   420
acacttctgg gactgccaga gactcttgca gaagctgaag aaaatcctac cttcccaaat   480
tctactatag atagccttgc agaaataatg atgaacctcg taaggatctc tgatgctgtc   540
tctattttct ggatttttcc tatcgtagat actacatata atggagtttt attagccgtc   600
tgtatcggct tcttcggaat caatgggatt tgttccacgt tccttatgct tacgaatcca   660
cgctctcgtc gagatagatg gaggaattta cgcatcatgg ttctttgcta tcgttctttg   720
ggaagcggaa tgaatctctt tgatcttagc aataatgtgc gcatggcagc acgtaggcat   780
gtgacatcat gtacagtagc tctctatgct atggtcactc tatttggatg gacagtagca   840
atacaagatg ctttgcaata tggtttccct agcgttcggg atgccttcta tagatattgc   900
ttacgccaca gatattgctt aactcaaaga aacgaagact ctctgcaaac tacaggaacg   960
cgctttcagg ttacccgtac acatctagaa gatcaacaga tggtggcttc tattttgaat   1020
ttgagtgttt ttgggctctt ttttggattc gtagggctaa tgaccacgtt tggaggatta   1080
gaaatctcac catcttgtcg gtgggatgca gcaaataacc gaacggtagg tatttttttag   1140
```

<210> 275
<211> 369
<212> PRT
<213> Chlamydia pneumoniae

<400> 275

```
Met Ala Pro Ile His Gly Ser Asn Ala Phe Val Glu Asp Ile Leu His
1               5                   10                  15

Ser His Pro Ser Pro Gln Ala Thr Tyr Phe Ser Ser Thr Arg Ala Gln
            20                  25                  30

Lys Leu His Glu Phe Lys Asp Arg His Pro Val Leu Thr Arg Ile Ala
        35                  40                  45

Ser Val Ile Ile Lys Ile Phe Lys Val Leu Ile Gly Leu Ile Ile Leu
    50                  55                  60

Pro Leu Gly Ile Tyr Trp Leu Cys Gln Thr Leu Cys Thr Asn Ser Ile
65                  70                  75                  80

Leu Pro Ser Lys Asn Leu Leu Lys Ile Phe Lys Lys Gln Pro Asn Thr
                85                  90                  95

Lys Thr Leu Lys Thr Asn Tyr Leu His Ala Leu Gln Asp Tyr Ser Ser
            100                 105                 110

Lys Asn Arg Val Ala Ser Met Arg Arg Val Pro Ile Leu Gln Asp Asn
        115                 120                 125

Val Leu Ile Asp Thr Leu Glu Ile Cys Leu Ser Gln Ala Pro Thr Asn
    130                 135                 140

Arg Trp Met Leu Ile Ser Leu Gly Ser Asp Cys Ser Leu Glu Glu Ile
145                 150                 155                 160

Ala Cys Lys Glu Ile Phe Asp Ser Trp Gln Arg Phe Ala Lys Leu Ile
                165                 170                 175

Gly Ala Asn Ile Leu Val Tyr Asn Tyr Pro Gly Val Met Ser Ser Thr
            180                 185                 190

Gly Ser Ser Ser Leu Lys Asp Leu Ala Ser Ala His Asn Ile Cys Thr
```

```
        195                 200                 205

Arg Tyr Leu Lys Asp Lys Glu Gln Gly Pro Gly Ala Lys Glu Ile Ile
    210                 215                 220

Thr Tyr Gly Tyr Ser Leu Gly Gly Leu Ile Gln Ala Glu Ala Leu Arg
225                 230                 235                 240

Asp Gln Lys Ile Val Ala Asn Asp Asp Thr Thr Trp Ile Ala Val Lys
                245                 250                 255

Asp Arg Cys Pro Leu Phe Ile Ser Pro Glu Gly Phe His Ser Cys Arg
            260                 265                 270

Arg Ile Gly Lys Leu Val Ala Arg Leu Phe Gly Trp Gly Thr Lys Ala
        275                 280                 285

Val Glu Arg Ser Gln Asp Leu Pro Cys Leu Glu Ile Phe Leu Tyr Pro
    290                 295                 300

Thr Asp Ser Leu Arg Arg Ser Thr Val Arg Gln Asn Lys Leu Leu Ala
305                 310                 315                 320

Pro Glu Leu Thr Leu Ala His Ala Ile Lys Asn Ser Pro Tyr Val Gln
                325                 330                 335

Asn Lys Glu Phe Ile Glu Val Arg Leu Ser Ser Asp Ile Asp Pro Ile
            340                 345                 350

Asp Ser Lys Thr Arg Val Ala Leu Ala Thr Pro Ile Leu Lys Lys Leu
        355                 360                 365

Ser


<210>   276
<211>   1110
<212>   DNA
<213>   Chlamydia pneumoniae

<400>   276
atggctccaa ttcacggaag taatgcgttt gttgaggata ttttacattc ccacccttct     60
ccacaagcga cttatttttc ttcaacacgc gcccaaaaac ttcatgagtt taaagacagg    120
catcccgtgc ttacacggat tgcttctgta attattaaaa tttttaaagt tctgataggg    180
ctgatcatcc ttcccttagg aatctactgg ctatgtcaaa cgctttgtac aaactcgatt    240
ctcccttcca agaatttatt aaaaattttc aagaagcaac ccaacactaa aaccttaaaa    300
actaattatt tgcatgcttt gcaagattat tcctcgaaaa accgcgttgc ttccatgaga    360
cgagttccta tcctccagga taatgttctc atcgacactt tggaaatatg cctttcacaa    420
gcacctacga atcgttggat gctcatttct ttaggaagtg actgtagctt ggaagaaatc    480
gcttgtaagg agatctttga ttcttggcaa agatttgcca agttgatagg ggccaatata    540
ctcgtttata actaccccgg agtcatgtcc agcacaggga gcagcagcct aaaggaccta    600
gcatcagctc ataatatttg tacaagatac cttaaagata aagaacaggg ccctggagca    660
aaagaaatca ttacctatgg gtactcccta ggaggtttga tacaagcaga agcattgcga    720
gaccagaaga ttgttgcaaa cgatgatact acttggatag cagtcaaaga taggtgtcct    780
ctctttatat ctccagaagg tttccacagt tgcagacgca taggaaagct agtagctcgt    840
ctttttggct gggggaccaa agccgtagag agaagccaag accttccctg cctagaaatt    900
tttctctatc ctacggattc cttacgaaga tcaacagtca gacagaacaa gctcttagca    960
cctgaactta ctctcgctca tgcgataaaa aatagtccct atgttcaaaa taaagaattt   1020
atagaagtac gattatcgtc tgatatcgat cccatcgaca gcaaaacaag agtggctctt   1080
gccacaccaa ttttgaaaaa gctctcttag                                    1110
```

<210> 277
<211> 486
<212> PRT
<213> Chlamydia pneumoniae

<400> 277

```
Met His Met Ser Asn Pro Ile Ser Leu Phe Ser Pro Ala Glu Leu Ile
1               5                   10                  15

Ala Lys Tyr Asn Leu Ile Pro Lys Thr Ser Pro Ile Tyr Pro Arg Arg
            20                  25                  30

Thr Glu Leu Ile Ile Leu Glu Glu Asn Ala Cys Gln Thr Arg Leu Thr
        35                  40                  45

Asn Val Ala Gln Val Leu His Pro Ser Ser Leu Phe Ser Met Ser Lys
    50                  55                  60

Lys Ile Leu Asn Pro Cys Gly Cys Ser Gly Gly Pro Leu Cys Trp Val
65                  70                  75                  80

Ile Leu Asn Ile Leu Ala Phe Ile Ile Thr Ser Val Leu Phe Ile Ile
                85                  90                  95

Leu Leu Pro Val Asn Leu Ile Val Ala Gly Leu Arg Leu Phe Met Pro
            100                 105                 110

Leu Pro Pro Lys Lys Ile Val Glu Asp Leu Ser Glu Pro Thr Thr Glu
        115                 120                 125

Glu Thr Asn Glu Val Ile Gln Pro Phe Ile Phe Ala Leu Gln Ala Leu
    130                 135                 140

Leu Phe Glu Asp Asn Lys Leu Arg Ser Phe Lys Ile Val Glu Gln Ser
145                 150                 155                 160

Val Gly Lys Ala Pro Leu Pro Asn Pro Phe Leu Asn Arg Leu Val Ala
                165                 170                 175

Ile Ser Pro Gln Glu Ser Gln Glu Ala Met Arg Lys Ile Pro Asp Leu
            180                 185                 190

Cys Ser Gln Leu Lys Lys Val Leu Lys Ser Leu Gly Val Leu Thr Pro
        195                 200                 205

Glu Trp Lys His Met Leu Lys Tyr Phe Glu Gly Leu Lys Asn Glu His
    210                 215                 220

Asp Ser Asn Pro Asp Lys Lys Thr Phe Pro Ile Leu Ile Lys Leu Leu
225                 230                 235                 240

Ile Glu Ala Leu Thr Gly Lys Ser Ser Leu Pro Lys Thr Pro Ser Thr
                245                 250                 255

Lys Glu Lys Met Gln Ala Ala Leu Phe Ile Ala Ser Ser Cys Lys Thr
            260                 265                 270

Cys Lys Pro Thr Trp Gly Glu Val Ile Thr Arg Ser Leu Asn Arg Leu
        275                 280                 285

Tyr Ser Ile Ala Asn Glu Gly Asp Asn Gln Leu Leu Ile Trp Val Gln
```

290 295 300

Glu Phe Lys Glu Arg Glu Leu Met Ser Ile Gln Asp Gly Asp Asp Ala
305 310 315 320

Glu Glu Tyr Arg Phe Ala Ala Gln Gln His Gly Glu Arg Tyr Thr Glu
325 330 335

Ala Ile Glu Gln Val Leu Arg Asn Glu Ser Ala Ala Lys Leu Gln Trp
340 345 350

His Val Ile Asn Thr Met Lys Phe Phe His Gly Lys Asn Leu Gly Leu
355 360 365

Val Thr Glu His Leu Gln Asp Thr Leu Gly Ala Leu Thr Leu Arg Gln
370 375 380

Thr Thr Val Asp Thr His Gln Gly Arg Glu Asp Ala Asp Leu Ser Ala
385 390 395 400

Ala Leu Phe Leu Asn Lys Tyr Leu Asn Ser Gly Asn Gln Leu Val Asn
405 410 415

Ser Val Phe Lys Ser Met Gln Lys Ala Asp Pro Glu Thr Lys Ala Leu
420 425 430

Ile Arg Glu Phe Ala Leu Asp Ile Leu Tyr Ala Ser Leu Arg Leu Pro
435 440 445

Gln Thr Ser Ala His Thr Glu Val Phe Ser Thr Leu Leu Met Asp Pro
450 455 460

Glu Thr Tyr Glu Pro Asn Lys Ala Cys Ile Ala Tyr Leu Leu Tyr Val
465 470 475 480

Leu Lys Ile Ile Glu Leu
485

<210> 278
<211> 1461
<212> DNA
<213> Chlamydia pneumoniae

<400> 278
atgcatatgt ctaaccccat ctctttgttt tcccctgcag agttaatagc aaagtacaat 60
ttaattccaa aaacttcgcc gatttatcct cggaggacgg aacttattat cttggaagaa 120
aatgcgtgtc aaacacgcct aaccaacgtg gctcaggtcc tacatccttc tagcctattc 180
agtatgtcaa aaaaaatact gaatccctgc gggtgctctg gtggtccctt atgttgggtg 240
attctcaaca tcctagcatt tattattact tcagtactgt ttatcattct tttaccggtg 300
aatctcatcg tagcaggtct tcgtctcttc atgcctcttc cccctaaaaa aatcgtagag 360
gatttaagtg aacctactac tgaagaaacg aatgaggtca ttcaaccctt cattttcgct 420
ttgcaagcgt tgctttttga ggataacaaa cttcgctctt ttaaaattgt tgaacaaagt 480
gtaggcaaag caccctacc taatcccttt ttaaatagac tagtagcaat ttcgccgcaa 540
gaaagccaag aagccatgcg gaagattccg gatctatgct cacaactgaa aaaagtatta 600
aagtctctag gcgtgctaac tccagaatgg aagcacatgc tgaagtactt tgagggactg 660
aaaaacgaac atgatagtaa tcctgataaa aagacgttcc caatattgat caagctcctc 720
atagaagctc ttactggaaa gtcctcttta cccaaaactc ctagtacaaa ggaaaaaatg 780
caagcggcct tatttattgc aagttcttgc aagacttgta agccgacttg gggagaagtc 840
ataaccagat ctcttaacag actctatagt atagctaatg aaggagacaa tcagcttctg 900
atttgggttc aagagtttaa agaacgagag ctgatgtcca tccaagatgg tgatgatgct 960
gaagagtatc ggtttgcggc tcagcaacac ggtgagcgtt acacagaggc aatagaacaa 1020

```
gttctacgaa acgagtcagc agccaaacta caatggcatg tgatcaacac tatgaaattc   1080
ttccatggga aaaatctcgg tctagttaca gaacacctac aagatactct cggcgcccta   1140
actttacgtc aaactacagt ggacacacat caaggcagag aagacgctga tttgtcagct   1200
gctcttttcc taaataagta tttaaattct ggaaatcaac ttgttaatag cgtctttaaa   1260
tccatgcaaa aagcagatcc agaaaccaaa gctttaatcc gtgagtttgc tctagatata   1320
ttatatgcat ccttacggct tcctcaaact tccgctcata ccgaggtctt ttctacactc   1380
ttaatggacc cagagaccta tgaacctaat aaagcttgta tcgcctactt gctctatgta   1440
ttaaagatca tcgaactata a                                              1461

<210>   279
<211>   527
<212>   PRT
<213>   Chlamydia pneumoniae

<400>   279
Met Pro Gly Ser Val Ser Ser Pro Pro Leu Ser Pro Val Ile Val Arg
1               5                   10                  15

Glu Arg Val Pro Ser Ser Ser Gly Ser Asp Leu Ile Gln Pro His Ala
            20                  25                  30

Val Leu Lys Ile Ser Ile Leu Ile Phe Ala Leu Val Thr Ile Leu Gly
        35                  40                  45

Ile Val Leu Val Val Leu Ser Ser Ala Leu Gly Ala Leu Pro Ser Leu
    50                  55                  60

Val Leu Thr Val Ser Gly Cys Ile Ala Ile Ala Val Gly Leu Ile Gly
65                  70                  75                  80

Leu Gly Ile Leu Val Thr Arg Leu Ile Leu Ser Thr Ile Arg Lys Val
                85                  90                  95

Asp Ala Met Gly Tyr Asp Ala Ala Val Lys Glu Glu Gln Tyr Leu Ser
            100                 105                 110

Arg Ile Arg Glu Leu Glu Ser Glu Asn Arg Glu Ile Arg Asp Arg Asn
        115                 120                 125

Arg Ala Val Glu Asp Gln Cys Ala His Leu Ser Glu Glu Asn Lys Asp
    130                 135                 140

Leu Arg Asp Pro Glu Tyr Leu His Gly Met Thr Glu Arg Leu Ile Ala
145                 150                 155                 160

Ser Leu Glu Ile Glu Asn Gln Ala Leu Val Ala Glu Asn Ile Leu Leu
                165                 170                 175

Lys Asp Trp Asn Ala Ser Leu Ser Arg Asp Phe Arg Ala Tyr Lys Gln
            180                 185                 190

Lys Phe Pro Leu Gly Ala Leu Glu Pro Trp Lys Glu Asp Ile Ala Cys
        195                 200                 205

Ile Met Glu Gln Asn Leu Phe Leu Lys Pro Glu Cys Ile Ala Met Val
    210                 215                 220

Lys Ser Leu Pro Leu Glu Thr Gln Arg Leu Phe Leu Tyr Pro Lys Gly
225                 230                 235                 240

Phe Gln Ser Leu Val Asn Arg Phe Ala Pro Arg Ser Arg Phe Phe Gln
```

245 250 255

Thr Pro Lys Tyr Glu Tyr Asn Ser Arg Asn Glu Asn Glu Asp Gly Lys
260 265 270

Val Ala Ala Val Cys Ala Arg Leu Lys Lys Glu Phe Phe Ser Ala Val
275 280 285

Leu Gly Ala Cys Ser Tyr Glu Glu Leu Gly Gly Ile Cys Glu Arg Ala
290 295 300

Val Ala Leu Lys Glu Thr Leu Pro Leu Pro Glu Ala Val Tyr Asp Thr
305 310 315 320

Leu Val Gln Glu Phe Pro Asn Leu Leu Thr Ala Glu Ser Leu Trp Lys
325 330 335

Glu Trp Cys Phe Tyr Ser Tyr Pro Tyr Leu Arg Pro Tyr Leu Ser Val
340 345 350

Asp Tyr Cys Lys Arg Leu Phe Val Gln Leu Phe Glu Glu Leu Cys Leu
355 360 365

Lys Leu Phe Thr Thr Gly Ser Pro Glu Asp Gln Ala Leu Val Arg Leu
370 375 380

Phe Ser Tyr Tyr Arg Asn His Ile Pro Ala Val Leu Ala Ser Phe Gly
385 390 395 400

Leu Pro Pro Pro Glu Thr Gly Gly Ser Val Phe Val Leu Leu Pro Lys
405 410 415

Gln Glu Asn Leu Leu Trp Ser Gln Ile Glu Val Leu Ala Thr Arg Tyr
420 425 430

Leu Lys Asp Thr Phe Val Arg Asn Ser Glu Trp Thr Gly Ser Phe Glu
435 440 445

Met Met Phe Ser Tyr Asn Glu Met Cys Lys Glu Ile Ser Glu Gly Arg
450 455 460

Ile Arg Phe Ala Glu Asp Tyr Glu Thr Arg His Ser Glu Glu Phe Pro
465 470 475 480

Pro Ser Pro Leu Ser Glu Glu Gly Glu Gly Glu Glu Phe Leu Pro Pro
485 490 495

Cys Ser Glu Glu Glu Val Ser Val Leu Glu Arg Pro Asp Leu Asp Val
500 505 510

Asp Ser Met Trp Val Trp His Pro Pro Val Pro Lys Gly Pro Leu
515 520 525

<210> 280
<211> 1584
<212> DNA
<213> Chlamydia pneumoniae

<400> 280
atgccaggtt ctgtgtcatc acctcctttg tctcctgtaa ttgtccgtga aagggtccca 60
tcctcttcag gatccgacct catacagcct catgctgttt taaagatctc catcctaatt 120

```
tttgcgcttg tgacaatttt aggaattgtt cttgtagtgt tgtctagtgc tttaggagct    180
cttcctagtt tagttttgac ggtttctggt tgtattgcaa tagctgtagg cctgattggt    240
ttagggattc ttgtgacacg gctgattctc tctacgatca gaaaagtaga tgccatgggt    300
tatgatgctg cggtcaaaga agagcagtat ttgtcacgta tcagagaatt agagtctgaa    360
aatagagaga ttagagatag aaatcgtgct gtcgaagatc agtgtgccca tttatccgaa    420
gagaacaagg accttaggga tcccgaatat ctacatggaa tgactgaaag gctcattgcg    480
agcttagaaa tagagaatca agctctcgta gctgagaaca ttcttctcaa agactggaat    540
gcaagcctat ctagagattt ccgcgcatat aagcaaaaat ttcctcttgg ggcattagaa    600
ccctggaaag aagatattgc atgtatcatg gaacaaaatc tctttttaaa accggaatgt    660
atcgcgatgg ttaagtctct tccattagag acgcaacggc tgtttttata tccaaaagga    720
tttcagtctt tagttaatcg atttgctccg cggtctcgct ttttccagac tccaaagtat    780
gaatataaca gtaggaatga aaatgaggac ggaaaggtag ccgcagtgtg cgcccgtttg    840
aaaaaagaat tcttcagtgc tgttttagga gcctgtagtt acgaagaact aggggcatt     900
tgtgaaagag cagtagcact taaagagacg ttgccattgc ctgaagctgt ctatgatacc    960
ctagttcagg agttcccaaa tcttcttact gctgagagtt tatggaaaga atggtgcttc   1020
tattcctatc cctaccttcg tccctatctt tctgtggatt actgtaagag gttatttgta   1080
caacttttg aggaactctg cctaaagctt tttacaacgg gatctccaga agaccaagct    1140
ttggttcgcc ttttctctta ctataggaat catattcccg cagtcttggc ctcatttggt   1200
ttgccccgc ctgagacagg ggggtctgta tttgtattgc taccaaaaca agaaaacctt    1260
ctttggagtc aaattgaggt gctggctaca aggtatctca aagatacctt cgtgagaaac   1320
tcagaatgga cgggctcttt cgagatgatg ttttcttata acgagatgtg taaggagatc   1380
tccgaaggaa ggattcgttt tgctgaagac tatgaaacga ggcattccga agaattccct   1440
ccttcccctc tctctgaaga aggagagggc gaagaattcc ttcctccttg ctctgaagaa   1500
gaggtttcgg ttcttgagcg cccagatcta gatgtagact ctatgtgggt ctggcatccg   1560
ccggtcccta agggacctct ttaa                                          1584
```

<210> 281
<211> 423
<212> PRT
<213> Chlamydia pneumoniae

<400> 281

```
Met Asp Lys Gln Ser Ser Gly Asn Ser Gly Cys Ile Trp His Pro Phe
1               5                   10                  15

Thr Gln Ser Ala Leu Asp Ser Thr Pro Ile Lys Ile Val Arg Gly Glu
            20                  25                  30

Gly Ala Tyr Leu Tyr Ala Glu Ser Gly Thr Arg Tyr Leu Asp Ala Ile
        35                  40                  45

Ser Ser Trp Trp Cys Asn Leu His Gly His Gly His Pro Tyr Ile Thr
    50                  55                  60

Lys Lys Leu Cys Glu Gln Ala Gln Lys Leu Glu His Val Ile Phe Ala
65                  70                  75                  80

Asn Phe Thr His Glu Pro Ala Leu Glu Leu Val Ser Lys Leu Ala Pro
                85                  90                  95

Leu Leu Pro Glu Gly Leu Glu Arg Phe Phe Phe Ser Asp Asn Gly Ser
            100                 105                 110

Thr Ser Ile Glu Ile Ala Met Lys Ile Ala Val Gln Tyr Tyr Tyr Asn
        115                 120                 125

Gln Asn Lys Ala Lys Ser His Phe Val Gly Leu Ser Asn Ala Tyr His
    130                 135                 140

Gly Asp Thr Phe Gly Ala Met Ser Ile Ala Gly Thr Ser Pro Thr Thr
145                 150                 155                 160
```

Val Pro Phe His Asp Leu Phe Leu Pro Ser Ser Thr Ile Ala Ala Pro
165 170 175

Tyr Tyr Gly Lys Glu Glu Leu Ala Ile Ala Gln Ala Lys Thr Val Phe
180 185 190

Ser Glu Ser Asn Ile Ala Ala Phe Ile Tyr Glu Pro Leu Leu Gln Gly
195 200 205

Ala Gly Gly Met Leu Met Tyr Asn Pro Glu Gly Leu Lys Glu Ile Leu
210 215 220

Lys Leu Ala Lys His Tyr Gly Val Leu Cys Ile Ala Asp Glu Ile Leu
225 230 235 240

Thr Gly Phe Gly Arg Thr Gly Pro Leu Phe Ala Ser Glu Phe Thr Asp
245 250 255

Ile Pro Pro Asp Ile Ile Cys Leu Ser Lys Gly Leu Thr Gly Gly Tyr
260 265 270

Leu Pro Leu Ala Leu Thr Val Thr Thr Lys Glu Ile His Asp Ala Phe
275 280 285

Val Ser Gln Asp Arg Met Lys Ala Leu Leu His Gly His Thr Phe Thr
290 295 300

Gly Asn Pro Leu Gly Cys Ser Ala Ala Leu Ala Ser Leu Asp Leu Thr
305 310 315 320

Leu Ser Pro Glu Cys Leu Gln Gln Arg Gln Met Ile Glu Arg Cys His
325 330 335

Gln Glu Phe Gln Glu Ala His Gly Ser Leu Trp Gln Arg Cys Glu Val
340 345 350

Leu Gly Thr Val Leu Ala Leu Asp Tyr Pro Ala Glu Ala Thr Gly Tyr
355 360 365

Phe Ser Gln Tyr Arg Asp His Leu Asn Arg Phe Phe Leu Glu Arg Gly
370 375 380

Val Leu Leu Arg Pro Leu Gly Asn Thr Leu Tyr Val Leu Pro Pro Tyr
385 390 395 400

Cys Ile Gln Glu Glu Asp Leu Arg Ile Ile Tyr Ser His Leu Gln Asp
405 410 415

Ala Leu Cys Leu Gln Pro Gln
420

<210> 282
<211> 1272
<212> DNA
<213> Chlamydia pneumoniae

<400> 282
atggacaagc aatcatcagg gaattcaggg tgtatctggc accccttcac tcaatctgca 60
ttagattcta cacccataaa gattgtaagg ggagaaggtg cttacctcta tgcggaatca 120
ggaacaagat atcttgatgc gatatcttca tggtggtgca acctccacgg tcatggcat 180

```
ccctacatta caaaaaaatt atgtgagcaa gcacagaagt tagaacatgt gatcttcgca    240
aatttcaccc atgaaccggc tctagagctc gtatcgaaac tcgctcccct ccttcctgaa    300
ggtctagaac gtttcttttt ctctgacaac ggatcaacgt ctatcgaaat agcaatgaaa    360
attgctgtgc aatattacta caatcaaaac aaggctaaga gccattttgt tggactcagc    420
aatgcctatc acggagatac atttggagct atgtcgatag ctggcacgag ccctactaca    480
gttccctttc atgatctttt tcttccttcc agtacaattg ctgctcccta ttatggcaag    540
gaagagcttg ccattgccca agcaaaaaca gtcttttctg aaagcaatat cgcagcgttt    600
atctatgagc cgctattgca aggtgctgga gggatgttaa tgtataatcc cgaaggccta    660
aaggagattc tcaagcttgc caagcattac ggggttctct gtattgctga tgaaattctt    720
actggctttg gccgtacggg tccactgttt gcttctgaat ttacagacat tcctcctgac    780
attatctgtc tttctaaagg tcttacagga ggctatctcc ctctagcctt gacagtaacc    840
actaaagaaa ttcatgatgc ctttgtctcc caagatcgga tgaaggcact gcttcatggc    900
cataccttca caggaaatcc tttaggctgt agtgctgccc tcgcttcttt ggatctcacc    960
ctatctccag aatgcctaca acaaaggcaa atgatagaac ggtgtcatca agagtttcaa   1020
gaagctcatg gttccctatg gcaacggtgt gaggttctgg gcacggtact cgctctagat   1080
taccctgcag aagctacagg atatttttca caatatagag accatctcaa tcgctttttc   1140
ttagaacgtg gagtccttct tcgtccttta gggaacacac tgtatgtgct gcccccctac   1200
tgtatccaag aagaagatct ccggattatt tattctcacc tacaggatgc cctatgtcta   1260
caaccacagt aa                                                       1272
```

<210> 283
<211> 331
<212> PRT
<213> Chlamydia pneumoniae

<400> 283

```
Met Arg Glu Glu Thr Val Ser Trp Ser Leu Glu Asp Ile Arg Glu Ile
1               5                   10                  15

Tyr His Thr Pro Val Phe Glu Leu Ile His Lys Ala Asn Ala Ile Leu
            20                  25                  30

Arg Ser Asn Phe Leu His Ser Glu Leu Gln Thr Cys Tyr Leu Ile Ser
        35                  40                  45

Ile Lys Thr Gly Gly Cys Val Glu Asp Cys Ala Tyr Cys Ala Gln Ser
    50                  55                  60

Ser Arg Tyr His Thr His Val Thr Pro Glu Pro Met Met Lys Ile Val
65                  70                  75                  80

Asp Val Val Glu Arg Ala Lys Arg Ala Val Glu Leu Gly Ala Thr Arg
                85                  90                  95

Val Cys Leu Gly Ala Ala Trp Arg Asn Ala Lys Asp Asp Arg Tyr Phe
            100                 105                 110

Asp Arg Val Leu Ala Met Val Lys Ser Ile Thr Asp Leu Gly Ala Glu
        115                 120                 125

Val Cys Cys Ala Leu Gly Met Leu Ser Glu Glu Gln Ala Lys Lys Leu
    130                 135                 140

Tyr Asp Ala Gly Leu Tyr Ala Tyr Asn His Asn Leu Asp Ser Ser Pro
145                 150                 155                 160

Glu Phe Tyr Glu Thr Ile Ile Thr Thr Arg Ser Tyr Glu Asp Arg Leu
                165                 170                 175

Asn Thr Leu Asp Val Val Asn Lys Ser Gly Ile Ser Thr Cys Cys Gly
            180                 185                 190
```

Gly Ile Val Gly Met Gly Glu Ser Glu Glu Asp Arg Ile Lys Leu Leu
195 200 205

His Val Leu Ala Thr Arg Asp His Ile Pro Glu Ser Val Pro Val Asn
210 215 220

Leu Leu Trp Pro Ile Asp Gly Thr Pro Leu Gln Asp Gln Pro Pro Ile
225 230 235 240

Ser Phe Trp Glu Val Leu Arg Thr Ile Ala Thr Ala Arg Val Val Phe
245 250 255

Pro Arg Ser Met Val Arg Leu Ala Ala Gly Arg Ala Phe Leu Thr Val
260 265 270

Glu Gln Gln Thr Leu Cys Phe Leu Ala Gly Ala Asn Ser Ile Phe Tyr
275 280 285

Gly Asp Lys Leu Leu Thr Val Glu Asn Asn Asp Ile Asp Glu Asp Ala
290 295 300

Glu Met Ile Lys Leu Leu Gly Leu Ile Pro Arg Pro Ser Phe Gly Ile
305 310 315 320

Glu Arg Gly Asn Pro Cys Tyr Ala Asn Asn Ser
325 330

<210> 284
<211> 996
<212> DNA
<213> Chlamydia pneumoniae

<400> 284
atgcgtgaag aaactgtatc ctggtcatta gaagacatcc gcgaaattta tcacactccc 60
gtatttgagc tgattcacaa agccaatgcc atattgcgta gtaatttcct ccattcagaa 120
ctgcagactt gctatctgat ttcgattaaa actggtggat gcgttgaaga ttgcgcctac 180
tgtgcccaat cttcccgcta tcatacccac gtcacaccag aacctatgat gaaaattgta 240
gacgttgtgg aaagggcaaa acgtgctgta gagctaggcg ccactcgtgt gtgtcttggg 300
gctgcctggc gcaatgctaa ggacgatcga tactttgata gagtcctcgc tatggtgaaa 360
agtatcacag atctcggagc cgaggtttgt tgtgctttag gcatgctctc cgaagagcaa 420
gctaaaaaac tgtatgatgc aggactttat gcctacaatc ataatttaga ctcttctccg 480
gaattctatg aaactataat cacaacacgt tcttatgaag atcgcctcaa cactcttgat 540
gtagtaaata aatctggcat tagtacatgc tgcggtggta ttgtaggtat gggagaatct 600
gaagaagacc gtataaagct tcttcatgtt cttgcaacaa gagatcatat cccagaatcc 660
gtacctgtaa atttactttg gccgattgac ggcacgcctt tgcaagacca gcctccgatt 720
tctttctggg aagtcttgcg aaccatagca acggcacggg ttgttttccc cagatccatg 780
gtacgacttg ctgcaggacg cgctttcctc acagtagaac aacaaacctt atgttttcta 840
gccggtgcca actccatatt ctatggagat aaactgttga ctgtagaaaa caatgatata 900
gatgaagatg ctgaaatgat caaactttta ggcttaatcc ctcgcccttc atttggaata 960
gaaagaggta acccatgtta tgccaacaat tcctaa 996

<210> 285
<211> 480
<212> PRT
<213> Chlamydia pneumoniae

<400> 285
Met Val Cys Pro Asn Asn Ser Trp Phe Arg Met Cys Gly Asn Phe Asn
1 5 10 15

Cys Glu Trp Val Glu Val Thr Thr Thr Glu Glu Thr Thr Arg Gln Ser
20 25 30

Ala Ser Asp Ile Ser Glu Glu Ala Gly Ser Ser Gly Gly Ala Ala Pro
35 40 45

Ile Thr Thr Gln Pro Thr Lys Ile Thr Lys Val Glu Lys Arg Val Gln
50 55 60

Phe Asn Thr Ala Gln Gly Asp Glu Ser Thr Ile His Met Ile Gln Glu
65 70 75 80

Ala Gly Glu Leu Val Asp Ser Ile Leu Ser His Arg Arg Thr Gln Gly
85 90 95

Cys Thr Glu Tyr Cys Tyr Asp Ser Tyr Ala Thr Gly Cys Gly Gln Arg
100 105 110

Cys Gly Ser Phe Gly Arg Leu Ile Cys Gly Thr Tyr Lys Ala Cys Cys
115 120 125

Leu Asp Arg Glu Asp Asn Gln Val Ala Gly Leu Val His Glu Cys Glu
130 135 140

Gln Thr His Gly Pro Ile Ala Val Ala Leu Ala Ala Lys Thr Met Gly
145 150 155 160

Leu Asn Leu Met Glu Leu Val Glu Lys Asn Thr Ile Leu Ser Glu Glu
165 170 175

Gln Lys Asn Glu Phe Arg Gln His Cys Ser Glu Ala Lys Thr Gln Leu
180 185 190

Tyr Gly Thr Met Gln Ser Leu Ser Gln Asn Phe Phe Leu Glu Gly Val
195 200 205

Asn Ser Ile Arg Glu Arg Gly Leu Asp Asp Ser Leu Val Gln Ala Val
210 215 220

Leu Ser Phe Ile Ala Thr Arg Ser Trp Glu Lys Thr Ile Glu Ser Glu
225 230 235 240

Glu Ala Ser Gly Thr Ser Ser Ala Ser Asn Ser Thr Arg Ile Pro Ala
245 250 255

Cys Tyr Ile Leu Asn Thr Ser Pro Leu Thr Thr Ser Arg Leu Ser Cys
260 265 270

Gly Ser Arg Asp Ala Arg Arg Pro Ser Ser Val Gly Ala Glu Pro Gln
275 280 285

Tyr Val Ala Lys Lys Tyr Asn Asp Asn Gly Met Ala Arg Gln Leu Gly
290 295 300

Lys Ile Gln Val Thr Asn Leu Lys Thr Gly Asp Phe Ser Ala Leu Gly
305 310 315 320

Pro Phe Gly Leu Leu Ile Val Lys Met Leu Asn Ser Phe Leu Leu Ser
325 330 335

Ala Ser Gln Ser Thr Ser Ser Ile Leu Lys His Thr Gly Gly Glu Ile

```
            340                 345                 350

Cys Tyr Thr Cys Pro Asn Phe Arg Asp Ile Val Val Leu Leu Met Leu
        355                 360                 365

Ala Ile Gly Tyr Cys Pro Ala Asn Thr Asp Glu Thr Ser Val Val Asp
    370                 375                 380

Ile His Met Ile Asp Asp Pro Ile Met Thr Ile Phe Tyr Arg Leu Gln
385                 390                 395                 400

Tyr Ser Tyr Arg Thr Gly Lys Thr Ser Ala Ser Phe Leu Lys Lys Lys
                405                 410                 415

Pro Ser Leu Val Arg Gln Glu Ser Leu Asp Cys Pro Thr Pro Ala Glu
            420                 425                 430

Ser Val Pro Leu Met Ser Ser Leu Glu Glu Glu Asp Glu Asn Glu Asp
        435                 440                 445

Asp Asp Glu Asp Gly Asn Leu Ala Tyr Gln Gln Arg Ile Leu Glu Cys
    450                 455                 460

Ser Gly His Leu Gln Thr Leu Phe Leu Gly Ile Lys Ile Asn Lys Glu
465                 470                 475                 480


<210>   286
<211>   1443
<212>   DNA
<213>   Chlamydia pneumoniae

<400>   286
atggtttgcc caaataattc ttggttcaga atgtgtggaa atttcaactg cgaatgggtt      60
gaagtaacaa caacagaaga aacaacgcgg caatcggctt cagatataag cgaagaagct     120
ggttcgagtg gaggagctgc tcctataact acgcaaccta ctaaaattac aaaagtagag     180
aaacgtgtcc aatttaatac tgctcaaggt gatgaaagta caatacacat gatccaagaa     240
gcaggagaat tggtagactc cattctatca catagacgaa cgcaaggatg tacagagtat     300
tgttatgaca gttacgcaac tggatgtggt cagcgttgcg gatcttttgg aagactcatt     360
tgtggaacgt ataaagcgtg ttgcttagac agagaggata atcaggttgc tggacttgtc     420
catgaatgcg aacagaccca tggtcctatt gccgttgctt tagctgctaa aactatgggc     480
ctcaacttaa tggaacttgt agaaaaaaac actattttgt ctgaagaaca gaaaaatgaa     540
tttagacagc attgctcgga agctaaaacc caactctatg gaacgatgca gagcctttct     600
caaaactttt tccttgaagg agtcaacagc attagagaac gcggtctaga cgattcacta     660
gtccaagccg tgctaagctt tattgctaca aggtcttggg aaaaaactat agaatcagag     720
gaagcctcag gaacatcttc tgcttctaat tctacacgca ttcctgcgtg ctatatctta     780
aatacgagcc ccttaacgac gtcacgccta tcctgtggat caagagatgc gcgacgccca     840
tcttcagtcg gtgcagagcc ccagtacgta gcaaaaaaat acaatgacaa tggcatggcc     900
agacaattag gaaaaatcca agtcaccaat ctaaaaacag gagatttttc agctttaggt     960
ccttttggtc tcctgattgt gaaaatgctg aatagctttc tcttatctgc atcacaaagc    1020
acatcttcta ttctaaagca cacaggtgga gaaatatgtt atacgtgccc aaattttcgt    1080
gatatcgtcg ttttattgat gttagcgatt ggctattgcc ctgcaaatac cgatgagaca    1140
tctgtcgtag atatacacat gatagatgat ccgattatga ccatcttcta tcgactacaa    1200
tacagctata gaacagggaa aacttcagca tcgtttttaa aaaagaaacc ctcattagta    1260
agacaggaaa gtcttgattg tcctacccct gcagaatctg tccctctcat gtcaagtctc    1320
gaagaagaag atgaaaatga agatgatgat gaggatggga atttggcgta tcaacagcgt    1380
atccttgaat gctcgggtca tttacaaact ctatttttag ggataaaaat aaacaaagaa    1440
taa                                                                   1443

<210>   287
<211>   167
```

<212> PRT
<213> Chlamydia pneumoniae

<400> 287
Met Thr Arg Ser Thr Ile Glu Ser Ser Asp Ser Leu Cys Ser Arg Ser
1 5 10 15

Phe Ser Gln Lys Leu Ser Val Gln Thr Leu Lys Asn Leu Cys Glu Ser
20 25 30

Arg Leu Met Lys Ile Thr Ser Leu Val Ile Ala Phe Leu Thr Leu Ile
35 40 45

Val Gly Gly Ala Leu Ile Ala Leu Ala Gly Gly Gly Val Leu Ser Phe
50 55 60

Pro Leu Gly Leu Ile Leu Gly Ser Val Leu Val Leu Phe Ser Ser Ile
65 70 75 80

Tyr Leu Val Ser Cys Cys Lys Phe Phe Thr Leu Lys Glu Met Thr Met
85 90 95

Thr Cys Ser Val Lys Ser Lys Ile Asn Ile Trp Phe Glu Lys Gln Arg
100 105 110

Asn Lys Asp Ile Glu Lys Ala Leu Glu Asn Pro Asp Leu Phe Gly Glu
115 120 125

Asn Lys Arg Asn Val Gly Asn Arg Ser Ala Arg Asn Gln Leu Glu Met
130 135 140

Ile Leu His Glu Thr Asp Gly Ile Ile Leu Lys Arg Tyr Met Lys Gly
145 150 155 160

Ala Lys Met Tyr Phe Tyr Leu
165

<210> 288
<211> 504
<212> DNA
<213> Chlamydia pneumoniae

<400> 288
atgactagaa gtactattga aagcagtgat tcgctatgct caaggtcttt ttctcaaaaa 60
ttaagtgtcc agacattaaa aaatctctgt gaaagtagat taatgaagat cacttctctt 120
gtgattgctt tcctaactct aattgtgggg ggtgctctta tagctttagc aggagggggg 180
gttctttctt tccctcttgg gctaatctta ggaagcgtac tcgttttgtt ttcttctatc 240
tatttagtct cttgttgtaa attttttact ttaaaagaga tgacaatgac ctgtagtgtc 300
aaatctaaaa tcaatatatg gtttgaaaag caacgaaaca aagacatcga aaaggcatta 360
gagaatccag atctctttgg agaaaataag agaaatgttg gaaatcgttc ggcaagaaat 420
caactagaaa tgatcttaca cgagactgac ggaattattt tgaaaagata tatgaaagga 480
gctaaaatgt acttttattt atga 504

<210> 289
<211> 195
<212> PRT
<213> Chlamydia pneumoniae

<400> 289
Met Asn Trp Val Pro Lys Thr Ile Asp His Val Asp Pro Glu Ser Glu
1 5 10 15

```
Ile Asp Ile Arg Lys Val Val Ser Cys Tyr Lys Leu Ile Lys Glu Cys
            20                  25                  30

Gln Pro Glu Phe Arg Ser Leu Ile Ser Glu Leu Leu Gly Val Ile Arg
        35                  40                  45

Cys Gly Leu Arg Leu Leu Lys Arg Ser Lys Tyr Gln Glu Gln Ala Arg
    50                  55                  60

Thr Val Ser Asp Glu Asp Ala Pro Leu Phe Cys Leu Thr Arg Ser Tyr
65                  70                  75                  80

Tyr Gln Asp Gly Tyr Leu Thr Pro Leu Arg Ala Gly Pro Arg Asp Leu
                85                  90                  95

Ile Asn His Tyr Ile His Leu Arg Arg Arg Glu Asn Pro Lys His Phe
            100                 105                 110

Phe Ser Pro Lys His Pro Cys Tyr Tyr Ala Arg Leu Ala Phe Asn Glu
        115                 120                 125

Ser Val Cys Val Tyr Arg Glu Leu Phe Asp Ile Glu Arg Leu Thr Lys
    130                 135                 140

Met Tyr Val Glu Gly Asp Tyr Ser Lys Glu Gln Glu Lys Asn Leu Gln
145                 150                 155                 160

Ala Ile Leu Ser Phe Val Lys Thr Leu Asp Glu Gly Lys Asp Phe Leu
                165                 170                 175

Ile Glu His Lys Asp Thr Asp Leu Ile Gly Arg Gly Phe Thr Asp Val
            180                 185                 190

Phe Cys Thr
        195

<210>   290
<211>   588
<212>   DNA
<213>   Chlamydia pneumoniae

<400>   290
atgaattggg ttccaaaaac aatagaccat gtagatccag aatcagagat agatatacgt   60
aaagtcgtct cctgctataa gttgataaaa gaatgtcaac ctgaatttcg atctcttata   120
agtgaattac taggagtgat tcggtgtggc ttaagactat taaaacgttc taagtatcaa   180
gaacaggcta gaactgtatc tgatgaagat gcacctcttt tctgcctgac tcgttcttat   240
tatcaagatg gttatctcac gccattaaga gcaggacctc gtgatcttat aaatcactat   300
atacacttgc gtcgccgaga gaatcctaag cattttttca gtcctaagca tccatgttat   360
tatgctcgat tggcttttaa tgagtcagtg tgtgtctata gagaactctt tgatatagag   420
cgacttacaa aaatgtatgt cgagggtgat tattctaaag aacaagagaa aaacctacag   480
gctattctta gttttgtgaa aactctagat gaaggaaagg actttcttat tgaacataaa   540
gataccgatc tcattgggag aggttttact gatgtgttct gcacttaa               588

<210>   291
<211>   155
<212>   PRT
<213>   Chlamydia pneumoniae

<400>   291
Met Gly Tyr Leu Pro Val Ser Ala Thr Asp Val Leu Phe Glu Ser Pro
```

```
1               5                   10                  15

Ala Ala Pro Leu Ile Asn Ser Ala Asn Thr Gln Asn Gln Lys Leu Ile
            20                  25                  30

Glu Leu Lys Gly Lys Gln Gln Ala Glu Ser Ser Pro Arg Thr Ile Thr
        35                  40                  45

Ser Val Ile Leu Glu Val Leu Leu Val Ile Gly Cys Cys Leu Ile Val
    50                  55                  60

Leu Ser Leu Leu Ala Ile Arg Pro Ala Leu Gln Phe Thr Leu Glu Thr
65                  70                  75                  80

Gly His Pro Ala Ala Ile Ala Val Leu Ala Val Ser Gly Thr Ile Leu
                85                  90                  95

Leu Val Ala Val Ile Ile Leu Phe Cys Phe Leu Ala Ala Val Pro Phe
            100                 105                 110

Ala Ala Lys Lys Thr Tyr Lys Tyr Val Lys Thr Val Asp Asp Tyr Ala
        115                 120                 125

Ser Trp His Ser His Gln Gln Thr Pro Thr Leu Gly Thr Ile Phe Ser
    130                 135                 140

Gly Ile Val Tyr Ala Glu Ser Gln Ala Gln Leu
145                 150                 155
```

<210> 292
<211> 468
<212> DNA
<213> Chlamydia pneumoniae

<400> 292

```
atgggatatc ttccagtatc tgctacggac gttctttttg aaagtccagc cgctccctta    60
atcaatagcg caaacacaca aaatcagaaa ctcatagaac tcaaggggaa gcagcaagct   120
gagtcttctc cacggacaat cacttctgtc atattggaag ttctcctagt gatcggatgc   180
tgcctcatag ttcttagttt attggcaatc cgccctgctc tgcaattcac tctagaaact   240
ggacatccag ctgccattgc agtccttgct gtctcaggaa caattctatt ggtggctgtt   300
atcatcttgt tttgctttct agcagctgtg ccattcgctg ctaagaaaac ttataaatat   360
gttaagacgg ttgatgacta tgcttcttgg cattctcatc agcaaacacc gaccctaggc   420
actatctttt caggtatcgt ctatgcagaa tcccaggcgc aattatag                468
```

<210> 293
<211> 175
<212> PRT
<213> Chlamydia pneumoniae

<400> 293

```
Val Ser Ser Thr Leu Asn Gly Val Phe Pro Ser Ser Leu Pro Glu Glu
1               5                   10                  15

Ser Ala Asp Leu Phe Ile Thr Asn Lys Glu Ile Val Ala Leu Gly Glu
            20                  25                  30

Lys Gly Asn Val Phe Leu Thr His Ser Ile Pro Met His Ile Ala Ala
        35                  40                  45

Ile Thr Ile Leu Val Ile Val Ala Leu Ala Gly Ile Ala Ile Ile Cys
    50                  55                  60
```

Leu Gly Cys Tyr Ser Gln Ser Ile Leu Leu Ile Ala Val Gly Ile Val
65 70 75 80

Leu Thr Ile Leu Thr Leu Leu Cys Leu Gln Ala Leu Val Gly Phe Ile
85 90 95

Lys Phe Ile Arg Gln Leu Pro Gln Gln Leu His Thr Thr Val Gln Phe
100 105 110

Ile Arg Glu Lys Ile Arg Pro Glu Ser Ser Leu Gln Leu Val Thr Asn
115 120 125

Ala Gln Arg Lys Thr Thr Gln Asp Thr Leu Lys Leu Tyr Glu Glu Leu
130 135 140

Cys Asp Leu Ser Gln Lys Glu Phe Lys Leu Gln Ser Thr Leu Tyr Gln
145 150 155 160

Lys Arg Phe Glu Leu Ser His Lys Asn Glu Lys Thr Asn Gln Asn
165 170 175

<210> 294
<211> 528
<212> DNA
<213> Chlamydia pneumoniae

<400> 294
gtgtctagta ctttaaacgg ggtatttccc tcatcccttc cggaagagtc tgctgattta 60
ttcattacga ataaggagat cgtagctttg ggggagaagg gcaatgtttt tctcacccac 120
tccattccta tgcatattgc tgcgattacg atcttagtga ttgtagctct tgctggaatc 180
gctattatct gtttgggttg ctatagccaa agcattctgt tgattgccgt tggcattgtt 240
cttactattt tgactcttct ctgcctacaa gccttggtag gatttattaa attcatccgg 300
cagctccctc agcagctcca tacgacagta caatttatca gggagaagat tcgacctgaa 360
tcctctctac agcttgtaac caatgcacag agaaaaacca ctcaagatac gctaaagtta 420
tacgaagaac tctgcgacct ctcacaaaaa gagttcaaac tgcaatcaac tctttatcaa 480
aaacgttttg agctttctca caagaatgaa aagacaaatc aaaactag 528

<210> 295
<211> 158
<212> PRT
<213> Chlamydia pneumoniae

<400> 295
Met Ser Glu Leu Ala Pro Cys Ser Thr Gly Leu Gln Met Val Pro His
1 5 10 15

Thr Gln Val His His Ala Leu Asp Thr Arg Arg Val Ile Leu Thr Ile
20 25 30

Ala Ala Cys Leu Ser Leu Ile Ala Gly Ile Val Leu Val Gly Leu Gly
35 40 45

Ala Ala Ala Ile Leu Pro Ser Leu Phe Gly Val Ile Gly Gly Met Ile
50 55 60

Leu Ile Leu Phe Ser Ser Ile Ala Leu Ile Tyr Leu Tyr Lys Lys Thr
65 70 75 80

Arg Glu Val Asp Gln Ile Ala Leu Glu Pro Leu Pro Glu Met Ile Ser
85 90 95

```
Lys Asp Gln Ser Ile Ile Asp Phe Val Lys Thr Arg Asp Tyr Ala Ser
        100                 105                 110

Leu Glu Lys Lys Ala Thr Phe Ala Tyr Thr His Thr His Tyr Tyr Asp
    115                 120                 125

Gly Ser Met Val Phe Tyr Arg Glu Ile Pro Arg Phe Met Leu Gly Ser
    130                 135                 140

Tyr Leu Ala Leu Arg Lys Asp Met Asp Arg Gln Ala Leu Phe
145                 150                 155

<210>   296
<211>   477
<212>   DNA
<213>   Chlamydia pneumoniae

<400>   296
atgagcgagc tcgccccctg ctcgacagga ttgcagatgg tcccccatac gcaggtccat    60
catgcccttg atacgcggag agtcattcta acgatagccg cctgtctgtc tttaattgca   120
ggaatcgtgt tggttggctt aggtgctgca gcaatcctgc cctcgctttt tggagtcatt   180
ggaggaatga ttcttattct gttttcttcg atcgccctca tttatttata caagaagaca   240
agggaggtgg atcagattgc tctggagcct cttcctgaga tgatttctaa agatcaaagc   300
attatagatt ttgtaaagac acgagactat gcatctttag aaaagaaagc gacctttgct   360
tatactcata ctcattatta cgatggaagc atggtcttct atagggagat ccctagattt   420
atgttaggct cttatctcgc gcttcgcaaa gacatggacc gccaagctct tttttga      477

<210>   297
<211>   237
<212>   PRT
<213>   Chlamydia pneumoniae

<400>   297
Val Ile Ser Gly Leu Leu Phe Leu Leu Val Arg Arg Glu Val Pro Thr
1               5                   10                  15

Val Arg Ser Glu Glu Ile Pro Arg Gly Val Ser Val Thr Pro Ser Glu
        20                  25                  30

Glu Pro Ala Leu Glu Lys Ala Gln Lys Glu Pro Glu Thr Lys Lys Ile
    35                  40                  45

Leu Asp Arg Leu Pro Lys Glu Leu Asp Gln Leu Asp Thr Tyr Ile Gln
    50                  55                  60

Glu Val Phe Ala Cys Leu Glu Arg Leu Lys Asp Pro Lys Tyr Glu Asp
65                  70                  75                  80

Arg Gly Leu Leu Thr Glu Ala Lys Glu Lys Leu Arg Val Phe Asp Val
                85                  90                  95

Val Glu Lys Asp Met Met Ser Glu Phe Leu Asp Ile Gln Arg Val Leu
            100                 105                 110

Asn Glu Glu Ala Tyr Tyr Val Glu His Cys Gln Asp Pro Leu Glu Asn
        115                 120                 125

Ile Ala Tyr Glu Ile Phe Ser Ser Gln Glu Leu Arg Asp Tyr Tyr Cys
    130                 135                 140
```

Ala Gly Val Cys Gly Tyr Leu Pro Ser Gly Asp Ala Arg Ala Asp Arg
145 150 155 160

Leu Lys Arg Ser Val Lys Glu Val Met Asp Arg Phe Met Arg Val Thr
165 170 175

Trp Lys Ser Trp Glu Ala Ser Val Met Leu Asp His Ser Tyr Gly Val
180 185 190

Ala Arg Glu Leu Phe Lys Lys Ala Val Gly Val Leu Glu Glu Ser Val
195 200 205

Tyr Lys Ile Leu Phe Lys Ser Tyr Arg Asp Ala Phe Tyr Glu Cys Glu
210 215 220

Lys Ala Lys Ile Gln Arg Asp Gly Arg Phe Lys Trp Leu
225 230 235

<210> 298
<211> 714
<212> DNA
<213> Chlamydia pneumoniae

<400> 298
gtgatttcgg gacttctatt ccttctagta agacgagagg ttccgacagt acgttcagag 60
gaaattccca gaggggtttc tgtgacccct tctgaagagc ctgctctaga gaaggctcaa 120
aaagaaccgg agacaaagaa aattttagat cggttgccga aggaattgga tcagttagat 180
acgtatattc aggaagtgtt tgcatgttta gagaggctga aggatcctaa gtacgaagat 240
cgaggtcttt taacagaggc gaaggagaaa cttcgagttt ttgacgttgt tgagaaagat 300
atgatgtcag agtttttaga catacaacga gtgttgaatg aggaagcata ttatgtagaa 360
cattgtcaag atcccctaga gaatatagcc tacgagattt tctcttccca agagcttcgt 420
gattactact gtgcaggggt gtgtgggtat ttgccttctg ggatgctcg agcggatcga 480
ttaaagagat cagttaagga ggtaatggat cgctttatga gggtgacctg gaaatcttgg 540
gaggcatcag tcatgttgga tcatagctat ggggtagcgc gagagttatt caagaaggca 600
gtaggagtac tagaggagag tgtctataaa attctgttta agagctatag agatgcgttt 660
tatgaatgtg agaaggcaaa gatccagagg gatgggcgtt tcaaatggtt atag 714

<210> 299
<211> 297
<212> PRT
<213> Chlamydia pneumoniae

<400> 299
Met Leu Leu Leu Ile Ser Gly Ala Leu Phe Leu Thr Leu Gly Ile Pro
1 5 10 15

Gly Leu Ser Ala Ala Ile Ser Phe Gly Leu Gly Ile Gly Leu Ser Ala
20 25 30

Leu Gly Gly Val Leu Met Ile Ser Gly Leu Leu Cys Leu Leu Val Lys
35 40 45

Arg Glu Ile Pro Thr Val Arg Pro Glu Glu Ile Pro Glu Gly Val Ser
50 55 60

Leu Ala Pro Ser Glu Glu Pro Ala Leu Gln Ala Ala Gln Lys Thr Leu
65 70 75 80

Ala Gln Leu Pro Lys Glu Leu Asp Gln Leu Asp Thr Asp Ile Gln Glu
85 90 95

```
Val Phe Ala Cys Leu Arg Lys Leu Lys Asp Ser Lys Tyr Glu Ser Arg
            100                 105                 110

Ser Phe Leu Asn Asp Ala Lys Lys Glu Leu Arg Val Phe Asp Phe Val
        115                 120                 125

Val Glu Asp Thr Leu Ser Glu Ile Phe Glu Leu Arg Gln Ile Val Ala
    130                 135                 140

Gln Glu Gly Trp Asp Leu Asn Phe Leu Ile Asn Gly Gly Arg Ser Leu
145                 150                 155                 160

Met Met Thr Ala Glu Ser Glu Ser Leu Asp Leu Phe His Val Ser Lys
                165                 170                 175

Arg Leu Gly Tyr Leu Pro Ser Gly Asp Val Arg Gly Glu Gly Leu Lys
            180                 185                 190

Lys Ser Ala Lys Glu Ile Val Ala Arg Leu Met Ser Leu His Cys Glu
        195                 200                 205

Ile His Lys Val Ala Val Ala Phe Asp Arg Asn Ser Tyr Ala Met Ala
    210                 215                 220

Glu Lys Ala Phe Ala Lys Ala Leu Gly Ala Leu Glu Glu Ser Val Tyr
225                 230                 235                 240

Arg Ser Leu Thr Gln Ser Tyr Arg Asp Lys Phe Leu Glu Ser Glu Arg
                245                 250                 255

Ala Lys Ile Pro Trp Asn Gly His Ile Thr Trp Leu Arg Asp Asp Ala
            260                 265                 270

Lys Ser Gly Cys Ala Glu Lys Lys Leu Gly Met Pro Arg Asn Val Gly
        275                 280                 285

Arg Asn Leu Gly Lys Gln Ser Phe Gly
    290                 295
```

```
<210>   300
<211>   894
<212>   DNA
<213>   Chlamydia pneumoniae

<400>   300
atgctcttac tgatttcagg agctctcttt ctgacgttag ggattccagg attgagtgca    60
gcaatttctt ttggattagg catcggtctc tccgcattag gaggagtgct gatgatttcg   120
ggactactat gtcttttagt aaaacgagag attccgacag tacgaccaga agaaattcct   180
gaaggggttt cgctggctcc ttctgaggag ccagctctac aggcagctca gaagacttta   240
gctcagctgc ctaaggaatt ggatcagtta gatacagata ttcaggaagt gttcgcatgt   300
ttaagaaagc tgaaagattc taagtatgaa agtcgaagtt ttttaaacga tgctaagaag   360
gagcttcgag tttttgactt tgtggttgag gataccctct cggagatttt cgagttgcgg   420
cagattgtgg ctcaagaggg atgggattta aactttttga tcaatggggg acgaagcctc   480
atgatgactg cagaatctga atcgcttgat ttgtttcatg tatcgaagcg gctagggtat   540
ttaccttctg gggatgttcg aggggagggg ttaaagaaat ctgcgaagga gatagtcgct   600
cgtttgatga gcttgcattg cgagattcac aaggtggcgg tagcgtttga taggaattcc   660
tatgcgatgg cagaaaaggc gtttgcgaaa gcgttgggag ctttagaaga gagtgtgtat   720
cggagtctga cgcagagtta tagagataaa tttttggaga gcgagagggc gaagatccca   780
tggaatgggc atataacctg gttaagagat gatgcgaaga gtgggtgtgc tgaaaagaag   840
ctcgggatgc cgaggaacgt tggaagaaat ttaggaaagc agtcttttgg gtag         894
```

<210> 301
<211> 107
<212> PRT
<213> Chlamydia pneumoniae

<400> 301
Met Lys Ile Lys Lys Ser Phe Gln Tyr Ser Leu Cys Gln Ala Lys Arg
1 5 10 15

Phe Gln Asn Met Leu Pro Asn His Phe Asp Pro Cys Leu Gln Pro Val
20 25 30

Asn Leu Gln Leu Lys Gln Asp Arg Leu Ala Tyr Gly Glu Leu Ile Ile
35 40 45

Leu Leu Ser Lys Tyr Gln Gln Lys Thr Phe Ser Ser Leu Leu Lys Glu
50 55 60

Glu Thr Cys Ser Leu Asn Arg Ala Lys Gln His Leu Leu Tyr Lys Ile
65 70 75 80

Leu Arg Asp Phe Asn Thr Met Gln His Leu Arg Ser Leu Gly Leu Asn
85 90 95

Gly Trp Gly Glu Ile Pro Met Ser Pro Cys Leu
100 105

<210> 302
<211> 324
<212> DNA
<213> Chlamydia pneumoniae

<400> 302
atgaagatta aaaaatcttt tcaatacagt ttatgccaag caaagagatt tcagaacatg 60
ctgccaaacc actttgatcc atgtttgcag ccagtgaatt tacaactcaa acaagacaga 120
ttggcatacg gggagctcat catattgcta tctaaatatc aacaaaagac cttttcctct 180
ttgttgaagg aagaaacatg ttctcttaat cgtgcgaagc agcacttatt gtataagatt 240
ttgagagatt ttaatactat gcagcatcta aggtccctcg gattaaatgg ttggggagag 300
atccctatga gtccttgcct ctaa 324

<210> 303
<211> 181
<212> PRT
<213> Chlamydia pneumoniae

<400> 303
Met Ser Leu Leu Asn Leu Pro Ser Ser Gln Asp Ser Ala Ser Glu Asp
1 5 10 15

Ser Thr Ser Gln Ser Gln Ile Phe Asp Pro Ile Arg Asn Arg Glu Leu
20 25 30

Val Ser Thr Pro Glu Glu Lys Val Arg Gln Arg Leu Leu Ser Phe Leu
35 40 45

Met His Lys Leu Asn Tyr Pro Lys Lys Leu Ile Ile Ile Glu Lys Glu
50 55 60

Leu Lys Thr Leu Phe Pro Leu Leu Met Arg Lys Gly Thr Leu Ile Pro
65 70 75 80

Lys Arg Arg Pro Asp Ile Leu Ile Ile Thr Pro Pro Thr Tyr Thr Asp
85 90 95

Ala Gln Gly Asn Thr His Asn Leu Gly Asp Pro Lys Pro Leu Leu Leu
100 105 110

Ile Glu Cys Lys Ala Leu Ala Val Asn Gln Asn Ala Leu Lys Gln Leu
115 120 125

Leu Ser Tyr Asn Tyr Ser Ile Gly Ala Thr Cys Ile Ala Met Ala Gly
130 135 140

Lys His Ser Gln Val Ser Ala Leu Phe Asn Pro Lys Thr Gln Thr Leu
145 150 155 160

Asp Phe Tyr Pro Gly Leu Pro Glu Tyr Ser Gln Leu Leu Asn Tyr Phe
165 170 175

Ile Ser Leu Asn Leu
180

<210> 304
<211> 546
<212> DNA
<213> Chlamydia pneumoniae

<400> 304
atgtccttat tgaaccttcc ctcaagccag gattctgcat ctgaggactc cacatcgcaa 60
tctcaaatct tcgatcccat tagaaatcgg gagttagttt ctactcccga agaaaaagtc 120
cgccaaaggt tgctctcctt cctaatgcat aagctgaact accctaagaa actcatcatc 180
atagaaaaag aactcaaaac tctttttcct ctgcttatgc gtaaaggaac cctaatccca 240
aaacgccgcc cagatattct catcatcact ccccccacat acacagacgc acagggaaac 300
actcacaacc taggcgaccc aaaacccctg ctacttatcg aatgtaaggc cttagccgta 360
aaccaaaatg cactcaaaca actccttagc tataactact ctatcggagc cacctgcatt 420
gctatggcag ggaaacactc tcaagtgtca gctctcttca atccaaaaac acaaactctt 480
gatttttatc ctggcctccc agagtattcc caactcctaa actactttat ttctttaaac 540
ttatag 546

<210> 305
<211> 223
<212> PRT
<213> Chlamydia pneumoniae

<400> 305
Met Ser Thr Thr Thr Val Lys His Phe Ile His Thr Ala Ser Arg Trp
1 5 10 15

Glu Pro Val Leu Lys Glu Ile Val Ala Ser Asn Tyr Trp His Ala Gln
20 25 30

Trp Ile Asn Thr Leu Ser Phe Leu Glu Asn Ser Gly Ala Lys Lys Ile
35 40 45

Ser Ala Ser Glu His Pro Thr Glu Val Lys Glu Glu Val Leu Lys His
50 55 60

Ala Ala Glu Glu Phe Arg His Gly His Tyr Leu Lys Thr Gln Ile Ser
65 70 75 80

Arg Ile Ser Glu Thr Ser Leu Pro Asp Tyr Thr Ser Lys Asn Leu Leu
85 90 95

Gly Gly Leu Leu Thr Lys Tyr Tyr Leu His Leu Leu Asp Leu Arg Thr
100 105 110

Cys Arg Val Leu Glu Asn Glu Tyr Ser Leu Ser Gly Gln Thr Leu Lys
115 120 125

Thr Ala Ala Tyr Ile Leu Val Thr Tyr Ala Ile Glu Leu Arg Ala Ser
130 135 140

Glu Leu Tyr Pro Leu Tyr His Asp Ile Leu Lys Glu Ala Gln Ser Lys
145 150 155 160

Ile Thr Val Lys Ser Ile Ile Leu Glu Glu Gln Gly His Leu Gln Glu
165 170 175

Met Glu Arg Glu Leu Lys Asp Leu Pro His Gly Glu Glu Leu Leu Gly
180 185 190

Tyr Ala Cys Gln Phe Glu Gly Glu Leu Cys Leu Gln Phe Val Glu Arg
195 200 205

Leu Glu Gln Met Ile Phe Asp Pro Ser Ser Thr Phe Thr Lys Phe
210 215 220

<210> 306
<211> 672
<212> DNA
<213> Chlamydia pneumoniae

<400> 306
atgtctacaa ccacagtaaa acactttatc cacacagcct ctcgttggga gcccgttctc 60
aaagagatcg tagcttccaa ctattggcat gcacaatgga taaataccct gtccttttta 120
gaaaatagtg gagcaaaaaa aatctccgca agtgaacatc ctacggaggt aaaggaagaa 180
gttttaaaac atgctgctga agaatttcgt catggtcact atctaaaaac tcagatttct 240
agaatctcag agacttctct ccctgactat acatctaaaa atcttctggg aggcttactt 300
acaaaatatt acctccatct tctagattta aggacgtgcc gagtactgga aaatgaatac 360
tccctatcgg gacaaacgtt aaaaactgca gcgtatattt tagttaccta cgcaatcgaa 420
cttcgtgctt ctgaacttta tcctctgtat cacgatattc tgaaagaagc tcaaagtaaa 480
ataacggtaa aatccattat cttagaagag caaggccatc tgcaagagat ggaacgtgaa 540
cttaaagatc tcccccacgg ggaggaactc ttaggctatg cttgccaatt cgaaggggag 600
ctttgcttgc agtttgtaga gagattagaa caaatgatct tcgatccttc ctcgactttt 660
acaaagttct ag 672

<210> 307
<211> 161
<212> PRT
<213> Chlamydia pneumoniae

<400> 307
Met Ser Ser Ser Glu Val Val Phe Gln Thr Val His Gly Leu Gly Phe
1 5 10 15

Gly Gly Leu Ser Ser Lys Ser Val Val Pro Phe Lys Lys Ser Leu Ser
20 25 30

Asp Ala Pro Arg Val Val Cys Ser Ile Leu Val Leu Thr Leu Gly Leu
35 40 45

Gly Ala Leu Val Cys Gly Ile Ala Ile Thr Cys Trp Cys Val Pro Gly
50 55 60

Val Ile Leu Met Gly Gly Ile Cys Ala Ile Val Leu Gly Ala Ile Ser
65 70 75 80

Leu Ala Leu Ser Leu Phe Trp Leu Trp Gly Leu Phe Ser Asn Cys Cys
85 90 95

Gly Ser Lys Arg Val Leu Pro Gly Glu Gly Leu Leu Arg Asp Lys Leu
100 105 110

Leu Asp Gly Gly Phe Ser Arg Ala Ala Pro Ser Gly Met Gly Leu Pro
115 120 125

Gly Asp Gly Ser Pro Arg Ala Ser Thr Pro Ser Cys Leu Glu Glu Leu
130 135 140

Gln Ala Glu Ile Gln Ala Val Thr Gln Ala Ile Asp Gln Met Ser Asp
145 150 155 160

Asp

<210> 308
<211> 486
<212> DNA
<213> Chlamydia pneumoniae

<400> 308
atgagcagtt cggaagttgt tttccagaca gttcatggcc ttggctttgg tggattgtct 60
tcaaaaagtg ttgtcccttt taagaaaagt ctttcggatg cgccccgtgt tgtgtgctcg 120
attttagttt tgactctggg gttgggagcg cttgtttgtg gtattgccat tacttgttgg 180
tgtgtcccgg gagttatttt aatgggggga atttgcgcta tagttttagg tgcaatttct 240
ttagctttaa gtctattttg gttgtggggt ttattttcta attgttgtgg ttctaagaga 300
gttttaccgg gtgagggatt gctacgggat aagcttttag atggtggatt ttcaagagcg 360
gcaccttcag gaatgggact tccgggtgat ggatctccaa gagcgtcaac gccatcttgc 420
ctagaggaac ttcaagcaga gatacaggca gttactcaag ctatcgatca gatgtcagat 480
gattga 486

<210> 309
<211> 123
<212> PRT
<213> Chlamydia pneumoniae

<400> 309
Leu Arg Ala Gly Gly Ser Leu Val Thr Thr Tyr Pro Lys Glu Gly Gln
1 5 10 15

Arg Leu Arg Ser Pro Glu Gln Leu Arg Val Leu Asp Asp Leu Val Gln
20 25 30

Ser Tyr Pro Asn His Leu His Ala Ile Glu Leu Asp Cys Gly Ala Ile
35 40 45

Pro Gln Asp Leu Ile Gly Ala Thr Tyr Ile Ile Thr Phe Ala Asp Phe
50 55 60

Ser Thr Tyr Ile Leu Ser Leu Arg Ser Tyr Gln Ala Asn Ser Pro Ser
65 70 75 80

Asp Asp Thr Trp Gly Ile Trp Phe Gly Ser Ile Asp Asp Pro Val Gln
85 90 95

Ala Val Ile Ser Phe Leu Lys Asp His Gly Phe Ala Leu Pro Ser Thr
100 105 110

Leu Ala Gln Asp Pro Leu Leu Cys Thr Asn Lys
115 120

<210> 310
<211> 372
<212> DNA
<213> Chlamydia pneumoniae

<400> 310
ttgcgagcag gaggtagtct tgttacaaca taccctaagg aaggtcagag attgcgctcc 60
ccagaacagt taagagttct ggatgattta gtgcaaagct atccaaatca cctacatgcg 120
attgaacttg attgtggtgc aatccctcaa gatttgatcg gagccaccta tatcatcacg 180
ttcgccgatt tttccaccta tattctctct ttaagaagct accaagccaa ttctccctcc 240
gatgatacat gggggatttg gtttggatct attgacgatc ctgttcaagc agtcatatca 300
tttttaaaag atcatggatt tgctcttccc tcgaccttag ctcaagatcc tttgctttgt 360
actaacaagt aa 372

<210> 311
<211> 156
<212> PRT
<213> Chlamydia pneumoniae

<400> 311
Met Ile Met Thr Thr Ile Ser Asn Ser Pro Ser Pro Ala Leu Asn Pro
1 5 10 15

Glu Leu Ser Leu Ile Pro Pro Pro Thr Leu Val Ser Ser Gly Thr Gln
20 25 30

Thr Ser Leu Ala Tyr Thr Ile Pro Ala Gln Gly Arg Arg Ser Thr Leu
35 40 45

Arg Ile Ile Leu Asp Ile Phe Ile Ile Ile Leu Gly Leu Ala Thr Ile
50 55 60

Ile Ser Thr Phe Ile Val Ile Phe Phe Leu Asn Gly Leu Asn Leu Leu
65 70 75 80

Ser Thr Pro Ser Ile Ile Ser Ser Ser Cys Leu Ile Ile Val Gly Leu
85 90 95

Leu Phe Leu Ile Met Gly Leu Tyr Phe Met Ile Ser Ser Leu Asp Gln
100 105 110

Gly Leu Val Gly Leu Leu Gln Lys Glu Leu Ser Gln Ala Glu Glu Arg
115 120 125

Glu Glu Glu Tyr Ile Gln Glu Ile Glu Ala Leu Arg Gly Ala Pro Arg
130 135 140

Ala Glu Ser Pro Thr Glu Ser Pro Ser Thr Trp Leu
145 150 155

<210> 312
<211> 471
<212> DNA
<213> Chlamydia pneumoniae

<400> 312
atgattatga ctactatatc taactcaccc tcccctgcat tgaatcccga actttccctt 60
attcctccac caacacttgt atcttcaggt acgcaaacat ctctagctta tacgatcccc 120
gcacaaggac gaagatccac cctacgtatt atattagata tattcattat cattcttggt 180
ttagctacga tcatttctac ctttattgtt attttctttt taaatgggct gaacttgctc 240
tcgaccccat ctattatctc ttcgtcatgt ttaatcattg ttggattgct ttttttgatt 300
atggggttat atttcatgat ctcgagtttg gatcaggggc ttgtaggcct tctgcaaaag 360
gaactctctc aagccgaaga aagagaagaa gagtatatcc aggaaatcga agctttaaga 420
ggagctccta gagcagaatc tcccacagag tctcctagta cctggttatg a 471

<210> 313
<211> 126
<212> PRT
<213> Chlamydia pneumoniae

<400> 313
Met Leu Ile Gly Arg Tyr Ser Ser Asp Asp Gln Phe Thr Glu Ala Thr
1               5                   10                  15

Lys Asn Thr Pro Thr Ile Ile Lys Leu Gly Phe Val Arg Asp Asn Leu
            20                  25                  30

Glu Gly Leu Thr Asn Pro Ile Ser Glu Ile Val Ser Glu Thr Ser Ser
        35                  40                  45

Ser Ile Lys Asp Ser Val Leu Arg Ser Leu Pro Ile Leu Gly Ser Ile
    50                  55                  60

Leu Gly Cys Ala Arg Leu Tyr Ser Thr Leu Ser Thr Asn Asp Pro Leu
65                  70                  75                  80

Asp Glu Thr Gln Glu Lys Ile Trp His Thr Ile Phe Gly Ala Leu Glu
                85                  90                  95

Thr Leu Gly Leu Gly Ile Leu Ile Leu Leu Phe Lys Ile Ile Phe Val
            100                 105                 110

Ile Leu His Cys Ile Phe His Leu Val Ile Gly Phe Cys Lys
        115                 120                 125

<210> 314
<211> 381
<212> DNA
<213> Chlamydia pneumoniae

<400> 314
atgctaatag gcagatacag tagtgatgac caattcactg aagcaacaaa aaacacccca 60
accataatta agctaggttt tgttagagat aatctcgagg gattaacgaa ccctatctct 120
gaaatcgtct cggaaacctc ctcttctatt aaagattccg ttcttcgctc tcttcctatt 180
ttagggtcca ttttaggatg cgcccgactt tacagcacac tctctacaaa tgatcctctt 240
gacgaaactc aagaaaagat ttggcacact atatttggag ccttagaaac cttaggctta 300
gggattctca tcctcttatt taaaattatt tttgttatat tacactgcat atttcatcta 360
gttattgggt tctgcaaata a 381

<210> 315
<211> 147
<212> PRT
<213> Chlamydia pneumoniae

<400> 315

Met Lys Thr Lys Met Asn Ser Arg Lys Lys Ala Gly Gln Trp Ala Ile
1 5 10 15

Phe Asn Ser Pro Thr Pro Gly Val Ser Ser Thr Leu Val Leu Ala Trp
20 25 30

Thr Pro Trp Gly Tyr Tyr Asp Lys Asp Val Gln Asp Ile Leu Glu Arg
35 40 45

Lys Asp Pro Met Ser Ser Ser Leu Ser Glu Lys Asp Ser Lys Glu Phe
50 55 60

Leu Lys Asn Leu Phe Val Asp Leu Leu Glu Asn Gly Phe Thr Ser Val
65 70 75 80

His Ile His Ala Glu Glu Ala Phe Thr Pro Leu Asp His Thr Gly Lys
85 90 95

Pro His Phe Lys Arg Asp Asn Val Tyr Leu Pro Gly Lys Leu Leu Gly
100 105 110

Ala Leu Asn Glu Ala Ala Val Gln Ala Asn Val Ser Ala Asp Thr Gln
115 120 125

Phe Thr Leu Phe Leu Thr Gln Asp Glu Cys Asn Pro Phe His Asp Lys
130 135 140

Lys Arg Gly
145

<210> 316
<211> 444
<212> DNA
<213> Chlamydia pneumoniae

<400> 316
atgaaaacta aaatgaactc tagaaaaaaa gcaggtcaat gggcaatttt caattctcca 60
actcctggtg tcagttcaac tttagtttta gcatggactc cttggggtta ttacgacaag 120
gatgtacaag atatcttaga aagaaaagat ccgatgagct cttcgctttc tgaaaaagac 180
tcaaaggagt tcttgaaaaa tctgtttgta gatctcttag aaaatggctt cacatcagta 240
catattcacg cagaagaagc tttcactcct cttgatcata ccgggaaacc tcactttaaa 300
agagacaatg tgtacttacc cggaaagttg ttaggcgcct tgaatgaggc tgcggtacaa 360
gccaatgtaa gtgcggatac tcaatttaca ttgttcctta ctcaagatga gtgcaatcct 420
tttcatgata agaaaagagg ttaa 444

<210> 317
<211> 65
<212> PRT
<213> Chlamydia pneumoniae

<400> 317
Leu Leu Lys Phe Phe Leu Val Cys Glu Glu Leu Cys Ile Leu Thr Val
1 5 10 15

Ala Thr His Arg Ala Leu Leu Glu Thr Pro Leu Ala Leu Ser Phe Phe
20 25 30

Lys Glu Leu Lys Thr Lys Tyr Val Tyr Arg Ala Lys Asp Ile Leu Gln
35 40 45

Leu His Asn Tyr Lys Gly Phe Thr Ile Leu Asn Thr Ser Pro Leu Cys

50 55 60

Ser
65

<210> 318
<211> 198
<212> DNA
<213> Chlamydia pneumoniae

<400> 318
ttgctaaagt tctttctagt atgtgaagag ttatgtatac ttactgttgc tacacataga 60
gctctcttag aaactccttt agctctatca tttttaaag aacttaagac aaaatatgtc 120
tacagggcga aagacatact acaactacat aactataaag gatttactat ccttaataca 180
tcaccgttat gttcttaa 198

<210> 319
<211> 110
<212> PRT
<213> Chlamydia pneumoniae

<400> 319
Leu Trp Ser His Phe Pro Arg Gly Phe Phe Met Leu Pro Phe Cys Pro
1 5 10 15

Thr Ile Leu Leu Ala Lys Pro Phe Leu Asn Ser Glu Asn Tyr Gly Leu
20 25 30

Glu Arg Leu Ala Ala Thr Val Asp Ser Tyr Phe Asp Leu Gly Gln Ser
35 40 45

Gln Ile Val Phe Leu Ser Lys Gln Asp Gln Gly Ile Thr Val Glu Glu
50 55 60

Leu Ser Ala Lys Asp Arg Lys Phe Lys Pro Gly Ser Met Asn Cys Thr
65 70 75 80

Leu Tyr Thr Glu Asp Pro Ile Leu Pro Ala His Asn Ser Phe Ser Asn
85 90 95

Cys Ser Asp Ile Gln Met Arg Thr Pro Ile Ser Pro Ile His
100 105 110

<210> 320
<211> 333
<212> DNA
<213> Chlamydia pneumoniae

<400> 320
ttgtggtcgc atttcccaag aggatttttt atgctccctt tttgccctac catccttctt 60
gctaaacctt ttttaaatag cgagaattac ggcttagaac gtttagctgc aaccgtagat 120
tcttattttg atctgggaca gtctcaaata gtcttcctaa gcaaacagga tcaaggaatc 180
actgtggaag aattgagtgc taaagatagg aaattcaagc caggctctat gaactgtaca 240
ctgtacactg aagatcctat cttacctgct cataattcct ttagtaattg ctctgatatt 300
caaatgcgta ctccgattag ccctatacat taa 333

<210> 321
<211> 246
<212> PRT
<213> Chlamydia pneumoniae

<400> 321
Val Glu Ala Gly Ala Asn Val Leu Val Ile Asp Thr Ala His Ala His
1 5 10 15

Ser Lys Gly Val Phe Gln Thr Val Leu Glu Ile Lys Ser Gln Phe Pro
20 25 30

Gln Ile Ser Leu Val Val Gly Asn Leu Val Thr Ala Glu Ala Ala Val
35 40 45

Ser Leu Ala Glu Ile Gly Val Asp Ala Val Lys Val Gly Ile Gly Pro
50 55 60

Gly Ser Ile Cys Thr Thr Arg Ile Val Ser Gly Val Gly Tyr Pro Gln
65 70 75 80

Ile Thr Ala Ile Thr Asn Val Ala Lys Ala Leu Lys Asn Ser Ala Val
85 90 95

Thr Val Ile Ala Asp Gly Arg Ile Arg Tyr Ser Gly Asp Val Val Lys
100 105 110

Ala Leu Ala Ala Gly Ala Asp Cys Val Met Leu Gly Ser Leu Leu Ala
115 120 125

Gly Thr Asp Glu Ala Pro Gly Asp Ile Val Ser Ile Asp Glu Lys Leu
130 135 140

Phe Lys Arg Tyr Arg Gly Met Gly Ser Leu Gly Ala Met Lys Gln Gly
145 150 155 160

Ser Ala Asp Arg Tyr Phe Gln Thr Gln Gly Gln Lys Lys Leu Val Pro
165 170 175

Gly Gly Val Glu Gly Leu Val Ala Tyr Lys Gly Ser Val His Asp Val
180 185 190

Leu Tyr Gln Ile Leu Gly Gly Ile Arg Ser Gly Met Gly Tyr Val Gly
195 200 205

Ala Glu Thr Leu Lys Asp Leu Lys Thr Lys Ala Ser Phe Val Arg Ile
210 215 220

Thr Glu Ser Gly Arg Ala Glu Ser His Ile His Asn Ile Tyr Lys Val
225 230 235 240

Gln Pro Thr Leu Asn Tyr
245

<210> 322
<211> 741
<212> DNA
<213> Chlamydia pneumoniae

<400> 322
gtggaagctg gagcaaatgt tctagtcatt gacacagctc atgcacactc taaaggagta 60
ttccaaacag ttttagaaat aaaatcccag ttcccacaaa tttctttagt tgtagggaat 120
cttgttacag ctgaagccgc agtttcctta gctgagattg gagttgacgc tgtaaaggta 180
ggtattggcc caggatctat ctgtacaact agaatcgttt caggggtcgg ttatccacaa 240
attactgcca ttacaaacgt agcaaaagct cttaaaaact ctgccgtgac tgtaattgct 300
gatgggagaa tccgctattc tggagatgtg gtaaaagcat tagcagcagg agcagactgt 360

gtcatgctag gaagtttgct tgcagggact gatgaagctc ctggggatat cgtttctatc 420
gatgagaagc tttttaaaag gtaccgcggc atgggatctt taggcgctat gaaacaagga 480
agtgctgacc ggtattttca aacacaggga cagaaaaagc tggttcctgg gggagttgaa 540
ggactagtcg cttataaagg ctctgtccac gatgtcctct atcaaatttt aggaggaata 600
cgctcaggta tggggtatgt tggagctgaa actctcaaag atttaaaaac taaggcttcc 660
tttgttcgaa ttactgaatc tggaagagct gaaagtcata ttcataatat ttacaaagtt 720
caaccaacct taaattatta a 741

<210> 323
<211> 159
<212> PRT
<213> Chlamydia pneumoniae

<400> 323
Leu Phe Ser Glu Gly Thr Ala Leu Asn Leu Phe Arg Ile Phe Ala Pro
1 5 10 15

Leu Arg Asn Arg Val Thr Thr Glu Tyr Ser Arg Ala Arg Gln Pro Asp
20 25 30

Leu His Arg Ile Ala Ile Val Tyr Ile Gly Val Leu Asp Ser Glu Ser
35 40 45

Ser Lys Ile Leu Glu Arg Leu Ile Ser Tyr Met Ser Cys Ile Tyr Ser
50 55 60

Glu Ser Gln Met Tyr Leu Arg Phe Phe Met Gly Lys Asn Val Asn Gln
65 70 75 80

Ser Ala Val Leu Ser Lys Leu His Val Glu Asn Leu His Ile Arg Cys
85 90 95

Gly Phe Phe Ser Glu Asp Ala Val Pro Glu Ser Glu Pro Phe Asp Leu
100 105 110

Ser Ile Tyr Val His Thr Asp Arg Ser Cys Pro Leu Pro Thr Lys Lys
115 120 125

Arg Ser Ser Ser Trp Glu Leu Gln Thr Val Glu Leu Pro Glu Ser Ile
130 135 140

Tyr Pro Gln Ser Glu Phe Leu Leu Met Arg Pro Arg Met Leu Ser
145 150 155

<210> 324
<211> 480
<212> DNA
<213> Chlamydia pneumoniae

<400> 324
ttgttctctg aggggacagc tctaaattta tttcgtatat ttgctccact acgcaaccgt 60
gtgactacag aatacagtcg tgctaggcaa cccgacctac atagaattgc catcgtctat 120
ataggagttc tcgattcaga aagttccaag atcctagagc ggctaatctc ttatatgagt 180
tgtatctatt ctgaatcgca aatgtattta agattcttta tgggcaagaa tgtaaatcaa 240
agtgctgtac tctcaaaatt acatgtagaa aatctgcaca tccgttgtgg gtttttcagc 300
gaggatgctg ttccagagag tgagcccttc gatctctcca tctacgtgca cacagatcgt 360
agctgtcctc tccctacgaa aaaacggagc agctcctggg aactccaaac tgtagaactc 420
ccagagtcaa tatatccaca gtcggaattc ctattgatga gacctcgaat gctttcgtag 480

<210> 325
<211> 228

<212> PRT
<213> Chlamydia pneumoniae

<400> 325

```
Met Arg Pro His Arg Lys His Val Ser Ser Lys Ser Leu Ala Leu Lys
1               5                   10                  15

Gln Ser Ala Ser Thr His Val Glu Ile Thr Thr Lys Ala Phe Arg Leu
            20                  25                  30

Ser Met Pro Leu Lys Gln Leu Ile Leu Glu Lys Ser Asp His Leu Pro
        35                  40                  45

Pro Met Glu Thr Ile Arg Val Val Leu Thr Ser His Lys Asp Lys Leu
    50                  55                  60

Gly Thr Glu Val His Val Val Ala Ser His Gly Lys Glu Ile Leu Gln
65                  70                  75                  80

Thr Lys Val His Asn Ala Asn Pro Tyr Thr Ala Val Ile Asn Ala Phe
                85                  90                  95

Lys Lys Ile Arg Thr Met Ala Asn Lys His Ser Asn Lys Arg Lys Asp
            100                 105                 110

Arg Thr Lys His Asp Leu Gly Leu Ala Ala Lys Glu Glu Arg Ile Ala
        115                 120                 125

Ile Gln Glu Glu Gln Glu Asp Arg Leu Ser Asn Glu Trp Leu Pro Val
    130                 135                 140

Glu Gly Leu Asp Ala Trp Asp Ser Leu Lys Thr Leu Gly Tyr Val Pro
145                 150                 155                 160

Ala Ser Ala Lys Lys Lys Ile Ser Lys Lys Lys Met Ser Ile Arg Met
                165                 170                 175

Leu Ser Gln Asp Glu Ala Ile Arg Gln Leu Glu Ser Ala Ala Glu Asn
            180                 185                 190

Phe Leu Ile Phe Leu Asn Glu Gln Glu His Lys Ile Gln Cys Ile Tyr
        195                 200                 205

Lys Lys His Asp Gly Asn Tyr Val Leu Ile Glu Pro Ser Leu Lys Pro
    210                 215                 220

Gly Phe Cys Ile
225
```

<210> 326
<211> 687
<212> DNA
<213> Chlamydia pneumoniae

<400> 326

```
atgagacctc atcgtaaaca cgtatcatct aaaagcttag ctttaaagca atctgcatca    60
actcatgtag agatcacaac aaaagccttt cgtctctcta tgcctctaaa acagctgatc   120
ctagagaaaa gcgaccacct ccccccatg  gaaacaatcc gtgtggtgct aacctctcat   180
aaagataagc taggcaccga ggtgcatgtt gtagcttctc atggcaaaga aatccttcaa   240
actaaggttc ataacgcaaa cccatacact gcagtgatca atgcttttaa gaaaatccgc   300
accatggcaa ataagcactc caataaacgt aaagacagga caaaacatga tctaggtctt   360
```

```
gcagcaaaag aagaacgtat cgcaatacag gaagaacaag aagatcgcct tagcaacgag    420
tggcttcctg tcgaaggcct cgatgcctgg gattctctaa aaactcttgg gtatgttccc    480
gcatcagcga aaaagaagat ctccaagaaa aagatgagca ttcgtatgct atctcaagac    540
gaggctatcc gccagctaga gtctgccgca gaaaacttcc tgatcttctt gaacgagcaa    600
gagcataaaa tccaatgcat ttataaaaaa catgacggca actatgtcct tattgaacct    660
tccctcaagc caggattctg catctga                                        687
```

<210> 327
<211> 126
<212> PRT
<213> Chlamydia pneumoniae

<400> 327

```
Met Glu Gln Pro Asn Cys Val Ile Gln Asp Thr Thr Thr Val Leu Tyr
1               5                   10                  15

Ala Leu Asn Ser Phe Asp Pro Arg Leu Ser Asp Asp Thr His Arg Leu
            20                  25                  30

Gly Lys Gln Ser Pro Leu Glu Ala Glu Asn Ala Leu Gly Glu Phe Ile
        35                  40                  45

Glu Gly Leu Asp Thr Asn Ser Phe Pro Leu Glu Glu Val Ala Ile Pro
    50                  55                  60

Ile Leu Pro Gly Tyr His Pro Lys Phe Tyr Leu Ser Phe Ile Asp Arg
65                  70                  75                  80

Asp Asp Gln Gly Val His Tyr Glu Val Leu Asp Gly Val Phe Leu Lys
                85                  90                  95

Thr Val Ala Ala Cys Ile Ile Glu Asn Ser Phe Leu Thr Asp Ser Met
            100                 105                 110

Ser Pro Glu Leu Leu Ser Glu Val Lys Glu Ala Leu Lys Arg
        115                 120                 125
```

<210> 328
<211> 381
<212> DNA
<213> Chlamydia pneumoniae

<400> 328

```
atggagcaac ccaattgtgt gattcaggat actacaactg ttttgtatgc cttaaatagc     60
tttgatccta gacttagtga tgacactcac agacttggga agcaatcacc tcttgaagca    120
gaaaatgctc ttggagaatt tattgaaggt ttggatacaa atagctttcc tttagaggaa    180
gttgccattc ccatcctgcc aggttatcac cctaagtttt atttatcttt catagatagg    240
gacgatcaag gtgtccacta tgaagtttta gatggcgtat tttaaagac agtcgctgct     300
tgtattatag agaactcctt cttaactgat tctatgagcc cggagcttct cagcgaagtt    360
aaggaagctc tgaaacgatg a                                              381
```

<210> 329
<211> 149
<212> PRT
<213> Chlamydia pneumoniae

<400> 329

```
Met Ala Val Gln Ser Ile Lys Glu Ala Val Thr Ser Ala Ala Thr Ser
1               5                   10                  15

Val Gly Cys Val Asn Cys Ser Arg Glu Ala Ile Pro Ala Phe Asn Thr
```

```
            20                  25                  30

Glu Glu Arg Ala Thr Ser Ile Ala Arg Ser Val Ile Ala Ala Ile Ile
        35                  40                  45

Ala Val Val Ala Ile Ser Leu Leu Gly Leu Gly Leu Val Val Leu Ala
    50                  55                  60

Gly Cys Cys Pro Leu Gly Met Ala Ala Gly Ala Ile Thr Met Leu Leu
65                  70                  75                  80

Gly Val Ala Leu Leu Ala Trp Ala Ile Leu Ile Thr Leu Arg Leu Leu
                85                  90                  95

Asn Ile Pro Lys Ala Glu Ile Pro Ser Pro Gly Asn Asn Gly Glu Pro
            100                 105                 110

Asn Glu Arg Asn Ser Ala Thr Pro Pro Leu Glu Gly Gly Val Ala Gly
        115                 120                 125

Glu Ala Gly Arg Gly Gly Gly Ser Pro Leu Thr Gln Leu Asp Leu Asn
    130                 135                 140

Ser Gly Ala Gly Ser
145

<210>   330
<211>   450
<212>   DNA
<213>   Chlamydia pneumoniae

<400>   330
atggctgttc aatctataaa agaagccgta acatcagccg caacatcagt aggatgtgta    60
aactgttcta gagaggctat accagcattt aatacagagg agagagcaac gagtattgct   120
agatctgtta tagcagctat cattgctgtt gtagctatct ccttactcgg actaggtctt   180
gtagttcttg ctggttgctg tcctttagga atggctgcgg gtgctataac aatgctgctg   240
ggtgtagcat tattagcttg ggcaatactg attactttga gactgcttaa tatacctaag   300
gctgaaatac cgagtccagg gaacaacggt gagcctaatg aaagaaattc agcaactcct   360
cctctagagg gtggtgttgc aggagaagcc ggtcgcggcg gggggtcacc tttaacccaa   420
cttgatctca attcaggggc gggaagttag                                    450

<210>   331
<211>   234
<212>   PRT
<213>   Chlamydia pneumoniae

<400>   331
Met Met Leu Arg Val Ile Glu Leu Pro Leu Leu Pro Ile Lys Gln Ala
1               5                   10                  15

Leu Glu Lys Ala Phe Val Gln Tyr Asn Ser Tyr Lys Ala Lys Leu Thr
            20                  25                  30

Lys Val Glu Pro Cys Phe Arg Glu Ser Pro Ala Tyr Ile Thr Ser Glu
        35                  40                  45

Glu Arg Leu Gln Ser Leu Asp Gln Thr Leu Glu Arg Ala Tyr Lys Glu
    50                  55                  60

Tyr Gln Lys Arg Phe Gln Glu Pro Ser Arg Leu Glu Ser Glu Val Ser
65                  70                  75                  80
```

Gly Cys Arg Glu His Leu Arg Glu Gln Val Lys Gln Phe Glu Thr Gln
85 90 95

Gly Leu Asp Leu Ile Lys Glu Glu Leu Ile Phe Val Ser Asp Val Leu
100 105 110

Phe Arg Lys Met Val Ser Cys Leu Val Ser Thr Val His Val Pro Phe
115 120 125

Met Glu Phe Tyr Tyr Glu Tyr Phe Glu Leu His Arg Leu Arg Leu Arg
130 135 140

Ala Gln Trp Met Ala Asn Ala Glu Ile Tyr Ser Lys Val Arg Lys Ala
145 150 155 160

Phe Pro Glu Met Leu Lys Glu Thr Leu Glu Lys Ala Lys Ala Pro Arg
165 170 175

Glu Glu Glu Tyr Trp Leu Leu Cys Glu Glu Arg Lys Ser Lys Glu Lys
180 185 190

Arg Leu Ile Leu Asn Lys Ile Glu Ala Ala Gln Gln Arg Val Lys Asp
195 200 205

Leu Glu Pro Pro Pro Ile Lys Glu Thr Gly Lys Gln Lys Arg Lys Lys
210 215 220

Glu Tyr Ser Phe Phe Ile Arg Leu Lys Ser
225 230

<210> 332
<211> 705
<212> DNA
<213> Chlamydia pneumoniae

<400> 332
atgatgcttc gtgtcataga gcttccacta cttcctataa agcaagcgtt ggagaaggct 60
tttgtacaat ataatagcta caaagcgaag ttaaccaagg tagaaccttg ctttagagag 120
agccctgcct atataactag cgaagagcga ctccagagtt tggatcagac tttagaacgt 180
gcgtacaaag agtaccagaa gagattccag gagccttcac gtttggaatc ggaagtaagt 240
ggatgtagag agcatcttag agagcaggta aaacaatttg aaactcaagg actagacttg 300
atcaaagaag agcttatttt tgttagtgat gtgttattcc gaaaaatggt cagttgtcta 360
gtgtcgacag tgcatgttcc ctttatggag ttttattatg agtattttga gttgcataga 420
ttgaggttgc gggcccaatg gatggcgaat gccgagattt atagcaaagt tagaaaagca 480
ttcccagaga tgttgaagga gaccttagaa aaagctaagg ctcccagaga agaagagtat 540
tggttacttt gcgaggagag aaagagtaag gagaagcgtt tgattctcaa caagatagag 600
gcagctcagc agcgggtaaa agatttagaa cctcctccta ttaaagagac agggaaacag 660
aaacggaaga aagaatattc gtttttcatt cgattaaaat cgtga 705

<210> 333
<211> 147
<212> PRT
<213> Chlamydia pneumoniae

<400> 333
Met Lys Lys Lys Val Thr Ile Asp Glu Ala Leu Lys Glu Ile Leu Arg
1 5 10 15

Leu Glu Gly Ala Ala Thr Gln Glu Glu Leu Cys Ala Lys Leu Leu Ala
20 25 30

Gln Gly Phe Ala Thr Thr Gln Ser Ser Val Ser Arg Trp Leu Arg Lys
35 40 45

Ile Gln Ala Val Lys Val Ala Gly Glu Arg Gly Ala Arg Tyr Ser Leu
50 55 60

Pro Ser Ser Thr Glu Lys Thr Thr Thr Arg His Leu Val Leu Ser Ile
65 70 75 80

Arg His Asn Ala Ser Leu Ile Val Ile Arg Thr Val Pro Gly Ser Ala
85 90 95

Ser Trp Ile Ala Ala Leu Leu Asp Gln Gly Leu Lys Asp Glu Ile Leu
100 105 110

Gly Thr Leu Ala Gly Asp Asp Thr Ile Phe Val Thr Pro Ile Asp Glu
115 120 125

Gly Arg Leu Pro Leu Leu Met Val Ser Ile Ala Asn Leu Leu Gln Val
130 135 140

Phe Leu Asp
145

<210> 334
<211> 444
<212> DNA
<213> Chlamydia pneumoniae

<400> 334
atgaaaaaaa aagtaactat agatgaggct ttaaaagaaa ttttacgtct tgaaggagcg 60
gcaactcagg aggaattatg tgcaaaactc ttagctcaag gttttgctac aacccagtcg 120
tctgtatctc gttggctacg aaagattcag gctgtaaagg ttgctggaga gcgtggtgct 180
cgttattctt taccctcttc aacagagaag accacgaccc gtcatttggt gctctctatt 240
cgccataacg cctctcttat tgtaattcgt acggttcctg gttcagcttc ttggatcgct 300
gctttgttag atcaagggct caaagatgaa attcttggaa ctttggcagg agatgacacg 360
atttttgtca ctcctataga tgaagggagg ctcccattgt tgatggtttc gattgcaaat 420
ttactgcaag ttttcttgga ttaa 444

<210> 335
<211> 128
<212> PRT
<213> Chlamydia pneumoniae

<400> 335
Met Ser Gln Cys Gln Ser Ser Ser Thr Ser Thr Trp Glu Trp Met Lys
1 5 10 15

Ser Phe Val Pro Asn Trp Lys Asn Pro Thr Pro Pro Leu Ser Pro Ile
20 25 30

Pro Ser Glu Asp Glu Phe Ile Leu Ala Tyr Glu Pro Phe Val Leu Pro
35 40 45

Lys Thr Asp Pro Glu Asn Ala Gln Ala Asn Pro Pro Gly Thr Ser Thr
50 55 60

Pro Asn Val Glu Asn Gly Ile Asp Asp Leu Asn Pro Leu Leu Gly Gln
65 70 75 80

Pro Asn Glu Gln Asn Asn Ala Asn Asn Pro Gly Thr Ser Gly Ser Asn
85 90 95

Pro Thr Ser Leu Pro Ala Pro Glu Arg Leu Pro Glu Thr Glu Glu Asn
100 105 110

Ser Gln Glu Glu Glu Gln Gly Ser Gln Asn Asn Glu Asp Leu Ile Gly
115 120 125

<210> 336
<211> 387
<212> DNA
<213> Chlamydia pneumoniae

<400> 336
atgtctcaat gtcagagtag cagtacatct acctgggaat ggatgaaatc ttttgtgcca 60
aactggaaga atccaactcc ccccttatct cctatacctt ctgaggacga atttatatta 120
gcatacgagc catttgttct accgaaaaca gatccagaaa acgcacaagc taatcctcca 180
ggcacatcta caccgaatgt agaaaacggg atcgatgatc tcaaccctct tctggggcaa 240
cccaacgaac aaaacaatgc caacaatcca ggaacttctg gatctaatcc tacatctcta 300
cccgcccccg aacgactccc tgaaactgaa gagaacagcc aagaagaaga acaaggatct 360
caaaataatg aggatcttat aggataa 387

<210> 337
<211> 129
<212> PRT
<213> Chlamydia pneumoniae

<400> 337
Leu Arg Glu Glu Gly Ser Val Ser Phe Arg Glu Tyr Phe Arg Ala Tyr
1 5 10 15

Met Cys Asp Lys Ile Val Ala Gln Lys Asn Phe Leu Phe Thr Leu Asp
20 25 30

Ala Val Ile Lys Gln Ala Gly Trp Arg Ser Gln Glu Lys Leu Asn Leu
35 40 45

Phe Tyr Val Glu Ser Gln Ala Leu Gly Arg Glu Ile Lys Val Ser Leu
50 55 60

Glu Glu Tyr Ile Gln Ser Met Val Gly Ile Leu Gly Ser Gln Arg Thr
65 70 75 80

Lys Lys Ser Phe Lys Phe Ser Val Asp Phe Thr Pro Leu Glu Gln Ala
85 90 95

Leu Gln Glu Arg Cys Ser Ser Asp Asp Asp Glu Asp Ala Thr Ala Thr
100 105 110

Ser Thr Ala Thr Gly Ala Thr Ala Ser Pro Thr Asp Met His Glu Asp
115 120 125

Glu

<210> 338
<211> 390
<212> DNA
<213> Chlamydia pneumoniae

<400> 338
ttgagagaag aaggtagtgt ttctttcaga gaatatttca gagcctatat gtgtgataaa 60
atcgtggcac agaagaactt cttatttact ttagacgctg taattaaaca ggccggttgg 120
agatcacaag agaaactcaa tttattttat gttgaaagtc aggctttagg aagagaaatc 180
aaagtcagct tagaggaata tattcagagt atggtcggga ttttgggatc tcagagaacc 240
aagaaaagct ttaagttttc tgtcgacttt accctttag agcaggctct acaagaaaga 300
tgctcttctg atgatgacga agatgcaaca gcaacttcga ccgctacagg ggcaacagca 360
tctccgactg acatgcacga agatgagtaa 390

<210> 339
<211> 222
<212> PRT
<213> Chlamydia pneumoniae

<400> 339
Met Leu Met Met Leu Met Met Ile Ile Gly Ile Thr Gly Gly Ser Gly
1 5 10 15

Ala Gly Lys Thr Thr Leu Thr Gln Asn Ile Lys Glu Ile Phe Gly Glu
20 25 30

Asp Val Ser Val Ile Cys Gln Asp Asn Tyr Tyr Lys Asp Arg Ser His
35 40 45

Tyr Thr Pro Glu Glu Arg Ala Asn Leu Ile Trp Asp His Pro Asp Ala
50 55 60

Phe Asp Asn Asp Leu Leu Ile Ser Asp Ile Lys Arg Leu Lys Asn Asn
65 70 75 80

Glu Ile Val Gln Ala Pro Val Phe Asp Phe Val Leu Gly Asn Arg Ser
85 90 95

Lys Thr Glu Ile Glu Thr Ile Tyr Pro Ser Lys Val Ile Leu Val Glu
100 105 110

Gly Ile Leu Val Phe Glu Asn Gln Glu Leu Arg Asp Leu Met Asp Ile
115 120 125

Arg Ile Phe Val Asp Thr Asp Ala Asp Glu Arg Ile Leu Arg Arg Met
130 135 140

Val Arg Asp Val Gln Glu Gln Gly Asp Ser Val Asp Cys Ile Met Ser
145 150 155 160

Arg Tyr Leu Ser Met Val Lys Pro Met His Glu Lys Phe Ile Glu Pro
165 170 175

Thr Arg Lys Tyr Ala Asp Ile Ile Val His Gly Asn Tyr Arg Gln Asn
180 185 190

Val Val Thr Asn Ile Leu Ser Gln Lys Ile Lys Asn His Leu Glu Asn
195 200 205

Ala Leu Glu Ser Asp Glu Thr Tyr Tyr Met Val Asn Ser Lys
210 215 220

<210> 340
<211> 669
<212> DNA

<213> Chlamydia pneumoniae

<400> 340
atgttgatga tgcttatgat gattattgga attacaggag gttctggagc tgggaaaacc 60
accctaaccc aaaacattaa agaaattttc ggtgaggatg tgagtgttat ctgccaagat 120
aattattaca aagatagatc tcattatact cctgaagaac gtgccaattt aatttgggat 180
catccggacg cctttgataa tgacttatta atttcagaca taaaacgtct aaaaaataat 240
gagattgtcc aagccccagt ttttgatttt gttttaggta atcgatctaa aacggagata 300
gaaacgatct atccatctaa agttattctt gttgaaggta ttctggtctt tgaaaatcaa 360
gaacttagag atcttatgga tattaggatc tttgtagaca ccgatgctga tgaaaggata 420
ctacgccgta tggttcgaga tgttcaagaa caaggagata gcgtggactg catcatgtct 480
cgttatcttt ctatggtaaa gcctatgcat gagaaattta tagagccgac tcggaaatat 540
gctgatatca ttgtacatgg aaattaccga caaaacgtag taacaaatat tttgtcacag 600
aaaattaaaa atcatttaga gaatgccctg gaaagcgatg agacgtatta tatggtcaac 660
tctaagtaa 669

<210> 341
<211> 117
<212> PRT
<213> Chlamydia pneumoniae

<400> 341
Val Gln Leu Phe Gln Tyr Met Asn Glu Ser Gly Trp Asp Trp Leu Cys
1 5 10 15

Asp Phe Asp Ser Gln Gly Glu Gly Phe Gln Leu Ser Arg Leu Val Gly
20 25 30

Leu Leu His Ser Ser Trp Ala Leu Tyr Glu Ala Lys Glu Gln Phe Tyr
35 40 45

Leu Pro Glu Val Ser Leu Leu Thr Trp Glu Glu Leu Ile Glu Met Gln
50 55 60

Leu Leu Ser Lys Pro Thr Lys His Gly Val Ala Lys Asp Leu Cys Asn
65 70 75 80

Val Phe Glu Lys His Phe Gln Arg Phe Arg Gln Tyr Leu Gly Ser Leu
85 90 95

Asp Leu Asn Gln Arg Phe Glu Asn Thr Phe Leu Asn Tyr Pro Lys Tyr
100 105 110

His Leu Asp Arg Glu
115

<210> 342
<211> 354
<212> DNA
<213> Chlamydia pneumoniae

<400> 342
gtgcaattat ttcaatatat gaatgagtcc ggatgggatt ggctttgtga ttttgattct 60
caaggcgagg gattccagtt atcacgtctg gttgggctgt tacattcgtc ctgggcatta 120
tacgaagcaa aagagcaatt ttaccttcct gaggtttctc tattgacctg ggaagaactg 180
atagaaatgc agttattaag caaaccaaca aaacacgggg ttgcaaaaga tctttgtaat 240
gtatttgaaa aacactttca aaggtttaga cagtacctag gttccttaga tctaaatcaa 300
aggttcgaaa ataccttctt gaattatcct aaataccatt tagataggga gtga 354

<210> 343
<211> 119

<212> PRT
<213> Chlamydia pneumoniae

<400> 343
Met Pro Val Ser Ser Ala Pro Leu Pro Thr Ser His Arg Pro Ser Ser
1 5 10 15

Gly Asn Leu Gly Leu Met Glu Pro Asn Ser Lys Ala Leu Lys Ala Lys
20 25 30

His Gln Asp Lys Thr Thr Lys Thr Ile Lys Leu Leu Val Lys Ile Leu
35 40 45

Val Ala Ile Leu Val Ile Glu Val Leu Gly Ile Ile Ala Ala Phe Phe
50 55 60

Ile Pro Gly Thr Pro Pro Ile Cys Leu Ile Ile Leu Gly Gly Leu Ile
65 70 75 80

Leu Thr Thr Val Leu Cys Val Leu Leu Leu Val Ile Lys Leu Ala Leu
85 90 95

Val Asn Lys Thr Glu Gly Thr Thr Ala Glu Gln Gln Ile Lys Arg Lys
100 105 110

Leu Ser Ser Lys Ser Ile Ser
115

<210> 344
<211> 360
<212> DNA
<213> Chlamydia pneumoniae

<400> 344
atgcccgtgt cctcagcccc cctacccaca agccaccgcc cttcctctgg aaatctaggc 60
ctcatggaac caaattccaa agctctaaaa gcaaagcatc aagataaaac gacgaagacg 120
attaaacttt tagttaaaat ccttgttgcc attctagtaa tagaagtttt aggaataatt 180
gcagctttct ttattcctgg gactcctccc atctgcttga ttatcctagg aggccttatt 240
cttacaacag tactctgtgt gcttcttctt gttataaagc ttgcccttgt aaacaaaacc 300
gaaggaacaa ctgctgaaca gcagataaaa cgtaaactct cttctaaaag tatttcttag 360

<210> 345
<211> 80
<212> PRT
<213> Chlamydia pneumoniae

<400> 345
Met Lys Pro Asn Ser Ile Ile Phe Leu Glu Asn Thr Lys His Tyr Pro
1 5 10 15

Asp Ile Phe Arg Glu Gly Phe Val Arg Asp Arg His Gly Leu Met Glu
20 25 30

Ala Ser Asp Trp Leu Leu Ser Thr Glu Ile Thr Ile Ile Arg Ser Ile
35 40 45

Leu Gly Ala Ile Pro Ile Leu Gly Asn Ile Leu Gly Ala Gly Arg Leu
50 55 60

Tyr Ser Val Trp Tyr Thr Ser Asp Glu Asp Trp Lys Lys Gln Val Val
65 70 75 80

<210> 346
<211> 243
<212> DNA
<213> Chlamydia pneumoniae

<400> 346
atgaagccaa atagtattat ttttttagaa aatactaagc attatcccga catctttcga 60
gaaggatttg ttcgtgatcg tcatggacta atggaagcct cggattggtt actttctacg 120
gaaattacga tcattcgctc cattctggga gctatcccta ttttaggaaa tattcttgga 180
gccggacgac tctatagcgt ttggtataca agtgacgaag attggaaaaa acaagtggtt 240
tga 243

<210> 347
<211> 181
<212> PRT
<213> Chlamydia pneumoniae

<400> 347
Met Pro Val Pro Ile Asp Asn Ser Ser Arg Asn Leu Gln Glu Val Pro
1 5 10 15

Glu Ser Leu Glu Asp Leu Glu Gln His Ala Glu Glu Ser Pro Thr His
20 25 30

Gln Ser Ala Glu Ser Ser Ser Leu Gln Leu Ser Leu Ala Ser Ser Ala
35 40 45

Ile Ser Ser Arg Val Glu Gln Leu Ser Ser Leu Val Leu Gly Met Glu
50 55 60

Asn Ser Asp Phe Ser Ser Leu Arg Asp Val Pro Ile Phe Ser Ala Ile
65 70 75 80

Tyr Glu Ser Ser Thr His Thr Pro Val Pro Thr Pro Leu Val Gly Val
85 90 95

Gly Tyr Ile Asn Gly Ser Gln Ser Gly Tyr Tyr Asp Thr Gln Arg Glu
100 105 110

Ser Leu His Leu Ser Gln Leu Leu Gly Ser Arg Arg Val Glu Val Val
115 120 125

Tyr Asn Gln Gly Asn Phe Met Glu Ala Ser Leu Leu Asn Leu Cys Pro
130 135 140

Arg Arg Pro Arg Arg Asp Pro Ser Pro Ile Ser Leu Ala Leu Leu Glu
145 150 155 160

Leu Trp Glu Ala Phe Phe Leu Glu His Pro Pro Gly Ser Thr Phe Asn
165 170 175

Pro Ile Phe Phe Trp
180

<210> 348
<211> 546
<212> DNA
<213> Chlamydia pneumoniae

<400> 348
atgcccgttc ctatagataa ttcctctcgc aacctacaag aagttccaga aagcctagaa 60
gacctcgaac aacacgcaga agaatctcct actcatcaaa gtgcagaaag cagttctttg 120
caactgtctc tagcctcctc agcaatttct agtagagtag aacaactatc ttccctcgtc 180
ttaggaatgg aaaattcaga tttctcctct ttaagagacg ttcctatctt ctcagctatc 240
tacgaatctt caacacacac acctgtcccc actcctctag ttggcgtggg atatatcaac 300
ggaagtcaat caggatacta cgatacacaa agagaatctc ttcacctcag ccaattgtta 360
ggaagccgaa gagttgaagt tgtctataac caaggaaact tcatggaggc ctctttgcta 420
aatctgtgcc ccagaagacc tcgaagagat ccctctccaa tttctttagc tctattagag 480
ctctgggaag catttttttt agaacacccc ccaggtagca cttttaatcc aatatttttt 540
tggtaa 546

<210> 349
<211> 133
<212> PRT
<213> Chlamydia pneumoniae

<400> 349
Leu Asn Phe Val Ser Thr Leu Thr Gly Ser Asp Phe Tyr Ala Pro Val
1 5 10 15

Leu Glu Lys Leu Glu Glu Ala Phe Ala Asp Thr Thr Gly Gln Val Ile
20 25 30

Leu Phe Ser Ser Ser Pro Asp Phe Ile Val His Pro Ile Ala Gln Gln
35 40 45

Leu Gly Ile Ser Ser Trp Tyr Ala Ser Cys Tyr Arg Asp Gln Ser Ala
50 55 60

Glu Gln Thr Ile Tyr Lys Lys Cys Leu Thr Gly Asp Lys Lys Ala Gln
65 70 75 80

Ile Leu Ser Tyr Ile Lys Lys Ile Asn Gln Ala Arg Ser His Thr Phe
85 90 95

Ser Asp His Ile Leu Asp Leu Pro Phe Leu Met Leu Gly Glu Glu Lys
100 105 110

Thr Val Val Arg Pro Gln Gly Arg Leu Lys Lys Met Ala Lys Lys Tyr
115 120 125

Tyr Trp Asn Ile Val
130

<210> 350
<211> 402
<212> DNA
<213> Chlamydia pneumoniae

<400> 350
ttgaattttg tatcgactct gaccggctcc gatttttatg ctcctgtttt agaaaaacta 60
gaagaagctt ttgcagatac cacaggacag gtgatccttt ttcttcttc tccagacttt 120
attgtccacc ccatagcgca gcaactcggg attagttctt ggtatgcgtc gtgttatcgc 180
gatcagtctg cagaacagac gatctataaa aaatgtctta caggggataa aaaagcgcaa 240
attttgagtt atattaaaaa aattaatcaa gcaagaagcc ataccttctc cgaccatatt 300
ttagatcttc cttttcttat gctgggagaa gagaaaaccg tcgttcgccc tcagggacga 360
ctcaagaaaa tggcaaaaaa atattactgg aatatcgttt aa 402

<210> 351
<211> 162

<212> PRT
<213> Chlamydia pneumoniae

<400> 351
Met Met His Arg Tyr Phe Ile Pro Leu Leu Ala Leu Leu Ile Phe Ser
1 5 10 15

Pro Ser Leu Val Arg Ala Glu Leu Gln Pro Ser Glu Asn Arg Lys Gly
20 25 30

Gly Trp Pro Thr Gln Leu Ser Cys Ala Glu Gly Ser Gln Leu Phe Cys
35 40 45

Lys Phe Glu Ala Ala Tyr Asn Asn Ala Ile Glu Glu Gly Lys Pro Gly
50 55 60

Ile Leu Val Phe Phe Ser Glu Arg Pro Thr Pro Glu Phe Ala Asp Leu
65 70 75 80

Thr Asn Gly Ser Phe Ser Leu Ser Thr Pro Ile Ala Lys Gly Phe Asn
85 90 95

Val Val Val Leu Cys Pro Gly Leu Ile Ser Pro Leu Asp Phe Phe His
100 105 110

Lys Met Asp Pro Val Ile Leu Tyr Met Gly Ser Phe Leu Glu Met Phe
115 120 125

Pro Glu Val Glu Ala Val Ser Gly Pro Arg Leu Cys Tyr Ile Leu Ile
130 135 140

Asp Glu Gln Gly Gly Ala Gln Cys Gln Ala Val Leu Pro Leu Glu Thr
145 150 155 160

Lys Asn

<210> 352
<211> 489
<212> DNA
<213> Chlamydia pneumoniae

<400> 352
atgatgcacc gttattttat tcctttatta gcacttctca ttttctctcc ttctttagtc 60
agggcagagc tacaaccaag tgaaaacaga aaagggggt ggcctacaca actttcctgt 120
gcagaaggtt cgcaactctt ctgtaaattc gaagctgcct ataataatgc aattgaggaa 180
gggaaacctg ggattttagt ctttttctct gagcgaccca caccagaatt tgccgactta 240
acgaatggtt cattttctct ctctacgcca atcgccaagg gctttaatgt cgttgtgtta 300
tgccccgggc ttatcagtcc cttagacttt ttccacaaaa tggatcctgt gattctctat 360
atgggaagtt ttctagagat gttccctgaa gtggaggcag ttagtggccc tcgcttatgt 420
tatatcttaa tagatgaaca gggtggggct caatgtcagg ctgtcctgcc tttagaaaca 480
aagaattag 489

<210> 353
<211> 219
<212> PRT
<213> Chlamydia pneumoniae

<400> 353
Met Gln Arg Ile Ile Ile Val Gly Ile Asp Thr Gly Val Gly Lys Thr
1 5 10 15

```
Ile Val Ser Ala Ile Leu Ala Arg Ala Leu Asn Ala Glu Tyr Trp Lys
            20                  25                  30

Pro Ile Gln Ala Gly Asn Leu Glu Asn Ser Asp Ser Asn Ile Val His
        35                  40                  45

Glu Leu Ser Gly Ala Tyr Cys His Pro Glu Ala Tyr Arg Leu His Lys
    50                  55                  60

Pro Leu Ser Pro His Lys Ala Ala Gln Ile Asp Asn Val Ser Ile Glu
65                  70                  75                  80

Glu Ser His Ile Cys Ala Pro Lys Thr Thr Ser Asn Leu Ile Ile Glu
                85                  90                  95

Thr Ser Gly Gly Phe Leu Ser Pro Cys Thr Ser Lys Arg Leu Gln Gly
            100                 105                 110

Asp Val Phe Ser Ser Trp Ser Cys Ser Trp Ile Leu Val Ser Gln Ala
        115                 120                 125

Tyr Leu Gly Ser Ile Asn His Thr Cys Leu Thr Val Glu Ala Met Arg
    130                 135                 140

Ser Arg Asn Leu Asn Ile Leu Gly Met Val Val Asn Gly Tyr Pro Glu
145                 150                 155                 160

Asp Glu Glu His Trp Leu Thr Gln Glu Ile Lys Leu Pro Ile Ile Gly
                165                 170                 175

Thr Leu Ala Lys Glu Lys Glu Ile Thr Lys Thr Ile Ile Ser Cys Tyr
            180                 185                 190

Ala Glu Gln Trp Lys Glu Val Trp Thr Ser Asn His Gln Gly Ile Gln
        195                 200                 205

Gly Val Ser Gly Thr Pro Ser Leu Asn Leu His
    210                 215

<210>   354
<211>   660
<212>   DNA
<213>   Chlamydia pneumoniae

<400>   354
atgcaacgta tcatcattgt aggaatcgac actggcgtag gaaaaaccat tgtcagtgct    60
atccttgcta gagcacttaa cgcagaatac tggaaaccta tacaagcagg gaatctagaa   120
aattcagata gcaatattgt tcatgagcta tcgggagcct actgtcatcc cgaagcttat   180
cgattgcata agcccttgtc tccacacaag gcagcgcaaa tcgataatgt aagtatcgaa   240
gagagtcata tttgtgcgcc aaaaacaact tcgaatctga ttattgagac ttcaggagga   300
tttttatccc cctgcacatc aaaaagactt cagggagatg tgttttcttc ttggtcatgt   360
tcttggattt tagtgagcca agcatatctc ggaagtatca atcacacctg tttaacggta   420
gaagcaatgc gctcacgaaa cctcaatatc ttaggtatgg tggtaaatgg gtatccagag   480
gacgaagagc actggctaac tcaagaaatc aagcttccta taatcgggac tcttgccaag   540
gaaaaagaaa tcacaaagac aatcataagc tgttatgccg aacaatggaa ggaagtatgg   600
acaagcaatc atcagggaat tcagggtgta tctggcaccc cttcactcaa tctgcattag   660

<210>   355
<211>   811
<212>   PRT
```

<213> Chlamydia pneumoniae

<400> 355

Met Gln Val Leu Leu Ser Pro Gln Leu Pro Pro Pro Pro Gln His Ser
1 5 10 15

Val Gly Ser Ile Ser Ser Pro Ser Lys Leu Arg Val Leu Ala Ile Thr
20 25 30

Phe Leu Val Phe Gly Met Leu Leu Leu Ile Ser Gly Ala Leu Phe Leu
35 40 45

Thr Leu Gly Ile Pro Gly Leu Ser Ala Ala Ile Ser Phe Gly Leu Gly
50 55 60

Ile Gly Leu Ser Ala Leu Gly Gly Val Leu Met Ile Ser Gly Leu Leu
65 70 75 80

Cys Leu Leu Val Lys Arg Glu Ile Pro Thr Val Arg Pro Glu Glu Ile
85 90 95

Pro Glu Gly Val Ser Leu Ala Pro Ser Glu Glu Pro Ala Leu Gln Ala
100 105 110

Ala Gln Lys Thr Leu Ala Gln Leu Pro Lys Glu Leu Asp Gln Leu Asp
115 120 125

Thr Asp Ile Gln Glu Val Phe Ala Cys Leu Arg Lys Leu Lys Asp Ser
130 135 140

Lys Tyr Glu Ser Arg Ser Phe Leu Asn Asp Ala Lys Lys Glu Leu Arg
145 150 155 160

Val Phe Asp Phe Val Val Glu Asp Thr Leu Ser Glu Ile Phe Glu Leu
165 170 175

Arg Gln Ile Val Ala Gln Glu Gly Trp Asp Leu Asn Phe Leu Ile Asn
180 185 190

Gly Gly Arg Ser Leu Met Met Thr Ala Glu Ser Glu Ser Leu Asp Leu
195 200 205

Phe His Val Ser Lys Arg Leu Gly Tyr Leu Pro Ser Gly Asp Val Arg
210 215 220

Gly Glu Gly Leu Lys Lys Ser Ala Lys Glu Ile Val Ala Arg Leu Met
225 230 235 240

Ser Leu His Cys Glu Ile His Lys Val Ala Val Ala Phe Asp Arg Asn
245 250 255

Ser Tyr Ala Met Ala Glu Lys Ala Phe Ala Lys Ala Leu Gly Ala Leu
260 265 270

Glu Glu Ser Val Tyr Arg Ser Leu Thr Gln Ser Tyr Arg Asp Lys Phe
275 280 285

Leu Glu Ser Glu Arg Ala Lys Ile Pro Trp Asn Gly His Ile Thr Trp
290 295 300

Leu Arg Asp Asp Ala Lys Ser Gly Cys Ala Glu Lys Lys Leu Arg Asp

305 310 315 320

Ala Glu Glu Arg Trp Lys Lys Phe Arg Lys Ala Val Phe Trp Val Glu
325 330 335

Glu Asp Gly Gly Phe Asp Ile Asn Asn Leu Leu Gly Asp Trp Gly Thr
340 345 350

Val Leu Asp Pro Tyr Arg Gln Glu Arg Met Asp Glu Ile Thr Phe His
355 360 365

Glu Leu Tyr Glu Lys Thr Thr Phe Leu Lys Arg Leu His Arg Lys Cys
370 375 380

Ala Leu Ala Lys Thr Thr Phe Glu Lys Lys Arg Ser Lys Lys Asn Leu
385 390 395 400

Gln Ala Val Glu Glu Ala Asn Ala Arg Arg Leu Lys Tyr Val Arg Asp
405 410 415

Trp Tyr Asp Gln Glu Phe Gln Lys Ala Gly Glu Arg Leu Glu Lys Leu
420 425 430

His Ala Leu Tyr Pro Glu Val Ser Val Ser Ile Arg Glu Asn Lys Ile
435 440 445

Gln Glu Thr Arg Ser Asn Leu Glu Lys Ala Tyr Glu Ala Ile Glu Glu
450 455 460

Asn Tyr Arg Cys Cys Val Arg Glu Gln Glu Asp Tyr Trp Lys Glu Glu
465 470 475 480

Glu Lys Arg Glu Ala Glu Phe Arg Glu Arg Gly Asn Lys Ile Leu Ser
485 490 495

Pro Glu Glu Leu Glu Ser Ser Leu Glu Gln Phe Asp His Gly Leu Lys
500 505 510

Asn Phe Ser Glu Lys Leu Met Glu Leu Glu Gly His Ile Leu Lys Leu
515 520 525

Gln Lys Glu Ala Thr Ala Glu Val Glu Asn Lys Ile Leu Ser Asp Ala
530 535 540

Glu Ser Arg Leu Glu Ile Val Phe Glu Asp Val Lys Glu Met Pro Cys
545 550 555 560

Arg Ile Glu Glu Ile Glu Lys Thr Leu Arg Met Ala Glu Leu Pro Leu
565 570 575

Leu Pro Thr Lys Lys Ala Phe Glu Lys Ala Cys Ser Gln Tyr Asn Ser
580 585 590

Cys Ala Glu Met Leu Glu Lys Val Lys Pro Tyr Cys Lys Glu Ser Leu
595 600 605

Ala Tyr Val Thr Ser Lys Glu Arg Leu Val Ser Leu Asp Glu Asp Leu
610 615 620

Arg Arg Ala Tyr Thr Glu Cys Gln Lys Arg Phe Gln Gly Asp Ser Gly
625 630 635 640

```
Leu Glu Ser Glu Val Arg Ala Cys Arg Glu Gln Leu Arg Glu Arg Ile
                645                 650                 655

Gln Glu Phe Glu Thr Gln Gly Leu Asp Leu Val Glu Lys Glu Leu Leu
            660                 665                 670

Cys Val Ser Ser Arg Leu Arg Asn Thr Glu Cys Asp Cys Val Ser Gly
        675                 680                 685

Val Lys Lys Glu Ala Pro Pro Gly Lys Lys Phe Tyr Ala Gln Tyr Tyr
    690                 695                 700

Asp Glu Ile Tyr Arg Val Arg Val Gln Ser Arg Trp Met Thr Met Ser
705                 710                 715                 720

Glu Arg Leu Arg Glu Gly Val Gln Ala Cys Asn Lys Met Leu Lys Ala
                725                 730                 735

Gly Leu Ser Glu Glu Asp Lys Val Leu Lys Glu Glu Glu Tyr Trp Leu
            740                 745                 750

Tyr Arg Glu Glu Arg Lys Asn Lys Glu Lys Arg Leu Val Gly Thr Lys
        755                 760                 765

Ile Val Ala Thr Gln Gln Arg Val Ala Ala Phe Glu Ser Ile Glu Val
    770                 775                 780

Pro Glu Ile Pro Glu Ala Pro Glu Glu Lys Pro Ser Leu Leu Asp Lys
785                 790                 795                 800

Ala Arg Ser Leu Phe Thr Arg Glu Asp His Thr
                805                 810

<210>   356
<211>   2436
<212>   DNA
<213>   Chlamydia pneumoniae

<400>   356
atgcaagtac ttctatctcc gcagctaccc cccccccccc aacactctgt agggtcgatt    60
tcttctccat ctaaacttcg cgttttagcg attacttttt tagtttttgg tatgctctta   120
ctgatttcag gagctctctt tctgacgtta gggattccag gattgagtgc agcaatttct   180
tttggattag gcatcggtct ctccgcatta ggaggagtgc tgatgatttc gggactacta   240
tgtcttttag taaaacgaga gattccgaca gtacgaccag aagaaattcc tgaaggggtt   300
tcgctggctc cttctgagga gccagctcta caggcagctc agaagacttt agctcagctg   360
cctaaggaat tggatcagtt agatacagat attcaggaag tgttcgcatg tttaagaaag   420
ctgaaagatt ctaagtatga aagtcgaagt tttttaaacg atgctaagaa ggagcttcga   480
gtttttgact ttgtggttga ggataccctc tcggagattt tcgagttgcg gcagattgtg   540
gctcaagagg gatgggattt aaactttttg atcaatgggg gacgaagcct catgatgact   600
gcagaatctg aatcgcttga tttgtttcat gtatcgaagc ggctagggta tttaccttct   660
ggggatgttc gaggggaggg gttaaagaaa tctgcgaagg agatagtcgc tcgtttgatg   720
agcttgcatt gcgagattca caaggtggcg gtagcgtttg ataggaattc ctatgcgatg   780
gcagaaaagg cgtttgcgaa agcgttggga gctttagaag agagtgtgta tcggagtctg   840
acgcagagtt atagagataa atttttggag agcgagaggg cgaagatccc atggaatggg   900
catataacct ggttaagaga tgatgcgaag agtgggtgtg ctgaaaagaa gcttcgggat   960
gccgaggaac gttggaagaa atttaggaaa gcagtctttt gggtagaaga agacgggggc  1020
tttgacatca ataatctcct tggagactgg gggacagtgc ttgatcctta tagacaagag  1080
agaatggacg agataacgtt ccatgagttg tatgaaaaaa ctacgttttt gaaaagactg  1140
cacagaaagt gtgcgttagc gaaaacaacc tttgaaaaga agagatctaa aaagaatttg  1200
caggcagtcg aggaggcgaa tgcacgtagg ttgaaatatg taagggattg gtatgatcag  1260
```

```
gagtttcaga aagcagggga gagattagag aaactgcatg ctttgtatcc tgaggtttca    1320
gtctctataa gagagaacaa aatacaagag acgcgctcta atttagagaa agcctatgag    1380
gctatcgaag agaactatcg ttgctgtgtc cgagagcaag aggactactg gaaagaagaa    1440
gagaaaaggg aagcggagtt tagggagagg ggaaacaaga ttctttctcc tgaggagctg    1500
gaaagttctt tggagcaatt cgaccatggt ttgaaaaatt tttctgagaa attaatggaa    1560
ttggaagggc atatcttaaa acttcagaaa gaagccacag cagaggtgga gaataaaata    1620
ctttcagatg cagagagccg ccttgagatt gtatttgaag atgtcaagga gatgccctgt    1680
cgaattgagg agatagagaa gacgctgcgt atggcggagc tgcccctact tcctacgaag    1740
aaggcgtttg agaaggcctg ctcacaatat aatagctgcg cagagatgtt ggagaaggtg    1800
aagccttact gcaaggagag cctcgcctat gtgactagca aagagcgttt agtgagcttg    1860
gatgaagatt tacgacgagc ctacacagag tgtcagaaga gattccaggg ggattcgggt    1920
ttggagtcgg aagtaagagc ctgtcgagag caactgcgag agcggatcca agagtttgaa    1980
actcaagggc tggacttggt ggaaaaagag ttgctttgtg tgagtagtag attaagaaat    2040
acagagtgcg attgtgtatc tggtgttaag aaagaagcac ctcctggtaa gaagttttat    2100
gcccagtatt atgatgagat ttatcgagtt agagttcaat cccgatggat gacgatgtct    2160
gagagattga gagagggagt tcaagcatgc aacaagatgt tgaaggcagg cctaagcgaa    2220
gaagataagg ttcttaaaga agaagagtat tggttgtatc gagaggagag aaagaataaa    2280
gagaaacgtt tggttggtac taagatagta gcaacgcagc agcgagttgc agcatttgaa    2340
tccatagaag ttcctgagat tcctgaggcc ccagaggaga aaccgagttt gctggataaa    2400
gcgcgttctt tatttactcg cgaggaccat acctag                              2436
```

<210> 357
<211> 212
<212> PRT
<213> Chlamydia pneumoniae

<400> 357

```
Met Ala Thr Ser Val Ala Pro Ser Pro Val Pro Glu Ser Ser Pro Leu
1               5                   10                  15

Ser His Ala Thr Glu Val Leu Asn Leu Pro Asn Ala Tyr Ile Thr Gln
            20                  25                  30

Pro His Pro Ile Pro Ala Ala Pro Trp Glu Thr Phe Arg Ser Lys Leu
        35                  40                  45

Ser Thr Lys His Thr Leu Cys Phe Ala Leu Thr Leu Leu Leu Thr Leu
    50                  55                  60

Gly Gly Thr Ile Ser Ala Gly Tyr Ala Gly Tyr Thr Gly Asn Trp Ile
65                  70                  75                  80

Ile Cys Gly Ile Gly Leu Gly Ile Ile Val Leu Thr Leu Ile Leu Ala
                85                  90                  95

Leu Leu Leu Ala Ile Pro Leu Lys Asn Lys Gln Thr Gly Thr Lys Leu
            100                 105                 110

Ile Asp Glu Ile Ser Gln Asp Ile Ser Ser Ile Gly Ser Gly Phe Val
        115                 120                 125

Gln Arg Tyr Gly Leu Met Phe Ser Thr Ile Lys Ser Val His Leu Pro
    130                 135                 140

Glu Leu Thr Thr Gln Asn Gln Glu Lys Thr Arg Ile Leu Asn Glu Ile
145                 150                 155                 160

Glu Ala Lys Lys Glu Ser Ile Gln Asn Leu Glu Leu Lys Ile Thr Glu
                165                 170                 175

Cys Gln Asn Lys Leu Ala Gln Lys Gln Pro Lys Arg Lys Ser Ser Gln
```

```
            180                 185                 190

Lys Ser Phe Met Arg Ser Ile Lys His Leu Ser Lys Asn Pro Val Ile
        195                 200                 205

Leu Phe Asp Cys
    210
```

<210> 358
<211> 639
<212> DNA
<213> Chlamydia pneumoniae

<400> 358

```
atggcaactt ccgtagcccc atcaccagtc cccgagagca gccctctctc tcatgctaca   60
gaagttctca atcttcctaa tgcttatatt acgcagcctc atccgattcc agcggctcct  120
tgggagacct ttcgctccaa actttccaca aagcatacgc tctgttttgc cttaacacta  180
ctgttaacct taggggggaac gatctcagca ggttacgcag gatatactgg aaactggatc  240
atctgtggca tcggcttggg aattatcgta ctcacactga ttcttgctct tcttctagca  300
atccctctta aaaataagca gacaggaaca aaactgattg atgagatatc tcaagacatt  360
tcctctatag gatcaggatt tgttcagaga tacgggttga tgttctctac aattaaaagc  420
gtgcatcttc cagagctgac aacacaaaat caagaaaaaa caagaatttt aaatgaaatt  480
gaagcgaaaa aggaatcgat ccaaaatctt gagcttaaaa ttactgagtg ccaaaacaag  540
ttagcacaga aacagccgaa acggaaatca tctcagaaat catttatgcg tagtattaag  600
cacctctcca agaaccctgt aattttgttc gattgctga                          639
```

<210> 359
<211> 114
<212> PRT
<213> Chlamydia pneumoniae

<400> 359

```
Met Ser Tyr Tyr Phe Ser Leu Trp Tyr Leu Lys Val Gln Gln His Phe
1               5                   10                  15

Gln Ala Ala Phe Asp Phe Thr Arg Ser Leu Cys Ser Arg Ile Ser Asn
            20                  25                  30

Phe Ala Leu Gly Val Ile Ala Leu Leu Pro Ile Ile Gly Gln Leu Tyr
        35                  40                  45

Val Gly Leu Asp Trp Leu Leu Ser Arg Ile Lys Lys Pro Glu Phe Pro
    50                  55                  60

Ser Asp Val Asp Gln Ile Val Arg Val Glu His Val Val Gly His Asp
65                  70                  75                  80

His Arg Ser Arg Val Glu Asp Ile Leu Lys Arg Gln Arg Leu Ser Leu
                85                  90                  95

Glu Pro Arg Asp Glu Gly Lys Val His Gly Asp Leu Pro Ser Ala Pro
            100                 105                 110

Phe Phe
```

<210> 360
<211> 345
<212> DNA
<213> Chlamydia pneumoniae

<400> 360
atgagttatt acttttctct ttggtatctg aaggtgcaac agcactttca agcagcattt 60
gattttactc gctccctgtg ttcacgaatt tctaattttg ctttgggagt gattgcattg 120
cttcctatta ttgggcagtt gtatgtaggg ctggactggc tcctctctag gataaaaaag 180
ccagaatttc cttccgatgt ggatcagatc gtgcgagtag aacacgtcgt gggtcacgac 240
catagaagtc gagttgaaga tattctaaag agacaaaggc tctcattaga gcctagagac 300
gaggggaagg ttcacggaga tctgccttca gctccttttt tttga 345

<210> 361
<211> 158
<212> PRT
<213> Chlamydia pneumoniae

<400> 361
Leu Asn Gln Asp Leu Gln Asn Val Tyr Gln Glu Cys Gln Lys Ala Thr
1 5 10 15

Gly Leu Glu Ser Glu Val Ser Ala Tyr Arg Asp His Leu Arg Glu Gln
20 25 30

Ile Thr Glu Phe Glu Thr Gln Gly Leu Asp Val Ile Lys Glu Glu Leu
35 40 45

Leu Phe Val Ser Ser Thr Leu Lys Ser Lys Leu Ser Tyr Asp Pro Leu
50 55 60

Ile Ala Asp Ile Pro Cys Met Lys Phe Tyr Glu Glu Tyr Tyr Asp Gly
65 70 75 80

Ile Asp Lys Ala Arg Val Gln Ser Arg Trp Leu Glu Lys Ser Glu Arg
85 90 95

Tyr Arg Lys Ala Lys Lys Gly Phe Gln Glu Met Leu Lys Glu Gly Leu
100 105 110

Phe Lys Glu Asp Gln Ala Leu Lys Lys Ala Glu Tyr Arg Leu Leu Arg
115 120 125

Glu Lys Arg Met Asn Lys Glu Lys Leu Leu Ile Cys Asn Lys Ile Glu
130 135 140

Ala Ala Gln Gln Arg Val Gln Glu Phe Gly Pro Ser Asp Ser
145 150 155

<210> 362
<211> 477
<212> DNA
<213> Chlamydia pneumoniae

<400> 362
ttgaatcagg atttacaaaa tgtataccaa gagtgccaga aggctacagg tttagaatcg 60
gaagtgagtg catatagaga tcatcttaga gagcagatca cagagtttga aactcaaggg 120
ctggacgtga taaaagaaga acttcttttt gtgagtagta ctctcaaaag taaattgagc 180
tatgatccat taatagcaga cattccctgt atgaagtttt atgaggagta ttatgatggc 240
attgataaag cgagagttca atcccgatgg ctggagaagt ctgagaggta tagaaaggcg 300
aagaagggat tccaagagat gctgaaggaa ggcctattca aagaagatca ggctttgaaa 360
aaagcagagt atagattact tcgagagaag agaatgaata aggagaagct tttgatttgc 420
aataagatag aagcagctca gcagcgagtc caagaatttg gaccctcgga ttcataa 477

<210> 363
<211> 165

<212> PRT
<213> Chlamydia pneumoniae

<400> 363
Met Asn Ile Pro Ala Pro Gln Val Pro Val Ile Asp Glu Pro Val Val
1 5 10 15

Asn Asn Thr Ser Ser Tyr Gly Leu Ser Leu Lys Ser Ser Leu Arg Pro
20 25 30

Ile Thr Tyr Leu Ile Leu Ala Ile Leu Ala Ile Ala Thr Leu Met Ser
35 40 45

Val Leu Tyr Phe Cys Gly Ile Ile Ser Val Gly Thr Phe Val Leu Gly
50 55 60

Met Leu Ile Pro Leu Ser Val Cys Ser Val Leu Cys Val Ala Tyr Leu
65 70 75 80

Phe Tyr Gln Gln Ser Ser Ile Glu Lys Thr Lys Val Phe Ser Ile Thr
85 90 95

Ser Pro Ser Val Phe Phe Ser Asp Glu Asp Leu Asn Leu Leu Leu Gly
100 105 110

Arg Glu Glu Asp Ser Val Ser Ala Ile Asp Glu Leu Leu Lys Asn Phe
115 120 125

Pro Ala Asp Asp Phe Arg Arg Pro Lys Met Leu Pro Tyr Ser Asn Phe
130 135 140

Leu Asp Glu Gln Gly Arg Pro Asn Glu Ser Arg Glu Glu Asp Ser His
145 150 155 160

Thr Ser Lys Ile Leu
165

<210> 364
<211> 498
<212> DNA
<213> Chlamydia pneumoniae

<400> 364
atgaacatac ccgctcccca agtaccagtc atagatgagc ctgtagtgaa caacacaagt 60
agctatggtc tttcattgaa aagtagttta agaccgatta cttatttgat tttagctatc 120
ttagctatag ccacactgat gtctgttctc tacttttgtg gcatcattag tgttgggacg 180
tttgttttgg gcatgctgat ccctctatcg gtctgctctg ttctttgcgt tgcctattta 240
ttctatcagc aatcttctat agaaaagact aaggtctttt ctataaccag tccttcagta 300
tttttctctg atgaggatct taatttactc ttaggtcgag aagaagattc agtgtctgca 360
attgatgaac ttcttaagaa ctttccagct gatgatttcc gtaggccgaa gatgcttcct 420
tattcaaatt ttctagatga gcagggaagg cctaatgaga gtagggaaga agactctcat 480
acttccaaga tcttataa 498

<210> 365
<211> 433
<212> PRT
<213> Chlamydia pneumoniae

<400> 365
Met Ser Met Thr Ile Val Pro His Ala Leu Phe Lys Asn His Cys Glu
1 5 10 15

Cys His Ser Thr Phe Pro Leu Ser Ser Arg Thr Ile Val Arg Ile Ala
20 25 30

Ile Ala Ser Leu Phe Cys Ile Gly Ala Leu Ala Ala Leu Gly Cys Leu
35 40 45

Ala Pro Pro Val Ser Tyr Ile Val Gly Ser Val Leu Ala Phe Ile Ala
50 55 60

Phe Val Ile Leu Ser Leu Val Ile Leu Ala Leu Ile Phe Gly Glu Lys
65 70 75 80

Lys Leu Pro Pro Thr Pro Arg Ile Ile Pro Asp Arg Phe Thr His Val
85 90 95

Ile Asp Glu Ala Tyr Gly Leu Ser Ile Ser Ala Phe Val Arg Glu Gln
100 105 110

Gln Val Thr Leu Ala Glu Phe Arg Gln Phe Ser Thr Ala Leu Leu Cys
115 120 125

Asn Ile Ser Pro Glu Glu Lys Ile Lys Gln Leu Pro Ser Glu Leu Arg
130 135 140

Ser Lys Val Glu Ser Phe Gly Ile Ser Arg Leu Ala Gly Asp Leu Glu
145 150 155 160

Lys Asn Asn Trp Pro Ile Phe Glu Asp Leu Leu Ser Gln Thr Cys Pro
165 170 175

Leu Tyr Trp Leu Gln Lys Phe Ile Ser Ala Gly Asp Pro Gln Val Cys
180 185 190

Arg Asp Leu Gly Val Pro Arg Glu Cys Tyr Gly Tyr Tyr Trp Leu Gly
195 200 205

Pro Leu Gly Tyr Ser Thr Ala Lys Ala Thr Ile Phe Cys Lys Glu Thr
210 215 220

His His Ile Leu Gln Gln Leu Thr Lys Glu Asp Val Leu Leu Leu Lys
225 230 235 240

Asn Lys Ala Leu Gln Glu Lys Trp Asp Thr Asp Glu Val Lys Ala Ile
245 250 255

Val Glu Arg Ile Tyr Thr Thr Tyr Thr Ala Arg Gly Thr Leu Lys Thr
260 265 270

Glu Ala Gly Gly Leu Thr Lys Glu Thr Ile Ser Lys Glu Leu Leu Leu
275 280 285

Leu Ser Leu His Gly Tyr Ser Phe Asp Gln Leu Gln Leu Ile Thr Gln
290 295 300

Leu Pro Arg Asp Ala Trp Asp Trp Leu Cys Phe Val Asp Asn Ser Thr
305 310 315 320

Ala Tyr Asn Leu Gln Leu Cys Ala Leu Val Gly Ala Leu Ser Ser Gln
325 330 335

```
Asn Leu Leu Asp Glu Ser Ser Ile Asp Phe Asp Val Asn Leu Gly Leu
            340                 345                 350

Tyr Val Ile Gln Asp Leu Lys Glu Ala Val Gln Ala Phe Ser Ala Ser
        355                 360                 365

Asp Glu Pro Lys Lys Glu Leu Gly Lys Phe Leu Leu Arg His Leu Ser
    370                 375                 380

Ser Val Ser Lys Arg Leu Glu Ser Val Leu Arg Gln Gly Leu His Arg
385                 390                 395                 400

Ile Ala Leu Glu His Gly Asn Ala Arg Ala Arg Val Tyr Asp Val Asn
                405                 410                 415

Phe Val Thr Gly Ala Arg Ile His Arg Lys Thr Ser Ile Phe Phe Lys
            420                 425                 430

Asp
```

```
<210>   366
<211>   1302
<212>   DNA
<213>   Chlamydia pneumoniae

<400>   366
atgagcatga cgatcgttcc acatgcttta tttaaaaatc attgcgagtg tcattctacc     60
tttcctttga gttcaaggac tattgtaaga atagccattg ccagcctctt ttgtataggt    120
gcattagcag ctttaggctg tttggctcct cccgtttctt atattgttgg gagtgtttta    180
gcttttattg cctttgtcat tctttcttta gtaattttag ctttgatttt tggagagaag    240
aagcttccac caacaccaag aatcattcct gatagattta ctcacgtgat agatgaagct    300
tatggccttt caatctctgc atttgtaaga gaacagcagg taacattagc cgagtttaga    360
caattttcta ctgccctgtt gtgtaacata tctcctgaag agaaaatcaa acaattgcct    420
tctgaattgc gaagtaaagt agagagtttt ggtattagca ggctcgcagg tgatttagaa    480
aagaataatt ggccaatatt tgaagatctt ttaagccaaa cctgccccgtt atattggctt    540
cagaaattta tatcagcagg agatccacaa gtttgtagag acctaggtgt ccctagagaa    600
tgttatgggt actattggct agggcctttg ggatacagta cagctaaggc tacaattttt    660
tgtaaagaga cgcatcatat tcttcaacaa ttaacgaaag aggacgttct tttattaaaa    720
aacaaggctc ttcaagagaa atgggatact gatgaagtca aagcaattgt agagcgtatc    780
tacactacct atacggcacg aggaactcta aagaccgaag caggggggact tacaaaagag    840
acaatcagta aggaattgct attgttgagc ttgcatggct attcttttga tcagctacag    900
ctgatcactc aacttcctag agatgcttgg gattggctgt gttttgtaga taacagtacc    960
gcatacaacc ttcagctttg tgctcttgta ggagctttgt catcccaaaa tcttcttgac   1020
gaatcttcta tcgattttga tgtaaaccta ggcctgtatg tgattcagga tctaaaagaa   1080
gctgttcaag cattttctgc ttctgatgag ccaaagaaag aactaggtaa attcttgtta   1140
aggcatttga gttcagtttc taagcgatta gagagtgtat taagacaggg tcttcacaga   1200
atagctctag agcatggaaa tgccagagct agggtttatg acgtcaattt tgtaacagga   1260
gctagaattc ataggaagac gagtatcttc tttaaagact aa                       1302

<210>   367
<211>   283
<212>   PRT
<213>   Chlamydia pneumoniae

<400>   367
Met Val Asn Ile Gln Pro Val Tyr Arg Asn Thr Gln Val Asn Tyr Ser
1               5                   10                  15

Gln Ala Thr Gln Phe Ser Val Cys Gln Pro Ala Leu Ser Leu Ile Ile
            20                  25                  30
```

```
Val Ser Val Val Ala Ala Val Leu Ala Ile Val Ala Leu Val Cys Ser
        35                  40                  45

Gln Ser Leu Leu Ser Ile Glu Leu Gly Thr Ala Leu Val Leu Val Ser
    50                  55                  60

Leu Ile Leu Phe Ala Ser Ala Met Phe Met Ile Tyr Lys Met Arg Gln
65                  70                  75                  80

Glu Pro Lys Glu Leu Leu Ile Pro Lys Lys Ile Met Glu Leu Ile Gln
                85                  90                  95

Glu His Tyr Pro Ser Ile Val Val Asp Phe Ile Arg Asp Gln Glu Val
            100                 105                 110

Ser Ile Tyr Glu Ile His His Leu Ile Ser Ile Leu Asn Lys Thr Asn
        115                 120                 125

Val Phe Asp Lys Ala Pro Val Tyr Leu Gln Glu Lys Leu Leu Gln Phe
    130                 135                 140

Gly Ile Glu Lys Phe Lys Asp Val His Pro Ser Lys Leu Pro Asn Phe
145                 150                 155                 160

Glu Glu Ile Leu Leu Gln His Cys Pro Leu His Trp Leu Gly Arg Leu
                165                 170                 175

Val Tyr Pro Met Val Ser Asp Val Thr Pro Gly Thr Tyr Gly Tyr Tyr
            180                 185                 190

Trp Cys Gly Pro Leu Gly Leu Tyr Glu Asn Ala Pro Ser Leu Phe Glu
        195                 200                 205

Arg Arg Ser Leu Leu Leu Leu Lys Lys Ile Ser Phe Gly Glu Phe Ala
    210                 215                 220

Leu Leu Glu Asp Gly Leu Lys Lys Asn Thr Trp Ser Ser Ser Glu Leu
225                 230                 235                 240

Val Gln Ile Arg Gln Asn Leu Phe Thr Arg Tyr Tyr Ala Asp Lys Glu
                245                 250                 255

Glu Val Asp Glu Ala Glu Leu Asn Ala Asp Tyr Glu Gln Phe Asp Ser
            260                 265                 270

Leu Leu His Leu Ile Phe Ser His Lys Leu Ser
        275                 280

<210>   368
<211>   852
<212>   DNA
<213>   Chlamydia pneumoniae

<400>   368
atggttaata tacagcctgt gtataggaat acccaagtca actatagtca ggctacccaa    60
ttttcggtgt gccagccagc gcttagcctg attatcgttt ctgttgttgc tgctgtactc   120
gctattgtag ctttggtatg cagtcaatct cttttatcca tagagttagg aactgctctt   180
gttctagttt ctcttattct ttttgcttct gctatgttta tgatttataa gatgagacaa   240
gaacctaagg agttgctgat ccctaagaaa atcatggaac tcatccaaga acattatcca   300
agtattgttg ttgattttat tagagatcag gaggtttcca tttatgagat acatcacttg   360
```

```
atctctattc ttaataagac gaatgttttc gacaaagcac cagtatattt acaagaaaaa   420
ctcttacagt ttggcattga gaagttcaaa gatgtacatc caagtaagct ccctaatttt   480
gaagaaattc ttctacagca ttgcccattg cattggttgg gacgtctggt atatcccatg   540
gtatcggatg tcactccagg aacctatgga tactattggt gtggtccttt aggactgtac   600
gagaacgctc cctctctttt tgaacgtcga tctcttctat tgttaaagaa aattagcttt   660
ggagagtttg ctcttttaga agatggtctc aagaaaaaca cgtggagttc ttcggaactc   720
gttcaaatca gacaaaacct ttttacaaga tattatgctg ataaagaaga ggtagatgaa   780
gcagagttaa acgctgatta cgaacagttt gattccctcc ttcaccttat tttttctcac   840
aagctctctt ga                                                       852

<210>   369
<211>   339
<212>   PRT
<213>   Chlamydia pneumoniae

<400>   369
Met Ala Thr Ile Ser Pro Ile Ser Leu Thr Val Asp His Pro Leu Val
1               5                   10                  15

Asp Thr Lys Lys Lys Ser Cys Ser Asn Phe Asp Lys Ile Gln Ser Arg
            20                  25                  30

Ile Leu Leu Ile Thr Ala Ile Phe Ala Val Leu Val Thr Ile Gly Thr
        35                  40                  45

Leu Leu Ile Gly Leu Leu Leu Asn Ile Pro Val Ile Tyr Phe Leu Thr
    50                  55                  60

Gly Ile Ser Phe Ile Ala Val Val Leu Ser Asn Phe Ile Leu Tyr Lys
65                  70                  75                  80

Arg Ala Thr Thr Leu Leu Lys Pro Arg Ala Cys Gly Lys His Lys Glu
                85                  90                  95

Ile Lys Pro Lys Arg Val Ser Thr Asn Leu Gln Tyr Ser Ser Ile Ser
            100                 105                 110

Ile Ala Ile Asn Arg Ser Lys Glu Asn Trp Glu His Gln Pro Lys Asp
        115                 120                 125

Leu Gln Asn Leu Pro Ala Pro Ser Ala Leu Leu Thr Asp Asn Pro Tyr
    130                 135                 140

Glu Ile Trp Lys Ala Lys His Ser Leu Phe Ser Leu Val Ser Leu Leu
145                 150                 155                 160

Pro Gly Gly Asn Pro Glu His Leu Leu Ile Ser Ala Ser Glu Asn Leu
                165                 170                 175

Gly Lys Thr Leu Leu Ile Glu Glu Thr Ser Gln Asn Ala Pro Ile Ser
            180                 185                 190

Ser Tyr Val Asp Thr Thr Pro Ser Pro Lys Ser Leu Leu Asn Glu Ala
        195                 200                 205

Ile Gln Glu Thr Arg Val Glu Ile Asn Thr Glu Leu Pro Ala Gly Asp
    210                 215                 220

Ser Gly Glu Arg Leu Tyr Trp Gln Pro Asp Phe Arg Gly Arg Val Phe
225                 230                 235                 240
```

Leu Pro Gln Ile Pro Thr Thr Pro Glu Ala Ile Tyr Gln Tyr Tyr Tyr
245 250 255

Ala Leu Tyr Val Thr Tyr Ile Gln Thr Ala Ile Asn Thr Asn Thr Gln
260 265 270

Ile Ile Gln Ile Pro Leu Tyr Ser Leu Arg Glu His Leu Tyr Ser Arg
275 280 285

Glu Leu Pro Pro Gln Ser Arg Met Gln Gln Ser Leu Ala Met Ile Thr
290 295 300

Ala Val Lys Tyr Met Ala Glu Leu His Pro Glu Tyr Pro Leu Thr Ile
305 310 315 320

Ala Cys Val Glu Arg Ser Leu Ala Gln Leu Pro Gln Glu Ser Ile Glu
325 330 335

Asp Leu Ser

<210> 370
<211> 1020
<212> DNA
<213> Chlamydia pneumoniae

<400> 370
atggctacaa tctcacccat atctttaact gtagatcatc ccctagtaga cactaaaaaa 60
aaatcctgca gcaactttga taagattcag tctcgaattc tattgattac tgcaatcttt 120
gctgtcttag ttactatagg gaccctactt attggtttgc ttttaaatat tcctgttatc 180
tatttcctca caggaatttc atttattgct gttgttctta gcaactttat cctttataaa 240
cgagcaacca ccctcttaaa accgcgtgct tgtggcaaac acaaagaaat aaaaccaaaa 300
agggtctcca ccaacctaca gtattcttct atctctatcg caatcaatcg ttctaaagaa 360
aactgggaac accaacccaa ggacctacag aatctccccg caccctctgc attactcaca 420
gataaccctt acgagatatg gaaagctaaa cattcactgt tttccctagt atccctccta 480
ccgggaggca atccagaaca tctcttaatt tcagcttccg aaaatttagg aaagactctg 540
ttaattgaag aaacctcgca aaatgcgcct atatcctcct acgtagatac cactccctcc 600
ccaaaatcct tgctcaatga ggcaattcag gaaaccaggg tagaaataaa tacagaactc 660
cctgcgggag attcaggaga acgtttatac tggcaacccg atttccgagg ccgcgtcttc 720
ctcccacaaa taccaacaac tcctgaagcc atctaccaat actactatgc actctatgtc 780
acttatatcc agactgcgat caatacgaac acccaaatta tccaaatccc tttatacagc 840
ttgagggagc atctctattc tagagaattg cccccgcaat caagaatgca acaatctttg 900
gctatgatta cagcagtaaa atacatggcc gagctgcacc cagaatatcc gctaactatt 960
gcttgtgttg aaagatcctt agcccaacta cctcaagaaa gtattgagga tctctcttag 1020

<210> 371
<211> 170
<212> PRT
<213> Chlamydia pneumoniae

<400> 371
Met Ser Glu Val Lys Pro Leu Phe Leu Lys Asn Asp Ser Phe Asp Leu
1 5 10 15

Ala Thr Gln Arg Phe Gln Asn Leu Ile Asn Met Leu Gln Glu Gln Ala
20 25 30

Glu Ile Tyr Asn Glu Tyr Glu Glu Lys Asn Ala Arg Val Gln Asn Glu
35 40 45

Ile Lys Glu Gln Lys Asp Phe Val Lys Arg Cys Ile Glu Asp Phe Glu

50 55 60

Ala Arg Gly Leu Gly Val Leu Lys Glu Glu Leu Ala Ser Leu Thr Arg
65 70 75 80

Asp Phe His Asp Lys Ala Lys Ala Glu Thr Ser Met Leu Ile Glu Cys
85 90 95

Pro Cys Ile Gly Phe Tyr Tyr Ser Ile His Gln Glu Glu Gln Arg Gln
100 105 110

Arg Gln Glu Arg Leu Gln Lys Met Ala Glu Arg Tyr Arg Asp Cys Lys
115 120 125

Gln Val Leu Glu Ala Val Gln Val Glu Gln Lys Asp Met Ile Ser Ser
130 135 140

Arg Val Val Val Asp Asp Ser Tyr Phe Glu Glu Glu Lys Glu Glu Gln
145 150 155 160

Lys Val Asp Asn Arg Lys Lys Glu Gln Asp
165 170

<210> 372
<211> 513
<212> DNA
<213> Chlamydia pneumoniae

<400> 372
atgtcagaag tgaagccttt gtttttaaag aatgactctt ttgatttggc aactcagaga 60
ttccagaatc taattaacat gctacaagag caagccgaga tatataacga gtatgaagaa 120
aagaatgcta gggttcagaa tgagattaag gagcaaaagg actttgtgaa aagatgcata 180
gaggactttg aagccagagg actgggggtg ctaaaagaag agcttgcatc tttgacgcgt 240
gatttccatg ataaagcaaa agcagagact tctatgctca ttgaatgtcc ttgtattggt 300
ttttattata gtattcatca ggaggaacaa aggcaaaggc aagaaaggct tcaaaagatg 360
gctgagcgct atagggactg taaacaagtc ttggaggctg tccaggtgga gcaaaaagat 420
atgatatctt ctagagtcgt tgtcgatgac agctactttg aagaagaaaa agaagaacaa 480
aaggtggata acagaaagaa agaacaggac tag 513

<210> 373
<211> 196
<212> PRT
<213> Chlamydia pneumoniae

<400> 373
Val Leu Gln Glu His Phe Phe Leu Ser Glu Asp Val Ile Thr Leu Ala
1 5 10 15

Gln Gln Leu Leu Gly His Lys Leu Ile Thr Thr His Glu Gly Leu Ile
20 25 30

Thr Ser Gly Tyr Ile Val Glu Thr Glu Ala Tyr Arg Gly Pro Asp Asp
35 40 45

Lys Ala Cys His Ala Tyr Asn Tyr Arg Lys Thr Gln Arg Asn Arg Ala
50 55 60

Met Tyr Leu Lys Gly Gly Ser Ala Tyr Leu Tyr Arg Cys Tyr Gly Met
65 70 75 80

His His Leu Leu Asn Val Val Thr Gly Pro Glu Asp Ile Pro His Ala

```
                85                  90                  95

Val Leu Ile Arg Ala Ile Leu Pro Asp Gln Gly Lys Glu Leu Met Ile
            100                 105                 110

Gln Arg Arg Gln Trp Arg Asp Lys Pro Pro His Leu Leu Thr Asn Gly
        115                 120                 125

Pro Gly Lys Val Cys Gln Ala Leu Gly Ile Ser Leu Glu Asn Asn Arg
    130                 135                 140

Gln Arg Leu Asn Thr Pro Ala Leu Tyr Ile Ser Lys Glu Lys Ile Ser
145                 150                 155                 160

Gly Thr Leu Thr Ala Thr Ala Arg Ile Gly Ile Asp Tyr Ala Gln Glu
                165                 170                 175

Tyr Arg Asp Val Pro Trp Arg Phe Leu Leu Ser Pro Glu Asp Ser Gly
            180                 185                 190

Lys Val Leu Ser
        195

<210>   374
<211>   591
<212>   DNA
<213>   Chlamydia pneumoniae

<400>   374
gtgctacaag aacatttttt tctatcggaa gatgtaatta cactagcgca acagctttta    60
ggacataaac tcatcacaac acatgagggt ctgataactt caggttacat tgtagaaacc   120
gaagcgtatc gtggccctga tgacaaagca tgccacgcct acaactacag aaaaactcag   180
aggaacagag cgatgtacct gaaaggaggc tctgcttacc tctaccgttg ctatggcatg   240
catcacctat tgaatgttgt cactggacct gaggacattc cccatgccgt cctgatccgg   300
gccatccttc ctgatcaagg caaagaactt atgatccaac gccgccaatg gagagataaa   360
cccccacacc ttctcaccaa tggacccgga aaagtgtgcc aagctctagg aatctctttg   420
gaaaacaata ggcaacgcct aaatacccca gctctctata tcagcaaaga aaaaatctct   480
gggactctaa cagcaactgc ccggatcggc atcgattatg ctcaagagta tcgtgatgtc   540
ccatggagat ttctcctatc cccagaagat tcgggaaaag ttttatctta a            591

<210>   375
<211>   184
<212>   PRT
<213>   Chlamydia pneumoniae

<400>   375
Met Val Glu Thr Val Leu His Asn Phe Gln Arg Tyr Leu Ser Lys Tyr
1               5                   10                  15

Leu Tyr Arg Val Phe Arg Phe Pro Cys Arg Lys Lys Thr Phe Leu Ser
            20                  25                  30

Ser His Arg Val Leu Ala Arg Pro Ser Phe Pro Val Asp Tyr Cys Pro
        35                  40                  45

Gly Lys Ile Tyr Asp Leu Gln Glu Ile Tyr Glu Glu Leu Asn Ala Gln
    50                  55                  60

Leu Phe Gln Gly Ala Leu Arg Leu Gln Ile Gly Trp Phe Gly Arg Lys
65                  70                  75                  80
```

Ala Thr Arg Lys Gly Lys Ser Val Val Leu Gly Leu Phe His Glu Asn
85 90 95

Glu Gln Leu Ile Arg Ile His Arg Ser Leu Asp Arg Gln Glu Ile Pro
100 105 110

Arg Phe Phe Met Glu Tyr Leu Val Tyr His Glu Met Val His Ser Val
115 120 125

Val Pro Arg Glu Tyr Ser Leu Ser Gly Arg Ser Ile Phe His Gly Lys
130 135 140

Lys Phe Lys Glu Tyr Glu Gln Arg Phe Pro Leu Tyr Asp Arg Ala Val
145 150 155 160

Ala Trp Glu Lys Ala Asn Ala Tyr Leu Leu Arg Gly Tyr Lys Lys Arg
165 170 175

Val Gly Gly Gly Tyr Gly Arg Ala
180

<210> 376
<211> 555
<212> DNA
<213> Chlamydia pneumoniae

<400> 376
atggttgaaa cagtacttca taatttccaa cgttatctga gcaagtatct ctatagggta 60
tttcgcttcc catgtcgtaa aaagacgttc ctatcttcgc acagggttct tgctcgtcct 120
tcattcccag tagactactg tccgggaaag atctatgatt tgcaggagat ctatgaggaa 180
ttgaatgcgc agttatttca aggtgcactg cgtttacaga ttggttggtt cggaaggaaa 240
gctaccagaa aaggcaagag tgttgtcttg ggattgtttc atgaaaatga acagttaatt 300
cgaattcatc gttctttaga tcggcaggaa atcccaagat tttttatgga atatcttgtg 360
tatcatgaaa tggttcatag tgtagtccct agagagtatt ctctatcggg gcgttcgatt 420
tttcatggta aaaagtttaa agaatacgaa caacgtttcc ccttgtatga tcgtgctgtt 480
gcttgggaaa aggcaaacgc ttatttattg cgagggtata aaaaaagagt aggtggagga 540
tatggcaggg catag 555

<210> 377
<211> 707
<212> PRT
<213> Chlamydia pneumoniae

<400> 377
Met Tyr Lys Arg Cys Val Leu Asp Lys Ile Leu Lys Gly Ile Val Ala
1 5 10 15

Gly Ser Leu Ile Leu Leu Tyr Trp Ser Ser Asp Leu Leu Glu Arg Asp
20 25 30

Ile Lys Ser Ile Lys Gly Asn Val Arg Asp Ile Gln Glu Asp Ile Arg
35 40 45

Glu Ile Ser Arg Val Val Lys Gln Gln Gln Thr Ser Gln Ala Ile Pro
50 55 60

Ala Ala Pro Gly Val Met Leu Ala Pro Lys Leu Val Arg Asp Glu Ala
65 70 75 80

Phe Ala Leu Leu Phe Gly Asp Pro Ser Tyr Pro Asn Leu Leu Ser Leu
85 90 95

```
Asp Pro Tyr Lys Gln Gln Thr Leu Pro Glu Leu Leu Gly Thr Asn Phe
            100                 105                 110

His Pro His Gly Ile Leu Arg Thr Ala His Val Gly Lys Pro Glu Asn
        115                 120                 125

Leu Ser Pro Phe Asn Gly Phe Asp Tyr Val Val Gly Phe Tyr Asp Leu
    130                 135                 140

Cys Ile Pro Ser Leu Ala Ser Pro His Val Gly Lys Tyr Glu Glu Phe
145                 150                 155                 160

Ser Pro Asp Leu Ala Val Lys Ile Glu Glu His Leu Val Glu Asp Gly
                165                 170                 175

Ser Gly Asp Lys Glu Phe His Ile Tyr Leu Arg Pro Asn Val Phe Trp
            180                 185                 190

Arg Pro Ile Asp Pro Lys Ala Leu Pro Lys His Val Gln Leu Asp Glu
        195                 200                 205

Val Phe Gln Arg Pro His Pro Val Thr Ala His Asp Ile Lys Phe Phe
    210                 215                 220

Tyr Asp Ala Val Met Asn Pro Tyr Val Ala Thr Met Arg Ala Val Ala
225                 230                 235                 240

Leu Arg Ser Cys Tyr Glu Asp Val Val Ser Val Ser Val Glu Asn Asp
                245                 250                 255

Leu Lys Leu Val Val Arg Trp Lys Ala His Thr Val Ile Asn Glu Glu
            260                 265                 270

Gly Lys Glu Glu Arg Lys Val Leu Tyr Ser Ala Phe Ser Asn Thr Leu
        275                 280                 285

Ser Leu Gln Pro Leu Pro Arg Phe Val Tyr Gln Tyr Phe Ala Asn Gly
    290                 295                 300

Glu Lys Ile Ile Glu Asp Glu Asn Ile Asp Thr Tyr Arg Thr Asn Ser
305                 310                 315                 320

Ile Trp Ala Gln Asn Phe Thr Met His Trp Ala Asn Asn Tyr Ile Val
                325                 330                 335

Ser Cys Gly Ala Tyr Tyr Phe Ala Gly Met Asp Asp Glu Lys Ile Val
            340                 345                 350

Phe Ser Arg Asn Pro Asp Phe Tyr Asp Pro Leu Ala Ala Leu Ile Asp
        355                 360                 365

Lys Arg Phe Val Tyr Phe Lys Glu Ser Thr Asp Ser Leu Phe Gln Asp
    370                 375                 380

Phe Lys Thr Gly Lys Ile Asp Ile Ser Tyr Leu Pro Pro Asn Gln Arg
385                 390                 395                 400

Asp Asn Phe Tyr Ser Phe Met Lys Ser Ser Ala Tyr Asn Lys Gln Val
                405                 410                 415
```

```
Ala Lys Gly Gly Ala Val Arg Glu Thr Val Ser Ala Asp Arg Ala Tyr
            420                 425                 430

Thr Tyr Ile Gly Trp Asn Cys Phe Ser Leu Phe Phe Gln Ser Arg Gln
        435                 440                 445

Val Arg Cys Ala Met Asn Met Ala Ile Asp Arg Glu Arg Ile Ile Glu
    450                 455                 460

Gln Cys Leu Asp Gly Gln Gly Tyr Thr Ile Ser Gly Pro Phe Ala Ser
465                 470                 475                 480

Ser Ser Pro Ser Tyr Asn Lys Gln Ile Glu Gly Trp His Tyr Ser Pro
                485                 490                 495

Glu Glu Ala Ala Arg Leu Leu Glu Glu Glu Gly Trp Ile Asp Thr Asp
            500                 505                 510

Gly Asp Gly Ile Arg Glu Lys Val Ile Asp Gly Val Ile Val Pro Phe
        515                 520                 525

Arg Phe Arg Leu Cys Tyr Tyr Val Lys Ser Val Thr Ala His Thr Ile
    530                 535                 540

Ala Asp Tyr Val Ala Thr Ala Cys Lys Glu Ile Gly Ile Glu Cys Ser
545                 550                 555                 560

Leu Leu Gly Leu Asp Met Ala Asp Leu Ser Gln Ala Phe Asp Glu Lys
                565                 570                 575

Asn Phe Asp Ala Leu Leu Met Gly Trp Cys Leu Gly Ile Pro Pro Glu
            580                 585                 590

Asp Pro Arg Ala Leu Trp His Ser Glu Gly Ala Met Glu Lys Gly Ser
        595                 600                 605

Ala Asn Val Val Gly Phe His Asn Glu Glu Ala Asp Lys Ile Ile Asp
    610                 615                 620

Arg Leu Ser Tyr Glu Tyr Asp Leu Lys Glu Arg Asn Arg Leu Tyr His
625                 630                 635                 640

Arg Phe His Glu Ile Ile His Glu Glu Ala Pro Tyr Ala Phe Leu Phe
                645                 650                 655

Ser Arg His Cys Ser Leu Leu Tyr Lys Asp Tyr Val Lys Asn Ile Phe
            660                 665                 670

Val Pro Thr His Arg Thr Asp Leu Ile Pro Glu Ala Gln Asp Glu Thr
        675                 680                 685

Val Asn Val Thr Met Val Trp Leu Glu Lys Lys Glu Asp Pro Cys Leu
    690                 695                 700

Ser Thr Ser
705

<210>   378
<211>   2124
<212>   DNA
<213>   Chlamydia pneumoniae
```

<400> 378
atgtataaaa gatgtgtgct agataaaatt ttaaagggga ttgtcgccgg ttctttaatt 60
ttgttatact ggtcctcaga cctacttgaa agagacatta agtcgataaa aggtaacgta 120
agagatattc aagaagacat tcgtgaaatc tcacgcgtag tgaaacaaca gcagacatca 180
caagctatcc ctgcggcacc tggggtgatg ctcgctccta agctcgtcag agacgaagct 240
tttgctctac tctttggaga tcctagttat cctaatttac tttccctaga cccctataaa 300
cagcagactc ttcctgaact tctaggaaca aatttccacc ctcatggtat cctacgcact 360
gcccatgtcg gaaaacccga aaatctgagc ccttttaatg gctttgatta tgtcgtgggc 420
ttttacgatc tctgtattcc tagtttagct tctccccacg tagggaaata cgaagaattt 480
tctccagatc tcgctgtgaa aatagaagaa catcttgttg aagatggttc tggggataaa 540
gagtttcaca tctatctgag gccgaatgtt ttttggcgtc ctatagatcc taaggccctt 600
ccaaaacacg ttcagttaga cgaagtattt caacgtcctc atcctgtgac agctcatgat 660
attaagtttt tctacgacgc tgttatgaac ccttatgtag caaccatgcg agcagtggct 720
ctgcgctctt gttatgaaga tgtggtttct gtctcagtag aaaacgattt aaaattagta 780
gtcagatgga aagcacacac ggtaatcaat gaagaaggaa aggaagagcg caaagtgctc 840
tactctgcat tttctaatac cttaagcttg cagcccctcc ctagatttgt atatcagtat 900
tttgctaacg ggaaaaaaat cattgaagat gagaatatcg atacctaccg aaccaattcc 960
atttgggcgc aaaacttcac tatgcattgg gcaaacaact atattgtaag ttgtggagcc 1020
tactactttg cagggatgga tgatgagaaa atcgtgtttt ctagaaatcc tgacttctat 1080
gatcctcttg cggctcttat tgacaagcgt ttcgtctatt ttaaggaaag cacagactcc 1140
ctattccaag attttaagac agggaaaata gacatctctt accttccacc caaccaaaga 1200
gataatttct atagttttat gaaaagctcc gcttataaca aacaggtagc taagggagga 1260
gccgtccgtg aaacagtctc agcagatcga gcatatacgt acataggatg gaattgcttt 1320
tcattatttt tccaaagccg acaggtgcgc tgtgctatga acatggcaat cgatagagag 1380
aggattatcg aacagtgctt ggatggccaa ggctatacga ttagtgggcc ttttgcttcg 1440
agttctcctt cttataataa acagatcgaa gggtggcatt attctccaga agaagcagct 1500
cgtctcctgg aagaagaggg atggatagat accgatggcg atggaatccg agaaaaagtt 1560
atcgatggtg tgattgtccc gttccgtttc cgttttatgct attatgtaaa gagtgtcacc 1620
gctcatacca ttgcagatta cgtagctact gcttgtaagg aaatcggaat cgagtgtagc 1680
cttctaggac tagatatggc cgatctttcg caagcttttg atgaaaagaa tttcgatgct 1740
cttttaatgg gatggtgttt aggaattcct cctgaggatc ctagggcttt atggcattct 1800
gaaggggcta tggaaaaggg ttcagcgaat gttgtaggtt tccataatga agaagctgat 1860
aaaatcatag acagactcag ctacgaatac gatctgaaag aacgtaatcg cctgtaccac 1920
cgtttccatg aaattattca tgaggaagct ccttatgctt tcttgttctc acgacattgt 1980
tccttacttt ataaggatta tgtaaaaaat attttcgtac ctacacatag aacagattta 2040
attcctgaag ctcaggatga gactgtcaac gtaactatgg tatggcttga gaagaaggag 2100
gatccgtgct taagtacatc ctaa 2124

<210> 379
<211> 217
<212> PRT
<213> Chlamydia pneumoniae

<400> 379
Met Lys Arg Val Ile Tyr Lys Thr Ile Phe Cys Gly Leu Thr Leu Leu
1 5 10 15

Thr Ser Leu Ser Ser Cys Ser Leu Asp Pro Lys Gly Tyr Asn Leu Glu
20 25 30

Thr Lys Asn Ser Arg Asp Leu Asn Gln Glu Ser Val Ile Leu Lys Glu
35 40 45

Asn Arg Glu Thr Pro Ser Leu Val Lys Arg Leu Ser Arg Arg Ser Arg
50 55 60

Arg Leu Phe Ala Arg Arg Asp Gln Thr Gln Lys Asp Thr Leu Gln Val
65 70 75 80

Gln Ala Asn Phe Lys Thr Tyr Ala Glu Lys Ile Ser Glu Gln Asp Glu

```
            85                  90                  95

Arg Asp Leu Ser Phe Val Val Ser Ser Ala Ala Glu Lys Ser Ser Ile
            100                 105                 110

Ser Leu Ala Leu Ser Gln Gly Glu Ile Lys Asp Ala Leu Tyr Arg Ile
        115                 120                 125

Arg Glu Val His Pro Leu Ala Leu Ile Glu Ala Leu Ala Glu Asn Pro
    130                 135                 140

Ala Leu Ile Glu Gly Met Lys Lys Met Gln Gly Arg Asp Trp Ile Trp
145                 150                 155                 160

Asn Leu Phe Leu Thr Gln Leu Ser Glu Val Phe Ser Gln Ala Trp Ser
                165                 170                 175

Gln Gly Val Ile Ser Glu Glu Asp Ile Ala Ala Phe Ala Ser Thr Leu
            180                 185                 190

Gly Leu Asp Ser Gly Thr Val Ala Ser Ile Val Gln Gly Glu Arg Trp
        195                 200                 205

Pro Glu Leu Val Asp Ile Val Ile Thr
    210                 215
```

<210> 380
<211> 654
<212> DNA
<213> Chlamydia pneumoniae

<400> 380
```
atgaaaagag tcatttataa aaccatattt tgcgggttaa ctttacttac aagtttgagt    60
agttgttccc tggatcctaa aggatataac ctagagacaa aaaactcgag ggacttaaat   120
caagagtctg ttatactgaa ggaaaaccgt gaaacacctt ctcttgttaa gagactctct   180
cgtcgttctc gaagactctt cgctcgacgt gatcaaactc agaaggatac gctgcaagtg   240
caagctaact ttaagaccta cgcagaaaag atttcagagc aggacgaaag agacctttct   300
ttcgttgtct cgtctgctgc agaaaagtct tcaatttcgt tagctttgtc tcagggtgaa   360
attaaggatg ctttgtaccg tatccgagaa gtccaccctc tagctttaat agaagctctt   420
gctgaaaacc ctgccttgat agaagggatg aaaaagatgc aaggccgtga ttggatttgg   480
aatcttttct taacacaatt aagtgaagta ttttctcaag cttggtctca aggggttatc   540
tctgaagaag atatcgccgc atttgcctcc accttaggtt tggactccgg gaccgttgcg   600
tccattgtcc aaggggaaag gtggcccgag cttgtggata tagtgataac ttaa          654
```

<210> 381
<211> 33
<212> DNA
<213> Artificial Sequence

<220>
<223> PCR primer

<400> 381
```
gcgtcccggg tcatatgaag tcttctttcc cca                                  33
```

<210> 382
<211> 27
<212> DNA
<213> Artificial Sequence

<220>

<223> PCR primer

<400> 382
gcgtctcgag atgaaagagt ttttgcg 27

<210> 383
<211> 34
<212> DNA
<213> Artificial Sequence

<220>
<223> PCR primer

<400> 383
gcgtcccggg tcatatgtca gctctgtttt ctga 34

<210> 384
<211> 27
<212> DNA
<213> Artificial Sequence

<220>
<223> PCR primer

<400> 384
gcgtctcgag gaattggtat tttgctc 27

<210> 385
<211> 33
<212> DNA
<213> Artificial Sequence

<220>
<223> PCR primer

<400> 385
gcgtcccggg tcatatggcc gatctcacat tag 33

<210> 386
<211> 28
<212> DNA
<213> Artificial Sequence

<220>
<223> PCR primer

<400> 386
gcgtctcgag gtccaagtta aggtagca 28

<210> 387
<211> 32
<212> DNA
<213> Artificial Sequence

<220>
<223> PCR primer

<400> 387
gcgtccgggt catatgggta tcaagggaac tg 32

<210> 388

<211> 25
<212> DNA
<213> Artificial Sequence

<220>
<223> PCR primer

<400> 388
gcgtctcgag aaatccgaat cttcc 25

<210> 389
<211> 37
<212> DNA
<213> Artificial Sequence

<220>
<223> PCR primer

<400> 389
gcgtcccggg tcatatgcaa gactctcaag actatag 37

<210> 390
<211> 26
<212> DNA
<213> Artificial Sequence

<220>
<223> PCR primer

<400> 390
gcgtctcgag aaatcggtat ttaccc 26

<210> 391
<211> 36
<212> DNA
<213> Artificial Sequence

<220>
<223> PCR primer

<400> 391
gcgtcccggg tgctagcact acgatttctt taaccc 36

<210> 392
<211> 27
<212> DNA
<213> Artificial Sequence

<220>
<223> PCR primer

<400> 392
gcgtctcgag aaaacgaaat ttgcttc 27

<210> 393
<211> 40
<212> DNA
<213> Artificial Sequence

<220>
<223> PCR primer

<400> 393
gcgtcccggg tcatatgttt aaactgctaa aaaatctatt 40

<210> 394
<211> 28
<212> DNA
<213> Artificial Sequence

<220>
<223> PCR primer

<400> 394
gcgtctcgag atgaaagaag agtcctcg 28

<210> 395
<211> 36
<212> DNA
<213> Artificial Sequence

<220>
<223> PCR primer

<400> 395
gcgtcccggg tcatatgtca tctcctgtaa ataaca 36

<210> 396
<211> 26
<212> DNA
<213> Artificial Sequence

<220>
<223> PCR primer

<400> 396
gcgtctcgag ctgaccatct cctgtt 26

<210> 397
<211> 32
<212> DNA
<213> Artificial Sequence

<220>
<223> PCR primer

<400> 397
gcgtcccggg tcatatgtgc aaggagtcca gt 32

<210> 398
<211> 28
<212> DNA
<213> Artificial Sequence

<220>
<223> PCR primer

<400> 398
gcgtctcgag attttcctta gcataacg 28

<210> 399
<211> 32

<212> DNA
<213> Artificial Sequence

<220>
<223> PCR primer

<400> 399
gcgtcccggg tcatatgtgt tccccatccc aa 32

<210> 400
<211> 35
<212> DNA
<213> Artificial Sequence

<220>
<223> PCR primer

<400> 400
gcgtctcgag tagtttttct ataaaacgaa agtct 35

<210> 401
<211> 34
<212> DNA
<213> Artificial Sequence

<220>
<223> PCR primer

<400> 401
gcgtcccggg tcatatgtgc tcctcctact cttc 34

<210> 402
<211> 30
<212> DNA
<213> Artificial Sequence

<220>
<223> PCR primer

<400> 402
gcgtctcgag ggggaaatag gtatatttga 30

<210> 403
<211> 32
<212> DNA
<213> Artificial Sequence

<220>
<223> PCR primer

<400> 403
gcgtcccggg tcatatgagc tgctcaaagc aa 32

<210> 404
<211> 33
<212> DNA
<213> Artificial Sequence

<220>
<223> PCR primer

<400> 404
gcgtctcgag acttaagata tcgatatttt tga 33

<210> 405
<211> 33
<212> DNA
<213> Artificial Sequence

<220>
<223> PCR primer

<400> 405
gcgtcccggg tcatatgtgc cataaggaag atg 33

<210> 406
<211> 28
<212> DNA
<213> Artificial Sequence

<220>
<223> PCR primer

<400> 406
gcgtctcgag accattgtct tgagtcat 28

<210> 407
<211> 32
<212> DNA
<213> Artificial Sequence

<220>
<223> PCR primer

<400> 407
gcgtcccggg tcatatgacc tcaccgatcc cc 32

<210> 408
<211> 26
<212> DNA
<213> Artificial Sequence

<220>
<223> PCR primer

<400> 408
gcgtctcgag agaagccggt agaggc 26

<210> 409
<211> 37
<212> DNA
<213> Artificial Sequence

<220>
<223> PCR primer

<400> 409
gcgtcccggg tcatatgact gaaaaagtta aagaagg 37

<210> 410
<211> 25
<212> DNA

<213> Artificial Sequence

<220>
<223> PCR primer

<400> 410
gcgtctcgag gaacatgccc cctaa 25

<210> 411
<211> 34
<212> DNA
<213> Artificial Sequence

<220>
<223> PCR primer

<400> 411
gcgtcccggg tcatatgcta catccactaa tggc 34

<210> 412
<211> 28
<212> DNA
<213> Artificial Sequence

<220>
<223> PCR primer

<400> 412
gcgtctcgag gaaagaataa cgagttcc 28

<210> 413
<211> 34
<212> DNA
<213> Artificial Sequence

<220>
<223> PCR primer

<400> 413
gcgtcccggg tcatatggca gatgcttctt tatc 34

<210> 414
<211> 27
<212> DNA
<213> Artificial Sequence

<220>
<223> PCR primer

<400> 414
gcgtctcgag gaatgagtat cttagcc 27

<210> 415
<211> 35
<212> DNA
<213> Artificial Sequence

<220>
<223> PCR primer

<400> 415

gcgtcccggg tcatatggct gttgttgaaa tcaat 35

<210> 416
<211> 35
<212> DNA
<213> Artificial Sequence

<220>
<223> PCR primer

<400> 416
gcgtccatgg cggccgcgaa ctggaactta cctcc 35

<210> 417
<211> 36
<212> DNA
<213> Artificial Sequence

<220>
<223> PCR primer

<400> 417
gcgtcccggg tgctagcgta gaagttatca tgcctt 36

<210> 418
<211> 34
<212> DNA
<213> Artificial Sequence

<220>
<223> PCR primer

<400> 418
gcgtccatgg cggccgcaaa tcgtaatttg cttc 34

<210> 419
<211> 33
<212> DNA
<213> Artificial Sequence

<220>
<223> PCR primer

<400> 419
gcgtggatcc catatggaga ctagactcgg agg 33

<210> 420
<211> 28
<212> DNA
<213> Artificial Sequence

<220>
<223> PCR primer

<400> 420
gcgtctcgag aaatgtggat tttagtcc 28

<210> 421
<211> 34
<212> DNA
<213> Artificial Sequence

<220>
<223> PCR primer

<400> 421
gcgtcccggg tgctagcaat gaaggtctcc aact 34

<210> 422
<211> 28
<212> DNA
<213> Artificial Sequence

<220>
<223> PCR primer

<400> 422
gcgtctcgag aaatctcatt ctactcgc 28

<210> 423
<211> 30
<212> DNA
<213> Artificial Sequence

<220>
<223> PCR primer

<400> 423
gcgtgaattc atatgttcgg gatgactcct 30

<210> 424
<211> 30
<212> DNA
<213> Artificial Sequence

<220>
<223> PCR primer

<400> 424
gcgtctcgag gaattttaag gtacttcctg 30

<210> 425
<211> 36
<212> DNA
<213> Artificial Sequence

<220>
<223> PCR primer

<400> 425
gcgtcccggg tgctagcact ccctactctc atagag 36

<210> 426
<211> 27
<212> DNA
<213> Artificial Sequence

<220>
<223> PCR primer

<400> 426
gcgtctcgag aaacttaaag gtcgttc 27

<210> 427
<211> 35
<212> DNA
<213> Artificial Sequence

<220>
<223> PCR primer

<400> 427
gcgtcccggg tcatatgata aaacaaatag gccgt 35

<210> 428
<211> 28
<212> DNA
<213> Artificial Sequence

<220>
<223> PCR primer

<400> 428
gcgtctcgag ttcgtaagca acttcaga 28

<210> 429
<211> 33
<212> DNA
<213> Artificial Sequence

<220>
<223> PCR primer

<400> 429
gcgtcccggg tcatatgaag cagatgcgtc ttt 33

<210> 430
<211> 34
<212> DNA
<213> Artificial Sequence

<220>
<223> PCR primer

<400> 430
gcgtccatgg cggccgcgaa actaagggag aggc 34

<210> 431
<211> 32
<212> DNA
<213> Artificial Sequence

<220>
<223> PCR primer

<400> 431
gcgtcccggg tcatatggat cccgcgtctg tt 32

<210> 432
<211> 34
<212> DNA
<213> Artificial Sequence

<220>
<223> PCR primer

<400> 432
gcgtccatgg cggccgcgaa tacaaaccgg atcc 34

<210> 433
<211> 39
<212> DNA
<213> Artificial Sequence

<220>
<223> PCR primer

<400> 433
gcgtcccggg tcatatgcat aaagtaatag ttttcattt 39

<210> 434
<211> 29
<212> DNA
<213> Artificial Sequence

<220>
<223> PCR primer

<400> 434
gcgtctcgag taaactagaa aaagtcgtc 29

<210> 435
<211> 35
<212> DNA
<213> Artificial Sequence

<220>
<223> PCR primer

<400> 435
gcgtcccggg tcatatgtca tcaaatctac atccc 35

<210> 436
<211> 27
<212> DNA
<213> Artificial Sequence

<220>
<223> PCR primer

<400> 436
gcgtctcgag aacgcgagct attttac 27

<210> 437
<211> 32
<212> DNA
<213> Artificial Sequence

<220>
<223> PCR primer

<400> 437
gcgtcccggg tgctagcagc gggggtatag ag 32

<210> 438
<211> 28
<212> DNA
<213> Artificial Sequence

<220>
<223> PCR primer

<400> 438
gcgtctcgag atacacgtgg gtattttc 28

<210> 439
<211> 32
<212> DNA
<213> Artificial Sequence

<220>
<223> PCR primer

<400> 439
gcgtcccggg tcatatgtgc cgcattgtag at 32

<210> 440
<211> 25
<212> DNA
<213> Artificial Sequence

<220>
<223> PCR primer

<400> 440
gcgtctcgag ctgtttgcat ctgcc 25

<210> 441
<211> 34
<212> DNA
<213> Artificial Sequence

<220>
<223> PCR primer

<400> 441
gcgtcccggg tgctagctca atagctattg caag 34

<210> 442
<211> 27
<212> DNA
<213> Artificial Sequence

<220>
<223> PCR primer

<400> 442
gcgtctcgag ttatcgaaat gtctttg 27

<210> 443
<211> 32
<212> DNA
<213> Artificial Sequence

<220>

<223> PCR primer

<400> 443
gcgtcccggg tcatatggca acacccgctc aa 32

<210> 444
<211> 36
<212> DNA
<213> Artificial Sequence

<220>
<223> PCR primer

<400> 444
gcgtccatgg cggccgctcc ttgaaattgc tcttgc 36

<210> 445
<211> 37
<212> DNA
<213> Artificial Sequence

<220>
<223> PCR primer

<400> 445
gcgtcccggg tcatatgtat aaaagatgtg tgctaga 37

<210> 446
<211> 27
<212> DNA
<213> Artificial Sequence

<220>
<223> PCR primer

<400> 446
gcgtctcgag ggatgtactt aagcacg 27

<210> 447
<211> 38
<212> DNA
<213> Artificial Sequence

<220>
<223> PCR primer

<400> 447
gcgtcccggg tcatatgaag ataaaatttt cttggaag 38

<210> 448
<211> 25
<212> DNA
<213> Artificial Sequence

<220>
<223> PCR primer

<400> 448
gcgtaagctt gggaagacga taccg 25

<210> 449

<211> 37
<212> DNA
<213> Artificial Sequence

<220>
<223> PCR primer

<400> 449
gcgtcccggg tcatatgctc tcggatcaat atatagg 37

<210> 450
<211> 28
<212> DNA
<213> Artificial Sequence

<220>
<223> PCR primer

<400> 450
gcgtctcgag tcgaatttct tttttagc 28

<210> 451
<211> 37
<212> DNA
<213> Artificial Sequence

<220>
<223> PCR primer

<400> 451
gcgtcccggg tcatatgaaa aaacaggtat atcaatg 37

<210> 452
<211> 28
<212> DNA
<213> Artificial Sequence

<220>
<223> PCR primer

<400> 452
gcgtaagctt aaacgctgaa attatacc 28

<210> 453
<211> 32
<212> DNA
<213> Artificial Sequence

<220>
<223> PCR primer

<400> 453
gcgtcccggg tcatatgtgt agcctttccc ct 32

<210> 454
<211> 26
<212> DNA
<213> Artificial Sequence

<220>
<223> PCR primer

<400> 454
gcgtctcgag gcgtgcatga atctta 26

<210> 455
<211> 37
<212> DNA
<213> Artificial Sequence

<220>
<223> PCR primer

<400> 455
gcgtcccggg tcatatggaa gaattagaag ttgttgt 37

<210> 456
<211> 29
<212> DNA
<213> Artificial Sequence

<220>
<223> PCR primer

<400> 456
gcgtctcgag tagtgttctc tttatcggt 29

<210> 457
<211> 33
<212> DNA
<213> Artificial Sequence

<220>
<223> PCR primer

<400> 457
gcgtcccggg tcatatgcta ggggctggaa acc 33

<210> 458
<211> 27
<212> DNA
<213> Artificial Sequence

<220>
<223> PCR primer

<400> 458
gcgtaagctt aaactgcaga cctgacg 27

<210> 459
<211> 38
<212> DNA
<213> Artificial Sequence

<220>
<223> PCR primer

<400> 459
gcgtcccggg tcatatgact gctgttctta ttcttaca 38

<210> 460
<211> 25

<212> DNA
<213> Artificial Sequence

<220>
<223> PCR primer

<400> 460
gcgtctcgag atctgaaagc ggagg 25

<210> 461
<211> 32
<212> DNA
<213> Artificial Sequence

<220>
<223> PCR primer

<400> 461
gcgtcccggg tcatatgatc ccatccccta cc 32

<210> 462
<211> 28
<212> DNA
<213> Artificial Sequence

<220>
<223> PCR primer

<400> 462
gcgtctcgag atcaggttgc tgagactt 28

<210> 463
<211> 35
<212> DNA
<213> Artificial Sequence

<220>
<223> PCR primer

<400> 463
gcgtcccggg tcatatgaat ctttcaaaca ggtct 35

<210> 464
<211> 31
<212> DNA
<213> Artificial Sequence

<220>
<223> PCR primer

<400> 464
gcgtctcgag atttttttcta gagagactct c 31

<210> 465
<211> 28
<212> DNA
<213> Artificial Sequence

<220>
<223> PCR primer

<400> 465
gtgcgtcata tggcaaccac tccactaa 28

<210> 466
<211> 31
<212> DNA
<213> Artificial Sequence

<220>
<223> PCR primer

<400> 466
actcgctagc ggccgctaat gaggtcccca g 31

<210> 467
<211> 30
<212> DNA
<213> Artificial Sequence

<220>
<223> PCR primer

<400> 467
gtgcgtcata tgaatttatt aggagctgct 30

<210> 468
<211> 34
<212> DNA
<213> Artificial Sequence

<220>
<223> PCR primer

<400> 468
actcgctagc ggccgcaaat ttgattttgc tacc 34

<210> 469
<211> 30
<212> DNA
<213> Artificial Sequence

<220>
<223> PCR primer

<400> 469
gtgcgtcata tggcagcaca agttgtatat 30

<210> 470
<211> 32
<212> DNA
<213> Artificial Sequence

<220>
<223> PCR primer

<400> 470
actcgctagc ggccgctggc gtagaagtga tc 32

<210> 471
<211> 27
<212> DNA

<213> Artificial Sequence

<220>
<223> PCR primer

<400> 471
gtgcgtcata tgttgcctgt agggaac 27

<210> 472
<211> 34
<212> DNA
<213> Artificial Sequence

<220>
<223> PCR primer

<400> 472
actcgctagc ggccgcgaat ctgaactgac caga 34

<210> 473
<211> 30
<212> DNA
<213> Artificial Sequence

<220>
<223> PCR primer

<400> 473
gtgcgtcata tggttaatcc tattggtcca 30

<210> 474
<211> 36
<212> DNA
<213> Artificial Sequence

<220>
<223> PCR primer

<400> 474
actcgctagc ggccgcttgg agataaccag aatata 36

<210> 475
<211> 32
<212> DNA
<213> Artificial Sequence

<220>
<223> PCR primer

<400> 475
gtgcgtcata tgttacacag ctcagaacta ga 32

<210> 476
<211> 35
<212> DNA
<213> Artificial Sequence

<220>
<223> PCR primer

<400> 476

actcgctagc ggccgcgaaa ataatacgga tacca 35

<210> 477
<211> 30
<212> DNA
<213> Artificial Sequence

<220>
<223> PCR primer

<400> 477
gtgcgtcata tggcaactgc tgaaaatata 30

<210> 478
<211> 26
<212> DNA
<213> Artificial Sequence

<220>
<223> PCR primer

<400> 478
gcgtctcgag gaattggaac ttaccc 26

<210> 479
<211> 33
<212> DNA
<213> Artificial Sequence

<220>
<223> PCR primer

<400> 479
gtgcgtgcta gcatttttta tgacaaactc tat 33

<210> 480
<211> 27
<212> DNA
<213> Artificial Sequence

<220>
<223> PCR primer

<400> 480
gcgtctcgag aaatgtgcaa tgactct 27

<210> 481
<211> 30
<212> DNA
<213> Artificial Sequence

<220>
<223> PCR primer

<400> 481
gtgcgtcata tgttgactca tcaagaggct 30

<210> 482
<211> 29
<212> DNA
<213> Artificial Sequence

<220>
<223> PCR primer

<400> 482
gcgtctcgag gaagggaggt tttttaggt 29

<210> 483
<211> 28
<212> DNA
<213> Artificial Sequence

<220>
<223> PCR primer

<400> 483
gtgcgtcata tgacatatct ggaagctc 28

<210> 484
<211> 32
<212> DNA
<213> Artificial Sequence

<220>
<223> PCR primer

<400> 484
actcgctagc ggccgcctcc acaattttta tg 32

<210> 485
<211> 36
<212> DNA
<213> Artificial Sequence

<220>
<223> PCR primer

<400> 485
gtgcgtcata tgccctttga tattacttat tataca 36

<210> 486
<211> 25
<212> DNA
<213> Artificial Sequence

<220>
<223> PCR primer

<400> 486
gcgtctcgag tcgtttccaa atcca 25

<210> 487
<211> 34
<212> DNA
<213> Artificial Sequence

<220>
<223> PCR primer

<400> 487
gtgcgtcata tgaaacaaca ctattctcta aata 34

<210> 488
<211> 27
<212> DNA
<213> Artificial Sequence

<220>
<223> PCR primer

<400> 488
gcgtctcgag tttcttgtgg tttttct 27

<210> 489
<211> 27
<212> DNA
<213> Artificial Sequence

<220>
<223> PCR primer

<400> 489
gtgcgtcata tgcgagaggt gcctaag 27

<210> 490
<211> 32
<212> DNA
<213> Artificial Sequence

<220>
<223> PCR primer

<400> 490
actcgctagc ggccgctctc ctagacagcc tt 32

<210> 491
<211> 31
<212> DNA
<213> Artificial Sequence

<220>
<223> PCR primer

<400> 491
gtgcgtcata tgaatgttgc ggatctcctt t 31

<210> 492
<211> 25
<212> DNA
<213> Artificial Sequence

<220>
<223> PCR primer

<400> 492
gcgtctcgag gaaggggttg gccgt 25

<210> 493
<211> 31
<212> DNA
<213> Artificial Sequence

<220>
<223> PCR primer

<400> 493
gtgcgtcata tgtgtaatca aaagccctct t 31

<210> 494
<211> 25
<212> DNA
<213> Artificial Sequence

<220>
<223> PCR primer

<400> 494
gcgtctcgag gggctgagga ggaac 25

<210> 495
<211> 33
<212> DNA
<213> Artificial Sequence

<220>
<223> PCR primer

<400> 495
gtgcgtgcta gcaaacacta cctatcattt tct 33

<210> 496
<211> 27
<212> DNA
<213> Artificial Sequence

<220>
<223> PCR primer

<400> 496
gcgtctcgag cagaaaggct tttcttt 27

<210> 497
<211> 33
<212> DNA
<213> Artificial Sequence

<220>
<223> PCR primer

<400> 497
gtgcgtcata tgaatttagg ctatgttaat tta 33

<210> 498
<211> 28
<212> DNA
<213> Artificial Sequence

<220>
<223> PCR primer

<400> 498
gcgtctcgag gttttgtttt ttgaaaga 28

<210> 499
<211> 28
<212> DNA
<213> Artificial Sequence

<220>
<223> PCR primer

<400> 499
gtgcgtcata tggcagcatc aggaggca 28

<210> 500
<211> 30
<212> DNA
<213> Artificial Sequence

<220>
<223> PCR primer

<400> 500
gcgtctcgag tgaccaagga tagggtttag 30

<210> 501
<211> 29
<212> DNA
<213> Artificial Sequence

<220>
<223> PCR primer

<400> 501
gtgcgtcata tgcctcgtgg tgacacttt 29

<210> 502
<211> 25
<212> DNA
<213> Artificial Sequence

<220>
<223> PCR primer

<400> 502
gcgtctcgag cgctgcttct tgctc 25

<210> 503
<211> 31
<212> DNA
<213> Artificial Sequence

<220>
<223> PCR primer

<400> 503
gtgcgtcata tgtgctctca aaaaacgaca a 31

<210> 504
<211> 25
<212> DNA
<213> Artificial Sequence

<220>

<223> PCR primer

<400> 504
gcgtctcgag tgaagaggcg ccatc 25

<210> 505
<211> 27
<212> DNA
<213> Artificial Sequence

<220>
<223> PCR primer

<400> 505
gtgcgtcata tggatgcgaa aatggga 27

<210> 506
<211> 28
<212> DNA
<213> Artificial Sequence

<220>
<223> PCR primer

<400> 506
gcgtctcgag tctttgacat tcaagagc 28

<210> 507
<211> 30
<212> DNA
<213> Artificial Sequence

<220>
<223> PCR primer

<400> 507
gtgcgtcata tgattcctac catgttaatg 30

<210> 508
<211> 31
<212> DNA
<213> Artificial Sequence

<220>
<223> PCR primer

<400> 508
gcgtctcgag gtcatacaat ttccttatat a 31

<210> 509
<211> 28
<212> DNA
<213> Artificial Sequence

<220>
<223> PCR primer

<400> 509
gtgcgtcata tgtgcactca cttaggct 28

<210> 510

<211> 28
<212> DNA
<213> Artificial Sequence

<220>
<223> PCR primer

<400> 510
gcgtctcgag cgagtagtta gcacaaac 28

<210> 511
<211> 30
<212> DNA
<213> Artificial Sequence

<220>
<223> PCR primer

<400> 511
gtgcgtgcta gcaagacaat cgtagcttca 30

<210> 512
<211> 31
<212> DNA
<213> Artificial Sequence

<220>
<223> PCR primer

<400> 512
actcgctagc ggccgcggct ggcatatagg t 31

<210> 513
<211> 34
<212> DNA
<213> Artificial Sequence

<220>
<223> PCR primer

<400> 513
gtgcgtgcta gcaaatcaag atgttctatt gata 34

<210> 514
<211> 26
<212> DNA
<213> Artificial Sequence

<220>
<223> PCR primer

<400> 514
gcgtctcgag tccaaaacaa ccctct 26

<210> 515
<211> 35
<212> DNA
<213> Artificial Sequence

<220>
<223> PCR primer

<400> 515
gtgcgtcata tgtgcgtaag ttatattaat tcctt 35

<210> 516
<211> 25
<212> DNA
<213> Artificial Sequence

<220>
<223> PCR primer

<400> 516
gcgtctcgag cagtcgggct tgttg 25

<210> 517
<211> 28
<212> DNA
<213> Artificial Sequence

<220>
<223> PCR primer

<400> 517
gtgcgtcata tgtcggatct tttacgag 28

<210> 518
<211> 32
<212> DNA
<213> Artificial Sequence

<220>
<223> PCR primer

<400> 518
gcgtctcgag ttttctacac tgttgtaata aa 32

<210> 519
<211> 27
<212> DNA
<213> Artificial Sequence

<220>
<223> PCR primer

<400> 519
gtgcgtcata tgaatcagct gctttct 27

<210> 520
<211> 28
<212> DNA
<213> Artificial Sequence

<220>
<223> PCR primer

<400> 520
gcgtctcgag agagaaggta attgtacc 28

<210> 521
<211> 36

<212> DNA
<213> Artificial Sequence

<220>
<223> PCR primer

<400> 521
gtgcgtcata tgtgtctact tattatctat ctctac 36

<210> 522
<211> 25
<212> DNA
<213> Artificial Sequence

<220>
<223> PCR primer

<400> 522
gcgtctcgag ttcagaaaaa tggct 25

<210> 523
<211> 27
<212> DNA
<213> Artificial Sequence

<220>
<223> PCR primer

<400> 523
gtgcgtcata tgtccccacg acgacaa 27

<210> 524
<211> 26
<212> DNA
<213> Artificial Sequence

<220>
<223> PCR primer

<400> 524
gcgtctcgag tcctgcagca tttagc 26

<210> 525
<211> 28
<212> DNA
<213> Artificial Sequence

<220>
<223> PCR primer

<400> 525
gtgcgtcata tgtgtgacgt acggtcta 28

<210> 526
<211> 32
<212> DNA
<213> Artificial Sequence

<220>
<223> PCR primer

<400> 526
actcgctagc ggccgcttca ccttgatttc ct 32

<210> 527
<211> 25
<212> DNA
<213> Artificial Sequence

<220>
<223> PCR primer

<400> 527
gtgcgtcata tgtgcgatgc aaaac 25

<210> 528
<211> 35
<212> DNA
<213> Artificial Sequence

<220>
<223> PCR primer

<400> 528
actcgctagc ggccgcggaa gtatgcttag atatt 35

<210> 529
<211> 30
<212> DNA
<213> Artificial Sequence

<220>
<223> PCR primer

<400> 529
gtgcgtcata tgtgctgtgg ttactctatt 30

<210> 530
<211> 36
<212> DNA
<213> Artificial Sequence

<220>
<223> PCR primer

<400> 530
actcgctagc ggccgcaaaa aggtcatagt atacct 36

<210> 531
<211> 31
<212> DNA
<213> Artificial Sequence

<220>
<223> PCR primer

<400> 531
gtgcgtcata tgaaaactgt gatattgaac a 31

<210> 532
<211> 31
<212> DNA

<213> Artificial Sequence

<220>
<223> PCR primer

<400> 532
gcgtctcgag ctgagcttct atttctatta t 31

<210> 533
<211> 27
<212> DNA
<213> Artificial Sequence

<220>
<223> PCR primer

<400> 533
gtgcgtcata tgcccattta tgggaaa 27

<210> 534
<211> 26
<212> DNA
<213> Artificial Sequence

<220>
<223> PCR primer

<400> 534
gcgtctcgag gttgagcaaa ggtttg 26

<210> 535
<211> 29
<212> DNA
<213> Artificial Sequence

<220>
<223> PCR primer

<400> 535
gtgcgtcata tgtattcgtg ttacagcaa 29

<210> 536
<211> 28
<212> DNA
<213> Artificial Sequence

<220>
<223> PCR primer

<400> 536
gcgtctcgag gaaaaattct ttagggag 28

<210> 537
<211> 27
<212> DNA
<213> Artificial Sequence

<220>
<223> PCR primer

<400> 537

gtgcgtcata tggccgctaa agcaaat 27

<210> 538
<211> 28
<212> DNA
<213> Artificial Sequence

<220>
<223> PCR primer

<400> 538
gcgtctcgag tgaaaatgaa aggatggt 28

<210> 539
<211> 32
<212> DNA
<213> Artificial Sequence

<220>
<223> PCR primer

<400> 539
gtgcgtgcta gcagtctata tcaaaaatgg tg 32

<210> 540
<211> 29
<212> DNA
<213> Artificial Sequence

<220>
<223> PCR primer

<400> 540
gcgtctcgag atctttcatt tggttatct 29

<210> 541
<211> 29
<212> DNA
<213> Artificial Sequence

<220>
<223> PCR primer

<400> 541
gtgcgtcata tgaaagattt ggggactct 29

<210> 542
<211> 29
<212> DNA
<213> Artificial Sequence

<220>
<223> PCR primer

<400> 542
gcgtctcgag gaatcctaag gcatacccta 29

<210> 543
<211> 33
<212> DNA
<213> Artificial Sequence

<220>
<223> PCR primer

<400> 543
gtgcgtgcta gcagtatagt cagaaattct gca 33

<210> 544
<211> 33
<212> DNA
<213> Artificial Sequence

<220>
<223> PCR primer

<400> 544
gcgtctcgag gaagctaaga ttatagctac ttt 33

<210> 545
<211> 25
<212> DNA
<213> Artificial Sequence

<220>
<223> PCR primer

<400> 545
gtgcgtgcta gcgcggccct ttcca 25

<210> 546
<211> 39
<212> DNA
<213> Artificial Sequence

<220>
<223> PCR primer

<400> 546
actcgctagc ggccgcttta tgtatatgga acagatagg 39

<210> 547
<211> 31
<212> DNA
<213> Artificial Sequence

<220>
<223> PCR primer

<400> 547
gtgcgtcata tgggacattt tattgatatt g 31

<210> 548
<211> 35
<212> DNA
<213> Artificial Sequence

<220>
<223> PCR primer

<400> 548
actcgctagc ggccgcatca tcaaggtaga taaag 35

<210> 549
<211> 33
<212> DNA
<213> Artificial Sequence

<220>
<223> PCR primer

<400> 549
gtgcgtcata tgggttattg ctatgtaatt aca 33

<210> 550
<211> 26
<212> DNA
<213> Artificial Sequence

<220>
<223> PCR primer

<400> 550
gcgtctcgag ttctgattgg actcca 26

<210> 551
<211> 29
<212> DNA
<213> Artificial Sequence

<220>
<223> PCR primer

<400> 551
gtgcgtcata tggtggcttt aacgatagc 29

<210> 552
<211> 30
<212> DNA
<213> Artificial Sequence

<220>
<223> PCR primer

<400> 552
actcgctagc ggccggcagc catcgtattc 30

<210> 553
<211> 26
<212> DNA
<213> Artificial Sequence

<220>
<223> PCR primer

<400> 553
gtgcgtcata tgttcaatat gcgagg 26

<210> 554
<211> 28
<212> DNA
<213> Artificial Sequence

<220>
<223> PCR primer

<400> 554
gcgtctcgag cttcttattt gaactttg 28

<210> 555
<211> 27
<212> DNA
<213> Artificial Sequence

<220>
<223> PCR primer

<400> 555
gtgcgtcata tgacagccgg agcagct 27

<210> 556
<211> 29
<212> DNA
<213> Artificial Sequence

<220>
<223> PCR primer

<400> 556
gcgtctcgag agcaccctca atttcattg 29

<210> 557
<211> 33
<212> DNA
<213> Artificial Sequence

<220>
<223> PCR primer

<400> 557
gtgcgtcata tgggatatgt tttctatgtg atc 33

<210> 558
<211> 28
<212> DNA
<213> Artificial Sequence

<220>
<223> PCR primer

<400> 558
gcgtctcgag gctactaaat cgaatcga 28

<210> 559
<211> 30
<212> DNA
<213> Artificial Sequence

<220>
<223> PCR primer

<400> 559
gtgcgtcata tgatccctgg attaagttca 30

<210> 560
<211> 32
<212> DNA
<213> Artificial Sequence

<220>
<223> PCR primer

<400> 560
actcgctagc ggccgcttca ctgggagctt ga 32

<210> 561
<211> 31
<212> DNA
<213> Artificial Sequence

<220>
<223> PCR primer

<400> 561
gtgcgtcata tgtaccagga gaatctaaga t 31

<210> 562
<211> 34
<212> DNA
<213> Artificial Sequence

<220>
<223> PCR primer

<400> 562
actcgctagc ggccgcgatt ttcttcttca gctc 34

<210> 563
<211> 30
<212> DNA
<213> Artificial Sequence

<220>
<223> PCR primer

<400> 563
gtgcgtcata tggaggaggt gtctgagtat 30

<210> 564
<211> 37
<212> DNA
<213> Artificial Sequence

<220>
<223> PCR primer

<400> 564
actcgctagc ggccgcatgt ttctttttac tctttct 37

<210> 565
<211> 27
<212> DNA
<213> Artificial Sequence

<220>

<223> PCR primer

<400> 565
gtgcgtcata tggctccagt ccgtgtt 27

<210> 566
<211> 27
<212> DNA
<213> Artificial Sequence

<220>
<223> PCR primer

<400> 566
gcgtctcgag aagtgttcgt tggaagt 27

<210> 567
<211> 32
<212> DNA
<213> Artificial Sequence

<220>
<223> PCR primer

<400> 567
gtgcgtcata tgaataagct actcaatttc gt 32

<210> 568
<211> 29
<212> DNA
<213> Artificial Sequence

<220>
<223> PCR primer

<400> 568
gcgtctcgag gaaaatctga attcttcct 29

<210> 569
<211> 30
<212> DNA
<213> Artificial Sequence

<220>
<223> PCR primer

<400> 569
gtgcgtcata tgttaaattc aagcaattca 30

<210> 570
<211> 29
<212> DNA
<213> Artificial Sequence

<220>
<223> PCR primer

<400> 570
gcgtctcgag aggaactaaa acctcatct 29

<210> 571

<211> 30
<212> DNA
<213> Artificial Sequence

<220>
<223> PCR primer

<400> 571
gtgcgtgcta gcgttttatt tcatgctcaa 30

<210> 572
<211> 40
<212> DNA
<213> Artificial Sequence

<220>
<223> PCR primer

<400> 572
actcgctagc ggccgcctta gaaagactat tttctaagta 40

<210> 573
<211> 26
<212> DNA
<213> Artificial Sequence

<220>
<223> PCR primer

<400> 573
gtgcgtcata tgtgcgtgat aatggg 26

<210> 574
<211> 29
<212> DNA
<213> Artificial Sequence

<220>
<223> PCR primer

<400> 574
gcgtctcgag attcatcttc gtaaagaat 29

<210> 575
<211> 26
<212> DNA
<213> Artificial Sequence

<220>
<223> PCR primer

<400> 575
gtgcgtcata tggcagttgg tggcgt 26

<210> 576
<211> 31
<212> DNA
<213> Artificial Sequence

<220>
<223> PCR primer

<400> 576
actcgctagc ggccgcctgt ccctctggag c 31

<210> 577
<211> 30
<212> DNA
<213> Artificial Sequence

<220>
<223> PCR primer

<400> 577
gtgcgtgcta gcagtgaaca caaaaaatca 30

<210> 578
<211> 35
<212> DNA
<213> Artificial Sequence

<220>
<223> PCR primer

<400> 578
actcgctagc ggccgcctta tcgtcgttat caata 35

<210> 579
<211> 29
<212> DNA
<213> Artificial Sequence

<220>
<223> PCR primer

<400> 579
gtgcgtcata tgcatgacgc acttctaag 29

<210> 580
<211> 23
<212> DNA
<213> Artificial Sequence

<220>
<223> PCR primer

<400> 580
gcgtctcgag tacagctgcg cga 23

<210> 581
<211> 31
<212> DNA
<213> Artificial Sequence

<220>
<223> PCR primer

<400> 581
gtgcgtcata tggttatggg aacctatatc g 31

<210> 582
<211> 30

<212> DNA
<213> Artificial Sequence

<220>
<223> PCR primer

<400> 582
gcgtctcgag tacatttgta ttgatttcag 30

<210> 583
<211> 27
<212> DNA
<213> Artificial Sequence

<220>
<223> PCR primer

<400> 583
gtgcgtcata tgaacgtccc tgattcc 27

<210> 584
<211> 25
<212> DNA
<213> Artificial Sequence

<220>
<223> PCR primer

<400> 584
gcgtctcgag gctagcggct ctttc 25

<210> 585
<211> 28
<212> DNA
<213> Artificial Sequence

<220>
<223> PCR primer

<400> 585
gtgcgtcata tgcagaagca tccttcct 28

<210> 586
<211> 33
<212> DNA
<213> Artificial Sequence

<220>
<223> PCR primer

<400> 586
actcgctagc ggccgctcct ctttaggaaa tgg 33

<210> 587
<211> 29
<212> DNA
<213> Artificial Sequence

<220>
<223> PCR primer

<400> 587
gtgcgtcata tgtcctcttt aggaaatgg 29

<210> 588
<211> 26
<212> DNA
<213> Artificial Sequence

<220>
<223> PCR primer

<400> 588
gcgtctcgag cagtgccaag taggga 26

<210> 589
<211> 31
<212> DNA
<213> Artificial Sequence

<220>
<223> PCR primer

<400> 589
gtgcgtcata tggcagtacg attaattgtt g 31

<210> 590
<211> 35
<212> DNA
<213> Artificial Sequence

<220>
<223> PCR primer

<400> 590
gcgtctcgag tttattgtag tctattttat atttc 35

<210> 591
<211> 29
<212> DNA
<213> Artificial Sequence

<220>
<223> PCR primer

<400> 591
gtgcgtgcta gcagcagaaa agacaatga 29

<210> 592
<211> 27
<212> DNA
<213> Artificial Sequence

<220>
<223> PCR primer

<400> 592
gcgtctcgag attttgagtg tcttgca 27

<210> 593
<211> 30
<212> DNA

<213> Artificial Sequence

<220>
<223> PCR primer

<400> 593
gtgcgtcata tgattaccat aaatcacgtg 30

<210> 594
<211> 28
<212> DNA
<213> Artificial Sequence

<220>
<223> PCR primer

<400> 594
gcgtctcgag tatccatcga cttatagc 28

<210> 595
<211> 30
<212> DNA
<213> Artificial Sequence

<220>
<223> PCR primer

<400> 595
gtgcgtgcta gctgtatttt cccttacgta 30

<210> 596
<211> 33
<212> DNA
<213> Artificial Sequence

<220>
<223> PCR primer

<400> 596
actcgctagc ggccgcggat tctgcatact ctg 33

<210> 597
<211> 30
<212> DNA
<213> Artificial Sequence

<220>
<223> PCR primer

<400> 597
gtgcgtcata tgtctcctct tcctaaaaaa 30

<210> 598
<211> 27
<212> DNA
<213> Artificial Sequence

<220>
<223> PCR primer

<400> 598

gcgtctcgag ggattcatta ctgacca 27

<210> 599
<211> 27
<212> DNA
<213> Artificial Sequence

<220>
<223> PCR primer

<400> 599
gtgcgtcata tgaaataccg cttcacg 27

<210> 600
<211> 27
<212> DNA
<213> Artificial Sequence

<220>
<223> PCR primer

<400> 600
gcgtctcgag attctgtagg gctacgt 27

<210> 601
<211> 32
<212> DNA
<213> Artificial Sequence

<220>
<223> PCR primer

<400> 601
gtgcgtcata tggtacactt ctctcataac cc 32

<210> 602
<211> 29
<212> DNA
<213> Artificial Sequence

<220>
<223> PCR primer

<400> 602
gcgtctcgag taagtttgta ttgcggtat 29

<210> 603
<211> 33
<212> DNA
<213> Artificial Sequence

<220>
<223> PCR primer

<400> 603
gtgcgtcata tgttgttatt agggacttta gga 33

<210> 604
<211> 23
<212> DNA
<213> Artificial Sequence

<220>
<223> PCR primer

<400> 604
gcgtctcgag tttcccaacc gca 23

<210> 605
<211> 25
<212> DNA
<213> Artificial Sequence

<220>
<223> PCR primer

<400> 605
gtgcgtcata tggctgcgaa tgctc 25

<210> 606
<211> 31
<212> DNA
<213> Artificial Sequence

<220>
<223> PCR primer

<400> 606
gcgtctcgag taatttaata ctctttgaag g 31

<210> 607
<211> 32
<212> DNA
<213> Artificial Sequence

<220>
<223> PCR primer

<400> 607
gtgcgtcata tgcctactca agttaaaaca ga 32

<210> 608
<211> 30
<212> DNA
<213> Artificial Sequence

<220>
<223> PCR primer

<400> 608
gcgtctcgag aagtttatat ttcagcactt 30

<210> 609
<211> 29
<212> DNA
<213> Artificial Sequence

<220>
<223> PCR primer

<400> 609
gtgcgtgcta gccatatagg attttgcca 29

<210> 610
<211> 27
<212> DNA
<213> Artificial Sequence

<220>
<223> PCR primer

<400> 610
gcgtctcgag gtacttagca aagcgat 27

<210> 611
<211> 31
<212> DNA
<213> Artificial Sequence

<220>
<223> PCR primer

<400> 611
gtgcgtgcta gcaagaagct atatcaccct a 31

<210> 612
<211> 24
<212> DNA
<213> Artificial Sequence

<220>
<223> PCR primer

<400> 612
gcgtctcgag cacaccgagg aaac 24

<210> 613
<211> 33
<212> DNA
<213> Artificial Sequence

<220>
<223> PCR primer

<400> 613
gtgcgtcata tggtagtttc agaagaaaaa gtc 33

<210> 614
<211> 25
<212> DNA
<213> Artificial Sequence

<220>
<223> PCR primer

<400> 614
gcgtctcgag acgtatgcgc aactg 25

<210> 615
<211> 30
<212> DNA
<213> Artificial Sequence

<220>
<223> PCR primer

<400> 615
gtgcgtcata tggaagtatt agaccgctct 30

<210> 616
<211> 25
<212> DNA
<213> Artificial Sequence

<220>
<223> PCR primer

<400> 616
gcgtctcgag cgagaaaaag cttcc 25

<210> 617
<211> 29
<212> DNA
<213> Artificial Sequence

<220>
<223> PCR primer

<400> 617
gtgcgtcata tgatgaagaa aattcgaaa 29

<210> 618
<211> 33
<212> DNA
<213> Artificial Sequence

<220>
<223> PCR primer

<400> 618
actcgctagc ggccgctaag cattcacaaa tga 33

<210> 619
<211> 30
<212> DNA
<213> Artificial Sequence

<220>
<223> PCR primer

<400> 619
gtgcgtcata tgcatatttt gcttgatcgt 30

<210> 620
<211> 33
<212> DNA
<213> Artificial Sequence

<220>
<223> PCR primer

<400> 620
gcgtctcgag tcttttaact aaatcttgtt ctt 33

<210> 621
<211> 32
<212> DNA
<213> Artificial Sequence

<220>
<223> PCR primer

<400> 621
gtgcgtcata tgcttgtcta ttgttttgat cc 32

<210> 622
<211> 28
<212> DNA
<213> Artificial Sequence

<220>
<223> PCR primer

<400> 622
gcgtctcgag aaaatatacg gaactcgc 28

<210> 623
<211> 31
<212> DNA
<213> Artificial Sequence

<220>
<223> PCR primer

<400> 623
gtgcgtgcta gcgaagttta tagtttttcc c 31

<210> 624
<211> 29
<212> DNA
<213> Artificial Sequence

<220>
<223> PCR primer

<400> 624
gcgtctcgag tttttgattc cttaagaag 29

<210> 625
<211> 33
<212> DNA
<213> Artificial Sequence

<220>
<223> PCR primer

<400> 625
gtgcgtcata tggaagttta tagttttcac cct 33

<210> 626
<211> 27
<212> DNA
<213> Artificial Sequence

<220>

<223> PCR primer

<400> 626
gcgtctcgag actccttgag aagggaa 27

<210> 627
<211> 31
<212> DNA
<213> Artificial Sequence

<220>
<223> PCR primer

<400> 627
gtgcgtcata tgcttaatca tgctaaaaag c 31

<210> 628
<211> 36
<212> DNA
<213> Artificial Sequence

<220>
<223> PCR primer

<400> 628
actcgctagc ggccgcctct tttattttag gaagct 36

<210> 629
<211> 34
<212> DNA
<213> Artificial Sequence

<220>
<223> PCR primer

<400> 629
gtgcgtcata tgaaaaaaaa atttattttc tact 34

<210> 630
<211> 33
<212> DNA
<213> Artificial Sequence

<220>
<223> PCR primer

<400> 630
actcgctagc ggccgccaca ctctgttctt ctg 33

<210> 631
<211> 32
<212> DNA
<213> Artificial Sequence

<220>
<223> PCR primer

<400> 631
gtgcgtcata tgttttctaa ggatttgact aa 32

<210> 632

<211> 25
<212> DNA
<213> Artificial Sequence

<220>
<223> PCR primer

<400> 632
gcgtctcgag cgaagcagaa gtcgt 25

<210> 633
<211> 30
<212> DNA
<213> Artificial Sequence

<220>
<223> PCR primer

<400> 633
gtgcgtcata tgaatatgcc tgttccttct 30

<210> 634
<211> 25
<212> DNA
<213> Artificial Sequence

<220>
<223> PCR primer

<400> 634
gcgtctcgag ggggcgtagg ttgta 25

<210> 635
<211> 27
<212> DNA
<213> Artificial Sequence

<220>
<223> PCR primer

<400> 635
gtgcgtcata tgtgttccct ggatcct 27

<210> 636
<211> 37
<212> DNA
<213> Artificial Sequence

<220>
<223> PCR primer

<400> 636
actcgctagc ggccgcagtt atcactatat ccacaag 37

<210> 637
<211> 33
<212> DNA
<213> Artificial Sequence

<220>
<223> PCR primer

<400> 637
gtgcgtgcta gccttgaaca ttctaaacaa gat 33

<210> 638
<211> 30
<212> DNA
<213> Artificial Sequence

<220>
<223> PCR primer

<400> 638
gcgtctcgag acgtagttta agagcagact 30

<210> 639
<211> 31
<212> DNA
<213> Artificial Sequence

<220>
<223> PCR primer

<400> 639
gtgcgtcata tgtgtctatc tgcctacata g 31

<210> 640
<211> 27
<212> DNA
<213> Artificial Sequence

<220>
<223> PCR primer

<400> 640
gcgtctcgag ttttgatgct tctttca 27

<210> 641
<211> 29
<212> DNA
<213> Artificial Sequence

<220>
<223> PCR primer

<400> 641
gtgcgtcata tggaccagaa aattgaaaa 29

<210> 642
<211> 27
<212> DNA
<213> Artificial Sequence

<220>
<223> PCR primer

<400> 642
gcgtctcgag agaggtcttc tgagtgc 27

<210> 643
<211> 32

<212> DNA
<213> Artificial Sequence

<220>
<223> PCR primer

<400> 643
gtgcgtcata tgaatttcca ttgtagtgta gt 32

<210> 644
<211> 27
<212> DNA
<213> Artificial Sequence

<220>
<223> PCR primer

<400> 644
gcgtctcgag gaacagttcg atttgtg 27

<210> 645
<211> 29
<212> DNA
<213> Artificial Sequence

<220>
<223> PCR primer

<400> 645
gtgcgtcata tgcttccttt atcagggca 29

<210> 646
<211> 32
<212> DNA
<213> Artificial Sequence

<220>
<223> PCR primer

<400> 646
actcgctagc ggccgcttct tcaggtttca gg 32

<210> 647
<211> 27
<212> DNA
<213> Artificial Sequence

<220>
<223> PCR primer

<400> 647
gtgcgtgcta gccgttatgc cgaggtc 27

<210> 648
<211> 25
<212> DNA
<213> Artificial Sequence

<220>
<223> PCR primer

<400> 648
gcgtctcgag ttcgtgcatt tggtg 25

<210> 649
<211> 29
<212> DNA
<213> Artificial Sequence

<220>
<223> PCR primer

<400> 649
gtgcgtcata tgttgaaaat ccagaaaaa 29

<210> 650
<211> 27
<212> DNA
<213> Artificial Sequence

<220>
<223> PCR primer

<400> 650
gcgtctcgag attcattttc ggaagag 27

<210> 651
<211> 32
<212> DNA
<213> Artificial Sequence

<220>
<223> PCR primer

<400> 651
gtgcgtcata tgagacgtta tcttttcatg gt 32

<210> 652
<211> 29
<212> DNA
<213> Artificial Sequence

<220>
<223> PCR primer

<400> 652
gcgtctcgag ccctttgctc tttacatag 29

<210> 653
<211> 31
<212> DNA
<213> Artificial Sequence

<220>
<223> PCR primer

<400> 653
gtgcgtacta gttgtcacct acagtcacta g 31

<210> 654
<211> 26
<212> DNA

```
<213>   Artificial Sequence

<220>
<223>   PCR primer

<400>   654
gcgtctcgag gaatcggagt ttggta                                          26

<210>   655
<211>   30
<212>   DNA
<213>   Artificial Sequence

<220>
<223>   PCR primer

<400>   655
gtgcgtacta gtaagtcctc tgtctcttgg                                      30

<210>   656
<211>   29
<212>   DNA
<213>   Artificial Sequence

<220>
<223>   PCR primer

<400>   656
gcgtctcgag gaaacaaaac ttagagccc                                       29

<210>   657
<211>   168
<212>   DNA
<213>   Artificial Sequence

<220>
<223>   Restriction linker

<400>   657
gatcccatat ggctagcccg gggaattcgt ccatggagtg agtcgactga ctcgagtgat    60
cgagctcctg agcggccgca tgaaggtata ccgatcgggc cccttaagca ggtacctcac   120
tcagctgact gagctcacta gctcgaggac tcgccggcgt actttcga                168

<210>   658
<211>   96
<212>   DNA
<213>   Artificial Sequence

<220>
<223>   Restriction linker

<400>   658
tcgacaagct tgcggccgca ctcgagcatc accatcacca tcactgatgt tcgaacgccg    60
gcgtgagcac gtagaggtag tggtagtgac tatcga                              96

<210>   659
<211>   12
<212>   DNA
<213>   Artificial Sequence

<220>
```

<223> Primer tail

<400> 659
gtgcgtcata tg 12

<210> 660
<211> 10
<212> DNA
<213> Artificial Sequence

<220>
<223> Primer tail

<400> 660
gcgtctcgag 10

<210> 661
<211> 12
<212> DNA
<213> Artificial Sequence

<220>
<223> Primer tail

<400> 661
gtgcgtgcta gc 12

<210> 662
<211> 16
<212> DNA
<213> Artificial Sequence

<220>
<223> Primer tail

<400> 662
actcgctagc ggccgc 16

<210> 663
<211> 12
<212> DNA
<213> Artificial Sequence

<220>
<223> Primer tail

<400> 663
gtgcgtacta gt 12

<210> 664
<211> 10
<212> DNA
<213> Artificial Sequence

<220>
<223> Primer tail

<400> 664
gcgtaagctt 10